(19) Europäisches Patentamt
European Patent Office
Office européen des brevets

(11) EP 2 370 467 B1

(12) **EUROPEAN PATENT SPECIFICATION**

(45) Date of publication and mention of the grant of the patent:
**07.09.2016 Bulletin 2016/36**

(21) Application number: **09740078.2**

(22) Date of filing: **01.10.2009**

(51) Int Cl.:
***C07K 16/28*** $^{(2006.01)}$ ***C07K 16/22*** $^{(2006.01)}$
***C07K 16/30*** $^{(2006.01)}$ ***C07K 16/40*** $^{(2006.01)}$

(86) International application number:
**PCT/EP2009/062792**

(87) International publication number:
**WO 2010/037835 (08.04.2010 Gazette 2010/14)**

# (54) CROSS-SPECIES-SPECIFIC PSCAXCD3, CD19XCD3, C-METXCD3, ENDOSIALINXCD3, EPCAMXC D3, IGF-1RXCD3 OR FAPALPHA XCD3 BISPECIFIC SINGLE CHAIN ANTIBODY

CROSS-SPECIES-SPEZIFISCHE BISPEZIFISCHE PSCAXCD3-, CD19XCD3-, C-METXCD3-, ENDOSIALINXCD3-, EPCAMXC D3-, IGF-1RXCD3- ODER FAPALPHA-XCD3-EINZELKETTENANTIKÖRPER

ANTICORPS MONOCATÉNAIRE BISPÉCIFIQUE PSCAXCD3 (CD19XCD3, C-METXCD3, ENDOSIALINXCD3, EPCAMXC D3, IGF-1RXCD3 OU FAPALPHA XCD3), SPÉCIFIQUE D'ESPÈCES CROISÉES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority:
**01.10.2008 US 101850 P**
**01.10.2008 US 101853 P**
**01.10.2008 US 101846 P**
**01.10.2008 US 101844 P**
**01.10.2008 US 101927 P**
**01.10.2008 US 101921 P**
**01.10.2008 US 101933 P**

(43) Date of publication of application:
**05.10.2011 Bulletin 2011/40**

(73) Proprietor: **Amgen Research (Munich) GmbH**
**81477 München (DE)**

(72) Inventors:
- **KUFER, Peter**
  **81477 Munich (DE)**
- **RAUM, Tobias**
  **81477 Munich (DE)**
- **KISCHEL, Roman**
  **81477 Munich (DE)**
- **LUTTERBÜSE, Ralf**
  **81477 Munich (DE)**
- **HOFFMANN, Patrick**
  **81477 Munich (DE)**
- **RAU, Doris**
  **81477 Munich (DE)**
- **KLINGER, Matthias**
  **81477 Munich (DE)**
- **MANGOLD, Susanne**
  **81477 Munich (DE)**
- **BLÜMEL, Claudia**
  **81477 Munich (DE)**

(74) Representative: **Schiweck, Weinzierl & Koch**
**European Patent Attorneys**
**Landsberger Straße 98**
**80339 München (DE)**

(56) References cited:
**WO-A1-2004/106381**
**WO-A1-2006/008096**
**WO-A2-2007/042261**
**WO-A2-2008/119565**
**WO-A2-2008/119566**
**WO-A2-2008/119567**
**NO-A- 20 064 183**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MALETZ K ET AL: "Bispecific single-chain antibodies as effective tools for eliminating epithelial cancer cells from human stem cell preparations by redirected cell cytotoxicity" INTERNATIONAL JOURNAL OF CANCER, JOHN WILEY & SONS, INC, UNITED STATES, SWITZERLAND, GERMANY LNKD- DOI:10.1002/IJC.1348, vol. 93, no. 3, 1 August 2001 (2001-08-01) , pages 409-416, XP002301955 ISSN: 0020-7136**
- **WOLF ET AL: "BiTEs: bispecific antibody constructs with unique anti-tumor activity" DRUG DISCOVERY TODAY, ELSEVIER, RAHWAY, NJ, US LNKD- DOI:10.1016/S1359-6446(05)03554-3, vol. 10, no. 18, 15 September 2005 (2005-09-15), pages 1237-1244, XP005103829 ISSN: 1359-6446**
- **ANOMYNOUS: "Phase II Study of the BiTE TM Blinatumomab (MT103) in Patients With Minimal Residual Disease of B-precursor Acute ALL" INTERNET CITATION 11 August 2008 (2008-08-11), pages 1-3, XP002572438 Retrieved from the Internet: URL:http://clinicaltrials.gov/archive/NCT00560794/2008_08_11> [retrieved on 2010-03-10]**
- **IM S Y ET AL: "Generation of a rabbit VH domain antibody polyspecific to c-Met and adenoviral knob protein", BIOCHEMICAL AND BIOPHYSICAL RESEARCH COMMUNICATIONS, ACADEMIC PRESS INC. ORLANDO, FL, US, vol. 339, no. 1, 6 January 2006 (2006-01-06), pages 305-312, XP024923457, ISSN: 0006-291X, DOI: 10.1016/J.BBRC.2005.10.208 [retrieved on 2006-01-06]**
- **WUEST T ET AL: "Construction of a bispecific single chain antibody for recruitment of cytotoxic T cells to the tumour stroma associated antigen fibroblast activation protein", JOURNAL OF BIOTECHNOLOGY, ELSEVIER SCIENCE PUBLISHERS, AMSTERDAM, NL, vol. 92, no. 2, 28 December 2001 (2001-12-28), pages 159-168, XP009099258, ISSN: 0168-1656**

## Description

The present invention relates to a bispecific single chain antibody molecule comprising a first binding domain which specifically binds to an epitope of human and Callithrix jacchus, Saguinus oedipus or Saimiri sciureus CD3 epsilon chain, wherein said epitope is part of an amino acid sequence comprised in the group consisting of SEQ ID NOs. 2, 4, 6, and 8 and comprises at least the amino acid sequence Gln-Asp-Gly-Asn-Glu, and a second binding domain binding to an antigen selected from the group consisting of Prostate Stem Cell Antigen (PSCA), B-Lymphocyte antigen CD19 (CD19), hepatocyte growth factor receptor (C-MET), Endosialin, the EGF-like domain 1 of EpCAM, encoded by exon 2, Fibroblast activation protein alpha (FAP alpha) and Insulin-like growth factor I receptor (IGF-IR or IGF-1R). The invention also provides nucleic acids encoding said bispecific single chain antibody molecule as well as vectors and host cells and a process for its production. The invention further relates to pharmaceutical compositions comprising said bispecific single chain antibody molecule and medical uses of said bispecific single chain antibody molecule.

T cell recognition is mediated by clonotypically distributed alpha beta and gamma delta T cell receptors (TcR) that interact with the peptide-loaded molecules of the peptide MHC (pMHC) (Davis & Bjorkman, Nature 334 (1988), 395-402). The antigen-specific chains of the TcR do not possess signalling domains but instead are coupled to the conserved multisubunit signalling apparatus CD3 (Call, Cell 111 (2002), 967-979, Alarcon, Immunol. Rev. 191 (2003), 38-46, Malissen Immunol. Rev. 191 (2003), 7-27). The mechanism by which TcR ligation is directly communicated to the signalling apparatus remains a fundamental question in T cell biology (Alarcon, loc. cit.; Davis, Cell 110 (2002), 285-287). It seems clear that sustained T cell responses involve coreceptor engagement, TcR oligomerization, and a higher order arrangement of TcR-pMHC complexes in the immunological synapse (Davis & van der Merwe, Curr. Biol. 11 (2001), R289-R291, Davis, Nat. Immunol. 4 (2003), 217-224). However very early TcR signalling occurs in the absence of these events and may involve a ligand-induced conformational change in CD3 epsilon (Alarcon, loc. cit., Davis (2002), loc. cit., Gil, J. Biol. Chem. 276 (2001), 11174-11179, Gil, Cell 109 (2002), 901-912). The epsilon, gamma, delta and zeta subunits of the signalling complex associate with each other to form a CD3 epsilon-gamma heterodimer, a CD3 epsilon-delta heterodimer, and a CD3 zeta-zeta homodimer (Call, loc. cit.). Various studies have revealed that the CD3 molecules are important for the proper cell surface expression of the alpha beta TcR and normal T cell development (Berkhout, J. Biol. Chem. 263 (1988), 8528-8536, Wang, J. Exp. Med. 188 (1998), 1375-1380, Kappes, Curr. Opin. Immunol. 7 (1995), 441-447). The solution structure of the ectodomain fragments of the mouse CD3 epsilon gamma heterodimer showed that the epsilon gamma subunits are both C2-set Ig domains that interact with each other to form an unusual side-to-side dimer configuration (Sun, Cell 105 (2001), 913-923). Although the cysteine-rich stalk appears to play an important role in driving CD3 dimerization (Su, loc. cit., Borroto, J. Biol. Chem. 273 (1998), 12807-12816), interaction by means of the extracellular domains of CD3 epsilon and CD3 gamma is sufficient for assembly of these proteins with TcR beta (Manolios, Eur. J. Immunol. 24 (1994), 84-92, Manolios & Li, Immunol. Cell Biol. 73 (1995), 532-536). Although still controversial, the dominant stoichiometry of the TcR most likely comprises one alpha beta TcR, one CD3 epsilon gamma heterodimer, one CD3 epsilon delta heterodimer and one CD3 zeta zeta homodimer (Call, loc. cit.). Given the central role of the human CD3 epsilon gamma heterodimer in the immune response, the crystal structure of this complex bound to the therapeutic antibody OKT3 has recently been elucidated (Kjer-Nielsen, PNAS 101, (2004), 7675-7680).

A number of therapeutic strategies modulate T cell immunity by targeting TcR signalling, particularly the anti-human CD3 monoclonal antibodies (mAbs) that are widely used clinically in immunosuppressive regimes. The CD3-specific mouse mAb OKT3 was the first mAb licensed for use in humans (Sgro, Toxicology 105 (1995), 23-29) and is widely used clinically as an immunosuppressive agent in transplantation (Chatenoud, Clin. Transplant 7 (1993), 422-430, Chatenoud, Nat. Rev. Immunol. 3 (2003), 123-132, Kumar, Transplant. Proc. 30 (1998), 1351-1352), type 1 diabetes (Chatenoud (2003), loc. cit.), and psoriasis (Utset, J. Rheumatol. 29 (2002), 1907-1913). Moreover, anti-CD3 mAbs can induce partial T cell signalling and clonal anergy (Smith, J. Exp. Med. 185 (1997), 1413-1422). OKT3 has been described in the literature as a potent T cell mitogen (Van Wauve, J. Immunol. 124 (1980), 2708-18) as well as a potent T cell killer (Wong, Transplantation 50 (1990), 683-9). OKT3 exhibits both of these activities in a time-dependent fashion; following early activation of T cells leading to cytokine release, upon further administration OKT3 later blocks all known T cell functions. It is due to this later blocking of T cell function that OKT3 has found such wide application as an immunosuppressant in therapy regimens for reduction or even abolition of allograft tissue rejection.

OKT3 reverses allograft tissue rejection most probably by blocking the function of all T cells, which play a major role in acute rejection. OKT3 reacts with and blocks the function of the CD3 complex in the membrane of human T cells, which is associated with the antigen recognition structure of T cells (TCR) and is essential for signal transduction. Which subunit of the TCR/CD3 is bound by OKT3 has been the subject of multiple studies. Though some evidence has pointed to a specificity of OKT3 for the epsilon-subunit of the TCR/CD3 complex (Tunnacliffe, Int. Immunol. 1 (1989), 546-50; Kjer-Nielsen, PNAS 101, (2004), 7675-7680). Further evidence has shown that OKT3 binding of the TCR/CD3 complex requires other subunits of this complex to be present (Salmeron, J. Immunol. 147 (1991), 3047-52).

Other well known antibodies specific for the CD3 molecule are listed in Tunnacliffe, Int. Immunol. 1 (1989), 546-50. As indicated above, such CD3 specific antibodies are able to induce various T cell responses such as lymphokine production (Von Wussow, J. Immunol. 127 (1981), 1197; Palacious, J. Immunol. 128 (1982), 337), proliferation (Van Wauve, J. Immunol. 124 (1980), 2708-18) and suppressor-T cell induction (Kunicka, in "Lymphocyte Typing II" 1 (1986), 223). That is, depending on the experimental conditions, CD3 specific monoclonal antibody can either inhibit or induce cytotoxicity (Leewenberg, J. Immunol. 134 (1985), 3770; Phillips, J. Immunol. 136 (1986) 1579; Platsoucas, Proc. Natl. Acad. Sci. USA 78 (1981), 4500; Itoh, Cell. Immunol. 108 (1987), 283-96; Mentzer, J. Immunol. 135 (1985), 34; Landegren, J. Exp. Med. 155 (1982), 1579; Choi (2001), Eur. J. Immunol. 31, 94-106; Xu (2000), Cell Immunol. 200, 16-26; Kimball (1995), Transpl. Immunol. 3, 212-221).

Although many of the CD3 antibodies described in the art have been reported to recognize the CD3 epsilon subunit of the CD3 complex, most of them bind in fact to conformational epitopes and, thus, only recognize CD3 epsilon in the native context of the TCR. Conformational epitopes are characterized by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The conformational epitopes bound by CD3 epsilon antibodies described in the art may be separated in two groups. In the major group, said epitopes are being formed by two CD3 subunits, e.g. of the CD3 epsilon chain and the CD3 gamma or CD3 delta chain. For example, it has been found in several studies that the most widely used CD3 epsilon monoclonal antibodies OKT3, WT31, UCHT1, 7D6 and Leu-4 did not bind to cells singly transfected with the CD3-epsilon chain. However, these antibodies stained cells doubly transfected with a combination of CD3 epsilon plus either CD3 gamma or CD3 delta (Tunnacliffe, loc. cit.; Law, Int. Immunol. 14 (2002), 389-400; Salmeron, J. Immunol. 147 (1991), 3047-52; Coulie, Eur. J. Immunol. 21 (1991), 1703-9). In a second smaller group, the conformational epitope is being formed within the CD3 epsilon subunit itself. A member of this group is for instance mAb APA 1/1 which has been raised against denatured CD3 epsilon (Risueno, Blood 106 (2005), 601-8). Taken together, most of the CD3 epsilon antibodies described in the art recognize conformational epitopes located on two or more subunits of CD3. The discrete amino acid residues forming the three-dimensional structure of these epitopes may hereby be located either on the CD3 epsilon subunit itself or on the CD3 epsilon subunit and other CD3 subunits such as CD3 gamma or CD3 delta.

Another problem with respect to CD3 antibodies is that many CD3 antibodies have been found to be species-specific. Anti-CD3 monoclonal antibodies - as holds true generally for any other monoclonal antibodies - function by way of highly specific recognition of their target molecules. They recognize only a single site, or epitope, on their target CD3 molecule. For example, one of the most widely used and best characterized monoclonal antibodies specific for the CD3 complex is OKT-3. This antibody reacts with chimpanzee CD3 but not with the CD3 homolog of other primates, such as macaques, or with dog CD3 (Sandusky et al., J. Med. Primatol. 15 (1986), 441-451). Similarly, WO2005/118635 or WO2007/033230 describe human monoclonal CD3 epsilon antibodies which react with human CD3 epsilon but not with CD3 epsilon of mouse, rat, rabbit, or non-chimpanzee primates, such as rhesus monkey, cynomolgus monkey or baboon monkey. The anti-CD3 monoclonal antibody UCHT-1 is also reactive with CD3 from chimpanzee but not with CD3 from macaques (own data). On the other hand, there are also examples of monoclonal antibodies, which recognize macaque antigens, but not their human counterparts. One example of this group is monoclonal antibody FN-18 directed to CD3 from macaques (Uda et al., J. Med. Primatol. 30 (2001), 141-147). Interestingly, it has been found that peripheral lymphocytes from about 12% of cynomolgus monkeys lacked reactivity with anti-rhesus monkey CD3 monoclonal antibody (FN-18) due to a polymorphism of the CD3 antigen in macaques. Uda et al. described a substitution of two amino acids in the CD3 sequence of cynomolgus monkeys, which are not reactive with FN-18 antibodies, as compared to CD3 derived from animals, which are reactive with FN-18 antibodies (Uda et al., J Med Primatol. 32 (2003), 105-10; Uda et al., J Med Primatol. 33 (2004), 34-7).

WO 2007/042261 discloses pharmaceutical compositions comprising bispecific single chain antibodies which comprise a first binding domain binding that binds to an epitope of human and non-chimpanzee primate CD3 comprising the amino acid sequence FSEXE, wherein "X" represents leucine or methionine, and (ii) a second binding domain binding to a cell surface antigen, wherein said first binding domain binds to human and non-chimpanzee primate CD3.

WO 2004/106381 discloses a pharmaceutical composition comprising a bispecific single chain antibody construct, said bispecific single chain antibody construct comprising binding domains specific for human CD3 and human CD19, wherein the corresponding variable heavy chain regions (VH) and the corresponding variable light chain regions (VL) regions are arranged, from N-terminus to C- terminus, in certain orders.

WO 2008/119565, WO 2008/119566 and WO 2008/119567 disclose polypeptides comprising a binding domain capable of binding to an epitope of human and non-chimpanzee primate CD3 epsilon chain, wherein the epitope is part of an amino acid sequence comprised in the amino acid sequence 1-27 of CD3 epsilon from human, Callithrix jacchus, Saguinus oedipus and Saimiri sciureus.

WO 2006/008096 discloses a composite polypeptide comprising a desired polypeptide and an expression enhancing domain, said desired polypeptide may be a bispecific single chain antibody.

Im et al. (2006), Biochem Biophys Res Comm (339)(1): 305-312 describes antibodies with affinity to both adenoviral coat proteins and a targeted antigen have been developed with the aim of providing the specific delivery of adenoviral gene therapy vehicle.

NO 20064183 discloses bispecific binding molecule, whereby said molecule comprises or consists of at least two domains, wherein one of said at least two domains specifically binds to/interacts with the human CD3 complex, and wherein a second domain is or contains at least one further antigen-interaction-site and/or at least one further effector domain.

Wuest et al. (2001), J. Biotechnology 92(2): 159-168 discloses bispecific antibodies directed against tumour associated antigens and the T cell receptor component CD3 for recruitment and tumour targeted activation of T cells.

The discriminatory ability, i.e. the species specificity, inherent not only to CD3 monoclonal antibodies (and fragments thereof), but to monoclonal antibodies in general, is a significant impediment to their development as therapeutic agents for the treatment of human diseases. In order to obtain market approval any new candidate medication must pass through rigorous testing. This testing can be subdivided into preclinical and clinical phases: Whereas the latter - further subdivided into the generally known clinical phases I, II and III - is performed in human patients, the former is performed in animals. The aim of pre-clinical testing is to prove that the drug candidate has the desired activity and most importantly is safe. Only when the safety in animals and possible effectiveness of the drug candidate has been established in preclinical testing this drug candidate will be approved for clinical testing in humans by the respective regulatory authority. Drug candidates can be tested for safety in animals in the following three ways, (i) in a relevant species, i.e. a species where the drug candidates can recognize the ortholog antigens, (ii) in a transgenic animal containing the human antigens and (iii) by use of a surrogate for the drug candidate that can bind the ortholog antigens present in the animal. Limitations of transgenic animals are that this technology is typically limited to rodents. Between rodents and man there are significant differences in the physiology and the safety results cannot be easily extrapolated to humans. The limitations of a surrogate for the drug candidate are the different composition of matter compared to the actual drug candidate and often the animals used are rodents with the limitation as discussed above. Therefore, preclinical data generated in rodents are of limited predictive power with respect to the drug candidate. The approach of choice for safety testing is the use of a relevant species, preferably a lower primate. The limitation now of monoclonal antibodies suitable for therapeutic intervention in man described in the art is that the relevant species are higher primates, in particular chimpanzees. Chimpanzees are considered as endangered species and due to their human-like nature, the use of such animals for drug safety testing has been banned in Europe and is highly restricted elsewhere. CD3 has also been successfully used as a target for bispecific single chain antibodies in order to redirect cytotoxic T cells to pathological cells, resulting in the depletion of the diseased cells from the respective organism (WO 99/54440; WO 04/106380). For example, Bargou et al. (Science 321 (2008): 974-7) have recently reported on the clinical activity of a CD19xCD3 bispecific antibody construct called blinatumomab, which has the potential to engage all cytotoxic T cells in human patients for lysis of cancer cells. Doses as low as 0.005 milligrams per square meter per day in non-Hodgkin's lymphoma patients led to an elimination of target cells in blood. Partial and complete tumor regressions were first observed at a dose level of 0.015 milligrams, and all seven patients treated at a dose level of 0.06 milligrams experienced a tumor regression. Blinatumomab also led to clearance of tumor cells from bone marrow and liver. Though this study established clincical proof of concept for the therapeutic potency of the bispecific single chain antibody format in treating blood-cell derived cancer, there is still need for successful concepts for therapies of other cancer types.

In 2008, an estimated 186,320 men will be newly diagnosed with prostate cancer in the United States and about 28,660 men will die from the disease. The most recent report available on cancer mortality shows that, in 2004, the overall death rate from prostate cancer among American men was 25 per 100,000. In the late 1980s, the widespread adoption of the prostate-specific antigen (PSA) test represented a major improvement in the management of prostate cancer. This test measures the amount of PSA protein in the blood, which is often elevated in patients with prostate cancer. In 1986, the U.S. Food and Drug Administration approved the use of the PSA test to monitor patients with prostate cancer and, in 1994, additionally approved its use as a screening test for this disease. Due to the widespread implementation of PSA testing in the United States, approximately 90 percent of all prostate cancers are currently diagnosed at an early stage, and, consequently, men are surviving longer after diagnosis. However, the results of two ongoing clinical trials, the NCI-sponsored Prostate, Lung, Colorectal, and Ovarian (PLCO) screening trial and the European Study of Screening for Prostate Cancer (ERSPC) will be needed to determine whether PSA screening actually saves lives. Ongoing clinical trials over the past 25 years have investigated the effectiveness of natural and synthetic compounds in the prevention of prostate cancer. For example, the Prostate Cancer Prevention Trial (PCPT), which enrolled nearly 19,000 healthy men, found that finasteride, a drug approved for the treatment of benign prostatic hyperplasia (BPH), which is a noncancerous enlargement of the prostate, reduced the risk of developing prostate cancer by 25 percent. Another trial, the Selenium and Vitamin E Cancer Prevention Trial (SELECT), is studying more than 35,000 men to determine whether daily supplements of selenium and vitamin E can reduce the incidence of prostate cancer in healthy men. Other prostate cancer prevention trials are currently evaluating the protective potential of multivitamins, vitamins C and D, soy, green tea, and lycopene, which is a natural compound found in tomatoes. One study, reported in 2005, showed that specific genes were fused in 60 to 80 percent of the prostate tumors analyzed. This study represents the first observation of non-random gene rearrangements in prostate cancer. This genetic alteration may eventually be used as a biomarker to aid in the diagnosis and, possibly, treatment of this disease. Other studies have shown that genetic variations in a specific region of chromosome 8 can increase a man's risk of developing prostate cancer. These genetic variations account for approximately 25 percent of the prostate cancers that occur in white men. They are the first validated genetic variants that increase the risk of developing prostate cancer and may help scientists better understand the genetic causes of this disease. There is also ongoing research that examines how proteins circulating in a patient's blood can be used to improve the diagnosis of prostate and other cancers. In 2005, scientists identified a group of specific proteins that are produced by a patient's immune system in response to prostate tumors. These proteins, a type of autoantibody, were able to detect the presence of prostate cancer cells in blood specimens with greater than 90 percent accuracy. When used in combination with PSA, these and other blood proteins may eventually be used to reduce the number of false-positive results obtained with PSA testing alone and, therefore, reduce the large number of unnecessary prostate biopsies that are performed each year due to false-positive PSA test results.

Apart from PSA, several other markers for prostate cancer have been identified, including e.g. the six-transmembrane epithelial antigen of the prostate (STEAP) (Hubert et al., PNAS 96 (1999), 14523-14528), the prostate-specific membrane antigen (PSM/PSMA) (Israeli et al., Cancer Res. 53 (1993), 227-230) and the Prostate stem cell antigen (PSCA) (Reiter et al., Proc. Nat. Acad. Sci. 95: 1735-1740, 1998). Prostate stem cell antigen (PSCA) is a 123 amino acid protein first identifed when looking for genes upregulated during cancer progression in the LAPC-4 prostate xenograft model (Reiter et al., loc. cit.). It is a glycosyl phosphatidylinositol-anchored cell-surface protein that belongs to the family of Thy-1/Ly-6 surface antigens. PSCA bears 30% homology to stem cell antigen type 2. Although the function of PSCA is yet to be elucidated, homologues of PSCA have diverse activities, and have themselves been implicated in carcinogenesis. Stem cell antigen type 2 has been shown to prevent apoptosis in immature thymocytes (Classon and Coverdale, PNAS 91 (1994), 5296-5300). Thy-1 activates T cells by signalling through src tyrosine kinases (Amoui et al., Eur. J. Immunol. 27 (1997), 1881-86). Ly-6 genes have been implicated in tumorigenesis and cell adhesion (Schrijvers et al., Exp. Cell. Res. 196 (1991), 264-69). Initial messenger RNA studies and subsequent monoclonal antibody (mAb) staining have revealed that PSCA is expressed on the cell surface of normal and malignant prostate cells (Reiter et al., loc. cit.; Gu et al., Oncogene 19 (2000), 1288-96; Ross et al., Cancer Res. 62 (2002), 2546-53). In normal prostate, PSCA mRNA has been detected in a subset of basal and secretory cells. In prostate carcinoma, PSCA mRNA expression has been detected in approximately 50-80% of primary and approximately 70% of metastatic cancers (Reiter et al., loc. cit.). Immunohistochemistry has been reported on 112 primary prostate cancers and nine prostate cancers metastatic to bone (Gu et al., loc. cit.). PSCA expression was detected in 94% and overexpressed in 40% of clinically localized prostate cancers. High levels of PSCA protein expression were also detected in nine out of nine prostate cancer bone metastases examined. Outside the prostate, PSCA is detected in the umbrella cell layer of the transitional epithelium, some renal collecting ducts, neuroendocrine cells of the stomach, and placental trophoblasts. Importantly, recent studies have reported an overexpression of PSCA in a large proportion of pancreatic cancers and invasive and non-invasive transitional cell carcinomas (Amara et al., Cancer Res. 61 (2001), 4660-4665; Argani et al., Cancer Res. 61 (2001), 4320-24). Because of its cell surface expression and its overexpression in a substantial proportion of various cancers, PSCA has been discussed as target for treatment strategies in cancer. However, future clinical correlation will be required to validate this possibility.

The expression of certain CD antigens is highly restricted to specific lineage lymphohematopoietic cells and over the past several years, antibodies directed against lymphoid-specific antigens have been used to develop treatments that were effective either in vitro or in animal models. In this respect CD19 has proved to be a very useful target. CD19 is expressed in the whole B lineage from the pro B cell to the mature B cell, it is not shed, is uniformly expressed on all lymphoma cells, and is absent from stem cells (Haagen, Clin Exp Immunol 90 (1992), 368-75, 14; Uckun, Proc. Natl. Acad. Sci. USA 85 (1988), 8603-7). The CD19 is involved in the development of certain B-cell mediated diseases such as various forms of non-Hodgkin lymphoma, B-cell mediated autoimmune diseases or the depletion of B-cells.

A further example for a molecule which is involved in the progression and spread of numerous human cancer types is the hepatocyte growth factor receptor MET (C-MET). The *MET* oncogene, encoding the receptor tyrosin kinase (RTK) for hepatocyte growth factor (HGF) and Scatter Factor (SF), controls genetic programs leading to cell growth, invasion, and protection from apoptosis. Deregulated activation of MET is critical not only for the acquisition of tumorigenic properties but also for the achievement of the invasive phenotype (Trusolino, L. & Comoglio, P. M. (2002) Nat. Rev. Cancer 2, 289-300). The role of *MET* in human tumors emerged from several experimental approaches and was unequivocally proven by the discovery of *MET*-activating mutations in inherited forms of carcinomas (Schmidt et al., Nat. Genet. 16 (1997), 68-73; Kim et al., J. Med. Genet. 40 (2003), e97). MET constitutive activation is frequent in sporadic cancers, and several studies have shown that the *MET* oncogene is overexpressed in tumors of specific histotypes or is activated through autocrine mechanisms (for a list see http://www.vai.org/met/). Besides, the *MET* gene is amplified in hematogenous metastases of colorectal carcinomas (Di Renzo et al., Clin. Cancer Res. 1 (1995), 147-154).

The Scatter Factor (SF) secreted in culture by fibroblasts, that has the ability to induce intercellular dissociation of epithelial cells, and the Hepatocyte Growth Factor (HGF), a potent mitogen for hepatocytes in culture derived from platelets or from blood of patients with acute liver failure, independently identified as Met ligands turned out to be the same molecule. Met and SF/HGF are widely expressed in a variety of tissues. The expression of Met (the receptor) is normally confined to cells of epithelial origin, while the expression of SF/HGF (the ligand) is restricted to cells of mesenchymal origin.

Met is a transmembrane protein produced as a single-chain precursor. The precursor is proteolytically cleaved at a furin site to produce a highly glycosylated and entirely extracellular $\alpha$-subunit of 50 kd and a $\beta$-subunit of 145 kd with a large extracellular region (involved in binding the ligand), a membrane spanning segment, and an intracellular region (containing the catalytic activity) (Giordano (1989) 339: 155-156). The $\alpha$ and $\beta$ chains are disulphide linked. The extracellular portion of Met contains a region of homology to semaphorins (Sema domain, which includes the full $\alpha$ chain and the N-terminal part of the $\beta$ chain of Met), a cysteine-rich Met Related Sequence (MRS) followed by glycineproline- rich (G-P) repeats, and four Immunoglobuline-like structures (Birchmeier et al., Nature Rev. 4 (2003), 915-25). The intracellular region of Met contains three regions: (1) a juxtamembrane segment that contains: (a) a serine residue (Ser 985) that, when phosphorylated by protein kinase C or by $Ca^{2+}$ calmodulin-dependent kinases downregulates the receptor kinase activity Gandino et al., J. Biol. Chem. 269 (1994), 1815-20); and (b) a tyrosine (Tyr 1003) that binds the ubiquitin ligase Cbl responsible for Met polyubiquitination, endocytosis and degradation (Peschard et al., Mol. Cell 8 (2001), 995-1004); (2) the tyrosine kinase domain that, upon receptor activation, undergoes transphosphorylation on Tyr1234 and Tyr1235; (3) the C-terminal region, which comprises two crucial tyrosines (Tyr1349 and Tyr1356) inserted in a degenerate motif that represents a multisubstrate docking site capable of recruiting several downstream adaptors containing Src homology-2 (SH2) domains Met receptor, as most Receptor Tyrosine Kinases (RTKs) use different tyrosines to bind specific signaling molecules. The two tyrosines of the docking sites have been demonstrated to be necessary and sufficient for the signal transduction both in vitro and in vivo (Maina et al., Cell 87 (1996), 531-542; Ponzetto et al., Cell 77 (1994), 261-71).

Though potent and selective preclinical drug candidates have been developed using C-MET as a tumor target, follow-up clinical trials have to reveal whether these drugs are indeed safe and show therapeutic efficacy in humans. In light of these uncertainties, there is still need for novel therapeutic concepts for cancer.

Cancer has surpassed heart disease as the top killer of Americans under 85, in 2005. Today, cancer ranks behind cardiovascular diseases as the second leading cause of death in Germany. Unless dramatic breakthroughs are achieved in cancer prevention in the next few years comparable to those achieved for cardiovascular disease, cancer will become the leading cause of death in Germany within 15-20 years. Inhibition of tumor angiogenesis is one of the anticancer strategies which has generated much excitement among clinicians and cancer research scientists in the last few years. In the course of these research efforts, several tumor endothelial markers have been identified. Tumor endothelial markers (TEMs) like Endosialin (= TEM1 or CD248) are overexpressed during tumor angiogenesis (St. Croix et al., Science 289 (2000), 1197-1202). Despite the fact that their functions have not been characterized in detail so far, it is well established that they are strongly expressed on vascular endothelial cells in developing embryos and tumors studies (Carson-Walter et al., Cancer Res. 61: 6649-6655, 2001). Accordingly, Endosialin, a 165-kDa type I transmembrane protein, is expressed on the cell surface of tumor blood vessel endothelium in a broad range of human cancers but not detected in blood vessels or other cell types in many normal tissues. It is a C-type lectin-like molecule of 757 amino acids composed of a signal leader peptide, five globular extracellular domains (including a C-type lectin domain, one domain with similarity to the Sushi/ccp/scr pattern, and three EGF repeats), followed by a mucin like region, a transmembrane segment, and a short cytoplasmic tail (Christian et al., J. Biol. Chem. 276: 7408-7414, 2001). The Endosialin core protein carries abundantly sialylated, *O*-linked oligosaccharides and is sensitive to *O*-sialoglycoprotein endopeptidase, placing it in the group of sialomucin-like molecules. The N-terminal 360 amino acids of Endosialin show homology to thrombomodulin, a receptor involved in regulating blood coagulation, and to complement receptor C1qRp. This structural relationship indicates a function for Endosialin as a tumor endothelial receptor. Although Endosialin mRNA is ubiquitously expressed on endothelial cells in normal human and murine somatic tissues, Endosialin protein is largely restricted to the corpus luteum and highly angiogenic tissues such as the granular tissue of healing wounds or tumors (Opavsky et al., J. Biol. Chem. 276 (2001, 38795-38807; Rettig et al., PNAS 89 (1992), 10832-36). Endosialin protein expression is upregulated on tumor endothelial cells of carcinomas (breast, kidney, lung, colorectal, colon, pancreas mesothelioma), sarcomas, and neuroectodermal tumors (melanoma, glioma, neuroblastoma) (Rettig et al., loc. cit.). In addition, Endosialin is expressed at a low level on a subset of tumor stroma fibroblasts (Brady et al., J. Neuropathol. Exp. Neurol. 63 (2004), 1274-83; Opavsky et al., loc. cit.). Because of its restricted normal tissue distribution and abundant expression on tumor endothelial cells of many different types of solid tumors, Endosialin has been discussed as a target for antibody-based antiangiogenic treatment strategies of cancer. However, so far, there are no effective therapeutic approaches using Endosialin as a tumor endothelial target.

A molecule which is very frequently and highly expressed on the majority of human adenocarcinoma and several squamous cell carcinoma cells is EpCAM (CD326) (Went et al., Br. J. Cancer 94 (20006), 128-135). Recent studies have shown that EpCAM is a signalling molecule that can upregulate nuclear expression of the proto-oncogene c-Myc and cyclins (Munz et al., Oncogene 23 (2004), 5748-58). When overexpressed in quiescent cells, EpCAM induces cell proliferation, growth factor independence and growth of colonies in soft agar, which are hallmarks of oncogenic proteins.

When EpCAM expression is knocked down by small interfering RNA (siRNA) in breast cancer cells, such cells cease to proliferate, migrate and be invasive (Osta et al., Cancer Res. 64 (2004), 5818-24). This oncogenic signalling of EpCAM may explain why EpCAM overexpression correlates with poor overall survival in a number of human malignancies, including breast and ovarian cancer (Spizzo et al., Gynecol. Oncol. 103 (2006), 483-8). EpCAM has already been employed as a target antigen in several antibody-based therapeutic approaches, including a human antibody (Oberneder et al., Eur. J. Cancer 42 (2006), 2530-8) and an EpCAMxCD3 single chain bispecific antibody called MT110. The characteristics of MT110 have recently been desribed in detail (Brischwein et al., 43 (2006), 1129-43). This antibody is active against a variety of human carcinoma lines expressing the target antigen and is currently being tested in a phase I study for safety and early signs of efficacy.

More specifically, cancer has surpassed heart disease as the top killer of Americans under 85 in 2005. Today, cancer ranks behind cardiovascular diseases as the second leading cause of death in Germany. Unless dramatic breakthroughs are achieved in cancer prevention in the next few years comparable to those achieved for cardiovascular disease, cancer will become the leading cause of death in Germany within 15-20 years. Among the more than 100 types of different cancers, epithelial cancer is the leading cause of cancer deaths in Germany. In epithelial cancer, invasion and metastasis of malignant epithelial cells into normal tissues is accompanied by adaptive changes in the mesenchyme-derived supporting stroma of the target organs. Altered gene expression in these non-transformed stromal cells has been discussed to provide potential targets for therapy. The cell surface protease fibroblast activation protein alpha (FAP alpha) is one example for such a target of activated tumor fibroblasts. Fibroblast activation protein alpha is an inducible cell surface glycoprotein that has originally been identified in cultured fibroblasts using monoclonal antibody F19. Immunohistochemical studies have shown that FAP alpha is transiently expressed in certain normal fetal mesenchymal tissues but that normal adult tissues as well as malignant epithelial, neural, and hematopoietic cells are generally FAP alpha-negative. However, most of the common types of epithelial cancers contain abundant FAP alpha-reactive stromal fibroblasts. Scanlan et al. (Proc. Nat. Acad. Sci. 91: 5657-5661, 1994) cloned a FAP alpha cDNA from a WI-38 human fibroblast cDNA expression library by immunoselection using antibody F19. The predicted 760-amino acid human FAP alpha protein is a type II integral membrane protein with a large C-terminal extracellular domain, which contains 6 potential N-glycosylation sites, 13 cysteine residues, and 3 segments that correspond to highly conserved catalytic domains of serine proteases; a hydrophobic transmembrane segment; and a short cytoplasmic tail. FAP-alpha shows 48% amino acid identity with dipeptidyl peptidase IV (DPP4) and 30% identity with DPP4-related protein (DPPX). Northern blot analysis detected a 2.8-kb FAP alpha mRNA in fibroblasts. Seprase is a 170-kD integral membrane gelatinase whose expression correlates with the invasiveness of human melanoma and carcinoma cells. Goldstein et al. (Biochim. Biophys. Acta 1361: 11-19, 1997) cloned and characterized the corresponding seprase cDNA. The authors found that seprase and FAP alpha are the same protein and products of the same gene. Pineiro-Sanchez et al. (J. Biol. Chem. 272: 7595-7601, 1997) isolated seprase/FAP alpha protein from the cell membranes and shed vesicles of human melanoma LOX cells. Serine protease inhibitors blocked the gelatinase activity of seprase/FAP alpha, suggesting that seprase/FAP alpha contains a catalytically active serine residue(s). The authors found that seprase/FAP alpha is composed of monomeric, N-glycosylated 97-kD subunits that are proteolytically inactive. They concluded that seprase/FAP alpha is similar to DPP4 in that their proteolytic activities are dependent upon subunit association. Due to its degrading activity of gelatine and heat-denatured type-I and type-IV collagen, a role for seprase/FAP alpha in extracellular matrix remodeling, tumor growth, and metastasis of cancers has been suggested. Moreover, seprase/FAP alpha shows a restricted expression pattern in normal tissues and a uniform expression in the supporting stroma of many malignant tumors. Therefore, seprase/FAP alpha may be used as a target for exploring the concept of tumor stroma targeting for immunotherapy of human epithelial cancer. However, though several clinical trials have been initiated to investigate seprase's/FAP alpha's role as a tumor antigen target, conventional immunotherapy approaches or inhibition of seprase/FAP alpha enzymatic activity so far did not yet result in therapeutic efficacy (see e.g. Welt et al., J. Clin. Oncol. 12:1193-203, 1994; Narra et al., Cancer Biol. Ther. 6, 1691-9, 2007; Henry et al., Clinical Cancer Research 13, 1736-1741, 2007).

Insulin-like growth factor I receptor (IGF-IR or IGF-1 R) is a receptor with tyrosine kinase activity having 70% homology with the insulin receptor IR. IGF-1R is a glycoprotein of molecular weight approximately 350,000. It is a hetero-tetrameric receptor of which each half-linked by disulfide bridges-is composed of an extracellular a-subunit and of a transmembrane [beta]-subunit. IGF-1 R binds IGF 1 and IGF 2 with a very high affinity but is equally capable of binding to insulin with an affinity 100 to 1000 times less. Conversely, the 1 R binds insulin with a very high affinity although the ICFs only bind to the insulin receptor with a 100 times lower affinity. The tyrosine kinase domain of IGF-1 R and of 1 R has a very high sequence homology although the zones of weaker homology respectively concern the cysteine-rich region situated on the alpha-subunit and the C-terminal part of the [beta]-subunit. The sequence differences observed in the a-subunit are situated in the binding zone of the ligands and are therefore at the origin of the relative affinities of IGF-1 R and of 1 R for the IGFs and insulin respectively. The differences in the C-terminal part of the [beta]-subunit result in a divergence in the signalling pathways of the two receptors; IGF-1R mediating mitogenic, differentiation and antiapoptosis effects, while the activation of the IR principally involves effects at the level of the metabolic pathways (Baserga et al., Biochim. Biophys. Acta, 1332: F105-126, 1997; Baserga R., Exp. Cell. Res., 253:1-6, 1999). The cytoplasmic tyrosine kinase proteins are activated by the binding of the ligand to the extracellular domain of the receptor. The activation of the kinases in its turn involves the stimulation of different intra-cellular substrates, including IRS-1, IRS-2, Shc and Grb 10 (Peruzzi F. et al., J. Cancer Res. Clin. Oncol., 125:166-173, 1999). The two major substrates of IGF-IR are IRS and Shc which mediate, by the activation of numerous effectors downstream, the majority of the growth and differentiation effects connected with the attachment of the IGFs to this receptor. The availability of substrates can consequently dictate the final biological effect connected with the activation of the IGF-1R. When IRS-1 predominates, the cells tend to proliferate and to transform. When Shc dominates, the cells tend to differentiate (Valentinis B. et al.; J. Biol. Chem. 274:12423-12430, 1999). It seems that the route principally involved for the effects of protection against apoptosis is the phosphatidyl-inositol 3-kinases (PI 3-kinases) route (Prisco M. et al., Horm. Metab. Res., 31:80-89, 1999; Peruzzi F. et al., J. Cancer Res. Clin. Oncol., 125:166-173, 1999). The role of the IGF system in carcinogenesis has become the subject of intensive research in the last ten years. This interest followed the discovery of the fact that in addition to its mitogenic and antiapoptosis properties, IGF-1R seems to be required for the establishment and the maintenance of a transformed phenotype. In fact, it has been well established that an overexpression or a constitutive activation of IGF-1 R leads, in a great variety of cells, to a growth of the cells independent of the support in media devoid of fetal calf serum, and to the formation of tumors in nude mice. This in itself is not a unique property since a great variety of products of overexpressed genes can transform cells, including a good number of receptors of growth factors. However, the crucial discovery which has clearly demonstrated the major role played by, IGF-1 R in the transformation has been the demonstration that the R-cells, in which the gene coding for IGF-1 R has been inactivated, are totally refractory to transformation by different agents which are usually capable of transforming the cells, such as the E5 protein of bovine papilloma virus, an overexpression of EGFR or of PDGFR, the T antigen of SV 40, activated ras or the combination of these two last factors (Sell C. et al., Proc. Natl. Acad. Sci., USA, 90: 11217-11221, 1993; Sell C. et al., Mol. Cell. Biol., 14:3604-3612, 1994; Morrione A. J., Virol., 69:5300-5303, 1995; Coppola D. et al., Mol. Cell. Biol., 14:458a-4595, 1994; DeAngelis T et al., J. Cell. Physiol., 164:214-221, 1995). IGF-1 R is expressed in a great variety of tumors and of tumor lines and the IGFs amplify the tumor growth via their attachment to IGF-1R. Other arguments in favor of the role of IGF-IR in carcinogenesis come from studies using murine monoclonal antibodies directed against the receptor or using negative dominants of IGF-IR. In effect, murine monoclonal antibodies directed against IGF-1R inhibit the proliferation of numerous cell lines in culture and the growth of tumor cells in vivo (Arteaga C. et al., Cancer Res., 49:6237-6241, 1989 Li et al., Biochem. Biophys. Res. Com., 196:92-98, 1993; Zia F et al., J. Cell. Biol., 24:269-275, 1996; Scotlandi K et al., Cancer Res., 58:4127-4131, 1998). It has likewise been shown in the works of Jiang et al. (Oncogene, 18:6071-6077, 1999) that a negative dominant of IGF-1 R is capable of inhibiting tumor proliferation.

Though there has been put much effort in identifying novel targets for therapeutic approaches for cancer, cancer is yet one of the most frequently diagnosed diseases. In light of this, there is still need for effective treatments for cancer.

The present invention provides for a bispecific single chain antibody molecule comprising a first binding domain which specifically binds to an epitope of human and Callithrix jacchus, Saguinus oedipus or Saimiri sciureus CD3ε (epsilon) chain, wherein said epitope is part of an amino acid sequence comprised in the group consisting of SEQ ID NOs. 2, 4, 6, and 8 and comprises at least the amino acid sequence Gln-Asp-Gly-Asn-Glu; and a second binding domain binding to an antigen selected from the group consisting of Prostate stem cell antigen (PSCA), B-Lymphocyte antigen CD19 (CD19), hepatocyte growth factor receptor (C-MET), Endosialin, the EGF-like domain 1 of EpCAM, encoded by exon 2, Fibroblast Activation Protein Alpha (FAP alpha) and Insulin-like growth factor I receptor (IGF-IR or IGF-1 R).

Though T cell-engaging bispecific single chain antibodies described in the art have great therapeutic potential for the treatment of malignant diseases, most of these bispecific molecules are limited in that they are species specific and recognize only human antigen, and - due to genetic similarity - likely the chimpanzee counterpart. The advantage of the present invention is the provision of a bispecific single chain antibody comprising a binding domain exhibiting cross-species specificity to human and non-chimpanzee primate of the CD3 epsilon chain.

In the present invention, an N-terminal 1-27 amino acid residue polypeptide fragment of the extracellular domain of CD3 epsilon was surprisingly identified which - in contrast to all other known epitopes of CD3 epsilon described in the art - maintains its three-dimensional structural integrity when taken out of its native environment in the CD3 complex (and optionally fused to a heterologous amino acid sequence such as EpCAM or an immunoglobulin Fc part).

The present invention, therefore, provides for a bispecific single chain antibody molecule comprising a first binding domain which specifically binds to an epitope of an N-terminal 1-27 amino acid residue polypeptide fragment of the extracellular domain of CD3 epsilon (which CD3 epsilon is, for example, taken out of its native environment and/or comprised by (presented on the surface of) a T-cell) of human and Callithrix jacchus, Saguinus oedipus or Saimiri sciureus CD3 epsilon chain, wherein said epitope is part of an amino acid sequence comprised in the group consisting of SEQ ID NOs. 2, 4, 6, and 8 and comprises at least the amino acid sequence Gln-Asp-Gly-Asn-Glu; and a second binding domain binding to prostate-specific membrane antigen (PSMA). Preferred non-chimpanzee primates are mentioned herein elsewhere. At least one (or a selection thereof or all) primate(s) selected from Callithrix jacchus; Saguinus oedipus, Saimiri sciureus, and Macaca fascicularis (either SEQ ID 2225 or 2226 or both), is (are) disclosed herein. Macaca mulatta, also known as Rhesus Monkey is also disclosed as another preferred primate. It is thus envisaged that antibodies of the invention bind to (are capable of binding to) the context independent epitope of an N-terminal 1-27 amino acid residue polypeptide fragment of the extracellular domain of CD3 epsilon of human and Callithrix jacchus, Saguinus oedipus, Saimiri sciureus, and Macaca fascicularis (either SEQ ID 2225 or 2226 or both), and optionally also to Macaca mulatta. A bispecific single chain antibody molecule comprising a first binding domain as defined herein can be obtained (is obtainable by) or can be manufactured in accordance with the protocol set out in the appended Examples (in particular Example 2). To this end, it is envisaged to (a) immunize mice with an N-terminal 1-27 amino acid residue polypeptide fragment of the extracellular domain of CD3 epsilon of human and/or Saimiri sciureus; (b) generation of an immune murine antibody scFv library; (c) identification of CD3 epsilon specific binders by testing the capability to bind to at least SEQ ID NOs. 2, 4, 6, and 8.

The context-independence of the CD3 epitope provided in this invention corresponds to the first 27 N-terminal amino acids of CD3 epsilon or functional fragments of this 27 amino acid stretch. The phrase "context-independent," as used herein in relation to the CD3 epitope means that binding of the herein described inventive binding molecules/antibody molecules does not lead to a change or modification of the conformation, sequence, or structure surrounding the antigenic determinant or epitope. In contrast, the CD3 epitope recognized by a conventional CD3 binding molecule (e.g. as disclosed in WO 99/54440 or WO 04/106380) is localized on the CD3 epsilon chain C-terminally to the N-terminal 1-27 amino acids of the context-independent epitope, where it only takes the correct conformation if it is embedded within the rest of the epsilon chain and held in the right sterical position by heterodimerization of the epsilon chain with either the CD3 gamma or delta chain. Anti-CD3 binding molecules/domains as part of a bispecific single chain antibody molecule as provided herein and generated (and directed) against a context-independent CD3 epitope provide for a surprising clinical improvement with regard to T cell redistribution and, thus, a more favourable safety profile. Without being bound by theory, since the CD3 epitope is context-independent, forming an autonomous selfsufficient subdomain without much influence on the rest of the CD3 complex, the CD3 binding molecules/domains provided herein induce less allosteric changes in CD3 conformation than the conventional CD3 binding molecules, which recognize context-dependent CD3 epitopes (e.g. as disclosed in WO 99/54440 or WO 04/106380).

The context-independence of the CD3 epitope which is recognized by the CD3 binding domain of the bispecific single chain antibody of the invention (PSCAxCD3, CD19xCD3, C-METxCD3. EndosialinxCD3, EpCAMxCD3, IGF-1RxCD3 or FAPαxCD3) is associated with less or no T cell redistribution (T cell redistribution equates with an initial episode of drop and subsequent recovery of absolute T cell counts) during the starting phase of treatment with said bispecific single chain antibodies of the invention. This results in a better safety profile of the bispecific single chain antibodies of the invention compared to conventional CD3 binding molecules known in the art, which recognize context-dependent CD3 epitopes. Particularly, because T cell redistribution during the starting phase of treatment with CD3 binding molecules is a major risk factor for adverse events, like CNS adverse events, the bispecific single chain antibodies of the invention has a substantial safety advantage over the CD3 binding molecules known in the art by recognizing a context-independent rather than a context-dependent CD3 epitope. Patients with such CNS adverse events related to T cell redistribution during the starting phase of treatment with conventional CD3 binding molecules usually suffer from confusion and disorientation, in some cases also from urinary incontinence. Confusion is a change in mental status in which the patient is not able to think with his or her usual level of clarity. The patient usually has difficulties to concentrate and thinking is not only blurred and unclear but often significantly slowed down. Patients with CNS adverse events related to T cell redistribution during the starting phase of treatment with conventional CD3 binding molecules may also suffer from loss of memory. Frequently, the confusion leads to the loss of ability to recognize people, places, time or dates. Feelings of disorientation are common in confusion, and the decision-making ability is impaired. CNS adverse events related to T cell redistribution during the starting phase of treatment with conventional CD3 binding molecules may further comprise blurred speech and/or word finding difficulties. This disorder may impair both, the expression and understanding of language as well as reading and writing. Besides urinary incontinence, vertigo and dizziness may also accompany CNS adverse events related to T cell redistribution during the starting phase of treatment with conventional CD3 binding molecules in some patients.

The maintenance of the three-dimensional structure within the mentioned 27 amino acid N-terminal polypeptide fragment of CD3 epsilon can be used for the generation of, preferably human, binding domains which are capable of binding to the N-terminal CD3 epsilon polypeptide fragment *in vitro* and to the native (CD3 epsilon subunit of the) CD3 complex on T cells *in vivo* with the same binding affinity. These data strongly indicate that the N-terminal fragment as described herein forms a tertiary conformation, which is similar to its structure normally existing *in vivo.* A very sensitive test for the importance of the structural integrity of the amino acids 1-27 of the N-terminal polypeptide fragment of CD3 epsilon was performed. Individual amino acids of amino acids 1-27 of the N-terminal polypeptide fragment of CD3 epsilon were changed to alanine (alanine scanning) to test the sensitivity of the amino acids 1-27 of the N-terminal polypeptide fragment of CD3 epsilon for minor disruptions. The CD3 binding domains as part of the bispecific single chain antibodies of the invention were used to test for binding to the alanine-mutants of amino acids 1-27 of the N-terminal polypeptide fragment of CD3 epsilon (see appended Example 5). Individual exchanges of the first five amino acid residues at the very N-terminal end of the fragment and two of the amino acids at positions 23 and 25 of the amino acids 1-27 of the N-terminal polypeptide fragment of CD3 epsilon were critical for binding of the antibody molecules. The substitution of amino acid residues in the region of position 1-5 comprising the residues Q (Glutamine at position 1), D (Aspartic acid at position 2), G (Glycine at position 3), N (Asparagine at position 4), and E (Glutamic acid at position 5) to Alanine abolished binding of the, preferably human, bispecific single chain antibodies of the invention to said fragment. While, for at least some of the, preferably human bispecific single chain antibodies of the invention, two amino acid residues at the C-terminus of the mentioned fragment T (Threonine at position 23) and I (Isoleucine at position 25) reduced the binding energy to the, preferably human, bispecific single chain antibodies of the invention.

Unexpectedly, it has been found that the thus isolated, preferably human, bispecific single chain antibodies of the invention not only recognize the human N-terminal fragment of CD3 epsilon, but also the corresponding homologous fragments of CD3 epsilon of various primates, including New-World Monkeys (Marmoset, Callithrix jacchus; Saguinus oedipus; Saimiri sciureus) and Old-World Monkeys (Macaca fascicularis, also known as Cynomolgus Monkey; or Macaca mulatta, also known as Rhesus Monkey). Thus, multi-primate specificity of the bispecific single chain antibodies of the invention was detected. The following sequence analyses confirmed that human and primates share a highly homologous sequence stretch at the N-terminus of the extracellular domain of CD3 epsilon.

The amino acid sequence of the aformentioned N-terminal fragments of CD3 epsilon are depicted in SEQ ID No. 2 (human), SEQ ID No. 4 (Callithrix jacchus); SEQ ID No. 6 (Saguinus oedipus); SEQ ID No. 8 (Saimiri sciureus); SEQ ID No. 2225 QDGNEEMGSITQTPYQVSISGTTILTC or SEQ ID No. 2226 QDGNEEMGSITQTPYQVSISGTTVILT (Macaca fascicularis, also known as Cynomolgus Monkey), and SEQ ID No. 2227 QDGNEEMGSITQTPYHVSISGTTVILT (Macaca mulatta, also known as Rhesus Monkey).

In one embodiment of the invention the second binding domain of the PSCAxCD3 bispecific single chain antibody of the invention binds to the Prostate stem cell antigen (PSCA). In alternative embodiments the second binding domain binds to CD19, C-MET, Endosialin (CD248), EpCAM, FAPα or IGF-1R. As shown in the following examples, the second binding domain of the EpCAMxCD3 bispecific single chain antibody of the invention binds to amino acid residues 26 to 61 of the EGF-like domain 1 of EpCAM which is encoded by Exon 2 of the EpCAM gene. Said amino acid residues 26 to 61 of the EGF-like domain 1 of human EpCAM are shown in SEQ ID NO. 571. Thus, the EpCAM-directed bispecific single chain molecules of this invention form a unique own class of EpCAM-binding molecules, that is clearly differentiated from EpCAM-binding molecules based on the EpCAM-binder HD69 described earlier. Said EpCAM-binder HD69 bind to a different epitope (i.e. an epitope not localized in the EGF-like domain 1 of human EpCAM). Preferably, the second binding domain of the PSCAxCD3 (respectively CD19xCD3, C-METxCD3, EndosialinxCD3, EpCAMxCD3, IGF-1 RxCD3 or FAPαxCD3) bispecific single chain antibody binds to the human PSCA (respectively CD19, C-MET, Endosialin, EpCAM, IGF-1R or FAPα) or a non-chimpanzee primate PSCA (respectively CD19, C-MET, Endosialin, EpCAM, IGF-1 R or FAPα); more preferred it binds to the human PSCA (respectively CD19, C-MET, Endosialin, EpCAM, IGF-1 R or FAPα) and a non-chimpanzee primate PSCA (respectively CD19, C-MET, Endosialin, EpCAM, IGF-1 R or FAPα) and therefore is cross-species specific; even more preferred to the human PSCA (respectively CD19, C-MET, Endosialin, EpCAM, IGF-1 R or FAPα) and the macaque PSCA (respectively CD19, C-MET, Endosialin, EpCAM, IGF-1R or FAPα) (and therefore is cross-species specific as well). Particularly preferred, the macaque PSCA (respectively CD19, C-MET, Endosialin, EpCAM, IGF-1 R or FAPα) is the Cynomolgus monkey PSCA (respectively CD19, C-MET, Endosialin, EpCAM, IGF-1 R or FAPα) and/or the Rhesus monkey PSCA (respectively CD19, C-MET, Endosialin, EpCAM, IGF-1 R or FAPα). It is to be understood, that the second binding domain of the EpCAMxCD3 bispecific single chain antibody of the invention preferably binds to the EGF-like domain 1 of the non-chimpanzee primate EpCAM which is encoded by Exon 2 of the non-chimpanzee primate EpCAM gene. As indicated above, the non-chimpanzee primate EpCAM is preferably a macaque EpCAM, more preferably the Cynomolgus monkey EpCAM and/or the Rhesus monkey EpCAM.

However, it is not excluded from the scope of the present invention, that the second binding domain may also bind to PSCA (respectively CD19, C-MET, Endosialin, EpCAM, IGF-1 R or FAPα) homologs of other species, such as to the chimpanzee PSCA (respectively CD19, C-MET, Endosialin, EpCAM, IGF-1 R or FAPα) or the PSCA (respectively CD19, C-MET, Endosialin, EpCAM, IGF-1 R or FAPα) homolog in rodents.

It will be understood that in a preferred embodiment, the cross-species specificity of the first and second binding domain of the antibodies of the invention is identical.

Prostate cancer is the second most cancer in men. For 2008, it is estimated that 186,320 men will be newly diagnosed with prostate cancer in the United States and about 28,660 men will die from the disease (see e.g. http://www.cancer.gov/cancertopics/types/prostate). Prostate cancer risk is strongly related to age: very few cases are registered in men under 50 and three-quarters of cases occur in men over 65 years. The largest number of cases is diagnosed in those aged 70-74. Currently, the growth rate of the older population is significantly higher than that of the total population. By 2025-2030, projections indicate that the population over 60 will be growing 3.5 times as rapidly as the total population. The proportion of older persons is projected to more than double worldwide over the next half century, which means that a further increase in incidence of diagnosed prostate cancer has to be expected. However, PSCA is not only a prostate cancer target. Rather, overexpression of PSCA has also been found in bladder cancer (Amara et al., Cancer Res 61 (2001): 4660-4665) and in pancreatic cancer (Argani et al., Cancer Res 61 (2001): 4320-4324). In light of the above, the PSCAxCD3 bispecific single chain antibody of the invention provides an advantageous tool in order to kill PSCA-expressing cancer cells of, including, but not limited to, prostate cancer, bladder cancer or pancreatic cancer in human. As shown in the following Examples, the cytotoxic activity of the PSCAxCD3 bispecific single chain antibody of the invention is higher than the cytotoxic activity of antibodies described in the art.

Advantageously, the present invention provides also PSCAxCD3 bispecific single chain antibodies comprising a second binding domain which binds both to the human PSCA and to the macaque PSCA homolog, i.e. the homolog of a non-chimpanzee primate. In a preferred embodiment, the bispecific single chain antibody thus comprises a second binding domain exhibiting cross-species specificity to the human and a non-chimpanzee primate Prostate stem cell antigen (PSCA). In this case, the identical bispecific single chain antibody molecule can be used both for preclinical evaluation of safety, activity and/or pharmacokinetic profile of these binding domains in primates and as drugs in humans. Put in other words, the same molecule can be used in preclinical animal studies as well as in clinical studies in humans. This leads to highly comparable results and a much-increased predictive power of the animal studies compared to species-specific surrogate molecules. Since both the CD3 and the PSCA binding domain of the PSCAxCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drugs in humans.

CD19 is a cell surface molecule expressed only by B lymphocytes and follicular dendritic cells of the hematopoietic system. It is the earliest of the B-lineage-restricted antigens to be expressed and is present on most pre-B cells and most non-T-cell acute lymphocytic leukemia cells and B-cell type chronic lymphocytic leukemia cells.

In a preferred embodiment, the bispecific single chain antibody of the invention comprises a second binding domain exhibiting cross-species specificity to the human and a non-chimpanzee primate CD19. In this case, the identical bispecific single chain antibody molecule can be used both for preclinical evaluation of safety, activity and/or pharmacokinetic profile of these binding domains in primates and as drug in humans. This leads to highly comparable results and a much-increased predictive power of the animal studies compared to species-specific surrogate molecules. Since in this embodiment both the CD3 and the CD19 binding domain of the CD19xCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drug in humans.

As described herein above, the *MET* oncogene, encoding the receptor tyrosin kinase (RTK) for hepatocyte growth factor (HGF), and Scatter Factor (SF), controls genetic programs leading to cell growth, invasion, and protection from apoptosis. As shown in the following Examples, the C-METxCD3 bispecific single chain antibody of the invention thus provides an advantageous tool in order to kill C-MET-expressing cancer cells, as exemplified by the human C-MET positive breast cancer cell line MDA-MB-231. The cytotoxic activity of the C-METxCD3 bispecific single chain antibody of the invention is higher than the cytotoxic activity of antibodies described in the art. Since preferably both the CD3 and the C-MET binding domain of the C-METxCD3 bispecific single chain antibody of the invention are cross-species specific, i.e. reactive with the human and non-chimpanzee primates' antigens, the C-METxCD3 bispecific single chain antibody of the invention can be used for preclinical evaluation of safety, activity and/or pharmacokinetic profile of these binding domains in primates and - in the identical form - as drugs in humans.

Advantageously, the present invention provides in a further alternative embodiment C-METxCD3 bispecific single chain antibodies comprising a second binding domain which binds both to the human C-MET and to the macaque C-MET homolog, i.e. the homolog of a non-chimpanzee primate. In a preferred embodiment, the bispecific single chain antibody thus comprises a second binding domain exhibiting cross-species specificity to the human and a non-chimpanzee primate C-MET. In this case, the identical bispecific single chain antibody molecule can be used both for preclinical evaluation of safety, activity and/or pharmacokinetic profile of these binding domains in primates and as drugs in humans. Put in other words, the same molecule can be used in preclinical animal studies as well as in clinical studies in humans. This leads to highly comparable results and a much-increased predictive power of the animal studies compared to species-specific surrogate molecules. Since both the CD3 and the C-MET binding domain of the C-METxCD3 bispecific single chain antibody of the invention are cross-species specific, i.e. reactive with the human and non-chimpanzee primates' antigens, it can be used both for preclinical evaluation of safety, activity and/or pharmacokinetic profile of these binding domains in primates and - in the identical form - as drugs in humans.

Angiogenesis, i.e. the formation of new capillaries, is essential to a number of important physiological events, both normal and pathological. Recently, increased attention has focused on the purification and characterization of inhibitors of this process, because of the potential therapeutic value of angiogenesis inhibitors in controlling solid tumors. Because of its restricted normal tissue distribution and abundant expression on tumor endothelial cells of many different types of solid tumors, Endosialin can be used as a target for antibody-based anti-angiogenic treatment strategies of cancer. In particular, targeting of tumor endothelial cells instead of the cancer cells has the advantage that target expression on the untransformed endothelial cells of tumor blood vessels is more stable than target expression on the genetically unstable cancer cells. The EndosialinxCD3 bispecific single chain antibody of the invention provides an advantageous tool in order to inhibit the formation of new capillaries in solid tumors which plays a major role in supporting the growth of the tumors. In this novel and inventive therapeutic approach, it is not the tumor cells which are targeted but tumor blood vessels. The EndosialinxCD3 bispecific single chain antibody of the invention recruits cytotoxic T cells to the Endosialin-positive endothelial cells in tumors, including, but not limited to, carcinomas (breast, kidney, lung, colorectal, colon, pancreas mesothelioma), sarcomas, and neuroectodermal tumors (melanoma, glioma, neuroblastoma), resulting in the depletion of the Endosialin-expressing endothelial cells from the tumor. In this way, tumor angegiogenesis is inhibited resulting in tumor regression or even depletion. As shown in the following Examples, the cytotoxic activity of the EndosialinxCD3 bispecific single chain antibody of the invention is higher than the activity of antibodies described in the art. Since the growth of solid neoplasms requires the recruitment of supporting blood vessels, the therapeutic use of the EndosialinxCD3 bispecific single chain antibody of the invention provides a novel and inventive approach for tumor endothelial targeting and killing.

Advantageously, the present invention provides also EndosialinxCD3 bispecific single chain antibodies comprising a second binding domain which binds both to the human Endosialin and to the macaque Endosialin homolog, i.e. the homolog of a non-chimpanzee primate. In a preferred embodiment, the bispecific single chain antibody thus comprises a second binding domain exhibiting cross-species specificity to the human and a non-chimpanzee primate Endosialin. In this case, the identical bispecific single chain antibody molecule can be used both for preclinical evaluation of safety, activity and/or pharmacokinetic profile of these binding domains in primates and as drug in humans. Put in other words, the same molecule can be used in preclinical animal studies as well as in clinical studies in humans. This leads to highly comparable results and a much-increased predictive power of the animal studies compared to species-specific surrogate molecules. Since both the CD3 and the Endosialin binding domain of the EndosialinxCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drug in humans.

EpCAM has recently been found to be expressed on so called "cancer stem cells" (Dalerba et al., PNAS 104 (2007), 10158-63; Dalerba et al., Ann. Rev. Med. 58 (2007), 267-84). In light of this, the EpCAMxCD3 bispecific single chain antibody of the invention not only provides an advantageous tool in order to kill EpCAM-expressing cancer cells in epithelial cancer or minimal residual cancer, but may also be useful for the elimination of the presumed culprits responsible for tumor relapse after therapy. In addition, the cytotoxic activity of the EpCAMxCD3 bispecific single chain antibody of the invention is higher than the cytotoxic activity of antibodies described in the art.

In a preferred embodiment, the bispecific single chain antibody of the invention comprises a second binding domain exhibiting cross-species specificity to the human and a non-chimpanzee primate EpCAM. In this case, the identical bispecific single chain antibody molecule can be used both for preclinical evaluation of safety, activity and/or pharmacokinetic profile of these binding domains in primates and as drug in humans. This leads to highly comparable results and a much-increased predictive power of the animal studies compared to species-specific surrogate molecules. Since in this embodiment both the CD3 and the EpCAM binding domain of the EpCAMxCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drug in humans.

The FAPalphaxCD3 bispecific single chain antibody of the invention provides an advantageous tool in order to attack the stroma of solid tumors, such as epithelial tumors, which plays a major role in supporting the growth and neovascularisation of tumors. In this novel and inventive therapeutic approach, it is not the tumor cells which are targeted but activated stromal fibroblasts. Previous study have shown that most of the common types of epithelial cancers, including more than 90% of primary and malignant breast, lung and colorectal carcinomas, contain abundant FAP alpha-reactive stromal fibroblasts (Scanlan et al., PNAS 91 (1994), 5657-5661 and references cited therein). In contrast, normal tissues and benign and premalignant epithelial lesions only rarely contain FAP alpha-positive stromal cells. The FAPalphaxCD3 bispecific single chain antibody of the invention recruits cytotoxic T cells to the FAP alpha-positive activated stromal fibroblasts in primary and malignant epithelial tumors resulting in the depletion of the stromal cells from the tumor. In particular, targeting of tumor stromal cells instead of the cancer cells has the advantage that target expression on the untransformed stromal cells is more stable than target expression on the genetically unstable cancer cells. As shown in the following Examples, the cytotoxic activity of the FAPalphaxCD3 bispecific single chain antibody of the invention is higher than the activity of antibodies described in the art. Since the growth of solid neoplasms requires the recruitment of a supporting stroma, the therapeutic use of the FAPalphaxCD3 bispecific single chain antibody of the invention provides a novel and inventive approach for tumor stromal targeting and killing.

Advantageously, the present invention provides also FAPalphaxCD3 bispecific single chain antibodies comprising a second binding domain which binds both to the human FAP alpha and to the macaque FAP alpha homolog, i.e. the homolog of a non-chimpanzee primate. In a preferred embodiment, the bispecific single chain antibody thus comprises a second binding domain exhibiting cross-species specificity to the human and a non-chimpanzee primate FAP alpha. In this case, the identical bispecific single chain antibody molecule can be used both for preclinical evaluation of safety, activity and/or pharmacokinetic profile of these binding domains in primates and as drugs in humans. Put in other words, the same molecule can be used in preclinical animal studies as well as in clinical studies in humans. This leads to highly comparable results and a much-increased predictive power of the animal studies compared to species-specific surrogate molecules. Since both the CD3 and the FAP alpha binding domain of the FAPalphaxCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drugs in humans.

As described herein above, IGF-1 R is a receptor (and, thus a cell surface antigen) with tyrosine kinase activity having 70% homology with the insulin receptor 1 R. IGF-1 R is expressed in a great variety of tumors and of tumor lines and the IGFs amplify the tumor growth via their attachment to IGF-1 R.

In a preferred embodiment, the bispecific single chain antibody of the invention comprises a second binding domain exhibiting cross-species specificity to the human and a non-chimpanzee primate IGF-1 R. In this case, the identical bispecific single chain antibody molecule can be used both for preclinical evaluation of safety, activity and/or pharmacokinetic profile of these binding domains in primates and as drug in humans. This leads to highly comparable results and a much-increased predictive power of the animal studies compared to species-specific surrogate molecules. Since in this embodiment both the CD3 and the IGF-1 R binding domain of the IGF-1 RxCD3 bispecific single chain antibody of the invention are cross-species specific, i.e. reactive with the human and non-chimpanzee primates, it can be used both for preclinical evaluation of safety, activity and/or pharmacokinetic profile of these binding domains in primates and - in the identical form - as drug in humans.

It has been found in the present invention that it is possible to generate a, preferably human, PSCAxCD3 (respectively CD19xCD3, C-METxCD3, EndosialinxCD3, EpCAMxCD3, IGF-1 RxCD3 or FAPαxCD3) bispecific single chain antibody wherein the identical molecule can be used in preclinical animal testing, as well as clinical studies and even in therapy in human. This is due to the unexpected identification of the, preferably human, PSCAxCD3 (respectively CD19xCD3, C-METxCD3, EndosialinxCD3, EpCAMxCD3, IGF-1 RxCD3 or FAPαxCD3) bispecific single chain antibody, which, in addition to binding to human CD3 epsilon and PSCA (respectively CD19, C-MET, Endosialin, EpCAM, IGF-1 R or FAPα), respectively, (and due to genetic similarity likely to the chimpanzee counterpart), also binds to the homologs of said antigens of non-chimpanzee primates, including New-World Monkeys and Old-World Monkeys. As shown in the following Examples, said preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention can be used as therapeutic agent or drug against various diseases, including, but not limited, to cancer.

The PSCAxCD3 bispecific single chain antibody is particularly advantageous for the therapy of prostate cancer, bladder cancer or pancreatic cancer.

Said preferably human CD19xCD3 bispecific single chain antibody of the invention can be used as therapeutic agent against various diseases, including but not limited to cancer, preferably B-cell malignancies, such as non-Hodgkin Lymphoma, B-cell mediated autoimmune diseases or the depletion of B-cells.

Said preferably human c-METxCD3 bispecific single chain antibody of the invention can be used as therapeutic agent against various diseases, including but not limited to cancer, preferably carcinoma, sarcoma, glioblastoma/astrocytoma, melanoma, mesothelioma, Wilms tumor or a hematopoietic malignancy such as leukemia, lymphoma or multiple myeloma.

Said preferably human EndosialinxCD3 bispecific single chain antibody of the invention provides a novel and inventive approach for tumor endothelium targeting and killing for including (but not limited to) carcinomas (breast, kidney, lung, colorectal, colon, pancreas mesothelioma), sarcomas, and neuroectodermal tumors (melanoma, glioma, neuroblastoma). The EndosialinxCD3 bispecific single chain antibody of the invention can deprive solid tumors of their supporting blood vessels, thereby inhibiting angiogenesis and consequently the growth of said neoplasms.

Said preferably human EpCAMxCD3 bispecific single chain antibody of the invention can be used as therapeutic agent against various diseases, including but not limited to cancer, preferably epthelial cancer.

Said preferably human IGF-1 RxCD3 bispecific single chain antibody of the invention can be used as therapeutic agent against various diseases, including but not limited to cancer, preferably bone or soft tissue cancer (e.g. Ewing sarcoma), breast, liver, lung, head and neck, colorectal, prostate, leiomyosarcoma, cervical and endometrial cancer, ovarian, prostate, and pancreatic cancer. IGF-1 RxCD3 bispecific single chain antibody of the invention can be used as therapeutic agent against autoimmune diseases, preferably psoriasis.

In view of the above, the need to construct a surrogate PSCAxCD3 (respectively CD19xCD3, C-METxCD3, EndosialinxCD3, EpCAMxCD3, IGF-1 RxCD3 or FAPαxCD3) bispecific single chain antibody for testing in a phylogenetic distant (from humans) species disappears. As a result, the identical molecule can be used in animal preclinical testing as is intended to be administered to humans in clinical testing as well as following market approval and therapeutic drug administration. The ability to use the same molecule for preclinical animal testing as in later administration to humans virtually eliminates, or at least greatly reduces, the danger that the data obtained in preclinical animal testing have limited applicability to the human case. In short, obtaining preclinical safety data in animals using the same molecule as will actually be administered to humans does much to ensure the applicability of the data to a human-relevant scenario. In contrast, in conventional approaches using surrogate molecules, said surrogate molecules have to be molecularly adapted to the animal test system used for preclinical safety assessment. Thus, the molecule to be used in human therapy in fact differs in sequence and also likely in structure from the surrogate molecule used in preclinical testing in pharmacokinetic parameters and/or biological activity, with the consequence that data obtained in preclinical animal testing have limited applicability / transferability to the human case. The use of surrogate molecules requires the construction, production, purification and characterization of a completely new construct. This leads to additional development costs and time necessary to obtain that molecule. In sum, surrogates have to be developed separately in addition to the actual drug to be used in human therapy, so that two lines of development for two molecules have to be carried out. Therefore, a major advantage of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention exhibiting cross-species specificity described herein is that the identical molecule can be used for therapeutics in humans and in preclinical animal testing.

It is preferred that at least one of said first or second binding domains of the bispecific single chain antibody of the invention is CDR-grafted, humanized or human, as set forth in more detail below. Preferably, both the first and second binding domains of the bispecific single chain antibody of the invention are CDR-grafted, humanized or human. For the preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention, the generation of an immune reaction against said binding molecules is excluded to the maximum possible extent upon administration of the molecule to human patients.

Another major advantage of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention is its applicability for preclinical testing in various primates. The behavior of a drug candidate in animals should ideally be indicative of the expected behavior of this drug candidate upon administration to humans. As a result, the data obtained from such preclinical testing should therefore generally have a highly predictive power for the human case. However, as learned from the tragic outcome of the recent Phase I clinical trial on TGN1412 (a CD28 monoclonal antibody), a drug candidate may act differently in a primate species than in humans: Whereas in preclinical testing of said antibody no or only limited adverse effects have been observed in animal studies performed with cynomolgus monkeys, six human patients developed multiple organ failure upon administration of said antibody (Lancet 368 (2006), 2206-7). The results of these dramatic, non-desired negative events suggest that it may not be sufficient to limit preclinical testing to only one (non-chimpanzee primate) species. The fact that the PSCAxCD3 (respectively CD19xCD3, C-METxCD3, EndosialinxCD3, EpCAMxCD3, IGF-1 RxCD3 or FAPαxCD3) bispecific single chain antibody of the invention binds to a series of New-World and Old-World Monkeys may help to overcome the problems faced in the case mentioned above. Accordingly, the present invention provides means and methods for minimizing species differences in effects when drugs for human therapy are being developed and tested.

With the, preferably human, cross-species specific PSCAxCD3 (respectively CD19xCD3, C-METxCD3, EndosialinxCD3, EpCAMxCD3, IGF-1RxCD3 or FAPαxCD3) bispecific single chain antibody of the invention it is also no longer necessary to adapt the test animal to the drug candidate intended for administration to humans, such as e.g. the creation of transgenic animals. The, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention exhibiting cross-species specificity according to the uses and the methods of invention can be directly used for preclinical testing in non-chimpanzee primates, without any genetic manipulation of the animals. As well known to those skilled in the art, approaches in which the test animal is adapted to the drug candidate always bear the risk that the results obtained in the preclinical safety testing are less representative and predictive for humans due to the modification of the animal. For example, in transgenic animals, the proteins encoded by the transgenes are often highly over-expressed. Thus, data obtained for the biological activity of an antibody against this protein antigen may be limited in their predictive value for humans in which the protein is expressed at much lower, more physiological levels.

A further advantage of the uses of the preferably human PSCAxCD3 (respectively CD19xCD3, C-METxCD3, EndosialinxCD3, EpCAMxCD3, IGF-1 RxCD3 or FAPαxCD3) bispecific single chain antibody of the invention exhibiting cross-species specificity is the fact that chimpanzees as an endangered species are avoided for animal testing. Chimpanzees are the closest relatives to humans and were recently grouped into the family of hominids based on the genome sequencing data (Wildman et al., PNAS 100 (2003), 7181). Therefore, data obtained with chimpanzee is generally considered to be highly predictive for humans. However, due to their status as endangered species, the number of chimpanzees, which can be used for medical experiments, is highly restricted. As stated above, maintenance of chimpanzees for animal testing is therefore both costly and ethically problematic. The uses of the, preferably h u m a n, PSCAxCD3 (respectively CD19xCD3, C-METxCD3, EndosialinxCD3, EpCAMxCD3, IGF-1 RxCD3 or FAPαxCD3) bispecific single chain antibody of the invention avoid both ethical objections and financial burden during preclinical testing without prejudicing the quality, i.e. applicability, of the animal testing data obtained. In light of this, the uses of the,

preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention provide for a reasonable alternative for studies in chimpanzees.

A still further advantage of the, preferably human, PSCAxCD3 (respectively CD19xCD3, C-METxCD3, EndosialinxCD3, EpCAMxCD3, IGF-1RxCD3 or FAPαxCD3) bispecific single chain antibody of the invention is the ability of extracting multiple blood samples when using it as part of animal preclinical testing, for example in the course of pharmacokinetic animal studies. Multiple blood extractions can be much more readily obtained with a non-chimpanzee primate than with lower animals, e.g. a mouse. The extraction of multiple blood samples allows continuous testing of blood parameters for the determination of the biological effects induced by the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention. Furthermore, the extraction of multiple blood samples enables the researcher to evaluate the pharmacokinetic profile of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein. In addition, potential side effects, which may be induced by said, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention reflected in blood parameters can be measured in different blood samples extracted during the course of the administration of said antibody. This allows the determination of the potential toxicity profile of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein.

The advantages of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein exhibiting cross-species specificity may be briefly summarized as follows:

First, the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein used in preclinical testing is the same as the one used in human therapy. Thus, it is no longer necessary to develop two independent molecules, which may differ in their pharmacokinetic properties and biological activity. This is highly advantageous in that e.g. the pharmacokinetic results are more directly transferable and applicable to the human setting than e.g. in conventional surrogate approaches.

Second, the uses of the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein for the preparation of therapeutics in human is less cost- and labor-intensive than surrogate approaches.

Third, the, preferably human, PSCAxCD3 (respectively CD19xCD3, C-METxCD3, EndosialinxCD3, EpCAMxCD3, IGF-1 RxCD3 or FAPαxCD3) bispecific single chain antibody of the invention as defined herein can be used for preclinical testing not only in one primate species, but in a series of different primate species, thereby limiting the risk of potential species differences between primates and human.

Fourth, chimpanzee as an endangered species for animal testing can be avoided if desired.

Fifth, multiple blood samples can be extracted for extensive pharmacokinetic studies.

Sixth, due to the human origin of the, preferably human, binding molecules according to a preferred embodiment of the invention the generation of an immune reaction against said binding molecules is minimalized when administered to human patients. Induction of an immune response with antibodies specific for a drug candidate derived from a non-human species as e.g. a mouse leading to the development of human-anti-mouse antibodies (HAMAs) against therapeutic molecules of murine origin is excluded.

Last but not least:

- the therapeutic use of the PSCAxCD3 bispecific single chain antibody of the invention provides a novel and inventive therapeutic approach for cancer, including, but not limited to, prostate cancer, bladder cancer or pancreatic cancer. The following examples clearly demonstrate for each construct the potent recruitment of cytotoxic activity of human and macaque effector cells against cells positive for PSCA.
- the therapeutic use of the CD19xCD3 bispecific single chain antibody of the invention provides a novel and inventive therapeutic approach for cancer, preferably B-cell malignancies, such as non-Hodgkin lymphoma, B-cell mediated autoimmune diseases or the depletion of B-cells. As shown in the following Examples, the cytotoxic activity of the CD19xCD3 bispecific single chain antibody of the invention is higher than the activity of antibodies described in the art.
- the therapeutic use of the C-METxCD3 bispecific single chain antibody of the invention provides a novel and inventive therapeutic approach for cancer, preferably carcinoma, sarcoma, glioblastoma/astrocytoma, melanoma, mesothelioma, Wilms tumor or a hematopoietic malignancy such as leukemia, lymphoma or multiple myeloma. As shown in the following Examples, the cytotoxic activity of the C-METxCD3 bispecific single chain antibody of the invention is higher than the activity of antibodies described in the art.
- the therapeutic use of the EndosialinxCD3 bispecific single chain antibody of the invention provides a novel and inventive approach for tumor endothelium targeting and killing for including (but not limited to) carcinomas (breast, kidney, lung, colorectal, colon, pancreas mesothelioma), sarcomas, and neuroectodermal tumors (melanoma, glioma, neuroblastoma). The EndosialinxCD3 bispecific single chain antibody of the invention can deprive solid tumors of their supporting blood vessels, thereby inhibiting angiogenesis and consequently the growth of said neoplasms.
- the therapeutic use of the EpCAMxCD3 bispecific single chain antibody of the invention provides a novel and inventive therapeutic approach for cancer, preferably epithelial cancer and/or a minimal residual cancer. The EpCAMxCD3 bispecific single chain antibody of the invention not only provides an advantageous tool in order to kill EpCAM-expressing cancer cells in cancer, preferably epithelial cancer or a minimal residual cancer, but may also be useful for the elimination of the presumed culprits responsible for tumor relapse after therapy. In addition, the cytotoxic activity of the EpCAMxCD3 bispecific single chain antibody of the invention is higher than the cytotoxic activity of antibodies described in the art.
- the therapeutic use of the FAPalphaxCD3 bispecific single chain antibody of the invention provides a novel and inventive approach for tumor stromal targeting and killing: The FAPalphaxCD3 bispecific single chain antibody of the invention deprives solid tumors, such as epithelial tumors, of their supporting stroma, thereby inhibiting the growth and neovascularisation of solid neoplasms.
- the therapeutic use of the IGF-1 RxCD3 bispecific single chain antibody of the invention provides a novel and inventive approach for cancer (preferably bone or soft tissue cancer (e.g. Ewing sarcoma), breast, liver, lung, head and neck, colorectal, prostate, leiomyosarcoma, cervical and endometrial cancer, ovarian, prostate, and pancreatic cancer) or autoimmune diseases (preferably psoriasis).

As noted herein above, the present invention provides polypeptides, i.e. bispecific single chain antibodies, comprising a first binding domain as defined in the claims and a second binding domain binding to PSCA (respectively CD19, C-MET, Endosialin, EpCAM, IGF-1R or FAP$\alpha$), wherein the second binding domain preferably also binds to PSCA (respectively CD19, C-MET, Endosialin, EpCAM, IGF-1 R or FAP$\alpha$) of a human and a non-chimpanzee primate. The advantage of bispecific single chain antibody molecules as drug candidates fulfilling the requirements of the preferred bispecific single chain antibody of the invention is the use of such molecules in preclinical animal testing as well as in clinical studies and even for therapy in human. In a preferred embodiment of the cross-species specific bispecific single chain antibodies of the invention the second binding domain binding to a cell surface antigen is human. In a cross-species specific bispecific molecule according to the invention the binding domain binding to an epitope of human and non-chimpanzee primate CD3 epsilon chain is located in the order VH-VL or VL-VH at the N-terminus or the C-terminus of the bispecific molecule. Examples for cross-species specific bispecific molecules according to the invention in different arrangements of the VH- and the VL-chain in the first and the second binding domain are described in the appended examples.

As used herein, a "bispecific single chain antibody" denotes a single polypeptide chain comprising two binding domains. Each binding domain comprises one variable region from an antibody heavy chain ("VH region"), wherein the VH region of the first binding domain specifically binds to the CD3$\varepsilon$ molecule, and the VH region of the second binding domain specifically binds to PSCA, CD19, C-MET, Endosialin, EpCAM, IGF-1R or FAP$\alpha$. The two binding domains are optionally linked to one another by a short polypeptide spacer. A non-limiting example for a polypeptide spacer is Gly-Gly-Gly-Gly-Ser (G-G-G-G-S) and repeats thereof. Each binding domain may additionally comprise one variable region from an antibody light chain ("VL region"), the VH region and VL region within each of the first and second binding domains being linked to one another via a polypeptide linker, for example of the type disclosed and claimed in EP 623679 B1, but in any case long enough to allow the VH region and VL region of the first binding domain and the VH region and VL region of the second binding domain to pair with one another such that, together, they are able to specifically bind to the respective first and second binding domains.

The term "protein" is well known in the art and describes biological compounds. Proteins comprise one or more amino acid chains (polypeptides), whereby the amino acids are bound among one another via a peptide bond. The term "polypeptide" as used herein describes a group of molecules, which consists of more than 30 amino acids. In accordance with the invention, the group of polypeptides comprises "proteins" as long as the proteins consist of a single polypeptide chain. Also in line with the definition the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

The term "binding domain" characterizes in connection with the present invention a domain of a polypeptide which specifically binds to/interacts with a given target structure/antigen/epitope. Thus, the binding domain is an "antigen-interaction-site". The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". The term "specifically recognizing" means in accordance with this invention that the antibody molecule is capable of specifically interacting with and/or binding to at least two, preferably at least three, more preferably at least four amino acids of an antigen, e.g. the human CD3 antigen as defined herein. Such binding may be exemplified by the specificity of a "lock-and-key-principle". Thus, specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the binding domain/antigen-interaction-site with its specific antigen may alternatively result in the initiation of a signal, e.g. due to the induction of a change of the conformation of the antigen, an oligomerization of the antigen, etc. A binding domain in line with the present invention is an antibody. The binding domain may be a monoclonal or polyclonal antibody or derived from a monoclonal or polyclonal antibody. The term "antibody" comprises derivatives or functional fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also comprises immunoglobulins (Ig's) of different classes (i.e. IgA, IgG, IgM, IgD and IgE) and subclasses (such as IgG1, IgG2 etc.).
The definition of the term "antibody" also includes embodiments such as chimeric, single chain and humanized antibodies, as well as antibody fragments, like, inter alia, Fab fragments. Antibody fragments or derivatives further comprise $F(ab')_2$, Fv, scFv fragments or single domain antibodies, single variable domain antibodies or immunoglobulin single variable domain comprising merely one variable domain, which might be VH or VL, that specifically bind to an antigen or epitope independently of other V regions or domains; see, for example, Harlow and Lane (1988) and (1999), loc. cit. Such immunoglobulin single variable domain encompasses not only an isolated antibody single variable domain polypeptide, but also larger polypeptides that comprise one or more monomers of an antibody single variable domain polypeptide sequence.
Various procedures are known in the art and may be used for the production of such antibodies and/or fragments. Thus, the (antibody) derivatives can also be produced by peptidomimetics. Further, techniques described for the production of single chain antibodies (see, inter alia, US Patent 4,946,778) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used.

Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon, PSCA, CD19, C-MET, Endosialin, EpCAM, IGF-1 R or FAP$\alpha$ (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

The term "specific interaction" as used in accordance with the present invention means that the binding domain does not or does not significantly cross-react with polypeptides which have similar structure as those bound by the binding domain, and which might be expressed by the same cells as the polypeptide of interest. Cross-reactivity of a panel of binding domains under investigation may be tested, for example, by assessing binding of said panel of binding domains under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999). Examples for the specific interaction of a binding domain with a specific antigen comprise the specificity of a ligand for its receptor. Said definition particularly comprises the interaction of ligands, which induce a signal upon binding to its specific receptor. Examples for said interaction, which is also particularly comprised by said definition, is the interaction of an antigenic determinant (epitope) with the binding domain (antigenic binding site) of an antibody.

The term "cross-species specificity" or "interspecies specificity" as used herein means binding of a binding domain described herein to the same target molecule in humans and non-chimpanzee primates. Thus, "cross-species specificity" or "interspecies specificity" is to be understood as an interspecies reactivity to the same molecule "X" (i.e. the homolog) expressed in different species, but not to a molecule other than "X". Cross-species specificity of a monoclonal antibody recognizing e.g. human CD3 epsilon, to a non-chimpanzee primate CD3 epsilon, e.g. macaque CD3 epsilon, can be determined, for instance, by FACS analysis. The FACS analysis is carried out in a way that the respective monoclonal antibody is tested for binding to human and non-chimpanzee primate cells, e.g. macaque cells, expressing said human and non-chimpanzee primate CD3 epsilon antigens, respectively. An appropriate assay is shown in the following examples. The above-mentioned subject matter applies mutatis mutandis for the PSCA, CD19, C-MET, Endosialin, EpCAM, IGF-1R and FAPα antigen: Cross-species specificity of a monoclonal antibody recognizing e.g. human PSCA, CD19, C-MET, Endosialin, EpCAM, IGF-1 R or FAPα, to a non-chimpanzee primate PSCA, CD19, C-MET, Endosialin, EpCAM, IGF-1 R or FAPα, e.g. macaque PSCA, CD19, C-MET, Endosialin, EpCAM, IGF-1R or FAPα, can be determined, for instance, by FACS analysis. The FACS analysis is carried out in a way that the respective monoclonal antibody is tested for binding to human and non-chimpanzee primate cells, e.g. macaque cells, expressing said human and non-chimpanzee primate PSCA, CD19, C-MET, Endosialin, EpCAM, IGF-1 R or FAPα antigens, respectively.

As used herein, CD3 epsilon denotes a molecule expressed as part of the T cell receptor and has the meaning as typically ascribed to it in the prior art. In human, it encompasses in individual or independently combined form all known CD3 subunits, for example CD3 epsilon, CD3 delta, CD3 gamma, CD3 zeta, CD3 alpha and CD3 beta. The non-chimpanzee primate, non-human CD3 antigens as referred to herein are, for example, *Macaca fascicularis* CD3 and *Macaca mulatta* CD3. In *Macaca fascicularis,* it encompasses CD3 epsilon FN-18 negative and CD3 epsilon FN-18 positive, CD3 gamma and CD3 delta. In *Macaca mulatta,* it encompasses CD3 epsilon, CD3 gamma and CD3 delta. Preferably, said CD3 as used herein is CD3 epsilon.

The human CD3 epsilon is indicated in GenBank Accession No.NM_000733 and comprises SEQ ID NO. 1. The human CD3 gamma is indicated in GenBank Accession NO. NM_000073. The human CD3 delta is indicated in GenBank Accession No. NM_000732.

The CD3 epsilon "FN-18 negative" of *Macaca fascicularis* (i.e. CD3 epsilon not recognized by monoclonal antibody FN-18 due to a polymorphism as set forth above) is indicated in GenBank Accession No. AB073994.

The CD3 epsilon "FN-18 positive" *of Macaca fascicularis* (i.e. CD3 epsilon recognized by monoclonal antibody FN-18) is indicated in GenBank Accession No. AB073993. The CD3 gamma of *Macaca fascicularis* is indicated in GenBank Accession No. AB073992. The CD3 delta of *Macaca fascicularis* is indicated in GenBank Accession No. AB073991.

The nucleic acid sequences and amino acid sequences of the respective CD3 epsilon, gamma and delta homologs *of Macaca mulatta* can be identified and isolated by recombinant techniques described in the art (Sambrook et al. Molecular Cloning: A Laboratory Manual; Cold Spring Harbor Laboratory Press, 3rd edition 2001). This applies mutatis mutandis to the CD3 epsilon, gamma and delta homologs of other non-chimpanzee primates as defined herein. The identification of the amino acid sequence of Callithrix jacchus, Saimiri sciureus und Saguinus oedipus is described in the appended examples. The amino acid sequence of the extracellular domain of the CD3 epsilon of *Callithrix jacchus* is depicted in SEQ ID NO: 3, the one of *Saguinus oedipus* is depicted in SEQ ID NO: 5 and the one of *Saimiri sciureus* is depicted in SEQ ID NO: 7.

The human PSCA is indicated in GenBank Accession No.NM_005672. The corresponding cDNA and amino acid sequences are shown in SEQ ID NOs. 444 and 443, respectively. The cloning of the PSCA homolog of macaque is demonstrated in the following examples, the corresponding cDNA and amino acid sequences are shown in SEQ ID NOs. 446 and 445, respectively.

The human CD19 is indicated in GenBank Accession No. NM_001770. On the basis of this sequence information it is possible for the person skilled in the art without any inventive ado to clone (and express) the macaque CD19 molecule. For example, the human CD19 cDNA or a fragment thereof indicated in GenBank Accession No. NM_001770 can be used as a hybridization probe in order to screen a macaque cDNA library (e.g. a cDNA library of Cynomolgus monkey or Rhesus monkey) under appropriate hybridization conditions. Recombinant techniques and screening methods (including hybridization approaches) in molecular biology are described e.g. in Sambrook et al. Molecular Cloning: A Laboratory Manual; Cold Spring Harbor Laboratory Press, 3rd edition 2001.

The human C-MET is indicated in GenBank Accession No.NM_000245. The corresponding cDNA and amino acid sequences are shown in SEQ ID NOs. 776 and 777, respectively. The cloning of the C-MET homolog of macaque is demonstrated in the following examples, the corresponding cDNA and amino acid sequences are shown in SEQ ID NOs. 788 and 789, respectively.

The human Endosialin is indicated in GenBank Accession No. NM_020404. The corresponding cDNA and amino acid sequences are shown in SEQ ID NOs. 913 and 914, respectively. The cloning of the Endosialin homolog of macaque is demonstrated in the following examples, the corresponding cDNA and amino acid sequences are shown in SEQ ID NOs. 915 and 916, respectively.

The human EpCAM is indicated in GenBank Accession No. NM_002354. The corresponding cDNA and amino acid sequences are shown in SEQ ID NOs. 1029 and 1028, respectively. As demonstrated in the following examples, the second binding domain of the EpCAMxCD3 bispecific single chain antibody of the invention binds to an epitope localized in amino acid residues 26 to 61 of the EGF-like domain 1 of EpCAM which is encoded by Exon 2 of the EpCAM gene. Said amino acids residues 26 to 61 of the EGF-like domain 1 of human EpCAM are shown in SEQ ID NO. 1130. On the basis of this sequence information it is possible for the person skilled in the art without any inventive ado to clone (and express) the macaque EpCAM molecule. For example, the human EpCAM cDNA or a fragment thereof indicated in GenBank Accession No. NM_002354 can be used as a hybridization probe in order to screen a macaque cDNA library (e.g. a cDNA library of Cynomolgus monkey or Rhesus monkey) under appropriate hybridization conditions. Recombinant techniques and screening methods (including hybridization approaches) in molecular biology are described e.g. in Sambrook et al. Molecular Cloning: A Laboratory Manual; Cold Spring Harbor Laboratory Press, 3rd edition 2001.
The human FAP alpha is indicated in GenBank Accession No. NM_004460. The corresponding cDNA and amino acid sequences are shown in SEQ ID NOs. 1149 and 1150, respectively. The cloning of the FAP alpha homolog of macaque is demonstrated in the following examples, the corresponding cDNA and amino acid sequences are shown in SEQ ID NOs. 1151 and 1152, respectively.
The human IGF-1R is indicated in GenBank Accession No. NM_000875. The corresponding cDNA and amino acid sequences are shown in SEQ ID NOs. 1988 and 1989, respectively. The coding sequence of macaque IGF-1 R as published in GenBank (Accession number M_001100407). The corresponding cDNA and amino acid sequences are shown in SEQ ID NOs. 1998 and 1999, respectively.

In line with the above, the term "epitope" defines an antigenic determinant, which is specifically bound/identified by a binding domain as defined herein. The binding domain may specifically bind to/interact with conformational or continuous epitopes, which are unique for the target structure, e.g. the human and non-chimpanzee primate CD3 epsilon chain or the human and non-chimpanzee primate PSCA, CD19, C-MET, Endosialin, EpCAM, IGF-1 R or FAP$\alpha$. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. Within the present invention, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast, a context-independent CD3 epitope as provided herein refers to an N-terminal 1-27 amino acid residue polypeptide or a functional fragment thereof of CD3 epsilon. This N-terminal 1-27 amino acid residue polypeptide or a functional fragment thereof maintains its three-dimensional structural integrity and correct conformation when taken out of its native environment in the CD3 complex. The context-independency of the N-terminal 1-27 amino acid residue polypeptide or a functional fragment thereof, which is part of the extracellular domain of CD3 epsilon, represents, thus, an epitope which is completely different to the epitopes of CD3 epsilon described in connection with a method for the preparation of human binding molecules in WO 2004/106380. Said method used solely expressed recombinant CD3 epsilon. The conformation of this solely expressed recombinant CD3 epsilon differed from that adopted in its natural form, that is, the form in which the CD3 epsilon subunit of the TCR/CD3 complex exists as part of a noncovalent complex with either the CD3 delta or the CD3-gamma subunit of the TCR/CD3 complex. When such solely expressed recombinant CD3 epsilon protein is used as an antigen for selection of antibodies from an antibody library, antibodies specific for this antigen are identified from the library although such a library does not contain antibodies with specificity for self-antigens/autoantigens. This is due to the fact that solely expressed recombinant CD3 epsilon protein does not exist in vivo; it is not an autoantigen. Consequently, subpopulations of B cells expressing antibodies specific for this protein have not been depleted in vivo; an antibody library constructed from such B cells would contain genetic material for antibodies specific for solely expressed recombinant CD3 epsilon protein.
However, since the context-independent N-terminal 1-27 amino acid residue polypeptide or a functional fragment thereof is an epitope, which folds in its native form, binding domains in line with the present invention cannot be identified by methods based on the approach described in WO 2004/106380. Therefore, it could be verified in tests that binding molecules as disclosed in WO 2004/106380 are not capable of binding to the N-terminal 1-27 amino acid residues of the CD3 epsilon chain. Hence, conventional anti-CD3 binding molecules or anti-CD3 antibody molecules (e.g. as disclosed in WO 99/54440) bind CD3 epsilon chain at a position which is more C-terminally located than the context-independent N-terminal 1-27 amino acid residue polypeptide or a functional fragment provided herein. Prior art antibody molecules OKT3 and UCHT-1 have also a specificity for the epsilon-subunit of the TCR/CD3 complex between amino acid residues 35 to 85 and, accordingly, the epitope of these antibodies is also more C-terminally located. In addition, UCHT-1 binds to the CD3 epsilon chain in a region between amino acid residues 43 to 77 (Tunnacliffe, Int. Immunol. 1 (1989), 546-50; Kjer-Nielsen, PNAS 101, (2004), 7675-7680; Salmeron, J. Immunol. 147 (1991), 3047-52). Therefore, prior art anti-CD3 molecules do not bind to and are not directed against the herein defined context-independent N-terminal 1-27 amino acid residue epitope (or a functional fragment thereof). In particular, the state of the art fails to provide anti-CD3 molecules which specifically binds to the context-independent N-terminal 1-27 amino acid residue epitope and which are cross-species specific, i.e. bind to human and non-chimpanzee primate CD3 epsilon.

For the generation of a, preferably human, binding domain comprised in a bispecific single chain antibody molecule of the invention, e.g. monoclonal antibodies binding to both the human and non-chimpanzee primate CD3 epsilon (e.g. macaque CD3 epsilon) or monoclonal antibodies binding to the human and/or non-chimpanzee primate PSCA, respectively CD19, C-MET, Endosialin, EpCAM, IGF-1 R or FAP$\alpha$, can be used.

As used herein, "human" and "man" refers to the species *Homo sapiens*. As far as the medical uses of the constructs described herein are concerned, human patients are to be treated with the same molecule.

It is preferred that at least one of said first or second binding domains of the bispecific single chain antibody of the invention is CDR-grafted, humanized or human. Preferably, both the first and second binding domains of the bispecific single chain antibody of the invention are CDR-grafted, humanized or human.

The term "human" antibody as used herein is to be understood as meaning that the bispecific single chain antibody as defined herein, comprises (an) amino acid sequence(s) contained in the human germline antibody repertoire. For the purposes of definition herein, said bispecific single chain antibody may therefore be considered human if it consists of such (a) human germline amino acid sequence(s), i.e. if the amino acid sequence(s) of the bispecific single chain antibody in question is (are) identical to (an) expressed human germline amino acid sequence(s). A bispecific single chain antibody as defined herein may also be regarded as human if it consists of (a) sequence(s) that deviate(s) from its (their) closest human germline sequence(s) by no more than would be expected due to the imprint of somatic hypermutation. Additionally, the antibodies of many non-human mammals, for example rodents such as mice and rats, comprise VH CDR3 amino acid sequences which one may expect to exist in the expressed human antibody repertoire as well. Any such sequence(s) of human or non-human origin which may be expected to exist in the expressed human repertoire would also be considered "human" for the purposes of the present invention.

As used herein, the term "humanized", "humanization", "human-like" or grammatically related variants thereof are used interchangeably to refer to a bispecific single chain antibody comprising in at least one of its binding domains at least one complementarity determining region ("CDR") from a non-human antibody or fragment thereof. Humanization approaches are described for example in WO 91/09968 and US 6,407,213. As non-limiting examples, the term encompasses the case in which a variable region of at least one binding domain comprises a single CDR region, for example the third CDR region of the VH (CDRH3), from another non-human animal, for example a rodent, as well as the case in which a or both variable region/s comprise at each of their respective first, second and third CDRs the CDRs from said non-human animal. In the event that all CDRs of a binding domain of the bispecific single chain antibody have been replaced by their corresponding equivalents from, for example, a rodent, one typically speaks of "CDR-grafting", and this term is to be understood as being encompassed by the term "humanized" or grammatically related variants thereof as used herein. The term "humanized" or grammatically related variants thereof also encompasses cases in which, in addition to replacement of one or more CDR regions within a VH and/or VL of the first and/or second binding domain further mutation/s (e.g. substitutions) of at least one single amino acid residue/s within the framework ("FR") regions between the CDRs has/have been effected such that the amino acids at that/those positions correspond/s to the amino acid/s at that/those position/s in the animal from which the CDR regions used for replacement is/are derived. As is known in the art, such individual mutations are often made in the framework regions following CDR-grafting in order to restore the original binding affinity of the non-human antibody used as a CDR-donor for its target molecule. The term "humanized" may further encompass (an) amino acid substitution(s) in the CDR regions from a non-human animal to the amino acid(s) of a corresponding CDR region from a human antibody, in addition to the amino acid substitutions in the framework regions as described above.

As used herein, the term "homolog" or "homology" is to be understood as follows: Homology among proteins and DNA is often concluded on the basis of sequence similarity, especially in bioinformatics. For example, in general, if two or more genes have highly similar DNA sequences, it is likely that they are homologous. But sequence similarity may arise from different ancestors: short sequences may be similar by chance, and sequences may be similar because both were selected to bind to a particular protein, such as a transcription factor. Such sequences are similar but not homologous. Sequence regions that are homologous are also called conserved. This is not to be confused with conservation in amino acid sequences in which the amino acid at a specific position has changed but the physio-chemical properties of the amino acid remain unchanged. Homologous sequences are of two types: orthologous and paralogous. Homologous sequences are orthologous if they were separated by a speciation event: when a species diverges into two separate species, the divergent copies of a single gene in the resulting species are said to be orthologous. Orthologs, or orthologous genes, are genes in different species that are similar to each other because they originated from a common ancestor. The strongest evidence that two similar genes are orthologous is the result of a phylogenetic analysis of the gene lineage. Genes that are found within one clade are orthologs, descended from a common ancestor. Orthologs often, but not always, have the same function. Orthologous sequences provide useful information in taxonomic classification studies of organisms. The pattern of genetic divergence can be used to trace the relatedness of organisms. Two organisms that are very closely related are likely to display very similar DNA sequences between two orthologs. Conversely, an organism that is further removed evolutionarily from another organism is likely to display a greater divergence in the sequence of the orthologs being studied. Homologous sequences are paralogous if they were separated by a gene duplication event: if a gene in an organism is duplicated to occupy two different positions in the same genome, then the two copies are paralogous. A set of sequences that are paralogous are called paralogs of each other. Paralogs typically have the same or similar function, but sometimes do not: due to lack of the original selective pressure upon one copy of the duplicated gene, this copy is free to mutate and acquire new functions. An example can be found in rodents such as rats and mice. Rodents have a pair of paralogous insulin genes, although it is unclear if any divergence in function has occurred. Paralogous genes often belong to the same species, but this is not necessary: for example, the hemoglobin gene of humans and the myoglobin gene of chimpanzees are paralogs. This is a common problem in bioinformatics: when genomes of different species have been sequenced and homologous genes have been found, one can not immediately conclude that these genes have the same or similar function, as they could be paralogs whose function has diverged.

As used herein, a "non-chimpanzee primate" or "non-chimp primate" or grammatical variants thereof refers to any primate animal (i.e. not human) other than chimpanzee, i.e. other than an animal of belonging to the genus *Pan*, and including the species *Pan paniscus* and *Pan troglodytes*, also known as *Anthropopithecus troglodytes* or *Simia satyrus*. It will be understood, however, that it is possible that the antibodies of the invention can also bind with their first and/or second binding domain to the respective epitopes/fragments etc. of said chimpanzees. The intention is merely to avoid animal tests which are carried out with chimpanzees, if desired. It is thus also envisaged that in another embodiment the antibodies of the present invention also bind with their first and/or second binding domain to the respective epitopes of chimpanzees. A "primate", "primate species", "primates" or grammatical variants thereof denote/s an order of eutherian mammals divided into the two suborders of prosimians and anthropoids and comprising apes, monkeys and lemurs. Specifically, "primates" as used herein comprises the suborder *Strepsirrhini* (non-tarsier prosimians), including the infraorder *Lemuriformes* (itself including the superfamilies *Cheirogaleoidea* and *Lemuroidea),* the infraorder *Chiromyiformes* (itself including the family *Daubentoniidae)* and the infraorder *Lorisiformes* (itself including the families *Lorisidae* and *Galagidae).* "Primates" as used herein also comprises the suborder *Haplorrhini,* including the infraorder *Tarsiiformes* (itself including the family *Tarsiidae),* the infraorder *Simiiformes* (itself including the *Platyrrhini*, or New-World monkeys, and the *Catarrhini,* including the *Cercopithecidea,* or Old-World Monkeys).

The non-chimpanzee primate species may be understood within the meaning of the invention to be a lemur, a tarsier, a gibbon, a marmoset (belonging to New-World Monkeys of the family *Cebidae)* or an Old-World Monkey (belonging to the superfamily *Cercopithecoidea).*

As used herein, an "Old-World Monkey" comprises any monkey falling in the superfamily *Cercopithecoidea,* itself subdivided into the families: the *Cercopithecinae*, which are mainly African but include the diverse genus of macaques which are Asian and North African; and the *Colobinae,* which include most of the Asian genera but also the African colobus monkeys.

Specifically, within the subfamily *Cercopithecinae*, an advantageous non-chimpanzee primate may be from the Tribe *Cercopithecini*, within the genus *Allenopithecus* (Allen's Swamp Monkey, *Allenopithecus nigroviridis);* within the genus *Miopithecus* (Angolan Talapoin, *Miopithecus talapoin*; Gabon Talapoin, *Miopithecus ogouensis);* within the genus *Erythrocebus* (Patas Monkey, *Erythrocebus patas*); within the genus *Chlorocebus* (Green Monkey, *Chlorocebus sabaceus;* Grivet, *Chlorocebus aethiops*; Bale Mountains Vervet, *Chlorocebus djamdjamensis;* Tantalus Monkey, *Chlorocebus tantalus;* Vervet Monkey, *Chlorocebus pygerythrus;* Malbrouck, *Chlorocebus cynosuros);* or within the genus *Cercopithecus* (Dryas Monkey or Salongo Monkey, *Cercopithecus dryas*; Diana Monkey, *Cercopithecus diana*; Roloway Monkey, *Cercopithecus roloway*; Greater Spot-nosed Monkey, *Cercopithecus nictitans*; Blue Monkey, *Cercopithecus mitis*; Silver Monkey, *Cercopithecus doggetti;* Golden Monkey, *Cercopithecus kandti;* Sykes's Monkey, *Cercopithecus albogularis;* Mona Monkey, *Cercopithecus mona*; Campbell's Mona Monkey, *Cercopithecus campbelli*; Lowe's Mona Monkey, *Cercopithecus lowei*; Crested Mona Monkey, *Cercopithecus pogonias;* Wolfs Mona Monkey, *Cercopithecus wolfi*; Dent's Mona Monkey, *Cercopithecus denti;* Lesser Spot-nosed Monkey, *Cercopithecus petaurista;* White-throated Guenon, *Cercopithecus erythrogaster*; Sclater's Guenon, *Cercopithecus sclateri;* Red-eared Guenon, *Cercopithecus erythrotis*; Moustached Guenon, *Cercopithecus cephus;* Red-tailed Monkey, *Cercopithecus ascanius;* L'Hoest's Monkey, *Cercopithecus lhoesti;* Preuss's Monkey, *Cercopithecus preussi*; Sun-tailed Monkey, *Cercopithecus solatus*; Hamlyn's Monkey or Owl-faced Monkey, *Cercopithecus hamlyni;* De Brazza's Monkey, *Cercopithecus neglectus*).

Alternatively, an advantageous non-chimpanzee primate, also within the subfamily *Cercopithecinae* but within the Tribe *Papionini,* may be from within the genus *Macaca* (Barbary Macaque, *Macaca sylvanus;* Lion-tailed Macaque, *Macaca silenus*; Southern Pig-tailed Macaque or Beruk, *Macaca nemestrina*; Northern Pig-tailed Macaque, *Macaca leonina*; Pagai Island Macaque or Bokkoi, *Macaca pagensis*; Siberut Macaque, *Macaca siberu;* Moor Macaque, *Macaca maura*; Booted Macaque, *Macaca ochreata*; Tonkean Macaque, *Macaca tonkeana*; Heck's Macaque, *Macaca hecki;* Gorontalo Macaque, *Macaca nigriscens*; Celebes Crested Macaque or Black "Ape", *Macaca nigra*; Cynomolgus monkey or Crab-eating Macaque or Long-tailed Macaque or Kera, *Macaca fascicularis;* Stump-tailed Macaque or Bear Macaque, *Macaca arctoides;* Rhesus Macaque, *Macaca mulatta;* Formosan Rock Macaque, *Macaca cyclopis*; Japanese Macaque, *Macaca fuscata;* Toque Macaque, *Macaca sinica;* Bonnet Macaque, *Macaca radiata;* Barbary Macaque, *Macaca sylvanmus*; Assam Macaque, *Macaca assamensis;* Tibetan Macaque or Milne-Edwards' Macaque, *Macaca thibetana;* Arunachal Macaque or Munzala, *Macaca munzala*); within the genus *Lophocebus* (Gray-cheeked Mangabey, *Lophocebus albigena*;

*Lophocebus albigena albigena; Lophocebus albigena osmani*; *Lophocebus albigena johnstoni;* Black Crested Mangabey, *Lophocebus aterrimus;* Opdenbosch's Mangabey, *Lophocebus opdenboschi;* Highland Mangabey, *Lophocebus kipunji);* within the genus *Papio* (Hamadryas Baboon, *Papio hamadryas;* Guinea Baboon, *Papio papio;* Olive Baboon, *Papio anubis;* Yellow Baboon, *Papio cynocephalus*; Chacma Baboon, *Papio ursinus);* within the genus *Theropithecus* (Gelada, *Theropithecus gelada);* within the genus *Cercocebus* (Sooty Mangabey, *Cercocebus atys*; *Cercocebus atys atys; Cercocebus atys lunulatus;* Collared Mangabey, *Cercocebus torquatus;* Agile Mangabey, *Cercocebus agilis*; Golden-bellied Mangabey, *Cercocebus chrysogaster*; Tana River Mangabey, *Cercocebus galeritus*; Sanje Mangabey, *Cercocebus sanjei*); or within the genus *Mandrillus* (Mandrill, *Mandrillus sphinx;* Drill, *Mandrillus leucophaeus*).

Most preferred is *Macaca fascicularis* (also known as Cynomolgus monkey and, therefore, in the Examples named "Cynomolgus") and *Macaca mulatta* (rhesus monkey, named "rhesus").

Within the subfamily *Colobinae,* an advantageous non-chimpanzee primate may be from the African group, within the genus *Colobus* (Black Colobus, *Colobus satanas*; Angola Colobus, *Colobus angolensis*; King Colobus, *Colobus polykomos;* Ursine Colobus, *Colobus vellerosus*; Mantled Guereza, *Colobus guereza);* within the genus *Piliocolobus* (Western Red Colobus, *Piliocolobus badius; Piliocolobus badius badius*; *Piliocolobus badius temminckii*; *Piliocolobus badius waldronae*; Pennant's Colobus, *Piliocolobus pennantii; Piliocolobus pennantii pennantii; Piliocolobus pennantii epieni*; *Piliocolobus pennantii bouvieri;* Preuss's Red Colobus, *Piliocolobus preussi;* Thollon's Red Colobus, *Piliocolobus tholloni*; Central African Red Colobus, *Piliocolobus foai*; *Piliocolobus foai foai*; *Piliocolobus foai ellioti; Piliocolobus foai oustaleti; Piliocolobus foai semlikiensis; Piliocolobus foai parmentierorum*; Ugandan Red Colobus, *Piliocolobus tephrosceles;* Uzyngwa Red Colobus, *Piliocolobus gordonorum*; Zanzibar Red Colobus, *Piliocolobus kirkii*; Tana River Red Colobus, *Piliocolobus rufomitratus*); or within the genus *Procolobus* (Olive Colobus, *Procolobus verus).*

Within the subfamily *Colobinae,* an advantageous non-chimpanzee primate may alternatively be from the Langur (leaf monkey) group, within the genus *Semnopithecus* (Nepal Gray Langur, *Semnopithecus schistaceus;* Kashmir Gray Langur, *Semnopithecus ajax*; Tarai Gray Langur, *Semnopithecus hector*; Northern Plains Gray Langur, *Semnopithecus entellus;* Black-footed Gray Langur, *Semnopithecus hypoleucos*; Southern Plains Gray Langur, *Semnopithecus dussumieri;* Tufted Gray Langur, *Semnopithecus priam);* within the *T. vetulus* group or the genus *Trachypithecus* (Purple-faced Langur, *Trachypithecus vetulus*; Nilgiri Langur, *Trachypithecus johnii*); within the *T. cristatus* group of the genus *Trachypithecus* (Javan Lutung, *Trachypithecus auratus;* Silvery Leaf Monkey or Silvery Lutung, *Trachypithecus cristatus*; Indochinese Lutung, *Trachypithecus germaini*; Tenasserim Lutung, *Trachypithecus barbei*); within the *T. obscurus* group of the genus *Trachypithecus* (Dusky Leaf Monkey or Spectacled Leaf Monkey, *Trachypithecus obscurus;* Phayre's Leaf Monkey, *Trachypithecus phayrei);* within the *T. pileatus* group of the genus *Trachypithecus* (Capped Langur, *Trachypithecus pileatus*; Shortridge's Langur, *Trachypithecus shortridgei;* Gee's Golden Langur, *Trachypithecus geei*); within the *T. francoisi* group of the genus *Trachypithecus* (Francois' Langur, *Trachypithecus francoisi*; Hatinh Langur, *Trachypithecus hatinhensis;* White-headed Langur, *Trachypithecus poliocephalus*; Laotian Langur, *Trachypithecus laotum*; Delacour's Langur, *Trachypithecus delacouri*; Indochinese Black Langur, *Trachypithecus ebenus);* or within the genus *Presbytis* (Sumatran Surili, *Presbytis melalophos*; Banded Surili, *Presbytis femoralis*; Sarawak Surili, *Presbytis chrysomelas*; White-thighed Surili, *Presbytis siamensis;* White-fronted Surili, *Presbytis frontata;* Javan Surili, *Presbytis comata*; Thomas's Langur, *Presbytis thomasi*; Hose's Langur, *Presbytis hosei;* Maroon Leaf Monkey, *Presbytis rubicunda*; Mentawai Langur or Joja, *Presbytis potenziani*; Natuna Island Surili, *Presbytis natunae*).

Within the subfamily *Colobinae*, an advantageous non-chimpanzee primate may alternatively be from the Odd-Nosed group, within the genus *Pygathrix* (Red-shanked Douc, *Pygathrix nemaeus;* Black-shanked Douc, *Pygathrix nigripes*; Gray-shanked Douc, *Pygathrix cinerea);* within the genus *Rhinopithecus* (Golden Snub-nosed Monkey, *Rhinopithecus roxellana*; Black Snub-nosed Monkey, *Rhinopithecus bieti*; Gray Snub-nosed Monkey, *Rhinopithecus brelichi*; Tonkin Snub-nosed Langur, *Rhinopithecus avunculus*); within the genus *Nasalis* (Proboscis Monkey, *Nasalis larvatus*); or within the genus *Simias* (Pig-tailed Langur, *Simias concolor*).

As used herein, the term "marmoset" denotes any New-World Monkeys of the genus *Callithrix*, for example belonging to the Atlantic marmosets of subgenus *Callithrix* (sic!) (Common Marmoset, *Callithrix (Callithrix) jacchus*; Black-tufted Marmoset, *Callithrix (Callithrix) penicillata;* Wied's Marmoset, *Callithrix (Callithrix) kuhlii;* White-headed Marmoset, *Callithrix (Callithrix) geoffroyi*; Buffy-headed Marmoset, *Callithrix (Callithrix) flaviceps*; Buffy-tufted Marmoset, *Callithrix (Callithrix) aurita*); belonging to the Amazonian marmosets of subgenus *Mico* (Rio Acari Marmoset, *Callithrix (Mico) acariensis*; Manicore Marmoset, *Callithrix (Mico) manicorensis*; Silvery Marmoset, *Callithrix (Mico) argentata*; White Marmoset, *Callithrix (Mico) leucippe;* Emilia's Marmoset, *Callithrix (Mico) emiliae*; Black-headed Marmoset, *Callithrix (Mico) nigriceps;* Marca's Marmoset, *Callithrix (Mico)marcai*; Black-tailed Marmoset, *Callithrix (Mico) melanura*; Santarem Marmoset, *Callithrix (Mico) humeralifera*; Maués Marmoset, *Callithrix (Mico) mauesi*; Gold-and-white Marmoset, *Callithrix (Mico) chrysoleuca*; Hershkovitz's Marmoset, *Callithrix (Mico) intermedia;* Satéré Marmoset, *Callithrix (Mico) saterei*); Roosmalens' Dwarf Marmoset belonging to the subgenus Callibella (*Callithrix (Callibella) humilis);* or the Pygmy Marmoset belonging to the subgenus Cebuella (*Callithrix (Cebuella) pygmaea*).

Other genera of the New-World Monkeys comprise tamarins of the genus *Saguinus* (comprising the S. oedipus-group, the S. *midas* group, the S. *nigricollis* group, the S. *mystax* group, the S. *bicolor* group and the S. *inustus* group) and squirrel monkeys of the genus *Samiri* (e.g. *Saimiri sciureus, Saimiri oerstedii*, *Saimiri ustus, Saimiri boliviensis*, *Saimiri vanzolini*)

It is disclosed that in the bispecific single chain antibody molecule of the invention, the non-chimpanzee primate may be an old world monkey. More preferably, the old world monkey is a monkey of the Papio genus Macaque genus. Most preferably, the monkey of the Macaque genus is Assamese macaque *(Macaca assamensis*), Barbary macaque *(Macaca sylvanus),* Bonnet macaque *(Macaca radiata),* Booted or Sulawesi-Booted macaque *(Macaca ochreata*), Sulawesi-crested macaque *(Macaca nigra),* Formosan rock macaque *(Macaca cyclopsis*), Japanese snow macaque or Japanese macaque *(Macaca fuscata),* Cynomologus monkey or crab-eating macaque or long-tailed macaque or Java macaque *(Macaca fascicularis*), Lion-tailed macaque *(Macaca silenus),* Pigtailed macaque *(Macaca nemestrina),* Rhesus macaque *(Macaca mulatta*),

Tibetan macaque *(Macaca thibetana),* Tonkean macaque *(Macaca tonkeana*), Toque macaque *(Macaca sinica),* Stump-tailed macaque or Red-faced macaque or Bear monkey *(Macaca arctoides),* or Moor macaque *(Macaca maurus).* Most preferably, the monkey of the Papio genus is *Hamadryas Baboon, Papio hamadryas*; *Guinea Baboon, Papio papio; Olive Baboon, Papio anubis*; *Yellow Baboon, Papio cynocephalus*; *Chacma Baboon, Papio ursinus*.

As described herein in the bispecific single chain antibody molecule of the invention, the non-chimpanzee primate is a new world monkey, the new world monkey is a monkey of the Callithrix genus (marmoset), the Saguinus genus or the Samiri genus, the monkey of the Callithrix genus is *Callithrix jacchus,* the monkey of the Saguinus genus is *Saguinus oedipus* and the monkey of the Samiri genus is *Saimiri sciureus*.

The term "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located in the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface molecules which are located on the plasma membrane are proteins which have been modified at a cysteine residue to bear a palmitoyl group, proteins modified at a C-terminal cysteine residue to bear a farnesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies. Examples of cell surface antigens are CD3 epsilon and PSCA, respectively CD19, C-MET, Endosialin, EpCAM, IGF-1R or FAP$\alpha$.

As described herein above, PSCA is a cell surface antigen which is a target for therapy of various cancers, including, but not limited to prostate cancer, bladder cancer or pancreatic cancer.

As also described herein above, CD19 is a cell surface antigen which is a target for therapy of various cancers, including, but not limited to B-cell malignancies such as non-Hodgkin Lymphoma, B-cell mediated autoimmune diseases or the depletion of B-cells.

As further described herein above, C-MET is a cell surface antigen which is a target for therapy of various cancers, including, but not limited to carcinomas, sarcomas, glioblastomas/astrocytomas, melanomas, mesotheliomas, Wilms tumors or hematopoietic malignancies such as leukemias, lymphomas or multiple myelomas. Moreover, as described herein above, Endosialin is a cell surface antigen which is a target for therapy of various cancers, including, but not limited to carcinomas (breast, kidney, lung, colorectal, colon, pancreas mesothelioma), sarcomas, and neuroectodermal tumors (melanoma, glioma, neuroblastoma).

As also described herein above, EpCAM is a cell surface antigen which is a target for therapy of various cancers, including, but not limited to epithelial cancer or a minimal residual cancer.

Furthermore, as described herein above, FAP alpha is a cell surface antigen which is a target for therapy of various cancers, including, but not limited to, epithelial cancer. Moreover, as described herein above, IGF-1R is a cell surface antigen which is a target for therapy of various cancers, including, but not limited to, bone or soft tissue cancer (e.g. Ewing sarcoma), breast, liver, lung, head and neck, colorectal, prostate, leiomyosarcoma, cervical and endometrial cancer, ovarian, prostate, and pancreatic cancer, and autoimmune diseases, including, but not limited to, preferably psoriasis.

In light of this, PSCA, respectively CD19, C-MET, Endosialin, EpCAM, IGF-1R or FAP$\alpha$ can also be characterized as a tumor antigen. The term "tumor antigen" as used herein may be understood as those antigens that are presented on tumor cells. These antigens can be presented on the cell surface with an extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue. Example for tumor antigens in line with the present invention are PSCA, CD19, C-MET, Endosialin, EpCAM, IGF-1 R and FAPα.
As described herein above the bispecific single chain antibody molecule of the invention specifically binds with the first binding domain to an epitope of human and *Callithrix* jacchus, Saguinus oedipus or Saimiri sciureus CD3ε (epsilon) chain, wherein said epitope is part of an amino acid sequence comprised in the group consisting of 27 amino acid residues as depicted in SEQ ID NOs. 2, 4, 6, or 8 and comprises at least the amino acid sequence Gln-Asp-Gly-Asn-Glu.

In line with the present invention it is preferred for the bispecific single chain antibody molecule of the invention that said epitope is part of an amino acid sequence comprising 26, 25, 24, 23, 22, 21, 20, 19, 18, 17, 16, 15, 14, 13, 12, 11, 10, 9, 8, 7, 6 or 5 amino acids.
As described the epitope comprises at least the amino acid sequence Gln-Asp-Gly-Asn-Glu (Q-D-G-N-E).
Within the present invention, a functional fragment of the N-terminal 1-27 amino acid residues means that said functional fragment is still a context-independent epitope maintaining its three-dimensional structural integrity when taken out of its native environment in the CD3 complex (and fused to a heterologous amino acid sequence such as EpCAM or an immunoglobulin Fc part, e.g. as shown in Example 3.1). The maintenance of the three-dimensional structure within the 27 amino acid N-terminal polypeptide or functional fragment thereof of CD3 epsilon can be used for the generation of binding domains which bind to the N-terminal CD3 epsilon polypeptide fragment *in vitro* and to the native (CD3 epsilon subunit of the) CD3 complex on T cells *in vivo* with the same binding affinity. Within the present disclosure a functional fragment of the N-terminal 1-27 amino acid residues means that CD3 binding domains provided herein can still bind to such functional fragments in a context-independent manner. The person skilled in the art is aware of methods for epitope mapping to determine which amino acid residues of an epitope are recognized by such anti-CD3 binding domains (e.g. alanine scanning; see appended examples).

In one embodiment of the invention, the bispecific single chain antibody molecule of the invention comprises a (first) binding domain which specifically binds to an epitope as defined in the claims and a second binding domain binding to the cell surface antigen PSCA. In alternative embodiments of the invention the said secong binding domain capable of binding to the cell surface antigen CD19, C-MET, Endosialin, EpCAM, IGF-1 R or FAPα
Within the present invention it is further preferred that the second binding domain binds to the human cell surface antigen PSCA, respectively CD19, C-MET, Endosialin, EpCAM, IGF-1R or FAPα and/or a non-chimpanzee primate PSCA, respectively CD19, C-MET, Endosialin, EpCAM, IGF-1R or FAPα. Particularly preferred, the second binding domain binds to the human cell surface antigen PSCA, respectively CD19, C-MET, Endosialin, EpCAM, IGF-1R or FAPα and a non-chimpanzee primate PSCA, respectively CD19, C-MET, Endosialin, EpCAM, IGF-1R or FAPα, preferably a macaque PSCA, respectively CD19, C-MET, Endosialin, EpCAM, IGF-1R or FAPα. It is to be understood, that the second binding domain binds to at least one non-chimpanzee primate PSCA, respectively CD19, C-MET, Endosialin, EpCAM, IGF-1R or FAPα, however, it may also bind to two, three or more, non-chimpanzee primate PSCA homologs, respectively CD19 homologs, C-MET homologs, Endosialin homologs, EpCAM homologs, IGF-1R homologs or FAPα homologs. For example, the second binding domain may bind to the Cynomogus monkey PSCA, respectively CD19, C-MET, Endosialin, EpCAM, IGF-1R or FAPα, and to the Rhesus monkey PSCA, respectively CD19, C-MET, Endosialin, EpCAM, IGF-1 R or FAPα.
For the generation of the second binding domain of the bispecific single chain antibody molecule of the invention, e.g. bispecific single chain antibodies as defined herein, monoclonal antibodies binding to both of the respective human and/or non-chimpanzee primate cell surface antigen such as PSCA, respectively CD19, C-MET, Endosialin, EpCAM, IGF-1R or FAPα, can be utilized. Appropriate binding domains for the bispecific polypeptide as defined herein e.g. can be derived from cross-species specific monoclonal antibodies by recombinant methods described in the art.
A monoclonal antibody binding to a human cell surface antigen and to the homolog of said cell surface antigen in a non-chimpanzee primate can be tested by FACS assays as set forth above. It is evident to those skilled in the art that cross-species specific antibodies can also be generated by hybridoma techniques described in the literature (Milstein and Köhler, Nature 256 (1975), 495-7). For example, mice may be alternately immunized with human and non-chimpanzee primate cell surface antigen, such as PSCA, respectively CD19, C-MET, Endosialin, EpCAM, IGF-1R or FAPα. From these mice, cross-species specific antibody-producing hybridoma cells are isolated via hybridoma technology and analysed by FACS as set forth above. The generation and analysis of bispecific polypeptides such as bispecific single chain antibodies exhibiting cross-species specificity as described herein is shown in the following examples. The advantages of the bispecific single chain antibodies exhibiting cross-species specificity include the points enumerated herein.

It is particularly preferred for the bispecific single chain antibody molecule of the invention that the first binding domain comprises a VL region comprising CDR-L1, CDR-L2 and CDR-L3 selected from:

(a) CDR-L1 as depicted in SEQ ID NO. 27, CDR-L2 as depicted in SEQ ID NO. 28 and CDR-L3 as depicted in SEQ ID NO. 29;
(b) CDR-L1 as depicted in SEQ ID NO. 117, CDR-L2 as depicted in SEQ ID NO. 118 and CDR-L3 as depicted in SEQ ID NO. 119; and

(c) CDR-L1 as depicted in SEQ ID NO. 153, CDR-L2 as depicted in SEQ ID NO. 154 and CDR-L3 as depicted in SEQ ID NO. 155.

The variable regions, i.e. the variable light chain ("L" or "VL") and the variable heavy chain ("H" or "VH") are understood in the art to provide the binding domain of an antibody. This variable regions harbor the complementary determining regions.

The term "complementary determining region" (CDR) is well known in the art to dictate the antigen specificity of an antibody. The term "CDR-L" or "L CDR" or "LCDR" refers to CDRs in the VL, whereas the term "CDR-H" or "H CDR" or "HCDR" refers to the CDRs in the VH.

In an alternatively preferred embodiment of the bispecific single chain antibody molecule of the invention the first binding domain comprises a VH region comprising CDR-H 1, CDR-H2 and CDR-H3 selected from:

(a) CDR-H1 as depicted in SEQ ID NO. 12, CDR-H2 as depicted in SEQ ID NO. 13 and CDR-H3 as depicted in SEQ ID NO. 14;

(b) CDR-H1 as depicted in SEQ ID NO. 30, CDR-H2 as depicted in SEQ ID NO. 31 and CDR-H3 as depicted in SEQ ID NO. 32;

(c) CDR-H1 as depicted in SEQ ID NO. 48, CDR-H2 as depicted in SEQ ID NO. 49 and CDR-H3 as depicted in SEQ ID NO. 50;

(d) CDR-H1 as depicted in SEQ ID NO. 66, CDR-H2 as depicted in SEQ ID NO. 67 and CDR-H3 as depicted in SEQ ID NO. 68;

(e) CDR-H1 as depicted in SEQ ID NO. 84, CDR-H2 as depicted in SEQ ID NO. 85 and CDR-H3 as depicted in SEQ ID NO. 86;

(f) CDR-H1 as depicted in SEQ ID NO. 102, CDR-H2 as depicted in SEQ ID NO. 103 and CDR-H3 as depicted in SEQ ID NO. 104;

(g) CDR-H1 as depicted in SEQ ID NO. 120, CDR-H2 as depicted in SEQ ID NO. 121 and CDR-H3 as depicted in SEQ ID NO. 122;

(h) CDR-H1 as depicted in SEQ ID NO. 138, CDR-H2 as depicted in SEQ ID NO. 139 and CDR-H3 as depicted in SEQ ID NO. 140;

(i) CDR-H1 as depicted in SEQ ID NO. 156, CDR-H2 as depicted in SEQ ID NO. 157 and CDR-H3 as depicted in SEQ ID NO. 158; and

(j) CDR-H1 as depicted in SEQ ID NO. 174, CDR-H2 as depicted in SEQ ID NO. 175 and CDR-H3 as depicted in SEQ ID NO. 176.

It is further preferred that the binding domain binding to an epitope of human and non-chimpanzee primate CD3ε chain comprises a VL region selected from the group consisting of a VL region as depicted in SEQ ID NO. 35, 39, 125, 129, 161 or 165.

It is alternatively preferred that the first binding domain comprises a VH region selected from the group consisting of a VH region as depicted in SEQ ID NO. 15, 19, 33, 37, 51, 55, 69, 73, 87, 91, 105, 109, 123, 127, 141, 145, 159, 163, 177 or 181.

More preferably, the bispecific single chain antibody molecule of the invention is characterized by the first binding domain, which comprises a VL region and a VH region selected from the group consisting of:

(a) a VL region as depicted in SEQ ID NO. 17 or 21 and a VH region as depicted in SEQ ID NO. 15 or 19;

(b) a VL region as depicted in SEQ ID NO. 35 or 39 and a VH region as depicted in SEQ ID NO. 33 or 37;

(c) a VL region as depicted in SEQ ID NO. 53 or 57 and a VH region as depicted in SEQ ID NO. 51 or 55;

(d) a VL region as depicted in SEQ ID NO. 71 or 75 and a VH region as depicted in SEQ ID NO. 69 or 73;

(e) a VL region as depicted in SEQ ID NO. 89 or 93 and a VH region as depicted in SEQ ID NO. 87 or 91;

(f) a VL region as depicted in SEQ ID NO. 107 or 111 and a VH region as depicted in SEQ ID NO. 105 or 109;

(g) a VL region as depicted in SEQ ID NO. 125 or 129 and a VH region as depicted in SEQ ID NO. 123 or 127;

(h) a VL region as depicted in SEQ ID NO. 143 or 147 and a VH region as depicted in SEQ ID NO. 141 or 145;

(i) a VL region as depicted in SEQ ID NO. 161 or 165 and a VH region as depicted in SEQ ID NO. 159 or 163; and

(j) a VL region as depicted in SEQ ID NO. 179 or 183 and a VH region as depicted in SEQ ID NO. 177 or 181.

According to a preferred embodiment of the bispecific single chain antibody molecule of the invention the pairs of VH-regions and VL-regions in the first binding domain binding to CD3 epsilon are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence. Put in other words, the domain arrangement in the CD3 binding domain of the bispecific single chain antibody molecule of the invention is preferably VH-VL, with said CD3 binding domain located C-terminally to the second (cell surface antigen, such as PSCA, CD19, C-MET, Endosialin, EpCAM, IGF-1R or FAPα) binding domain. Preferably the VH-VL comprises or is SEQ ID NO. 185.

A preferred embodiment of the above described bispecific single chain antibody molecule of the invention is characterized by the first binding domain comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187.

The invention further relates to an above described bispecific single chain antibody, wherein the second binding domain binds to the cell surface antigen PSCA, CD19, C-MET, Endosialin, EpCAM, IGF-1R or FAPα.

**PSCAxCD3**

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 382 - 384 and CDR L1-3 of SEQ ID NO: 377 - 379;
b) CDR H1-3 of SEQ ID NO: 400 - 402 and CDR L1-3 of SEQ ID NO: 395 - 397;
c) CDR H1-3 of SEQ ID NO: 414 - 416 and CDR L1-3 of SEQ ID NO: 409 - 411;
d) CDR H1-3 of SEQ ID NO: 432 - 434 and CDR L1-3 of SEQ ID NO: 427 - 429;
e) CDR H1-3 of SEQ ID NO: 1215 - 1217 and CDR L1-3 of SEQ ID NO: 1220 - 1222;
f) CDR H1-3 of SEQ ID NO: 1187 - 1189 and CDR L1-3 of SEQ ID NO: 1192 - 1194;
g) CDR H1-3 of SEQ ID NO: 1173 - 1175 and CDR L1-3 of SEQ ID NO: 1178 - 1180;
h) CDR H1-3 of SEQ ID NO: 1229 - 1231 and CDR L1-3 of SEQ ID NO: 1234 - 1236;
i) CDR H1-3 of SEQ ID NO: 1201 - 1203 and CDR L1-3 of SEQ ID NO: 1206 - 1208;
k) CDR H1-3 of SEQ ID NO: 1257 - 1259 and CDR L1-3 of SEQ ID NO: 1262 - 1264; and
l) CDR H1-3 of SEQ ID NO: 1243 - 1245 and CDR L1-3 of SEQ ID NO: 1248 - 1250.

The sequences of the corresponding VL- and VH-regions of the second binding domain of the bispecific single chain antibody molecule of the invention as well as of the respective scFvs are shown in the sequence listing.
In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH PSCA-VL PSCA-VH CD3-VL CD3 or VL PSCA-VH PSCA-VH CD3-VL CD3.

A particularly preferred embodiment of the invention concerns an above characterized polypeptide, wherein the bispecific single chain antibody molecule comprises a sequence selected from:

(a) an amino acid sequence as depicted in any of SEQ ID NOs: 389, 421, 439, 391, 405, 423, 441, 1226, 1198, 1184, 1240, 1212, 1268 or 1254
(b) an amino acid sequence encoded by a nucleic acid sequence as depicted in any of SEQ ID NOs: 390, 422, 440, 392, 406, 424, 442, 1227, 1199, 1185, 1241, 1213, 1269 or 1255; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 389, 421, 439, 391, 405, 423, 441, 1226, 1198, 1184, 1240, 1212, 1268 or 1254, as well as to an amino acid sequences at least 85% identical, preferably 90 %, more preferred at least 95 % identical, most preferred at least 96, 97, 98, or 99 % identical to the amino acid sequence of SEQ ID NOs: 389, 421, 439, 391, 405, 423, 441, 1226, 1198, 1184, 1240, 1212, 1268 or 1254. The invention relates also to the corresponding nucleic acid sequences as depicted in any of SEQ ID NOs: 390, 422, 440, 392, 406, 424, 442, 1227, 1199, 1185, 1241, 1213, 1269 or 1255 as well as to nucleic acid sequences at least 85% identical, preferably 90 %, more preferred at least 95 % identical, most preferred at least 96, 97, 98, or 99 % identical to the nucleic acid sequences shown in SEQ ID NOs: 390, 422, 440, 392, 406, 424, 442, 1227, 1199, 1185, 1241, 1213, 1269 or 1255. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the PSCAxCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen PSCA recognized by their second binding domain.

**CD19xCD3**

According to an alternatively preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 478 - 480 and CDR L1-3 of SEQ ID NO: 473 - 475;
b) CDR H1-3 of SEQ ID NO: 530 - 532 and CDR L1-3 of SEQ ID NO: 525 - 527;
c) CDR H1-3 of SEQ ID NO: 518 - 520 and CDR L1-3 of SEQ ID NO: 513 - 515;
d) CDR H1-3 of SEQ ID NO: 506 - 508 and CDR L1-3 of SEQ ID NO: 501 - 503;
e) CDR H1-3 of SEQ ID NO: 494 - 496 and CDR L1-3 of SEQ ID NO: 489 - 491;
f) CDR H1-3 of SEQ ID NO: 542 - 544 and CDR L1-3 of SEQ ID NO: 537 - 539;
g) CDR H1-3 of SEQ ID NO: 554 - 556 and CDR L1-3 of SEQ ID NO: 549 - 551;
h) CDR H1-3 of SEQ ID NO: 566 - 568 and CDR L1-3 of SEQ ID NO: 561 - 563;
i) CDR H1-3 of SEQ ID NO: 578 - 580 and CDR L1-3 of SEQ ID NO: 573 - 575;
j) CDR H1-3 of SEQ ID NO: 590 - 592 and CDR L1-3 of SEQ ID NO: 585 - 587;
k) CDR H1-3 of SEQ ID NO: 602 - 604 and CDR L1-3 of SEQ ID NO: 597 - 599;
l) CDR H1-3 of SEQ ID NO: 614 - 616 and CDR L1-3 of SEQ ID NO: 609 - 611;
m) CDR H1-3 of SEQ ID NO: 626 - 628 and CDR L1-3 of SEQ ID NO: 621 - 623;
n) CDR H1-3 of SEQ ID NO: 638 - 640 and CDR L1-3 of SEQ ID NO: 633 - 635;
o) CDR H1-3 of SEQ ID NO: 650 - 652 and CDR L1-3 of SEQ ID NO: 645 - 647;
p) CDR H1-3 of SEQ ID NO: 662 - 664 and CDR L1-3 of SEQ ID NO: 657 - 659;
q) CDR H1-3 of SEQ ID NO: 674 - 676 and CDR L1-3 of SEQ ID NO: 669 - 671;
r) CDR H1-3 of SEQ ID NO: 686 - 688 and CDR L1-3 of SEQ ID NO: 681 - 683;
s) CDR H1-3 of SEQ ID NO: 698 - 700 and CDR L1-3 of SEQ ID NO: 693 - 695;
t) CDR H1-3 of SEQ ID NO: 710 - 712 and CDR L1-3 of SEQ ID NO: 705 - 707;
u) CDR H1-3 of SEQ ID NO: 722 - 724 and CDR L1-3 of SEQ ID NO: 717 - 719;
v) CDR H1-3 of SEQ ID NO: 734 - 736 and CDR L1-3 of SEQ ID NO: 729 - 731;
w) CDR H1-3 of SEQ ID NO: 746 - 748 and CDR L1-3 of SEQ ID NO: 741 - 743;
x) CDR H1-3 of SEQ ID NO: 758 - 760 and CDR L1-3 of SEQ ID NO: 753 - 755;
y) CDR H1-3 of SEQ ID NO: 1271 - 1273 and CDR L1-3 of SEQ ID NO: 1276 - 1278;
z) CDR H1-3 of SEQ ID NO: 1285 - 1287 and CDR L1-3 of SEQ ID NO: 1290 - 1292;

aa) CDR H1-3 of SEQ ID NO: 1299 - 1301 and CDR L1-3 of SEQ ID NO: 1304 - 1306;
ab) CDR H1-3 of SEQ ID NO: 1313 - 1315 and CDR L1-3 of SEQ ID NO: 1318 - 1320;
ac) CDR H1-3 of SEQ ID NO: 1327 - 1329 and CDR L1-3 of SEQ ID NO: 1332 - 1334;
ad) CDR H1-3 of SEQ ID NO: 1341 - 1343 and CDR L1-3 of SEQ ID NO: 1346 - 1348;
ae) CDR H1-3 of SEQ ID NO: 1355 - 1357 and CDR L1-3 of SEQ ID NO: 1360 - 1362;
af) CDR H1-3 of SEQ ID NO: 1369 - 1371 and CDR L1-3 of SEQ ID NO: 1374 - 1376; and
ag) CDR H1-3 of SEQ ID NO: 1383 - 1385 and CDR L1-3 of SEQ ID NO: 1388 - 1390.

The sequences of the corresponding VL- and VH-regions of the second binding domain of the bispecific single chain antibody molecule of the invention as well as of the respective scFvs are shown in the sequence listing.
In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH CD19-VL CD19-VH CD3-VL CD3 or VL CD19-VH CD19-VH CD3-VL CD3. More preferably, the binding domains are arranged in the order VL CD19-VH CD19-VH CD3-VL CD3.

A particularly preferred embodiment of the invention concerns an above characterized polypeptide, wherein the bispecific single chain antibody molecule comprises a sequence selected from:

(a) an amino acid sequence as depicted in any of SEQ ID NOs. 481, 485, 483, 533, 521, 509, 497, 545, 557, 569, 581, 593, 605, 617, 629, 641, 653, 665, 677, 689, 701, 713, 725, 737, 749, 761, 1282, 1296, 1310, 1324, 1338, 1352, 1366, 1380 or 1394 ;
(b) an amino acid sequence encoded by a nucleic acid sequence as depicted in any of SEQ ID NOs: 482, 486, 484, 534, 522, 510, 498, 546, 558, 570, 582, 594, 606, 618, 630, 642, 654, 666, 678, 690, 702, 714, 726, 738, 750, 762, 1283, 1297, 1311, 1325, 1339, 1353, 1367, 1381 or 1395; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 481, 485, 483, 533, 521, 509, 497, 545, 557, 569, 581, 593, 605, 617, 629, 641, 653, 665, 677, 689, 701, 713, 725, 737, 749, 761, 1282, 1296, 1310, 1324, 1338, 1352, 1366, 1380 or 1394, as well as to an amino acid sequences at least 85% identical, preferably 90 %, more preferred at least 95 % identical, most preferred at least 96, 97, 98, or 99 % identical to the amino acid sequence of SEQ ID NOs: 481, 485, 483, 533, 521, 509, 497, 545, 557, 569, 581, 593, 605, 617, 629, 641, 653, 665, 677, 689, 701, 713, 725, 737, 749, 761, 1282, 1296, 1310, 1324, 1338, 1352, 1366, 1380 or 1394. The invention relates also to the corresponding nucleic acid sequences as depicted in any of SEQ ID NOs: 482, 486, 484, 534, 522, 510, 498, 546, 558, 570, 582, 594, 606, 618, 630, 642, 654, 666, 678, 690, 702, 714, 726, 738, 750, 762, 1283, 1297, 1311, 1325, 1339, 1353, 1367, 1381 or 1395 as well as to nucleic acid sequences at least 85% identical, preferably 90 %, more preferred at least 95 % identical, most preferred at least 96, 97, 98, or 99 % identical to the nucleic acid sequences shown in SEQ ID NOs: 482, 486, 484, 534, 522, 510, 498, 546, 558, 570, 582, 594, 606, 618, 630, 642, 654, 666, 678, 690, 702, 714, 726, 738, 750, 762, 1283, 1297, 1311, 1325, 1339, 1353, 1367, 1381 or 1395. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the CD19xCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen CD19 recognized by their second binding domain.

**c-METxCD3**

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 821 - 823 and CDR L1-3 of SEQ ID NO: 816 - 818;
b) CDR H1-3 of SEQ ID NO: 836 - 838 and CDR L1-3 of SEQ ID NO: 833 - 835;
c) CDR H1-3 of SEQ ID NO: 845 - 847 and CDR L1-3 of SEQ ID NO: 840 - 842;
d) CDR H1-3 of SEQ ID NO: 863 - 865 and CDR L1-3 of SEQ ID NO: 858 - 860;
e) CDR H1-3 of SEQ ID NO: 881 - 883 and CDR L1-3 of SEQ ID NO: 876 - 878;
f) CDR H1-3 of SEQ ID NO: 899 - 901 and CDR L1-3 of SEQ ID NO: 894 - 896;
g) CDR H1-3 of SEQ ID NO: 1401 - 1403 and CDR L1-3 of SEQ ID NO: 1406 - 1408;
h) CDR H1-3 of SEQ ID NO: 1415 - 1417 and CDR L1-3 of SEQ ID NO: 1420 - 1422;
i) CDR H1-3 of SEQ ID NO: 1429 - 1431 and CDR L1-3 of SEQ ID NO: 1434 - 1436;
j) CDR H1-3 of SEQ ID NO: 1443 - 1445 and CDR L1-3 of SEQ ID NO: 1448 - 1450;
k) CDR H1-3 of SEQ ID NO: 1457 - 1459 and CDR L1-3 of SEQ ID NO: 1462 - 1464;

l) CDR H1-3 of SEQ ID NO: 1471 - 1473 and CDR L1-3 of SEQ ID NO: 1476 - 1478;
m) CDR H1-3 of SEQ ID NO: 1639 - 1641 and CDR L1-3 of SEQ ID NO: 1644 - 1646;
n) CDR H1-3 of SEQ ID NO: 1625 - 1627 and CDR L1-3 of SEQ ID NO: 1630 - 1632;
o) CDR H1-3 of SEQ ID NO: 1611 - 1613 and CDR L1-3 of SEQ ID NO: 1616 - 1618;
p) CDR H1-3 of SEQ ID NO: 1597 - 1599 and CDR L1-3 of SEQ ID NO: 1602 - 1604;
q) CDR H1-3 of SEQ ID NO: 1569 - 1571 and CDR L1-3 of SEQ ID NO: 1574 - 1576;
r) CDR H1-3 of SEQ ID NO: 1555 - 1557 and CDR L1-3 of SEQ ID NO: 1560 - 1562;
s) CDR H1-3 of SEQ ID NO: 1583 - 1585 and CDR L1-3 of SEQ ID NO: 1588 - 1590;
t) CDR H1-3 of SEQ ID NO: 1541 - 1543 and CDR L1-3 of SEQ ID NO: 1546 - 1548;
u) CDR H1-3 of SEQ ID NO: 1513 - 1515 and CDR L1-3 of SEQ ID NO: 1518 - 1520;
v) CDR H1-3 of SEQ ID NO: 1527 - 1529 and CDR L1-3 of SEQ ID NO: 1532 - 1534;
w) CDR H1-3 of SEQ ID NO: 1499 - 1501 and CDR L1-3 of SEQ ID NO: 1504 - 1506; and
x) CDR H1-3 of SEQ ID NO: 1485 - 1487 and CDR L1-3 of SEQ ID NO: 1490 - 1492.

The sequences of the corresponding VL- and VH-regions of the second binding domain of the bispecific single chain antibody molecule of the invention as well as of the respective scFvs are shown in the sequence listing.
In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH C-MET-VL C-MET-VH CD3-VL CD3 or VL C-MET-VH C-MET-VH CD3-VL CD3.
A particularly preferred embodiment of the invention concerns an above characterized polypeptide, wherein the bispecific single chain antibody molecule comprises a sequence selected from:

(a) an amino acid sequence as depicted in any of SEQ ID NOs. 829, 853, 871, 889, 831, 855, 873, 891, 905, 1412, 1426, 1440, 1454, 1468 or 1482;
(b) an amino acid sequence encoded by a nucleic acid sequence as depicted in any of SEQ ID NOs: 830, 854, 872, 890, 832, 856, 874, 892, 906, 1413, 1427, 1441, 1455, 1469, or 1483; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 829, 853, 871, 889, 831, 855, 873, 891, 905, 1412, 1426, 1440, 1454, 1468 or 1482;, as well as to an amino acid sequences at least 85% identical, preferably 90 %, more preferred at least 95 % identical, most preferred at least 96, 97, 98, or 99 % identical to the amino acid sequence of SEQ ID NOs: 829, 853, 871, 889, 831, 855, 873, 891, 905, 1412, 1426, 1440, 1454, 1468 or 1482;. The invention relates also to the corresponding nucleic acid sequences as depicted in any of SEQ ID NOs: 830, 854, 872, 890, 832, 856, 874, 892, 906, 1413, 1427, 1441, 1455, 1469, or 1483; as well as to nucleic acid sequences at least 85% identical, preferably 90 %, more preferred at least 95 % identical, most preferred at least 96, 97, 98, or 99 % identical to the nucleic acid sequences shown in SEQ ID NOs: 830, 854, 872, 890, 832, 856, 874, 892, 906, 1413, 1427, 1441, 1455, 1469, or 1483;. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).
Preferred domain arrangements in the C-METxCD3 bispecific single chain antibody constructs of the invention are shown in the following examples.
In a preferred embodiment of the invention, the bispecific single chain antibodies are cross-species specific for CD3 epsilon and for the human and non-chimpanzee primate cell surface antigen C-MET recognized by their second binding domain.

**EndosialinxCD3**

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

CDR H1-3 of SEQ ID NO: 910 - 912 and CDR L1-3 of SEQ ID NO: 907 - 909.

Starting from these CDR sequences of the heavy and light chain for the second binding domain the person skilled in the art can produce a bispecific single chain antibody molecule of the invention without any further inventive ado. In particular, the CDR sequences can be positioned in a framework of a VL and a VH chain and arranged in form of a scFv.
According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 1653 - 1655 and CDR L1-3 of SEQ ID NO: 1658 - 1660;
b) CDR H1-3 of SEQ ID NO: 1667 - 1669 and CDR L1-3 of SEQ ID NO: 1672 - 1674;
c) CDR H1-3 of SEQ ID NO: 1681 - 1683 and CDR L1-3 of SEQ ID NO: 1686 - 1688; and
d) CDR H1-3 of SEQ ID NO: 1695 - 1697 and CDR L1-3 of SEQ ID NO: 1700 - 1702;
e) CDR H1-3 of SEQ ID NO: 1709 - 1711 and CDR L1-3 of SEQ ID NO: 1714 - 1716; and
f) CDR H1-3 of SEQ ID NO: 1723 - 1725 and CDR L1-3 of SEQ ID NO: 1728 - 1730.

In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH Endosialin -VL Endosialin -VH CD3-VL CD3 or VL Endosialin -VH Endosialin -VH CD3-VL CD3.

A particularly preferred embodiment of the invention concerns an above characterized polypeptide, wherein the bispecific single chain antibody molecule comprises an above characterized first binding domain capable of binding to an epitope of human and non-chimpanzee primate CD3ε and a second binding domain capable of binding to Endosialin and comprising the CDR H1, 2 and 3 of SEQ ID NOs: 910 - 912 and the CDR L1, 2 and 3 of SEQ ID NOs: 907 - 909, or CDR amino acid sequences at least 50%, 60%, 70%, 75%, 80%, 85%, or 90 % identical, more preferred at least 95% identical, most preferred at least 96%, 97%, 98%, or 99% identical to each of the respective amino acid sequences of the above defined CDRs. It is to be understood that the sequence identity is determined over the entire CDRH or CDRL amino acid sequence.
A particularly preferred embodiment of the invention concerns an above characterized polypeptide, wherein the bispecific single chain antibody molecule comprises a sequence selected from:

(a) an amino acid sequence as depicted in any of SEQ ID NOs. 1664, 1678, 1692, 1706, 1720, or 1734;
(b) an amino acid sequence encoded by a nucleic acid sequence as depicted in any of SEQ ID NOs: 1665, 1679, 1693, 1707, 1721, or 1735; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 1664, 1678, 1692, 1706, 1720, or 1734, as well as to an amino acid sequences at least 85% identical, preferably 90 %, more preferred at least 95 % identical, most preferred at least 96, 97, 98, or 99 % identical to the amino acid sequence of SEQ ID NOs: 1664, 1678, 1692, 1706, 1720, or 1734. The invention relates also to the corresponding nucleic acid sequences as depicted in any of SEQ ID NOs: 1665, 1679, 1693, 1707, 1721, or 1735; as well as to nucleic acid sequences at least 85% identical, preferably 90 %, more preferred at least 95 % identical, most preferred at least 96, 97, 98, or 99 % identical to the nucleic acid sequences shown in SEQ ID NOs: 1665, 1679, 1693, 1707, 1721, or 1735.
If not indicated otherwise, it is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence or CDR sequence having e.g. 50% (60%, 70%, 75%, 80%, 85%, 90%, 95%, 96%, 97%, 98% or 99%) sequence identity to the nucleotide or amino acid or CDR sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the EndosialinxCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen Endosialin recognized by their second binding domain.

## EpCAMxCD3

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 940 - 942 and CDR L1-3 of SEQ ID NO: 935 - 937;
b) CDR H1-3 of SEQ ID NO: 956 - 958 and CDR L1-3 of SEQ ID NO: 951 - 953;
c) CDR H1-3 of SEQ ID NO: 968 - 970 and CDR L1-3 of SEQ ID NO: 963 - 965; and
d) CDR H1-3 of SEQ ID NO: 980 - 982 and CDR L1-3 of SEQ ID NO: 975-977;
e) CDR H1-3 of SEQ ID NO: 992 - 994 and CDR L1-3 of SEQ ID NO: 987 - 989;
f) CDR H1-3 of SEQ ID NO: 1004 - 1006 and CDR L1-3 of SEQ ID NO: 999 - 1001;
g) CDR H1-3 of SEQ ID NO: 1028 - 1030 and CDR L1-3 of SEQ ID NO: 1023 - 1025;
h) CDR H1-3 of SEQ ID NO: 1040 - 1042 and CDR L1-3 of SEQ ID NO: 1035 -1037;
i) CDR H1-3 of SEQ ID NO: 1052 - 1054 and CDR L1-3 of SEQ ID NO: 1047 - 1049;
j) CDR H1-3 of SEQ ID NO: 1074 - 1076 and CDR L1-3 of SEQ ID NO: 1069 - 1071;
k) CDR H1-3 of SEQ ID NO: 1086 - 1088 and CDR L1-3 of SEQ ID NO: 1081 - 1083;
l) CDR H1-3 of SEQ ID NO: 1098 - 1000 and CDR L1-3 of SEQ ID NO: 1093 - 1095;
m) CDR H1-3 of SEQ ID NO: 1110 - 1112 and CDR L1-3 of SEQ ID NO: 1105 - 1107;
n) CDR H1-3 of SEQ ID NO: 1122 - 1124 and CDR L1-3 of SEQ ID NO: 1117 - 1119;
o) CDR H1-3 of SEQ ID NO: 1016 - 1018 and CDR L1-3 of SEQ ID NO: 1011 - 1013; and
p) CDR H1-3 of SEQ ID NO: 1765 - 1767 and CDR L1-3 of SEQ ID NO: 1770 - 1772.

The sequences of the corresponding VL- and VH-regions of the second binding domain of the bispecific single chain antibody molecule of the invention as well as of the respective scFvs are shown in the sequence listing.
In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH EpCAM-VL EpCAM-VH CD3-VL CD3 or VL EpCAM-VH EpCAM-VH CD3-VL CD3. More preferably, the binding domains are arranged in the order VL EpCAM-VH EpCAM-VH CD3-VL CD3.

A particularly preferred embodiment of the invention concerns an above characterized polypeptide, wherein the bispecific single chain antibody molecule comprises a sequence selected from:

(a) an amino acid sequence as depicted in any of SEQ ID NOs. 944, 948, 946, 960, 972, 984, 996, 1008, 1032, 1044, 1056, 1078, 1090, 1102, 1114, 1126, 1020, or 1776;
(b) an amino acid sequence encoded by a nucleic acid sequence as depicted in any of SEQ ID NOs: 945, 949, 947, 961, 973, 985, 979, 1009, 1033, 1045, 1057, 1079, 1091, 1103, 1115, 1127, 1021, or 1777; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 944, 948, 946, 960, 972, 984, 996, 1008, 1032, 1044, 1056, 1078, 1090, 1102, 1114, 1126, 1020, or 1776, as well as to an amino acid sequences at least 85% identical, preferably 90 %, more preferred at least 95 % identical, most preferred at least 96, 97, 98, or 99 % identical to the amino acid sequence of SEQ ID NOs: 944, 948, 946, 960, 972, 984, 996, 1008, 1032, 1044, 1056, 1078, 1090, 1102, 1114, 1126, 1020, or 1776. The invention relates also to the corresponding nucleic acid sequences as depicted in any of SEQ ID NOs: 945, 949, 947, 961, 973, 985, 979, 1009, 1033, 1045, 1057, 1079, 1091, 1103, 1115, 1127, 1021, or 1777 as well as to nucleic acid sequences at least 85% identical, preferably 90 %, more preferred at least 95 % identical, most preferred at least 96, 97, 98, or 99 % identical to the nucleic acid sequences shown in SEQ ID NOs: 945, 949, 947, 961, 973, 985, 979, 1009, 1033, 1045, 1057, 1079, 1091, 1103, 1115, 1127, 1021, or 1777. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the EpCAMxCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen EpCAM recognized by their second binding domain.

**FAPα xCD3**

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from:

CDR H1-3 of SEQ ID NO: 1137 - 1139 and CDR L1-3 of SEQ ID NO: 1132 - 1134.

The sequences of the corresponding VL- and VH-regions of the second binding domain of the bispecific single chain antibody molecule of the invention as well as of the respective scFvs are shown in the sequence listing.
According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 1137 - 1139 and CDR L1-3 of SEQ ID NO: 1132 - 1134;
b) CDR H1-3 of SEQ ID NO: 1849 - 1851and CDR L1-3 of SEQ ID NO: 1854 - 1856;
c) CDR H1-3 of SEQ ID NO: 1835 - 1837and CDR L1-3 of SEQ ID NO: 1840 - 1842; and
d) CDR H1-3 of SEQ ID NO: 1779 - 1781and CDR L1-3 of SEQ ID NO: 1784 - 1786;
e) CDR H1-3 of SEQ ID NO: 1793 - 1795 and CDR L1-3 of SEQ ID NO: 1798 - 1800;
f) CDR H1-3 of SEQ ID NO: 1863 - 1865 and CDR L1-3 of SEQ ID NO: 1868 - 1870;
g) CDR H1-3 of SEQ ID NO: 1807 - 1809 and CDR L1-3 of SEQ ID NO: 1812 - 1814;
h) CDR H1-3 of SEQ ID NO: 1821 - 1823 and CDR L1-3 of SEQ ID NO: 1826 - 1828;
i) CDR H1-3 of SEQ ID NO: 1891 - 1893 and CDR L1-3 of SEQ ID NO: 1896 - 1898;
j) CDR H1-3 of SEQ ID NO: 1877 - 1879 and CDR L1-3 of SEQ ID NO: 1882 - 1884;
k) CDR H1-3 of SEQ ID NO: 1961 - 1963 and CDR L1-3 of SEQ ID NO: 1966 - 1968;
l) CDR H1-3 of SEQ ID NO: 1947 - 1949 and CDR L1-3 of SEQ ID NO: 1952-1954;
m) CDR H1-3 of SEQ ID NO: 1975 - 1977 and CDR L1-3 of SEQ ID NO: 1980 - 1982;
n) CDR H1-3 of SEQ ID NO: 1933 - 1935 and CDR L1-3 of SEQ ID NO: 1938 - 1940;
o) CDR H1-3 of SEQ ID NO: 1919 - 1921 and CDR L1-3 of SEQ ID NO: 1924 - 1926; and
p) CDR H1-3 of SEQ ID NO: 1905 - 1907 and CDR L1-3 of SEQ ID NO: 1910 - 1912.

In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH FAP alpha -VL FAP alpha -VH CD3-VL CD3 or VL FAP alpha -VH FAP alpha -VH CD3-VL CD3. More preferred, the binding domains are arranged in the order VL FAP alpha -VH FAP alpha -VH CD3-VL CD3.

A particularly preferred embodiment of the invention concerns an above characterized polypeptide, wherein the bispecific single chain antibody molecule comprises a sequence selected from:

(a) an amino acid sequence as depicted in any of SEQ ID NOs. 1143, 1147, 1145, 1860, 1846, 1790, 1804, 1874, 1818, or 1832;
(b) an amino acid sequence encoded by a nucleic acid sequence as depicted in any of SEQ ID NOs: 1144, 1148, 1146, 1861, 1847, 1791, 1805, 1875, 1818 or 1833; and

(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 1143, 1147, 1145, 1860, 1846, 1790, 1804, 1874, 1818, or 1832, as well as to an amino acid sequences at least 85% identical, preferably 90 %, more preferred at least 95 % identical, most preferred at least 96, 97, 98, or 99 % identical to the amino acid sequence of SEQ ID NOs: 1143, 1147, 1145, 1860, 1846, 1790, 1804, 1874, 1818, or 1832. The invention relates also to the corresponding nucleic acid sequences as depicted in any of SEQ ID NOs: 1144, 1148, 1146, 1861, 1847, 1791, 1805, 1875, 1818 or 1833, as well as to nucleic acid sequences at least 85% identical, preferably 90 %, more preferred at least 95 % identical, most preferred at least 96, 97, 98, or 99 % identical to the nucleic acid sequences shown in SEQ ID NOs: 1144, 1148, 1146, 1861, 1847, 1791, 1805, 1875, 1818 or 1833. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the FAPalphaxCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen FAP alpha recognized by their second binding domain.

**IGF-1RxCD3**

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from the group consisting of:

a) CDR H1-3 of SEQ ID NO: 2016 - 2018and CDR L1-3 of SEQ ID NO: 2021 - 2023;
b) CDR H1-3 of SEQ ID NO: 2030 - 2032 and CDR L1-3 of SEQ ID NO: 2035 - 2037;
c) CDR H1-3 of SEQ ID NO: 2044 - 2046 and CDR L1-3 of SEQ ID NO: 2049 - 2051; and
d) CDR H1-3 of SEQ ID NO: 2058 - 2060 and CDR L1-3 of SEQ ID NO: 2063 - 2065;
e) CDR H1-3 of SEQ ID NO: 2072 - 2074 and CDR L1-3 of SEQ ID NO: 2077 - 2079;
f) CDR H1-3 of SEQ ID NO: 2086 - 2088 and CDR L1-3 of SEQ ID NO: 2091 - 2093;
g) CDR H1-3 of SEQ ID NO: 2100 - 2102 and CDR L1-3 of SEQ ID NO: 2105 - 2107;
h) CDR H1-3 of SEQ ID NO: 2114 - 2116 and CDR L1-3 of SEQ ID NO: 2119 - 2121;
i) CDR H1-3 of SEQ ID NO: 2128 - 2130 and CDR L1-3 of SEQ ID NO: 2133 - 2135;
j) CDR H1-3 of SEQ ID NO: 2142 - 2144 and CDR L1-3 of SEQ ID NO: 2147 - 2149:
k) CDR H1-3 of SEQ ID NO: 2156 - 2158 and CDR L1-3 of SEQ ID NO: 2161 - 2163;
l) CDR H1-3 of SEQ ID NO: 2170 - 2172 and CDR L1-3 of SEQ ID NO: 2175 - 2177;
m) CDR H1-3 of SEQ ID NO: 2184 - 2186 and CDR L1-3 of SEQ ID NO: 2189 - 2191;
n) CDR H1-3 of SEQ ID NO: 2198 - 2200 and CDR L1-3 of SEQ ID NO: 2203 - 2205; and
o) CDR H1-3 of SEQ ID NO: 2212 - 2214 and CDR L1-3 of SEQ ID NO: 2217 - 2219.

The sequences of the corresponding VL- and VH-regions of the second binding domain of the bispecific single chain antibody molecule of the invention as well as of the respective scFvs are shown in the sequence listing.
In the bispecific single chain antibody molecule of the invention the binding domains are arranged in the order VL-VH-VH-VL, VL-VH-VL-VH, VH-VL-VH-VL or VH-VL-VL-VH, as exemplified in the appended examples. Preferably, the binding domains are arranged in the order VH IGF-1 R-VL IGF-1 R-VH CD3-VL CD3 or VL IGF-1 R-VH IGF-1 R-VH CD3-VL CD3.

A particularly preferred embodiment of the invention concerns an above characterized polypeptide, wherein the bispecific single chain antibody molecule comprises a sequence selected from:

(a) an amino acid sequence as depicted in any of SEQ ID NOs: 2027, 2041, 2055, 2069, 2083, 2097, 2111, 2125, 2139, 2153, 2167, 2181, 2195, 2209, or 2223;
(b) an amino acid sequence encoded by a nucleic acid sequence as depicted in any of SEQ ID NOs: 2028, 2042, 2056, 2070, 2084, 2098, 2112, 2126, 2140, 2154, 2168, 2182, 2196, 2210, or 2224; and
(c) an amino acid sequence at least 90 % identical, more preferred at least 95 % identical, most preferred at least 96 % identical to the amino acid sequence of (a) or (b).

The invention relates to a bispecific single chain antibody molecule comprising an amino acid sequence as depicted in any of SEQ ID NOs: 2027, 2041, 2055, 2069, 2083, 2097, 2111, 2125, 2139, 2153, 2167, 2181, 2195, 2209, or 2223, as well as to an amino acid sequences at least 85% identical, preferably 90 %, more preferred at least 95 % identical, most preferred at least 96, 97, 98, or 99 % identical to the amino acid sequence of SEQ ID NOs: 2027, 2041, 2055, 2069, 2083, 2097, 2111, 2125, 2139, 2153, 2167, 2181, 2195, 2209, or 2223. The invention relates also to the corresponding nucleic acid sequences as depicted in any of SEQ ID NOs: 2028, 2042,2056,2070,2084,2098,2112,2126,2140,2154,2168,2182,2196,2210,or 2224 as well as to nucleic acid sequences at least 85% identical, preferably 90 %, more preferred at least 95 % identical, most preferred at least 96, 97, 98, or 99 % identical to the nucleic acid sequences shown in SEQ ID NOs: 2028, 2042, 2056, 2070, 2084, 2098, 2112, 2126, 2140, 2154, 2168, 2182, 2196, 2210, or 2224. It is to be understood that the sequence identity is determined over the entire nucleotide or amino acid sequence. For sequence alignments, for example, the programs Gap or BestFit can be used (Needleman and Wunsch J. Mol. Biol. 48 (1970), 443-453; Smith and Waterman, Adv. Appl. Math 2 (1981), 482-489), which is contained in the GCG software package (Genetics Computer Group, 575 Science Drive, Madison, Wisconsin, USA 53711 (1991). It is a routine method for those skilled in the art to determine and identify a nucleotide or amino acid sequence having e.g. 85% (90%, 95%, 96%, 97%, 98% or 99%) sequence identity to the nucleotide or amino acid sequences of the bispecific single single chain antibody of the invention. For example, according to Crick's Wobble hypothesis, the 5' base on the anti-codon is not as spatially confined as the other two bases, and could thus have non-standard base pairing. Put in other words: the third position in a codon triplet may vary so that two triplets which differ in this third position may encode the same amino acid residue. Said hypothesis is well known to the person skilled in the art (see e.g. http://en.wikipedia.org/wiki/Wobble_Hypothesis; Crick, J Mol Biol 19 (1966): 548-55).

Preferred domain arrangements in the IGF-1 RxCD3 bispecific single chain antibody constructs of the invention are shown in the following examples.

In a preferred embodiment of the invention, the bispecific single chain antibodies are cross-species specific for CD3 epsilon and for the human and non-chimpanzee primate cell surface antigen IGF-1 R recognized by their second binding domain.

In an alternative embodiment the present invention provides a nucleic acid sequence encoding an above described bispecific single chain antibody molecule of the invention.

The present invention also relates to a vector comprising the nucleic acid molecule of the present invention.
Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook et al. (loc cit.) and Ausubel, Current Protocols in Molecular Biology, Green Publishing Associates and Wiley Interscience, N.Y. (1989), (1994). Alternatively, the polynucleotides and vectors of the invention can be reconstituted into liposomes for delivery to target cells. As discussed in further details below, a cloning vector was used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

Preferably said vector comprises a nucleic acid sequence which is a regulatory sequence operably linked to said nucleic acid sequence defined herein.
The term "regulatory sequence" refers to DNA sequences, which are necessary to effect the expression of coding sequences to which they are ligated. The nature of such control sequences differs depending upon the host organism. In prokaryotes, control sequences generally include promoter, ribosomal binding site, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.
The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.
Thus, the recited vector is preferably an expression vector. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, e.g., the $P_L$, *lac, trp* or *tac* promoter in *E. coli,* and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells.
Beside elements, which are responsible for the initiation of transcription such regulatory elements may also comprise transcription termination signals, such as the SV40-poly-A site or the t k-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art; see also the appended Examples. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (In-vitrogene), pEF-DHFR, pEF-ADA or pEF-neo (Mack et al. PNAS (1995) 92, 7021-7025 and Raum et al. Cancer Immunol Immunother (2001) 50(3), 141-150) or pSPORT1 (GIBCO BRL).
Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the bispecific single chain antibody molecule of the invention may follow; see, e.g., the appended examples.
An alternative expression system, which can be used to express a cell cycle interacting protein is an insect system. In one such system, *Autographa californica* nuclear polyhedrosis virus (AcNPV) is used as a vector to express foreign genes in *Spodoptera frugiperda* cells or in *Trichoplusia* larvae. The coding sequence of a recited nucleic acid molecule may be cloned into a nonessential region of the virus, such as the polyhedrin gene, and placed under control of the polyhedrin promoter. Successful insertion of said coding sequence will render the polyhedrin gene inactive and produce recombinant virus lacking coat protein coat. The recombinant viruses are then used to infect S. *frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227).
Additional regulatory elements may include transcriptional as well as translational enhancers. Advantageously, the above-described vectors of the invention comprise a selectable and/or scorable marker.
Selectable marker genes useful for the selection of transformed cells and, e.g., plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).
Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or ß-glucuronidase (Jefferson, EMBO J. 6 (1987), 3901-3907). This embodiment is particularly useful for simple and rapid screening of cells, tissues and organisms containing a recited vector.
As described above, the recited nucleic acid molecule can be used alone or as part of a vector to express the bispecific single chain antibody molecule of the invention in cells, for, e.g., purification but also for gene therapy purposes. The nucleic acid molecules or vectors containing the DNA sequence(s) encoding any one of the above described bispecific single chain antibody molecule of the invention is introduced into the cells which in turn produce the polypeptide of interest. Gene therapy, which is based on introducing therapeutic genes into cells by ex-vivo or in-vivo techniques is one of the most important applications of gene transfer. Suitable vectors, methods or gene-delivery systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Verma, Nature 389 (1994), 239; Isner, Lancet 348 (1996), 370-374; Muhlhauser, Circ. Res. 77 (1995), 1077-1086; Onodera, Blood 91 (1998), 30-36; Verma, Gene Ther. 5 (1998), 692-699; Nabel, Ann. N.Y. Acad. Sci. 811 (1997), 289-292; Verzeletti, Hum. Gene Ther. 9 (1998), 2243-51; Wang, Nature Medicine 2 (1996), 714-716; WO 94/29469; WO 97/00957, US 5,580,859; US 5,589,466; or Schaper, Current Opinion in Biotechnology 7 (1996), 635-640; dos Santos Coura and Nardi Virol J. (2007), 4:99. The recited nucleic acid molecules and vectors may be designed for direct introduction or for introduction via liposomes, or viral vectors (e.g., adenoviral, retroviral) into the cell.

The invention also provides for a host cell transformed or transfected with a vector of the invention. Said host cell may be produced by introducing the above described vector of the invention or the above described nucleic acid molecule of the invention into the host. The presence of at least one vector or at least one nucleic acid molecule in the host may mediate the expression of a gene encoding the above described single chain antibody constructs.

The described nucleic acid molecule or vector of the invention, which is introduced in the host may either integrate into the genome of the host or it may be maintained extrachromosomally.

The host cell can be any prokaryote or eukaryotic cell.

The term "prokaryote" is meant to include all bacteria, which can be transformed or transfected with DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. Especially preferred is the use of a plasmid or a virus containing the coding sequence of the bispecific single chain antibody molecule of the invention and genetically fused thereto an N-terminal FLAG-tag and/or C-terminal His-tag. Preferably, the length of said FLAG-tag is about 4 to 8 amino acids, most preferably 8 amino acids. An above described polynucleotide can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, loc cit.). Preferably, said the host is a bacterium or an insect, fungal, plant or animal cell.

It is particularly envisaged that the recited host may be a mammalian cell. Particularly preferred host cells comprise CHO cells, COS cells, myeloma cell lines like SP2/0 or NS/0. As illustrated in the appended examples, particularly preferred are CHO-cells as hosts.

More preferably said host cell is a human cell or human cell line, e.g. per.c6 (Kroos, Biotechnol. Prog., 2003, 19:163-168).

In a further embodiment, the present invention thus relates to a process for the production of a bispecific single chain antibody molecule of the invention, said process comprising culturing a host cell of the invention under conditions allowing the expression of the bispecific single chain antibody molecule of the invention and recovering the produced polypeptide from the culture.

The transformed hosts can be grown in fermentors and cultured according to techniques known in the art to achieve optimal cell growth. The bispecific single chain antibody molecule of the invention can then be isolated from the growth medium, cellular lysates, or cellular membrane fractions. The isolation and purification of the, e.g., microbially expressed bispecific single chain antibody molecules may be by any conventional means such as, for example, preparative chromatographic separations and immunological separations such as those involving the use of monoclonal or polyclonal antibodies directed, e.g., against a tag of the bispecific single chain antibody molecule of the invention or as described in the appended examples.

The conditions for the culturing of a host, which allow the expression are known in the art to depend on the host system and the expression system/vector used in such process. The parameters to be modified in order to achieve conditions allowing the expression of a recombinant polypeptide are known in the art. Thus, suitable conditions can be determined by the person skilled in the art in the absence of further inventive input.

Once expressed, the bispecific single chain antibody molecule of the invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure polypeptides of at least about 90 to 95% homogeneity are preferred, and 98 to 99% or more homogeneity are most preferred, for pharmaceutical uses. Once purified, partially or to homogeneity as desired, the bispecific single chain antibody molecule of the invention may then be used therapeutically (including extracorporeally) or in developing and performing assay procedures. Furthermore, examples for methods for the recovery of the bispecific single chain antibody molecule of the invention from a culture are described in detail in the appended examples. The recovery can also be achieved by a method for the isolation of the bispecific single chain antibody molecule of the invention binding to an epitope of human and non-chimpanzee primate CD3 epsilon as defined herein (CD3ε, the method comprising the steps of:

(a) contacting the polypeptide(s) with an N-terminal fragment of the extracellular domain of CD3ε of maximal 27 amino acids comprising the amino acid sequence Gln-Asp-Gly-Asn-Glu-Glu-Met-Gly (SEQ ID NO. 341) or Gln-Asp-Gly-Asn-Glu-Glu-Ile-Gly (SEQ ID NO. 342), fixed via its C-terminus to a solid phase;
(b) eluting the bound polypeptide(s) from said fragment; and
(c) isolating the polypeptide(s) from the eluate of (b).

It is preferred that the polypeptide(s) isolated by the above method of the invention are human.
This method or the isolation of the bispecific single chain antibody molecule of the invention is understood as a method for the isolation of one or more different polypeptides with the same specificity for the fragment of the extracellular domain of CD3ε comprising at its N-terminus the amino acid sequence Gln-Asp-Gly-Asn-Glu-Glu-Met-Gly (SEQ ID NO. 341) or Gln-Asp-Gly-Asn-Glu-Glu-Ile-Gly (SEQ ID NO. 342) from a plurality of polypeptide candidates as well as a method for the purification of a polypeptide from a solution. A non-limiting example for the latter method for the purification of a bispecific single chain antibody molecule from a solution is e.g. the purification of a recombinantly expressed bispecific single chain antibody molecule from a culture supernatant or a preparation from such culture.
As stated above the fragment used in this method is an N-terminal fragment of the extracellular domain of the primate CD3ε molecule. The amino acid sequence of the extracellular domain of the CD3ε molecule of different species is depicted in SEQ ID NOs: 1, 3, 5 and 7. The two forms of the N-terminal octamer are depicted in SEQ ID NOs: 341 and 342. It is preferred that this N-terminus is freely available for binding of the polypeptides to be identified by the method of the invention. The term "freely available" is understood in the context of the invention as free of additional motives such as a His-tag. The interference of such a His-tag with a binding molecule identified by the method of the invention is described in the appended Examples 6 and 20.
According to this method said fragment is fixed via its C-terminus to a solid phase. The person skilled in the art will easily and without any inventive ado elect a suitable solid phase support dependent from the used embodiment of the method of the invention. Examples for a solid support comprise but are not limited to matrices like beads (e.g. agarose beads, sepharose beads, polystyrol beads, dextran beads), plates (culture plates or MultiWell plates) as well as chips known e.g. from Biacore®. The selection of the means and methods for the fixation/immobilization of the fragment to said solid support depend on the election of the solid support. A commonly used method for the fixation/immobilization is a coupling via an N-hydroxysuccinimide (NHS) ester. The chemistry underlying this coupling as well as alternative methods for the fixation/immobilization are known to the person skilled in the art, e.g. from Hermanson "Bioconjugate Techniques", Academic Press, Inc. (1996). For the fixation to/immobilization on chromatographic supports the following means are commonly used: NHS-activated sepharose (e.g. HiTrap-NHS of GE Life Science-Amersham), CnBr-activated sepharose (e.g. GE Life Science-Amersham), NHS-activated dextran beads (Sigma) or activated polymethacrylate. These reagents may also be used in a batch approach. Moreover, dextran beads comprising iron oxide (e.g. available from Miltenyi) may be used in a batch approach. These beads may be used in combination with a magnet for the separation of the beads from a solution. Polypeptides can be immobilized on a Biacore chip (e.g. CM5 chips) by the use of NHS activated carboxymethyldextran. Further examples for an appropriate solid support are amine reactive MultiWell plates (e.g. Nunc Immobilizer™ plates).
According to this method said fragment of the extracellular domain of CD3 epsilon can be directly coupled to the solid support or via a stretch of amino acids, which might be a linker or another protein/polypeptide moiety. Alternatively, the extracellular domain of CD3 epsilon can be indirectly coupled via one or more adaptor molecule(s).

Means and methods for the eluation of a peptide or polypeptide bound to an immobilized epitope are well known in the art. The same holds true for methods for the isolation of the identified polypeptide(s) from the eluate.
A method for the isolation of one or more different bispecific single chain antibody molecule(s) with the same specificity for the fragment of the extracellular domain of CD3ε comprising at its N-terminus the amino acid sequence Gln-Asp-Gly-Asn-Glu-Glu-X-Gly (with X being Met or Ile) from a plurality of polypeptide candidates may comprise one or more steps of the following methods for the selection of antigen-specific entities:

CD3ε specific binding domains can be selected from antibody derived repertoires. A phage display library can be constructed based on standard procedures, as for example disclosed in "Phage Display: A Laboratory Manual"; Ed. Barbas, Burton, Scott & Silverman; Cold Spring Harbor Laboratory Press, 2001. The format of the antibody fragments in the antibody library can be scFv, but may generally also be a Fab fragment or even a single domain antibody fragment. For the isolation of antibody fragments naïve antibody fragment libraries may be used. For the selection of potentially low immunogenic binding entities in later therapeutic use, human antibody fragment libraries may be favourable for the direct selection of human antibody fragments. In some cases they may form the basis for synthetic antibody libraries (Knappik et al. J Mol. Biol. 2000, 296:57 ff). The corresponding format may be Fab, scFv (as described below) or domain antibodies (dAbs, as reviewed in Holt et al., Trends Biotechnol. 2003, 21:484 ff).

It is also known in the art that in many cases there is no immune human antibody source available against the target antigen. Therefore animals are immunized with the target antigen and the respective antibody libraries isolated from animal tissue as e.g. spleen or PBMCs. The N-terminal fragment may be biotinylated or covalently linked to proteins like KLH or bovine serum albumin (BSA). According to common approaches rodents are used for immunization. Some immune antibody repertoires of non-human origin may be especially favourable for other reasons, e.g. for the presence of single domain antibodies (VHH) derived from cameloid species (as described in Muyldermans, J Biotechnol. 74:277; De Genst et al. Dev Como Immunol. 2006, 30:187 ff). Therefore a corresponding format of the antibody library may be Fab, scFv (as described below) or single domain antibodies (VHH).

In one possible approach ten weeks old F1 mice from balb/c x C57black crossings can be immunized with whole cells e.g. expressing transmembrane EpCAM N-terminally displaying as translational fusion the N-terminal amino acids 1 to 27 of the mature CD3ε chain. Alternatively, mice can be immunized with 1-27 CD3 epsilon-Fc fusion protein (a corresponding approach is described in the appended Example 2). After booster immunization(s), blood samples can be taken and antibody serum titer against the CD3-positive T cells can be tested e.g. in FACS analysis. Usually, serum titers are significantly higher in immunized than in non-immunized animals.

Immunized animals may form the basis for the construction of immune antibody libraries. Examples of such libraries comprise phage display libraries. Such libraries may be generally constructed based on standard procedures, as for example disclosed in "Phage Display: A Laboratory Manual"; Ed. Barbas, Burton, Scott & Silverman; Cold Spring Harbor Laboratory Press, 2001.

The non-human antibodies can also be humanized via phage display due to the generation of more variable antibody libraries that can be subsequently enriched for binders during selection.

In a phage display approach any one of the pools of phages that displays the antibody libraries forms a basis to select binding entities using the respective antigen as target molecule. The central step in which antigen specific, antigen bound phages are isolated is designated as panning. Due to the display of the antibody fragments on the surface of the phages, this general method is called phage display. One preferred method of selection is the use of small proteins such as the filamentous phage N2 domain translationally fused to the N-terminus of the scFv displayed by the phage. Another display method known in the art, which may be used to isolate binding entities is the ribosome display method (reviewed in Groves & Osbourn, Expert Opin Biol Ther. 2005, 5:125 ff; Lipovsek & Pluckthun, J Immunol Methods 2004, 290:52 ff).

In order to demonstrate binding of scFv phage particles to a 1-27 CD3ε-Fc fusion protein a phage library carrying the cloned scFv-repertoire can be harvested from the respective culture supernatant by PEG (polyethyleneglycole). ScFv phage particles may be incubated with immobilized CD3ε Fc fusion protein. The immobilized CD3ε Fc fusion protein may be coated to a solid phase. Binding entities can be eluted and the eluate can be used for infection of fresh uninfected bacterial hosts. Bacterial hosts successfully transduced with a phagemid copy, encoding a human scFv-fragment, can be selected again for carbenicillin resistance and subsequently infected with e.g. VCMS 13 helper phage to start the second round of antibody display and in vitro selection. A total of 4 to 5 rounds of selections is carried out, normally. The binding of isolated binding entities can be tested on CD3 epsilon positive Jurkat cells, HPBall cells, PBMCs or transfected eukaryotic cells that carry the N-terminal CD3ε sequence fused to surface displayed EpCAM using a flow cytometric assay (see appended Example 4).

Preferably, the above method may be a method, wherein the fragment of the extracellular domain of CD3ε consists of one or more fragments of a polypeptide having an amino acid sequence of any one depicted in SEQ ID NOs. 2, 4, 6 or 8. More preferably, said fragment is 8, 9, 10, 11, 12, 13, 14, 15, 16, 17, 18, 19, 20, 21, 22, 23, 24, 25, 26, or 27 amino acid residues in length.

This method of identification of a bispecific single chain antibody molecule may be a method of screening a plurality of bispecific single chain antibody molecules comprising a cross-species specific binding domain binding to an epitope of human and non-chimpanzee primate CD3ε. Alternatively, the method of identification is a method of purification/isolation of a bispecific single chain antibody molecule comprising a cross-species specific binding domain binding to an epitope of human and non-chimpanzee primate CD3ε.

Furthermore, the invention provides for a composition comprising a bispecific single chain antibody molecule of the invention or a bispecific single chain antibody as produced by the process disclosed above. Preferably, said composition is a pharmaceutical composition.

The invention provides also for a bispecific single chain antibody molecule as defined herein, or produced according to the process as defined herein, wherein said bispecific single chain antibody molecule is for use in the prevention, treatment or amelioration of cancer or autoimmune diseases.

Preferably for a PSCAxCD3 bispecific single chain antibody molecule, said cancer is prostate cancer, bladder cancer or pancreatic cancer.

For a CD19xCD3 bispecific single chain antibody molecule it is preferred that said cancer is a B-cell malignancy, such as B-NHL (B cell non-Hodgkin Lymphoma), B-ALL (acute lymphoblastic B cell leukemia), B-CLL (chronic lymphocytic B cell leukemia), or Multiple Myeloma (wherein the bispecific single chain antibody molecule of the invention advantageously depletes CD19-positive cancer stem cells/cancer initiating cells). However, the bispecific single chain antibody molecule is also for use in the prevention, treatment or amelioration of B-cell mediated autoimmune diseases or autoimmune diseases with a pathogenic B cell contribution such as rheumatoid arthritis or the depletion of B-cells.

For a c-METxCD3 bispecific single chain antibody molecule it is preferred that said cancer is a carcinoma, sarcoma, glioblastoma/astrocytoma, melanoma, mesothelioma, Wilms tumor or a hematopoietic malignancy such as leukemia, lymphoma or multiple myeloma. A comprehensive list of various cancer types which may be treated with the bispecific single chain antibody can be found e.g. in http://www.val.org/met/.
For an EndosialinxCD3 bispecific single chain antibody molecule it is preferred that said cancer includes, but is not limited to, carcinomas (breast, kidney, lung, colorectal, colon, pancreas mesothelioma), sarcomas, and neuroectodermal tumors (melanoma, glioma, neuroblastoma).
For an EpCAMxCD3 bispecific single chain antibody molecule it is preferred that said cancer is epithelial cancer or a minimal residual cancer.
For a FAP$\alpha\times$CD3 bispecific single chain antibody molecule it is preferred that said cancer is epithelial cancer.
For a IGF-1 RxCD3 bispecific single chain antibody molecule it is preferred that said cancer is bone or soft tissue cancer (e.g. Ewing sarcoma), breast, liver, lung, head and neck, colorectal, prostate, leiomyosarcoma, cervical and endometrial cancer, ovarian, prostate, and pancreatic cancer. Alternatively, the IGF-1 RxCD3 bispecific single chain antibody molecule of the invention is also used in the prevention, treatment or amelioration of autoimmune diseases, preferably psoriasis.
It is preferred that the bispecific single chain is further comprising suitable formulations of carriers, stabilizers and/or excipients. Moreover, it is preferred that said bispecific single chain antibody molecule is suitable to be administered in combination with an additional drug. Said drug may be a non-proteinaceous compound or a proteinaceous compound and may be administered simultaneously or non-simultaneously with the bispecific single chain antibody molecule as defined herein.

In accordance with the invention, the term "pharmaceutical composition" relates to a composition for administration to a patient, preferably a human patient. The particular preferred pharmaceutical composition of this invention comprises bispecific single chain antibodies directed against and generated against context-independent CD3 epitopes. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient. The pharmaceutical composition comprising the bispecific single chain antibodies directed against and generated against context-independent CD3 epitopes of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.
The continuous or uninterrupted administration of these bispecific single chain antibodies directed against and generated against context-independent CD3 epitopes of this invention may be intravenuous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.
The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The composition of the present invention, comprising in particular bispecific single chain antibodies directed against and generated against context-independent CD3 epitopes may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate. The compositions of the present invention can be administered to the subject at a suitable dose which can be determined e.g. by dose escalating studies by administration of increasing doses of the bispecific single chain antibody molecule of the invention exhibiting cross-species specificity described herein to non-chimpanzee primates, for instance macaques. As set forth above, the bispecific single chain antibody molecule of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the bispecific single chain antibody molecule of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the bispecific single chain antibody molecule of the invention defined herein, further biologically active agents, depending on the intended use of the composition. Such agents might be drugs acting on the gastro-intestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1 - 12). "Efficacy" or "in vivo efficacy" as used herein refers to the response to therapy by the pharmaceutical composition of the invention, using e.g. standardized NCI response criteria. The success or *in vivo* efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various cancer specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that effect the ability of a particular drug to treat a given condition, is established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above. "Half-life" means the time where 50% of an administered drug are eliminated through biological processes, e.g. metabolism, excretion, etc.
By "hepatic first-pass metabolism" is meant the propensity of a drug to be metabolized upon first contact with the liver, i.e. during its first pass through the liver. "Volume of distribution" means the degree of retention of a drug throughout the various compartments of the body, like e.g. intracellular and extracellular spaces, tissues and organs, etc. and the distribution of the drug within these compartments.
"Degree of blood serum binding" means the propensity of a drug to interact with and bind to blood serum proteins, such as albumin, leading to a reduction or loss of biological activity of the drug.
Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma.
"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetik parameters of bispecific single chain antibodies exhibiting cross-species specificity, which may be determined in preclinical animal testing in non-chimpanzee primates as outlined above are also set forth e.g. in the publication by Schlereth et al. (Cancer Immunol. Immunother. 20 (2005), 1 - 12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", "in vivo safety" or "tolerability" as used herein defines the administration of a drug without inducing severe adverse events directly after administration (local tolerance) and during a longer period of application of the drug.

"Safety", "in vivo safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviating to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance haematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective and non-toxic dose" as used herein refers to a tolerable dose of the bispecific single chain antibody as defined herein which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

Moreover, the invention relates to a pharmaceutical composition comprising a bispecific single chain antibody molecule of this invention or produced according to the process according to the invention for the prevention, treatment or amelioration of cancer or autoimmune diseases.

Preferably, said cancer is for a PSCAxCD3 bispecific single chain antibody molecule prostate cancer, bladder cancer or pancreatic cancer.
Preferably, said cancer is for a CD19xCD3 bispecific single chain antibody molecule a B-cell malignancy such as non-Hodgkin Lymphoma, B-cell mediated autoimmune diseases or the depletion of B-cells.
Preferably, said cancer is for a c-METxCD3 bispecific single chain antibody molecule is a carcinoma, sarcoma, glioblastoma/astrocytoma, melanoma, mesothelioma, Wilms tumor or a hematopoietic malignancy such as leukemia, lymphoma or multiple myeloma.
Preferably, said cancer is for an EndosialinxCD3 bispecific single chain antibody molecule a carcinoma (breast, kidney, lung, colorectal, colon, pancreas mesothelioma), a sarcoma, or a neuroectodermal tumor (melanoma, glioma, neuroblastoma).
Preferably, said cancer is for an EpCAMxCD3 bispecific single chain antibody molecule epithelial cancer or a minimal residual cancer.
Preferably, said cancer is for a FAP$\alpha\times$CD3 bispecific single chain antibody molecule epithelial cancer.

Preferably, said cancer is for a an IGF-1 RxCD3 bispecific single chain antibody molecule bone or soft tissue cancer (e.g. Ewing sarcoma), breast, liver, lung, head and neck, colorectal, prostate, leiomyosarcoma, cervical and endometrial cancer, ovarian, prostate, and pancreatic cancer. Alternatively, the IGF-1 RxCD3 bispecific single chain antibody molecule/polypeptide as defined herein above is also used in the prevention, treatment or amelioration of autoimmune diseases, preferably psoriasis.
Preferably, said pharmaceutical composition further comprises suitable formulations of carriers, stabilizers and/or excipients.

A further aspect of the disclosure relates to a use of a bispecific single chain antibody molecule/polypeptide as defined herein above or produced according to a process defined herein above, for the preparation of a pharmaceutical composition for the prevention, treatment or amelioration of a disease. Preferably, said disease is cancer or autoimmune diseases.

More preferably for a PSCAxCD3 bispecific single chain antibody molecule/polypeptide as defined herein above, said cancer is prostate cancer, bladder cancer or pancreatic cancer.
More preferably for a CD19xCD3 bispecific single chain antibody molecule/polypeptide as defined herein above, said cancer is a B-cell malignancy such as non-Hodgkin Lymphoma, B-cell mediated autoimmune diseases or the depletion of B-cells.
More preferably for a c-METxCD3 bispecific single chain antibody molecule/polypeptide as defined herein above, said cancer is a carcinoma, sarcoma, glioblastoma/astrocytoma, melanoma, mesothelioma, Wilms tumor or a hematopoietic malignancy such as leukemia, lymphoma or multiple myeloma.
More preferably for an EndosialinxCD3 bispecific single chain antibody molecule/polypeptide as defined herein above, said cancer is a carcinoma (breast, kidney, lung, colorectal, colon, pancreas mesothelioma), a sarcoma, or a neuroectodermal tumor (melanoma, glioma, neuroblastoma).
More preferably for an EpCAMxCD3 bispecific single chain antibody molecule/polypeptide as defined herein above, said cancer is epithelial cancer or a minimal residual cancer.
More preferably for a FAP$\alpha\times$CD3 bispecific single chain antibody molecule/polypeptide as defined herein above, said cancer is epithelial cancer.
More Preferably for an IGF-1RxCD3 bispecific single chain antibody molecule/polypeptide as defined herein above, said cancer is bone or soft tissue cancer (e.g. Ewing sarcoma), breast, liver, lung, head and neck, colorectal, prostate, leiomyosarcoma, cervical and endometrial cancer, ovarian, prostate, and pancreatic cancer. Alternatively, the IGF-1RxCD3 bispecific single chain antibody molecule/polypeptide as defined herein above is also used in the prevention, treatment or amelioration of autoimmune diseases, preferably psoriasis.

In another preferred embodiment of use of the bispecific single chain antibody molecule of the invention said pharmaceutical composition is suitable to be administered in combination with an additional drug, i.e. as part of a co-therapy. In said co-therapy, an active agent may be optionally included in the same pharmaceutical composition as the bispecific single chain antibody molecule of the invention, or may be included in a separate pharmaceutical composition. In this latter case, said separate pharmaceutical composition is suitable for administration prior to, simultaneously as or following administration of said pharmaceutical composition comprising the bispecific single chain antibody molecule of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.
Preferably, said proteinaceous compound or non-proteinaceous compound may be administered simultaneously or non-simultaneously with the bispecific single chain antibody molecule of the invention, a nucleic acid molecule as defined hereinabove, a vector as defined as defined hereinabove, or a host as defined as defined hereinabove.

Another aspect of the disclosure relates to an effective amount of a pharmaceutical composition of the invention for use in a method for the prevention, treatment or amelioration of a disease in a subject in the need thereof. Preferably, said disease is cancer or autoimmune diseases.
More preferably for a PSCAxCD3 bispecific single chain antibody molecule/polypeptide as defined herein above" said cancer is prostate cancer, bladder cancer or pancreatic cancer.
More preferably for a CD19xCD3 bispecific single chain antibody molecule/polypeptide as defined herein above, said cancer is a B-cell malignancy such as non-Hodgkin Lymphoma, B-cell mediated autoimmune diseases or the depletion of B-cells.
More preferably for a c-METxCD3 bispecific bispecific single chain antibody molecule/polypeptide as defined herein above, said cancer is a carcinoma, sarcoma, glioblastoma/astrocytoma, melanoma, mesothelioma, Wilms tumor or a hematopoietic malignancy such as leukemia, lymphoma or multiple myeloma.
More preferably for an EndosialinxCD3 bispecific bispecific single chain antibody molecule/polypeptide as defined herein above, said cancer is a carcinoma (breast, kidney, lung, colorectal, colon, pancreas mesothelioma), a sarcoma, or a neuroectodermal tumor (melanoma, glioma, neuroblastoma).

More preferably for an EpCAMxCD3 bispecific single chain antibody molecule/polypeptide as defined herein above, said cancer is epithelial cancer or a minimal residual cancer.
More preferably for a FAPαxCD3 bispecific single chain antibody molecule/polypeptide as defined herein above, said cancer is epithelial cancer
More preferably for an IGF-1RxCD3 bispecific single chain antibody molecule/polypeptide as defined herein above, said cancer is bone or soft tissue cancer (e.g. Ewing sarcoma), breast, liver, lung, head and neck, colorectal, prostate, leiomyosarcoma, cervical and endometrial cancer, ovarian, prostate, and pancreatic cancer. Alternatively, the IGF-1RxCD3 bispecific single chain antibody molecule/polypeptide as defined herein above is also used in the prevention, treatment or amelioration of autoimmune diseases, preferably psoriasis.

In another preferred embodiment of the pharmaceutical composition for the use as described herein, said pharmaceutical composition is suitable to be administered in combination with an additional drug, i.e. as part of a co-therapy. In said co-therapy, an active agent may be optionally included in the same pharmaceutical composition as the bispecific single chain antibody molecule of the invention, or may be included in a separate pharmaceutical composition. In this latter case, said separate pharmaceutical composition is suitable for administration prior to, simultaneously as or following administration of said pharmaceutical composition comprising the bispecific single chain antibody molecule of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.
Preferably, said proteinaceous compound or non-proteinaceous compound may be administered simultaneously or non-simultaneously with the bispecific single chain antibody molecule of the invention, a nucleic acid molecule as defined hereinabove, a vector as defined as defined hereinabove, or a host as defined as defined hereinabove.

It is preferred for the above described pharmaceutical composition for the use as described herein that said subject is a human.

In a further aspect, the invention relates to a kit comprising a bispecific single chain antibody molecule of the invention, a nucleic acid molecule of the invention, a vector of the invention, or a host cell of the invention.
These and other embodiments are disclosed and encompassed by the description and Examples of the present disclosure.
Recombinant techniques and methods in immunology are described e.g. in Sambrook et al. Molecular Cloning: A Laboratory Manual; Cold Spring Harbor Laboratory Press, 3rd edition 2001; Lefkovits; Immunology Methods Manual; The Comprehensive Sourcebook of Techniques; Academic Press, 1997; Golemis; Protein-Protein Interactions: A Molecular Cloning Manual; Cold Spring Laboratory Press, 2002. Further literature concerning any one of the antibodies, methods, uses and compounds to be employed in accordance with the present invention may be retrieved from public libraries and databases, using for example electronic devices. For example, the public database "Medline", available on **the Internet, may be utilized, for example under** http://www.ncbi.nlm.nih.gov/PubMed/medline.html. Further databases and addresses such as http://www.ncbi.nlm.nih.gov/ or listed at the EMBL-services homepage under http://www.embl.de/services/index.html are known to the person skilled in the art and can also be obtained using, e. g., http://www.google.com.
The figures show:

**Figure 1** Fusion of the N-terminal amino acids 1-27 of primate CD3 epsilon to a heterologous soluble protein.

**Figure 2**
The figure shows the average absorption values of quadruplicate samples measured in an ELISA assay detecting the presence of a construct consisting of the N-terminal amino acids 1-27 of the mature human CD3 epsilon chain fused to the hinge and Fc gamma portion of human IgG1 and a C-terminal 6 Histidine tag in a supernatant of transiently transfected 293 cells. The first column labeled "27 aa huCD3E" shows the average absorption value for the construct, the second column labeled "irrel. SN" shows the average value for a supernatant of 293 cells transfected with an irrelevant construct as negative control. The comparison of the values obtained for the construct with the values obtained for the negative control clearly demonstrates the presence of the recombinant construct.

**Figure 3** The figure shows the average absorption values of quadruplicate samples measured in an ELISA assay detecting the binding of the cross species specific anti-CD3 binding molecules in form of crude preparations of periplasmatically expressed single-chain antibodies to a construct comprising the N-terminal 1-27 amino acids of the mature human CD3 epsilon chain fused to the hinge and Fc gamma portion of human IgG1 and a C-terminal His6 tag. The columns show from left to right the average absorption values for the specificities designated as A2J HLP, I2C HLP E2M HLP, F7O HLP, G4H HLP, H2C HLP, E1 L HLP, F12Q HLP, F6A HLP and H1 E HLP. The rightmost column labelled "neg. contr." shows the average absorption value for the single-chain preparation of a murine anti-human CD3 antibody as negative control. The comparison of the values obtained for the anti-CD3 specificities with the values obtained for the negative control clearly demonstrates the strong binding of the anti-CD3 specificities to the N-terminal 1-27 amino acids of the mature human CD3 epsilon chain.

**Figure 4** Fusion of the N-terminal amino acids 1-27 of primate CD3 epsilon to a heterologous membrane bound protein.

**Figure 5** Histogram overlays of different transfectants tested in a FACS assay detecting the presence of recombinant transmembrane fusion proteins consisting of cynomolgus EpCAM and the N-terminal 1-27 amino acids of the human, marmoset, tamarin, squirrel monkey and domestic swine CD3 epsilon chain respectively. The histogram overlays from left to right and top to bottom show the results for the transfectants expressing the constructs comprising the human 27 mer, marmoset 27 mer, tamarin 27 mer, squirrel monkey 27 mer and swine 27 mer respectively. In the individual overlays the thin line represents a sample incubated with PBS with 2% FCS instead of anti-Flag M2 antibody as negative control and the bold line shows a sample incubated with the anti-Flag M2 antibody. For each construct the overlay of the histograms shows binding of the anti-Flag M2 antibody to the transfectants, which clearly demonstrates the expression of the recombinant constructs on the transfectants.

**Figure 6** Histogram overlays of different transfectants tested in a FACS assay detecting the binding of the cross-species specific anti-CD3 binding molecules in form of crude preparations of periplasmatically expressed single-chain antibodies to the N-terminal amino acids 1-27 of the human, marmoset, tamarin and squirrel monkey CD3 epsilon chain respectively fused to cynomolgus EpCAM.

**Figure 6A:**

The histogram overlays from left to right and top to bottom show the results for the transfectants expressing the 1-27 CD3-EpCAM comprising the human 27 mer tested with the CD3 specific binding molecules designated H2C HLP, F12Q HLP, E2M HLP and G4H HLP respectively.

**Figure 6B:** The histogram overlays from left to right and top to bottom show the results for the transfectants expressing the 1-27 CD3-EpCAM comprising the marmoset 27 mer tested with the CD3 specific binding molecules designated H2C HLP, F12Q HLP, E2M HLP and G4H HLP respectively.

**Figure 6C:** The histogram overlays from left to right and top to bottom show the results for the transfectants expressing the 1-27 CD3-EpCAM comprising the tamarin 27 mer tested with the CD3 specific binding molecules designated H2C HLP, F12Q HLP, E2M HLP and G4H HLP respectively.

**Figure 6D:** The histogram overlays from left to right and top to bottom show the results for the transfectants expressing the 1-27 CD3-EpCAM comprising the squirrel monkey 27 mer tested with the CD3 specific binding molecules designated H2C HLP, F12Q HLP, E2M HLP and G4H HLP respectively.

**Figure 6E:**

The histogram overlays from left to right and top to bottom show the results for the transfectants expressing the 1-27 CD3-EpCAM comprising the swine 27 mer tested with the CD3 specific binding molecules designated H2C HLP, F12Q HLP, E2M HLP and G4H HLP respectively.

In the individual overlays the thin line represents a sample incubated with a single-chain preparation of a murine anti-human CD3-antibody as negative control and the bold line shows a sample incubated with the respective anti-CD3 binding molecules indicated. Considering the lack of binding to the swine 27 mer transfectants and the expression levels of the constructs shown in Figure 5 the overlays of the histograms show specific and strong binding of the tested anti-CD3 specificities of the fully cross-species specific human bispecific single chain antibodies to cells expressing the recombinant transmembrane fusion proteins comprising the N-terminal amino acids 1-27 of the human, marmoset, tamarin and squirrel monkey CD3 epsilon chain respectively fused to cynomolgus EpCAM and show therefore multi primate cross-species specificity of the anti-CD3 binding molecules.

**Figure 7** FACS assay for detection of human CD3 epsilon on transfected murine EL4 T cells. Graphical analysis shows an overlay of histograms. The bold line shows transfected cells incubated with the anti-human CD3 antibody UCHT-1. The thin line represents cells incubated with a mouse IgG1 isotype control. Binding of the anti CD3 antibody UCHT1 clearly shows expression of the human CD3 epsilon chain on the cell surface of transfected murine EL4 T cells.

**Figure 8**

Binding of cross-species specific anti CD3 antibodies to alanine-mutants in an alanine scanning experiment. In the individual Figures the columns show from left to right the calculated binding values in arbitrary units in logarithmic scale for the wild-type transfectant (WT) and for all alanine-mutants from the position 1 to 27. The binding values are calculated using the following formula:

$$value_Sample(x,y) = \frac{Sample(x,y) - neg_Contr.(x)}{(UCHT-1(x) - neg_Contr.(x)) * \frac{WT(y) - neg_Contr.(wt)}{UCHT-1(wt) - neg_Contr.(wt)}}$$

In this equation *value_Sample* means the value in arbitrary units of binding depicting the degree of binding of a specific anti-CD3 antibody to a specific alanine-mutant as shown in the Figure, *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT-1* means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, x specifies the respective transfectant, *y* specifies the respective anti-CD3 antibody and *wt* specifies that the respective transfectant is the wild-type. Individual alanine-mutant positions are labelled with the single letter code of the wild-type amino acid and the number of the position.

**Figure 8A:**

The figure shows the results for cross-species specific anti CD3 antibody A2J HLP expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8B:** The figure shows the results for cross-species specific anti CD3 antibody E2M HLP, expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8C:** The figure shows the results for cross-species specific anti CD3 antibody H2C HLP, expressed as chimeric IgG molecule. Reduced binding activity is observed for mutations to alanine at position 4 (asparagine). Complete loss of binding is observed for mutations to alanine glutamine at position 1 (glutamine), at position 2 (aspartate), at position 3 (glycine) and at position 5 (glutamate).

**Figure 8D:** shows the results for cross-species specific anti CD3 antibody F12Q HLP, tested as periplasmatically expressed single-chain antibody. Complete loss of binding is observed for mutations to alanine at position 1 (glutamine), at position 2 (aspartate), at position 3 (glycine) and at position 5 (glutamate).

**Figure 9**

FACS assay detecting the binding of the cross-species specific anti-CD3 binding molecule H2C HLP to human CD3 with and without N-terminal His6 tag.

Histogram overlays are performed of the EL4 cell line transfected with wild-type human CD3 epsilon chain (left histogram) or the human CD3 epsilon chain with N-terminal His6 tag (right histogram) tested in a FACS assay detecting the binding of cross-species specific binding molecule H2C HLP. Samples are incubated with an appropriate isotype control as negative control (thin line), anti-human CD3 antibody UCHT-1 as positive control (dotted line) and cross-species specific anti-CD3 antibody H2C HLP in form of a chimeric IgG molecule (bold line).

Histogram overlays show comparable binding of the UCHT-1 antibody to both transfectants as compared to the isotype control demonstrating expression of both recombinant constructs. Histogram overlays also show binding of the anti-CD3 binding molecule H2C HLP only to the wild-type human CD3 epsilon chain but not to the His6-human CD3 epsilon chain. These results demonstrate that a free N-terminus is essential for binding of the cross-species specific anti-CD3 binding molecule H2C HLP.

**Figure 10** FACS binding analysis of designated cross-species specific bispecific single chain constructs to CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 10. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 11** FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 10. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 12** FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 10. The thick line represents cells incubated with 2 μg/ml purified monomeric protein that are subsequently incubated with the anti-his antibody and the PE labeled detection antibody. The thin histogram line reflects the negative control: cells only incubated with the anti-his antibody and the detection antibody.

**Figure 13**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 11.

**Figure 14**

Cytotoxicity activity induced by designated cross-species specific MCSP specific single chain constructs redirected to indicated target cell lines. A) and B) The macaque T cell line 4119 LnPx are used as effector cells, CHO cells transfected with cynomolgus MCSP D3 as target cells. The assay is performed as described in Example 11.

**Figure 15**

Cytotoxicity activity induced by designated cross-species specific MCSP specific single chain constructs redirected to indicated target cell lines. A) and B) Stimulated CD4-/CD56- human PBMCs are used as effector cells, CHO cells transfected with human MCSP D3 as target cells. The assay is performed as described in Example 11.

**Figure 16**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 11.

**Figure 17**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 11.

**Figure 18**

Plasma stability of MCSP and CD3 cross-species specific bispecific single chain antibodies tested by the measurement of cytotoxicity activity induced by samples of the designated single chain constructs incubated with 50% human plasma at 37°C and 4°C for 24 hours respectively or with addition of 50% human plasma immediately prior to cytotoxicity testing or without addition of plasma. CHO cells transfected with human MCSP are used as target cell line and stimulated CD4-/CD56- human PBMCs are used as effector cells. The assay is performed as described in Example 12.

**Figure 19** Initial drop and recovery (i.e. redistribution) of absolute T cell counts (open squares), in peripheral blood of B-NHL patients (patent numbers 1, 7, 23, 30, 31, and 33 of Table 4), who had essentially no circulating CD19-positive target B cells (filled triangles), during the starting phase of intravenous infusion with the CD3 binding molecule CD19xCD3 recognizing a conventional context dependent CD3 epitope. Absolute cell counts are given in 1000 cells per microliter blood. The first data point shows baseline counts immediately prior to the start of infusion. The CD19xCD3 dose is given in parentheses beside the patient number.

**Figure 20**

**(A)** Repeated T cell redistribution (open squares) in B-NHL patient #19 (Table 4) who had no circulating CD19-positive target B cells (filled triangles) and developed CNS symptoms under continuous intravenous infusion with CD19xCD3 at a starting dose of 5$\mu$g/m$^2$/24h for one day followed by a sudden dose increase to 15$\mu$g/m$^2$/24h. Absolute cell counts are given in 1000 cells per microliter blood. The first data point shows baseline counts immediately prior to the start of infusion. After recovery of circulating T cells from the first episode of redistribution triggered by the treatment start at 5$\mu$g/m$^2$/24h the stepwise dose increase from 5 to 15$\mu$g/m$^2$/24h triggered a second episode of T cell redistribution that was associated with the development of CNS symptoms dominated by confusion and disorientation.

**(B)** Repeated T cell redistribution in a B-NHL patient, who developed CNS symptoms under repeated intravenous bolus infusion with CD19xCD3 at 1.5$\mu$g/m$^2$. Absolute cell counts are given in 1000 cells per microliter blood. The infusion time for each bolus administration was 2 to 4 hours. Vertical arrows indicate the start of bolus infusions. Data points at the beginning of each bolus administration show the T cell counts immediately prior to start of bolus infusion. Each bolus infusion triggered an episode of T cell redistribution followed by recovery of the T cell counts prior to the next bolus infusion. Finally the third episode of T cell redistribution was associated with the development of CNS symptoms in this patient.

**Figure 21**

Complex T cell redistribution pattern (open squares) in B-NHL patient #20 (Table 4) without circulating CD19-positive target B cells (filled triangles), during ramp initiation of the CD19xCD3 infusion i.e. even gradual increase of flow-rate from almost zero to 15$\mu$g/m$^2$/24h during the first 24 hours of treatment. Absolute cell counts are given in 1000 cells per microliter blood. The first data point shows baseline counts immediately prior to the start of infusion. The CD19xCD3 dose is given in parentheses beside the patient number. T cells reappearing in the circulating blood after the initial redistribution triggered by the first exposure to CD19xCD3 are partially induced to redisappear from circulating blood again by still increasing levels of CD19xCD3 during the ramp phase.

**Figure 22**

T and B cell counts during treatment with CD19xCD3 of B-NHL patient #13 (Table 4) who had a significant number of circulating CD19-positive target B (lymphoma) cells (filled triangles). Absolute cell counts are given in 1000 cells per microliter blood. The first data point shows baseline counts immediately prior to the start of infusion. The CD19xCD3 dose is given in parentheses beside the patient number. T cells (open squares) disappear completely from the circulation upon start of CD19xCD3 infusion and do not reappear until the circulating CD19-positive B (lymphoma) cells (filled triangles) are depleted from the peripheral blood.

**Figure 23**

Repeated T cell redistribution (open squares) in B-NHL patient #24 (Table 4), who had essentially no circulating CD19-positive target B cells (filled triangles) and developed CNS symptoms upon initiation of CD19xCD3 infusion without additional HSA as required for stabilisation of the drug (upper panel). After first recovery of circulating T cells from initial redistribution the uneven drug flow due to the lack of stabilizing HSA triggered a second episode of T cell redistribution that was associated with the development of CNS symptoms dominated by confusion and disorientation. When the same patient was restarted correctly with CD19xCD3 solution containing additional HSA for drug stabilisation, no repeated T cell redistribution was observed (lower panel) and the patient did not again develop any CNS symptoms. Absolute cell counts are given in 1000 cells per microliter blood. The first data point shows baseline counts immediately prior to the start of infusion. The CD19xCD3 dose is given in parentheses beside the patient number.

**Figure 24**

Model of T cell adhesion to endothelial cells induced by monovalent binding to context dependent CD3 epitopes. Monovalent interaction of a conventional CD3 binding molecule to its context dependent epitope on CD3 epsilon can lead to an allosteric change in the conformation of CD3 followed by the recruitment of Nck2 to the cytoplasmic domain of CD3 epsilon (Gil et al. (2002) Cell 109: 901). As Nck2 is directly linked to integrins via PINCH and ILK (Legate et al. (2006) Nat Rev Mol Cell Biol 7: 20), recruitment of Nck2 to the cytoplasmic domain of CD3 epsilon following an allosteric change in the conformation of CD3 through binding of a conventional CD3 binding molecule (like the CD19xCD3 of example 13) to its context dependent epitope on CD3 epsilon, can increase the adhesiveness of T cells to endothelial cells by transiently switching integrins on the T cell surface into their more adhesive isoform via inside-out-signalling.

**Figure 25**

Cytotoxic activity of CD33-AF5 VH-VL x I2C VH-VL test material used for the in vivo study in cynomolgus monkeys as described in Example 14. Specific lysis of CD33-positive target cells was determined in a standard $^{51}$Chromium release assay at increasing concentrations of CD33-AF5 VH-VL x I2C VH-VL. Assay duration was 18 hours. The macaque T cell line 4119 LnPx was used as source of effector cells. CHO cells transfected with cynomolgus CD33 served as target cells. Effector- to target cell ratio (E:T-ratio) was 10:1. The concentration of CD33-AF5 VH-VL x I2C VH-VL required for half-maximal target cell lysis (EC50) was calculated from the dose response curve with a value of 2.7 ng/ml.

**Figure 26**

**(A)** Dose- and time-dependent depletion of CD33-positive monocytes from the peripheral blood of cynomolgus monkeys through intravenous continuous infusion of CD33-AF5 VH-VL x I2C VH-VL as described in Example 14. The percentage relative to baseline (i.e. 100%) of absolute circulating CD33-positive monocyte counts after the duration of treatment as indicated above the columns is shown for each of two cynomolgus monkeys per dose level. The dose level (i.e. infusion flow-rate) is indicated below the columns. No depletion of circulating CD33-positive monocytes was observed in animals 1 and 2 treated for 7 days at a dose of 30 $\mu g/m^2/24h$. In animals 3 and 4 treated for 7 days at a dose of 60 $\mu g/m^2/24h$ circulating CD33-positive monocyte counts were reduced to 68% and 40% of baseline, respectively. At 240 $\mu g/m^2/24h$ circulating CD33-positive monocytes were almost completely depleted from the peripheral blood after 3 days of treatment (animals 5 and 6). At 1000 $\mu g/m^2/24h$ depletion of circulating CD33-positive monocytes from the peripheral blood was completed already after 1 day of treatment (animals 7 and 8).

**(B)** Course of T cell and CD33-monocyte counts in peripheral blood of two cynomolgus monkeys during continuous infusion of CD33-AF5 VH-VL x I2C VH-VL for 14 days at 120 $\mu g/m^2/24h$. Absolute cell counts are given in 1000 cells per microliter blood. The first data point shows baseline counts immediately prior to the start of infusion. After initial mobilisation of CD33-monocytes during the first 12 hours upon start of infusion CD33-monocytes in peripheral blood (filled triangles) are depleted by two thirds (animal 10) and 50% (animal 9) relative to the respective baseline counts during the further course of infusion. Circulating T cell counts (open squares) show a limited initial drop followed by recovery still during the presence of circulating CD33-positive monocytic target cells.

**Figure 27**

Cytotoxic activity of MCSP-G4 VH-VL x I2C VH-VL test material used for the in vivo study in cynomolgus monkeys as described in Example 15. Specific lysis of MCSP-positive target cells was determined in a standard $^{51}$Chromium release assay at increasing concentrations of MCSP-G4 VH-VL x I2C VH-VL. Assay duration was 18 hours. The macaque T cell line 4119 LnPx was used as source of effector cells. CHO cells transfected with cynomolgus MCSP served as target cells. Effector- to target cell ratio (E:T-ratio) was 10:1. The concentration of MCSP-G4 VH-VL x I2C VH-VL required for half-maximal target cell lysis (EC50) was calculated from the dose response curve with a value of 1.9 ng/ml.

**Figure 28**

Absence of initial episodes of drop and subsequent recovery of absolute T cell counts (i.e. redistribution) in peripheral blood of cynomolgus monkeys during the starting phase of intravenous infusion with the CD3 binding molecule MCSP-G4 VH-VL x I2C VH-VL recognizing an essentially context independent CD3 epitope. Absolute cell counts are given in 1000 cells per microliter blood. The first data point shows baseline counts immediately prior to the start of infusion. The MCSP-G4 VH-VL x I2C VH-VL dose is given in parentheses beside the animal number. In the known absence of MCSP-positive target cells from the circulating blood of cynomolgus monkeys there is no induction of T cell redistribution (i.e. an initial episode of drop and subsequent recovery of absolute T cell counts) through target cell mediated crosslinking of CD3. Moreover, induction of T cell redistribution (i.e. an initial episode of drop and subsequent recovery of absolute T cell counts) through a signal, which the T cells may receive through exclusive interaction with a CD3 binding site only, can be avoided by the use of CD3 binding molecules like MCSP-G4 VH-VL x I2C VH-VL recognizing an essentially context independent CD3 epitope.

**Figure 29**

FACS binding analysis of designated cross-species specific bispecific constructs to CHO cells transfected with human CD33, the human CD3+ T cell line HPB-ALL, CHO cells transfected with macaque CD33 and macaque PBMC respectively. The FACS staining is performed as described in Example 16.4. The bold lines represent cells incubated with 5 μg/ml purified bispecific single chain construct or cell culture supernatant of transfected cells expressing the cross-species specific bispecific antibody constructs. The filled histograms reflect the negative controls. Supernatant of untransfected CHO cells was used as negative control. For each cross-species specific bispecific single chain construct the overlay of the histograms shows specific binding of the construct to human and macaque CD33 and human and macaque CD3.

**Figure 30**

The diagrams show results of chromium release assays measuring cytotoxic activity induced by designated cross-species specific CD33 specific single chain constructs redirected to the indicated target cell lines. Effector cells were also used as indicated. The assays are performed as described in Example 16.5. The diagrams clearly demonstrate for each construct the potent recruitment of cytotoxic activity of human and macaque effector cells against human and macaque CD33 transfected CHO cells, respectively.

**Figure 31**

SDS PAGE gel and Western blot monitoring the purification of the cross-species specific bispecific single chain molecule designated E292F3 HL x I2C HL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 17.2. The Western blot detecting the histidine$_6$ tag confirms the identity of the protein band in the eluate as the cross-species specific bispecific single chain molecule. The faint signal for the flow through sample in this sensitive detection method further shows the nearly complete capture of bispecific single chain molecules by the purification method.

**Figure 32**

SDS PAGE gel and Western blot monitoring the purification of the cross-species specific bispecific single chain molecule designated V207C12 HL x H2C HL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 17.2. The Western blot detecting the histidine$_6$ tag confirms the identity of the protein band in the eluate as the cross-species specific bispecific single chain molecule. The faint signal for the flow through sample in this sensitive detection method further shows the nearly complete capture of bispecific single chain molecules by the purification method.

**Figure 33**

SDS PAGE gel and Western blot monitoring the purification of the cross-species specific bispecific single chain molecule designated AFSHLxF12QHL. Samples from the eluate, the cell culture supernatant (SN) and the flow through of the column (FT) were analyzed as indicated. A protein marker (M) was applied as size reference. A strong protein band with a molecular weight between 50 and 60 kDa in the SDS PAGE gel demonstrates the efficient purification of the cross-species specific bispecific single chain molecule to a very high degree of purity with the one-step purification method described in Example 17.2. The Western blot detecting the $histidine_6$ tag confirms the identity of the protein band in the eluate as the cross-species specific bispecific single chain molecule. The signal in the flow through sample in this sensitive detection method is explained by saturation of the affinity column due to the high concentration of bispecific single chain molecules in the supernatant.

**Figure 34**

Standard curve of AF5HLxI2CHL in 50% macaque monkey serum. The upper diagram shows the standard curve generated for the assay as described in Example 18.2.

The lower diagram shows results for quality control samples of AF5HLxI2CHL in 50% macaque monkey serum. The recovery rates are above 90% for the high and mid QC sample and above 80% for the low QC sample.

Thus the assay allows for detection of AF5HLxI2CHL in serum samples in the range from 10 ng/ml to 200 ng/ml (before dilution).

**Figure 35**

Standard curve of MCSP-G4 HL x I2C HL in 50% macaque monkey serum. The upper diagram shows the standard curve generated for the assay as described in Example 18.2.

The lower diagram shows results for quality control samples of MCSP-G4 HL x I2C HL in 50% macaque monkey serum. The recovery rates are above 98% for the high and mid QC sample and above 85% for the low QC sample.

Thus the assay allows for detection of MCSP-G4 HL x I2C HL in serum samples in the range from 10 ng/ml to 200 ng/ml (before dilution).

**Figure 36**

FACS binding analysis of an anti-Flag antibody to CHO cells transfected with the 1-27 N-terminal amino acids of CD3 epsilon of the designated species fused to cynomolgus EpCAM. The FACS staining was performed as described in Example 19.1. The bold lines represent cells incubated with the anti-Flag antibody. The filled histograms reflect the negative controls. PBS with 2 % FCS was used as negative control. The histograms show strong and comparable binding of the anti-Flag antibody to all transfectants indicating strong and equal expression of the transfected constructs.

**Figure 37**

FACS binding analysis of the I2C IgG1 construct to CHO cells expressing the 1-27 N-terminal amino acids of CD3 epsilon of the designated species fused to cynomolgus EpCAM. The FACS staining is performed as described in Example 19.3. The bold lines represent cells incubated with 50 μl cell culture supernatant of cells expressing the I2C IgG1 construct. The filled histograms reflect the negative control. Cells expressing the 1-27 N-terminal amino acids of CD3 epsilon of swine fused to cynomolgus EpCAM were used as negative control. In comparison with the negative control the histograms clearly demonstrate binding of the I2C IgG1 construct to 1-27 N-terminal amino acids of CD3 epsilon of human, marmoset, tamarin and squirrel monkey.

**Figure 38**

FACS binding analysis of the I2C IgG1 construct as described in Example 19.2 to human CD3 with and without N-terminal His6 tag as described in Examples 6.1 and 5.1 respectively. The bold lines represent cells incubated with the anti-human CD3 antibody UCHT-1, the penta-His antibody (Qiagen) and cell culture supernatant of cells expressing the I2C IgG1 construct respectively as indicated. The filled histograms reflect cells incubated with an irrelevant murine IgG1 antibody as negative control.

The upper two histogram overlays show comparable binding of the UCHT-1 antibody to both transfectants as compared to the isotype control demonstrating expression of both recombinant constructs. The centre histogram overlays show binding of the penta his antibody to the cells expressing the His6-human CD3 epsilon chain (His6-CD3) but not to the cells expressing the wild-type CD3 epsilon chain (WT-CD3). The lower Histogram overlays show binding of the I2C IgG1 construct to the wild-type human CD3 epsilon chain but not to the His6-human CD3 epsilon chain. These results demonstrate that a free N-terminus is essential for binding of the cross-species specific anti-CD3 binding molecule I2C to the CD3 epsilon chain.

**Figure 39**

FACS binding analysis of designated cross-species specific bispecific single chain constructs to CHO cells transfected with human MCSP D3, the human CD3+ T cell line HPB-ALL, CHO cells transfected with macaque MCSP D3 and the macaque T cell line 4119 LnPx respectively. The FACS staining was performed as described in Example 10. The bold lines represents cells incubated with 2 μg/ml purified bispecific single chain construct or cell supernatant containing the bispecific single chain construct respectively. The filled histograms reflect the negative controls. Supernatant of untransfected CHO cells was used as negative control for binding to the T cell lines. A single chain construct with irrelevant target specificity was used as negative control for binding to the MCSP D3 transfected CHO cells. For each cross-species specific bispecific single chain construct the overlay of the histograms shows specific binding of the construct to human and macaque MCSP D3 and human and macaque CD3.

**Figure 40**

Cytotoxic activity induced by designated cross-species specific MCSP D3 specific single chain constructs redirected to the indicated target cell lines. Effector cells and effector to target ratio were also used as indicated. The assay is performed as described in Example 11. The diagrams clearly demonstrate potent cross-species specific recruitment of cytotoxic activity by each construct.

**Figure 41**

FACS binding analysis of designated cross-species specific bispecific single chain constructs to CHO cells transfected with human CD33, the human CD3+ T cell line HPB-ALL, CHO cells transfected with macaque CD33 and macaque PBMC respectively. The FACS staining was performed as described in Example 21.2. The bold lines represent cells incubated with cell culture supernatant of transfected cells expressing the cross-species specific bispecific antibody constructs. The filled histograms reflect the negative controls. Supernatant of untransfected CHO cells was used as negative control. For each cross-species specific bispecific single chain construct the overlay of the histograms shows specific binding of the construct to human and macaque CD33 and human and macaque CD3.

**Figure 42**

The diagrams show results of chromium release assays measuring cytotoxic activity induced by designated cross-species specific CD33 specific single chain constructs redirected to the indicated target cell lines. Effector cells were also used as indicated. The assays are performed as described in Example 21.3. The diagrams clearly demonstrate for each construct the potent recruitment of cytotoxic activity of human and macaque effector cells against human and macaque CD33 transfected CHO cells, respectively.

**Figure 43**

T cell redistribution in a chimpanzee under weekly intravenous bolus infusion with PBS/5% HSA and PBS/5% HSA plus single-chain EpCAM/CD3-bispecific antibody construct at doses of 1.6, 2.0, 3.0 and 4.5 $\mu$g/kg. The infusion time for each bolus administration was 2 hours. Vertical arrows indicate the start of bolus infusions. Data points at the beginning of each bolus administration show the T cell counts immediately prior to start of bolus infusion. Each bolus infusion of the single-chain EpCAM/CD3-bispecific antibody construct, which recognizes a conventional context dependent CD3 epitope, triggered an episode of T cell redistribution followed by recovery of T cells to baseline values prior to the next bolus infusion.

**Figure 44**

CD3 specific ELISA analysis of periplasmic preparations containing Flag tagged scFv protein fragments from selected clones. Periplasmic preparations of soluble scFv protein fragments were added to wells of an ELISA plate, which had been coated with soluble human CD3 epsilon (aa 1-27)- Fc fusion protein and had been additionally blocked with PBS 3% BSA. Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an ELISA reader. Clone names are presented on the x axis.

**Figure 45**

ELISA analysis of periplasmic preparations containing Flag tagged scFv protein fragments from selected clones. The same periplasmic preparations of soluble scFv protein fragments as in Figure 44 were added to wells of an ELISA plate which had not been coated with human CD3 epsilon (aa 1-27)- Fc fusion protein but with huIgG1 (Sigma) and blocked with 3% BSA in PBS.

Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an ELISA reader. Clone names are presented on the x axis.

**Figure 46**

Figures 46A-G: FACS binding analysis of designated cross-species specific bispecific single chain constructs to CHO cells transfected with human PSCA, the human CD3+ T cell line HPB-ALL, CHO cells transfected with macaque PSCA and to the macaque T cell line 4119LnPx, respectively. The FACS staining was performed as described in Example 24.5. The bold lines represent cells incubated with cell culture supernatant of transfected cells expressing the cross-species specific bispecific antibody constructs. The filled histograms reflect the negative controls. Supernatant of untransfected cells was used as a negative control. For each cross-species specific bispecific single chain construct the overlay of the histograms shows specific binding of the construct to human and macaque PSCA and human and macaque CD3.

**Figure 47**

Figure 47A-C: The diagrams show results of chromium release assays measuring cytotoxic activity induced by the designated cross-species specific bispecific single chain constructs redirected to the indicated target cell lines. Effector cells were also used as indicated. The assays were performed as described in Example 24.6. The diagrams clearly demonstrate for each construct the potent recruitment of cytotoxic activity of human and macaque effector cells against cells positive for human and macaque PSCA, respectively.

**Figure 48**

FACS binding analysis of designated cross-species specific bispecific single chain constructs to CHO cells transfected with human PSCA, the human CD3+ T cell line HPB-ALL, CHO cells transfected with macaque PSCA and to the macaque T cell line 4119LnPx, respectively. The FACS staining was performed as described in Example 45.5. The bold lines represent cells incubated with cell culture supernatant of transfected cells expressing the cross-species specific bispecific antibody constructs. The filled histograms show the negative controls. Supernatant of untransfected cells was used as negative control. For each cross-species specific bispecific single chain construct the overlay of the histograms shows specific binding of the construct to human and macaque PSCA and human and macaque CD3.

**Figure 49**

The diagrams show results of chromium release assays measuring cytotoxic activity induced by designated cross-species specific bispecific single chain constructs redirected to the indicated target cell lines. Effector cells were also used as indicated. The assays were performed as described in Example 24.6. The diagrams clearly demonstrate for each construct the potent recruitment of cytotoxic activity of human and macaque effector T cells against target cells positive for human and macaque PSCA, respectively.

**Figure 50**

FACS binding analysis of designated cross-species specific bispecific single chain constructs to CHO cells transfected with human CD19, the human CD3-positive T cell line HPB-ALL, and the CD3-positive macaque T cell line 4119 LnPx. The FACS staining is performed as described in Example 25.4. The thick-line histograms represent cells incubated with cell culture supernatant and subsequently with a murine anti-His-tag antibody followed by a PE-labeled anti-murine Ig detection antibody. The thin-lined histograms represent the negative control i.e. cells only incubated with the anti-His-tag antibody and the anti-murine Ig detection antibody.

**Figure 51**

Cytotoxic T cell activity redirected by designated cross-species specific bispecific single chain constructs against the indicated target cell line. A) Stimulated CD4/CD56-depleted human PBMCs are used as effector T cells and CHO cells transfected with human CD19 as target cells. B) The macaque T cell line 4119 LnPx is used as source of effector cells and CHO cells transfected with human CD19 are used as target cells. The assay is performed as described in Example 25.5.

**Figure 52**

FACS binding analysis of the designated cross-species specific bispecific single chain constructs to the C-MET positive human breast cancer cell line MDA-MB-231, the human CD3+ T cell line HPB-ALL and to the macaque CD3+ T cell line 4119LnPx respectively. The FACS staining was performed as described in Example 26.5. The bold lines represent cells incubated with cell culture supernatant of transfected cells expressing the cross-species specific bispecific antibody constructs. The filled histograms represent the negative controls. Supernatant of untransfected cells was used as a negative control. For each cross-species specific bispecific single chain construct the overlay of the histograms shows specific binding of the construct to C-MET and human and macaque CD3.

**Figure 53**

The diagrams show results of chromium release assays measuring cytotoxic activity induced by the designated cross-species specific bispecific single chain constructs redirected to the indicated target cell lines. Effector cells were also used as indicated. The assays were performed as described in Example 26.6. The diagrams clearly demonstrate for each construct the potent recruitment of cytotoxic activity of human effector cells against cells positive for C-MET.

**Figure 54**

FACS binding analysis of designated cross-species specific bispecific single chain constructs to CHO cells expressing human cMET as described in Example 27.1, the human CD3+ T cell line HPB-ALL, CHO cells expressing macaque cMET as described in Example 27.1 and the macaque T cell line 4119LnPx, respectively. The FACS staining was performed as described in Example 27.2. The bold lines represent cells incubated with cell culture supernatant of transfected cells expressing the cross-species specific bispecific antibody constructs. The filled histograms show the negative controls. Supernatant of untransfected CHO cells was used as negative control. For each cross-species specific bispecific single chain construct the overlay of the histograms shows specific binding of the construct to human and macaque cMET and human and macaque CD3.

**Figure 55**

The diagrams show results of chromium release assays measuring cytotoxic activity induced by designated cross-species specific cMET specific single chain constructs redirected to the indicated target cell line generated as described in Example 27.1. Effector cells were also used as indicated. The assays were performed as described in Example 27.4. The diagrams clearly demonstrate for each construct the potent recruitment of cytotoxic activity of macaque effector T cells against target cells positive for macaque cMET.

**Figure 56**

FACS binding analysis of designated cross-species specific scFv antibodies to CHO cells expressing human cMET as described in Example 27.1 and CHO cells expressing macaque cMET as described in Example 27.1, respectively. The FACS staining was performed as described in Example 27.3. The bold lines represent cells incubated with periplasmic preparations containing the cross-species specific scFv antibodies. The filled histograms show the negative controls. The Buffer used for periplasmic preparations was used as negative control. For each cross-species specific scFv antibody the overlay of the histograms shows specific binding of the construct to human and macaque cMET.

**Figure 57**

FACS binding analysis of a cross-species specific scFv-antibody fragment to CHO cells transfected with human Endosialin and to CHO cells transfected with macaque Endosialin. The FACS staining was performed as described in Example 28.3. The bold lines represent cells incubated with a periplasmic preparation containing the scFv-antibody fragment. The thin lines represent the negative controls. Untransfected CHO cells were used as a negative control. The overlays of the histograms show specific binding of the scFv-antibody fragment to human and macaque Endosialin.

**Figure 58**

FACS binding analysis of designated cross-species specific bispecific single chain constructs to CHO cells transfected with human CD248, the human CD3+ T cell line HPB-ALL, CHO cells transfected with macaque CD248 and the macaque T cell line 4119LnPx, respectively. The FACS staining was performed as described in Example 29.1. The bold lines represent cells incubated with cell culture supernatant of transfected cells expressing the cross-species specific bispecific antibody constructs. The thin lines show the negative controls. Supernatant of untransfected CHO cells was used as negative control. For each cross-species specific bispecific single chain construct the overlay of the histograms shows specific binding of the construct to human and macaque CD248 and human and macaque CD3.

**Figure 59**

The diagrams show results of chromium release assays measuring cytotoxic activity induced by designated cross-species specific CD248 specific single chain constructs redirected to the indicated target cell lines. Effector cells were also used as indicated. The assays were performed as described in Example 29.1. The diagrams clearly demonstrate for each construct the potent recruitment of cytotoxic activity of human and macaque effector T cells against target cells positive for human and macaque CD248, respectively.

**Figure 60**

FACS binding analysis of designated cross-species specific bispecific single chain constructs to CHO cells transfected with human EpCAM, human CD3+ T cell line HPB-ALL, and the macaque T cell line 4119 LnPx. The FACS staining was performed as described in Example 30.4. The thick line represents cells incubated with cell culture supernatant that were subsequently incubated with the anti-his antibody and the PE labeled detection antibody. The thin histogram line shows the negative control: cells only incubated with the anti-his antibody and the detection antibody.

**Figure 61**

Cytotoxic activity induced by designated cross-species specific bispecific single chain constructs redirected to the indicated target cell lines. A) Stimulated CD4-/CD56-human PBMCs are used as effector cells, CHO cells transfected with human EpCAM as target cells. B) The macaque T cell line 4119 LnPx were used as effector cells, CHO cells transfected with human EpCAM as target cells. The assay was performed as described in Example 30.5.

**Figure 62**

Cytotoxic activity induced by designated cross-species specific bispecific single chain constructs redirected to the indicated target cell lines. A) Stimulated CD4-/CD56-human PBMCs are used as effector cells, CHO cells transfected with human EpCAM as target cells. B) The macaque T cell line 4119 LnPx were used as effector cells, CHO cells transfected with human EpCAM as target cells. The assay was performed as described in Example 30.5.

**Figure 63**

Cytotoxic activity induced by designated cross-species specific bispecific single chain constructs redirected to the indicated target cell lines. A) and B) Stimulated CD4-/CD56- human PBMCs are used as effector cells, CHO cells transfected with human EpCAM as target cells. The assay was performed as described in Example 30.5.

**Figure 64 and 65**

Cytotoxic activity induced by designated cross-species specific bispecific single chain constructs redirected to the indicated target cell lines. A) and B) The macaque T cell line 4119 LnPx were used as effector cells, CHO cells transfected with human EpCAM as target cells. The assay was performed as described in Example 30.5

**Figure 66**

Cytotoxic activity induced by designated cross-species specific bispecific single chain constructs redirected to the indicated target cell lines. A) Stimulated CD4-/CD56-human PBMCs are used as effector cells, CHO cells transfected with human EpCAM as target cells. B) The macaque T cell line 4119 LnPx were used as effector cells, CHO cells transfected with human EpCAM as target cells. The assay was performed as described in Example 30.5

**Figure 67**

FACS binding analysis of designated cross-species specific bispecific single chain constructs to CHO cells transfected with human, mouse or human-mouse hybrid EpCAM. The FACS staining was performed as described in Example 30.7. The bars represent the median fluorescence intensity of the designated constructs to the stated EpCAM antigens.

**Figure 68**

In the FACS analysis, the indicated constructs showed binding to CD3 and FAPalpha compared to the negative control. Cross-species specificity of the bispecific antibodies to human and macaque CD3 and FAP alpha antigens, respectively, was demonstrated. The assay was performed as described in Example 31

**Figure 69**

All of the generated cross-species specific bispecific single chain antibody constructs demonstrated cytotoxic activity against human FAPalpha positive target cells elicited by stimulated human CD4/CD56 depleted PBMC and macaque FAPalpha positive target cells elicited by the macaque T cell line 4119LnPx. The assay was performed as described in Example 31

**Figure 70**

FACS binding analysis of designated cross-species specific bispecific single chain constructs to CHO cells transfected with human FAPalpha, the human CD3+ T cell line HPB-ALL, CHO cells transfected with macaque FAPalpha and the macaque T cell line 4119LnPx, respectively. The FACS staining was performed as described in Example 32.1. The bold lines represent cells incubated with cell culture supernatant of transfected cells expressing the cross-species specific bispecific antibody constructs. The thin lines show the negative controls. Supernatant of untransfected CHO cells was used as negative control. For each cross-species specific bispecific single chain construct the overlay of the histograms shows specific binding of the construct to human and macaque FAPalpha and human and macaque CD3.

**Figure 71**

The diagrams show results of chromium release assays measuring cytotoxic activity induced by designated cross-species specific FAPalpha specific single chain constructs redirected to the indicated target cell lines. Effector cells were also used as indicated. The assays were performed as described in Example 32.1. The diagrams clearly demonstrate for each construct the potent recruitment of cytotoxic activity of human and macaque effector T cells against target cells positive for human and macaque FAPalpha, respectively.

**Figure 72**

FACS binding analysis of designated cross-species specific scFv antibodies to CHO cells transfected with human FAPalpha and CHO cells transfected with macaque FAPalpha, respectively. The FACS staining was performed as described in Example 32.2. The bold lines represent cells incubated with periplasmic preparations containing the cross-species specific scFv antibodies. The filled histograms show the negative controls. The Buffer used for periplasmic preparations was used as negative control. For each cross-species specific scFv antibody the overlay of the histograms shows specific binding of the construct to human and macaque FAPalpha.

**Figure 73**

FACS binding analysis of designated cross-species specific bispecific single chain constructs to CHO cells transfected with human IGF-1 R, the human CD3+ T cell line HPB-ALL, CHO cells transfected with macaque IGF-1 R and to the macaque T cell line 4119LnPx, respectively. The FACS staining was performed as described in Example 33.3. The bold lines represent cells incubated with cell culture supernatant of transfected cells expressing the cross-species specific bispecific antibody constructs. The filled histograms show the negative controls. Cell culture medium was used as negative control. For each cross-species specific bispecific single chain construct the overlay of the histograms shows specific binding of the construct to human and macaque IGF-1 R and human and macaque CD3.

**Figure 74**

The diagrams show results of chromium release assays measuring cytotoxic activity induced by designated cross-species specific bispecific single chain constructs redirected to the indicated target cell lines. Effector cells were also used as indicated. The assays were performed as described in Example 33.3. The diagrams clearly demonstrate for each construct the recruitment of cytotoxic activity of human and macaque effector T cells against target cells positive for human and macaque IGF-1 R, respectively.

The present invention is additionally described by way of the following illustrative non-limiting examples that provide a better understanding of the present invention and of its many advantages.

## EXAMPLES

### 1. Identification of CD3epsilon sequences from blood samples of non-human primates

Blood samples of the following non-human primates were used for CD3epsilon-identification: *Callithrix jacchus, Saguinus oedipus* and *Saimiris ciureus.* Fresh heparin-treated whole blood samples were prepared for isolating total cellular RNA according to manufacturer's protocol (QIAamp RNA Blood Mini Kit, Qiagen). The extracted mRNA was transcribed into cDNA according to published protocols. In brief, 10 μl of precipitated RNA was incubated with 1.2 μl of 10x hexanucleotide mix (Roche) at 70 °C for 10 minutes and stored on ice. A reaction mix consisting of 4 μl of 5x superscript II buffer, 0.2 μl of 0.1 M dithiothreitole, 0.8 μl of superscript II (Invitrogen), 1.2 μl of desoxyribonucleoside triphosphates (25 μM), 0.8 μl of RNase Inhibitor (Roche) and 1.8 μl of DNase and RNase free water (Roth) was added. The reaction mix was incubated at room temperature for 10 minutes followed by incubation at 42 °C for 50 minutes and at 90 °C for 5 minutes. The reaction was cooled on ice before adding 0.8 μl of RNaseH (1 U/μl, Roche) and incubated for 20 minutes at 37 °C.
The first-strand cDNAs from each species were subjected to separate 35-cycle polymerase chain reactions using Taq DNA polymerase (Sigma) and the following primer combination designed on database research: forward primer 5'-AGAGTTCTGGGCCTCTGC-3' (SEQ ID NO: 253); reverse primer 5'-CGGATGGGCTCATAGTCTG-3' (SEQ ID NO: 254);. The amplified 550 bp-bands were gel purified (Gel Extraction Kit, Qiagen) and sequenced (Sequiserve, Vaterstetten/Germany, see sequence listing).

CD3epsilon *Callithrix jacchus* Nucleotides

```
CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTCGTAAATA
GTCAAAACAAAGAAGGTCATGAGGACCACCTGTTACTGGAGGACTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT
```

Amino acids (SEQ ID NO: 3)

```
QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD
```

CD3epsilon *Saguinus oedipus* Nucleotides

```
CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTTGTAAATA
GTCAAAACAAAGAAGGTCATGAGGACCACCTGTTACTGGAGGATTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT
```

Amino acids (SEQ ID NO: 5)

```
QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD
```

CD3epsilon *Saimiris ciureus* Nucleotides

CAGGACGGTAATGAAGAGATTGGTGATACTACCCAGAACCCATATAAAGTTTCCATCTCAGG
AACCACAGTAACACTGACATGCCCTCGGTATGATGGACAGGAAATAAAATGGCTCGTAAATG
ATCAAAACAAAGAAGGTCATGAGGACCACCTGTTACTGGAAGATTTTTCAGAAATGGAACAA
AGTGGTTATTATGCCTGCCTCTCCAAAGAGACCCCCACAGAAGAGGCGAGCCATTATCTCTA
CCTGAAGGCAAGAGTGTGTGAGAACTGCGTGGAGGTGGAT

Amino acids (SEQ ID NO: 7)

QDGNEEIGDTTQNPYKVSISGTTVTLTCPRYDGQEIKWLVNDQNKEGHEDHLLLEDFSEMEQ
SGYYACLSKETPTEEASHYLYLKARVCENCVEVD

**2. Generation of cross-species specific single chain antibody fragments (scFv) binding to the N-terminal amino acids 1-27 of CD3epsilon of man and different non-chimpanzee primates**

**2.1. Immunization of mice using the N-terminus of CD3epsilon separated from its native CD3-context by fusion to a heterologous soluble protein**

Ten weeks old F1 mice from balb/c x C57black crossings were immunized with the CD3epsilon-Fc fusion protein carrying themost N-terminal amino acids 1-27 of the mature CD3epsilon chain (1-27 CD3-Fc) of man and/or saimiris ciureus. To this end 40 $\mu$g of the 1-27 CD3-Fc fusion protein with 10 nmol of a thioate-modified CpG-Oligonucleotide (5'-tccatgacgttcctgatgct-3') (SEQ ID No. 343) in 300 ul PBS were injected per mouse intra-peritoneally. Mice receive booster immunizations after 21, 42 and optionally 63 days in the same way. Ten days after the first booster immunization, blood samples were taken and antibody serum titer against 1-27 CD3-Fc fusion protein iwa tested by ELISA. Additionally, the titer against the CD3-positive human T cell line HPBall was tested in flow cytometry according to standard protocols. Serum titers were significantly higher in immunized than in non-immunized animals.

**2.2. Generation of an immune murine antibody scFv library: Construction of a combinatorial antibody library and phage display**

Three days after the last injection the murine spleen cells were harvested for the preparation of total RNA according to standard protocols.

A library of murine immunoglobuline (Ig) light chain (kappa) variable region (VK) and Ig heavy chain variable region (VH) DNA-fragments was constructed by RT-PCR on murine spleen RNA using VK-and VH specific primer. cDNA was synthesized according to standard protocols.
The primers were designed in a way to give rise to a 5'-Xhol and a 3'-BstEII recognition site for the amplified heavy chain V-fragments and to a 5'-Sacl and a 3'-Spel recognition site for amplified VK DNA fragments.
For the PCR-amplification of the VH DNA-fragments eight different 5'-VH-family specific primers (MVH1 (GC)AG GTG CAG CTC GAG GAG TCA GGA CCT (SEQ ID No. 344); MVH2 GAG GTC CAG CTC GAG CAG TCT GGA CCT (SEQ ID No. 345); MVH3 CAG GTC CAA CTC GAG CAG CCT GGG GCT (SEQ ID No. 346); MVH4 GAG GTT CAG CTC GAG CAG TCT GGG GCA (SEQ ID No. 347); MVH5 GA(AG) GTG AAG CTC GAG GAG TCT GGA GGA (SEQ ID No. 348); MVH6 GAG GTG AAG CTT CTC GAG TCT GGA GGT (SEQ ID No. 349); MVH7 GAA GTG AAG CTC GAG GAG TCT GGG GGA (SEQ ID No. 350); MVH8 GAG GTT CAG CTC GAG CAG TCT GGA GCT (SEQ ID No. 351)) were each combined with one 3'-VH primer (3'MuVHBstEII tga gga gac ggt gac cgt ggt ccc ttg gcc cca g (SEQ ID No. 352)); for the PCR amplification of the VK-chain fragments seven different 5'-VK-family specific primers (MUVK1 CCA GTT CCG AGC TCG TTG TGA CTC AGG AAT CT (SEQ ID No. 353); MUVK2 CCA GTT CCG AGC TCG TGT TGA CGC AGC CGC CC (SEQ ID No. 354); MUVK3 CCA GTT CCG AGC TCG TGC TCA CCC AGT CTC CA (SEQ ID No. 355); MUVK4 CCA GTT CCG AGC TCC AGA TGA CCC AGT CTC CA (SEQ ID No. 356); MUVK5 CCA GAT GTG AGC TCG TGA TGA CCC AGA CTC CA (SEQ ID No. 357); MUVK6 CCA GAT GTG AGC TCG TCA TGA CCC AGT CTC CA (SEQ ID No. 358); MUVK7 CCA GTT CCG AGC TCG TGA TGA CAC AGT CTC CA (S E Q ID No. 3 59)) were each combined with one 3'-VK primer (3'MuVkHindIII/BsiW1 tgg tgc act agt cgt acg ttt gat ctc aag ctt ggt ccc (SEQ ID No. 360)).
The following PCR program was used for amplification: denaturation at 94°C for 20 sec; primer annealing at 52°C for 50 sec and primer extension at 72°C for 60 sec and 40 cycles, followed by a 10 min final extension at 72°C.
450 ng of the kappa light chain fragments (Sacl-Spel digested) were ligated with 1400 ng of the phagemid pComb3H5Bhis (Sacl-Spel digested; large fragment). The resulting combinatorial antibody library was then transformed into 300 ul of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells were then harvested by centrifugation and plasmid preparation was carried out using a commercially available plasmid preparation kit (Qiagen).
2800 ng of this plasmid-DNA containing the VK-library (XhoI-BstEII digested; large fragment) were ligated with 900 ng of the heavy chain V-fragments (XhoI-BstEII digested) and again transformed into two 300 ul aliquots of electrocompetent E. coli XL1 Blue cells by electroporation (2.5 kV, 0.2 cm gap cuvette, 25 uFD, 200 Ohm) resulting in a total VH-VK scFv (single chain variable fragment) library size of more than $10^7$ independent clones.
After phenotype expression and slow adaptation to carbenicillin, the E. coli cells containing the antibody library were transferred into SB-Carbenicillin (50 ug/mL) selection medium. The E. coli cells containing the antibody library wass then infected with an infectious dose of $10^{12}$ particles of helper phage VCSM13 resulting in the production and secretion of filamentous M13 phage, wherein phage particle contains single stranded pComb3H5BHis-DNA encoding a murine scFv-fragment and displayed the corresponding scFv-protein as a translational fusion to phage coat protein III. This pool of phages displaying the antibody library was later used for the selection of antigen binding entities.

**2.3. Phage display based selection of CD3-specific binders**

The phage library carrying the cloned scFv-repertoire was harvested from the respective culture supernatant by PEG8000/NaCl precipitation and centrifugation. Approximately $10^{11}$ to $10^{12}$ scFv phage particles were resuspended in 0.4 ml of PBS/0.1% BSA and incubated with $10^5$ to $10^7$ Jurkat cells (a CD3-positive human T-cell line) for 1 hour on ice under slow agitation. These Jurkat cells were grown beforehand in RPMI medium enriched with fetal calf serum (10 %), glutamine and penicillin/streptomycin, harvested by centrifugation, washed in PBS and resuspended in PBS/1 % FCS (containing Na Azide). scFv phage which do not specifically bind to the Jurkat cells were eliminated by up to five washing steps with PBS/1 % FCS (containing Na Azide). After washing, binding entities were eluted from the cells by resuspending the cells in HCl-glycine pH 2.2 (10 min incubation with subsequent vortexing) and after neutralization with 2 M Tris pH 12, the eluate was used for infection of a fresh uninfected E. coli XL1 Blue culture (OD600 > 0.5). The E. coli culture containing E. coli cells successfully transduced with a phagemid copy, encoding a human scFv-fragment, were again selected for carbenicillin resistance and subsequently infected with VCMS 13 helper phage to start the second round of antibody display and in vitro selection. A total of 4 to 5 rounds of selections were carried out, normally.

**2.4. Screening for CD3-specific binders**

Plasmid DNA corresponding to 4 and 5 rounds of panning was isolated from E. coli cultures after selection. For the production of soluble scFv-protein, VH-VL-DNA fragments were excised from the plasmids (XhoI-SpeI). These fragments were cloned via the same restriction sites in the plasmid pComb3H5BFlag/His differing from the original pComb3H5BHis in that the expression construct (e.g. scFv) includes a Flag-tag (TGD YKDDDDK) between the scFv and the His6-tag and the additional phage proteins were deleted. After ligation, each pool (different rounds of panning) of plasmid DNA was transformed into 100 $\mu$l heat shock competent E. coli TG1 or XLI blue and plated onto carbenicillin LB-agar. Single colonies were picked into 100 ul of LB carb (50 ug/ml).
E. coli transformed with pComb3H5BHis containing a VL-and VH-segment produce soluble scFv in sufficient amounts after excision of the gene III fragment and induction with 1 mM IPTG. Due to a suitable signal sequence, the scFv-chain was exported into the periplasma where it folds into a functional conformation.
Single E. coli TG1 bacterial colonies from the transformation plates were picked for periplasmic small scale preparations and grown in SB-medium (e.g. 10 ml) supplemented with 20 mM MgCl2 and carbenicillin 50$\mu$g/ml (and re-dissolved in PBS (e.g. 1 ml) after harvesting. By four rounds of freezing at -70°C and thawing at 37°C, the outer membrane of the bacteria was destroyed by temperature shock and the soluble periplasmic proteins including the scFvs were released into the supernatant.
After elimination of intact cells and cell-debris by centrifugation, the supernatant containing the human anti-human CD3-scFvs was collected and used for further examination.

**2.5. Identification of CD3-specific binders**

Binding of the isolated scFvs was tested by flow cytometry on eukaryotic cells, which on their surface express a heterologous protein displaying at its N-terminus the first 27 N-terminal amino acids of CD3epsilon.
As described in Example 4, the first amino acids 1-27 of the N-terminal sequence of the mature CD3 epsilon chain of the human T cell receptor complex (amino acid sequence: QDGNEEMGGITQTPYKVSISGTTVILT SEQ ID NO: 2) were fused to the N-terminus of the transmembrane protein EpCAM so that the N-terminus was located at the outer cell surface. Additionally, a FLAG epitope was inserted between the N-terminal 1-27 CD3epsilon sequence and the EpCAM sequence. This fusion product was expressed in human embryonic kidney (HEK) and Chinese hamster ovary (CHO) cells. Eukaryotic cells displaying the 27 most N-terminal amino acids of mature CD3epsilon of other primate species were prepared in the same way for Saimiri ciureus (Squirrel monkey) (CD3epsilon N-terminal amino acid sequence: QDGNEEIGDTTQNPYKVSISGTTVTLT SEQ ID NO: 8), for Callithrix jacchus (CD3epsilon N-terminal amino acid sequence: QDGNEEMGDTTQNPYKVSISGTTVTLT SEQ ID NO: 4) and for Saguinus oedipus (CD3epsilon N-terminal amino acid sequence: QDGNEEMGDTTQNPYKVSISGTTVTLT SEQ ID NO: 6).
For flow cytometry 2,5x$10^5$ cells are incubated with 50 ul supernatant or with 5 μg/ml of the purified constructs in 50 μl PBS with 2% FCS. The binding of the constructs was detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) was used. The samples were measured on a FACSscan (BD biosciences, Heidelberg, FRG).
Binding was always confirmed by flowcytometry as described in the foregoing paragraph on primary T cells of man and different primates (e.g. saimiris ciureus, callithrix jacchus, saguinus oedipus).

**2.6. Generation of human/humanized equivalents of non-human CD3epsilon specific scFvs**

The VH region of the murine anti-CD3 scFv was aligned against human antibody germline amino acid sequences. The human antibody germline VH sequence was chosen which has the closest homology to the non-human VH and a direct alignment of the two amino acid sequences was performed. There were a number of framework residues of the non-human VH that differ from the human VH framework regions ("different framework positions"). Some of these residues may contribute to the binding and activity of the antibody to its target.
To construct a library that contain the murine CDRs and at every framework position that differs from the chosen human VH sequence both possibilities (the human and the maternal murine amino acid residue), degenerated oligonucleotides were synthesized. These oligonucleotides incorporate at the differing positions the human residue with a probability of 75 % and the murine residue with a probability of 25 %.
For one human VH e.g. six of these oligonucleotides had to be synthesized that overlap in a terminal stretch of approximately 20 nucleotides. To this end every second primer was an antisense primer. Restriction sites needed for later cloning within the oligonucleotides were deleted.
These primers may have a length of 60 to 90 nucleotides, depending on the number of primers that were needed to span over the whole V sequence.
These e.g. six primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods. This PCR product was then used as a template for a standard PCR reaction using primers that incorporate N-terminal and C-terminal suitable cloning restriction sites.
The DNA fragment of the correct size (for a VH approximately 350 nucleotides) was isolated by agarose gel electrophoresis according to standard methods. In this way sufficient VH DNA fragment was amplified. This VH fragment was now a pool of VH fragments that have each one a different amount of human and murine residues at the respective differing framework positions (pool of humanized VH). The same procedure was performed for the VL region of the murine anti-CD3 scFv (pool of humanized VL).
The pool of humanized VH was then combined with the pool of humanized VL in the phage display vector pComb3H5Bhis to form a library of functional scFvs from which - after display on filamentous phage - anti-CD3 binders were selected, screened, identified and confirmed as described above for the parental non-human (murine) anti-CD3 scFv. Single clones were then analyzed for favorable properties and amino acid sequence. Those scFvs which were closest in amino acid sequence homology to human germline V-segments are preferred particularly those wherein at least one CDR among CDR I and II of VH and CDR I and II of VLkappa or CDR I and II of VLlambda shows more than 80% amino acid sequence identity to the closest respective CDR of all human germline V-segments. Anti-CD3 scFvs were converted into recombinant bispecific single chain antibodies as described in the following Examples 9, 16, and 24.

**3. Generation of a recombinant fusion protein of the N-terminal amino acids 1-27 of the human CD3 epsilon chain fused to the Fc-part of an IgG1 (1-27 CD3-Fc).**

**3.1. Cloning and expression of 1-27 CD3-Fc**

The coding sequence of the 1-27 N-terminal amino acids of the human CD3 epsilon chain fused to the hinge and Fc gamma region of human immunoglobulin IgG1 as well as an 6 Histidine Tag were obtained by gene synthesis according to standard protocols (cDNA sequence and amino acid sequence of the recombinant fusion protein are listed under SEQ ID NOs 230 and 229). The gene synthesis fragment was designed as to contain first a Kozak site for eukaryotic expression of the construct, followed by an 19 amino acid immunoglobulin leader peptide, followed in frame by the coding sequence of the first 27 amino acids of the extracellular portion of the mature human CD3 epsilon chain, followed in frame by the coding sequence of the hinge region and Fc gamma portion of human IgG1, followed in frame by the coding sequence of a 6 Histidine tag and a stop codon (Figure 1). The gene synthesis fragment was also designed as to introduce restriction sites at the beginning and at the end of the cDNA coding for the fusion protein. The introduced restriction sites, EcoRI at the 5' end and SalI at the 3' end, are utilized in the following cloning procedures. The gene synthesis fragment was cloned via EcoRI and SalI into a plasmid designated pEF-DHFR (pEF-DHFR is described in Mack et al. Proc. Natl. Acad. Sci. USA 92 (1995) 7021-7025 and Raum et al. Cancer Immunol Immunother 50 (2001) 141-150) following standard protocols. A sequence verified plasmid was used for transfection in the FreeStyle 293 Expression System (Invitrogen GmbH, Karlsruhe, Germany) according to the manufacturers protocol. After 3 days cell culture supernatants of the transfectants were harvested and tested for the presence of the recombinant construct in an ELISA assay. Goat anti-human IgG, Fc-gamma fragment specific antibody (obtained from Jackson ImmunoResearch Europe Ltd., Newmarket, Suffolk, UK) was diluted in PBS to 5 μg/ml and coated with 100 μl per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with 3 % BSA in PBS (bovine Albumin, fraction V, Sigma-Aldrich Chemie GmbH, Taufkirchen, Germany) for 60 minutes at room temperature (RT). Subsequently, wells were washed again PBS/Tween and then incubated with cell culture supernatants for 60 minutes at RT. After washing wells were incubated with a peroxidase conjugated anti-His6 antibody (Roche Diagnostics GmbH, Roche Applied Science, Mannheim, Germany) diluted 1:500 in PBS with 1 % BSA for 60 minutes at RT. Subsequently, wells were washed with 200 μl PBS/Tween and 100 μl of the SIGMAFAST OPD (SIGMAFAST OPD [o-Phenylenediamine dihydrochloride] substrate solution (Sigma-Aldrich Chemie GmbH, Taufkirchen, Germany) was added according to the manufacturers protocol. The reaction was stopped by adding 100 μl 1 M $H_2SO_4$. Color reaction was measured on a PowerWaveX microplate spectrophotometer (BioTek Instruments, Inc., Winooski, Vermont, USA) at 490 nm and subtraction of background absorption at 620 nm. As shown in Figure 2 presence of the construct as compared to irrelevant supernatant of mock-transfected HEK 293 cells used as negative control was clearly detectable.

**3.2. Binding assay of cross-species specific single chain antibodies to 1-27 CD3-Fc.**

Binding of crude preparations of periplasmatically expressed cross-species specific single chain antibodies specific for CD3 epsilon to 1-27 CD3-Fc was tested in an ELISA assay. Goat anti-human IgG, Fc-gamma fragment specific antibody (Jackson ImmunoResearch Europe Ltd., Newmarket, Suffolk, UK) was diluted in PBS to 5 μg/ml and coated with 100 μl per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with PBS with 3 % BSA (bovine Albumin, fraction V, Sigma-Aldrich Chemie GmbH, Taufkirchen, Germany) for 60 minutes at RT. Subsequently, wells were washed with PBS/Tween and incubated with supernatants of cells expressing the 1-27 CD3-Fc construct for 60 minutes at RT. Wells were washed with PBS/Tween and incubated with crude preparations of periplasmatically expressed cross-species specific single-chain antibodies as described above for 60 minutes at room temperature. After washing with PBS/Tween wells were incubated with peroxidase conjugated anti-Flag M2 antibody (Sigma-Aldrich Chemie GmbH, Taufkirchen, Germany) diluted 1:10000 in PBS with 1 % BSA for 60 minutes at RT. Wells were washed with PBS/Tween and incubated with 100 μl of the SIGMAFAST OPD (OPD [o-Phenylenediamine dihydrochloride] substrate solution (Sigma-Aldrich Chemie GmbH, Taufkirchen, Germany) according to the manufacturers protocol. Color reaction was stopped with 100 μl 1 M $H_2SO_4$ and measured on a PowerWaveX microplate spectrophotometer (BioTek Instruments, Inc., Winooski, Vermont, USA) at 490 nm and subtraction of background absorption at 620 nm. Strong binding of cross-species specific human single chain antibodies specific for CD3 epsilon to the 1-27 CD3-Fc construct compared to a murine anti CD3 single-chain antibody was observed (Figure 3).

**4. Generation of recombinant transmembrane fusion proteins of the N-terminal amino acids 1-27 of CD3 epsilon from different non-chimpanzee primates fused to EpCAM from cynomolgus monkey (1-27 CD3-EpCAM).**

**4.1. Cloning and expression of 1-27 CD3-EpCAM**

CD3 epsilon was isolated from different non-chimpanzee primates (marmoset, tamarin, squirrel monkey) and swine. The coding sequences of the 1-27 N-terminal amino acids of CD3 epsilon chain of the mature human, common marmoset (*Callithrix jacchus*), cottontop tamarin (*Saguinus oedipus*), common squirrel monkey (*Saimiri sciureus*) and domestic swine (*Sus scrofa*; used as negative control) fused to the N-terminus of Flag tagged cynomolgus EpCAM were obtained by gene synthesis according to standard protocols. cDNA sequence and amino acid sequence of the recombinant fusion proteins are listed under SEQ ID NOs 231 to 240). The gene synthesis fragments were designed as to contain first a BsrGI site to allow fusion in correct reading frame with the coding sequence of a 19 amino acid immunoglobulin leader peptide already present in the target expression vector, which is followed in frame by the coding sequence of the N-terminal 1-27 amino acids of the extracellular portion of the mature CD3 epsilon chains, which is followed in frame by the coding sequence of a Flag tag and followed in frame by the coding sequence of the mature cynomolgus EpCAM transmembrane protein (Figure 4). The gene synthesis fragments were also designed to introduce a restriction site at the end of the cDNA coding for the fusion protein. The introduced restriction sites BsrGI at the 5' end and SalI at the 3' end, were utilized in the following cloning procedures. The gene synthesis fragments were then cloned via BsrGI and SalI into a derivative of the plasmid designated pEF DHFR (pEF-DHFR is described in Mack et al. Proc. Natl. Acad. Sci. USA 92 (1995) 7021-7025), which already contained the coding sequence of the 19 amino acid immunoglobulin leader peptide following standard protocols.
Sequence verified plasmids were used to transiently transfect 293-HEK cells using the MATra-A Reagent (IBA GmbH, Göttingen, Germany) and 12 $\mu$g of plasmid DNA for adherent 293-HEK cells in 175 ml cell culture flasks according to the manufacturers protocol. After 3 days of cell culture the transfectants were tested for cell surface expression of the recombinant transmembrane protein via an FACS assay according to standard protocols. For that purpose a number of $2,5x10^5$ cells were incubated with the anti-Flag M2 antibody (Sigma-Aldrich Chemie GmbH, Taufkirchen, Germany) at 5 $\mu$g/ml in PBS with 2% FCS. Bound antibody was detected with an R-Phycoerythrin-conjugated affinity purified F(ab')2 fragment, goat anti-mouse IgG, Fc-gamma fragment specific 1:100 in PBS with 2% FCS (Jackson ImmunoResearch Europe Ltd., Newmarket, Suffolk, UK). The samples were measured on a FACScalibur (BD biosciences, Heidelberg, Germany). Expression of the Flag tagged recombinant transmembrane fusion proteins consisting of cynomolgus EpCAM and the 1-27 N-terminal amino acids of the human, marmoset, tamarin, squirrel monkey and swine CD3 epsilon chain respectively on transfected cells was clearly detectable (Figure 5).

**4.2. Binding of cross-species specific anti-CD3 single chain antibodies to the 1-27 CD3-EpCAM**

Binding of crude preparations of periplasmatically expressed cross-species specific anti CD3 single-chain antibodies to the 1-27 N-terminal amino acids of the human, marmoset, tamarin and squirrel monkey CD3 epsilon chains respectively fused to cynomolgus Ep-CAM was tested in an FACS assay according to standard protocols.
For that purpose a number of $2,5x10^5$ cells were incubated with crude preparations of periplasmatically expressed cross-species specific anti CD3 single-chain antibodies (preparation was performed as described above and according to standard protocols) and a single-chain murine anti-human CD3 antibody as negative control. As secondary antibody the Penta-His antibody (Qiagen GmbH, Hildesheim, Germany) was used at 5 $\mu$g/ml in 50 $\mu$l PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACScalibur (BD biosciences, Heidelberg, Germany). As shown in Figures 6 (A to E) binding of single chain antibodies to the transfectants expressing the recombinant transmembrane fusion proteins consisting of the 1-27 N-terminal amino acids of CD3 epsilon of the human, marmoset, tamarin or squirrel monkey fused to cynomolgus EpCAM was observed. No binding of cross-species specific single chain antibodies was observed to a fusion protein consisting of the 1-27 N-terminal CD3 epsilon of swine fused to cynomolgus EpCAM used as negative control.
Multi-primate cross-species specificity of the anti-CD3 single chain antibodies was shown. Signals obtained with the anti Flag M2 antibody and the cross-species specific single chain antibodies were comparable, indicating a strong binding activity of the cross-species specific single chain antibodies to the N-terminal amino acids 1-27 of CD3 epsilon.

**5. Binding analysis of cross-species specific anti-CD3 single chain antibodies by alanine-scanning of mouse cells transfected with the human CD3 epsilon chain and its alanine mutants**

**5.1. Cloning and expression of human wild-type CD3 epsilon**

The coding sequence of the human CD3 epsilon chain was obtained by gene synthesis according to standard protocols (cDNA sequence and amino acid sequence of the human CD3 epsilon chain are listed under SEQ ID NOs 242 and 241). The gene synthesis fragment was designed as to contain a Kozak site for eukaryotic expression of the construct and restriction sites at the beginning and the end of the cDNA coding for human CD3 epsilon. The introduced restriction sites EcoRI at the 5' end and SalI at the 3' end, were utilized in the following cloning procedures. The gene synthesis fragment was then cloned via EcoRI and SalI into a plasmid designated pEF NEO following standard protocols. pEF NEO was derived of pEF DHFR (Mack et al. Proc. Natl. Acad. Sci. USA 92 (1995) 7021-7025) by replacing the cDNA of the DHFR with the cDNA of the neomycin resistance by conventional molecular cloning. A sequence verified plasmid was used to transfect the murine T cell line EL4 (ATCC No. TIB-39) cultivated in RPMI with stabilized L-glutamine supplemented with 10 % FCS, 1% penicillin/streptomycin, 1% HEPES, 1% pyruvate, 1% non-essential amino acids (all Biochrom AG Berlin, Germany) at 37 °C, 95 % humidity and 7 % $CO_2$. Transfection was performed with the SuperFect Transfection Reagent (Qiagen GmbH, Hilden, Germany) and 2 μg of plasmid DNA according to the manufacturer's protocol. After 24 hours the cells were washed with PBS and cultivated again in the aforementioned cell culture medium with600 μg/ml G418 for selection (PAA Laboratories GmbH, Pasching, Austria). 16 to 20 days after transfection the outgrowth of resistant cells was observed. After additional 7 to 14 days cells were tested for expression of human CD3 epsilon by FACS analysis according to standard protocols. $2,5x10^5$ cells were incubated with anti-human CD3 antibody UCHT-1 (BD biosciences, Heidelberg, Germany) at 5 μg/ml in PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACSCalibur (BD biosciences, Heidelberg, Germany). Expression of human wild-type CD3 on transfected EL4 cells is shown in Figure 7.

**5.2. Cloning and expression of the cross-species specific anti-CD3 single chain antibodies as IgG1 antibodies**

In order to provide improved means of detection of binding of the cross-species specific single chain anti-CD3 antibodies H2C HLP, A2J HLP and E2M HLP were converted into IgG1 antibodies with murine IgG1 and human lambda constant regions. cDNA sequences coding for the heavy and light chains of respective IgG antibodies were obtained by gene synthesis according to standard protocols. The gene synthesis fragments for each specificity were designed as to contain first a Kozak site to allow eukaryotic expression of the construct, which is followed by an 19 amino acid immunoglobulin leader peptide (SEQ ID NOs 244 and 243), which is followed in frame by the coding sequence of the respective heavy chain variable region or respective light chain variable region, followed in frame by the coding sequence of the heavy chain constant region of murine IgG1 (SEQ ID NOs 246 and 245) or the coding sequence of the human lambda light chain constant region (SEQ ID NO 248 and 247), respectively. Restriction sites were introduced at the beginning and the end of the cDNA coding for the fusion protein. Restriction sites EcoRI at the 5' end and SalI at the 3' end were used for the following cloning procedures. The gene synthesis fragments were cloned via EcoRI and SalI into a plasmid designated pEF DHFR (Mack et al. Proc. Natl. Acad. Sci. USA 92 (1995) 7021-7025) for the heavy chain constructs and pEF ADA (pEF ADA is described in Raum et al., Cancer Immunol Immunother., 50(3), (2001), 141-50) for the light chain constructs) according to standard protocols. Sequence verified plasmids were used for co-transfection of respective light and heavy chain constructs in the FreeStyle 293 Expression System (Invitrogen GmbH, Karlsruhe, Germany) according to the manufacturers protocol. After 3 days cell culture supernatants of the transfectants were harvested and used for the alanine-scanning experiment.

**5.3. Cloning and expression of alanine mutants of human CD3 epsilon for alanine-scanning**

27 cDNA fragments coding for the human CD3 epsilon chain with an exchange of one codon of the wild-type sequence of human CD3 epsilon into a codon coding for alanine (GCC) for each amino acid of amino acids 1-27 of the extracellular domain of the mature human CD3 epsilon chain respectively were obtained by gene synthesis.
Except for the exchanged codon the cDNA fragments were identical to the aforementioned human wild-type CD3 cDNA fragment. Only one codon was replaced in each construct compared to the human wild-type CD3 cDNA fragment described above. Restriction sites EcoRI and SalI were introduced into the cDNA fragments at identical positions compared to the wild-type construct. All alanine-scanning constructs were cloned into pEF NEO and sequence verified plasmids were transfected into EL4 cells. Transfection and selection of transfectants was performed as described above. As result a panel of expressed constructs was obtained wherein the first amino acid of the human CD3 epsilon chain, glutamine (Q, Gln) at position 1 was replaced by alanine. The last amino acid replaced by alanine was the threonine (T, Thr) at position 27 of mature human wild-type CD3 epsilon. For each amino acid between glutamine 1 and threonine 27 respective transfectants with an exchange of the wild-type amino acid into alanine were generated.

### 5.4. Alanine-scanning experiment

Chimeric IgG antibodies as described in 5.2 and cross-species specific single chain antibodies specific for CD3 epsilon were tested in alanine-scanning experiment.

Binding of the antibodies to the EL4 cell lines transfected with the alanine-mutant constructs of human CD3 epsilon as described in 5.3 was tested by FACS assay according to standard protocols. 2,5x$10^5$ cells of the respective transfectants were incubated with 50 µl of cell culture supernatant containing the chimeric IgG antibodies or with 50 µl of crude preparations of periplasmatically expressed single-chain antibodies. For samples incubated with crude preparations of periplasmatically expressed single-chain antibodies the anti-Flag M2 antibody (Sigma-Aldrich Chemie GmbH, Taufkirchen, Germany) was used as secondary antibody at 5 µg/ml in 50 µl PBS with 2% FCS. For samples incubated with the chimeric IgG antibodies a secondary antibody was not necessary. For all samples the binding of the antibody molecules was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACSCalibur (BD biosciences, Heidelberg, Germany). Differential binding of chimeric IgG molecules or cross-species specific single-chain antibodies to the EL4 cell lines transfected with the alanine-mutants of human CD3 epsilon was detected. As negative control either an isotype control or a crude preparation of a periplasmatically expressed single-chain antibody of irrelevant specificity was used respectively. UCHT-1 antibody was used as positive control for the expression level of the alanine-mutants of human CD3 epsilon. The EL4 cell lines transfected with the alanine-mutants for the amino acids tyrosine at position 15, valine at position 17, isoleucine at position 19, valine at position 24 or leucine at position 26 of the mature CD3 epsilon chain were not evaluated due to very low expression levels (data not shown). Binding of the cross-species specific single chain antibodies and the single chain antibodies in chimeric IgG format to the EL4 cell lines transfected with the alanine-mutants of human CD3 epsilon is shown in Figure 8 (A-D) as relative binding in arbitrary units with the geometric mean fluorescence values of the respective negative controls subtracted from all respective geometric mean fluorescence sample values. To compensate for different expression levels all sample values for a certain transfectant were then divided through the geometric mean fluorescence value of the UCHT-1 antibody for the respective transfectant. For comparison with the wild-type sample value of a specificity all sample values of the respective specificity were finally divided through the wild-type sample value, thereby setting the wild-type sample value to 1 arbitrary unit of binding.

The calculations used are shown in detail in the following formula:

$$value_Sample(x,y) = \frac{Sample(x,y) - neg_Contr.(x)}{(UCHT-1(x) - neg_Contr.(x)) * \dfrac{WT(y) - neg_Contr.(wt)}{UCHT-1(wt) - neg_Contr.(wt)}}$$

In this equation *value_Sample* means the value in arbitrary units of binding depicting the degree of binding of a specific anti-CD3 antibody to a specific alanine-mutant as shown in Figure 8 (A-D), *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT-1* means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, x specifies the respective transfectant, *y* specifies the respective anti-CD3 antibody and *wt* specifies that the respective transfectant is the wild-type.

As can be seen in Figure 8 (A-D) the IgG antibody A2J HLP showed a pronounced loss of binding for the amino acids asparagine at position 4, threonine at position 23 and isoleucine at position 25 of the mature CD3 epsilon chain. A complete loss of binding of IgG antibody A2J HLP was observed for the amino acids glutamine at position 1, aspartate at position 2, glycine at position 3 and glutamate at position 5 of the mature CD3 epsilon chain. IgG antibody E2M HLP showed a pronounced loss of binding for the amino acids asparagine at position 4, threonine at position 23 and isoleucine at position 25 of the mature CD3 epsilon chain. IgG antibody E2M HLP showed a complete loss of binding for the amino acids glutamine at position 1, aspartate at position 2, glycine at position 3 and glutamate at position 5 of the mature CD3 epsilon chain. IgG antibody H2C HLP showed an intermediate loss of binding for the amino acid asparagine at position 4 of the mature CD3 epsilon chain and it showed a complete loss of binding for the amino acids glutamine at position 1, aspartate at position 2, glycine at position 3 and glutamate at position 5 of the mature CD3 epsilon chain. Single chain antibody F12Q HLP showed an essentially complete loss of binding for the amino acids glutamine at position 1, aspartate at position 2, glycine at position 3 of the mature CD3 epsilon chain and glutamate at position 5 of the mature CD3 epsilon chain.

**6. Binding analysis of the cross-species specific anti-CD3 binding molecule H2C HLP to the human CD3 epsilon chain with and without N-terminal His6 tag transfected into the murine T cell line EL4**

**6.1. Cloning and expression of the human CD3 epsilon chain with N-terminal six histidine tag (His6 tag)**

A cDNA fragment coding for the human CD3 epsilon chain with a N-terminal His6 tag was obtained by gene synthesis. The gene synthesis fragment was designed as to contain first a Kozak site for eukaryotic expression of the construct, which is followed in frame by the coding sequence of a 19 amino acid immunoglobulin leader peptide, which is followed in frame by the coding sequence of a His6 tag which is followed in frame by the coding sequence of the mature human CD3 epsilon chain (the cDNA and amino acid sequences of the construct are listed as SEQ ID NOs 256 and 255).
The gene synthesis fragment was also designed as to contain restriction sites at the beginning and the end of the cDNA. The introduced restriction sites EcoRI at the 5' end and SalI at the 3' end, were used in the following cloning procedures. The gene synthesis fragment was then cloned via EcoRI and SalI into a plasmid designated pEF-NEO (as described above) following standard protocols. A sequence verified plasmid was used to transfect the murine T cell line EL4. Transfection and selection of the transfectants were performed as described above. After 34 days of cell culture the transfectants were used for the assay described below.

**6.2. Binding of the cross-species specific anti-CD3 binding molecule H2C HLP to the human CD3 epsilon chain with and without N-terminal His6 tag**

A chimeric IgG antibody with the binding specificity H2C HLP specific for CD3 epsilon was tested for binding to human CD3 epsilon with and without N-terminal His6 tag. Binding of the antibody to the EL4 cell lines transfected the His6-human CD3 epsilon and wild-type human CD3 epsilon respectively was tested by an FACS assay according to standard protocols. $2,5x10^5$ cells of the transfectants were incubated with 50 μl of cell culture supernatant containing the chimeric IgG antibody or 50 μl of the respective control antibodies at 5μg/ml in PBS with 2% FCS. As negative control an appropriate isotype control and as positive control for expression of the constructs the CD3 specific antibody UCHT-1 were used respectively. The binding of the antibodies was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK).

Samples were measured on a FACSCalibur (BD biosciences, Heidelberg, Germany). Compared to the EL4 cell line transfected with wild-type human CD3 epsilon a clear loss of binding of the chimeric IgG with binding specificity H2C HLP to human-CD3 epsilon with an N-terminal His6 tag was detected. These results showed that a free N-terminus of CD3 epsilon is essential for binding of the cross-species specific anti-CD3 binding specificity H2C HLP to the human CD3 epsilon chain (Figure 9).

**7. Cloning and expression of the C-terminal, transmembrane and truncated extracellular domains of human MCSP**

The coding sequence of the C-terminal, transmembrane and truncated extracellular domain of human MCSP (amino acids 1538 - 2322) was obtained by gene synthesis according to standard protocols (cDNA sequence and amino acid sequence of the recombinant construct for expression of the C-terminal, transmembrane and truncated extracellular domain of human MCSP (designated as human D3) are listed under SEQ ID NOs 250 and 249). The gene synthesis fragment was designed as to contain first a Kozak site to allow eukaryotic expression of the construct followed by the coding sequence of an 19 amino acid immunoglobulin leader peptide followed in frame by a FLAG tag, followed in frame by a sequence containing several restriction sites for cloning purposes and coding for a 9 amino acid artificial linker (SRTRSGSQL), followed in frame by the coding sequence of the C-terminal, transmembrane and truncated extracellular domain of human MCSP and a stop codon. Restriction sites were introduced at the beginning and at the end of the DNA fragment. The restriction sites EcoRI at the 5' end and SalI at the 3' end were used in the following cloning procedures. The fragment was digested with EcoRI and SalI and cloned into pEF-DHFR (pEF-DHFR is described in Mack et al. Proc. Natl. Acad. Sci. USA 92 (1995) 7021-7025) following standard protocols. A sequence verified plasmid was used to transfect CHO/dhfr- cells (ATCC No. CRL 9096). Cells were cultivated in RPMI 1640 with stabilized glutamine, supplemented with 10% FCS, 1% penicillin/streptomycin (all obtained from Biochrom AG Berlin, Germany) and nucleosides from a stock solution of cell culture grade reagents (Sigma-Aldrich Chemie GmbH, Taufkirchen, Germany) to a final

concentration of 10 μg/ml Adenosine, 10 μg/ml Deoxyadenosine and 10 μg/ml Thymidine, in an incubator at 37 °C, 95% humidity and 7% $CO_2$. Transfection was performed using the PolyFect Transfection Reagent (Qiagen GmbH, Hilden, Germany) and 5 μg of plasmid DNA according to the manufacturer's protocol. After cultivation for 24 hours cells were washed once with PBS and cultivated again in RPMI 1640 with stabilized glutamine and 1% penicillin/streptomycin. Thus the cell culture medium did not contain nucleosides and thereby selection was applied on the transfected cells.

Approximately 14 days after transfection the outgrowth of resistant cells was observed. After an additional 7 to 14 days the transfectants were tested for expression of the construct by FACS analysis. 2,5x$10^5$ cells were incubated with 50 μl of an anti-Flag-M2 antibody (Sigma-Aldrich Chemie GmbH, Taufkirchen, Germany) diluted to 5 μg/ml in PBS with 2% FCS. The binding of the antibody was detected with a R-Phycoerythrin-conjugated affinity purified F(ab')2 fragment, goat anti-mouse IgG, Fc-gamma fragment specific diluted 1:100 in PBS with 2% FCS (ImmunoResearch Europe Ltd., Newmarket, Suffolk, UK). The samples were measured on a FACScalibur (BD biosciences, Heidelberg, Germany). **8. Cloning and expression of the C-terminal, transmembrane and truncated extracellular domains of macaque MCSP**

The cDNA sequence of the C-terminal, transmembrane and truncated extracellular domains of macaque MCSP (designated as macaque D3) was obtained by a set of three PCRs on macaque skin cDNA (Cat No. C1534218-Cy-BC; BioCat GmbH, Heidelberg, Germany) using the following reaction conditions: 1 cycle at 94°C,3 min., 40 cycles with 94°C for 0,5 min., 52°C for 0,5 min. and 72°C for 1,75 min., terminal cycle of 72°C for 3 min.. The following primers were used:

forward primer: 5'-GATCTGGTCTACACCATCGAGC-3' (SEQ ID No. 361)
reverse primer: 5'-GGAGCTGCTGCTGGCTCAGTGAGG-3' (SEQ ID No. 362)
forward primer: 5'- TTCCAGCTGAGCATGTCTGATGG-3' (SEQ ID No. 363)
reverse primer: 5'- CGATCAGCATCTGGGCCCAGG-3' (SEQ ID No. 364)
forward primer: 5'- GTGGAGCAGTTCACTCAGCAGGACC-3' (SEQ ID No. 365)
reverse primer: 5'- GCCTTCACACCCAGTACTGGCC-3' (SEQ ID No. 366)

Those PCRs generated three overlapping fragments (A: 1-1329, B: 1229-2428, C: 1782-2547) which were isolated and sequenced according to standard protocols using the PCR primers and thereby provided a 2547 bp portion of the cDNA sequence of macaque MCSP (the cDNA sequence and amino acid sequence of this portion of macaque MCSP are listed under SEQ ID NOs 252 and 251) from 74 bp upstream of the coding sequence of the C-terminal domain to 121 bp downstream of the stop codon. Another PCR using the following reaction conditions: 1 cycle at 94°C for 3 min, 10 cycles with 94°C for 1 min, 52°C for 1 min and 72°C for 2,5 min, terminal cycle of 72°C for 3 min was used to fuse the PCR products of the aforementioned reactions A and B. The following primers are used:

forward primer: 5'-tcccgtacgagatctggatcccaattggatggcggactcgtgctgttctcacacagagg-3' (SEQ ID No. 367)
reverse primer: 5'-agtgggtcgactcacacccagtactggccattcttaagggcaggg-3' (SEQ ID No. 368)

The primers for this PCR were designed to introduce restriction sites at the beginning and at the end of the cDNA fragment coding for the C-terminal, transmembrane and truncated extracellular domains of macaque MCSP. The introduced restriction sites Mfel at the 5' end and SalI at the 3' end, were used in the following cloning procedures. The PCR fragment was then cloned via Mfel and SalI into a Bluescript plasmid containing the EcoRI/Mfel fragment of the aforementioned plasmid pEF-DHFR (pEF-DHFR is described in Raum et al. Cancer Immunol Immunother 50 (2001) 141-150) by replacing the C-terminal, transmembrane and truncated extracellular domains of human MCSP. The gene synthesis fragment contained the coding sequences of the immunoglobulin leader peptide and the Flag tag as well as the artificial linker (SRTRSGSQL) in frame to the 5' end of the cDNA fragment coding for the C-terminal, transmembrane and truncated extracellular domains of macaque MCSP. This vector was used to transfect CHO/dhfr- cells (ATCC No. CRL 9096). Cells were cultivated in RPMI 1640 with stabilized glutamine supplemented with 10% FCS, 1% penicillin/streptomycin (all from Biochrom AG Berlin, Germany) and nucleosides from a stock solution of cell culture grade reagents (Sigma-Aldrich Chemie GmbH, Taufkirchen, Germany) to a final concentration of 10 μg/ml Adenosine, 10 μg/ml Deoxyadenosine and 10 μg/ml Thymidine, in an incubator at 37 °C, 95% humidity and 7% CO2. Transfection was performed with PolyFect Transfection Reagent (Qiagen GmbH, Hilden, Germany) and 5 μg of plasmid DNA according to the manufacturer's protocol. After cultivation for 24 hours cells were washed once with PBS and cultivated again in RPMI 1640 with stabilized glutamine and 1% penicillin/streptomycin. Thus the cell culture medium did not contain nucleosides and thereby selection was applied on the transfected cells.

Approximately 14 days after transfection the outgrowth of resistant cells is observed. After an additional 7 to 14 days the transfectants were tested for expression of the recombinant construct via FACS. 2,5x$10^5$ cells were incubated with 50 μl of an anti-Flag-M2 antibody (Sigma-Aldrich Chemie GmbH, Taufkirchen, Germany) diluted to 5 μg/ml in PBS with 2% FCS. Bound antibody was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd.,

Newmarket, Suffolk, UK). Samples were measured on a FACScalibur (BD biosciences, Heidelberg, Germany).

**9. Generation and characterisation of MCSP and CD3 cross-species specific bispecific single chain molecules**

Bispecific single chain antibody molecules each comprising a binding domain cross-species specific for human and non-chimpanzee primate CD3 epsilon as well as a binding domain cross-species-specific for human and non-chimpanzee primate MCSP, are designed as set out in the following Table 1:

Table 1: Formats of MCSP and CD3 cross-species specific bispecific single chain antibodies

| SEQ ID (nucl/prot) | Formats of protein constructs (N → C) |
|---|---|
| 190/189 | MCSP-G4 HL x H2C HL |
| 192/191 | MCSP-G4 HL x F12Q HL |
| 194/193 | MCSP-G4 HL x I2C HL |
| 196/195 | MCSP-G4 HLP x F6A HLP |
| 198/197 | MCSP-G4 HLP x H2C HLP |
| 202/201 | MCSP-G4 HLP x G4H HLP |
| 206/205 | MCSP-G4 HLP x E1 L HLP |
| 208/207 | MCSP-G4 HLP x E2M HLP |
| 212/211 | MCSP-G4 HLP x F12Q HL |
| 214/213 | MCSP-G4 HLP x I2C HL |
| 216/215 | MCSP-D2 HL x H2C HL |
| 218/217 | MCSP-D2 HL x F12Q HL |
| 220/219 | MCSP-D2 HL x I2C HL |
| 222/221 | MCSP-D2 HLP x H2C HLP |
| 224/223 | MCSP-F9 HL x H2C HL |
| 226/225 | MCSP-F9 HLP x H2C HLP |
| 228/227 | MCSP-F9 HLP x G4H HLP |
| 318/317 | MCSP-A9 HL x H2C HL |
| 320/319 | MCSP-A9 HL x F12Q HL |
| 322/321 | MCSP-A9 HL x I2C HL |
| 324/323 | MCSP-C8 HL x I2C HL |
| 328/327 | MCSP-B7 HL x I2C HL |
| 326/325 | MCSP-B8 HL x I2C HL |
| 330/329 | MCSP-G8 HL x I2C HL |
| 332/331 | MCSP-D5 HL x I2C HL |
| 334/333 | MCSP-F7 HL x I2C HL |
| 336/335 | MCSP-G5 HL x I2C HL |
| 338/337 | MCSP-F8 HL x I2C HL |
| 340/339 | MCSP-G10 HL x I2C HL |

The aforementioned constructs containing the variable heavy-chain (VH) and variable light-chain (VL) domains cross-species specific for human and macaque MCSP D3 and the VH and VL domains cross-species specific for human and macaque CD3 were obtained by gene synthesis. The gene synthesis fragments were designed as to contain first a Kozak site for eukaryotic expression of the construct, followed by a 19 amino acid immunoglobulin leader peptide,

followed in frame by the coding sequence of the respective bispecific single chain antibody molecule, followed in frame by the coding sequence of a histidine$_6$-tag and a stop codon. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were transfected stably or transiently into DHFR-deficient CHO-cells (ATCC No. CRL 9096) by electroporation or alternatively into HEK 293 (human embryonal kidney cells, ATCC Number: CRL-1573) in a transient manner according to standard protocols.
Eukaryotic protein expression in DHFR deficient CHO cells was performed as described by Kaufmann R.J. (1990) Methods Enzymol. 185, 537-566. Gene amplification of the constructs was induced by addition of increasing concentrations of methothrexate (MTX) up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein is stored at -20°C.
Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).
Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.
Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.
The bispecific single chain antibody has a molecular weight of about 52 kDa under native conditions as determined by gel filtration in phosphate buffered saline (PBS). All constructs were purified according to this method.
Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

Alternatively, constructs were transiently expressed in DHFR deficient CHO cells. In brief, 4 x 105 cells per construct were cultivated in 3 ml RPMI 1640 all medium with stabilized glutamine supplemented with 10% fetal calf serum, 1% penicillin/streptomycin and nucleosides from a stock solution of cell culture grade reagents (Sigma-Aldrich Chemie GmbH, Taufkirchen, Germany) to a final concentration of 10 μg/ml Adenosine, 10 μg/ml Deoxyadenosine and 10 μg/ml Thymidine, in an incubator at 37 °C, 95% humidity and 7% CO2 one day before transfection. Transfection was performed with Fugene 6 Transfection Reagent (Roche, # 11815091001) according to the manufacturer's protocol. 94 μl OptiMEM medium (Invitrogen) and 6 μl Fugene 6 are mixed and incubated for 5 minutes at room temperature. Subsequently, 1.5 μg DNA per construct were added, mixed and incubated for 15 minutes at room temperature. Meanwhile, the DHFR deficient CHO cells were washed with 1x PBS and resuspended in 1.5 ml RPMI 1640 all medium. The transfection mix was diluted with 600 μl RPMI 1640 all medium, added to the cells and incubated overnight at 37 °C, 95% humidity and 7% CO2. The day after transfection the incubation volume of each approach was extended to 5 ml RPMI 1640 all medium. Supernatant was harvested after 3 days of incubation.

**10. Flow cytometric binding analysis of the MCSP and CD3 cross-species specific bispecific antibodies**

In order to test the functionality of the cross-species specific bispecific antibody constructs regarding the capability to bind to human and macaque MCSP D3 and CD3, respectively, a FACS analysis was performed. For this purpose CHO cells transfected with human MCSP D3 (as described in Example 7) and the human CD3 positive T cell leukemia cell line HPB-ALL (DSMZ, Braunschweig, ACC483) were used to test the binding to human antigens. The binding reactivity to macaque antigens was tested by using the generated macaque MCSP D3 transfectant (described in Example 8) and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of the purified protein of the cross-species specific bispecific antibody constructs (2 μg/ml) or cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines. A single chain construct with irrelevant target specificity was used as negative control for binding to the MCSP-D3 transfected CHO cells.
Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
The bispecific binding of the single chain molecules listed above, which are cross-species specific for MCSP D3 and cross-species specific for human and macaque CD3 was clearly detectable as shown in Figures 10, 11, 12 and 39. In the FACS analysis all constructs showed binding to CD3 and MCSP D3 as compared to the respective negative controls. Cross-species specificity of the bispecific antibodies to human and macaque CD3 and MCSP D3 antigens was demonstrated.

**11. Bioactivity of MCSP and CD3 cross-species specific bispecific single chain antibodies**

Bioactivity of the generated bispecific single chain antibodies was analyzed by chromium 51 ($^{51}Cr$) release in vitro cytotoxicity assays using the MCSP D3 positive cell lines described in Examples 7 and 8. As effector cells stimulated human CD4/CD56 depleted PBMC, stimulated human PBMC or the macaque T cell line 4119LnPx are used as specified in the respective figures.
Generation of the stimulated CD4/CD56 depleted PBMC was performed as follows:

> Coating of a Petri dish (145 mm diameter, Greiner bio-one GmbH, Kremsmünster) was carried out with a commercially available anti-CD3 specific antibody (e.g. OKT3, Othoclone) in a final concentration of 1 μg/ml for 1 hour at 37°C. Unbound protein was removed by one washing step with PBS. The fresh PBMC were isolated from peripheral blood (30 - 50 ml human blood) by Ficoll gradient centrifugation according to standard protocols. 3 - 5 x $10^7$ PBMC were added to the precoated petri dish in 120 ml of RPMI 1640 with stabilized glutamine / 10% FCS / IL-2 20 U/ml (Proleukin, Chiron) and stimulated for 2 days. On the third day the cells were collected and washed once with RPMI 1640. IL-2 was added to a final concentration of 20 U/ml and the cells were cultivated again for one day in the same cell culture medium as above. By depletion of CD4+ T cells and CD56+ NK cells according to standard protocols CD8+ cytotoxic T lymphocytes (CTLs) were enriched.

Target cells were washed twice with PBS and labelled with 11.1 MBq $^{51}Cr$ in a final volume of 100 μl RPMI with 50% FCS for 45 minutes at 37°C. Subsequently the labelled target cells were washed 3 times with 5 ml RPMI and then used in the cytotoxicity assay. The assay was performed in a 96 well plate in a total volume of 250μl supplemented RPMI (as above) with an E:T ratio 10:1.1 μg/ml of the cross-species specific bispecific single chain antibody molecules and 20 threefold dilutions thereof were applied. If using supernatant containing the cross-species specific bispecific single chain antibody molecules, 21 two- and 20 threefold dilutions thereof were applied for the macaque and the human cytotoxicity assay, respectively. The assay time was 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gamma counter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program were used for comparison of bioactivity.

As shown in Figures 13 to 17 and 40, all of the generated cross-species specific bispecific single chain antibody constructs demonstrate cytotoxic activity against human MCSP D3 positive target cells elicited by stimulated human CD4/CD56 depleted PBMC or stimulated PBMC and against macaque MCSP D3 positive target cells elicited by the macaque T cell line 4119LnPx.

**12. Plasma stability of MCSP and CD3 cross-species specific bispecific single chain antibodies**

Stability of the generated bispecific single chain antibodies in human plasma was analyzed by incubation of the bispecific single chain antibodies in 50% human Plasma at 37°C and 4°C for 24 hours and subsequent testing of bioactivity.
Bioactivity was studied in a chromium 51 ($^{51}Cr$) release in vitro cytotoxicity assay using a MCSP positive CHO cell line (expressing MCSP as cloned according to example 14 or 15) as target and stimulated human CD8 positive T cells as effector cells.
$EC_{50}$ values calculated by the analysis program as described above were used for comparison of bioactivity of bispecific single chain antibodies incubated with 50% human plasma for 24 hours at 37°C and 4°C respectively with bispecific single chain antibodies without addition of plasma or mixed with the same amount of plasma immediately prior to the assay.
As shown in Figure 18 and Table 2 the bioactivity of the G4 H-L x I2C H-L, G4 H-L x H2C H-L and G4 H-L x F12Q H-L bispecific antibodies was not significantly reduced as compared with the controls without the addition of plasma or with addition of plasma immediately before testing of bioactivity.

Table 2: bioactivity of the bispecific antibodies without or with the addition of Plasma

| Construct | Without plasma | With plasma | Plasma 37°C | Plasma 4°C |
|---|---|---|---|---|
| G4 H-L x I2C H-L | 300 | 796 | 902 | 867 |
| G4 H-L x H2C H-L | 496 | 575 | 2363 | 1449 |
| G4 H-L x F12Q H-L | 493 | 358 | 1521 | 1040 |

**13. Redistribution of circulating T cells in the absence of circulating target cells by first exposure to CD3 binding molecules directed at conventional i.e. context dependent CD3 epitopes is a major risk factor for adverse events related to the initiation of treatment**

**T cell redistribution in patients with B-cell Non-Hodgkin-Lymphoma (B-NHL) following initiation of treatment with the conventional CD3 binding molecule**

A conventional CD19xCD3 binding molecules is a CD3 binding molecule of the bispecific tandem scFv format (Loffler (2000, Blood, Volume 95, Number 6) or WO 99/54440). It consists of two different binding portions directed at (i) CD19 on the surface of normal and malignant human B cells and (ii) CD3 on human T cells. By crosslinking CD3 on T cells with CD19 on B cells, this construct triggers the redirected lysis of normal and malignant B cells by the cytotoxic activity of T cells.

The CD3 epitope recognized by such a conventional CD3 binding molecule is localized on the CD3 epsilon chain, where it only takes the correct conformation if it is embedded within the rest of the epsilon chain and held in the right position by heterodimerization of the epsilon chain with either the CD3 gamma or delta chain. Interaction of this highly context dependent epitope with a conventional CD3 binding molecule (see e.g. Loffler (2000, Blood, Volume 95, Number 6) or WO 99/54440) - even when it occurs in a purely monovalent fashion and without any crosslinking - can induce an allosteric change in the conformation of CD3 leading to the exposure of an otherwise hidden proline-rich region within the cytoplasmic domain of CD3 epsilon. Once exposed, the proline-rich region can recruit the signal transduction molecule Nck2, which is capable of triggering further intracellular signals. Although this is not sufficient for full T cell activation, which definitely requires crosslinking of several CD3 molecules on the T cell surface, e.g. by crosslinking of several anti-CD3 molecules bound to several CD3 molecules on a T cell by several CD19 molecules on the surface of a B cell, pure monovalent interaction of conventional CD3 binding molecules to their context dependent epitope on CD3 epsilon is still not inert for T cells in terms of signalling. Without being bound by theory, monovalent conventional CD3 binding molecules (known in the art) may induce some T cell reactions when infused into humans even in those cases where no circulating target cells are available for CD3 crosslinking. An important T cell reaction to the intravenous infusion of monovalent conventional CD19xCD3 binding molecule into B-NHL patients who have essentially no circulating CD19-positive B cells is the redistribution of T cells after start of treatment. It has been found in a phase I clinical trial that this T cell reaction occurs during the starting phase of intravenous CD19xCD3 binding molecule infusion in all individuals without circulating CD19-positive target B cells essentially independent of the CD19xCD3 binding molecule dose (Fig. 19). However, sudden increases in CD19xCD3 binding molecule exposure have been found to trigger virtually the same redistributional T cell reaction in these patients as the initial exposure of T cells to CD19xCD3 binding molecule at treatment start (Fig. 20 A) and even gradual increases in CD19xCD3 binding molecule exposure still can have redistributional effects on circulating T cells (Fig. 21). Moreover, it has been found that this essentially dose-independent redistributional T cell reaction in the absence of circulating target cells as triggered by conventional CD3 binding molecules like the CD19xCD3 binding molecule (e.g. disclosed in WO 99/54440) in 100% of all treated individuals is a major risk factor for adverse events related to the initiation of treatment.
According to the study protocol, patients with relapsed histologically confirmed indolent B-cell Non-Hodgkin-Lymphoma (B-NHL) including mantle cell lymphoma were recruited in an open-label, multi-center phase I interpatient dose-escalation trial.
The study protocol was approved by the independent ethics committees of all participating centers and sent for notification to the responsible regulatory authority.
Measurable disease (at least one lesion ≥ 1.5 cm) as documented by CT scan was required for inclusion into the study. Patients received conventional CD19xCD3 binding molecule by continuous intravenous infusion with a portable minipump system over four weeks at constant flow rate (i.e. dose level). Patients were hospitalized during the first two weeks of treatment before they were released from the hospital and continued treatment at home. Patients without evidence of disease progression after four weeks were offered to continue treatment for further four weeks. So far six different dose levels were tested without reaching a maximum tolerated dose (MTD): 0.5, 1.5, 5, 15, 30 and 60 $\mu g/m^2/24h$. Cohorts consisted of three patients each if no adverse events defined by the study protocol as DLT (dose limiting toxicity) were observed. In case of one DLT among the first three patients the cohort was expanded to six patients, which - in the absence of a second DLT - allowed further dose escalation. Accordingly, dose levels without DLT in cohorts with 3 patients or with one DLT in cohorts with 6 patients were regarded as safe. Study treatment was stopped in all patients who developed a DLT. At 15 and 30 $\mu g/m^2/24h$ different modes of treatment initiation during the first 24h were tested in several additional cohorts: (i) Stepwise increase after 5 $\mu g/m^2/24h$ for the first 24h to 15 $\mu g/m^2/24h$ maintenance dose (patient cohort 15-step), (ii) even continuous increase of flow-rate from almost zero to 15 or 30 $\mu g/m^2/24h$ (patient cohorts 15-ramp and 30-ramp) and (iii) start with the maintenance dose from the very beginning (patient cohorts 15-flat, 30-flat and 60-flat). Patient cohorts at dose levels 0.5, 1.5 and 5 $\mu g/m^2/24h$ were all started with the maintenance dose from the very beginning (i.e. flat initiation).
Time courses of absolute B- and T-cell counts in peripheral blood were determined by four color FACS analysis as follows:

**Collection of blood samples and routine analysis**

In patient cohorts 15-ramp, 15-flat, 30-ramp, 30-flat and 60-flat blood samples (6 ml) were obtained before and 0.75, 2, 6, 12, 24, 30, 48 hours after start of CD19xCD3 binding molecule (as disclosed in WO 99/54440) infusion as well as on treatment days 8, 15, 17, 22, 24, 29, 36, 43, 50, 57 and 4 weeks after end of conventional CD19xCD3 binding molecule infusion using EDTA-containing Vacutainer™ tubes (Becton Dickinson) which were shipped for analysis at 4°C. In patient cohorts 15-step blood samples (6 ml) were obtained before and 6, 24, 30, 48 hours after start of CD19xCD3 binding molecule infusion as well as on treatment days 8, 15, 22, 29, 36, 43, 50, 57 and 4 weeks after end of CD19xCD3 binding molecule infusion. At dose levels 0.5, 1.5 and 5 $\mu g/m^2/24h$ blood samples (6 ml) were obtained before and 6, 24, 48 hours after start of CD19xCD3 binding molecule infusion as well as on treatment days 8, 15, 22, 29, 36, 43, 50, 57 and 4 weeks after end of CD19xCD3 binding molecule infusion. In some cases slight variations of these time points occurred for operational reasons. FACS analysis of lymphocyte subpopulations was performed within 24 - 48 h after blood sample collection. Absolute numbers of leukocyte subpopulations in the blood samples were determined through differential blood analysis on a CoulterCounter™ (Coulter).

**Isolation of PBMC from blood samples**

PBMC (peripheral blood mononuclear cells) isolation was performed by an adapted Ficoll™ gradient separation protocol. Blood was transferred at room temperature into 10 ml Leucosep™ tubes (Greiner) pre-loaded with 3 ml Biocoll™ solution (Biochrom). Centrifugation was carried out in a swing-out rotor for 15 min at 1700xg and 22°C without deceleration. The PBMC above the Biocoll™ layer were isolated, washed once with FACS buffer (PBS / 2% FBS [Foetal Bovine Serum; Biochrom]), centrifuged and resuspended in FACS buffer. Centrifugation during all wash steps was carried out in a swing-out rotor for 4 min at 800xg and 4°C. If necessary, lysis of erythrocytes was performed by incubating the isolated PBMC in 3 ml erythrocyte lysis buffer (8.29 g $NH_4Cl$, 1.00 g $KHCO_3$, 0.037 g EDTA, ad 1.0 l $H_2O_{bidest}$, pH 7.5) for 5 min at room temperature followed by a washing step with FACS buffer.

**Staining of PBMC with fluorescence-labeled antibodies against cell surface molecules**

Monoclonal antibodies were obtained from Invitrogen ([1]Cat. No. MHCD1301, [2]Cat. No. MHCD1401), Dako ([5]Cat. No. C7224) or Becton Dickinson ([3]Cat. No. 555516, [4]Cat. No. 345766) used according to the manufacturers' recommendations. $5x10^5$ - $1x10^6$ cells were stained with the following antibody combination: anti-CD13[1] / anti- CD14[2] (FITC) x anti-CD56[3] (PE) x anti-CD3[4] (PerCP) x anti-CD19[5] (APC). Cells were pelleted in V-shaped 96 well multititer plates (Greiner) and the supernatant was removed. Cell pellets were resuspended in a total volume of 100 μl containing the specific antibodies diluted in FACS buffer. Incubation was carried out in the dark for 30 min at 4°C. Subsequently, samples were washed twice with FACS buffer and cell pellets were resuspended in FACS buffer for flowcytometric analysis.

**Flowcytometric detection of stained lymphocytes by FACS**

Data collection was performed with a 4 color BD FACSCalibur™ (Becton Dickinson). For each measurement $1x10^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding any myeloid cells via CD13/14-staining) as determined by FACS were correlated with the lymphocyte count from the differential blood analysis to calculate absolute cell numbers of T cells ($CD3^+$, $CD56^-$, $CD13/14^-$) and B cells ($CD19^+$, $CD13/14^-$).

T cell redistribution during the starting phase of conventional CD19xCD3 binding molecule (e.g. disclosed in WO 99/54440) treatment in all those patients who had essentially no circulating CD19-positive B cells at treatment start is shown in (Fig. 19). For comparison, a representative example of T cell redistribution during the starting phase of CD19xCD3 binding molecule treatment in a patient with a significant number of circulating CD19-positive B cells is shown in Fig. 22.

In both cases (i.e. essentially no or many circulating B cells) circulating T cell counts rapidly decrease upon treatment start. However, in the absence of circulating B cells T cells tend to return into the circulating blood very early, while the return of T cells into the circulating blood of those patients who have a significant number of circulating B cells at treatment start is usually delayed until these circulating B cells are depleted. Thus, the T cell redistribution patterns mainly differ in the kinetics of T cell reappearance in the circulating blood.

Assessment of efficacy based on CT scan was carried out by central reference radiology after 4 weeks of treatment and in patients receiving additional 4 weeks also after 8 weeks of treatment plus in all cases four weeks after end of treatment.

Disappearance and/or normalization in size of all known lesions (including an enlarged spleen) plus clearance of bone marrow from lymphoma cells in cases of bone marrow infiltration was counted as complete response (CR). Reduction by at least 50% from baseline of the sum of products of the two biggest diameters (SPD) of each predefined target lesion was defined as partial response (PR); a reduction by at least 25% was regarded a minimal response (MR). Progressive disease (PD) was defined as ≥ 50% increase of SPD from baseline. SPD deviations from baseline between +50% and -25% were regarded as stable disease (SD).

Patient demographics, doses received and clinical outcome in 34 patients are summarized in Table 3. Clinical anti-tumor activity of the CD19xCD3 binding molecule was clearly dose dependent: Consistent depletion of circulating CD19-positive B (lymphoma) cell from peripheral blood was observed from 5 $\mu g/m^2/24h$ onwards. At 15 $\mu g/m^2/24h$ and 30 $\mu g/m^2/24h$ first objective clinical responses (PRs and CRs) were recorded as well as cases of partial and complete elimination of B lymphoma cells from infiltrated bone marrow. Finally, at 60 $\mu g/m^2/24h$ the response rate increased to 100% (PRs and CRs) and bone marrow clearance from B lymphoma cells was complete in all evaluable cases.

The CD19xCD3 binding molecule was well tolerated by the majority of patients. Most frequent adverse events of grades 1-4 in 34 patients, regardless of causality are summarized in Table 4. CD19xCD3 binding molecule-related adverse events usually were transient and fully reversible. In particular, there were 2 patients (patients # 19 and # 24 in Table 3) essentially without circulating CD19-positive B cells whose treatment was stopped early because of CNS adverse events (lead symptoms: confusion and disorientation) related to repeated T cell redistribution during the starting phase of CD19xCD3 binding molecule infusion.

One of these patients (#19) was in cohort 15-step. He received 5 $\mu g/m^2/24h$ CD19xCD3 binding molecule for the first 24h followed by sudden increase to 15 $\mu g/m^2/24h$ maintenance dose. The corresponding T cell redistribution pattern shows that circulating T cell counts rapidly decreased upon start of infusion at 5 $\mu g/m^2/24h$ followed by early reappearance of T cells in the circulating blood essentially without circulating CD19-positive B cells. As a consequence, the peripheral T cell counts had fully recovered when the CD19xCD3 binding molecule dose was increased after 24h from 5 to 15 $\mu g/m^2/24h$. Therefore the dose step could trigger a second episode of T cell redistribution as shown in Fig. 20A. This repeated T cell redistribution was related with CNS side effects (lead symptoms: confusion and disorientation) in this patient, which led to the stop of infusion. The relationship between repeated T cell redistribution and such CNS adverse events was also observed in previous phase I clinical trials in B-NHL patients who received CD19xCD3 binding molecule (e.g. disclosed in WO 99/54440) as repeated bolus infusion for 2 to 4 hours each usually followed by 2 days of treatment free interval (Fig. 20 B). Every single bolus infusion triggered one episode of T cell redistribution consisting of a fast decrease in circulating T cell counts and T cell recovery prior to the next bolus infusion. In total, CNS adverse events related to repeated T cell redistribution were observed in 5 out of 21 patients. Fig. 20B shows the representative example of one patient from the bolus infusion trials, who developed CNS symptoms after the third episode of T cell redistribution. Typically, patients with CNS adverse events in the bolus infusion trials also had low circulating B cell counts.

The second patient (#24) from the continuous infusion trial, whose treatment was stopped early because of CNS adverse events (lead symptoms: confusion and disorientation) related to repeated T cell redistribution during the starting phase of CD19xCD3 binding molecule infusion, was in cohort 15-flat. By mistake, this patient received an CD19xCD3 binding molecule infusion without additional HSA as required for stabilization of the drug. The resulting uneven drug flow triggered repeated episodes of T cell redistribution instead of only one (Fig. 23A) with the consequence that the infusion had to be stopped because of developing CNS symptoms. Yet, when the same patient was restarted correctly with CD19xCD3 binding molecule solution containing additional HSA for drug stabilization (e.g. disclosed in WO 99/54440), no repeated T cell redistribution was observed and the patient did not again develop any CNS symptoms (Fig. 23B). Because this patient also had essentially no circulating B cells, the circulating T cells could react with fast redistribution kinetics even to subtle changes in drug exposure as observed. The CNS adverse events related to T cell redistribution in patients who have essentially no circulating target cells can be explained by a transient increase of T cell adhesiveness to the endothelial cells followed by massive simultaneous adhesion of circulating T cells to the blood vessel walls with a consecutive drop of T cell numbers in the circulating blood as observed. The massive simultaneous attachment of T cells to the blood vessel walls can cause an increase in endothelial permeability and endothelial cell activation. The consequences of increased endothelial permeability are fluid shifts from the intravascular compartment into interstitial tissue compartments including the CNS interstitium. Endothelial cell activation by attached T cells can have procoagulatory effects (Monaco et al. J Leukoc Biol 71 (2002) 659-668) with possible disturbances in blood flow (including cerebral blood flow) particularly with regard to capillary microcirculation. Thus, CNS adverse events related to T cell redistribution in patients essentially without circulating target cells can be the consequence of capillary leak and/or disturbances in capillary microcirculation through adherence of T cells to endothelial cells. The endothelial stress caused by one episode of T cell redistribution is tolerated by the majority of patients, while the enhanced endothelial stress caused by repeated T cell redistribution frequently causes CNS adverse events. More than one episode of T cell redistribution may be less risky only in patients who have low baseline counts of circulating T cells. However, also the limited endothelial stress caused by one episode of T cell redistribution can cause CNS adverse events in rare cases of increased susceptibility for such events as observed in 1 out of 21 patients in the bolus infusion trials with the CD19xCD3 binding molecule.

Without being bound by theory, the transient increase of T cell adhesiveness to the endothelial cells in patients who have essentially no circulating target cells can be explained as T cell reaction to the monovalent interaction of a conventional CD3 binding molecule, like the CD19xCD3 binding molecule (e.g. WO 99/54440), to its context dependent epitope on CD3 epsilon resulting in an allosteric change in the conformation of CD3 followed by the recruitment of Nck2 to the cytoplasmic domain of CD3 epsilon as described above. As Nck2 is directly linked to integrins via PINCH and ILK (Fig 28), recruitment of Nck2 to the cytoplasmic domain of CD3 epsilon following an allosteric change in the conformation of CD3 through binding of a conventional CD3 binding molecule, like the CD19xCD3 binding molecule, to its context dependent epitope on CD3 epsilon, can increase the adhesiveness of T cells to endothelial cells by transiently switching integrins on the T cell surface into their more adhesive isoform via inside-out-signalling.

Table 3. Patient demographics and clinical outcome

| Cohort | Patient | Age/ Sex | Disease (Ann Arbor Classification) | Dose Level [$mg/m^2$/Day] | Clearance of Bone Marrow | Best Response* (CR Duration in Months or Weeks) |
|---|---|---|---|---|---|---|
| 1 | 1 | 71/m | IC, Binet C | 0.0005 | None | SD |
| | 2 | 67/f | MCL, Stage IV/A/E | 0.0005 | n.d. | PD |
| | 3 | 67/m | CLL, Stage IV/B/E | 0.0005 | n.d. | MR |
| 2 | 4 | 69/m | MCL, Stage IV/B | 0.0015 | n.i. | SD |
| | 5 | 49/m | MCL, Stage IV/A/S | 0.0015 | n.d. | SD |
| | 6 | 71/m | MCL, Stage IV/B/E | 0.0015 | n.i. | PD |

(continued)

| Cohort | Patient | Age/ Sex | Disease (Ann Arbor Classification) | Dose Level [mg/m²/Day] | Clearance of Bone Marrow | Best Response* (CR Duration in Months or Weeks) |
|---|---|---|---|---|---|---|
| | 7 | 77/m | MCL, Stage IV/B/E/S | 0.0015 | n.i. | SD |
| | 8 | 65/m | CLL, Stage IV/B/E/S | 0.0015 | n.d. | PD |
| | 9 | 75/m | FL, Stage II/B | 0.0015 | n.i. | SD |
| 3 | 10 | 58/m | MCL, Stage III/B/S | 0.005 | n.i. | PD |
| | 11 | 68/f | FL, Stage IV/B | 0.005 | n.d. | SD |
| | 12 | 65/m | MCL, Stage III/A/E | 0.005 | n.i. | SD |
| 4[a] | 13 | 60/m | SLL, Stage IV/B/S | 0.015 | Complete | **PR** |
| | 14 | 73/m | MCL, Stage II/A/E | 0.015 | n.i. | SD |
| | 15 | 44/m | FL, Stage IV/B/E/S | 0.015 | Partial | **PR** |
| | 16 | 61/m | FL, Stage IV/A/S | 0.015 | Complete | **CR** (7mo) |
| | 17 | 67/m | MZL, Stage IV/B/S | 0.015 | n.i. | n.e. |
| | 18 | 64/m | FL, Stage IV/A/E | 0.015 | n.i. | PD |
| | 19 | 75/m | MCL, Stage III/A | 0.015 | n.i. | n.e. |
| | 20 | 65/f | FL; Stage III/A | 0.015 | n.i. | SD |
| | 21 | 60/m | MCL, Stage IV/A/E | 0.015 | None | SD |
| | 22 | 67/f | FL, Stage IV/B | 0.015 | Complete | MR |
| | 23 | 67/m | DLBCL, Stage III/B | 0.015 | n.i. | n.e. |
| | 24 | 65/f | FL, Stage III/A | 0.015 | n.d. | SD |
| | 25 | 74/f | WD, Stage IV/B | 0.015 | Partial | SD |
| 5 | 26 | 67/m | MCL, Stage IV/A | 0.03 | Complete | SD |
| | 27 | 48/m | FL, Stage III/A | 0.03 | n.i. | PD |
| | 28 | 58/m | MCL, Stage III/A | 0.03 | n.i. | **CR** (10mo+) |
| | 29 | 45/f | MCL, Stage IV/B | 0.03 | Partial | PD |
| | 30 | 59/m | MZL, Stage III/A | 0.03 | n.i. | n.e. |
| | 31 | 43/m | FL, Stage III/A | 0.03 | n.i. | MR |
| 6 | 32 | 72/m | MCL, Stage IV/A | 0.06 | Complete | **PR** |
| | 33 | 55/m | MCL, Stage IV/B | 0.06 | Complete | **CR** (4mo+) |
| | 34 | 52/m | FL, Stage IV/A | 0.06 | n.i. | **CR**[b] (1w+) |

*centrally confirmed complete (CR) and partial (PR) responses by Cheson criteria in bold; MR, minimal response (≥25 to <50%); SD, stable disease; PD, progressive disease; duration from first documentation of response in parentheses; + denotes an ongoing response

[a]Cohort 4 was expanded to study three different schedules of treatment initiation

[b]PR after 8 weeks of treatment that turned into a CR after an additional treatment cycle of 4 weeks at the same dose following 7 weeks of treatment free interval n.e.: not evaluable, because of treatment period <7 d n.d.: not determined (infiltrated, but no second biopsy performed at end of treatment) n.i.: not infiltrated at start of treatment

Table 4. Incidence of adverse events observed during treatment

| Adverse events regardless of relationship, occuring in ≥ 3 patients (N=34) | Grade 1-4 N (%) | Grade 3-4 N (%) |
|---|---|---|
| Pyrexia | 22 (64.7) | 2 (5.9) |
| Leukopenia | 21 (61.8) | 11 (32.4) |
| Lymphopenia | 21 (61.8) | 21 (61.8) |
| Coagulopathy (increase in D-dimers) | 16 (47.1) | 6 (17.6) |
| Enzyme abnormality (AP, LDH, CRP) | 16 (47.1) | 10 (29.4) |
| Hepatic function abnormality (ALT, AST, GGT) | 16 (47.1) | 1 (2.9) |
| Anaemia | 13 (38.2) | 5 (14.7) |
| Chills | 13 (38.2) | 0 (0.0) |
| Headache | 12 (35.3) | 1 (2.9) |
| Hypokalaemia | 12 (35.3) | 2 (5.9) |
| Thrombocytopenia | 12 (35.3) | 6 (17.6) |
| Weight increased | 12 (35.3) | 0 (0.0) |
| Hyperglycaemia | 11 (32.4) | 2 (5.9) |
| Neutropenia | 11 (32.4) | 8 (23.5) |
| Haematuria | 10 (29.4) | 0 (0.0) |
| Oedema peripheral | 10 (29.4) | 2 (5.9) |
| Anorexia | 9 (26.5) | 1 (2.9) |
| Diarrhoea | 9 (26.5) | 0 (0.0) |
| Weight decreased | 9 (26.5) | 0 (0.0) |
| Fatigue | 8 (23.5) | 1 (2.9) |
| Proteinuria | 8 (23.5) | 0 (0.0) |
| Hypocalcaemia | 7 (20.6) | 2 (5.9) |
| Pancreatic enzyme abnormality | 7 (20.6) | 0 (0.0) |
| Cough | 6 (17.6) | 0 (0.0) |
| Dyspnoea | 6 (17.6) | 0 (0.0) |
| Back pain | 5 (14.7) | 0 (0.0) |
| Catheter site pain | 5 (14.7) | 0 (0.0) |
| Hyperbilirubinaemia | 5 (14.7) | 2 (5.9) |
| Hypoalbuminaemia | 5 (14.7) | 0 (0.0) |
| Hypogammaglobulinaemia | 5 (14.7) | 1 (2.9) |
| Hypoproteinaemia | 5 (14.7) | 0 (0.0) |
| Pleural effusion | 5 (14.7) | 1 (2.9) |
| Vomiting | 5 (14.7) | 0 (0.0) |
| Asthenia | 4 (11.8) | 1 (2.9) |
| Confusional state | 4 (11.8) | 0 (0.0) |
| Constipation | 4 (11.8) | 0 (0.0) |
| Dizziness | 4 (11.8) | 0 (0.0) |

(continued)

| Adverse events regardless of relationship, occuring in ≥ 3 patients (N=34) | Grade 1-4 N (%) | Grade 3-4 N (%) |
|---|---|---|
| Hypertension | 4 (11.8) | 0 (0.0) |
| Hyponatraemia | 4 (11.8) | 2 (5.9) |
| Mucosal dryness | 4 (11.8) | 0 (0.0) |
| Muscle spasms | 4 (11.8) | 0 (0.0) |
| Nausea | 4 (11.8) | 0 (0.0) |
| Night sweats | 4 (11.8) | 0 (0.0) |
| Abdominal pain | 3 (8.8) | 1 (2.9) |
| Ascites | 3 (8.8) | 0 (0.0) |
| Hypercoagulation | 3 (8.8) | 0 (0.0) |
| Hyperhidrosis | 3 (8.8) | 0 (0.0) |
| Hypoglobulinaemia | 3 (8.8) | 0 (0.0) |
| Insomnia | 3 (8.8) | 0 (0.0) |
| Liver disorder | 3 (8.8) | 1 (2.9) |
| Nasopharyngitis | 3 (8.8) | 0 (0.0) |
| Pruritus | 3 (8.8) | 0 (0.0) |

Abbreviations used are: AE, adverse event; AP, alkaline phosphatase; LDH, lactate dehydrogenase; CRP, C-reactive protein; ALT, alanine transaminase; AST, aspartate transaminase; GGT, gamma-glutamyl transferase; AE data from the additional treatment cycle of patient 34 not yet included.

As explained above, conventional CD3 binding molecules (e.g. disclosed in WO 99/54440) capable of binding to a context-dependent epitope, though functional, lead to the undesired effect of T cell redistribution in patients causing CNS adverse events. In contrast, binding molecules of the present invention, by binding to the context-independent N-terminal 1-27 amino acids of the CD3 epsilon chain, do not lead to such T cell redistribution effects. As a consequence, the CD3 binding molecules of the invention are associated with a better safety profile compared to conventional CD3 binding molecules.

**14. Bispecific CD3 binding molecules of the invention inducing T cell mediated target cell lysis by recognizing a surface target antigen deplete target antigen positive cells in vivo**

**A bispecific CD3 binding molecule of the invention recognizing CD33 as target antigen depletes CD33-positive circulating monocytes from the peripheral blood of cynomolgus monkeys**

CD33-AF5 VH-VL x I2C VH-VL (amino acid sequence: SEQ ID NO.267) was produced by expression in CHO cells using the coding nucleotide sequence SEQ ID NO. 268. The coding sequences of (i) an N-terminal immunoglobulin heavy chain leader comprising a start codon embedded within a Kozak consensus sequence and (ii) a C-terminal $His_6$-tag followed by a stop codon were both attached in frame to the nucleotide sequence SEQ ID NO 268 prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the CD3 binding molecule CD33-AF5 VH-VL x I2C VH-VL into the culture supernatant and gene amplification with methotrexat for increasing expression levels were carried out as described (Mack et al. Proc. Natl. Acad. Sci. USA 92 (1995) 7021-7025). The analytical SEC-profile of CD33-AF5 VH-VL x I2C VH-VL for use in cynomolgus monkeys revealed that the test material almost exclusively consisted of monomer. The potency of the test material was measured in a cytotoxicity assay as described in example 16.5 using CHO cells transfected with cynomolgus CD33 as target cells and the macaque T cell line 4119LnPx as source of effector cells (FIG. 25). The concentration of CD33-AF5 VH-VL x I2C VH-VL required for half-maximal target cell lysis by the effector T cells (EC50) was determined to be 2.7 ng/ml.

Young (approx. 3 years old) adult cynomolgus monkeys (Macaca fascicularis) were treated by continuous intravenous infusion of CD3 binding molecule CD33-AF5 VH-VL x I2C VH-VL at different flow-rates (i.e. dose levels) to study depletion of circulating CD33-positive monocytes from the peripheral blood. This situation is equivalent to the treatment with the conventional CD3 binding molecule CD19xCD3 (specific for CD19 on B cells and CD3 on T cells) of those B-NHL patients, who have circulating CD19-positive target B cells (see e.g. WO99/54440). Depletion of circulating CD19-positive target B cells from the peripheral blood had turned out as a valid surrogate for the general clinical efficacy of the conventional CD3 binding molecule (CD19xCD3 as provided in WO99/54440) in patients with CD19-positive B-cell malignomas like B-NHL. Likewise, depletion of circulating CD33-positive monocytes from the peripheral blood is regarded as a valid surrogate of the general clinical efficacy of CD33-directed bispecific CD3 binding molecules of the invention like CD33-AF5 VH-VL x I2C VH-VL in patients with CD33-positive myeloid malignomas like AML (acute myeloid leukemia).
Continuous infusion was carried out according to the Swivel method as follows: The monkeys are catheterized via the vena femoralis into the vena cava caudalis using a vein catheter. The catheter is tunneled subcutaneously to the dorsal shoulder region and exteriorized at the caudal scapula. Then a tube is passed through a jacket and a protection spring. The jacket is fastened around the animal and the catheter, via the tube, is connected to an infusion pump.
Administration solution (1.25 M lysine, 0.1 % tween 80, pH 7) without test material was infused continuously at 48 ml/24h for 7 days prior to treatment start to allow acclimatization of the animals to the infusion conditions. Treatment was started by adding CD33-AF5 VH-VL x I2C VH-VL test material to the administration solution at the amount required for each individual dose level to be tested (i.e. flow rate of CD33-AF5 VH-VL x I2C VH-VL). The infusion reservoir was changed every day throughout the whole acclimatization and treatment phase. Planned treatment duration was 7 days except for the 120μg/m$^2$/24h dose level, where animals received 14 days of treatment.
Time courses of absolute counts in circulating T cells and CD33-positive monocytes were determined by 4- or 3-colour FACS analysis, respectively:

**Collection of blood samples and routine analysis**

Blood samples (1 ml) were obtained before and 0.75, 2, 6, 12, 24, 30, 48, 72 hours after start of continuous infusion with MCSP-G4 VH-VL x I2C VH-VL as well as after 7 and 14 days (and after 9 days at the 120 μg/m$^2$/24h dose level) of treatment using EDTA-containing Vacutainer™ tubes (Becton Dickinson) which were shipped for analysis at 4°C. In some cases slight variations of these time points occurred for operational reasons. FACS analysis of lymphocyte subpopulations was performed within 24 - 48 h after blood sample collection. Absolute numbers of leukocyte subpopulations in the blood samples were determined through differential blood analysis in a routine veterinary lab.

**Isolation of PBMC from blood samples**

PBMC (peripheral blood mononuclear cells) were isolated in analogy to the protocol described in example 13, above, with adaptations of the used volumes.

**Staining of PBMC with fluorescence-labeled antibodies against cell surface molecules**

Monoclonal antibodies reactive with cynomolgus antigens were obtained from Becton Dickinson ([1]Cat. No. 345784, [2]Cat. No. 556647, [3]Cat. No. 552851, [6]Cat. No. 557710), Beckman Coulter ([4]Cat. No. IM2470) and Miltenyi ([5]Cat. No. 130-091-732) and used according to the manufacturers' recommendations. 5x10$^5$ - 1x10$^6$ cells were stained with the following antibody combinations: anti-CD14[1] (FITC) x anti-CD56[2] (PE) x anti-CD3[3] (PerCP) x anti-CD19[4] (APC) and anti-CD14[1] (FITC) x anti-CD33[5] (PE) x anti-CD16[6] (Alexa Fluor 647™). Additional steps were performed as described in example 13, above.

**Flowcytometric detection of stained lymphocytes by FACS**

Data collection was performed with a 4 color BD FACSCalibur™ (Becton Dickinson).
For each measurement 1x10$^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding myeloid cells via CD14-staining) as determined by FACS were correlated with the lymphocyte count from the differential blood analysis to calculate absolute cell numbers of T cells (CD3$^+$, CD56$^-$, CD14$^-$). Absolute numbers of CD33-positive monocytes were calculated by multiplying the monocyte counts from the differential blood analysis with the corresponding ratios of CD33-positive monocytes (CD33$^+$, CD14$^+$) to all monocytes (CD14$^+$) as determined by FACS.
The percentage compared to baseline (i.e. 100%) of absolute circulating CD33-positive monocyte counts at the end of treatment with CD33-AF5 VH-VL x I2C VH-VL in 4 cohorts of 2 cynomolgus monkeys with inter-cohort dose escalation from 30 over 60 and 240 to 1000 μg/m$^2$/24h are shown in FIG 26A.

As shown in FIG 26A, continuous intravenous infusion of CD33-AF5 VH-VL x I2C VH-VL induces depletion of circulating CD33-positive monocytes in a dose-dependent manner. While there was still no detectable depletion of circulating CD33-positive monocytes at 30 $\mu g/m^2/24h$, a first trend towards a reduction of CD33-positive monocyte counts became visible at 60 $\mu g/m^2/24h$ after 7 days of treatment.
At 240 $\mu g/m^2/24h$ circulating CD33-positive monocytes were almost completely depleted from the peripheral blood after 3 days of treatment. This was reached even faster at 1000 $\mu g/m^2/24h$, where depletion of the circulating CD33-positive monocytes from the peripheral blood was completed already after 1 day of treatment.
This finding was confirmed by the results shown in Figure 26B demonstrating depletion of circulating CD33-positive monocytes by two thirds and 50% compared to the respective baseline in two cynomolgus monkeys treated by continuous infusion with CD33-AF5 VH-VL x I2C VH-VL at 120 $\mu g/m^2/24h$ for 14 days.
This outcome is a clear signal clinical efficacy of the CD3 binding molecules of the invention in general and of bispecific CD33-directed CD3 binding molecules of the invention for the treatment of CD33-positive malignomas like AML in particularly.
Moreover, the T cell redistribution during the starting phase of treatment with CD33-AF5 VH-VL x I2C VH-VL in the presence of circulating target cells (i.e. CD33-positive monocytes) seems to be less pronounced than T cell redistribution during the starting phase of treatment with conventional CD19xCD3 constructs, as described in WO99/54440 in B-NHL patients with a significant number of circulating target cells (i.e. CD19-positive B cells) as shown in Fig 22. While T cells disappear completely from the circulation upon start of CD19xCD3 infusion and do not reappear until the circulating CD19-positive target B cells are depleted from the peripheral blood (FIG 22), initial disappearance of circulating T cells is incomplete upon infusion start with CD33-AF5 VH-VL x I2C VH-VL and T cell counts recover still during the presence of circulating CD33-positive target cells (FIG 26B). This confirms that CD3 binding molecules of the invention (directed against and generated against an epitope of human and non-chimpanzee primates CD3ε (epsilon) chain and being a part or fragment or the full length of the amino acid sequence as provided in SEQ ID Nos. 2, 4, 6, or 8 as defined in the claims) by recognizing a context-independent CD3 epitope show a more favorable T cell redistribution profile than conventional CD3 binding molecules recognizing a context-dependent CD3 epitope, like the binding molecules provided in WO99/54440.

### 15. CD3 binding molecules of the invention directed at essentially context independent CD3 epitopes by inducing less redistribution of circulating T cells in the absence of circulating target cells reduce the risk of adverse events related to the initiation of treatment

#### Reduced T cell redistribution in cynomolgus monkeys following initiation of treatment with a representative cross-species specific CD3 binding molecule of the invention

MCSP-G4 VH-VL x I2C VH-VL (amino acid sequence: SEQ ID NO. 193) was produced by expression in CHO cells using the coding nucleotide sequence SEQ ID NO. 194. The coding sequences of (i) an N-terminal immunoglobulin heavy chain leader comprising a start codon embedded within a Kozak consensus sequence and (ii) a C-terminal His6-tag followed by a stop codon were both attached in frame to the nucleotide sequence SEQ ID NO. 194 prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the CD3 binding molecule MCSP-G4 VH-VL x I2C VH-VL into the culture supernatant and gene amplification with methotrexat for increasing expression levels were carried out as described (Mack et al. Proc. Natl. Acad. Sci. USA 92 (1995) 7021-7025). Test material for treatment of cynomolgus monkeys was produced in a 200-liter fermenter. Protein purification from the harvest was based on IMAC affinity chromatography targeting the C-terminal His6-tag of MCSP-G4 VH-VL x I2C VH-VL followed by preparative size exclusion chromatography (SEC). The total yield of final endotoxin-free test material was 40 mg. The test material consisted of 70 % monomer, 30% dimer and a small contamination of higher multimer. The potency of the test material was measured in a cytotoxicity assay as described in example 11 using CHO cells transfected with cynomolgus MCSP as target cells and the macaque T cell line 4119LnPx as source of effector cells (FIG 27). The concentration of MCSP-G4 VH-VL x I2C VH-VL required for half-maximal target cell lysis by the effector T cells (EC50) was determined to be 1.9 ng/ml.
Young (approx. 3 years old) adult cynomolgus monkeys (Macaca fascicularis) were treated by continuous intravenous infusion of CD3 binding molecule MCSP-G4 VH-VL x I2C VH-VL at different flow-rates (i.e. dose levels) to study redistribution of circulating T cells following initiation of treatment in the absence of circulating target cells. Although the CD3 binding molecule MCSP-G4 VH-VL x I2C VH-VL can recognize both cynomolgus MCSP and cynomolgus CD3, there are no circulating blood cells expressing MCSP. Therefore, the only interaction possible in the circulating blood is binding of the CD3-specific arm of MCSP-G4 VH-VL x I2C VH-VL to CD3 on T cells. This situation is equivalent to the treatment with the conventional CD3 binding molecule (CD19xCD3 binding molecule specific for CD19 on B cells and CD3 on T cells) of those B-NHL patients, who have no circulating CD19-positive target B cells as described in example 13.

Continuous infusion was carried out according to the Swivel method as follows: The monkeys are catheterized via the vena femoralis into the vena cava caudalis using a vein catheter. The catheter is tunneled subcutaneously to the dorsal shoulder region and exteriorized at the caudal scapula. Then a tube is passed through a jacket and a protection spring. The jacket is fastened around the animal and the catheter, via the tube, is connected to an infusion pump.
Administration solution (1.25 M lysine, 0.1 % tween 80, pH 7) without test material was infused continuously at 48 ml/24h for 7 days prior to treatment start to allow acclimatization of the animals to the infusion conditions. Treatment was started by adding MCSP-G4 VH-VL x I2C VH-VL test material to the administration solution at the amount required for each individual dose level to be tested (i.e. flow rate of MCSP-G4 VH-VL x I2C VH-VL). The infusion reservoir was changed every day throughout the whole acclimatization and treatment phase. Treatment duration was 7 days.
Time courses of absolute T-cell counts in peripheral blood were determined by four color FACS analysis as follows:

**Collection of blood samples and routine analysis**

Blood samples (1 ml) were obtained before and 0.75, 2, 6, 12, 24, 30, 48, 72 hours after start of continuous infusion with MCSP-G4 VH-VL x I2C VH-VL as well as after 7 days of treatment using EDTA-containing Vacutainer™ tubes (Becton Dickinson) which were shipped for analysis at 4°C. In some cases slight variations of these time points occurred for operational reasons. FACS analysis of lymphocyte subpopulations was performed within 24 - 48 h after blood sample collection.
Absolute numbers of leukocyte subpopulations in the blood samples were determined through differential blood analysis in a routine veterinary lab.

**Isolation of PBMC from blood samples**

PBMC were isolated in analogy to the protocol described in example 13, above, with adaptations of the used volumes.

**Staining of PBMC with fluorescence-labeled antibodies against cell surface molecules**

Monoclonal antibodies reactive with cynomolgus antigens were obtained from Becton Dickinson ([1]Cat. No. 345784, [2]Cat. No. 556647, [3]Cat. No. 552851) and Beckman Coulter ([4]Cat. No. IM2470) used according to the manufacturers' recommendations. $5x10^5$ - $1x10^6$ cells were stained with the following antibody combination: anti-CD14[1] (FITC) x anti-CD56[2] (PE) x anti-CD3[3] (PerCP) x anti-CD19[4] (APC). Additional steps were performed as described in example 13, above.

**Flowcytometric detection of stained lymphocytes by FACS**

Data collection was performed with a 4 color BD FACSCalibur™ (Becton Dickinson).
For each measurement $1x10^4$ cells of defined lymphocyte subpopulations were acquired. Statistical analysis was performed with the program CellQuest Pro™ (Becton Dickinson) to obtain lymphocyte subpopulation percentages and to classify cell surface molecule expression intensity. Subsequently, percentages of single lymphocyte subsets related to total lymphocytes (i.e. B plus T plus NK cells excluding myeloid cells via CD14-staining) as determined by FACS were correlated with the lymphocyte count from the differential blood analysis to calculate absolute cell numbers of T cells ($CD3^+$, $CD56^-$, $CD14^-$).
T cell redistribution during the starting phase of treatment with MCSP-G4 VH-VL x I2C VH-VL in cynomolgus monkeys at dose levels of 60, 240 and 1000 $\mu g/m^2/24h$ is shown in Fig 28. These animals showed no signs at all of any T cell redistribution during the starting phase of treatment, i.e. T cell counts rather increased than decreased upon treatment initiation. Given that T cell redistribution is consistently observed in 100% of all patients without circulating target cells, upon treatment initiation with the conventional CD3 binding molecule (e.g. CD19xCD3 construct as described in WO 99/54440) against a context dependent CD3 epitope, it was demonstrated that substantially less T cell redistribution in the absence of circulating target cells upon treatment initiation can be observed with a CD3 binding molecule of the invention directed and generated against an epitope of human an non-chimpanzee primate CD3 epsilon chain as defined by the amino acid sequence of anyone of SEQ ID NOs: 2, 4, 6, or 8 as defined in the claims. This is in clear contrast to CD3-binding molecules directed against a context-dependent CD3 epitope, like the constructs described in WO 99/54440, The binding molecules against context-independent CD3 epitopes, as (inter alia) provided in any one of SEQ ID NOs: 2, 4, 6, or 8 provide for this substantially less (detrimental and non-desired) T cell redistribution. Because T cell redistribution during the starting phase of treatment with CD3 binding molecules is a major risk factor for CNS adverse events, the CD3 binding molecules provided herein and capable of recognizing a context independent CD3 epitope have a substantial advantage over the CD3 binding molecules known in the art and directed against context-dependent CD3 epitopes. Indeed none of the cynomolgus monkeys treated with MCSP-G4 VH-VL x I2C VH-VL showed any signs of CNS symptoms.

The context-independence of the CD3 epitope is provided in this invention and corresponds to the first 27 N-terminal amino acids of CD3 epsilon) or fragments of this 27 amino acid stretch. This context-independent epitope is taken out of its native environment within the CD3 complex and fused to heterologous amino acid sequences without loss of its structural integrity. Anti-CD3 binding molecules as provided herein and generated (and directed) against a context-independent CD3 epitope provide for a surprising clinical improvement with regard to T cell redistribution and, thus, a more favorable safety profile. Without being bound by theory, since their CD3 epitope is context-independent, forming an autonomous selfsufficient subdomain without much influence on the rest of the CD3 complex, the CD3 binding molecules provided herein induce less allosteric changes in CD3 conformation than the conventional CD3 binding molecules (like molecules provided in WO 99/54440), which recognize context-dependent CD3 epitopes like molecules provided in WO 99/54440. As a consequence (again without being bound by theory), the induction of intracellular NcK2 recruitment by the CD3 binding molecules provided herein is also reduced resulting in less isoform switch of T cell integrins and less adhesion of T cells to endothelial cells. It is preferred that preparations of CD3 binding molecules of the disclosure (directed against and generated against a context-independent epitope as defined herein) essentially consists of monomeric molecules.

These monomeric molecules are even more efficient (than dimeric or multimeric molecules) in avoiding T cell redistribution and thus the risk of CNS adverse events during the starting phase of treatment.

## 16. Generation and characterization of CD33 and CD3 cross-species specific bispecific single chain molecules

### 16.1. Generation of CHO cells expressing human CD33

The coding sequence of human CD33 as published in GenBank (Accession number NM_001772) was obtained by gene synthesis according to standard protocols. The gene synthesis fragment was designed as to contain first a Kozak site for eukaryotic expression of the construct, followed by a 19 amino acid immunoglobulin leader peptide, followed in frame by the coding sequence of the mature human CD33 protein, followed in frame by the coding sequence of serine glycine dipeptide, a histidine$_6$-tag and a stop codon (the cDNA and amino acid sequence of the construct is listed under SEQ ID Nos 305 and 306). The gene synthesis fragment was also designed as to introduce restriction sites at the beginning and at the end of the fragment. The introduced restriction sites, EcoRI at the 5' end and SalI at the 3' end, were utilised in the following cloning procedures. The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

### 16.2. Generation of CHO cells expressing the extracellular domain of macaque CD33

The cDNA sequence of macaque CD33 was obtained by a set of 3 PCRs on cDNA from macaque monkey bone marrow prepared according to standard protocols. The following reaction conditions: 1 cycle at 94°C for 3 minutes followed by 35 cycles with 94°C for 1 minute, 53°C for 1 minute and 72°C for 2 minutes followed by a terminal cycle of 72°C for 3 minutes and the following primers were used:

1. forward primer:

    5'-gaggaattcaccatgccgctgctgctactgctgcccctgctgtgggcaggggccctggctatgg-3' (SEQ ID No. 369)
    reverse primer: 5'-gatttgtaactgtatttggtacttcc-3' (SEQ ID No. 370)

2.

    forward primer: 5'-attccgcctccttggggatcc-3' (SEQ ID No. 371)
    reverse primer: 5'-gcataggagacattgagctggatgg-3' (SEQ ID No. 372)

3. forward primer:

5'-gcaccaacctgacctgtcagg-3' (SEQ ID No. 373)
reverse primer: 5'-agtgggtcgactcactgggtcctgacctctgagtattcg-3' (SEQ ID No. 374)

Those PCRs generate three overlapping fragments, which were isolated and sequenced according to standard protocols using the PCR primers, and thereby provided a portion of the cDNA sequence of macaque CD33 from the second nucleotide of codon +2 to the third nucleotide of codon +340 of the mature protein.
To generate a construct for expression of macaque CD33 a cDNA fragment was obtained by gene synthesis according to standard protocols (the cDNA and amino acid sequence of the construct is listed under SEQ ID Nos 307 and 308). In this construct the coding sequence of macaque CD33 from amino acid +3 to +340 of the mature CD33 protein was fused into the coding sequence of human CD33 replacing the human coding sequence of the amino acids +3 to +340. The gene synthesis fragment was also designed as to contain a Kozak site for eukaryotic expression of the construct and restriction sites at the beginning and the end of the fragment containing the cDNA coding for essentially the whole extracellular domain of macaque CD33, the macaque CD33 transmembrane domain and a macaque-human chimeric intracellular CD33 domain. The introduced restriction sites XbaI at the 5' end and SalI at the 3' end, were utilised in the following cloning procedures. The gene synthesis fragment was then cloned via XbaI and SalI into a plasmid designated pEF-DHFR (pEF-DHFR is described in Raum et al. Cancer Immunol Immunother 50 (2001) 141-150). A sequence verified clone of this plasmid was used to transfect CHO/dhfr- cells as described above.

### 16.3. Generation of CD33 and CD3 cross-species specific bispecific antibody molecules

### Cloning of cross-species specific binding molecules

Generally, bispecific antibody molecules, each comprising a domain with a binding specificity cross-species specific for human and non-chimpanzee primate CD3 epsilon as well as a domain with a binding specificity cross-species specific for human and non-chimpanzee primate CD33, were designed as set out in the following Table 5:

Table 5: Formats of anti-CD3 and anti-CD33 cross-species specific bispecific molecules

| SEQ ID (nucl/prot) | Formats of protein constructs (N → C) |
|---|---|
| 276/275 | AH11HLxH2CHL |
| 258/257 | AH3HLxH2CHL |
| 270/269 | AC8HLxH2CHL |
| 264/263 | AF5HLxH2CHL |
| 288/287 | F2HLxH2CHL |
| 300/299 | E11HLxH2CHL |
| 282/281 | B3HLxH2CHL |
| 294/293 | B10HLxH2CHL |
| 278/277 | AH11HLxF12QHL |
| 260/259 | AH3HLxF12QHL |
| 272/271 | AC8HLxF12QHL |
| 266/265 | AF5HLxF12QHL |
| 290/289 | F2HLxF12QHL |
| 302/301 | E11HLxF12QHL |
| 284/283 | B3HLxF12QHL |
| 296/295 | B10HLxF12QHL |
| 280/279 | AH11HLxI2CHL |
| 262/261 | AH3HLxI2CHL |
| 274/273 | AC8HLxI2CHL |
| 268/267 | AF5HLxI2CHL |

(continued)

| SEQ ID (nucl/prot) | Formats of protein constructs (N → C) |
|---|---|
| 292/291 | F2HLxI2CHL |
| 304/303 | E11HLxI2CHL |
| 286/285 | B3HLxI2CHL |
| 298/297 | B10HLxI2CHL |

The aforementioned constructs containing the variable light-chain (L) and variable heavy-chain (H) domains cross-species specific for human and macaque CD33 and the CD3 specific VH and VL combinations cross-species specific for human and macaque CD3 were obtained by gene synthesis. The gene synthesis fragments were designed and eukaryotic protein expression was performed similar as described in example 9 for the MCSP and CD3 cross-species specific single chain molecules. The same holds true for the expression and purification of the CD33 and CD3 cross-species specific single chain molecules.

In the Western Blot a single band was detected at 52 kD corresponding to the purified bispecific antibody.

### 16.4. Flow cytometric binding analysis of the CD33 and CD3 cross-species specific bispecific antibodies

In order to test the functionality of the cross-species specific bispecific antibody constructs regarding the capability to bind to human and macaque CD33 and CD3, respectively, a FACS analysis was performed similar to the analysis described for the analysis of the MCSP and CD3 cross-species specific bispecific antibodies in example 10 using CHO cells expressing the human or macaque CD33 extracellular domains (see example 16.1 and 16.2).
The specific binding of human and non-chimpanzee primate CD3 of the CD3 binding molecules of the invention was clearly detectable as shown in Figure 29. In the FACS analysis all constructs show binding to CD3 and CD33 as compared to the respective negative controls. Cross-species specificity of the bispecific antibodies to human and macaque CD3 and CD33 antigens is demonstrated.

### 16.5. Bioactivity of CD33 and CD3 cross-species specific bispecific antibodies

Bioactivity of the generated bispecific antibodies was analyzed by chromium 51 ($^{51}Cr$) release in vitro cytotoxicity assays using the CD33 positive cell lines described in Examples 16.1 and 16.2. As effector cells stimulated human CD4/CD56 depleted PBMC or the macaque T cell line 4119LnPx were used as specified in the respective figures. The cytotoxicity assays were performed similar to the setting described for the bioactivity analysis of the MCSP and CD3 cross-species specific bispecific antibodies in example 11 using CHO cells expressing the human or macaque CD33 extracellular domains (see example 16.1 and 16.2) as target cells.
As shown in Figure 30, all of the generated cross-species specific bispecific constructs demonstrate cytotoxic activity against human CD33 positive target cells elicited by stimulated human CD4/CD56 depleted PBMC and against macaque CD33 positive target cells elicited by the macaque T cell line 4119LnPx.

## 17. Purification of cross-species specific bispecific single chain molecules by an affinity procedure based on the context independent CD3 epsilon epitope corresponding to the N-terminal amino acids 1-27

### 17.1 Generation of an affinity column displaying the isolated context independent human CD3 epsilon epitope corresponding to the N-terminal amino acids 1-27

The plasmid for expression of the construct 1-27 CD3-Fc consisting of the 1-27 N-terminal amino acids of the human CD3 epsilon chain fused to the hinge and Fc gamma region of human immunoglobulin IgG1 described above (Example 3; cDNA sequence and amino acid sequence of the recombinant fusion protein are listed under SEQ ID NOs 230 and 229) was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. For the isolation of the fusion protein a goat anti-human fc affinity column was prepared according to standard protocols using a commercially available affinity purified goat anti-human IgG fc fragment specific antibody with minimal cross-reaction to bovine, horse, and mouse serum proteins (Jackson ImmunoResearch Europe Ltd.). Using this affinity column the fusion protein was isolated out of cell culture supernatant on an Äkta Explorer System (GE Amersham) and eluted by citric acid. The eluate was neutralized and concentrated. After dialysis against amine free coupling buffer the purified fusion protein was coupled to an N-Hydroxy-Succinimide NHS activated 1 ml HiTrap column (GE Amersham).
After coupling remaining NHS groups were blocked and the column was washed and stored at 5°C in storage buffer containing 0.1% sodium azide.

**17.2 Purification of cross-species specific bispecific single chain molecules using a human CD3 peptide affinity column**

200 ml cell culture supernatant of cells expressing cross-species specific bispecific single chain molecules were 0.2 μm sterile filtered and applied to the CD3 peptide affinity column using an Äkta Explorer system (GE Amersham). The column was then washed with phosphate buffered saline PBS pH 7.4 to wash out unbound sample. Elution was done with an acidic buffer pH 3.0 containing 20 mM Citric acid and 1 M sodium chloride. Eluted protein was neutralized immediately by 1 M Trishydroxymethylamine TRIS pH 8.3 contained in the collection tubes of the fraction collector.
Protein analysis was done by SDS PAGE and Western Blot.
For SDS PAGE BisTris Gels 4-12% are used (Invitrogen). The running buffer was 1 x MES-SDS-Puffer (Invitrogen). As protein standard 15 μl prestained Sharp Protein Standard (Invitrogen) was applied. Electrophoresis was performed for 60 minutes at 200 volts 120 mA max. Gels were washed in demineralised water and stained with Coomassie for one hour. Gels are destained in demineralised water for 3 hours. Pictures are taken with a Syngene Gel documentation system.
For Western Blot a double of the SDS PAGE gel was generated and proteins were electroblotted onto a nitrocellulose membrane. The membrane was blocked with 2% bovine serum albumin in PBS and incubated with a biotinylated murine Penta His antibody (Qiagen). As secondary reagent a streptavidin alkaline phosphatase conjugate (DAKO) was used. Blots were developed with BCIP/NBT substrate solution (Pierce).
As demonstrated in Figures 31, 32 and 33 the use of a human CD3 peptide affinity column as described above allows the highly efficient purification of the bispecific single chain molecules from cell culture supernatant. The cross-species specific anti-CD3 single chain antibodies contained in the bispecific single chain molecules therefore enable via their specific binding properties an efficient generic one-step method of purification for the cross-species specific bispecific single chain molecules, without the need of any tags solely attached for purification purposes.

**18. Generic pharmacokinetic assay for cross-species specific bispecific single chain molecules**

**18.1 Production of 1-27 CD3-Fc for use in the pharmacokinetic assay**

The coding sequence of the 1-27 N-terminal amino acids of the human CD3 epsilon chain fused to the hinge and Fc gamma region of human immunoglobulin IgG1 was obtained by gene synthesis according to standard protocols (cDNA sequence and amino acid sequence of the recombinant fusion protein are listed under SEQ ID NOs 309 and 310). The gene synthesis fragment was designed as to contain first a Kozak site for eukaryotic expression of the construct, followed by a 19 amino acid immunoglobulin leader peptide, followed in frame by the coding sequence of the first 27 amino acids of the extracellular portion of the mature human CD3 epsilon chain, followed in frame by the coding sequence of the hinge region and Fc gamma portion of human IgG1 and a stop codon. The gene synthesis fragment was also designed and cloned as described in example 3.1, supra. A clone with sequence-verified nucleotide sequence was transfected into DHFR deficient CHO cells for eukaryotic expression of the construct. Eukaryotic protein expression in DHFR deficient CHO cells was performed as described in example 9, supra. For the isolation of the fusion protein a goat anti-human fc affinity column was prepared according to standard protocols using a commercially available affinity purified goat anti-human IgG fc fragment specific antibody with minimal cross-reaction to bovine, horse, and mouse serum proteins (Jackson ImmunoResearch Europe Ltd.). Using this affinity column the fusion protein was isolated out of cell culture supernatant on an Äkta Explorer System (GE Amersham) and eluted by citric acid. The eluate was neutralized and concentrated.

**18.2 Pharmacokinetic assay for cross-species specific bispecific single chain molecules**

The assay is based on the ECL-ELISA technology using ruthenium labelled detection on carbon plates measured on a Sektor Imager device (MSD). In a first step, carbon plates (MSD High Bind Plate 96 well Cat: L15xB-3) were coated with 5 μl/well at 50 ng/ml of the purified 1-27 CD3-Fc described in Example 18.1. The plate was then dried overnight at 25°C. Subsequently plates were blocked with 5% BSA (Paesel&Lorei #100568) in PBS at 150 μl/well for 1 h at 25°C in an incubator while shaking (700 rpm). In the next step plates were washed three times with 0.05% Tween in PBS. A standard curve in 50% macaque serum in PBS was generated by serial 1:4 dilution starting at 100 ng/ml of the respective cross-species specific bispecific single chain molecule to be detected in the assay. Quality control (QC) samples were prepared in 50% macaque serum in PBS ranging from 1 ng/ml to 50 ng/ml of the respective cross-species specific bispecific single chain molecule dependent on the expected sample serum concentrations. Standard, QC or unknown samples were transferred to the carbon plates at 10 μl/well and incubated for 90 min at 25°C in the incubator while shaking (700 rpm). Subsequently plates were washed three times with 0.05% Tween in PBS. For detection 25 μl/well of penta-His-Biotin antibody (Qiagen, 200 μg/ml in 0.05% Tween in PBS) was added and incubated for 1 h at 25°C in an incubator while shaking (700 rpm). In a second detection step 25 μl/well Streptavidin-SulfoTag solution (MSD; Cat: R32AD-1; Lot: WO010903) was added and incubated for 1 h at 25°C in an incubator while shaking (700 rpm). Subsequently plates were washed three times with 0.05% Tween in PBS. Finally 150 μl/well MSD Reading Buffer (MSD, Cat: R9ZC-1) was added and plates were read in the Sektor Imager device.
Figures 34 and 35 demonstrate the feasibility of detection of cross-species specific bispecific single chain molecules in serum samples of macaque monkeys for cross-species specific bispecific single chain molecules. The cross-species specific anti-CD3 single chain antibodies contained in the bispecific single chain molecules enable therefore via their specific binding properties a highly sensitive generic assay for detection of the cross-species specific bispecific single chain molecules. The assay set out above can be used in the context of formal toxicological studies that are needed for drug development and can be easily adapted for measurement of patient samples in connection with the clinical application of cross-species specific bispecific single chain molecules.

**19. Generation of recombinant transmembrane fusion proteins of the N-terminal amino acids 1-27 of CD3 epsilon from different non-chimpanzee primates fused to EpCAM from cynomolgus monkey (1-27 CD3-EpCAM).**

**19.1 Cloning and expression of 1-27 CD3-EpCAM**

CD3 epsilon was isolated from different non-chimpanzee primates (marmoset, tamarin, squirrel monkey) and swine. The coding sequences of the 1-27 N-terminal amino acids of CD3 epsilon chain of the mature human, common marmoset (*Callithrix jacchus*), cottontop tamarin (*Saguinus oedipus),* common squirrel monkey (*Saimiri sciureus*) and domestic swine (*Sus scrofa*; used as negative control) fused to the N-terminus of Flag tagged cynomolgus EpCAM were obtained by gene synthesis according to standard protocols (cDNA sequence and amino acid sequence of the recombinant fusion proteins are listed under SEQ ID NOs 231 to 240). The gene synthesis fragments were designed as to contain first a BsrGI site to allow for fusion in correct reading frame with the coding sequence of a 19 amino acid immunoglobulin leader peptide already present in the target expression vector, which was followed in frame by the coding sequence of the N-terminal 1-27 amino acids of the extracellular portion of the mature CD3 epsilon chains, which was followed in frame by the coding sequence of a Flag tag and followed in frame by the coding sequence of the mature cynomolgus EpCAM transmembrane protein. The gene synthesis fragments were also designed to introduce a restriction site at the end of the cDNA coding for the fusion protein. The introduced restriction sites BsrGI at the 5' end and SalI at the 3' end, were utilized in the following cloning procedures. The gene synthesis fragments were then cloned via BsrGI and SalI into a derivative of the plasmid designated pEF-DHFR (pEF-DHFR is described in Raum et al. Cancer Immunol Immunother 50 (2001) 141-150), which already contains the coding sequence of the 19 amino acid immunoglobulin leader peptide following standard protocols. Sequence verified plasmids were used to transfect DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.
Transfectants were tested for cell surface expression of the recombinant transmembrane protein via an FACS assay according to standard protocols. For that purpose a number of $2.5 \times 10^5$ cells were incubated with 50 μl of the anti-Flag M2 antibody (Sigma-Aldrich Chemie GmbH, Taufkirchen, Germany) at 5 μg/ml in PBS with 2% FCS. Bound antibody was detected with an R-Phycoerythrin-conjugated affinity purified F(ab')2 fragment, goat anti-mouse IgG, Fc-gamma fragment specific 1:100 in PBS with 2% FCS (Jackson ImmunoResearch Europe Ltd., Newmarket, Suffolk, UK). Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002). Expression of the Flag tagged recombinant transmembrane fusion proteins consisting of cynomolgus EpCAM and the 1-27 N-terminal amino acids of the human, marmoset, tamarin, squirrel monkey and swine CD3 epsilon chain respectively on transfected cells is clearly detectable (Figure 36).

### 19.2 Cloning and expression of the cross-species specific anti-CD3 single chain antibody I2C HL in form of an IgG1 antibody

In order to provide improved means of detection of binding of the cross-species specific single chain anti-CD3 antibody the I2C VHVL specificity is converted into an IgG1 antibody with murine IgG1 and murine kappa constant regions. cDNA sequences coding for the heavy chain of the IgG antibody were obtained by gene synthesis according to standard protocols. The gene synthesis fragments were designed as to contain first a Kozak site to allow for eukaryotic expression of the construct, which is followed by an 19 amino acid immunoglobulin leader peptide, which is followed in frame by the coding sequence of the heavy chain variable region or light chain variable region, followed in frame by the coding sequence of the heavy chain constant region of murine IgG1 as published in GenBank (Accession number AB097849) or the coding sequence of the murine kappa light chain constant region as published in GenBank (Accession number D14630), respectively.
Restriction sites were introduced at the beginning and the end of the cDNA coding for the fusion protein. Restriction sites EcoRI at the 5' end and SalI at the 3' end were used for the following cloning procedures. The gene synthesis fragments were cloned via EcoRI and SalI into a plasmid designated pEF-DHFR (pEF-DHFR is described in Raum et al. Cancer Immunol Immunother 50 (2001) 141-150) for the heavy chain construct and pEFADA (pEFADA is described in Raum et al. loc cit.) for the light chain construct according to standard protocols. Sequence verified plasmids were used for co-transfection of respective light and heavy chain constructs into DHFR deficient CHO cells for eukaryotic expression of the construct. Eukaryotic protein expression in DHFR deficient CHO cells was performed as described by Kaufmann R.J. (1990) loc cit. Gene amplification of the constructs was induced by increasing concentrations of methotrexate (MTX) to a final concentration of up to 20 nM MTX and deoxycoformycin (dCF) to a final concentration of up to 300 nM dCF. After two passages of stationary culture cell culture supernatant was collected and used in the subsequent experiment.

### 19.3 Binding of the cross-species specific anti-CD3 single chain antibody I2C HL in form of an IgG1 antibody to 1-27 CD3-EpCAM

Binding of the generated I2C IgG1 construct to the 1-27 N-terminal amino acids of the human, marmoset, tamarin and squirrel monkey CD3 epsilon chains respectively fused to cynomolgus Ep-CAM as described in Example 19.1 was tested in a FACS assay according to standard protocols. For that purpose a number of $2.5x10^5$ cells were incubated with 50 μl of cell culture supernatant containing the I2C IgG1 construct as described in Example 19.2. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Flow cytometry was performed on a FACS-Calibur apparatus, the Cell Quest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
As shown in Figure 37 binding of the I2C IgG1 construct to the transfectants expressing the recombinant transmembrane fusion proteins consisting of the 1-27 N-terminal amino acids of CD3 epsilon of human, marmoset, tamarin or squirrel monkey fused to cynomolgus EpCAM as compared to the negative control consisting of the 1-27 N-terminal amino acids of CD3 epsilon of swine fused to cynomolgus EpCAM was observed. Thus multi-primate cross-species specificity of I2C was demonstrated. Signals obtained with the anti Flag M2 antibody and the I2C IgG1 construct were comparable, indicating a strong binding activity of the cross-species specific specificity I2C to the N-terminal amino acids 1-27 of CD3 epsilon.

### 20. Binding of the cross-species specific anti-CD3 binding molecule I2C to the human CD3 epsilon chain with and without N-terminal His6 tag

A chimeric IgG1 antibody with the binding specificity I2C as described in Example 19.2 specific for CD3 epsilon was tested for binding to human CD3 epsilon with and without N-terminal His6 tag. Binding of the antibody to the EL4 cell lines transfected with His6-human CD3 epsilon as described in Example 6.1 and wild-type human CD3 epsilon as described in Example 5.1 respectively was tested by a FACS assay according to standard protocols. $2.5x10^5$ cells of the transfectants were incubated with 50 μl of cell culture supernatant containing the I2C IgG1 construct or 50 μl of the respective control antibodies at 5μg/ml in PBS with 2% FCS. As negative control an appropriate isotype control and as positive control for expression of the constructs the CD3 specific antibody UCHT-1 were used respectively. Detection of the His6 tag was performed with the penta His antibody (Qiagen). The binding of the antibodies was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
Comparable binding of the anti-human CD3 antibody UCHT-1 to both transfectants demonstrates approximately equal levels of expression of the constructs. The binding of the penta His antibody confirmed the presence of the His6 tag on the His6-human CD3 construct but not on the wild-type construct.
Compared to the EL4 cell line transfected with wild-type human CD3 epsilon a clear loss of binding of the I2C IgG1 construct to human-CD3 epsilon with an N-terminal His6 tag was detected. These results show that a free N-terminus of CD3 epsilon is essential for binding of the cross-species specific anti-CD3 binding specificity I2C to the human CD3 epsilon chain (Figure 28).

### 21. Generation of CD33 and CD3 cross-species specific bispecific single chain molecules

### 21.1 Generation of CD33 and CD3 cross-species specific bispecific single chain molecules

Generally, bispecific single chain antibody molecules, each comprising a domain with a binding specificity cross-species specific for human and macaque CD3epsilon as well as a domain with a binding specificity cross-species specific for human and macaque CD33, were designed as set out in the following Table 6:

Table 6: Formats of anti-CD3 and anti-CD3[3] cross-species specific bispecific single chain antibody molecules

| SEQ ID (nucl/prot) | Formats of protein constructs (NO C) |
|---|---|
| 316/315 | I2CHLxAF5HL |
| 314/313 | F12QHLxAF5HL |
| 312/311 | H2CHLxAF5HL |

The aforementioned constructs containing the variable light-chain (L) and variable heavy-chain (H) domains cross-species specific for human and macaque CD33 and the CD3 specific VH and VL combinations cross-species specific for human and macaque CD3 were obtained by gene synthesis. The gene synthesis fragments were designed in analogy to the procedure described in example 9 for the MCSP and CD3 cross-species specific single chain molecules. A clone with sequence-verified nucleotide sequence was transfected into DHFR deficient CHO cells for eukaryotic expression of the construct. Eukaryotic protein expression in DHFR deficient CHO cells was performed as also described in example 9 for the MCSP and CD3 cross-species specific single chain molecules and used in the subsequent experiments.

### 21.2 Flow cytometric binding analysis of the CD33 and CD3 cross-species specific bispecific antibodies

In order to test the functionality of the cross-species specific bispecific antibody constructs regarding the capability to bind to human and macaque CD33 and CD3, respectively, a FACS analysis is performed similar to the analysis described for the analysis of the MCSP and CD3 cross-species specific bispecific antibodies in example 10 using CHO cells expressing the human or macyque CD33 extracellular domains (see examples 16.1 and 16.2).
The bispecific binding of the single chain molecules listed above, which were cross-species specific for CD33 and cross-species specific for human and non-chimpanzee primate CD3 was clearly detectable as shown in Figure 41. In the FACS analysis all constructs showed binding to CD3 and CD33 as compared to the respective negative controls. Cross-species specificity of the bispecific antibodies to human and macaque CD3 and CD33 antigens was demonstrated.

### 21.3. Bioactivity of CD33 and CD3 cross-species specific bispecific single chain antibodies

Bioactivity of the generated bispecific single chain antibodies was analyzed by chromium 51 ($^{51}Cr$) release in vitro cytotoxicity assays using the CD33 positive cell lines described in Examples 16.1 and 16.2. As effector cells stimulated human CD4/CD56 depleted PBMC or the macaque T cell line 4119LnPx were used as specified in the respective figures. The cytotoxicity assays were performed similar to the procedure described for the bioactivity analysis of the MCSP and CD3 cross-species specific bispecific antibodies in example 11 using CHO cells expressing the human or macaque CD33 extracellular domains (see example 16.1 and 16.2) as target cells.
As shown in Figure 42, all of the generated cross-species specific bispecific single chain antibody constructs demonstrate cytotoxic activity against human CD33 positive target cells elicited by stimulated human CD4/CD56 depleted PBMC and against macaque CD33 positive target cells elicited by the macaque T cell line 4119LnPx.

**22. Redistribution of circulating chimpanzee T cells upon exposure to a conventional bispecific CD3 binding molecule directed at a target molecule which is absent from circulating blood cells**

A single male chimpanzee was subjected to dose escalation with intravenous single-chain EpCAM/CD3-bispecific antibody construct (Schlereth (2005) Cancer Res 65: 2882). Like in the conventional single-chain CD19/CD3-bispecific antibody construct (Loffler (2000, Blood, Volume 95, Number 6) or WO 99/54440), the CD3 arm of said EpCAM/CD3-construct is also directed against a conventional context dependent epitope of human and chimpanzee CD3. At day 0, the animal received 50ml PBS/5% HSA without test material, followed by 50ml PBS/5% HSA plus single-chain EpCAM/CD3-bispecific antibody construct at 1.6, 2.0, 3.0 and 4.5 μg/kg on days 7, 14, 21 and 28, respectively. The infusion period was 2 hours per administration. For each weekly infusion the chimpanzee was sedated with 2-3 mg/kg Telazol intramuscularly, intubated and placed on isoflurane/$O_2$ anesthesia with stable mean blood pressures. A second intravenous catheter was placed in an opposite limb to collect (heparinized) whole blood samples at the time points indicated in Figure 43 for FACS analysis of circulating blood cells. After standard erythrocyte lysis, T cells were stained with a FITC-labeled antibody reacting with chimpanzee CD2 (Becton Dickinson) and the percentage of T cells per total lymphocytes determined by flowcytometry. As shown in Figure 43, every administration of single-chain EpCAM/CD3-bispecific antibody construct induced a rapid drop of circulating T cells as observed with single-chain CD19/CD3-bispecific antibody construct in B-NHL patients, who had essentially no circulating target B (lymphoma) cells. As there are no EpCAM-positive target cells in the circulating blood of humans and chimpanzees, the drop of circulating T cells upon exposure to the single-chain EpCAM/CD3-bispecific antibody construct can be attributed solely to a signal, which the T cells receive through pure interaction of the CD3 arm of the construct with a conventional context dependent CD3 epitope in the absence of any target cell mediated crosslinking. Like the redistribution of T cells induced through their exposure to single-chain CD19/CD3-bispecific antibody construct in B-NHL patients, who had essentially no circulating target B (lymphoma) cells, the T cell redistribution in the chimpanzee upon exposure to the single-chain EpCAM/CD3-bispecific antibody construct can be explained by a conformational change of CD3 following the binding event to a context dependent CD3 epitope further resulting in the transient increase of T cell adhesiveness to blood vessel endothelium (see Example 13). This finding confirms, that conventional CD3 binding molecules directed to context dependent CD3 epitopes - solely through this interaction - can lead to a redistribution pattern of peripheral blood T cells, which is associated with the risk of CNS adverse events in humans as describe in Example 13.

**23. Specific binding of scFv clones to the N-terminus of human CD3 epsilon**

**23.1 Bacterial expression of scFv constructs in E. coli XL1 Blue**

As previously mentioned, *E. coli* XL1 Blue transformed with pComb3H5Bhis/Flag containing a VL- and VH-segment produce soluble scFv in sufficient amounts after excision of the gene III fragment and induction with 1 mM IPTG. The scFv-chain is exported into the periplasma where it folds into a functional conformation.
The following scFv clones were chosen for this experiment:

i) ScFvs 4-10, 3-106, 3-114, 3-148, 4-48, 3-190 and 3-271 as described in WO 2004/106380.
ii) ScFvs from the human anti-CD3epsilon binding clones H2C, F12Q and I2C as described herein.

For periplasmic preparations, bacterial cells transformed with the respective scFv containing plasmids allowing for periplasmic expression were grown in SB-medium supplemented with 20 mM $MgCl_2$ and carbenicillin 50 μg/ml and redissolved in PBS after harvesting. By four rounds of freezing at -70°C and thawing at 37°C, the outer membrane of the bacteria was destroyed by osmotic shock and the soluble periplasmic proteins including the scFvs were released into the supernatant. After elimination of intact cells and cell-debris by centrifugation, the supernatant containing the human anti-human CD3-scFvs was collected and used for further examination. These crude supernatants containing scFv will be further termed periplasmic preparations (PPP).

**23.2 Binding of scFvs to human CD3 epsilon (aa 1-27)-Fc fusion protein**

ELISA experiments were carried out by coating the human CD3 epsilon (aa 1-27)- Fc fusion protein to the wells of 96 well plastic plates (Nunc, maxisorb) typically at 4° C over night. The antigen coating solution was then removed, wells washed once with PBS/0.05 % Tween 20 and subsequently blocked with PBS/3 % BSA for at least one hour. After removal of the blocking solution, PPPs and control solutions were added to the wells and incubated for typically one hour at room temperature. The wells were then washed three times with PBS/0.05 % Tween 20. Detection of scFvs bound to immobilized antigen was carried out using a Biotin-labeled anti FLAG-tag antibody (M2 anti Flag-Bio, Sigma, typically at a final concentration of 1 μg/ml PBS) and detected with a peroxidase-labeled Streptavidine (Dianova, 1 μg/ml PBS). The signal was developed by adding ABTS substrate solution and measured at a wavelength of 405 nm. Unspecific binding of the test-samples to the blocking agent and/or the human IgG1 portion of the human CD3 epsilon (aa 1-27)-Fc fusion protein was examined by carrying out the identical assay with the identical reagents and identical timing on ELISA plates which were coated with human IgG1 (Sigma). PBS was used as a negative control.

As shown in Figure 44, scFvs H2C, F12Q and I2C show strong binding signals on human CD3 epsilon (aa 1-27)- Fc fusion protein. The human scFvs 3-106, 3-114, 3-148, 3-190, 3-271, 4-10 and 4-48 (as described in WO 2004/106380) do not show any significant binding above negative control level.

To exclude the possibility that the positive binding of scFvs H2C, F12Q and I2C to wells coated with human CD3 epsilon (aa 1-27)- Fc fusion protein might be due to binding to BSA (used as a blocking agent) and/or the human IgG1 Fc-gamma-portion of the human CD3 epsilon (aa 1-27)- Fc fusion protein, a second ELISA experiment was performed in parallel. In this second ELISA experiment, all parameters were identical to those in the first ELISA experiment, except that in the second ELISA experiment human IgG1 (Sigma) was coated instead of human CD3 epsilon (aa 1-27)- Fc fusion protein. As shown in Figure 45, none of the scFvs tested showed any significant binding to BSA and/or human IgG1 above background level.

Taken together, these results allow the conclusion that conventional CD3 binding molecules recognizing a context-dependent epitope of CD3 epsilon (e.g. as disclosed in WO 2004/106380) do not bind specifically to the human CD3 epsilon (aa 1-27)-region, whereas the scFvs H2C, F12Q and I2C binding a context-independent epitope of CD3 epsilon clearly show specific binding to the N-terminal 27 amino acids of human CD3 epsilon.

### 24. Generation and characterization of PSCA and CD3 cross-species specific bispecific single chain antibody molecules

#### 24.1 Generation of CHO cells expressing human PSCA

The coding sequence of human PSCA as published in GenBank (Accession number NM_005672) was obtained by gene synthesis according to standard protocols. The gene synthesis fragment was designed as to contain first a Kozak site for eukaryotic expression of the construct, followed by a 19 amino acid immunoglobulin leader peptide, followed in frame by the coding sequence of a FLAG tag, followed in frame by the coding sequence of the mature human PSCA protein and a stop codon (the corresponding sequences of the construct are listed under SEQ ID NOs. 443 and 444). The gene synthesis fragment was also designed as to introduce restriction sites at the beginning and at the end of the fragment. The introduced restriction sites, EcoRI at the 5' end and SalI at the 3' end, were utilised in the following cloning procedures. The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

#### 24.2 Generation of CHO cells expressing macaque PSCA

The cDNA sequence of macaque PSCA was obtained by a PCR on cDNA from macaque monkey (cynomolgus) prostate prepared according to standard protocols.

The following reaction conditions: 1 cycle at 94°C for 2 minutes followed by 40 cycles with 94°C for 1 minute, 55°C for 1 minute and 72°C for 1 minute followed by a terminal cycle of 72°C for 3 minutes and the following primers were used:

forward primer: 5'- CACCACAGCCCACCAGTGACC -3' (SEQ ID NO. 447)
reverse primer: 5'- GAGGCCTGGGGCACCACACCC -3' (SEQ ID NO. 448)

The PCR reactions were performed under addition of PCR grade betain to a final concentration of 1 M. This PCR generated a DNA fragment, which was isolated and sequenced according to standard protocols using the PCR primers, and thereby provided the cDNA sequence coding macaque PSCA. To generate a construct for expression of macaque PSCA a cDNA fragment was obtained by gene synthesis according to standard protocols (the corresponding sequences are listed under SEQ ID NOs: 445 and 446). The gene synthesis fragment was designed as to contain first a Kozak site for eukaryotic expression of the construct, followed by a 19 amino acid immunoglobulin leader peptide, followed in frame by the coding sequence of a FLAG tag, followed in frame by the coding sequence of the mature macaque PSCA protein and a stop codon. The gene synthesis fragment was also designed as to introduce restriction sites at the beginning and at the end of the fragment. The introduced restriction sites, EcoRI at the 5' end and SalI at the 3' end, were utilised in the following cloning procedures. The gene synthesis fragment was cloned via EcoRI and SalI into a plasmid designated pEF-DHFR (pEF-DHFR is described in Raum et al. Cancer Immunol Immunother 50 (2001) 141-150) following standard protocols.
The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 24.3 Generation of PSCA and CD3 cross-species specific bispecific single chain antibody molecules

#### Cloning of cross-species specific PSCAxCD3 bispecific single chain antibody molecules

Generally, bispecific single chain antibody molecules, each comprising a domain with a binding specificity cross-species specific for human and non-chimpanzee primate CD3 epsilon as well as a domain with a binding specificity cross-species specific for human and non-chimpanzee primate PSCA, were designed as set out in the following Table 7:

Table 7: Formats of anti-CD3 and anti-PSCA cross-species specific bispecific single chain antibody molecules

| SEQ ID NO. (nucl/prot) | Formats of protein constructs (N → C) |
|---|---|
| 390/389 | PSCA1HLxI2CHL |
| 422/421 | PSCA3HLx12CHL |
| 440/439 | PSCA4HLxI2CHL |
| 392/391 | PSCA1LHxI2CHL |
| 406/405 | PSCA2LHxI2CHL |
| 424/423 | PSCA3LHxI2CHL |
| 442/441 | PSCA4LHxI2CHL |
| 1227/1226 | PC16E12HLxI2CHL |
| 1199/1198 | PC32D5HLxI2CHL |
| 1185/1184 | PC32B8HLxI2CHL |
| 1241/1240 | PC08F4HLxI2CHL |
| 1213/1212 | PC17D10HLxI2CHL |
| 1269/1268 | PC17H10HLxI2CHL |
| 1255/1254 | PC16F5HLxI2CHL |

The aforementioned constructs containing the variable light-chain (L) and variable heavy-chain (H) domains cross-species specific for human and macaque PSCA and the CD3 specific VH and VL combinations cross-species specific for human and macaque CD3 were obtained by gene synthesis. The gene synthesis fragments were designed and eukaryotic protein expression was performed in analogy to the procedure described in example 9 for the MCSP and CD3 cross-species specific single chain molecules.

### 24.4 Expression and purification of the PSCAxCD3 bispecific single chain antibody molecules

The bispecific single chain antibody molecules were expressed in Chinese hamster ovary cells (CHO) or HEK293 cells as described herein above for the MCSPxCD3 bispecific single chan antibodies.
The isolation and analysis of the expressed bispecific single chan antibodies has also been described herein above in Example 9.

**24.5 Flow cytometric binding analysis of the PSCA and CD3 cross-species specific bispecific antibodies**

In order to test the functionality of the cross-species specific bispecific antibody constructs regarding the capability to bind to human and macaque PSCA and CD3, respectively, a FACS analysis was performed. For this purpose CHO cells transfected with human PSCA as described in Example 24.1 and the human CD3 positive T cell leukemia cell line HPB-ALL (DSMZ, Braunschweig, ACC483) were used to test the binding to human antigens. The binding reactivity to macaque antigens was tested by using the generated macaque PSCA transfectant described in Example 24.2 and a macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of cell culture supernatant of transfected cells expressing the cross-species specific bispecific antibody constructs. The cells were washed twice in PBS with 2% FCS and binding of the construct was detected with a murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in PBS with 2% FCS. Supernatant of untransfected cells was used as a negative control.
Flow cytometry was performed on a FACS-Calibur apparatus, the CellQuest software was used to acquire a n d analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
The bispecific binding of the single chain molecules listed above, which are cross-species specific for PSCA and CD3 was clearly detectable as shown in Figure 46 and 48. In the FACS analysis all constructs showed binding to CD3 and PSCA compared to the negative control. Cross-species specificity of the bispecific antibodies to human and macaque CD3 and PSCA antigens, respectively, was demonstrated.

**24.6 Bioactivity of PSCA and CD3 cross-species specific bispecific single chain antibodies**

Bioactivity of the generated bispecific single chain antibodies was analyzed by chromium 51 ($^{51}$Cr) release in vitro cytotoxicity assays using the CHO cells transfected with human PSCA described in Example 24.1 and the CHO cells transfected with macaque PSCA described in Example 24.2. As effector cells stimulated human CD4/CD56 depleted PBMC or the macaque T cell line 4119LnPx were used, respectively.
The generation of stimulated human PBMC was described herein above in Example 11.
Target cells were prepared and the assay was performed in analogy to the procedure described for the MCSPxCD3 bispecific single chain antibodies in example 11.
As shown in Figure 47 and 49 all of the generated cross-species specific bispecific single chain antibody constructs demonstrated cytotoxic activity against human PSCA positive target cells elicited by stimulated human CD4/CD56 depleted PBMC and macaque PSCA positive target cells elicited by the macaque T cell line 4119LnPx.

**24.7 Bispecific single chain antibody constructs directed against human and non-chimpanzee primate CD3 and PSCA**

The human antibody germline VH sequence VH1 1-f (http://vbase.mrc-cpe.cam.ac.uk/) is chosen as framework context for CDRH1 (SEQ ID NO. 414), CDRH2 (SEQ ID NO. 415) and CDRH3 (SEQ ID NO. 416). Likewise the same human antibody germline VH sequence is chosen as framework context for CDRH1 (SEQ ID NO. 400), CDRH2 (SEQ ID NO. 401) and CDRH3 (SEQ ID NO. 402). For each human VH several degenerated oligonucleotides have to be synthesized that overlap in a terminal stretch of approximately 15-20 nucleotides. To this end every second primer is an antisense primer. For VH1 1-f the following set of oligonucleotides is used:

5' P3-VH-A-XhoI

CCT GAT CTC GAG AGC GGC SCA GAS STG RAA AAG CCA GGC GCC ACG GTG AAG ATT (SEQ ID NO. 449)

5' P3-VH-B

GGC GCC ACG GTG AAG ATT TCC TGC AAG SYC AGC GGC WAC ACG TTC ACC GGC TAC TAC ATC CAC TGG GTG (SEQ ID NO. 450)

3' P3-VH-C

GTA GGA GGT GAA GCC GTT GTT GGG GTC CAC TCT GCC SAT CCA TTC CAG GCY CTT CCC GKG GGM CTG TTK CAC CCA GTG GAT GTA GTA (SEQ ID NO. 451)

5' P3-VH-D

AAC GGC TTC ACC TCC TAC AAC CAG AAG TTC AAG GGC ARG GYC AYA MTK ACC GYG GAC AMG AGC ACC RRC ACC GCC TAC ATG GAA CTG (SEQ ID NO. 452)

3' P3-VH-E

GCT GTC GAA GAA GTT GCC CRC GCA ATA GTA CAC GGC GGT GTC CTC GCT GSK CAG GCT KCT CAG TTC CAT GTA GGC GGT (SEQ ID NO. 453)

3' P3-VH-F-BstEII

CAC GTC GGT GAC CGT GGT TCC CTG GCC CCA GCT GTC GAA GAA GTT GCC (SEQ ID NO. 454)

As another set of oligonucleotides for VH1 1-f the following primers are used:

5'-PSCA2VH-A-XHO1

CAG GTG CTCGAG YCA GGC GCC GAA STG RWG AAG CCT GGC GCC MCA GTG AAG MTA TCC TGC AAG GYC AGC GGC TAC ACC TTC ACC AAC (SEQ ID NO. 455)

3'-PSCA2VH-B

GCT GTC GCT GGG GTC GAT CCT GCC YAT CCA TTC CAG GCC CYT GCC GGG CSY CTG TTK CAC CCA GTT CAG CCA GTA GTT GGT GAA GGT GTA GCC (SEQ ID NO. 456)

5'-PSCA2VH-C

AGG ATC GAC CCC AGC GAC AGC GAG ATC CAC TAC GAC CAG AAG TTC AAG GAC ARA GYC ACC MTA ACC GYG GAC AMG AGC ACC RRC ACC GCC TAC (SEQ ID NO. 457)

3'-PSCA2VH-D

GGC CAG GGC GCA ATA GTA CAC GGC GGT GTC CTC GCT TST CAG GCT GGA CAG CTS SAT GTA GGC GGT GYY GGT GCT C (SEQ ID NO. 458)

3'-PSCA2VH-E-BSTE2

AGA CAC GGT GAC CGT GGT GCC CTG GCC CCA GTA GGC CAT GGC GTA GAT GCC GGT CAG GGC GCA ATA GTA CAC (SEQ ID NO. 459)

Each of these primer sets spans over the whole corresponding VH sequence.
Within each set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.
Each VH PCR product is then used as a template for a standard PCR reaction using primers that incorporate N-terminal and C-terminal suitable cloning restriction sites.
The DNA fragment of the correct size (for a VH approximately 350 nucleotides) is isolated by agarose gel electrophoresis according to standard methods. In this way sufficient VH DNA fragment is amplified.
The human antibody germline VL sequence VkII A1 (http://vbase.mrc-cpe.cam.ac.uk/) is chosen as framework context for CDRL1 (SEQ ID NO. 409), CDRL2 (SEQ ID NO. 410) and CDRL3 (SEQ ID NO. 411). Likewise human antibody germline VL sequence VkII A19 (http://vbase.mrc-cpe.cam.ac.uk/) is chosen as framework context for CDRL1 (SEQ ID NO. 395), CDRL2 (SEQ ID NO. 396) and CDRL3 (SEQ ID NO. 397). For each human VL several degenerated oligonucleotides have to be synthesized that overlap in a terminal stretch of approximately 15-20 nucleotides. To this end every second primer is an antisense primer. Restriction sites needed for later cloning within the oligonucleotides are deleted. For VkII A1 the following oligonucleotides are used:

5' P3-VL-A-SacI

CCT GTA GAG CTC GTC ATG ACC CAG TCC CCC CTG TCC CTG MSC GTG ACC MTC GGC CAG CCA GCC AGC ATC (SEQ ID NO. 460)

3' P3-VL-B

CCA GTT CAG GTA GGT CTT GCC GTC GCT GTC CAG CAG GGA CTG GCT GGA CTT GCA AGA GAT GCT GGC TGG CTG GCC (SEQ ID NO. 461)

5' P3-VL-C

AAG ACC TAC CTG AAC TGG YTS CWG CAG AGG CCA GGC CAG AGC CCC ARG AGG CTG ATC TAC CTG GTG TCC ACC CTG (SEQ ID NO. 462)

3' P3-VL-D

GGT GAA GTC GGT GCC GGA GCC GCT GCC GGA GAA TCT GTC TGG CAC GCC GCT GTC CAG GGT GGA CAC CAG GTA (SEQ ID NO. 463)

5' P3-VL-E

TCC GGC ACC GAC TTC ACC CTG AAG ATC AGC AGG GTG GAG GCC GAG GAC STG GGC GTG TAC TAC TGC TGG CAG GGC ACC (SEQ ID NO. 464)

3' P3-VL-F-BsiWI/SpeI

CCA GAC ACT AGT CGT ACG CTT GAT TTC CAG CTT GGT CCC TCC GCC GAA GGT CCT TGG GAA GTG GGT GCC CTG CCA GCA GTA (SEQ ID NO. 465)

For VkII A19 the oligonucleotides are as follows:

5'-PSCA2VL-a-SAC1

CAG ACA GAG CTC GTG ATG ACC CAG KCA SCT CYA AGC STG CCA GTG ACC CCA GGC GAG YCA GYG TCC ATC AGC TGC AGG TCC AGC (SEQ ID NO. 466)

3'-PSCA2VL-b

CTG GGG GCT CTG GCC TGG CYT CTG CAG AWA CCA GTA CAG GTA GGT GTT GCC GTT GCT GTG CAG CAG GCT CTT GCT GGA CCT GCA GCT GAT G (SEQ ID NO. 467)

5'-PSCA2VL-c

CCA GGC CAG AGC CCC CAG CTG CTG ATC TAC AGG ATG AGC AAC CTG GCT AGC GGC GTG CCA GAC AGA TTC AGC GGC AGC GGC TCT GGA ACC (SEQ ID NO. 468)

3'-PSCA2VL-d

CAG GCA GTA GTA CAC GCC CAC GTC CTC GGC CTC CAC CCT GCT GAT CYT CAG GGT GAA GKC GGT TCC AGA GCC GCT GCC (SEQ ID NO. 469)

3'-PSCA2VL-e-BsiW1-Spe1

GTG CTG ACT AGT CGT ACG CTT GAT TTC CAG CTT GGT CCC TTG GCC GAA GGT GTA GGG GTA TTC CAG GTG CTG CAG GCA GTA GTA CAC GCC (SEQ ID NO. 470)

Each of these primer sets spans over the whole corresponding VL sequence.

Within each set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.

Each VL PCR product is then used as a template for a standard PCR reaction using primers that incorporate N-terminal and C-terminal suitable cloning restriction sites.

The DNA fragment of the correct size (for a VL approximately 330 nucleotides) is isolated by agarose gel electrophoresis according to standard methods. In this way sufficient VL DNA fragment is amplified.

The final VH1 1-f (P3)-based VH PCR product (i.e. the repertoire of human/humanized VH) is then combined with the final VkII A1 -based VL PCR product (i.e. the repertoire of human/humanized VL) and the final VH1 1-f (PSCA2)-based VH PCR product (i.e. the repertoire of human/humanized VH) with the final VkII A19-based VL PCR product (i.e. the repertoire of human/humanized VL) in the phage display vector pComb3H5Bhis to form two different libraries of functional scFvs from which - after display on filamentous phage - anti-PSCA binders are selected, screened, identified and confirmed as described as follows:

450 ng of the light chain fragments (SacI-SpeI digested) are ligated with 1400 ng of the phagemid pComb3H5Bhis

(SacI-SpeI digested; large fragment). The resulting combinatorial antibody library is then transformed into 300 ul of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants are selected for carbenicilline resistance encoded by the pComb3H5BHis vector in 100 ml of liquid super broth (SB)-culture over night. Cells are then harvested by centrifugation and plasmid preparation is carried out using a commercially available plasmid preparation kit (Qiagen).
2800 ng of this plasmid-DNA containing the VL-library (XhoI-BstEII digested; large fragment) are ligated with 900 ng of the heavy chain V-fragments (XhoI-BstEII digested) and again transformed into two 300 ul aliquots of electrocompetent E. coli XL1 Blue cells by electroporation (2.5 kV, 0.2 cm gap cuvette, 25 uFD, 200 Ohm) resulting in a total VH-VL scFv (single chain variable fragment) library size of more than $10^7$ independent clones.

After phenotype expression and slow adaptation to carbenicillin, the E. coli cells containing the antibody library are transferred into SB-Carbenicillin (50 ug/mL) selection medium. The E. coli cells containing the antibody library is then infected with an infectious dose of $10^{12}$ particles of helper phage VCSM13 resulting in the production and secretion of filamentous M13 phage, wherein phage particle contains single stranded pComb3H5BHis-DNA encoding a scFv-fragment and displayed the corresponding scFv-protein as a translational fusion to phage coat protein III. This pool of phages displaying the antibody library is used for the selection of antigen binding entities.
For this purpose the phage library carrying the cloned scFv-repertoire is harvested from the respective culture supernatant by PEG8000/NaCl precipitation and centrifugation. Approximately $10^{11}$to $10^{12}$ scFv phage particles are resuspended in 0.4 ml of PBS/0.1% BSA and incubated with $10^5$ to $10^7$ PSCA transfected CHO cells (see example 24.1) for 1 hour on ice under slow agitation. These PSCA transfected CHO cells are harvested beforehand by centrifugation, washed in PBS and resuspended in PBS/1 % FCS (containing Na Azide). scFv phage which do not specifically bind to the PSCA transfected CHO cells are eliminated by up to five washing steps with PBS/1 % FCS (containing Na Azide). After washing, binding entities are eluted from the cells by resuspending the cells in HCl-glycine pH 2.2 (10 min incubation with subsequent vortexing) and after neutralization with 2 M Tris pH 12, the eluate is used for infection of a fresh uninfected E. coli XL1 Blue culture (OD600 > 0.5). The E. coli culture containing E. coli cells successfully transduced with a phagemid copy, encoding a human/humanized scFv-fragment, are again selected for carbenicillin resistance and subsequently infected with VCMS 13 helper phage to start the second round of antibody display and in vitro selection. A total of 4 to 5 rounds of selections are carried out, normally.
In order to screen for PSCA specific binders plasmid DNA corresponding to 4 and 5 rounds of panning is isolated from E. coli cultures after selection. For the production of soluble scFv-protein, VH-VL-DNA fragments are excised from the plasmids (XhoI-SpeI). These fragments are cloned via the same restriction sites into the plasmid pComb3H5BFlag/His differing from the original pComb3H5BHis in that the expression construct (e.g. scFv) includes a Flag-tag (DYKDDDDK) between the scFv and the His6-tag and the additional phage proteins are deleted. After ligation, each pool (different rounds of panning) of plasmid DNA is transformed into 100 μl heat shock competent E. coli TG1 or XLI blue and plated onto carbenicillin LB-agar. Single colonies are picked into 100 ul of LB carb (50 ug/ml).
E. coli transformed with pComb3H5BHis containing a VL- and VH-segment produce soluble scFv in sufficient amounts after excision of the gene III fragment and induction with 1 mM IPTG. Due to a suitable signal sequence, the scFv-chain is exported into the periplasma where it folds into a functional conformation.
Single E. coli TG1 bacterial colonies from the transformation plates are picked for periplasmic small scale preparations and grown in SB-medium (e.g. 10 ml) supplemented with 20 mM $MgCl_2$ and carbenicillin 50μg/ml (and re-dissolved in PBS (e.g. 1 ml) after harvesting. By four rounds of freezing at -70°C and thawing at 37°C, the outer membrane of the bacteria is destroyed by temperature shock and the soluble periplasmic proteins including the scFvs are released into the supernatant.
After elimination of intact cells and cell-debris by centrifugation, the supernatant containing the anti-PSCA scFvs is collected and used for the identification of PSCA specific binders as follows:

Binding of scFvs to PSCA is tested by flow cytometry on PSCA transfected CHO cells (see example 24.1); untransfected CHO cell are used as negative control.

For flow cytometry 2,5x$10^5$ cells are incubated with 50 ul of scFv periplasmic preparation or with 5 μg/ml of purified scFv in 50 μl PBS with 2% FCS. The binding of scFv is detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified $F(ab')_2$ fragment, goat anti-mouse IgG (Fc-gamma fragment specific), diluted 1:100 in 50 μl PBS with 2% FCS (Dianova, Hamburg, FRG) is used. The samples are measured on a FACSscan (BD biosciences, Heidelberg, FRG).
Single clones are then analyzed for favourable properties and amino acid sequence. PSCA specific scFvs are converted into recombinant bispecific single chain antibodies by joining them via a $Gly_4Ser_1$-linker with the CD3 specific scFv I2C (SEQ ID NO. 185) or any other CD3 specific scFv of the invention to result, for example, in constructs with the domain arrangement $VH_{PSCA}$ - $(Gly_4Ser_1)_3$ -$VL_{PSCA}$- Gly4$Ser_1$-$VH_{CD3}$ - $(Gly_4Ser_1)_3$ - $VL_{CD3}$ or $VL_{PSCA}$ - $(Gly_4Ser_1)_3$ -$VH_{PSCA}$-$Gly_4Ser_1$-$VH_{CD3}$-$(Gly_4Ser_1)_3$- $VL_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal $His_6$-tag followed by a stop codon are both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). Stable transfection of DHFR-deficient CHO cells, selection for DHFR-positive transfectants secreting the bispecific single chain antibodies into the culture supernatant and gene amplification with methotrexat for increasing expression levels are carried out as described (Mack et al. Proc. Natl. Acad. Sci. USA 92 (1995) 7021-7025). All other state of the art procedures are carried out according to standard protocols (Sambrook, Molecular Cloning; A Laboratory Manual, 3rd edition, Cold Spring Harbour Laboratory Press, Cold Spring Harbour, New York (2001)).

Identification of functional bispecific single-chain antibody constructs is carried out by flowcytometric analysis of culture supernatant from transfected CHO cells. For this purpose CD3 binding is tested on the human CD3 positive T cell leukemia cell line HPB-ALL (DSMZ, Braunschweig, ACC483) and on the macaque T cell line 4119LnPx. Binding to tumor specific PSCA epitopes is tested on PSCA transfected CHO cells (see example 24.1). 200.000 cells of the respective cell line are incubated for 30 min. on ice with 50 μl of cell culture supernatant. The cells are washed twice in PBS with 2% FCS and bound bispecific single-chain antibody construct is detected with a murine anti-His antibody (Penta His antibody; Qiagen; diluted 1:20 in 50 μl PBS with 2% FCS). After washing, bound anti-His antibodies are detected with an Fc gamma-specific antibody (Dianova) conjugated to phycoerythrin, diluted 1:100 in PBS with 2% FCS. Supernatant of untransfected CHO cells is used as negative control.

Flow cytometry is performed on a FACS-Calibur apparatus; the CellQuest software is used to acquire and analyze the data (Becton Dickinson biosciences, Heidelberg).

FACS staining and measuring of the fluorescence intensity are performed as described in Current Protocols in Immunology (Coligan, Kruisbeek, Margulies, Shevach and Strober, Wiley-Interscience, 2002).

Only those constructs showing bispecific binding to human and macaque CD3 as well as to human and macaque PSCA are selected for further use.

For protein production CHO cells expressing fully functional bispecific single chain antibody and adapted to nucleoside-free HyQ PF CHO liquid soy medium (with 4.0 mM L-Glutamine with 0.1 % Pluronic F - 68; HyClone) are grown in roller bottles with nucleoside-free HyQ PF CHO liquid soy medium (with 4.0 mM L-Glutamine with 0.1% Pluronic F - 68; HyClone) for 7 days. Culture supernatant is cleared from cells by centrifugation and stored at -20°C until purification. As chromatography equipment for purification of bispecific single chain antibody from culture supernatant Akta® Explorer System (GE Health Systems) and Unicorn® Software are used. Immobilized metal affinity chromatography ("IMAC") is performed using a Fractogel EMD chelate® (Merck) which is loaded with $ZnCl_2$ according to the protocol provided by the manufacturer. The column is equilibrated with buffer A (20 mM sodium phosphate buffer pH 7.2, 0.1 M NaCl) and the cell culture supernatant (500 ml) is applied to the column (10 ml) at a flow rate of 3 ml/min. The column is washed with buffer A to remove unbound sample. Bound protein is eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 are pooled for further purification. All chemicals are of research grade and purchased from Sigma (Deisenhofen) or Merck (Darmstadt).

Gel filtration chromatography is performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) are subjected to standard SDS-PAGE and Western Blot for detection. Prior to purification, the column is calibrated for molecular weight determination (molecular weight marker kit, Sigma MW GF-200). Protein concentrations are determined using OD280 nm.

Purified bispecific single chain antibody protein is analyzed in SDS PAGE under reducing conditions performed with pre-cast 4-12% Bis Tris gels (Invitrogen). Sample preparation and application are performed according to the protocol provided by the manufacturer. The molecular weight is determined with MultiMark protein standard (Invitrogen). The gel is stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in PBS. All constructs are purified according to this method.

Western Blot is performed using an Optitran® BA-S83 membrane and the Invitrogen Blot Module according to the protocol provided by the manufacturer. For detection of the bispecific single chain antibody protein antibodies an anti-His Tag antibody is used (Penta His, Qiagen). A Goat-anti-mouse Ig antibody labeled with alkaline phosphatase (AP) (Sigma) is used as secondary antibody and BCIP/NBT (Sigma) as substrate. A single band is detected at 52 kD corresponding to the purified bispecific single chain antibody.

The potency in the human and the non-chimpanzee primate system of bispecific single chain antibodies interacting with human and macaque CD3 and with human and macaque PSCA is determined by a cytotoxicity assay based on chromium 51 ($^{51}Cr$) release using PSCA transfected CHO cells as target cells (see example 24.1) and stimulated human CD4/CD56 depleted PBMC or the macaque T cell line 4119LnPx as effector cells.

Generation of stimulated human PBMC is performed as follows:

A Petri dish (85 mm diameter, Nunc) is coated with a commercially available anti-CD3 specific antibody (e.g. OKT3, Othoclone) in a final concentration of 1 μg/ml for 1 hour at 37°C. Unbound protein is removed by one washing step with PBS. The fresh PBMC are isolated from peripheral blood (30 - 50 ml human blood) by Ficoll gradient centrifugation according to standard protocols. 3 - 5 x $10^7$ PBMC are added to the precoated petri dish in 50 ml of RPMI 1640 with stabilized glutamine / 10% FCS / IL-2 20 U/ml (Proleukin, Chiron) and stimulated for 2 days. On the third day the cells are collected and washed once with RPMI 1640. IL-2 is added to a final concentration of 20 U/ml and the cells are cultivated again for one day in the same cell culture medium as above.

Target cells are washed twice with PBS and labelled with 11.1 MBq $^{51}Cr$ in a final volume of 100 μl RPMI with 50% FCS for 45 minutes at 37°C. Subsequently the labelled target cells are washed 3 times with 5 ml RPMI and then used in the cytotoxicity assay. The assay is performed in a 96 well plate in a total volume of 250μl supplemented RPMI (as above) with an E:T ratio of 10:1. 1 μg/ml of the cross-species specific bispecific single chain antibody molecules and 20 threefold dilutions thereof are applied. The assay time is 18 hours and cytotoxicity is measured as relative values of released chromium in the supernatant related to the difference of maximum lysis (addition of Triton-X) and spontaneous lysis (without effector cells). All measurements are done in quadruplicates. Measurement of chromium activity in the supernatants is performed with a Wizard 3" gamma counter (Perkin Elmer Life Sciences GmbH, Köln, Germany). Analysis of the experimental data is performed with Prism 4 for Windows (version 4.02, GraphPad Software Inc., San Diego, California, USA). Sigmoidal dose response curves typically have $R^2$ values >0.90 as determined by the software. $EC_{50}$ values as measure of potency are calculated by the analysis program.

### 25. Generation and characterization of CD19 and CD3 cross-species specific bispecific single chain antibody molecules

#### 25.1. Cloning and expression of human CD19 antigen on CHO cells

The sequence of the human CD19 antigen (NM_001770 Homo sapiens CD19 molecule (CD19), mRNA, National Center for Biotechnology Information, http://www.ncbi.nlm.nih.gov/entrez) was used to obtain a synthetic molecule by gene synthesis according to standard protocols. The gene synthesis fragment was also designed as to contain a Kozak site for eukaryotic expression of the construct and restriction sites at the beginning and the end of the DNA. The introduced restriction sites EcoRI at the 5' end and SalI at the 3' end were utilised during the cloning step into the expression plasmid designated pEFDHFR (Raum et al. Cancer Immunol Immunother 50 (2001) 141-150). After sequence verification the plasmid was used to transfect CHO/dhfr- cells as follows. A sequence verified plasmid was used to transfect CHO/dhfr- cells (ATCC No. CRL 9096; cultured in RPMI 1640 with stabilized glutamine obtained from Biochrom AG Berlin, Germany, supplemented with 10% FCS, 1% penicillin/streptomycin all obtained from Biochrom AG Berlin, Germany and nucleosides from a stock solution of cell culture grade reagents obtained from Sigma-Aldrich Chemie GmbH, Taufkirchen, Germany, to a final concentration of 10 μg/ml Adenosine, 10 μg/ml Deoxyadenosine and 10 μg/ml Thymidine, in an incubator at 37 C, 95% humidity and 7% CO2). Transfection was performed using the PolyFect Transfection Reagent (Qiagen GmbH, Hilden, Germany) and 5 μg of plasmid DNA according to the manufacturer's protocol. After culture period of 24 hours cells were washed once with PBS and again cultured in the aforementioned cell culture medium except that the medium was not supplemented with nucleosides and dialysed FCS (obtained from Biochrom AG Berlin, Germany) was used. Thus the cell culture medium did not contain nucleosides and thereby selection was applied on the transfected cells. Approximately 14 days after transfection the outgrowth of resistant cells was observed. After an additional 7 to 14 days the transfectants were tested positive for expression of the construct via FACS. Eukaryotic protein expression in DHFR deficient CHO cells is performed as described by Kaufmann R.J. (1990) Methods Enzymol. 185, 537-566. Gene amplification of the construct is induced by increasing concentrations of methothrexate (MTX) to a final concentration of up to 20 nM MTX.

#### 25.2. Generation of CD19 and CD3 cross-species specific bispecific single chain molecules

Generally, bispecific single chain antibody molecules, each comprising a domain with a binding specificity for the human and the macaque CD3 antigen as well as a domain with a binding specificity for the human CD19 antigen, were designed as set out in the following Table 8:

**Table 8: Formats of anti-CD3 and anti-CD19 cross-species specific bispecific single chain antibody molecules**

| SEQ ID (nucl/prot) | Formats of protein constructs (N → C) |
|---|---|
| 482/481 | HD37 LH x I2C HL |
| 486/485 | HD37 LH x F12Q HL |
| 484/483 | HD37 LH x H2C HL |
| 534/533 | hCD19 47-A3 LH x I2C HL |
| 522/521 | hCD19 46-B11 LH x I2C HL |
| 510/509 | hCD19 45-A10 LH x I2C HL |
| 498/497 | hCD19 26-D6 LH x I2C HL |
| 546/545 | HD37-DT LH x I2C HL |
| 558/557 | HD37-A LH x I2C HL |
| 570/569 | HD37-G LH x I2C HL |
| 582/581 | HD37-S LH x I2C HL |
| 594/593 | HD37-T LH x I2C HL |
| 606/605 | HD37-I LH x I2C HL |
| 618/617 | HD37-L LH x I2C HL |
| 630/629 | HD37-V LH x I2C HL |
| 642/641 | HD37-E LH x I2C HL |
| 654/653 | HD37-Q LH x I2C HL |
| 666/665 | HD37-N LH x I2C HL |
| 678/677 | HD37-K LH x I2C HL |
| 690/689 | HD37-R LH x I2C HL |
| 702/701 | HD37-H LH x I2C HL |
| 714/713 | HD37-Y LH x I2C HL |
| 726/725 | HD37-P LH x I2C HL |
| 738/737 | HD37-F LH x I2C HL |
| 750/749 | HD37-W LH x I2C HL |
| 762/761 | HD37-M LH x I2C HL |
| 1283/1282 | hCD19 5-A9 LH x I2C HL |
| 1297/1296 | hCD19 2-C6 LH x I2C HL |
| 1311/1310 | hCD19 4-C7 LH x I2C HL |
| 1325/1324 | hCD19 2-D7 LH x I2C HL |
| 1339/1338 | hCD19 2-D4 LH x I2C HL |
| 1353/1352 | hCD19 5-G3 LH x I2C HL |
| 1367/1366 | hCD19 4-E10 LH x I2C HL |
| 1381/1380 | hCD19 4-E3 LH X I2C HL |
| 1395/1394 | hCD19 3-H7 LH X I2C HL |

The aforementioned constructs containing the variable light-chain (L) and variable heavy-chain (H) domains specific for human CD19 and the CD3 specific VH and VL combinations cross-species specific for human and macaque CD3 were obtained by gene synthesis. The gene synthesis fragments were designed and eukaryotic protein expression was performed in analogy to the procedure described in example 9 for the MCSPxCD3 cross-species specific single chain molecules.

**25.3. Expression and purification of the bispecific single chain antibody molecules**

The bispecific single chain antibody molecules were expressed in Chinese hamster ovary cells (CHO) or HEK 293 cells as described herein above for the MCSPxCD3 bispecific single chain antibodies.
The isolation and analysis of the expressed bispecific single chain antibodies has also been described herein above in Example 9.

**25.4. Flow cytometric binding analysis of the CD19 and CD3 cross-species specific bispecific antibodies**

In order to test the functionality of the cross-species specific bispecific antibody constructs with regard to binding capability to human CD19 as well as to human and macaque CD3, a FACS analysis was performed. For this purpose the CHO cells transfected with human CD19 as described in example 25.1 and human CD3 positive T cell leukemia cell line HPB-ALL (DSMZ, Braunschweig, ACC483) were used to check the binding to human antigens. The binding reactivity to macaque CD3 was tested by using a macaque T cell line 4119LnPx (kindly provided by Prof Fickenscher, Hygiene Institute, Virology, Erlangen-Nuernberg; Knappe A, et al., and Fickenscher H: Herpesvirus saimiri-transformed macaque T cells are tolerated and do not cause lymphoma after autologous reinfusion. Blood 2000;95:3256-61.) 200,000 cells of the respective cell population were incubated for 30 min on ice with 50 μl of the purified protein of the cross-species specific bispecific antibody constructs (e. g. 2 μg/ml) Alternatively the cell culture supernatant of transiently produced proteins was used. The cells were washed twice in PBS and binding of the construct was detected with an unlabeled murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in 50 μl PBS with 2% FCS. Fresh culture medium was used as a negative control.
Flow cytometry was performed on a FACS-Calibur apparatus, the CellQuest software was used to aquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
In the FACS analysis shown in Figure 50 all tested constructs showed binding to human and macaque CD3 and to human CD19.

**25.5. Bioactivity of CD19 and CD3 cross-species specific bispecific single chain antibodies**

The bioactivity of bispecific single chain antibodies was analyzed by chromium 51 release in vitro cytotoxicity assays using the CD19 positive cell line described in example 25.1. As effector cells stimulated human CD8 positive T cells or the macaque T cell line 4119LnPx were used.
The generation of stimulated human PBMC was described herein above in Example 11.
Target cells prepared and the assay was performed in analogy to the procedure described for the MCSPxCD3 bispecific single chain antibodies in example 11.
In the T cell cytotoxicity assay shown in Figure 51 all tested bispecific single chain antibody constructs revealed cytotoxic activity against human CD19 positive target cells elicited by human CD8+ cells and against human CD19 positive target cells elicited by the macaque T cell line 4119LnPx. As a negative control, an irrelevant bispecific single chain antibody was used.

**25.6. Generation of additional CD19 and CD3 cross-species specific bispecific single chain molecules**

The human antibody germline VH sequence VH1 1-46 (http://vbase.mrc-cpe.cam.ac.uk/) is chosen as framework context for CDRH1 (SEQ ID NO 473), CDRH2 (SEQ ID NO 474) and CDRH3 (SEQ ID NO 475). For the human VH several degenerated oligonucleotides have to be synthesized that overlap in a terminal stretch of approximately 15-20 nucleotides. To this end every second primer is an antisense primer. For VH1 1-46 the following oligonucleotides are used (5'- to 3'):

5'-37VH-aXho1

CAG CTG CTC GAG TCT GGG GCT GAG STG RWG ARG CCT GGG KCC TCA GTG AAG RTT TCC TGC AAG GCT TCT GGC (SEQ ID NO 763)

3'-37VH-b

cca ctc aag acc ctg tcc agg GSS ctg cYt cac cca gtt cat cca gta gct aGw gaa tgY ata gcc aga agc ctt gca gga (SEQ ID NO 764)

5'-37VH-c

GGA CAG GGT CTT GAG TGG ATK GGA CAG ATT TGG CCT GGA GAT GGT GAT ACT AAC TAC AAT GGA AAG TTC AAG (SEQ ID NO 765)

3'-37VH-d

cag gct gct gag ttS cat gta gRc tgt gct ggt gga tKY gtc ASS agt caK agt gRc tYt acc ctt gaa ctt tcc att gta g (SEQ ID NO 766)

5'-37VH-e

C ATG SAA CTC AGC AGC CTG SSA TCT GAG GAC ACT GCG GTC TAT TWC TGT GCA AGA CGG GAG ACT ACG ACG G (SEQ ID NO 767)

3'-37VH-fBstE2

gga gac ggt gac cgt ggt ccc ttg gcc cca gta gtc cat agc ata gta ata acg gcc tac cgt cgt agt ctc ccg tct tgc (SEQ ID NO 768)

This primer set spans over the whole corresponding VH sequence.
Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72 °C for 10 minutes in a PCR cycler. Subsequently the product is run in an agarose gel electrophoresis and the product of a size from 200 to 400 isolated from the gel according to standard methods.
The VH PCR product is then used as a template for a standard PCR reaction using primers that incorporate N-terminal and C-terminal suitable cloning restriction sites.
The DNA fragment of the correct size (for a VH approximately 350 nucleotides) is isolated by agarose gel electrophoresis according to standard methods. In this way sufficient VH DNA fragment is amplified.
The human antibody germline VL sequence Vkl 012 (http://vbase.mrc-cpe.cam.ac.uk/) is chosen as framework context for CDRL1 (SEQ ID NO 478), CDRL2 (SEQ ID NO 479) and CDRL3 (SEQ ID NO 480). For the human VL several degenerated oligonucleotides have to be synthesized that overlap in a terminal stretch of approximately 15-20 nucleotides. To this end every second primer is an antisense primer. Restriction sites needed for later cloning within the oligonucleotides are deleted. For Vkl 012 the following oligonucleotides are used (5'- to 3'):

5'-37VL-A-SacI

CAG CTG GAG CTC CAG ATG ACC CAG TCT CCA KCT TCT TTG KCT GYG TCT STA GGG SAS AGA GYC ACC ATC WCC TGC AAG GCC AGC (SEQ ID NO 769)

3'-37VL-B

ctg ttg gta cca gtt caa ata aSt SNN acc atc ata atc aac act ttg gct ggc ctt gca ggt gat ggt (SEQ ID NO 770)

5'-37VL-C

TTG AAC TGG TAC CAA CAG AWA CCA GGA MAG SCA CCC AAA CTC CTC ATC TAT GAT GCA TCC AAT CTA GTT TCT (SEQ ID NO 771)

3'-37VL-D

gag ggt gaa gtc tgt ccc aga ccc act gcc act aaa cct ggR tgg gaY ccc aga aac tag att gga tgc (SEQ ID NO 772)

5'-37VL-E

GGG ACA GAC TTC ACC CTC AMC ATC MRT YCT STG SAG MMG GWG GAT KYC GCA ACC TAT YAC TGT CAG CAA AGT ACT GAG (SEQ ID NO 773)

3'-37VL-F-BsiW1Spe1

ACT CAG ACT AGT CGT ACG ttt gat ctc cac ctt ggt ccc ttg acc gaa cgt cca cgg atc ctc agt act ttg ctg aca g (SEQ ID NO 774)

This primer sets spans over the whole corresponding VL sequence.

Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72 °C for 10 minutes in a PCR cycler. Subsequently the product is run in an agarose gel electrophoresis and the product of a size from 200 to 400 isolated from the gel according to standard methods.

The VL PCR product is then used as a template for a standard PCR reaction using primers that incorporate N-terminal and C-terminal suitable cloning restriction sites.

The DNA fragment of the correct size (for a VL approximately 330 nucleotides) is isolated by agarose gel electrophoresis according to standard methods. In this way sufficient VL DNA fragment is amplified.

The final VH1 1-46 -based VH PCR product (i.e. the repertoire of human/humanized VH) is then combined with the final Vkl 012 -based VL PCR product (i.e. the repertoire of human/humanized VL) in the phage display vector pComb3H5Bhis to form a library of functional scFvs from which - after display on filamentous phage - anti-CD19 binders are selected, screened, identified and confirmed as described in the following:

450 ng of the light chain fragments (SacI-SpeI digested) are ligated with 1400 ng of the phagemid pComb3H5Bhis (SacI-SpeI digested; large fragment). The resulting combinatorial antibody library is then transformed into 300 μl of electrocompetent Escherichia coli XL1 Blue cells by electroporation (2.5 kV, 0.2 cm gap cuvette, 25 μFD, 200 Ohm,

Biorad gene-pulser) resulting in a library size of more than 107 independent clones. After one hour of phenotype expression, positive transformants are selected for carbenicilline resistance encoded by the pComb3H5BHis vector in 100 ml of liquid super broth (SB)-culture over night. Cells are then harvested by centrifugation and plasmid preparation is carried out using a commercially available plasmid preparation kit (Qiagen).
2800 ng of this plasmid-DNA containing the VL-library (XhoI-BstEII digested; large fragment) are ligated with 900 ng of the heavy chain V-fragments (XhoI-BstEII digested) and again transformed into two 300 μl aliquots of electrocompetent E. coli XL1 Blue cells by electroporation (2.5 kV, 0.2 cm gap cuvette, 25 μFD, 200 Ohm) resulting in a scFv library with a size of more than 107 independent clones.

After phenotype expression and slow adaptation to carbenicilline, the E. coli cells containing the antibody library are transferred into SB-carbenicilline (SB with 50 μg/mL carbenicilline) selection medium. The E. coli cells containing the antibody library are then infected with an infectious dose of 1012 particles of helper phage VCSM13 resulting in the production and secretion of filamentous M13 phage, wherein each phage particle contains single stranded pComb3H5BHis-DNA encoding a scFv-fragment and displays the corresponding scFv-protein as a translational fusion to phage coat protein III. This pool of phages displaying the antibody library is used for the selection of antigen binding entities.
For this purpose the phage library carrying the cloned scFv-repertoire is harvested from the respective culture supernatant by PEG8000/NaCl precipitation and centrifugation. Approximately 1011 to 1012 scFv phage particles are resuspended in 0.4 ml of PBS/0.1% BSA and incubated with 105 to 107 CD19 transfected CHO cells (see example 1) for 1 hour on ice under slow agitation. These CD19 transfected CHO cells are harvested beforehand by centrifugation, washed in PBS and resuspended in PBS/1 % FCS (containing Na Azide). scFv phage which do not specifically bind to the CD19 transfected CHO cells are eliminated by up to five washing steps with PBS/1 % FCS (containing Na Azide). After washing, binding entities are eluted from the cells by resuspending the cells in HCl-glycine pH 2.2 (10 min incubation with subsequent vortexing) and after neutralization with 2 M Tris pH 12, the eluate is used for infection of a fresh uninfected E. coli XL1 Blue culture (OD600 > 0.5). The E. coli culture containing E. coli cells successfully transduced with a phagemid copy, encoding a human/humanized scFv-fragment, are again selected for carbenicilline resistance and subsequently infected with VCMS 13 helper phage to start the second round of antibody display and in vitro selection. A total of 4 to 5 rounds of selections are carried out, normally.
In order to screen for CD19 specific binders plasmid DNA corresponding to 4 and 5 rounds of panning is isolated from E. coli cultures after selection. For the production of soluble scFv-protein, the scFv-DNA fragments are excised from the plasmids (XhoI-SpeI). These fragments are cloned via the same restriction sites into the plasmid pComb3H5BFlag/His differing from the original pComb3H5BHis in that the expression construct (i.e. the scFv) includes a Flag-tag (DYKDDDDK, SEQ ID NO 775) at its C-terminus before the His6-tag and that phage protein III/N2 domain and protein III/CT domain had been deleted. After ligation, each pool (different rounds of panning) of plasmid DNA is transformed into 100 μl heat shock competent E. coli TG1 or XLI blue and plated onto carbenicilline LB-agar. Single colonies are picked into 100 μl of LB carb (LB with 50 μg/ml carbenicilline).
E. coli transformed with pComb3H5BFlag/His containing a scFv-DNA fragment produce soluble scFv-protein in sufficient amounts after induction with 1 mM IPTG. Due to a suitable signal sequence, the scFv is exported into the periplasma where it folds into a functional conformation.
Single E. coli TG1 bacterial colonies from the transformation plates are picked for periplasmic small scale preparations and grown in SB-medium (e.g. 10 ml) supplemented with 20 mM MgCl2 and carbenicilline 50μg/ml (and re-dissolved in PBS (e.g. 1 ml) after harvesting. A temperature shock is applied by four rounds of freezing at -70°C and thawing at 37°C whereby the outer membrane of the bacteria is destroyed and the soluble periplasmic proteins including the scFvs are released into the supernatant. After elimination of intact cells and cell-debris by centrifugation, the supernatant containing the anti-CD19 scFvs is collected and used for the identification of CD19 specific binders as follows:
Binding of scFvs to CD19 is tested by flow cytometry on CD19 transfected CHO cells (see example 25.1); untransfected CHO cells are use as negative control.
For flow cytometry 2.5x105 cells are incubated with 50 μl of scFv periplasmic preparation or with 5 μg/ml of purified scFv in 50 μl PBS with 2% FCS. The binding of scFv is detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) is used. The samples are measured on a FACSscan (BD biosciences, Heidelberg, FRG).
Single clones are then analyzed for favourable properties and amino acid sequence. CD19 specific scFvs are converted into recombinant bispecific single chain antibodies by joining them via a Gly4Ser1-linker with the CD3 specific scFv I2C (amino acid sequence SEQ ID NO 185, nucleic acid sequence SEQ ID NO 186) or any other CD3 specific scFv of the invention to result in constructs with the domain arrangement VLCD19 - (Gly4Ser1)3 -VHCD19- Gly4Ser1-VHCD3 - (Gly4Ser1)3 - VLCD3. For expression in CHO cells the coding sequences of (i) an N-terminal immunoglobulin heavy chain leader comprising a start codon embedded within a Kozak consensus sequence and (ii) a C-terminal His6-tag followed by a stop codon are both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). Transfection of the generated expression plasmids, protein expression and purification of cross-species specific bispecific antibody constructs are performed as described in Examples 25.2 and 25.3. All other state of the art procedures are carried out according to standard protocols (Sambrook, Molecular Cloning; A Laboratory Manual, 3rd edition, Cold Spring Harbour Laboratory Press, Cold Spring Harbour, New York (2001)).
Identification of functional bispecific single-chain antibody constructs is carried out by flow cytometric binding analysis of culture supernatant from transfected cells expressing the cross-species specific bispecific antibody constructs. Analysis is perfomed as described in Example 25.4.
Only those constructs showing bispecific binding to human and macaque CD3 as well as to CD19 are selected for further use.

The cytotoxic activity of cross-species specific bispecific single chain antibody constructs against CD19 positive target cells elicited by effector T cells is analyzed as described in Example 25.5. CHO-cells transfected with human CD19 are used as target cells and stimulated CD4/CD56-depleted human PBMCs or the macaque T cell line 4119LnPx as effector T cells. Only those constructs showing potent redirected T cell cytotoxicity against CD19-positive target cells are selected for further use.

## 26. Generation and characterization of C-MET and CD3 cross-species specific bispecific single chain antibody molecules

### 26.1 Generation of CHO cells expressing human C-MET

The coding sequence of human C-MET as published in GenBank (Accession number NM_000245) was obtained by gene synthesis according to standard protocols. The gene synthesis fragment was designed as to contain first a Kozak site for eukaryotic expression of the construct followed by the coding sequence of the human C-MET protein and a stop codon (the cDNA and amino acid sequence of the construct is listed under SEQ ID Nos 776 and 777). The gene synthesis fragment was also designed as to introduce restriction sites at the beginning and at the end of the fragment. The introduced restriction sites, EcoRI at the 5' end and SalI at the 3' end, were utilised in the following cloning procedures. Internal restriction sites were removed by silent mutation of the coding sequence in the gene synthesis fragment (SalI: nucleotide 366 from C to G; EcoRI and XbaI: nucleotides 2059 to 2061 from TCT to AGC; EcoRI: nucleotide 2304 from T to C). The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 26.2 Generation of CHO cells expressing macaque C-MET

The cDNA sequence of macaque C-MET (cynomolgus) was obtained by a set of 5 PCRs on cDNA from macaque monkey Liver prepared according to standard protocols. The following reaction conditions: 1 cycle at 94°C for 2 minutes followed by 40 cycles with 94°C for 1 minute, 56°C for 1 minute and 72 °C for 3 minutes followed by a terminal cycle of 72 °C for 3 minutes and the following primers were used:

4. forward primer: 5'-aggaattcaccatgaaggcccccgctgtgcttgcacc-3' (SEQ ID NO: 778) reverse primer: 5'-ctccagaggcatttccatgtagg-3' (SEQ ID NO: 779)
5. forward primer: 5'-gtccaaagggaaactctagatgc-3' (SEQ ID NO: 780) reverse primer: 5'-ggagacactggatgggagtccagg-3' (SEQ ID NO: 781)
6. forward primer: 5'-catcagagggtcgcttcatgcagg-3' (SEQ ID NO: 782) reverse primer: 5'-gctttggttttcagggggagttgc-3' (SEQ ID NO: 783)
7. forward primer: 5'-atccaaccaaatcttttattagtggtgg-3' (SEQ ID NO: 784) reverse primer: 5'-gacttcattgaaatgcacaatcagg-3' (SEQ ID NO: 785)
8. forward primer: 5'-tgctctaaatccagagctggtcc-3' (SEQ ID NO: 786) reverse primer: 5'-gtcagataagaaattccttagaatcc-3' (SEQ ID NO: 787)

These PCRs generated five overlapping fragments, which were isolated and sequenced according to standard protocols using the PCR primers, and thereby provided a portion of the cDNA sequence coding macaque C-MET from codon 10 of the leader peptide to the last codon of the mature protein. To generate a construct for expression of macaque C-MET a cDNA fragment was obtained by gene synthesis according to standard protocols (the cDNA and amino acid sequence of the construct is listed under SEQ ID Nos 788 and 789). In this construct the coding sequence of macaque C-MET from amino acid 10 of the leader peptide to the last amino acid of the mature C-MET protein followed by a stop codon was fused in frame to the coding sequence of the amino acids 1 to 9 of the leader peptide of the human C-MET protein. The gene synthesis fragment was also designed as to contain a Kozak site for eukaryotic expression of the construct and restriction sites at the beginning and the end of the fragment containing the cDNA. The introduced restriction sites, EcoRI at the 5' end and SalI at the 3' end, were utilised in the following cloning procedures.
Internal restriction sites were removed by silent mutation of the coding sequence in the gene synthesis fragment (SalI: nucleotide 366 from C to G; EcoRI: nucleotide 2055 from G to C). The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 26.3 Generation of C-MET and CD3 cross-species specific bispecific single chain molecules

### Cloning of cross-species specific binding molecules

Generally, bispecific single chain antibody molecules, each comprising a domain with a binding specificity cross-species specific for human and non-chimpanzee primate CD3epsilon as well as a domain with a binding specificity for C-MET, were designed as set out in the following Table 9:

Table 9: Formats of anti-C-MET and anti-CD3 cross-species specific bispecific single chain antibody molecules

| SEQ ID (nucl/prot) | Formats of protein constructs (N → C) |
|---|---|
| 830/829 | MET1HLxI2CHL |
| 854/853 | MET4HLxI2CHL |
| 872/871 | MET5HLxI2CHL |
| 890/889 | MET6HLxI2CHL |
| 832/831 | MET1LHxI2CHL |
| 856/855 | MET4LHxI2CHL |
| 874/873 | MET5LHxI2CHL |
| 892/891 | MET6LHxI2CHL |
| 906/905 | MET7LHxI2CHL |

The aforementioned constructs containing the variable light-chain (L) and variable heavy-chain (H) domains specific for C-MET and the CD3 specific VH and VL combinations cross-species specific for human and macaque CD3 were obtained by gene synthesis. The gene synthesis fragments were designed and eukaryotic protein expression was performed in analogy to the procedure described in example 9 for the MCSPxCD3 cross-species specific single chain molecules. The bispecific single chain antibody molecules were expressed in Chinese hamster ovary cells (CHO) or HEK 293 cells as described herein above for the MCSPxCD3 bispecific single chain antibodies.
The isolation and analysis of the expressed bispecific single chain antibodies has also been described herein above in Example 9.
As shown in Figure 55 all of the generated cross-species specific bispecific single chain antibody constructs demonstrated cytotoxic activity against macaque cMET positive target cells elicited by the macaque T cell line 4119LnPx.

**26.5 Flow cytometric binding analysis of the C-MET and CD3 cross-species specific bispecific antibodies**

In order to test the functionality of the cross-species specific bispecific antibody constructs regarding the capability to bind to C-MET and human and macaque CD3, respectively, a FACS analysis was performed. For this purpose the human C-MET positive breast cancer cell line MDA-MB-231 (ATCC No. HTB-26) and the human CD3 positive T cell leukemia cell line HPB-ALL (DSMZ, Braunschweig, ACC483) were used to test the binding to human antigens. The binding reactivity to macaque CD3 was tested by using the macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with with 50 μl of cell culture supernatant of transfected cells expressing the cross-species specific bispecific antibody constructs. The cells were washed twice in PBS with 2% FCS and binding of the construct was detected with a murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in PBS with 2% FCS. Supernatant of untransfected cells was used as a negative control.
Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
The bispecific binding of the single chain molecules listed above, which are specific for C-MET and cross-species specific for human and non-chimpanzee primate CD3 was clearly detectable as shown in Figure 52. In the FACS analysis all constructs showed binding to CD3 and C-MET compared to the negative control. Cross-species specificity of the bispecific antibodies for human and macaque CD3 was demonstrated.

**26.6 Bioactivity of C-MET and CD3 cross-species specific bispecific single chain antibodies**

Bioactivity of the generated bispecific single chain antibodies was analyzed by chromium 51 ($^{51}$Cr) release in vitro cytotoxicity assays using the MDA-MB-231 cell line. As effector cells stimulated human CD4/CD56 depleted PBMC were used.
The generation of stimulated human PBMC was described herein above in Example 11. Target cells prepared and the assay was performed in analogy to the procedure described for the MCSPxCD3 bispecific single chain antibodies in example 11.
As shown in Figure 53 all of the generated cross-species specific bispecific single chain antibody constructs demonstrated cytotoxic activity against C-MET positive target cells elicited by stimulated human CD4/CD56 depleted PBMC.

**26.7 Generation of additional C-MET and CD3 cross-species specific bispecific single chain molecules**

The human antibody germline VH sequence VH1 1-03 (http://vbase.mrc-cpe.cam.ac.uk/) is chosen as framework context for CDRH1 (SEQ ID NO 821), CDRH2 (SEQ ID NO 822) and CDRH3 (SEQ ID NO 823). Likewise human antibody germline VH sequence VH1 1-46 (http://vbase.mrc-cpe.cam.ac.uk/) is chosen as framework context for CDRH1 (SEQ ID NO 836), CDRH2 (SEQ ID NO 837) and CDRH3 (SEQ ID NO 838). For each human VH several degenerated oligonucleotides have to be synthesized that overlap in a terminal stretch of approximately 15-20 nucleotides. To this end every second primer is an antisense primer. For VH1 1-03 the following oligonucleotides are used:

5'CM1-VH-A-XhoI (SEQ ID NO: 790)

CCA TGT CTC GAG TCT GGG SCT GAA STG RWG ARG CCT GGG GCT TCA GTG AAA RTG TCC TGC ARG GCT TCG GGC TAT ACC TTC

3'CM1-VH-B (SEQ ID NO: 791)

AT CCA CTC AAG CCY TTG TCC AGG CSY CTG TYT AAC CCA GTG CAA CCA GTA GCT GGT GAA GGT ATA GCC CGA AGC

5'CM1-VH-C (SEQ ID NO: 792)

GG CTT GAG TGG ATK GGC ATG ATT GAT CCT TCC AAT AGT GAC ACT AGG TTT AAT CCG AAC TTC AAG GAC

3'CM1-VH-D (SEQ ID NO: 793)

GCT GCT GAG CWS CAT GTA GGC TGT GYT GGM AGA TST GTC TMY AKT SAW TGT GRC CYT GTC CTT GAA GTT CGG ATT

5'CM1-VH-E (SEQ ID NO: 794)

GCC TAC ATG SWA CTC AGC AGC CTG ASA TCT GMG GAC ACT GCA GTC TAT TAC TGT GCC ASA TAT GGT AGC TAC GTT

3'CM1-VH-F-BstEII (SEQ ID NO: 795)

CAT GTA GGT GAC CGA GGT TCC TTG ACC CCA GTA GTC CAG AGG GGA AAC GTA GCT ACC ATA

For VH1 1-46 the oligonucleotides are as follows:

5' CM3-VH-A-XhoI (SEQ ID NO: 796)

CCA TGT CTC GAG TCT GGG RCT GAA STG RWG AAG CCT GGG GCT TCA GTG AAG STG TCC TGC AAG GCT TCT

3' CM3-VH-B (SEQ ID NO: 797)

CTC AAG GCC TTG TCC AGG CSY CTG CYT CAC CCA GTG TAT CCA GTA ACT GGT GAA GGT GTA GCC AGA AGC CTT GCA GGA

5' CM3-VH-C (SEQ ID NO: 798)

CCT GGA CAA GGC CTT GAG TGG ATK GGA GAG ATT AAT CCT AGC AGC GGT CGT ACTA AC TAC AAC GAG AAA TTC

3' CM3-VH-D (SEQ ID NO: 799)

C TGT GGA GGT AGA TKT GTC TMY AGT CAY TGT GAC CYT GTT CTT GAA TTT CTC GTT GTA GTT

5' CM3-VH-E (SEQ ID NO: 800)

A TCT ACC TCC ACA GYC TAC ATG SAA CTC AGC ARC CTG ASA TCT GAG GAC ACT GCG GTC TAT TAC TGT GCA

3' CM3-VH-F-BstEII (SEQ ID NO: 801)

CAT GTA GGT GAC CGT GGT GCC TTG GCC CCA GTA GCC CCT WCT TGC ACA GTA ATA GAC CGC

Each of these primer sets spans over the whole corresponding VH sequence. Within each set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72 °C for 10 minutes in a PCR cycler. Subsequently the product is run in an agarose gel electrophoresis and the product of a size from 200 to 400 isolated from the gel according to standard methods. Each VH PCR product is then used as a template for a standard PCR reaction using primers that incorporate N-terminal and C-terminal suitable cloning restriction sites. The DNA fragment of the correct size (for a VH approximately 350 nucleotides) is isolated by agarose gel electrophoresis according to standard methods. In this way sufficient VH DNA fragment is amplified.

The human antibody germline VL sequence VkII O11 (http://vbase.mrc-cpe.cam.ac.uk/) is chosen as framework context for CDRL1 (SEQ ID NO 816), CDRL2 (SEQ ID NO 817) and CDRL3 (SEQ ID NO 818). Likewise human antibody germline VL sequence VkII A1 (http://vbase.mrc-cpe.cam.ac.uk/) is chosen as framework context for CDRL1 (SEQ ID NO 833), CDRL2 (SEQ ID NO 834) and CDRL3 (SEQ ID NO 835). For each human VL several degenerated oligonucleotides have to be synthesized that overlap in a terminal stretch of approximately 15-20 nucleotides. To this end every second primer is an antisense primer. Restriction sites needed for later cloning within the oligonucleotides are deleted. For VkII O11 the following oligonucleotides are used:

5' CM1-VL-A-SacI (SEQ ID NO: 802)

CCT GTA GAG CTC GTG ATG ACC CAG ACT CCA YYC TCC CTA MCT GTG ACA SYT GGA GAG MMG GYT TCT RTC AGC TGC AAG TCC AGT

3' CM1-VL-B (SEQ ID NO: 803)

GTA CCA GGC CAA GTA GTT CTT CTG ACT GCT AGT ATA TAA AAG GGA CTG ACT GGA CTT GCA GCT GA

5' CM1-VL-C (SEQ ID NO: 804)

TAC TTG GCC TGG TAC CWG CAG AAA CCA GGT CAG TCT CCT MAA CTG CTG ATT TAC TGG GCA TCC ACT AGG

3' CM1-VL-D (SEQ ID NO: 805)

GAG AGT GAA ATC TGT CCC AGA TCC ACT GCC TGA GAA GCG ATC AGG GAC CCC AGA TTC CC TAGT GGA TGC CCA GTA

5' CM1-VL-E (SEQ ID NO: 806)

ACA GAT TTC ACT CTC AMA ATC TCC AGW GTG RAG GCT GAS GAC STG GSA GTT TAT TAC TGT CAG CAA TAT

3' CM1-VL-F-BsiWI/SpeI (SEQ ID NO: 807)

CCT CAG ACT AGT CGT ACG TTT GAT CTC CAA CTT TGT GCC TCC ACC GAA CGT CCA CGG ATA GGC ATA ATA TTG CTG ACA GTA ATA

For VkII A1 the oligonucleotides are as follows:

5' CM3-VL-A-SacI (SEQ ID NO: 808)

CCT GTA GAG CTC GTG ATG ACC CAA TCT CCA SYT TCT TTG SCT GTG ACT CTA GGG CAG CSG GCC TCC ATC TCC TGC

3' CM3-VL-Ba (SEQ ID NO: 809)

GAA CCA ACT CAT ATA ACT ACC ACC ATC ATA ATC AAC ACT TTG GCT GGC CTT GCA GGA GAT GGA GGC

3' CM3-VL-Bb (SEQ ID NO: 810)

GAA CCA ACT CAT ATA ACT ACC ACC ATC ATA ATC AAC ACT AGA TTG GCT GGC CTT GCA GGA GAT GGA GGC

5' CM3-VL-C (SEQ ID NO: 811)

AGT TAT ATG AGT TGG TTC CAA CAG AGA CCA GGA CAG YCA CCC ARA CKC CTC ATC TMT GCT GCA TCC AAT CTA

3' CM3-VL-D (SEQ ID NO: 812)
GGT GAA GTC TGT CCC AGA GCC ACT GCC ACT AAA CCT GKC TGG GAY CCC AGA TTC TAG ATT GGA TGC AGC

5' CM3-VL-E (SEQ ID NO: 813)

TCT GGG ACA GAC TTC ACC CTC AAK ATC YMT CST GTG GAG GMG GAG GAT GTT GSA RYC TAT TACT GT CAG CAA AGT

3' CM3-VL-F-BsiWI/SpeI (SEQ ID NO: 814)

CCT CAG ACT AGT CGT ACG TTT GAT CTC CAG CTT GGT CCC CTG ACC GAA CGT GAG CGG ATC CTC ATA ACT TTG CTG ACA GTA ATA

Each of these primer sets spans over the whole corresponding VL sequence.
Within each set primers are mixed in equal amounts (e.g. 1 µl of each primer (primer stocks 20 to 100 µM) to a 20 µl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72 °C for 10 minutes in a PCR cycler. Subsequently the product is run in an agarose gel electrophoresis and the product of a size from 200 to 400 isolated from the gel according to standard methods.
Each VL PCR product is then used as a template for a standard PCR reaction using primers that incorporate N-terminal and C-terminal suitable cloning restriction sites.
The DNA fragment of the correct size (for a VL approximately 330 nucleotides) is isolated by agarose gel electrophoresis according to standard methods. In this way sufficient VL DNA fragment is amplified.
The final VH1 1-03 -based VH PCR product (i.e. the repertoire of human/humanized VH) is then combined with the final VkII O11 -based VL PCR product (i.e. the repertoire of human/humanized VL) and the final VH1 1-46-based VH PCR product (i.e. the repertoire of human/humanized VH) with the final VkII A1-based VL PCR product (i.e. the repertoire of human/humanized VL) in the phage display vector pComb3H5Bhis to form two different libraries of functional scFvs from which - after display on filamentous phage - anti-C-MET binders are selected, screened, identified and confirmed as described in the following:

450 ng of the light chain fragments (SacI-SpeI digested) are ligated with 1400 ng of the phagemid pComb3H5Bhis (SacI-SpeI digested; large fragment). The resulting combinatorial antibody library is then transformed into 300 μl of electrocompetent Escherichia coli XL1 Blue cells by electroporation (2.5 kV, 0.2 cm gap cuvette, 25 μFD, 200 Ohm, Biorad gene-pulser) resulting in a library size of more than $10^7$ independent clones. After one hour of phenotype expression, positive transformants are selected for carbenicilline resistance encoded by the pComb3H5BHis vector in 100 ml of liquid super broth (SB)-culture over night. Cells are then harvested by centrifugation and plasmid preparation is carried out using a commercially available plasmid preparation kit (Qiagen).
2800 ng of this plasmid-DNA containing the VL-library (XhoI-BstEII digested; large fragment) are ligated with 900 ng of the heavy chain V-fragments (XhoI-BstEII digested) and again transformed into two 300 μl aliquots of electrocompetent E. coli XL1 Blue cells by electroporation (2.5 kV, 0.2 cm gap cuvette, 25 μFD, 200 Ohm) resulting in a total VH-VL scFv (single chain variable fragment) library size of more than $10^7$ independent clones.

After phenotype expression and slow adaptation to carbenicillin, the E. coli cells containing the antibody library are transferred into SB-carbenicillin (SB with 50 μg/mL carbenicillin) selection medium. The E. coli cells containing the antibody library are then infected with an infectious dose of $10^{12}$ particles of helper phage VCSM13 resulting in the production and secretion of filamentous M13 phage, wherein each phage particle contains single stranded pComb3H5BHis-DNA encoding a scFv-fragment and displays the corresponding scFv-protein as a translational fusion to phage coat protein III. This pool of phages displaying the antibody library is used for the selection of antigen binding entities.
For this purpose the phage library carrying the cloned scFv-repertoire is harvested from the respective culture supernatant by PEG8000/NaCl precipitation and centrifugation. Approximately $10^{11}$ to $10^{12}$ scFv phage particles are resuspended in 0.4 ml of PBS/0.1 % BSA and incubated with $10^5$ to $10^7$ C-MET transfected CHO cells (see example 26.1) for 1 hour on ice under slow agitation. These C-MET transfected CHO cells are harvested beforehand by centrifugation, washed in PBS and resuspended in PBS/1 % FCS (containing Na Azide). scFv phage which do not specifically bind to the C-MET transfected CHO cells are eliminated by up to five washing steps with PBS/1 % FCS (containing Na Azide). After washing, binding entities are eluted from the cells by resuspending the cells in HCl-glycine pH 2.2 (10 min incubation with subsequent vortexing) and after neutralization with 2 M Tris pH 12, the eluate is used for infection of a fresh uninfected E. coli XL1 Blue culture (OD600 > 0.5). The E. coli culture containing E. coli cells successfully transduced with a phagemid copy, encoding a human/humanized scFv-fragment, are again selected for carbenicillin resistance and subsequently infected with VCMS 13 helper phage to start the second round of antibody display and in vitro selection. A total of 4 to 5 rounds of selections are carried out, normally.

In order to screen for C-MET specific binders plasmid DNA corresponding to 4 and 5 rounds of panning is isolated from E. coli cultures after selection. For the production of soluble scFv-protein, VH-VL-DNA fragments are excised from the plasmids (XhoI-SpeI). These fragments are cloned via the same restriction sites into the plasmid pComb3H5BFlag/His differing from the original pComb3H5BHis in that the expression construct (e.g. scFv) includes a Flag-tag (DYKDDDDK) between the scFv and the His6-tag and the additional phage proteins are deleted. After ligation, each pool (different rounds of panning) of plasmid DNA is transformed into 100 μl heat shock competent E. coli TG1 or XLI blue and plated onto carbenicillin LB-agar. Single colonies are picked into 100 μl of LB carb (LB with 50 μg/ml carbenicillin).
E. coli transformed with pComb3H5BHis containing a VL-and VH-segment produce soluble scFv in sufficient amounts after excision of the gene III fragment and induction with 1 mM IPTG. Due to a suitable signal sequence, the scFv is exported into the periplasma where it folds into a functional conformation.
Single E. coli TG1 bacterial colonies from the transformation plates are picked for periplasmic small scale preparations and grown in SB-medium (e.g. 10 ml) supplemented with 20 mM $MgCl_2$ and carbenicillin 50μg/ml (and re-dissolved in PBS (e.g. 1 ml) after harvesting. A temperature shock is applied by four rounds of freezing at -70°C and thawing at 37°C whereby the outer membrane of the bacteria is destroyed and the soluble periplasmic proteins including the scFvs are released into the supernatant. After elimination of intact cells and cell-debris by centrifugation, the supernatant containing the anti-C-MET scFvs is collected and used for the identification of C-MET specific binders as follows:

Binding of scFvs to C-MET is tested by flow cytometry on C-MET transfected CHO cells (see example 247.1); untransfected CHO cells are use as negative control.

For flow cytometry 2.5x$10^5$ cells are incubated with 50 μl of scFv periplasmic preparation or with 5 μg/ml of purified scFv in 50 μl PBS with 2% FCS. The binding of scFv is detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl

PBS with 2% FCS (Dianova, Hamburg, FRG) is used. The samples are measured on a FACSscan (BD biosciences, Heidelberg, FRG).

Single clones are then analyzed for favourable properties and amino acid sequence. C-MET specific scFvs are converted into recombinant bispecific single chain antibodies by joining them via a Gly4$Ser_1$-linker with the CD3 specific scFv I2C (SEQ ID: 185) or any other CD3 specific scFv of the invention to result in constructs with the domain arrangement $VH_{C-MET}$ - $(Gly_4Ser_1)_3$ -$VL_{C-MET}$- $Ser_1Gly_4Ser_1$-$VH_{CD3}$ - $(Gly_4Ser_1)_3$- $VL_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal $His_6$-tag followed by a stop codon are both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). Transfection of the generated expression plasmids, protein expression and purification of cross-species specific bispecific antibody constructs are performed as described in Examples 26.3 and 26.4. All other state of the art procedures are carried out according to standard protocols (Sambrook, Molecular Cloning; A Laboratory Manual, 3rd edition, Cold Spring Harbour Laboratory Press, Cold Spring Harbour, New York (2001)).

Identification of functional bispecific single-chain antibody constructs is carried out by flow cytometric binding analysis of culture supernatant from transfected cells expressing the cross-species specific bispecific antibody constructs. Analysis is perfomed as described in Example 26.5 except that in addition C-MET transfected CHO cells as described in examples 26.1 and 26.2 are used.

Only those constructs showing bispecific binding to human and macaque CD3 as well as to C-MET are selected for further use.

Cytotoxic activity of the generated cross-species specific bispecific single chain antibody constructs against C-MET positive target cells elicited by effector cells is analyzed as described in Example 26.6 except that in addition C-MET transfected CHO cells as described in examples 26.1 and 26.2 are used as target cells and the macaque T cell line 4119LnPx is used as effector cells. Only those constructs showing potent recruitment of cytotoxic activity of effector cells against cells positive for C-MET are selected for further use.

**27. Generation and characterization of additional cMET and CD3 cross-species specific bispecific single chain molecules**

**27.1 Generation of CHO cells with enhanced expression of human cMET and CHO cells with enhanced expression of macaque cMET extracellular domains**

The modified coding sequences of human cMET and macaque cMET as described above were used for the construction of artificial cDNA sequences encoding fusion proteins of the extracellular domains of human cMET and macaque cMET, respectively, with a truncated variant of human EpCAM. To generate a construct for expression of these cMET fusion proteins cDNA fragments were obtained by gene synthesis according to standard protocols (the cDNA and amino acid sequence of the constructs is listed under SEQ ID NOs 767 and 777 for human cMET and SEQ ID NOs 788 and 789 for macaque cMET). The gene synthesis fragments were designed as to contain first a Kozak site for eukaryotic expression of the construct, followed by the coding sequence of a 19 amino acid immunoglobulin leader peptide, followed in frame by the the coding sequence of the human cMET or macaque cMET protein from amino acid 1 to 908 of the mature protein corresponding to the extracellular domains of human cMET and macaque cMET, respectively, followed in frame by the coding sequence of an artificial $Ser_1$-$Gly_4$-$Ser_1$-$Gly_1$-linker, followed in frame by the coding sequence of the transmembrane domain and intracellular domain of human EpCAM (as published in GenBank; Accession number NM_002354; amino acids 266 to 314 [as counted from the start codon] except for a point mutation at position 279 with isoleucine instead of valine) and a stop codon. The gene synthesis fragments were also designed as to introduce restriction sites at the beginning and at the end of the fragment. The introduced restriction sites, EcoRI at the 5' end and SalI at the 3' end, were utilized in the following cloning procedures. The gene synthesis fragments were cloned via EcoRI and SalI into a plasmid designated pEF-DHFR (pEF-DHFR is described in Raum et al. Cancer Immunol Immunother 50 (2001) 141-150) following standard protocols. Clones with sequence-verified nucleotide sequence were transfected into DHFR deficient CHO cells for eukaryotic expression of the constructs. Eukaryotic protein expression in DHFR deficient CHO cells was performed as described by Kaufmann R.J. (1990) Methods Enzymol. 185, 537-566. Gene amplification of the constructs was induced by increasing concentrations of methotrexate (MTX) to a final concentration of up to 20 nM MTX.

**27.2 Generation of cMET and CD3 cross-species specific bispecific single chain molecules**

**Cloning of cross-species specific binding molecules**

Bispecific single chain antibody molecules, with a binding specificity cross-species specific for human and non-chimpanzee primate CD3epsilon as well as a binding specificity cross-species specific for human and non-chimpanzee primate cMET, were designed as set out in the following Table 10:

Table 10: Formats of anti-CD3 and anti-cMET cross-species specific bispecific single chain antibody molecules

| SEQ ID (nucl/prot) | Formats of protein constructs (N → C) |
|---|---|
| 1413/1412/ | ME86H11HLxI2CHL |
| 1427/1426/ | ME62A12HLxI2CHL |
| 1441/1440 | ME63F2HLxI2CHL |
| 1455/1454 | ME62D11HLxI2CHL |
| 1469/1468 | ME62C10HLxI2CHL |
| 1483/1482 | ME62A4HLxI2CHL |

Generation, expression and purification of these cross-species specific bispecific single chain molecules was performed as described above.
The flow cytometric binding analysis of the cMET and CD3 cross-species specific bispecific antibodies was performed as described above. The bispecific binding of the single chain molecules listed above, which are cross-species specific for cMET and cross-species specific for human and non-chimpanzee primate CD3 was clearly detectable as shown in Figure 54. In the FACS analysis all constructs showed binding to CD3 and cMET compared to the negative control. Cross-species specificity of the bispecific antibodies to human and macaque CD3 and cMET antigens was demonstrated. Analysis of bioactivity by chromium 51 ($^{51}Cr$) release in vitro cytotoxicity assays is performed as described above. Based on demonstrated cross-species specific bispecific binding and recruited cytotoxicity cross-species specific binding molecules are selected for further use.

**27.3 Generation and flow cytometric binding analysis of cross-species specific single chain antibody fragments (scFv) binding to cMET**

scFv cross-species specific for cMET were generated as described above and designated as set out in the following Table 11:

Table 11: Designation of cross-species specific single chain antibody fragments

| SEQ ID (nucl/prot) | Designation |
|---|---|
| 1649/1648 | ME06F2HL |
| 1635/1634 | ME06E10HL |
| 1621/1620 | ME06D2HL |
| 1607/1606 | ME06D1HL |
| 1579/1578 | ME06C7HL |
| 1565/1564 | ME06C6HL |
| 1593/1592 | ME06B7HL |
| 1551/1550 | ME05F6HL |
| 1523/1522 | ME05D7HL |
| 1537/1536 | ME05B7HL |
| 1509/1508 | ME99B1 HL |
| 1495/1494 | ME75H6HL |

The flow cytometric binding analysis of periplasmic preparations containing scFv cross-species specific for cMET using CHO cells expressing human cMET as described in Example 27.1 and CHO cells expressing macaque cMET as described in Example 27.1 was performed as described above. The binding of the scFv listed above, which are cross-species specific for cMET was clearly detectable as shown in Figure 56. In the FACS analysis all constructs showed binding to cMET compared to the negative control. Cross-species specificity of the scFv antibodies to human and macaque cMET antigens was demonstrated.
Cloning of cross-species specific binding molecules based on the scFvs and expression and purification of these cross-species specific bispecific single chain molecules is performed as described above. Flow cytometric analysis of cross-species specific bispecific binding and analysis of bioactivity by chromium 51 ($^{51}$Cr) release in vitro cytotoxicity assays is performed as described above. Based on demonstrated cross-species specific bispecific binding and recruited cytotoxicity cross-species specific binding molecules are selected for further use.

Human/humanized equivalents of non-human scFvs cross-species specific for cMET contained in the selected cross-species specific bispecific single chain molecules are generated as described herein. Cloning of cross-species specific binding molecules based on these human/humanized scFvs and expression and purification of these cross-species specific bispecific single chain molecules is performed as described above. Flow cytometric analysis of cross-species specific bispecific binding and analysis of bioactivity by chromium 51 ($^{51}$Cr) release in vitro cytotoxicity assays is performed as described above. Based on demonstrated cross-species specific bispecific binding and recruited cytotoxicity cross-species specific binding molecules are selected for further use.

## 28. Generation and characterization of Endosialin and CD3 cross-species specific bispecific single chain antibody molecules

### 28.1 Generation of CHO cells expressing human Endosialin

The coding sequence of human Endosialin as published in GenBank (Accession number NM_020404) was obtained by gene synthesis according to standard protocols. The gene synthesis fragment was designed as to contain first a Kozak site for eukaryotic expression of the construct, followed by the coding sequence of human Endosialin, followed in frame by the coding sequence of a FLAG tag and a stop codon (the cDNA and amino acid sequence of the construct is listed under SEQ ID Nos 913 and 914). The gene synthesis fragment was also designed as to introduce restriction sites at the beginning and at the end of the fragment. The introduced restriction sites, EcoRI at the 5' end and XbaI at the 3' end, were utilised in the following cloning procedures. The gene synthesis fragment was cloned via EcoRI and XbaI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 28.2 Generation of CHO cells expressing macaque Endosialin

The cDNA sequence of macaque Endosialin was obtained by a set of 2 PCRs on cDNA from macaque monkey (cynomolgus) colon prepared according to standard protocols. The following reaction conditions: 1 cycle at 95°C for 5 minutes followed by 40 cycles with 95°C for 45 seconds, 50°C for 45 seconds and 72 °C for 2 minutes followed by a terminal cycle of 72 °C for 5 minutes and the following primers were used for the first PCR:

forward primer: 5'-atatgaattcgccaccatgctgctgcgcctgttgctggcc-3' SEQ ID NO 917
reverse primer: 5'-gtcttcatcttcctcatcctcccc-3' SEQ ID NO 918

The following reaction conditions: 1 cycle at 95°C for 5 minutes followed by 40 cycles with 95°C for 45 seconds, 58°C for 45 seconds and 72 °C for 2 minutes followed by a terminal cycle of 72 °C for 5 minutes and the following primers were used for the second PCR:

forward primer: 5'-gtcaactacgttggtggcttcgagtg-3' SEQ ID NO 919
reverse primer: 5'-ggtctagatcacttatcgtcatcatctttgtagtccacgctggttctgcaggtctgc-3' SEQ ID NO 920

The PCR reactions were performed under addition of PCR grade betain to a final concentration of 1 M. Those PCRs generated two overlapping fragments, which were isolated and sequenced according to standard protocols using the PCR primers, and thereby provided a portion of the cDNA sequence coding macaque Endosialin from codon 9 of the leader peptide to codon 733 of the mature protein. To generate a construct for expression of macaque Endosialin a cDNA fragment was obtained by gene synthesis according to standard protocols (the cDNA and amino acid sequence of the construct is listed under SEQ ID Nos 915 and 916). In this construct the coding sequence of macaque Endosialin from amino acid 9 of the leader peptide to amino acid 733 of the mature Endosialin protein, followed in frame by the coding sequence of amino acid 734 to the last amino acid of the mature human Endosialin protein, followed in frame by the coding sequence of a FLAG tag and a stop codon was fused in frame to the coding sequence of the amino acids 1 to 8 of the leader peptide of the human Endosialin protein. The gene synthesis fragment was also designed as to contain a Kozak site for eukaryotic expression of the construct and restriction sites at the beginning and the end of the fragment containing the cDNA. The introduced restriction sites, EcoRI at the 5' end and XbaI at the 3' end, were utilised in the following cloning procedures. The gene synthesis fragment was cloned via EcoRI and XbaI into a plasmid designated pEF-DHFR (pEF-DHFR is described in Raum et al. Cancer Immunol Immunother 50 (2001) 141-150) following standard protocols.

The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 28.3 Cross-species specific binding to Endosialin of a scFv-antibody fragment

The cDNA of a scFv-antibody fragment was obtained by gene synthesis according to standard protocols. The cDNA fragment was cloned via suitable restriction sites into the plasmid pComb3H5BFlag/His differing from the original pComb3H5BHis (described below) in that the expression construct (e.g. scFv) includes a Flag-tag (DYKDDDDK SEQ ID NO 933) between the scFv-fragment and the His6-tag and the additional phage proteins are deleted. After ligation, a sequence verified clone of the plasmid DNA was transformed into 100 μl heat shock competent E. coli TG1 or XLI blue and plated onto carbenicillin LB-agar. Single colonies were picked into 100 μl of LB carb (LB with 50 μg/ml carbenicillin).

After induction with 1 mM IPTG E. coli transformed with pComb3H5BFlag/His containing the coding sequence of the cross-species specific single-chain antibody produced soluble scFv in sufficient amounts. Due to a suitable signal sequence, the scFv-chain is exported into the periplasma where it folds into a functional conformation.

Single E. coli bacterial colonies from the transformation plates were picked for periplasmic small scale preparations and grown in SB-medium (e.g. 10 ml) supplemented with 20 mM $MgCl_2$ and carbenicillin 50μg/ml (and re-dissolved in PBS (e.g. 1 ml) after harvesting. A temperature shock was applied by four rounds of freezing at -70°C and thawing at 37°C whereby the outer membrane of the bacteria is destroyed and the soluble periplasmic proteins including the scFv-molecules are released into the supernatant. After elimination of intact cells and cell-debris by centrifugation the supernatant containing the scFv-antibody fragment was collected and used in the subsequent flow cytometric binding analysis with the CHO cells transfected with human Endosialin as described in Example 28.1 and the macaque Endosialin transfectant described in Example 28.2.

To this end 200.000 cells of the respective cell lines were incubated for 30 min on ice with 50 μl of the periplasmic preparation containing the cross-species specific single-chain antibody. The cells were washed twice in PBS with 2% FCS and binding of the construct was detected with a murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in PBS with 2% FCS. Untransfected CHO cells were used as a negative control. Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

As shown in Figure 46, specific binding of the scFv-antibody fragment to both, human and macaque Endosialin could be demonstrated compared to the negative control.

### 28.4 Generation of Endosialin and CD3 cross-species specific bispecific single chain molecules

The human antibody germline VH sequence VH1 1-03 (http://vbase.mrc-cpe.cam.ac.uk/) is chosen as framework context for CDRH1 (SEQ ID 910), CDRH2 (SEQ ID 911) and CDRH3 (SEQ ID 912). For the human VH several degenerated oligonucleotides have to be synthesized that overlap in a terminal stretch of approximately 15-20 nucleotides. To this end every second primer is an antisense primer. For VH1 1-03 the following set of oligonucleotides is used:

5'ED-VH-A-XhoI

CCA GCT CTC GAG TCA GGA SCT GAG STG RWG AAG CCT GGG GCT TCA GTG AAG RTG TCC TGC AAG GCT TCT GGA TAC ACA TTC ACT SEQ ID NO 921

3'ED-VH-B

AAT ATA TCC MAT CCA CTC AAG GCK CTK TCC AKK TSY CTG CYT CAY CCA GTG TAT AAC ATA GTC AGT GAA TGT GTA TCC AGA SEQ ID NO 922

5'ED-VH-C

CTT GAG TGG ATK GGA TAT ATT AAT CCT TAT GAT GAT GAT ACTA CC TAC AAC CAG AAG TTC AAG GGC SEQ ID NO 923

3'ED-VH-D

T GAG CTS CAT GTA GGC TGT GYT GGM GGA TKT GWC TMS AGT MAW TGT GRC CYG GCC CTT GAA CTT CTG GTT SEQ ID NO 924

5'ED-VH-E

C ACA GCC TAC ATG SAA CTC ARC AGC CTG ASA TCT GAG GAC ACT GCA GTC TAT TAC TGT GCA AGA AGG GGG SEQ ID NO 925

3'ED-VH-F-BstEII

CCT GAT GGT GAC CAA GGT TCC TTG ACC CCA GTA GTC CAT AGA ATA GTC GAA GTA ACC ATC ATA GGA GTT CCC CCT TCT TGC ACA GTA SEQ ID NO 926

This primer set spans over the whole corresponding VH sequence.
Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.
The VH PCR product is then used as a template for a standard PCR reaction using primers that incorporate N-terminal and C-terminal suitable cloning restriction sites. The DNA fragment of the correct size (for a VH approximately 350 nucleotides) is isolated by agarose gel electrophoresis according to standard methods. In this way sufficient VH DNA fragment is amplified.
The human antibody germline VL sequence VkI L8 (http://vbase.mrc-cpe.cam.ac.uk/) is chosen as framework context for CDRL1 (SEQ ID 907), CDRL2 (SEQ ID 908) and CDRL3 (SEQ ID 909). For the human VL several degenerated oligonucleotides have to be synthesized that overlap in a terminal stretch of approximately 15-20 nucleotides. To this end every second primer is an antisense primer. Restriction sites needed for later cloning within the oligonucleotides are deleted. For VkI L8 the following oligonucleotides are used:

5'ED-VL-A-SacI

CCA GTC GAG CTC CAG CTG ACC CAG TCT CMA ARM TTC MTG TCC RCA TCA GTA GGA GAC AGA GTC NNS ATC ACC TGC AGG GCC AG (SEQ ID NO 927)

3'ED-VL-B

TCC TGG TTT CTG TTG ATA CCA GGC TAC AGC AGT ACC CAC ATT CTG ACT GGC CCT GCA GGT GAT (SEQ ID NO 928)

5'ED-VL-C

TAT CAA CAG AAA CCA GGA MAA KCC CCT AAA TTA CTG ATT TACT CG GCA TCG AAT CGG TAC ACT GGA GTC CCT (SEQ ID NO 929)

3'ED-VL-D

GCT GAT GGT GAG AGT GAA MTC TGT CCC AGA TCC ACT GCC TGA GAA GCG AYY AGG GAC TCC AGT GTA CCG (SEQ ID NO 930)

5'ED-VL-E

TTC ACT CTC ACC ATC AGC ART MTG CAG YCT GAA GAC YTS GCA RMT TAT TWC TGC CAG CAA TAT ACC AAC (SEQ ID NO 931)

3'ED-VL-F-BsiWI/SpeI

CCT GAT ACT AGT CGT ACG TTT TAT TTC CAG CTT GGT CCC CTG TCC AAA CGT ATA CAT GGG ATA GTT GGT ATA TTG CTG GCA (SEQ ID NO 932)

This primer set spans over the whole corresponding VL sequence.
Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62 °C for 1 minute, 59 °C for 1 minute, 56 °C for 1 minute, 52 °C for 1 minute, 50 °C for 1 minute and at 72 °C for 10 minutes in a PCR cycler. Subsequently the product is run in an agarose gel electrophoresis and the product of a size from 200 to 400 isolated from the gel according to standard methods.
The VL PCR product is then used as a template for a standard PCR reaction using primers that incorporate N-terminal and C-terminal suitable cloning restriction sites. The DNA fragment of the correct size (for a VL approximately 330 nucleotides) is isolated by agarose gel electrophoresis according to standard methods. In this way sufficient VL DNA fragment is amplified.
The final VH1 1-03 -based VH PCR product (i.e. the repertoire of human/humanized VH) is then combined with the final Vkl L8 -based VL PCR product (i.e. the repertoire of human/humanized VL) in the phage display vector pComb3H5Bhis to form a library of functional scFvs from which - after display on filamentous phage - anti-Endosialin binders are selected, screened, identified and confirmed as described in the following:

450 ng of the light chain fragments (SacI-SpeI digested) are ligated with 1400 ng of the phagemid pComb3H5Bhis (SacI-SpeI digested; large fragment). The resulting combinatorial antibody library is then transformed into 300 μl of electrocompetent Escherichia coli XL1 Blue cells by electroporation (2.5 kV, 0.2 cm gap cuvette, 25 μFD, 200 Ohm, Biorad gene-pulser) resulting in a library size of more than $10^7$ independent clones. After one hour of phenotype expression, positive transformants are selected for carbenicillin resistance encoded by the pComb3H5BHis vector in 100 ml of liquid super broth (SB)-culture over night. Cells are then harvested by centrifugation and plasmid preparation is carried out using a commercially available plasmid preparation kit (Qiagen).

2800 ng of this plasmid-DNA containing the VL-library (XhoI-BstEII digested; large fragment) are ligated with 900 ng of the heavy chain V-fragments (XhoI-BstEII digested) and again transformed into two 300 μl aliquots of electrocompetent E. coli XL1 Blue cells by electroporation (2.5 kV, 0.2 cm gap cuvette, 25 μFD, 200 Ohm) resulting in a total VH-VL scFv (single chain variable fragment) library size of more than $10^7$ independent clones.

After phenotype expression and slow adaptation to carbenicillin, the E. coli cells containing the antibody library are transferred into SB-carbenicillin (SB with 50 μg/mL carbenicillin) selection medium. The E. coli cells containing the antibody library are then infected with an infectious dose of $10^{12}$ particles of helper phage VCSM13 resulting in the production and secretion of filamentous M13 phage, wherein each phage particle contains single stranded pComb3H5BHis-DNA encoding a scFv-fragment and displays the corresponding scFv-protein as a translational fusion to phage coat protein III. This pool of phages displaying the antibody library is used for the selection of antigen binding entities.

For this purpose the phage library carrying the cloned scFv-repertoire is harvested from the respective culture supernatant by PEG8000/NaCl precipitation and centrifugation. Approximately $10^{11}$ to $10^{12}$ scFv phage particles are resuspended in 0.4 ml of PBS/0.1% BSA and incubated with $10^5$ to $10^7$ Endosialin transfected CHO cells (see example 28.1) for 1 hour on ice under slow agitation. These Endosialin transfected CHO cells are harvested beforehand by centrifugation, washed in PBS and resuspended in PBS/1 % FCS (containing Na Azide). scFv phage which do not specifically bind to the Endosialin transfected CHO cells are eliminated by up to five washing steps with PBS/1 % FCS (containing Na Azide). After washing, binding entities are eluted from the cells by resuspending the cells in HCl-glycine pH 2.2 (10 min incubation with subsequent vortexing) and after neutralization with 2 M Tris pH 12, the eluate is used for infection of a fresh uninfected E. coli XL1 Blue culture (OD600 > 0.5). The E. coli culture containing E. coli cells successfully transduced with a phagemid copy, encoding a human/humanized scFv-fragment, are again selected for carbenicillin resistance and subsequently infected with VCMS 13 helper phage to start the second round of antibody display and in vitro selection. A total of 4 to 5 rounds of selections are carried out, normally.

In order to screen for Endosialin specific binders plasmid DNA corresponding to 4 and 5 rounds of panning is isolated from E. coli cultures after selection. For the production of soluble scFv-protein, VH-VL-DNA fragments are excised from the plasmids (XhoI-SpeI). These fragments are cloned via the same restriction sites into the plasmid pComb3H5BFlag/His differing from the original pComb3H5BHis in that the expression construct (e.g. scFv) includes a Flag-tag (DYKDDDDK) between the scFv and the His6-tag and the additional phage proteins are deleted. After ligation, each pool (different rounds of panning) of plasmid DNA is transformed into 100 μl heat shock competent E. coli TG1 or XLI blue and plated onto carbenicillin LB-agar. Single colonies are picked into 100 μl of LB carb (50 μg/ml).

After induction with 1 mM IPTG E. coli transformed with pComb3H5BFlag/His containing a VL-and VH-segment produce soluble scFv in sufficient amounts. Due to a suitable signal sequence, the scFv-chain is exported into the periplasma where it folds into a functional conformation.

Single E. coli TG1 bacterial colonies from the transformation plates are picked for periplasmic small scale preparations and grown in SB-medium (e.g. 10 ml) supplemented with 20 mM $MgCl_2$ and carbenicillin 50 μg/ml (and re-dissolved in PBS (e.g. 1 ml) after harvesting. A temperature shock is applied by four rounds of freezing at -70°C and thawing at 37°C whereby the outer membrane of the bacteria is destroyed and the soluble periplasmic proteins including the scFvs are released into the supernatant. After elimination of intact cells and cell-debris by centrifugation, the supernatant containing the anti-Endosialin scFvs is collected and used for the identification of Endosialin specific binders as follows:

Binding of scFvs to Endosialin is tested by flow cytometry on Endosialin transfected CHO cells (see example 28.1); untransfected CHO cells are use as negative control.

For flow cytometry $2.5x10^5$ cells are incubated with 50 μl of scFv periplasmic preparation or with 5 μg/ml of purified scFv in 50 μl PBS with 2% FCS. The binding of scFv is detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) is used. The samples are measured on a FACSscan (BD biosciences, Heidelberg, FRG).

Single clones are then analyzed for favourable properties and amino acid sequence. Endosialin specific scFvs are converted into recombinant bispecific single chain antibodies by joining them via a Gly4Ser1-linker with the CD3 specific scFv 12C (SEQ ID NO: 185) or any other CD3 specific scFv of the invention to result in constructs with the domain arrangement VH Endosialin - (Gly4Ser1)3 -VL Endosialin - Ser1Gly4Ser1-VHCD3 - (Gly4Ser1)3 - VLCD3 or alternative domain arrangements.

For expression in CHO cells the coding sequences of (i) an N-terminal immunoglobulin heavy chain leader comprising a start codon embedded within a Kozak consensus sequence and (ii) a C-terminal His6-tag followed by a stop codon are both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). A clone with sequence-verified nucleotide sequence is transfected into DHFR deficient CHO cells for eukaryotic expression of the construct. Eukaryotic protein expression in DHFR deficient CHO cells is performed as described by Kaufmann R.J. (1990) Methods Enzymol. 185, 537-566. Gene amplification of the construct is induced by increasing concentrations of methotrexate (MTX) to a final concentration of up to 20 nM MTX.

**28.5 Expression and purification of bispecific single chain antibody molecules**

Bispecific single chain antibody molecules are expressed in Chinese hamster ovary cells (CHO or HEK 293 cells as described herein above for the MCSPxCD3 bispecific single chain antibodies.
The isolation and analysis of the expressed bispecific single chain antibodies has also been described herein above in Example 9.

**28.6 Flow cytometric binding analysis of the Endosialin and CD3 cross-species specific bispecific antibodies**

In order to test the functionality of cross-species specific bispecific antibody constructs regarding the capability to bind to human and macaque Endosialin and CD3, respectively, a FACS analysis is performed. For this purpose CHO cells transfected with human Endosialin as described in Example 28.1 and the human CD3 positive T cell leukemia cell line HPB-ALL (DSMZ, Braunschweig, ACC483) are used to test the binding to human antigens. The binding reactivity to macaque antigens is tested by using the generated macaque Endosialin transfectant described in Example 28.2 and a macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61). 200.000 cells of the respective cell lines are incubated for 30 min on ice with 50 μl of cell culture supernatant of transfected cells expressing the cross-species specific bispecific antibody constructs. The cells are washed twice in PBS with 2% FCS and binding of the construct is detected with a murine Penta His antibody (Qiagen; diluted 1:20 in 50 μl PBS with 2% FCS). After washing, bound anti His antibodies are detected with an Fc gamma-specific antibody (Dianova) conjugated to phycoerythrin, diluted 1:100 in PBS with 2% FCS. Supernatant of untransfected cells is used as a negative control.
Flow cytometry is performed on a FACS-Calibur apparatus, the CellQuest software is used to acquire and analyze the data (Becton Dickinson biosciences, Heidelberg). FACS staining and measuring of the fluorescence intensity are performed as described in Current Protocols in Immunology (Coligan, Kruisbeek, Margulies, Shevach and Strober, Wiley-Interscience, 2002).
Only those constructs showing bispecific binding to human and macaque CD3 as well as to Endosialin are selected for further use.

**28.7 Bioactivity of Endosialin and CD3 cross-species specific bispecific single chain antibodies**

Bioactivity of generated bispecific single chain antibodies is analyzed by chromium 51 ($^{51}$Cr) release in vitro cytotoxicity assays using the CHO cells transfected with human Endosialin described in Example 28.1 and the CHO cells transfected with macaque Endosialin described in Example 28.2. As effector cells stimulated human CD4/CD56 depleted PBMC or the macaque T cell line 4119LnPx are used, respectively.
The generation of stimulated human PBMC was described herein above in Example 11.
Target cells prepared and the assay was performed in analogy to the procedure described for the MCSPxCD3 bispecific single chain antibodies in example 11.
Only those constructs showing potent recruitment of cytotoxic activity of effector cells against cells positive for Endosialin are selected for further use.

**29. Generation and characterization of CD248 (Endosialin) and CD3 cross-species specific bispecific single chain molecules**

**29.1 Generation of CD248 and CD3 cross-species specific bispecific single chain molecules**

**Cloning of cross-species specific binding molecules**

Bispecific single chain antibody molecules, with a binding specificity cross-species specific for human and non-chimpanzee primate CD3epsilon as well as a binding specificity cross-species specific for human and non-chimpanzee primate CD248, were designed as set out in the following Table 13:

Table 13: Formats of anti-CD3 and anti-CD248 cross-species specific bispecific single chain antibody molecules

| SEQ ID (nucl/prot) | Formats of protein constructs (N → C) |
|---|---|
| 1665/1664 | EN00B12HLxI2CHL |
| 1679/1678 | EN00C3HLxI2CHL |
| 1693/1692 | EN01D5HLxI2CHL |
| 1707/1706 | EN00E4HLxI2CHL |
| 1721/1720 | EN00F7HLxI2CHL |
| 1735/1734 | EN00H6HLxI2CHL |

Generation, expression and purification of these cross-species specific bispecific single chain molecules was performed as described above.
The flow cytometric binding analysis of the CD248 and CD3 cross-species specific bispecific antibodies was performed as described above. The bispecific binding of the single chain molecules listed above, which are cross-species specific for CD248 and cross-species specific for human and non-chimpanzee primate CD3 was clearly detectable as shown in Figure 58. In the FACS analysis all constructs showed binding to CD3 and CD248 compared to the negative control. Cross-species specificity of the bispecific antibodies to human and macaque CD3 and CD248 antigens was demonstrated.
Bioactivity of the generated bispecific single chain antibodies was analyzed by chromium 51 ($^{51}Cr$) release in vitro cytotoxicity assays as described above. As shown in Figure 59 all of the generated cross-species specific bispecific single chain antibody constructs demonstrated cytotoxic activity against human CD248 positive target cells elicited by stimulated human CD4/CD56 depleted PBMC and macaque CD248 positive target cells elicited by the macaque T cell line 4119LnPx.

**30. Generation and characterization of EpCAM and CD3 cross-species specific bispecific single chain antibody molecules**

**30.1 Cloning and expression of human EpCAM antigen on CHO cells**

The sequence of the human EpCAM antigen ('NM_002354, Homo sapiens tumor-associated calcium signal transducer 1 (TACSTD1), mRNA, National Center for Biotechnology Information, http://www.ncbi.nlm.nih.gov/entrez) was used to obtain a synthetic molecule by gene synthesis according to standard protocols. The gene synthesis fragment was also designed as to contain a Kozak site for eukaryotic expression of the construct and and restriction sites at the beginning and the end of the DNA. The introduced restriction sites XbaI at the 5' end and SalI at the 3' end were utilised during the cloning step into the expression plasmid designated pEFDHFR as described in Raum et al. (loc cit.). After sequence verification the plasmid was used to transfect CHO/dhfr- cells as follows. A sequence verified plasmid was used to transfect CHO/dhfr- cells (ATCC No. CRL 9096; cultivated in RPMI 1640 with stabilized glutamine obtained from Biochrom AG Berlin, Germany, supplemented with 10% FCS, 1% penicillin/streptomycin all obtained from Biochrom AG Berlin, Germany and nucleosides from a stock solution of cell culture grade reagents obtained from Sigma-Aldrich Chemie GmbH, Taufkirchen, Germany, to a final concentration of 10 μg/ml Adenosine, 10 μg/ml Deoxyadenosine and 10 μg/ml Thymidine, in an incubator at 37°C, 95% humidity and 7% $CO_2$). Transfection was performed using the PolyFect Transfection Reagent (Qiagen GmbH, Hilden, Germany) and 5 μg of plasmid DNA according to the manufacturer's protocol. After culturing for 24 hours cells were washed once with PBS and again cultured in the aforementioned cell culture medium except that the medium was not supplemented with nucleosides and dialysed FCS (obtained from Biochrom AG Berlin, Germany) was used. Thus the cell culture medium did not contain nucleosides and thereby selection was applied on the transfected cells. Approximately 14 days after transfection the outgrowth of resistant cells was observed. After an additional 7 to 14 days the transfectants were tested positive for EpCAM-expression by FACS. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

**30.2 Generation of EpCAM and CD3 cross-species specific bispecific single chain molecules**

Generally, bispecific single chain antibody molecules, each comprising a domain with a binding specificity for the human and the macaque CD3 antigen as well as a domain with a binding specificity for the human EPCAM antigen, were designed as set out in the following Table 14:

Table 14: Formats of anti-CD3 and anti-EpCAM cross-species specific bispecific single chain antibody molecules

| SEQ ID (nucl/prot) | Formats of protein constructs (N → C) |
|---|---|
| 945/944 | Ep 5-10 LH x I2C HL |
| 949/948 | Ep 5-10 LH x F12Q HL |
| 947/946 | Ep 5-10 LH x H2C HL |
| 961/960 | hEp 14-A1 LH x I2C HL |
| 973/972 | hEp 14-D2 LH x I2C HL |
| 985/984 | hEp 14-H8 LH x I2C HL |
| 997/996 | hEp 14-A6 LH x I2C HL |
| 1009/1008 | hEp 14-01 LH x I2C HL |
| 1033/1032 | hEp 14-H4 LH x I2C HL |
| 1045/1044 | hEp 17-A6 LH x I2C HL |
| 1057/1056 | hEp 17-E9 LH x I2C HL |
| 1079/1078 | hEp 18-E3 LH x I2C HL |
| 1091/1090 | hEp 18-F11 LH x I2C HL |
| 1103/1102 | hEp 18-F12 LH x I2C HL |
| 1115/1114 | hEp 18-G1 LH x I2C HL |
| 1127/1126 | hEp 18-G9 LH x I2C HL |
| 1021/1020 | hEp 14-G6 LH x I2C HL |
| 1777/1776 | hEp 18-A6 LH x I2C HL |

The aforementioned constructs containing the variable light-chain (L) and variable heavy-chain (H) domains specific for human EpCAM and the CD3 specific VH and VL combinations cross-species specific for human and macaque CD3 were obtained by gene synthesis. The gene synthesis fragments were designed and eukaryotic protein expression was performed in analogy to the procedure described in example 9 for the MCSPxCD3 cross-species specific single chain molecules.

### 30.3 Expression and purification of the single domain bispecific single chain antibody molecules

The single domain bispecific single chain antibody molecules were expressed in Chinese hamster ovary cells (CHO) or HEK 293 cells as described herein above for the MCSPxCD3 bispecific single chain antibodies.
The isolation and analysis of the expressed bispecific single chain antibodies has also been described herein above in Example 9.

### 30.4 Flow cytometric binding analysis of the EpCAM and CD3 cross-species specific bispecific antibodies

In order to test the functionality of the cross-species specific bispecific antibody constructs with regard to binding capability to human EpCAM and human / macaque CD3, a FACS analysis was performed. For this purpose the CHO cells transfected with human EpCAM as described in Example 30.1 and human CD3 positive T cell leukemia cell line HPB-ALL (DSMZ, Braunschweig, ACC483) were used to check the binding to human antigens. The binding reactivity to macaque CD3 was tested by using a macaque T cell line 4119LnPx (kindly provided by Prof Fickenscher, Hygiene Institute, Virology, Erlangen-Nuernberg; Knappe A, et al., and Fickenscher H: Herpesvirus saimiri-transformed macaque T cells are tolerated and do not cause lymphoma after autologous reinfusion. Blood 2000;95:3256-61.) 200,000 cells of the respective cell population were incubated for 30 min on ice with 50 μl of the purified protein of the cross-species specific bispecific antibody constructs (e. g. 2 μg/ml) Alternatively the cell culture supernatant of transiently produced proteins was used.

The cells were washed twice in PBS and binding of the construct was detected with an unlabeled murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in 50 μl PBS with 2% FCS. Fresh culture medium was used as a negative control.
Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

The binding ability of all EpCAM-directed bispecific single chain molecules were clearly detectable as shown in Figure 60. In the FACS analysis, all constructs showed binding to human and macaque CD3 and to human EpCAM compared to the negative control using culture medium and 1. and 2. detection antibody. In summary, the cross-species specificity of the bispecific antibody to human and macaque CD3 and human EpCAM could clearly be demonstrated.

### 30.5 Bioactivity of EpCAM and CD3 cross-species specific bispecific single chain antibodies

Bioactivity of the generated bispecific single chain antibodies was analyzed by chromium 51 release in vitro cytotoxicity assays using the EpCAM positive cell line described in example 30.1. As effector cells stimulated human CD8 positive T cells or the macaque T cell line 4119LnPx were used.
Stimulated CD8+ T cells were obtained as follows:
The generation of stimulated human PBMC was described herein above in Example 11.
Target cells prepared and the assay was performed in analogy to the procedure described for the MCSPxCD3 bispecific single chain antibodies in example 11.
As shown in Figures 61 to 66, all of the indicated cross-species specific bispecific single chain antibody constructs revealed cytotoxic activity against human EpCAM positive target cells elicited by human CD8+ cells and against human EpCAM positive target cells elicited by the macaque T cell line 4119LnPx. As a negative control, an irrelevant bispecific single chain antibody was used.

### 30.6 Cloning and expression of murine EpCAM antigen and a human-murine EpCAM hybrid antigen on CHO cells

The sequence of the mouse EpCAM antigen ('NM 008532, Mus musculus tumor-associated calcium signal transducer 1 (Tacstd1), mRNA., National Center for Biotechnology Information, http://www.ncbi.nlm.nih.gov/entrez) was used to obtain a synthetic molecule by gene synthesis according to standard protocols. The gene synthesis fragment was also designed as to contain a Kozak site for eukaryotic expression of the construct and restriction sites at the beginning and the end of the DNA. The introduced restriction sites XbaI at the 5' end and SalI at the 3' end were utilised during the cloning step into the expression plasmid designated pEFDHFR.
After sequence verification the plasmid was used to transfect CHO/DHFR$^-$ cells as described in example 30.1. The human-murine EpCAM hybrid antigen was generated by exchanging the murine Exon 2 (amino acids no 26 - 61) for the human Exon 2 amino acids to enable the identification of the epitopes recognised by the cross-species specific bispecific antibody constructs. The deduced sequence was used to obtain a synthetic molecule by gene synthesis and CHO cells were transfected as described above.

### 30.7 Flow cytometric binding analysis of the EpCAM and CD3 cross-species specific bispecific antibodies to different EpCAM antigens

In order to analyze the binding ability of the cross-species specific bispecific antibody constructs with regard to binding abilities to different epitopes on human or murine or on a human-mouse hybrid EpCAM, a FACS flow cytometry was performed. For this purpose the CHO cells transfected with human EpCAM as described in example 1, Cho cells transfected with murine EpCAM (example 30.6) and CHO cells transfected with a human-mouse EpCAM hybrid (example 30.6) were used to check the different binding patterns. 200,000 cells of the respective cell population were incubated for 30 min on ice with 50 μl of the cell culture supernatant of transiently produced proteins.
The cells were washed twice in PBS and binding of the construct was detected with an unlabeled murine Penta His antibody (Qiagen; diluted 1:20 in 50 μl PBS with 2% FCS). After washing, bound anti His antibodies were detected with an Fc gamma-specific antibody (Dianova) conjugated to phycoerythrin, diluted 1:100 in 50 μl PBS with 2% FCS. Fresh culture medium was used instead of cell culture supernatant from CHO cells transfected with the respective bispecific single chain antibody molecules as a negative control. As positive control for the expression of the murine EpCAM the detection with a rat derived unlabeled antibody specific for murine EpCAM (BD Pharmingen, #552370, Rat IgG2a,k) followed by PE labelled anti Rat IgG2a, κ specific antibody (BD Pharmingen, Heidelberg, #553930) was used.
Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002). For the purpose of comparison the median values of the fluorescence intensity was used to generate a bar chart.

The binding ability of all EpCAM-directed bispecific single chain molecules to CHO cells transfected with human EpCAM was clearly detectable as shown in Figure 67.

None of the human EpCAM specific molecules elicited a median value significantly above the negative control when binding to murine EpCAM was analysed. The binding properties of the human EpCAM specific single chain bispecific antibody molecules to human-mouse EpCAM hybrid antigen revealed two subsets of molecules: Those without a detectable binding to the human-mouse EpCAM hybrid antigen (HD69 HL x I2C HL) and those with a median fluorescence intensity nearly at the level of the binding strength to the human EpCAM antigen (hEp 14-A1 LH x I2C HL; hEp 14-H8 LH x I2C HL; hEp 14-A6 LH x I2C HL; hEp 14-D1 LH x I2C HL; hEp 14-H4 LH x I2C HL; hEp 17-A6 LH x I2C HL; hEp 17-E9 LH x I2C HL; hEp 18-E3 LH x I2C HL; hEp 18-F11 LAH x I2C HL; hEp 18-F12 LH x I2C HL; hEp 18-G1 LH x I2C HL; hEp 18-G9 LH x I2C HL; for SEQ ID NOs of the constructs see table 14). The divergent binding patterns of these two subsets of human EpCAM specific single chain bispecific antibody molecules demonstrate different epitopes on the human EpCAM antigen. Transplantation of the human EpCAM Exon 2 domain into the backbone of murine EpCAM led to gain of binding by the EpCAM-directed bispecific single chain molecules of this invention but not by the HD69 HL x I2C HL molecule.

Thus, the second binding domain of the EpCAM-CD3 bispecific single chain antibody of the invention binds to an epitope localized in amino acid residues 26 to 61 of the EGF-like domain 1 of EpCAM which is encoded by Exon 2 of the EpCAM gene. Said amino acid residues 26 to 61 of the EGF-like domain 1 of human EpCAM encoded by exon 2 of the EpCAM gene are shown in SEQ ID NO. 1130.

Accordingly the epitope of the EpCAM-directed bispecific single chain molecules of this invention is mapped to the Exon 2 region of human EpCAM, while other regions of EpCAM participate in forming the epitope of the HD69 HL x I2C HL molecule.

Thus, the EpCAM-directed bispecific single chain molecules of this invention form a unique own class of EpCAM-binding molecules, that is clearly differentiated from former EpCAM-binding molecules based on the EpCAM-binder HD69.

### 31. Generation and characterization of FAP alpha and CD3 cross-species specific bispecific single chain antibody molecules

#### 31.1 Generation of CHO cells expressing human FAP alpha

The coding sequence of human FAPalpha as published in GenBank (Accession number NM_004460) was obtained by gene synthesis according to standard protocols. The gene synthesis fragment was designed as to contain first a Kozak site for eukaryotic expression of the construct, followed by the coding sequence of the human FAPalpha protein and a stop codon (the cDNA and amino acid sequence of the construct is listed under SEQ ID NOs. 1149 and 1150). The gene synthesis fragment was also designed as to introduce restriction sites at the beginning and at the end of the fragment. The introduced restriction sites, XmaI at the 5' end and SalI at the 3' end, were utilised in the following cloning procedures. The gene synthesis fragment was cloned via XmaI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

#### 31.2 Generation of CHO cells expressing macaque FAP alpha

The cDNA sequence of macaque FAP alpha (Cynomolgus) was obtained by a set of four PCRs on cDNA from macaque monkey skin prepared according to standard protocols. The following reaction conditions: 1 cycle at 94°C for 3 minutes followed by 40 cycles with 94°C for 0.5 minutes, 56°C for 0.5 minutes and 72°C for 3 minutes followed by a terminal cycle of 72°C for 3 minutes and the following primers were used:

forward primer: 5'-cagcttccaactacaaagacagac-3' (SEQ ID NO. 1153)
reverse primer: 5'-tttcctcttcataaacccagtctgg-3' (SEQ ID NO. 1154)
forward primer: 5'-ttgaaacaaagaccaggagatccacc-3' (SEQ ID NO. 1155)

reverse primer: 5'-agatggcaagtaacacacttcttgc-3' (SEQ ID NO. 1156)
forward primer: 5'-gaagaaacatctacagaattagcattgg-3' (SEQ ID NO. 1157)
reverse primer: 5'-cacatttgaaaagaccagttccagatgc-3' (SEQ ID NO. 1158)
forward primer: 5'-agattacagctgtcagaaaattcatagaaatgg-3' (SEQ ID NO. 1159)
reverse primer: 5'-atataaggttttcagattctgatacaggc-3' (SEQ ID NO. 1160)

These PCRs generated four overlapping fragments, which were isolated and sequenced according to standard protocols using the PCR primers, and thereby provided the cDNA sequence coding macaque FAPalpha. To generate a construct for expression of macaque FAPalpha a cDNA fragment was obtained by gene synthesis according to standard protocols (the cDNA and amino acid sequence of the construct is listed under SEQ ID NOs. 1151 and 1152). This construct contains the complete coding sequence of macaque FAPalpha followed by a stop codon. The gene synthesis fragment was also designed as to contain a Kozak site for eukaryotic expression of the construct and restriction sites at the beginning and the end of the fragment containing the cDNA. The introduced restriction sites, EcoRI at the 5' end and SalI at the 3' end, were utilised in the following cloning procedures. The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

### 31.3 Generation of FAP alpha and CD3 cross-species specific bispecific single chain molecules

### Cloning of cross-species specific binding molecules

Generally, bispecific single chain antibody molecules, each comprising a domain with a binding specificity cross-species specific for human and non-chimpanzee primate CD3 epsilon as well as a domain with a binding specificity cross-species specific for human and non-chimpanzee primate FAP alpha, were designed as set out in the following Table 15:

Table 15: Formats of anti-CD3 and anti-FAP alpha cross-species specific bispecific single chain antibody molecules

| SEQ ID NO. **(nucl/prot)** | Formats of protein constructs (N → C) |
|---|---|
| 1144/1143 | FAPA-1 LH x I2C HL |
| 1148/1147 | FAPA-1 LH x F12Q HL |
| 1146/1145 | FAPA-1 LH x H2C HL |

The aforementioned constructs containing the variable light-chain (L) and variable heavy-chain (H) domains cross-species specific for human and macaque FAP alpha and the CD3 specific VH and VL combinations cross-species specific for human and macaque CD3 were obtained by gene synthesis. The gene synthesis fragments were designed and eukaryotic protein expression was performed in analogy to the procedure described in example 9 for the MCSPxCD3 cross-species specific single chain molecules.

### 31.4 Expression and purification of the bispecific single chain antibody molecules

The bispecific single chain antibody molecules were expressed in Chinese hamster ovary cells (CHO) or HEK 293 cells as described herein above for the MCSPxCD3 bispecific single chain antibodies.
The isolation and analysis of the expressed bispecific single chain antibodies has also been described herein above in Example 9.

### 31.5 Flow cytometric binding analysis of the FAP alpha and CD3 cross-species specific bispecific antibodies

In order to test the functionality of the cross-species specific bispecific antibody constructs regarding the capability to bind to human and macaque FAPalpha and CD3, respectively, a FACS analysis was performed. For this purpose CHO cells transfected with human FAPalpha as described in Example 31.1 and the human CD3 positive T cell leukemia cell line HPB-ALL (DSMZ, Braunschweig, ACC483) were used to test the binding to human antigens. The binding reactivity to macaque antigens was tested by using the generated macaque FAPalpha transfectant described in Example 31.2 and a macaque T cell line 4119LnPx (kindly provided by Prof Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of cell culture supernatant of transfected cells expressing the cross-species specific bispecific antibody constructs. The cells were washed twice in PBS with 2% FCS and binding of the construct was detected with a murine Penta His antibody (Qiagen; diluted 1:100 in 50 μl PBS with 2% FCS). After washing, bound anti His antibodies were detected with an Fc gamma-specific antibody (Dianova) conjugated to phycoerythrin, diluted 1:100 in PBS with 2% FCS. Supernatant of untransfected cells was used as a negative control.
Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).
The bispecific binding of the single chain molecules listed above, which are cross-species specific for FAPalpha and cross-species specific for human and non-chimpanzee primate CD3 was clearly detectable as shown in Figure 68. In the FACS analysis all constructs showed binding to CD3 and FAPalpha compared to the negative control. Cross-species specificity of the bispecific antibodies to human and macaque CD3 and FAPalpha antigens was demonstrated.

**31.6 Bioactivity of FAPalpha and CD3 cross-species specific bispecific single chain antibodies**

Bioactivity of the generated bispecific single chain antibodies was analyzed by chromium 51 ($^{51}Cr$) release in vitro cytotoxicity assays using the CHO cells transfected with human FAPalpha described in Example 31.1 and the CHO cells transfected with macaque FAPalpha described in Example 31.2. As effector cells stimulated human CD4/CD56 depleted PBMC or the macaque T cell line 4119LnPx were used, respectively.

The generation of stimulated human PBMC was described herein above in Example 11.
Target cells prepared and the assay was performed in analogy to the procedure described for the MCSPxCD3 bispecific single chain antibodies in example 11.
As shown in Figure 69 all of the generated cross-species specific bispecific single chain antibody constructs demonstrated cytotoxic activity against human FAPalpha positive target cells elicited by stimulated human CD4/CD56 depleted PBMC and macaque FAPalpha positive target cells elicited by the macaque T cell line 4119LnPx.

**31.7 Generation of additional FAP alpha and CD3 cross-species specific bispecific single chain antibody molecules**

The human antibody germline VH sequence VH1 1-03 (http://vbase.mrc-cpe.cam.ac.uk/) is chosen as framework context for CDRH1 (SEQ ID NO. 1137), CDRH2 (SEQ ID NO. 1138) and CDRH3 (SEQ ID NO. 1139). For the human VH several degenerated oligonucleotides have to be synthesized that overlap in a terminal stretch of approximately 15-20 nucleotides. To this end every second primer is an antisense primer. For VH1 1-03 the following oligonucleotides are used:

5'FAP-VH-A-XhoI

CCG CTA CTC GAG TCT GGA SCT GAG STG RWG AAG CCT GGG GCT TCA GTA AAG (SEQ ID NO. 1161)

3'FAP-VH-B

CTG TCT CAC CCA GTG TAT GGT GTA TTC AGT GAA TGT GTA TCY AGA AGY CTT GCA GGA CAY CTT TAC TGA AGC CCC (SEQ ID NO. 1162)

5'FAP-VH-C

CAC TGG GTG AGA CAG KCC CMT GGA MAG AGM CTT GAG TGG ATK GGA GGT ATT AAT CCT AAC AAT GGT ATT CCT AAC TAC (SEQ ID NO. 1163)

**3'FAP-VH-D**

CAT GTA GGC GGT GCT GGM GGA CKT GYC TMY AGT TAW TGT GRC CCT GCC CTT GAA CTT CTG ATT GTA GTT AGG AAT ACC (SEQ ID NO. 1164)

**5'FAP-VH-E**

AGC ACC GCC TAC ATG GAG CTC MGC AGC CTG ASA TCT GAG GAT ACT GCG GTC TAT TWC TGT GCA AGA AGA AGA ATC GCC (SEQ ID NO. 1165)

**3'FAP-VH-F-BstEII**

CCA GTA GGT GAC CAG GGT TCC TTG ACC CCA GTA GTC CAT AGC ATG GCC CTC GTC GTA ACC ATA GGC GAT TCT TCT TCT TGC ACA (SEQ ID NO. 1166)

This primer set spans over the whole corresponding VH sequence.
Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.
The VH PCR product is then used as a template for a standard PCR reaction using primers that incorporate N-terminal and C-terminal suitable cloning restriction sites.
The DNA fragment of the correct size (for a VH approximately 350 nucleotides) is isolated by agarose gel electrophoresis according to standard methods. In this way sufficient VH DNA fragment is amplified.
The human antibody germline VL sequence VkII O11 (http://vbase.mrc-cpe.cam.ac.uk/) is chosen as framework context for CDRL1 (SEQ ID NO. 1132), CDRL2 (SEQ ID NO. 1133) and CDRL3 (SEQ ID NO. 1134). For this human VL several degenerated oligonucleotides have to be synthesized that overlap in a terminal stretch of approximately 15-20 nucleotides. To this end every second primer is an antisense primer. Restriction sites needed for later cloning within the oligonucleotides are deleted. For VkIII O11 the following oligonucleotides are used:

5'FAP-VL-A-SacI

CGA CCT GAG CTC GTG ATG ACA CAG ACT CCA YYC TCC CTA SCT GTG ACA SYT GGA GAG MMG GYT TCT ATS AGC TGC AAG TCC AGT CAG (SEQ ID NO. 1167)

3'FAP-VL-B

AGA CTG CCC TGG CTT CTG CWG GWA CCA GGC CAA GTA GTT CTT TTG ATT ACG ACT ATA TAA AAG GCT CTG ACT GGA CTT GCA GCT (SEQ ID NO. 1168)

**5'FAP-VL-C**

CAG AAG CCA GGG CAG TCT CCT MAA CTG CTG ATT TWC TGG GCA TCC ACTA GG GAA TCT GGG GTC CCT GAT CGC TTC TCA GGC AGT GGA (SEQ ID NO. 1169)

**3'FAP-VL-D**

ATA TTG CTG ACA GTM ATA AAC TSC CAS GTC CTC AGC CTS CAC WCT GCT GAT CKT GAG AKT GAA ATC CGT CCC ARA TCC ACT GCC TGA GAA (SEQ ID NO. 1170)

**3'FAP-VL-E-BsiWI/SpeI**

CCA GTA ACT AGT CGT ACG TTT GAT CTC CAC CTT GGT CCC ACC ACC GAA CGT GAG CGG ATA GCT AAA ATA TTG CTG ACA GT (SEQ ID NO. 1171)

This primer set spans over the whole corresponding VL sequence.
Within the set primers are mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix is incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product is run in an agarose gel electrophoresis and the product of a size from 200 to 400 isolated from the gel according to standard methods.
The VL PCR product is then used as a template for a standard PCR reaction using primers that incorporate N-terminal and C-terminal suitable cloning restriction sites.
The DNA fragment of the correct size (for a VL approximately 330 nucleotides) is isolated by agarose gel electrophoresis according to standard methods. In this way sufficient VL DNA fragment is amplified.
The final VH1 1-03 -based VH PCR product (i.e. the repertoire of human/humanized VH) is then combined with the final VkII O11 -based VL PCR product (i.e. the repertoire of human/humanized VL) in the phage display vector pComb3H5Bhis to form a library of functional scFvs from which - after display on filamentous phage - anti-FAPalpha binders are selected, screened, identified and confirmed as described in the following:

450 ng of the light chain fragments (SacI-SpeI digested) are ligated with 1400 ng of the phagemid pComb3H5Bhis (SacI-SpeI digested; large fragment). The resulting combinatorial antibody library is then transformed into 300 μl of electrocompetent Escherichia coli XL1 Blue cells by electroporation (2.5 kV, 0.2 cm gap cuvette, 25 μFD, 200 Ohm, Biorad gene-pulser) resulting in a library size of more than $10^7$ independent clones. After one hour of phenotype expression, positive transformants are selected for carbenicillin resistance encoded by the pComb3H5BHis vector in 100 ml of liquid super broth (SB)-culture over night. Cells are then harvested by centrifugation and plasmid preparation is carried out using a commercially available plasmid preparation kit (Qiagen).
2800 ng of this plasmid-DNA containing the VL-library (XhoI-BstEII digested; large fragment) are ligated with 900 ng of the heavy chain V-fragments (XhoI-BstEII digested) and again transformed into two 300 μl aliquots of electrocompetent E. coli XL1 Blue cells by electroporation (2.5 kV, 0.2 cm gap cuvette, 25 μFD, 200 Ohm) resulting in a total VH-VL scFv (single chain variable fragment) library size of more than $10^7$ independent clones.

After phenotype expression and slow adaptation to carbenicillin, the E. coli cells containing the antibody library are transferred into SB-carbenicillin (SB with 50 μg/mL carbenicillin) selection medium. The E. coli cells containing the antibody library are then infected with an infectious dose of $10^{12}$ particles of helper phage VCSM13 resulting in the production and secretion of filamentous M13 phage, wherein each phage particle contains single stranded pComb3H5BHis-DNA encoding a scFv-fragment and displays the corresponding scFv-protein as a translational fusion to phage coat protein III. This pool of phages displaying the antibody library is used for the selection of antigen binding entities.
For this purpose the phage library carrying the cloned scFv-repertoire is harvested from the respective culture supernatant by PEG8000/NaCl precipitation and centrifugation. Approximately $10^{11}$ to $10^{12}$ scFv phage particles are resuspended in 0.4 ml of PBS/0.1 % BSA and incubated with $10^5$ to $10^7$ FAPalpha transfected CHO cells (see example 31.1) for 1 hour on ice under slow agitation. These FAPalpha transfected CHO cells are harvested beforehand by centrifugation, washed in PBS and resuspended in PBS/1 % FCS (containing 0.05% Na Azide). scFv phage which do not specifically bind to the FAPalpha transfected CHO cells are eliminated by up to five washing steps with PBS/1 % FCS (containing 0.05% Na Azide). After washing, binding entities are eluted from the cells by resuspending the cells in HCl-glycine pH 2.2 (10 min incubation with subsequent vortexing) and after neutralization with 2 M Tris pH 12, the eluate is used for infection of a fresh uninfected E. coli XL1 Blue culture (OD600 > 0.5). The E. coli culture containing E. coli cells successfully transduced with a phagemid copy, encoding a human/humanized scFv-fragment, are again selected for carbenicillin resistance and subsequently infected with VCMS 13 helper phage to start the second round of antibody display and in vitro selection. Typically a total of 4 to 5 rounds of selections are carried out.

In order to screen for FAPalpha specific binders plasmid DNA corresponding to 4 and 5 rounds of panning is isolated from E. coli cultures after selection. For the production of soluble scFv-protein, VH-VL-DNA fragments are excised from the plasmids (XhoI-SpeI). These fragments are cloned via the same restriction sites into the plasmid pComb3H5BFlag/His differing from the original pComb3H5BHis in that the expression construct (e.g. scFv) includes a Flag-tag (DYKDDDDK) between the scFv and the His6-tag and the additional phage proteins are deleted. After ligation, each pool (different rounds of panning) of plasmid DNA is transformed into 100 μl heat shock competent E. coli TG1 or XLI blue and plated onto carbenicillin LB-agar. Single colonies are picked into 100 μl of LB carb (LB with 50 μg/ml carbenicillin).
E. coli transformed with pComb3H5BFlag/His containing a VL-and VH-segment produce soluble scFv in sufficient amounts after induction with 1 mM IPTG. Due to a suitable signal sequence, the scFv is exported into the periplasma where it folds into a functional conformation.
Single E. coli bacterial colonies from the transformation plates are picked for periplasmic small scale preparations and grown in SB-medium (e.g. 10 ml) supplemented with 20 mM $MgCl_2$ and carbenicillin 50μg/ml (and re-dissolved in PBS (e.g. 1 ml) after harvesting. A temperature shock is applied by four rounds of freezing at -70°C and thawing at 37°C whereby the outer membrane of the bacteria is destroyed and the soluble periplasmic proteins including the scFvs are released into the supernatant. After elimination of intact cells and cell-debris by centrifugation, the supernatant containing the anti-FAPalpha scFvs is collected and used for the identification of FAPalpha specific binders as follows:

Binding of scFvs to FAPalpha is tested by flow cytometry on FAPalpha transfected CHO cells (see example 31.1); untransfected CHO cells are use as negative control.

For flow cytometry $2.5x10^5$ cells are incubated with 50 μl of scFv periplasmic preparation or with 5 μg/ml of purified scFv in 50 μl PBS with 2% FCS. The binding of scFv is detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) is used. The samples are measured on a FACSscan (BD biosciences, Heidelberg, FRG).
Single clones are then analyzed for favorable properties and amino acid sequence. FAPalpha specific scFvs are converted into recombinant bispecific single chain antibodies by joining them via a $Gly_4Ser_1$-linker with the CD3 specific scFv I2C (SEQ ID NO. 185) or any other CD3 specific scFv of the invention to result in constructs with the domain arrangement $VL_{FAPal}p_{ha}$ - $(Gly_4Ser_1)_3$-$VH_{FAPalpha}$-$Ser_1Gly_4Ser_1$-$VH_{CD3}$ - $(Gly_4Ser_1)_3$-$VL_{CD3}$ or alternative domain arrangements. For expression in CHO cells the coding sequences of (i) an N-terminal immunoglobulin heavy chain leader comprising a start codon embedded within a Kozak consensus sequence and (ii) a C-terminal $His_6$-tag followed by a stop codon are both attached in frame to the nucleotide sequence encoding the bispecific single chain antibodies prior to insertion of the resulting DNA-fragment as obtained by gene synthesis into the multiple cloning site of the expression vector pEF-DHFR (Raum et al. Cancer Immunol Immunother 50 (2001) 141-150). Transfection of the generated expression plasmids, protein expression and purification of cross-species specific bispecific antibody constructs are performed as described in Examples 31.3 and 31.4. All other state of the art procedures are carried out according to standard protocols (Sambrook, Molecular Cloning; A Laboratory Manual, 3rd edition, Cold Spring Harbour Laboratory Press, Cold Spring Harbour, New York (2001)).
Identification of functional bispecific single-chain antibody constructs is carried out by flow cytometric binding analysis of culture supernatant from transfected cells expressing the cross-species specific bispecific antibody constructs. Analysis is performed as described in Example 31.5. Only those constructs showing bispecific binding to human and macaque CD3 as well as to FAPalpha are selected for further use.
Cytotoxic activity of the generated cross-species specific bispecific single chain antibody constructs against FAPalpha positive target cells elicited by effector cells is analyzed as described in Example 31.6. Only those constructs showing potent recruitment of cytotoxic activity of effector cells against cells positive for FAPalpha are selected for further use.

### 32. Generation and characterization of additional FAPalpha and CD3 cross-species specific bispecific single chain molecules

#### 32.1 Generation of FAPalpha and CD3 cross-species specific bispecific single chain molecules

#### Cloning of cross-species specific binding molecules

Bispecific single chain antibody molecules, with a binding specificity cross-species specific for human and non-chimpanzee primate CD3epsilon as well as a binding specificity cross-species specific for human and non-chimpanzee primate FAPalpha, were designed as set out in the following Table 16:

Table 16: Formats of anti-CD3 and anti-FAPalpha cross-species specific bispecific single chain antibody molecules

| SEQ ID (nucl/prot) | Formats of protein constructs (N → C) |
|---|---|
| 1861/1860 | FA19D12HLxI2CHL |
| 1847/1846 | FA20H3HLxI2CHL |
| 1791/1790 | FA22A9HLxI2CHL |
| 1805/1804 | FA22C11HLxI2CHL |
| 1875/1874 | FA19D9HLxI2CHL |
| 1819/1818 | FA22D8HLxI2CHL |
| 1833/1832 | FA22E8HLxI2CHL |

Generation, expression and purification of these cross-species specific bispecific single chain molecules was performed as described above.
The flow cytometric binding analysis of the FAPalpha and CD3 cross-species specific bispecific antibodies was performed as described above. The bispecific binding of the single chain molecules listed above, which are cross-species specific for FAPalpha and cross-species specific for human and non-chimpanzee primate CD3 was clearly detectable as shown in Figure 70. In the FACS analysis all constructs showed binding to CD3 and FAPalpha compared to the negative control. Cross-species specificity of the bispecific antibodies to human and macaque CD3 and FAPalpha antigens was demonstrated.
Bioactivity of the generated bispecific single chain antibodies was analyzed by chromium 51 ($^{51}$Cr) release in vitro cytotoxicity assays as described above. As shown in Figure 71 all of the generated cross-species specific bispecific single chain antibody constructs demonstrated cytotoxic activity against human FAPalpha positive target cells elicited by stimulated human CD4/CD56 depleted PBMC and macaque FAPalpha positive target cells elicited by the macaque T cell line 4119LnPx.

**32.2 Generation and flow cytometric binding analysis of cross-species specific single chain antibody fragments (scFv) binding to FAPalpha**

scFv cross-species specific for FAPalpha were generated as described above and designated as set out in the following Table 17:

Table 17: Designation of cross-species specific single chain antibody fragments

| SEQ ID (nucl/prot) | Designation |
|---|---|
| 1901/1900 | 86C12HL |
| 1887/1886 | 87G9HL |
| 1971/1970 | 86F12HL |
| 1957/1956 | 86F10HL |
| 1985/1984 | 86F2HL |
| 1943/1942 | 86E5HL |
| 1929/1928 | 86D6HL |
| 1915/1914 | 86D2HL |

The flow cytometric binding analysis of periplasmic preparations containing scFv cross-species specific for FAPalpha using CHO cells transfected with human FAPalpha and CHO cells transfected with macaque FAPalpha was performed as described above. The binding of the scFv listed above, which are cross-species specific for FAPalpha was clearly detectable as shown in Figure 72. In the FACS analysis all constructs showed binding to FAPalpha compared to the negative control. Cross-species specificity of the scFv antibodies to human and macaque FAPalpha antigens was demonstrated.
Cloning of cross-species specific binding molecules based on the scFvs and expression and purification of these cross-species specific bispecific single chain molecules is performed as described above. Flow cytometric analysis of cross-species specific bispecific binding and analysis of bioactivity by chromium 51 ($^{51}Cr$) release in vitro cytotoxicity assays is performed as described above. Based on demonstrated cross-species specific bispecific binding and recruited cytotoxicity cross-species specific binding molecules are selected for further use.

Human/humanized equivalents of non-human scFvs cross-species specific for FAPalpha contained in the selected cross-species specific bispecific single chain molecules are generated as described herein. Cloning of cross-species specific binding molecules based on these human/humanized scFvs and expression and purification of these cross-species specific bispecific single chain molecules is performed as described above. Flow cytometric analysis of cross-species specific bispecific binding and analysis of bioactivity by chromium 51 ($^{51}Cr$) release in vitro cytotoxicity assays is performed as described above. Based on demonstrated cross-species specific bispecific binding and recruited cytotoxicity cross-species specific binding molecules are selected for further use.

**33. Generation and characterization of IGF-1R and CD3 cross-species specific bispecific single chain molecules**

**33.1 Generation of CHO cells expressing human IGF-1R**

The coding sequence of human IGF-1 R as published in GenBank (Accession number NM_000875) was obtained by gene synthesis according to standard protocols. The gene synthesis fragment was designed as to contain first a Kozak site for eukaryotic expression of the construct followed by the coding sequence of the human IGF-1 R protein and a stop codon (the cDNA and amino acid sequence of the construct is listed under SEQ ID Nos 2011 and 2012). The gene synthesis fragment was also designed as to introduce restriction sites at the beginning and at the end of the fragment. The introduced restriction sites, EcoRI at the 5' end and SalI at the 3' end, were utilized in the following cloning procedures. Undesirable internal restriction sites were removed by silent mutation of the coding sequence in the gene synthesis fragment. The gene synthesis fragment was cloned via EcoRI and SalI into a plasmid designated pEF-DHFR (pEF-DHFR is described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**33.2 Generation of CHO cells expressing macaque IGF-1R**

The coding sequence of macaque IGF-1 R as published in GenBank (Accession number XM_001100407) was obtained by gene synthesis according to standard protocols. The gene synthesis fragment was designed as to contain first a Kozak site for eukaryotic expression of the construct followed by the coding sequence of the macaque IGF-1 R protein and a stop codon (the cDNA and amino acid sequence of the construct is listed under SEQ ID Nos 2013 and 2014). The gene synthesis fragment was also designed as to introduce restriction sites at the beginning and at the end of the fragment. The introduced restriction sites, EcoRI at the 5' end and SalI at the 3' end, were utilized in the following cloning procedures. Undesirable internal restriction sites were removed by silent mutation of the coding sequence in the gene synthesis fragment. The gene synthesis fragment was cloned via EcoRI and SalI into a plasmid designated pEF-DHFR (pEF-DHFR was described in Raum et al. Cancer Immunol Immunother 50 (2001) 141-150) following standard protocols. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methotrexate (MTX) to a final concentration of up to 20 nM MTX.

**33.3 Generation of IGF-1R and CD3 cross-species specific bispecific single chain molecules**

**Cloning of cross-species specific binding molecules**

Generally, bispecific single chain antibody molecules, each comprising a domain with a binding specificity cross-species specific for human and non-chimpanzee primate CD3epsilon as well as a domain with a binding specificity cross-species specific for human and non-chimpanzee primate IGF-1R, were designed as set out in the following Table 18:

Table 18: Formats of anti-CD3 and anti-IGF-1R cross-species specific bispecific single chain antibody molecules

| SEQ ID (nucl/prot) | Formats of protein constructs (N → C) |
|---|---|
| 2028/2027 | IGF1R2HLxI2CHL |
| 2042/2041 | IGF1R7HLxI2CHL |
| 2056/2055 | IGF1R9HLxI2CHL |
| 2070/2069 | IGF1R10HLxI2CHL |
| 2084/2083 | IGF1R11HLxI2CHL |
| 2098/2097 | IGF1R12HLxI2CHL |
| 2112/2111 | IGF1R13HLxI2CHL |
| 2126/2125 | IGF1R15HLxI2CHL |
| 2140/2139 | IGF1R16HLxI2CHL |
| 2154/2153 | IGF1R17HLxI2CHL |
| 2168/2167 | IGF1R19HLxI2CHL |
| 2182/2181 | IGF1R20HLxI2CHL |
| 2196/2195 | IGF1R21IHLxI2CHL |
| 2210/2209 | IGF1R23HLxI2CHL |
| 2224/2223 | IGF1R24HLxI2CHL |

Generation, expression and purification of these cross-species specific bispecific single chain molecules was performed as described above.

The flow cytometric binding analysis of the IGF-1 R and CD3 cross-species specific bispecific antibodies was performed as described above. The bispecific binding of the single chain molecules listed above, which are cross-species specific for IGF-1R and cross-species specific for human and non-chimpanzee primate CD3 was clearly detectable as shown in Figure 73. In the FACS analysis all constructs showed binding to CD3 and IGF-1R compared to the negative control. Cross-species specificity of the bispecific antibodies to human and macaque CD3 and IGF-1R antigens was demonstrated.

Bioactivity of the generated bispecific single chain antibodies was analyzed by chromium 51 ($^{51}Cr$) release in vitro cytotoxicity assays as described above. All the cross-species specific bispecific single chain antibody constructs shown in Figure 74 demonstrated cytotoxic activity against human IGF-1 R positive target cells elicited by stimulated human CD4/CD56 depleted PBMC and macaque IGF-1 R positive target cells elicited by the macaque T cell line 4119LnPx.

Human/humanized equivalents of non-human scFvs cross-species specific for IGF-1 R contained in the cross-species specific bispecific single chain molecules are generated as described herein. Cloning of cross-species specific binding molecules based on these human/humanized scFvs and expression and purification of these cross-species specific bispecific single chain molecules is performed as described above. Flow cytometric analysis of cross-species specific bispecific binding and analysis of bioactivity by chromium 51 ($^{51}Cr$) release in vitro cytotoxicity assays is performed as described above. Based on demonstrated cross-species specific bispecific binding and recruited cytotoxicity cross-species specific binding molecules are selected for further use.

| SEQ ID NO. | DESIGNATION | SOURCE | TYPE | SEQUENCE |
|---|---|---|---|---|
| 1. | Human CD3 epsilon extracellular domain | human | aa | QDGNEEMGGITQTPYKVSISGTTVILTCPQYPGSEILWQHNDKNIGGDEDDKNIGSDEDHLSLKE FSELEQSGYYVCYPRGSKPEDANFYLYLRARVCENCMEMD |
| 2. | Human CD3 epsilon 1-27 | human | aa | QDGNEEMGGITQTPYKVSISGTTVILT |
| 3. | *Callithrix jacchus* CD3 epsilon extracellular domain | *Callithrix jacchus* | aa | QDGNEEMGDTTQNPYKVSISGTTVTLTCPRYDGHEIKWLVNSQNKEGHEDHLLLEDFSEMEQSGY YACLSKETPAEEASHYLYLKARVCENCVEVD |
| 4. | *Callithrix jacchus* CD3 epsilon 1-27 | *Callithrix jacchus* | aa | QDGNEEMGDTTQNPYKVSISGTTVTLT |
| 5. | *Saguinus oedipus* CD3 epsilon extracellular domain | *Saguinus oedipus* | aa | QDGNEEMGDTTQNPYKVSISGTTVTLTCPRYDGHEIKWLVNSQNKEGHEDHLLLEDFSEMEQSGY YACLSKETPAEEASHYLYLKARVCENCVEVD |
| 6. | *Saguinus oedipus* CD3 epsilon 1-27 | *Saguinus oedipus* | aa | QDGNEEMGDTTQNPYKVSISGTTVTLT |
| 7. | *Saimiri sciureus* CD3 epsilon extracellular domain | *Saimiri sciureus* | aa | QDGNEEIGDTTQNPYKVSISGTTVTLTCPRYDGQEIKWLVNDQNKEGHEDHLLLEDFSEMEQSGY YACLSKETPTEEASHYLYLKARVCENCVEVD |
| 8. | *Saimiri sciureus* CD3 epsilon 1-27 | *Saimiri sciureus* | aa | QDGNEEIGDTTQNPYKVSISGTTVTLT |
| 9. | CDR-L1 of F6A | artificial | aa | GSSTGAVTSGYYPN |
| 10. | CDR-L2 of F6A | artificial | aa | GTKFLAP |
| 11. | CDR-L3 of F6A | artificial | aa | ALWYSNRWV |
| 12. | CDR-H1 of F6A | artificial | aa | IYAMN |
| 13. | CDR-H2 of F6A | artificial | aa | RIRSKYNNYATYYADSVKS |
| 14. | CDR-H3 of F6A | artificial | aa | HGNFGNSYVSFFAY |
| 15. | VH of F6A | artificial | aa | EVQLVESGGGLVQPGGSLKLSCAASGFTFNIYAMNWVRQAPGKGLEWVARIRSKYNNYATYYADS VKSRFTISRDDSKNTAYLQMNNLKTEDTAVYYCVRHGNFGNSYVSFFAYWGQGTLVTVSS |
| 16. | VH of F6A | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATATCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TATCCTTCTTCGCTTACTGGGGCCAAGGGACTCTGGTCACCGTCTCCTCA |
| 17. | VL of F6A | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |

| | | | | |
|---|---|---|---|---|
| 18. | VL of F6A | artificial | nt | CAGACTGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 19. | VH-P of F6A | artificial | aa | EVQLLESGGGLVQPGGSLKLSCAASGFTFNIYAMNWVRQAPGKGLEWVARIRSKYNNYATYYADS<br>VKSRFTISRDDSKNTAYLQMNNLKTEDTAVYYCVRHGNFGNSYVSFFAYWGQGTLVTVSS |
| 20. | VH-P of F6A | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATATCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAGCAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACG<br>TATCCTTCTTCGCTTACTGGGGCCAAGGGACTCTGGTCACCGTCTCCTCA |
| 21. | VL-P of F6A | artificial | aa | ELVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 22. | VL-P of F6A | artificial | nt | GAGCTCGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 23. | VH-VL of F6A | artificial | aa | EVQLVESGGGLVQPGGSLKLSCAASGFTFNIYAMNWVRQAPGKGLEWVARIRSKYNNYATYYADS<br>VKSRFTISRDDSKNTAYLQMNNLKTEDTAVYYCVRHGNFGNSYVSFFAYWGQGTLVTVSSGGGGS<br>GGGGSGGGGSQTVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 24. | VH-VL of F6A | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATATCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAGCAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACG<br>TATCCTTCTTCGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG |

| | | | | |
|---|---|---|---|---|
| | | | | TGTTCGGTGGAGGAACCAAACTGACTGTCCTA |
| 25. | VH-VL-P of F6A | artificial | aa | EVQLLESGGGLVQPGGSLKLSCAASGFTFNIYAMNWVRQAPGKGLEWVARIRSKYNNYATYYADS<br>VKSRFTISRDDSKNTAYLQMNNLKTEDTAVYYCVRHGNFGNSYVSFFAYWGQGTLVTVSSGGGGS<br>GGGGSGGGGSELVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 26. | VH-VL-P of F6A | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATATCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAGCAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACG<br>TATCCTTCTTCGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 27. | CDR-L1 of H2C | artificial | aa | GSSTGAVTSGYYPN |
| 28. | CDR-L2 of H2C | artificial | aa | GTKFLAP |
| 29. | CDR-L3 of H2C | artificial | aa | ALWYSNRWV |
| 30. | CDR-H1 of H2C | artificial | aa | KYAMN |
| 31. | CDR-H2 of H2C | artificial | aa | RIRSKYNNYATYYADSVKD |
| 32. | CDR-H3 of H2C | artificial | aa | HGNFGNSYISYWAY |
| 33. | VH of H2C | artificial | aa | EVQLVESGGGLVQPGGSLKLSCAASGFTFNKYAMNWVRQAPGKGLEWVARIRSKYNNYATYYADS<br>VKDRFTISRDDSKNTAYLQMNNLKTEDTAVYYCVRHGNFGNSYISYWAYWGQGTLVTVSS |
| 34. | VH of H2C | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTACTGGGCTTACTGGGGCCAAGGGACTCTGGTCACCGTCTCCTCA |

| | | | | |
|---|---|---|---|---|
| 35. | VL of H2C | artificial | aa | QTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 36. | VL of H2C | artificial | nt | CAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 37. | VH-P of H2C | artificial | aa | EVQLLESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSS |
| 38. | VH-P of H2C | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCA |
| 39. | VL-P of H2C | artificial | aa | ELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 40. | VL-P of H2C | artificial | nt | GAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 41. | VH-VL of H2C | artificial | aa | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 42. | VH-VL of H2C | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTC |

| | | | | |
|---|---|---|---|---|
| | | | | CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 43. | VH-VL-P of H2C | artificial | aa | EVQLLESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSELVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 44. | VH-VL-P of H2C | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 45. | CDR-L1 of H1E | artificial | aa | GSSTGAVTSGYYPN |
| 46. | CDR-L2 of H1E | artificial | aa | GTKFLAP |
| 47. | CDR-L3 of H1E | artificial | aa | ALWYSNRWV |
| 48. | CDR-H1 of H1E | artificial | aa | SYAMN |
| 49. | CDR-H2 of H1E | artificial | aa | RIRSKYNNYATYYADSVKG |
| 50. | CDR-H3 of H1E | artificial | aa | HGNFGNSYLSFWAY |
| 51. | VH of H1E | artificial | aa | EVQLVESGGGLEQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYLSFWAYWGQGTLVTVSS |
| 52. | VH of H1E | artificial | nt | GAGGTGCAGCTGGTCGAGTCTGGAGGAGGATTGGAGCAGCCTGGAGGGTCATTGAAACTCTCATG TGCAGCCTCTGGATTCACCTTCAATTCGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACC TATCCTTCTGGGCTTACTGGGGCCAAGGGACTCTGGTCACCGTCTCCTC |
| 53. | VL of H1E | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 54. | VL of H1E | artificial | nt | CAGACTGTTGTGACTCAGGAACCTTCACTCACCGTATCACCTGGTGGAACAGTCACACTCACTTG |

| | | | | |
|---|---|---|---|---|
| | | | | TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 55. | VH-P of H1E | artificial | aa | EVQLLESGGGLEQPGGSLKLSCAASGFTFNSYAMNWVRQAPGKGLEWVARIRSKYNNYATYYADS<br>VKGRFTISRDDSKNTAYLQMNNLKTEDTAVYYCVRHGNFGNSYLSFWAYWGQGTLVTVSS |
| 56. | VH-P of H1E | artificial | nt | GAGGTGCAGCTGCTCGAGTCTGGAGGAGGATTGGAGCAGCCTGGAGGGTCATTGAAACTCTCATG<br>TGCAGCCTCTGGATTCACCTTCAATTCGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACC<br>TATCCTTCTGGGCTTACTGGGGCCAAGGGACTCTGGTCACCGTCTCCTCA |
| 57. | VL-P of H1E | artificial | aa | ELVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 58. | VL-P of H1E | artificial | nt | GAGCTCGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 59. | VH-VL of H1E | artificial | aa | EVQLVESGGGLEQPGGSLKLSCAASGFTFNSYAMNWVRQAPGKGLEWVARIRSKYNNYATYYADS<br>VKGRFTISRDDSKNTAYLQMNNLKTEDTAVYYCVRHGNFGNSYLSFWAYWGQGTLVTVSSGGGGS<br>GGGGSGGGGSQTVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 60. | VH-VL of H1E | artificial | nt | GAGGTGCAGCTGGTCGAGTCTGGAGGAGGATTGGAGCAGCCTGGAGGGTCATTGAAACTCTCATG<br>TGCAGCCTCTGGATTCACCTTCAATTCGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACC<br>TATCCTTCTGGGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |

| | | | | |
|---|---|---|---|---|
| 61. | VH-VL-P of H1E | artificial | aa | EVQLLESGGGLEQPGGSLKLSCAASGFTFNSYAMNWVRQAPGKGLEWVARIRSKYNNYATYYADS<br>VKGRFTISRDDSKNTAYLQMNNLKTEDTAVYYCVRHGNFGNSYLSFWAYWGQGTLVTVSSGGGGS<br>GGGGSGGGGSELVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 62. | VH-VL-P of H1E | artificial | nt | GAGGTGCAGCTGCTCGAGTCTGGAGGAGGATTGGAGCAGCCTGGAGGGTCATTGAAACTCTCATG<br>TGCAGCCTCTGGATTCACCTTCAATTCGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACC<br>TATCCTTCTGGGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 63. | CDR-L1 of G4H | artificial | aa | GSSTGAVTSGYYPN |
| 64. | CDR-L2 of G4H | artificial | aa | GTKFLAP |
| 65. | CDR-L3 of G4H | artificial | aa | ALWYSNRWV |
| 66. | CDR-H1 of G4H | artificial | aa | RYAMN |
| 67. | CDR-H2 of G4H | artificial | aa | RIRSKYNNYATYYADSVKG |
| 68. | CDR-H3 of G4H | artificial | aa | HGNFGNSYLSYFAY |
| 69. | VH of G4H | artificial | aa | EVQLVESGGGLVQPGGSLKLSCAASGFTFNRYAMNWVRQAPGKGLEWVARIRSKYNNYATYYADS<br>VKGRFTISRDDSKNTAYLQMNNLKTEDTAVYYCVRHGNFGNSYLSYFAYWGQGTLVTVSS |
| 70. | VH of G4H | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATCGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTACTTCGCTTACTGGGGCCAAGGGACTCTGGTCACCGTCTCCTCA |
| 71. | VL of G4H | artificial | aa | QTVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 72. | VL of G4H | artificial | nt | CAGACTGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA |

| | | | | |
|---|---|---|---|---|
| | | | | ATATTACTGTGCTCTATGGTACAGCAACCGCTGGGTGTTCGGTGGAGGAACCAAACTGACTGTCCTA |
| 73. | VH-P of G4H | artificial | aa | EVQLLESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSS |
| 74. | VH-P of G4H | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATCGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGGGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACTTATCCTACTTCGCTTACTGGGGCCAAGGGACTCTGGTCACCGTCTCCTCA |
| 75. | VL-P of G4H | artificial | aa | ELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 76. | VL-P of G4H | artificial | nt | GAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 77. | VH-VL of G4H | artificial | aa | EVQLVESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 78. | VH-VL of G4H | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATCGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGGGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACTTATCCTACTTCGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 79. | VH-VL-P of G4H | artificial | aa | EVQLLESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSSGGGGSGGGGSGGGGSELVVTQEPSLTVSPGGTVTLTCGSSTGAVTSGYYPNWVQQKPGQAPRGLIGGTKF |

| | | | | |
|---|---|---|---|---|
| | | | | LAPGTPARFSGSLLGGKAALTLSGVQPEDEAEYYCALWYSNRWVFGGGTKLTVL |
| 80. | VH-VL-P of G4H | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATCGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACT TATCCTACTTCGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 81. | CDR-L1 of A2J | artificial | aa | RSSTGAVTSGYYPN |
| 82. | CDR-L2 of A2J | artificial | aa | ATDMRPS |
| 83. | CDR-L3 of A2J | artificial | aa | ALWYSNRWV |
| 84. | CDR-H1 of A2J | artificial | aa | VYAMN |
| 85. | CDR-H2 of A2J | artificial | aa | RIRSKYNNYATYYADSVKK |
| 86. | CDR-H3 of A2J | artificial | aa | HGNFGNSYLSWWAY |
| 87. | VH of A2J | artificial | aa | EVQLVESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYLSWWAYWGQGTLVTVSS |
| 88. | VH of A2J | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATGTCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACT TATCCTGGTGGGCTTACTGGGGCCAAGGGACTCTGGTCACCGTCTCCTCA |
| 89. | VL of A2J | artificial | aa | QTVVTQEPSLTVSPGGTVTLTCRSSTGAVTSGYYPNWVQQKPGQAPRGLIGATDMRPSGTPARFS GSLLGGKAALTLSGVQPEDEAEYYCALWYSNRWVFGGGTKLTVL |
| 90. | VL of A2J | artificial | nt | CAGACTGTTGTGACTCAGGAACCTTCACTCACCGTATCACCTGGTGGAACAGTCACACTCACTTG TCGCTCCTCGACTGGGGCTGTTACATCTGGCTACTACCCAAACTGGGTCCAACAAAAACCAGGTC AGGCACCCCGTGGTCTAATAGGTGCCACTGACATGAGGCCCTCTGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 91. | VH-P of A2J | artificial | aa | EVQLLESGGGLVQPGGSLKLSCAASGFTFNVYAMNWVRQAPGKGLEWVARIRSKYNNYATYYADS |

| | | | | |
|---|---|---|---|---|
| | | | | VKKRFTISRDDSKNTAYLQMNNLKTEDTAVYYCVRHGNFGNSYLSWWAYWGQGTLVTVSS |
| 92. | VH-P of A2J | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATGTCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTGGTGGGCTTACTGGGGCCAAGGGACTCTGGTCACCGTCTCCTCA |
| 93. | VL-P of A2J | artificial | aa | ELVVTQEPSLTVSPGGTVTLTCRSSTGAVTSGYYPNWVQQKPGQAPRGLIGATDMRPSGTPARFS<br>GSLLGGKAALTLSGVQPEDEAEYYCALWYSNRWVFGGGTKLTVL |
| 94. | VL-P of A2J | artificial | nt | GAGCTCGTTGTGACTCAGGAACCTTCACTCACCGTATCACCTGGTGGAACAGTCACACTCACTTG<br>TCGCTCCTCGACTGGGGCTGTTACATCTGGCTACTACCCAAACTGGGTCCAACAAAAACCAGGTC<br>AGGCACCCCGTGGTCTAATAGGTGCCACTGACATGAGGCCCTCTGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 95. | VH-VL of A2J | artificial | aa | EVQLVESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYLSWWAYWGQGTLVTVSSGGGGS<br>GGGGSGGGGSQTVVTQEPSLTVSPGGTVTLTCRSSTGAVTSGYYPNWVQQKPGQAPRGLIGATDM<br>RPSGTPARFSGSLLGGKAALTLSGVQPEDEAEYYCALWYSNRWVFGGGTKLTVL |
| 96. | VH-VL of A2J | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATGTCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTGGTGGGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTCGCTCCTCGACTGGGGCTGTTACATCTGGCTACT<br>ACCCAAACTGGGTCCAACAAAAACCAGGTCAGGCACCCCGTGGTCTAATAGGTGCCACTGACATG<br>AGGCCCTCTGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 97. | VH-VL-P of A2J | artificial | aa | EVQLLESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYLSWWAYWGQGTLVTVSSGGGGS<br>GGGGSGGGGSELVVTQEPSLTVSPGGTVTLTCRSSTGAVTSGYYPNWVQQKPGQAPRGLIGATDM<br>RPSGTPARFSGSLLGGKAALTLSGVQPEDEAEYYCALWYSNRWVFGGGTKLTVL |
| 98. | VH-VL-P of A2J | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATGTCTACGCCATGAACTGGGTCCGCCAGGCTCCAGGAAAGG |

| | | | | |
|---|---|---|---|---|
| | | | | GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTGGTGGGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTCGCTCCTCGACTGGGGCTGTTACATCTGGCTACT<br>ACCCAAACTGGGTCCAACAAAAACCAGGTCAGGCACCCCGTGGTCTAATAGGTGCCACTGACATG<br>AGGCCCTCTGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 99. | CDR-L1 of E1L | artificial | aa | GSSTGAVTSGYYPN |
| 100. | CDR-L2 of E1L | artificial | aa | GTKFLAP |
| 101. | CDR-L3 of E1L | artificial | aa | ALWYSNRWV |
| 102. | CDR-H1 of E1L | artificial | aa | KYAMN |
| 103. | CDR-H2 of E1L | artificial | aa | RIRSKYNNYATYYADSVKS |
| 104. | CDR-H3 of E1L | artificial | aa | HGNFGNSYTSYYAY |
| 105. | VH of E1L | artificial | aa | EVQLVESGGGLVQPGGSLKLSCAASGFTFNKYAMNWVRQAPGKGLEWVARIRSKYNNYATYYADS<br>VKSRFTISRDDSKNTAYLQMNNLKTEDTAVYYCVRHGNFGNSYTSYYAYWGQGTLVTVSS |
| 106. | VH of E1L | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAATCGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>CATCCTACTACGCTTACTGGGGCCAAGGGACTCTGGTCACCGTCTCCTCA |
| 107. | VL of E1L | artificial | aa | QTVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 108. | VL of E1L | artificial | nt | CAGACTGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 109. | VH-P of E1L | artificial | aa | EVQLLESGGGLVQPGGSLKLSCAASGFTFNKYAMNWVRQAPGKGLEWVARIRSKYNNYATYYADS<br>VKSRFTISRDDSKNTAYLQMNNLKTEDTAVYYCVRHGNFGNSYTSYYAYWGQGTLVTVSS |
| 110. | VH-P of E1L | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG |

| | | | | |
|---|---|---|---|---|
| | | | | GTTTGGAATGGGTTGCTCGCATAAGAAGTAAATATAATAATTATGCAACATATTATGCCGATTCAGTGAAATCGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACACATCCTACTACGCTTACTGGGGCCAAGGGACTCTGGTCACCGTCTCCTCA |
| 111. | VL-P of E1L | artificial | aa | ELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 112. | VL-P of E1L | artificial | nt | GAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 113. | VH-VL of E1L | artificial | aa | EVQLVESGGGLVQPGGSLKLSCAASGFTFNKYAMNWVRQAPGKGLEWVARIRSKYNNYATYYADSVKSRFTISRDDSKNTAYLQMNNLKTEDTAVYYCVRHGNFGNSYTSYYAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 114. | VH-VL of E1L | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAATCGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACACATCCTACTACGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 115. | VH-VL-P of E1L | artificial | aa | EVQLLESGGGLVQPGGSLKLSCAASGFTFNKYAMNWVRQAPGKGLEWVARIRSKYNNYATYYADSVKSRFTISRDDSKNTAYLQMNNLKTEDTAVYYCVRHGNFGNSYTSYYAYWGQGTLVTVSSGGGGSGGGGSGGGGSELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 116. | VH-VL-P of E1L | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAATCGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACA |

| | | | | |
|---|---|---|---|---|
| | | | | CATCCTACTACGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 117. | CDR-L1 of E2M | artificial | aa | RSSTGAVTSGYYPN |
| 118. | CDR-L2 of E2M | artificial | aa | ATDMRPS |
| 119. | CDR-L3 of E2M | artificial | aa | ALWYSNRWV |
| 120. | CDR-H1 of E2M | artificial | aa | GYAMN |
| 121. | CDR-H2 of E2M | artificial | aa | RIRSKYNNYATYYADSVKE |
| 122. | CDR-H3 of E2M | artificial | aa | HRNFGNSYLSWFAY |
| 123. | VH of E2M | artificial | aa | EVQLVESGGGLVQPGGSLKLSCAASGFTFNGYAMNWVRQAPGKGLEWVARIRSKYNNYATYYADS<br>VKERFTISRDDSKNTAYLQMNNLKTEDTAVYYCVRHRNFGNSYLSWFAYWGQGTLVTVSS |
| 124. | VH of E2M | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATGGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGAGAGGTTCACCATCTCCAGAGATGATTCAAAAAACACTGCCTATCTACAAATGAACAA<br>CTTGAAAACTGAGGACACTGCCGTGTACTACTGTGTGAGACATAGGAACTTCGGTAATAGCTACT<br>TATCCTGGTTCGCTTACTGGGGCCAAGGGACTCTGGTCACCGTCTCCTCA |
| 125. | VL of E2M | artificial | aa | QTVVTQEPSLTVSPGGTVTLTCRSSTGAVTSGYYPNWVQQKPGQAPRGLIGATDMRPSGTPARFS<br>GSLLGGKAALTLSGVQPEDEAEYYCALWYSNRWVFGGGTKLTVL |
| 126. | VL of E2M | artificial | nt | CAGACTGTTGTGACTCAGGAACCTTCACTCACCGTATCACCTGGTGGAACAGTCACACTCACTTG<br>TCGCTCCTCGACTGGGGCTGTTACATCTGGCTACTACCCAAACTGGGTCCAACAAAAACCAGGTC<br>AGGCACCCCGTGGTCTAATAGGTGCCACTGACATGAGGCCCTCTGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 127. | VH-P of E2M | artificial | aa | EVQLLESGGGLVQPGGSLKLSCAASGFTFNGYAMNWVRQAPGKGLEWVARIRSKYNNYATYYADS<br>VKERFTISRDDSKNTAYLQMNNLKTEDTAVYYCVRHRNFGNSYLSWFAYWGQGTLVTVSS |
| 128. | VH-P of E2M | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATGGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGAGAGGTTCACCATCTCCAGAGATGATTCAAAAAACACTGCCTATCTACAAATGAACAA<br>CTTGAAAACTGAGGACACTGCCGTGTACTACTGTGTGAGACATAGGAACTTCGGTAATAGCTACT |

| | | | | |
|---|---|---|---|---|
| | | | | TATCCTGGTTCGCTTACTGGGGCCAAGGGACTCTGGTCACCGTCTCCTCA |
| 129. | VL-P of E2M | artificial | aa | ELVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |
| 130. | VL-P of E2M | artificial | nt | GAGCTCGTTGTGACTCAGGAACCTTCACTCACCGTATCACCTGGTGGAACAGTCACACTCACTTGTCGCTCCTCGACTGGGGCTGTTACATCTGGCTACTACCCAAACTGGGTCCAACAAAAACCAGGTCAGGCACCCCGTGGTCTAATAGGTGCCACTGACATGAGGCCCTCTGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 131. | VH-VL of E2M | artificial | aa | EVQLVESGGGLVQPGGSLKLSCAASGFTFNGYAMNWVRQAPGKGLEWVARIRSKYNNYATYYADSVKERFTISRDDSKNTAYLQMNNLKTEDTAVYYCVRHRNFGNSYLSWFAYWGQGTLVTVSSGGGGSGGGGSGGGGSQTVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |
| 132. | VH-VL of E2M | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATGGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGAGAGGTTCACCATCTCCAGAGATGATTCAAAAAACACTGCCTATCTACAAATGAACAACTTGAAAACTGAGGACACTGCCGTGTACTACTGTGTGAGACATAGGAACTTCGGTAATAGCTACTTATCCTGGTTCGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTCGCTCCTCGACTGGGGCTGTTACATCTGGCTACTACCCAAACTGGGTCCAACAAAAACCAGGTCAGGCACCCCGTGGTCTAATAGGTGCCACTGACATGAGGCCCTCTGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 133. | VH-VL-P of E2M | artificial | aa | EVQLLESGGGLVQPGGSLKLSCAASGFTFNGYAMNWVRQAPGKGLEWVARIRSKYNNYATYYADSVKERFTISRDDSKNTAYLQMNNLKTEDTAVYYCVRHRNFGNSYLSWFAYWGQGTLVTVSSGGGGSGGGGSGGGGSELVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |
| 134. | VH-VL-P of E2M | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATGGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGAGAGGTTCACCATCTCCAGAGATGATTCAAAAAACACTGCCTATCTACAAATGAACAACTTGAAAACTGAGGACACTGCCGTGTACTACTGTGTGAGACATAGGAACTTCGGTAATAGCTACTTATCCTGGTTCGCTTACTGGGGCCAAGGGACTCTGGTCACCGTCTCCTCAGGTGGTGGTGGTTCTGGCGGCGGCGGCTCCGGTGGTGGTGGTTCTGAGCTCGTTGTGACTCAGGAACCTTCACTCACCGTATCACCTGGTGGAACAGTCACACTCACTTGTCGCTCCTCGACTGGGGCTGTTACATCTGGCTACT |

| | | | | |
|---|---|---|---|---|
| | | | | ACCCAAACTGGGTCCAACAAAAACCAGGTCAGGCACCCCGTGGTCTAATAGGTGCCACTGACATG<br>AGGCCCTCTGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 135. | CDR-L1 of F7O | artificial | aa | GSSTGAVTSGYYPN |
| 136. | CDR-L2 of F7O | artificial | aa | GTKFLAP |
| 137. | CDR-L3 of F7O | artificial | aa | ALWYSNRWV |
| 138. | CDR-H1 of F7O | artificial | aa | VYAMN |
| 139. | CDR-H2 of F7O | artificial | aa | RIRSKYNNYATYYADSVKK |
| 140. | CDR-H3 of F7O | artificial | aa | HGNFGNSYISWWAY |
| 141. | VH of F7O | artificial | aa | EVQLVESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYISWWAYWGQGTLVTVSS |
| 142. | VH of F7O | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATGTGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTGGTGGGCTTACTGGGGCCAAGGGACTCTGGTCACCGTCTCCTCA |
| 143. | VL of F7O | artificial | aa | QTVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 144. | VL of F7O | artificial | nt | CAGACTGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 145. | VH-P of F7O | artificial | aa | EVQLLESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYISWWAYWGQGTLVTVSS |
| 146. | VH-P of F7O | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATGTGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTGGTGGGCTTACTGGGGCCAAGGGACTCTGGTCACCGTCTCCTCA |
| 147. | VL-P of F7O | artificial | aa | ELVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |

| | | | | |
|---|---|---|---|---|
| 148. | VL-P of F7O | artificial | nt | GAGCTCGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 149. | VH-VL of F7O | artificial | aa | EVQLVESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYISWWAYWGQGTLVTVSSGGGGS<br>GGGGSGGGGSQTVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 150. | VH-VL of F7O | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATGTGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTGGTGGGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 151. | VH-VL-P of F7O | artificial | aa | EVQLLESGGGLVQPGGSLKLSCAASGFTFNVYAMNWVRQAPGKGLEWVARIRSKYNNYATYYADS<br>VKKRFTISRDDSKNTAYLQMNNLKTEDTAVYYCVRHGNFGNSYISWWAYWGQGTLVTVSSGGGGS<br>GGGGSGGGGSELVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 152. | VH-VL-P of F7O | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATGTGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAAAGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTGGTGGGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG |

| | | | | |
|---|---|---|---|---|
| | | | | TGTTCGGTGGAGGAACCAAACTGACTGTCCTA |
| 153. | CDR-L1 of F12Q | artificial | aa | GSSTGAVTSGNYPN |
| 154. | CDR-L2 of F12Q | artificial | aa | GTKFLAP |
| 155. | CDR-L3 of F12Q | artificial | aa | VLWYSNRWV |
| 156. | CDR-H1 of F12Q | artificial | aa | SYAMN |
| 157. | CDR-H2 of F12Q | artificial | aa | RIRSKYNNYATYYADSVKG |
| 158. | CDR-H3 of F12Q | artificial | aa | HGNFGNSYVSWWAY |
| 159. | VH of F12Q | artificial | aa | EVQLVESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSS |
| 160. | VH of F12Q | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCA |
| 161. | VL of F12Q | artificial | aa | QTVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 162. | VL of F12Q | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAACTGACTGTCC TA |
| 163. | VH-P of F12Q | artificial | aa | EVQLLESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSS |
| 164. | VH-P of F12Q | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCA |
| 165. | VL-P of F12Q | artificial | aa | ELVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 166. | VL-P of F12Q | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA |

| | | | | |
|---|---|---|---|---|
| | | | | GGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 167. | VH-VL of F12Q | artificial | aa | EVQLVESGGGLVQPGGSLKLSCAASGFTFNSYAMNWVRQAPGKGLEWVARIRSKYNNYATYYADSVKGRFTISRDDSKNTAYLQMNNLKTEDTAVYYCVRHGNFGNSYVSWWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 168. | VH-VL of F12Q | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATAGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGGCAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTTTCCTGGTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 169. | VH-VL-P of F12Q | artificial | aa | EVQLLESGGGLVQPGGSLKLSCAASGFTFNSYAMNWVRQAPGKGLEWVARIRSKYNNYATYYADSVKGRFTISRDDSKNTAYLQMNNLKTEDTAVYYCVRHGNFGNSYVSWWAYWGQGTLVTVSSGGGGSGGGGSGGGGSELVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 170. | VH-VL-P of F12Q | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATAGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGGCAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTTTCCTGGTGGGCTTACTGGGGCCAAGGGACTCTGGTCACCGTCTCCTCAGGTGGTGGTGGTTCTGGCGGCGGCGGCTCCGGTGGTGGTGGTTCTGAGCTCGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 171. | CDR-L1 of I2C | artificial | aa | GSSTGAVTSGNYPN |
| 172. | CDR-L2 of I2C | artificial | aa | GTKFLAP |

| | | | | |
|---|---|---|---|---|
| 173. | CDR-L3 of I2C | artificial | aa | VLWYSNRWV |
| 174. | CDR-H1 of I2C | artificial | aa | KYAMN |
| 175. | CDR-H2 of I2C | artificial | aa | RIRSKYNNYATYYADSVKD |
| 176. | CDR-H3 of I2C | artificial | aa | HGNFGNSYISYWAY |
| 177. | VH of I2C | artificial | aa | EVQLVESGGGLVQPGGSLKLSCAASGFTFNKYAMNWVRQAPGKGLEWVARIRSKYNNYATYYADS<br>VKDRFTISRDDSKNTAYLQMNNLKTEDTAVYYCVRHGNFGNSYISYWAYWGQGTLVTVSS |
| 178. | VH of I2C | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTACTGGGCTTACTGGGGCCAAGGGACTCTGGTCACCGTCTCCTCA |
| 179. | VL of I2C | artificial | aa | QTVVTQEPSLTVSPGGTVTLTCGSSTGAVTSGNYPNWVQQKPGQAPRGLIGGTKFLAPGTPARFS<br>GSLLGGKAALTLSGVQPEDEAEYYCVLWYSNRWVFGGGTKLTVL |
| 180. | VL of I2C | artificial | nt | CAGACTGTTGTGACTCAGGAACCTTCACTCACCGTATCACCTGGTGGAACAGTCACACTCACTTG<br>TGGCTCCTCGACTGGGGCTGTTACATCTGGCAACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGTTCTATGGTACAGCAACCGCTGGGTGTTCGGTGGAGGAACCAAACTGACTGTCC<br>TA |
| 181. | VH-P of I2C | artificial | aa | EVQLLESGGGLVQPGGSLKLSCAASGFTFNKYAMNWVRQAPGKGLEWVARIRSKYNNYATYYADS<br>VKDRFTISRDDSKNTAYLQMNNLKTEDTAVYYCVRHGNFGNSYISYWAYWGQGTLVTVSS |
| 182. | VH-P of I2C | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTACTGGGCTTACTGGGGCCAAGGGACTCTGGTCACCGTCTCCTCA |
| 183. | VL-P of I2C | artificial | aa | ELVVTQEPSLTVSPGGTVTLTCGSSTGAVTSGNYPNWVQQKPGQAPRGLIGGTKFLAPGTPARFS<br>GSLLGGKAALTLSGVQPEDEAEYYCVLWYSNRWVFGGGTKLTVL |
| 184. | VL-P of I2C | artificial | nt | GAGCTCGTTGTGACTCAGGAACCTTCACTCACCGTATCACCTGGTGGAACAGTCACACTCACTTG<br>TGGCTCCTCGACTGGGGCTGTTACATCTGGCAACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGTTCTATGGTACAGCAACCGCTGGGTGTTCGGTGGAGGAACCAAACTGACTGTCC<br>TA |

| | | | | |
|---|---|---|---|---|
| 185. | VH-VL of I2C | artificial | aa | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 186. | VH-VL of I2C | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 187. | VH-VL-P of I2C | artificial | aa | EVQLLESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSELVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 188. | VH-VL-P of I2C | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTGAGCTCGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 189. | MCSP-G4 VH-VL x H2C VH-VL | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGGGGSDIVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIKSGGGGSEVQLVESGGGLVQPGGSLKLSCAASGFTFNKYAMNWVRQAPGKGLEWVARIRSKYNNYATYYADSVKDRFTISR |

| | | | | |
|---|---|---|---|---|
| | | | | DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 190. | MCSP-G4 VH-VL x H2C VH-VL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 191. | MCSP-G4 VH-VL x F12Q VH-VL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYVSWWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 192. | MCSP-G4 VH-VL x F12Q VH-VL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG |

| | | | | |
|---|---|---|---|---|
| | | | | GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTTTCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 193. | MCSP-G4 VH-VL x I2C VH-VL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 194. | MCSP-G4 VH-VL x I2C VH-VL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC |

| | | | | |
|---|---|---|---|---|
| | | | | CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGACAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG TCCTA |
| 195. | MCSP-G4 VH-VL-P x F6A VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLVQPGGSLKLSCAASGFTFNIYAMNWVRQAPGKGLEWVARIRSKYNNYATYYADSVKSRFTISR DDSKNTAYLQMNNLKTEDTAVYYCVRHGNFGNSYVSFFAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 196. | MCSP-G4 VH-VL-P x F6A VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA |

| | | | | |
|---|---|---|---|---|
| | | | | GGATTGGTGCAGCCTGGAGGGTCATTGAAACTCTCATGTGCAGCCTCTGGATTCACCTTCAATAT<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAAGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTATCCTTCTTCGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 197. | MCSP-G4 VH-VL-P x H2C VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 198. | MCSP-G4 VH-VL-P x H2C VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG |

| | | | | |
|---|---|---|---|---|
| | | | | GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 199. | MCSP-G4 VH-VL-P x H1E VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLEQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYLSFWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 200. | MCSP-G4 VH-VL-P x H1E VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGAGCAGCCTGGAGGGTCATTGAAACTCTCATGTGCAGCCTCTGGATTCACCTTCAATTC<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGGAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACCTATCCTTCTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC |

| | | | | |
|---|---|---|---|---|
| | | | | AGAATATTACTGTGCTCTATGGTACAGCAACCGCTGGGTGTTCGGTGGAGGAACCAAACTGACTG<br>TCCTA |
| 201. | MCSP-G4 VH-VL-P x G4H VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNRYAMNWVRQAPGKGLEWVARIRSKYNNYATYYADSVKGRFTISR<br>DDSKNTAYLQMNNLKTEDTAVYYCVRHGNFGNSYLSYFAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 202. | MCSP-G4 VH-VL-P x G4H VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATCG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGGAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACTTATCCTACTTCGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 203. | MCSP-G4 VH-VL-P x A2J VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG |

| | | | | |
|---|---|---|---|---|
| | | | | VPDRFSGSGSGTDFTLTISGLQAEDVAVYYCQQHYSTPFTFGPGTKVDIKSGGGGSEVQLLESGG<br>GLVQPGGSLKLSCAASGFTFNVYAMNWVRQAPGKGLEWVARIRSKYNNYATYYADSVKKRFTISR<br>DDSKNTAYLQMNNLKTEDTAVYYCVRHGNFGNSYLSWWAYWGQGTLVTVSSGGGGSGGGGSGGGG<br>SELVVTQEPSLTVSPGGTVTLTCRSSTGAVTSGYYPNWVQQKPGQAPRGLIGATDMRPSGTPARF<br>SGSLLGGKAALTLSGVQPEDEAEYYCALWYSNRWVFGGGTKLTVL |
| 204. | MCSP-G4 VH-VL-P x A2J VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATGT<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAAAGAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACTTATCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTCGCTCCTCGACTGGGGCTGTTACATCTGGCTACTACCCAAACTGGGTCCAACAAAAACCAG<br>GTCAGGCACCCCGTGGTCTAATAGGTGCCACTGACATGAGGCCCTCTGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 205. | MCSP-G4 VH-VL-P x E1L VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNKYAMNWVRQAPGKGLEWVARIRSKYNNYATYYADSVKSRFTISR<br>DDSKNTAYLQMNNLKTEDTAVYYCVRHGNFGNSYTSYYAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |

| | | | | |
|---|---|---|---|---|
| 206. | MCSP-G4 VH-VL-P x E1L VH-VL-P | artificial | nt | CAGGTGCAGCTGGTCCAGTCTGGGGCTGAGGTGAAGAGGCCTGGGGCCTCAATGAAGGTCTCCTGCAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGTGGTTCTGAGCTCGTGATGACCCAGTCTCCAGACTCCCTGGCTGTGTCTCTGGGCGAGAGGGCCACCATCAACTGCAAGTCCAGCCAGAGTGTTTTAAACAGCTCCAACAATAGGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGTGGCCTGCAGGCTGAAGATGTGGCAGTTTATTACTGTCAGCAACATTATAGTACTCCATTCACTTTTGGCCCTGGGACCAAAGTGGATATCAAATCCGGAGGTGGTGGATCCGAGGTGCAGCTGCTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAATCGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACACATCCTACTACGCTTACTGGGGCCAAGGGACTCTGGTCACCGTCTCCTCAGGTGGTGGTGGTTCTGGCGGCGGCGGCTCCGGTGGTGGTGGTTCTGAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 207. | MCSP-G4 VH-VL-P x E2M VH-VL-P | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGGGGSELVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIKSGGGGSEVQLLESGGGLVQPGGSLKLSCAASGFTFNGYAMNWVRQAPGKGLEWVARIRSKYNNYATYYADSVKERFTISRDDSKNTAYLQMNNLKTEDTAVYYCVRHRNFGNSYLSWFAYWGQGTLVTVSSGGGGSGGGGSGGGGSELVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |
| 208. | MCSP-G4 VH-VL-P x E2M VH-VL-P | artificial | nt | CAGGTGCAGCTGGTCCAGTCTGGGGCTGAGGTGAAGAGGCCTGGGGCCTCAATGAAGGTCTCCTGCAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTC |

| | | | | |
|---|---|---|---|---|
| | | | | AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATGG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGAGAGGTTCACCATCTCCAGA<br>GATGATTCAAAAAACACTGCCTATCTACAAATGAACAACTTGAAAACTGAGGACACTGCCGTGTA<br>CTACTGTGTGAGACATAGGAACTTCGGTAATAGCTACTTATCCTGGTTCGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTCGCTCCTCGACTGGGGCTGTTACATCTGGCTACTACCCAAACTGGGTCCAACAAAAACCAG<br>GTCAGGCACCCCGTGGTCTAATAGGTGCCACTGACATGAGGCCCTCTGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 209. | MCSP-G4 VH-VL-P x F7O VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNVYAMNWVRQAPGKGLEWVARIRSKYNNYATYYADSVKKRFTISR<br>DDSKNTAYLQMNNLKTEDTAVYYCVRHGNFGNSYISWWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 210. | MCSP-G4 VH-VL-P x F7O VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA |

| | | | | |
|---|---|---|---|---|
| | | | | GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATGT<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAAAGAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 211. | MCSP-G4 VH-VL-P x F12Q VH-VL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYVSWWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 212. | MCSP-G4 VH-VL-P x F12Q VH-VL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGCAGGTTCACCATCTCCAGA |

| | | | | |
|---|---|---|---|---|
| | | | | GATGATTCAAAAAACACTGCCTATCTACAAATGAACAACTTGAAAACTGAGGACACTGCCGTGTA<br>CTACTGTGTGAGACATGGGAACTTCGGTAATAGCTACGTTTCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 213. | MCSP-G4 VH-VL-P x I2C VH-VL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 214. | MCSP-G4 VH-VL-P x I2C VH-VL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG |

| | | | | |
|---|---|---|---|---|
| | | | | GTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 215. | MCSP-D2 VH-VL x H2C VH-VL | artificial | aa | QVQLVQSGAEVKKPGASVKVSCKASGYTFTGYYMHWVRQAPGQGLEWMGWINPNSGGTSYAQKFQGRVTMTRDTSTSTVYMELSNLRSDDTAVYYCAKSWVSWFASWGQGTLVTVSSGGGGSGGGGSGGGGSDIVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 216. | MCSP-D2 VH-VL x H2C VH-VL | artificial | nt | CAGGTGCAGCTGGTCCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAGCTACGCACAGAAGTTCCAGGGCAGAGTCACCATGACTAGGGACACGTCCACGAGCACAGTCTACATGGAGCTGAGCAACCTGAGATCTGACGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGTGGTTCTGATATCGTGATGACCCAGTCTCCAGACTCCCTGGCTGTGTCTCTGGGCGAGAGGGCCACCATCAACTGCAAGTCCAGCCAGAGTGTTTTAAACAGCTCCAACAATAGGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCAGTGGCCTGCAGGCTGAAGATGTGGCAGTTTATTACTGTCAGCAACATTATAGTACTCCATTCACTTTTGGCCCTGGGACCAAAGTGGATATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 217. | MCSP-D2 VH-VL x | artificial | aa | QVQLVQSGAEVKKPGASVKVSCKASGYTFTGYYMHWVRQAPGQGLEWMGWINPNSGGTSYAQKFQ |

| | | | | |
|---|---|---|---|---|
| | F12Q VH-VL | | | GRVTMTRDTSTSTVYMELSNLRSDDTAVYYCAKSWVSWFASWGQGTLVTVSSGGGGSGGGGSGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYVSWWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 218. | MCSP-D2 VH-VL x F12Q VH-VL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG<br>GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTTTCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 219. | MCSP-D2 VH-VL x I2C VH-VL | artificial | aa | QVQLVQSGAEVKKPGASVKVSCKASGYTFTGYYMHWVRQAPGQGLEWMGWINPNSGGTSYAQKFQ<br>GRVTMTRDTSTSTVYMELSNLRSDDTAVYYCAKSWVSWFASWGQGTLVTVSSGGGGSGGGGSGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG |

| | | | | |
|---|---|---|---|---|
| | | | | SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 220. | MCSP-D2 VH-VL x I2C VH-VL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG<br>GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 221. | MCSP-D2 VH-VL-P x H2C VH-VL-P | artificial | aa | QVQLVQSGAEVKKPGASVKVSCKASGYTFTGYYMHWVRQAPGQGLEWMGWINPNSGGTSYAQKFQ<br>GRVTMTRDTSTSTVYMELSNLRSDDTAVYYCAKSWVSWFASWGQGTLVTVSSGGGGSGGGGSGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 222. | MCSP-D2 VH-VL-P x H2C VH-VL-P | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG |

| | | | | |
|---|---|---|---|---|
| | | | | GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 223. | MCSP-F9 VH-VL x H2C VH-VL | artificial | aa | QVQLQESGPGLVKPSETLSLTCVVSGGSISSSNWWSWVRQPPGKGLEWLGTIYYNGNTYYNPSLK<br>SRVTISVDTSKNQFSLRLSSVTAADTAVYYCAKSWVSWFASWGQGTLVTVSSGGGGSGGGGSGGG<br>GSDIVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHYSVPFTFGPGTKVDIKGGGGSEVQLVESGGG<br>LVQPGGSLKLSCAASGFTFNKYAMNWVRQAPGKGLEWVARIRSKYNNYATYYADSVKDRFTISRD<br>DSKNTAYLQMNNLKTEDTAVYYCVRHGNFGNSYISYWAYWGQGTLVTVSSGGGGSGGGGSGGGGS<br>QTVVTQEPSLTVSPGGTVTLTCGSSTGAVTSGYYPNWVQQKPGQAPRGLIGGTKFLAPGTPARFS<br>GSLLGGKAALTLSGVQPEDEAEYYCALWYSNRWVFGGGTKLTVL |
| 224. | MCSP-F9 VH-VL x H2C VH-VL | artificial | nt | CAGGTGCAGCTGCAAGAGTCTGGCCCAGGACTGGTGAAGCCTTCGGAGACCCTGTCCCTCACCTG<br>CGTTGTCTCTGGTGGCTCCATCAGCAGTAGTAACTGGTGGAGCTGGGTCCGCCAGCCCCCAGGGA<br>AGGGACTGGAGTGGCTTGGGACTATCTATTATAATGGGAATACCTACTACAACCCGTCCCTCAAG<br>AGTCGAGTCACCATCTCCGTAGACACGTCCAAGAACCAGTTCTCCCTGAGGCTGAGCTCTGTGAC<br>CGCCGCAGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT |

| | | | | |
|---|---|---|---|---|
| | | | | ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCAGTTTATTACTGTCAGCAACATTATAGTGTTCCATTCACTTTCGGCCCTG<br>GGACCAAAGTGGATATCAAAGGAGGTGGTGGCTCCGAGGTGCAGCTGGTCGAGTCTGGAGGAGGA<br>TTGGTGCAGCCTGGAGGGTCATTGAAACTCTCATGTGCAGCCTCTGGATTCACCTTCAATAAGTA<br>CGCCATGAACTGGGTCCGCCAGGCTCCAGGAAAGGGTTTGGAATGGGTTGCTCGCATAAGAAGTA<br>AATATAATAATTATGCAACATATTATGCCGATTCAGTGAAAGACAGGTTCACCATCTCCAGAGAT<br>GATTCAAAAAACACTGCCTATCTACAAATGAACAACTTGAAAACTGAGGACACTGCCGTGTACTA<br>CTGTGTGAGACATGGGAACTTCGGTAATAGCTACATATCCTACTGGGCTTACTGGGGCCAAGGGA<br>CTCTGGTCACCGTCTCCTCAGGTGGTGGTGGTTCTGGCGGCGGCGGCTCCGGTGGTGGTGGTTCT<br>CAGACTGTTGTGACTCAGGAACCTTCACTCACCGTATCACCTGGTGGAACAGTCACACTCACTTG<br>TGGCTCCTCGACTGGGGCTGTTACATCTGGCTACTACCCAAACTGGGTCCAACAAAAACCAGGTC<br>AGGCACCCCGTGGTCTAATAGGTGGGACTAAGTTCCTCGCCCCCGGTACTCCTGCCAGATTCTCA<br>GGCTCCCTGCTTGGAGGCAAGGCTGCCCTCACCCTCTCAGGGGTACAGCCAGAGGATGAGGCAGA<br>ATATTACTGTGCTCTATGGTACAGCAACCGCTGGGTGTTCGGTGGAGGAACCAAACTGACTGTCC<br>TA |
| 225. | MCSP-F9 VH-VL-P x H2C VH-VL-P | artificial | aa | EVQLQESGPGLVKPSETLSLTCVVSGGSISSSNWWSWVRQPPGKGLEWLGTIYYNGNTYYNPSLK<br>SRVTISVDTSKNQFSLRLSSVTAADTAVYYCAKSWVSWFASWGQGTLVTVSSGGGGSGGGGSGGG<br>GSELVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHYSVPFTFGPGTKVDIKSGGGGSEVQLLESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 226. | MCSP-F9 VH-VL-P x H2C VH-VL-P | artificial | nt | GAGGTGCAGCTGCAAGAGTCTGGCCCAGGACTGGTGAAGCCTTCGGAGACCCTGTCCCTCACCTG<br>CGTTGTCTCTGGTGGCTCCATCAGCAGTAGTAACTGGTGGAGCTGGGTCCGCCAGCCCCCAGGGA<br>AGGGACTGGAGTGGCTTGGGACTATCTATTATAATGGGAATACCTACTACAACCCGTCCCTCAAG<br>AGTCGAGTCACCATCTCCGTAGACACGTCCAAGAACCAGTTCTCCCTGAGGCTGAGCTCTGTGAC<br>CGCCGCAGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT<br>ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCAGTTTATTACTGTCAGCAACATTATAGTGTTCCATTCACTTTCGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATAA |

| | | | | |
|---|---|---|---|---|
| | | | | GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 227. | MCSP-F9 VH-VL-P x G4H VH-VL-P | artificial | aa | EVQLQESGPGLVKPSETLSLTCVVSGGSISSSNWWSWVRQPPGKGLEWLGTIYYNGNTYYNPSLK<br>SRVTISVDTSKNQFSLRLSSVTAADTAVYYCAKSWVSWFASWGQGTLVTVSSGGGGSGGGGSGGG<br>GSELVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHYSVPFTFGPGTKVDIKSGGGGSEVQLLESGG<br>GLVQPGGSLKLSCAASGFTFNRYAMNWVRQAPGKGLEWVARIRSKYNNYATYYADSVKGRFTISR<br>DDSKNTAYLQMNNLKTEDTAVYYCVRHGNFGNSYLSYFAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 228. | MCSP-F9 VH-VL-P x G4H VH-VL-P | artificial | nt | GAGGTGCAGCTGCAAGAGTCTGGCCCAGGACTGGTGAAGCCTTCGGAGACCCTGTCCCTCACCTG<br>CGTTGTCTCTGGTGGCTCCATCAGCAGTAGTAACTGGTGGAGCTGGGTCCGCCAGCCCCCAGGGA<br>AGGGACTGGAGTGGCTTGGGACTATCTATTATAATGGGAATACCTACTACAACCCGTCCCTCAAG<br>AGTCGAGTCACCATCTCCGTAGACACGTCCAAGAACCAGTTCTCCCTGAGGCTGAGCTCTGTGAC<br>CGCCGCAGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT<br>ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCAGTTTATTACTGTCAGCAACATTATAGTGTTCCATTCACTTTCGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATCG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGGAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACTTATCCTACTTCGCTTACTGGGGCCAAG<br>GGACTCTGGTCACCGTCTCCTCAGGTGGTGGTGGTTCTGGCGGCGGCGGCTCCGGTGGTGGTGGT |

| | | | | |
|---|---|---|---|---|
| | | | | TCTGAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 229. | 1-27 CD3 epsilon-Fc | artificial | aa | QDGNEEMGGITQTPYKVSISGTTVILTSGEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGKHHHHHH |
| 230. | 1-27 CD3 epsilon-Fc | artificial | nt | ATGGGATGGAGCTGTATCATCCTCTTCTTGGTAGCAACAGCTACAGGTGTACACTCCCAAGATGGTAATGAAGAAATGGGTGGTATTACACAGACACCATATAAAGTCTCCATCTCTGGAACCACAGTAATATTGACATCCGGAGAGCCCAAATCTTGTGACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATCCCGGGAGGAGATGACCAAGAACCAGGTCAGCCTGACCTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTATAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCCCCGGGTAAACATCATCACCATCATCAT |
| 231. | human 1-27 CD3 epsilon -EpCAM | artificial | aa | QDGNEEMGGITQTPYKVSISGTTVILTDYKDDDDKTASFAAAQKECVCENYKLAVNCFLNDNGQCQCTSIGAQNTVLCSKLAAKCLVMKAEMNGSKLGRRAKPEGALQNNDGLYDPDCDESGLFKAKQCNGTSTCWCVNTAGVRRTDKDTEITCSERVRTYWIIIELKHKAREKPYDVQSLRTALEEAIKTRYQLDPKFITNILYEDNVITIDLVQNSSQKTQNDVDIADVAYYFEKDVKGESLFHSKKMDLRVNGEQLDLDPGQTLIYYVDEKAPEFSMQGLKAGVIAVIVVVVIAIVAGIVVLVISRKKRMAKYEKAEIKEMGEMHRELNA |
| 232. | human 1-27 CD3 epsilon -EpCAM | artificial | nt | ATGGGATGGAGCTGTATCATCCTCTTCTTGGTAGCAACAGCTACAGGTGTACACTCCCAAGATGGTAATGAAGAAATGGGTGGTATTACACAGACACCATATAAAGTCTCCATCTCTGGAACCACAGTAATATTGACAGATTACAAGGACGACGATGACAAGACTGCGAGTTTTGCCGCAGCTCAGAAAGAATGTGTCTGTGAAAACTACAAGCTGGCCGTAAACTGCTTTTTGAATGACAATGGTCAATGCCAGTGTACTTCGATTGGTGCACAAAATACTGTCCTTTGCTCAAAGCTGGCTGCCAAATGTTTGGTGATGAAGGCAGAAATGAACGGCTCAAAACTTGGGAGAAGAGCGAAACCTGAAGGGGCTCTCCAGAACAATGAT |

| | | | | |
|---|---|---|---|---|
| | | | | GGCCTTTACGATCCTGACTGCGATGAGAGCGGGCTCTTTAAGGCCAAGCAGTGCAACGGCACCTC<br>CACGTGCTGGTGTGTGAACACTGCTGGGGTCAGAAGAACTGACAAGGACACTGAAATAACCTGCT<br>CTGAGCGAGTGAGAACCTACTGGATCATCATTGAATTAAAACACAAAGCAAGAGAAAAACCTTAT<br>GATGTTCAAAGTTTGCGGACTGCACTTGAGGAGGCGATCAAAACGCGTTATCAACTGGATCCAAA<br>ATTTATCACAAATATTTTGTATGAGGATAATGTTATCACTATTGATCTGGTTCAAAATTCTTCTC<br>AGAAAACTCAGAATGATGTGGACATAGCTGATGTGGCTTATTATTTTGAAAAAGATGTTAAAGGT<br>GAATCCTTGTTTCATTCTAAGAAAATGGACCTGAGAGTAAATGGGGAACAACTGGATCTGGATCC<br>TGGTCAAACTTTAATTTATTATGTCGATGAAAAAGCACCTGAATTCTCAATGCAGGGTCTAAAAG<br>CTGGTGTTATTGCTGTTATTGTGGTTGTGGTGATAGCAATTGTTGCTGGAATTGTTGTGCTGGTT<br>ATTTCCAGAAAGAAGAGAATGGCAAAGTATGAGAAGGCTGAGATAAAGGAGATGGGTGAGATGCA<br>TAGGGAACTCAATGCA |
| 233. | marmoset 1-27 CD3 epsilon -EpCAM | artificial | aa | QDGNEEMGDTTQNPYKVSISGTTVTLTDYKDDDDKTASFAAAQKECVCENYKLAVNCFLNDNGQC<br>QCTSIGAQNTVLCSKLAAKCLVMKAEMNGSKLGRRAKPEGALQNNDGLYDPDCDESGLFKAKQCN<br>GTSTCWCVNTAGVRRTDKDTEITCSERVRTYWIIIELKHKAREKPYDVQSLRTALEEAIKTRYQL<br>DPKFITNILYEDNVITIDLVQNSSQKTQNDVDIADVAYYFEKDVKGESLFHSKKMDLRVNGEQLD<br>LDPGQTLIYYVDEKAPEFSMQGLKAGVIAVIVVVVIAIVAGIVVLVISRKKRMAKYEKAEIKEMG<br>EMHRELNA |
| 234. | marmoset 1-27 CD3 epsilon -EpCAM | artificial | nt | ATGGGATGGAGCTGTATCATCCTCTTCTTGGTAGCAACAGCTACAGGTGTACACTCCCAGGACGG<br>TAATGAAGAAATGGGTGATACTACACAGAACCCATATAAAGTTTCCATCTCAGGAACCACAGTAA<br>CACTGACAGATTACAAGGACGACGATGACAAGACTGCGAGTTTTGCCGCAGCTCAGAAAGAATGT<br>GTCTGTGAAAACTACAAGCTGGCCGTAAACTGCTTTTTGAATGACAATGGTCAATGCCAGTGTAC<br>TTCGATTGGTGCACAAAATACTGTCCTTTGCTCAAAGCTGGCTGCCAAATGTTTGGTGATGAAGG<br>CAGAAATGAACGGCTCAAAACTTGGGAGAAGAGCGAAACCTGAAGGGGCTCTCCAGAACAATGAT<br>GGCCTTTACGATCCTGACTGCGATGAGAGCGGGCTCTTTAAGGCCAAGCAGTGCAACGGCACCTC<br>CACGTGCTGGTGTGTGAACACTGCTGGGGTCAGAAGAACTGACAAGGACACTGAAATAACCTGCT<br>CTGAGCGAGTGAGAACCTACTGGATCATCATTGAATTAAAACACAAAGCAAGAGAAAAACCTTAT<br>GATGTTCAAAGTTTGCGGACTGCACTTGAGGAGGCGATCAAAACGCGTTATCAACTGGATCCAAA<br>ATTTATCACAAATATTTTGTATGAGGATAATGTTATCACTATTGATCTGGTTCAAAATTCTTCTC<br>AGAAAACTCAGAATGATGTGGACATAGCTGATGTGGCTTATTATTTTGAAAAAGATGTTAAAGGT<br>GAATCCTTGTTTCATTCTAAGAAAATGGACCTGAGAGTAAATGGGGAACAACTGGATCTGGATCC<br>TGGTCAAACTTTAATTTATTATGTCGATGAAAAAGCACCTGAATTCTCAATGCAGGGTCTAAAAG<br>CTGGTGTTATTGCTGTTATTGTGGTTGTGGTGATAGCAATTGTTGCTGGAATTGTTGTGCTGGTT<br>ATTTCCAGAAAGAAGAGAATGGCAAAGTATGAGAAGGCTGAGATAAAGGAGATGGGTGAGATGCA<br>TAGGGAACTCAATGCA |
| 235. | tamarin 1-27 CD3 epsilon -EpCAM | artificial | aa | QDGNEEMGDTTQNPYKVSISGTTVTLTDYKDDDDKTASFAAAQKECVCENYKLAVNCFLNDNGQC<br>QCTSIGAQNTVLCSKLAAKCLVMKAEMNGSKLGRRAKPEGALQNNDGLYDPDCDESGLFKAKQCN<br>GTSTCWCVNTAGVRRTDKDTEITCSERVRTYWIIIELKHKAREKPYDVQSLRTALEEAIKTRYQL |

| | | | | |
|---|---|---|---|---|
| | | | | DPKFITNILYEDNVITIDLVQNSSQKTQNDVDIADVAYYFEKDVKGESLFHSKKMDLRVNGEQLD<br>LDPGQTLIYYVDEKAPEFSMQGLKAGVIAVIVVVVIAIVAGIVVLVISRKKRMAKYEKAEIKEMG<br>EMHRELNA |
| 236. | tamarin 1-27 CD3 epsilon -EpCAM | artificial | nt | ATGGGATGGAGCTGTATCATCCTCTTCTTGGTAGCAACAGCTACAGGTGTACACTCCCAGGACGG<br>TAATGAAGAAATGGGTGATACTACACAGAACCCATATAAAGTTTCCATCTCAGGAACCACAGTAA<br>CACTGACAGATTACAAGGACGACGATGACAAGACTGCGAGTTTTGCCGCAGCTCAGAAAGAATGT<br>GTCTGTGAAAACTACAAGCTGGCCGTAAACTGCTTTTTGAATGACAATGGTCAATGCCAGTGTAC<br>TTCGATTGGTGCACAAAATACTGTCCTTTGCTCAAAGCTGGCTGCCAAATGTTTGGTGATGAAGG<br>CAGAAATGAACGGCTCAAAACTTGGGAGAAGAGCGAAACCTGAAGGGGCTCTCCAGAACAATGAT<br>GGCCTTTACGATCCTGACTGCGATGAGAGCGGGCTCTTTAAGGCCAAGCAGTGCAACGGCACCTC<br>CACGTGCTGGTGTGTGAACACTGCTGGGGTCAGAAGAACTGACAAGGACACTGAAATAACCTGCT<br>CTGAGCGAGTGAGAACCTACTGGATCATCATTGAATTAAAACACAAAGCAAGAGAAAAACCTTAT<br>GATGTTCAAAGTTTGCGGACTGCACTTGAGGAGGCGATCAAAACGCGTTATCAACTGGATCCAAA<br>ATTTATCACAAATATTTTGTATGAGGATAATGTTATCACTATTGATCTGGTTCAAAATTCTTCTC<br>AGAAAACTCAGAATGATGTGGACATAGCTGATGTGGCTTATTATTTTGAAAAAGATGTTAAAGGT<br>GAATCCTTGTTTCATTCTAAGAAAATGGACCTGAGAGTAAATGGGGAACAACTGGATCTGGATCC<br>TGGTCAAACTTTAATTTATTATGTCGATGAAAAAGCACCTGAATTCTCAATGCAGGGTCTAAAAG<br>CTGGTGTTATTGCTGTTATTGTGGTTGTGGTGATAGCAATTGTTGCTGGAATTGTTGTGCTGGTT<br>ATTTCCAGAAAGAAGAGAATGGCAAAGTATGAGAAGGCTGAGATAAAGGAGATGGGTGAGATGCA<br>TAGGGAACTCAATGCA |
| 237. | squirrel monkey 1-27 CD3 epsilon - EpCAM | artificial | aa | QDGNEEIGDTTQNPYKVSISGTTVTLTDYKDDDDKTASFAAAQKECVCENYKLAVNCFLNDNGQC<br>QCTSIGAQNTVLCSKLAAKCLVMKAEMNGSKLGRRAKPEGALQNNDGLYDPDCDESGLFKAKQCN<br>GTSTCWCVNTAGVRRTDKDTEITCSERVRTYWIIIELKHKAREKPYDVQSLRTALEEAIKTRYQL<br>DPKFITNILYEDNVITIDLVQNSSQKTQNDVDIADVAYYFEKDVKGESLFHSKKMDLRVNGEQLD<br>LDPGQTLIYYVDEKAPEFSMQGLKAGVIAVIVVVVIAIVAGIVVLVISRKKRMAKYEKAEIKEMG<br>EMHRELNA |
| 238. | squirrel monkey 1-27 CD3 epsilon - EpCAM | artificial | nt | ATGGGATGGAGCTGTATCATCCTCTTCTTGGTAGCAACAGCTACAGGTGTACACTCCCAGGACGG<br>TAATGAAGAGATTGGTGATACTACCCAGAACCCATATAAAGTTTCCATCTCAGGAACCACAGTAA<br>CACTGACAGATTACAAGGACGACGATGACAAGACTGCGAGTTTTGCCGCAGCTCAGAAAGAATGT<br>GTCTGTGAAAACTACAAGCTGGCCGTAAACTGCTTTTTGAATGACAATGGTCAATGCCAGTGTAC<br>TTCGATTGGTGCACAAAATACTGTCCTTTGCTCAAAGCTGGCTGCCAAATGTTTGGTGATGAAGG<br>CAGAAATGAACGGCTCAAAACTTGGGAGAAGAGCGAAACCTGAAGGGGCTCTCCAGAACAATGAT<br>GGCCTTTACGATCCTGACTGCGATGAGAGCGGGCTCTTTAAGGCCAAGCAGTGCAACGGCACCTC<br>CACGTGCTGGTGTGTGAACACTGCTGGGGTCAGAAGAACTGACAAGGACACTGAAATAACCTGCT<br>CTGAGCGAGTGAGAACCTACTGGATCATCATTGAATTAAAACACAAAGCAAGAGAAAAACCTTAT<br>GATGTTCAAAGTTTGCGGACTGCACTTGAGGAGGCGATCAAAACGCGTTATCAACTGGATCCAAA<br>ATTTATCACAAATATTTTGTATGAGGATAATGTTATCACTATTGATCTGGTTCAAAATTCTTCTC |

| | | | | |
|---|---|---|---|---|
| | | | | AGAAAACTCAGAATGATGTGGACATAGCTGATGTGGCTTATTATTTTGAAAAAGATGTTAAAGGT<br>GAATCCTTGTTTCATTCTAAGAAAATGGACCTGAGAGTAAATGGGGAACAACTGGATCTGGATCC<br>TGGTCAAACTTTAATTTATTATGTCGATGAAAAAGCACCTGAATTCTCAATGCAGGGTCTAAAAG<br>CTGGTGTTATTGCTGTTATTGTGGTTGTGGTGATAGCAATTGTTGCTGGAATTGTTGTGCTGGTT<br>ATTTCCAGAAAGAAGAGAATGGCAAAGTATGAGAAGGCTGAGATAAAGGAGATGGGTGAGATGCA<br>TAGGGAACTCAATGCA |
| 239. | swine 1-27 CD3 epsilon -EpCAM | artificial | aa | QEDIERPDEDTQKTFKVSISGDKVELTDYKDDDDKTASFAAAQKECVCENYKLAVNCFLNDNGQC<br>QCTSIGAQNTVLCSKLAAKCLVMKAEMNGSKLGRRAKPEGALQNNDGLYDPDCDESGLFKAKQCN<br>GTSTCWCVNTAGVRRTDKDTEITCSERVRTYWIIIELKHKAREKPYDVQSLRTALEEAIKTRYQL<br>DPKFITNILYEDNVITIDLVQNSSQKTQNDVDIADVAYYFEKDVKGESLFHSKKMDLRVNGEQLD<br>LDPGQTLIYYVDEKAPEFSMQGLKAGVIAVIVVVVIAIVAGIVVLVISRKKRMAKYEKAEIKEMG<br>EMHRELNA |
| 240. | swine 1-27 CD3 epsilon -EpCAM | artificial | nt | ATGGGATGGAGCTGTATCATCCTCTTCTTGGTAGCAACAGCTACAGGTGTACACTCCCAAGAAGA<br>CATTGAAAGACCAGATGAAGATACACAGAAAACATTTAAAGTCTCCATCTCTGGAGACAAAGTAG<br>AGCTGACAGATTACAAGGACGACGATGACAAGACTGCGAGTTTTGCCGCAGCTCAGAAAGAATGT<br>GTCTGTGAAAACTACAAGCTGGCCGTAAACTGCTTTTTGAATGACAATGGTCAATGCCAGTGTAC<br>TTCGATTGGTGCACAAAATACTGTCCTTTGCTCAAAGCTGGCTGCCAAATGTTTGGTGATGAAGG<br>CAGAAATGAACGGCTCAAAACTTGGGAGAAGAGCGAAACCTGAAGGGGCTCTCCAGAACAATGAT<br>GGCCTTTACGATCCTGACTGCGATGAGAGCGGGCTCTTTAAGGCCAAGCAGTGCAACGGCACCTC<br>CACGTGCTGGTGTGTGAACACTGCTGGGGTCAGAAGAACTGACAAGGACACTGAAATAACCTGCT<br>CTGAGCGAGTGAGAACCTACTGGATCATCATTGAATTAAAACACAAAGCAAGAGAAAAACCTTAT<br>GATGTTCAAAGTTTGCGGACTGCACTTGAGGAGGCGATCAAAACGCGTTATCAACTGGATCCAAA<br>ATTTATCACAAATATTTTGTATGAGGATAATGTTATCACTATTGATCTGGTTCAAAATTCTTCTC<br>AGAAAACTCAGAATGATGTGGACATAGCTGATGTGGCTTATTATTTTGAAAAAGATGTTAAAGGT<br>GAATCCTTGTTTCATTCTAAGAAAATGGACCTGAGAGTAAATGGGGAACAACTGGATCTGGATCC<br>TGGTCAAACTTTAATTTATTATGTCGATGAAAAAGCACCTGAATTCTCAATGCAGGGTCTAAAAG<br>CTGGTGTTATTGCTGTTATTGTGGTTGTGGTGATAGCAATTGTTGCTGGAATTGTTGTGCTGGTT<br>ATTTCCAGAAAGAAGAGAATGGCAAAGTATGAGAAGGCTGAGATAAAGGAGATGGGTGAGATGCA<br>TAGGGAACTCAATGCA |
| 241. | human CD3 epsilon chain | human | aa | QDGNEEMGGITQTPYKVSISGTTVILTCPQYPGSEILWQHNDKNIGGDEDDKNIGSDEDHLSLKE<br>FSELEQSGYYVCYPRGSKPEDANFYLYLRARVCENCMEMDVMSVATIVIVDICITGGLLLLVYYW<br>SKNRKAKAKPVTRGAGAGGRQRGQNKERPPPVPNPDYEPIRKGQRDLYSGLNQRRI |
| 242. | human CD3 epsilon chain | human | nt | ATGCAGTCGGGCACTCACTGGAGAGTTCTGGGCCTCTGCCTCTTATCAGTTGGCGTTTGGGGGCA<br>AGATGGTAATGAAGAAATGGGTGGTATTACACAGACACCATATAAAGTCTCCATCTCTGGAACCA<br>CAGTAATATTGACATGCCCTCAGTATCCTGGATCTGAAATACTATGGCAACACAATGATAAAAAC<br>ATAGGCGGTGATGAGGATGATAAAAACATAGGCAGTGATGAGGATCACCTGTCACTGAAGGAATT<br>TTCAGAATTGGAGCAAAGTGGTTATTATGTCTGCTACCCCAGAGGAAGCAAACCAGAAGATGCGA |

| | | | | |
|---|---|---|---|---|
| | | | | ACTTTTATCTCTACCTGAGGGCACGCGTGTGTGAGAACTGCATGGAGATGGATGTGATGTCGGTG<br>GCCACAATTGTCATAGTGGACATCTGCATCACTGGGGGCTTGCTGCTGCTGGTTTACTACTGGAG<br>CAAGAATAGAAAGGCCAAGGCCAAGCCTGTGACACGAGGAGCGGGTGCTGGCGGCAGGCAAAGGG<br>GACAAAACAAGGAGAGGCCACCACCTGTTCCCAACCCAGACTATGAGCCCATCCGGAAAGGCCAG<br>CGGGACCTGTATTCTGGCCTGAATCAGAGACGCATC |
| 243. | 19 amino acid immunoglobulin leader peptide | artificial | aa | MGWSCIILFLVATATGVHS |
| 244. | 19 amino acid immunoglobulin leader peptide | artificial | nt | ATGGGATGGAGCTGTATCATCCTCTTCTTGGTAGCAACAGCTACAGGTGTACACTCC |
| 245. | murine IgG1 heavy chain constant region | murine | aa | AKTTPPSVYPLAPGSAAQTNSMVTLGCLVKGYFPEPVTVTWNSGSLSSGVHTFPAVLQSDLYTLS<br>SSVTVPSSTWPSETVTCNVAHPASSTKVDKKIVPRDCGCKPCICTVPEVSSVFIFPPKPKDVLTI<br>TLTPKVTCVVVDISKDDPEVQFSWFVDDVEVHTAQTQPREEQFNSTFRSVSELPIMHQDWLNGKE<br>FKCRVNSAAFPAPIEKTISKTKGRPKAPQVYTIPPPKEQMAKDKVSLTCMITDFFPEDITVEWQW<br>NGQPAENYKNTQPIMDTDGSYFVYSKLNVQKSNWEAGNTFTCSVLHEGLHNHHTEKSLSHSPGK |
| 246. | murine IgG1 heavy chain constant region | murine | nt | GCCAAAACGACACCCCCATCTGTCTATCCACTGGCCCCTGGATCTGCTGCCCAAACTAACTCCAT<br>GGTGACCCTGGGATGCCTGGTCAAGGGCTATTTCCCTGAGCCAGTGACAGTGACCTGGAACTCTG<br>GATCCCTGTCCAGCGGTGTGCACACCTTCCCAGCTGTCCTGCAGTCTGACCTCTACACTCTGAGC<br>AGCTCAGTGACTGTCCCCTCCAGCACCTGGCCCAGCGAGACCGTCACCTGCAACGTTGCCCACCC<br>GGCCAGCAGCACCAAGGTGGACAAGAAAATTGTGCCCAGGGATTGTGGTTGTAAGCCTTGCATAT<br>GTACAGTCCCAGAAGTATCATCTGTCTTCATCTTCCCCCCAAAGCCCAAGGATGTGCTCACCATT<br>ACTCTGACTCCTAAGGTCACGTGTGTTGTGGTAGACATCAGCAAGGATGATCCCGAGGTCCAGTT<br>CAGCTGGTTTGTAGATGATGTGGAGGTGCACACAGCTCAGACGCAACCCCGGGAGGAGCAGTTCA<br>ACAGCACTTTCCGCTCAGTCAGTGAACTTCCCATCATGCACCAGGACTGGCTCAATGGCAAGGAG<br>TTCAAATGCAGGGTCAACAGTGCAGCTTTCCCTGCCCCCATCGAGAAAACCATCTCCAAAACCAA<br>AGGCAGACCGAAGGCTCCACAGGTGTACACCATTCCACCTCCCAAGGAGCAGATGGCCAAGGATA<br>AAGTCAGTCTGACCTGCATGATAACAGACTTCTTCCCTGAAGACATTACTGTGGAGTGGCAGTGG<br>AATGGGCAGCCAGCGGAGAACTACAAGAACACTCAGCCCATCATGGACACAGATGGCTCTTACTT<br>CGTCTACAGCAAGCTCAATGTGCAGAAGAGCAACTGGGAGGCAGGAAATACTTTCACCTGCTCTG<br>TGTTACATGAGGGCCTGCACAACCACCATACTGAGAAGAGCCTCTCCCACTCTCCTGGTAAA |
| 247. | human lambda light chain constant region | human | aa | GQPKAAPSVTLFPPSSEELQANKATLVCLISDFYPGAVTVAWKADSSPVKAGVETTTPSKQSNNK<br>YAASSYLSLTPEQWKSHKSYSCQVTHEGSTVEKTVAPTECS |
| 248. | human lambda light chain constant region | human | nt | GGTCAGCCCAAGGCTGCCCCCTCGGTCACTCTGTTCCCACCCTCCTCTGAGGAGCTTCAAGCCAA<br>CAAGGCCACACTGGTGTGTCTCATAAGTGACTTCTACCCGGGAGCCGTGACAGTGGCCTGGAAGG<br>CAGATAGCAGCCCCGTCAAGGCGGGAGTGGAGACCACCACACCCTCCAAACAAAGCAACAACAAG |

| | | | | |
|---|---|---|---|---|
| | | | | TACGCGGCCAGCAGCTACCTGAGCCTGACGCCTGAGCAGTGGAAGTCCCACAAAAGCTACAGCTG<br>CCAGGTCACGCATGAAGGGAGCACCGTGGAGAAGACAGTGGCCCCTACAGAATGTTCA |
| 249. | c-terminal domain construct of human MCSP | human | aa | DYKDDDDKSRTRSGSQLDGGLVLFSHRGTLDGGFRFRLSDGEHTSPGHFFRVTAQKQVLLSLKGS<br>QTLTVCPGSVQPLSSQTLRASSSAGTDPQLLLYRVVRGPQLGRLFHAQQDSTGEALVNFTQAEVY<br>AGNILYEHEMPPEPFWEAHDTLELQLSSPPARDVAATLAVAVSFEAACPQRPSHLWKNKGLWVPE<br>GQRARITVAALDASNLLASVPSPQRSEHDVLFQVTQFPSRGQLLVSEEPLHAGQPHFLQSQLAAG<br>QLVYAHGGGGTQQDGFHFRAHLQGPAGASVAGPQTSEAFAITVRDVNERPPQPQASVPLRLTRGS<br>RAPISRAQLSVVDPDSAPGEIEYEVQRAPHNGFLSLVGGGLGPVTRFTQADVDSGRLAFVANGSS<br>VAGIFQLSMSDGASPPLPMSLAVDILPSAIEVQLRAPLEVPQALGRSSLSQQQLRVVSDREEPEA<br>AYRLIQGPQYGHLLVGGRPTSAFSQFQIDQGEVVFAFTNSSSSHDHFRVLALARGVNASAVVNVT<br>VRALLHVWAGGPWPQGATLRLDPTVLDAGELANRTDSVPRFRLLEGPRHGRVVRVPRARTEPGGS<br>QLVEQFTQQDLEDGRLGLEVGRPEGRAPGPAGDSLTLELWAQGVPPAVASLDFATEPYNAARPYS<br>VALLSVPEAARTEAGKPESSTPTGEPGPMASSPEPAVAKGGFLSFLEANMFSVIIPMCLVLLLLA<br>LILPLLFYLRKRNKTGKHDVQVLTAKPRNGLAGDTETFRKVEPGQAIPLTAVPGQGPPPGGQPDP<br>ELLQFCRTPNPALKNGQYWV |
| 250. | c-terminal domain construct of human MCSP | human | nt | ATGGGATGGAGCTGTATCATCCTCTTCTTGGTAGCAACAGCTACAGGTGTACACTCCGACTACAA<br>AGACGATGACGACAAGTCCCGTACGAGATCTGGATCCCAATTGGACGGCGGGCTCGTGCTGTTCT<br>CACACAGAGGAACCCTGGATGGAGGCTTCCGCTTCCGCCTCTCTGACGGCGAGCACACTTCCCCC<br>GGACACTTCTTCCGAGTGACGGCCCAGAAGCAAGTGCTCCTCTCGCTGAAGGGCAGCCAGACACT<br>GACTGTCTGCCCAGGGTCCGTCCAGCCACTCAGCAGTCAGACCCTCAGGGCCAGCTCCAGCGCAG<br>GCACTGACCCCCAGCTCCTGCTCTACCGTGTGGTGCGGGGCCCCCAGCTAGGCCGGCTGTTCCAC<br>GCCCAGCAGGACAGCACAGGGGAGGCCCTGGTGAACTTCACTCAGGCAGAGGTCTACGCTGGGAA<br>TATTCTGTATGAGCATGAGATGCCCCCCGAGCCCTTTTGGGAGGCCCATGATACCCTAGAGCTCC<br>AGCTGTCCTCGCCGCCTGCCCGGGACGTGGCCGCCACCCTTGCTGTGGCTGTGTCTTTTGAGGCT<br>GCCTGTCCCCAGCGCCCCAGCCACCTCTGGAAGAACAAAGGTCTCTGGGTCCCCGAGGGCCAGCG<br>GGCCAGGATCACCGTGGCTGCTCTGGATGCCTCCAATCTCTTGGCCAGCGTTCCATCACCCCAGC<br>GCTCAGAGCATGATGTGCTCTTCCAGGTCACACAGTTCCCCAGCCGCGGCCAGCTGTTGGTGTCC<br>GAGGAGCCCCTCCATGCTGGGCAGCCCCACTTCCTGCAGTCCCAGCTGGCTGCAGGGCAGCTAGT<br>GTATGCCCACGGCGGTGGGGGCACCCAGCAGGATGGCTTCCACTTTCGTGCCCACCTCCAGGGGC<br>CAGCAGGGGCCTCCGTGGCTGGACCCCAAACCTCAGAGGCCTTTGCCATCACGGTGAGGGATGTA<br>AATGAGCGGCCCCCTCAGCCACAGGCCTCTGTCCCACTCCGGCTCACCCGAGGCTCTCGTGCCCC<br>CATCTCCCGGGCCCAGCTGAGTGTGGTGGACCCAGACTCAGCTCCTGGGGAGATTGAGTACGAGG<br>TCCAGCGGGCACCCCACAACGGCTTCCTCAGCCTGGTGGGTGGTGGCCTGGGGCCCGTGACCCGC<br>TTCACGCAAGCCGATGTGGATTCAGGGCGGCTGGCCTTCGTGGCCAACGGGAGCAGCGTGGCAGG<br>CATCTTCCAGCTGAGCATGTCTGATGGGGCCAGCCCACCCCTGCCCATGTCCCTGGCTGTGGACA<br>TCCTACCATCCGCCATCGAGGTGCAGCTGCGGGCACCCCTGGAGGTGCCCCAAGCTTTGGGGCGC<br>TCCTCACTGAGCCAGCAGCAGCTCCGGGTGGTTTCAGATCGGGAGGAGCCAGAGGCAGCATACCG |

| | | | | |
|---|---|---|---|---|
| | | | | GTTGATCCAGGGACCCCAGTATGGGCATCTCCTGGTGGGCGGGCGGCCCACCTCGGCCTTCAGCC AATTCCAGATAGACCAGGGCGAGGTGGTCTTTGCCTTCACCAACTCCTCCTCCTCTCATGACCAC TTCAGAGTCCTGGCACTGGCTAGGGGTGTCAATGCATCAGCCGTAGTGAACGTCACTGTGAGGGC TCTGCTGCATGTGTGGGCAGGTGGGCCATGGCCCCAGGGTGCCACCCTGCGCCTGGACCCCACCG TCCTAGATGCTGGCGAGCTGGCCAACCGCACAGACAGTGTGCCGCGCTTCCGCCTCCTGGAGGGA CCCCGGCATGGCCGCGTGGTCCGCGTGCCCCGAGCCAGGACGGAGCCCGGGGGCAGCCAGCTGGT GGAGCAGTTCACTCAGCAGGACCTTGAGGACGGGAGGCTGGGGCTGGAGGTGGGCAGGCCAGAGG GGAGGGCCCCCGGCCCCGCAGGTGACAGTCTCACTCTGGAGCTGTGGGCACAGGGCGTCCCGCCT GCTGTGGCCTCCCTGGACTTTGCCACTGAGCCTTACAATGCTGCCCGGCCCTACAGCGTGGCCCT GCTCAGTGTCCCCGAGGCCGCCCGGACGGAAGCAGGGAAGCCAGAGAGCAGCACCCCCACAGGCG AGCCAGGCCCCATGGCATCCAGCCCTGAGCCCGCTGTGGCCAAGGGAGGCTTCCTGAGCTTTCTA GAGGCCAACATGTTCAGCGTCATCATCCCCATGTGCCTGGTACTTCTGCTCCTGGCGCTCATCCT GCCCCTGCTCTTCTACCTCCGAAAACGCAACAAGACGGGCAAGCATGACGTCCAGGTCCTGACTG CCAAGCCCCGCAACGGCCTGGCTGGTGACACCGAGACCTTTCGCAAGGTGGAGCCAGGCCAGGCC ATCCCGCTCACAGCTGTGCCTGGCCAGGGGCCCCCTCCAGGAGGCCAGCCTGACCCAGAGCTGCT GCAGTTCTGCCGGACACCCAACCCTGCCCTTAAGAATGGCCAGTACTGGGTG |
| 251. | partial sequence of cynomolgus MCSP | Macaque (Cynomolgus) | aa | PSNGRVVLRAAPGTEVRSFTQAQLDGGLVLFSHRGTLDGGFRFGLSDGEHTSSGHFFRVTAQKQV LLSLEGSRTLTVCPGSVQPLSSQTLRASSSAGTDPQLLLYRVVRGPQLGRLFHAQQDSTGEALVN FTQAEVYAGNILYEHEMPTEPFWEAHDTLELQLSSPPARDVAATLAVAVSFEAACPQRPSHLWKN KGLWVPEGQRAKITMAALDASNLLASVPSSQRLEHDVLFQVTQFPSRGQLLVSEEPLHAGQPHFL QSQLAAGQLVYAHGGGGTQQDGFHFRAHLQGPAGATVAGPQTSEAFAITVRDVNERPPQPQASVP LRITRGSRAPISRAQLSVVDPDSAPGEIEYEVQRAPHNGFLSLVGGGPGPVNRFTQADVDSGRLA FVANGSSVAGVFQLSMSDGASPPLPMSLAVDILPSAIEVQLQAPLEVPQALGRSSLSQQQLRVVS DREEPEAAYRLIQGPKYGHLLVGGQPASAFSQLQIDQGEVVFAFTNFSSSHDHFRVLALARGVNA SAVVNITVRALLHVWAGGPWPQGATLRLDPTILDAGELANRTGSVPRFRLLEGPRHGRVVRVPRA RMEPGGSQLVEQFTQQDLEDGRLGLEVGRPEGRAPSPTGDSLTLELWAQGVPPAVASLDFATEPY NAARPYSVALLSVPEATRTEAGKPESSTPTGEPGPMASSPVPAVAKGGFLGFLEANMFSVIIPXC LVLLLLALILPLLFYLRKRNKTGKHDVQVLTAKPRNGLAGDTETFRKVEPGQAIPLTAVPGQGPP PGGQPDPELLQFCRTPNPALKNGQYWV |
| 252. | partial sequence of cynomolgus MCSP | Macaque (Cynomolgus) | nt | CCCAGCAACGGACGGGTAGTGCTGCGGGCGGCGCCGGGCACCGAGGTGCGCAGCTTCACGCAGGC CCAGCTGGATGGCGGACTCGTGCTGTTCTCACACAGAGGAACCCTGGATGGAGGCTTCCGCTTCG GCCTCTCCGATGGCGAGCACACTTCCTCTGGACACTTCTTCCGAGTGACGGCCCAGAAGCAAGTG CTCCTCTCGCTGGAGGGCAGCCGGACACTGACTGTCTGCCCAGGGTCCGTGCAGCCACTCAGCAG TCAGACCCTCAGAGCCAGCTCCAGCGCAGGCACCGACCCCCAGCTCCTGCTCTACCGTGTGGTGC GGGGCCCCCAGCTAGGCCGGCTGTTCCATGCCCAGCAGGACAGCACAGGGGAGGCCCTGGTGAAC TTCACTCAGGCAGAGGTCTATGCTGGGAATATTCTGTATGAGCATGAGATGCCCACCGAGCCCTT CTGGGAGGCCCATGATACCCTAGAGCTCCAGCTGTCCTCACCACCTGCCCCGGGACGTGGCTGCCA |

| | | | | |
|---|---|---|---|---|
| | | | | CCCTTGCTGTGGCTGTGTCTTTTGAGGCTGCCTGTCCCCAGCGCCCCAGCCACCTCTGGAAGAAC<br>AAAGGTCTCTGGGTCCCCGAGGGCCAGCGGGCCAAGATCACCATGGCTGCCCTGGATGCCTCCAA<br>CCTCTTGGCCAGCGTTCCATCATCCCAGCGCCTAGAGCATGATGTGCTCTTCCAGGTCACGCAGT<br>TCCCCAGCCGGGGCCAGCTATTGGTGTCTGAGGAGCCCCTCCACGCTGGGCAGCCCCACTTCCTG<br>CAGTCCCAGCTGGCTGCAGGGCAGCTAGTGTATGCCCACGGCGGTGGGGGTACCCAACAGGATGG<br>CTTCCACTTTCGTGCCCACCTCCAGGGGCCAGCAGGGGCCACCGTGGCTGGACCCCAAACCTCAG<br>AGGCTTTTGCCATCACGGTGCGGGATGTAAATGAGCGGCCCCCTCAGCCACAGGCCTCTGTCCCA<br>CTCCGGATCACCCGAGGCTCTCGAGCCCCCATCTCCCGGGCCCAGCTGAGTGTCGTGGACCCAGA<br>CTCAGCTCCTGGGGAGATTGAGTATGAGGTCCAGCGGGCACCCCACAACGGCTTCCTCAGCCTGG<br>TGGGTGGTGGCCCGGGGCCCGTGAACCGCTTCACGCAAGCCGATGTGGATTCGGGGCGGCTGGCC<br>TTCGTGGCCAACGGGAGCAGCGTAGCAGGCGTCTTCCAGCTGAGCATGTCTGATGGGGCCAGCCC<br>ACCGCTGCCCATGTCCCTGGCCGTGGACATCCTACCATCCGCCATCGAGGTGCAGCTGCAGGCAC<br>CCCTGGAGGTGCCCCAAGCTTTGGGGCGCTCCTCACTGAGCCAGCAGCAGCTCCGGGTGGTTTCA<br>GATAGGGAGGAGCCAGAGGCAGCATACCGCCTCATCCAGGGACCAAAGTACGGGCATCTCCTGGT<br>GGGTGGGCAGCCCGCCTCGGCCTTCAGCCAACTCCAGATAGACCAGGGCGAGGTGGTCTTTGCCT<br>TCACCAACTTCTCCTCCTCTCATGACCACTTCAGAGTCCTGGCACTGGCTAGGGGTGTCAACGCA<br>TCAGCCGTAGTGAACATCACTGTGAGGGCTCTGCTGCACGTGTGGGCAGGTGGGCCATGGCCCCA<br>GGGTGCTACCCTGCGCCTGGACCCAACCATCCTAGATGCTGGCGAGCTGGCCAACCGCACAGGCA<br>GTGTGCCCCGCTTCCGCCTCCTGGAGGGACCCCGGCATGGCCGCGTGGTCCGTGTGCCCCGAGCC<br>AGGATGGAGCCTGGGGGCAGCCAGCTGGTGGAGCAGTTCACTCAGCAGGACCTTGAGGATGGGAG<br>GCTGGGGCTGGAGGTGGGCAGGCCAGAGGGAAGGGCCCCCAGCCCCACAGGCGACAGTCTCACTC<br>TGGAGCTGTGGGCACAGGGCGTCCCACCTGCTGTGGCCTCCCTGGACTTTGCCACTGAGCCTTAC<br>AATGCTGCCCGGCCCTACAGCGTGGCCCTGCTCAGTGTCCCCGAGGCCACCCGGACGGAAGCAGG<br>GAAGCCAGAGAGCAGCACCCCCACAGGCGAGCCAGGCCCCATGGCATCTAGCCCTGTGCCTGCTG<br>TGGCCAAGGGAGGCTTCCTGGGCTTCCTTGAGGCCAACATGTTCAGTGTCATCATCCCCRTGTGC<br>CTGGTCCTTCTGCTCCTGGCGCTCATCTTGCCCCTGCTCTTCTACCTCCGAAAACGCAACAAGAC<br>GGGCAAGCATGACGTCCAGGTCCTGACTGCCAAGCCCCGCAATGGTCTGGCTGGTGACACTGAGA<br>CCTTTCGCAAGGTGGAGCCAGGCCAGGCCATCCCGCTCACAGCTGTGCCTGGCCAGGGGCCCCCT<br>CCGGGAGGCCAGCCTGACCCAGAGCTGCTGCAGTTCTGCCGGACACCCAACCCTGCCCTTAAGAA<br>TGGCCAGTACTGGGTG |
| 253. | PCR primer for CD3 epsilon chain - forward primer | artificial | nt | AGAGTTCTGGGCCTCTGC |
| 254. | PCR primer for CD3 epsilon chain - reverse primer | artificial | nt | CGGATGGGCTCATAGTCTG |
| 255. | His6-human CD3 | artificial | aa | HHHHHHQDGNEEMGGITQTPYKVSISGTTVILTCPQYPGSEILWQHNDKNIGGDEDDKNIGSDED |

| | | | | |
|---|---|---|---|---|
| | epsilon | | | HLSLKEFSELEQSGYYVCYPRGSKPEDANFYLYLRARVCENCMEMDVMSVATIVIVDICITGGLL<br>LLVYYWSKNRKAKAKPVTRGAGAGGRQRGQNKERPPPVPNPDYEPIRKGQRDLYSGLNQRRI |
| 256. | His6-human CD3 epsilon | artificial | nt | ATGGGATGGAGCTGTATCATCCTCTTCTTGGTAGCAACAGCTACAGGTGTACACTCCCATCATCA<br>CCATCATCATCAAGATGGTAATGAAGAAATGGGTGGTATTACACAGACACCATATAAAGTCTCCA<br>TCTCTGGAACCACAGTAATATTGACATGCCCTCAGTATCCTGGATCTGAAATACTATGGCAACAC<br>AATGATAAAAACATAGGCGGTGATGAGGATGATAAAAACATAGGCAGTGATGAGGATCACCTGTC<br>ACTGAAGGAATTTTCAGAATTGGAGCAAAGTGGTTATTATGTCTGCTACCCCAGAGGAAGCAAAC<br>CAGAAGATGCGAACTTTTATCTCTACCTGAGGGCACGCGTGTGTGAGAACTGCATGGAGATGGAT<br>GTGATGTCGGTGGCCACAATTGTCATAGTGGACATCTGCATCACTGGGGGCTTGCTGCTGCTGGT<br>TTACTACTGGAGCAAGAATAGAAAGGCCAAGGCCAAGCCTGTGACACGAGGAGCGGGTGCTGGCG<br>GCAGGCAAAGGGGACAAAACAAGGAGAGGCCACCACCTGTTCCCAACCCAGACTATGAGCCCATC<br>CGGAAAGGCCAGCGGGACCTGTATTCTGGCCTGAATCAGAGACGCATC |
| 257. | CD33 AH3 HL x H2C HL | artificial | aa | QVQLVQSGAEVKKPGESVKVSCKASGYTFTNYGMNWVRQAPGQGLEWMGWINTYTGEPTYADDFK<br>GRVTMSSDTSTSTAYLEINSLRSDDTAIYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 258. | CD33 AH3 HL x H2C HL | artificial | nt | CAGGTGCAGCTGGTGCAGTCTGGAGCTGAGGTGAAAAAGCCTGGAGAGTCAGTCAAGGTCTCCTG<br>CAAGGCTAGCGGGTATACCTTCACAAACTATGGAATGAACTGGGTGAGGCAGGCTCCAGGACAGG<br>GTTTAGAGTGGATGGGCTGGATAAACACCTACACTGGAGAGCCAACATATGCTGATGACTTCAAG<br>GGACGGGTTACCATGTCTTCGGATACCTCTACCAGCACTGCCTATTTGGAAATCAACAGCCTCAG<br>AAGTGATGACACGGCTATATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG |

| | | | | |
|---|---|---|---|---|
| | | | | CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCTACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGCTCTATGGTACAGCAACCGCTGGGTGTTCGGTGGAG<br>GAACCAAACTGACTGTCCTA |
| 259. | CD33 AH3 HL x F12Q HL | artificial | aa | QVQLVQSGAEVKKPGESVKVSCKASGYTFTNYGMNWVRQAPGQGLEWMGWINTYTGEPTYADDFK<br>GRVTMSSDTSTSTAYLEINSLRSDDTAIYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 260. | CD33 AH3 HL x F12Q HL | artificial | nt | CAGGTGCAGCTGGTGCAGTCTGGAGCTGAGGTGAAAAAGCCTGGAGAGTCAGTCAAGGTCTCCTG<br>CAAGGCTAGCGGGTATACCTTCACAAACTATGGAATGAACTGGGTGAGGCAGGCTCCAGGACAGG<br>GTTTAGAGTGGATGGGCTGGATAAACACCTACACTGGAGAGCCAACATATGCTGATGACTTCAAG<br>GGACGGGTTACCATGTCTTCGGATACCTCTACCAGCACTGCCTATTTGGAAATCAACAGCCTCAG<br>AAGTGATGACACGGCTATATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA |

| | | | | |
|---|---|---|---|---|
| | | | | GCCAGAGGATGAGGCAGAATATTACTGTGTTCTATGGTACAGCAACCGCTGGGTGTTCGGTGGAG<br>GAACCAAACTGACTGTCCTA |
| 261. | CD33 AH3 HL x I2C HL | artificial | aa | QVQLVQSGAEVKKPGESVKVSCKASGYTFTNYGMNWVRQAPGQGLEWMGWINTYTGEPTYADDFK<br>GRVTMSSDTSTSTAYLEINSLRSDDTAIYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 262. | CD33 AH3 HL x I2C HL | artificial | nt | CAGGTGCAGCTGGTGCAGTCTGGAGCTGAGGTGAAAAAGCCTGGAGAGTCAGTCAAGGTCTCCTG<br>CAAGGCTAGCGGGTATACCTTCACAAACTATGGAATGAACTGGGTGAGGCAGGCTCCAGGACAGG<br>GTTTAGAGTGGATGGGCTGGATAAACACCTACACTGGAGAGCCAACATATGCTGATGACTTCAAG<br>GGACGGGTTACCATGTCTTCGGATACCTCTACCAGCACTGCCTATTTGGAAATCAACAGCCTCAG<br>AAGTGATGACACGGCTATATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 263. | CD33 AF5 HL x H2C HL | artificial | aa | QVQLVQSGAEVKKPGASVKVSCKASGYTFTNYGMNWVKQAPGQGLKWMGWINTYTGEPTYADDFK<br>GRVTMTSDTSTSTAYLELHNLRSDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS |

| | | | | |
|---|---|---|---|---|
| | | | | TRESGIPDRFSGSGSGTDFTLTIDSLQPEDSATYYCQQSAHFPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 264. | CD33 AF5 HL x H2C HL | artificial | nt | CAGGTGCAGCTGGTGCAGTCTGGAGCTGAGGTGAAGAAGCCTGGAGCGTCAGTCAAGGTCTCCTGCAAGGCTAGCGGGTATACCTTCACAAACTATGGAATGAACTGGGTGAAGCAGGCTCCAGGACAGGGTTTAAAGTGGATGGGCTGGATAAACACCTACACTGGAGAGCCAACATATGCTGATGACTTCAAGGGACGGGTTACCATGACTTCGGATACCTCTACCAGCACTGCCTATTTGGAACTCCACAACCTCAGAAGTGATGACACGGCTGTATATTACTGTGCGCGCTGGAGTTGGAGTGATGGTTACTACGTTTACTTTGACTACTGGGGCCAAGGCACTACGGTCACCGTCTCCTCAGGTGGTGGTGGTTCTGGCGGCGGCGGCTCCGGTGGTGGTGGTTCTGACATCGTGATGACACAGTCTCCAGACTCCCTGACTGTGTCTCTGGGCGAGAGGACCACCATCAACTGCAAGTCCAGCCAGAGTGTTTTAGACAGCTCCAAGAATAAGAACTCCTTAGCTTGGTACCAGCAGAAACCAGGACAGCCTCCTAAATTACTCCTTTCCTGGGCATCTACGCGGGAATCCGGGATCCCTGACCGATTCAGTGGCAGCGGGTCTGGGACAGATTTCACTCTCACTATTGACAGCCTGCAGCCTGAAGATTCTGCAACTTACTATTGTCAACAGTCTGCCCACTTCCCGATCACCTTTGGCCAAGGGACACGACTGGAGATTAAATCCGGAGGTGGTGGCTCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 265. | CD33 AF5 HL x F12Q HL | artificial | aa | QVQLVQSGAEVKKPGASVKVSCKASGYTFTNYGMNWVKQAPGQGLKWMGWINTYTGEPTYADDFKGRVTMTSDTSTSTAYLELHNLRSDDTAVYYCARWSWSDGYYVYFDYWGQGTTVTVSSggggsggggsggggsDIVMTQSPDSLTVSLGERTTINCKSSQSVLDSSKNKNSLAWYQQKPGQPPKLLLSWASTRESGIPDRFSGSGSGTDFTLTIDSLQPEDSATYYCQQSAHFPITFGQGTRLEIKSGGGGSEVQLVESGGGLVQPGGSLKLSCAASGFTFNSYAMNWVRQAPGKGLEWVARIRSKYNNYATYYADSVKGRFTISRDDSKNTAYLQMNNLKTEDTAVYYCVRHGNFGNSYVSWWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |

| | | | | |
|---|---|---|---|---|
| 266. | CD33 AF5 HL x F12Q HL | artificial | nt | CAGGTGCAGCTGGTGCAGTCTGGAGCTGAGGTGAAGAAGCCTGGAGCGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTTCGGATACCTCTACCAGCACTGCCTATTTGGAACTCCACAACCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 267. | CD33 AF5 HL x I2C HL | artificial | aa | QVQLVQSGAEVKKPGASVKVSCKASGYTFTNYGMNWVKQAPGQGLKWMGWINTYTGEPTYADDFK<br>GRVTMTSDTSTSTAYLELHNLRSDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 268. | CD33 AF5 HL x I2C HL | artificial | nt | CAGGTGCAGCTGGTGCAGTCTGGAGCTGAGGTGAAGAAGCCTGGAGCGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTTCGGATACCTCTACCAGCACTGCCTATTTGGAACTCCACAACCTCAG<br>AAGTGATGACACGGCTGTATATTACTGTGCGCGCTGGAGTTGGAGTGATGGTTACTACGTTTACT |

| | | | | |
|---|---|---|---|---|
| | | | | TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 269. | CD33 AC8 HL x H2C HL | artificial | aa | QVQLVQSGAEVKKPGESVKVSCKASGYTFTNYGMNWVKQAPGQGLKWMGWINTYTGEPTYADDFK<br>GRVTMTTDTSTSTAYMEIRNLRNDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 270. | CD33 AC8 HL x H2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTACGGATACCTCTACCAGCACTGCCTATATGGAAATCCGCAACCTCAG<br>AAATGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC |

| | | | | |
|---|---|---|---|---|
| | | | | TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCTACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGCTCTATGGTACAGCAACCGCTGGGTGTTCGGTGGAG<br>GAACCAAACTGACTGTCCTA |
| 271. | CD33 AC8 HL x F12Q HL | artificial | aa | QVQLVQSGAEVKKPGESVKVSCKASGYTFTNYGMNWVKQAPGQGLKWMGWINTYTGEPTYADDFK<br>GRVTMTTDTSTSTAYMEIRNLRNDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 272. | CD33 AC8 HL x F12Q HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTACGGATACCTCTACCAGCACTGCCTATATGGAAATCCGCAACCTCAG<br>AAATGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG |

| | | | | |
|---|---|---|---|---|
| | | | | TTCACCATCTCCAGAGATGATTCAAAAAACACTGCCTATCTACAAATGAACAACTTGAAAACTGA GGACACTGCCGTGTACTACTGTGTGAGACATGGGAACTTCGGTAATAGCTACGTTTCCTGGTGGG CTTACTGGGGCCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATCACCTGGTGG AACAGTCACACTCACTTGTGGCTCCTCGACTGGGGCTGTTACATCTGGCAACTACCCAAACTGGG TCCAACAAAAACCAGGTCAGGCACCCCGTGGTCTAATAGGTGGGACTAAGTTCCTCGCCCCCGGT ACTCCTGCCAGATTCTCAGGCTCCCTGCTTGGAGGCAAGGCTGCCCTCACCCTCTCAGGGGTACA GCCAGAGGATGAGGCAGAATATTACTGTGTTCTATGGTACAGCAACCGCTGGGTGTTCGGTGGAG GAACCAAACTGACTGTCCTA |
| 273. | CD33 AC8 HL x I2C HL | artificial | aa | QVQLVQSGAEVKKPGESVKVSCKASGYTFTNYGMNWVKQAPGQGLKWMGWINTYTGEPTYADDFK GRVTMTTDTSTSTAYMEIRNLRNDDTAVYYCARWSWSDGYYVYFDYWGQGTTVTVSSggggsggg gsggggsDIVMTQSPDSLTVSLGERTTINCKSSQSVLDSSKNKNSLAWYQQKPGQPPKLLLSWAS TRESGIPDRFSGSGSGTDFTLTIDSLQPEDSATYYCQQSAHFPITFGQGTRLEIKSGGGGSEVQL VESGGGLVQPGGSLKLSCAASGFTFNKYAMNWVRQAPGKGLEWVARIRSKYNNYATYYADSVKDR FTISRDDSKNTAYLQMNNLKTEDTAVYYCVRHGNFGNSYISYWAYWGQGTLVTVSSGGGGSGGGG SGGGGSQTVVTQEPSLTVSPGGTVTLTCGSSTGAVTSGNYPNWVQQKPGQAPRGLIGGTKFLAPG TPARFSGSLLGGKAALTLSGVQPEDEAEYYCVLWYSNRWVFGGGTKLTVL |
| 274. | CD33 AC8 HL x I2C HL | artificial | nt | CAGGTGCAGCTGGTGCAGTCTGGAGCTGAGGTGAAGAAGCCTGGAGAGTCAGTCAAGGTCTCCTG CAAGGCTAGCGGGTATACCTTCACAAACTATGGAATGAACTGGGTGAAGCAGGCTCCAGGACAGG GTTTAAAGTGGATGGGCTGGATAAACACCTACACTGGAGAGCCAACATATGCTGATGACTTCAAG GGACGGGTTACCATGACTACGGATACCTCTACCAGCACTGCCTATATGGAAATCCGCAACCTCAG AAATGATGACACGGCTGTATATTACTGTGCGCGCTGGAGTTGGAGTGATGGTTACTACGTTTACT TTGACTACTGGGGCCAAGGCACTACGGTCACCGTCTCCTCAGGTGGTGGTGGTTCTGGCGGCGGC GGCTCCGGTGGTGGTGGTTCTGACATCGTGATGACACAGTCTCCAGACTCCCTGACTGTGTCTCT GGGCGAGAGGACCACCATCAACTGCAAGTCCAGCCAGAGTGTTTTAGACAGCTCCAAGAATAAGA ACTCCTTAGCTTGGTACCAGCAGAAACCAGGACAGCCTCCTAAATTACTCCTTTCCTGGGCATCT ACGCGGGAATCCGGGATCCCTGACCGATTCAGTGGCAGCGGGTCTGGGACAGATTTCACTCTCAC TATTGACAGCCTGCAGCCTGAAGATTCTGCAACTTACTATTGTCAACAGTCTGCCCACTTCCCGA TCACCTTTGGCCAAGGGACACGACTGGAGATTAAATCCGGAGGTGGTGGCTCCGAGGTGCAGCTG GTCGAGTCTGGAGGAGGATTGGTGCAGCCTGGAGGGTCATTGAAACTCTCATGTGCAGCCTCTGG ATTCACCTTCAATAAGTACGCCATGAACTGGGTCCGCCAGGCTCCAGGAAAGGGTTTGGAATGGG TTGCTCGCATAAGAAGTAAATATAATAATTATGCAACATATTATGCCGATTCAGTGAAAGACAGG TTCACCATCTCCAGAGATGATTCAAAAAACACTGCCTATCTACAAATGAACAACTTGAAAACTGA GGACACTGCCGTGTACTACTGTGTGAGACATGGGAACTTCGGTAATAGCTACATATCCTACTGGG CTTACTGGGGCCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATCACCTGGTGG AACAGTCACACTCACTTGTGGCTCCTCGACTGGGGCTGTTACATCTGGCAACTACCCAAACTGGG |

| | | | | |
|---|---|---|---|---|
| | | | | TCCAACAAAAACCAGGTCAGGCACCCCGTGGTCTAATAGGTGGGACTAAGTTCCTCGCCCCCGGT ACTCCTGCCAGATTCTCAGGCTCCCTGCTTGGAGGCAAGGCTGCCCTCACCCTCTCAGGGGTACA GCCAGAGGATGAGGCAGAATATTACTGTGTTCTATGGTACAGCAACCGCTGGGTGTTCGGTGGAG GAACCAAACTGACTGTCCTA |
| 275. | CD33 AH11 HL x H2C HL | artificial | aa | QVQLVQSGAEVKKPGESVKVSCKASGYTFTNYGMNWVKQAPGQGLKWMGWINTYTGEPTYADDFK GRVTMTSDTSTSTAYMEISSLRSDDTAVYYCARWSWSDGYYVYFDYWGQGTTVTVSSggggsggg gsggggsDIVMTQSPDSLTVSLGERTTINCKSSQSVLDSSKNKNSLAWYQQKPGQPPKLLLSWAS TRESGIPDRFSGSGSGTDFTLTIDSLQPEDSATYYCQQSAHFPITFGQGTRLEIKSGGGGSEVQL VESGGGLVQPGGSLKLSCAASGFTFNKYAMNWVRQAPGKGLEWVARIRSKYNNYATYYADSVKDR FTISRDDSKNTAYLQMNNLKTEDTAVYYCVRHGNFGNSYISYWAYWGQGTLVTVSSGGGGSGGGG SGGGGSQTVVTQEPSLTVSPGGTVTLTCGSSTGAVTSGYYPNWVQQKPGQAPRGLIGGTKFLAPG TPARFSGSLLGGKAALTLSGVQPEDEAEYYCALWYSNRWVFGGGTKLTVL |
| 276. | CD33 AH11 HL x H2C HL | artificial | nt | CAGGTGCAGCTGGTGCAGTCTGGAGCTGAGGTGAAGAAGCCTGGAGAGTCAGTCAAGGTCTCCTG CAAGGCTAGCGGGTATACCTTCACAAACTATGGAATGAACTGGGTGAAGCAGGCTCCAGGACAGG GTTTAAAGTGGATGGGCTGGATAAACACCTACACTGGAGAGCCAACATATGCTGATGACTTCAAG GGACGGGTTACCATGACTTCGGATACCTCTACCAGCACTGCCTATATGGAAATCAGCAGCCTCAG AAGTGATGACACGGCTGTATATTACTGTGCGCGCTGGAGTTGGAGTGATGGTTACTACGTTTACT TTGACTACTGGGGCCAAGGCACTACGGTCACCGTCTCCTCAGGTGGTGGTGGTTCTGGCGGCGGC GGCTCCGGTGGTGGTGGTTCTGACATCGTGATGACACAGTCTCCAGACTCCCTGACTGTGTCTCT GGGCGAGAGGACCACCATCAACTGCAAGTCCAGCCAGAGTGTTTTAGACAGCTCCAAGAATAAGA ACTCCTTAGCTTGGTACCAGCAGAAACCAGGACAGCCTCCTAAATTACTCCTTTCCTGGGCATCT ACGCGGGAATCCGGGATCCCTGACCGATTCAGTGGCAGCGGGTCTGGGACAGATTTCACTCTCAC TATTGACAGCCTGCAGCCTGAAGATTCTGCAACTTACTATTGTCAACAGTCTGCCCACTTCCCGA TCACCTTTGGCCAAGGGACACGACTGGAGATTAAATCCGGAGGTGGTGGCTCCGAGGTGCAGCTG GTCGAGTCTGGAGGAGGATTGGTGCAGCCTGGAGGGTCATTGAAACTCTCATGTGCAGCCTCTGG ATTCACCTTCAATAAGTACGCCATGAACTGGGTCCGCCAGGCTCCAGGAAAGGGTTTGGAATGGG TTGCTCGCATAAGAAGTAAATATAATAATTATGCAACATATTATGCCGATTCAGTGAAAGACAGG TTCACCATCTCCAGAGATGATTCAAAAAACACTGCCTATCTACAAATGAACAACTTGAAAACTGA GGACACTGCCGTGTACTACTGTGTGAGACATGGGAACTTCGGTAATAGCTACATATCCTACTGGG CTTACTGGGGCCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATCACCTGGTGG AACAGTCACACTCACTTGTGGCTCCTCGACTGGGGCTGTTACATCTGGCTACTACCCAAACTGGG TCCAACAAAAACCAGGTCAGGCACCCCGTGGTCTAATAGGTGGGACTAAGTTCCTCGCCCCCGGT ACTCCTGCCAGATTCTCAGGCTCCCTGCTTGGAGGCAAGGCTGCCCTCACCCTCTCAGGGGTACA GCCAGAGGATGAGGCAGAATATTACTGTGCTCTATGGTACAGCAACCGCTGGGTGTTCGGTGGAG GAACCAAACTGACTGTCCTA |
| 277. | CD33 AH11 HL x | artificial | aa | QVQLVQSGAEVKKPGESVKVSCKASGYTFTNYGMNWVKQAPGQGLKWMGWINTYTGEPTYADDFK |

| | | | | |
|---|---|---|---|---|
| | F12Q HL | | | GRVTMTSDTSTSTAYMEISSLRSDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 278. | CD33 AH11 HL x F12Q HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTTCGGATACCTCTACCAGCACTGCCTATATGGAAATCAGCAGCCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 279. | CD33 AH11 HL x I2C HL | artificial | aa | QVQLVQSGAEVKKPGESVKVSCKASGYTFTNYGMNWVKQAPGQGLKWMGWINTYTGEPTYADDFK<br>GRVTMTSDTSTSTAYMEISSLRSDDTAVYYCARWSWSDGYYVYFDYWGQGTTVTVSSggggsggg<br>gsggggsDIVMTQSPDSLTVSLGERTTINCKSSQSVLDSSKNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG |

| | | | | |
|---|---|---|---|---|
| | | | | SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 280. | CD33 AH11 HL x I2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAAAGTGGATGGGCTGGATAAACACCTACACTGGAGAGCCAACATATGCTGATGACTTCAAG<br>GGACGGGTTACCATGACTTCGGATACCTCTACCAGCACTGCCTATATGGAAATCAGCAGCCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAAGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 281. | CD33 B3 HL x H2C HL | artificial | aa | QVQLVQSGAEVKKPGESVKVSCKASGYTFTNYGMNWVKQAPGQGLEWMGWINTYTGETNYADKFQ<br>GRVTFTSDTSTSTAYMELRNLKSDDTAVYYCARWSWSDGYYVYFDYWGQGTTVTVSSggggsggg<br>gsggggsDIVMTQSPDSMTVSLGERTTINCKSSQSVLDSSTNKNSLAWYQQKPGQPPKLLLSWAS<br>TRESGIPDRFSGSGSGTDFTLTIDSLQPEDSATYYCQQSAHFPITFGQGTRLDIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 282. | CD33 B3 HL x H2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGACAAACTATGCTGATAAGTTCCAG |

| | | | | |
|---|---|---|---|---|
| | | | | GGACGCGTTACCTTCACTTCGGATACCTCTACCAGCACTGCCTATATGGAACTCCGCAACCTCAA AAGTGATGACACGGCTGTATATTACTGTGCGCGCTGGAGTTGGAGTGATGGTTACTACGTTTACT TTGACTACTGGGGCCAAGGCACTACGGTCACCGTCTCCTCAGGTGGTGGTGGTTCTGGCGGCGGC GGCTCCGGTGGTGGTGGTTCTGACATCGTGATGACACAGTCTCCAGACTCCATGACTGTGTCTCT GGGCGAGAGGACCACCATCAACTGCAAGTCCAGCCAGAGTGTTTTAGACAGCTCCACGAATAAGA ACTCCTTAGCTTGGTACCAGCAGAAACCAGGACAGCCTCCTAAATTACTCCTTTCCTGGGCATCT ACGCGGGAATCCGGGATCCCTGACCGATTCAGTGGCAGCGGGTCTGGGACAGATTTCACTCTCAC TATTGACAGCCTGCAGCCTGAAGATTCTGCAACTTACTATTGTCAACAGTCTGCCCACTTCCCGA TCACCTTTGGCCAAGGGACACGACTGGACATTAAATCCGGAGGTGGTGGCTCCGAGGTGCAGCTG GTCGAGTCTGGAGGAGGATTGGTGCAGCCTGGAGGGTCATTGAAACTCTCATGTGCAGCCTCTGG ATTCACCTTCAATAAGTACGCCATGAACTGGGTCCGCCAGGCTCCAGGAAAGGGTTTGGAATGGG TTGCTCGCATAAGAAGTAAATATAATAATTATGCAACATATTATGCCGATTCAGTGAAAGACAGG TTCACCATCTCCAGAGATGATTCAAAAAACACTGCCTATCTACAAATGAACAACTTGAAAACTGA GGACACTGCCGTGTACTACTGTGTGAGACATGGGAACTTCGGTAATAGCTACATATCCTACTGGG CTTACTGGGGCCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATCACCTGGTGG AACAGTCACACTCACTTGTGGCTCCTCGACTGGGGCTGTTACATCTGGCTACTACCCAAACTGGG TCCAACAAAAACCAGGTCAGGCACCCCGTGGTCTAATAGGTGGGACTAAGTTCCTCGCCCCCGGT ACTCCTGCCAGATTCTCAGGCTCCCTGCTTGGAGGCAAGGCTGCCCTCACCCTCTCAGGGGTACA GCCAGAGGATGAGGCAGAATATTACTGTGCTCTATGGTACAGCAACCGCTGGGTGTTCGGTGGAG GAACCAAACTGACTGTCCTA |
| 283. | CD33 B3 HL x F12Q HL | artificial | aa | QVQLVQSGAEVKKPGESVKVSCKASGYTFTNYGMNWVKQAPGQGLEWMGWINTYTGETNYADKFQ GRVTFTSDTSTSTAYMELRNLKSDDTAVYYCARWSWSDGYYVYFDYWGQGTTVTVSSggggsggg gsggggsDIVMTQSPDSMTVSLGERTTINCKSSQSVLDSSTNKNSLAWYQQKPGQPPKLLLSWAS TRESGIPDRFSGSGSGTDFTLTIDSLQPEDSATYYCQQSAHFPITFGQGTRLDIKSGGGGSEVQL VESGGGLVQPGGSLKLSCAASGFTFNSYAMNWVRQAPGKGLEWVARIRSKYNNYATYYADSVKGR FTISRDDSKNTAYLQMNNLKTEDTAVYYCVRHGNFGNSYVSWWAYWGQGTLVTVSSGGGGSGGGG SGGGGSQTVVTQEPSLTVSPGGTVTLTCGSSTGAVTSGNYPNWVQQKPGQAPRGLIGGTKFLAPG TPARFSGSLLGGKAALTLSGVQPEDEAEYYCVLWYSNRWVFGGGTKLTVL |
| 284. | CD33 B3 HL x F12Q HL | artificial | nt | CAGGTGCAGCTGGTGCAGTCTGGAGCTGAGGTGAAGAAGCCTGGAGAGTCAGTCAAGGTCTCCTG CAAGGCTAGCGGGTATACCTTCACAAACTATGGAATGAACTGGGTGAAGCAGGCTCCAGGACAGG GTTTAGAGTGGATGGGCTGGATAAACACCTACACTGGAGAGACAAACTATGCTGATAAGTTCCAG GGACGCGTTACCTTCACTTCGGATACCTCTACCAGCACTGCCTATATGGAACTCCGCAACCTCAA AAGTGATGACACGGCTGTATATTACTGTGCGCGCTGGAGTTGGAGTGATGGTTACTACGTTTACT TTGACTACTGGGGCCAAGGCACTACGGTCACCGTCTCCTCAGGTGGTGGTGGTTCTGGCGGCGGC GGCTCCGGTGGTGGTGGTTCTGACATCGTGATGACACAGTCTCCAGACTCCATGACTGTGTCTCT GGGCGAGAGGACCACCATCAACTGCAAGTCCAGCCAGAGTGTTTTAGACAGCTCCACGAATAAGA |

| | | | | |
|---|---|---|---|---|
| | | | | ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGACATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 285. | CD33 B3 HL x I2C HL | artificial | aa | QVQLVQSGAEVKKPGESVKVSCKASGYTFTNYGMNWVKQAPGQGLEWMGWINTYTGETNYADKFQ<br>GRVTFTSDTSTSTAYMELRNLKSDDTAVYYCARWSWSDGYYVYFDYWGQGTTVTVSSggggsggg<br>gsggggsDIVMTQSPDSMTVSLGERTTINCKSSQSVLDSSTNKNSLAWYQQKPGQPPKLLLSWAS<br>TRESGIPDRFSGSGSGTDFTLTIDSLQPEDSATYYCQQSAHFPITFGQGTRLDIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 286. | CD33 B3 HL x I2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGACAAACTATGCTGATAAGTTCCAG<br>GGACGCGTTACCTTCACTTCGGATACCTCTACCAGCACTGCCTATATGGAACTCCGCAACCTCAA<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCATGACTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGACATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG |

| | | | | |
|---|---|---|---|---|
| | | | | ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 287. | CD33 F2 HL x H2C HL | artificial | aa | QVQLVQSGAEVKKPGESVKVSCKASGYTFTNYGMNWVKQAPGQGLEWMGWINTYTGETNYADKFQ<br>GRVTFTSDTSTSTAYMELRNLKSDDTAVYYCARWSWSDGYYVYFDYWGQGTTVTVSSggggsggg<br>gsggggsDIVMTQSPDSLSVSLGERTTINCKSSQSVLDSSTNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 288. | CD33 F2 HL x H2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGACAAACTATGCTGATAAGTTCCAG<br>GGACGCGTTACCTTCACTTCGGATACCTCTACCAGCACTGCCTATATGGAACTCCGCAACCTCAA<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGTCTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC |

| | | | | |
|---|---|---|---|---|
| | | | | TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCTACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGCTCTATGGTACAGCAACCGCTGGGTGTTCGGTGGAG<br>GAACCAAACTGACTGTCCTA |
| 289. | CD33 F2 HL x F12Q HL | artificial | aa | QVQLVQSGAEVKKPGESVKVSCKASGYTFTNYGMNWVKQAPGQGLEWMGWINTYTGETNYADKFQ<br>GRVTFTSDTSTSTAYMELRNLKSDDTAVYYCARWSWSDGYYVYFDYWGQGTTVTVSSggggsggg<br>gsggggsDIVMTQSPDSLSVSLGERTTINCKSSQSVLDSSTNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 290. | CD33 F2 HL x F12Q HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGACAAACTATGCTGATAAGTTCCAG<br>GGACGCGTTACCTTCACTTCGGATACCTCTACCAGCACTGCCTATATGGAACTCCGCAACCTCAA<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGTCTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG |

| | | | | |
|---|---|---|---|---|
| | | | | GAACCAAACTGACTGTCCTA |
| 291. | CD33 F2 HL x I2C HL | artificial | aa | QVQLVQSGAEVKKPGESVKVSCKASGYTFTNYGMNWVKQAPGQGLEWMGWINTYTGETNYADKFQ<br>GRVTFTSDTSTSTAYMELRNLKSDDTAVYYCARWSWSDGYYVYFDYWGQGTTVTVSSggggsggg<br>gsggggsDIVMTQSPDSLSVSLGERTTINCKSSQSVLDSSTNKNSLAWYQQKPGQPPKLLLSWAS<br>TRESGIPDRFSGSGSGTDFTLTIDSLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 292. | CD33 F2 HL x I2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGACAAACTATGCTGATAAGTTCCAG<br>GGACGCGTTACCTTCACTTCGGATACCTCTACCAGCACTGCCTATATGGAACTCCGCAACCTCAA<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGTCTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 293. | CD33 B10 HL x H2C HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLRSDDTAVYYCARWSWSDGYYVYFDYWGQGTTVTVSSggggsggg<br>gsggggsDIVMTQSPDSLTVSLGERTTINCKSSQSVLDSSNNKNSLAWYQQKPGQPPKLLLSWAS<br>TRESGIPDRFSGSGSGTDFTLTIDGLQPEDSATYYCQQSAHFPITFGQGTRLEIKSGGGGSEVQL |

| | | | | |
|---|---|---|---|---|
| | | | | VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 294. | CD33 B10 HL x H2C HL | artificial | nt | CAGGTGCAGCTGGTGCAGTCTGGAGCTGAGGTGAAGAAGCCTGGTGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAACAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGTTCTGGGACAGATTTCACTCTCAC<br>TATTGACGGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCTACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGCTCTATGGTACAGCAACCGCTGGGTGTTCGGTGGAG<br>GAACCAAACTGACTGTCCTA |
| 295. | CD33 B10 HL x F12Q HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLRSDDTAVYYCARWSWSDGYYVYFDYWGQGTTVTVSSggggsggg<br>gsggggsDIVMTQSPDSLTVSLGERTTINCKSSQSVLDSSNNKNSLAWYQQKPGQPPKLLLSWAS<br>TRESGIPDRFSGSGSGTDFTLTIDGLQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 296. | CD33 B10 HL x | artificial | nt | CAGGTGCAGCTGGTGCAGTCTGGAGCTGAGGTGAAGAAGCCTGGTGAGTCAGTCAAGGTCTCCTG |

| | | | | |
|---|---|---|---|---|
| | F12Q HL | | | CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAACAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGTTCTGGGACAGATTTCACTCTCAC<br>TATTGACGGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 297. | CD33 B10 HL x I2C HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLRSDDTAVYYCARWSWSDGYYVYFDYWGQGTTVTVSSggggsggg<br>gsggggsDIVMTQSPDSLTVSLGERTTINCKSSQSVLDSSNNKNSLAWYQQKPGQPPKLLLSWAS<br>TRESGIPDRFSGSGSGTDFTLTIDGLQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 298. | CD33 B10 HL x I2C HL | artificial | nt | CAGGTGCAGCTGGTGCAGTCTGGAGCTGAGGTGAAGAAGCCTGGTGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCAG<br>AAGTGATGACACGGCTGTATATTACTGTGCGCGCTGGAGTTGGAGTGATGGTTACTACGTTTACT<br>TTGACTACTGGGGCCAAGGCACTACGGTCACCGTCTCCTCAGGTGGTGGTGGTTCTGGCGGCGGC |

| | | | | |
|---|---|---|---|---|
| | | | | GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCAACAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGTTCTGGGACAGATTTCACTCTCAC<br>TATTGACGGCCTGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 299. | CD33 E11 HL x H2C HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLGGDDTAVYYCARWSWSDGYYVYFDYWGQGTSVTVSSggggsggg<br>gsggggsDIVMTQSPDSLTVSLGERTTINCKSSQSVLDSSTNKNSLAWYQQKPGQPPKLLLSWAS<br>TRESGIPDRFSGSGSGTDFTLTIDSPQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGYYPNWVQQKPGQAPRGLIGGTKFLAPG<br>TPARFSGSLLGGKAALTLSGVQPEDEAEYYCALWYSNRWVFGGGTKLTVL |
| 300. | CD33 E11 HL x H2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCGG<br>AGGTGATGACACGGCTGTATATTACTGTGCGCGCTGGAGTTGGAGTGATGGTTACTACGTTTACT<br>TTGACTACTGGGGCCAAGGCACTTCGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCCGCAGCCTGAAGATTCTGCAACTTACTATTGTCAACAGTCTGCCCACTTCCCGA |

| | | | | |
|---|---|---|---|---|
| | | | | TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCTACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGCTCTATGGTACAGCAACCGCTGGGTGTTCGGTGGAG<br>GAACCAAACTGACTGTCCTA |
| 301. | CD33 E11 HL x F12Q HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLGGDDTAVYYCARWSWSDGYYVYFDYWGQGTSVTVSSggggsggg<br>gsggggsDIVMTQSPDSLTVSLGERTTINCKSSQSVLDSSTNKNSLAWYQQKPGQPPKLLLSWAS<br>TRESGIPDRFSGSGSGTDFTLTIDSPQPEDSATYYCQQSAHFPITFGQGTRLEIKSGGGGSEVQL<br>VESGGGLVQPGGSLKLSCAASGFTFNSYAMNWVRQAPGKGLEWVARIRSKYNNYATYYADSVKGR<br>FTISRDDSKNTAYLQMNNLKTEDTAVYYCVRHGNFGNSYVSWWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 302. | CD33 E11 HL x F12Q HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCGG<br>AGGTGATGACACGGCTGTATATTACTGTGCGCGCTGGAGTTGGAGTGATGGTTACTACGTTTACT<br>TTGACTACTGGGGCCAAGGCACTTCGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCCGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAGCTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGGCAGG<br>TTCACCATCTCCAGAGATGATTCAAAAAACACTGCCTATCTACAAATGAACAACTTGAAAACTGA |

| | | | | |
|---|---|---|---|---|
| | | | | GGACACTGCCGTGTACTACTGTGTGAGACATGGGAACTTCGGTAATAGCTACGTTTCCTGGTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT<br>ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 303. | CD33 E11 HL x I2C HL | artificial | aa | QVQLVQSGAEVKKPGESVKVSCKASGYTFTNYGMNWVKQAPGQGLEWMGWINTYTGEPTYADKFQ<br>GRVTMTTDTSTSTAYMEIRNLGGDDTAVYYCARWSWSDGYYVYFDYWGQGTSVTVSSggggsggg<br>gsggggsDIVMTQSPDSLTVSLGERTTINCKSSQSVLDSSTNKNSLAWYQQKPGQPPKLLLSWAS<br>TRESGIPDRFSGSGSGTDFTLTIDSPQPEDSATYYCQQSAHFPITFGQGTRLEIKSGGGGSEVQL<br>VESGGGLVQPGGSLKLSCAASGFTFNKYAMNWVRQAPGKGLEWVARIRSKYNNYATYYADSVKDR<br>FTISRDDSKNTAYLQMNNLKTEDTAVYYCVRHGNFGNSYISYWAYWGQGTLVTVSSGGGGSGGGG<br>SGGGGSQTVVTQEPSLTVSPGGTVTLTCGSSTGAVTSGNYPNWVQQKPGQAPRGLIGGTKFLAPG<br>TPARFSGSLLGGKAALTLSGVQPEDEAEYYCVLWYSNRWVFGGGTKLTVL |
| 304. | CD33 E11 HL x I2C HL | artificial | nt | CAGGTGCAGCTGGTGCAGTCTGGAGCTGAGGTGAAGAAGCCTGGAGAGTCAGTCAAGGTCTCCTG<br>CAAGGCTAGCGGGTATACCTTCACAAACTATGGAATGAACTGGGTGAAGCAGGCTCCAGGACAGG<br>GTTTAGAGTGGATGGGCTGGATAAACACCTACACTGGAGAGCCAACCTATGCTGATAAGTTCCAG<br>GGACGCGTTACCATGACTACGGATACCTCTACCAGCACTGCCTATATGGAAATCCGCAACCTCGG<br>AGGTGATGACACGGCTGTATATTACTGTGCGCGCTGGAGTTGGAGTGATGGTTACTACGTTTACT<br>TTGACTACTGGGGCCAAGGCACTTCGGTCACCGTCTCCTCAGGTGGTGGTGGTTCTGGCGGCGGC<br>GGCTCCGGTGGTGGTGGTTCTGACATCGTGATGACACAGTCTCCAGACTCCCTGACTGTGTCTCT<br>GGGCGAGAGGACCACCATCAACTGCAAGTCCAGCCAGAGTGTTTTAGACAGCTCCACGAATAAGA<br>ACTCCTTAGCTTGGTACCAGCAGAAACCAGGACAGCCTCCTAAATTACTCCTTTCCTGGGCATCT<br>ACGCGGGAATCCGGGATCCCTGACCGATTCAGTGGCAGCGGGTCTGGGACAGATTTCACTCTCAC<br>TATTGACAGCCCGCAGCCTGAAGATTCTGCAACTTACTATTGTCAACAGTCTGCCCACTTCCCGA<br>TCACCTTTGGCCAAGGGACACGACTGGAGATTAAATCCGGAGGTGGTGGCTCCGAGGTGCAGCTG<br>GTCGAGTCTGGAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTTTGGAATGGG<br>TTGCTCGCATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGAAAACTGA<br>GGACACTGCCGTGTACTACTGTGTGAGACATGGGAACTTCGGTAATAGCTACATATCCTACTGGG<br>CTTACTGGGGCCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATCACCTGGTGG<br>AACAGTCACACTCACTTGTGGCTCCTCGACTGGGGCTGTTACATCTGGCAACTACCCAAACTGGG<br>TCCAACAAAAACCAGGTCAGGCACCCCGTGGTCTAATAGGTGGGACTAAGTTCCTCGCCCCCGGT |

| | | | | |
|---|---|---|---|---|
| | | | | ACTCCTGCCAGATTCTCAGGCTCCCTGCTTGGAGGCAAGGCTGCCCTCACCCTCTCAGGGGTACA<br>GCCAGAGGATGAGGCAGAATATTACTGTGTTCTATGGTACAGCAACCGCTGGGTGTTCGGTGGAG<br>GAACCAAACTGACTGTCCTA |
| 305. | CD33 | human | nt | ATGGGATGGAGCTGTATCATCCTCTTCTTGGTAGCAACAGCTACAGGTGTACACTCCGATCCAAA<br>TTTCTGGCTGCAAGTGCAGGAGTCAGTGACGGTACAGGAGGGTTTGTGCGTCCTCGTGCCCTGCA<br>CTTTCTTCCATCCCATACCCTACTACGACAAGAACTCCCCAGTTCATGGTTACTGGTTCCGGGAA<br>GGAGCCATTATATCCGGGGACTCTCCAGTGGCCACAAACAAGCTAGATCAAGAAGTACAGGAGGA<br>GACTCAGGGCAGATTCCGCCTCCTTGGGGATCCCAGTAGGAACAACTGCTCCCTGAGCATCGTAG<br>ACGCCAGGAGGAGGGATAATGGTTCATACTTCTTTCGGATGGAGAGAGGAAGTACCAAATACAGT<br>TACAAATCTCCCCAGCTCTCTGTGCATGTGACAGACTTGACCCACAGGCCCAAAATCCTCATCCC<br>TGGCACTCTAGAACCCGGCCACTCCAAAAACCTGACCTGCTCTGTGTCCTGGGCCTGTGAGCAGG<br>GAACACCCCCGATCTTCTCCTGGTTGTCAGCTGCCCCCACCTCCCTGGGCCCCAGGACTACTCAC<br>TCCTCGGTGCTCATAATCACCCCACGGCCCCAGGACCACGGCACCAACCTGACCTGTCAGGTGAA<br>GTTCGCTGGAGCTGGTGTGACTACGGAGAGAACCATCCAGCTCAACGTCACCTATGTTCCACAGA<br>ACCCAACAACTGGTATCTTTCCAGGAGATGGCTCAGGGAAACAAGAGACCAGAGCAGGAGTGGTT<br>CATGGGGCCATTGGAGGAGCTGGTGTTACAGCCCTGCTCGCTCTTTGTCTCTGCCTCATCTTCTT<br>CATAGTGAAGACCCACAGGAGGAAAGCAGCCAGGACAGCAGTGGGCAGGAATGACACCCACCCTA<br>CCACAGGGTCAGCCTCCCCGAAACACCAGAAGAAGTCCAAGTTACATGGCCCCACTGAAACCTCA<br>AGCTGTTCAGGTGCCGCCCCTACTGTGGAGATGGATGAGGAGCTGCATTATGCTTCCCTCAACTT<br>TCATGGGATGAATCCTTCCAAGGACACCTCCACCGAATACTCAGAGGTCAGGACCCAGTCCGGGC<br>ATCATCACCATCATCATTGA |
| 306. | CD33 | human | aa | MGWSCIILFLVATATGVHSDPNFWLQVQESVTVQEGLCVLVPCTFFHPIPYYDKNSPVHGYWFRE<br>GAIISGDSPVATNKLDQEVQEETQGRFRLLGDPSRNNCSLSIVDARRRDNGSYFFRMERGSTKYS<br>YKSPQLSVHVTDLTHRPKILIPGTLEPGHSKNLTCSVSWACEQGTPPIFSWLSAAPTSLGPRTTH<br>SSVLIITPRPQDHGTNLTCQVKFAGAGVTTERTIQLNVTYVPQNPTTGIFPGDGSGKQETRAGVV<br>HGAIGGAGVTALLALCLCLIFFIVKTHRRKAARTAVGRNDTHPTTGSASPKHQKKSKLHGPTETS<br>SCSGAAPTVEMDEELHYASLNFHGMNPSKDTSTEYSEVRTQSGHHHHHH |
| 307. | CD33 | macaque | nt | ATGCCGCTGCTGCTACTGCTGCCCCTGCTGTGGGCAGGGGCCCTGGCTATGGATCCAAGAGTCAG<br>GCTGGAAGTGCAGGAGTCAGTGACAGTACAGGAGGGTTTGTGCGTCCTTGTGCCCTGCACTTTCT<br>TCCATCCCGTACCCTACCACACCAGGAATTCCCCAGTTCATGGTTACTGGTTCCGGGAAGGAGCC<br>ATTGTATCCTTGGACTCTCCAGTGGCCACAAACAAGCTAGATCAAGAAGTACAGGAGGAGACCCA<br>GGGCCGATTCCGCCTCCTTGGGGATCCCAGTAGGAACAACTGCTCCCTGAGCATCGTAGATGCCA<br>GGAGGAGGGATAACGGTTCATACTTCTTTCGGATGGAGAAAGGAAGTACCAAATACAGTTACAAA<br>TCTACCCAGCTCTCTGTGCATGTGACAGACTTGACCCACAGGCCCCAAATCCTCATCCCTGGAGC<br>CCTAGACCCTGACCACTCCAAAAACCTGACCTGCTCTGTGCCCTGGGCCTGTGAGCAGGGAACAC<br>CTCCAATCTTCTCCTGGATGTCAGCTGCCCCCACCTCCCTGGGCCTCAGGACCACTCACTCCTCG<br>GTGCTCATAATCACCCCACGGCCCCAGGACCACGGCACCAACCTCACCTGTCAGGTGAAGTTCCC |

| | | | | |
|---|---|---|---|---|
| | | | | TGGAGCTGGCGTGACCACGGAGAGAACCATCCAGCTCAATGTCTCCTATGCTTCACAGAACCCAAGAACTGATATCTTTCTAGGAGACGGCTCAGGGAAACAAGGAGTGGTTCAGGGAGCCATCGGGGGAGCTGGTGTCACAGTCCTGCTCGCTCTTTGTCTCTGCCTCATCTTCTTCACAGTGAAGACTCACAGGAGGAAAGCAGCCAGGACAGCAGTGGGCAGGATCGACACCCACCCCGCCACAGGGCCAACATCCTCGAAACACCAGAAGAAGTCCAAGTTACATGGCGCCACTGAAACCTCAGGCTGTTCAGGTACCACCCTTACTGTGGAGATGGATGAGGAGCTGCACTACGCTTCCCTCAACTTTCATGGGATGAATCCTTCTGAGGACACCTCCACCGAATACTCAGAGGTCAGGACCCAGTGA |
| 308. | CD33 | macaque | aa | MPLLLLLPLLWAGALAMDPRVRLEVQESVTVQEGLCVLVPCTFFHPVPYHTRNSPVHGYWFREGAIVSLDSPVATNKLDQEVQEETQGRFRLLGDPSRNNCSLSIVDARRRDNGSYFFRMEKGSTKYSYKSTQLSVHVTDLTHRPQILIPGALDPDHSKNLTCSVPWACEQGTPPIFSWMSAAPTSLGLRTTHSSVLIITPRPQDHGTNLTCQVKFPGAGVTTERTIQLNVSYASQNPRTDIFLGDGSGKQGVVQGAIGGAGVTVLLALCLCLIFFTVKTHRRKAARTAVGRIDTHPATGPTSSKHQKKSKLHGATETSGCSGTTLTVEMDEELHYASLNFHGMNPSEDTSTEYSEVRTQ |
| 309. | 1-27 CD3-Fc + Leader | artificial | nt | ATGGGATGGAGCTGTATCATCCTCTTCTTGGTAGCAACAGCTACAGGTGTACACTCCCAAGATGGTAATGAAGAAATGGGTGGTATTACACAGACACCATATAAAGTCTCCATCTCTGGAACCACAGTAATATTGACATCCGGAGAGCCCAAATCTTGTGACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATCCCGGGAGGAGATGACCAAGAACCAGGTCAGCCTGACCTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTATAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCCCCGGGTAAATAG |
| 310. | 1-27 CD3-Fc + Leader | artificial | aa | MGWSCIILFLVATATGVHSQDGNEEMGGITQTPYKVSISGTTVILTSGEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 311. | CD33 UD H2C HL x AF5 HL | artificial | aa | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVLSGGGGSQVQLVQSGAEVKKPGASVKVSCKASGYTFTNYGMNWVKQAPGQGLKWMGWINTYTGEPTYADDFKGRVTMTSDTSTSTAYLELHNLRSDDTAVYYCARWSWSDGYYVYFDYWGQGTTVTVSSGGGGSGGGGSGGG |

| | | | | |
|---|---|---|---|---|
| | | | | GSDIVMTQSPDSLTVSLGERTTINCKSSQSVLDSSKNKNSLAWYQQKPGQPPKLLLSWASTRESG<br>IPDRFSGSGSGTDFTLTIDSLQPEDSATYYCQQSAHFPITFGQGTRLEIK |
| 312. | CD33 UD H2C HL x AF5 HL | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATAAGTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACA<br>TATCCTACTGGGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTATCCGGAGGTGGTGGCTCCCAGGTGCAGCTGGTC<br>CAGTCTGGAGCTGAGGTGAAGAAGCCTGGAGCGTCAGTCAAGGTCTCCTGCAAGGCTAGCGGGTA<br>TACCTTCACAAACTATGGAATGAACTGGGTGAAGCAGGCTCCAGGACAGGGTTTAAAGTGGATGG<br>GCTGGATAAACACCTACACTGGAGAGCCAACATATGCTGATGACTTCAAGGGACGGGTTACCATG<br>ACTTCGGATACCTCTACCAGCACTGCCTATTTGGAACTCCACAACCTCAGAAGTGATGACACGGC<br>TGTATATTACTGTGCGCGCTGGAGTTGGAGTGATGGTTACTACGTTTACTTTGACTACTGGGGCC<br>AAGGCACTACGGTCACCGTCTCCTCAGGTGGTGGTGGTTCTGGCGGCGGCGGCTCCGGTGGTGGT<br>GGTTCTGACATCGTGATGACACAGTCTCCAGACTCCCTGACTGTGTCTCTGGGCGAGAGGACCAC<br>CATCAACTGCAAGTCCAGCCAGAGTGTTTTAGACAGCTCCAAGAATAAGAACTCCTTAGCTTGGT<br>ACCAGCAGAAACCAGGACAGCCTCCTAAATTACTCCTTTCCTGGGCATCTACGCGGGAATCCGGG<br>ATCCCTGACCGATTCAGTGGCAGCGGGTCTGGGACAGATTTCACTCTCACTATTGACAGCCTGCA<br>GCCTGAAGATTCTGCAACTTACTATTGTCAACAGTCTGCCCACTTCCCGATCACCTTTGGCCAAG<br>GGACACGACTGGAGATTAAA |
| 313. | CD33 UD F12Q HL x AF5 HL | artificial | aa | EVQLVESGGGLVQPGGSLKLSCAASGFTFNSYAMNWVRQAPGKGLEWVARIRSKYNNYATYYADS<br>VKGRFTISRDDSKNTAYLQMNNLKTEDTAVYYCVRHGNFGNSYVSWWAYWGQGTLVTVSSGGGGS<br>GGGGSGGGGSQTVVTQEPSLTVSPGGTVTLTCGSSTGAVTSGNYPNWVQQKPGQAPRGLIGGTKF<br>LAPGTPARFSGSLLGGKAALTLSGVQPEDEAEYYCVLWYSNRWVFGGGTKLTVLSGGGGSQVQLV<br>QSGAEVKKPGASVKVSCKASGYTFTNYGMNWVKQAPGQGLKWMGWINTYTGEPTYADDFKGRVTM<br>TSDTSTSTAYLELHNLRSDDTAVYYCARWSWSDGYYVYFDYWGQGTTVTVSSGGGGSGGGGSGGG<br>GSDIVMTQSPDSLTVSLGERTTINCKSSQSVLDSSKNKNSLAWYQQKPGQPPKLLLSWASTRESG<br>IPDRFSGSGSGTDFTLTIDSLQPEDSATYYCQQSAHFPITFGQGTRLEIK |
| 314. | CD33 UD F12Q HL x AF5 HL | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATAGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA |

| | | | | |
|---|---|---|---|---|
| | | | | GTGAAAGGCAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTTTCCTGGTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTATCCGGAGGTGGTGGCTCCCAGGTGCAGCTGGTCCAGTCTGGAGCTGAGGTGAAGAAGCCTGGAGCGTCAGTCAAGGTCTCCTGCAAGGCTAGCGGGTATACCTTCACAAACTATGGAATGAACTGGGTGAAGCAGGCTCCAGGACAGGGTTTAAAGTGGATGGGCTGGATAAACACCTACACTGGAGAGCCAACATATGCTGATGACTTCAAGGGACGGGTTACCATGACTTCGGATACCTCTACCAGCACTGCCTATTTGGAACTCCACAACCTCAGAAGTGATGACACGGCTGTATATTACTGTGCGCGCTGGAGTTGGAGTGATGGTTACTACGTTTACTTTGACTACTGGGGCCAAGGCACTACGGTCACCGTCTCCTCAGGTGGTGGTGGTTCTGGCGGCGGCGGCTCCGGTGGTGGTGGTTCTGACATCGTGATGACACAGTCTCCAGACTCCCTGACTGTGTCTCTGGGCGAGAGGACCACCATCAACTGCAAGTCCAGCCAGAGTGTTTTAGACAGCTCCAAGAATAAGAACTCCTTAGCTTGGTACCAGCAGAAACCAGGACAGCCTCCTAAATTACTCCTTTCCTGGGCATCTACGCGGGAATCCGGGATCCCTGACCGATTCAGTGGCAGCGGGTCTGGGACAGATTTCACTCTCACTATTGACAGCCTGCAGCCTGAAGATTCTGCAACTTACTATTGTCAACAGTCTGCCCACTTCCCGATCACCTTTGGCCAAGGGACACGACTGGAGATTAAA |
| 315. | CD33 UD I2C HL x AF5 HL | artificial | aa | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLSGGGGSQVQLVQSGAEVKKPGASVKVSCKASGYTFTNYGMNWVKQAPGQGLKWMGWINTYTGEPTYADDFKGRVTMTSDTSTSTAYLELHNLRSDDTAVYYCARWSWSDGYYVYFDYWGQGTTVTVSSGGGGSGGGGSGGGSDIVMTQSPDSLTVSLGERTTINCKSSQSVLDSSKNKNSLAWYQQKPGQPPKLLLSWASTRESGIPDRFSGSGSGTDFTLTIDSLQPEDSATYYCQQSAHFPITFGQGTRLEIK |
| 316. | CD33 UD I2C HL x AF5 HL | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACT |

| | | | | |
|---|---|---|---|---|
| | | | | ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGTTCTATGGTACAGCAACCGCTGGG<br>TGTTCGGTGGAGGAACCAAACTGACTGTCCTATCCGGAGGTGGTGGCTCCCAGGTGCAGCTGGTC<br>CAGTCTGGAGCTGAGGTGAAGAAGCCTGGAGCGTCAGTCAAGGTCTCCTGCAAGGCTAGCGGGTA<br>TACCTTCACAAACTATGGAATGAACTGGGTGAAGCAGGCTCCAGGACAGGGTTTAAAGTGGATGG<br>GCTGGATAAACACCTACACTGGAGAGCCAACATATGCTGATGACTTCAAGGGACGGGTTACCATG<br>ACTTCGGATACCTCTACCAGCACTGCCTATTTGGAACTCCACAACCTCAGAAGTGATGACACGGC<br>TGTATATTACTGTGCGCGCTGGAGTTGGAGTGATGGTTACTACGTTTACTTTGACTACTGGGGCC<br>AAGGCACTACGGTCACCGTCTCCTCAGGTGGTGGTGGTTCTGGCGGCGGCGGCTCCGGTGGTGGT<br>GGTTCTGACATCGTGATGACACAGTCTCCAGACTCCCTGACTGTGTCTCTGGGCGAGAGGACCAC<br>CATCAACTGCAAGTCCAGCCAGAGTGTTTTAGACAGCTCCAAGAATAAGAACTCCTTAGCTTGGT<br>ACCAGCAGAAACCAGGACAGCCTCCTAAATTACTCCTTTCCTGGGCATCTACGCGGGAATCCGGG<br>ATCCCTGACCGATTCAGTGGCAGCGGGTCTGGGACAGATTTCACTCTCACTATTGACAGCCTGCA<br>GCCTGAAGATTCTGCAACTTACTATTGTCAACAGTCTGCCCACTTCCCGATCACCTTTGGCCAAG<br>GGACACGACTGGAGATTAAA |
| 317. | MCSP-A9 HL x H2C HL | artificial | aa | QVQLVQSGAEVKKPGASVKVSCKASGYPFTGYYMHWVRQAPGQGLEWMGWINPNSGGTNYAQKFQ<br>GRVTITADESTSTAYMELSRLRSDDTAVYYCAKSWVSWFASWGQGTLVTVSSGGGGSGGGGSGGG<br>GSDIVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHFNTPFAFGQGTKLEIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 318. | MCSP-A9 HL x H2C HL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACCCCTTCACCGGCTACTACATGCACTGGGTGCGACAGGCCCCTGGACAAG<br>GGCTTGAGTGGATGGGATGGATCAACCCTAACAGTGGTGGCACAAACTATGCACAGAAGTTTCAG<br>GGCAGAGTCACGATTACCGCGGACGAATCCACGAGCACAGCCTACATGGAGCTGAGCAGGCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT<br>ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCGGTTTATTACTGTCAACAACATTTTAATACTCCGTTCGCTTTTGGCCAGG<br>GGACCAAGCTGGAGATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA |

| | | | | |
|---|---|---|---|---|
| | | | | GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 319. | MCSP-A9 HL x F12Q HL | artificial | aa | QVQLVQSGAEVKKPGASVKVSCKASGYPFTGYYMHWVRQAPGQGLEWMGWINPNSGGTNYAQKFQ<br>GRVTITADESTSTAYMELSRLRSDDTAVYYCAKSWVSWFASWGQGTLVTVSSGGGGSGGGGSGGG<br>GSDIVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHFNTPFAFGQGTKLEIKSGGGGSEVQLVESGG<br>GLVQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYVSWWAYWGQGTLVTVSSGGGGSGGGGSGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 320. | MCSP-A9 HL x F12Q HL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACCCCTTCACCGGCTACTACATGCACTGGGTGCGACAGGCCCCTGGACAAG<br>GGCTTGAGTGGATGGGATGGATCAACCCTAACAGTGGTGGCACAAACTATGCACAGAAGTTTCAG<br>GGCAGAGTCACGATTACCGCGGACGAATCCACGAGCACAGCCTACATGGAGCTGAGCAGGCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT<br>ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCGGTTTATTACTGTCAACAACATTTTAATACTCCGTTCGCTTTTGGCCAGG<br>GGACCAAGCTGGAGATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTTTCCTGGTGGGCTTACTGGGGCCAAG<br>GGACTCTGGTCACCGTCTCCTCAGGTGGTGGTGGTTCTGGCGGCGGCGGCTCCGGTGGTGGTGGT |

| | | | | |
|---|---|---|---|---|
| | | | | TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG TCCTA |
| 321. | MCSP-A9 HL x I2C HL | artificial | aa | QVQLVQSGAEVKKPGASVKVSCKASGYPFTGYYMHWVRQAPGQGLEWMGWINPNSGGTNYAQKFQ GRVTITADESTSTAYMELSRLRSDDTAVYYCAKSWVSWFASWGQGTLVTVSSGGGGSGGGGSGGG GSDIVMTQSPDSLAVSLGERATINCKSSQSVLSSSNNKNYLNWYQQKPGQPPKLLIYWASTRESG VPDRFSGSGSGTDFTLTISSLQAEDVAVYYCQQHFNTPFAFGQGTKLEIKSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 322. | MCSP-A9 HL x I2C HL | artificial | nt | CAGGTGCAGCTGGTCCAGTCTGGGGCTGAGGTGAAGAAGCCTGGGGCCTCAGTGAAGGTCTCCTG CAAGGCTTCTGGATACCCCTTCACCGGCTACTACATGCACTGGGTGCGACAGGCCCCTGGACAAG GGCTTGAGTGGATGGGATGGATCAACCCTAACAGTGGTGGCACAAACTATGCACAGAAGTTTCAG GGCAGAGTCACGATTACCGCGGACGAATCCACGAGCACAGCCTACATGGAGCTGAGCAGGCTGAG ATCTGACGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGACATCGTGATGACACAGTCTCCAGACTCCCTGGCTGTGTCTCTGGGCGAGAGGGCCAC CATCAACTGCAAGTCCAGCCAGAGTGTCTTATCCAGCTCCAACAATAAGAACTACTTAAATTGGT ACCAGCAGAAACCAGGACAGCCTCCTAAGTTGCTCATTTACTGGGCATCTACCCGGGAATCCGGG GTCCCTGACCGATTCAGTGGCAGCGGGTCTGGGACAGATTTCACTCTCACCATCAGCAGCCTGCA GGCTGAAGATGTGGCGGTTTATTACTGTCAACAACATTTTAATACTCCGTTCGCTTTTGGCCAGG GGACCAAGCTGGAGATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGACAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG |

| | | | | |
|---|---|---|---|---|
| | | | | TCCTA |
| 323. | MCSP-C8 HL x I2C HL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLAVSLGERATINCKSSQSVLNSKNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 324. | MCSP-C8 HL x I2C HL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACCCAGTCTCCAGACTCCCTGGCTGTGTCTCTGGGCGAGAGGGCCAC<br>CATCAACTGCAAGTCCAGCCAGAGTGTTTTAAACAGCAAGAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 325. | MCSP-B8 HL x I2C HL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLTVSPGERATINCKSSQSVLNSKNNRNYLAWYQQKPGQPPKLLIYWASTRESG<br>VPDRFSGSGSGTDFTLTIDSLQPEDSATYYCQQHYSTPFTFGQGTRLEIKSGGGGSEVQLVESGG |

| | | | | |
|---|---|---|---|---|
| | | | | GLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 326. | MCSP-B8 HL x I2C HL | artificial | nt | CAGGTGCAGCTGGTCCAGTCTGGGGCTGAGGTGAAGAGGCCTGGGGCCTCAATGAAGGTCTCCTGCAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGTGGTTCTGACATCGTGATGACCCAGTCTCCAGACTCCCTGACTGTGTCTCCGGGCGAGAGGGCCACCATCAACTGCAAGTCCAGCCAGAGTGTTTTAAACAGCAAGAACAATAGGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCGATTCCCTGCAGCCTGAAGATAGTGCAACTTATTACTGTCAGCAACATTATAGTACTCCATTCACTTTTGGCCAGGGGACCAGACTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 327. | MCSP-B7 HL x I2C HL | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGGGGSDIVMTQSPDSLTVSLGERTTINCKSSQSVLNSKNNRNYLAWYQQKPGQPPKLLIYWASTRESGVPDRFSGSGSGTDFTLTIDSLQPEDIATYYCQQHYSTPFTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 328. | MCSP-B7 HL x I2C | artificial | nt | CAGGTGCAGCTGGTCCAGTCTGGGGCTGAGGTGAAGAGGCCTGGGGCCTCAATGAAGGTCTCCTG |

| | | | | |
|---|---|---|---|---|
| | HL | | | CAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGTGGTTCTGACATCGTGATGACCCAGTCTCCAGACTCCCTGACTGTGTCTCTGGGCGAGAGGACCACCATCAACTGCAAGTCCAGCCAGAGTGTTTTAAACAGCAAGAACAATAGGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCGATTCCCTGCAGCCTGAAGATATTGCAACTTATTACTGTCAGCAACATTATAGTACTCCATTCACTTTTGGCCAGGGGACCAGACTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 329. | MCSP-G8 HL x I2C HL | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGGGGSDIVMTQSPDSLTVSLGERATINCKSSQSVLNSKNNRNYLAWYQQKPGQPPKLLIYWASTRESGVPDRFSGSGSGTDFTLTIDSLQPEDSATYYCQQHYSTPFTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 330. | MCSP-G8 HL x I2C HL | artificial | nt | CAGGTGCAGCTGGTCCAGTCTGGGGCTGAGGTGAAGAGGCCTGGGGCCTCAATGAAGGTCTCCTGCAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGT |

| | | | | |
|---|---|---|---|---|
| | | | | GGTTCTGACATCGTGATGACCCAGTCTCCAGACTCCCTGACTGTGTCTCTGGGCGAGAGGGCCAC<br>CATCAACTGCAAGTCCAGCCAGAGTGTTTTAAACAGCAAGAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCGATTCCCTGCA<br>GCCTGAAGATAGTGCAACTTATTACTGTCAGCAACATTATAGTACTCCATTCACTTTTGGCCAGG<br>GGACCAGACTGGAGATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 331. | MCSP-D5 HL x I2C HL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLAVSLGERATINCKSSQSVLNSKNNRNYLAWYQQKPGQPPKLLIYWASTRESG<br>VPDRFSGSGSGTDFTLTIDSLQAEDSATYYCQQHYSTPFTFGQGTRLEIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 332. | MCSP-D5 HL x I2C HL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACCCAGTCTCCAGACTCCCTGGCTGTGTCTCTGGGCGAGAGGGCCAC<br>CATCAACTGCAAGTCCAGCCAGAGTGTTTTAAACAGCAAGAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCGATTCCCTGCA<br>GGCTGAAGATAGTGCAACTTATTACTGTCAGCAACATTATAGTACTCCATTCACTTTTGGCCAGG |

| | | | | |
|---|---|---|---|---|
| | | | | GGACCAGACTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 333. | MCSP-F7 HL x I2C HL | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGGGGSDIVMTQSPDSLAVSLGERATINCKSSQSVLNSKNNRNYLAWYQQKPGQPPKLLIYWASTRESGVPDRFSGSGSGTDFTLTIDVLQPEDIATYYCQQHYSTPFTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 334. | MCSP-F7 HL x I2C HL | artificial | nt | CAGGTGCAGCTGGTCCAGTCTGGGGCTGAGGTGAAGAGGCCTGGGGCCTCAATGAAGGTCTCCTGCAAGGCTTCTGGGTACACCTTCACCAACTACTATATACACTGGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTCCTGGTTTGCTTCCTGGGGTCAAGGAACCTTGGTCACCGTCTCCTCAGGTGGTGGTGGTTCTGGCGGCGGCGGCTCCGGTGGTGGTGGTTCTGACATCGTGATGACCCAGTCTCCAGACTCCCTGGCTGTGTCTCTGGGCGAGAGGGCCACCATCAACTGCAAGTCCAGCCAGAGTGTTTTAAACAGCAAGAACAATAGGAACTACTTAGCTTGGTACCAGCAGAAACCAGGACAGCCTCCTAAGCTGCTCATTTACTGGGCATCTACCCGGGAATCCGGGGTCCCTGACCGATTCAGTGGCAGCGGGTCTGGGACAGATTTCACTCTCACCATCGATGTCCTGCAGCCTGAAGATATTGCAACTTATTACTGTCAGCAACATTATAGTACTCCATTCACTTTTGGCCAGGGGACCAGACTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTA |

| | | | | |
|---|---|---|---|---|
| | | | | CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG TCCTA |
| 335. | MCSP-G5 HL x I2C HL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSDIVMTQSPDSLAVSLGDRATINCKSSQSVLNSKNNRNYLAWYQQKPGQPPKLLIYWASTRESG VPDRFSGSGSGTDFTLTIDSLQPEDSATYYCQQHYSTPFTFGQGTRLEIKSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 336. | MCSP-G5 HL x I2C HL | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGACATCGTGATGACCCAGTCTCCAGACTCCCTGGCTGTGTCTCTGGGCGACAGGGCCAC CATCAACTGCAAGTCCAGCCAGAGTGTTTTAAACAGCAAGAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCGATTCCCTGCA GCCTGAGGATAGTGCAACTTATTACTGTCAGCAACATTATAGTACTCCATTCACTTTTGGCCAGG GGACCAGACTGGAGATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGACAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC |

| | | | | |
|---|---|---|---|---|
| | | | | TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 337. | MCSP-F8 HL x I2C HL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG<br>GSDIVMTQSPDSLTVSLGERATINCKSSQSVLNSKNNRNYLAWYQQKPGQPPKLLIYWASTRESG<br>VPDRFSGSGSGTDFTLTIDSLQAEDSAIYYCQQHYSTPFTFGQGTRLEIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 338. | MCSP-F8 HL x I2C HL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACCCAGTCTCCAGACTCCCTGACTGTGTCTCTGGGCGAGAGGGCCAC<br>CATCAACTGCAAGTCCAGCCAGAGTGTTTTAAACAGCAAGAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCGATTCCCTGCA<br>GGCTGAAGATAGTGCAATTTATTACTGTCAGCAACATTATAGTACTCCATTCACTTTTGGCCAGG<br>GGACCAGACTGGAGATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 339. | MCSP-G10 HL x I2C HL | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGG |

| | | | | |
|---|---|---|---|---|
| | | | | GSDIVMTQSPDSLAVSLGERATINCKSSQSVLSSSNNKNYLNWYQQKPGQPPKLLIYWASTRESG<br>VPDRFSGSGSGTDFTLTISSLQAEDVAVYYCQQHFNTPFAFGQGTKLEIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 340. | MCSP-G10 HL x I2C HL | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGACATCGTGATGACACAGTCTCCAGACTCCCTGGCTGTGTCTCTGGGCGAGAGGGCCAC<br>CATCAACTGCAAGTCCAGCCAGAGTGTCTTATCCAGCTCCAACAATAAGAACTACTTAAATTGGT<br>ACCAGCAGAAACCAGGACAGCCTCCTAAGTTGCTCATTTACTGGGCATCTACCCGGGAATCCGGG<br>GTCCCTGACCGATTCAGTGGCAGCGGGTCTGGGACAGATTTCACTCTCACCATCAGCAGCCTGCA<br>GGCTGAAGATGTGGCGGTTTATTACTGTCAACAACATTTTAATACTCCGTTCGCTTTTGGCCAGG<br>GGACCAAGCTGGAGATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 341. | Human CD3 epsilon 1-8 (N-terminus) | human | aa | QDGNEEMG |
| 342. | *Saimiri sciureus* CD3 epsilon 1-8 (N-terminus) | *Saimiri sciureus* | aa | QDGNEEIG |
| 343. | Thioate-modified CpG-Oligonucleotide | artificial | nt | TCCATGACGTTCCTGATGCT |

| | | | | |
|---|---|---|---|---|
| 344. | MVH1 | artificial | nt | (GC)AGGTGCAGCTCGAGGAGTCAGGACCT |
| 345. | MVH2 | artificial | nt | GAGGTCCAGCTCGAGCAGTCTGGACCT |
| 346. | MVH3 | artificial | nt | CAGGTCCAACTCGAGCAGCCTGGGGCT |
| 347. | MVH4 | artificial | nt | GAGGTTCAGCTCGAGCAGTCTGGGGCA |
| 348. | MVH5 | artificial | nt | GA(AG)GTGAAGCTCGAGGAGTCTGGAGGA |
| 349. | MVH6 | artificial | nt | GAGGTGAAGCTTCTCGAGTCTGGAGGT |
| 350. | MVH7 | artificial | nt | GAAGTGAAGCTCGAGGAGTCTGGGGGA |
| 351. | MVH8 | artificial | nt | GAGGTTCAGCTCGAGCAGTCTGGAGCT |
| 352. | MuVHBstEII | artificial | nt | TGAGGAGACGGTGACCGTGGTCCCTTGGCCCCAG |
| 353. | MUVK1 | artificial | nt | CCAGTTCCGAGCTCGTTGTGACTCAGGAATCT |
| 354. | MUVK2 | artificial | nt | CCAGTTCCGAGCTCGTGTTGACGCAGCCGCCC |
| 355. | MUVK3 | artificial | nt | CCAGTTCCGAGCTCGTGCTCACCCAGTCTCCA |
| 356. | MUVK4 | artificial | nt | CCAGTTCCGAGCTCCAGATGACCCAGTCTCCA |
| 357. | MUVK5 | artificial | nt | CCAGATGTGAGCTCGTGATGACCCAGACTCCA |
| 358. | MUVK6 | artificial | nt | CCAGATGTGAGCTCGTCATGACCCAGTCTCCA |
| 359. | MUVK7 | artificial | nt | CCAGTTCCGAGCTCGTGATGACACAGTCTCCA |
| 360. | MuVkHindIII/BsiW1 | artificial | nt | TGGTGCACTAGTCGTACGTTTGATCTCAAGCTTGGTCCC |
| 361. | forward primer | artificial | nt | GATCTGGTCTACACCATCGAGC |
| 362. | reverse primer | artificial | nt | GGAGCTGCTGCTGGCTCAGTGAGG |
| 363. | forward primer | artificial | nt | TTCCAGCTGAGCATGTCTGATGG |
| 364. | reverse primer | artificial | nt | CGATCAGCATCTGGGCCCAGG |
| 365. | forward primer | artificial | nt | GTGGAGCAGTTCACTCAGCAGGACC |
| 366. | reverse primer | artificial | nt | GCCTTCACACCCAGTACTGGCC |
| 367. | forward primer | artificial | nt | TCCCGTACGAGATCTGGATCCCAATTGGATGGCGGACTCGTGCTGTTCTCACACAGAGG |
| 368. | reverse primer | artificial | nt | AGTGGGTCGACTCACACCCAGTACTGGCCATTCTTAAGGGCAGGG |
| 369. | forward primer | artificial | nt | GAGGAATTCACCATGCCGCTGCTGCTACTGCTGCCCCTGCTGTGGGCAGGGGCCCTGGCTATGG |
| 370. | reverse primer | artificial | nt | GATTTGTAACTGTATTTGGTACTTCC |
| 371. | forward primer | artificial | nt | ATTCCGCCTCCTTGGGGATCC |
| 372. | reverse primer | artificial | nt | GCATAGGAGACATTGAGCTGGATGG |
| 373. | forward primer | artificial | nt | GCACCAACCTGACCTGTCAGG |
| 374. | reverse primer | artificial | nt | AGTGGGTCGACTCACTGGGTCCTGACCTCTGAGTATTCG |
| 375. | PSCA-1 VL | artificial | aa | DIQLTQSPSSLSASVGDRVTITCSASSSVRFIHWYQQKPGKAPKRLIYDTSKLASGVPSRFSGSG SGTDFTLTISSLQPEDFATYYCQQWSSSPFTFGQGTKVEIK |
| 376. | PSCA-1 VL | artificial | nt | GACATCCAGCTGACCCAGAGCCCATCCAGCCTGTCTGCTAGCGTGGGCGACAGAGTGACCATCAC CTGCAGCGCCAGCAGCAGCGTGCGGTTCATCCACTGGTATCAGCAGAAGCCCGGCAAGGCCCCCA |

| | | | | |
|---|---|---|---|---|
| | | | | AGAGGCTGATCTACGACACCAGCAAGCTGGCTAGCGGCGTGCCAAGCAGATTCAGCGGCAGCGGC TCCGGCACCGACTTCACCCTGACCATCAGCAGCCTGCAGCCAGAGGACTTCGCTACCTATTACTG CCAGCAGTGGAGCAGCAGCCCCTTCACCTTCGGCCAGGGCACCAAGGTGGAGATCAAG |
| 377. | PSCA-1 LCDR1 | artificial | aa | SASSSVRFIH |
| 378. | PSCA-1 LCDR2 | artificial | aa | DTSKLAS |
| 379. | PSCA-1 LCDR3 | artificial | aa | QQWSSSPFT |
| 380. | PSCA-1 H | artificial | aa | EVQLVESGGGLVQPGGSLRLSCAASGFNIKDYYIHWVRQAPGKGLEWVAWIDPENGDTEFVPKFQ GRATISADTSKNTAYLQMNSLRAEDTAVYYCKTGGFWGQGTLVTVSS |
| 381. | PSCA-1 H | artificial | nt | GAGGTGCAGCTGGTCGAGTCTGGCGGAGGACTGGTGCAGCCAGGCGGCTCCCTGAGACTGAGCTG CGCCGCCAGCGGCTTCAACATCAAGGACTACTACATCCACTGGGTCCGGCAGGCTCCAGGCAAGG GACTGGAATGGGTGGCCTGGATCGACCCAGAGAACGGCGACACCGAGTTCGTGCCCAAGTTCCAG GGCAGAGCCACCATCAGCGCCGACACCTCCAAGAACACCGCCTACCTGCAGATGAACAGCCTGAG GGCCGAGGACACCGCCGTGTACTACTGCAAGACCGGCGGCTTCTGGGGACAGGGCACCCTGGTCA CCGTGTCCTCC |
| 382. | PSCA-1 HCDR1 | artificial | aa | DYYIH |
| 383. | PSCA-1 HCDR2 | artificial | aa | WIDPENGDTEFVPKFQG |
| 384. | PSCA-1 HCDR3 | artificial | aa | GGF |
| 385. | PSCA-1 HL | artificial | aa | EVQLVESGGGLVQPGGSLRLSCAASGFNIKDYYIHWVRQAPGKGLEWVAWIDPENGDTEFVPKFQ GRATISADTSKNTAYLQMNSLRAEDTAVYYCKTGGFWGQGTLVTVSSGGGGSGGGGSGGGGSDIQ LTQSPSSLSASVGDRVTITCSASSSVRFIHWYQQKPGKAPKRLIYDTSKLASGVPSRFSGSGSGT DFTLTISSLQPEDFATYYCQQWSSSPFTFGQGTKVEIK |
| 386. | PSCA-1 HL | artificial | nt | GAGGTGCAGCTGGTCGAGTCTGGCGGAGGACTGGTGCAGCCAGGCGGCTCCCTGAGACTGAGCTG CGCCGCCAGCGGCTTCAACATCAAGGACTACTACATCCACTGGGTCCGGCAGGCTCCAGGCAAGG GACTGGAATGGGTGGCCTGGATCGACCCAGAGAACGGCGACACCGAGTTCGTGCCCAAGTTCCAG GGCAGAGCCACCATCAGCGCCGACACCTCCAAGAACACCGCCTACCTGCAGATGAACAGCCTGAG GGCCGAGGACACCGCCGTGTACTACTGCAAGACCGGCGGCTTCTGGGGACAGGGCACCCTGGTCA CCGTGTCCTCCGGTGGTGGTGGTTCTGGCGGCGGCGGCTCCGGTGGTGGTGGTTCTGACATCCAG CTGACCCAGAGCCCATCCAGCCTGTCTGCTAGCGTGGGCGACAGAGTGACCATCACCTGCAGCGC CAGCAGCAGCGTGCGGTTCATCCACTGGTATCAGCAGAAGCCCGGCAAGGCCCCCAAGAGGCTGA TCTACGACACCAGCAAGCTGGCTAGCGGCGTGCCAAGCAGATTCAGCGGCAGCGGCTCCGGCACC GACTTCACCCTGACCATCAGCAGCCTGCAGCCAGAGGACTTCGCTACCTATTACTGCCAGCAGTG GAGCAGCAGCCCCTTCACCTTCGGCCAGGGCACCAAGGTGGAGATCAAG |
| 387. | PSCA-1 LH | artificial | aa | DIQLTQSPSSLSASVGDRVTITCSASSSVRFIHWYQQKPGKAPKRLIYDTSKLASGVPSRFSGSG SGTDFTLTISSLQPEDFATYYCQQWSSSPFTFGQGTKVEIKGGGGSGGGGSGGGGSEVQLVESGG GLVQPGGSLRLSCAASGFNIKDYYIHWVRQAPGKGLEWVAWIDPENGDTEFVPKFQGRATISADT SKNTAYLQMNSLRAEDTAVYYCKTGGFWGQGTLVTVSS |

| | | | | |
|---|---|---|---|---|
| 388. | PSCA-1 LH | artificial | nt | GACATCCAGCTGACCCAGAGCCCATCCAGCCTGTCTGCTAGCGTGGGCGACAGAGTGACCATCAC<br>CTGCAGCGCCAGCAGCAGCGTGCGGTTCATCCACTGGTATCAGCAGAAGCCCGGCAAGGCCCCCA<br>AGAGGCTGATCTACGACACCAGCAAGCTGGCTAGCGGCGTGCCAAGCAGATTCAGCGGCAGCGGC<br>TCCGGCACCGACTTCACCCTGACCATCAGCAGCCTGCAGCCAGAGGACTTCGCTACCTATTACTG<br>CCAGCAGTGGAGCAGCAGCCCCTTCACCTTCGGCCAGGGCACCAAGGTGGAGATCAAGGGTGGTG<br>GTGGTTCTGGCGGCGGCGGCTCCGGTGGTGGTGGTTCTGAGGTGCAGCTGGTCGAGTCTGGCGGA<br>GGACTGGTGCAGCCAGGCGGCTCCCTGAGACTGAGCTGCGCCGCCAGCGGCTTCAACATCAAGGA<br>CTACTACATCCACTGGGTCCGGCAGGCTCCAGGCAAGGGACTGGAATGGGTGGCCTGGATCGACC<br>CAGAGAACGGCGACACCGAGTTCGTGCCCAAGTTCCAGGGCAGAGCCACCATCAGCGCCGACACC<br>TCCAAGAACACCGCCTACCTGCAGATGAACAGCCTGAGGGCCGAGGACACCGCCGTGTACTACTG<br>CAAGACCGGCGGCTTCTGGGGACAGGGCACCCTGGTCACCGTGTCCTCC |
| 389. | PSCA-1 HL x I2C HL | artificial | aa | EVQLVESGGGLVQPGGSLRLSCAASGFNIKDYYIHWVRQAPGKGLEWVAWIDPENGDTEFVPKFQ<br>GRATISADTSKNTAYLQMNSLRAEDTAVYYCKTGGFWGQGTLVTVSSGGGGSGGGGSGGGGSDIQ<br>LTQSPSSLSASVGDRVTITCSASSSVRFIHWYQQKPGKAPKRLIYDTSKLASGVPSRFSGSGSGT<br>DFTLTISSLQPEDFATYYCQQWSSSPFTFGQGTKVEIKSGGGGSEVQLVESGGGLVQPGGSLKLS<br>CAASGFTFNKYAMNWVRQAPGKGLEWVARIRSKYNNYATYYADSVKDRFTISRDDSKNTAYLQMN<br>NLKTEDTAVYYCVRHGNFGNSYISYWAYWGQGTLVTVSSGGGGSGGGGSGGGGSQTVVTQEPSLT<br>VSPGGTVTLTCGSSTGAVTSGNYPNWVQQKPGQAPRGLIGGTKFLAPGTPARFSGSLLGGKAALT<br>LSGVQPEDEAEYYCVLWYSNRWVFGGGTKLTVL |
| 390. | PSCA-1 HL x I2C HL | artificial | nt | GAGGTGCAGCTGGTCGAGTCTGGCGGAGGACTGGTGCAGCCAGGCGGCTCCCTGAGACTGAGCTG<br>CGCCGCCAGCGGCTTCAACATCAAGGACTACTACATCCACTGGGTCCGGCAGGCTCCAGGCAAGG<br>GACTGGAATGGGTGGCCTGGATCGACCCAGAGAACGGCGACACCGAGTTCGTGCCCAAGTTCCAG<br>GGCAGAGCCACCATCAGCGCCGACACCTCCAAGAACACCGCCTACCTGCAGATGAACAGCCTGAG<br>GGCCGAGGACACCGCCGTGTACTACTGCAAGACCGGCGGCTTCTGGGGACAGGGCACCCTGGTCA<br>CCGTGTCCTCCGGTGGTGGTGGTTCTGGCGGCGGCGGCTCCGGTGGTGGTGGTTCTGACATCCAG<br>CTGACCCAGAGCCCATCCAGCCTGTCTGCTAGCGTGGGCGACAGAGTGACCATCACCTGCAGCGC<br>CAGCAGCAGCGTGCGGTTCATCCACTGGTATCAGCAGAAGCCCGGCAAGGCCCCCAAGAGGCTGA<br>TCTACGACACCAGCAAGCTGGCTAGCGGCGTGCCAAGCAGATTCAGCGGCAGCGGCTCCGGCACC<br>GACTTCACCCTGACCATCAGCAGCCTGCAGCCAGAGGACTTCGCTACCTATTACTGCCAGCAGTG<br>GAGCAGCAGCCCCTTCACCTTCGGCCAGGGCACCAAGGTGGAGATCAAGTCCGGAGGTGGTGGAT<br>CCGAGGTGCAGCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGCTCCAGGAAA<br>GGGTTTGGAATGGGTTGCTCGCATAAGAAGTAAATATAATAATTATGCAACATATTATGCCGATT<br>CAGTGAAAGACAGGTTCACCATCTCCAGAGATGATTCAAAAAACACTGCCTATCTACAAATGAAC<br>AACTTGAAAACTGAGGACACTGCCGTGTACTACTGTGTGAGACATGGGAACTTCGGTAATAGCTA<br>CATATCCTACTGGGCTTACTGGGGCCAAGGGACTCTGGTCACCGTCTCCTCAGGTGGTGGTGGTT<br>CTGGCGGCGGCGGCTCCGGTGGTGGTGGTTCTCAGACTGTTGTGACTCAGGAACCTTCACTCACC |

| | | | | |
|---|---|---|---|---|
| | | | | GTATCACCTGGTGGAACAGTCACACTCACTTGTGGCTCCTCGACTGGGGCTGTTACATCTGGCAA<br>CTACCCAAACTGGGTCCAACAAAAACCAGGTCAGGCACCCCGTGGTCTAATAGGTGGGACTAAGT<br>TCCTCGCCCCCGGTACTCCTGCCAGATTCTCAGGCTCCCTGCTTGGAGGCAAGGCTGCCCTCACC<br>CTCTCAGGGGTACAGCCAGAGGATGAGGCAGAATATTACTGTGTTCTATGGTACAGCAACCGCTG<br>GGTGTTCGGTGGAGGAACCAAACTGACTGTCCTA |
| 391. | PSCA-1 LH x I2C HL | artificial | aa | DIQLTQSPSSLSASVGDRVTITCSASSSVRFIHWYQQKPGKAPKRLIYDTSKLASGVPSRFSGSG<br>SGTDFTLTISSLQPEDFATYYCQQWSSSPFTFGQGTKVEIKGGGGSGGGGSGGGGSEVQLVESGG<br>GLVQPGGSLRLSCAASGFNIKDYYIHWVRQAPGKGLEWVAWIDPENGDTEFVPKFQGRATISADT<br>SKNTAYLQMNSLRAEDTAVYYCKTGGFWGQGTLVTVSSGGGGSEVQLVESGGGLVQPGGSLKLSC<br>AASGFTFNKYAMNWVRQAPGKGLEWVARIRSKYNNYATYYADSVKDRFTISRDDSKNTAYLQMNN<br>LKTEDTAVYYCVRHGNFGNSYISYWAYWGQGTLVTVSSGGGGSGGGGSGGGGSQTVVTQEPSLTV<br>SPGGTVTLTCGSSTGAVTSGNYPNWVQQKPGQAPRGLIGGTKFLAPGTPARFSGSLLGGKAALTL<br>SGVQPEDEAEYYCVLWYSNRWVFGGGTKLTVL |
| 392. | PSCA-1 LH x I2C HL | artificial | nt | GACATCCAGCTGACCCAGAGCCCATCCAGCCTGTCTGCTAGCGTGGGCGACAGAGTGACCATCAC<br>CTGCAGCGCCAGCAGCAGCGTGCGGTTCATCCACTGGTATCAGCAGAAGCCCGGCAAGGCCCCCA<br>AGAGGCTGATCTACGACACCAGCAAGCTGGCTAGCGGCGTGCCAAGCAGATTCAGCGGCAGCGGC<br>TCCGGCACCGACTTCACCCTGACCATCAGCAGCCTGCAGCCAGAGGACTTCGCTACCTATTACTG<br>CCAGCAGTGGAGCAGCAGCCCCTTCACCTTCGGCCAGGGCACCAAGGTGGAGATCAAGGGTGGTG<br>GTGGTTCTGGCGGCGGCGGCTCCGGTGGTGGTGGTTCTGAGGTGCAGCTGGTCGAGTCTGGCGGA<br>GGACTGGTGCAGCCAGGCGGCTCCCTGAGACTGAGCTGCGCCGCCAGCGGCTTCAACATCAAGGA<br>CTACTACATCCACTGGGTCCGGCAGGCTCCAGGCAAGGGACTGGAATGGGTGGCCTGGATCGACC<br>CAGAGAACGGCGACACCGAGTTCGTGCCCAAGTTCCAGGGCAGAGCCACCATCAGCGCCGACACC<br>TCCAAGAACACCGCCTACCTGCAGATGAACAGCCTGAGGGCCGAGGACACCGCCGTGTACTACTG<br>CAAGACCGGCGGCTTCTGGGGACAGGGCACCCTGGTCACCGTGTCCTCCGGAGGTGGTGGATCCG<br>AGGTGCAGCTGGTCGAGTCTGGAGGAGGATTGGTGCAGCCTGGAGGGTCATTGAAACTCTCATGT<br>GCAGCCTCTGGATTCACCTTCAATAAGTACGCCATGAACTGGGTCCGCCAGGCTCCAGGAAAGGG<br>TTTGGAATGGGTTGCTCGCATAAGAAGTAAATATAATAATTATGCAACATATTATGCCGATTCAG<br>TGAAAGACAGGTTCACCATCTCCAGAGATGATTCAAAAAACACTGCCTATCTACAAATGAACAAC<br>TTGAAAACTGAGGACACTGCCGTGTACTACTGTGTGAGACATGGGAACTTCGGTAATAGCTACAT<br>ATCCTACTGGGCTTACTGGGGCCAAGGGACTCTGGTCACCGTCTCCTCAGGTGGTGGTGGTTCTG<br>GCGGCGGCGGCTCCGGTGGTGGTGGTTCTCAGACTGTTGTGACTCAGGAACCTTCACTCACCGTA<br>TCACCTGGTGGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGGACTAAGTTCC<br>TCGCCCCCGGTACTCCTGCCAGATTCTCAGGCTCCCTGCTTGGAGGCAAGGCTGCCCTCACCCTC<br>TCAGGGGTACAGCCAGAGGATGAGGCAGAATATTACTGTGTTCTATGGTACAGCAACCGCTGGGT<br>GTTCGGTGGAGGAACCAAACTGACTGTCCTA |
| 393. | PSCA-2 VL | artificial | aa | DIVMTQAAPSVPVTPGESVSISCRSSKSLLHSNGNTYLYWFLQRPGQSPQLLIYRMSNLASGVPD |

| | | | | |
|---|---|---|---|---|
| | | | | RFSGSGSGTAFTLRISRVEAEDVGVYYCLQHLEYPYTFGGGTKLELK |
| 394. | PSCA-2 VL | artificial | nt | GACATCGTGATGACCCAGGCCGCTCCAAGCGTGCCAGTGACCCCAGGCGAGAGCGTGTCCATCAG<br>CTGCAGGTCCAGCAAGAGCCTGCTGCACAGCAACGGCAACACCTACCTGTACTGGTTTCTGCAGA<br>GGCCAGGCCAGAGCCCCCAGCTGCTGATCTACAGGATGAGCAACCTGGCTAGCGGCGTGCCAGAC<br>AGATTCAGCGGCAGCGGCTCTGGAACCGCCTTCACCCTGAGGATCAGCAGGGTGGAGGCCGAGGA<br>CGTGGGCGTGTACTACTGCCTGCAGCACCTGGAATACCCCTACACCTTCGGCGGAGGGACCAAGC<br>TGGAACTGAAG |
| 395. | PSCA-2 LCDR1 | artificial | aa | RSSKSLLHSNGNTYLY |
| 396. | PSCA-2 LCDR2 | artificial | aa | RMSNLAS |
| 397. | PSCA-2 LCDR3 | artificial | aa | LQHLEYPYT |
| 398. | PSCA-2 H | artificial | aa | QVQVQQPGAELVKPGAPVKLSCKASGYTFTNYWLNWVKQRPGRGLEWIGRIDPSDSEIHYDQKFK<br>DKATLTVDKSSSTAYIQLSSLTSEDSAVYYCALTGIYAMAYWGQGTSVTVSS |
| 399. | PSCA-2 H | artificial | nt | CAGGTGCAGGTGCAGCAGCCAGGCGCCGAACTGGTCAAGCCTGGCGCCCCAGTGAAGCTGTCCTG<br>CAAGGCCAGCGGCTACACCTTCACCAACTACTGGCTGAACTGGGTGAAACAGAGGCCCGGCAGGG<br>GCCTGGAATGGATCGGCAGGATCGACCCCAGCGACAGCGAGATCCACTACGACCAGAAGTTCAAG<br>GACAAGGCCACCCTGACCGTGGACAAGAGCAGCAGCACCGCCTACATCCAGCTGTCCAGCCTGAC<br>CAGCGAGGACAGCGCCGTGTACTATTGCGCCCTGACCGGCATCTACGCCATGGCCTACTGGGGCC<br>AGGGCACCAGCGTGACCGTGTCTTCC |
| 400. | PSCA-2 HCDR1 | artificial | aa | NYWLN |
| 401. | PSCA-2 HCDR2 | artificial | aa | RIDPSDSEIHYDQKFKD |
| 402. | PSCA-2 HCDR3 | artificial | aa | TGIYAMAY |
| 403. | PSCA-2 LH | artificial | aa | DIVMTQAAPSVPVTPGESVSISCRSSKSLLHSNGNTYLYWFLQRPGQSPQLLIYRMSNLASGVPD<br>RFSGSGSGTAFTLRISRVEAEDVGVYYCLQHLEYPYTFGGGTKLELKGGGGSGGGGSGGGGSQVQ<br>VQQPGAELVKPGAPVKLSCKASGYTFTNYWLNWVKQRPGRGLEWIGRIDPSDSEIHYDQKFKDKA<br>TLTVDKSSSTAYIQLSSLTSEDSAVYYCALTGIYAMAYWGQGTSVTVSS |
| 404. | PSCA-2 LH | artificial | nt | GACATCGTGATGACCCAGGCCGCTCCAAGCGTGCCAGTGACCCCAGGCGAGAGCGTGTCCATCAG<br>CTGCAGGTCCAGCAAGAGCCTGCTGCACAGCAACGGCAACACCTACCTGTACTGGTTTCTGCAGA<br>GGCCAGGCCAGAGCCCCCAGCTGCTGATCTACAGGATGAGCAACCTGGCTAGCGGCGTGCCAGAC<br>AGATTCAGCGGCAGCGGCTCTGGAACCGCCTTCACCCTGAGGATCAGCAGGGTGGAGGCCGAGGA<br>CGTGGGCGTGTACTACTGCCTGCAGCACCTGGAATACCCCTACACCTTCGGCGGAGGGACCAAGC<br>TGGAACTGAAGGGTGGTGGTGGTTCTGGCGGCGGCGGCTCCGGTGGTGGTGGTTCTCAGGTGCAG<br>GTGCAGCAGCCAGGCGCCGAACTGGTCAAGCCTGGCGCCCCAGTGAAGCTGTCCTGCAAGGCCAG<br>CGGCTACACCTTCACCAACTACTGGCTGAACTGGGTGAAACAGAGGCCCGGCAGGGGCCTGGAAT<br>GGATCGGCAGGATCGACCCCAGCGACAGCGAGATCCACTACGACCAGAAGTTCAAGGACAAGGCC<br>ACCCTGACCGTGGACAAGAGCAGCAGCACCGCCTACATCCAGCTGTCCAGCCTGACCAGCGAGGA<br>CAGCGCCGTGTACTATTGCGCCCTGACCGGCATCTACGCCATGGCCTACTGGGGCCAGGGCACCA |

| | | | | |
|---|---|---|---|---|
| | | | | GCGTGACCGTGTCTTCC |
| 405. | PSCA-2 LH x I2C HL | artificial | aa | DIVMTQAAPSVPVTPGESVSISCRSSKSLLHSNGNTYLYWFLQRPGQSPQLLIYRMSNLASGVPDRFSGSGSGTAFTLRISRVEAEDVGVYYCLQHLEYPYTFGGGTKLELKGGGGSGGGGSGGGGSQVQVQQPGAELVKPGAPVKLSCKASGYTFTNYWLNWVKQRPGRGLEWIGRIDPSDSEIHYDQKFKDKATLTVDKSSSTAYIQLSSLTSEDSAVYYCALTGIYAMAYWGQGTSVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 406. | PSCA-2 LH x I2C HL | artificial | nt | GACATCGTGATGACCCAGGCCGCTCCAAGCGTGCCAGTGACCCCAGGCGAGAGCGTGTCCATCAGCTGCAGGTCCAGCAAGAGCCTGCTGCACAGCAACGGCAACACCTACCTGTACTGGTTTCTGCAGAGGCCAGGCCAGAGCCCCCAGCTGCTGATCTACAGGATGAGCAACCTGGCTAGCGGCGTGCCAGACAGATTCAGCGGCAGCGGCTCTGGAACCGCCTTCACCCTGAGGATCAGCAGGGTGGAGGCCGAGGACGTGGGCGTGTACTACTGCCTGCAGCACCTGGAATACCCCTACACCTTCGGCGGAGGGACCAAGCTGGAACTGAAGGGTGGTGGTGGTTCTGGCGGCGGCGGCTCCGGTGGTGGTGGTTCTCAGGTGCAGGTGCAGCAGCCAGGCGCCGAACTGGTCAAGCCTGGCGCCCCAGTGAAGCTGTCCTGCAAGGCCAGCGGCTACACCTTCACCAACTACTGGCTGAACTGGGTGAAACAGAGGCCCGGCAGGGGCCTGGAATGGATCGGCAGGATCGACCCCAGCGACAGCGAGATCCACTACGACCAGAAGTTCAAGGACAAGGCCACCCTGACCGTGGACAAGAGCAGCAGCACCGCCTACATCCAGCTGTCCAGCCTGACCAGCGAGGACAGCGCCGTGTACTATTGCGCCCTGACCGGCATCTACGCCATGGCCTACTGGGGCCAGGGCACCAGCGTGACCGTGTCTTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 407. | PSCA-3 VL | artificial | aa | DVVMTQTPLTLSVTIGQPASISCKSSQSLLDSDGKTYLNWLLQRPGQSPKRLIYLVSTLDSGVPDRFTGSGSGTDFTLKISRVEAEDLGVYYCWQGTHFPRTFGGGTKLEIK |
| 408. | PSCA-3 VL | artificial | nt | GACGTGGTCATGACCCAGACCCCCCTGACCCTGAGCGTGACCATCGGCCAGCCAGCCAGCATCTCTTGCAAGTCCAGCCAGTCCCTGCTGGACAGCGACGGCAAGACCTACCTGAACTGGCTGCTGCAGAGGCCAGGCCAGAGCCCCAAGAGGCTGATCTACCTGGTGTCCACCCTGGACAGCGGCGTGCCAGAC |

| | | | | |
|---|---|---|---|---|
| | | | | AGATTCACCGGCAGCGGCTCCGGCACCGACTTCACCCTGAAGATCAGCAGGGTGGAGGCCGAGGA CCTGGGCGTGTACTACTGCTGGCAGGGCACCCACTTCCCAAGGACCTTCGGCGGAGGGACCAAGC TGGAAATCAAG |
| 409. | PSCA-3 LCDR1 | artificial | aa | KSSQSLLDSDGKTYLN |
| 410. | PSCA-3 LCDR2 | artificial | aa | LVSTLDS |
| 411. | PSCA-3 LCDR3 | artificial | aa | WQGTHFPRT |
| 412. | PSCA-3 H | artificial | aa | EVQLQQSGPDLEKPGASVKISCKPSGNSFTGYYIHWVKQSHGKSLEWIGRVDPNNGFTSYNQKFK GKAILTVDKSSSTAYMELRSLTSEDSAVYYCVGNFFDSWGQGTTLTVSS |
| 413. | PSCA-3 H | artificial | nt | GAAGTGCAGCTGCAGCAGAGCGGCCCAGACCTGGAAAAGCCAGGCGCCAGCGTGAAGATTTCCTG CAAGCCCAGCGGCAACAGCTTCACCGGCTACTACATCCACTGGGTGAAACAGTCCCACGGGAAGA GCCTGGAATGGATCGGCAGAGTGGACCCCAACAACGGCTTCACCTCCTACAACCAGAAGTTCAAG GGCAAGGCCATCCTGACCGTGGACAAGAGCAGCAGCACCGCCTACATGGAACTGAGAAGCCTGAC CAGCGAGGACAGCGCCGTGTACTATTGCGTGGGCAACTTCTTCGACAGCTGGGGCCAGGGAACCA CACTGACCGTGTCCTCC |
| 414. | PSCA-3 HCDR1 | artificial | aa | GYYIH |
| 415. | PSCA-3 HCDR2 | artificial | aa | RVDPNNGFTSYNQKFKG |
| 416. | PSCA-3 HCDR3 | artificial | aa | NFFDS |
| 417. | PSCA-3 HL | artificial | aa | EVQLQQSGPDLEKPGASVKISCKPSGNSFTGYYIHWVKQSHGKSLEWIGRVDPNNGFTSYNQKFK GKAILTVDKSSSTAYMELRSLTSEDSAVYYCVGNFFDSWGQGTTLTVSSGGGGSGGGGSGGGGSD VVMTQTPLTLSVTIGQPASISCKSSQSLLDSDGKTYLNWLLQRPGQSPKRLIYLVSTLDSGVPDR FTGSGSGTDFTLKISRVEAEDLGVYYCWQGTHFPRTFGGGTKLEIK |
| 418. | PSCA-3 HL | artificial | nt | GAAGTGCAGCTGCAGCAGAGCGGCCCAGACCTGGAAAAGCCAGGCGCCAGCGTGAAGATTTCCTG CAAGCCCAGCGGCAACAGCTTCACCGGCTACTACATCCACTGGGTGAAACAGTCCCACGGGAAGA GCCTGGAATGGATCGGCAGAGTGGACCCCAACAACGGCTTCACCTCCTACAACCAGAAGTTCAAG GGCAAGGCCATCCTGACCGTGGACAAGAGCAGCAGCACCGCCTACATGGAACTGAGAAGCCTGAC CAGCGAGGACAGCGCCGTGTACTATTGCGTGGGCAACTTCTTCGACAGCTGGGGCCAGGGAACCA CACTGACCGTGTCCTCCGGTGGTGGTGGTTCTGGCGGCGGCGGCTCCGGTGGTGGTGGTTCTGAC GTGGTCATGACCCAGACCCCCCTGACCCTGAGCGTGACCATCGGCCAGCCAGCCAGCATCTCTTG CAAGTCCAGCCAGTCCCTGCTGGACAGCGACGGCAAGACCTACCTGAACTGGCTGCTGCAGAGGC CAGGCCAGAGCCCCAAGAGGCTGATCTACCTGGTGTCCACCCTGGACAGCGGCGTGCCAGACAGA TTCACCGGCAGCGGCTCCGGCACCGACTTCACCCTGAAGATCAGCAGGGTGGAGGCCGAGGACCT GGGCGTGTACTACTGCTGGCAGGGCACCCACTTCCCAAGGACCTTCGGCGGAGGGACCAAGCTGG AAATCAAG |
| 419. | PSCA-3 LH | artificial | aa | DVVMTQTPLTLSVTIGQPASISCKSSQSLLDSDGKTYLNWLLQRPGQSPKRLIYLVSTLDSGVPD RFTGSGSGTDFTLKISRVEAEDLGVYYCWQGTHFPRTFGGGTKLEIKGGGGSGGGGSGGGGSEVQ LQQSGPDLEKPGASVKISCKPSGNSFTGYYIHWVKQSHGKSLEWIGRVDPNNGFTSYNQKFKGKA |

| | | | | |
|---|---|---|---|---|
| | | | | ILTVDKSSSTAYMELRSLTSEDSAVYYCVGNFFDSWGQGTTLTVSS |
| 420. | PSCA-3 LH | artificial | nt | GACGTGGTCATGACCCAGACCCCCCTGACCCTGAGCGTGACCATCGGCCAGCCAGCCAGCATCTC<br>TTGCAAGTCCAGCCAGTCCCTGCTGGACAGCGACGGCAAGACCTACCTGAACTGGCTGCTGCAGA<br>GGCCAGGCCAGAGCCCCAAGAGGCTGATCTACCTGGTGTCCACCCTGGACAGCGGCGTGCCAGAC<br>AGATTCACCGGCAGCGGCTCCGGCACCGACTTCACCCTGAAGATCAGCAGGGTGGAGGCCGAGGA<br>CCTGGGCGTGTACTACTGCTGGCAGGGCACCCACTTCCCAAGGACCTTCGGCGGAGGGACCAAGC<br>TGGAAATCAAGGGTGGTGGTGGTTCTGGCGGCGGCGGCTCCGGTGGTGGTGGTTCTGAAGTGCAG<br>CTGCAGCAGAGCGGCCCAGACCTGGAAAAGCCAGGCGCCAGCGTGAAGATTTCCTGCAAGCCCAG<br>CGGCAACAGCTTCACCGGCTACTACATCCACTGGGTGAAACAGTCCCACGGGAAGAGCCTGGAAT<br>GGATCGGCAGAGTGGACCCCAACAACGGCTTCACCTCCTACAACCAGAAGTTCAAGGGCAAGGCC<br>ATCCTGACCGTGGACAAGAGCAGCAGCACCGCCTACATGGAACTGAGAAGCCTGACCAGCGAGGA<br>CAGCGCCGTGTACTATTGCGTGGGCAACTTCTTCGACAGCTGGGGCCAGGGAACCACACTGACCG<br>TGTCCTCC |
| 421. | PSCA-3 HL x I2C HL | artificial | aa | EVQLQQSGPDLEKPGASVKISCKPSGNSFTGYYIHWVKQSHGKSLEWIGRVDPNNGFTSYNQKFK<br>GKAILTVDKSSSTAYMELRSLTSEDSAVYYCVGNFFDSWGQGTTLTVSSGGGGSGGGGSGGGGSD<br>VVMTQTPLTLSVTIGQPASISCKSSQSLLDSDGKTYLNWLLQRPGQSPKRLIYLVSTLDSGVPDR<br>FTGSGSGTDFTLKISRVEAEDLGVYYCWQGTHFPRTFGGGTKLEIKSGGGGSEVQLVESGGGLVQ<br>PGGSLKLSCAASGFTFNKYAMNWVRQAPGKGLEWVARIRSKYNNYATYYADSVKDRFTISRDDSK<br>NTAYLQMNNLKTEDTAVYYCVRHGNFGNSYISYWAYWGQGTLVTVSSGGGGSGGGGSGGGGSQTV<br>VTQEPSLTVSPGGTVTLTCGSSTGAVTSGNYPNWVQQKPGQAPRGLIGGTKFLAPGTPARFSGSL<br>LGGKAALTLSGVQPEDEAEYYCVLWYSNRWVFGGGTKLTVL |
| 422. | PSCA-3 HL x I2C HL | artificial | nt | GAAGTGCAGCTGCAGCAGAGCGGCCCAGACCTGGAAAAGCCAGGCGCCAGCGTGAAGATTTCCTG<br>CAAGCCCAGCGGCAACAGCTTCACCGGCTACTACATCCACTGGGTGAAACAGTCCCACGGGAAGA<br>GCCTGGAATGGATCGGCAGAGTGGACCCCAACAACGGCTTCACCTCCTACAACCAGAAGTTCAAG<br>GGCAAGGCCATCCTGACCGTGGACAAGAGCAGCAGCACCGCCTACATGGAACTGAGAAGCCTGAC<br>CAGCGAGGACAGCGCCGTGTACTATTGCGTGGGCAACTTCTTCGACAGCTGGGGCCAGGGAACCA<br>CACTGACCGTGTCCTCCGGTGGTGGTGGTTCTGGCGGCGGCGGCTCCGGTGGTGGTGGTTCTGAC<br>GTGGTCATGACCCAGACCCCCCTGACCCTGAGCGTGACCATCGGCCAGCCAGCCAGCATCTCTTG<br>CAAGTCCAGCCAGTCCCTGCTGGACAGCGACGGCAAGACCTACCTGAACTGGCTGCTGCAGAGGC<br>CAGGCCAGAGCCCCAAGAGGCTGATCTACCTGGTGTCCACCCTGGACAGCGGCGTGCCAGACAGA<br>TTCACCGGCAGCGGCTCCGGCACCGACTTCACCCTGAAGATCAGCAGGGTGGAGGCCGAGGACCT<br>GGGCGTGTACTACTGCTGGCAGGGCACCCACTTCCCAAGGACCTTCGGCGGAGGGACCAAGCTGG<br>AAATCAAGTCCGGAGGTGGTGGATCCGAGGTGCAGCTGGTCGAGTCTGGAGGAGGATTGGTGCAG<br>CCTGGAGGGTCATTGAAACTCTCATGTGCAGCCTCTGGATTCACCTTCAATAAGTACGCCATGAA<br>CTGGGTCCGCCAGGCTCCAGGAAAGGGTTTGGAATGGGTTGCTCGCATAAGAAGTAAATATAATA<br>ATTATGCAACATATTATGCCGATTCAGTGAAAGACAGGTTCACCATCTCCAGAGATGATTCAAAA<br>AACACTGCCTATCTACAAATGAACAACTTGAAAACTGAGGACACTGCCGTGTACTACTGTGTGAG |

| | | | | |
|---|---|---|---|---|
| | | | | ACATGGGAACTTCGGTAATAGCTACATATCCTACTGGGCTTACTGGGGCCAAGGGACTCTGGTCA<br>CCGTCTCCTCAGGTGGTGGTGGTTCTGGCGGCGGCGGCTCCGGTGGTGGTGGTTCTCAGACTGTT<br>GTGACTCAGGAACCTTCACTCACCGTATCACCTGGTGGAACAGTCACACTCACTTGTGGCTCCTC<br>GACTGGGGCTGTTACATCTGGCAACTACCCAAACTGGGTCCAACAAAAACCAGGTCAGGCACCCC<br>GTGGTCTAATAGGTGGGACTAAGTTCCTCGCCCCCGGTACTCCTGCCAGATTCTCAGGCTCCCTG<br>CTTGGAGGCAAGGCTGCCCTCACCCTCTCAGGGGTACAGCCAGAGGATGAGGCAGAATATTACTG<br>TGTTCTATGGTACAGCAACCGCTGGGTGTTCGGTGGAGGAACCAAACTGACTGTCCTA |
| 423. | PSCA-3 LH x I2C HL | artificial | aa | DVVMTQTPLTLSVTIGQPASISCKSSQSLLDSDGKTYLNWLLQRPGQSPKRLIYLVSTLDSGVPD<br>RFTGSGSGTDFTLKISRVEAEDLGVYYCWQGTHFPRTFGGGTKLEIKGGGGSGGGGSGGGGSEVQ<br>LQQSGPDLEKPGASVKISCKPSGNSFTGYYIHWVKQSHGKSLEWIGRVDPNNGFTSYNQKFKGKA<br>ILTVDKSSSTAYMELRSLTSEDSAVYYCVGNFFDSWGQGTTLTVSSGGGGSEVQLVESGGGLVQP<br>GGSLKLSCAASGFTFNKYAMNWVRQAPGKGLEWVARIRSKYNNYATYYADSVKDRFTISRDDSKN<br>TAYLQMNNLKTEDTAVYYCVRHGNFGNSYISYWAYWGQGTLVTVSSGGGGSGGGGSGGGGSQTVV<br>TQEPSLTVSPGGTVTLTCGSSTGAVTSGNYPNWVQQKPGQAPRGLIGGTKFLAPGTPARFSGSLL<br>GGKAALTLSGVQPEDEAEYYCVLWYSNRWVFGGGTKLTVL |
| 424. | PSCA-3 LH x I2C HL | artificial | nt | GACGTGGTCATGACCCAGACCCCCCTGACCCTGAGCGTGACCATCGGCCAGCCAGCCAGCATCTC<br>TTGCAAGTCCAGCCAGTCCCTGCTGGACAGCGACGGCAAGACCTACCTGAACTGGCTGCTGCAGA<br>GGCCAGGCCAGAGCCCCAAGAGGCTGATCTACCTGGTGTCCACCCTGGACAGCGGCGTGCCAGAC<br>AGATTCACCGGCAGCGGCTCCGGCACCGACTTCACCCTGAAGATCAGCAGGGTGGAGGCCGAGGA<br>CCTGGGCGTGTACTACTGCTGGCAGGGCACCCACTTCCCAAGGACCTTCGGCGGAGGGACCAAGC<br>TGGAAATCAAGGGTGGTGGTGGTTCTGGCGGCGGCGGCTCCGGTGGTGGTGGTTCTGAAGTGCAG<br>CTGCAGCAGAGCGGCCCAGACCTGGAAAAGCCAGGCGCCAGCGTGAAGATTTCCTGCAAGCCCAG<br>CGGCAACAGCTTCACCGGCTACTACATCCACTGGGTGAAACAGTCCCACGGGAAGAGCCTGGAAT<br>GGATCGGCAGAGTGGACCCCAACAACGGCTTCACCTCCTACAACCAGAAGTTCAAGGGCAAGGCC<br>ATCCTGACCGTGGACAAGAGCAGCAGCACCGCCTACATGGAACTGAGAAGCCTGACCAGCGAGGA<br>CAGCGCCGTGTACTATTGCGTGGGCAACTTCTTCGACAGCTGGGGCCAGGGAACCACACTGACCG<br>TGTCCTCCGGAGGTGGTGGATCCGAGGTGCAGCTGGTCGAGTCTGGAGGAGGATTGGTGCAGCCT<br>GGAGGGTCATTGAAACTCTCATGTGCAGCCTCTGGATTCACCTTCAATAAGTACGCCATGAACTG<br>GGTCCGCCAGGCTCCAGGAAAGGGTTTGGAATGGGTTGCTCGCATAAGAAGTAAATATAATAATT<br>ATGCAACATATTATGCCGATTCAGTGAAAGACAGGTTCACCATCTCCAGAGATGATTCAAAAAAC<br>ACTGCCTATCTACAAATGAACAACTTGAAAACTGAGGACACTGCCGTGTACTACTGTGTGAGACA<br>TGGGAACTTCGGTAATAGCTACATATCCTACTGGGCTTACTGGGGCCAAGGGACTCTGGTCACCG<br>TCTCCTCAGGTGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGGCTCCTCGAC<br>TGGGGCTGTTACATCTGGCAACTACCCAAACTGGGTCCAACAAAAACCAGGTCAGGCACCCCGTG<br>GTCTAATAGGTGGGACTAAGTTCCTCGCCCCCGGTACTCCTGCCAGATTCTCAGGCTCCCTGCTT<br>GGAGGCAAGGCTGCCCTCACCCTCTCAGGGGTACAGCCAGAGGATGAGGCAGAATATTACTGTGT |

| | | | | |
|---|---|---|---|---|
| | | | | TCTATGGTACAGCAACCGCTGGGTGTTCGGTGGAGGAACCAAACTGACTGTCCTA |
| 425. | PSCA-4 VL | artificial | aa | QVVLTQSPAIMSASPGEKVTMACSASSSVRFIHWYQQKSGTSPKRWIYDTSKLASGVPTRFSGSG SGTSYSLTISTMEAEDAATYYCQQWSSSPFTFGSGTKLIIK |
| 426. | PSCA-4 VL | artificial | nt | CAGGTGGTGCTGACCCAGAGCCCAGCCATCATGAGCGCCAGCCCAGGCGAGAAAGTGACCATGGC CTGCAGCGCCAGCAGCAGCGTGCGGTTCATCCACTGGTATCAGCAGAAGTCCGGCACCAGCCCCA AGAGATGGATCTACGACACCAGCAAGCTGGCTAGCGGCGTGCCAACCAGATTCAGCGGAAGCGGC TCCGGCACCTCCTACAGCCTGACCATCAGCACCATGGAAGCCGAGGACGCCGCCACCTACTACTG CCAGCAGTGGAGCAGCAGCCCCTTCACCTTCGGCAGCGGCACCAAGCTGATCATCAAG |
| 427. | PSCA-4 LCDR1 | artificial | aa | SASSSVRFIH |
| 428. | PSCA-4 LCDR2 | artificial | aa | DTSKLAS |
| 429. | PSCA-4 LCDR3 | artificial | aa | QQWSSSPFT |
| 430. | PSCA-4 H | artificial | aa | EVQLQQSGAELVRSGASVKLSCTASGFNIKDYYIHWVNQRPDQGLEWIGWIDPENGDTEFVPKFQ GKATMTADIFSNTAYLHLSSLTSEDTAVYYCKTGGFWGQGTLVTVSS |
| 431. | PSCA-4 H | artificial | nt | GAAGTGCAGCTGCAGCAGTCTGGAGCCGAGCTGGTCAGATCTGGAGCCAGCGTGAAGCTGTCTTG CACCGCCAGCGGCTTCAACATCAAGGACTACTACATCCACTGGGTGAACCAGAGGCCCGACCAGG GCCTGGAATGGATCGGCTGGATCGACCCCGAGAACGGCGACACCGAGTTCGTGCCCAAGTTCCAG GGCAAGGCCACCATGACCGCCGACATCTTCAGCAACACCGCCTACCTGCACCTGAGCAGCCTGAC CTCTGAGGACACCGCCGTGTACTACTGCAAGACCGGCGGCTTCTGGGGACAGGGCACCCTGGTCA CCGTGTCCTCC |
| 432. | PSCA-4 HCDR1 | artificial | aa | DYYIH |
| 433. | PSCA-4 HCDR2 | artificial | aa | WIDPENGDTEFVPKFQG |
| 434. | PSCA-4 HCDR3 | artificial | aa | GGF |
| 435. | PSCA-4 HL | artificial | aa | EVQLQQSGAELVRSGASVKLSCTASGFNIKDYYIHWVNQRPDQGLEWIGWIDPENGDTEFVPKFQ GKATMTADIFSNTAYLHLSSLTSEDTAVYYCKTGGFWGQGTLVTVSSGGGGSGGGGSGGGGSQVV LTQSPAIMSASPGEKVTMACSASSSVRFIHWYQQKSGTSPKRWIYDTSKLASGVPTRFSGSGSGT SYSLTISTMEAEDAATYYCQQWSSSPFTFGSGTKLIIK |
| 436. | PSCA-4 HL | artificial | nt | GAAGTGCAGCTGCAGCAGTCTGGAGCCGAGCTGGTCAGATCTGGAGCCAGCGTGAAGCTGTCTTG CACCGCCAGCGGCTTCAACATCAAGGACTACTACATCCACTGGGTGAACCAGAGGCCCGACCAGG GCCTGGAATGGATCGGCTGGATCGACCCCGAGAACGGCGACACCGAGTTCGTGCCCAAGTTCCAG GGCAAGGCCACCATGACCGCCGACATCTTCAGCAACACCGCCTACCTGCACCTGAGCAGCCTGAC CTCTGAGGACACCGCCGTGTACTACTGCAAGACCGGCGGCTTCTGGGGACAGGGCACCCTGGTCA CCGTGTCCTCCGGTGGTGGTGGTTCTGGCGGCGGCGGCTCCGGTGGTGGTGGTTCTCAGGTGGTG CTGACCCAGAGCCCAGCCATCATGAGCGCCAGCCCAGGCGAGAAAGTGACCATGGCCTGCAGCGC CAGCAGCAGCGTGCGGTTCATCCACTGGTATCAGCAGAAGTCCGGCACCAGCCCCAAGAGATGGA TCTACGACACCAGCAAGCTGGCTAGCGGCGTGCCAACCAGATTCAGCGGAAGCGGCTCCGGCACC TCCTACAGCCTGACCATCAGCACCATGGAAGCCGAGGACGCCGCCACCTACTACTGCCAGCAGTG |

| | | | | |
|---|---|---|---|---|
| | | | | GAGCAGCAGCCCCTTCACCTTCGGCAGCGGCACCAAGCTGATCATCAAG |
| 437. | PSCA-4 LH | artificial | aa | QVVLTQSPAIMSASPGEKVTMACSASSSVRFIHWYQQKSGTSPKRWIYDTSKLASGVPTRFSGSG<br>SGTSYSLTISTMEAEDAATYYCQQWSSSPFTFGSGTKLIIKGGGGSGGGGSGGGGSEVQLQQSGA<br>ELVRSGASVKLSCTASGFNIKDYYIHWVNQRPDQGLEWIGWIDPENGDTEFVPKFQGKATMTADI<br>FSNTAYLHLSSLTSEDTAVYYCKTGGFWGQGTLVTVSS |
| 438. | PSCA-4 LH | artificial | nt | CAGGTGGTGCTGACCCAGAGCCCAGCCATCATGAGCGCCAGCCCAGGCGAGAAAGTGACCATGGC<br>CTGCAGCGCCAGCAGCAGCGTGCGGTTCATCCACTGGTATCAGCAGAAGTCCGGCACCAGCCCCA<br>AGAGATGGATCTACGACACCAGCAAGCTGGCTAGCGGCGTGCCAACCAGATTCAGCGGAAGCGGC<br>TCCGGCACCTCCTACAGCCTGACCATCAGCACCATGGAAGCCGAGGACGCCGCCACCTACTACTG<br>CCAGCAGTGGAGCAGCAGCCCCTTCACCTTCGGCAGCGGCACCAAGCTGATCATCAAGGGTGGTG<br>GTGGTTCTGGCGGCGGCGGCTCCGGTGGTGGTGGTTCTGAAGTGCAGCTGCAGCAGTCTGGAGCC<br>GAGCTGGTCAGATCTGGAGCCAGCGTGAAGCTGTCTTGCACCGCCAGCGGCTTCAACATCAAGGA<br>CTACTACATCCACTGGGTGAACCAGAGGCCCGACCAGGGCCTGGAATGGATCGGCTGGATCGACC<br>CCGAGAACGGCGACACCGAGTTCGTGCCCAAGTTCCAGGGCAAGGCCACCATGACCGCCGACATC<br>TTCAGCAACACCGCCTACCTGCACCTGAGCAGCCTGACCTCTGAGGACACCGCCGTGTACTACTG<br>CAAGACCGGCGGCTTCTGGGGACAGGGCACCCTGGTCACCGTGTCCTCC |
| 439. | PSCA-4 HL x I2C HL | artificial | aa | EVQLQQSGAELVRSGASVKLSCTASGFNIKDYYIHWVNQRPDQGLEWIGWIDPENGDTEFVPKFQ<br>GKATMTADIFSNTAYLHLSSLTSEDTAVYYCKTGGFWGQGTLVTVSSGGGGSGGGGSGGGGSQVV<br>LTQSPAIMSASPGEKVTMACSASSSVRFIHWYQQKSGTSPKRWIYDTSKLASGVPTRFSGSGSGT<br>SYSLTISTMEAEDAATYYCQQWSSSPFTFGSGTKLIIKSGGGGSEVQLVESGGGLVQPGGSLKLS<br>CAASGFTFNKYAMNWVRQAPGKGLEWVARIRSKYNNYATYYADSVKDRFTISRDDSKNTAYLQMN<br>NLKTEDTAVYYCVRHGNFGNSYISYWAYWGQGTLVTVSSGGGGSGGGGSGGGGSQTVVTQEPSLT<br>VSPGGTVTLTCGSSTGAVTSGNYPNWVQQKPGQAPRGLIGGTKFLAPGTPARFSGSLLGGKAALT<br>LSGVQPEDEAEYYCVLWYSNRWVFGGGTKLTVL |
| 440. | PSCA-4 HL x I2C HL | artificial | nt | GAAGTGCAGCTGCAGCAGTCTGGAGCCGAGCTGGTCAGATCTGGAGCCAGCGTGAAGCTGTCTTG<br>CACCGCCAGCGGCTTCAACATCAAGGACTACTACATCCACTGGGTGAACCAGAGGCCCGACCAGG<br>GCCTGGAATGGATCGGCTGGATCGACCCCGAGAACGGCGACACCGAGTTCGTGCCCAAGTTCCAG<br>GGCAAGGCCACCATGACCGCCGACATCTTCAGCAACACCGCCTACCTGCACCTGAGCAGCCTGAC<br>CTCTGAGGACACCGCCGTGTACTACTGCAAGACCGGCGGCTTCTGGGGACAGGGCACCCTGGTCA<br>CCGTGTCCTCCGGTGGTGGTGGTTCTGGCGGCGGCGGCTCCGGTGGTGGTGGTTCTCAGGTGGTG<br>CTGACCCAGAGCCCAGCCATCATGAGCGCCAGCCCAGGCGAGAAAGTGACCATGGCCTGCAGCGC<br>CAGCAGCAGCGTGCGGTTCATCCACTGGTATCAGCAGAAGTCCGGCACCAGCCCCAAGAGATGGA<br>TCTACGACACCAGCAAGCTGGCTAGCGGCGTGCCAACCAGATTCAGCGGAAGCGGCTCCGGCACC<br>TCCTACAGCCTGACCATCAGCACCATGGAAGCCGAGGACGCCGCCACCTACTACTGCCAGCAGTG<br>GAGCAGCAGCCCCTTCACCTTCGGCAGCGGCACCAAGCTGATCATCAAGTCCGGAGGTGGTGGAT<br>CCGAGGTGCAGCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGCTCCAGGAAA |

| | | | | |
|---|---|---|---|---|
| | | | | GGGTTTGGAATGGGTTGCTCGCATAAGAAGTAAATATAATAATTATGCAACATATTATGCCGATT<br>CAGTGAAAGACAGGTTCACCATCTCCAGAGATGATTCAAAAAACACTGCCTATCTACAAATGAAC<br>AACTTGAAAACTGAGGACACTGCCGTGTACTACTGTGTGAGACATGGGAACTTCGGTAATAGCTA<br>CATATCCTACTGGGCTTACTGGGGCCAAGGGACTCTGGTCACCGTCTCCTCAGGTGGTGGTGGTT<br>CTGGCGGCGGCGGCTCCGGTGGTGGTGGTTCTCAGACTGTTGTGACTCAGGAACCTTCACTCACC<br>GTATCACCTGGTGGAACAGTCACACTCACTTGTGGCTCCTCGACTGGGGCTGTTACATCTGGCAA<br>CTACCCAAACTGGGTCCAACAAAAACCAGGTCAGGCACCCCGTGGTCTAATAGGTGGGACTAAGT<br>TCCTCGCCCCCGGTACTCCTGCCAGATTCTCAGGCTCCCTGCTTGGAGGCAAGGCTGCCCTCACC<br>CTCTCAGGGGTACAGCCAGAGGATGAGGCAGAATATTACTGTGTTCTATGGTACAGCAACCGCTG<br>GGTGTTCGGTGGAGGAACCAAACTGACTGTCCTA |
| 441. | PSCA-4 LH x I2C HL | artificial | aa | QVVLTQSPAIMSASPGEKVTMACSASSSVRFIHWYQQKSGTSPKRWIYDTSKLASGVPTRFSGSG<br>SGTSYSLTISTMEAEDAATYYCQQWSSSPFTFGSGTKLIIKGGGGSGGGGSGGGGSEVQLQQSGA<br>ELVRSGASVKLSCTASGFNIKDYYIHWVNQRPDQGLEWIGWIDPENGDTEFVPKFQGKATMTADI<br>FSNTAYLHLSSLTSEDTAVYYCKTGGFWGQGTLVTVSSGGGGSEVQLVESGGGLVQPGGSLKLSC<br>AASGFTFNKYAMNWVRQAPGKGLEWVARIRSKYNNYATYYADSVKDRFTISRDDSKNTAYLQMNN<br>LKTEDTAVYYCVRHGNFGNSYISYWAYWGQGTLVTVSSGGGGSGGGGSGGGGSQTVVTQEPSLTV<br>SPGGTVTLTCGSSTGAVTSGNYPNWVQQKPGQAPRGLIGGTKFLAPGTPARFSGSLLGGKAALTL<br>SGVQPEDEAEYYCVLWYSNRWVFGGGTKLTVL |
| 442. | PSCA-4 LH x I2C HL | artificial | nt | CAGGTGGTGCTGACCCAGAGCCCAGCCATCATGAGCGCCAGCCCAGGCGAGAAAGTGACCATGGC<br>CTGCAGCGCCAGCAGCAGCGTGCGGTTCATCCACTGGTATCAGCAGAAGTCCGGCACCAGCCCCA<br>AGAGATGGATCTACGACACCAGCAAGCTGGCTAGCGGCGTGCCAACCAGATTCAGCGGAAGCGGC<br>TCCGGCACCTCCTACAGCCTGACCATCAGCACCATGGAAGCCGAGGACGCCGCCACCTACTACTG<br>CCAGCAGTGGAGCAGCAGCCCCTTCACCTTCGGCAGCGGCACCAAGCTGATCATCAAGGGTGGTG<br>GTGGTTCTGGCGGCGGCGGCTCCGGTGGTGGTGGTTCTGAAGTGCAGCTGCAGCAGTCTGGAGCC<br>GAGCTGGTCAGATCTGGAGCCAGCGTGAAGCTGTCTTGCACCGCCAGCGGCTTCAACATCAAGGA<br>CTACTACATCCACTGGGTGAACCAGAGGCCCGACCAGGGCCTGGAATGGATCGGCTGGATCGACC<br>CCGAGAACGGCGACACCGAGTTCGTGCCCAAGTTCCAGGGCAAGGCCACCATGACCGCCGACATC<br>TTCAGCAACACCGCCTACCTGCACCTGAGCAGCCTGACCTCTGAGGACACCGCCGTGTACTACTG<br>CAAGACCGGCGGCTTCTGGGGACAGGGCACCCTGGTCACCGTGTCCTCCGGAGGTGGTGGATCCG<br>AGGTGCAGCTGGTCGAGTCTGGAGGAGGATTGGTGCAGCCTGGAGGGTCATTGAAACTCTCATGT<br>GCAGCCTCTGGATTCACCTTCAATAAGTACGCCATGAACTGGGTCCGCCAGGCTCCAGGAAAGGG<br>TTTGGAATGGGTTGCTCGCATAAGAAGTAAATATAATAATTATGCAACATATTATGCCGATTCAG<br>TGAAAGACAGGTTCACCATCTCCAGAGATGATTCAAAAAACACTGCCTATCTACAAATGAACAAC<br>TTGAAAACTGAGGACACTGCCGTGTACTACTGTGTGAGACATGGGAACTTCGGTAATAGCTACAT<br>ATCCTACTGGGCTTACTGGGGCCAAGGGACTCTGGTCACCGTCTCCTCAGGTGGTGGTGGTTCTG<br>GCGGCGGCGGCTCCGGTGGTGGTGGTTCTCAGACTGTTGTGACTCAGGAACCTTCACTCACCGTA<br>TCACCTGGTGGAACAGTCACACTCACTTGTGGCTCCTCGACTGGGGCTGTTACATCTGGCAACTA |

| | | | | |
|---|---|---|---|---|
| | | | | CCCAAACTGGGTCCAACAAAAACCAGGTCAGGCACCCCGTGGTCTAATAGGTGGGACTAAGTTCC TCGCCCCCGGTACTCCTGCCAGATTCTCAGGCTCCCTGCTTGGAGGCAAGGCTGCCCTCACCCTC TCAGGGGTACAGCCAGAGGATGAGGCAGAATATTACTGTGTTCTATGGTACAGCAACCGCTGGGT GTTCGGTGGAGGAACCAAACTGACTGTCCTA |
| 443. | PSCA | human | aa | MGWSCIILFLVATATGVHSDYKDDDDKLLCYSCKAQVSNEDCLQVENCTQLGEQCWTARIRAVGL LTVISKGCSLNCVDDSQDYYVGKKNITCCDTDLCNASGAHALQPAAAILALLPALGLLLWGPGQL |
| 444. | PSCA | human | nt | ATGGGATGGAGCTGTATCATCCTCTTCTTGGTAGCAACAGCTACAGGTGTACACTCCGATTACAA GGACGACGATGACAAGCTGCTGTGCTACTCCTGCAAAGCCCAGGTGAGCAACGAGGACTGCCTGC AGGTGGAGAACTGCACCCAGCTGGGGGAGCAGTGCTGGACCGCGCGCATCCGCGCAGTTGGCCTC CTGACCGTCATCAGCAAAGGCTGCAGCTTGAACTGCGTGGATGACTCACAGGACTACTACGTGGG CAAGAAGAACATCACGTGCTGTGACACCGACTTGTGCAACGCCAGCGGGGCCCATGCCCTGCAGC CGGCTGCTGCCATCCTTGCGCTGCTCCCTGCACTCGGCCTGCTGCTCTGGGGACCCGGCCAGCTC TAG |
| 445. | PSCA | Macaque (Cynomol gus) | aa | MGWSCIILFLVATATGVHSDYKDDDDKLLCYSCKAQVSNEDCLNVENCTQPEEQCWTERIRAVGL LTVISKGCSSNCVDDSQDYYVGKKNITCCDTDLCNASGAHALQPAAAILALLPALSLLLWGPRQL |
| 446. | PSCA | Macaque (Cynomol gus) | nt | ATGGGATGGAGCTGTATCATCCTCTTCTTGGTAGCAACAGCTACAGGTGTACACTCCGATTACAA GGACGACGATGACAAGCTGCTGTGCTACTCCTGCAAGGCCCAGGTGAGCAACGAGGACTGCCTGA ATGTGGAGAACTGCACGCAGCCGGAGGAGCAGTGCTGGACCGAGCGCATCCGCGCCGTGGGCCTC CTGACCGTCATCAGCAAAGGCTGCAGCTCAAACTGCGTGGATGACTCACAGGACTACTACGTGGG CAAGAAGAACATCACCTGCTGTGACACCGACCTGTGCAACGCCAGCGGGGCCCATGCACTGCAGC CGGCTGCTGCCATCCTGGCACTGCTCCCTGCACTCAGCCTGCTGCTTTGGGGCCCCAGACAGCTG TAG |
| 447. | forward primer | artificial | nt | CACCACAGCCCACCAGTGACC |
| 448. | reverse primer | artificial | nt | GAGGCCTGGGGCACCACACCC |
| 449. | P3-VH-A-Xhol | artificial | nt | CCTGATCTCGAGAGCGGCSCAGASSTGRAAAAGCCAGGCGCCACGGTGAAGATT |
| 450. | P3-VH-B | artificial | nt | GGCGCCACGGTGAAGATTTCCTGCAAGSYCAGCGGCWACACGTTCACCGGCTACTACATCCACTG GGTG |
| 451. | P3-VH-C | artificial | nt | GTAGGAGGTGAAGCCGTTGTTGGGGTCCACTCTGCCSATCCATTCCAGGCYCTTCCCGKGGGMCT GTTKCACCCAGTGGATGTAGTA |
| 452. | P3-VH-D | artificial | nt | AACGGCTTCACCTCCTACAACCAGAAGTTCAAGGGCARGGYCAYAMTKACCGYGGACAMGAGCAC CRRCACCGCCTACATGGAACTG |
| 453. | P3-VH-E | artificial | nt | GCTGTCGAAGAAGTTGCCCRCGCAATAGTACACGGCGGTGTCCTCGCTGSKCAGGCTKCTCAGTT CCATGTAGGCGGT |
| 454. | P3-VH-F-BstEII | artificial | nt | CACGTCGGTGACCGTGGTTCCCTGGCCCCAGCTGTCGAAGAAGTTGCC |
| 455. | PSCA2VH-A-XHO1 | artificial | nt | CAGGTGCTCGAGYCAGGCGCCGAASTGRWGAAGCCTGGCGCCMCAGTGAAGMTATCCTGCAAGGY |

| | | | | |
|---|---|---|---|---|
| | | | | CAGCGGCTACACCTTCACCAAC |
| 456. | PSCA2VH-B | artificial | nt | GCTGTCGCTGGGGTCGATCCTGCCYATCCATTCCAGGCCCYTGCCGGGCSYCTGTTKCACCCAGT TCAGCCAGTAGTTGGTGAAGGTGTAGCC |
| 457. | PSCA2VH-C | artificial | nt | AGGATCGACCCCAGCGACAGCGAGATCCACTACGACCAGAAGTTCAAGGACARAGYCACCMTAAC CGYGGACAMGAGCACCRRCACCGCCTAC |
| 458. | PSCA2VH-D | artificial | nt | GGCCAGGGCGCAATAGTACACGGCGGTGTCCTCGCTTSTCAGGCTGGACAGCTSSATGTAGGCGG TGYYGGTGCTC |
| 459. | PSCA2VH-E-BSTE2 | artificial | nt | AGACACGGTGACCGTGGTGCCCTGGCCCCAGTAGGCCATGGCGTAGATGCCGGTCAGGGCGCAAT AGTACAC |
| 460. | P3-VL-A-SacI | artificial | nt | CCTGTAGAGCTCGTCATGACCCAGTCCCCCCTGTCCCTGMSCGTGACCMTCGGCCAGCCAGCCAG CATC |
| 461. | P3-VL-B | artificial | nt | CCAGTTCAGGTAGGTCTTGCCGTCGCTGTCCAGCAGGGACTGGCTGGACTTGCAAGAGATGCTGG CTGGCTGGCC |
| 462. | P3-VL-C | artificial | nt | AAGACCTACCTGAACTGGYTSCWGCAGAGGCCAGGCCAGAGCCCCARGAGGCTGATCTACCTGGT GTCCACCCTG |
| 463. | P3-VL-D | artificial | nt | GGTGAAGTCGGTGCCGGAGCCGCTGCCGGAGAATCTGTCTGGCACGCCGCTGTCCAGGGTGGACA CCAGGTA |
| 464. | P3-VL-E | artificial | nt | TCCGGCACCGACTTCACCCTGAAGATCAGCAGGGTGGAGGCCGAGGACSTGGGCGTGTACTACTG CTGGCAGGGCACC |
| 465. | P3-VL-F-BsiWI/SpeI | artificial | nt | CCAGACACTAGTCGTACGCTTGATTTCCAGCTTGGTCCCTCCGCCGAAGGTCCTTGGGAAGTGGG TGCCCTGCCAGCAGTA |
| 466. | PSCA2VL-a-SAC1 | artificial | nt | CAGACAGAGCTCGTGATGACCCAGKCASCTCYAAGCSTGCCAGTGACCCCAGGCGAGYCAGYGTC CATCAGCTGCAGGTCCAGC |
| 467. | PSCA2VL-b | artificial | nt | CTGGGGGCTCTGGCCTGGCYTCTGCAGAWACCAGTACAGGTAGGTGTTGCCGTTGCTGTGCAGCA GGCTCTTGCTGGACCTGCAGCTGATG |
| 468. | PSCA2VL-c | artificial | nt | CCAGGCCAGAGCCCCCAGCTGCTGATCTACAGGATGAGCAACCTGGCTAGCGGCGTGCCAGACAG ATTCAGCGGCAGCGGCTCTGGAACC |
| 469. | PSCA2VL-d | artificial | nt | CAGGCAGTAGTACACGCCCACGTCCTCGGCCTCCACCCTGCTGATCYTCAGGGTGAAGKCGGTTC CAGAGCCGCTGCC |
| 470. | PSCA2VL-e-BsiW1-Spe1 | artificial | nt | GTGCTGACTAGTCGTACGCTTGATTTCCAGCTTGGTCCCTTGGCCGAAGGTGTAGGGGTATTCCA GGTGCTGCAGGCAGTAGTACACGCC |

| | | | | |
|---|---|---|---|---|
| 471. | HD37 L | artificial | aa | DIQLTQSPASLAVSLGQRATISCKASQSVDYDGDSYLNWYQQIPGQPPKLLIYDASNLVSGIPPR FSGSGSGTDFTLNIHPVEKVDAATYHCQQSTEDPWTFGGGTKLEIK |
| 472. | HD37 L | artificial | nt | GATATCCAGCTGACCCAGTCTCCAGCTTCTTTGGCTGTGTCTCTAGGGCAGAGGGCCACCATCTC |

| | | | | |
|---|---|---|---|---|
| | | | | CTGCAAGGCCAGCCAAAGTGTTGATTATGATGGTGATAGTTATTTGAACTGGTACCAACAGATTCCAGGACAGCCACCCAAACTCCTCATCTATGATGCATCCAATCTAGTTTCTGGGATCCCACCCAGGTTTAGTGGCAGTGGGTCTGGGACAGACTTCACCCTCAACATCCATCCTGTGGAGAAGGTGGATGCTGCAACCTATCACTGTCAGCAAAGTACTGAGGATCCGTGGACGTTCGGTGGAGGGACCAAGCTCGAGATCAAA |
| 473. | HD37 LCDR1 | artificial | aa | KASQSVDYDGDSYLN |
| 474. | HD37 LCDR2 | artificial | aa | DASNLVS |
| 475. | HD37 LCDR3 | artificial | aa | QQSTEDPWT |
| 476. | HD37 H | artificial | aa | QVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSS |
| 477. | HD37 H | artificial | nt | CAGGTGCAGCTGCAGCAGTCTGGGGCTGAGCTGGTGAGGCCTGGGTCCTCAGTGAAGATTTCCTGCAAGGCTTCTGGCTATGCATTCAGTAGCTACTGGATGAACTGGGTGAAGCAGAGGCCTGGACAGGGTCTTGAGTGGATTGGACAGATTTGGCCTGGAGATGGTGATACTAACTACAATGGAAAGTTCAAGGGTAAAGCCACTCTGACTGCAGACGAATCCTCCAGCACAGCCTACATGCAACTCAGCAGCCTAGCATCTGAGGACTCTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 478. | HD37 HCDR1 | artificial | aa | SYWMN |
| 479. | HD37 HCDR2 | artificial | aa | QIWPGDGDTNYNGKFKG |
| 480. | HD37 HCDR3 | artificial | aa | RETTTVGRYYYAMDY |
| 481. | HD37 LH x I2C HL | artificial | aa | DIQLTQSPASLAVSLGQRATISCKASQSVDYDGDSYLNWYQQIPGQPPKLLIYDASNLVSGIPPRFSGSGSGTDFTLNIHPVEKVDAATYHCQQSTEDPWTFGGGTKLEIKGGGGSGGGGSGGGGSQVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |

| | | | | |
|---|---|---|---|---|
| 482. | HD37 LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGATAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 483. | HD37 LH x H2C HL | artificial | aa | DIQLTQSPASLAVSLGQRATISCKASQSVDYDGDSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGYYPNWVQQKPGQAPRGLIGGTKFLAPGT<br>PARFSGSLLGGKAALTLSGVQPEDEAEYYCALWYSNRWVFGGGTKLTVL |
| 484. | HD37 LH x H2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGATAGTTATTTGAACTGGTACCAACAGATTC<br>CAGGACAGCCACCCAAACTCCTCATCTATGATGCATCCAATCTAGTTTCTGGGATCCCACCCAGG |

| | | | | |
|---|---|---|---|---|
| | | | | TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCTACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGCTCTATGGTACAGCAACCGCTGGGTGTTCGGTGGAGGAA<br>CCAAACTGACTGTCCTA |
| 485. | HD37 LH x F12Q HL | artificial | aa | DIQLTQSPASLAVSLGQRATISCKASQSVDYDGDSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNSYAMNWVRQAPGKGLEWVARIRSKYNNYATYYADSVKGRF<br>TISRDDSKNTAYLQMNNLKTEDTAVYYCVRHGNFGNSYVSWWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 486. | HD37 LH x F12Q HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGATAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG |

| | | | | |
|---|---|---|---|---|
| | | | | CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAGCTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGGCAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACGTTTCCTGGTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 487. | hCD19 26-D6 L | artificial | aa | DIQMTQSPASLSASLGDRVTITCKASQSVDYDGDSYLNWYQQKPGKAPKLLIYDASNLVSGIPSR<br>FSGSGSGTDFTLTISSLQQVDFATYYCQQSTEDPWTFGQGTKVEIK |
| 488. | hCD19 26-D6 L | artificial | nt | GACATCCAGATGACCCAGTCTCCAGCTTCTTTGTCTGCGTCTCTAGGGGACAGAGTCACCATCAC<br>CTGCAAGGCCAGCCAAAGTGTTGATTATGATGGTGATAGTTATTTGAACTGGTACCAACAGAAAC<br>CAGGAAAGGCACCCAAACTCCTCATCTATGATGCATCCAATCTAGTTTCTGGGATCCCATCCAGG<br>TTTAGTGGCAGTGGGTCTGGGACAGACTTCACCCTCACCATCAGTTCTCTGCAGCAGGTGGATTT<br>CGCAACCTATTACTGTCAGCAAAGTACTGAGGATCCGTGGACGTTCGGTCAAGGGACCAAGGTGG<br>AGATCAAA |
| 489. | hCD19 26-D6 LCDR1 | artificial | aa | KASQSVDYDGDSYLN |
| 490. | hCD19 26-D6 LCDR2 | artificial | aa | DASNLVS |

| | | | | |
|---|---|---|---|---|
| 491. | hCD19 26-D6 LCDR3 | artificial | aa | QQSTEDPWT |
| 492. | hCD19 26-D6 H | artificial | aa | QVQLVQSGAEVKKPGASVKVSCKASGYTFTSYWMNWVKQGPGQGLEWMGQIWPGDGDTNYNGKFKGRVTMTADASTSTVYMELSSLRSEDTAVYFCARRETTTVGRYYYAMDYWGQGTTVTVSS |
| 493. | hCD19 26-D6 H | artificial | nt | CAGGTGCAGCTGGTCCAGTCTGGGGCTGAGGTGAAGAAGCCTGGGGCCTCAGTGAAGGTTTCCTGCAAGGCTTCTGGCTATACATTCACTAGCTACTGGATGAACTGGGTGAAGCAGGGCCCTGGACAGGGTCTTGAGTGGATGGGACAGATTTGGCCTGGAGATGGTGATACTAACTACAATGGAAAGTTCAAGGGTAGAGTCACTATGACTGCTGACGCATCCACCAGCACAGTCTACATGGAACTCAGCAGCCTGCGATCTGAGGACACTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 494. | hCD19 26-D6 HCDR1 | artificial | aa | SYWMN |
| 495. | hCD19 26-D6 HCDR2 | artificial | aa | QIWPGDGDTNYNGKFKG |
| 496. | hCD19 26-D6 HCDR3 | artificial | aa | RETTTVGRYYYAMDY |
| 497. | hCD19 26-D6 LH x I2C HL | artificial | aa | DIQMTQSPASLSASLGDRVTITCKASQSVDYDGDSYLNWYQQKPGKAPKLLIYDASNLVSGIPSRFSGSGSGTDFTLTISSLQQVDFATYYCQQSTEDPWTFGQGTKVEIKGGGGSGGGGSGGGGSQVQLVQSGAEVKKPGASVKVSCKASGYTFTSYWMNWVKQGPGQGLEWMGQIWPGDGDTNYNGKFKGRVTMTADASTSTVYMELSSLRSEDTAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 498. | hCD19 26-D6 LH x I2C HL | artificial | nt | GACATCCAGATGACCCAGTCTCCAGCTTCTTTGTCTGCGTCTCTAGGGGACAGAGTCACCATCACCTGCAAGGCCAGCCAAAGTGTTGATTATGATGGTGATAGTTATTTGAACTGGTACCAACAGAAACCAGGAAAGGCACCCAAACTCCTCATCTATGATGCATCCAATCTAGTTTCTGGGATCCCATCCAGGTTTAGTGGCAGTGGGTCTGGGACAGACTTCACCCTCACCATCAGTTCTCTGCAGCAGGTGGATTTCGCAACCTATTACTGTCAGCAAAGTACTGAGGATCCGTGGACGTTCGGTCAAGGGACCAAGGTGG |

| | | | | |
|---|---|---|---|---|
| | | | | AGATCAAAGGTGGTGGTGGTTCTGGCGGCGGCGGCTCCGGTGGTGGTGGTTCTCAGGTGCAGCTG<br>GTCCAGTCTGGGGCTGAGGTGAAGAAGCCTGGGGCCTCAGTGAAGGTTTCCTGCAAGGCTTCTGG<br>CTATACATTCACTAGCTACTGGATGAACTGGGTGAAGCAGGGCCCTGGACAGGGTCTTGAGTGGA<br>TGGGACAGATTTGGCCTGGAGATGGTGATACTAACTACAATGGAAAGTTCAAGGGTAGAGTCACT<br>ATGACTGCTGACGCATCCACCAGCACAGTCTACATGGAACTCAGCAGCCTGCGATCTGAGGACAC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 499. | hCD19 45-A10 L | artificial | aa | DIQMTQSPASLSVSVGERVTITCKASQSVDYDGDSYLNWYQQKPGKAPKLLIYDASNLVSGVPPR<br>FSGSGSGTDFTLTISSVQPEDFATYYCQQSTEDPWTFGQGTKVEIK |
| 500. | hCD19 45-A10 L | artificial | nt | GACATCCAGATGACCCAGTCTCCAGCTTCTTTGTCTGTGTCTGTAGGGGAGAGAGTCACCATCAC<br>CTGCAAGGCCAGCCAAAGTGTTGATTATGATGGTGATAGTTATTTGAACTGGTACCAACAGAAAC<br>CAGGAAAGGCACCCAAACTCCTCATCTATGATGCATCCAATCTAGTTTCTGGGGTCCCACCCAGG<br>TTTAGTGGCAGTGGGTCTGGGACAGACTTCACCCTCACCATCAGTTCTGTGCAGCCGGAGGATTT<br>CGCAACCTATTACTGTCAGCAAAGTACTGAGGATCCGTGGACGTTCGGTCAAGGGACCAAGGTGG<br>AGATCAAA |
| 501. | hCD19 45-A10 LCDR1 | artificial | aa | KASQSVDYDGDSYLN |
| 502. | hCD19 45-A10 LCDR2 | artificial | aa | DASNLVS |

| | | | | |
|---|---|---|---|---|
| 503. | hCD19 45-A10 LCDR3 | artificial | aa | QQSTEDPWT |
| 504. | hCD19 45-A10 H | artificial | aa | QVQLVQSGAELKKPGSSVKISCKASGYSFTSYWMNWVKQAPGQGLEWMGQIWPGDGDTNYNGKFKGKATMTADPSTSTAYMELSSLGSEDTAVYYCARRETTTVGRYYYAMDYWGQGTTVTVSS |
| 505. | hCD19 45-A10 H | artificial | nt | CAGGTGCAGCTGGTCCAGTCTGGGGCTGAGCTGAAGAAGCCTGGGTCCTCAGTGAAGATTTCCTGCAAGGCTTCTGGCTATTCATTCACTAGCTACTGGATGAACTGGGTGAAGCAGGCCCCTGGACAGGGTCTTGAGTGGATGGGACAGATTTGGCCTGGAGATGGTGATACTAACTACAATGGAAAGTTCAAGGGTAAAGCCACTATGACTGCTGACCCATCCACCAGCACAGCCTACATGGAACTCAGCAGCCTGGGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCC |
| 506. | hCD19 45-A10 HCDR1 | artificial | aa | SYWMN |
| 507. | hCD19 45-A10 HCDR2 | artificial | aa | QIWPGDGDTNYNGKFKG |
| 508. | hCD19 45-A10 HCDR3 | artificial | aa | RETTTVGRYYYAMDY |
| 509. | hCD19 45-A10 LH x I2C HL | artificial | aa | DIQMTQSPASLSVSVGERVTITCKASQSVDYDGDSYLNWYQQKPGKAPKLLIYDASNLVSGVPPRFSGSGSGTDFTLTISSVQPEDFATYYCQQSTEDPWTFGQGTKVEIKGGGGSGGGGSGGGGSQVQLVQSGAELKKPGSSVKISCKASGYSFTSYWMNWVKQAPGQGLEWMGQIWPGDGDTNYNGKFKGKATMTADPSTSTAYMELSSLGSEDTAVYYCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 510. | hCD19 45-A10 LH x I2C HL | artificial | nt | GACATCCAGATGACCCAGTCTCCAGCTTCTTTGTCTGTGTCTGTAGGGGAGAGAGTCACCATCACCTGCAAGGCCAGCCAAAGTGTTGATTATGATGGTGATAGTTATTTGAACTGGTACCAACAGAAACCAGGAAAGGCACCCAAACTCCTCATCTATGATGCATCCAATCTAGTTTCTGGGGTCCCACCCAGGTTTAGTGGCAGTGGGTCTGGGACAGACTTCACCCTCACCATCAGTTCTGTGCAGCCGGAGGATTTCGCAACCTATTACTGTCAGCAAAGTACTGAGGATCCGTGGACGTTCGGTCAAGGGACCAAGGTGG |

| | | | | |
|---|---|---|---|---|
| | | | | AGATCAAAGGTGGTGGTGGTTCTGGCGGCGGCGGCTCCGGTGGTGGTGGTTCTCAGGTGCAGCTG GTCCAGTCTGGGGCTGAGCTGAAGAAGCCTGGGTCCTCAGTGAAGATTTCCTGCAAGGCTTCTGG CTATTCATTCACTAGCTACTGGATGAACTGGGTGAAGCAGGCCCCTGGACAGGGTCTTGAGTGGA TGGGACAGATTTGGCCTGGAGATGGTGATACTAACTACAATGGAAAGTTCAAGGGTAAAGCCACT ATGACTGCTGACCCATCCACCAGCACAGCCTACATGGAACTCAGCAGCCTGGGATCTGAGGACAC TGCGGTCTATTACTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 511. | hCD19 46-B11 L | artificial | aa | DIQMTQSPASLSASVGERVTITCKASQSVDYDGDSYLNWYQQIPGKPPKLLIYDASNLVSGIPSR FSGSGSGTDFTLTIHSLEQEDFATYYCQQSTEDPWTFGQGTKVEIK |
| 512. | hCD19 46-B11 L | artificial | nt | GACATCCAGATGACCCAGTCTCCAGCTTCTTTGTCTGCGTCTGTAGGGGAAAGAGTCACCATCAC CTGCAAGGCCAGCCAAAGTGTTGATTATGATGGTGATAGTTATTTGAACTGGTACCAACAGATAC CAGGAAAGCCACCCAAACTCCTCATCTATGATGCATCCAATCTAGTTTCTGGGATCCCATCCAGG TTTAGTGGCAGTGGGTCTGGGACAGACTTCACCCTCACCATCCATTCTCTGGAGCAGGAGGATTT CGCAACCTATTACTGTCAGCAAAGTACTGAGGATCCGTGGACGTTCGGTCAAGGGACCAAGGTGG AGATCAAA |
| 513. | hCD19 46-B11 LCDR1 | artificial | aa | KASQSVDYDGDSYLN |
| 514. | hCD19 46-B11 LCDR2 | artificial | aa | DASNLVS |
| 515. | hCD19 46-B11 LCDR3 | artificial | aa | QQSTEDPWT |

| | | | | |
|---|---|---|---|---|
| 516. | hCD19 46-B11 H | artificial | aa | QVQLVQSGAEVVRPGASVKVSCKASGYSFTSYWMNWVRQPPGQGLEWIGQIWPGDGDTNYNGKFK<br>GRATMTADASTSTVYMELSSLRSEDTAVYYCARRETTTVGRYYYAMDYWGQGTTVTVSS |
| 517. | hCD19 46-B11 H | artificial | nt | CAGGTGCAGCTGGTCCAGTCTGGGGCTGAGGTGGTGAGGCCTGGGGCCTCAGTGAAGGTTTCCTG<br>CAAGGCTTCTGGCTATTCATTCACTAGCTACTGGATGAACTGGGTGAGGCAGCCCCCTGGACAGG<br>GTCTTGAGTGGATTGGACAGATTTGGCCTGGAGATGGTGATACTAACTACAATGGAAAGTTCAAG<br>GGTAGAGCCACTATGACTGCTGACGCATCCACCAGCACAGTCTACATGGAACTCAGCAGCCTGCG<br>ATCTGAGGACACTGCGGTCTATTACTGTGCAAGACGGGAGACTACGACGGTAGGCCGTTATTACT<br>ATGCTATGGACTACTGGGGCCAAGGGACCACGGTCACCGTCTCCTCC |
| 518. | hCD19 46-B11 HCDR1 | artificial | aa | SYWMN |
| 519. | hCD19 46-B11 HCDR2 | artificial | aa | QIWPGDGDTNYNGKFKG |
| 520. | hCD19 46-B11 HCDR3 | artificial | aa | RETTTVGRYYYAMDY |
| 521. | hCD19 46-B11LH x I2C HL | artificial | aa | DIQMTQSPASLSASVGERVTITCKASQSVDYDGDSYLNWYQQIPGKPPKLLIYDASNLVSGIPSR<br>FSGSGSGTDFTLTIHSLEQEDFATYYCQQSTEDPWTFGQGTKVEIKGGGGSGGGGSGGGGSQVQL<br>VQSGAEVVRPGASVKVSCKASGYSFTSYWMNWVRQPPGQGLEWIGQIWPGDGDTNYNGKFKGRAT<br>MTADASTSTVYMELSSLRSEDTAVYYCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 522. | hCD19 46-B11LH x I2C HL | artificial | nt | GACATCCAGATGACCCAGTCTCCAGCTTCTTTGTCTGCGTCTGTAGGGGAAAGAGTCACCATCAC<br>CTGCAAGGCCAGCCAAAGTGTTGATTATGATGGTGATAGTTATTTGAACTGGTACCAACAGATAC<br>CAGGAAAGCCACCCAAACTCCTCATCTATGATGCATCCAATCTAGTTTCTGGGATCCCATCCAGG<br>TTTAGTGGCAGTGGGTCTGGGACAGACTTCACCCTCACCATCCATTCTCTGGAGCAGGAGGATTT<br>CGCAACCTATTACTGTCAGCAAAGTACTGAGGATCCGTGGACGTTCGGTCAAGGGACCAAGGTGG<br>AGATCAAAGGTGGTGGTGGTTCTGGCGGCGGCGGCTCCGGTGGTGGTGGTTCTCAGGTGCAGCTG<br>GTCCAGTCTGGGGCTGAGGTGGTGAGGCCTGGGGCCTCAGTGAAGGTTTCCTGCAAGGCTTCTGG<br>CTATTCATTCACTAGCTACTGGATGAACTGGGTGAGGCAGCCCCCTGGACAGGGTCTTGAGTGGA<br>TTGGACAGATTTGGCCTGGAGATGGTGATACTAACTACAATGGAAAGTTCAAGGGTAGAGCCACT<br>ATGACTGCTGACGCATCCACCAGCACAGTCTACATGGAACTCAGCAGCCTGCGATCTGAGGACAC<br>TGCGGTCTATTACTGTGCAAGACGGGAGACTACGACGGTAGGCCGTTATTACTATGCTATGGACT |

| | | | | |
|---|---|---|---|---|
| | | | | ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 523. | hCD19 47-A3 L | artificial | aa | DIQMTQSPASLSASVGERVTITCKASQSVDYDGDSYLNWYQQIPGKAPKLLIYDASNLVSGVPSR<br>FSGSGSGTDFTLTISSLQTEDFATYYCQQSTEDPWTFGQGTKVEIK |
| 524. | hCD19 47-A3 L | artificial | nt | GACATCCAGATGACCCAGTCTCCAGCTTCTTTGTCTGCGTCTGTAGGGGAGAGAGTCACCATCAC<br>CTGCAAGGCCAGCCAAAGTGTTGATTATGATGGTGATAGTTATTTGAACTGGTACCAACAGATAC<br>CAGGAAAGGCACCCAAACTCCTCATCTATGATGCATCCAATCTAGTTTCTGGGGTCCCATCCAGG<br>TTTAGTGGCAGTGGGTCTGGGACAGACTTCACCCTCACCATCAGTTCTCTGCAGACGGAGGATTT<br>CGCAACCTATTACTGTCAGCAAAGTACTGAGGATCCGTGGACGTTCGGTCAAGGGACCAAGGTGG<br>AGATCAAA |
| 525. | hCD19 47-A3 LCDR1 | artificial | aa | KASQSVDYDGDSYLN |
| 526. | hCD19 47-A3 LCDR2 | artificial | aa | DASNLVS |
| 527. | hCD19 47-A3 LCDR3 | artificial | aa | QQSTEDPWT |
| 528. | hCD19 47-A3 H | artificial | aa | QVQLVQSGAEVKKPGASVKISCKASGYAFTSYWMNWVRQRPGQGLEWIGQIWPGDGDTNYNGKFK<br>GRVTMTADASTSTVYMELSSLGSEDTAVYFCARRETTTVGRYYYAMDYWGQGTTVTVSS |

| | | | | |
|---|---|---|---|---|
| 529. | hCD19 47-A3 H | artificial | nt | CAGGTGCAGCTGGTCCAGTCTGGGGCTGAGGTGAAGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGCATCCACCAGCACAGTCTACATGGAACTCAGCAGCCTGGGATCTGAGGACACTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 530. | hCD19 47-A3 HCDR1 | artificial | aa | SYWMN |
| 531. | hCD19 47-A3 HCDR2 | artificial | aa | QIWPGDGDTNYNGKFKG |
| 532. | hCD19 47-A3 HCDR3 | artificial | aa | RETTTVGRYYYAMDY |
| 533. | hCD19 47-A3 LH x I2C HL | artificial | aa | DIQMTQSPASLSASVGERVTITCKASQSVDYDGDSYLNWYQQIPGKAPKLLIYDASNLVSGVPSRFSGSGSGTDFTLTISSLQTEDFATYYCQQSTEDPWTFGQGTKVEIKGGGGSGGGGSGGGGSQVQLVQSGAEVKKPGASVKISCKASGYAFTSYWMNWVRQRPGQGLEWIGQIWPGDGDTNYNGKFKGRVTMTADASTSTVYMELSSLGSEDTAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 534. | hCD19 47-A3 LH x I2C HL | artificial | nt | GACATCCAGATGACCCAGTCTCCAGCTTCTTTGTCTGCGTCTGTAGGGGAGAGAGTCACCATCACCTGCAAGGCCAGCCAAAGTGTTGATTATGATGGTGATAGTTATTTGAACTGGTACCAACAGATACCAGGAAAGGCACCCAAACTCCTCATCTATGATGCATCCAATCTAGTTTCTGGGGTCCCATCCAGGTTTAGTGGCAGTGGGTCTGGGACAGACTTCACCCTCACCATCAGTTCTCTGCAGACGGAGGATTTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGAAGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGCATCCACCAGCACAGTCTACATGGAACTCAGCAGCCTGGGATCTGAGGACACTGCGGTCTATTTCTGTGCAAGACGGGAGACTACGACGGTAGGCCGTTATTACTATGCTATGGACTACTGGGGCCAAGGGACCACGGTCACCGTCTCCTCCGGAGGTGGTGGATCCGAGGTGCAGCTGGTC |

| | | | | |
|---|---|---|---|---|
| | | | | GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 535. | HD37-DT L | artificial | aa | DIQLTQSPASLAVSLGQRATISCKASQSVDYDGDTYLNWYQQIPGQPPKLLIYDASNLVSGIPPR<br>FSGSGSGTDFTLNIHPVEKVDAATYHCQQSTEDPWTFGGGTKLEIK |
| 536. | HD37-DT L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGATACTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |
| 537. | HD37-DT LCDR1 | artificial | aa | KASQSVDYDGDTYLN |
| 538. | HD37-DT LCDR2 | artificial | aa | DASNLVS |
| 539. | HD37-DT LCDR3 | artificial | aa | QQSTEDPWT |
| 540. | HD37-DT H | artificial | aa | QVQLQQSGAELVRPGSSVKISCKASGYAFSSYWMNWVKQRPGQGLEWIGQIWPGDGDTNYNGKFK<br>GKATLTADESSSTAYMQLSSLASEDSAVYFCARRETTTVGRYYYAMDYWGQGTTVTVSS |
| 541. | HD37-DT H | artificial | nt | CAGGTGCAGCTGCAGCAGTCTGGGGCTGAGCTGGTGAGGCCTGGGTCCTCAGTGAAGATTTCCTG<br>CAAGGCTTCTGGCTATGCATTCAGTAGCTACTGGATGAACTGGGTGAAGCAGAGGCCTGGACAGG<br>GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC |

| | | | | |
|---|---|---|---|---|
| | | | | ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 542. | HD37-DT HCDR1 | artificial | aa | SYWMN |
| 543. | HD37-DT HCDR2 | artificial | aa | QIWPGDGDTNYNGKFKG |
| 544. | HD37-DT HCDR3 | artificial | aa | RETTTVGRYYYAMDY |
| 545. | HD37-DT LH x I2C HL | artificial | aa | DIQLTQSPASLAVSLGQRATISCKASQSVDYDGDTYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 546. | HD37-DT LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTGATACTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC |

| | | | | |
|---|---|---|---|---|
| | | | | AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 547. | HD37-A L | artificial | aa | DIQLTQSPASLAVSLGQRATISCKASQSVDYDGASYLNWYQQIPGQPPKLLIYDASNLVSGIPPR FSGSGSGTDFTLNIHPVEKVDAATYHCQQSTEDPWTFGGGTKLEIK |
| 548. | HD37-A L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTGCTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 549. | HD37-A LCDR1 | artificial | aa | KASQSVDYDGASYLN |
| 550. | HD37-A LCDR2 | artificial | aa | DASNLVS |
| 551. | HD37-A LCDR3 | artificial | aa | QQSTEDPWT |
| 552. | HD37-A H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 553. | HD37-A H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 554. | HD37-A HCDR1 | artificial | aa | SYWMN |
| 555. | HD37-A HCDR2 | artificial | aa | QIWPGDGDTNYNGKFKG |

| | | | | |
|---|---|---|---|---|
| 556. | HD37-A HCDR3 | artificial | aa | RETTTVGRYYYAMDY |
| 557. | HD37-A LH x I2C HL | artificial | aa | DIQLTQSPASLAVSLGQRATISCKASQSVDYDGASYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 558. | HD37-A LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTGCTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |

| | | | | |
|---|---|---|---|---|
| 559. | HD37-G L | artificial | aa | DIQLTQSPASLAVSLGQRATISCKASQSVDYDGGSYLNWYQQIPGQPPKLLIYDASNLVSGIPPR FSGSGSGTDFTLNIHPVEKVDAATYHCQQSTEDPWTFGGGTKLEIK |
| 560. | HD37-G L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTGGTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 561. | HD37-G LCDR1 | artificial | aa | KASQSVDYDGGSYLN |
| 562. | HD37-G LCDR2 | artificial | aa | DASNLVS |
| 563. | HD37-G LCDR3 | artificial | aa | QQSTEDPWT |
| 564. | HD37-G H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 565. | HD37-G H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 566. | HD37-G HCDR1 | artificial | aa | SYWMN |
| 567. | HD37-G HCDR2 | artificial | aa | QIWPGDGDTNYNGKFKG |
| 568. | HD37-G HCDR3 | artificial | aa | RETTTVGRYYYAMDY |
| 569. | HD37-G LH x I2C HL | artificial | aa | DIQLTQSPASLAVSLGQRATISCKASQSVDYDGGSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV |

| | | | | |
|---|---|---|---|---|
| | | | | ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 570. | HD37-G LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTGGTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 571. | HD37-S L | artificial | aa | DIQLTQSPASLAVSLGQRATISCKASQSVDYDGSSYLNWYQQIPGQPPKLLIYDASNLVSGIPPR FSGSGSGTDFTLNIHPVEKVDAATYHCQQSTEDPWTFGGGTKLEIK |
| 572. | HD37-S L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTCTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC |

| | | | | |
|---|---|---|---|---|
| | | | | TGCAACCTATCACTGTCAGCAAAGTACTGAGGATCCGTGGACGTTCGGTGGAGGGACCAAGCTCG AGATCAAA |
| 573. | HD37-S LCDR1 | artificial | aa | KASQSVDYDGSSYLN |
| 574. | HD37-S LCDR2 | artificial | aa | DASNLVS |
| 575. | HD37-S LCDR3 | artificial | aa | QQSTEDPWT |
| 576. | HD37-S H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 577. | HD37-S H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 578. | HD37-S HCDR1 | artificial | aa | SYWMN |
| 579. | HD37-S HCDR2 | artificial | aa | QIWPGDGDTNYNGKFKG |
| 580. | HD37-S HCDR3 | artificial | aa | RETTTVGRYYYAMDY |
| 581. | HD37-S LH x I2C HL | artificial | aa | DIQLTQSPASLAVSLGQRATISCKASQSVDYDGSSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 582. | HD37-S LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTCTAGTTATTTGAACTGGTACCAACAGATTC |

| | | | | |
|---|---|---|---|---|
| | | | | CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 583. | HD37-T L | artificial | aa | DIQLTQSPASLAVSLGQRATISCKASQSVDYDGTSYLNWYQQIPGQPPKLLIYDASNLVSGIPPR<br>FSGSGSGTDFTLNIHPVEKVDAATYHCQQSTEDPWTFGGGTKLEIK |
| 584. | HD37-T L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTACAAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |
| 585. | HD37-T LCDR1 | artificial | aa | KASQSVDYDGTSYLN |
| 586. | HD37-T LCDR2 | artificial | aa | DASNLVS |

| | | | | |
|---|---|---|---|---|
| 587. | HD37-T LCDR3 | artificial | aa | QQSTEDPWT |
| 588. | HD37-T H | artificial | aa | QVQLQQSGAELVRPGSSVKISCKASGYAFSSYWMNWVKQRPGQGLEWIGQIWPGDGDTNYNGKFK<br>GKATLTADESSSTAYMQLSSLASEDSAVYFCARRETTTVGRYYYAMDYWGQGTTVTVSS |
| 589. | HD37-T H | artificial | nt | CAGGTGCAGCTGCAGCAGTCTGGGGCTGAGCTGGTGAGGCCTGGGTCCTCAGTGAAGATTTCCTG<br>CAAGGCTTCTGGCTATGCATTCAGTAGCTACTGGATGAACTGGGTGAAGCAGAGGCCTGGACAGG<br>GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC<br>ATCTGAGGACTCTGCGGTCTATTTCTGTGCAAGACGGGAGACTACGACGGTAGGCCGTTATTACT<br>ATGCTATGGACTACTGGGGCCAAGGGACCACGGTCACCGTCTCCTCC |
| 590. | HD37-T HCDR1 | artificial | aa | SYWMN |
| 591. | HD37-T HCDR2 | artificial | aa | QIWPGDGDTNYNGKFKG |
| 592. | HD37-T HCDR3 | artificial | aa | RETTTVGRYYYAMDY |
| 593. | HD37-T LH x I2C HL | artificial | aa | DIQLTQSPASLAVSLGQRATISCKASQSVDYDGTSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 594. | HD37-T LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTACAAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC |

| | | | | |
|---|---|---|---|---|
| | | | | TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 595. | HD37-I L | artificial | aa | DIQLTQSPASLAVSLGQRATISCKASQSVDYDGISYLNWYQQIPGQPPKLLIYDASNLVSGIPPR FSGSGSGTDFTLNIHPVEKVDAATYHCQQSTEDPWTFGGGTKLEIK |
| 596. | HD37-I L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTATCAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 597. | HD37-I LCDR1 | artificial | aa | KASQSVDYDGISYLN |
| 598. | HD37-I LCDR2 | artificial | aa | DASNLVS |
| 599. | HD37-I LCDR3 | artificial | aa | QQSTEDPWT |
| 600. | HD37-I H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 601. | HD37-I H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG |

| | | | | |
|---|---|---|---|---|
| | | | | GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC<br>ATCTGAGGACTCTGCGGTCTATTTCTGTGCAAGACGGGAGACTACGACGGTAGGCCGTTATTACT<br>ATGCTATGGACTACTGGGGCCAAGGGACCACGGTCACCGTCTCCTCC |
| 602. | HD37-I HCDR1 | artificial | aa | SYWMN |
| 603. | HD37-I HCDR2 | artificial | aa | QIWPGDGDTNYNGKFKG |
| 604. | HD37-I HCDR3 | artificial | aa | RETTTVGRYYYAMDY |
| 605. | HD37-I LH x I2C HL | artificial | aa | DIQLTQSPASLAVSLGQRATISCKASQSVDYDGISYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 606. | HD37-I LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTATCAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT |

| | | | | |
|---|---|---|---|---|
| | | | | ACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 607. | HD37-L L | artificial | aa | DIQLTQSPASLAVSLGQRATISCKASQSVDYDGLSYLNWYQQIPGQPPKLLIYDASNLVSGIPPRFSGSGSGTDFTLNIHPVEKVDAATYHCQQSTEDPWTFGGGTKLEIK |
| 608. | HD37-L L | artificial | nt | GATATCCAGCTGACCCAGTCTCCAGCTTCTTTGGCTGTGTCTCTAGGGCAGAGGGCCACCATCTCCTGCAAGGCCAGCCAAAGTGTTGATTATGATGGTCTGAGTTATTTGAACTGGTACCAACAGATTCCAGGACAGCCACCCAAACTCCTCATCTATGATGCATCCAATCTAGTTTCTGGGATCCCACCCAGGTTTAGTGGCAGTGGGTCTGGGACAGACTTCACCCTCAACATCCATCCTGTGGAGAAGGTGGATGCTGCAACCTATCACTGTCAGCAAAGTACTGAGGATCCGTGGACGTTCGGTGGAGGGACCAAGCTCGAGATCAAA |
| 609. | HD37-L LCDR1 | artificial | aa | KASQSVDYDGLSYLN |
| 610. | HD37-L LCDR2 | artificial | aa | DASNLVS |
| 611. | HD37-L LCDR3 | artificial | aa | QQSTEDPWT |
| 612. | HD37-L H | artificial | aa | QVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSS |
| 613. | HD37-L H | artificial | nt | CAGGTGCAGCTGCAGCAGTCTGGGGCTGAGCTGGTGAGGCCTGGGTCCTCAGTGAAGATTTCCTGCAAGGCTTCTGGCTATGCATTCAGTAGCTACTGGATGAACTGGGTGAAGCAGAGGCCTGGACAGGGTCTTGAGTGGATTGGACAGATTTGGCCTGGAGATGGTGATACTAACTACAATGGAAAGTTCAAGGGTAAAGCCACTCTGACTGCAGACGAATCCTCCAGCACAGCCTACATGCAACTCAGCAGCCTAGCATCTGAGGACTCTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 614. | HD37-L HCDR1 | artificial | aa | SYWMN |

| | | | | |
|---|---|---|---|---|
| 615. | HD37-L HCDR2 | artificial | aa | QIWPGDGDTNYNGKFKG |
| 616. | HD37-L HCDR3 | artificial | aa | RETTTVGRYYYAMDY |
| 617. | HD37-L LH x I2C HL | artificial | aa | DIQLTQSPASLAVSLGQRATISCKASQSVDYDGLSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 618. | HD37-L LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCTGAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |

| | | | | |
|---|---|---|---|---|
| 619. | HD37-V L | artificial | aa | DIQLTQSPASLAVSLGQRATISCKASQSVDYDGVSYLNWYQQIPGQPPKLLIYDASNLVSGIPPR<br>FSGSGSGTDFTLNIHPVEKVDAATYHCQQSTEDPWTFGGGTKLEIK |
| 620. | HD37-V L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGTCAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |
| 621. | HD37-V LCDR1 | artificial | aa | KASQSVDYDGVSYLN |
| 622. | HD37-V LCDR2 | artificial | aa | DASNLVS |
| 623. | HD37-V LCDR3 | artificial | aa | QQSTEDPWT |
| 624. | HD37-V H | artificial | aa | QVQLQQSGAELVRPGSSVKISCKASGYAFSSYWMNWVKQRPGQGLEWIGQIWPGDGDTNYNGKFK<br>GKATLTADESSSTAYMQLSSLASEDSAVYFCARRETTTVGRYYYAMDYWGQGTTVTVSS |
| 625. | HD37-V H | artificial | nt | CAGGTGCAGCTGCAGCAGTCTGGGGCTGAGCTGGTGAGGCCTGGGTCCTCAGTGAAGATTTCCTG<br>CAAGGCTTCTGGCTATGCATTCAGTAGCTACTGGATGAACTGGGTGAAGCAGAGGCCTGGACAGG<br>GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC<br>ATCTGAGGACTCTGCGGTCTATTTCTGTGCAAGACGGGAGACTACGACGGTAGGCCGTTATTACT<br>ATGCTATGGACTACTGGGGCCAAGGGACCACGGTCACCGTCTCCTCC |
| 626. | HD37-V HCDR1 | artificial | aa | SYWMN |
| 627. | HD37-V HCDR2 | artificial | aa | QIWPGDGDTNYNGKFKG |
| 628. | HD37-V HCDR3 | artificial | aa | RETTTVGRYYYAMDY |
| 629. | HD37-V LH x I2C HL | artificial | aa | DIQLTQSPASLAVSLGQRATISCKASQSVDYDGVSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL |

| | | | | |
|---|---|---|---|---|
| | | | | QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 630. | HD37-V LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGTCAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 631. | HD37-E L | artificial | aa | DIQLTQSPASLAVSLGQRATISCKASQSVDYDGESYLNWYQQIPGQPPKLLIYDASNLVSGIPPR<br>FSGSGSGTDFTLNIHPVEKVDAATYHCQQSTEDPWTFGGGTKLEIK |
| 632. | HD37-E L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGAGAGTTATTTGAACTGGTACCAACAGATTC |

| | | | | |
|---|---|---|---|---|
| | | | | CAGGACAGCCACCCAAACTCCTCATCTATGATGCATCCAATCTAGTTTCTGGGATCCCACCCAGGTTTAGTGGCAGTGGGTCTGGGACAGACTTCACCCTCAACATCCATCCTGTGGAGAAGGTGGATGCTGCAACCTATCACTGTCAGCAAAGTACTGAGGATCCGTGGACGTTCGGTGGAGGGACCAAGCTCGAGATCAAA |
| 633. | HD37-E LCDR1 | artificial | aa | KASQSVDYDGESYLN |
| 634. | HD37-E LCDR2 | artificial | aa | DASNLVS |
| 635. | HD37-E LCDR3 | artificial | aa | QQSTEDPWT |
| 636. | HD37-E H | artificial | aa | QVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSS |
| 637. | HD37-E H | artificial | nt | CAGGTGCAGCTGCAGCAGTCTGGGGCTGAGCTGGTGAGGCCTGGGTCCTCAGTGAAGATTTCCTGCAAGGCTTCTGGCTATGCATTCAGTAGCTACTGGATGAACTGGGTGAAGCAGAGGCCTGGACAGGGTCTTGAGTGGATTGGACAGATTTGGCCTGGAGATGGTGATACTAACTACAATGGAAAGTTCAAGGGTAAAGCCACTCTGACTGCAGACGAATCCTCCAGCACAGCCTACATGCAACTCAGCAGCCTAGCATCTGAGGACTCTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 638. | HD37-E HCDR1 | artificial | aa | SYWMN |
| 639. | HD37-E HCDR2 | artificial | aa | QIWPGDGDTNYNGKFKG |
| 640. | HD37-E HCDR3 | artificial | aa | RETTTVGRYYYAMDY |
| 641. | HD37-E LH x I2C HL | artificial | aa | DIQLTQSPASLAVSLGQRATISCKASQSVDYDGESYLNWYQQIPGQPPKLLIYDASNLVSGIPPRFSGSGSGTDFTLNIHPVEKVDAATYHCQQSTEDPWTFGGGTKLEIKGGGGSGGGGSGGGGSQVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |

| | | | | |
|---|---|---|---|---|
| 642. | HD37-E LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTGAGAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 643. | HD37-Q L | artificial | aa | DIQLTQSPASLAVSLGQRATISCKASQSVDYDGQSYLNWYQQIPGQPPKLLIYDASNLVSGIPPR<br>FSGSGSGTDFTLNIHPVEKVDAATYHCQQSTEDPWTFGGGTKLEIK |
| 644. | HD37-Q L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCAGAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |

| | | | | |
|---|---|---|---|---|
| 645. | HD37-Q LCDR1 | artificial | aa | KASQSVDYDGQSYLN |
| 646. | HD37-Q LCDR2 | artificial | aa | DASNLVS |
| 647. | HD37-Q LCDR3 | artificial | aa | QQSTEDPWT |
| 648. | HD37-Q H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 649. | HD37-Q H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 650. | HD37-Q HCDR1 | artificial | aa | SYWMN |
| 651. | HD37-Q HCDR2 | artificial | aa | QIWPGDGDTNYNGKFKG |
| 652. | HD37-Q HCDR3 | artificial | aa | RETTTVGRYYYAMDY |
| 653. | HD37-Q LH x I2C HL | artificial | aa | DIQLTQSPASLAVSLGQRATISCKASQSVDYDGQSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 654. | HD37-Q LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTCAGAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG |

| | | | | |
|---|---|---|---|---|
| | | | | AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 655. | HD37-N L | artificial | aa | DIQLTQSPASLAVSLGQRATISCKASQSVDYDGNSYLNWYQQIPGQPPKLLIYDASNLVSGIPPR FSGSGSGTDFTLNIHPVEKVDAATYHCQQSTEDPWTFGGGTKLEIK |
| 656. | HD37-N L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTAACAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 657. | HD37-N LCDR1 | artificial | aa | KASQSVDYDGNSYLN |
| 658. | HD37-N LCDR2 | artificial | aa | DASNLVS |
| 659. | HD37-N LCDR3 | artificial | aa | QQSTEDPWT |

| | | | | |
|---|---|---|---|---|
| 660. | HD37-N H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 661. | HD37-N H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 662. | HD37-N HCDR1 | artificial | aa | SYWMN |
| 663. | HD37-N HCDR2 | artificial | aa | QIWPGDGDTNYNGKFKG |
| 664. | HD37-N HCDR3 | artificial | aa | RETTTVGRYYYAMDY |
| 665. | HD37-N LH x I2C HL | artificial | aa | DIQLTQSPASLAVSLGQRATISCKASQSVDYDGNSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 666. | HD37-N LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTAACAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC |

| | | | | |
|---|---|---|---|---|
| | | | | GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 667. | HD37-K L | artificial | aa | DIQLTQSPASLAVSLGQRATISCKASQSVDYDGKSYLNWYQQIPGQPPKLLIYDASNLVSGIPPR FSGSGSGTDFTLNIHPVEKVDAATYHCQQSTEDPWTFGGGTKLEIK |
| 668. | HD37-K L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTAAGAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 669. | HD37-K LCDR1 | artificial | aa | KASQSVDYDGKSYLN |
| 670. | HD37-K LCDR2 | artificial | aa | DASNLVS |
| 671. | HD37-K LCDR3 | artificial | aa | QQSTEDPWT |
| 672. | HD37-K H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 673. | HD37-K H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT |

| | | | | |
|---|---|---|---|---|
| | | | | ATGCTATGGACTACTGGGGCCAAGGGACCACGGTCACCGTCTCCTCC |
| 674. | HD37-K HCDR1 | artificial | aa | SYWMN |
| 675. | HD37-K HCDR2 | artificial | aa | QIWPGDGDTNYNGKFKG |
| 676. | HD37-K HCDR3 | artificial | aa | RETTTVGRYYYAMDY |
| 677. | HD37-K LH x I2C HL | artificial | aa | DIQLTQSPASLAVSLGQRATISCKASQSVDYDGKSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 678. | HD37-K LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTAAGAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC |

| | | | | |
|---|---|---|---|---|
| | | | | AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 679. | HD37-R L | artificial | aa | DIQLTQSPASLAVSLGQRATISCKASQSVDYDGRSYLNWYQQIPGQPPKLLIYDASNLVSGIPPR FSGSGSGTDFTLNIHPVEKVDAATYHCQQSTEDPWTFGGGTKLEIK |
| 680. | HD37-R L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTCGTAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 681. | HD37-R LCDR1 | artificial | aa | KASQSVDYDGRSYLN |
| 682. | HD37-R LCDR2 | artificial | aa | DASNLVS |
| 683. | HD37-R LCDR3 | artificial | aa | QQSTEDPWT |
| 684. | HD37-R H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 685. | HD37-R H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 686. | HD37-R HCDR1 | artificial | aa | SYWMN |
| 687. | HD37-R HCDR2 | artificial | aa | QIWPGDGDTNYNGKFKG |
| 688. | HD37-R HCDR3 | artificial | aa | RETTTVGRYYYAMDY |

| | | | | |
|---|---|---|---|---|
| 689. | HD37-R LH x I2C HL | artificial | aa | DIQLTQSPASLAVSLGQRATISCKASQSVDYDGRSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 690. | HD37-R LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCGTAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 691. | HD37-H L | artificial | aa | DIQLTQSPASLAVSLGQRATISCKASQSVDYDGHSYLNWYQQIPGQPPKLLIYDASNLVSGIPPR<br>FSGSGSGTDFTLNIHPVEKVDAATYHCQQSTEDPWTFGGGTKLEIK |

| | | | | |
|---|---|---|---|---|
| 692. | HD37-H L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCACAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |
| 693. | HD37-H LCDR1 | artificial | aa | KASQSVDYDGHSYLN |
| 694. | HD37-H LCDR2 | artificial | aa | DASNLVS |
| 695. | HD37-H LCDR3 | artificial | aa | QQSTEDPWT |
| 696. | HD37-H H | artificial | aa | QVQLQQSGAELVRPGSSVKISCKASGYAFSSYWMNWVKQRPGQGLEWIGQIWPGDGDTNYNGKFK<br>GKATLTADESSSTAYMQLSSLASEDSAVYFCARRETTTVGRYYYAMDYWGQGTTVTVSS |
| 697. | HD37-H H | artificial | nt | CAGGTGCAGCTGCAGCAGTCTGGGGCTGAGCTGGTGAGGCCTGGGTCCTCAGTGAAGATTTCCTG<br>CAAGGCTTCTGGCTATGCATTCAGTAGCTACTGGATGAACTGGGTGAAGCAGAGGCCTGGACAGG<br>GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC<br>ATCTGAGGACTCTGCGGTCTATTTCTGTGCAAGACGGGAGACTACGACGGTAGGCCGTTATTACT<br>ATGCTATGGACTACTGGGGCCAAGGGACCACGGTCACCGTCTCCTCC |
| 698. | HD37-H HCDR1 | artificial | aa | SYWMN |
| 699. | HD37-H HCDR2 | artificial | aa | QIWPGDGDTNYNGKFKG |
| 700. | HD37-H HCDR3 | artificial | aa | RETTTVGRYYYAMDY |
| 701. | HD37-H LH x I2C HL | artificial | aa | DIQLTQSPASLAVSLGQRATISCKASQSVDYDGHSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS |

| | | | | |
|---|---|---|---|---|
| | | | | GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 702. | HD37-H LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTCACAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 703. | HD37-Y L | artificial | aa | DIQLTQSPASLAVSLGQRATISCKASQSVDYDGYSYLNWYQQIPGQPPKLLIYDASNLVSGIPPR FSGSGSGTDFTLNIHPVEKVDAATYHCQQSTEDPWTFGGGTKLEIK |
| 704. | HD37-Y L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTACAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |

| | | | | |
|---|---|---|---|---|
| 705. | HD37-Y LCDR1 | artificial | aa | KASQSVDYDGYSYLN |
| 706. | HD37-Y LCDR2 | artificial | aa | DASNLVS |
| 707. | HD37-Y LCDR3 | artificial | aa | QQSTEDPWT |
| 708. | HD37-Y H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 709. | HD37-Y H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 710. | HD37-Y HCDR1 | artificial | aa | SYWMN |
| 711. | HD37-Y HCDR2 | artificial | aa | QIWPGDGDTNYNGKFKG |
| 712. | HD37-Y HCDR3 | artificial | aa | RETTTVGRYYYAMDY |
| 713. | HD37-Y LH x I2C HL | artificial | aa | DIQLTQSPASLAVSLGQRATISCKASQSVDYDGYSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 714. | HD37-Y LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTACAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC |

| | | | | |
|---|---|---|---|---|
| | | | | TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 715. | HD37-P L | artificial | aa | DIQLTQSPASLAVSLGQRATISCKASQSVDYDGPSYLNWYQQIPGQPPKLLIYDASNLVSGIPPR<br>FSGSGSGTDFTLNIHPVEKVDAATYHCQQSTEDPWTFGGGTKLEIK |
| 716. | HD37-P L | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCCAAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAA |
| 717. | HD37-P LCDR1 | artificial | aa | KASQSVDYDGPSYLN |
| 718. | HD37-P LCDR2 | artificial | aa | DASNLVS |
| 719. | HD37-P LCDR3 | artificial | aa | QQSTEDPWT |

| | | | | |
|---|---|---|---|---|
| 720. | HD37-P H | artificial | aa | QVQLQQSGAELVRPGSSVKISCKASGYAFSSYWMNWVKQRPGQGLEWIGQIWPGDGDTNYNGKFK<br>GKATLTADESSSTAYMQLSSLASEDSAVYFCARRETTTVGRYYYAMDYWGQGTTVTVSS |
| 721. | HD37-P H | artificial | nt | CAGGTGCAGCTGCAGCAGTCTGGGGCTGAGCTGGTGAGGCCTGGGTCCTCAGTGAAGATTTCCTG<br>CAAGGCTTCTGGCTATGCATTCAGTAGCTACTGGATGAACTGGGTGAAGCAGAGGCCTGGACAGG<br>GTCTTGAGTGGATTGGACAGATTTGGCCTGGAGATGGTGATACTAACTACAATGGAAAGTTCAAG<br>GGTAAAGCCACTCTGACTGCAGACGAATCCTCCAGCACAGCCTACATGCAACTCAGCAGCCTAGC<br>ATCTGAGGACTCTGCGGTCTATTTCTGTGCAAGACGGGAGACTACGACGGTAGGCCGTTATTACT<br>ATGCTATGGACTACTGGGGCCAAGGGACCACGGTCACCGTCTCCTCC |
| 722. | HD37-P HCDR1 | artificial | aa | SYWMN |
| 723. | HD37-P HCDR2 | artificial | aa | QIWPGDGDTNYNGKFKG |
| 724. | HD37-P HCDR3 | artificial | aa | RETTTVGRYYYAMDY |
| 725. | HD37-P LH x I2C HL | artificial | aa | DIQLTQSPASLAVSLGQRATISCKASQSVDYDGPSYLNWYQQIPGQPPKLLIYDASNLVSGIPPR<br>FSGSGSGTDFTLNIHPVEKVDAATYHCQQSTEDPWTFGGGTKLEIKGGGGSGGGGSGGGGSQVQL<br>QQSGAELVRPGSSVKISCKASGYAFSSYWMNWVKQRPGQGLEWIGQIWPGDGDTNYNGKFKGKAT<br>LTADESSSTAYMQLSSLASEDSAVYFCARRETTTVGRYYYAMDYWGQGTTVTVSSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 726. | HD37-P LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTCCAAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC |

| | | | | |
|---|---|---|---|---|
| | | | | GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 727. | HD37-F L | artificial | aa | DIQLTQSPASLAVSLGQRATISCKASQSVDYDGFSYLNWYQQIPGQPPKLLIYDASNLVSGIPPR FSGSGSGTDFTLNIHPVEKVDAATYHCQQSTEDPWTFGGGTKLEIK |
| 728. | HD37-F L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTTCAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 729. | HD37-F LCDR1 | artificial | aa | KASQSVDYDGFSYLN |
| 730. | HD37-F LCDR2 | artificial | aa | DASNLVS |
| 731. | HD37-F LCDR3 | artificial | aa | QQSTEDPWT |
| 732. | HD37-F H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 733. | HD37-F H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT |

| | | | | |
|---|---|---|---|---|
| | | | | ATGCTATGGACTACTGGGGCCAAGGGACCACGGTCACCGTCTCCTCC |
| 734. | HD37-F HCDR1 | artificial | aa | SYWMN |
| 735. | HD37-F HCDR2 | artificial | aa | QIWPGDGDTNYNGKFKG |
| 736. | HD37-F HCDR3 | artificial | aa | RETTTVGRYYYAMDY |
| 737. | HD37-F LH x I2C HL | artificial | aa | DIQLTQSPASLAVSLGQRATISCKASQSVDYDGFSYLNWYQQIPGQPPKLLIYDASNLVSGIPPR FSGSGSGTDFTLNIHPVEKVDAATYHCQQSTEDPWTFGGGTKLEIKGGGGSGGGGSGGGGSQVQL QQSGAELVRPGSSVKISCKASGYAFSSYWMNWVKQRPGQGLEWIGQIWPGDGDTNYNGKFKGKAT LTADESSSTAYMQLSSLASEDSAVYFCARRETTTVGRYYYAMDYWGQGTTVTVSSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 738. | HD37-F LH x I2C HL | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTTCAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAAGGTGGTGGTGGTTCTGGCGGCGGCGGCTCCGGTGGTGGTGGTTCTCAGGTGCAGCTG CAGCAGTCTGGGGCTGAGCTGGTGAGGCCTGGGTCCTCAGTGAAGATTTCCTGCAAGGCTTCTGG CTATGCATTCAGTAGCTACTGGATGAACTGGGTGAAGCAGAGGCCTGGACAGGGTCTTGAGTGGA TTGGACAGATTTGGCCTGGAGATGGTGATACTAACTACAATGGAAAGTTCAAGGGTAAAGCCACT CTGACTGCAGACGAATCCTCCAGCACAGCCTACATGCAACTCAGCAGCCTAGCATCTGAGGACTC TGCGGTCTATTTCTGTGCAAGACGGGAGACTACGACGGTAGGCCGTTATTACTATGCTATGGACT ACTGGGGCCAAGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGGAATGGGTTG CTCGCATAAGAAGTAAATATAATAATTATGCAACATATTATGCCGATTCAGTGAAAGACAGGTTC ACCATCTCCAGAGATGATTCAAAAAACACTGCCTATCTACAAATGAACAACTTGAAAACTGAGGA CACTGCCGTGTACTACTGTGTGAGACATGGGAACTTCGGTAATAGCTACATATCCTACTGGGCTT ACTGGGGCCAAGGGACTCTGGTCACCGTCTCCTCAGGTGGTGGTGGTTCTGGCGGCGGCGGCTCC GGTGGTGGTGGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAAACTGGGTCC |

| | | | | |
|---|---|---|---|---|
| | | | | AACAAAAACCAGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAGGGGTACAGCC AGAGGATGAGGCAGAATATTACTGTGTTCTATGGTACAGCAACCGCTGGGTGTTCGGTGGAGGAA CCAAACTGACTGTCCTA |
| 739. | HD37-W L | artificial | aa | DIQLTQSPASLAVSLGQRATISCKASQSVDYDGWSYLNWYQQIPGQPPKLLIYDASNLVSGIPPR FSGSGSGTDFTLNIHPVEKVDAATYHCQQSTEDPWTFGGGTKLEIK |
| 740. | HD37-W L | artificial | nt | GATATCCAGCTGACCCAGTCTCCAGCTTCTTTGGCTGTGTCTCTAGGGCAGAGGGCCACCATCTC CTGCAAGGCCAGCCAAAGTGTTGATTATGATGGTTGGAGTTATTTGAACTGGTACCAACAGATTC CAGGACAGCCACCCAAACTCCTCATCTATGATGCATCCAATCTAGTTTCTGGGATCCCACCCAGG TTTAGTGGCAGTGGGTCTGGGACAGACTTCACCCTCAACATCCATCCTGTGGAGAAGGTGGATGC TGCAACCTATCACTGTCAGCAAAGTACTGAGGATCCGTGGACGTTCGGTGGAGGGACCAAGCTCG AGATCAAA |
| 741. | HD37-W LCDR1 | artificial | aa | KASQSVDYDGWSYLN |
| 742. | HD37-W LCDR2 | artificial | aa | DASNLVS |
| 743. | HD37-W LCDR3 | artificial | aa | QQSTEDPWT |
| 744. | HD37-W H | artificial | aa | QVQLQQSGAELVRPGSSVKISCKASGYAFSSYWMNWVKQRPGQGLEWIGQIWPGDGDTNYNGKFK GKATLTADESSSTAYMQLSSLASEDSAVYFCARRETTTVGRYYYAMDYWGQGTTVTVSS |
| 745. | HD37-W H | artificial | nt | CAGGTGCAGCTGCAGCAGTCTGGGGCTGAGCTGGTGAGGCCTGGGTCCTCAGTGAAGATTTCCTG CAAGGCTTCTGGCTATGCATTCAGTAGCTACTGGATGAACTGGGTGAAGCAGAGGCCTGGACAGG GTCTTGAGTGGATTGGACAGATTTGGCCTGGAGATGGTGATACTAACTACAATGGAAAGTTCAAG GGTAAAGCCACTCTGACTGCAGACGAATCCTCCAGCACAGCCTACATGCAACTCAGCAGCCTAGC ATCTGAGGACTCTGCGGTCTATTTCTGTGCAAGACGGGAGACTACGACGGTAGGCCGTTATTACT ATGCTATGGACTACTGGGGCCAAGGGACCACGGTCACCGTCTCCTCC |
| 746. | HD37-W HCDR1 | artificial | aa | SYWMN |
| 747. | HD37-W HCDR2 | artificial | aa | QIWPGDGDTNYNGKFKG |
| 748. | HD37-W HCDR3 | artificial | aa | RETTTVGRYYYAMDY |

| | | | | |
|---|---|---|---|---|
| 749. | HD37-W LH x I2C HL | artificial | aa | DIQLTQSPASLAVSLGQRATISCKASQSVDYDGWSYLNWYQQIPGQPPKLLIYDASNLVSGIPPRFSGSGSGTDFTLNIHPVEKVDAATYHCQQSTEDPWTFGGGTKLEIKGGGGSGGGGSGGGGSQVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 750. | HD37-W LH x I2C HL | artificial | nt | GATATCCAGCTGACCCAGTCTCCAGCTTCTTTGGCTGTGTCTCTAGGGCAGAGGGCCACCATCTCCTGCAAGGCCAGCCAAAGTGTTGATTATGATGGTTGGAGTTATTTGAACTGGTACCAACAGATTCCAGGACAGCCACCCAAACTCCTCATCTATGATGCATCCAATCTAGTTTCTGGGATCCCACCCAGGTTTAGTGGCAGTGGGTCTGGGACAGACTTCACCCTCAACATCCATCCTGTGGAGAAGGTGGATGCTGCAACCTATCACTGTCAGCAAAGTACTGAGGATCCGTGGACGTTCGGTGGAGGGACCAAGCTCGAGATCAAAGGTGGTGGTGGTTCTGGCGGCGGCGGCTCCGGTGGTGGTGGTTCTCAGGTGCAGCTGCAGCAGTCTGGGGCTGAGCTGGTGAGGCCTGGGTCCTCAGTGAAGATTTCCTGCAAGGCTTCTGGCTATGCATTCAGTAGCTACTGGATGAACTGGGTGAAGCAGAGGCCTGGACAGGGTCTTGAGTGGATTGGACAGATTTGGCCTGGAGATGGTGATACTAACTACAATGGAAAGTTCAAGGGTAAAGCCACTCTGACTGCAGACGAATCCTCCAGCACAGCCTACATGCAACTCAGCAGCCTAGCATCTGAGGACTCTGCGGTCTATTTCTGTGCAAGACGGGAGACTACGACGGTAGGCCGTTATTACTATGCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 751. | HD37-M L | artificial | aa | DIQLTQSPASLAVSLGQRATISCKASQSVDYDGMSYLNWYQQIPGQPPKLLIYDASNLVSGIPPRFSGSGSGTDFTLNIHPVEKVDAATYHCQQSTEDPWTFGGGTKLEIK |

| | | | | |
|---|---|---|---|---|
| 752. | HD37-M L | artificial | nt | GATATCCAGCTGACCCAGTCTCCAGCTTCTTTGGCTGTGTCTCTAGGGCAGAGGGCCACCATCTCCTGCAAGGCCAGCCAAAGTGTTGATTATGATGGTATGAGTTATTTGAACTGGTACCAACAGATTCCAGGACAGCCACCCAAACTCCTCATCTATGATGCATCCAATCTAGTTTCTGGGATCCCACCCAGGTTTAGTGGCAGTGGGTCTGGGACAGACTTCACCCTCAACATCCATCCTGTGGAGAAGGTGGATGCTGCAACCTATCACTGTCAGCAAAGTACTGAGGATCCGTGGACGTTCGGTGGAGGGACCAAGCTCGAGATCAAA |
| 753. | HD37-M LCDR1 | artificial | aa | KASQSVDYDGMSYLN |
| 754. | HD37-M LCDR2 | artificial | aa | DASNLVS |
| 755. | HD37-M LCDR3 | artificial | aa | QQSTEDPWT |
| 756. | HD37-M H | artificial | aa | QVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSS |
| 757. | HD37-M H | artificial | nt | CAGGTGCAGCTGCAGCAGTCTGGGGCTGAGCTGGTGAGGCCTGGGTCCTCAGTGAAGATTTCCTGCAAGGCTTCTGGCTATGCATTCAGTAGCTACTGGATGAACTGGGTGAAGCAGAGGCCTGGACAGGGTCTTGAGTGGATTGGACAGATTTGGCCTGGAGATGGTGATACTAACTACAATGGAAAGTTCAAGGGTAAAGCCACTCTGACTGCAGACGAATCCTCCAGCACAGCCTACATGCAACTCAGCAGCCTAGCATCTGAGGACTCTGCGGTCTATTTCTGTGCAAGACGGGAGACTACGACGGTAGGCCGTTATTACTATGCTATGGACTACTGGGGCCAAGGGACCACGGTCACCGTCTCCTCC |
| 758. | HD37-M HCDR1 | artificial | aa | SYWMN |
| 759. | HD37-M HCDR2 | artificial | aa | QIWPGDGDTNYNGKFKG |
| 760. | HD37-M HCDR3 | artificial | aa | RETTTVGRYYYAMDY |
| 761. | HD37-M LH x I2C HL | artificial | aa | DIQLTQSPASLAVSLGQRATISCKASQSVDYDGMSYLNWYQQIPGQPPKLLIYDASNLVSGIPPRFSGSGSGTDFTLNIHPVEKVDAATYHCQQSTEDPWTFGGGTKLEIKGGGGSGGGGSGGGGSQVQLQQSGAELVRPGSSVKISCKASGYAFSSYWMNWVKQRPGQGLEWIGQIWPGDGDTNYNGKFKGKATLTADESSSTAYMQLSSLASEDSAVYFCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGS |

| | | | | |
|---|---|---|---|---|
| | | | | GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 762. | HD37-M LH x I2C HL | artificial | nt | GATATCCAGCTGACCCAGTCTCCAGCTTCTTTGGCTGTGTCTCTAGGGCAGAGGGCCACCATCTC<br>CTGCAAGGCCAGCCAAAGTGTTGATTATGATGGTATGAGTTATTTGAACTGGTACCAACAGATTC<br>CAGGACAGCCACCCAAACTCCTCATCTATGATGCATCCAATCTAGTTTCTGGGATCCCACCCAGG<br>TTTAGTGGCAGTGGGTCTGGGACAGACTTCACCCTCAACATCCATCCTGTGGAGAAGGTGGATGC<br>TGCAACCTATCACTGTCAGCAAAGTACTGAGGATCCGTGGACGTTCGGTGGAGGGACCAAGCTCG<br>AGATCAAAGGTGGTGGTGGTTCTGGCGGCGGCGGCTCCGGTGGTGGTGGTTCTCAGGTGCAGCTG<br>CAGCAGTCTGGGGCTGAGCTGGTGAGGCCTGGGTCCTCAGTGAAGATTTCCTGCAAGGCTTCTGG<br>CTATGCATTCAGTAGCTACTGGATGAACTGGGTGAAGCAGAGGCCTGGACAGGGTCTTGAGTGGA<br>TTGGACAGATTTGGCCTGGAGATGGTGATACTAACTACAATGGAAAGTTCAAGGGTAAAGCCACT<br>CTGACTGCAGACGAATCCTCCAGCACAGCCTACATGCAACTCAGCAGCCTAGCATCTGAGGACTC<br>TGCGGTCTATTTCTGTGCAAGACGGGAGACTACGACGGTAGGCCGTTATTACTATGCTATGGACT<br>ACTGGGGCCAAGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAGCCTCTGGATT<br>CACCTTCAATAAGTACGCCATGAACTGGGTCCGCCAGGCTCCAGGAAAGGGTTTGGAATGGGTTG<br>CTCGCATAAGAAGTAAATATAATAATTATGCAACATATTATGCCGATTCAGTGAAAGACAGGTTC<br>ACCATCTCCAGAGATGATTCAAAAAACACTGCCTATCTACAAATGAACAACTTGAAAACTGAGGA<br>CACTGCCGTGTACTACTGTGTGAGACATGGGAACTTCGGTAATAGCTACATATCCTACTGGGCTT<br>ACTGGGGCCAAGGGACTCTGGTCACCGTCTCCTCAGGTGGTGGTGGTTCTGGCGGCGGCGGCTCC<br>GGTGGTGGTGGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAAACTGGGTCC<br>AACAAAAACCAGGTCAGGCACCCCGTGGTCTAATAGGTGGGACTAAGTTCCTCGCCCCCGGTACT<br>CCTGCCAGATTCTCAGGCTCCCTGCTTGGAGGCAAGGCTGCCCTCACCCTCTCAGGGGTACAGCC<br>AGAGGATGAGGCAGAATATTACTGTGTTCTATGGTACAGCAACCGCTGGGTGTTCGGTGGAGGAA<br>CCAAACTGACTGTCCTA |
| 763. | 37VH-aXho1 | artificial | nt | CAGCTGCTCGAGTCTGGGGCTGAGSTGRWGARGCCTGGGKCCTCAGTGAAGRTTTCCTGCAAGGC<br>TTCTGGC |
| 764. | 37VH-b | artificial | nt | CCACTCAAGACCCTGTCCAGGGSSCTGCYTCACCCAGTTCATCCAGTAGCTAGWGAATGYATAGC<br>CAGAAGCCTTGCAGGA |
| 765. | 37VH-c | artificial | nt | GGACAGGGTCTTGAGTGGATKGGACAGATTTGGCCTGGAGATGGTGATACTAACTACAATGGAAA<br>GTTCAAG |

| | | | | |
|---|---|---|---|---|
| 766. | 37VH-d | artificial | nt | CAGGCTGCTGAGTTSCATGTAGRCTGTGCTGGTGGATKYGTCASSAGTCAKAGTGRCTYTACCCT TGAACTTTCCATTGTAG |
| 767. | 37VH-e | artificial | nt | CATGSAACTCAGCAGCCTGSSATCTGAGGACACTGCGGTCTATTWCTGTGCAAGACGGGAGACTA CGACGG |
| 768. | 37VH-fBstE2 | artificial | nt | GGAGACGGTGACCGTGGTCCCTTGGCCCCAGTAGTCCATAGCATAGTAATAACGGCCTACCGTCG TAGTCTCCCGTCTTGC |
| 769. | 37VL-A-Sacl | artificial | nt | CAGCTGGAGCTCCAGATGACCCAGTCTCCAKCTTCTTTGKCTGYGTCTSTAGGGSASAGAGYCAC CATCWCCTGCAAGGCCAGC |
| 770. | 37VL-B | artificial | nt | CTGTTGGTACCAGTTCAAATAASTSNNACCATCATAATCAACACTTTGGCTGGCCTTGCAGGTGA TGGT |
| 771. | 37VL-C | artificial | nt | TTGAACTGGTACCAACAGAWACCAGGAMAGSCACCCAAACTCCTCATCTATGATGCATCCAATCT AGTTTCT |
| 772. | 37VL-D | artificial | nt | GAGGGTGAAGTCTGTCCCAGACCCACTGCCACTAAACCTGGRTGGGAYCCCAGAAACTAGATTGG ATGC |
| 773. | 37VL-E | artificial | nt | GGGACAGACTTCACCCTCAMCATCMRTYCTSTGSAGMMGGWGGATKYCGCAACCTATYACTGTCA GCAAAGTACTGAG |
| 774. | 37VL-F-BsiW1Spe1 | artificial | nt | ACTCAGACTAGTCGTACGTTTGATCTCCACCTTGGTCCCTTGACCGAACGTCCACGGATCCTCAG TACTTTGCTGACAG |
| 775. | Flag-tag | artificial | aa | DYKDDDDK |
| 776. | human cMET construct | artificial | nt | ATGAAGGCCCCCGCTGTGCTTGCACCTGGCATCCTCGTGCTCCTGTTTACCTTGGTGCAGAGGAG CAATGGGGAGTGTAAAGAGGCACTAGCAAAGTCCGAGATGAATGTGAATATGAAGTATCAGCTTC CCAACTTCACCGCGGAAACACCCATCCAGAATGTCATTCTACATGAGCATCACATTTTCCTTGGT GCCACTAACTACATTTATGTTTTAAATGAGGAAGACCTTCAGAAGGTTGCTGAGTACAAGACTGG GCCTGTGCTGGAACACCCAGATTGTTTCCCATGTCAGGACTGCAGCAGCAAAGCCAATTTATCAG GAGGTGTTTGGAAAGATAACATCAACATGGCTCTAGTTGTGGACACCTACTATGATGATCAACTC ATTAGCTGTGGCAGCGTCAACAGAGGGACCTGCCAGCGACATGTCTTTCCCCACAATCATACTGC TGACATACAGTCGGAGGTTCACTGCATATTCTCCCCACAGATAGAAGAGCCCAGCCAGTGTCCTG |

ACTGTGTGGTGAGCGCCCTGGGAGCCAAAGTCCTTTCATCTGTAAAGGACCGGTTCATCAACTTC
TTTGTAGGCAATACCATAAATTCTTCTTATTTCCCAGATCATCCATTGCATTCGATATCAGTGAG
AAGGCTAAAGGAAACGAAAGATGGTTTTATGTTTTTGACGGACCAGTCCTACATTGATGTTTTAC
CTGAGTTCAGAGATTCTTACCCCATTAAGTATGTCCATGCCTTTGAAAGCAACAATTTTATTTAC
TTCTTGACGGTCCAAAGGGAAACTCTAGATGCTCAGACTTTTCACACAAGAATAATCAGGTTCTG
TTCCATAAACTCTGGATTGCATTCCTACATGGAAATGCCTCTGGAGTGTATTCTCACAGAAAAGA
GAAAAAAGAGATCCACAAAGAAGGAAGTGTTTAATATACTTCAGGCTGCGTATGTCAGCAAGCCT
GGGGCCCAGCTTGCTAGACAAATAGGAGCCAGCCTGAATGATGACATTCTTTTCGGGGTGTTCGC
ACAAAGCAAGCCAGATTCTGCCGAACCAATGGATCGATCTGCCATGTGTGCATTCCCTATCAAAT
ATGTCAACGACTTCTTCAACAAGATCGTCAACAAAAACAATGTGAGATGTCTCCAGCATTTTTAC
GGACCCAATCATGAGCACTGCTTTAATAGGACACTTCTGAGAAATTCATCAGGCTGTGAAGCGCG
CCGTGATGAATATCGAACAGAGTTTACCACAGCTTTGCAGCGCGTTGACTTATTCATGGGTCAAT
TCAGCGAAGTCCTCTTAACATCTATATCCACCTTCATTAAAGGAGACCTCACCATAGCTAATCTT
GGGACATCAGAGGGTCGCTTCATGCAGGTTGTGGTTTCTCGATCAGGACCATCAACCCCTCATGT
GAATTTTCTCCTGGACTCCCATCCAGTGTCTCCAGAAGTGATTGTGGAGCATACATTAAACCAAA
ATGGCTACACACTGGTTATCACTGGGAAGAAGATCACGAAGATCCCATTGAATGGCTTGGGCTGC
AGACATTTCCAGTCCTGCAGTCAATGCCTCTCTGCCCCACCCTTTGTTCAGTGTGGCTGGTGCCA
CGACAAATGTGTGCGATCGGAGGAATGCCTGAGCGGGACATGGACTCAACAGATCTGTCTGCCTG
CAATCTACAAGGTTTTCCCAAATAGTGCACCCCTTGAAGGAGGGACAAGGCTGACCATATGTGGC
TGGGACTTTGGATTTCGGAGGAATAATAAATTTGATTTAAAGAAAACTAGAGTTCTCCTTGGAAA
TGAGAGCTGCACCTTGACTTTAAGTGAGAGCACGATGAATACATTGAAATGCACAGTTGGTCCTG
CCATGAATAAGCATTTCAATATGTCCATAATTATTTCAAATGGCCACGGGACAACACAATACAGT
ACATTCTCCTATGTGGATCCTGTAATAACAAGTATTTCGCCGAAATACGGTCCTATGGCTGGTGG
CACTTTACTTACTTTAACTGGAAATTACCTAAACAGTGGGAATAGCAGACACATTTCAATTGGTG
GAAAAACATGTACTTTAAAAAGTGTGTCAAACAGTATTCTTGAATGTTATACCCCAGCCCAAACC
ATTTCAACTGAGTTTGCTGTTAAATTGAAAATTGACTTAGCCAACCGAGAGACAAGCATCTTCAG
TTACCGTGAAGATCCCATTGTCTATGAAATTCATCCAACCAAATCTTTTATTAGTGGTGGGAGCA
CAATAACAGGTGTTGGGAAAAACCTGAACTCAGTTAGTGTCCCGAGAATGGTCATAAATGTGCAT
GAAGCAGGAAGGAACTTTACAGTGGCATGTCAACATCGCTCTAATTCAGAGATAATCTGTTGTAC
CACTCCTTCCCTGCAACAGCTGAATCTGCAACTCCCCCTGAAAACCAAAGCCTTTTTCATGTTAG
ATGGGATCCTTTCCAAATACTTTGATCTCATTTATGTACATAATCCTGTGTTTAAGCCTTTTGAA
AAGCCAGTGATGATCTCAATGGGCAATGAAAATGTACTGGAAATTAAGGGAAATGATATTGACCC
TGAAGCAGTTAAAGGTGAAGTGTTAAAAGTTGGAAATAAGAGCTGTGAGAATATACACTTACATT
CTGAAGCCGTTTTATGCACGGTCCCCAATGACCTGCTGAAATTGAACAGCGAGCTAAATATAGAG
TGGAAGCAAGCAATTTCTTCAACCGTCCTTGGAAAAGTAATAGTTCAACCAGATCAGAATTTCAC
AGGATTGATTGCTGGTGTTGTCTCAATATCAACAGCACTGTTATTACTACTTGGGTTTTTCCTGT
GGCTGAAAAAGAGAAAGCAAATTAAAGATCTGGGCAGTGAATTAGTTCGCTACGATGCAAGAGTA

| | | | | |
|---|---|---|---|---|
| | | | | CACACTCCTCATTTGGATAGGCTTGTAAGTGCCCGAAGTGTAAGCCCAACTACAGAAATGGTTTC<br>AAATGAATCTGTAGACTACCGAGCTACTTTTCCAGAAGATCAGTTTCCTAATTCATCTCAGAACG<br>GTTCATGCCGACAAGTGCAGTATCCTCTGACAGACATGTCCCCCATCCTAACTAGTGGGGACTCT<br>GATATATCCAGTCCATTACTGCAAAATACTGTCCACATTGACCTCAGTGCTCTAAATCCAGAGCT<br>GGTCCAGGCAGTGCAGCATGTAGTGATTGGGCCCAGTAGCCTGATTGTGCATTTCAATGAAGTCA<br>TAGGAAGAGGGCATTTTGGTTGTGTATATCATGGGACTTTGTTGGACAATGATGGCAAGAAAATT<br>CACTGTGCTGTGAAATCCTTGAACAGAATCACTGACATAGGAGAAGTTTCCCAATTTCTGACCGA<br>GGGAATCATCATGAAAGATTTTAGTCATCCCAATGTCCTCTCGCTCCTGGGAATCTGCCTGCGAA<br>GTGAAGGGTCTCCGCTGGTGGTCCTACCATACATGAAACATGGAGATCTTCGAAATTTCATTCGA<br>AATGAGACTCATAATCCAACTGTAAAAGATCTTATTGGCTTTGGTCTTCAAGTAGCCAAAGGCAT<br>GAAATATCTTGCAAGCAAAAAGTTTGTCCACAGAGACTTGGCTGCAAGAAACTGTATGCTGGATG<br>AAAAATTCACAGTCAAGGTTGCTGATTTTGGTCTTGCCAGAGACATGTATGATAAAGAATACTAT<br>AGTGTACACAACAAAACAGGTGCAAAGCTGCCAGTGAAGTGGATGGCTTTGGAAAGTCTGCAAAC<br>TCAAAAGTTTACCACCAAGTCAGATGTGTGGTCCTTTGGCGTGCTCCTCTGGGAGCTGATGACAA<br>GAGGAGCCCCACCTTATCCTGACGTAAACACCTTTGATATAACTGTTTACTTGTTGCAAGGGAGA<br>AGACTCCTACAACCCGAATACTGCCCAGACCCCTTATATGAAGTAATGCTAAAATGCTGGCACCC<br>TAAAGCCGAAATGCGCCCATCCTTTTCTGAACTGGTGTCCCGGATATCAGCGATCTTCTCTACTT<br>TCATTGGGGAGCACTATGTCCATGTGAACGCTACTTATGTGAACGTAAAATGTGTCGCTCCGTAT<br>CCTTCTCTGTTGTCATCAGAAGATAACGCTGATGATGAGGTGGACACACGACCAGCCTCCTTCTG<br>GGAGACATCATAG |
| 777. | human cMET construct | artificial | aa | MKAPAVLAPGILVLLFTLVQRSNGECKEALAKSEMNVNMKYQLPNFTAETPIQNVILHEHHIFLG<br>ATNYIYVLNEEDLQKVAEYKTGPVLEHPDCFPCQDCSSKANLSGGVWKDNINMALVVDTYYDDQL<br>ISCGSVNRGTCQRHVFPHNHTADIQSEVHCIFSPQIEEPSQCPDCVVSALGAKVLSSVKDRFINF<br>FVGNTINSSYFPDHPLHSISVRRLKETKDGFMFLTDQSYIDVLPEFRDSYPIKYVHAFESNNFIY<br>FLTVQRETLDAQTFHTRIIRFCSINSGLHSYMEMPLECILTEKRKKRSTKKEVFNILQAAYVSKP<br>GAQLARQIGASLNDDILFGVFAQSKPDSAEPMDRSAMCAFPIKYVNDFFNKIVNKNNVRCLQHFY<br>GPNHEHCFNRTLLRNSSGCEARRDEYRTEFTTALQRVDLFMGQFSEVLLTSISTFIKGDLTIANL<br>GTSEGRFMQVVVSRSGPSTPHVNFLLDSHPVSPEVIVEHTLNQNGYTLVITGKKITKIPLNGLGC<br>RHFQSCSQCLSAPPFVQCGWCHDKCVRSEECLSGTWTQQICLPAIYKVFPNSAPLEGGTRLTICG<br>WDFGFRRNNKFDLKKTRVLLGNESCTLTLSESTMNTLKCTVGPAMNKHFNMSIIISNGHGTTQYS<br>TFSYVDPVITSISPKYGPMAGGTLLTLTGNYLNSGNSRHISIGGKTCTLKSVSNSILECYTPAQT<br>ISTEFAVKLKIDLANRETSIFSYREDPIVYEIHPTKSFISGGSTITGVGKNLNSVSVPRMVINVH<br>EAGRNFTVACQHRSNSEIICCTTPSLQQLNLQLPLKTKAFFMLDGILSKYFDLIYVHNPVFKPFE<br>KPVMISMGNENVLEIKGNDIDPEAVKGEVLKVGNKSCENIHLHSEAVLCTVPNDLLKLNSELNIE<br>WKQAISSTVLGKVIVQPDQNFTGLIAGVVSISTALLLLLGFFLWLKKRKQIKDLGSELVRYDARV<br>HTPHLDRLVSARSVSPTTEMVSNESVDYRATFPEDQFPNSSQNGSCRQVQYPLTDMSPILTSGDS<br>DISSPLLQNTVHIDLSALNPELVQAVQHVVIGPSSLIVHFNEVIGRGHFGCVYHGTLLDNDGKKI |

| | | | | |
|---|---|---|---|---|
| | | | | HCAVKSLNRITDIGEVSQFLTEGIIMKDFSHPNVLSLLGICLRSEGSPLVVLPYMKHGDLRNFIR NETHNPTVKDLIGFGLQVAKGMKYLASKKFVHRDLAARNCMLDEKFTVKVADFGLARDMYDKEYY SVHNKTGAKLPVKWMALESLQTQKFTTKSDVWSFGVLLWELMTRGAPPYPDVNTFDITVYLLQGR RLLQPEYCPDPLYEVMLKCWHPKAEMRPSFSELVSRISAIFSTFIGEHYVHVNATYVNVKCVAPY PSLLSSEDNADDEVDTRPASFWETS |
| 778. | forward primer | artificial | nt | AGGAATTCACCATGAAGGCCCCCGCTGTGCTTGCACC |
| 779. | reverse primer | artificial | nt | CTCCAGAGGCATTTCCATGTAGG |
| 780. | forward primer | artificial | nt | GTCCAAAGGGAAACTCTAGATGC |
| 781. | reverse primer | artificial | nt | GGAGACACTGGATGGGAGTCCAGG |
| 782. | forward primer | artificial | nt | CATCAGAGGGTCGCTTCATGCAGG |
| 783. | reverse primer | artificial | nt | GCTTTGGTTTTCAGGGGGAGTTGC |
| 784. | forward primer | artificial | nt | ATCCAACCAAATCTTTTATTAGTGGTGG |
| 785. | reverse primer | artificial | nt | GACTTCATTGAAATGCACAATCAGG |
| 786. | forward primer | artificial | nt | TGCTCTAAATCCAGAGCTGGTCC |
| 787. | reverse primer | artificial | nt | GTCAGATAAGAAATTCCTTAGAATCC |
| 788. | macaque cMET construct | artificial | nt | ATGAAGGCCCCCGCTGTGCTTGCACCTGGCATCCTCGTGCTCCTGTTTACCTTGGTGCAGAGGAG CAATGGGGAGTGTAAAGAGGCACTAGCAAAGTCCGAGATGAATGTGAATATGAAGTATCAGCTTC CCAACTTCACCGCGGAAACAGCCATCCAGAATGTCATTCTACATGAGCATCACATTTTCCTCGGT GCCACCAACTACATTTATGTTTTAAATGAGGAAGACCTTCAGAAGGTTGCTGAGTACAAGACCGG GCCTGTGCTGGAACACCCAGATTGTTTCCCGTGTCAGGACTGCAGCAGCAAAGCCAATTTATCAG GAGGCGTTTGGAAAGATAACATCAACATGGCTCTGGTTGTGGACACCTACTATGATGATCAACTC ATTAGCTGTGGCAGCGTCAACAGAGGGACCTGCCAGCGACATGTCTTTCCCCATAATCATACTGC TGACATACAGTCGGAGGTTCACTGCATATTCTCCCCACAGATAGAAGAGCCCAACCAGTGCCCTG ACTGTGTGGTGAGCGCCCTGGGAGCCAAAGTCCTTTCATCTGTAAAGGACCGGTTCATCAACTTC TTTGTAGGCAATACCATAAATTCTTCTTATTTCCCACATCATCCGTTGCATTCGATATCAGTGAG AAGGCTAAAGGAAACGAAAGATGGTTTTATGTTTTTGACAGACCAGTCCTACATTGATGTTTTAC CTGAGTTCAGAGATTCTTACCCCATTAAGTACATCCACGCTTTTGAAAGCAACAATTTTATTTAC TTCTTGACGGTCCAAAGGGAAACTCTAAATGCTCAGACTTTTCACACAAGAATAATCAGGTTCTG TTCCTTAAACTCTGGATTGCACTCCTACATGGAAATGCCTCTAGAGTGTATTCTCACAGAAAAGA GAAAAAAGAGATCCACAAAGAAGGAAGTGTTTAATATCCTTCAGGCTGCATATGTCAGCAAGCCC |

| | | | | |
|---|---|---|---|---|
| GGGGCCCAGCTTGCTAGACAAATAGGAGCCAGCCTGAATGATGACATTCTCTTTGGGGTGTTCGC<br>ACAAAGCAAGCCAGATTCTGCCGAACCAATGGATCGATCTGCAATGTGTGCATTTCCTATCAAAT<br>ATGTCAACGACTTCTTCAACAAGATCGTCAACAAAAACAATGTGAGATGTCTCCAGCATTTTTAT<br>GGACCCAATCATGAGCACTGTTTTAATAGGACACTTCTGAGAAATTCATCAGGCTGTGAAGCGCG<br>CCGTGATGAATATCGAGCAGAGTTTACCACAGCTTTGCAGCGGGTTGACTTATTCATGGGTCAAT<br>TCAGCGAAGTCCTCTTAACATCTATATCCACCTTCGTGAAAGGAGACCTCACCATCGCTAATCTT<br>GGGACATCAGAGGGTCGCTTCATGCAGGTTGTGGTTTCTCGATCAGGACCATCAACCCCTCATGT<br>GAATTTTCTCCTGGACTCTCACCCGGTGTCTCCAGAAGTGATTGTGGAGCATCCATTAAACCAGA<br>ATGGCTACACACTGGTTGTCACGGGGAAGAAGATCACCAAGATCCCATTGAATGGCTTGGCTGC<br>AGACATTTCCAGTCCTGCAGTCAGTGCCTCTCTGCCCCACCCTTTGTGCAGTGTGGCTGGTGCCA<br>CGACAAATGTGTGCGATCGGAGGAATGCCCCAGTGGGACATGGACTCAACAGATCTGTCTGCCTG<br>CAATCTACAAGGTTTTCCCAACTAGTGCACCCCTTGAAGGAGGGACAAGGCTGACCATATGTGGC<br>TGGGACTTTGGATTTCGGAGGAATAATAAATTTGATTTAAAGAAAACTAGAGTTCTCCTTGGAAA<br>TGAGAGCTGCACCTTGACTTTAAGTGAGAGCACGATGAATACATTGAAATGCACAGTTGGTCCTG<br>CCATGAATAAACATTTCAATATGTCCATAATTATTTCAAATGGCCACGGGACAACACAATACAGT<br>ACATTTTCCTATGTGGATCCTATAATAACAAGTATTTCGCCAAAATATGGCCCTATGGCTGGTGG<br>CACTTTACTTACTTTAACTGGAAATTACCTAAACAGTGGCAATTCTAGACACATTTCAATTGGTG<br>GAAAAACATGTACTTTAAAAAGTGTGTCAAACAGTATTCTCGAATGTTATACCCCAGCCCAAACC<br>ATTTCAACTGAGTTTGCTGTTAAATTGAAAATTGACTTAGCCAACCGAGAGACAAGCATCTTCAG<br>TTACCGGGAAGACCCCATTGTCTATGAAATTCATCCAACCAAATCTTTTATTAGTGGTGGAGCA<br>CAATAACAGGTGTTGGGAAAAACCTGCATTCAGTTAGTGTCCCGAGGATGGTCATAAATGTGCAT<br>GAAGCAGGAAGGAACTTTACAGTGGCATGTCAGCATCGCTCTAATTCAGAGATAATCTGCTGTAC<br>CACTCCTTCCCTGCAACAGCTGAATCTGCAACTCCCCCTGAAAACCAAAGCCTTTTTCATGTTAG<br>ATGGGATCCTTTCCAAATACTTTGATCTCATTTATGTACATAATCCTGTGTTTAAGCCTTTTGAA<br>AAGCCAGTAATGATCTCAATGGGCAATGAAAATGTACTGGAAATTAAGGGAAATGATATTGACCC<br>TGAAGCAGTTAAAGGTGAAGTGTTAAAAGTTGGAAATAAGAGCTGTGAGAATATACACTTACATT<br>CTGAAGCCGTTTTATGCACGGTCCCCAATGACCTGCTGAAATTGAACAGCGAGCTAAATATAGAG<br>TGGAAGCAAGCAATTTCTTCAACCGTCCTTGGAAAAGTAATAGTTCAACCAGATCAGAATTTCAC<br>CGGATTGATTGCTGGTGTTGTCTCAATATCAATAGCACTCTTACTACTACTTGGGCTTTTCCTGT<br>GGCTGAAAAAGAGAAAGCAAATTAAAGATCTGGGCAGTGAATTAGTTCGCTATGATGCAAGAGTA<br>CACACTCCTCATTTGGATAGGCTTGTAAGTGCCCGAAGTGTAAGCCCAACAACAGAAATGGTTTC<br>AAATGAATCTGTAGACTACCGAGCTACTTTTCCAGAAGATCAGTTTCCTAATTCATCTCAGAACG<br>GTTCATGCCGACAAGTGCAGTATCCTCTGACAGACATGTCCCCCATCCTGACTAGTGGGGACTCT<br>GATATATCCAGTCCATTACTGCAAAACACTGTCCACATTGACCTCAGCGCTCTAAATCCAGAGCT<br>GGTCCAGGCAGTGCAGCATGTAGTGATTGGGCCCAGTAGCCTGATTGTGCATTTCAATGAAGTCA<br>TAGGAAGAGGGCATTTTGGTTGTGTATATCATGGGACTTTGTTGGACAATGATGGCAAGAAAATT<br>CACTGTGCTGTGAAATCCTTGAACAGAATCACTGACATAGGAGAAGTTTCCCAGTTTCTGACCGA | | | | |

| | | | | |
|---|---|---|---|---|
| | | | | GGGAATCATCATGAAAGATTTTAGTCATCCCAATGTCCTCTCGCTCCTGGGAATCTGCCTGCGAAGTGAAGGGTCTCCGCTGGTGGTCCTACCCTACATGAAACATGGAGATCTTCGAAATTTCATTCGAAATGAGACTCATAATCCAACTGTAAAAGATCTTATTGGCTTTGGTCTTCAAGTAGCCAAAGGCATGAAATATCTTGCAAGCAAAAAGTTTGTCCACAGAGACTTGGCTGCAAGAAACTGTATGCTGGATGAAAAATTCACAGTCAAGGTTGCTGATTTTGGTCTTGCCAGAGACATGTATGATAAAGAATACTATAGTGTGCACAACAAAACAGGTGCAAAGCTGCCAGTGAAGTGGATGGCTTTGGAAAGTCTGCAAACTCAAAAGTTTACCACCAAGTCAGATGTGTGGTCCTTTGGTGTGCTCCTCTGGGAGCTCATGACAAGAGGAGCCCCACCCTATCCTGATGTGAACACCTTTGATATAACTGTTTACTTGTTGCAAGGGAGAAGGCTCCTACAACCCGAATACTGCCCAGACCCCTTATATGAAGTAATGCTAAAATGCTGGCACCCTAAAGCAGAAATGCGCCCATCCTTTTCTGAACTGGTGTCCCGGATATCAGCGATCTTCTCTACTTTCATTGGGGAGCACTACGTCCATGTAAACGCTACTTATGTGAACGTAAAATGTGTCGCTCCATATCCTTCTCTGTTGTCATCAGAAGATAACGCTGATGACGAGGTGGACACATGA |
| 789. | macaque cMET construct | artificial | aa | MKAPAVLAPGILVLLFTLVQRSNGECKEALAKSEMNVNMKYQLPNFTAETAIQNVILHEHHIFLGATNYIYVLNEEDLQKVAEYKTGPVLEHPDCFPCQDCSSKANLSGGVWKDNINMALVVDTYYDDQLISCGSVNRGTCQRHVFPHNHTADIQSEVHCIFSPQIEEPNQCPDCVVSALGAKVLSSVKDRFINFFVGNTINSSYFPHHPLHSISVRRLKETKDGFMFLTDQSYIDVLPEFRDSYPIKYIHAFESNNFIYFLTVQRETLNAQTFHTRIIRFCSLNSGLHSYMEMPLECILTEKRKKRSTKKEVFNILQAAYVSKPGAQLARQIGASLNDDILFGVFAQSKPDSAEPMDRSAMCAFPIKYVNDFFNKIVNKNNVRCLQHFYGPNHEHCFNRTLLRNSSGCEARRDEYRAEFTTALQRVDLFMGQFSEVLLTSISTFVKGDLTIANLGTSEGRFMQVVVSRSGPSTPHVNFLLDSHPVSPEVIVEHPLNQNGYTLVVTGKKITKIPLNGLGCRHFQSCSQCLSAPPFVQCGWCHDKCVRSEECPSGTWTQQICLPAIYKVFPTSAPLEGGTRLTICGWDFGFRRNNKFDLKKTRVLLGNESCTLTLSESTMNTLKCTVGPAMNKHFNMSIIISNGHGTTQYSTFSYVDPIITSISPKYGPMAGGTLLTLTGNYLNSGNSRHISIGGKTCTLKSVSNSILECYTPAQTISTEFAVKLKIDLANRETSIFSYREDPIVYEIHPTKSFISGGSTITGVGKNLHSVSVPRMVINVHEAGRNFTVACQHRSNSEIICCTTPSLQQLNLQLPLKTKAFFMLDGILSKYFDLIYVHNPVFKPFEKPVMISMGNENVLEIKGNDIDPEAVKGEVLKVGNKSCENIHLHSEAVLCTVPNDLLKLNSELNIEWKQAISSTVLGKVIVQPDQNFTGLIAGVVSISIALLLLLGLFLWLKKRKQIKDLGSELVRYDARVHTPHLDRLVSARSVSPTTEMVSNESVDYRATFPEDQFPNSSQNGSCRQVQYPLTDMSPILTSGDSDISSPLLQNTVHIDLSALNPELVQAVQHVVIGPSSLIVHFNEVIGRGHFGCVYHGTLLDNDGKKIHCAVKSLNRITDIGEVSQFLTEGIIMKDFSHPNVLSLLGICLRSEGSPLVVLPYMKHGDLRNFIRNETHNPTVKDLIGFGLQVAKGMKYLASKKFVHRDLAARNCMLDEKFTVKVADFGLARDMYDKEYYSVHNKTGAKLPVKWMALESLQTQKFTTKSDVWSFGVLLWELMTRGAPPYPDVNTFDITVYLLQGRRLLQPEYCPDPLYEVMLKCWHPKAEMRPSFSELVSRISAIFSTFIGEHYVHVNATYVNVKCVAPYPSLLSSEDNADDEVDT |
| 790. | CM1-VH-A-XhoI | artificial | nt | CCATGTCTCGAGTCTGGGSCTGAASTGRWGARGCCTGGGGCTTCAGTGAAARTGTCCTGCARGGCTTCGGGCTATACCTTC |

| | | | | |
|---|---|---|---|---|
| 791. | CM1-VH-B | artificial | nt | ATCCACTCAAGCCYTTGTCCAGGCSYCTGTYTAACCCAGTGCAACCAGTAGCTGGTGAAGGTATAGCCCGAAGC |
| 792. | CM1-VH-C | artificial | nt | GGCTTGAGTGGATKGGCATGATTGATCCTTCCAATAGTGACACTAGGTTTAATCCGAACTTCAAGGAC |
| 793. | CM1-VH-D | artificial | nt | GCTGCTGAGCWSCATGTAGGCTGTGYTGGMAGATSTGTCTMYAKTSAWTGTGRCCYTGTCCTTGAAGTTCGGATT |
| 794. | CM1-VH-E | artificial | nt | GCCTACATGSWACTCAGCAGCCTGASATCTGMGGACACTGCAGTCTATTACTGTGCCASATATGGTAGCTACGTT |
| 795. | CM1-VH-F-BstEII | artificial | nt | CATGTAGGTGACCGAGGTTCCTTGACCCCAGTAGTCCAGAGGGGAAACGTAGCTACCATA |
| 796. | CM3-VH-A-XhoI | artificial | nt | CCATGTCTCGAGTCTGGGRCTGAASTGRWGAAGCCTGGGGCTTCAGTGAAGSTGTCCTGCAAGGCTTCT |
| 797. | CM3-VH-B | artificial | nt | CTCAAGGCCTTGTCCAGGCSYCTGCYTCACCCAGTGTATCCAGTAACTGGTGAAGGTGTAGCCAGAAGCCTTGCAGGA |
| 798. | CM3-VH-C | artificial | nt | CCTGGACAAGGCCTTGAGTGGATKGGAGAGATTAATCCTAGCAGCGGTCGTACTAACTACAACGAGAAATTC |
| 799. | CM3-VH-D | artificial | nt | CTGTGGAGGTAGATKTGTCTMYAGTCAYTGTGACCYTGTTCTTGAATTTCTCGTTGTAGTT |
| 800. | CM3-VH-E | artificial | nt | ATCTACCTCCACAGYCTACATGSAACTCAGCARCCTGASATCTGAGGACACTGCGGTCTATTACTGTGCA |
| 801. | CM3-VH-F-BstEII | artificial | nt | CATGTAGGTGACCGTGGTGCCTTGGCCCCAGTAGCCCCTWCTTGCACAGTAATAGACCGC |
| 802. | CM1-VL-A-SacI | artificial | nt | CCTGTAGAGCTCGTGATGACCCAGACTCCAYYCTCCCTAMCTGTGACASYTGGAGAGMMGGYTTCTRTCAGCTGCAAGTCCAGT |
| 803. | CM1-VL-B | artificial | nt | GTACCAGGCCAAGTAGTTCTTCTGACTGCTAGTATATAAAAGGGACTGACTGGACTTGCAGCTGA |
| 804. | CM1-VL-C | artificial | nt | TACTTGGCCTGGTACCWGCAGAAACCAGGTCAGTCTCCTMAACTGCTGATTTACTGGGCATCCACTAGG |
| 805. | CM1-VL-D | artificial | nt | GAGAGTGAAATCTGTCCCAGATCCACTGCCTGAGAAGCGATCAGGGACCCCAGATTCCCTAGTGGATGCCCAGTA |
| 806. | CM1-VL-E | artificial | nt | ACAGATTTCACTCTCAMAATCTCCAGWGTGRAGGCTGASGACSTGGSAGTTTATTACTGTCAGCAATAT |
| 807. | CM1-VL-F-BsiWI/SpeI | artificial | nt | CCTCAGACTAGTCGTACGTTTGATCTCCAACTTTGTGCCTCCACCGAACGTCCACGGATAGGCATAATATTGCTGACAGTAATA |
| 808. | CM3-VL-A-SacI | artificial | nt | CCTGTAGAGCTCGTGATGACCCAATCTCCASYTTCTTTGSCTGTGACTCTAGGGCAGCSGGCCTCCATCTCCTGC |
| 809. | CM3-VL-Ba | artificial | nt | GAACCAACTCATATAACTACCACCATCATAATCAACACTTTGGCTGGCCTTGCAGGAGATGGAGGC |

| | | | | |
|---|---|---|---|---|
| 810. | CM3-VL-Bb | artificial | nt | GAACCAACTCATATAACTACCACCATCATAATCAACACTAGATTGGCTGGCCTTGCAGGAGATGGAGGC |
| 811. | CM3-VL-C | artificial | nt | AGTTATATGAGTTGGTTCCAACAGAGACCAGGACAGYCACCCARACKCCTCATCTMTGCTGCATCCAATCTA |
| 812. | CM3-VL-D | artificial | nt | GGTGAAGTCTGTCCCAGAGCCACTGCCACTAAACCTGKCTGGGAYCCCAGATTCTAGATTGGATGCAGC |
| 813. | CM3-VL-E | artificial | nt | TCTGGGACAGACTTCACCCTCAAKATCYMTCSTGTGGAGGMGGAGGATGTTGSARYCTATTACTGTCAGCAAAGT |
| 814. | CM3-VL-F-BsiWI/SpeI | artificial | nt | CCTCAGACTAGTCGTACGTTTGATCTCCAGCTTGGTCCCCTGACCGAACGTGAGCGGATCCTCATAACTTTGCTGACAGTAATA |
| 815. | MET-1 L | artificial | aa | DIMMSQSPSSLTVSVGEKVTVSCKSSQSLLYTSSQKNYLAWYQQKPGQSPKLLIYWASTRESGVPDRFTGSGSGTDFTLTITSVKADDLAVYYCQQYYAYPWTFGGGTKLEIK |
| 816. | MET-1 L-CDR1 | artificial | aa | KSSQSLLYTSSQKNYLA |
| 817. | MET-1 L-CDR2 | artificial | aa | WASTRES |
| 818. | MET-1 L-CDR3 | artificial | aa | QQYYAYPWT |
| 819. | MET-1 L | artificial | nt | GATATTATGATGAGTCAGTCCCCCAGCAGCCTGACCGTGTCCGTGGGCGAGAAGGTGACCGTGTCTTGCAAGAGCAGCCAGAGCCTGCTGTACACCAGCAGCCAGAAGAACTATCTGGCTTGGTATCAGCAGAAGCCCGGCCAGAGCCCAAAGCTGCTGATCTACTGGGCCAGCACCCGGGAGAGCGGAGTGCCCGACAGATTCACCGGCAGCGGCTCCGGCACAGACTTCACCCTGACCATCACCAGCGTGAAGGCCGACGACCTGGCCGTGTACTACTGCCAGCAGTACTACGCCTACCCCTGGACCTTCGGCGGAGGGACCAAGCTGGAGATCAAG |
| 820. | MET-1 H | artificial | aa | QVQLQQSGPELVRPGASVKMSCRASGYTFTSYWLHWVKQRPGQGLEWIGMIDPSNSDTRFNPNFKDKATLNVDRSSNTAYMLLSSLTSADSAVYYCATYGSYVSPLDYWGQGTSVTVSS |
| 821. | MET-1 H-CDR1 | artificial | aa | SYWLH |
| 822. | MET-1 H-CDR2 | artificial | aa | MIDPSNSDTRFNPNFKD |
| 823. | MET-1 H-CDR3 | artificial | aa | YGSYVSPLDY |
| 824. | MET-1 H | artificial | nt | CAGGTGCAGCTGCAGCAGAGCGGACCCGAGCTGGTGCGGCCAGGCGCCTCCGTGAAGATGAGCTGCAGGGCCAGCGGCTACACCTTCACCAGCTACTGGCTGCACTGGGTGAAACAGAGACCTGGACAGGGACTGGAGTGGATCGGCATGATCGACCCCAGCAACAGCGACACCAGGTTCAACCCCAACTTCAAG |

| | | | | |
|---|---|---|---|---|
| | | | | GACAAGGCCACCCTGAACGTGGACAGGTCCAGCAACACCGCCTACATGCTGCTGTCCAGCCTGAC<br>CTCTGCCGACAGCGCCGTGTATTACTGTGCCACCTACGGCAGCTACGTGTCCCCCCTGGACTACT<br>GGGGCCAGGGCACCTCTGTGACAGTGTCCTCC |
| 825. | MET-1 HL | artificial | aa | QVQLQQSGPELVRPGASVKMSCRASGYTFTSYWLHWVKQRPGQGLEWIGMIDPSNSDTRFNPNFK<br>DKATLNVDRSSNTAYMLLSSLTSADSAVYYCATYGSYVSPLDYWGQGTSVTVSSGGGGSGGGGSG<br>GGGSDIMMSQSPSSLTVSVGEKVTVSCKSSQSLLYTSSQKNYLAWYQQKPGQSPKLLIYWASTRE<br>SGVPDRFTGSGSGTDFTLTITSVKADDLAVYYCQQYYAYPWTFGGGTKLEIK |
| 826. | MET-1 HL | artificial | nt | CAGGTGCAGCTGCAGCAGAGCGGACCCGAGCTGGTGCGGCCAGGCGCCTCCGTGAAGATGAGCTG<br>CAGGGCCAGCGGCTACACCTTCACCAGCTACTGGCTGCACTGGGTGAAACAGAGACCTGGACAGG<br>GACTGGAGTGGATCGGCATGATCGACCCCAGCAACAGCGACACCAGGTTCAACCCCAACTTCAAG<br>GACAAGGCCACCCTGAACGTGGACAGGTCCAGCAACACCGCCTACATGCTGCTGTCCAGCCTGAC<br>CTCTGCCGACAGCGCCGTGTATTACTGTGCCACCTACGGCAGCTACGTGTCCCCCCTGGACTACT<br>GGGGCCAGGGCACCTCTGTGACAGTGTCCTCCGGTGGTGGTGGTTCTGGCGGCGGCGGCTCCGGT<br>GGTGGTGGTTCTGATATTATGATGAGTCAGTCCCCCAGCAGCCTGACCGTGTCCGTGGGCGAGAA<br>GGTGACCGTGTCTTGCAAGAGCAGCCAGAGCCTGCTGTACACCAGCAGCCAGAAGAACTATCTGG<br>CTTGGTATCAGCAGAAGCCCGGCCAGAGCCCAAAGCTGCTGATCTACTGGGCCAGCACCCGGGAG<br>AGCGGAGTGCCCGACAGATTCACCGGCAGCGGCTCCGGCACAGACTTCACCCTGACCATCACCAG<br>CGTGAAGGCCGACGACCTGGCCGTGTACTACTGCCAGCAGTACTACGCCTACCCCTGGACCTTCG<br>GCGGAGGGACCAAGCTGGAGATCAAG |
| 827. | MET-1 LH | artificial | aa | DIMMSQSPSSLTVSVGEKVTVSCKSSQSLLYTSSQKNYLAWYQQKPGQSPKLLIYWASTRESGVP<br>DRFTGSGSGTDFTLTITSVKADDLAVYYCQQYYAYPWTFGGGTKLEIKGGGGSGGGGSGGGGSQV<br>QLQQSGPELVRPGASVKMSCRASGYTFTSYWLHWVKQRPGQGLEWIGMIDPSNSDTRFNPNFKDK<br>ATLNVDRSSNTAYMLLSSLTSADSAVYYCATYGSYVSPLDYWGQGTSVTVSS |
| 828. | MET-1 LH | artificial | nt | GATATTATGATGAGTCAGTCCCCCAGCAGCCTGACCGTGTCCGTGGGCGAGAAGGTGACCGTGTC<br>TTGCAAGAGCAGCCAGAGCCTGCTGTACACCAGCAGCCAGAAGAACTATCTGGCTTGGTATCAGC<br>AGAAGCCCGGCCAGAGCCCAAAGCTGCTGATCTACTGGGCCAGCACCCGGGAGAGCGGAGTGCCC<br>GACAGATTCACCGGCAGCGGCTCCGGCACAGACTTCACCCTGACCATCACCAGCGTGAAGGCCGA<br>CGACCTGGCCGTGTACTACTGCCAGCAGTACTACGCCTACCCCTGGACCTTCGGCGGAGGGACCA<br>AGCTGGAGATCAAGGGTGGTGGTGGTTCTGGCGGCGGCGGCTCCGGTGGTGGTGGTTCTCAGGTG<br>CAGCTGCAGCAGAGCGGACCCGAGCTGGTGCGGCCAGGCGCCTCCGTGAAGATGAGCTGCAGGGC<br>CAGCGGCTACACCTTCACCAGCTACTGGCTGCACTGGGTGAAACAGAGACCTGGACAGGGACTGG<br>AGTGGATCGGCATGATCGACCCCAGCAACAGCGACACCAGGTTCAACCCCAACTTCAAGGACAAG |

| | | | | |
|---|---|---|---|---|
| | | | | GCCACCCTGAACGTGGACAGGTCCAGCAACACCGCCTACATGCTGCTGTCCAGCCTGACCTCTGC<br>CGACAGCGCCGTGTATTACTGTGCCACCTACGGCAGCTACGTGTCCCCCCTGGACTACTGGGGCC<br>AGGGCACCTCTGTGACAGTGTCCTCC |
| 829. | MET-1 HL x I2C HL | artificial | aa | QVQLQQSGPELVRPGASVKMSCRASGYTFTSYWLHWVKQRPGQGLEWIGMIDPSNSDTRFNPNFK<br>DKATLNVDRSSNTAYMLLSSLTSADSAVYYCATYGSYVSPLDYWGQGTSVTVSSGGGGSGGGGSG<br>GGGSDIMMSQSPSSLTVSVGEKVTVSCKSSQSLLYTSSQKNYLAWYQQKPGQSPKLLIYWASTRE<br>SGVPDRFTGSGSGTDFTLTITSVKADDLAVYYCQQYYAYPWTFGGGTKLEIKSGGGGSEVQLVES<br>GGGLVQPGGSLKLSCAASGFTFNKYAMNWVRQAPGKGLEWVARIRSKYNNYATYYADSVKDRFTI<br>SRDDSKNTAYLQMNNLKTEDTAVYYCVRHGNFGNSYISYWAYWGQGTLVTVSSGGGGSGGGGSGG<br>GGSQTVVTQEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 830. | MET-1 HL x I2C HL | artificial | nt | CAGGTGCAGCTGCAGCAGAGCGGACCCGAGCTGGTGCGGCCAGGCGCCTCCGTGAAGATGAGCTG<br>CAGGGCCAGCGGCTACACCTTCACCAGCTACTGGCTGCACTGGGTGAAACAGAGACCTGGACAGG<br>GACTGGAGTGGATCGGCATGATCGACCCCAGCAACAGCGACACCAGGTTCAACCCCAACTTCAAG<br>GACAAGGCCACCCTGAACGTGGACAGGTCCAGCAACACCGCCTACATGCTGCTGTCCAGCCTGAC<br>CTCTGCCGACAGCGCCGTGTATTACTGTGCCACCTACGGCAGCTACGTGTCCCCCCTGGACTACT<br>GGGGCCAGGGCACCTCTGTGACAGTGTCCTCCGGTGGTGGTGGTTCTGGCGGCGGCGGCTCCGGT<br>GGTGGTGGTTCTGATATTATGATGAGTCAGTCCCCCAGCAGCCTGACCGTGTCCGTGGGCGAGAA<br>GGTGACCGTGTCTTGCAAGAGCAGCCAGAGCCTGCTGTACACCAGCAGCCAGAAGAACTATCTGG<br>CTTGGTATCAGCAGAAGCCCGGCCAGAGCCCAAAGCTGCTGATCTACTGGGCCAGCACCCGGGAG<br>AGCGGAGTGCCCGACAGATTCACCGGCAGCGGCTCCGGCACAGACTTCACCCTGACCATCACCAG<br>CGTGAAGGCCGACGACCTGGCCGTGTACTACTGCCAGCAGTACTACGCCTACCCCTGGACCTTCG<br>GCGGAGGGACCAAGCTGGAGATCAAGTCCGGAGGTGGTGGATCCGAGGTGCAGCTGGTCGAGTCT<br>GGAGGAGGATTGGTGCAGCCTGGAGGGTCATTGAAACTCTCATGTGCAGCCTCTGGATTCACCTT<br>CAATAAGTACGCCATGAACTGGGTCCGCCAGGCTCCAGGAAAGGGTTTGGAATGGGTTGCTCGCA<br>TAAGAAGTAAATATAATAATTATGCAACATATTATGCCGATTCAGTGAAAGACAGGTTCACCATC<br>TCCAGAGATGATTCAAAAAACACTGCCTATCTACAAATGAACAACTTGAAAACTGAGGACACTGC<br>CGTGTACTACTGTGTGAGACATGGGAACTTCGGTAATAGCTACATATCCTACTGGGCTTACTGGG<br>GCCAAGGGACTCTGGTCACCGTCTCCTCAGGTGGTGGTGGTTCTGGCGGCGGCGGCTCCGGTGGT<br>GGTGGTTCTCAGACTGTTGTGACTCAGGAACCTTCACTCACCGTATCACCTGGTGGAACAGTCAC<br>ACTCACTTGTGGCTCCTCGACTGGGGCTGTTACATCTGGCAACTACCCAAACTGGGTCCAACAAA<br>AACCAGGTCAGGCACCCCGTGGTCTAATAGGTGGGACTAAGTTCCTCGCCCCCGGTACTCCTGCC<br>AGATTCTCAGGCTCCCTGCTTGGAGGCAAGGCTGCCCTCACCCTCTCAGGGGTACAGCCAGAGGA<br>TGAGGCAGAATATTACTGTGTTCTATGGTACAGCAACCGCTGGGTGTTCGGTGGAGGAACCAAAC<br>TGACTGTCCTA |
| 831. | MET-1 LH x I2C HL | artificial | aa | DIMMSQSPSSLTVSVGEKVTVSCKSSQSLLYTSSQKNYLAWYQQKPGQSPKLLIYWASTRESGVP<br>DRFTGSGSGTDFTLTITSVKADDLAVYYCQQYYAYPWTFGGGTKLEIKGGGGSGGGGSGGGGSQV |

| | | | | |
|---|---|---|---|---|
| | | | | QLQQSGPELVRPGASVKMSCRASGYTFTSYWLHWVKQRPGQGLEWIGMIDPSNSDTRFNPNFKDKATLNVDRSSNTAYMLLSSLTSADSAVYYCATYGSYVSPLDYWGQGTSVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 832. | MET-1 LH x I2C HL | artificial | nt | GATATTATGATGAGTCAGTCCCCCAGCAGCCTGACCGTGTCCGTGGGCGAGAAGGTGACCGTGTCTTGCAAGAGCAGCCAGAGCCTGCTGTACACCAGCAGCCAGAAGAACTATCTGGCTTGGTATCAGCAGAAGCCCGGCCAGAGCCCAAAGCTGCTGATCTACTGGGCCAGCACCCGGGAGAGCGGAGTGCCCGACAGATTCACCGGCAGCGGCTCCGGCACAGACTTCACCCTGACCATCACCAGCGTGAAGGCCGACGACCTGGCCGTGTACTACTGCCAGCAGTACTACGCCTACCCCTGGACCTTCGGCGGAGGGACCAAGCTGGAGATCAAGGGTGGTGGTGGTTCTGGCGGCGGCGGCTCCGGTGGTGGTGGTTCTCAGGTGCAGCTGCAGCAGAGCGGACCCGAGCTGGTGCGGCCAGGCGCCTCCGTGAAGATGAGCTGCAGGGCCAGCGGCTACACCTTCACCAGCTACTGGCTGCACTGGGTGAAACAGAGACCTGGACAGGGACTGGAGTGGATCGGCATGATCGACCCCAGCAACAGCGACACCAGGTTCAACCCCAACTTCAAGGACAAGGCCACCCTGAACGTGGACAGGTCCAGCAACACCGCCTACATGCTGCTGTCCAGCCTGACCTCTGCCGACAGCGCCGTGTATTACTGTGCCACCTACGGCAGCTACGTGTCCCCCCTGGACTACTGGGGCCAGGGCACCTCTGTGACAGTGTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 833. | MET-3 L-CDR1 | artificial | aa | KASQSVDYDGGSYMS |
| 834. | MET-3 L-CDR2 | artificial | aa | AASNLES |
| 835. | MET-3 L-CDR3 | artificial | aa | QQSYEDPLT |

| | | | | |
|---|---|---|---|---|
| 836. | MET-3 H-CDR1 | artificial | aa | SYWIH |
| 837. | MET-3 H-CDR2 | artificial | aa | EINPSSGRTNYNEKFKN |
| 838. | MET-3 H-CDR3 | artificial | aa | RGY |
| 839. | MET 4 L | artificial | aa | DIQMTQSPSSVSASVGDRVTITCRASQGINTWLAWYQQKPGKAPKLLIYAASSLKSGVPSRFSGS GSGADFTLTISSLQPEDFATYYCQQANSFPLTFGGGTKVEIK |
| 840. | MET-4 L-CDR1 | artificial | aa | RASQGINTWLA |
| 841. | MET-4 L-CDR2 | artificial | aa | AASSLKS |
| 842. | MET-4 L-CDR3 | artificial | aa | QQANSFPLT |
| 843. | MET-4 L | artificial | nt | GACATCCAGATGACCCAGAGCCCCAGCAGCGTGTCTGCCAGCGTGGGCGACAGAGTGACCATCAC ATGTAGAGCTAGCCAGGGCATCAACACCTGGCTGGCCTGGTATCAGCAGAAGCCCGGCAAGGCCC CCAAGCTGCTGATCTACGCCGCTTCCTCCCTGAAATCTGGCGTGCCCAGCAGATTCAGCGGCAGC GGAAGCGGAGCCGACTTCACCCTGACCATCAGCAGCCTGCAGCCCGAGGACTTCGCCACCTACTA CTGCCAGCAGGCCAACAGCTTCCCCCTGACCTTCGGCGGAGGGACCAAGGTGGAGATCAAG |
| 844. | MET-4 H | artificial | aa | QVQLVQSGAEVKKPGASVKVSCEASGYTFTSYGFSWVRQAPGQGLEWMGWISASNGNTYYAQKLQ GRVTMTTDTSTSSAYMELRSLRSDDTAVYYCARVYADYADYWGQGTLVTVSS |
| 845. | MET-4 H-CDR1 | artificial | aa | SYGFS |
| 846. | MET-4 H-CDR2 | artificial | aa | WISASNGNTYYAQKLQG |
| 847. | MET-4 H-CDR3 | artificial | aa | VYADYADY |
| 848. | MET-4 H | artificial | nt | CAGGTGCAGCTGGTGCAGAGCGGCGCTGAGGTGAAGAAGCCAGGCGCCAGCGTCAAGGTGTCCTG CGAGGCCAGCGGCTACACCTTCACCAGCTACGGCTTCAGCTGGGTGCGGCAGGCTCCTGGACAGG GACTGGAATGGATGGGCTGGATCAGCGCCAGCAACGGCAACACCTACTACGCCCAGAAGCTGCAG GGCCGCGTCACCATGACCACCGACACCAGCACCAGCAGCGCCTACATGGAGCTGAGAAGCCTGAG ATCCGACGACACCGCCGTGTACTACTGCGCCAGGGTGTACGCCGACTACGCTGACTACTGGGGCC AGGGCACCCTGGTGACAGTGTCTTCC |

| | | | | |
|---|---|---|---|---|
| 849. | MET-4 HL | artificial | aa | QVQLVQSGAEVKKPGASVKVSCEASGYTFTSYGFSWVRQAPGQGLEWMGWISASNGNTYYAQKLQGRVTMTTDTSTSSAYMELRSLRSDDTAVYYCARVYADYADYWGQGTLVTVSSGGGGSGGGGSGGGGSDIQMTQSPSSVSASVGDRVTITCRASQGINTWLAWYQQKPGKAPKLLIYAASSLKSGVPSRFSGSGSGADFTLTISSLQPEDFATYYCQQANSFPLTFGGGTKVEIK |
| 850. | MET-4 HL | artificial | nt | CAGGTGCAGCTGGTGCAGAGCGGCGCTGAGGTGAAGAAGCCAGGCGCCAGCGTCAAGGTGTCCTGCGAGGCCAGCGGCTACACCTTCACCAGCTACGGCTTCAGCTGGGTGCGGCAGGCTCCTGGACAGGGACTGGAATGGATGGGCTGGATCAGCGCCAGCAACGGCAACACCTACTACGCCCAGAAGCTGCAGGGCCGCGTCACCATGACCACCGACACCAGCACCAGCAGCGCCTACATGGAGCTGAGAAGCCTGAGATCCGACGACACCGCCGTGTACTACTGCGCCAGGGTGTACGCCGACTACGCTGACTACTGGGGCCAGGGCACCCTGGTGACAGTGTCTTCCGGTGGTGGTGGTTCTGGCGGCGGCGGCTCCGGTGGTGGTGGTTCTGACATCCAGATGACCCAGAGCCCCAGCAGCGTGTCTGCCAGCGTGGGCGACAGAGTGACCATCACATGTAGAGCTAGCCAGGGCATCAACACCTGGCTGGCCTGGTATCAGCAGAAGCCCGGCAAGGCCCCCAAGCTGCTGATCTACGCCGCTTCCTCCCTGAAATCTGGCGTGCCCAGCAGATTCAGCGGCAGCGGAAGCGGAGCCGACTTCACCCTGACCATCAGCAGCCTGCAGCCCGAGGACTTCGCCACCTACTACTGCCAGCAGGCCAACAGCTTCCCCCTGACCTTCGGCGGAGGGACCAAGGTGGAGATCAAG |
| 851. | MET-4 LH | artificial | aa | DIQMTQSPSSVSASVGDRVTITCRASQGINTWLAWYQQKPGKAPKLLIYAASSLKSGVPSRFSGSGSGADFTLTISSLQPEDFATYYCQQANSFPLTFGGGTKVEIKGGGGSGGGGSGGGGSQVQLVQSGAEVKKPGASVKVSCEASGYTFTSYGFSWVRQAPGQGLEWMGWISASNGNTYYAQKLQGRVTMTTDTSTSSAYMELRSLRSDDTAVYYCARVYADYADYWGQGTLVTVSS |
| 852. | MET-4 LH | artificial | nt | GACATCCAGATGACCCAGAGCCCCAGCAGCGTGTCTGCCAGCGTGGGCGACAGAGTGACCATCACATGTAGAGCTAGCCAGGGCATCAACACCTGGCTGGCCTGGTATCAGCAGAAGCCCGGCAAGGCCCCCAAGCTGCTGATCTACGCCGCTTCCTCCCTGAAATCTGGCGTGCCCAGCAGATTCAGCGGCAGCGGAAGCGGAGCCGACTTCACCCTGACCATCAGCAGCCTGCAGCCCGAGGACTTCGCCACCTACTACTGCCAGCAGGCCAACAGCTTCCCCCTGACCTTCGGCGGAGGGACCAAGGTGGAGATCAAGGGTGGTGGTGGTTCTGGCGGCGGCGGCTCCGGTGGTGGTGGTTCTCAGGTGCAGCTGGTGCAGAGCGGCGCTGAGGTGAAGAAGCCAGGCGCCAGCGTCAAGGTGTCCTGCGAGGCCAGCGGCTACACCTTCACCAGCTACGGCTTCAGCTGGGTGCGGCAGGCTCCTGGACAGGGACTGGAATGGATGGGCTGGATCAGCGCCAGCAACGGCAACACCTACTACGCCCAGAAGCTGCAGGGCCGCGTCACCATGACCACCGACACCAGCACCAGCAGCGCCTACATGGAGCTGAGAAGCCTGAGATCCGACGACACCGCCGTGTACTACTGCGCCAGGGTGTACGCCGACTACGCTGACTACTGGGGCCAGGGCACCCTGGTGACAGTGTCTTCC |

| | | | | |
|---|---|---|---|---|
| 853. | MET-4 HL x I2C HL | artificial | aa | QVQLVQSGAEVKKPGASVKVSCEASGYTFTSYGFSWVRQAPGQGLEWMGWISASNGNTYYAQKLQGRVTMTTDTSTSSAYMELRSLRSDDTAVYYCARVYADYADYWGQGTLVTVSSGGGGSGGGGSGGGGSDIQMTQSPSSVSASVGDRVTITCRASQGINTWLAWYQQKPGKAPKLLIYAASSLKSGVPSRFSGSGSGADFTLTISSLQPEDFATYYCQQANSFPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 854. | MET-4 HL x I2C HL | artificial | nt | CAGGTGCAGCTGGTGCAGAGCGGCGCTGAGGTGAAGAAGCCAGGCGCCAGCGTCAAGGTGTCCTGCGAGGCCAGCGGCTACACCTTCACCAGCTACGGCTTCAGCTGGGTGCGGCAGGCTCCTGGACAGGGACTGGAATGGATGGGCTGGATCAGCGCCAGCAACGGCAACACCTACTACGCCCAGAAGCTGCAGGGCCGCGTCACCATGACCACCGACACCAGCACCAGCAGCGCCTACATGGAGCTGAGAAGCCTGAGATCCGACGACACCGCCGTGTACTACTGCGCCAGGGTGTACGCCGACTACGCTGACTACTGGGGCCAGGGCACCCTGGTGACAGTGTCTTCCGGTGGTGGTGGTTCTGGCGGCGGCGGCTCCGGTGGTGGTGGTTCTGACATCCAGATGACCCAGAGCCCCAGCAGCGTGTCTGCCAGCGTGGGCGACAGAGTGACCATCACATGTAGAGCTAGCCAGGGCATCAACACCTGGCTGGCCTGGTATCAGCAGAAGCCCGGCAAGGCCCCCAAGCTGCTGATCTACGCCGCTTCCTCCCTGAAATCTGGCGTGCCCAGCAGATTCAGCGGCAGCGGAAGCGGAGCCGACTTCACCCTGACCATCAGCAGCCTGCAGCCCGAGGACTTCGCCACCTACTACTGCCAGCAGGCCAACAGCTTCCCCCTGACCTTCGGCGGAGGGACCAAGGTGGAGATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 855. | MET-4 LH x I2C HL | artificial | aa | DIQMTQSPSSVSASVGDRVTITCRASQGINTWLAWYQQKPGKAPKLLIYAASSLKSGVPSRFSGSGSGADFTLTISSLQPEDFATYYCQQANSFPLTFGGGTKVEIKGGGGSGGGGSGGGGSQVQLVQSGAEVKKPGASVKVSCEASGYTFTSYGFSWVRQAPGQGLEWMGWISASNGNTYYAQKLQGRVTMTTDTSTSSAYMELRSLRSDDTAVYYCARVYADYADYWGQGTLVTVSSGGGGSEVQLVESGGGLVQPGG |

| | | | | |
|---|---|---|---|---|
| | | | | SLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 856. | MET-4 LH x I2C HL | artificial | nt | GACATCCAGATGACCCAGAGCCCCAGCAGCGTGTCTGCCAGCGTGGGCGACAGAGTGACCATCACATGTAGAGCTAGCCAGGGCATCAACACCTGGCTGGCCTGGTATCAGCAGAAGCCCGGCAAGGCCCCCAAGCTGCTGATCTACGCCGCTTCCTCCCTGAAATCTGGCGTGCCCAGCAGATTCAGCGGCAGCGGAAGCGGAGCCGACTTCACCCTGACCATCAGCAGCCTGCAGCCCGAGGACTTCGCCACCTACTACTGCCAGCAGGCCAACAGCTTCCCCCTGACCTTCGGCGGAGGGACCAAGGTGGAGATCAAGGGTGGTGGTGGTTCTGGCGGCGGCGGCTCCGGTGGTGGTGGTTCTCAGGTGCAGCTGGTGCAGAGCGGCGCTGAGGTGAAGAAGCCAGGCGCCAGCGTCAAGGTGTCCTGCGAGGCCAGCGGCTACACCTTCACCAGCTACGGCTTCAGCTGGGTGCGGCAGGCTCCTGGACAGGGACTGGAATGGATGGGCTGGATCAGCGCCAGCAACGGCAACACCTACTACGCCCAGAAGCTGCAGGGCCGCGTCACCATGACCACCGACACCAGCACCAGCAGCGCCTACATGGAGCTGAGAAGCCTGAGATCCGACGACACCGCCGTGTACTACTGCGCCAGGGTGTACGCCGACTACGCTGACTACTGGGGCCAGGGCACCCTGGTGACAGTGTCTTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 857. | MET-5 L | artificial | aa | EIVLTQSPGTLSLSPGERATLSCRASQSVSNNYLAWYQQKPGQAPRLLIFGASSRATGIPDRFSGSGSGTDFTLTISRLEPEDFAVYYCQQYDISPMYSFGQGTKLEMK |
| 858. | MET-5 L-CDR1 | artificial | aa | RASQSVSNNYLA |
| 859. | MET-5 L-CDR2 | artificial | aa | GASSRAT |

| | | | | |
|---|---|---|---|---|
| 860. | MET-5 L-CDR3 | artificial | aa | QQYDISPMYS |
| 861. | MET-5 L | artificial | nt | GAGATCGTGCTGACCCAGAGCCCAGGCACCCTGAGCCTGAGCCCAGGCGAGAGAGCCACCCTGAG CTGCAGAGCCAGCCAGAGCGTGTCCAACAACTATCTGGCTTGGTATCAGCAGAAACCAGGACAGG CTCCCAGACTGCTGATCTTCGGCGCCAGCTCTAGAGCCACCGGCATCCCCGACAGATTCAGTGGT AGCGGCTCCGGCACAGACTTCACCCTGACCATTTCCAGACTGGAACCCGAGGACTTCGCCGTGTA CTACTGCCAGCAGTACGACATCAGCCCCATGTACAGCTTCGGCCAGGGCACCAAGCTGGAGATGA AG |
| 862. | MET-5 H | artificial | aa | QVQLQESGPGLVKPSQTLSLTCTVSGGSISSGGYYWSWIRQHPGKGLEWIGYIYYSGSTYYNPSL KSRVTISVDTSKNQFSLKLSSVTAADTAVYYCARDGPLGYCSSTSCPVTGEYYYYGMDVWGQGTT VTVSS |
| 863. | MET-5 H-CDR1 | artificial | aa | SGGYYWS |
| 864. | MET-5 H-CDR2 | artificial | aa | YIYYSGSTYYNPSLKS |
| 865. | MET-5 H-CDR3 | artificial | aa | DGPLGYCSSTSCPVTGEYYYYGMDV |
| 866. | MET-5 H | artificial | nt | CAGGTGCAGCTGCAGGAGAGCGGGCCTGGACTGGTGAAGCCCAGCCAGACCCTGTCTCTGACCTG CACCGTGTCCGGCGGCAGCATCAGCAGCGGCGGCTACTACTGGTCCTGGATCAGGCAGCACCCAG GCAAGGGCCTGGAGTGGATCGGCTACATCTACTATTCTGGATCCACCTACTACAACCCCAGCCTG AAGTCCAGAGTGACCATCTCCGTGGACACCAGCAAGAACCAGTTCAGCCTGAAGCTGTCCAGCGT GACAGCCGCCGACACCGCCGTGTATTATTGCGCCAGGGACGGCCCACTGGGATATTGTTCCTCCA CATCCTGCCCAGTGACCGGCGAGTACTACTACTACGGCATGGACGTGTGGGGACAGGGCACCACC GTGACCGTGTCTTCC |
| 867. | MET-5 HL | artificial | aa | QVQLQESGPGLVKPSQTLSLTCTVSGGSISSGGYYWSWIRQHPGKGLEWIGYIYYSGSTYYNPSL KSRVTISVDTSKNQFSLKLSSVTAADTAVYYCARDGPLGYCSSTSCPVTGEYYYYGMDVWGQGTT VTVSSGGGGSGGGGSGGGGSEIVLTQSPGTLSLSPGERATLSCRASQSVSNNYLAWYQQKPGQAP RLLIFGASSRATGIPDRFSGSGSGTDFTLTISRLEPEDFAVYYCQQYDISPMYSFGQGTKLEMK |
| 868. | MET-5 HL | artificial | nt | CAGGTGCAGCTGCAGGAGAGCGGGCCTGGACTGGTGAAGCCCAGCCAGACCCTGTCTCTGACCTG CACCGTGTCCGGCGGCAGCATCAGCAGCGGCGGCTACTACTGGTCCTGGATCAGGCAGCACCCAG GCAAGGGCCTGGAGTGGATCGGCTACATCTACTATTCTGGATCCACCTACTACAACCCCAGCCTG AAGTCCAGAGTGACCATCTCCGTGGACACCAGCAAGAACCAGTTCAGCCTGAAGCTGTCCAGCGT GACAGCCGCCGACACCGCCGTGTATTATTGCGCCAGGGACGGCCCACTGGGATATTGTTCCTCCA |

| | | | | |
|---|---|---|---|---|
| | | | | CATCCTGCCCAGTGACCGGCGAGTACTACTACTACGGCATGGACGTGTGGGGACAGGGCACCACC<br>GTGACCGTGTCTTCCGGTGGTGGTGGTTCTGGCGGCGGCGGCTCCGGTGGTGGTGGTTCTGAGAT<br>CGTGCTGACCCAGAGCCCAGGCACCCTGAGCCTGAGCCCAGGCGAGAGAGCCACCCTGAGCTGCA<br>GAGCCAGCCAGAGCGTGTCCAACAACTATCTGGCTTGGTATCAGCAGAAACCAGGACAGGCTCCC<br>AGACTGCTGATCTTCGGCGCCAGCTCTAGAGCCACCGGCATCCCCGACAGATTCAGTGGTAGCGG<br>CTCCGGCACAGACTTCACCCTGACCATTTCCAGACTGGAACCCGAGGACTTCGCCGTGTACTACT<br>GCCAGCAGTACGACATCAGCCCCATGTACAGCTTCGGCCAGGGCACCAAGCTGGAGATGAAG |
| 869. | MET-5 LH | artificial | aa | EIVLTQSPGTLSLSPGERATLSCRASQSVSNNYLAWYQQKPGQAPRLLIFGASSRATGIPDRFSG<br>SGSGTDFTLTISRLEPEDFAVYYCQQYDISPMYSFGQGTKLEMKGGGGSGGGGSGGGGSQVQLQE<br>SGPGLVKPSQTLSLTCTVSGGSISSGGYYWSWIRQHPGKGLEWIGYIYYSGSTYYNPSLKSRVTI<br>SVDTSKNQFSLKLSSVTAADTAVYYCARDGPLGYCSSTSCPVTGEYYYYGMDVWGQGTTVTVSS |
| 870. | MET-5 LH | artificial | nt | GAGATCGTGCTGACCCAGAGCCCAGGCACCCTGAGCCTGAGCCCAGGCGAGAGAGCCACCCTGAG<br>CTGCAGAGCCAGCCAGAGCGTGTCCAACAACTATCTGGCTTGGTATCAGCAGAAACCAGGACAGG<br>CTCCCAGACTGCTGATCTTCGGCGCCAGCTCTAGAGCCACCGGCATCCCCGACAGATTCAGTGGT<br>AGCGGCTCCGGCACAGACTTCACCCTGACCATTTCCAGACTGGAACCCGAGGACTTCGCCGTGTA<br>CTACTGCCAGCAGTACGACATCAGCCCCATGTACAGCTTCGGCCAGGGCACCAAGCTGGAGATGA<br>AGGGTGGTGGTGGTTCTGGCGGCGGCGGCTCCGGTGGTGGTGGTTCTCAGGTGCAGCTGCAGGAG<br>AGCGGGCCTGGACTGGTGAAGCCCAGCCAGACCCTGTCTCTGACCTGCACCGTGTCCGGCGGCAG<br>CATCAGCAGCGGCGGCTACTACTGGTCCTGGATCAGGCAGCACCCAGGCAAGGGCCTGGAGTGGA<br>TCGGCTACATCTACTATTCTGGATCCACCTACTACAACCCCAGCCTGAAGTCCAGAGTGACCATC<br>TCCGTGGACACCAGCAAGAACCAGTTCAGCCTGAAGCTGTCCAGCGTGACAGCCGCCGACACCGC<br>CGTGTATTATTGCGCCAGGGACGGCCCACTGGGATATTGTTCCTCCACATCCTGCCCAGTGACCG<br>GCGAGTACTACTACTACGGCATGGACGTGTGGGGACAGGGCACCACCGTGACCGTGTCTTCC |
| 871. | MET-5 HL x I2C HL | artificial | aa | QVQLQESGPGLVKPSQTLSLTCTVSGGSISSGGYYWSWIRQHPGKGLEWIGYIYYSGSTYYNPSL<br>KSRVTISVDTSKNQFSLKLSSVTAADTAVYYCARDGPLGYCSSTSCPVTGEYYYYGMDVWGQGTT<br>VTVSSGGGGSGGGGSGGGGSEIVLTQSPGTLSLSPGERATLSCRASQSVSNNYLAWYQQKPGQAP<br>RLLIFGASSRATGIPDRFSGSGSGTDFTLTISRLEPEDFAVYYCQQYDISPMYSFGQGTKLEMKS<br>GGGGSEVQLVESGGGLVQPGGSLKLSCAASGFTFNKYAMNWVRQAPGKGLEWVARIRSKYNNYAT<br>YYADSVKDRFTISRDDSKNTAYLQMNNLKTEDTAVYYCVRHGNFGNSYISYWAYWGQGTLVTVSS<br>GGGGSGGGGSGGGGSQTVVTQEPSLTVSPGGTVTLTCGSSTGAVTSGNYPNWVQQKPGQAPRGLI<br>GGTKFLAPGTPARFSGSLLGGKAALTLSGVQPEDEAEYYCVLWYSNRWVFGGGTKLTVL |
| 872. | MET-5 HL x I2C HL | artificial | nt | CAGGTGCAGCTGCAGGAGAGCGGGCCTGGACTGGTGAAGCCCAGCCAGACCCTGTCTCTGACCTG<br>CACCGTGTCCGGCGGCAGCATCAGCAGCGGCGGCTACTACTGGTCCTGGATCAGGCAGCACCCAG<br>GCAAGGGCCTGGAGTGGATCGGCTACATCTACTATTCTGGATCCACCTACTACAACCCCAGCCTG |

| | | | | |
|---|---|---|---|---|
| | | | | AAGTCCAGAGTGACCATCTCCGTGGACACCAGCAAGAACCAGTTCAGCCTGAAGCTGTCCAGCGT<br>GACAGCCGCCGACACCGCCGTGTATTATTGCGCCAGGGACGGCCCACTGGGATATTGTTCCTCCA<br>CATCCTGCCCAGTGACCGGCGAGTACTACTACTACGGCATGGACGTGTGGGGACAGGGCACCACC<br>GTGACCGTGTCTTCCGGTGGTGGTGGTTCTGGCGGCGGCGGCTCCGGTGGTGGTGGTTCTGAGAT<br>CGTGCTGACCCAGAGCCCAGGCACCCTGAGCCTGAGCCCAGGCGAGAGAGCCACCCTGAGCTGCA<br>GAGCCAGCCAGAGCGTGTCCAACAACTATCTGGCTTGGTATCAGCAGAAACCAGGACAGGCTCCC<br>AGACTGCTGATCTTCGGCGCCAGCTCTAGAGCCACCGGCATCCCCGACAGATTCAGTGGTAGCGG<br>CTCCGGCACAGACTTCACCCTGACCATTTCCAGACTGGAACCCGAGGACTTCGCCGTGTACTACT<br>GCCAGCAGTACGACATCAGCCCCATGTACAGCTTCGGCCAGGGCACCAAGCTGGAGATGAAGTCC<br>GGAGGTGGTGGATCCGAGGTGCAGCTGGTCGAGTCTGGAGGAGGATTGGTGCAGCCTGGAGGGTC<br>ATTGAAACTCTCATGTGCAGCCTCTGGATTCACCTTCAATAAGTACGCCATGAACTGGGTCCGCC<br>AGGCTCCAGGAAAGGGTTTGGAATGGGTTGCTCGCATAAGAAGTAAATATAATAATTATGCAACA<br>TATTATGCCGATTCAGTGAAAGACAGGTTCACCATCTCCAGAGATGATTCAAAAAACACTGCCTA<br>TCTACAAATGAACAACTTGAAAACTGAGGACACTGCCGTGTACTACTGTGTGAGACATGGGAACT<br>TCGGTAATAGCTACATATCCTACTGGGCTTACTGGGGCCAAGGGACTCTGGTCACCGTCTCCTCA<br>GGTGGTGGTGGTTCTGGCGGCGGCGGCTCCGGTGGTGGTGGTTCTCAGACTGTTGTGACTCAGGA<br>ACCTTCACTCACCGTATCACCTGGTGGAACAGTCACACTCACTTGTGGCTCCTCGACTGGGGCTG<br>TTACATCTGGCAACTACCCAAACTGGGTCCAACAAAAACCAGGTCAGGCACCCCGTGGTCTAATA<br>GGTGGGACTAAGTTCCTCGCCCCCGGTACTCCTGCCAGATTCTCAGGCTCCCTGCTTGGAGGCAA<br>GGCTGCCCTCACCCTCTCAGGGGTACAGCCAGAGGATGAGGCAGAATATTACTGTGTTCTATGGT<br>ACAGCAACCGCTGGGTGTTCGGTGGAGGAACCAAACTGACTGTCCTA |
| 873. | MET-5 LH x I2C HL | artificial | aa | EIVLTQSPGTLSLSPGERATLSCRASQSVSNNYLAWYQQKPGQAPRLLIFGASSRATGIPDRFSG<br>SGSGTDFTLTISRLEPEDFAVYYCQQYDISPMYSFGQGTKLEMKGGGGSGGGGSGGGGSQVQLQE<br>SGPGLVKPSQTLSLTCTVSGGSISSGGYYWSWIRQHPGKGLEWIGYIYYSGSTYYNPSLKSRVTI<br>SVDTSKNQFSLKLSSVTAADTAVYYCARDGPLGYCSSTSCPVTGEYYYYGMDVWGQGTTVTVSSG<br>GGGSEVQLVESGGGLVQPGGSLKLSCAASGFTFNKYAMNWVRQAPGKGLEWVARIRSKYNNYATY<br>YADSVKDRFTISRDDSKNTAYLQMNNLKTEDTAVYYCVRHGNFGNSYISYWAYWGQGTLVTVSSG<br>GGGSGGGGSGGGGSQTVVTQEPSLTVSPGGTVTLTCGSSTGAVTSGNYPNWVQQKPGQAPRGLIG<br>GTKFLAPGTPARFSGSLLGGKAALTLSGVQPEDEAEYYCVLWYSNRWVFGGGTKLTVL |
| 874. | MET-5 LH x I2C HL | artificial | nt | GAGATCGTGCTGACCCAGAGCCCAGGCACCCTGAGCCTGAGCCCAGGCGAGAGAGCCACCCTGAG<br>CTGCAGAGCCAGCCAGAGCGTGTCCAACAACTATCTGGCTTGGTATCAGCAGAAACCAGGACAGG<br>CTCCCAGACTGCTGATCTTCGGCGCCAGCTCTAGAGCCACCGGCATCCCCGACAGATTCAGTGGT<br>AGCGGCTCCGGCACAGACTTCACCCTGACCATTTCCAGACTGGAACCCGAGGACTTCGCCGTGTA<br>CTACTGCCAGCAGTACGACATCAGCCCCATGTACAGCTTCGGCCAGGGCACCAAGCTGGAGATGA<br>AGGGTGGTGGTGGTTCTGGCGGCGGCGGCTCCGGTGGTGGTGGTTCTCAGGTGCAGCTGCAGGAG |

| | | | | |
|---|---|---|---|---|
| | | | | AGCGGGCCTGGACTGGTGAAGCCCAGCCAGACCCTGTCTCTGACCTGCACCGTGTCCGGCGGCAG CATCAGCAGCGGCGGCTACTACTGGTCCTGGATCAGGCAGCACCCAGGCAAGGGCCTGGAGTGGA TCGGCTACATCTACTATTCTGGATCCACCTACTACAACCCCAGCCTGAAGTCCAGAGTGACCATC TCCGTGGACACCAGCAAGAACCAGTTCAGCCTGAAGCTGTCCAGCGTGACAGCCGCCGACACCGC CGTGTATTATTGCGCCAGGGACGGCCCACTGGGATATTGTTCCTCCACATCCTGCCCAGTGACCG GCGAGTACTACTACTACGGCATGGACGTGTGGGGACAGGGCACCACCGTGACCGTGTCTTCCGGA GGTGGTGGATCCGAGGTGCAGCTGGTCGAGTCTGGAGGAGGATTGGTGCAGCCTGGAGGGTCATT GAAACTCTCATGTGCAGCCTCTGGATTCACCTTCAATAAGTACGCCATGAACTGGGTCCGCCAGG CTCCAGGAAAGGGTTTGGAATGGGTTGCTCGCATAAGAAGTAAATATAATAATTATGCAACATAT TATGCCGATTCAGTGAAAGACAGGTTCACCATCTCCAGAGATGATTCAAAAAACACTGCCTATCT ACAAATGAACAACTTGAAAACTGAGGACACTGCCGTGTACTACTGTGTGAGACATGGGAACTTCG GTAATAGCTACATATCCTACTGGGCTTACTGGGGCCAAGGGACTCTGGTCACCGTCTCCTCAGGT GGTGGTGGTTCTGGCGGCGGCGGCTCCGGTGGTGGTGGTTCTCAGACTGTTGTGACTCAGGAACC TTCACTCACCGTATCACCTGGTGGAACAGTCACACTCACTTGTGGCTCCTCGACTGGGGCTGTTA CATCTGGCAACTACCCAAACTGGGTCCAACAAAAACCAGGTCAGGCACCCCGTGGTCTAATAGGT GGGACTAAGTTCCTCGCCCCCGGTACTCCTGCCAGATTCTCAGGCTCCCTGCTTGGAGGCAAGGC TGCCCTCACCCTCTCAGGGGTACAGCCAGAGGATGAGGCAGAATATTACTGTGTTCTATGGTACA GCAACCGCTGGGTGTTCGGTGGAGGAACCAAACTGACTGTCCTA |
| 875. | MET-6 L | artificial | aa | DIQMTQSPSSVSASVGDRVTITCRASQGISSWLAWYQQKPGKAPKLLIYAASSLQSGVPSRFSGS GSGTDFTLTISSLQSEDFATYYCQQANSFPITFGPGTKVEIK |
| 876. | MET-6 L-CDR1 | artificial | aa | RASQGISSWLA |
| 877. | MET-6 L-CDR2 | artificial | aa | AASSLQS |
| 878. | MET-6 L-CDR3 | artificial | aa | QQANSFPIT |
| 879. | MET-6 L | artificial | nt | GACATCCAGATGACCCAGAGCCCCAGCAGCGTGTCTGCCAGCGTGGGCGACAGAGTGACCATCAC CTGCAGAGCTTCCCAGGGCATCTCTAGCTGGCTGGCCTGGTATCAGCAGAAGCCCGGCAAGGCCC CCAAGCTCCTGATCTATGCTGCTTCCAGCCTGCAGAGCGGCGTGCCCAGCAGATTCAGCGGCAGC GGCTCCGGCACAGACTTCACCCTGACCATCAGCTCCCTGCAGTCCGAGGACTTCGCCACCTACTA CTGCCAGCAGGCCAACAGCTTCCCCATCACCTTCGGCCCAGGCACCAAGGTGGAGATCAAG |
| 880. | MET-6 H | artificial | aa | QLQLQESGPGLVKPSETLSLTCTVSGGSISSSSYYGGWIRQPPGKGLDWIGSIYYSGNTYYNPSL KSRVTISVDTSKNQFSLKLSSVTAADTAVYYCARHSWDYFDYWDQGTLVTVSS |

| | | | | |
|---|---|---|---|---|
| 881. | MET-6 H-CDR1 | artificial | aa | SSSYYGG |
| 882. | MET-6 H-CDR2 | artificial | aa | SIYYSGNTYYNPSLKS |
| 883. | MET-6 H-CDR3 | artificial | aa | HSWDYFDY |
| 884. | MET-6 H | artificial | nt | CAGCTCCAGCTCCAGGAGAGCGGGCCTGGACTGGTGAAGCCCAGCGAGACACTGAGCCTGACCTGCACCGTGTCCGGCGGCAGCATCAGCAGCAGCAGCTACTACGGCGGCTGGATCAGACAGCCACCCGGCAAAGGACTGGATTGGATCGGATCCATCTATTATAGTGGAAACACCTACTACAACCCCAGCCTGAAGTCCCGCGTGACCATCTCCGTGGACACCAGCAAGAACCAGTTCAGCCTGAAGCTGTCCAGCGTGACAGCCGCCGACACCGCCGTGTACTACTGCGCCAGGCACAGCTGGGACTACTTCGACTACTGGGACCAGGGCACCCTGGTGACCGTGTCTTCC |
| 885. | MET-6 HL | artificial | aa | QLQLQESGPGLVKPSETLSLTCTVSGGSISSSSYYGGWIRQPPGKGLDWIGSIYYSGNTYYNPSLKSRVTISVDTSKNQFSLKLSSVTAADTAVYYCARHSWDYFDYWDQGTLVTVSSGGGGSGGGGSGGGGSDIQMTQSPSSVSASVGDRVTITCRASQGISSWLAWYQQKPGKAPKLLIYAASSLQSGVPSRFSGSGSGTDFTLTISSLQSEDFATYYCQQANSFPITFGPGTKVEIK |
| 886. | MET-6 HL | artificial | nt | CAGCTCCAGCTCCAGGAGAGCGGGCCTGGACTGGTGAAGCCCAGCGAGACACTGAGCCTGACCTGCACCGTGTCCGGCGGCAGCATCAGCAGCAGCAGCTACTACGGCGGCTGGATCAGACAGCCACCCGGCAAAGGACTGGATTGGATCGGATCCATCTATTATAGTGGAAACACCTACTACAACCCCAGCCTGAAGTCCCGCGTGACCATCTCCGTGGACACCAGCAAGAACCAGTTCAGCCTGAAGCTGTCCAGCGTGACAGCCGCCGACACCGCCGTGTACTACTGCGCCAGGCACAGCTGGGACTACTTCGACTACTGGGACCAGGGCACCCTGGTGACCGTGTCTTCCGGTGGTGGTGGTTCTGGCGGCGGCGGCTCCGGTGGTGGTGGTTCTGACATCCAGATGACCCAGAGCCCCAGCAGCGTGTCTGCCAGCGTGGGCGACAGAGTGACCATCACCTGCAGAGCTTCCCAGGGCATCTCTAGCTGGCTGGCCTGGTATCAGCAGAAGCCCGGCAAGGCCCCCAAGCTCCTGATCTATGCTGCTTCCAGCCTGCAGAGCGGCGTGCCCAGCAGATTCAGCGGCAGCGGCTCCGGCACAGACTTCACCCTGACCATCAGCTCCCTGCAGTCCGAGGACTTCGCCACCTACTACTGCCAGCAGGCCAACAGCTTCCCCATCACCTTCGGCCCAGGCACCAAGGTGGAGATCAAG |
| 887. | MET-6 LH | artificial | aa | DIQMTQSPSSVSASVGDRVTITCRASQGISSWLAWYQQKPGKAPKLLIYAASSLQSGVPSRFSGSGSGTDFTLTISSLQSEDFATYYCQQANSFPITFGPGTKVEIKGGGGSGGGGSGGGGSQLQLQESGPGLVKPSETLSLTCTVSGGSISSSSYYGGWIRQPPGKGLDWIGSIYYSGNTYYNPSLKSRVTISVDTSKNQFSLKLSSVTAADTAVYYCARHSWDYFDYWDQGTLVTVSS |

| | | | | |
|---|---|---|---|---|
| 888. | MET-6 LH | artificial | nt | GACATCCAGATGACCCAGAGCCCCAGCAGCGTGTCTGCCAGCGTGGGCGACAGAGTGACCATCACCTGCAGAGCTTCCCAGGGCATCTCTAGCTGGCTGGCCTGGTATCAGCAGAAGCCCGGCAAGGCCCCCAAGCTCCTGATCTATGCTGCTTCCAGCCTGCAGAGCGGCGTGCCCAGCAGATTCAGCGGCAGCGGCTCCGGCACAGACTTCACCCTGACCATCAGCTCCCTGCAGTCCGAGGACTTCGCCACCTACTACTGCCAGCAGGCCAACAGCTTCCCCATCACCTTCGGCCCAGGCACCAAGGTGGAGATCAAGGGTGGTGGTGGTTCTGGCGGCGGCGGCTCCGGTGGTGGTGGTTCTCAGCTCCAGCTCCAGGAGAGCGGGCCTGGACTGGTGAAGCCCAGCGAGACACTGAGCCTGACCTGCACCGTGTCCGGCGGCAGCATCAGCAGCAGCAGCTACTACGGCGGCTGGATCAGACAGCCACCCGGCAAAGGACTGGATTGGATCGGATCCATCTATTATAGTGGAAACACCTACTACAACCCCAGCCTGAAGTCCCGCGTGACCATCTCCGTGGACACCAGCAAGAACCAGTTCAGCCTGAAGCTGTCCAGCGTGACAGCCGCCGACACCGCCGTGTACTACTGCGCCAGGCACAGCTGGGACTACTTCGACTACTGGGACCAGGGCACCCTGGTGACCGTGTCTTCC |
| 889. | MET-6 HL x I2C HL | artificial | aa | QLQLQESGPGLVKPSETLSLTCTVSGGSISSSSYYGGWIRQPPGKGLDWIGSIYYSGNTYYNPSLKSRVTISVDTSKNQFSLKLSSVTAADTAVYYCARHSWDYFDYWDQGTLVTVSSGGGGSGGGGSGGGGSDIQMTQSPSSVSASVGDRVTITCRASQGISSWLAWYQQKPGKAPKLLIYAASSLQSGVPSRFSGSGSGTDFTLTISSLQSEDFATYYCQQANSFPITFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 890. | MET-6 HL x I2C HL | artificial | nt | CAGCTCCAGCTCCAGGAGAGCGGGCCTGGACTGGTGAAGCCCAGCGAGACACTGAGCCTGACCTGCACCGTGTCCGGCGGCAGCATCAGCAGCAGCAGCTACTACGGCGGCTGGATCAGACAGCCACCCGGCAAAGGACTGGATTGGATCGGATCCATCTATTATAGTGGAAACACCTACTACAACCCCAGCCTGAAGTCCCGCGTGACCATCTCCGTGGACACCAGCAAGAACCAGTTCAGCCTGAAGCTGTCCAGCGTGACAGCCGCCGACACCGCCGTGTACTACTGCGCCAGGCACAGCTGGGACTACTTCGACTACTGGGACCAGGGCACCCTGGTGACCGTGTCTTCCGGTGGTGGTGGTTCTGGCGGCGGCGGCTCCGGTGGTGGTGGTTCTGACATCCAGATGACCCAGAGCCCCAGCAGCGTGTCTGCCAGCGTGGGCGACAGAGTGACCATCACCTGCAGAGCTTCCCAGGGCATCTCTAGCTGGCTGGCCTGGTATCAGCAGAAGCCCGGCAAGGCCCCCAAGCTCCTGATCTATGCTGCTTCCAGCCTGCAGAGCGGCGTGCCCAGCAGATTCAGCGGCAGCGGCTCCGGCACAGACTTCACCCTGACCATCAGCTCCCTGCAGTCCGAGGACTTCGCCACCTACTACTGCCAGCAGGCCAACAGCTTCCCCATCACCTTCGGCCCAGGCACCAAGGTGGAGATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAAC |

| | | | | |
|---|---|---|---|---|
| | | | | ACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 891. | MET-6 LH x I2C HL | artificial | aa | DIQMTQSPSSVSASVGDRVTITCRASQGISSWLAWYQQKPGKAPKLLIYAASSLQSGVPSRFSGSGSGTDFTLTISSLQSEDFATYYCQQANSFPITFGPGTKVEIKGGGGSGGGGSGGGGSQLQLQESGPGLVKPSETLSLTCTVSGGSISSSSYYGGWIRQPPGKGLDWIGSIYYSGNTYYNPSLKSRVTISVDTSKNQFSLKLSSVTAADTAVYYCARHSWDYFDYWDQGTLVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 892. | MET-6 LH x I2C HL | artificial | nt | GACATCCAGATGACCCAGAGCCCCAGCAGCGTGTCTGCCAGCGTGGGCGACAGAGTGACCATCACCTGCAGAGCTTCCCAGGGCATCTCTAGCTGGCTGGCCTGGTATCAGCAGAAGCCCGGCAAGGCCCCCAAGCTCCTGATCTATGCTGCTTCCAGCCTGCAGAGCGGCGTGCCCAGCAGATTCAGCGGCAGCGGCTCCGGCACAGACTTCACCCTGACCATCAGCTCCCTGCAGTCCGAGGACTTCGCCACCTACTACTGCCAGCAGGCCAACAGCTTCCCCATCACCTTCGGCCCAGGCACCAAGGTGGAGATCAAGGGTGGTGGTGGTTCTGGCGGCGGCGGCTCCGGTGGTGGTGGTTCTCAGCTCCAGCTCCAGGAGAGCGGGCCTGGACTGGTGAAGCCCAGCGAGACACTGAGCCTGACCTGCACCGTGTCCGGCGGCAGCATCAGCAGCAGCAGCTACTACGGCGGCTGGATCAGACAGCCACCCGGCAAAGGACTGGATTGGATCGGATCCATCTATTATAGTGGAAACACCTACTACAACCCCAGCCTGAAGTCCCGCGTGACCATCTCCGTGGACACCAGCAAGAACCAGTTCAGCCTGAAGCTGTCCAGCGTGACAGCCGCCGACACCGCCGTGTACTACTGCGCCAGGCACAGCTGGGACTACTTCGACTACTGGGACCAGGGCACCCTGGTGACCGTGTCTTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGG |

| | | | | |
|---|---|---|---|---|
| | | | | GGCTGTTACATCTGGCAACTACCCAAACTGGGTCCAACAAAAACCAGGTCAGGCACCCCGTGGTC TAATAGGTGGGACTAAGTTCCTCGCCCCCGGTACTCCTGCCAGATTCTCAGGCTCCCTGCTTGGA GGCAAGGCTGCCCTCACCCTCTCAGGGGTACAGCCAGAGGATGAGGCAGAATATTACTGTGTTCT ATGGTACAGCAACCGCTGGGTGTTCGGTGGAGGAACCAAACTGACTGTCCTA |
| 893. | MET-7 L | artificial | aa | DIQMTQSPSSVSASVGDRVIITCRASQGISSWLAWYQQKPGKAPKLLIYAASSLKSGVPSRFSGS GSGTDFTVTISSLQPEDFATYYCQQSNSLPWTFGQGTKVEIK |
| 894. | MET-7 L-CDR1 | artificial | aa | RASQGISSWLA |
| 895. | MET-7 L-CDR2 | artificial | aa | AASSLKS |
| 896. | MET-7 L-CDR3 | artificial | aa | QQSNSLPWT |
| 897. | MET-7 L | artificial | nt | GACATCCAGATGACCCAGAGCCCCAGCAGCGTGTCTGCCAGCGTGGGCGACAGAGTGATCATCAC CTGCAGAGCTTCCCAGGGCATCTCTAGCTGGCTGGCCTGGTATCAGCAGAAGCCCGGCAAGGCCC CCAAGCTGCTCATCTATGCTGCTTCCAGCCTGAAGTCCGGCGTGCCCAGCAGATTCAGCGGCAGC GGCTCCGGCACAGACTTCACCGTGACCATCAGCAGCCTGCAGCCCGAGGACTTCGCCACCTACTA CTGCCAGCAGAGCAACAGCCTGCCCTGGACCTTCGGCCAGGGCACCAAGGTGGAGATCAAG |
| 898. | MET-7 H | artificial | aa | EVQLVESGGGLVQPGGSLRLSCAASGFTFSRYSMNWVRQAPGKGLEWVSYISSRSSTIYYADSVK GRFTMSRDNAKNSLYMQMNSLRDEDTAVYYCGYGDYDYFDYWGQGTLVTVSS |
| 899. | MET-7 H-CDR1 | artificial | aa | RYSMN |
| 900. | MET-7 H-CDR2 | artificial | aa | YISSRSSTIYYADSVKG |
| 901. | MET-7 H-CDR3 | artificial | aa | GDYDYFDY |
| 902. | MET-7 H | artificial | nt | GAGGTGCAGCTGGTCGAGTCTGGCGGAGGACTGGTGCAGCCAGGCGGCAGCCTGAGACTGTCCTG TGCTGCTTCTGGATTCACCTTCAGCAGGTACAGCATGAACTGGGTCCGCCAGGCTCCTGGTAAAG GACTGGAGTGGGTCTCCTACATCAGCAGCAGGTCCAGCACCATCTACTACGCCGACAGCGTGAAG GGCAGGTTCACCATGAGCAGGGACAACGCCAAGAACAGCCTGTACATGCAGATGAACAGCCTGAG GGACGAGGACACCGCCGTGTACTACTGCGGCTACGGCGACTACGACTACTTCGACTACTGGGGCC AGGGAACCCTGGTGACCGTGTCTTCC |
| 903. | MET-7 LH | artificial | aa | DIQMTQSPSSVSASVGDRVIITCRASQGISSWLAWYQQKPGKAPKLLIYAASSLKSGVPSRFSGS GSGTDFTVTISSLQPEDFATYYCQQSNSLPWTFGQGTKVEIKGGGGSGGGGSGGGGSEVQLVESG |

| | | | | |
|---|---|---|---|---|
| | | | | GGLVQPGGSLRLSCAASGFTFSRYSMNWVRQAPGKGLEWVSYISSRSSTIYYADSVKGRFTMSRD NAKNSLYMQMNSLRDEDTAVYYCGYGDYDYFDYWGQGTLVTVSS |
| 904. | MET-7 LH | artificial | nt | GACATCCAGATGACCCAGAGCCCCAGCAGCGTGTCTGCCAGCGTGGGCGACAGAGTGATCATCAC CTGCAGAGCTTCCCAGGGCATCTCTAGCTGGCTGGCCTGGTATCAGCAGAAGCCCGGCAAGGCCC CCAAGCTGCTCATCTATGCTGCTTCCAGCCTGAAGTCCGGCGTGCCCAGCAGATTCAGCGGCAGC GGCTCCGGCACAGACTTCACCGTGACCATCAGCAGCCTGCAGCCCGAGGACTTCGCCACCTACTA CTGCCAGCAGAGCAACAGCCTGCCCTGGACCTTCGGCCAGGGCACCAAGGTGGAGATCAAGGGTG GTGGTGGTTCTGGCGGCGGCGGCTCCGGTGGTGGTGGTTCTGAGGTGCAGCTGGTCGAGTCTGGC GGAGGACTGGTGCAGCCAGGCGGCAGCCTGAGACTGTCCTGTGCTGCTTCTGGATTCACCTTCAG CAGGTACAGCATGAACTGGGTCCGCCAGGCTCCTGGTAAAGGACTGGAGTGGGTCTCCTACATCA GCAGCAGGTCCAGCACCATCTACTACGCCGACAGCGTGAAGGGCAGGTTCACCATGAGCAGGGAC AACGCCAAGAACAGCCTGTACATGCAGATGAACAGCCTGAGGGACGAGGACACCGCCGTGTACTA CTGCGGCTACGGCGACTACGACTACTTCGACTACTGGGGCCAGGGAACCCTGGTGACCGTGTCTT CC |
| 905. | MET-7 LH x I2C HL | artificial | aa | DIQMTQSPSSVSASVGDRVIITCRASQGISSWLAWYQQKPGKAPKLLIYAASSLKSGVPSRFSGS GSGTDFTVTISSLQPEDFATYYCQQSNSLPWTFGQGTKVEIKGGGGSGGGGSGGGGSEVQLVESG GGLVQPGGSLRLSCAASGFTFSRYSMNWVRQAPGKGLEWVSYISSRSSTIYYADSVKGRFTMSRD NAKNSLYMQMNSLRDEDTAVYYCGYGDYDYFDYWGQGTLVTVSSGGGGSEVQLVESGGGLVQPGG SLKLSCAASGFTFNKYAMNWVRQAPGKGLEWVARIRSKYNNYATYYADSVKDRFTISRDDSKNTA YLQMNNLKTEDTAVYYCVRHGNFGNSYISYWAYWGQGTLVTVSSGGGGSGGGGSGGGGSQTVVTQ EPSLTVSPGGTVTLTCGSSTGAVTSGNYPNWVQQKPGQAPRGLIGGTKFLAPGTPARFSGSLLGG KAALTLSGVQPEDEAEYYCVLWYSNRWVFGGGTKLTVL |
| 906. | MET-7 LH x I2C HL | artificial | nt | GACATCCAGATGACCCAGAGCCCCAGCAGCGTGTCTGCCAGCGTGGGCGACAGAGTGATCATCAC CTGCAGAGCTTCCCAGGGCATCTCTAGCTGGCTGGCCTGGTATCAGCAGAAGCCCGGCAAGGCCC CCAAGCTGCTCATCTATGCTGCTTCCAGCCTGAAGTCCGGCGTGCCCAGCAGATTCAGCGGCAGC GGCTCCGGCACAGACTTCACCGTGACCATCAGCAGCCTGCAGCCCGAGGACTTCGCCACCTACTA CTGCCAGCAGAGCAACAGCCTGCCCTGGACCTTCGGCCAGGGCACCAAGGTGGAGATCAAGGGTG GTGGTGGTTCTGGCGGCGGCGGCTCCGGTGGTGGTGGTTCTGAGGTGCAGCTGGTCGAGTCTGGC GGAGGACTGGTGCAGCCAGGCGGCAGCCTGAGACTGTCCTGTGCTGCTTCTGGATTCACCTTCAG CAGGTACAGCATGAACTGGGTCCGCCAGGCTCCTGGTAAAGGACTGGAGTGGGTCTCCTACATCA GCAGCAGGTCCAGCACCATCTACTACGCCGACAGCGTGAAGGGCAGGTTCACCATGAGCAGGGAC AACGCCAAGAACAGCCTGTACATGCAGATGAACAGCCTGAGGGACGAGGACACCGCCGTGTACTA CTGCGGCTACGGCGACTACGACTACTTCGACTACTGGGGCCAGGGAACCCTGGTGACCGTGTCTT CCGGAGGTGGTGGATCCGAGGTGCAGCTGGTCGAGTCTGGAGGAGGATTGGTGCAGCCTGGAGGG |

| | | | | |
|---|---|---|---|---|
| | | | | TCATTGAAACTCTCATGTGCAGCCTCTGGATTCACCTTCAATAAGTACGCCATGAACTGGGTCCG<br>CCAGGCTCCAGGAAAGGGTTTGGAATGGGTTGCTCGCATAAGAAGTAAATATAATAATTATGCAA<br>CATATTATGCCGATTCAGTGAAAGACAGGTTCACCATCTCCAGAGATGATTCAAAAAACACTGCC<br>TATCTACAAATGAACAACTTGAAAACTGAGGACACTGCCGTGTACTACTGTGTGAGACATGGAA<br>CTTCGGTAATAGCTACATATCCTACTGGGCTTACTGGGGCCAAGGGACTCTGGTCACCGTCTCCT<br>CAGGTGGTGGTGGTTCTGGCGGCGGCGGCTCCGGTGGTGGTGGTTCTCAGACTGTTGTGACTCAG<br>GAACCTTCACTCACCGTATCACCTGGTGGAACAGTCACACTCACTTGTGGCTCCTCGACTGGGGC<br>TGTTACATCTGGCAACTACCCAAACTGGGTCCAACAAAAACCAGGTCAGGCACCCCGTGGTCTAA<br>TAGGTGGGACTAAGTTCCTCGCCCCCGGTACTCCTGCCAGATTCTCAGGCTCCCTGCTTGGAGGC<br>AAGGCTGCCCTCACCCTCTCAGGGGTACAGCCAGAGGATGAGGCAGAATATTACTGTGTTCTATG<br>GTACAGCAACCGCTGGGTGTTCGGTGGAGGAACCAAACTGACTGTCCTA |
| 907. | ENSI L-CDR1 | artificial | aa | RASQNVGTAVA |
| 908. | ENSI L-CDR2 | artificial | aa | SASNRYT |
| 909. | ENSI L-CDR3 | artificial | aa | QQYTNYPMYT |
| 910. | ENSI H-CDR1 | artificial | aa | DYVIH |
| 911. | ENSI H-CDR2 | artificial | aa | YINPYDDDTTYNQKFKG |
| 912. | ENSI H-CDR3 | artificial | aa | RGNSYDGYFDYSMDY |
| 913. | Human endosialin | artificial | nt | ATGCTGCTGCGCCTGTTGCTGGCCTGGGCGGCCGCAGGGCCCACACTGGGCCAGGACCCCTGGGC<br>TGCTGAGCCCCGTGCCGCCTGCGGCCCCAGCAGCTGCTACGCTCTCTTCCCACGGCGCCGCACCT<br>TCCTGGAGGCCTGGCGGGCCTGCCGCGAGCTGGGGGGCGACCTGGCCACTCCTCGGACCCCCGAG<br>GAGGCCCAGCGTGTGGACAGCCTGGTGGGTGCGGGCCCAGCCAGCCGGCTGCTGTGGATCGGGCT<br>GCAGCGGCAGGCCCGGCAATGCCAGCTGCAGCGCCCACTGCGCGGCTTCACGTGGACCACAGGGG<br>ACCAGGACACGGCTTTCACCAACTGGGCCCAGCCAGCCTCTGGAGGCCCCTGCCCGGCCCAGCGC<br>TGTGTGGCCCTGGAGGCAAGTGGCGAGCACCGCTGGCTGGAGGGCTCGTGCACGCTGGCTGTCGA<br>CGGCTACCTGTGCCAGTTTGGCTTCGAGGGCGCCTGCCCGGCGCTGCAAGATGAGGCGGGCCAGG<br>CCGGCCCAGCCGTGTATACCACGCCCTTCCACCTGGTCTCCACAGAGTTTGAGTGGCTGCCCTTC<br>GGCTCTGTGGCCGCTGTGCAGTGCCAGGCTGGCAGGGGAGCCTCTCTGCTCTGCGTGAAGCAGCC<br>TGAGGGAGGTGTGGGCTGGTCACGGGCTGGCCCCTGTGCCTGGGGACTGGCTGCAGCCCTGACA<br>ACGGGGGCTGCGAACACGAATGTGTGGAGGAGGTGGATGGTCACGTGTCCTGCCGCTGCACTGAG<br>GGCTTCCGGCTGGCAGCAGACGGGCGCAGTTGCGAGGACCCCTGTGCCCAGGCTCCGTGCGAGCA<br>GCAGTGTGAGCCCGGTGGGCCACAAGGCTACAGCTGCCACTGTCGCCTGGGTTTCCGGCCAGCGG<br>AGGATGATCCGCACCGCTGTGTGGACACAGATGAGTGCCAGATTGCCGGTGTGTGCCAGCAGATG<br>TGTGTCAACTACGTTGGTGGCTTCGAGTGTTATTGTAGCGAGGGACATGAGCTGGAGGCTGATGG<br>CATCAGCTGCAGCCCTGCAGGGGCCATGGGTGCCCAGGCTTCCCAGGACCTCGGAGATGAGTTGC<br>TGGATGACGGGGAGGATGAGGAAGATGAAGACGAGGCCTGGAAGGCCTTCAACGGTGGCTGGACG<br>GAGATGCCTGGGATCCTGTGGATGGAGCCTACGCAGCCGCCTGACTTTGCCCTGGCCTATAGACC |

| | | | | |
|---|---|---|---|---|
| | | | | GAGCTTCCCAGAGGACAGAGAGCCACAGATACCCTACCCGGAGCCCACCTGGCCACCCCCGCTCA<br>GTGCCCCCAGGGTCCCCTACCACTCCTCAGTGCTCTCCGTCACCCGGCCTGTGGTGGTCTCTGCC<br>ACGCATCCCACACTGCCTTCTGCCCACCAGCCTCCTGTGATCCCTGCCACACACCCAGCTTTGTC<br>CCGTGACCACCAGATCCCCGTGATCGCAGCCAACTATCCAGATCTGCCTTCTGCCTACCAACCCG<br>GTATTCTCTCTGTCTCTCATTCAGCACAGCCTCCTGCCCACCAGCCCCCTATGATCTCAACCAAA<br>TATCCGGAGCTCTTCCCTGCCCACCAGTCCCCCATGTTTCCAGACACCCGGGTCGCTGGCACCCA<br>GACCACCACTCATTTGCCTGGAATCCCACCTAACCATGCCCCTCTGGTCACCACCCTCGGTGCCC<br>AGCTACCCCCTCAAGCCCCAGATGCCCTTGTCCTCAGAACCCAGGCCACCCAGCTTCCCATTATC<br>CCAACTGCCCAGCCCTCTCTGACCACCACCTCCAGGTCCCCTGTGTCTCCTGCCCATCAAATCTC<br>TGTGCCTGCTGCCACCCAGCCCGCAGCCCTCCCCACCCTCCTGCCCTCTCAGAGCCCCACTAACC<br>AGACCTCACCCATCAGCCCTACACATCCCCATTCCAAAGCCCCCCAAATCCCAAGGGAAGATGGC<br>CCCAGTCCCAAGTTGGCCCTGTGGCTGCCCTCACCAGCTCCCACAGCAGCCCCAACAGCCCTGGG<br>GGAGGCTGGTCTTGCCGAGCACAGCCAGAGGGATGACCGGTGGCTGCTGGTGGCACTCCTGGTGC<br>CAACGTGTGTCTTTTTGGTGGTCCTGCTTGCACTGGGCATCGTGTACTGCACCCGCTGTGGCCCC<br>CATGCACCCAACAAGCGCATCACTGACTGCTATCGCTGGGTCATCCATGCTGGGAGCAAGAGCCC<br>AACAGAACCCATGCCCCCCAGGGGCAGCCTCACAGGGGTGCAGACCTGCAGAACCAGCGTGGACT<br>ACAAAGATGATGACGATAAGTGA |
| 914. | Human endosialin | artificial | aa | MLLRLLLAWAAAGPTLGQDPWAAEPRAACGPSSCYALFPRRRTFLEAWRACRELGGDLATPRTPE<br>EAQRVDSLVGAGPASRLLWIGLQRQARQCQLQRPLRGFTWTTGDQDTAFTNWAQPASGGPCPAQR<br>CVALEASGEHRWLEGSCTLAVDGYLCQFGFEGACPALQDEAGQAGPAVYTTPFHLVSTEFEWLPF<br>GSVAAVQCQAGRGASLLCVKQPEGGVGWSRAGPLCLGTGCSPDNGGCEHECVEEVDGHVSCRCTE<br>GFRLAADGRSCEDPCAQAPCEQQCEPGGPQGYSCHCRLGFRPAEDDPHRCVDTDECQIAGVCQQM<br>CVNYVGGFECYCSEGHELEADGISCSPAGAMGAQASQDLGDELLDDGEDEEDEDEAWKAFNGGWT<br>EMPGILWMEPTQPPDFALAYRPSFPEDREPQIPYPEPTWPPPLSAPRVPYHSSVLSVTRPVVVSA<br>THPTLPSAHQPPVIPATHPALSRDHQIPVIAANYPDLPSAYQPGILSVSHSAQPPAHQPPMISTK<br>YPELFPAHQSPMFPDTRVAGTQTTTHLPGIPPNHAPLVTTLGAQLPPQAPDALVLRTQATQLPII<br>PTAQPSLTTTSRSPVSPAHQISVPAATQPAALPTLLPSQSPTNQTSPISPTHPHSKAPQIPREDG<br>PSPKLALWLPSPAPTAAPTALGEAGLAEHSQRDDRWLLVALLVPTCVFLVVLLALGIVYCTRCGP<br>HAPNKRITDCYRWVIHAGSKSPTEPMPPRGSLTGVQTCRTSVDYKDDDDK |
| 915. | Macaque endosialin | artificial | nt | ATGCTGCTGCGCCTGTTGCTGGCCTGGGCGGCCGCAGGGCCCACACTGGGCCAGGACCCCTGGGC<br>TGCTGAGCCCCGCTCCGCCTGCGGCCCCAGCAGCTGCTACGCTCTCTTTCCACGGCGCCGCACCT<br>TTCTGGAGGCCTGGCGGGCCTGCCGCGAGCTGGGGGGCGACCTGGCCACTCCTCGGACCCCCGAG<br>GAGGCCCAGCGTGTGGACAACCTGGTGGGTGCAGGCCCGGCCAGCCGGCTGCTGTGGATCGGCCT<br>GCAGCGGCAGGCCCGGCAATGCCAGCTGCAGCGCCCACTGCGAGGCTTCACGTGGACCACAGGGG<br>ACCAGGACACGGCTTTCACCAACTGGGCCCAGCCAGCCACTGGAGGCCCCTGCCCGGCCCAGCGC<br>TGTGTGGCCCTGGAGGCGAGTGGCGAGCACCGCTGGCTGGAGGGCTCATGCACGCTGGCTGTCGA<br>CGGCTACTTGTGCCAGTTTGGCTTCGAGGGTGCCTGCCCAGCGCTGCAAGACGAAGCGGGCCAGG |

| | | | | |
|---|---|---|---|---|
| | | | | CCGGCCCAGCCGTCTATACCACGCCCTTCCACCTGGTCTCCACAGAGTTTGAGTGGCTACCCTTC<br>GGCTCTGTGGCCGCTGTGCAGTGCCAGGCTGGCAGGGGAGCCTCTCTGCTCTGCGTGAAGCAGCC<br>CGAGGGAGGTGTGGGCTGGTCACGGGCAGGGCCCCTGTGCCTGGGGGCTGGCTGCAGCCCTGACA<br>ACGGGGGCTGCGAACACGAATGTGTGGAGGAGGTGGATGGTCACGTGTCCTGCCGCTGCACTGAG<br>GGCTTCCGGCTGGCAGCAGACGGGCGCAGTTGCGAGGATCCCTGTGCCCAGGCTCCATGCGAGCA<br>GCAGTGTGAGCCCGGTGGGCCACAAGGCTACAGCTGCCACTGTCGCCTGGGTTTCCGACCTGCAG<br>AGGATGATCCGCACCGCTGTGTGGACACAGATGAGTGCCAGATTGCCGGTGTGTGCCAGCAGATG<br>TGTGTCAACTACGTTGGTGGCTTCGAGTGTTATTGTAGTGAGGGTCATGAGCTGGAGGCTGATGG<br>CATCAGCTGCAGCCCTGCAGGGGCCATGGGTGCCCGGGCTTCCCAGGACCTCGGAGATGAGTTGC<br>TGGATGATGGGGAGGATGAGGAAGATGAAGACGAGGCCTGGGAGGCCTTTGGCGGTGGCTGGACG<br>GAGATGCCTGGGATCCTGTGGATGGAGCCTACTCAGCCGCCTGACTTTGCCCTGGCCTATAGACC<br>GAGCTTCCCAGAGGACAGAGAGCCACAGATACCCTACCCGGAGCCCACCTGGCCGCCCCCGCTCA<br>GTGCCCCCAGGGTCCCCTACCACTCCTCAGTGCTCTCCGTCACCCGGCCTGTGGTGGTCTCTGCC<br>ACGCGCCCCACACTGCCTTCCGCCCACCAGCCTCCTGTGATCCCTGCCACACACCCAGCTTTGTC<br>CCGTGACCACCAGATCCCCGTGATCGCAGCCAACTATCCAGATCTGCCTTCTGCCTACCAACCCG<br>GTATTCTCTCTGTCTCTCATCCAGCACAGCCCCCTGCCCACCAGCCCCCCATGATCTCAACCAGA<br>TATCCTGAGCTGTTCCCTGCCCACCAGTCCCCCATGTTTCCAGACACCCAGGTCGCTGGCACCCA<br>GACCACCACTCATTTGCCTGCAATCCCACCTAACCGTGCCCCTCTGGTCACCACACTCGGTGCCC<br>ATCAACCCCCTCAAGCCCCAGATGCCCCTGTCCTCAGAACCCAGGCTACCCAGCTTCCCATTATC<br>CCTACTGCCCAGCCGTCTCTGACCACCAGCTCCAGGTCCCCTGTGTCTCCTGCCCATCAAGTCTC<br>TGTGCCTGCTGCCACCCAGCCCCCAGCCCTCCCCACCCTCCTGCCCTCTCAGAGCCCCACTAACC<br>AGACCTCACCCATCAGCCCTACACATCCCCATTCCAAAGTCCCCCAAATCCCAAGGGAAGATGGC<br>CCCAGTCCCAAGCTGGCCCTGTGGCTGCCCTCAGCAGCTCCCACAGCAGCCCCAACAGCCCTGGG<br>GGAGGCTAGTCTTGCCGAGCACATCCAGAGGGATGACCGGTGGCTTCTGGTGGCACTCCTCGTGC<br>CAACGTGTGTCTTTTTGGTGGTCCTGCTTGCACTGGGCATTGTGTACTGCACCCGCTGTGGCCCC<br>CATGCACCCAACAAGCGCATCACTGACTGCTATCGCTGGGTCACCCATGCTGGGAGCAAGAGTCC<br>AACAGAACCCATGCCCCCCAGGGGCAGCCTCACAGGGGTGCAGACCTGCAGAACCAGCGTGGACT<br>ACAAAGATGATGACGATAAGTGA |
| 916. | Macaque endosialin | artificial | aa | MLLRLLLAWAAAGPTLGQDPWAAEPRSACGPSSCYALFPRRRTFLEAWRACRELGGDLATPRTPE<br>EAQRVDNLVGAGPASRLLWIGLQRQARQCQLQRPLRGFTWTTGDQDTAFTNWAQPATGGPCPAQR<br>CVALEASGEHRWLEGSCTLAVDGYLCQFGFEGACPALQDEAGQAGPAVYTTPFHLVSTEFEWLPF<br>GSVAAVQCQAGRGASLLCVKQPEGGVGWSRAGPLCLGAGCSPDNGGCEHECVEEVDGHVSCRCTE<br>GFRLAADGRSCEDPCAQAPCEQQCEPGGPQGYSCHCRLGFRPAEDDPHRCVDTDECQIAGVCQQM<br>CVNYVGGFECYCSEGHELEADGISCSPAGAMGARASQDLGDELLDDGEDEEDEDEAWEAFGGGWT<br>EMPGILWMEPTQPPDFALAYRPSFPEDREPQIPYPEPTWPPPLSAPRVPYHSSVLSVTRPVVVSA<br>TRPTLPSAHQPPVIPATHPALSRDHQIPVIAANYPDLPSAYQPGILSVSHPAQPPAHQPPMISTR<br>YPELFPAHQSPMFPDTQVAGTQTTTHLPAIPPNRAPLVTTLGAHQPPQAPDAPVLRTQATQLPII |

| | | | | |
|---|---|---|---|---|
| | | | | PTAQPSLTTSSRSPVSPAHQVSVPAATQPPALPTLLPSQSPTNQTSPISPTHPHSKVPQIPREDG PSPKLALWLPSAAPTAAPTALGEASLAEHIQRDDRWLLVALLVPTCVFLVVLLALGIVYCTRCGP HAPNKRITDCYRWVTHAGSKSPTEPMPPRGSLTGVQTCRTSVDYKDDDDK |
| 917. | Forward primer | artificial | nt | ATATGAATTCGCCACCATGCTGCTGCGCCTGTTGCTGGCC |
| 918. | Reverse primer | artificial | nt | GTCTTCATCTTCCTCATCCTCCCC |
| 919. | Forward primer | artificial | nt | GTCAACTACGTTGGTGGCTTCGAGTG |
| 920. | Reverse primer | artificial | nt | GGTCTAGATCACTTATCGTCATCATCTTTGTAGTCCACGCTGGTTCTGCAGGTCTGC |
| 921. | Primer 5'ED-VH-A-XhoI | artificial | nt | CCAGCTCTCGAGTCAGGASCTGAGSTGRWGAAGCCTGGGGCTTCAGTGAAGRTGTCCTGCAAGGC TTCTGGATACACATTCACT |
| 922. | Primer 3'ED-VH-B | artificial | nt | AAT ATA TCC MAT CCA CTC AAG GCK CTK TCC AKK TSY CTG CYT CAY CCA GTG TAT AAC ATA GTC AGT GAA TGT GTA TCC AGA |
| 923. | Primer 5'ED-VH-C | artificial | nt | CTT GAG TGG ATK GGA TAT ATT AAT CCT TAT GAT GAT GAT ACTA CC TAC AAC CAG AAG TTC AAG GGC |
| 924. | Primer 3'ED-VH-D | artificial | nt | T GAG CTS CAT GTA GGC TGT GYT GGM GGA TKT GWC TMS AGT MAW TGT GRC CYG GCC CTT GAA CTT CTG GTT |
| 925. | Primer 5'ED-VH-E | artificial | nt | C ACA GCC TAC ATG SAA CTC ARC AGC CTG ASA TCT GAG GAC ACT GCA GTC TAT TAC TGT GCA AGA AGG GGG |
| 926. | Primer 3'ED-VH-F-BstEII | artificial | nt | CCT GAT GGT GAC CAA GGT TCC TTG ACC CCA GTA GTC CAT AGA ATA GTC GAA GTA ACC ATC ATA GGA GTT CCC CCT TCT TGC ACA GTA |
| 927. | Primer 5'ED-VL-A-SacI | artificial | nt | CCA GTC GAG CTC CAG CTG ACC CAG TCT CMA ARM TTC MTG TCC RCA TCA GTA GGA GAC AGA GTC NNS ATC ACC TGC AGG GCC AG |
| 928. | Primer 3'ED-VL-B | artificial | nt | TCC TGG TTT CTG TTG ATA CCA GGC TAC AGC AGT ACC CAC ATT CTG ACT GGC CCT GCA GGT GAT |
| 929. | Primer 5'ED-VL-C | artificial | nt | TAT CAA CAG AAA CCA GGA MAA KCC CCT AAA TTA CTG ATT TACT CG GCA TCG AAT CGG TAC ACT GGA GTC CCT |
| 930. | Primer 3'ED-VL-D | artificial | nt | GCT GAT GGT GAG AGT GAA MTC TGT CCC AGA TCC ACT GCC TGA GAA GCG AYY AGG GAC TCC AGT GTA CCG |
| 931. | Primer 5'ED-VL-E | artificial | nt | TTC ACT CTC ACC ATC AGC ART MTG CAG YCT GAA GAC YTS GCA RMT TAT TWC TGC CAG CAA TAT ACC AAC |
| 932. | Primer 3'ED-VL-F-BsiWI/SpeI | artificial | nt | CCT GAT ACT AGT CGT ACG TTT TAT TTC CAG CTT GGT CCC CTG TCC AAA CGT ATA CAT GGG ATA GTT GGT ATA TTG CTG GCA |
| 933. | Flag-tag | artificial | nt | DYKDDDDK |
| 934. | Ep 5-10 VL | artificial | aa | ELVMTQSPSSLTVTAGEKVTMSCKSSQSLLNSGNQKNYLTWYQQKPGQPPKLLIYWASTRESGVP DRFTGSGSGTDFTLTISSVQAEDLAVYYCQNDYSYPLTFGAGTKLEIK |
| 935. | Ep 5-10 LCDR1 | artificial | aa | KSSQSLLNSGNQKNYLT |

| | | | | |
|---|---|---|---|---|
| 936. | Ep 5-10 LCDR2 | artificial | aa | WASTRES |
| 937. | Ep 5-10 LCDR3 | artificial | aa | QNDYSYPLT |
| 938. | Ep 5-10 VL | artificial | nt | GAGCTCGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGACCTGGTACCAGCAGAAACCAGGGCAGCCTCCTAAACTGTTGATCTACTGGGCATCCACTAGGGAATCTGGGGTCCCTGATCGCTTCACAGGCAGTGGATCTGGAACAGATTTCACTCTCACCATCAGCAGTGTGCAGGCTGAAGACCTGGCAGTTTATTACTGTCAGAATGATTATAGTTATCCGCTCACGTTCGGTGCTGGGACCAAGCTTGAGATCAAA |
| 939. | Ep 5-10 HL | artificial | aa | EVQLLEQSGAELVRPGTSVKISCKASGYAFTNYWLGWVKQRPGHGLEWIGDIFPGSGNIHYNEKFKGKATLTADKSSSTAYMQLSSLTFEDSAVYFCARLRNWDEPMDYWGQGTTVTVSS |
| 940. | Ep 5-10 HCDR1 | artificial | aa | NYWLG |
| 941. | Ep 5-10 HCDR2 | artificial | aa | DIFPGSGNIHYNEKFKG |
| 942. | Ep 5-10 HCDR3 | artificial | aa | LRNWDEPMDY |
| 943. | Ep 5-10 HL | artificial | nt | GAGGTGCAGCTGCTCGAGCAGTCTGGAGCTGAGCTGGTAAGGCCTGGGACTTCAGTGAAGATATCCTGCAAGGCTTCTGGATACGCCTTCACTAACTACTGGCTAGGTTGGGTAAAGCAGAGGCCTGGACATGGACTTGAGTGGATTGGAGATATTTTCCCTGGAAGTGGTAATATCCACTACAATGAGAAGTTCAAGGGCAAAGCCACACTGACTGCAGACAAATCTTCGAGCACAGCCTATATGCAGCTCAGTAGCCTGACATTTGAGGACTCTGCTGTCTATTTCTGTGCAAGACTGAGGAACTGGGACGAGCCTATGGACTACTGGGGCCAAGGGACCACGGTCACCGTCTCCTCC |
| 944. | Ep 5-10 LH x I2C HL | artificial | aa | ELVMTQSPSSLTVTAGEKVTMSCKSSQSLLNSGNQKNYLTWYQQKPGQPPKLLIYWASTRESGVPDRFTGSGSGTDFTLTISSVQAEDLAVYYCQNDYSYPLTFGAGTKLEIKGGGGSGGGGSGGGGSEVQLLEQSGAELVRPGTSVKISCKASGYAFTNYWLGWVKQRPGHGLEWIGDIFPGSGNIHYNEKFKGKATLTADKSSSTAYMQLSSLTFEDSAVYFCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 945. | Ep 5-10 LH x I2C HL | artificial | nt | GAGCTCGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGACCTGGTACCAGCAGAAACCAGGGCAGCCTCCTAAACTGTTGATCTACTGGGCATCCACTAGGGAATCTGGGGTCCCTGATCGCTTCACAGGCAGTGGATCTGGAACAGATTTCACTCTCACCATCAGCAGTGTGCAGGCTGAAGACCTGGCAGTTTATTACTGTCAGAATGATTATAGTTATCCGCTCACGTTCGGTGCTGGGACCAAGCTTGAGATCAAAGGTGGTGGTGGTTCTGGCGGCGGCGGCTCCGGTGGTGGTGGTTCTGAGGTG |

| | | | | |
|---|---|---|---|---|
| | | | | CAGCTGCTCGAGCAGTCTGGAGCTGAGCTGGTAAGGCCTGGGACTTCAGTGAAGATATCCTGCAA<br>GGCTTCTGGATACGCCTTCACTAACTACTGGCTAGGTTGGGTAAAGCAGAGGCCTGGACATGGAC<br>TTGAGTGGATTGGAGATATTTTCCCTGGAAGTGGTAATATCCACTACAATGAGAAGTTCAAGGGC<br>AAAGCCACACTGACTGCAGACAAATCTTCGAGCACAGCCTATATGCAGCTCAGTAGCCTGACATT<br>TGAGGACTCTGCTGTCTATTTCTGTGCAAGACTGAGGAACTGGGACGAGCCTATGGACTACTGGG<br>GCCAAGGGACCACGGTCACCGTCTCCTCCGGAGGTGGTGGATCCGAGGTGCAGCTGGTCGAGTCT<br>GGAGGAGGATTGGTGCAGCCTGGAGGGTCATTGAAACTCTCATGTGCAGCCTCTGGATTCACCTT<br>CAATAAGTACGCCATGAACTGGGTCCGCCAGGCTCCAGGAAAGGGTTTGGAATGGGTTGCTCGCA<br>TAAGAAGTAAATATAATAATTATGCAACATATTATGCCGATTCAGTGAAAGACAGGTTCACCATC<br>TCCAGAGATGATTCAAAAAACACTGCCTATCTACAAATGAACAACTTGAAAACTGAGGACACTGC<br>CGTGTACTACTGTGTGAGACATGGGAACTTCGGTAATAGCTACATATCCTACTGGGCTTACTGGG<br>GCCAAGGGACTCTGGTCACCGTCTCCTCAGGTGGTGGTGGTTCTGGCGGCGGCGGCTCCGGTGGT<br>GGTGGTTCTCAGACTGTTGTGACTCAGGAACCTTCACTCACCGTATCACCTGGTGGAACAGTCAC<br>ACTCACTTGTGGCTCCTCGACTGGGGCTGTTACATCTGGCAACTACCCAAACTGGGTCCAACAAA<br>AACCAGGTCAGGCACCCCGTGGTCTAATAGGTGGGACTAAGTTCCTCGCCCCCGGTACTCCTGCC<br>AGATTCTCAGGCTCCCTGCTTGGAGGCAAGGCTGCCCTCACCCTCTCAGGGGTACAGCCAGAGGA<br>TGAGGCAGAATATTACTGTGTTCTATGGTACAGCAACCGCTGGGTGTTCGGTGGAGGAACCAAAC<br>TGACTGTCCTA |
| 946. | Ep 5-10 LH x H2C HL | artificial | aa | ELVMTQSPSSLTVTAGEKVTMSCKSSQSLLNSGNQKNYLTWYQQKPGQPPKLLIYWASTRESGVP<br>DRFTGSGSGTDFTLTISSVQAEDLAVYYCQNDYSYPLTFGAGTKLEIKGGGGSGGGGSGGGGSEV<br>QLLEQSGAELVRPGTSVKISCKASGYAFTNYWLGWVKQRPGHGLEWIGDIFPGSGNIHYNEKFKG<br>KATLTADKSSSTAYMQLSSLTFEDSAVYFCARLRNWDEPMDYWGQGTTVTVSSGGGGSEVQLVES<br>GGGLVQPGGSLKLSCAASGFTFNKYAMNWVRQAPGKGLEWVARIRSKYNNYATYYADSVKDRFTI<br>SRDDSKNTAYLQMNNLKTEDTAVYYCVRHGNFGNSYISYWAYWGQGTLVTVSSGGGGSGGGGSGG<br>GGSQTVVTQEPSLTVSPGGTVTLTCGSSTGAVTSGYYPNWVQQKPGQAPRGLIGGTKFLAPGTPA<br>RFSGSLLGGKAALTLSGVQPEDEAEYYCALWYSNRWVFGGGTKLTVL |
| 947. | Ep 5-10 LH x H2C HL | artificial | nt | GAGCTCGTGATGACACAGTCTCCATCCTCCCTGACTGTGACAGCAGGAGAGAAGGTCACTATGAG<br>CTGCAAGTCCAGTCAGAGTCTGTTAAACAGTGGAAATCAAAAGAACTACTTGACCTGGTACCAGC<br>AGAAACCAGGGCAGCCTCCTAAACTGTTGATCTACTGGGCATCCACTAGGGAATCTGGGGTCCCT<br>GATCGCTTCACAGGCAGTGGATCTGGAACAGATTTCACTCTCACCATCAGCAGTGTGCAGGCTGA<br>AGACCTGGCAGTTTATTACTGTCAGAATGATTATAGTTATCCGCTCACGTTCGGTGCTGGGACCA<br>AGCTTGAGATCAAAGGTGGTGGTGGTTCTGGCGGCGGCGGCTCCGGTGGTGGTGGTTCTGAGGTG<br>CAGCTGCTCGAGCAGTCTGGAGCTGAGCTGGTAAGGCCTGGGACTTCAGTGAAGATATCCTGCAA<br>GGCTTCTGGATACGCCTTCACTAACTACTGGCTAGGTTGGGTAAAGCAGAGGCCTGGACATGGAC<br>TTGAGTGGATTGGAGATATTTTCCCTGGAAGTGGTAATATCCACTACAATGAGAAGTTCAAGGGC<br>AAAGCCACACTGACTGCAGACAAATCTTCGAGCACAGCCTATATGCAGCTCAGTAGCCTGACATT |

| | | | | |
|---|---|---|---|---|
| | | | | TGAGGACTCTGCTGTCTATTTCTGTGCAAGACTGAGGAACTGGGACGAGCCTATGGACTACTGGG GCCAAGGGACCACGGTCACCGTCTCCTCCGGAGGTGGTGGATCCGAGGTGCAGCTGGTCGAGTCT GGAGGAGGATTGGTGCAGCCTGGAGGGTCATTGAAACTCTCATGTGCAGCCTCTGGATTCACCTT CAATAAGTACGCCATGAACTGGGTCCGCCAGGCTCCAGGAAAGGGTTTGGAATGGGTTGCTCGCA TAAGAAGTAAATATAATAATTATGCAACATATTATGCCGATTCAGTGAAAGACAGGTTCACCATC TCCAGAGATGATTCAAAAAACACTGCCTATCTACAAATGAACAACTTGAAAACTGAGGACACTGC CGTGTACTACTGTGTGAGACATGGGAACTTCGGTAATAGCTACATATCCTACTGGGCTTACTGGG GCCAAGGGACTCTGGTCACCGTCTCCTCAGGTGGTGGTGGTTCTGGCGGCGGCGGCTCCGGTGGT GGTGGTTCTCAGACTGTTGTGACTCAGGAACCTTCACTCACCGTATCACCTGGTGGAACAGTCAC ACTCACTTGTGGCTCCTCGACTGGGGCTGTTACATCTGGCTACTACCCAAACTGGGTCCAACAAA AACCAGGTCAGGCACCCCGTGGTCTAATAGGTGGGACTAAGTTCCTCGCCCCCGGTACTCCTGCC AGATTCTCAGGCTCCCTGCTTGGAGGCAAGGCTGCCCTCACCCTCTCAGGGGTACAGCCAGAGGA TGAGGCAGAATATTACTGTGCTCTATGGTACAGCAACCGCTGGGTGTTCGGTGGAGGAACCAAAC TGACTGTCCTA |
| 948. | Ep 5-10 LH x F12Q HL | artificial | aa | ELVMTQSPSSLTVTAGEKVTMSCKSSQSLLNSGNQKNYLTWYQQKPGQPPKLLIYWASTRESGVP DRFTGSGSGTDFTLTISSVQAEDLAVYYCQNDYSYPLTFGAGTKLEIKGGGGSGGGGSGGGGSEV QLLEQSGAELVRPGTSVKISCKASGYAFTNYWLGWVKQRPGHGLEWIGDIFPGSGNIHYNEKFKG KATLTADKSSSTAYMQLSSLTFEDSAVYFCARLRNWDEPMDYWGQGTTVTVSSGGGGSEVQLVES GGGLVQPGGSLKLSCAASGFTFNSYAMNWVRQAPGKGLEWVARIRSKYNNYATYYADSVKGRFTI SRDDSKNTAYLQMNNLKTEDTAVYYCVRHGNFGNSYVSWWAYWGQGTLVTVSSGGGGSGGGGSGG GGSQTVVTQEPSLTVSPGGTVTLTCGSSTGAVTSGNYPNWVQQKPGQAPRGLIGGTKFLAPGTPA RFSGSLLGGKAALTLSGVQPEDEAEYYCVLWYSNRWVFGGGTKLTVL |
| 949. | Ep 5-10 LH x F12Q HL | artificial | nt | GAGCTCGTGATGACACAGTCTCCATCCTCCCTGACTGTGACAGCAGGAGAGAAGGTCACTATGAG CTGCAAGTCCAGTCAGAGTCTGTTAAACAGTGGAAATCAAAAGAACTACTTGACCTGGTACCAGC AGAAACCAGGGCAGCCTCCTAAACTGTTGATCTACTGGGCATCCACTAGGGAATCTGGGGTCCCT GATCGCTTCACAGGCAGTGGATCTGGAACAGATTTCACTCTCACCATCAGCAGTGTGCAGGCTGA AGACCTGGCAGTTTATTACTGTCAGAATGATTATAGTTATCCGCTCACGTTCGGTGCTGGGACCA AGCTTGAGATCAAAGGTGGTGGTGGTTCTGGCGGCGGCGGCTCCGGTGGTGGTGGTTCTGAGGTG CAGCTGCTCGAGCAGTCTGGAGCTGAGCTGGTAAGGCCTGGGACTTCAGTGAAGATATCCTGCAA GGCTTCTGGATACGCCTTCACTAACTACTGGCTAGGTTGGGTAAAGCAGAGGCCTGGACATGGAC TTGAGTGGATTGGAGATATTTTCCCTGGAAGTGGTAATATCCACTACAATGAGAAGTTCAAGGGC AAAGCCACACTGACTGCAGACAAATCTTCGAGCACAGCCTATATGCAGCTCAGTAGCCTGACATT TGAGGACTCTGCTGTCTATTTCTGTGCAAGACTGAGGAACTGGGACGAGCCTATGGACTACTGGG GCCAAGGGACCACGGTCACCGTCTCCTCCGGAGGTGGTGGATCCGAGGTGCAGCTGGTCGAGTCT GGAGGAGGATTGGTGCAGCCTGGAGGGTCATTGAAACTCTCATGTGCAGCCTCTGGATTCACCTT CAATAGCTACGCCATGAACTGGGTCCGCCAGGCTCCAGGAAAGGGTTTGGAATGGGTTGCTCGCA TAAGAAGTAAATATAATAATTATGCAACATATTATGCCGATTCAGTGAAAGGCAGGTTCACCATC |

| | | | | |
|---|---|---|---|---|
| | | | | TCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTTTCCTGGTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 950. | hEp 14-A1 L | artificial | aa | DIVMTQTPLSLPVTPGEPVSISCKSSQSLLNSGNQKNYLTWYLQKPGQSPQLLIYWASTRESGVPDRFSGSGSGTDFTLTISSVQAEDVGVYYCQNDYSYPLTFGQGTKLEIK |
| 951. | hEp 14-A1 LCDR1 | artificial | aa | KSSQSLLNSGNQKNYLT |
| 952. | hEp 14-A1 LCDR2 | artificial | aa | WASTRES |
| 953. | hEp 14-A1 LCDR3 | artificial | aa | QNDYSYPLT |
| 954. | hEp 14-A1 L | artificial | nt | GACATCGTGATGACACAGACTCCACTCTCCCTGCCTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCACAATCAGCAGTGTGCAGGCTGAAGACGTGGGAGTTTATTACTGTCAGAATGATTATAGTTATCCGCTCACGTTCGGTCAAGGGACCAAGCTTGAGATCAAA |
| 955. | hEp 14-A1 H | artificial | aa | QVQLVQSGAEVKKPGSSVKISCKASGYTFSNYWLGWVRQAPGQGLEWIGDIFPGSGNIHYNEKFKGRVTITADKSTSTAYMELSSLTSEDTAVYYCARLRNWDEPMDYWGQGTTVTVSS |
| 956. | hEp 14-A1 HCDR1 | artificial | aa | NYWLG |
| 957. | hEp 14-A1 HCDR2 | artificial | aa | DIFPGSGNIHYNEKFKG |
| 958. | hEp 14-A1 HCDR3 | artificial | aa | LRNWDEPMDY |
| 959. | hEp 14-A1 H | artificial | nt | CAGGTGCAGCTGGTCCAGTCTGGAGCTGAGGTGAAAAAGCCTGGGTCTTCAGTGAAGATATCCTGCAAGGCTTCTGGATACACCTTCTCTAACTACTGGCTAGGTTGGGTAAGGCAGGCGCCTGGACAGGGACTTGAGTGGATTGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCC |
| 960. | hEp 14-A1 LH x I2C HL | artificial | aa | DIVMTQTPLSLPVTPGEPVSISCKSSQSLLNSGNQKNYLTWYLQKPGQSPQLLIYWASTRESGVPDRFSGSGSGTDFTLTISSVQAEDVGVYYCQNDYSYPLTFGQGTKLEIKGGGGSGGGGSGGGGSQV |

| | | | | |
|---|---|---|---|---|
| | | | | QLVQSGAEVKKPGSSVKISCKASGYTFSNYWLGWVRQAPGQGLEWIGDIFPGSGNIHYNEKFKGRVTITADKSTSTAYMELSSLTSEDTAVYYCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 961. | hEp 14-A1 LH x I2C HL | artificial | nt | GACATCGTGATGACACAGACTCCACTCTCCCTGCCTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCACAATCAGCAGTGTGCAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAAGCCTGGGTCTTCAGTGAAGATATCCTGCAAGGCTTCTGGATACACCTTCTCTAACTACTGGCTAGGTTGGGTAAGGCAGGCGCCTGGACAGGGACTTGAGTGGATTGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 962. | hEp 14-D2 L | artificial | aa | DIVMTQTPLSLPVTPGEQVSISCKSSQSLLNSGNQKNYLTWYQQKPGQSPQLLIYWASTRESGVPDRFSGSGSGTDFTLKISRVEAEDVGVYYCQNDYSYPLTFGQGTKLEIK |
| 963. | hEp 14-D2 LCDR1 | artificial | aa | KSSQSLLNSGNQKNYLT |
| 964. | hEp 14-D2 LCDR2 | artificial | aa | WASTRES |
| 965. | hEp 14-D2 LCDR3 | artificial | aa | QNDYSYPLT |

| | | | | |
|---|---|---|---|---|
| 966. | hEp 14-D2 L | artificial | nt | GACATCGTGATGACACAGACTCCACTCTCCCTGCCTGTGACACCAGGAGAGCAGGTCTCTATCAG<br>CTGCAAGTCCAGTCAGAGTCTGTTAAACAGTGGAAATCAAAAGAACTACTTGACCTGGTACCAGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCAAAATCAGCCGTGTGGAGGCTGA<br>AGACGTGGGAGTTTATTACTGTCAGAATGATTATAGTTATCCGCTCACGTTCGGTCAAGGGACCA<br>AGCTTGAGATCAAA |
| 967. | hEp 14-D2 H | artificial | aa | QVQLVQSGAEVKKPGSSVKVSCKASGYTFSNYWLGWVRQAPGQGLEWMGDIFPGSGNIHYNEKFK<br>GRVTITADKSTSTAYMELSSLTSEDTAVYYCARLRNWDEPMDYWGQGTTVTVSS |
| 968. | hEp 14-D2 HCDR1 | artificial | aa | NYWLG |
| 969. | hEp 14-D2 HCDR2 | artificial | aa | DIFPGSGNIHYNEKFKG |
| 970. | hEp 14-D2 HCDR3 | artificial | aa | LRNWDEPMDY |
| 971. | hEp 14-D2 H | artificial | nt | CAGGTGCAGCTGGTCCAGTCTGGAGCTGAGGTGAAAAAGCCTGGGTCTTCAGTGAAGGTATCCTG<br>CAAGGCTTCTGGATACACCTTCTCTAACTACTGGCTAGGTTGGGTAAGGCAGGCGCCTGGACAGG<br>GACTTGAGTGGATGGGAGATATTTTCCCTGGAAGTGGTAATATCCACTACAATGAGAAGTTCAAG<br>GGCAGAGTCACAATCACTGCAGACAAATCTACGAGCACAGCCTATATGGAACTCAGTAGCCTGAC<br>ATCTGAGGACACTGCTGTCTATTACTGTGCAAGACTGAGGAACTGGGACGAGCCTATGGACTACT<br>GGGGCCAAGGGACCACGGTCACCGTCTCCTCC |
| 972. | hEp 14-D2 LH x I2C HL | artificial | aa | DIVMTQTPLSLPVTPGEQVSISCKSSQSLLNSGNQKNYLTWYQQKPGQSPQLLIYWASTRESGVP<br>DRFSGSGSGTDFTLKISRVEAEDVGVYYCQNDYSYPLTFGQGTKLEIKGGGGSGGGGSGGGGSQV<br>QLVQSGAEVKKPGSSVKVSCKASGYTFSNYWLGWVRQAPGQGLEWMGDIFPGSGNIHYNEKFKGR<br>VTITADKSTSTAYMELSSLTSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 973. | hEp 14-D2 LH x I2C HL | artificial | nt | GACATCGTGATGACACAGACTCCACTCTCCCTGCCTGTGACACCAGGAGAGCAGGTCTCTATCAG<br>CTGCAAGTCCAGTCAGAGTCTGTTAAACAGTGGAAATCAAAAGAACTACTTGACCTGGTACCAGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCAAAATCAGCCGTGTGGAGGCTGA<br>AGACGTGGGAGTTTATTACTGTCAGAATGATTATAGTTATCCGCTCACGTTCGGTCAAGGGACCA<br>AGCTTGAGATCAAAGGTGGTGGTGGTTCTGGCGGCGGCGGCTCCGGTGGTGGTGGTTCTCAGGTG<br>CAGCTGGTCCAGTCTGGAGCTGAGGTGAAAAAGCCTGGGTCTTCAGTGAAGGTATCCTGCAAGGC<br>TTCTGGATACACCTTCTCTAACTACTGGCTAGGTTGGGTAAGGCAGGCGCCTGGACAGGGACTTG<br>AGTGGATGGGAGATATTTTCCCTGGAAGTGGTAATATCCACTACAATGAGAAGTTCAAGGGCAGA<br>GTCACAATCACTGCAGACAAATCTACGAGCACAGCCTATATGGAACTCAGTAGCCTGACATCTGA |

| | | | | |
|---|---|---|---|---|
| | | | | GGACACTGCTGTCTATTACTGTGCAAGACTGAGGAACTGGGACGAGCCTATGGACTACTGGGGCC AAGGGACCACGGTCACCGTCTCCTCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATTCACCTTCAA TAAGTACGCCATGAACTGGGTCCGCCAGGCTCCAGGAAAGGGTTTGGAATGGGTTGCTCGCATAA GAAGTAAATATAATAATTATGCAACATATTATGCCGATTCAGTGAAAGACAGGTTCACCATCTCC AGAGATGATTCAAAAAACACTGCCTATCTACAAATGAACAACTTGAAAACTGAGGACACTGCCGT GTACTACTGTGTGAGACATGGGAACTTCGGTAATAGCTACATATCCTACTGGGCTTACTGGGGCC AAGGGACTCTGGTCACCGTCTCCTCAGGTGGTGGTGGTTCTGGCGGCGGCGGCTCCGGTGGTGGT GGTTCTCAGACTGTTGTGACTCAGGAACCTTCACTCACCGTATCACCTGGTGGAACAGTCACACT CACTTGTGGCTCCTCGACTGGGGCTGTTACATCTGGCAACTACCCAAACTGGGTCCAACAAAAAC CAGGTCAGGCACCCCGTGGTCTAATAGGTGGGACTAAGTTCCTCGCCCCCGGTACTCCTGCCAGA TTCTCAGGCTCCCTGCTTGGAGGCAAGGCTGCCCTCACCCTCTCAGGGGTACAGCCAGAGGATGA GGCAGAATATTACTGTGTTCTATGGTACAGCAACCGCTGGGTGTTCGGTGGAGGAACCAAACTGA CTGTCCTA |
| 974. | hEp 14-H8 L | artificial | aa | DIVMTQTPLSLPVTPGEPASISCKSSQSLLNSGNQKNYLTWYQQKPGQSPKLLIYWASTRESGVP DRFSGSGSGTDFTLKISRVEAEDVGVYYCQNDYSYPLTFGQGTKLEIK |
| 975. | hEp 14-H8 LCDR1 | artificial | aa | KSSQSLLNSGNQKNYLT |
| 976. | hEp 14-H8 LCDR2 | artificial | aa | WASTRES |
| 977. | hEp 14-H8 LCDR3 | artificial | aa | QNDYSYPLT |
| 978. | hEp 14-H8 L | artificial | nt | GACATCGTGATGACACAGACTCCACTCTCCCTGCCTGTGACACCAGGAGAGCCGGCCTCTATCAG CTGCAAGTCCAGTCAGAGTCTGTTAAACAGTGGAAATCAAAAGAACTACTTGACCTGGTACCAGC AGAAACCAGGGCAGTCTCCTAAACTGTTGATCTACTGGGCATCCACTAGGGAATCTGGGGTCCCT GATCGCTTCTCAGGCAGTGGATCTGGAACAGATTTCACTCTCAAAATCAGCCGTGTGGAGGCTGA AGACGTGGGAGTTTATTACTGTCAGAATGATTATAGTTATCCGCTCACGTTCGGTCAAGGGACCA AGCTTGAGATCAAA |
| 979. | hEp 14-H8 H | artificial | aa | QVQLVQSGAELKKPGSSVKVSCKASGYTFSNYWLGWVRQTPGQGLEWMGDIFPGSGNIHYNEKFK GRVTITADKSTSTAYMELSSLRSEDTAVYYCARLRNWDEPMDYWGQGTTVTVSS |
| 980. | hEp 14-H8 HCDR1 | artificial | aa | NYWLG |
| 981. | hEp 14-H8 HCDR2 | artificial | aa | DIFPGSGNIHYNEKFKG |
| 982. | hEp 14-H8 HCDR3 | artificial | aa | LRNWDEPMDY |
| 983. | hEp 14-H8 H | artificial | nt | CAGGTGCAGCTGGTCCAGTCTGGAGCTGAGCTGAAAAAGCCTGGGTCTTCAGTGAAGGTATCCTG CAAGGCTTCTGGATACACCTTCTCTAACTACTGGCTAGGTTGGGTAAGGCAGACGCCTGGACAGG GACTTGAGTGGATGGGAGATATTTTCCCTGGAAGTGGTAATATCCACTACAATGAGAAGTTCAAG |

| | | | | |
|---|---|---|---|---|
| | | | | GGCAGAGTCACAATCACTGCAGACAAATCTACGAGCACAGCCTATATGGAACTCAGTAGCCTGAGATCTGAGGACACTGCTGTCTATTACTGTGCAAGACTGAGGAACTGGGACGAGCCTATGGACTACTGGGGCCAAGGGACCACGGTCACCGTCTCCTCC |
| 984. | hEp 14-H8 LH x I2C HL | artificial | aa | DIVMTQTPLSLPVTPGEPASISCKSSQSLLNSGNQKNYLTWYQQKPGQSPKLLIYWASTRESGVPDRFSGSGSGTDFTLKISRVEAEDVGVYYCQNDYSYPLTFGQGTKLEIKGGGGSGGGGSGGGGSQVQLVQSGAELKKPGSSVKVSCKASGYTFSNYWLGWVRQTPGQGLEWMGDIFPGSGNIHYNEKFKGRVTITADKSTSTAYMELSSLRSEDTAVYYCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 985. | hEp 14-H8 LH x I2C HL | artificial | nt | GACATCGTGATGACACAGACTCCACTCTCCCTGCCTGTGACACCAGGAGAGCCGGCCTCTATCAGCTGCAAGTCCAGTCAGAGTCTGTTAAACAGTGGAAATCAAAAGAACTACTTGACCTGGTACCAGCAGAAACCAGGGCAGTCTCCTAAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGCTGAAAAAGCCTGGGTCTTCAGTGAAGGTATCCTGCAAGGCTTCTGGATACACCTTCTCTAACTACTGGCTAGGTTGGGTAAGGCAGACGCCTGGACAGGGACTTGAGTGGATGGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGAGATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 986. | hEp 14-A6 L | artificial | aa | DIVMTQTPFSLTVTPGETVSISCKSSQSLLNSGNQKNYLTWYLQKPGQSPQLLIYWASTRESGVPDRFSGSGSGTDFTLTISSVEAEDVGVYYCQNDYSYPLTFGQGTKLEIK |

| | | | | |
|---|---|---|---|---|
| 987. | hEp 14-A6 LCDR1 | artificial | aa | KSSQSLLNSGNQKNYLT |
| 988. | hEp 14-A6 LCDR2 | artificial | aa | WASTRES |
| 989. | hEp 14-A6 LCDR3 | artificial | aa | QNDYSYPLT |
| 990. | hEp 14-A6 L | artificial | nt | GACATCGTGATGACACAGACTCCATTCTCCCTGACTGTGACACCAGGAGAGACGGTCTCTATCAG<br>CTGCAAGTCCAGTCAGAGTCTGTTAAACAGTGGAAATCAAAAGAACTACTTGACCTGGTACCTGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCAGTGTGGAGGCTGA<br>AGACGTGGGAGTTTATTACTGTCAGAATGATTATAGTTATCCGCTCACGTTCGGTCAAGGGACCA<br>AGCTTGAGATCAAA |
| 991. | hEp 14-A6 H | artificial | aa | QVQLVQSGAELVKPGSSVKVSCKASGYTFSNYWLGWVRQAPGQGLEWIGDIFPGSGNIHYNEKFK<br>GKVTITADKSTSTAYMELSSLRFEDTAVYYCARLRNWDEPMDYWGQGTTVTVSS |
| 992. | hEp 14-A6 HCDR1 | artificial | aa | NYWLG |
| 993. | hEp 14-A6 HCDR2 | artificial | aa | DIFPGSGNIHYNEKFKG |
| 994. | hEp 14-A6 HCDR3 | artificial | aa | LRNWDEPMDY |
| 995. | hEp 14-A6 H | artificial | nt | CAGGTGCAGCTGGTCCAGTCTGGAGCTGAGCTGGTAAAGCCTGGGTCTTCAGTGAAGGTATCCTG<br>CAAGGCTTCTGGATACACCTTCTCTAACTACTGGCTAGGTTGGGTAAGGCAGGCGCCTGGACAGG<br>GACTTGAGTGGATTGGAGATATTTTCCCTGGAAGTGGTAATATCCACTACAATGAGAAGTTCAAG<br>GGCAAAGTCACAATCACTGCAGACAAATCTACGAGCACAGCCTATATGGAACTCAGTAGCCTGAG<br>ATTTGAGGACACTGCTGTCTATTACTGTGCAAGACTGAGGAACTGGGACGAGCCTATGGACTACT<br>GGGGCCAAGGGACCACGGTCACCGTCTCCTCC |
| 996. | hEp 14-A6 LH x I2C HL | artificial | aa | DIVMTQTPFSLTVTPGETVSISCKSSQSLLNSGNQKNYLTWYLQKPGQSPQLLIYWASTRESGVP<br>DRFSGSGSGTDFTLTISSVEAEDVGVYYCQNDYSYPLTFGQGTKLEIKGGGGSGGGGSGGGGSQV<br>QLVQSGAELVKPGSSVKVSCKASGYTFSNYWLGWVRQAPGQGLEWIGDIFPGSGNIHYNEKFKGK<br>VTITADKSTSTAYMELSSLRFEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 997. | hEp 14-A6 LH x I2C HL | artificial | nt | GACATCGTGATGACACAGACTCCATTCTCCCTGACTGTGACACCAGGAGAGACGGTCTCTATCAG<br>CTGCAAGTCCAGTCAGAGTCTGTTAAACAGTGGAAATCAAAAGAACTACTTGACCTGGTACCTGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCAGTGTGGAGGCTGA<br>AGACGTGGGAGTTTATTACTGTCAGAATGATTATAGTTATCCGCTCACGTTCGGTCAAGGGACCA |

| | | | | |
|---|---|---|---|---|
| | | | | AGCTTGAGATCAAAGGTGGTGGTGGTTCTGGCGGCGGCGGCTCCGGTGGTGGTGGTTCTCAGGTGCAGCTGGTCCAGTCTGGAGCTGAGCTGGTAAAGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATTGGAGATATTTTCCCTGGAAGTGGTAATATCCACTACAATGAGAAGTTCAAGGGCAAAGTCACAATCACTGCAGACAAATCTACGAGCACAGCCTATATGGAACTCAGTAGCCTGAGATTTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 998. | hEp 14-D1 L | artificial | aa | DIVMTQTPFSLTVTPGEPASISCKSSQSLLNSGNQKNYLTWYQQKPGQPPQLLIYWASTRESGVPDRFSGSGSGTDFTLKISRVEAEDVAVYYCQNDYSYPLTFGQGTKLEIK |
| 999. | hEp 14-D1 LCDR1 | artificial | aa | KSSQSLLNSGNQKNYLT |
| 1000. | hEp 14-D1 LCDR2 | artificial | aa | WASTRES |
| 1001. | hEp 14-D1 LCDR3 | artificial | aa | QNDYSYPLT |
| 1002. | hEp 14-D1 L | artificial | nt | GACATCGTGATGACACAGACTCCATTCTCCCTGACTGTGACACCAGGAGAGCCGGCCTCTATCAGCTGCAAGTCCAGTCAGAGTCTGTTAAACAGTGGAAATCAAAAGAACTACTTGACCTGGTACCAGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGCAGTTTATTACTGTCAGAATGATTATAGTTATCCGCTCACGTTCGGTCAAGGGACCAAGCTTGAGATCAAA |
| 1003. | hEp 14-D1 H | artificial | aa | QVQLVQSGAELKKPGSSVKISCKASGYTFSNYWLGWVRQAPGQGLEWMGDIFPGSGNIHYNEKFKGRVTLTADKSTSTAYMELSSLTSEDTAVYYCARLRNWDEPMDYWGQGTTVTVSS |
| 1004. | hEp 14-D1 HCDR1 | artificial | aa | NYWLG |

| | | | | |
|---|---|---|---|---|
| 1005. | hEp 14-D1 HCDR2 | artificial | aa | DIFPGSGNIHYNEKFKG |
| 1006. | hEp 14-D1 HCDR3 | artificial | aa | LRNWDEPMDY |
| 1007. | hEp 14-D1 H | artificial | nt | CAGGTGCAGCTGGTCCAGTCTGGAGCTGAGCTGAAAAAGCCTGGGTCTTCAGTGAAGATATCCTG<br>CAAGGCTTCTGGATACACCTTCTCTAACTACTGGCTAGGTTGGGTAAGGCAGGCGCCTGGACAGG<br>GACTTGAGTGGATGGGAGATATTTTCCCTGGAAGTGGTAATATCCACTACAATGAGAAGTTCAAG<br>GGCAGAGTCACACTCACTGCAGACAAATCTACGAGCACAGCCTATATGGAACTCAGTAGCCTGAC<br>ATCTGAGGACACTGCTGTCTATTACTGTGCAAGACTGAGGAACTGGGACGAGCCTATGGACTACT<br>GGGGCCAAGGGACCACGGTCACCGTCTCCTCC |
| 1008. | hEp 14-D1 LH x I2C HL | artificial | aa | DIVMTQTPFSLTVTPGEPASISCKSSQSLLNSGNQKNYLTWYQQKPGQPPQLLIYWASTRESGVP<br>DRFSGSGSGTDFTLKISRVEAEDVAVYYCQNDYSYPLTFGQGTKLEIKGGGGSGGGGSGGGGSQV<br>QLVQSGAELKKPGSSVKISCKASGYTFSNYWLGWVRQAPGQGLEWMGDIFPGSGNIHYNEKFKGR<br>VTLTADKSTSTAYMELSSLTSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 1009. | hEp 14-D1 LH x I2C HL | artificial | nt | GACATCGTGATGACACAGACTCCATTCTCCCTGACTGTGACACCAGGAGAGCCGGCCTCTATCAG<br>CTGCAAGTCCAGTCAGAGTCTGTTAAACAGTGGAAATCAAAAGAACTACTTGACCTGGTACCAGC<br>AGAAACCAGGGCAGCCTCCTCAACTGTTGATCTACTGGGCATCCACTAGGGAATCTGGGGTCCCT<br>GATCGCTTCTCAGGCAGTGGATCTGGAACAGATTTCACTCTCAAAATCAGCCGTGTGGAGGCTGA<br>AGACGTGGCAGTTTATTACTGTCAGAATGATTATAGTTATCCGCTCACGTTCGGTCAAGGGACCA<br>AGCTTGAGATCAAAGGTGGTGGTGGTTCTGGCGGCGGCGGCTCCGGTGGTGGTGGTTCTCAGGTG<br>CAGCTGGTCCAGTCTGGAGCTGAGCTGAAAAAGCCTGGGTCTTCAGTGAAGATATCCTGCAAGGC<br>TTCTGGATACACCTTCTCTAACTACTGGCTAGGTTGGGTAAGGCAGGCGCCTGGACAGGGACTTG<br>AGTGGATGGGAGATATTTTCCCTGGAAGTGGTAATATCCACTACAATGAGAAGTTCAAGGGCAGA<br>GTCACACTCACTGCAGACAAATCTACGAGCACAGCCTATATGGAACTCAGTAGCCTGACATCTGA<br>GGACACTGCTGTCTATTACTGTGCAAGACTGAGGAACTGGGACGAGCCTATGGACTACTGGGGCC<br>AAGGGACCACGGTCACCGTCTCCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA<br>GAAGTAAATATAATAATTATGCAACATATTATGCCGATTCAGTGAAAGACAGGTTCACCATCTCC<br>AGAGATGATTCAAAAAACACTGCCTATCTACAAATGAACAACTTGAAAACTGAGGACACTGCCGT<br>GTACTACTGTGTGAGACATGGGAACTTCGGTAATAGCTACATATCCTACTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT |

| | | | | |
|---|---|---|---|---|
| | | | | CACTTGTGGCTCCTCGACTGGGGCTGTTACATCTGGCAACTACCCAAACTGGGTCCAACAAAAAC CAGGTCAGGCACCCCGTGGTCTAATAGGTGGGACTAAGTTCCTCGCCCCCGGTACTCCTGCCAGA TTCTCAGGCTCCCTGCTTGGAGGCAAGGCTGCCCTCACCCTCTCAGGGGTACAGCCAGAGGATGA GGCAGAATATTACTGTGTTCTATGGTACAGCAACCGCTGGGTGTTCGGTGGAGGAACCAAACTGA CTGTCCTA |
| 1010. | hEp 14-G6 L | artificial | aa | DIVMTQTPLSLTVTPGEPVSISCKSSQSLLNSGNQKNYLTWYLQKPGQPPQLLIYWASTRESGVP DRFSGSGSGTDFTLKISRVQAEDVGVYYCQNDYSYPLTFGQGTKLEIK |
| 1011. | hEp 14-G6 LCDR1 | artificial | aa | KSSQSLLNSGNQKNYLT |
| 1012. | hEp 14-G6 LCDR2 | artificial | aa | WASTRES |
| 1013. | hEp 14-G6 LCDR3 | artificial | aa | QNDYSYPLT |
| 1014. | hEp 14-G6 L | artificial | nt | GACATCGTGATGACACAGACTCCACTCTCCCTGACTGTGACACCAGGAGAGCCGGTCTCTATCAG CTGCAAGTCCAGTCAGAGTCTGTTAAACAGTGGAAATCAAAAGAACTACTTGACCTGGTACCTGC AGAAACCAGGGCAGCCTCCTCAACTGTTGATCTACTGGGCATCCACTAGGGAATCTGGGGTCCCT GATCGCTTCTCAGGCAGTGGATCTGGAACAGATTTCACTCTCAAAATCAGCCGTGTGCAGGCTGA AGACGTGGGAGTTTATTACTGTCAGAATGATTATAGTTATCCGCTCACGTTCGGTCAAGGGACCA AGCTTGAGATCAAA |
| 1015. | hEp 14-G6 H | artificial | aa | QVQLVQSGAEVKKPGSSVKVSCKASGYTFSNYWLGWVRQAPGQGLEWMGDIFPGSGNIHYNEKFK GRATITADKSTSTAYMELSSLTSEDTAVYYCARLRNWDEPMDYWGQGTTVTVSS |
| 1016. | hEp 14-G6 HCDR1 | artificial | aa | NYWLG |
| 1017. | hEp 14-G6 HCDR2 | artificial | aa | DIFPGSGNIHYNEKFKG |
| 1018. | hEp 14-G6 HCDR3 | artificial | aa | LRNWDEPMDY |
| 1019. | hEp 14-G6 H | artificial | nt | CAGGTGCAGCTGGTCCAGTCTGGAGCTGAGGTGAAAAAGCCTGGGTCTTCAGTGAAGGTATCCTG CAAGGCTTCTGGATACACCTTCTCTAACTACTGGCTAGGTTGGGTAAGGCAGGCGCCTGGACAGG GACTTGAGTGGATGGGAGATATTTTCCCTGGAAGTGGTAATATCCACTACAATGAGAAGTTCAAG GGCAGAGCCACAATCACTGCAGACAAATCTACGAGCACAGCCTATATGGAACTCAGTAGCCTGAC ATCTGAGGACACTGCTGTCTATTACTGTGCAAGACTGAGGAACTGGGACGAGCCTATGGACTACT GGGGCCAAGGGACCACGGTCACCGTCTCCTCC |
| 1020. | hEp 14-G6 LH x I2C HL | artificial | aa | DIVMTQTPLSLTVTPGEPVSISCKSSQSLLNSGNQKNYLTWYLQKPGQPPQLLIYWASTRESGVP DRFSGSGSGTDFTLKISRVQAEDVGVYYCQNDYSYPLTFGQGTKLEIKGGGGSGGGGSGGGGSQV QLVQSGAEVKKPGSSVKVSCKASGYTFSNYWLGWVRQAPGQGLEWMGDIFPGSGNIHYNEKFKGR ATITADKSTSTAYMELSSLTSEDTAVYYCARLRNWDEPMDYWGQGTTVTVSSGGGGSEVQLVESG GGLVQPGGSLKLSCAASGFTFNKYAMNWVRQAPGKGLEWVARIRSKYNNYATYYADSVKDRFTIS RDDSKNTAYLQMNNLKTEDTAVYYCVRHGNFGNSYISYWAYWGQGTLVTVSSGGGGSGGGGSGGG |

| | | | | |
|---|---|---|---|---|
| | | | | GSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1021. | hEp 14-G6 LH x I2C HL | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGCAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATGGGAGATATTTTCCCTGGAAGTGGTAATATCCACTACAATGAGAAGTTCAAGGGCAGAGCCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1022. | hEp 14-H4 L | artificial | aa | DIVMTQTPLSLPVTPGETASISCKSSQSLLNSGNQKNYLTWYQQKPGQSPQLLIYWASTRESGVPDRFSGSGSGTDFTLKISSVEAEDVGVYYCQNDYSYPLTFGQGTKLEIK |
| 1023. | hEp 14-H4 LCDR1 | artificial | aa | KSSQSLLNSGNQKNYLT |
| 1024. | hEp 14-H4 LCDR2 | artificial | aa | WASTRES |
| 1025. | hEp 14-H4 LCDR3 | artificial | aa | QNDYSYPLT |
| 1026. | hEp 14-H4 L | artificial | nt | GACATCGTGATGACACAGACTCCACTCTCCCTGCCTGTGACACCAGGAGAGACGGCCTCTATCAGCTGCAAGTCCAGTCAGAGTCTGTTAAACAGTGGAAATCAAAAGAACTACTTGACCTGGTACCAGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCAGTGTGGAGGCTGA |

| | | | | |
|---|---|---|---|---|
| | | | | AGACGTGGGAGTTTATTACTGTCAGAATGATTATAGTTATCCGCTCACGTTCGGTCAAGGGACCA<br>AGCTTGAGATCAAA |
| 1027. | hEp 14-H4 H | artificial | aa | QVQLVQSGAEVVKPGSSVKVSCKASGYTFSNYWLGWVRQAPGQGLEWMGDIFPGSGNIHYNEKFK<br>GRVTITADKSTSTAYMELSSLTSEDTAVYYCARLRNWDEPMDYWGQGTTVTVSS |
| 1028. | hEp 14-H4 HCDR1 | artificial | aa | NYWLG |
| 1029. | hEp 14-H4 HCDR2 | artificial | aa | DIFPGSGNIHYNEKFKG |
| 1030. | hEp 14-H4 HCDR3 | artificial | aa | LRNWDEPMDY |
| 1031. | hEp 14-H4 H | artificial | nt | CAGGTGCAGCTGGTCCAGTCTGGAGCTGAGGTGGTAAAGCCTGGGTCTTCAGTGAAGGTATCCTG<br>CAAGGCTTCTGGATACACCTTCTCTAACTACTGGCTAGGTTGGGTAAGGCAGGCGCCTGGACAGG<br>GACTTGAGTGGATGGGAGATATTTTCCCTGGAAGTGGTAATATCCACTACAATGAGAAGTTCAAG<br>GGCAGAGTCACAATCACTGCAGACAAATCTACGAGCACAGCCTATATGGAACTCAGTAGCCTGAC<br>ATCTGAGGACACTGCTGTCTATTACTGTGCAAGACTGAGGAACTGGGACGAGCCTATGGACTACT<br>GGGGCCAAGGGACCACGGTCACCGTCTCCTCC |
| 1032. | hEp 14-H4 LH x I2C HL | artificial | aa | DIVMTQTPLSLPVTPGETASISCKSSQSLLNSGNQKNYLTWYQQKPGQSPQLLIYWASTRESGVP<br>DRFSGSGSGTDFTLKISSVEAEDVGVYYCQNDYSYPLTFGQGTKLEIKGGGGSGGGGSGGGGSQV<br>QLVQSGAEVVKPGSSVKVSCKASGYTFSNYWLGWVRQAPGQGLEWMGDIFPGSGNIHYNEKFKGR<br>VTITADKSTSTAYMELSSLTSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 1033. | hEp 14-H4 LH x I2C HL | artificial | nt | GACATCGTGATGACACAGACTCCACTCTCCCTGCCTGTGACACCAGGAGAGACGGCCTCTATCAG<br>CTGCAAGTCCAGTCAGAGTCTGTTAAACAGTGGAAATCAAAAGAACTACTTGACCTGGTACCAGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCAAAATCAGCAGTGTGGAGGCTGA<br>AGACGTGGGAGTTTATTACTGTCAGAATGATTATAGTTATCCGCTCACGTTCGGTCAAGGGACCA<br>AGCTTGAGATCAAAGGTGGTGGTGGTTCTGGCGGCGGCGGCTCCGGTGGTGGTGGTTCTCAGGTG<br>CAGCTGGTCCAGTCTGGAGCTGAGGTGGTAAAGCCTGGGTCTTCAGTGAAGGTATCCTGCAAGGC<br>TTCTGGATACACCTTCTCTAACTACTGGCTAGGTTGGGTAAGGCAGGCGCCTGGACAGGGACTTG<br>AGTGGATGGGAGATATTTTCCCTGGAAGTGGTAATATCCACTACAATGAGAAGTTCAAGGGCAGA<br>GTCACAATCACTGCAGACAAATCTACGAGCACAGCCTATATGGAACTCAGTAGCCTGACATCTGA<br>GGACACTGCTGTCTATTACTGTGCAAGACTGAGGAACTGGGACGAGCCTATGGACTACTGGGGCC<br>AAGGGACCACGGTCACCGTCTCCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA |

| | | | | |
|---|---|---|---|---|
| | | | | GAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1034. | hEp 17-A6 L | artificial | aa | DIVMTQTPLSLTVTPGEPVSISCKSSQSLLNSGNQKNYLTWYLQKPGQPPQLLIYWASTRESGVPDRFSGSGSGTDFTLKISRVEAEDVGVYYCQNDYSYPLTFGQGTKLEIK |
| 1035. | hEp 17-A6 LCDR1 | artificial | aa | KSSQSLLNSGNQKNYLT |
| 1036. | hEp 17-A6 LCDR2 | artificial | aa | WASTRES |
| 1037. | hEp 17-A6 LCDR3 | artificial | aa | QNDYSYPLT |
| 1038. | hEp 17-A6 L | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAA |
| 1039. | hEp 17-A6 H | artificial | aa | QVQLVQSGAEVKRPGSSVKISCKASGYTFSNYWLGWVKQAPGQGLEWMGDIFPGSGNIHYNEKFKGRVTITADKSTSTAYMELSSLTSEDTAVYFCARLRNWDEPMDYWGQGTTVTVSS |
| 1040. | hEp 17-A6 HCDR1 | artificial | aa | NYWLG |
| 1041. | hEp 17-A6 HCDR2 | artificial | aa | DIFPGSGNIHYNEKFKG |
| 1042. | hEp 17-A6 HCDR3 | artificial | aa | LRNWDEPMDY |
| 1043. | hEp 17-A6 H | artificial | nt | CAGGTGCAGCTGGTCCAGTCTGGAGCTGAGGTGAAAAGGCCTGGGTCTTCAGTGAAGATATCCTGCAAGGCTTCTGGATACACCTTCTCTAACTACTGGCTAGGTTGGGTAAAGCAGGCGCCTGGACAGGGACTTGAGTGGATGGGAGATATTTTCCCCGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTTCTGTGCAAGACTGAGGAACTGGGACGAGCCTATGGACTACTGGGGCCAAGGGACCACGGTCACCGTCTCCTCC |

| | | | | |
|---|---|---|---|---|
| 1044. | hEp 17-A6 LH x I2C HL | artificial | aa | DIVMTQTPLSLTVTPGEPVSISCKSSQSLLNSGNQKNYLTWYLQKPGQPPQLLIYWASTRESGVPDRFSGSGSGTDFTLKISRVEAEDVGVYYCQNDYSYPLTFGQGTKLEIKGGGGSGGGGSGGGGSQVQLVQSGAEVKRPGSSVKISCKASGYTFSNYWLGWVKQAPGQGLEWMGDIFPGSGNIHYNEKFKGRVTITADKSTSTAYMELSSLTSEDTAVYFCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1045. | hEp 17-A6 LH x I2C HL | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAGGCCTGGGTCTTCAGTGAAGATATCCTGCAAGGCTTCTGGATACACCTTCTCTAACTACTGGCTAGGTTGGGTAAAGCAGGCGCCTGGACAGGGACTTGAGTGGATGGGAGATATTTTCCCCGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTTCTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1046. | hEp 17-E9 L | artificial | aa | DIVMTQTPLSLTVTPGEPVSISCKSSQSLLNSGNQKNYLTWYLQKPGQPPKLLIYWASTRESGVPDRFSGSGSGTDFTLNISRVEAEDVGVYYCQNDYSYPLTFGQGTKLEIK |
| 1047. | hEp 17-E9 LCDR1 | artificial | aa | KSSQSLLNSGNQKNYLT |

| | | | | |
|---|---|---|---|---|
| 1048. | hEp 17-E9 LCDR2 | artificial | aa | WASTRES |
| 1049. | hEp 17-E9 LCDR3 | artificial | aa | QNDYSYPLT |
| 1050. | hEp 17-E9 L | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTAAACTGTTGATCTACTGGGCATCCACTAGGGAATCTGGGGTCCCTGATCGCTTCTCAGGCAGTGGATCTGGAACAGATTTCACTCTCAACATCAGCCGTGTGGAGGCTGAAGACGTGGGAGTTTATTACTGTCAGAATGATTATAGTTATCCGCTCACGTTCGGTCAAGGGACCAAGCTTGAGATCAAA |
| 1051. | hEp 17-E9 H | artificial | aa | QVQLVQSGAEVKKPGSSVKVSCKASGYTFSNYWLGWVKQAPGHGLEWMGDIFPGSGNIHYNEKFKGRVTITADKSTSTAYMELSSLRSEDTAVYYCARLRNWDEPMDYWGQGTTVTVSS |
| 1052. | hEp 17-E9 HCDR1 | artificial | aa | NYWLG |
| 1053. | hEp 17-E9 HCDR2 | artificial | aa | DIFPGSGNIHYNEKFKG |
| 1054. | hEp 17-E9 HCDR3 | artificial | aa | LRNWDEPMDY |
| 1055. | hEp 17-E9 H | artificial | nt | CAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAAGCAGGCGCCTGGACATGGACTTGAGTGGATGGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGAGATCTGAGGACACTGCTGTCTATTACTGTGCAAGACTGAGGAACTGGGACGAGCCTATGGACTACTGGGGCCAAGGGACCACGGTCACCGTCTCCTCC |
| 1056. | hEp 17-E9 LH x I2C HL | artificial | aa | DIVMTQTPLSLTVTPGEPVSISCKSSQSLLNSGNQKNYLTWYLQKPGQPPKLLIYWASTRESGVPDRFSGSGSGTDFTLNISRVEAEDVGVYYCQNDYSYPLTFGQGTKLEIKGGGGSGGGGSGGGGSQVQLVQSGAEVKKPGSSVKVSCKASGYTFSNYWLGWVKQAPGHGLEWMGDIFPGSGNIHYNEKFKGRVTITADKSTSTAYMELSSLRSEDTAVYYCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1057. | hEp 17-E9 LH x I2C HL | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTAAACTGTTGATCTACTGGGCATCCACTAGGGAATCTGGGGTCCCTGATCGCTTCTCAGGCAGTGGATCTGGAACAGATTTCACTCTCAACATCAGCCGTGTGGAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTG |

| | | | | |
|---|---|---|---|---|
| | | | | CAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAAGCAGGCGCCTGGACATGGACTTGAGTGGATGGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGAGATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1058. | hEp 18-A6 L | artificial | aa | DIVMTQTPLSLPVTPGEQVSMSCKSSQSLLNSGNQKNYLTWYLQKPGQSPQLLIYWASTRESGVPDRFSGSGSGTDFTLNISSVEAEDVGVYYCQNDYSYPLTFGQGTKLEIK |
| 1059. | hEp 18-A6 LCDR1 | artificial | aa | KSSQSLLNSGNQKNYLT |
| 1060. | hEp 18-A6 LCDR2 | artificial | aa | WASTRES |
| 1061. | hEp 18-A6 LCDR3 | artificial | aa | QNDYSYPLT |
| 1062. | hEp 18-A6 L | artificial | nt | GACATCGTGATGACACAGACTCCACTCTCCCTGCCTGTGACACCAGGAGAGCAGGTCTCTATGAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAACATCAGCAGTGTGGAGGCTGAAGACGTGGGAGTTTATTACTGTCAGAATGATTATAGTTATCCGCTCACGTTCGGTCAAGGGACCAAGCTTGAGATCAAA |
| 1063. | hEp 18-A6 H | artificial | aa | QVQLVQSGAEVKKPGSSVKVSCKASGYTFSNYWLGWVRQTPGQGLEWMGDIFPGSGNIHYNEKFKGRVTITADKSTSTAYMELSSLTSEDTAVYYCARLRNWDEPMDYWGQGTTVTVSS |
| 1064. | hEp 18-A6 HCDR1 | artificial | aa | NYWLG |
| 1065. | hEp 18-A6 HCDR2 | artificial | aa | DIFPGSGNIHYNEKFKG |

| | | | | |
|---|---|---|---|---|
| 1066. | hEp 18-A6 HCDR3 | artificial | aa | LRNWDEPMDY |
| 1067. | hEp 18-A6 H | artificial | nt | CAGGTGCAGCTGGTCCAGTCTGGAGCTGAGGTGAAAAAGCCTGGGTCTTCAGTGAAGGTATCCTG CAAGGCTTCTGGATACACCTTCTCTAACTACTGGCTAGGTTGGGTAAGGCAGACGCCTGGACAGG GACTTGAGTGGATGGGAGATATTTTCCCTGGAAGTGGTAATATCCACTACAATGAGAAGTTCAAG GGCAGAGTCACAATCACTGCAGACAAATCTACGAGCACAGCCTATATGGAACTCAGTAGCCTGAC ATCTGAGGACACTGCTGTCTATTACTGTGCAAGACTGAGGAACTGGGACGAGCCTATGGACTACT GGGGCCAAGGGACCACGGTCACCGTCTCCTCC |
| 1068. | hEp 18-E3 L | artificial | aa | DIVMTQTPLSLPVTPGEPVSISCKSSQSLLNSGNQKNYLTWYLQKPGQPPQLLIYWASTRESGVP DRFSGSGSGTDFTLTISSVQAEDVGVYYCQNDYSYPLTFGQGTKLEIK |
| 1069. | hEp 18-E3 LCDR1 | artificial | aa | KSSQSLLNSGNQKNYLT |
| 1070. | hEp 18-E3 LCDR2 | artificial | aa | WASTRES |
| 1071. | hEp 18-E3 LCDR3 | artificial | aa | QNDYSYPLT |
| 1072. | hEp 18-E3 L | artificial | nt | GACATCGTGATGACACAGACTCCACTCTCCCTGCCTGTGACACCAGGAGAGCCGGTCTCTATCAG CTGCAAGTCCAGTCAGAGTCTGTTAAACAGTGGAAATCAAAAGAACTACTTGACCTGGTACCTGC AGAAACCAGGGCAGCCTCCTCAACTGTTGATCTACTGGGCATCCACTAGGGAATCTGGGGTCCCT GATCGCTTCTCAGGCAGTGGATCTGGAACAGATTTCACTCTCACCATCAGCAGTGTGCAGGCTGA AGACGTGGGAGTTTATTACTGTCAGAATGATTATAGTTATCCGCTCACGTTCGGTCAAGGGACCA AGCTTGAGATCAAA |
| 1073. | hEp 18-E3 H | artificial | aa | QVQLVQSGAELVRPGSSVKISCKASGYAFSNYWLGWVRQAPGQGLEWMGDIFPGSGNIHYNEKFK GRVTLTADKSTSTAYMQLSSLTSEDTAVYYCARLRNWDEPMDYWGQGTTVTVSS |
| 1074. | hEp 18-E3 HCDR1 | artificial | aa | NYWLG |
| 1075. | hEp 18-E3 HCDR2 | artificial | aa | DIFPGSGNIHYNEKFKG |
| 1076. | hEp 18-E3 HCDR3 | artificial | aa | LRNWDEPMDY |
| 1077. | hEp 18-E3 H | artificial | nt | CAGGTGCAGCTGGTCCAGTCTGGAGCTGAGCTGGTAAGGCCTGGGTCTTCAGTGAAGATATCCTG CAAGGCTTCTGGATACGCCTTCTCTAACTACTGGCTAGGTTGGGTAAGGCAGGCGCCTGGACAGG GACTTGAGTGGATGGGAGATATTTTCCCTGGAAGTGGTAATATCCACTACAATGAGAAGTTCAAG GGCAGAGTCACACTCACTGCAGACAAATCTACGAGCACAGCCTATATGCAACTCAGTAGCCTGAC ATCTGAGGACACTGCTGTCTATTACTGTGCAAGACTGAGGAACTGGGACGAGCCTATGGACTACT GGGGCCAAGGGACCACGGTCACCGTCTCCTCC |
| 1078. | hEp 18-E3 LH x I2C HL | artificial | aa | DIVMTQTPLSLPVTPGEPVSISCKSSQSLLNSGNQKNYLTWYLQKPGQPPQLLIYWASTRESGVP DRFSGSGSGTDFTLTISSVQAEDVGVYYCQNDYSYPLTFGQGTKLEIKGGGGSGGGGSGGGGSQV QLVQSGAELVRPGSSVKISCKASGYAFSNYWLGWVRQAPGQGLEWMGDIFPGSGNIHYNEKFKGR |

| | | | | |
|---|---|---|---|---|
| | | | | VTLTADKSTSTAYMQLSSLTSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 1079. | hEp 18-E3 LH x I2C HL | artificial | nt | GACATCGTGATGACACAGACTCCACTCTCCCTGCCTGTGACACCAGGAGAGCCGGTCTCTATCAG<br>CTGCAAGTCCAGTCAGAGTCTGTTAAACAGTGGAAATCAAAAGAACTACTTGACCTGGTACCTGC<br>AGAAACCAGGGCAGCCTCCTCAACTGTTGATCTACTGGGCATCCACTAGGGAATCTGGGGTCCCT<br>GATCGCTTCTCAGGCAGTGGATCTGGAACAGATTTCACTCTCACCATCAGCAGTGTGCAGGCTGA<br>AGACGTGGGAGTTTATTACTGTCAGAATGATTATAGTTATCCGCTCACGTTCGGTCAAGGGACCA<br>AGCTTGAGATCAAAGGTGGTGGTGGTTCTGGCGGCGGCGGCTCCGGTGGTGGTGGTTCTCAGGTG<br>CAGCTGGTCCAGTCTGGAGCTGAGCTGGTAAGGCCTGGGTCTTCAGTGAAGATATCCTGCAAGGC<br>TTCTGGATACGCCTTCTCTAACTACTGGCTAGGTTGGGTAAGGCAGGCGCCTGGACAGGGACTTG<br>AGTGGATGGGAGATATTTTCCCTGGAAGTGGTAATATCCACTACAATGAGAAGTTCAAGGGCAGA<br>GTCACACTCACTGCAGACAAATCTACGAGCACAGCCTATATGCAACTCAGTAGCCTGACATCTGA<br>GGACACTGCTGTCTATTACTGTGCAAGACTGAGGAACTGGGACGAGCCTATGGACTACTGGGGCC<br>AAGGGACCACGGTCACCGTCTCCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA<br>GAAGTAAATATAATAATTATGCAACATATTATGCCGATTCAGTGAAAGACAGGTTCACCATCTCC<br>AGAGATGATTCAAAAAACACTGCCTATCTACAAATGAACAACTTGAAAACTGAGGACACTGCCGT<br>GTACTACTGTGTGAGACATGGGAACTTCGGTAATAGCTACATATCCTACTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT<br>CACTTGTGGCTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGATGA<br>GGCAGAATATTACTGTGTTCTATGGTACAGCAACCGCTGGGTGTTCGGTGGAGGAACCAAACTGA<br>CTGTCCTA |
| 1080. | hEp 18-F11 L | artificial | aa | DIVMTQTPPSLTVTAGEPVSISCKSSQSLLNSGNQKNYLTWYLQKPGQSPQLLIYWASTRESGVP<br>DRFSGSGSGTDFTLKISSVQAEDVGVYYCQNDYSYPLTFGQGTKLEIK |
| 1081. | hEp 18-F11 LCDR1 | artificial | aa | KSSQSLLNSGNQKNYLT |
| 1082. | hEp 18-F11 LCDR2 | artificial | aa | WASTRES |
| 1083. | hEp 18-F11 LCDR3 | artificial | aa | QNDYSYPLT |

| | | | | |
|---|---|---|---|---|
| 1084. | hEp 18-F11 L | artificial | nt | GACATCGTGATGACACAGACTCCACCCTCCCTGACTGTGACAGCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCAGTGTGCAGGCTGAAGACGTGGGAGTTTATTACTGTCAGAATGATTATAGTTATCCGCTCACGTTCGGTCAAGGGACCAAGCTTGAGATCAAA |
| 1085. | hEp 18-F11 H | artificial | aa | QVQLVQSGAEVKRPGSSVKVSCKASGYTFSNYWLGWVRQAPGQGLEWIGDIFPGSGNIHYNEKFKGRVTITADKSTSTAYMELSSLRFEDTAVYYCARLRNWDEPMDYWGQGTTVTVSS |
| 1086. | hEp 18-F11 HCDR1 | artificial | aa | NYWLG |
| 1087. | hEp 18-F11 HCDR2 | artificial | aa | DIFPGSGNIHYNEKFKG |
| 1088. | hEp 18-F11 HCDR3 | artificial | aa | LRNWDEPMDY |
| 1089. | hEp 18-F11 H | artificial | nt | CAGGTGCAGCTGGTCCAGTCTGGAGCTGAGGTGAAAAGGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATTGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGAGATTTGAGGACACTGCTGTCTATTACTGTGCAAGACTGAGGAACTGGGACGAGCCTATGGACTACTGGGGCCAAGGGACCACGGTCACCGTCTCCTCC |
| 1090. | hEp 18-F11 LH x I2C HL | artificial | aa | DIVMTQTPPSLTVTAGEPVSISCKSSQSLLNSGNQKNYLTWYLQKPGQSPQLLIYWASTRESGVPDRFSGSGSGTDFTLKISSVQAEDVGVYYCQNDYSYPLTFGQGTKLEIKGGGGSGGGGSGGGGSQVQLVQSGAEVKRPGSSVKVSCKASGYTFSNYWLGWVRQAPGQGLEWIGDIFPGSGNIHYNEKFKGRVTITADKSTSTAYMELSSLRFEDTAVYYCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1091. | hEp 18-F11 LH x I2C HL | artificial | nt | GACATCGTGATGACACAGACTCCACCCTCCCTGACTGTGACAGCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCAGTGTGCAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAGGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATTGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGAGATTTGA |

| | | | | |
|---|---|---|---|---|
| | | | | GGACACTGCTGTCTATTACTGTGCAAGACTGAGGAACTGGGACGAGCCTATGGACTACTGGGGCC<br>AAGGGACCACGGTCACCGTCTCCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA<br>GAAGTAAATATAATAATTATGCAACATATTATGCCGATTCAGTGAAAGACAGGTTCACCATCTCC<br>AGAGATGATTCAAAAAACACTGCCTATCTACAAATGAACAACTTGAAAACTGAGGACACTGCCGT<br>GTACTACTGTGTGAGACATGGGAACTTCGGTAATAGCTACATATCCTACTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT<br>CACTTGTGGCTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGATGA<br>GGCAGAATATTACTGTGTTCTATGGTACAGCAACCGCTGGGTGTTCGGTGGAGGAACCAAACTGA<br>CTGTCCTA |
| 1092. | hEp 18-F12 L | artificial | aa | DIVMTQTPLSLTVTPGEPVSISCKSSQSLLNSGNQKNYLTWYLQKPGQPPQLLIYWASTRESGVP<br>DRFSGSGSGTDFTLTISRVEAEDVGVYYCQNDYSYPLTFGQGTKLEIK |
| 1093. | hEp 18-F12 LCDR1 | artificial | aa | KSSQSLLNSGNQKNYLT |
| 1094. | hEp 18-F12 LCDR2 | artificial | aa | WASTRES |
| 1095. | hEp 18-F12 LCDR3 | artificial | aa | QNDYSYPLT |
| 1096. | hEp 18-F12 L | artificial | nt | GACATCGTGATGACACAGACTCCACTCTCCCTGACTGTGACACCAGGAGAGCCGGTCTCTATCAG<br>CTGCAAGTCCAGTCAGAGTCTGTTAAACAGTGGAAATCAAAAGAACTACTTGACCTGGTACCTGC<br>AGAAACCAGGGCAGCCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCCGTGTGGAGGCTGA<br>AGACGTGGGAGTTTATTACTGTCAGAATGATTATAGTTATCCGCTCACGTTCGGTCAAGGGACCA<br>AGCTTGAGATCAAA |
| 1097. | hEp 18-F12 H | artificial | aa | QVQLVQSGAEVKKPGSSVKVSCKASGYTFSNYWLGWVRQRPGQGLEWIGDIFPGSGNIHYNEKFK<br>GRATITADKSTSTAYMELSSLRSEDTAVYYCARLRNWDEPMDYWGQGTTVTVSS |
| 1098. | hEp 18-F12 HCDR1 | artificial | aa | NYWLG |
| 1099. | hEp 18-F12 HCDR2 | artificial | aa | DIFPGSGNIHYNEKFKG |
| 1100. | hEp 18-F12 HCDR3 | artificial | aa | LRNWDEPMDY |
| 1101. | hEp 18-F12 L | artificial | nt | CAGGTGCAGCTGGTCCAGTCTGGAGCTGAGGTGAAAAAGCCTGGGTCTTCAGTGAAGGTATCCTG<br>CAAGGCTTCTGGATACACCTTCTCTAACTACTGGCTAGGTTGGGTAAGGCAGAGGCCTGGACAGG<br>GACTTGAGTGGATTGGAGATATTTTCCCTGGAAGTGGTAATATCCACTACAATGAGAAGTTCAAG |

| | | | | |
|---|---|---|---|---|
| | | | | GGCAGAGCCACAATCACTGCAGACAAATCTACGAGCACAGCCTATATGGAACTCAGTAGCCTGAG<br>ATCTGAGGACACTGCTGTCTATTACTGTGCAAGACTGAGGAACTGGGACGAGCCTATGGACTACT<br>GGGGCCAAGGGACCACGGTCACCGTCTCCTCC |
| 1102. | hEp 18-F12 LH x I2C HL | artificial | aa | DIVMTQTPLSLTVTPGEPVSISCKSSQSLLNSGNQKNYLTWYLQKPGQPPQLLIYWASTRESGVP<br>DRFSGSGSGTDFTLTISRVEAEDVGVYYCQNDYSYPLTFGQGTKLEIKGGGGSGGGGSGGGGSQV<br>QLVQSGAEVKKPGSSVKVSCKASGYTFSNYWLGWVRQRPGQGLEWIGDIFPGSGNIHYNEKFKGR<br>ATITADKSTSTAYMELSSLRSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 1103. | hEp 18-F12 LH x I2C HL | artificial | nt | GACATCGTGATGACACAGACTCCACTCTCCCTGACTGTGACACCAGGAGAGCCGGTCTCTATCAG<br>CTGCAAGTCCAGTCAGAGTCTGTTAAACAGTGGAAATCAAAAGAACTACTTGACCTGGTACCTGC<br>AGAAACCAGGGCAGCCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCCGTGTGGAGGCTGA<br>AGACGTGGGAGTTTATTACTGTCAGAATGATTATAGTTATCCGCTCACGTTCGGTCAAGGGACCA<br>AGCTTGAGATCAAAGGTGGTGGTGGTTCTGGCGGCGGCGGCTCCGGTGGTGGTGGTTCTCAGGTG<br>CAGCTGGTCCAGTCTGGAGCTGAGGTGAAAAAGCCTGGGTCTTCAGTGAAGGTATCCTGCAAGGC<br>TTCTGGATACACCTTCTCTAACTACTGGCTAGGTTGGGTAAGGCAGAGGCCTGGACAGGGACTTG<br>AGTGGATTGGAGATATTTTCCCTGGAAGTGGTAATATCCACTACAATGAGAAGTTCAAGGGCAGA<br>GCCACAATCACTGCAGACAAATCTACGAGCACAGCCTATATGGAACTCAGTAGCCTGAGATCTGA<br>GGACACTGCTGTCTATTACTGTGCAAGACTGAGGAACTGGGACGAGCCTATGGACTACTGGGGCC<br>AAGGGACCACGGTCACCGTCTCCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA<br>GAAGTAAATATAATAATTATGCAACATATTATGCCGATTCAGTGAAAGACAGGTTCACCATCTCC<br>AGAGATGATTCAAAAAACACTGCCTATCTACAAATGAACAACTTGAAAACTGAGGACACTGCCGT<br>GTACTACTGTGTGAGACATGGGAACTTCGGTAATAGCTACATATCCTACTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT<br>CACTTGTGGCTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGATGA<br>GGCAGAATATTACTGTGTTCTATGGTACAGCAACCGCTGGGTGTTCGGTGGAGGAACCAAACTGA<br>CTGTCCTA |
| 1104. | hEp 18-G1 L | artificial | aa | DIVMTQTPLSLTVTPGETVSISCKSSQSLLNSGNQKNYLTWYLQKPGQSPQLLIYWASTRESGVP<br>DRFSGSGSGTDFTLTISSVEAEDVGVYYCQNDYSYPLTFGQGTKLEIK |

| | | | | |
|---|---|---|---|---|
| 1105. | hEp 18-G1 LCDR1 | artificial | aa | KSSQSLLNSGNQKNYLT |
| 1106. | hEp 18-G1 LCDR2 | artificial | aa | WASTRES |
| 1107. | hEp 18-G1 LCDR3 | artificial | aa | QNDYSYPLT |
| 1108. | hEp 18-G1 L | artificial | nt | GACATCGTGATGACACAGACTCCACTCTCCCTGACTGTGACACCAGGAGAGACGGTCTCTATCAG<br>CTGCAAGTCCAGTCAGAGTCTGTTAAACAGTGGAAATCAAAAGAACTACTTGACCTGGTACCTGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCAGTGTGGAGGCTGA<br>AGACGTGGGAGTTTATTACTGTCAGAATGATTATAGTTATCCGCTCACGTTCGGTCAAGGGACCA<br>AGCTTGAGATCAAA |
| 1109. | hEp 18-G1 H | artificial | aa | QVQLVQSGAEVVKPGSSVKVSCKASGYTFSNYWLGWVRQTPGQGLEWIGDIFPGSGNIHYNEKFK<br>GKVTITADKSTSTAYMELSSLGSEDTAVYYCARLRNWDEPMDYWGQGTTVTVSS |
| 1110. | hEp 18-G1 HCDR1 | artificial | aa | NYWLG |
| 1111. | hEp 18-G1 HCDR2 | artificial | aa | DIFPGSGNIHYNEKFKG |
| 1112. | hEp 18-G1 HCDR3 | artificial | aa | LRNWDEPMDY |
| 1113. | hEp 18-G1 H | artificial | nt | CAGGTGCAGCTGGTCCAGTCTGGAGCTGAGGTGGTAAAGCCTGGGTCTTCAGTGAAGGTATCCTG<br>CAAGGCTTCTGGATACACCTTCTCTAACTACTGGCTAGGTTGGGTAAGGCAGACGCCTGGACAGG<br>GACTTGAGTGGATTGGAGATATTTTCCCTGGAAGTGGTAATATCCACTACAATGAGAAGTTCAAG<br>GGCAAAGTCACAATCACTGCAGACAAATCTACGAGCACAGCCTATATGGAACTCAGTAGCCTGGG<br>ATCTGAGGACACTGCTGTCTATTACTGTGCAAGACTGAGGAACTGGGACGAGCCTATGGACTACT<br>GGGGCCAAGGGACCACGGTCACCGTCTCCTCC |
| 1114. | hEp 18-G1 LH x I2C HL | artificial | aa | DIVMTQTPLSLTVTPGETVSISCKSSQSLLNSGNQKNYLTWYLQKPGQSPQLLIYWASTRESGVP<br>DRFSGSGSGTDFTLTISSVEAEDVGVYYCQNDYSYPLTFGQGTKLEIKGGGGSGGGGSGGGGSQV<br>QLVQSGAEVVKPGSSVKVSCKASGYTFSNYWLGWVRQTPGQGLEWIGDIFPGSGNIHYNEKFKGK<br>VTITADKSTSTAYMELSSLGSEDTAVYYCARLRNWDEPMDYWGQGTTVTVSSGGGGSEVQLVESG<br>GGLVQPGGSLKLSCAASGFTFNKYAMNWVRQAPGKGLEWVARIRSKYNNYATYYADSVKDRFTIS<br>RDDSKNTAYLQMNNLKTEDTAVYYCVRHGNFGNSYISYWAYWGQGTLVTVSSGGGGSGGGGSGGG<br>GSQTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 1115. | hEp 18-G1 LH x I2C HL | artificial | nt | GACATCGTGATGACACAGACTCCACTCTCCCTGACTGTGACACCAGGAGAGACGGTCTCTATCAG<br>CTGCAAGTCCAGTCAGAGTCTGTTAAACAGTGGAAATCAAAAGAACTACTTGACCTGGTACCTGC<br>AGAAACCAGGGCAGTCTCCTCAACTGTTGATCTACTGGGCATCCACTAGGGAATCTGGGGTCCCT<br>GATCGCTTCTCAGGCAGTGGATCTGGAACAGATTTCACTCTCACAATCAGCAGTGTGGAGGCTGA<br>AGACGTGGGAGTTTATTACTGTCAGAATGATTATAGTTATCCGCTCACGTTCGGTCAAGGGACCA |

| | | | | |
|---|---|---|---|---|
| | | | | AGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGGTAAAGCCTGGGTCTTCAGTGAAGGTATCCTGCAAGGCTTCTGGATACACCTTCTCTAACTACTGGCTAGGTTGGGTAAGGCAGACGCCTGGACAGGGACTTGAGTGGATTGGAGATATTTTCCCTGGAAGTGGTAATATCCACTACAATGAGAAGTTCAAGGGCAAAGTCACAATCACTGCAGACAAATCTACGAGCACAGCCTATATGGAACTCAGTAGCCTGGGATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1116. | hEp 18-G9 L | artificial | aa | DIVMTQTPLSLTVTPGEPVSISCKSSQSLLNSGNQKNYLTWYLQKPGQSPQLLIYWASTRESGVPDRFSGSGSGTDFTLKISRVEAEDVGVYYCQNDYSYPLTFGQGTKLEIK |
| 1117. | hEp 18-G9 LCDR1 | artificial | aa | KSSQSLLNSGNQKNYLT |
| 1118. | hEp 18-G9 LCDR2 | artificial | aa | WASTRES |
| 1119. | hEp 18-G9 LCDR3 | artificial | aa | QNDYSYPLT |
| 1120. | hEp 18-G9 L | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAA |
| 1121. | hEp 18-G9 H | artificial | aa | QVQLVQSGAEVKKPGSSVKVSCKASGYTFSNYWLGWVRQTPGQGLEWMGDIFPGSGNIHYNEKFKGRATITADKSTSTAYMELSSLTSEDTAVYYCARLRNWDEPMDYWGQGTTVTVSS |
| 1122. | hEp 18-G9 HCDR1 | artificial | aa | NYWLG |

| | | | | |
|---|---|---|---|---|
| 1123. | hEp 18-G9 HCDR2 | artificial | aa | DIFPGSGNIHYNEKFKG |
| 1124. | hEp 18-G9 HCDR3 | artificial | aa | LRNWDEPMDY |
| 1125. | hEp 18-G9 H | artificial | nt | CAGGTGCAGCTGGTCCAGTCTGGAGCTGAGGTGAAAAAGCCTGGGTCTTCAGTGAAGGTATCCTG CAAGGCTTCTGGATACACCTTCTCTAACTACTGGCTAGGTTGGGTAAGGCAGACGCCTGGACAGG GACTTGAGTGGATGGGAGATATTTTCCCTGGAAGTGGTAATATCCACTACAATGAGAAGTTCAAG GGCAGAGCCACAATCACTGCAGACAAATCTACGAGCACAGCCTATATGGAACTCAGTAGCCTGAC ATCTGAGGACACTGCTGTCTATTACTGTGCAAGACTGAGGAACTGGGACGAGCCTATGGACTACT GGGGCCAAGGGACCACGGTCACCGTCTCCTCC |
| 1126. | hEp 18-G9 LH x I2C HL | artificial | aa | DIVMTQTPLSLTVTPGEPVSISCKSSQSLLNSGNQKNYLTWYLQKPGQSPQLLIYWASTRESGVP DRFSGSGSGTDFTLKISRVEAEDVGVYYCQNDYSYPLTFGQGTKLEIKGGGGSGGGGSGGGGSQV QLVQSGAEVKKPGSSVKVSCKASGYTFSNYWLGWVRQTPGQGLEWMGDIFPGSGNIHYNEKFKGR ATITADKSTSTAYMELSSLTSEDTAVYYCARLRNWDEPMDYWGQGTTVTVSSGGGGSEVQLVESG GGLVQPGGSLKLSCAASGFTFNKYAMNWVRQAPGKGLEWVARIRSKYNNYATYYADSVKDRFTIS RDDSKNTAYLQMNNLKTEDTAVYYCVRHGNFGNSYISYWAYWGQGTLVTVSSGGGGSGGGGSGGG GSQTVVTQEPSLTVSPGGTVTLTCGSSTGAVTSGNYPNWVQQKPGQAPRGLIGGTKFLAPGTPAR FSGSLLGGKAALTLSGVQPEDEAEYYCVLWYSNRWVFGGGTKLTVL |
| 1127. | hEp 18-G9 LH x I2C HL | artificial | nt | GACATCGTGATGACACAGACTCCACTCTCCCTGACTGTGACACCAGGAGAGCCGGTCTCTATCAG CTGCAAGTCCAGTCAGAGTCTGTTAAACAGTGGAAATCAAAAGAACTACTTGACCTGGTACCTGC AGAAACCAGGGCAGTCTCCTCAACTGTTGATCTACTGGGCATCCACTAGGGAATCTGGGGTCCCT GATCGCTTCTCAGGCAGTGGATCTGGAACAGATTTCACTCTCAAAATCAGCCGTGTGGAGGCTGA AGACGTGGGAGTTTATTACTGTCAGAATGATTATAGTTATCCGCTCACGTTCGGTCAAGGGACCA AGCTTGAGATCAAAGGTGGTGGTGGTTCTGGCGGCGGCGGCTCCGGTGGTGGTGGTTCTCAGGTG CAGCTGGTCCAGTCTGGAGCTGAGGTGAAAAAGCCTGGGTCTTCAGTGAAGGTATCCTGCAAGGC TTCTGGATACACCTTCTCTAACTACTGGCTAGGTTGGGTAAGGCAGACGCCTGGACAGGGACTTG AGTGGATGGGAGATATTTTCCCTGGAAGTGGTAATATCCACTACAATGAGAAGTTCAAGGGCAGA GCCACAATCACTGCAGACAAATCTACGAGCACAGCCTATATGGAACTCAGTAGCCTGACATCTGA GGACACTGCTGTCTATTACTGTGCAAGACTGAGGAACTGGGACGAGCCTATGGACTACTGGGGCC AAGGGACCACGGTCACCGTCTCCTCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATTCACCTTCAA TAAGTACGCCATGAACTGGGTCCGCCAGGCTCCAGGAAAGGGTTTGGAATGGGTTGCTCGCATAA GAAGTAAATATAATAATTATGCAACATATTATGCCGATTCAGTGAAAGACAGGTTCACCATCTCC AGAGATGATTCAAAAAACACTGCCTATCTACAAATGAACAACTTGAAAACTGAGGACACTGCCGT GTACTACTGTGTGAGACATGGGAACTTCGGTAATAGCTACATATCCTACTGGGCTTACTGGGGCC AAGGGACTCTGGTCACCGTCTCCTCAGGTGGTGGTGGTTCTGGCGGCGGCGGCTCCGGTGGTGGT GGTTCTCAGACTGTTGTGACTCAGGAACCTTCACTCACCGTATCACCTGGTGGAACAGTCACACT |

| | | | | |
|---|---|---|---|---|
| | | | | CACTTGTGGCTCCTCGACTGGGGCTGTTACATCTGGCAACTACCCAAACTGGGTCCAACAAAAAC CAGGTCAGGCACCCCGTGGTCTAATAGGTGGGACTAAGTTCCTCGCCCCCGGTACTCCTGCCAGA TTCTCAGGCTCCCTGCTTGGAGGCAAGGCTGCCCTCACCCTCTCAGGGGTACAGCCAGAGGATGA GGCAGAATATTACTGTGTTCTATGGTACAGCAACCGCTGGGTGTTCGGTGGAGGAACCAAACTGA CTGTCCTA |
| 1128. | human EpCAM | human | aa | MAPPQVLAFGLLLAAATATFAAAQEECVCENYKLAVNCFVNNNRQCQCTSVGAQNTVICSKLAAK CLVMKAEMNGSKLGRRAKPEGALQNNDGLYDPDCDESGLFKAKQCNGTSTCWCVNTAGVRRTDKD TEITCSERVRTYWIIIELKHKAREKPYDSKSLRTALQKEITTRYQLDPKFITSILYENNVITIDL VQNSSQKTQNDVDIADVAYYFEKDVKGESLFHSKKMDLTVNGEQLDLDPGQTLIYYVDEKAPEFS MQGLKAGVIAVIVVVVIAVVAGIVVLVISRKKRMAKYEKAEIKEMGEMHRELNA |
| 1129. | human EpCAM cDNA | human | nt | cggcgagcgagcaccttcgacgcggtccggggacccccctcgtcgctgtcctcccgacgcggacccgcgtgcc ccaggcctcgcgctgcccggccggctcctcgtgtcccactcccggcgcacgccctcccgcgagtcccgggcc cctcccgcgcccctcttctcggcgcgcgcgcagcatggcgcccccgcaggtcctcgcgttcgggcttctgct tgccgcggcgacggcgacttttgccgcagctcaggaagaatgtgtctgtgaaaactacaagctggccgtaaa ctgctttgtgaataataatcgtcaatgccagtgtacttcagttggtgcacaaaatactgtcatttgctcaaa gctggctgccaaatgtttggtgatgaaggcagaaatgaatggctcaaaacttgggagaagagcaaaacctga aggggccctccagaacaatgatgggctttatgatcctgactgcgatgagagcgggctctttaaggccaagca gtgcaacggcacctccacgtgctggtgtgtgaacactgctggggtcagaagaacagacaaggacactgaaat aacctgctctgagcgagtgagaacctactggatcatcattgaactaaaacacaaagcaagagaaaaacctta tgatagtaaaagtttgcggactgcacttcagaaggagatcacaacgcgttatcaactggatccaaaatttat cacgagtattttgtatgagaataatgttatcactattgatctggttcaaaattcttctcaaaaaactcagaa tgatgtggacatagctgatgtggcttattattttgaaaaagatgttaaaggtgaatccttgtttcattctaa gaaaatggacctgacagtaaatggggaacaactggatctggatcctggtcaaactttaatttattatgttga tgaaaaagcacctgaattctcaatgcagggtctaaaagctggtgttattgctgttattgtggttgtggtgat agcagttgttgctggaattgttgtgctggttatttccagaaagaagagaatggcaaagtatgagaaggctga gataaaggagatgggtgagatgcatagggaactcaatgcataactatataatttgaagattatagaagaagg gaaatagcaaatggacacaaattacaaatgtgtgtgcgtgggacgaagacatctttgaaggtcatgagtttg ttagtttaacatcatatatttgtaatagtgaaacctgtactcaaaatataagcagcttgaaactggctttac caatcttgaaatttgaccacaagtgtcttatatatgcagatctaatgtaaaatccagaacttggactccatc gttaaaattatttatgtgtaacattcaaatgtgtgcattaaatatgcttccacagtaaaatctgaaaaactg atttgtgattgaaagctgcctttctatttacttgagtcttgtacatacatactttttttatgagctatgaaat aaaacattttaaactg |
| 1130. | aa 26-61 of the EGF-like domain 1 of human EpCAM, encoded by exon 2 | human | aa | ECVCENYKLAVNCFVNNNRQCQCTSVGAQNTVICSK |
| 1131. | FAP L | artificial | aa | DIVMTQSPDSLAVSLGERATINCKSSQSLLYSRNQKNYLAWYQQKPGQPPKLLIFWASTRESGVP DRFSGSGFGTDFTLTISSLQAEDVAVYYCQQYFSYPLTFGQGTKVEIK |
| 1132. | FAP L-CDR1 | artificial | aa | KSSQSLLYSRNQKNYLA |

| | | | | |
|---|---|---|---|---|
| 1133. | FAP L-CDR2 | artificial | aa | WASTRES |
| 1134. | FAP L-CDR3 | artificial | aa | QQYFSYPLT |
| 1135. | FAP L | artificial | nt | GACATTGTGATGACCCAGTCTCCAGACTCTTTGGCTGTGTCTCTAGGGGAGAGGGCCACCATCAACTGCAAGTCCAGTCAGAGCCTTTTATATTCTAGAAATCAAAAGAACTACTTGGCCTGGTATCAGCAGAAACCAGGACAGCCACCCAAACTCCTCATCTTTTGGGCTAGCACTAGGGAATCTGGGGTACCTGATAGGTTCAGTGGCAGTGGGTTTGGGACAGACTTCACCCTCACCATTAGCAGCCTGCAGGCTGAAGATGTGGCAGTTTATTACTGTCAGCAATATTTTAGCTATCCGCTCACGTTCGGACAAGGGACCAAGGTGGAAATAAAA |
| 1136. | FAP H | artificial | aa | QVQLVQSGAEVKKPGASVKVSCKTSRYTFTEYTIHWVRQAPGQRLEWIGGINPNNGIPNYNQKFKGRVTITVDTSASTAYMELSSLRSEDTAVYYCARRRIAYGYDEGHAMDYWGQGTLVTVSS |
| 1137. | FAP H-CDR1 | artificial | aa | EYTIH |
| 1138. | FAP H-CDR2 | artificial | aa | GINPNNGIPNYNQKFKG |
| 1139. | FAP H-CDR3 | artificial | aa | RRIAYGYDEGHAMDY |
| 1140. | FAP H | artificial | nt | CAGGTGCAACTAGTGCAGTCCGGCGCCGAAGTGAAGAAACCCGGTGCTTCCGTGAAAGTCAGCTGTAAAACTAGTAGATACACCTTCACTGAATACACCATACACTGGGTTAGACAGGCCCCTGGCCAAAGGCTGGAGTGGATAGGAGGTATTAATCCTAACAATGGTATTCCTAACTACAACCAGAAGTTCAAGGGCCGGGTCACCATCACCGTAGACACCTCTGCCAGCACCGCCTACATGGAACTGTCCAGCCTGCGCTCCGAGGACACTGCAGTCTACTACTGCGCCAGAAGAAGAATCGCCTATGGTTACGACGAGGGCCATGCTATGGACTACTGGGGTCAAGGAACCCTTGTCACCGTCTCCTCC |
| 1141. | FAP LH | artificial | aa | DIVMTQSPDSLAVSLGERATINCKSSQSLLYSRNQKNYLAWYQQKPGQPPKLLIFWASTRESGVPDRFSGSGFGTDFTLTISSLQAEDVAVYYCQQYFSYPLTFGQGTKVEIKGGGGSGGGGSGGGGSQVQLVQSGAEVKKPGASVKVSCKTSRYTFTEYTIHWVRQAPGQRLEWIGGINPNNGIPNYNQKFKGRVTITVDTSASTAYMELSSLRSEDTAVYYCARRRIAYGYDEGHAMDYWGQGTLVTVSS |
| 1142. | FAP LH | artificial | nt | GACATTGTGATGACCCAGTCTCCAGACTCTTTGGCTGTGTCTCTAGGGGAGAGGGCCACCATCAACTGCAAGTCCAGTCAGAGCCTTTTATATTCTAGAAATCAAAAGAACTACTTGGCCTGGTATCAGCAGAAACCAGGACAGCCACCCAAACTCCTCATCTTTTGGGCTAGCACTAGGGAATCTGGGGTACCTGATAGGTTCAGTGGCAGTGGGTTTGGGACAGACTTCACCCTCACCATTAGCAGCCTGCAGGCTGAAGATGTGGCAGTTTATTACTGTCAGCAATATTTTAGCTATCCGCTCACGTTCGGACAAGGGACCAAGGTGGAAATAAAAGGTGGTGGTGGTTCTGGCGGCGGCGGCTCCGGTGGTGGTGGTTCTCAGGTGCAACTAGTGCAGTCCGGCGCCGAAGTGAAGAAACCCGGTGCTTCCGTGAAAGTCAGCTGTAAAACTAGTAGATACACCTTCACTGAATACACCATACACTGGGTTAGACAGGCCCCTGGCCAAAGGCTGGAGTGGATAGGAGGTATTAATCCTAACAATGGTATTCCTAACTACAACCAGAAGTTCAAGGGCCGGGTCACCATCACCGTAGACACCTCTGCCAGCACCGCCTACATGGAACTGTCCAGCCTGCGCTCCGAGGACACTGCAGTCTACTACTGCGCCAGAAGAAGAATCGCCTATGGTTACGACGAGGGCCATGCTATGGACTACTGGGGTCAAGGAACCCTTGTCACCGTCTCCTCC |
| 1143. | FAP LH x I2C HL | artificial | aa | DIVMTQSPDSLAVSLGERATINCKSSQSLLYSRNQKNYLAWYQQKPGQPPKLLIFWASTRESGVP |

| | | | | |
|---|---|---|---|---|
| | | | | DRFSGSGFGTDFTLTISSLQAEDVAVYYCQQYFSYPLTFGQGTKVEIKGGGGSGGGGSGGGGSQV<br>QLVQSGAEVKKPGASVKVSCKTSRYTFTEYTIHWVRQAPGQRLEWIGGINPNNGIPNYNQKFKGR<br>VTITVDTSASTAYMELSSLRSEDTAVYYCARRRIAYGYDEGHAMDYWGQGTLVTVSSGGGGSEVQ<br>LVESGGGLVQPGGSLKLSCAASGFTFNKYAMNWVRQAPGKGLEWVARIRSKYNNYATYYADSVKD<br>RFTISRDDSKNTAYLQMNNLKTEDTAVYYCVRHGNFGNSYISYWAYWGQGTLVTVSSGGGGSGGG<br>GSGGGGSQTVVTQEPSLTVSPGGTVTLTCGSSTGAVTSGNYPNWVQQKPGQAPRGLIGGTKFLAP<br>GTPARFSGSLLGGKAALTLSGVQPEDEAEYYCVLWYSNRWVFGGGTKLTVL |
| 1144. | FAP LH x I2C HL | artificial | nt | GACATTGTGATGACCCAGTCTCCAGACTCTTTGGCTGTGTCTCTAGGGGAGAGGGCCACCATCAA<br>CTGCAAGTCCAGTCAGAGCCTTTTATATTCTAGAAATCAAAAGAACTACTTGGCCTGGTATCAGC<br>AGAAACCAGGACAGCCACCCAAACTCCTCATCTTTTGGGCTAGCACTAGGGAATCTGGGGTACCT<br>GATAGGTTCAGTGGCAGTGGGTTTGGGACAGACTTCACCCTCACCATTAGCAGCCTGCAGGCTGA<br>AGATGTGGCAGTTTATTACTGTCAGCAATATTTTAGCTATCCGCTCACGTTCGGACAAGGGACCA<br>AGGTGGAAATAAAAGGTGGTGGTGGTTCTGGCGGCGGCGGCTCCGGTGGTGGTGGTTCTCAGGTG<br>CAACTAGTGCAGTCCGGCGCCGAAGTGAAGAAACCCGGTGCTTCCGTGAAAGTCAGCTGTAAAAC<br>TAGTAGATACACCTTCACTGAATACACCATACACTGGGTTAGACAGGCCCCTGGCCAAAGGCTGG<br>AGTGGATAGGAGGTATTAATCCTAACAATGGTATTCCTAACTACAACCAGAAGTTCAAGGGCCGG<br>GTCACCATCACCGTAGACACCTCTGCCAGCACCGCCTACATGGAACTGTCCAGCCTGCGCTCCGA<br>GGACACTGCAGTCTACTACTGCGCCAGAAGAAGAATCGCCTATGGTTACGACGAGGGCCATGCTA<br>TGGACTACTGGGGTCAAGGAACCCTTGTCACCGTCTCCTCCGGAGGTGGTGGATCCGAGGTGCAG<br>CTGGTCGAGTCTGGAGGAGGATTGGTGCAGCCTGGAGGGTCATTGAAACTCTCATGTGCAGCCTC<br>TGGATTCACCTTCAATAAGTACGCCATGAACTGGGTCCGCCAGGCTCCAGGAAAGGGTTTGGAAT<br>GGGTTGCTCGCATAAGAAGTAAATATAATAATTATGCAACATATTATGCCGATTCAGTGAAAGAC<br>AGGTTCACCATCTCCAGAGATGATTCAAAAAACACTGCCTATCTACAAATGAACAACTTGAAAAC<br>TGAGGACACTGCCGTGTACTACTGTGTGAGACATGGGAACTTCGGTAATAGCTACATATCCTACT<br>GGGCTTACTGGGGCCAAGGGACTCTGGTCACCGTCTCCTCAGGTGGTGGTGGTTCTGGCGGCGGC<br>GGCTCCGGTGGTGGTGGTTCTCAGACTGTTGTGACTCAGGAACCTTCACTCACCGTATCACCTGG<br>TGGAACAGTCACACTCACTTGTGGCTCCTCGACTGGGGCTGTTACATCTGGCAACTACCCAAACT<br>GGGTCCAACAAAAACCAGGTCAGGCACCCCGTGGTCTAATAGGTGGGACTAAGTTCCTCGCCCCC<br>GGTACTCCTGCCAGATTCTCAGGCTCCCTGCTTGGAGGCAAGGCTGCCCTCACCCTCTCAGGGGT<br>ACAGCCAGAGGATGAGGCAGAATATTACTGTGTTCTATGGTACAGCAACCGCTGGGTGTTCGGTG<br>GAGGAACCAAACTGACTGTCCTA |
| 1145. | FAP LH x H2C HL | artificial | aa | DIVMTQSPDSLAVSLGERATINCKSSQSLLYSRNQKNYLAWYQQKPGQPPKLLIFWASTRESGVP<br>DRFSGSGFGTDFTLTISSLQAEDVAVYYCQQYFSYPLTFGQGTKVEIKGGGGSGGGGSGGGGSQV<br>QLVQSGAEVKKPGASVKVSCKTSRYTFTEYTIHWVRQAPGQRLEWIGGINPNNGIPNYNQKFKGR<br>VTITVDTSASTAYMELSSLRSEDTAVYYCARRRIAYGYDEGHAMDYWGQGTLVTVSSGGGGSEVQ<br>LVESGGGLVQPGGSLKLSCAASGFTFNKYAMNWVRQAPGKGLEWVARIRSKYNNYATYYADSVKD<br>RFTISRDDSKNTAYLQMNNLKTEDTAVYYCVRHGNFGNSYISYWAYWGQGTLVTVSSGGGGSGGG |

| | | | | |
|---|---|---|---|---|
| | | | | GSGGGGSQTVVTQEPSLTVSPGGTVTLTCGSSTGAVTSGYYPNWVQQKPGQAPRGLIGGTKFLAP<br>GTPARFSGSLLGGKAALTLSGVQPEDEAEYYCALWYSNRWVFGGGTKLTVL |
| 1146. | FAP LH x H2C HL | artificial | nt | GACATTGTGATGACCCAGTCTCCAGACTCTTTGGCTGTGTCTCTAGGGGAGAGGGCCACCATCAA<br>CTGCAAGTCCAGTCAGAGCCTTTTATATTCTAGAAATCAAAAGAACTACTTGGCCTGGTATCAGC<br>AGAAACCAGGACAGCCACCCAAACTCCTCATCTTTTGGGCTAGCACTAGGGAATCTGGGGTACCT<br>GATAGGTTCAGTGGCAGTGGGTTTGGGACAGACTTCACCCTCACCATTAGCAGCCTGCAGGCTGA<br>AGATGTGGCAGTTTATTACTGTCAGCAATATTTTAGCTATCCGCTCACGTTCGGACAAGGGACCA<br>AGGTGGAAATAAAAGGTGGTGGTGGTTCTGGCGGCGGCGGCTCCGGTGGTGGTGGTTCTCAGGTG<br>CAACTAGTGCAGTCCGGCGCCGAAGTGAAGAAACCCGGTGCTTCCGTGAAAGTCAGCTGTAAAAC<br>TAGTAGATACACCTTCACTGAATACACCATACACTGGGTTAGACAGGCCCCTGGCCAAAGGCTGG<br>AGTGGATAGGAGGTATTAATCCTAACAATGGTATTCCTAACTACAACCAGAAGTTCAAGGGCCGG<br>GTCACCATCACCGTAGACACCTCTGCCAGCACCGCCTACATGGAACTGTCCAGCCTGCGCTCCGA<br>GGACACTGCAGTCTACTACTGCGCCAGAAGAAGAATCGCCTATGGTTACGACGAGGGCCATGCTA<br>TGGACTACTGGGGTCAAGGAACCCTTGTCACCGTCTCCTCCGGAGGTGGTGGATCCGAGGTGCAG<br>CTGGTCGAGTCTGGAGGAGGATTGGTGCAGCCTGGAGGGTCATTGAAACTCTCATGTGCAGCCTC<br>TGGATTCACCTTCAATAAGTACGCCATGAACTGGGTCCGCCAGGCTCCAGGAAAGGGTTTGGAAT<br>GGGTTGCTCGCATAAGAAGTAAATATAATAATTATGCAACATATTATGCCGATTCAGTGAAAGAC<br>AGGTTCACCATCTCCAGAGATGATTCAAAAAACACTGCCTATCTACAAATGAACAACTTGAAAAC<br>TGAGGACACTGCCGTGTACTACTGTGTGAGACATGGGAACTTCGGTAATAGCTACATATCCTACT<br>GGGCTTACTGGGGCCAAGGGACTCTGGTCACCGTCTCCTCAGGTGGTGGTGGTTCTGGCGGCGGC<br>GGCTCCGGTGGTGGTGGTTCTCAGACTGTTGTGACTCAGGAACCTTCACTCACCGTATCACCTGG<br>TGGAACAGTCACACTCACTTGTGGCTCCTCGACTGGGGCTGTTACATCTGGCTACTACCCAAACT<br>GGGTCCAACAAAAACCAGGTCAGGCACCCCGTGGTCTAATAGGTGGGACTAAGTTCCTCGCCCCC<br>GGTACTCCTGCCAGATTCTCAGGCTCCCTGCTTGGAGGCAAGGCTGCCCTCACCCTCTCAGGGGT<br>ACAGCCAGAGGATGAGGCAGAATATTACTGTGCTCTATGGTACAGCAACCGCTGGGTGTTCGGTG<br>GAGGAACCAAACTGACTGTCCTA |
| 1147. | FAP LH x F12Q HL | artificial | aa | DIVMTQSPDSLAVSLGERATINCKSSQSLLYSRNQKNYLAWYQQKPGQPPKLLIFWASTRESGVP<br>DRFSGSGFGTDFTLTISSLQAEDVAVYYCQQYFSYPLTFGQGTKVEIKGGGGSGGGGSGGGGSQV<br>QLVQSGAEVKKPGASVKVSCKTSRYTFTEYTIHWVRQAPGQRLEWIGGINPNNGIPNYNQKFKGR<br>VTITVDTSASTAYMELSSLRSEDTAVYYCARRIAYGYDEGHAMDYWGQGTLVTVSSGGGGSEVQ<br>LVESGGGLVQPGGSLKLSCAASGFTFNSYAMNWVRQAPGKGLEWVARIRSKYNNYATYYADSVKG<br>RFTISRDDSKNTAYLQMNNLKTEDTAVYYCVRHGNFGNSYVSWWAYWGQGTLVTVSSGGGGSGGG<br>GSGGGGSQTVVTQEPSLTVSPGGTVTLTCGSSTGAVTSGNYPNWVQQKPGQAPRGLIGGTKFLAP<br>GTPARFSGSLLGGKAALTLSGVQPEDEAEYYCVLWYSNRWVFGGGTKLTVL |
| 1148. | FAP LH x F12Q HL | artificial | nt | GACATTGTGATGACCCAGTCTCCAGACTCTTTGGCTGTGTCTCTAGGGGAGAGGGCCACCATCAA<br>CTGCAAGTCCAGTCAGAGCCTTTTATATTCTAGAAATCAAAAGAACTACTTGGCCTGGTATCAGC<br>AGAAACCAGGACAGCCACCCAAACTCCTCATCTTTTGGGCTAGCACTAGGGAATCTGGGGTACCT |

| | | | | |
|---|---|---|---|---|
| | | | | GATAGGTTCAGTGGCAGTGGGTTTGGGACAGACTTCACCCTCACCATTAGCAGCCTGCAGGCTGA<br>AGATGTGGCAGTTTATTACTGTCAGCAATATTTTAGCTATCCGCTCACGTTCGGACAAGGGACCA<br>AGGTGGAAATAAAAGGTGGTGGTGGTTCTGGCGGCGGCGGCTCCGGTGGTGGTGGTTCTCAGGTG<br>CAACTAGTGCAGTCCGGCGCCGAAGTGAAGAAACCCGGTGCTTCCGTGAAAGTCAGCTGTAAAAC<br>TAGTAGATACACCTTCACTGAATACACCATACACTGGGTTAGACAGGCCCCTGGCCAAAGGCTGG<br>AGTGGATAGGAGGTATTAATCCTAACAATGGTATTCCTAACTACAACCAGAAGTTCAAGGGCCGG<br>GTCACCATCACCGTAGACACCTCTGCCAGCACCGCCTACATGGAACTGTCCAGCCTGCGCTCCGA<br>GGACACTGCAGTCTACTACTGCGCCAGAAGAAGAATCGCCTATGGTTACGACGAGGGCCATGCTA<br>TGGACTACTGGGGTCAAGGAACCCTTGTCACCGTCTCCTCCGGAGGTGGTGGATCCGAGGTGCAG<br>CTGGTCGAGTCTGGAGGAGGATTGGTGCAGCCTGGAGGGTCATTGAAACTCTCATGTGCAGCCTC<br>TGGATTCACCTTCAATAGCTACGCCATGAACTGGGTCCGCCAGGCTCCAGGAAAGGGTTTGGAAT<br>GGGTTGCTCGCATAAGAAGTAAATATAATAATTATGCAACATATTATGCCGATTCAGTGAAAGGC<br>AGGTTCACCATCTCCAGAGATGATTCAAAAAACACTGCCTATCTACAAATGAACAACTTGAAAAC<br>TGAGGACACTGCCGTGTACTACTGTGTGAGACATGGGAACTTCGGTAATAGCTACGTTTCCTGGT<br>GGGCTTACTGGGGCCAAGGGACTCTGGTCACCGTCTCCTCAGGTGGTGGTGGTTCTGGCGGCGGC<br>GGCTCCGGTGGTGGTGGTTCTCAGACTGTTGTGACTCAGGAACCTTCACTCACCGTATCACCTGG<br>TGGAACAGTCACACTCACTTGTGGCTCCTCGACTGGGGCTGTTACATCTGGCAACTACCCAAACT<br>GGGTCCAACAAAAACCAGGTCAGGCACCCCGTGGTCTAATAGGTGGGACTAAGTTCCTCGCCCCC<br>GGTACTCCTGCCAGATTCTCAGGCTCCCTGCTTGGAGGCAAGGCTGCCCTCACCCTCTCAGGGGT<br>ACAGCCAGAGGATGAGGCAGAATATTACTGTGTTCTATGGTACAGCAACCGCTGGGTGTTCGGTG<br>GAGGAACCAAACTGACTGTCCTA |
| 1149. | FAP alpha | human | nt | ATGAAGACTTGGGTAAAAATCGTATTTGGAGTTGCCACCTCTGCTGTGCTTGCCTTATTGGTGAT<br>GTGCATTGTCTTACGCCCTTCAAGAGTTCATAACTCTGAAGAAAATACAATGAGAGCACTCACAC<br>TGAAGGATATTTTAAATGGAACATTTTCTTATAAAACATTTTTTCCAAACTGGATTTCAGGACAA<br>GAATATCTTCATCAATCTGCAGATAACAATATAGTACTTTATAATATTGAAACAGGACAATCATA<br>TACCATTTTGAGTAATAGAACCATGAAAAGTGTGAATGCTTCAAATTACGGCTTATCACCTGATC<br>GGCAATTTGTATATCTAGAAAGTGATTATTCAAAGCTTTGGAGATACTCTTACACAGCAACATAT<br>TACATCTATGACCTTAGCAATGGAGAATTTGTAAGAGGAAATGAGCTTCCTCGTCCAATTCAGTA<br>TTTATGCTGGTCGCCTGTTGGGAGTAAATTAGCATATGTCTATCAAAACAATATCTATTTGAAAC<br>AAAGACCAGGAGATCCACCTTTTCAAATAACATTTAATGGAAGAGAAAATAAAATATTTAATGGA<br>ATCCCAGACTGGGTTTATGAAGAGGAAATGCTTGCTACAAAATATGCTCTCTGGTGGTCTCCTAA<br>TGGAAAATTTTTGGCATATGCGGAATTTAATGATACGGATATACCAGTTATTGCCTATTCCTATT<br>ATGGCGATGAACAATATCCTAGAACAATAAATATTCCATACCCAAAGGCTGGAGCTAAGAATCCC<br>GTTGTTCGGATATTTATTATCGATACCACTTACCCTGCGTATGTAGGTCCCCAGGAAGTGCCTGT<br>TCCAGCAATGATAGCCTCAAGTGATTATTATTTCAGTTGGCTCACGTGGGTTACTGATGAACGAG<br>TATGTTTGCAGTGGCTAAAAAGAGTCCAGAATGTTTCGGTCCTGTCTATATGTGACTTCAGGGAA<br>GACTGGCAGACATGGGATTGTCCAAAGACCCAGGAGCATATAGAAGAAAGCAGAACTGGATGGGC |

| | | | | |
|---|---|---|---|---|
| | | | | TGGTGGATTCTTTGTTTCAACACCAGTTTTCAGCTATGATGCCATTTCGTACTACAAAATATTTA GTGACAAGGATGGCTACAAACATATTCACTATATCAAAGACACTGTGGAAAATGCTATTCAAATT ACAAGTGGCAAGTGGGAGGCCATAAATATATTCAGAGTAACACAGGATTCACTGTTTTATTCTAG CAATGAATTTGAAGAATACCCTGGAAGAAGAAACATCTACAGAATTAGCATTGGAAGCTATCCTC CAAGCAAGAAGTGTGTTACTTGCCATCTAAGGAAAGAAAGGTGCCAATATTACACAGCAAGTTTC AGCGACTACGCCAAGTACTATGCACTTGTCTGCTACGGCCCAGGCATCCCCATTTCCACCCTTCA TGATGGACGCACTGATCAAGAAATTAAAATCCTGGAAGAAAACAAGGAATTGGAAAATGCTTTGA AAAATATCCAGCTGCCTAAAGAGGAAATTAAGAAACTTGAAGTAGATGAAATTACTTTATGGTAC AAGATGATTCTTCCTCCTCAATTTGACAGATCAAAGAAGTATCCCTTGCTAATTCAAGTGTATGG TGGTCCCTGCAGTCAGAGTGTAAGGTCTGTATTTGCTGTTAATTGGATATCTTATCTTGCAAGTA AGGAAGGGATGGTCATTGCCTTGGTGGATGGTCGAGGAACAGCTTTCCAAGGTGACAAACTCCTC TATGCAGTGTATCGAAAGCTGGGTGTTTATGAAGTTGAAGACCAGATTACAGCTGTCAGAAAATT CATAGAAATGGGTTTCATTGATGAAAAAAGAATAGCCATATGGGGCTGGTCCTATGGAGGATACG TTTCATCACTGGCCCTTGCATCTGGAACTGGTCTTTTCAAATGTGGTATAGCAGTGGCTCCAGTC TCCAGCTGGGAATATTACGCGTCTGTCTACACAGAGAGATTCATGGGTCTCCCAACAAAGGATGA TAATCTTGAGCACTATAAGAATTCAACTGTGATGGCAAGAGCAGAATATTTCAGAAATGTAGACT ATCTTCTCATCCACGGAACAGCAGATGATAATGTGCACTTTCAAAACTCAGCACAGATTGCTAAA GCTCTGGTTAATGCACAAGTGGATTTCCAGGCAATGTGGTACTCTGACCAGAACCACGGCTTATC CGGCCTGTCCACGAACCACTTATACACCCACATGACCCACTTCCTAAAGCAGTGTTTCTCTTTGT CAGACTAA |
| 1150. | FAP alpha | human | aa | MKTWVKIVFGVATSAVLALLVMCIVLRPSRVHNSEENTMRALTLKDILNGTFSYKTFFPNWISGQ EYLHQSADNNIVLYNIETGQSYTILSNRTMKSVNASNYGLSPDRQFVYLESDYSKLWRYSYTATY YIYDLSNGEFVRGNELPRPIQYLCWSPVGSKLAYVYQNNIYLKQRPGDPPFQITFNGRENKIFNG IPDWVYEEEMLATKYALWWSPNGKFLAYAEFNDTDIPVIAYSYYGDEQYPRTINIPYPKAGAKNP VVRIFIIDTTYPAYVGPQEVPVPAMIASSDYYFSWLTWVTDERVCLQWLKRVQNVSVLSICDFRE DWQTWDCPKTQEHIEESRTGWAGGFFVSTPVFSYDAISYYKIFSDKDGYKHIHYIKDTVENAIQI TSGKWEAINIFRVTQDSLFYSSNEFEEYPGRRNIYRISIGSYPPSKKCVTCHLRKERCQYYTASF SDYAKYYALVCYGPGIPISTLHDGRTDQEIKILEENKELENALKNIQLPKEEIKKLEVDEITLWY KMILPPQFDRSKKYPLLIQVYGGPCSQSVRSVFAVNWISYLASKEGMVIALVDGRGTAFQGDKLL YAVYRKLGVYEVEDQITAVRKFIEMGFIDEKRIAIWGWSYGGYVSSLALASGTGLFKCGIAVAPV SSWEYYASVYTERFMGLPTKDDNLEHYKNSTVMARAEYFRNVDYLLIHGTADDNVHFQNSAQIAK ALVNAQVDFQAMWYSDQNHGLSGLSTNHLYTHMTHFLKQCFSLSD |
| 1151. | FAP alpha | Macaque (cynomol-gus) | nt | ATGAAGACTTGGGTAAAAATCGTATTTGGAGTTGCCACCTCTGCTGTGCTTGCCTTATTGGTGAT GTGCATTGTCTTACGCCCTCCAAGAGTTCATAACTCTGAAGAAAATACAATGAGAGCACTCACAC TGAAGGATATTTTAAATGGGACATTTTCTTATAAAACATTTTTTCCAAACTGGATTTCAGGACAA GAATATCTTCATCAATCTGCAGATAACAATATAGTACTTTATAATATTGAAACAGGACAATCATA TACCATTTTGAGTAACAGAACCATGAAAAGTGTGAATGCTTCAAATTATGGCTTATCACCTGATC |

| | | | | |
|---|---|---|---|---|
| | | | | GGCAATTTGTATATCTAGAAAGTGATTATTCAAAGCTTTGGAGATACTCTTACACAGCAACATAT<br>TACATCTATGACCTTAGCAATGGAGAATTTGTAAGAGGAAATGAGCTTCCTCGTCCAATTCAGTA<br>TTTATGCTGGTCGCCTGTTGGGAGTAAATTAGCATATGTCTATCAAAACAATATCTATTTGAAAC<br>AAAGACCAGGAGATCCACCTTTTCAAATAACATTTAATGGAAGAGAAAATAAAATATTTAATGGA<br>ATCCCAGACTGGGTTTATGAAGAGGAAATGCTTGCTACAAAATATGCTCTCTGGTGGTCTCCTAA<br>TGGAAAATTTTTGGCATATGCGGAATTTAATGATACAGATATACCAGTTATTGCCTATTCCTATT<br>ATGGCGATGAACAATATCCCAGAACAATAAATATTCCATACCCAAAGGCYGGAGCTAAGAATCCT<br>TTTGTTCGGATATTTATTATCGATACCACTTACCCTGCGTATGTAGGTCCCCAGGAAGTGCCTGT<br>TCCAGCAATGATAGCCTCAAGTGATTATTATTTCAGTTGGCTCACGTGGGTTACTGATGAACGAG<br>TATGTTTGCAGTGGCTAAAAAGAGTCCAGAATGTTTCGGTCTTGTCTATATGTGATTTCAGGGAA<br>GACTGGCAGACATGGGATTGTCCAAAGACCCAGGAGCATATAGAAGAAAGCAGAACTGGATGGGC<br>TGGTGGATTCTTTGTTTCAACACCAGTTTTCAGCTATGATGCCATTTCATACTACAAAATATTTA<br>GTGACAAGGATGGCTACAAACATATTCACTATATCAAAGACACTGTGGAAAATGCTATTCAAATT<br>ACAAGTGGCAAGTGGGAGGCCATAAATATATTCAGAGTAACACAGGATTCACTGTTTTATTCTAG<br>CAATGAATTTGAAGATTACCCTGGAAGAAGAAACATCTACAGAATTAGCATTGGAAGCTATCCTC<br>CAAGCAAGAAGTGTGTTACTTGCCATCTAAGGAAAGAAAGGTGCCAATATTACACAGCAAGTTTC<br>AGCGACTACGCCAAGTACTATGCACTTGTCTGCTATGGCCCAGGCATCCCCATTTCCACCCTTCA<br>TGACGGACGCACTGATCAAGAAATTAAAATCCTGGAAGAAAACAAGGAATTGGAAAATGCTTTGA<br>AAAATATCCAGCTGCCTAAAGAGGAAATTAAGAAACTTGAAGTAGATGAAATTACTTTATGGTAC<br>AAGATGATTCTTCCTCCTCAATTTGACAGATCAAAGAAGTATCCCTTGCTAATTCAAGTGTATGG<br>TGGTCCCTGCAGTCAGAGTGTRAGGTCTGTATTTGCTGTTAATTGGATATCKTATCTTGCAAGTA<br>AGGAAGGGATGGTCATTGCCTTGGTGGATGGTCGAGGAACAGCTTTCCAAGGTGACAAACTCCTG<br>TATGCAGTGTATCGAAAGCTGGGTGTTTATGAAGTTGAAGACCAGATTACAGCTGTCAGAAAATT<br>CATAGAAATGGGTTTCATTGATGAAAAAAGAATAGCCATATGGGGCTGGTCCTATGGAGGATAYG<br>TTTCATCACTGGCCCTTGCATCTGGAACTGGTCTTTTCAAATGTGGGATAGCAGTGGCTCCAGTC<br>TCCAGCTGGGAATATTACGCGTCTGTCTACACAGAAAGATTCATGGGTCTTCCCACAAAGGATGA<br>TAATCTTGAGCACTACAAAAATTCAACTGTGATGGCAAGAGCAGAATATTTCAGAAATGTAGACT<br>ATCTTCTCATCCACGGAACAGCAGATGATAATGTGCACTTTCAAAACTCAGCACAGATTGCTAAA<br>GCTCTGGTTAATGCACAAGTGGATTTCCAGGCAATGTGGTACTCTGACCAGAACCACGGCTTATC<br>CGGCCTGTCCACGAACCACTTATACACCCACATGACCCACTTTCTAAAGCAGTGTTTTTCTTTGT<br>CGGACTAA |
| 1152. | FAP alpha | Macaque (cynomol-gus) | aa | MKTWVKIVFGVATSAVLALLVMCIVLRPPRVHNSEENTMRALTLKDILNGTFSYKTFFPNWISGQ<br>EYLHQSADNNIVLYNIETGQSYTILSNRTMKSVNASNYGLSPDRQFVYLESDYSKLWRYSYTATY<br>YIYDLSNGEFVRGNELPRPIQYLCWSPVGSKLAYVYQNNIYLKQRPGDPPFQITFNGRENKIFNG<br>IPDWVYEEEMLATKYALWWSPNGKFLAYAEFNDTDIPVIAYSYYGDEQYPRTINIPYPKAGAKNP<br>FVRIFIIDTTYPAYVGPQEVPVPAMIASSDYYFSWLTWVTDERVCLQWLKRVQNVSVLSICDFRE<br>DWQTWDCPKTQEHIEESRTGWAGGFFVSTPVFSYDAISYYKIFSDKDGYKHIHYIKDTVENAIQI |

| | | | | |
|---|---|---|---|---|
| | | | | TSGKWEAINIFRVTQDSLFYSSNEFEDYPGRRNIYRISIGSYPPSKKCVTCHLRKERCQYYTASF SDYAKYYALVCYGPGIPISTLHDGRTDQEIKILEENKELENALKNIQLPKEEIKKLEVDEITLWY KMILPPQFDRSKKYPLLIQVYGGPCSQSVRSVFAVNWISYLASKEGMVIALVDGRGTAFQGDKLL YAVYRKLGVYEVEDQITAVRKFIEMGFIDEKRIAIWGWSYGGYVSSLALASGTGLFKCGIAVAPV SSWEYYASVYTERFMGLPTKDDNLEHYKNSTVMARAEYFRNVDYLLIHGTADDNVHFQNSAQIAK ALVNAQVDFQAMWYSDQNHGLSGLSTNHLYTHMTHFLKQCFSLSD |
| 1153. | forward primer | artificial | nt | CAGCTTCCAACTACAAAGACAGAC |
| 1154. | reverse primer | artificial | nt | TTTCCTCTTCATAAACCCAGTCTGG |
| 1155. | forward primer | artificial | nt | TTGAAACAAAGACCAGGAGATCCACC |
| 1156. | reverse primer | artificial | nt | AGATGGCAAGTAACACACTTCTTGC |
| 1157. | forward primer | artificial | nt | GAAGAAACATCTACAGAATTAGCATTGG |
| 1158. | reverse primer | artificial | nt | CACATTTGAAAAGACCAGTTCCAGATGC |
| 1159. | forward primer | artificial | nt | AGATTACAGCTGTCAGAAAATTCATAGAAATGG |
| 1160. | reverse primer | artificial | nt | ATATAAGGTTTTCAGATTCTGATACAGGC |
| 1161. | 5'FAP-VH-A-XhoI | artificial | nt | CCG CTA CTC GAG TCT GGA SCT GAG STG RWG AAG CCT GGG GCT TCA GTA AAG |
| 1162. | 3'FAP-VH-B | artificial | nt | CTG TCT CAC CCA GTG TAT GGT GTA TTC AGT GAA TGT GTA TCY AGA AGY CTT GCA GGA CAY CTT TAC TGA AGC CCC |
| 1163. | 5'FAP-VH-C | artificial | nt | CAC TGG GTG AGA CAG KCC CMT GGA MAG AGM CTT GAG TGG ATK GGA GGT ATT AAT CCT AAC AAT GGT ATT CCT AAC |
| 1164. | 3'FAP-VH-D | artificial | nt | CAT GTA GGC GGT GCT GGM GGA CKT GYC TMY AGT TAW TGT GRC CCT GCC CTT GAA CTT CTG ATT GTA GTT AGG AAT ACC |
| 1165. | 5'FAP-VH-E | artificial | nt | AGC ACC GCC TAC ATG GAG CTC MGC AGC CTG ASA TCT GAG GAT ACT GCG GTC TAT TWC TGT GCA AGA AGA AGA ATC GCC |
| 1166. | 3'FAP-VH-F-BstEII | artificial | nt | CCA GTA GGT GAC CAG GGT TCC TTG ACC CCA GTA GTC CAT AGC ATG GCC CTC GTC GTA ACC ATA GGC GAT TCT TCT TCT TGC ACA |
| 1167. | 5'FAP-VL-A-SacI | artificial | nt | CGA CCT GAG CTC GTG ATG ACA CAG ACT CCA YYC TCC CTA SCT GTG ACA SYT GGA GAG MMG GYT TCT ATS AGC TGC AAG TCC AGT CAG |
| 1168. | 3'FAP-VL-B | artificial | nt | AGA CTG CCC TGG CTT CTG CWG GWA CCA GGC CAA GTA GTT CTT TTG ATT ACG ACT ATA TAA AAG GCT CTG ACT GGA CTT GCA GCT |
| 1169. | 5'FAP-VL-C | artificial | nt | CAG AAG CCA GGG CAG TCT CCT MAA CTG CTG ATT TWC TGG GCA TCC ACTA GG GAA TCT GGG GTC CCT GAT CGC TTC TCA GGC AGT GGA |
| 1170. | 3'FAP-VL-D | artificial | nt | ATA TTG CTG ACA GTM ATA AAC TSC CAS GTC CTC AGC CTS CAC WCT GCT GAT CKT GAG AKT GAA ATC CGT CCC ARA TCC ACT GCC TGA GAA |

| | | | | |
|---|---|---|---|---|
| 1171. | 3'FAP-VL-E-BsiWI/SpeI | artificial | nt | CCA GTA ACT AGT CGT ACG TTT GAT CTC CAC CTT GGT CCC ACC ACC GAA CGT GAG CGG ATA GCT AAA ATA TTG CTG ACA GT |
| 1172. | PC32B8-H | artificial | aa | EVQLVQSGAEVKKPGATVKISCKVSGYTFTNYWLNWVKQAPGRGLEWIGRIDPSDSEIHYDQKFKDRVTITADKSTNTAYIELSSLRSEDTAVYYCALTGIYAMAYWGQGTTVTVSS |
| 1173. | PC32B8-HCDR1 | artificial | aa | NYWLN |
| 1174. | PC32B8-HCDR2 | artificial | aa | RIDPSDSEIHYDQKFKD |
| 1175. | PC32B8-HCDR3 | artificial | aa | TGIYAMAY |
| 1176. | PC32B8-H | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGGCGCCCGGCAGGGGCCTGGAATGGATAGGCAGGATCGACCCCAGCGACAGCGAGATCCACTACGACCAGAAGTTCAAGGACAGAGTCACCATAACCGCGGACAAGAGCACCAACACCGCCTACATCGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCA |
| 1177. | PC32B8-L | artificial | aa | DIVMTQSPPSLPVTPGEPASISCRSSKSLLHSNGNTYLYWYLQKPGQSPQLLIYRMSNLASGVPDRFSGSGSGTDFTLKISRVEAEDVGVYYCLQHLEYPYTFGQGTKLEIK |
| 1178. | PC32B8-LCDR1 | artificial | aa | RSSKSLLHSNGNTYLY |
| 1179. | PC32B8-LCDR2 | artificial | aa | RMSNLAS |
| 1180. | PC32B8-LCDR3 | artificial | aa | LQHLEYPYT |
| 1181. | PC32B8-L | artificial | nt | GACATCGTGATGACCCAGTCACCTCCAAGCCTGCCAGTGACCCCAGGCGAGCCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1182. | PC32B8-HL | artificial | aa | EVQLVQSGAEVKKPGATVKISCKVSGYTFTNYWLNWVKQAPGRGLEWIGRIDPSDSEIHYDQKFKDRVTITADKSTNTAYIELSSLRSEDTAVYYCALTGIYAMAYWGQGTTVTVSSGGGGSGGGGSGGGGSDIVMTQSPPSLPVTPGEPASISCRSSKSLLHSNGNTYLYWYLQKPGQSPQLLIYRMSNLASGVPDRFSGSGSGTDFTLKISRVEAEDVGVYYCLQHLEYPYTFGQGTKLEIK |

| | | | | |
|---|---|---|---|---|
| 1183. | PC32B8-HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGGCGCCCGGCAGGGGCCTGGAATGGATAGGCAGGATCGACCCCAGCGACAGCGAGATCCACTACGACCAGAAGTTCAAGGACAGAGTCACCATAACCGCGGACAAGAGCACCAACACCGCCTACATCGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGTCACCTCCAAGCCTGCCAGTGACCCCAGGCGAGCCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1184. | PC32B8 HL x I2C HL | artificial | aa | EVQLVQSGAEVKKPGATVKISCKVSGYTFTNYWLNWVKQAPGRGLEWIGRIDPSDSEIHYDQKFKDRVTITADKSTNTAYIELSSLRSEDTAVYYCALTGIYAMAYWGQGTTVTVSSGGGGSGGGGSGGGGSDIVMTQSPPSLPVTPGEPASISCRSSKSLLHSNGNTYLYWYLQKPGQSPQLLIYRMSNLASGVPDRFSGSGSGTDFTLKISRVEAEDVGVYYCLQHLEYPYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1185. | PC32B8 HL x I2C HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGGCGCCCGGCAGGGGCCTGGAATGGATAGGCAGGATCGACCCCAGCGACAGCGAGATCCACTACGACCAGAAGTTCAAGGACAGAGTCACCATAACCGCGGACAAGAGCACCAACACCGCCTACATCGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGTCACCTCCAAGCCTGCCAGTGACCCCAGGCGAGCCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACC |

| | | | | |
|---|---|---|---|---|
| | | | | AAGCTGGAAATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1186. | PC32D5-H | artificial | aa | EVQLVQSGAEVVKPGATVKISCKVSGYTFTNYWLNWVKQAPGRGLEWIGRIDPSDSEIHYDQKFKDRVTITADKSTSTAYMELSSLRSEDTAVYYCALTGIYAMAYWGQGTTVTVSS |
| 1187. | PC32D5-HCDR1 | artificial | aa | NYWLN |
| 1188. | PC32D5-HCDR2 | artificial | aa | RIDPSDSEIHYDQKFKD |
| 1189. | PC32D5-HCDR3 | artificial | aa | TGIYAMAY |
| 1190. | PC32D5-H | artificial | nt | GAGGTGCAGCTGGTCCAGTCAGGCGCCGAAGTGGTGAAGCCTGGCGCCACAGTGAAGATATCCTGCAAGGTCAGCGGCTACACCTTCACCAACTACTGGCTGAACTGGGTGAAACAGGCGCCCGGCAGGGGCCTGGAATGGATAGGCAGGATCGACCCCAGCGACAGCGAGATCCACTACGACCAGAAGTTCAAGGACAGAGTCACCATAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCA |
| 1191. | PC32D5-L | artificial | aa | DIVMTQSPLSLPVTPGESASISCRSSKSLLHSNGNTYLYWYLQKPGQSPQLLIYRMSNLASGVPDRFSGSGSGTDFTLRISRVEAEDVGVYYCLQHLEYPYTFGQGTKLEIK |
| 1192. | PC32D5-LCDR1 | artificial | aa | RSSKSLLHSNGNTYLY |
| 1193. | PC32D5-LCDR2 | artificial | aa | RMSNLAS |
| 1194. | PC32D5-LCDR3 | artificial | aa | LQHLEYPYT |
| 1195. | PC32D5-L | artificial | nt | GACATCGTGATGACCCAGTCACCTCTAAGCCTGCCAGTGACCCCAGGCGAGTCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAGGATCAGCAGGGTGGAGGCCGAGGACGTGGGCGTGTACTA |

| | | | | |
|---|---|---|---|---|
| | | | | CTGCCTGCAGCACCTGGAATACCCCTACACCTTCGGCCAAGGGACCAAGCTGGAA<br>ATCAAG |
| 1196. | PC32D5-HL | artificial | aa | EVQLVQSGAEVVKPGATVKISCKVSGYTFTNYWLNWVKQAPGRGLEWIGRIDPSDSEI<br>HYDQKFKDRVTITADKSTSTAYMELSSLRSEDTAVYYCALTGIYAMAYWGQGTTVTVSS<br>GGGGSGGGGSGGGGSDIVMTQSPLSLPVTPGESASISCRSSKSLLHSNGNTYLYWYL<br>QKPGQSPQLLIYRMSNLASGVPDRFSGSGSGTDFTLRISRVEAEDVGVYYCLQHLEYP<br>YTFGQGTKLEIK |
| 1197. | PC32D5-HL | artificial | nt | GAGGTGCAGCTGGTCCAGTCAGGCGCCGAAGTGGTGAAGCCTGGCGCCACAGTG<br>AAGATATCCTGCAAGGTCAGCGGCTACACCTTCACCAACTACTGGCTGAACTGGGT<br>GAAACAGGCGCCCGGCAGGGGCCTGGAATGGATAGGCAGGATCGACCCCAGCGA<br>CAGCGAGATCCACTACGACCAGAAGTTCAAGGACAGAGTCACCATAACCGCGGACA<br>AGAGCACCAGCACCGCCTACATGGAGCTGTCCAGCCTGAGAAGCGAGGACACCGC<br>CGTGTACTATTGCGCCCTGACCGGCATCTACGCCATGGCCTACTGGGGCCAGGGC<br>ACCACGGTCACCGTCTCCTCAGGTGGTGGTGGTTCTGGCGGCGGCGGCTCCGGTG<br>GTGGTGGTTCTGACATCGTGATGACCCAGTCACCTCTAAGCCTGCCAGTGACCCCA<br>GGCGAGTCAGCGTCCATCAGCTGCAGGTCCAGCAAGAGCCTGCTGCACAGCAACG<br>GCAACACCTACCTGTACTGGTATCTGCAGAAGCCAGGCCAGAGCCCCCAGCTGCT<br>GATCTACAGGATGAGCAACCTGGCTAGCGGCGTGCCAGACAGATTCAGCGGCAGC<br>GGCTCTGGAACCGACTTCACCCTGAGGATCAGCAGGGTGGAGGCCGAGGACGTG<br>GGCGTGTACTACTGCCTGCAGCACCTGGAATACCCCTACACCTTCGGCCAAGGGA<br>CCAAGCTGGAAATCAAG |
| 1198. | PC32D5 HL x I2C HL | artificial | aa | EVQLVQSGAEVVKPGATVKISCKVSGYTFTNYWLNWVKQAPGRGLEWIGRIDPSDSEI<br>HYDQKFKDRVTITADKSTSTAYMELSSLRSEDTAVYYCALTGIYAMAYWGQGTTVTVSS<br>GGGGSGGGGSGGGGSDIVMTQSPLSLPVTPGESASISCRSSKSLLHSNGNTYLYWYL<br>QKPGQSPQLLIYRMSNLASGVPDRFSGSGSGTDFTLRISRVEAEDVGVYYCLQHLEYP<br>YTFGQGTKLEIKSGGGGSEVQLVESGGGLVQPGGSLKLSCAASGFTFNKYAMNWVRQ<br>APGKGLEWVARIRSKYNNYATYYADSVKDRFTISRDDSKNTAYLQMNNLKTEDTAVYY<br>CVRHGNFGNSYISYWAYWGQGTLVTVSSGGGGSGGGGSGGGGSQTVVTQEPSLTVS<br>PGGTVTLTCGSSTGAVTSGNYPNWVQQKPGQAPRGLIGGTKFLAPGTPARFSGSLLG<br>GKAALTLSGVQPEDEAEYYCVLWYSNRWVFGGGTKLTVL |
| 1199. | PC32D5 HL x I2C HL | artificial | nt | GAGGTGCAGCTGGTCCAGTCAGGCGCCGAAGTGGTGAAGCCTGGCGCCACAGTG<br>AAGATATCCTGCAAGGTCAGCGGCTACACCTTCACCAACTACTGGCTGAACTGGGT<br>GAAACAGGCGCCCGGCAGGGGCCTGGAATGGATAGGCAGGATCGACCCCAGCGA<br>CAGCGAGATCCACTACGACCAGAAGTTCAAGGACAGAGTCACCATAACCGCGGACA |

| | | | | |
|---|---|---|---|---|
| | | | | AGAGCACCAGCACCGCCTACATGGAGCTGTCCAGCCTGAGAAGCGAGGACACCGC<br>CGTGTACTATTGCGCCCTGACCGGCATCTACGCCATGGCCTACTGGGGCCAGGGC<br>ACCACGGTCACCGTCTCCTCAGGTGGTGGTGGTTCTGGCGGCGGCGGCTCCGGTG<br>GTGGTGGTTCTGACATCGTGATGACCCAGTCACCTCTAAGCCTGCCAGTGACCCCA<br>GGCGAGTCAGCGTCCATCAGCTGCAGGTCCAGCAAGAGCCTGCTGCACAGCAACG<br>GCAACACCTACCTGTACTGGTATCTGCAGAAGCCAGGCCAGAGCCCCCAGCTGCT<br>GATCTACAGGATGAGCAACCTGGCTAGCGGCGTGCCAGACAGATTCAGCGGCAGC<br>GGCTCTGGAACCGACTTCACCCTGAGGATCAGCAGGGTGGAGGCCGAGGACGTG<br>GGCGTGTACTACTGCCTGCAGCACCTGGAATACCCCTACACCTTCGGCCAAGGGA<br>CCAAGCTGGAAATCAAGTCCGGAGGTGGTGGATCCGAGGTGCAGCTGGTCGAGTC<br>TGGAGGAGGATTGGTGCAGCCTGGAGGGTCATTGAAACTCTCATGTGCAGCCTCTG<br>GATTCACCTTCAATAAGTACGCCATGAACTGGGTCCGCCAGGCTCCAGGAAAGGGT<br>TTGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAA<br>ACTGGGTCCAACAAAAACCAGGTCAGGCACCCCGTGGTCTAATAGGTGGGACTAAG<br>TTCCTCGCCCCCGGTACTCCTGCCAGATTCTCAGGCTCCCTGCTTGGAGGCAAGGC<br>TGCCCTCACCCTCTCAGGGGTACAGCCAGAGGATGAGGCAGAATATTACTGTGTTC<br>TATGGTACAGCAACCGCTGGGTGTTCGGTGGAGGAACCAAACTGACTGTCCTA |
| 1200. | PC17D10-H | artificial | aa | EVQLVQSGAEVKKPGATVKISCKVSGYTFTNYWLNWVKQTPGKGLEWIGRIDPSDSEIH<br>YDQKFKDKVTITADKSTSTAYMELSSLRSEDTAVYYCALTGIYAMAYWGQGTTVTVSS |
| 1201. | PC17D10-HCDR1 | artificial | aa | NYWLN |
| 1202. | PC17D10-HCDR2 | artificial | aa | RIDPSDSEIHYDQKFKD |
| 1203. | PC17D10-HCDR3 | artificial | aa | TGIYAMAY |
| 1204. | PC17D10-H | artificial | nt | GAGGTGCAGCTGGTCCAGTCAGGCGCCGAAGTGAAGAAGCCTGGCGCCACAGTGA<br>AGATATCCTGCAAGGTCAGCGGCTACACCTTCACCAACTACTGGCTGAACTGGGTG<br>AAACAGACGCCCGGCAAGGGCCTGGAATGGATAGGCAGGATCGACCCCAGCGACA<br>GCGAGATCCACTACGACCAGAAGTTCAAGGACAAAGTCACCATAACCGCGGACAAG<br>AGCACCAGCACCGCCTACATGGAGCTGTCCAGCCTGAGAAGCGAGGACACCGCCG<br>TGTACTATTGCGCCCTGACCGGCATCTACGCCATGGCCTACTGGGGCCAGGGCAC<br>CACGGTCACCGTCTCCTCA |

| | | | | |
|---|---|---|---|---|
| 1205. | PC17D10-L | artificial | aa | DIVMTQAPLSLPVTPGEPVSISCRSSKSLLHSNGNTYLYWYLQKPGQSPQLLIYRMSNLASGVPDRFSGSGSGTDFTLRISRVEAEDVGVYYCLQHLEYPYTFGQGTKLEIK |
| 1206. | PC17D10-LCDR1 | artificial | aa | RSSKSLLHSNGNTYLY |
| 1207. | PC17D10-LCDR2 | artificial | aa | RMSNLAS |
| 1208. | PC17D10-LCDR3 | artificial | aa | LQHLEYPYT |
| 1209. | PC17D10-L | artificial | nt | GACATCGTGATGACCCAGGCACCTCTAAGCCTGCCAGTGACCCCAGGCGAGCCAGTGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAGGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1210. | PC17D10-HL | artificial | aa | EVQLVQSGAEVKKPGATVKISCKVSGYTFTNYWLNWVKQTPGKGLEWIGRIDPSDSEIHYDQKFKDKVTITADKSTSTAYMELSSLRSEDTAVYYCALTGIYAMAYWGQGTTVTVSSGGGGSGGGGSGGGGSDIVMTQAPLSLPVTPGEPVSISCRSSKSLLHSNGNTYLYWYLQKPGQSPQLLIYRMSNLASGVPDRFSGSGSGTDFTLRISRVEAEDVGVYYCLQHLEYPYTFGQGTKLEIK |
| 1211. | PC17D10-HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGACGCCCGGCAAGGGCCTGGAATGGATAGGCAGGATCGACCCCAGCGACAGCGAGATCCACTACGACCAGAAGTTCAAGGACAAAGTCACCATAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGGCACCTCTAAGCCTGCCAGTGACCCCAGGCGAGCCAGTGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAGGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1212. | PC17D10 HL x I2C HL | artificial | aa | EVQLVQSGAEVKKPGATVKISCKVSGYTFTNYWLNWVKQTPGKGLEWIGRIDPSDSEIHYDQKFKDKVTITADKSTSTAYMELSSLRSEDTAVYYCALTGIYAMAYWGQGTTVTVSSGGGGSGGGGSGGGGSDIVMTQAPLSLPVTPGEPVSISCRSSKSLLHSNGNTYLYWYLQKPGQSPQLLIYRMSNLASGVPDRFSGSGSGTDFTLRISRVEAEDVGVYYCLQHLEYPYT |

| | | | | |
|---|---|---|---|---|
| | | | | FGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1213. | PC17D10 HL x I2C HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGACGCCCGGCAAGGGCCTGGAATGGATAGGCAGGATCGACCCCAGCGACAGCGAGATCCACTACGACCAGAAGTTCAAGGACAAAGTCACCATAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGGCACCTCTAAGCCTGCCAGTGACCCCAGGCGAGCCAGTGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAGGATCAGCAGGGTGGAGGCCGAGGACGTGGGCGTGTACTACTGCCTGCAGCACCTGGAATACCCCTACACCTTCGGCCAAGGGACCAAGCTGGAAATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1214. | PC16E12-H | artificial | aa | EVQLVQSGAEVKKPGAPVKISCKVSGYTFTNYWLNWVKQRPGKGLEWIGRIDPSDSEIHYDQKFKDRVTITVDKSTSTAYIELSSLRSEDTAVYYCALTGIYAMAYWGQGTTVTVSS |
| 1215. | PC16E12-HCDR1 | artificial | aa | NYWLN |
| 1216. | PC16E12-HCDR2 | artificial | aa | RIDPSDSEIHYDQKFKD |

| | | | | |
|---|---|---|---|---|
| 1217. | PC16E12-HCDR3 | artificial | aa | TGIYAMAY |
| 1218. | PC16E12-H | artificial | nt | GAGGTGCAGCTGGTCCAGTCAGGCGCCGAAGTGAAGAAGCCTGGCGCCCCAGTG<br>AAGATATCCTGCAAGGTCAGCGGCTACACCTTCACCAACTACTGGCTGAACTGGGT<br>GAAACAGAGGCCCGGCAAGGGCCTGGAATGGATAGGTAGGATCGACCCCAGCGAC<br>AGCGAGATCCACTACGACCAGAAGTTCAAGGACAGAGTCACCATAACCGTGGACAA<br>GAGCACCAGCACCGCCTACATCGAGCTGTCCAGCCTGAGAAGCGAGGACACCGCC<br>GTGTACTATTGCGCCCTGACCGGCATCTACGCCATGGCCTACTGGGGCCAGGGCA<br>CCACGGTCACCGTCTCCTCA |
| 1219. | PC16E12-L | artificial | aa | DIVMTQSPLSLPVTPGEPVSISCRSSKSLLHSNGNTYLYWYLQKPGQSPQLLIYRMSNL<br>ASGVPDRFSGSGSGTDFTLKISRVEAEDVGVYYCLQHLEYPYTFGQGTKLEIK |
| 1220. | PC16E12-LCDR1 | artificial | aa | RSSKSLLHSNGNTYLY |
| 1221. | PC16E12-LCDR2 | artificial | aa | RMSNLAS |
| 1222. | PC16E12-LCDR3 | artificial | aa | LQHLEYPYT |
| 1223. | PC16E12-L | artificial | nt | GACATCGTGATGACCCAGTCACCTCTAAGCCTGCCAGTGACCCCAGGCGAGCCAG<br>TGTCCATCAGCTGCAGGTCCAGCAAGAGCCTGCTGCACAGCAACGGCAACACCTA<br>CCTGTACTGGTATCTGCAGAAGCCAGGCCAGAGCCCCCAGCTGCTGATCTACAGG<br>ATGAGCAACCTGGCTAGCGGCGTGCCAGACAGATTCAGCGGCAGCGGCTCTGGAA<br>CCGACTTCACCCTGAAGATCAGCAGGGTGGAGGCCGAGGACGTGGGCGTGTACTA<br>CTGCCTGCAGCACCTGGAATACCCCTACACCTTCGGCCAAGGGACCAAGCTGGAA<br>ATCAAG |
| 1224. | PC16E12-HL | artificial | aa | EVQLVQSGAEVKKPGAPVKISCKVSGYTFTNYWLNWVKQRPGKGLEWIGRIDPSDSEI<br>HYDQKFKDRVTITVDKSTSTAYIELSSLRSEDTAVYYCALTGIYAMAYWGQGTTVTVSS<br>GGGGSGGGGSGGGGSDIVMTQSPLSLPVTPGEPVSISCRSSKSLLHSNGNTYLYWYL<br>QKPGQSPQLLIYRMSNLASGVPDRFSGSGSGTDFTLKISRVEAEDVGVYYCLQHLEYP<br>YTFGQGTKLEIK |
| 1225. | PC16E12-HL | artificial | nt | GAGGTGCAGCTGGTCCAGTCAGGCGCCGAAGTGAAGAAGCCTGGCGCCCCAGTG<br>AAGATATCCTGCAAGGTCAGCGGCTACACCTTCACCAACTACTGGCTGAACTGGGT<br>GAAACAGAGGCCCGGCAAGGGCCTGGAATGGATAGGTAGGATCGACCCCAGCGAC<br>AGCGAGATCCACTACGACCAGAAGTTCAAGGACAGAGTCACCATAACCGTGGACAA<br>GAGCACCAGCACCGCCTACATCGAGCTGTCCAGCCTGAGAAGCGAGGACACCGCC<br>GTGTACTATTGCGCCCTGACCGGCATCTACGCCATGGCCTACTGGGGCCAGGGCA<br>CCACGGTCACCGTCTCCTCAGGTGGTGGTGGTTCTGGCGGCGGCGGCTCCGGTG<br>GTGGTGGTTCTGACATCGTGATGACCCAGTCACCTCTAAGCCTGCCAGTGACCCCA<br>GGCGAGCCAGTGTCCATCAGCTGCAGGTCCAGCAAGAGCCTGCTGCACAGCAACG |

| | | | | |
|---|---|---|---|---|
| | | | | GCAACACCTACCTGTACTGGTATCTGCAGAAGCCAGGCCAGAGCCCCCAGCTGCT<br>GATCTACAGGATGAGCAACCTGGCTAGCGGCGTGCCAGACAGATTCAGCGGCAGC<br>GGCTCTGGAACCGACTTCACCCTGAAGATCAGCAGGGTGGAGGCCGAGGACGTGG<br>GCGTGTACTACTGCCTGCAGCACCTGGAATACCCCTACACCTTCGGCCAAGGGACC<br>AAGCTGGAAATCAAG |
| 1226. | PC16E12 HL x I2C HL | artificial | aa | EVQLVQSGAEVKKPGAPVKISCKVSGYTFTNYWLNWVKQRPGKGLEWIGRIDPSDSEI<br>HYDQKFKDRVTITVDKSTSTAYIELSSLRSEDTAVYYCALTGIYAMAYWGQGTTVTVSS<br>GGGGSGGGGSGGGGSDIVMTQSPLSLPVTPGEPVSISCRSSKSLLHSNGNTYLYWYL<br>QKPGQSPQLLIYRMSNLASGVPDRFSGSGSGTDFTLKISRVEAEDVGVYYCLQHLEYP<br>YTFGQGTKLEIKSGGGGSEVQLVESGGGLVQPGGSLKLSCAASGFTFNKYAMNWVRQ<br>APGKGLEWVARIRSKYNNYATYYADSVKDRFTISRDDSKNTAYLQMNNLKTEDTAVYY<br>CVRHGNFGNSYISYWAYWGQGTLVTVSSGGGGSGGGGSGGGGSQTVVTQEPSLTVS<br>PGGTVTLTCGSSTGAVTSGNYPNWVQQKPGQAPRGLIGGTKFLAPGTPARFSGSLLG<br>GKAALTLSGVQPEDEAEYYCVLWYSNRWVFGGGTKLTVL |
| 1227. | PC16E12 HL x I2C HL | artificial | nt | GAGGTGCAGCTGGTCCAGTCAGGCGCCGAAGTGAAGAAGCCTGGCGCCCCAGTG<br>AAGATATCCTGCAAGGTCAGCGGCTACACCTTCACCAACTACTGGCTGAACTGGGT<br>GAAACAGAGGCCCGGCAAGGGCCTGGAATGGATAGGTAGGATCGACCCCAGCGAC<br>AGCGAGATCCACTACGACCAGAAGTTCAAGGACAGAGTCACCATAACCGTGGACAA<br>GAGCACCAGCACCGCCTACATCGAGCTGTCCAGCCTGAGAAGCGAGGACACCGCC<br>GTGTACTATTGCGCCCTGACCGGCATCTACGCCATGGCCTACTGGGGCCAGGGCA<br>CCACGGTCACCGTCTCCTCAGGTGGTGGTGGTTCTGGCGGCGGCGGCTCCGGTG<br>GTGGTGGTTCTGACATCGTGATGACCCAGTCACCTCTAAGCCTGCCAGTGACCCCA<br>GGCGAGCCAGTGTCCATCAGCTGCAGGTCCAGCAAGAGCCTGCTGCACAGCAACG<br>GCAACACCTACCTGTACTGGTATCTGCAGAAGCCAGGCCAGAGCCCCCAGCTGCT<br>GATCTACAGGATGAGCAACCTGGCTAGCGGCGTGCCAGACAGATTCAGCGGCAGC<br>GGCTCTGGAACCGACTTCACCCTGAAGATCAGCAGGGTGGAGGCCGAGGACGTGG<br>GCGTGTACTACTGCCTGCAGCACCTGGAATACCCCTACACCTTCGGCCAAGGGACC<br>AAGCTGGAAATCAAGTCCGGAGGTGGTGGATCCGAGGTGCAGCTGGTCGAGTCTG<br>GAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTT<br>TGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC |

| | | | | |
|---|---|---|---|---|
| | | | | AGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1228. | PC08F4-H | artificial | aa | EVQLVQSGAEVKKPGATVKISCKVSGYTFTNYWLNWVQQRPGKGLEWIGRIDPSDSEIHYDQKFKDRVTLTADKSTSTAYMELSSLRSEDTAVYYCALTGIYAMAYWGQGTTVTVSS |
| 1229. | PC08F4-HCDR1 | artificial | aa | NYWLN |
| 1230. | PC08F4-HCDR2 | artificial | aa | RIDPSDSEIHYDQKFKD |
| 1231. | PC08F4-HCDR3 | artificial | aa | TGIYAMAY |
| 1232. | PC08F4-H | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGCAACAGAGGCCCGGCAAGGGCCTGGAATGGATAGGCAGGATCGACCCCAGCGACAGCGAGATCCACTACGACCAGAAGTTCAAGGACAGAGTCACCCTAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCA |
| 1233. | PC08F4-L | artificial | aa | DIVMTQSPLSLPVTPGESASISCRSSKSLLHSNGNTYLYWYLQKPGQSPQLLIYRMSNLASGVPDRFSGSGSGTDFTLKISRVEAEDVGVYYCLQHLEYPYTFGQGTKLEIK |
| 1234. | PC08F4-LCDR1 | artificial | aa | RSSKSLLHSNGNTYLY |
| 1235. | PC08F4-LCDR2 | artificial | aa | RMSNLAS |
| 1236. | PC08F4-LCDR3 | artificial | aa | LQHLEYPYT |
| 1237. | PC08F4-L | artificial | nt | GACATCGTGATGACCCAGTCACCTCTAAGCCTGCCAGTGACCCCAGGCGAGTCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1238. | PC08F4-HL | artificial | aa | EVQLVQSGAEVKKPGATVKISCKVSGYTFTNYWLNWVQQRPGKGLEWIGRIDPSDSEIHYDQKFKDRVTLTADKSTSTAYMELSSLRSEDTAVYYCALTGIYAMAYWGQGTTVTVSSGGGGSGGGGSGGGGSDIVMTQSPLSLPVTPGESASISCRSSKSLLHSNGNTYLYWYLQKPGQSPQLLIYRMSNLASGVPDRFSGSGSGTDFTLKISRVEAEDVGVYYCLQHLEYP |

| | | | | |
|---|---|---|---|---|
| | | | | YTFGQGTKLEIK |
| 1239. | PC08F4-HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGCAACAGAGGCCCGGCAAGGGCCTGGAATGGATAGGCAGGATCGACCCCAGCGACAGCGAGATCCACTACGACCAGAAGTTCAAGGACAGAGTCACCCTAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGTCACCTCTAAGCCTGCCAGTGACCCCAGGCGAGTCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1240. | PC08F4 HL x I2C HL | artificial | aa | EVQLVQSGAEVKKPGATVKISCKVSGYTFTNYWLNWVQQRPGKGLEWIGRIDPSDSEIHYDQKFKDRVTLTADKSTSTAYMELSSLRSEDTAVYYCALTGIYAMAYWGQGTTVTVSSGGGGSGGGGSGGGGSDIVMTQSPLSLPVTPGESASISCRSSKSLLHSNGNTYLYWYLQKPGQSPQLLIYRMSNLASGVPDRFSGSGSGTDFTLKISRVEAEDVGVYYCLQHLEYPYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1241. | PC08F4 HL x I2C HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGCAACAGAGGCCCGGCAAGGGCCTGGAATGGATAGGCAGGATCGACCCCAGCGACAGCGAGATCCACTACGACCAGAAGTTCAAGGACAGAGTCACCCTAACCGCGGACAAGAGCACCAGCACCGCCTACATGGAGCTGTCCAGCCTGAGAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGTCACCTCTAAGCCTGCCAGTGACCCCAGGCGAGTCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGG |

| | | | | |
|---|---|---|---|---|
| | | | | GCGTGTACTACTGCCTGCAGCACCTGGAATACCCCTACACCTTCGGCCAAGGGACC<br>AAGCTGGAAATCAAGTCCGGAGGTGGTGGATCCGAGGTGCAGCTGGTCGAGTCTG<br>GAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTT<br>TGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAA<br>ACTGGGTCCAACAAAAACCAGGTCAGGCACCCCGTGGTCTAATAGGTGGGACTAAG<br>TTCCTCGCCCCCGGTACTCCTGCCAGATTCTCAGGCTCCCTGCTTGGAGGCAAGGC<br>TGCCCTCACCCTCTCAGGGGTACAGCCAGAGGATGAGGCAGAATATTACTGTGTTC<br>TATGGTACAGCAACCGCTGGGTGTTCGGTGGAGGAACCAAACTGACTGTCCTA |
| 1242. | PC16F5-H | artificial | aa | EVQLVQSGAEVMKPGATVKISCKVSGYTFTNYWLNWVKQAPGKGLEWIGRIDPSDSEI<br>HYDQKFKDRVTITANKSTSTAYMELSSLTSEDTAVYYCALTGIYAMAYWGQGTTVTVSS |
| 1243. | PC16F5-HCDR1 | artificial | aa | NYWLN |
| 1244. | PC16F5-HCDR2 | artificial | aa | RIDPSDSEIHYDQKFKD |
| 1245. | PC16F5-HCDR3 | artificial | aa | TGIYAMAY |
| 1246. | PC16F5-H | artificial | nt | CAGGTGCAGCTGGTCCAGTCAGGACCTGAGGTGATGAAGCCTGGGGCTTCAGTGA<br>AGATGTCCTGCAAGGCTTCTGGATACACATTCACTGACTATGTTATACACTGGGTGA<br>GGCAGGCACCTGGAAAGCGCCTTGAGTGGATGGGATATATTAATCCTTATGATGAT<br>GATACTACCTACAACCAGAAGTTCAAGGGCCGGGTCACAATTACTGTAGACAAATC<br>CGCCAGCACAGCCTACATGGAACTCAACAGCCTGACATCTGAGGACACTGCAGTCT<br>ATTACTGTGCAAGAAGGGGGAACTCCTATGATGGTTACTTCGACTATTCTATGGACT<br>ACTGGGGTCAAGGAACCTTGGTCACCGTCTCCTCA |
| 1247. | PC16F5-L | artificial | aa | DIVMTQSPLSLPVTPGEPVSISCRSSKSLLHSNGNTYLYWYLQKPGQSPQLLIYRMSNL<br>ASGVPDRFSGSGSGTDFTLKISRVEAEDVGVYYCLQHLEYPYTFGQGTKLEIK |
| 1248. | PC16F5-LCDR1 | artificial | aa | RSSKSLLHSNGNTYLY |
| 1249. | PC16F5-LCDR2 | artificial | aa | RMSNLAS |
| 1250. | PC16F5-LCDR3 | artificial | aa | LQHLEYPYT |
| 1251. | PC16F5-L | artificial | nt | GACATCCAGCTGACCCAGTCTCCAAGCTTCATGTCCGCATCAGTAGGAGACAGAGT<br>CACCATCACCTGCAGGGCCAGTCAGAATGTGGGTACTGCTGTAGCCTGGTATCAAC<br>AGAAACCAGGACAAGCCCCTAAATTACTGATTTACTCGGCATCGAATCGGTACACTG<br>GAGTCCCTAGTCGCTTCTCAGGCAGTGGATCTGGGACAGAGTTCACTCTCACCATC |

| | | | | |
|---|---|---|---|---|
| | | | | AGCAGTCTGCAGTCTGAAGACTTCGCAACTTATTACTGCCAGCAATATACCAACTATCCCATGTATACGTTTGGACAGGGGACCAAGCTGGAAATAAAA |
| 1252. | PC16F5-HL | artificial | aa | EVQLVQSGAEVMKPGATVKISCKVSGYTFTNYWLNWVKQAPGKGLEWIGRIDPSDSEIHYDQKFKDRVTITANKSTSTAYMELSSLTSEDTAVYYCALTGIYAMAYWGQGTTVTVSSGGGGSGGGGSGGGGSDIVMTQSPLSLPVTPGEPVSISCRSSKSLLHSNGNTYLYWYLQKPGQSPQLLIYRMSNLASGVPDRFSGSGSGTDFTLKISRVEAEDVGVYYCLQHLEYPYTFGQGTKLEIK |
| 1253. | PC16F5-HL | artificial | nt | GAGGTGCAGCTGGTCCAGTCAGGCGCCGAAGTGATGAAGCCTGGCGCCACAGTGAAGATATCCTGCAAGGTCAGCGGCTACACCTTCACCAACTACTGGCTGAACTGGGTGAAACAGGCGCCCGGCAAGGGCCTGGAATGGATAGGCAGGATCGACCCCAGCGACAGCGAGATCCACTACGACCAGAAGTTCAAGGACAGAGTCACCATAACCGCGAACAAGAGCACCAGCACCGCCTACATGGAGCTGTCCAGCCTGACAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGTCACCTCTAAGCCTGCCAGTGACCCCAGGCGAGCCAGTGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1254. | PC176F5 HL x I2C HL | artificial | aa | EVQLVQSGAEVMKPGATVKISCKVSGYTFTNYWLNWVKQAPGKGLEWIGRIDPSDSEIHYDQKFKDRVTITANKSTSTAYMELSSLTSEDTAVYYCALTGIYAMAYWGQGTTVTVSSGGGGSGGGGSGGGGSDIVMTQSPLSLPVTPGEPVSISCRSSKSLLHSNGNTYLYWYLQKPGQSPQLLIYRMSNLASGVPDRFSGSGSGTDFTLKISRVEAEDVGVYYCLQHLEYPYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1255. | PC16F5 HL x I2C HL | artificial | nt | GAGGTGCAGCTGGTCCAGTCAGGCGCCGAAGTGATGAAGCCTGGCGCCACAGTGAAGATATCCTGCAAGGTCAGCGGCTACACCTTCACCAACTACTGGCTGAACTGGGTGAAACAGGCGCCCGGCAAGGGCCTGGAATGGATAGGCAGGATCGACCCCAGCGACAGCGAGATCCACTACGACCAGAAGTTCAAGGACAGAGTCACCATAACCGCGAACAAGAGCACCAGCACCGCCTACATGGAGCTGTCCAGCCTGACAAGCGAGGACACCGCCGTGTACTATTGCGCCCTGACCGGCATCTACGCCATGGCCTACTGGGGCCAGGGCA |

| | | | | |
|---|---|---|---|---|
| | | | | CCACGGTCACCGTCTCCTCAGGTGGTGGTGGTTCTGGCGGCGGCGGCTCCGGTG GTGGTGGTTCTGACATCGTGATGACCCAGTCACCTCTAAGCCTGCCAGTGACCCCA GGCGAGCCAGTGTCCATCAGCTGCAGGTCCAGCAAGAGCCTGCTGCACAGCAACG GCAACACCTACCTGTACTGGTATCTGCAGAAGCCAGGCCAGAGCCCCCAGCTGCT GATCTACAGGATGAGCAACCTGGCTAGCGGCGTGCCAGACAGATTCAGCGGCAGC GGCTCTGGAACCGACTTCACCCTGAAGATCAGCAGGGTGGAGGCCGAGGACGTGG GCGTGTACTACTGCCTGCAGCACCTGGAATACCCCTACACCTTCGGCCAAGGGACC AAGCTGGAAATCAAGTCCGGAGGTGGTGGATCCGAGGTGCAGCTGGTCGAGTCTG GAGGAGGATTGGTGCAGCCTGGAGGGTCATTGAAACTCTCATGTGCAGCCTCTGG ATTCACCTTCAATAAGTACGCCATGAACTGGGTCCGCCAGGCTCCAGGAAAGGGTT TGGAATGGGTTGCTCGCATAAGAAGTAAATATAATAATTATGCAACATATTATGCCG ATTCAGTGAAAGACAGGTTCACCATCTCCAGAGATGATTCAAAAAACACTGCCTATC TACAAATGAACAACTTGAAAACTGAGGACACTGCCGTGTACTACTGTGTGAGACATG GGAACTTCGGTAATAGCTACATATCCTACTGGGCTTACTGGGGCCAAGGGACTCTG GTCACCGTCTCCTCAGGTGGTGGTGGTTCTGGCGGCGGCGGCTCCGGTGGTGGT GGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAA ACTGGGTCCAACAAAAACCAGGTCAGGCACCCCGTGGTCTAATAGGTGGGACTAAG TTCCTCGCCCCCGGTACTCCTGCCAGATTCTCAGGCTCCCTGCTTGGAGGCAAGGC TGCCCTCACCCTCTCAGGGGTACAGCCAGAGGATGAGGCAGAATATTACTGTGTTC TATGGTACAGCAACCGCTGGGTGTTCGGTGGAGGAACCAAACTGACTGTCCTA |
| 1256. | PC17H10-H | artificial | aa | EVQLVQSGAEVKKPGATVKISCKVSGYTFTNYWLNWVKQTPGKGLEWMGRIDPSDSEI HYDQKFKDRVTLTADTSTDTAYMELSSLTSEDTAVYYCALTGIYAMAYWGQGTTVTVS S |
| 1257. | PC17H10-HCDR1 | artificial | aa | NYWLN |
| 1258. | PC17H10-HCDR2 | artificial | aa | RIDPSDSEIHYDQKFKD |
| 1259. | PC17H10-HCDR3 | artificial | aa | TGIYAMAY |
| 1260. | PC17H10-H | artificial | nt | GAGGTGCAGCTGGTCCAGTCAGGCGCCGAAGTGAAGAAGCCTGGCGCCACAGTGA AGATATCCTGCAAGGTCAGCGGCTACACCTTCACCAACTACTGGCTGAACTGGGTG AAACAGACGCCCGGCAAGGGCCTGGAATGGATGGGCAGGATCGACCCCAGCGAC AGCGAGATCCACTACGACCAGAAGTTCAAGGACAGAGTCACCCTAACCGCGGACA CGAGCACCGACACCGCCTACATGGAGCTGTCCAGCCTGACAAGCGAGGACACCGC CGTGTACTATTGCGCCCTGACCGGCATCTACGCCATGGCCTACTGGGGCCAGGGC ACCACGGTCACCGTCTCCTCA |
| 1261. | PC17H10-L | artificial | aa | DIVMTQAPLSLPVTPGEPASISCRSSKSLLHSNGNTYLYWYLQKPGQSPQLLIYRMSNL ASGVPDRFSGSGSGTDFTLKISRVEAEDVGVYYCLQHLEYPYTFGQGTKLEIK |

| | | | | |
|---|---|---|---|---|
| 1262. | PC17H10-LCDR1 | artificial | aa | RSSKSLLHSNGNTYLY |
| 1263. | PC17H10-LCDR2 | artificial | aa | RMSNLAS |
| 1264. | PC17H10-LCDR3 | artificial | aa | LQHLEYPYT |
| 1265. | PC17H10-L | artificial | nt | GACATCGTGATGACCCAGGCACCTCTAAGCCTGCCAGTGACCCCAGGCGAGCCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1266. | PC17H10-HL | artificial | aa | EVQLVQSGAEVKKPGATVKISCKVSGYTFTNYWLNWVKQTPGKGLEWMGRIDPSDSEIHYDQKFKDRVTLTADTSTDTAYMELSSLTSEDTAVYYCALTGIYAMAYWGQGTTVTVSSGGGGSGGGGSGGGGSDIVMTQAPLSLPVTPGEPASISCRSSKSLLHSNGNTYLYWYLQKPGQSPQLLIYRMSNLASGVPDRFSGSGSGTDFTLKISRVEAEDVGVYYCLQHLEYPYTFGQGTKLEIK |
| 1267. | PC17H10-HL | artificial | nt | GAGGTGCAGCTGGTCCAGTCAGGCGCCGAAGTGAAGAAGCCTGGCGCCACAGTGAAGATATCCTGCAAGGTCAGCGGCTACACCTTCACCAACTACTGGCTGAACTGGGTGAAACAGACGCCCGGCAAGGGCCTGGAATGGATGGGCAGGATCGACCCCAGCGACAGCGAGATCCACTACGACCAGAAGTTCAAGGACAGAGTCACCCTAACCGCGGACACGAGCACCGACACCGCCTACATGGAGCTGTCCAGCCTGACAAGCGAGGACACCGCCGTGTACTATTGCGCCCTGACCGGCATCTACGCCATGGCCTACTGGGGCCAGGGCACCACGGTCACCGTCTCCTCAGGTGGTGGTGGTTCTGGCGGCGGCGGCTCCGGTGGTGGTGGTTCTGACATCGTGATGACCCAGGCACCTCTAAGCCTGCCAGTGACCCCAGGCGAGCCAGCGTCCATCAGCTGCAGGTCCAGCAAGAGCCTGCTGCACAGCAACGGCAACACCTACCTGTACTGGTATCTGCAGAAGCCAGGCCAGAGCCCCCAGCTGCTGATCTACAGGATGAGCAACCTGGCTAGCGGCGTGCCAGACAGATTCAGCGGCAGCGGCTCTGGAACCGACTTCACCCTGAAGATCAGCAGGGTGGAGGCCGAGGACGTGGGCGTGTACTACTGCCTGCAGCACCTGGAATACCCCTACACCTTCGGCCAAGGGACCAAGCTGGAAATCAAG |
| 1268. | PC17H10 HL X I2C HL | artificial | aa | EVQLVQSGAEVKKPGATVKISCKVSGYTFTNYWLNWVKQTPGKGLEWMGRIDPSDSEIHYDQKFKDRVTLTADTSTDTAYMELSSLTSEDTAVYYCALTGIYAMAYWGQGTTVTVSSGGGGSGGGGSGGGGSDIVMTQAPLSLPVTPGEPASISCRSSKSLLHSNGNTYLYWYLQKPGQSPQLLIYRMSNLASGVPDRFSGSGSGTDFTLKISRVEAEDVGVYYCLQHLEYPYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVS |

| | | | | |
|---|---|---|---|---|
| | | | | PGGTVTLTCGSSTGAVTSGNYPNWVQQKPGQAPRGLIGGTKFLAPGTPARFSGSLLG<br>GKAALTLSGVQPEDEAEYYCVLWYSNRWVFGGGTKLTVL |
| 1269. | PC17H10 HL X I2C HL | artificial | nt | GAGGTGCAGCTGGTCCAGTCAGGCGCCGAAGTGAAGAAGCCTGGCGCCACAGTGA<br>AGATATCCTGCAAGGTCAGCGGCTACACCTTCACCAACTACTGGCTGAACTGGGTG<br>AAACAGACGCCCGGCAAGGGCCTGGAATGGATGGGCAGGATCGACCCCAGCGAC<br>AGCGAGATCCACTACGACCAGAAGTTCAAGGACAGAGTCACCCTAACCGCGGACA<br>CGAGCACCGACACCGCCTACATGGAGCTGTCCAGCCTGACAAGCGAGGACACCGC<br>CGTGTACTATTGCGCCCTGACCGGCATCTACGCCATGGCCTACTGGGGCCAGGGC<br>ACCACGGTCACCGTCTCCTCAGGTGGTGGTGGTTCTGGCGGCGGCGGCTCCGGTG<br>GTGGTGGTTCTGACATCGTGATGACCCAGGCACCTCTAAGCCTGCCAGTGACCCCA<br>GGCGAGCCAGCGTCCATCAGCTGCAGGTCCAGCAAGAGCCTGCTGCACAGCAACG<br>GCAACACCTACCTGTACTGGTATCTGCAGAAGCCAGGCCAGAGCCCCCAGCTGCT<br>GATCTACAGGATGAGCAACCTGGCTAGCGGCGTGCCAGACAGATTCAGCGGCAGC<br>GGCTCTGGAACCGACTTCACCCTGAAGATCAGCAGGGTGGAGGCCGAGGACGTGG<br>GCGTGTACTACTGCCTGCAGCACCTGGAATACCCCTACACCTTCGGCCAAGGGACC<br>AAGCTGGAAATCAAGTCCGGAGGTGGTGGATCCGAGGTGCAGCTGGTCGAGTCTG<br>GAGGAGGATTGGTGCAGCCTGGAGGGTCATTGAAACTCTCATGTGCAGCCTCTGG<br>ATTCACCTTCAATAAGTACGCCATGAACTGGGTCCGCCAGGCTCCAGGAAAGGGTT<br>TGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAA<br>ACTGGGTCCAACAAAAACCAGGTCAGGCACCCCGTGGTCTAATAGGTGGGACTAAG<br>TTCCTCGCCCCCGGTACTCCTGCCAGATTCTCAGGCTCCCTGCTTGGAGGCAAGGC<br>TGCCCTCACCCTCTCAGGGGTACAGCCAGAGGATGAGGCAGAATATTACTGTGTTC<br>TATGGTACAGCAACCGCTGGGTGTTCGGTGGAGGAACCAAACTGACTGTCCTA |
| 1270. | hCD19 5-A9-H | artificial | aa | QVQLVQSGAEVVRPGASVKISCKASGYAFTSYWMNWVRQRPGQGLEWIGQIWPGDG<br>DTNYNGKFKGRVTMTADESTSTAYMELSSLRSEDTAVYYCARRETTTVGRYYYAMDY<br>WGQGTTVTVSS |
| 1271. | hCD19 5-A9-HCDR1 | artificial | aa | SYWMN |
| 1272. | hCD19 5-A9-HCDR2 | artificial | aa | QIWPGDGDTNYNGKFKG |
| 1273. | hCD19 5-A9-HCDR3 | artificial | aa | RETTTVGRYYYAMDY |
| 1274. | hCD19 5-A9-H | artificial | nt | CAGGTGCAGCTGGTCCAGTCTGGGGCTGAGGTGGTGAGGCCTGGGGCCTCAGTG |

| | | | | |
|---|---|---|---|---|
| | | | | AAGATTTCCTGCAAGGCTTCTGGCTATGCATTCACTAGCTACTGGATGAACTGGGTG AGGCAGCGCCCTGGACAGGGTCTTGAGTGGATTGGACAGATTTGGCCTGGAGATG GTGATACTAACTACAATGGAAAGTTCAAGGGTAGAGTCACTATGACTGCTGACGAAT CCACCAGCACAGCCTACATGGAACTCAGCAGCCTGCGATCTGAGGACACTGCGGT CTATTACTGTGCAAGACGGGAGACTACGACGGTAGGCCGTTATTACTATGCTATGG ACTACTGGGGCCAAGGGACCACGGTCACCGTCTCCTCA |
| 1275. | hCD19 5-A9-L | artificial | aa | DIQMTQSPSSLSVSVGERVTITCKASQSVDYDGTSYLNWYQQKPGQAPKLLIYDASNLV SGVPSRFSGSGSGTDFTLTISSLQKEDFATYYCQQSTEDPWTFGQGTKVEIK |
| 1276. | hCD19 5-A9-LCDR1 | artificial | aa | KASQSVDYDGTSYLN |
| 1277. | hCD19 5-A9-LCDR2 | artificial | aa | DASNLVS |
| 1278. | hCD19 5-A9-LCDR3 | artificial | aa | QQSTEDPWT |
| 1279. | hCD19 5-A9-L | artificial | nt | GACATCCAGATGACCCAGTCTCCATCTTCTTTGTCTGTGTCTGTAGGGGAGAGAGT CACCATCACCTGCAAGGCCAGCCAAAGTGTTGATTATGATGGTACCAGTTATTTGAA CTGGTACCAACAGAAACCAGGACAGGCACCCAAACTCCTCATCTATGATGCATCCA ATCTAGTTTCTGGGGTCCCATCCAGGTTTAGTGGCAGTGGGTCTGGGACAGACTTC ACCCTCACCATCAGTTCTCTGCAGAAGGAGGATTTCGCAACCTATTACTGTCAGCAA AGTACTGAGGATCCGTGGACGTTCGGTCAAGGGACCAAGGTGGAGATCAAA |
| 1280. | hCD19 5-A9-LH | artificial | aa | DIQMTQSPSSLSVSVGERVTITCKASQSVDYDGTSYLNWYQQKPGQAPKLLIYDASNLV SGVPSRFSGSGSGTDFTLTISSLQKEDFATYYCQQSTEDPWTFGQGTKVEIKGGGGSG GGGSGGGGSQVQLVQSGAEVVRPGASVKISCKASGYAFTSYWMNWVRQRPGQGLE WIGQIWPGDGDTNYNGKFKGRVTMTADESTSTAYMELSSLRSEDTAVYYCARRETTTV GRYYYAMDYWGQGTTVTVSS |
| 1281. | hCD19 5-A9-LH | artificial | nt | GACATCCAGATGACCCAGTCTCCATCTTCTTTGTCTGTGTCTGTAGGGGAGAGAGT CACCATCACCTGCAAGGCCAGCCAAAGTGTTGATTATGATGGTACCAGTTATTTGAA CTGGTACCAACAGAAACCAGGACAGGCACCCAAACTCCTCATCTATGATGCATCCA ATCTAGTTTCTGGGGTCCCATCCAGGTTTAGTGGCAGTGGGTCTGGGACAGACTTC ACCCTCACCATCAGTTCTCTGCAGAAGGAGGATTTCGCAACCTATTACTGTCAGCAA AGTACTGAGGATCCGTGGACGTTCGGTCAAGGGACCAAGGTGGAGATCAAAGGTG GTGGTGGTTCTGGCGGCGGCGGCTCCGGTGGTGGTGGTTCTCAGGTGCAGCTGG TCCAGTCTGGGGCTGAGGTGGTGAGGCCTGGGGCCTCAGTGAAGATTTCCTGCAA GGCTTCTGGCTATGCATTCACTAGCTACTGGATGAACTGGGTGAGGCAGCGCCCTG GACAGGGTCTTGAGTGGATTGGACAGATTTGGCCTGGAGATGGTGATACTAACTAC AATGGAAAGTTCAAGGGTAGAGTCACTATGACTGCTGACGAATCCACCAGCACAGC CTACATGGAACTCAGCAGCCTGCGATCTGAGGACACTGCGGTCTATTACTGTGCAA GACGGGAGACTACGACGGTAGGCCGTTATTACTATGCTATGGACTACTGGGGCCAA GGGACCACGGTCACCGTCTCCTCA |

| | | | | |
|---|---|---|---|---|
| 1282. | hCD19 5-A9 LH x I2C HL | artificial | aa | DIQMTQSPSSLSVSVGERVTITCKASQSVDYDGTSYLNWYQQKPGQAPKLLIYDASNLV<br>SGVPSRFSGSGSGTDFTLTISSLQKEDFATYYCQQSTEDPWTFGQGTKVEIKGGGGSG<br>GGGSGGGGSQVQLVQSGAEVVRPGASVKISCKASGYAFTSYWMNWVRQRPGQGLE<br>WIGQIWPGDGDTNYNGKFKGRVTMTADESTSTAYMELSSLRSEDTAVYYCARRETTTV<br>GRYYYAMDYWGQGTTVTVSSGGGGSEVQLVESGGGLVQPGGSLKLSCAASGFTFNK<br>YAMNWVRQAPGKGLEWVARIRSKYNNYATYYADSVKDRFTISRDDSKNTAYLQMNNL<br>KTEDTAVYYCVRHGNFGNSYISYWAYWGQGTLVTVSSGGGGSGGGGSGGGGSQTVV<br>TQEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 1283. | hCD19 5-A9 LH x I2C HL | artificial | nt | GACATCCAGATGACCCAGTCTCCATCTTCTTTGTCTGTGTCTGTAGGGGAGAGAGT<br>CACCATCACCTGCAAGGCCAGCCAAAGTGTTGATTATGATGGTACCAGTTATTTGAA<br>CTGGTACCAACAGAAACCAGGACAGGCACCCAAACTCCTCATCTATGATGCATCCA<br>ATCTAGTTTCTGGGGTCCCATCCAGGTTTAGTGGCAGTGGGTCTGGGACAGACTTC<br>ACCCTCACCATCAGTTCTCTGCAGAAGGAGGATTTCGCAACCTATTACTGTCAGCAA<br>AGTACTGAGGATCCGTGGACGTTCGGTCAAGGGACCAAGGTGGAGATCAAAGGTG<br>GTGGTGGTTCTGGCGGCGGCGGCTCCGGTGGTGGTGGTTCTCAGGTGCAGCTGG<br>TCCAGTCTGGGGCTGAGGTGGTGAGGCCTGGGGCCTCAGTGAAGATTTCCTGCAA<br>GGCTTCTGGCTATGCATTCACTAGCTACTGGATGAACTGGGTGAGGCAGCGCCCTG<br>GACAGGGTCTTGAGTGGATTGGACAGATTTGGCCTGGAGATGGTGATACTAACTAC<br>AATGGAAAGTTCAAGGGTAGAGTCACTATGACTGCTGACGAATCCACCAGCACAGC<br>CTACATGGAACTCAGCAGCCTGCGATCTGAGGACACTGCGGTCTATTACTGTGCAA<br>GACGGGAGACTACGACGGTAGGCCGTTATTACTATGCTATGGACTACTGGGGCCAA<br>GGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAG<br>CCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATT<br>ATGCCGATTCAGTGAAAGACAGGTTCACCATCTCCAGAGATGATTCAAAAAACACTG<br>CCTATCTACAAATGAACAACTTGAAAACTGAGGACACTGCCGTGTACTACTGTGTGA<br>GACATGGGAACTTCGGTAATAGCTACATATCCTACTGGGCTTACTGGGGCCAAGGG<br>ACTCTGGTCACCGTCTCCTCAGGTGGTGGTGGTTCTGGCGGCGGCGGCTCCGGTG<br>GTGGTGGTTCTCAGACTGTTGTGACTCAGGAACCTTCACTCACCGTATCACCTGGT<br>GGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGG<br>ACTAAGTTCCTCGCCCCCGGTACTCCTGCCAGATTCTCAGGCTCCCTGCTTGGAGG<br>CAAGGCTGCCCTCACCCTCTCAGGGGTACAGCCAGAGGATGAGGCAGAATATTACT<br>GTGTTCTATGGTACAGCAACCGCTGGGTGTTCGGTGGAGGAACCAAACTGACTGTC |

| | | | | |
|---|---|---|---|---|
| | | | | CTA |
| 1284. | hCD19 2-C6-H | artificial | aa | QVQLVQSGAEVKKPGASVKISCKASGYAFTSYWMNWVRQAPGQGLEWIGQIWPGDGDTNYNGKFKGRVTLTADTSTSTAYMELSSLQSEDTAVYYCARRETTTVGRYYYAMDYWGQGTTVTVSS |
| 1285. | hCD19 2-C6-HCDR1 | artificial | aa | SYWMN |
| 1286. | hCD19 2-C6-HCDR2 | artificial | aa | QIWPGDGDTNYNGKFKG |
| 1287. | hCD19 2-C6-HCDR3 | artificial | aa | RETTTVGRYYYAMDY |
| 1288. | hCD19 2-C6-H | artificial | nt | CAGGTGCAGCTGGTCCAGTCTGGGGCTGAGGTGAAGAAGCCTGGGGCCTCAGTGAAGATTTCCTGCAAGGCTTCTGGCTATGCATTCACTAGCTACTGGATGAACTGGGTGAGGCAGGCCCCTGGACAGGGTCTTGAGTGGATTGGACAGATTTGGCCTGGAGATGGTGATACTAACTACAATGGAAAGTTCAAGGGTAGAGTCACTCTGACTGCTGACACATCCACCAGCACAGCCTACATGGAACTCAGCAGCCTACAATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1289. | hCD19 2-C6-L | artificial | aa | DIQMTQSPSSLSASVGERVTITCKASQSVDYDGSSYLNWYQQKPGKAPKLLIYDASNLVSGVPPRFSGSGSGTDFTLTIHSLQPEDFATYYCQQSTEDPWTFGQGTKVEIK |
| 1290. | hCD19 2-C6-LCDR1 | artificial | aa | KASQSVDYDGSSYLN |
| 1291. | hCD19 2-C6-LCDR2 | artificial | aa | DASNLVS |
| 1292. | hCD19 2-C6-LCDR3 | artificial | aa | QQSTEDPWT |
| 1293. | hCD19 2-C6-L | artificial | nt | GACATCCAGATGACCCAGTCTCCATCTTCTTTGTCTGCGTCTGTAGGGGAGAGAGTCACCATCACCTGCAAGGCCAGCCAAAGTGTTGATTATGATGGTTCGAGTTATTTGAACTGGTACCAACAGAAACCAGGAAAGGCACCCAAACTCCTCATCTATGATGCATCCAATCTAGTTTCTGGGGTCCCACCCAGGTTTAGTGGCAGTGGGTCTGGGACAGACTTCACCCTCACCATCCATTCTCTGCAGCCGGAGGATTTCGCAACCTATTACTGTCAGCAAAGTACTGAGGATCCGTGGACGTTCGGTCAAGGGACCAAGGTGGAGATCAAA |
| 1294. | hCD19 2-C6-LH | artificial | aa | DIQMTQSPSSLSASVGERVTITCKASQSVDYDGSSYLNWYQQKPGKAPKLLIYDASNLVSGVPPRFSGSGSGTDFTLTIHSLQPEDFATYYCQQSTEDPWTFGQGTKVEIKGGGGSGGGGSGGGGSQVQLVQSGAEVKKPGASVKISCKASGYAFTSYWMNWVRQAPGQGLEWIGQIWPGDGDTNYNGKFKGRVTLTADTSTSTAYMELSSLQSEDTAVYYCARRETTTVGRYYYAMDYWGQGTTVTVSS |
| 1295. | hCD19 2-C6-LH | artificial | nt | GACATCCAGATGACCCAGTCTCCATCTTCTTTGTCTGCGTCTGTAGGGGAGAGAGTCACCATCACCTGCAAGGCCAGCCAAAGTGTTGATTATGATGGTTCGAGTTATTTGAACTGGTACCAACAGAAACCAGGAAAGGCACCCAAACTCCTCATCTATGATGCATCCA |

| | | | | |
|---|---|---|---|---|
| | | | | ATCTAGTTTCTGGGGTCCCACCCAGGTTTAGTGGCAGTGGGTCTGGGACAGACTTCACCCTCACCATCCATTCTCTGCAGCCGGAGGATTTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGAAGAAGCCTGGGGCCTCAGTGAAGATTTCCTGCAAGGCTTCTGGCTATGCATTCACTAGCTACTGGATGAACTGGGTGAGGCAGGCCCCTGGACAGGGTCTTGAGTGGATTGGACAGATTTGGCCTGGAGATGGTGATACTAACTACAATGGAAAGTTCAAGGGTAGAGTCACTCTGACTGCTGACACATCCACCAGCACAGCCTACATGGAACTCAGCAGCCTACAATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1296. | hCD19 2-C6 LH x I2C HL | artificial | aa | DIQMTQSPSSLSASVGERVTITCKASQSVDYDGSSYLNWYQQKPGKAPKLLIYDASNLVSGVPPRFSGSGSGTDFTLTIHSLQPEDFATYYCQQSTEDPWTFGQGTKVEIKGGGGSGGGGSGGGGSQVQLVQSGAEVKKPGASVKISCKASGYAFTSYWMNWVRQAPGQGLEWIGQIWPGDGDTNYNGKFKGRVTLTADTSTSTAYMELSSLQSEDTAVYYCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1297. | hCD19 2-C6 LH x I2C HL | artificial | nt | GACATCCAGATGACCCAGTCTCCATCTTCTTTGTCTGCGTCTGTAGGGGAGAGAGTCACCATCACCTGCAAGGCCAGCCAAAGTGTTGATTATGATGGTTCGAGTTATTTGAACTGGTACCAACAGAAACCAGGAAAGGCACCCAAACTCCTCATCTATGATGCATCCAATCTAGTTTCTGGGGTCCCACCCAGGTTTAGTGGCAGTGGGTCTGGGACAGACTTCACCCTCACCATCCATTCTCTGCAGCCGGAGGATTTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGAAGAAGCCTGGGGCCTCAGTGAAGATTTCCTGCAAGGCTTCTGGCTATGCATTCACTAGCTACTGGATGAACTGGGTGAGGCAGGCCCCTGGACAGGGTCTTGAGTGGATTGGACAGATTTGGCCTGGAGATGGTGATACTAACTACAATGGAAAGTTCAAGGGTAGAGTCACTCTGACTGCTGACACATCCACCAGCACAGCCTACATGGAACTCAGCAGCCTACAATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGA |

| | | | | |
|---|---|---|---|---|
| | | | | AAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1298. | hCD19 4-C7-H | artificial | aa | QVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1299. | hCD19 4-C7-HCDR1 | artificial | aa | SYWMN |
| 1300. | hCD19 4-C7-HCDR2 | artificial | aa | QIWPGDGDTNYNGKFKG |
| 1301. | hCD19 4-C7-HCDR3 | artificial | aa | RETTTVGRYYYAMDY |
| 1302. | hCD19 4-C7-H | artificial | nt | CAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1303. | hCD19 4-C7-L | artificial | aa | DIQMTQSPSSLSASLGDRVTITCKASQSVDYDGTSYLNWYQQKPGQPPKLLIYDASNLVSGVPSRFSGSGSGTDFTLTISPLEQEDFATYYCQQSTEDPWTFGQGTKVEIK |
| 1304. | hCD19 4-C7-LCDR1 | artificial | aa | KASQSVDYDGSSYLN |
| 1305. | hCD19 4-C7-LCDR2 | artificial | aa | DASNLVS |
| 1306. | hCD19 4-C7-LCDR3 | artificial | aa | QQSTEDPWT |
| 1307. | hCD19 4-C7-L | artificial | nt | GACATCCAGATGACCCAGTCTCCATCTTCTTTGTCTGCGTCTCTAGGGGACAGAGTCACCATCACCTGCAAGGCCAGCCAAAGTGTTGATTATGATGGTACGAGTTATTTGAACTGGTACCAACAGAAACCAGGACAGCCACCCAAACTCCTCATCTATGATGCATCCA |

| | | | | |
|---|---|---|---|---|
| | | | | ATCTAGTTTCTGGGGTCCCATCCAGGTTTAGTGGCAGTGGGTCTGGGACAGACTTCACCCTCACCATCAGTCCTCTGGAGCAGGAGGATTTCGCAACCTATTACTGTCAGCAAAGTACTGAGGATCCGTGGACGTTCGGTCAAGGGACCAAGGTGGAGATCAAA |
| 1308. | hCD19 4-C7-LH | artificial | aa | DIQMTQSPSSLSASLGDRVTITCKASQSVDYDGTSYLNWYQQKPGQPPKLLIYDASNLVSGVPSRFSGSGSGTDFTLTISPLEQEDFATYYCQQSTEDPWTFGQGTKVEIKGGGGSGGGGSGGGGSQVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1309. | hCD19 4-C7-LH | artificial | nt | GACATCCAGATGACCCAGTCTCCATCTTCTTTGTCTGCGTCTCTAGGGGACAGAGTCACCATCACCTGCAAGGCCAGCCAAAGTGTTGATTATGATGGTACGAGTTATTTGAACTGGTACCAACAGAAACCAGGACAGCCACCCAAACTCCTCATCTATGATGCATCCAATCTAGTTTCTGGGGTCCCATCCAGGTTTAGTGGCAGTGGGTCTGGGACAGACTTCACCCTCACCATCAGTCCTCTGGAGCAGGAGGATTTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1310. | hCD19 4-C7 LH x I2C HL | artificial | aa | DIQMTQSPSSLSASLGDRVTITCKASQSVDYDGTSYLNWYQQKPGQPPKLLIYDASNLVSGVPSRFSGSGSGTDFTLTISPLEQEDFATYYCQQSTEDPWTFGQGTKVEIKGGGGSGGGGSGGGGSQVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1311. | hCD19 4-C7 LH x I2C HL | artificial | nt | GACATCCAGATGACCCAGTCTCCATCTTCTTTGTCTGCGTCTCTAGGGGACAGAGTCACCATCACCTGCAAGGCCAGCCAAAGTGTTGATTATGATGGTACGAGTTATTTGAACTGGTACCAACAGAAACCAGGACAGCCACCCAAACTCCTCATCTATGATGCATCCAATCTAGTTTCTGGGGTCCCATCCAGGTTTAGTGGCAGTGGGTCTGGGACAGACTTCACCCTCACCATCAGTCCTCTGGAGCAGGAGGATTTCGCAACCTATTACTGTCAGCA |

| | | | | |
|---|---|---|---|---|
| | | | | AAGTACTGAGGATCCGTGGACGTTCGGTCAAGGGACCAAGGTGGAGATCAAAGGT<br>GGTGGTGGTTCTGGCGGCGGCGGCTCCGGTGGTGGTGGTTCTCAGGTGCAGCTG<br>GTCCAGTCTGGGGCTGAGGTGGTGAAGCCTGGGGCCTCAGTGAAGATTTCCTGCA<br>AGGCTTCTGGCTATGCATTCACTAGCTACTGGATGAACTGGGTGAGGCAGCGCCCT<br>GGACAGGGTCTTGAGTGGATTGGACAGATTTGGCCTGGAGATGGTGATACTAACTA<br>CAATGGAAAGTTCAAGGGTAGAGTCACTATGACTGCTGACGAATCCACCAGCACAG<br>CCTACATGGAACTCAGCAGCCTGCGATCTGAGGACACTGCGGTCTATTACTGTGCA<br>AGACGGGAGACTACGACGGTAGGCCGTTATTACTATGCTATGGACTACTGGGGCCA<br>AGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAG<br>CCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATT<br>ATGCCGATTCAGTGAAAGACAGGTTCACCATCTCCAGAGATGATTCAAAAAACACTG<br>CCTATCTACAAATGAACAACTTGAAAACTGAGGACACTGCCGTGTACTACTGTGTGA<br>GACATGGGAACTTCGGTAATAGCTACATATCCTACTGGGCTTACTGGGGCCAAGGG<br>ACTCTGGTCACCGTCTCCTCAGGTGGTGGTGGTTCTGGCGGCGGCGGCTCCGGTG<br>GTGGTGGTTCTCAGACTGTTGTGACTCAGGAACCTTCACTCACCGTATCACCTGGT<br>GGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGG<br>ACTAAGTTCCTCGCCCCCGGTACTCCTGCCAGATTCTCAGGCTCCCTGCTTGGAGG<br>CAAGGCTGCCCTCACCCTCTCAGGGGTACAGCCAGAGGATGAGGCAGAATATTACT<br>GTGTTCTATGGTACAGCAACCGCTGGGTGTTCGGTGGAGGAACCAAACTGACTGTC<br>CTA |
| 1312. | hCD19 2-D7-H | artificial | aa | QVQLVQSGAEVVKPGASVKISCKASGYAFTSYWMNWVRQRPGQGLEWIGQIWPGDG<br>DTNYNGKFKGRVTMTADESTSTAYMELSSLRSEDTAVYYCARRETTTVGRYYYAMDY<br>WGQGTTVTVSS |
| 1313. | hCD19 2-D7-HCDR1 | artificial | aa | SYWMN |
| 1314. | hCD19 2-D7-HCDR2 | artificial | aa | QIWPGDGDTNYNGKFKG |
| 1315. | hCD19 2-D7-HCDR3 | artificial | aa | RETTTVGRYYYAMDY |
| 1316. | hCD19 2-D7-H | artificial | nt | CAGGTGCAGCTGGTCCAGTCTGGGGCTGAGGTGGTGAAGCCTGGGGCCTCAGTGA<br>AGATTTCCTGCAAGGCTTCTGGCTATGCATTCACTAGCTACTGGATGAACTGGGTGA<br>GGCAGCGCCCTGGACAGGGTCTTGAGTGGATTGGACAGATTTGGCCTGGAGATGG<br>TGATACTAACTACAATGGAAAGTTCAAGGGTAGAGTCACTATGACTGCTGACGAATC |

| | | | | |
|---|---|---|---|---|
| | | | | CACCAGCACAGCCTACATGGAACTCAGCAGCCTGCGATCTGAGGACACTGCGGTC TATTACTGTGCAAGACGGGAGACTACGACGGTAGGCCGTTATTACTATGCTATGGA CTACTGGGGCCAAGGGACCACGGTCACCGTCTCCTCA |
| 1317. | hCD19 2-D7-L | artificial | aa | DIQMTQSPSSLSASVGERVTITCKASQSVDYDGSSYLNWYQQKPGKAPKLLIYDASNLV SGVPPRFSGSGSGTDFTLTIHSLQPEDFATYYCQQSTEDPWTFGQGTKVEIK |
| 1318. | hCD19 2-D7-LCDR1 | artificial | aa | KASQSVDYDGSSYLN |
| 1319. | hCD19 2-D7-LCDR2 | artificial | aa | DASNLVS |
| 1320. | hCD19 2-D7-LCDR3 | artificial | aa | QQSTEDPWT |
| 1321. | hCD19 2-D7-L | artificial | nt | GACATCCAGATGACCCAGTCTCCATCTTCTTTGTCTGCGTCTGTAGGGGAGAGAGT CACCATCACCTGCAAGGCCAGCCAAAGTGTTGATTATGATGGTTCGAGTTATTTGAA CTGGTACCAACAGAAACCAGGAAAGGCACCCAAACTCCTCATCTATGATGCATCCA ATCTAGTTTCTGGGGTCCCACCCAGGTTTAGTGGCAGTGGGTCTGGGACAGACTTC ACCCTCACCATCCATTCTCTGCAGCCGGAGGATTTCGCAACCTATTACTGTCAGCAA AGTACTGAGGATCCGTGGACGTTCGGTCAAGGGACCAAGGTGGAGATCAAA |
| 1322. | hCD19 2-D7-LH | artificial | aa | DIQMTQSPSSLSASVGERVTITCKASQSVDYDGSSYLNWYQQKPGKAPKLLIYDASNLV SGVPPRFSGSGSGTDFTLTIHSLQPEDFATYYCQQSTEDPWTFGQGTKVEIKGGGGSG GGGSGGGGSQVQLVQSGAEVVKPGASVKISCKASGYAFTSYWMNWVRQRPGQGLE WIGQIWPGDGDTNYNGKFKGRVTMTADESTSTAYMELSSLRSEDTAVYYCARRETTTV GRYYYAMDYWGQGTTVTVSS |
| 1323. | hCD19 2-D7-LH | artificial | nt | GACATCCAGATGACCCAGTCTCCATCTTCTTTGTCTGCGTCTGTAGGGGAGAGAGT CACCATCACCTGCAAGGCCAGCCAAAGTGTTGATTATGATGGTTCGAGTTATTTGAA CTGGTACCAACAGAAACCAGGAAAGGCACCCAAACTCCTCATCTATGATGCATCCA ATCTAGTTTCTGGGGTCCCACCCAGGTTTAGTGGCAGTGGGTCTGGGACAGACTTC ACCCTCACCATCCATTCTCTGCAGCCGGAGGATTTCGCAACCTATTACTGTCAGCAA AGTACTGAGGATCCGTGGACGTTCGGTCAAGGGACCAAGGTGGAGATCAAAGGTG GTGGTGGTTCTGGCGGCGGCGGCTCCGGTGGTGGTGGTTCTCAGGTGCAGCTGG TCCAGTCTGGGGCTGAGGTGGTGAAGCCTGGGGCCTCAGTGAAGATTTCCTGCAA GGCTTCTGGCTATGCATTCACTAGCTACTGGATGAACTGGGTGAGGCAGCGCCCTG GACAGGGTCTTGAGTGGATTGGACAGATTTGGCCTGGAGATGGTGATACTAACTAC AATGGAAAGTTCAAGGGTAGAGTCACTATGACTGCTGACGAATCCACCAGCACAGC CTACATGGAACTCAGCAGCCTGCGATCTGAGGACACTGCGGTCTATTACTGTGCAA GACGGGAGACTACGACGGTAGGCCGTTATTACTATGCTATGGACTACTGGGGCCAA GGGACCACGGTCACCGTCTCCTCA |
| 1324. | hCD19 2-D7 LH x I2C HL | artificial | aa | DIQMTQSPSSLSASVGERVTITCKASQSVDYDGSSYLNWYQQKPGKAPKLLIYDASNLV SGVPPRFSGSGSGTDFTLTIHSLQPEDFATYYCQQSTEDPWTFGQGTKVEIKGGGGSG GGGSGGGGSQVQLVQSGAEVVKPGASVKISCKASGYAFTSYWMNWVRQRPGQGLE |

| | | | | |
|---|---|---|---|---|
| | | | | WIGQIWPGDGDTNYNGKFKGRVTMTADESTSTAYMELSSLRSEDTAVYYCARRETTTV<br>GRYYYAMDYWGQGTTVTVSSGGGGSEVQLVESGGGLVQPGGSLKLSCAASGFTFNK<br>YAMNWVRQAPGKGLEWVARIRSKYNNYATYYADSVKDRFTISRDDSKNTAYLQMNNL<br>KTEDTAVYYCVRHGNFGNSYISYWAYWGQGTLVTVSSGGGGSGGGGSGGGGSQTVV<br>TQEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 1325. | hCD19 2-D7 LH x I2C HL | artificial | nt | GACATCCAGATGACCCAGTCTCCATCTTCTTTGTCTGCGTCTGTAGGGGAGAGAGT<br>CACCATCACCTGCAAGGCCAGCCAAAGTGTTGATTATGATGGTTCGAGTTATTTGAA<br>CTGGTACCAACAGAAACCAGGAAAGGCACCCAAACTCCTCATCTATGATGCATCCA<br>ATCTAGTTTCTGGGGTCCCACCCAGGTTTAGTGGCAGTGGGTCTGGGACAGACTTC<br>ACCCTCACCATCCATTCTCTGCAGCCGGAGGATTTCGCAACCTATTACTGTCAGCAA<br>AGTACTGAGGATCCGTGGACGTTCGGTCAAGGGACCAAGGTGGAGATCAAAGGTG<br>GTGGTGGTTCTGGCGGCGGCGGCTCCGGTGGTGGTGGTTCTCAGGTGCAGCTGG<br>TCCAGTCTGGGGCTGAGGTGGTGAAGCCTGGGGCCTCAGTGAAGATTTCCTGCAA<br>GGCTTCTGGCTATGCATTCACTAGCTACTGGATGAACTGGGTGAGGCAGCGCCCTG<br>GACAGGGTCTTGAGTGGATTGGACAGATTTGGCCTGGAGATGGTGATACTAACTAC<br>AATGGAAAGTTCAAGGGTAGAGTCACTATGACTGCTGACGAATCCACCAGCACAGC<br>CTACATGGAACTCAGCAGCCTGCGATCTGAGGACACTGCGGTCTATTACTGTGCAA<br>GACGGGAGACTACGACGGTAGGCCGTTATTACTATGCTATGGACTACTGGGGCCAA<br>GGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAG<br>CCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATT<br>ATGCCGATTCAGTGAAAGACAGGTTCACCATCTCCAGAGATGATTCAAAAAACACTG<br>CCTATCTACAAATGAACAACTTGAAAACTGAGGACACTGCCGTGTACTACTGTGTGA<br>GACATGGGAACTTCGGTAATAGCTACATATCCTACTGGGCTTACTGGGGCCAAGGG<br>ACTCTGGTCACCGTCTCCTCAGGTGGTGGTGGTTCTGGCGGCGGCGGCTCCGGTG<br>GTGGTGGTTCTCAGACTGTTGTGACTCAGGAACCTTCACTCACCGTATCACCTGGT<br>GGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGG<br>ACTAAGTTCCTCGCCCCCGGTACTCCTGCCAGATTCTCAGGCTCCCTGCTTGGAGG<br>CAAGGCTGCCCTCACCCTCTCAGGGGTACAGCCAGAGGATGAGGCAGAATATTACT<br>GTGTTCTATGGTACAGCAACCGCTGGGTGTTCGGTGGAGGAACCAAACTGACTGTC<br>CTA |
| 1326. | hCD19 2-D4-H | artificial | aa | QVQLVQSGAEVVKPGASVKISCKASGYAFTSYWMNWVRQRPGQGLEWIGQIWPGDG<br>DTNYNGKFKGRVTMTADESTSTAYMELSSLRSEDTAVYYCARRETTTVGRYYYAMDY |

| | | | | |
|---|---|---|---|---|
| | | | | WGQGTTVTVSS |
| 1327. | hCD19 2-D4-HCDR1 | artificial | aa | SYWMN |
| 1328. | hCD19 2-D4-HCDR2 | artificial | aa | QIWPGDGDTNYNGKFKG |
| 1329. | hCD19 2-D4-HCDR3 | artificial | aa | RETTTVGRYYYAMDY |
| 1330. | hCD19 2-D4-H | artificial | nt | CAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1331. | hCD19 2-D4-L | artificial | aa | DIQMTQSPASLSVSVGERVTITCKASQSVDYDGTSYLNWYQQKPGQAPKLLIYDASNLVSGVPSRFSGSGSGTDFTLTISSLQPEDFATYYCQQSTEDPWTFGQGTKVEIK |
| 1332. | hCD19 2-D4-LCDR1 | artificial | aa | KASQSVDYDGSSYLN |
| 1333. | hCD19 2-D4-LCDR2 | artificial | aa | DASNLVS |
| 1334. | hCD19 2-D4-LCDR3 | artificial | aa | QQSTEDPWT |
| 1335. | hCD19 2-D4-L | artificial | nt | GACATCCAGATGACCCAGTCTCCAGCTTCTTTGTCTGTGTCTGTAGGGGAGAGAGTCACCATCACCTGCAAGGCCAGCCAAAGTGTTGATTATGATGGTACCAGTTATTTGAACTGGTACCAACAGAAACCAGGACAGGCACCCAAACTCCTCATCTATGATGCATCCAATCTAGTTTCTGGGGTCCCATCCAGGTTTAGTGGCAGTGGGTCTGGGACAGACTTCACCCTCACCATCAGTTCTCTGCAGCCGGAGGATTTCGCAACCTATTACTGTCAGCAAAGTACTGAGGATCCGTGGACGTTCGGTCAAGGGACCAAGGTGGAGATCAAA |
| 1336. | hCD19 2-D4-LH | artificial | aa | DIQMTQSPASLSVSVGERVTITCKASQSVDYDGTSYLNWYQQKPGQAPKLLIYDASNLVSGVPSRFSGSGSGTDFTLTISSLQPEDFATYYCQQSTEDPWTFGQGTKVEIKGGGGSGGGGSGGGGSQVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1337. | hCD19 2-D4-LH | artificial | nt | GACATCCAGATGACCCAGTCTCCAGCTTCTTTGTCTGTGTCTGTAGGGGAGAGAGTCACCATCACCTGCAAGGCCAGCCAAAGTGTTGATTATGATGGTACCAGTTATTTGAACTGGTACCAACAGAAACCAGGACAGGCACCCAAACTCCTCATCTATGATGCATCCAATCTAGTTTCTGGGGTCCCATCCAGGTTTAGTGGCAGTGGGTCTGGGACAGACTTCACCCTCACCATCAGTTCTCTGCAGCCGGAGGATTTCGCAACCTATTACTGTCAGCAAAGTACTGAGGATCCGTGGACGTTCGGTCAAGGGACCAAGGTGGAGATCAAAGGTG |

| | | | | |
|---|---|---|---|---|
| | | | | GTGGTGGTTCTGGCGGCGGCGGCTCCGGTGGTGGTGGTTCTCAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1338. | hCD19 2-D4 LH x I2C HL | artificial | aa | DIQMTQSPASLSVSVGERVTITCKASQSVDYDGTSYLNWYQQKPGQAPKLLIYDASNLVSGVPSRFSGSGSGTDFTLTISSLQPEDFATYYCQQSTEDPWTFGQGTKVEIKGGGGSGGGGSGGGGSQVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1339. | hCD19 2-D4 LH x I2C HL | artificial | nt | GACATCCAGATGACCCAGTCTCCAGCTTCTTTGTCTGTGTCTGTAGGGGAGAGAGTCACCATCACCTGCAAGGCCAGCCAAAGTGTTGATTATGATGGTACCAGTTATTTGAACTGGTACCAACAGAAACCAGGACAGGCACCCAAACTCCTCATCTATGATGCATCCAATCTAGTTTCTGGGGTCCCATCCAGGTTTAGTGGCAGTGGGTCTGGGACAGACTTCACCCTCACCATCAGTTCTCTGCAGCCGGAGGATTTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGA |

| | | | | |
|---|---|---|---|---|
| | | | | GACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1340. | hCD19 5-G3-H | artificial | aa | QVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1341. | hCD19 5-G3-HCDR1 | artificial | aa | SYWMN |
| 1342. | hCD19 5-G3-HCDR2 | artificial | aa | QIWPGDGDTNYNGKFKG |
| 1343. | hCD19 5-G3-HCDR3 | artificial | aa | RETTTVGRYYYAMDY |
| 1344. | hCD19 5-G3-H | artificial | nt | CAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1345. | hCD19 5-G3-L | artificial | aa | DIQMTQSPSSLSASVGDRVTITCKASQSVDYDGLSYLNWYQQKPGQAPKLLIYDASNLVSGIPSRFSGSGSGTDFTLTISSLEPEDFATYYCQQSTEDPWTFGQGTKVEIK |
| 1346. | hCD19 5-G3-LCDR1 | artificial | aa | KASQSVDYDGSSYLN |
| 1347. | hCD19 5-G3-LCDR2 | artificial | aa | DASNLVS |
| 1348. | hCD19 5-G3-LCDR3 | artificial | aa | QQSTEDPWT |
| 1349. | hCD19 5-G3-L | artificial | nt | GACATCCAGATGACCCAGTCTCCATCTTCTTTGTCTGCGTCTGTAGGGGACAGAGTCACCATCACCTGCAAGGCCAGCCAAAGTGTTGATTATGATGGTCTCAGTTATTTGAACTGGTACCAACAGAAACCAGGACAGGCACCCAAACTCCTCATCTATGATGCATCCAATCTAGTTTCTGGGATCCCATCCAGGTTTAGTGGCAGTGGGTCTGGGACAGACTTCACCCTCACCATCAGTTCTCTGGAGCCGGAGGATTTCGCAACCTATTACTGTCAGCAAAGTACTGAGGATCCGTGGACGTTCGGTCAAGGGACCAAGGTGGAGATCAAA |

| | | | | |
|---|---|---|---|---|
| 1350. | hCD19 5-G3-LH | artificial | aa | DIQMTQSPSSLSASVGDRVTITCKASQSVDYDGLSYLNWYQQKPGQAPKLLIYDASNLV SGIPSRFSGSGSGTDFTLTISSLEPEDFATYYCQQSTEDPWTFGQGTKVEIKGGGGSG GGGSGGGGS QVQLVQSGAEVVKPGASVKISCKASGYAFTSYWMNWVRQRPGQGLEWIGQIWPGDG DTNYNGKFKGRVTMTADESTSTAYMELSSLRSEDTAVYYCARRETTTVGRYYYAMDY WGQGTTVTVSS |
| 1351. | hCD19 5-G3-LH | artificial | nt | GACATCCAGATGACCCAGTCTCCATCTTCTTTGTCTGCGTCTGTAGGGGACAGAGT CACCATCACCTGCAAGGCCAGCCAAAGTGTTGATTATGATGGTCTCAGTTATTTGAA CTGGTACCAACAGAAACCAGGACAGGCACCCAAACTCCTCATCTATGATGCATCCA ATCTAGTTTCTGGGATCCCATCCAGGTTTAGTGGCAGTGGGTCTGGGACAGACTTC ACCCTCACCATCAGTTCTCTGGAGCCGGAGGATTTCGCAACCTATTACTGTCAGCA AAGTACTGAGGATCCGTGGACGTTCGGTCAAGGGACCAAGGTGGAGATCAAAGGT GGTGGTGGTTCTGGCGGCGGCGGCTCCGGTGGTGGTGGTTCTCAGGTGCAGCTG GTCCAGTCTGGGGCTGAGGTGGTGAAGCCTGGGGCCTCAGTGAAGATTTCCTGCA AGGCTTCTGGCTATGCATTCACTAGCTACTGGATGAACTGGGTGAGGCAGCGCCCT GGACAGGGTCTTGAGTGGATTGGACAGATTTGGCCTGGAGATGGTGATACTAACTA CAATGGAAAGTTCAAGGGTAGAGTCACTATGACTGCTGACGAATCCACCAGCACAG CCTACATGGAACTCAGCAGCCTGCGATCTGAGGACACTGCGGTCTATTACTGTGCA AGACGGGAGACTACGACGGTAGGCCGTTATTACTATGCTATGGACTACTGGGGCCA AGGGACCACGGTCACCGTCTCCTCA |
| 1352. | hCD19 5-G3 LH x I2C HL | artificial | aa | DIQMTQSPSSLSASVGDRVTITCKASQSVDYDGLSYLNWYQQKPGQAPKLLIYDASNLV SGIPSRFSGSGSGTDFTLTISSLEPEDFATYYCQQSTEDPWTFGQGTKVEIKGGGGSG GGGSGGGGSQVQLVQSGAEVVKPGASVKISCKASGYAFTSYWMNWVRQRPGQGLE WIGQIWPGDGDTNYNGKFKGRVTMTADESTSTAYMELSSLRSEDTAVYYCARRETTTV GRYYYAMDYWGQGTTVTVSSGGGGSEVQLVESGGGLVQPGGSLKLSCAASGFTFNK YAMNWVRQAPGKGLEWVARIRSKYNNYATYYADSVKDRFTISRDDSKNTAYLQMNNL KTEDTAVYYCVRHGNFGNSYISYWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPA RFSGSLLGGKAALTLSGVQPEDEAEYYCVLWYSNRWVFGGGTKLTVL |
| 1353. | hCD19 5-G3 LH x I2C HL | artificial | nt | GACATCCAGATGACCCAGTCTCCATCTTCTTTGTCTGCGTCTGTAGGGGACAGAGT CACCATCACCTGCAAGGCCAGCCAAAGTGTTGATTATGATGGTCTCAGTTATTTGAA CTGGTACCAACAGAAACCAGGACAGGCACCCAAACTCCTCATCTATGATGCATCCA ATCTAGTTTCTGGGATCCCATCCAGGTTTAGTGGCAGTGGGTCTGGGACAGACTTC ACCCTCACCATCAGTTCTCTGGAGCCGGAGGATTTCGCAACCTATTACTGTCAGCA AAGTACTGAGGATCCGTGGACGTTCGGTCAAGGGACCAAGGTGGAGATCAAAGGT |

| | | | | |
|---|---|---|---|---|
| | | | | GGTGGTGGTTCTGGCGGCGGCGGCTCCGGTGGTGGTGGTTCTCAGGTGCAGCTG<br>GTCCAGTCTGGGGCTGAGGTGGTGAAGCCTGGGGCCTCAGTGAAGATTTCCTGCA<br>AGGCTTCTGGCTATGCATTCACTAGCTACTGGATGAACTGGGTGAGGCAGCGCCCT<br>GGACAGGGTCTTGAGTGGATTGGACAGATTTGGCCTGGAGATGGTGATACTAACTA<br>CAATGGAAAGTTCAAGGGTAGAGTCACTATGACTGCTGACGAATCCACCAGCACAG<br>CCTACATGGAACTCAGCAGCCTGCGATCTGAGGACACTGCGGTCTATTACTGTGCA<br>AGACGGGAGACTACGACGGTAGGCCGTTATTACTATGCTATGGACTACTGGGGCCA<br>AGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAG<br>CCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATT<br>ATGCCGATTCAGTGAAAGACAGGTTCACCATCTCCAGAGATGATTCAAAAAACACTG<br>CCTATCTACAAATGAACAACTTGAAAACTGAGGACACTGCCGTGTACTACTGTGTGA<br>GACATGGGAACTTCGGTAATAGCTACATATCCTACTGGGCTTACTGGGGCCAAGGG<br>ACTCTGGTCACCGTCTCCTCAGGTGGTGGTGGTTCTGGCGGCGGCGGCTCCGGTG<br>GTGGTGGTTCTCAGACTGTTGTGACTCAGGAACCTTCACTCACCGTATCACCTGGT<br>GGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGG<br>ACTAAGTTCCTCGCCCCCGGTACTCCTGCCAGATTCTCAGGCTCCCTGCTTGGAGG<br>CAAGGCTGCCCTCACCCTCTCAGGGGTACAGCCAGAGGATGAGGCAGAATATTACT<br>GTGTTCTATGGTACAGCAACCGCTGGGTGTTCGGTGGAGGAACCAAACTGACTGTC<br>CTA |
| 1354. | hCD19 4-E10-H | artificial | aa | QVQLVQSGAEVVKPGASVKISCKASGYAFTSYWMNWVRQRPGQGLEWIGQIWPGDG<br>DTNYNGKFKGRVTMTADESTSTAYMELSSLRSEDTAVYYCARRETTTVGRYYYAMDY<br>WGQGTTVTVSS |
| 1355. | hCD19 4-E10-HCDR1 | artificial | aa | SYWMN |
| 1356. | hCD19 4-E10-HCDR2 | artificial | aa | QIWPGDGDTNYNGKFKG |
| 1357. | hCD19 4-E10-HCDR3 | artificial | aa | RETTTVGRYYYAMDY |
| 1358. | hCD19 4-E10-H | artificial | nt | CAGGTGCAGCTGGTCCAGTCTGGGGCTGAGGTGGTGAAGCCTGGGGCCTCAGTGA<br>AGATTTCCTGCAAGGCTTCTGGCTATGCATTCACTAGCTACTGGATGAACTGGGTGA<br>GGCAGCGCCCTGGACAGGGTCTTGAGTGGATTGGACAGATTTGGCCTGGAGATGG<br>TGATACTAACTACAATGGAAAGTTCAAGGGTAGAGTCACTATGACTGCTGACGAATC<br>CACCAGCACAGCCTACATGGAACTCAGCAGCCTGCGATCTGAGGACACTGCGGTC |

| | | | | |
|---|---|---|---|---|
| | | | | TATTACTGTGCAAGACGGGAGACTACGACGGTAGGCCGTTATTACTATGCTATGGACTACTGGGGCCAAGGGACCACGGTCACCGTCTCCTCA |
| 1359. | hCD19 4-E10-L | artificial | aa | DIQMTQSPSSLSASVGDRVTITCKASQSVDYDGTSYLNWYQQKPGQAPKLLIYDASNLVSGVPSRFSGSGSGTDFTLNISSVQPEDVATYYCQQSTEDPWTFGQGTKVEIK |
| 1360. | hCD19 4-E10-LCDR1 | artificial | aa | RSSKSLLHSNGNTYLY |
| 1361. | hCD19 4-E10-LCDR2 | artificial | aa | RMSNLAS |
| 1362. | hCD19 4-E10-LCDR3 | artificial | aa | LQHLEYPYT |
| 1363. | hCD19 4-E10-L | artificial | nt | GACATCCAGATGACCCAGTCTCCATCTTCTTTGTCTGCGTCTGTAGGGGACAGAGTCACCATCACCTGCAAGGCCAGCCAAAGTGTTGATTATGATGGTACGAGTTATTTGAACTGGTACCAACAGAAACCAGGACAGGCACCCAAACTCCTCATCTATGATGCATCCAATCTAGTTTCTGGGGTCCCATCCAGGTTTAGTGGCAGTGGGTCTGGGACAGACTTCACCCTCAACATCAGTTCTGTGCAGCCGGAGGATGTCGCAACCTATTACTGTCAGCAAAGTACTGAGGATCCGTGGACGTTCGGTCAAGGGACCAAGGTGGAGATCAAA |
| 1364. | hCD19 4-E10-LH | artificial | aa | DIQMTQSPSSLSASVGDRVTITCKASQSVDYDGTSYLNWYQQKPGQAPKLLIYDASNLVSGVPSRFSGSGSGTDFTLNISSVQPEDVATYYCQQSTEDPWTFGQGTKVEIKGGGGSGGGGSGGGGSQVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1365. | hCD19 4-E10-LH | artificial | nt | GACATCCAGATGACCCAGTCTCCATCTTCTTTGTCTGCGTCTGTAGGGGACAGAGTCACCATCACCTGCAAGGCCAGCCAAAGTGTTGATTATGATGGTACGAGTTATTTGAACTGGTACCAACAGAAACCAGGACAGGCACCCAAACTCCTCATCTATGATGCATCCAATCTAGTTTCTGGGGTCCCATCCAGGTTTAGTGGCAGTGGGTCTGGGACAGACTTCACCCTCAACATCAGTTCTGTGCAGCCGGAGGATGTCGCAACCTATTACTGTCAGCAAAGTACTGAGGATCCGTGGACGTTCGGTCAAGGGACCAAGGTGGAGATCAAAGGTGGTGGTGGTTCTGGCGGCGGCGGCTCCGGTGGTGGTGGTTCTCAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1366. | hCD19 4-E10 LH x | artificial | aa | DIQMTQSPSSLSASVGDRVTITCKASQSVDYDGTSYLNWYQQKPGQAPKLLIYDASNLV |

| | | | | |
|---|---|---|---|---|
| | I2C HL | | | SGVPSRFSGSGSGTDFTLNISSVQPEDVATYYCQQSTEDPWTFGQGTKVEIKGGGGS<br>GGGGSGGGGSQVQLVQSGAEVVKPGASVKISCKASGYAFTSYWMNWVRQRPGQGL<br>EWIGQIWPGDGDTNYNGKFKGRVTMTADESTSTAYMELSSLRSEDTAVYYCARRETTT<br>VGRYYYAMDYWGQGTTVTVSSGGGGSEVQLVESGGGLVQPGGSLKLSCAASGFTFN<br>KYAMNWVRQAPGKGLEWVARIRSKYNNYATYYADSVKDRFTISRDDSKNTAYLQMNN<br>LKTEDTAVYYCVRHGNFGNSYISYWAYWGQGTLVTVSSGGGGSGGGGSGGGGSQTV<br>VTQEPSLTVSPGGTVTLTCGSSTGAVTSGNYPNWVQQKPGQAPRGLIGGTKFLAPGTP<br>ARFSGSLLGGKAALTLSGVQPEDEAEYYCVLWYSNRWVFGGGTKLTVL |
| 1367. | hCD19 4-E10 LH x I2C HL | artificial | nt | GACATCCAGATGACCCAGTCTCCATCTTCTTTGTCTGCGTCTGTAGGGGACAGAGT<br>CACCATCACCTGCAAGGCCAGCCAAAGTGTTGATTATGATGGTACGAGTTATTTGAA<br>CTGGTACCAACAGAAACCAGGACAGGCACCCAAACTCCTCATCTATGATGCATCCA<br>ATCTAGTTTCTGGGGTCCCATCCAGGTTTAGTGGCAGTGGGTCTGGGACAGACTTC<br>ACCCTCAACATCAGTTCTGTGCAGCCGGAGGATGTCGCAACCTATTACTGTCAGCA<br>AAGTACTGAGGATCCGTGGACGTTCGGTCAAGGGACCAAGGTGGAGATCAAAGGT<br>GGTGGTGGTTCTGGCGGCGGCGGCTCCGGTGGTGGTGGTTCTCAGGTGCAGCTG<br>GTCCAGTCTGGGGCTGAGGTGGTGAAGCCTGGGGCCTCAGTGAAGATTTCCTGCA<br>AGGCTTCTGGCTATGCATTCACTAGCTACTGGATGAACTGGGTGAGGCAGCGCCCT<br>GGACAGGGTCTTGAGTGGATTGGACAGATTTGGCCTGGAGATGGTGATACTAACTA<br>CAATGGAAAGTTCAAGGGTAGAGTCACTATGACTGCTGACGAATCCACCAGCACAG<br>CCTACATGGAACTCAGCAGCCTGCGATCTGAGGACACTGCGGTCTATTACTGTGCA<br>AGACGGGAGACTACGACGGTAGGCCGTTATTACTATGCTATGGACTACTGGGGCCA<br>AGGGACCACGGTCACCGTCTCCTCCGGAGGTGGTGGATCCGAGGTGCAGCTGGTC<br>GAGTCTGGAGGAGGATTGGTGCAGCCTGGAGGGTCATTGAAACTCTCATGTGCAG<br>CCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATT<br>ATGCCGATTCAGTGAAAGACAGGTTCACCATCTCCAGAGATGATTCAAAAAACACTG<br>CCTATCTACAAATGAACAACTTGAAAACTGAGGACACTGCCGTGTACTACTGTGTGA<br>GACATGGGAACTTCGGTAATAGCTACATATCCTACTGGGCTTACTGGGGCCAAGGG<br>ACTCTGGTCACCGTCTCCTCAGGTGGTGGTGGTTCTGGCGGCGGCGGCTCCGGTG<br>GTGGTGGTTCTCAGACTGTTGTGACTCAGGAACCTTCACTCACCGTATCACCTGGT<br>GGAACAGTCACACTCACTTGTGGCTCCTCGACTGGGGCTGTTACATCTGGCAACTA<br>CCCAAACTGGGTCCAACAAAAACCAGGTCAGGCACCCCGTGGTCTAATAGGTGGG<br>ACTAAGTTCCTCGCCCCCGGTACTCCTGCCAGATTCTCAGGCTCCCTGCTTGGAGG<br>CAAGGCTGCCCTCACCCTCTCAGGGGTACAGCCAGAGGATGAGGCAGAATATTACT<br>GTGTTCTATGGTACAGCAACCGCTGGGTGTTCGGTGGAGGAACCAAACTGACTGTC<br>CTA |

| | | | | |
|---|---|---|---|---|
| 1368. | hCD19 4-E3-H | artificial | aa | QVQLVQSGAEVVKPGASVKISCKASGYAFTSYWMNWVRQRPGQGLEWIGQIWPGDG DTNYNGKFKGRVTMTADESTSTAYMELSSLRSEDTAVYYCARRETTTVGRYYYAMDY WGQGTTVTVSS |
| 1369. | hCD19 4-E3-HCDR1 | artificial | aa | SYWMN |
| 1370. | hCD19 4-E3-HCDR2 | artificial | aa | QIWPGDGDTNYNGKFKG |
| 1371. | hCD19 4-E3-HCDR3 | artificial | aa | RETTTVGRYYYAMDY |
| 1372. | hCD19 4-E3-H | artificial | nt | CAGGTGCAGCTGGTCCAGTCTGGGGCTGAGGTGGTGAAGCCTGGGGCCTCAGTGA AGATTTCCTGCAAGGCTTCTGGCTATGCATTCACTAGCTACTGGATGAACTGGGTGA GGCAGCGCCCTGGACAGGGTCTTGAGTGGATTGGACAGATTTGGCCTGGAGATGG TGATACTAACTACAATGGAAAGTTCAAGGGTAGAGTCACTATGACTGCTGACGAATC CACCAGCACAGCCTACATGGAACTCAGCAGCCTGCGATCTGAGGACACTGCGGTC TATTACTGTGCAAGACGGGAGACTACGACGGTAGGCCGTTATTACTATGCTATGGA CTACTGGGGCCAAGGGACCACGGTCACCGTCTCCTCA |
| 1373. | hCD19 4-E3-L | artificial | aa | DIQMTQSPSSLAASVGERVTITCKASQSVDYDGTSYLNWYQQKPGKAPKLLIYDASNLV SGVPSRFSGSGSGTDFTLTINSLEKEDFATYYCQQSTEDPWTFGQGTKVEIK |
| 1374. | hCD19 4-E3-LCDR1 | artificial | aa | RSSKSLLHSNGNTYLY |
| 1375. | hCD19 4-E3-LCDR2 | artificial | aa | RMSNLAS |
| 1376. | hCD19 4-E3-LCDR3 | artificial | aa | LQHLEYPYT |
| 1377. | hCD19 4-E3-L | artificial | nt | GACATCCAGATGACCCAGTCTCCATCTTCTTTGGCTGCGTCTGTAGGGGAGAGAGT CACCATCACCTGCAAGGCCAGCCAAAGTGTTGATTATGATGGTACGAGTTATTTGAA CTGGTACCAACAGAAACCAGGAAAGGCACCCAAACTCCTCATCTATGATGCATCCA ATCTAGTTTCTGGGGTCCCATCCAGGTTTAGTGGCAGTGGGTCTGGGACAGACTTC ACCCTCACCATCAATTCTCTGGAGAAGGAGGATTTCGCAACCTATTACTGTCAGCAA AGTACTGAGGATCCGTGGACGTTCGGTCAAGGGACCAAGGTGGAGATCAAA |
| 1378. | hCD19 4-E3-LH | artificial | aa | DIQMTQSPSSLAASVGERVTITCKASQSVDYDGTSYLNWYQQKPGKAPKLLIYDASNLV SGVPSRFSGSGSGTDFTLTINSLEKEDFATYYCQQSTEDPWTFGQGTKVEIKGGGGSG GGGSGGGGSQVQLVQSGAEVVKPGASVKISCKASGYAFTSYWMNWVRQRPGQGLE WIGQIWPGDGDTNYNGKFKGRVTMTADESTSTAYMELSSLRSEDTAVYYCARRETTTV GRYYYAMDYWGQGTTVTVSS |
| 1379. | hCD19 4-E3-LH | artificial | nt | GACATCCAGATGACCCAGTCTCCATCTTCTTTGGCTGCGTCTGTAGGGGAGAGAGT CACCATCACCTGCAAGGCCAGCCAAAGTGTTGATTATGATGGTACGAGTTATTTGAA CTGGTACCAACAGAAACCAGGAAAGGCACCCAAACTCCTCATCTATGATGCATCCA ATCTAGTTTCTGGGGTCCCATCCAGGTTTAGTGGCAGTGGGTCTGGGACAGACTTC ACCCTCACCATCAATTCTCTGGAGAAGGAGGATTTCGCAACCTATTACTGTCAGCAA AGTACTGAGGATCCGTGGACGTTCGGTCAAGGGACCAAGGTGGAGATCAAAGGTG GTGGTGGTTCTGGCGGCGGCGGCTCCGGTGGTGGTGGTTCTCAGGTGCAGCTGG |

| | | | | |
|---|---|---|---|---|
| | | | | TCCAGTCTGGGGCTGAGGTGGTGAAGCCTGGGGCCTCAGTGAAGATTTCCTGCAA GGCTTCTGGCTATGCATTCACTAGCTACTGGATGAACTGGGTGAGGCAGCGCCCTG GACAGGGTCTTGAGTGGATTGGACAGATTTGGCCTGGAGATGGTGATACTAACTAC AATGGAAAGTTCAAGGGTAGAGTCACTATGACTGCTGACGAATCCACCAGCACAGC CTACATGGAACTCAGCAGCCTGCGATCTGAGGACACTGCGGTCTATTACTGTGCAA GACGGGAGACTACGACGGTAGGCCGTTATTACTATGCTATGGACTACTGGGGCCAA GGGACCACGGTCACCGTCTCCTCA |
| 1380. | hCD19 4-E3 LH X I2C HL | artificial | aa | DIQMTQSPSSLAASVGERVTITCKASQSVDYDGTSYLNWYQQKPGKAPKLLIYDASNLV SGVPSRFSGSGSGTDFTLTINSLEKEDFATYYCQQSTEDPWTFGQGTKVEIKGGGGSG GGGSGGGGSQVQLVQSGAEVVKPGASVKISCKASGYAFTSYWMNWVRQRPGQGLE WIGQIWPGDGDTNYNGKFKGRVTMTADESTSTAYMELSSLRSEDTAVYYCARRETTTV GRYYYAMDYWGQGTTVTVSSGGGGSEVQLVESGGGLVQPGGSLKLSCAASGFTFNK YAMNWVRQAPGKGLEWVARIRSKYNNYATYYADSVKDRFTISRDDSKNTAYLQMNNL KTEDTAVYYCVRHGNFGNSYISYWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPA RFSGSLLGGKAALTLSGVQPEDEAEYYCVLWYSNRWVFGGGTKLTVL |
| 1381. | hCD19 4-E3 LH X I2C HL | artificial | nt | GACATCCAGATGACCCAGTCTCCATCTTCTTTGGCTGCGTCTGTAGGGGAGAGAGT CACCATCACCTGCAAGGCCAGCCAAAGTGTTGATTATGATGGTACGAGTTATTTGAA CTGGTACCAACAGAAACCAGGAAAGGCACCCAAACTCCTCATCTATGATGCATCCA ATCTAGTTTCTGGGGTCCCATCCAGGTTTAGTGGCAGTGGGTCTGGGACAGACTTC ACCCTCACCATCAATTCTCTGGAGAAGGAGGATTTCGCAACCTATTACTGTCAGCAA AGTACTGAGGATCCGTGGACGTTCGGTCAAGGGACCAAGGTGGAGATCAAAGGTG GTGGTGGTTCTGGCGGCGGCGGCTCCGGTGGTGGTGGTTCTCAGGTGCAGCTGG TCCAGTCTGGGGCTGAGGTGGTGAAGCCTGGGGCCTCAGTGAAGATTTCCTGCAA GGCTTCTGGCTATGCATTCACTAGCTACTGGATGAACTGGGTGAGGCAGCGCCCTG GACAGGGTCTTGAGTGGATTGGACAGATTTGGCCTGGAGATGGTGATACTAACTAC AATGGAAAGTTCAAGGGTAGAGTCACTATGACTGCTGACGAATCCACCAGCACAGC CTACATGGAACTCAGCAGCCTGCGATCTGAGGACACTGCGGTCTATTACTGTGCAA GACGGGAGACTACGACGGTAGGCCGTTATTACTATGCTATGGACTACTGGGGCCAA GGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAG CCTCTGGATTCACCTTCAATAAGTACGCCATGAACTGGGTCCGCCAGGCTCCAGGA AAGGGTTTGGAATGGGTTGCTCGCATAAGAAGTAAATATAATAATTATGCAACATATT ATGCCGATTCAGTGAAAGACAGGTTCACCATCTCCAGAGATGATTCAAAAAACACTG CCTATCTACAAATGAACAACTTGAAAACTGAGGACACTGCCGTGTACTACTGTGTGA GACATGGGAACTTCGGTAATAGCTACATATCCTACTGGGCTTACTGGGGCCAAGGG |

| | | | | |
|---|---|---|---|---|
| | | | | ACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1382. | hCD19 3-H7 -H | artificial | aa | QVQLVQSGAEVVKPGASVKISCKASGYAFTSYWMNWVRQRPGQGLEWIGQIWPGDGDTNYNGKFKGRVTMTADESTSTAYMELSSLRSEDTAVYYCARRETTTVGRYYYAMDYWGQGTTVTVSS |
| 1383. | hCD19 3-H7 – HCDR1 | artificial | aa | SYWMN |
| 1384. | hCD19 3-H7 – HCDR2 | artificial | aa | QIWPGDGDTNYNGKFKG |
| 1385. | hCD19 3-H7 – HCDR3 | artificial | aa | RETTTVGRYYYAMDY |
| 1386. | hCD19 3-H7 -H | artificial | nt | CAGGTGCAGCTGGTCCAGTCTGGGGCTGAGGTGGTGAAGCCTGGGGCCTCAGTGAAGATTTCCTGCAAGGCTTCTGGCTATGCATTCACTAGCTACTGGATGAACTGGGTGAGGCAGCGCCCTGGACAGGGTCTTGAGTGGATTGGACAGATTTGGCCTGGAGATGGTGATACTAACTACAATGGAAAGTTCAAGGGTAGAGTCACTATGACTGCTGACGAATCCACCAGCACAGCCTACATGGAACTCAGCAGCCTGCGATCTGAGGACACTGCGGTCTATTACTGTGCAAGACGGGAGACTACGACGGTAGGCCGTTATTACTATGCTATGGACTACTGGGGCCAAGGGACCACGGTCACCGTCTCCTCA |
| 1387. | hCD19 3-H7 –L | artificial | aa | DIQMTQSPSSLSASVGDRVTITCKASQSVDYDGTSYLNWYQQKPGKAPKLLIYDASNLVSGVPSRFSGSGSGTDFTLNISSLQPEDVATYYCQQSTEDPWTFGQGTKVEIK |
| 1388. | hCD19 3-H7 – LCDR1 | artificial | aa | RSSKSLLHSNGNTYLY |
| 1389. | hCD19 3-H7 – LCDR2 | artificial | aa | RMSNLAS |
| 1390. | hCD19 3-H7 – LCDR3 | artificial | aa | LQHLEYPYT |
| 1391. | hCD19 3-H7 -L | artificial | nt | GACATCCAGATGACCCAGTCTCCATCTTCTTTGTCTGCGTCTGTAGGGGACAGAGTCACCATCACCTGCAAGGCCAGCCAAAGTGTTGATTATGATGGTACGAGTTATTTGAACTGGTACCAACAGAAACCAGGAAAGGCACCCAAACTCCTCATCTATGATGCATCCAATCTAGTTTCTGGGGTCCCATCCAGGTTTAGTGGCAGTGGGTCTGGGACAGACTTC |

| | | | | |
|---|---|---|---|---|
| | | | | ACCCTCAACATCAGTTCTCTGCAGCCGGAGGATGTCGCAACCTATTACTGTCAGCA AAGTACTGAGGATCCGTGGACGTTCGGTCAAGGGACCAAGGTGGAGATCAAA |
| 1392. | hCD19 3-H7 -LH | artificial | aa | DIQMTQSPSSLSASVGDRVTITCKASQSVDYDGTSYLNWYQQKPGKAPKLLIYDASNLV SGVPSRFSGSGSGTDFTLNISSLQPEDVATYYCQQSTEDPWTFGQGTKVEIKGGGGS GGGGSGGGGSQVQLVQSGAEVVKPGASVKISCKASGYAFTSYWMNWVRQRPGQGL EWIGQIWPGDGDTNYNGKFKGRVTMTADESTSTAYMELSSLRSEDTAVYYCARRETTT VGRYYYAMDYWGQGTTVTVSS |
| 1393. | hCD19 3-H7 -LH | artificial | nt | GACATCCAGATGACCCAGTCTCCATCTTCTTTGTCTGCGTCTGTAGGGGACAGAGT CACCATCACCTGCAAGGCCAGCCAAAGTGTTGATTATGATGGTACGAGTTATTTGAA CTGGTACCAACAGAAACCAGGAAAGGCACCCAAACTCCTCATCTATGATGCATCCA ATCTAGTTTCTGGGGTCCCATCCAGGTTTAGTGGCAGTGGGTCTGGGACAGACTTC ACCCTCAACATCAGTTCTCTGCAGCCGGAGGATGTCGCAACCTATTACTGTCAGCA AAGTACTGAGGATCCGTGGACGTTCGGTCAAGGGACCAAGGTGGAGATCAAAGGT GGTGGTGGTTCTGGCGGCGGCGGCTCCGGTGGTGGTGGTTCTCAGGTGCAGCTG GTCCAGTCTGGGGCTGAGGTGGTGAAGCCTGGGGCCTCAGTGAAGATTTCCTGCA AGGCTTCTGGCTATGCATTCACTAGCTACTGGATGAACTGGGTGAGGCAGCGCCCT GGACAGGGTCTTGAGTGGATTGGACAGATTTGGCCTGGAGATGGTGATACTAACTA CAATGGAAAGTTCAAGGGTAGAGTCACTATGACTGCTGACGAATCCACCAGCACAG CCTACATGGAACTCAGCAGCCTGCGATCTGAGGACACTGCGGTCTATTACTGTGCA AGACGGGAGACTACGACGGTAGGCCGTTATTACTATGCTATGGACTACTGGGGCCA AGGGACCACGGTCACCGTCTCCTCA |
| 1394. | hCD19 3-H7 LH X I2C HL | artificial | aa | DIQMTQSPSSLSASVGDRVTITCKASQSVDYDGTSYLNWYQQKPGKAPKLLIYDASNLV SGVPSRFSGSGSGTDFTLNISSLQPEDVATYYCQQSTEDPWTFGQGTKVEIKGGGGS GGGGSGGGGSQVQLVQSGAEVVKPGASVKISCKASGYAFTSYWMNWVRQRPGQGL EWIGQIWPGDGDTNYNGKFKGRVTMTADESTSTAYMELSSLRSEDTAVYYCARRETTT VGRYYYAMDYWGQGTTVTVSSGGGGSEVQLVESGGGLVQPGGSLKLSCAASGFTFN KYAMNWVRQAPGKGLEWVARIRSKYNNYATYYADSVKDRFTISRDDSKNTAYLQMNN LKTEDTAVYYCVRHGNFGNSYISYWAYWGQGTLVTVSSGGGGSGGGGSGGGGSQTV VTQEPSLTVSPGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL |
| 1395. | hCD19 3-H7 LH X I2C HL | artificial | nt | GACATCCAGATGACCCAGTCTCCATCTTCTTTGTCTGCGTCTGTAGGGGACAGAGT CACCATCACCTGCAAGGCCAGCCAAAGTGTTGATTATGATGGTACGAGTTATTTGAA CTGGTACCAACAGAAACCAGGAAAGGCACCCAAACTCCTCATCTATGATGCATCCA ATCTAGTTTCTGGGGTCCCATCCAGGTTTAGTGGCAGTGGGTCTGGGACAGACTTC ACCCTCAACATCAGTTCTCTGCAGCCGGAGGATGTCGCAACCTATTACTGTCAGCA |

| | | | | |
|---|---|---|---|---|
| | | | | AAGTACTGAGGATCCGTGGACGTTCGGTCAAGGGACCAAGGTGGAGATCAAAGGT GGTGGTGGTTCTGGCGGCGGCGGCTCCGGTGGTGGTGGTTCTCAGGTGCAGCTG GTCCAGTCTGGGGCTGAGGTGGTGAAGCCTGGGGCCTCAGTGAAGATTTCCTGCA AGGCTTCTGGCTATGCATTCACTAGCTACTGGATGAACTGGGTGAGGCAGCGCCCT GGACAGGGTCTTGAGTGGATTGGACAGATTTGGCCTGGAGATGGTGATACTAACTA CAATGGAAAGTTCAAGGGTAGAGTCACTATGACTGCTGACGAATCCACCAGCACAG CCTACATGGAACTCAGCAGCCTGCGATCTGAGGACACTGCGGTCTATTACTGTGCA AGACGGGAGACTACGACGGTAGGCCGTTATTACTATGCTATGGACTACTGGGGCCA AGGGACCACGGTCACCGTCTCCTCCGGAGGTGGTGGATCCGAGGTGCAGCTGGTC GAGTCTGGAGGAGGATTGGTGCAGCCTGGAGGGTCATTGAAACTCTCATGTGCAG CCTCTGGATTCACCTTCAATAAGTACGCCATGAACTGGGTCCGCCAGGCTCCAGGA AAGGGTTTGGAATGGGTTGCTCGCATAAGAAGTAAATATAATAATTATGCAACATATT ATGCCGATTCAGTGAAAGACAGGTTCACCATCTCCAGAGATGATTCAAAAAACACTG CCTATCTACAAATGAACAACTTGAAAACTGAGGACACTGCCGTGTACTACTGTGTGA GACATGGGAACTTCGGTAATAGCTACATATCCTACTGGGCTTACTGGGGCCAAGGG ACTCTGGTCACCGTCTCCTCAGGTGGTGGTGGTTCTGGCGGCGGCGGCTCCGGTG GTGGTGGTTCTCAGACTGTTGTGACTCAGGAACCTTCACTCACCGTATCACCTGGT GGAACAGTCACACTCACTTGTGGCTCCTCGACTGGGGCTGTTACATCTGGCAACTA CCCAAACTGGGTCCAACAAAAACCAGGTCAGGCACCCCGTGGTCTAATAGGTGGG ACTAAGTTCCTCGCCCCCGGTACTCCTGCCAGATTCTCAGGCTCCCTGCTTGGAGG CAAGGCTGCCCTCACCCTCTCAGGGGTACAGCCAGAGGATGAGGCAGAATATTACT GTGTTCTATGGTACAGCAACCGCTGGGTGTTCGGTGGAGGAACCAAACTGACTGTC CTA |
| 1396. | cMET-1 | artificial | nt | atgggatggagctgtatcatcctcttcttggtagcaacagctacaggtgtacactccgagtgtaaagaggcactag caaagtccgagatgaatgtgaatatgaagtatcagcttcccaacttcaccgcggaaacacccatccagaatgtc attctacatgagcatcacattttccttggtgccactaactacatttatgttttaaatgaggaagaccttcagaaggttgc tgagtacaagactgggcctgtgctggaacacccagattgtttcccatgtcaggactgcagcagcaaagccaattt atcaggaggtgtttggaaagataacatcaacatggctctagttgtggacacctactatgatgatcaactcattagct gtggcagcgtcaacagagggacctgccagcgacatgtctttccccacaatcatactgctgacatacagtcggag gttcactgcatattctccccacagatagaagagcccagccagtgtcctgactgtgtggtgagcgccctgggagcc aaagtcctttcatctgtaaaggaccggttcatcaacttctttgtaggcaataccataaattcttcttatttcccagatcat ccattgcattcgatatcagtgagaaggctaaaggaaacgaaagatggttttatgtttttgacggaccagtcctacat tgatgttttacctgagttcagagattcttaccccattaagtatgtccatgcctttgaaagcaacaattttatttacttcttga cggtccaaagggaaactctagatgctcagacttttcacacaagaataatcaggttctgttccataaactctggattg |

| | | | | |
|---|---|---|---|---|
| | | | | cattcctacatggaaatgcctctggagtgtattctcacagaaaagagaaaaaagagatccacaaagaaggaa<br>gtgtttaatatacttcaggctgcgtatgtcagcaagcctggggcccagcttgctagacaaataggagccagcctg<br>aatgatgacattcttttcggggtgttcgcacaaagcaagccagattctgccgaaccaatggatcgatctgccatgt<br>gtgcattccctatcaaatatgtcaacgacttcttcaacaagatcgtcaacaaaaacaatgtgagatgtctccagca<br>tttttacggacccaatcatgagcactgctttaataggacacttctgagaaattcatcaggctgtgaagcgcgccgtg<br>atgaatatcgaacagagtttaccacagctttgcagcgcgttgacttattcatgggtcaattcagcgaagtcctcttaa<br>catctatatccaccttcattaaaggagacctcaccatagctaatcttgggacatcagagggtcgcttcatgcaggtt<br>gtggtttctcgatcaggaccatcaacccctcatgtgaattttctcctggactcccatccagtgtctccagaagtgattg<br>tggagcatacattaaaccaaaatggctacacactggttatcactgggaagaagatcacgaagatcccattgaat<br>ggcttgggctgcagacatttccagtcctgcagtcaatgcctctctgccccaccctttgttcagtgtggctggtgccac<br>gacaaatgtgtgcgatcggaggaatgcctgagcgggacatggactcaacagatctgtctgcctgcaatctacaa<br>ggttttcccaaatagtgcaccccttgaaggagggacaaggctgaccatatgtggctgggactttggatttcggagg<br>aataataaatttgatttaaagaaaactagagttctccttggaaatgagagctgcaccttgactttaagtgagagcac<br>gatgaatacattgaaatgcacagttggtcctgccatgaataagcatttcaatatgtccataattatttcaaatggcca<br>cgggacaacacaatacagtacattctcctatgtggatcctgtaataacaagtatttcgccgaaatacggtcctatg<br>gctggtggcactttacttactttaactggaaattacctaaacagtgggaatagcagacacatttcaattggtggaaa<br>aacatgtactttaaaaagtgtgtcaaacagtattcttgaatgttataccccagcccaaaccatttcaactgagtttgct<br>gttaaattgaaaattgacttagccaaccgagagacaagcatcttcagttaccgtgaagatcccattgtctatgaaat<br>tcatccaaccaaatcttttattagtggtgggagcacaataacaggtgttgggaaaaacctgaactcagttagtgtcc<br>cgagaatggtcataaatgtgcatgaagcaggaaggaactttacagtggcatgtcaacatcgctctaattcagag<br>ataatctgttgtaccactccttccctgcaacagctgaatctgcaactccccctgaaaaccaaagccttttcatgttag<br>atgggatcctttccaaatactttgatctcatttatgtacataatcctgtgtttaagcctttgaaaagccagtgatgatctc<br>aatgggcaatgaaaatgtactggaaattaagggaaatgatattgaccctgaagcagttaaaggtgaagtgttaa<br>aagttggaaataagagctgtgagaatatacacttacattctgaagccgttttatgcacggtccccaatgacctgctg<br>aaattgaacagcgagctaaatatagagtggaagcaagcaatttcttcaaccgtccttggaaaagtaatagttcaa<br>ccagatcagaatttcacatccggaggtggtggatccggggctggtgttattgctgttattgtggttgtggtgatagca<br>attgttgctggaattgttgtgctggttatttccagaaagaagagaatggcaaagtatgagaaggctgagataaagg<br>agatgggtgagatgcatagggaactcaatgcataa |
| 1397. | cMET-2 | artificial | aa | mgwsciilflvatatgvhseckealaksemnvnmkyqlpnftaetpiqnvilhehhiflgatnyiyvlneedlqkva<br>eyktgpvlehpdcfpcqdcsskanlsggvwkdninmalvvdtyyddqliscgsvnrgtcqrhvfphnhtadiqs<br>evhcifspqieepsqcpdcvvsalgakvlssvkdrfinffvgntinssyfpdhplhsisvrrlketkdgfmfltdqsyi<br>dvlpefrdsypikyvhafesnnfiyfltvqretldaqtfhtriirfcsinsglhsymempleciltekrkkrstkkevfnilq<br>aayvskpgaqlarqigaslnddilfgvfaqskpdsaepmdrsamcafpikyvndffnkivnknnvrclqhfygpn |

| | | | | |
|---|---|---|---|---|
| | | | | hehcfnrtllrnssgcearrdeyrtefttalqrvdlfmgqfsevlltsistfikgdltianlgtsegrfmqvvvsrsgpstph vnflldshpvspevivehtlnqngytlvitgkkitkiplnglgcrhfqscsqclsappfvqcgwchdkcvrseeclsgt wtqqiclpaiykvfpnsapleggtrlticgwdfgfrrnnkfdlkktrvllgnesctltlsestmntlkctvgpamnkhfn msiiisnghgttqystfsyvdpvitsispkygpmaggtlltltgnylnsgnsrhisiggktctlksvsnsilecytpaqtis tefavklkidlanretsifsyredpivyeihptksfisggstitgvgknlnsvsvprmvinvheagrnftvacqhrsns eiiccttpslqqlnlqlplktkaffmldgilskyfdliyvhnpvfkpfekpvmismgnenvleikgndidpeavkgevl kvgnkscenihlhseavlctvpndllklnselniewkqaisstvlgkvivqpdqnftsggggsgagviavivvvviai vagivvlvisrkkrmakyekaeikemgemhrelna |
| 1398. | cMET-3 | artificial | nt | atgggatggagctgtatcatcctcttcttggtagcaacagctacaggtgtacactccgagtgtaaagaggcactag caaagtccgagatgaatgtgaatatgaagtatcagcttcccaacttcaccgcggaaacagccatccagaatgtc attctacatgagcatcacattttcctcggtgccaccaactacatttatgttttaaatgaggaagaccttcagaaggttg ctgagtacaagaccgggcctgtgctggaacacccagattgtttcccgtgtcaggactgcagcagcaaagccaat ttatcaggaggcgtttggaaagataacatcaacatggctctggttgtggacacctactatgatgatcaactcattag ctgtggcagcgtcaacagagggacctgccagcgacatgtctttccccataatcatactgctgacatacagtcgga ggttcactgcatattctccccacagatagaagagcccaaccagtgccctgactgtgtggtgagcgccctgggag ccaaagtcctttcatctgtaaaggaccggttcatcaacttctttgtaggcaataccataaattcttcttatttcccacatc atccgttgcattcgatatcagtgagaaggctaaaggaaacgaaagatggttttatgtttttgacagaccagtcctac attgatgttttacctgagttcagagattcttaccccattaagtacatccacgcttttgaaagcaacaattttatttacttctt gacggtccaaagggaaactctaaatgctcagacttttcacacaagaataatcaggttctgttccttaaactctggat tgcactcctacatggaaatgcctctagagtgtattctcacagaaaagagaaaaaagagatccacaaagaagg aagtgtttaatatccttcaggctgcatatgtcagcaagcccggggcccagcttgctagacaaataggagccagcc tgaatgatgacattctctttggggtgttcgcacaaagcaagccagattctgccgaaccaatggatcgatctgcaat gtgtgcatttcctatcaaatatgtcaacgacttcttcaacaagatcgtcaacaaaaacaatgtgagatgtctccagc atttttatggacccaatcatgagcactgttttaataggacacttctgagaaattcatcaggctgtgaagcgcgccgtg atgaatatcgagcagagtttaccacagctttgcagcgggttgacttattcatgggtcaattcagcgaagtcctcttaa catctatatccaccttcgtgaaaggagacctcaccatcgctaatcttgggacatcagagggtcgcttcatgcaggtt gtggtttctcgatcaggaccatcaacccctcatgtgaattttctcctggactctcacccggtgtctccagaagtgattg tggagcatccattaaaccagaatggctacacactggttgtcacggggaagaagatcaccaagatcccattgaat ggcttgggctgcagacatttccagtcctgcagtcagtgcctctctgccccaccctttgtgcagtgtggctggtgccac gacaaatgtgtgcgatcggaggaatgccccagtgggacatggactcaacagatctgtctgcctgcaatctacaa ggttttcccaactagtgcaccccttgaaggagggacaaggctgaccatatgtggctgggactttggatttcggagg aataataaatttgatttaaagaaaactagagttctccttggaaatgagagctgcaccttgactttaagtgagagcac gatgaatacattgaaatgcacagttggtcctgccatgaataaacatttcaatatgtccataattatttcaaatggcca |

| | | | | |
|---|---|---|---|---|
| | | | | cgggacaacacaatacagtacattttcctatgtggatcctataataacaagtatttcgccaaaatatggccctatggctggtggcactttacttactttaactggaaattacctaaacagtggcaattctagacacatttcaattggtggaaaaacatgtactttaaaaagtgtgtcaaacagtattctcgaatgttataccccagcccaaaccatttcaactgagtttgctgttaaattgaaaattgacttagccaaccgagagacaagcatcttcagttaccgggaagaccccattgtctatgaaattcatccaaccaaatcttttattagtggtgggagcacaataacaggtgttgggaaaaacctgcattcagttagtgtcccgaggatggtcataaatgtgcatgaagcaggaaggaactttacagtggcatgtcagcatcgctctaattcagagataatctgctgtaccactccttccctgcaacagctgaatctgcaactccccctgaaaaccaaagcctttttcatgttagatgggatcctttccaaatactttgatctcatttatgtacataatcctgtgtttaagccttttgaaaagccagtaatgatctcaatgggcaatgaaaatgtactggaaattaagggaaatgatattgaccctgaagcagttaaaggtgaagtgttaaaagttggaaataagagctgtgagaatatacacttacattctgaagccgttttatgcacggtccccaatgacctgctgaaattgaacagcgagctaaatatagagtggaagcaagcaatttcttcaaccgtccttggaaaagtaatagttcaaccagatcagaatttcacctccggaggtggtggatccggggctggtgttattgctgttattgtggttgtggtgatagcaattgttgctggaattgttgtgctggttatttccagaaagaagagaatggcaaagtatgagaaggctgagataaaggagatgggtgagatgcatagggaactcaatgcataa |
| 1399. | cMET-4 | artificial | aa | mgwsciilflvatatgvhseckealaksemnvnmkyqlpnftaetaiqnvilhehhiflgatnyiyvlneedlqkvaeyktgpvlehpdcfpcqdcsskanlsggvwkdninmalvvdtyyddqliscgsvnrgtcqrhvfphnhtadiqsevhcifspqieepnqcpdcvvsalgakvlssvkdrfinffvgntinssyfphhplhsisvrrlketkdgfmfltdqsyidvlpefrdsypikyihafesnnfiyfltvqretlnaqtfhtriirfcslnsglhsymempleciltekrkkrstkkevfnilqaayvskpgaqlarqigaslnddilfgvfaqskpdsaepmdrsamcafpikyvndffnkivnknnvrclqhfygpnhehcfnrtllrnssgcearrdeyraefttalqrvdlfmgqfsevlltsistfvkgdltianlgtsegrfmqvvvsrsgpstphvnflldshpvspevivehplnqngytlvvtgkkitkiplnglgcrhfqscsqclsappfvqcgwchdkcvrseecpsgtwtqqiclpaiykvfptsapleggtrlticgwdfgfrrnnkfdlkktrvllgnesctltlsestmntlkctvgpamnkhfnmsiiisnghgttqystfsyvdpiitsispkygpmaggtlltltgnylnsgnsrhisiggktctlksvsnsilecytpaqtistefavklkidlanretsifsyredpivyeihptksfisggstitgvgknlhsvsvprmvinvheagrnftvacqhrsnseiicttpslqqlnlqlplktkaffmldgilskyfdliyvhnpvfkpfekpvmismgnenvleikgndidpeavkgevlkvgnkscenihlhseavlctvpndllklnselniewkqaisstvlgkvivqpdqnftsggggsgagviavivvviaivagivvlvisrkkrmakyekaeikemgemhrelna |
| 1400. | ME86H11-H | artificial | aa | QVQLVQSGAEVKRPGASVKVSCRASGYTFTSYWLHWVRQGPGQRLEWIGMIDPSNSDTRFNPNFKDRATFTVDTSASTAYMELSSLTSEDTAVYYCATYGSYVSPLDYWGQGTSVTVSS |
| 1401. | ME86H11-HCDR1 | artificial | aa | SYWLH |
| 1402. | ME86H11-HCDR2 | artificial | aa | MIDPSNSDTRFNPNFKD |
| 1403. | ME86H11-HCDR3 | artificial | aa | YGSYVSPLDY |
| 1404. | ME86H11-H | artificial | nt | CAGGTGCAGCTGGTCCAGTCTGGGGCTGAAGTGAAGAGGCCTGGGGCTTCAGTGA |

| | | | | |
|---|---|---|---|---|
| | | | | AAGTGTCCTGCAGGGCTTCGGGCTATACCTTCACCAGCTACTGGTTGCACTGGGTTAGACAGGGGCCTGGACAAAGGCTTGAGTGGATAGGCATGATTGATCCTTCCAATAGTGACACTAGGTTTAATCCGAACTTCAAGGACAGGGCCACATTCACTGTAGACACATCTGCCAGCACAGCCTACATGGAGCTCAGCAGCCTGACATCTGAGGACACTGCAGTCTATTACTGTGCCACATATGGTAGCTACGTTTCCCCTCTGGACTACTGGGGTCAAGGAACCTCGGTCACCGTCTCCTCA |
| 1405. | ME86H11-L | artificial | aa | DIVMTQTPFSLPVTLGETVSISCKSSQSLLYTSSQKNYLAWYLQKPGQSPQLLIYWASTRESGVPDRFSGSGSGTDFTLKISRVEAEDVGVYYCQQYYAYPWTFGGGTKLEIK |
| 1406. | ME86H11-LCDR1 | artificial | aa | KSSQSLLYTSSQKNYLA |
| 1407. | ME86H11-LCDR2 | artificial | aa | WASTRES |
| 1408. | ME86H11-LCDR3 | artificial | aa | QQYYAYPWT |
| 1409. | ME86H11-L | artificial | nt | GACATCGTGATGACCCAGACTCCATTCTCCCTACCTGTGACACTTGGAGAGACGGTTTCTATCAGCTGCAAGTCCAGTCAGTCCCTTTTATATACTAGCAGTCAGAAGAACTACTTGGCCTGGTACCTGCAGAAACCAGGTCAGTCTCCTCAACTGCTGATTTACTGGGCATCCACTAGGGAATCTGGGGTCCCTGATCGCTTCTCAGGCAGTGGATCTGGGACAGATTTCACTCTCAAAATCTCCAGAGTGGAGGCTGAGGACGTGGGAGTTTATTACTGTCAGCAATATTATGCCTATCCGTGGACGTTCGGTGGAGGCACAAAGTTGGAGATCAAA |
| 1410. | ME86H11-HL | artificial | aa | QVQLVQSGAEVKRPGASVKVSCRASGYTFTSYWLHWVRQGPGQRLEWIGMIDPSNSDTRFNPNFKDRATFTVDTSASTAYMELSSLTSEDTAVYYCATYGSYVSPLDYWGQGTSVTVSSGGGGSGGGGSGGGGSDIVMTQTPFSLPVTLGETVSISCKSSQSLLYTSSQKNYLAWYLQKPGQSPQLLIYWASTRESGVPDRFSGSGSGTDFTLKISRVEAEDVGVYYCQQYYAYPWTFGGGTKLEIK |
| 1411. | ME86H11-HL | artificial | nt | CAGGTGCAGCTGGTCCAGTCTGGGGCTGAAGTGAAGAGGCCTGGGGCTTCAGTGAAAGTGTCCTGCAGGGCTTCGGGCTATACCTTCACCAGCTACTGGTTGCACTGGGTTAGACAGGGGCCTGGACAAAGGCTTGAGTGGATAGGCATGATTGATCCTTCCAATAGTGACACTAGGTTTAATCCGAACTTCAAGGACAGGGCCACATTCACTGTAGACACATCTGCCAGCACAGCCTACATGGAGCTCAGCAGCCTGACATCTGAGGACACTGCAGTCTATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCATTCTCCCTACCTGTGACACTTGGAGAGACGGTTTCTATCAGCTGCAAGTCCAGTCAGTCCCTTTTATATACTAGCAGTCAGAAGAACTACTTGGCCTGGTACCTGCAGAAACCAGGTCAGTCTCCTCAACTGCTGATTTACTGGGCATCCACTAGGGAATCTGGGGTCCCTGATCGCTTCTCAGGCAGTGGATCTGGGACAGATTTCACTCTCAAAATCTCCAGAGTGGAGGCTGAGGACGTGGGAGTTTATTACTGTCAGCAATATTATGCCTATCCGTGGACGTTCGGTGGAGGCACAAAG |

| | | | | |
|---|---|---|---|---|
| | | | | TTGGAGATCAAA |
| 1412. | ME86H11 HL x I2C HL | artificial | aa | QVQLVQSGAEVKRPGASVKVSCRASGYTFTSYWLHWVRQGPGQRLEWIGMIDPSNS<br>DTRFNPNFKDRATFTVDTSASTAYMELSSLTSEDTAVYYCATYGSYVSPLDYWGQGTS<br>VTVSSGGGGSGGGGSGGGGSDIVMTQTPFSLPVTLGETVSISCKSSQSLLYTSSQKNY<br>LAWYLQKPGQSPQLLIYWASTRESGVPDRFSGSGSGTDFTLKISRVEAEDVGVYYCQQ<br>YYAYPWTFGGGTKLEIKSGGGGSEVQLVESGGGLVQPGGSLKLSCAASGFTFNKYAM<br>NWVRQAPGKGLEWVARIRSKYNNYATYYADSVKDRFTISRDDSKNTAYLQMNNLKTED<br>TAVYYCVRHGNFGNSYISYWAYWGQGTLVTVSSGGGGSGGGGSGGGGSQTVVTQE<br>PSLTVSPGGTVTLTCGSSTGAVTSGNYPNWVQQKPGQAPRGLIGGTKFLAPGTPARFS<br>GSLLGGKAALTLSGVQPEDEAEYYCVLWYSNRWVFGGGTKLTVL |
| 1413. | ME86H11 HL x I2C HL | artificial | nt | CAGGTGCAGCTGGTCCAGTCTGGGGCTGAAGTGAAGAGGCCTGGGGCTTCAGTGA<br>AAGTGTCCTGCAGGGCTTCGGGCTATACCTTCACCAGCTACTGGTTGCACTGGGTT<br>AGACAGGGGCCTGGACAAAGGCTTGAGTGGATAGGCATGATTGATCCTTCCAATAG<br>TGACACTAGGTTTAATCCGAACTTCAAGGACAGGGCCACATTCACTGTAGACACATC<br>TGCCAGCACAGCCTACATGGAGCTCAGCAGCCTGACATCTGAGGACACTGCAGTCT<br>ATTACTGTGCCACATATGGTAGCTACGTTTCCCCTCTGGACTACTGGGGTCAAGGA<br>ACCTCGGTCACCGTCTCCTCAGGTGGTGGTGGTTCTGGCGGCGGCGGCTCCGGTG<br>GTGGTGGTTCTGACATCGTGATGACCCAGACTCCATTCTCCCTACCTGTGACACTT<br>GGAGAGACGGTTTCTATCAGCTGCAAGTCCAGTCAGTCCCTTTTATATACTAGCAGT<br>CAGAAGAACTACTTGGCCTGGTACCTGCAGAAACCAGGTCAGTCTCCTCAACTGCT<br>GATTTACTGGGCATCCACTAGGGAATCTGGGGTCCCTGATCGCTTCTCAGGCAGTG<br>GATCTGGGACAGATTTCACTCTCAAAATCTCCAGAGTGGAGGCTGAGGACGTGGGA<br>GTTTATTACTGTCAGCAATATTATGCCTATCCGTGGACGTTCGGTGGAGGCACAAAG<br>TTGGAGATCAAATCCGGAGGTGGTGGATCCGAGGTGCAGCTGGTCGAGTCTGGAG<br>GAGGATTGGTGCAGCCTGGAGGGTCATTGAAACTCTCATGTGCAGCCTCTGGATTC<br>ACCTTCAATAAGTACGCCATGAACTGGGTCCGCCAGGCTCCAGGAAAGGGTTTGGA<br>ATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAA<br>ATGAACAACTTGAAAACTGAGGACACTGCCGTGTACTACTGTGTGAGACATGGGAA<br>CTTCGGTAATAGCTACATATCCTACTGGGCTTACTGGGGCCAAGGGACTCTGGTCA<br>CCGTCTCCTCAGGTGGTGGTGGTTCTGGCGGCGGCGGCTCCGGTGGTGGTGGTTC<br>TCAGACTGTTGTGACTCAGGAACCTTCACTCACCGTATCACCTGGTGGAACAGTCA<br>CACTCACTTGTGGCTCCTCGACTGGGGCTGTTACATCTGGCAACTACCCAAACTGG<br>GTCCAACAAAAACCAGGTCAGGCACCCCGTGGTCTAATAGGTGGGACTAAGTTCCT<br>CGCCCCCGGTACTCCTGCCAGATTCTCAGGCTCCCTGCTTGGAGGCAAGGCTGCC<br>CTCACCCTCTCAGGGGTACAGCCAGAGGATGAGGCAGAATATTACTGTGTTCTATG |

| | | | | |
|---|---|---|---|---|
| | | | | GTACAGCAACCGCTGGGTGTTCGGTGGAGGAACCAAACTGACTGTCCTA |
| 1414. | ME62A12-H | artificial | aa | QVQLVQSGAEVVKPGASVKVSCKASGYTFTSYWLHWVRQTPGQGLEWIGMIDPSNSDTRFNPNFKDKVTFTVDTSASTAYMELSSLRSEDTAVYYCATYGSYVSPLDYWGQGTSVTVSS |
| 1415. | ME62A12-HCDR1 | artificial | aa | SYWLH |
| 1416. | ME62A12-HCDR2 | artificial | aa | MIDPSNSDTRFNPNFKD |
| 1417. | ME62A12-HCDR3 | artificial | aa | YGSYVSPLDY |
| 1418. | ME62A12-H | artificial | nt | CAGGTGCAGCTGGTCCAGTCTGGGGCTGAAGTGGTGAAGCCTGGGGCTTCAGTGAAAGTGTCCTGCAAGGCTTCGGGCTATACCTTCACCAGCTACTGGTTGCACTGGGTTAGACAGACGCCTGGACAAGGGCTTGAGTGGATAGGCATGATTGATCCTTCCAATAGTGACACTAGGTTTAATCCGAACTTCAAGGACAAGGTCACATTCACTGTAGACACATCTGCCAGCACAGCCTACATGGAACTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCA |
| 1419. | ME62A12-L | artificial | aa | DIVMTQTPLSLPVTLGEKVSVSCKSSQSLLYTSSQKNYLAWYQQKPGQSPKLLIYWASTRESGVPDRFSGSGSGTDFTLTISRVEAEDLGVYYCQQYYAYPWTFGGGTKLEIK |
| 1420. | ME62A12-LCDR1 | artificial | aa | KSSQSLLYTSSQKNYLA |
| 1421. | ME62A12-LCDR2 | artificial | aa | WASTRES |
| 1422. | ME62A12-LCDR3 | artificial | aa | QQYYAYPWT |
| 1423. | ME62A12-L | artificial | nt | GACATCGTGATGACCCAGACTCCACTCTCCCTACCTGTGACACTTGGAGAGAAGGTTTCTGTCAGCTGCAAGTCCAGTCAGTCCCTTTTATATACTAGCAGTCAGAAGAACTACTTGGCCTGGTACCAGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCACAATCTCCAGAGTGGAGGCTGAGGACCTGGGAGTTTATTACTGTCAGCAATATTATGCCTATCCGTGGACGTTCGGTGGAGGCACAAAGTTGGAGATCAAA |
| 1424. | ME62A12-HL | artificial | aa | QVQLVQSGAEVVKPGASVKVSCKASGYTFTSYWLHWVRQTPGQGLEWIGMIDPSNSDTRFNPNFKDKVTFTVDTSASTAYMELSSLRSEDTAVYYCATYGSYVSPLDYWGQGTSVTVSSGGGGSGGGGSGGGGSDIVMTQTPLSLPVTLGEKVSVSCKSSQSLLYTSSQKNYLAWYQQKPGQSPKLLIYWASTRESGVPDRFSGSGSGTDFTLTISRVEAEDLGVYYCQQYYAYPWTFGGGTKLEIK |
| 1425. | ME62A12-HL | artificial | nt | CAGGTGCAGCTGGTCCAGTCTGGGGCTGAAGTGGTGAAGCCTGGGGCTTCAGTGAAAGTGTCCTGCAAGGCTTCGGGCTATACCTTCACCAGCTACTGGTTGCACTGGGTTAGACAGACGCCTGGACAAGGGCTTGAGTGGATAGGCATGATTGATCCTTCCAATAGTGACACTAGGTTTAATCCGAACTTCAAGGACAAGGTCACATTCACTGTAGACACATCTGCCAGCACAGCCTACATGGAACTCAGCAGCCTGAGATCTGAGGACACTGCAGTCT |

| | | | | |
|---|---|---|---|---|
| | | | | ATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCACTCTCCCTACCTGTGACACTTGGAGAGAAGGTTTCTGTCAGCTGCAAGTCCAGTCAGTCCCTTTTATATACTAGCAGTCAGAAGAACTACTTGGCCTGGTACCAGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCACAATCTCCAGAGTGGAGGCTGAGGACCTGGGAGTTTATTACTGTCAGCAATATTATGCCTATCCGTGGACGTTCGGTGGAGGCACAAAGTTGGAGATCAAA |
| 1426. | ME62A12 HL x I2C HL | artificial | aa | QVQLVQSGAEVVKPGASVKVSCKASGYTFTSYWLHWVRQTPGQGLEWIGMIDPSNSDTRFNPNFKDKVTFTVDTSASTAYMELSSLRSEDTAVYYCATYGSYVSPLDYWGQGTSVTVSSGGGGSGGGGSGGGGSDIVMTQTPLSLPVTLGEKVSVSCKSSQSLLYTSSQKNYLAWYQQKPGQSPKLLIYWASTRESGVPDRFSGSGSGTDFTLTISRVEAEDLGVYYCQQYYAYPWTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1427. | ME62A12 HL x I2C HL | artificial | nt | CAGGTGCAGCTGGTCCAGTCTGGGGCTGAAGTGGTGAAGCCTGGGGCTTCAGTGAAAGTGTCCTGCAAGGCTTCGGGCTATACCTTCACCAGCTACTGGTTGCACTGGGTTAGACAGACGCCTGGACAAGGGCTTGAGTGGATAGGCATGATTGATCCTTCCAATAGTGACACTAGGTTTAATCCGAACTTCAAGGACAAGGTCACATTCACTGTAGACACATCTGCCAGCACAGCCTACATGGAACTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCACTCTCCCTACCTGTGACACTTGGAGAGAAGGTTTCTGTCAGCTGCAAGTCCAGTCAGTCCCTTTTATATACTAGCAGTCAGAAGAACTACTTGGCCTGGTACCAGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCACAATCTCCAGAGTGGAGGCTGAGGACCTGGGAGTTTATTACTGTCAGCAATATTATGCCTATCCGTGGACGTTCGGTGGAGGCACAAAGTTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAA |

| | | | | |
|---|---|---|---|---|
| | | | | ATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1428. | ME63F2-H | artificial | aa | QVQLVQSGAEVKRPGASVKVSCKASGYTFTSYWLHWVRQTPGQRLEWIGMIDPSNSDTRFNPNFKDRVTMTVDTSANTAYMELSSLRSEDTAVYYCATYGSYVSPLDYWGQGTSVTVSS |
| 1429. | ME63F2-HCDR1 | artificial | aa | SYWLH |
| 1430. | ME63F2-HCDR2 | artificial | aa | MIDPSNSDTRFNPNFKD |
| 1431. | ME63F2-HCDR3 | artificial | aa | YGSYVSPLDY |
| 1432. | ME63F2-H | artificial | nt | CAGGTGCAGCTGGTCCAGTCTGGGGCTGAAGTGAAGAGGCCTGGGGCTTCAGTGAAAGTGTCCTGCAAGGCTTCGGGCTATACCTTCACCAGCTACTGGTTGCACTGGGTTAGACAGACGCCTGGACAAAGGCTTGAGTGGATAGGCATGATTGATCCTTCCAATAGTGACACTAGGTTTAATCCGAACTTCAAGGACAGGGTCACAATGACTGTAGACACATCTGCCAACACAGCCTACATGGAACTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCA |
| 1433. | ME63F2-L | artificial | aa | DIVMTQTPLSLPVTVGEQASISCKSSQSLLYTSSQKNYLAWYQQKPGQSPKLLIYWASTRESGVPDRFSGSGSGTDFTLKISRVEAEDLGVYYCQQYYAYPWTFGGGTKLEIK |
| 1434. | ME63F2-LCDR1 | artificial | aa | KSSQSLLYTSSQKNYLA |
| 1435. | ME63F2-LCDR2 | artificial | aa | WASTRES |
| 1436. | ME63F2-LCDR3 | artificial | aa | QQYYAYPWT |
| 1437. | ME63F2-L | artificial | nt | GACATCGTGATGACCCAGACTCCACTCTCCCTACCTGTGACAGTTGGAGAGCAGGCTTCTATCAGCTGCAAGTCCAGTCAGTCCCTTTTATATACTAGCAGTCAGAAGAACTACTTGGCCTGGTACCAGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCAAAATCTCCAGAGTGGAGGCTGAGGACCTGGGAGTTTATTACTGTCAGCAATATTATGCCTATCCGTGGACGTTCGGTGGAGGCACAAAGTTGGAGATCAAA |
| 1438. | ME63F2-HL | artificial | aa | QVQLVQSGAEVKRPGASVKVSCKASGYTFTSYWLHWVRQTPGQRLEWIGMIDPSNSDTRFNPNFKDRVTMTVDTSANTAYMELSSLRSEDTAVYYCATYGSYVSPLDYWGQGTS |

| | | | | |
|---|---|---|---|---|
| | | | | VTVSSGGGGSGGGGSGGGGSDIVMTQTPLSLPVTVGEQASISCKSSQSLLYTSSQKNY<br>LAWYQQKPGQSPKLLIYWASTRESGVPDRFSGSGSGTDFTLKISRVEAEDLGVYYCQQ<br>YYAYPWTFGGGTKLEIK |
| 1439. | ME63F2-HL | artificial | nt | CAGGTGCAGCTGGTCCAGTCTGGGGCTGAAGTGAAGAGGCCTGGGGCTTCAGTGA<br>AAGTGTCCTGCAAGGCTTCGGGCTATACCTTCACCAGCTACTGGTTGCACTGGGTT<br>AGACAGACGCCTGGACAAAGGCTTGAGTGGATAGGCATGATTGATCCTTCCAATAG<br>TGACACTAGGTTTAATCCGAACTTCAAGGACAGGGTCACAATGACTGTAGACACATC<br>TGCCAACACAGCCTACATGGAACTCAGCAGCCTGAGATCTGAGGACACTGCAGTCT<br>ATTACTGTGCCACATATGGTAGCTACGTTTCCCCTCTGGACTACTGGGGTCAAGGA<br>ACCTCGGTCACCGTCTCCTCAGGTGGTGGTGGTTCTGGCGGCGGCGGCTCCGGTG<br>GTGGTGGTTCTGACATCGTGATGACCCAGACTCCACTCTCCCTACCTGTGACAGTT<br>GGAGAGCAGGCTTCTATCAGCTGCAAGTCCAGTCAGTCCCTTTTATATACTAGCAGT<br>CAGAAGAACTACTTGGCCTGGTACCAGCAGAAACCAGGTCAGTCTCCTAAACTGCT<br>GATTTACTGGGCATCCACTAGGGAATCTGGGGTCCCTGATCGCTTCTCAGGCAGTG<br>GATCTGGGACAGATTTCACTCTCAAAATCTCCAGAGTGGAGGCTGAGGACCTGGGA<br>GTTTATTACTGTCAGCAATATTATGCCTATCCGTGGACGTTCGGTGGAGGCACAAAG<br>TTGGAGATCAAA |
| 1440. | ME63F2 HL x I2C HL | artificial | aa | QVQLVQSGAEVKRPGASVKVSCKASGYTFTSYWLHWVRQTPGQRLEWIGMIDPSNSD<br>TRFNPNFKDRVTMTVDTSANTAYMELSSLRSEDTAVYYCATYGSYVSPLDYWGQGTS<br>VTVSSGGGGSGGGGSGGGGSDIVMTQTPLSLPVTVGEQASISCKSSQSLLYTSSQKNY<br>LAWYQQKPGQSPKLLIYWASTRESGVPDRFSGSGSGTDFTLKISRVEAEDLGVYYCQQ<br>YYAYPWTFGGGTKLEIKSGGGGSEVQLVESGGGLVQPGGSLKLSCAASGFTFNKYAM<br>NWVRQAPGKGLEWVARIRSKYNNYATYYADSVKDRFTISRDDSKNTAYLQMNNLKTED<br>TAVYYCVRHGNFGNSYISYWAYWGQGTLVTVSSGGGGSGGGGSGGGGSQTVVTQE<br>PSLTVSPGGTVTLTCGSSTGAVTSGNYPNWVQQKPGQAPRGLIGGTKFLAPGTPARFS<br>GSLLGGKAALTLSGVQPEDEAEYYCVLWYSNRWVFGGGTKLTVL |
| 1441. | ME63F2 HL x I2C HL | artificial | nt | CAGGTGCAGCTGGTCCAGTCTGGGGCTGAAGTGAAGAGGCCTGGGGCTTCAGTGA<br>AAGTGTCCTGCAAGGCTTCGGGCTATACCTTCACCAGCTACTGGTTGCACTGGGTT<br>AGACAGACGCCTGGACAAAGGCTTGAGTGGATAGGCATGATTGATCCTTCCAATAG<br>TGACACTAGGTTTAATCCGAACTTCAAGGACAGGGTCACAATGACTGTAGACACATC<br>TGCCAACACAGCCTACATGGAACTCAGCAGCCTGAGATCTGAGGACACTGCAGTCT<br>ATTACTGTGCCACATATGGTAGCTACGTTTCCCCTCTGGACTACTGGGGTCAAGGA<br>ACCTCGGTCACCGTCTCCTCAGGTGGTGGTGGTTCTGGCGGCGGCGGCTCCGGTG<br>GTGGTGGTTCTGACATCGTGATGACCCAGACTCCACTCTCCCTACCTGTGACAGTT<br>GGAGAGCAGGCTTCTATCAGCTGCAAGTCCAGTCAGTCCCTTTTATATACTAGCAGT<br>CAGAAGAACTACTTGGCCTGGTACCAGCAGAAACCAGGTCAGTCTCCTAAACTGCT |

| | | | | |
|---|---|---|---|---|
| | | | | GATTTACTGGGCATCCACTAGGGAATCTGGGGTCCCTGATCGCTTCTCAGGCAGTGGATCTGGGACAGATTTCACTCTCAAAATCTCCAGAGTGGAGGCTGAGGACCTGGGAGTTTATTACTGTCAGCAATATTATGCCTATCCGTGGACGTTCGGTGGAGGCACAAAGTTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1442. | ME62D11-H | artificial | aa | QVQLVQSGPEVVKPGASVKVSCKASGYTFTSYWLHWVKQAPGQGLEWIGMIDPSNSDTRFNPNFKDRATLTVDTSASTAYMLLSSLRSEDTAVYYCATYGSYVSPLDYWGQGTSVTVSS |
| 1443. | ME62D11-HCDR1 | artificial | aa | SYWLH |
| 1444. | ME62D11-HCDR2 | artificial | aa | MIDPSNSDTRFNPNFKD |
| 1445. | ME62D11-HCDR3 | artificial | aa | YGSYVSPLDY |
| 1446. | ME62D11-H | artificial | nt | CAGGTGCAGCTGGTCCAGTCTGGGCCTGAAGTGGTGAAGCCTGGGGCTTCAGTGAAAGTGTCCTGCAAGGCTTCGGGCTATACCTTCACCAGCTACTGGTTGCACTGGGTTAAACAGGCGCCTGGACAAGGGCTTGAGTGGATAGGCATGATTGATCCTTCCAATAGTGACACTAGGTTTAATCCGAACTTCAAGGACAGGGCCACATTGACTGTAGACACATCTGCCAGCACAGCCTACATGCTACTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCA |
| 1447. | ME62D11-L | artificial | aa | DIVMTQTPSSLPVTLGETVSISCKSSQSLLYTSSQKNYLAWYLQKPGQSPQLLIYWASTRESGVPDRFSGSGSGTDFTLTISRVKAEDVAVYYCQQYYAYPWTFGGGTKLEIK |
| 1448. | ME62D11-LCDR1 | artificial | aa | KSSQSLLYTSSQKNYLA |
| 1449. | ME62D11-LCDR2 | artificial | aa | WASTRES |
| 1450. | ME62D11-LCDR3 | artificial | aa | QQYYAYPWT |
| 1451. | ME62D11-L | artificial | nt | GACATCGTGATGACCCAGACTCCATCCTCCCTACCTGTGACACTTGGAGAGACGGT |

| | | | | |
|---|---|---|---|---|
| | | | | TTCTATCAGCTGCAAGTCCAGTCAGTCCCTTTTATATACTAGCAGTCAGAAGAACTACTTGGCCTGGTACCTGCAGAAACCAGGTCAGTCTCCTCAACTGCTGATTTACTGGGCATCCACTAGGGAATCTGGGGTCCCTGATCGCTTCTCAGGCAGTGGATCTGGGACAGATTTCACTCTCACAATCTCCAGAGTGAAGGCTGAGGACGTGGCAGTTTATTACTGTCAGCAATATTATGCCTATCCGTGGACGTTCGGTGGAGGCACAAAGTTGGAGATCAAA |
| 1452. | ME62D11-HL | artificial | aa | QVQLVQSGPEVVKPGASVKVSCKASGYTFTSYWLHWVKQAPGQGLEWIGMIDPSNSDTRFNPNFKDRATLTVDTSASTAYMLLSSLRSEDTAVYYCATYGSYVSPLDYWGQGTSVTVSSGGGGSGGGGSGGGGSDIVMTQTPSSLPVTLGETVSISCKSSQSLLYTSSQKNYLAWYLQKPGQSPQLLIYWASTRESGVPDRFSGSGSGTDFTLTISRVKAEDVAVYYCQQYYAYPWTFGGGTKLEIK |
| 1453. | ME62D11-HL | artificial | nt | CAGGTGCAGCTGGTCCAGTCTGGGCCTGAAGTGGTGAAGCCTGGGGCTTCAGTGAAAGTGTCCTGCAAGGCTTCGGGCTATACCTTCACCAGCTACTGGTTGCACTGGGTTAAACAGGCGCCTGGACAAGGGCTTGAGTGGATAGGCATGATTGATCCTTCCAATAGTGACACTAGGTTTAATCCGAACTTCAAGGACAGGGCCACATTGACTGTAGACACATCTGCCAGCACAGCCTACATGCTACTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCATCCTCCCTACCTGTGACACTTGGAGAGACGGTTTCTATCAGCTGCAAGTCCAGTCAGTCCCTTTTATATACTAGCAGTCAGAAGAACTACTTGGCCTGGTACCTGCAGAAACCAGGTCAGTCTCCTCAACTGCTGATTTACTGGGCATCCACTAGGGAATCTGGGGTCCCTGATCGCTTCTCAGGCAGTGGATCTGGGACAGATTTCACTCTCACAATCTCCAGAGTGAAGGCTGAGGACGTGGCAGTTTATTACTGTCAGCAATATTATGCCTATCCGTGGACGTTCGGTGGAGGCACAAAGTTGGAGATCAAA |
| 1454. | ME62D11 HL x I2C HL | artificial | aa | QVQLVQSGPEVVKPGASVKVSCKASGYTFTSYWLHWVKQAPGQGLEWIGMIDPSNSDTRFNPNFKDRATLTVDTSASTAYMLLSSLRSEDTAVYYCATYGSYVSPLDYWGQGTSVTVSSGGGGSGGGGSGGGGSDIVMTQTPSSLPVTLGETVSISCKSSQSLLYTSSQKNYLAWYLQKPGQSPQLLIYWASTRESGVPDRFSGSGSGTDFTLTISRVKAEDVAVYYCQQYYAYPWTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1455. | ME62D11 HL x I2C HL | artificial | nt | CAGGTGCAGCTGGTCCAGTCTGGGCCTGAAGTGGTGAAGCCTGGGGCTTCAGTGAAAGTGTCCTGCAAGGCTTCGGGCTATACCTTCACCAGCTACTGGTTGCACTGGGTT |

| | | | | |
|---|---|---|---|---|
| | | | | AAACAGGCGCCTGGACAAGGGCTTGAGTGGATAGGCATGATTGATCCTTCCAATAG TGACACTAGGTTTAATCCGAACTTCAAGGACAGGGCCACATTGACTGTAGACACATC TGCCAGCACAGCCTACATGCTACTCAGCAGCCTGAGATCTGAGGACACTGCAGTCT ATTACTGTGCCACATATGGTAGCTACGTTTCCCCTCTGGACTACTGGGGTCAAGGA ACCTCGGTCACCGTCTCCTCAGGTGGTGGTGGTTCTGGCGGCGGCGGCTCCGGTG GTGGTGGTTCTGACATCGTGATGACCCAGACTCCATCCTCCCTACCTGTGACACTT GGAGAGACGGTTTCTATCAGCTGCAAGTCCAGTCAGTCCCTTTTATATACTAGCAGT CAGAAGAACTACTTGGCCTGGTACCTGCAGAAACCAGGTCAGTCTCCTCAACTGCT GATTTACTGGGCATCCACTAGGGAATCTGGGGTCCCTGATCGCTTCTCAGGCAGTG GATCTGGGACAGATTTCACTCTCACAATCTCCAGAGTGAAGGCTGAGGACGTGGCA GTTTATTACTGTCAGCAATATTATGCCTATCCGTGGACGTTCGGTGGAGGCACAAAG TTGGAGATCAAATCCGGAGGTGGTGGATCCGAGGTGCAGCTGGTCGAGTCTGGAG GAGGATTGGTGCAGCCTGGAGGGTCATTGAAACTCTCATGTGCAGCCTCTGGATTC ACCTTCAATAAGTACGCCATGAACTGGGTCCGCCAGGCTCCAGGAAAGGGTTTGGA ATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAA ATGAACAACTTGAAAACTGAGGACACTGCCGTGTACTACTGTGTGAGACATGGGAA CTTCGGTAATAGCTACATATCCTACTGGGCTTACTGGGGCCAAGGGACTCTGGTCA CCGTCTCCTCAGGTGGTGGTGGTTCTGGCGGCGGCGGCTCCGGTGGTGGTGGTTC TCAGACTGTTGTGACTCAGGAACCTTCACTCACCGTATCACCTGGTGGAACAGTCA CACTCACTTGTGGCTCCTCGACTGGGGCTGTTACATCTGGCAACTACCCAAACTGG GTCCAACAAAAACCAGGTCAGGCACCCCGTGGTCTAATAGGTGGGACTAAGTTCCT CGCCCCCGGTACTCCTGCCAGATTCTCAGGCTCCCTGCTTGGAGGCAAGGCTGCC CTCACCCTCTCAGGGGTACAGCCAGAGGATGAGGCAGAATATTACTGTGTTCTATG GTACAGCAACCGCTGGGTGTTCGGTGGAGGAACCAAACTGACTGTCCTA |
| 1456. | ME62C10-H | artificial | aa | QVQLVQSGAELKKPGASVKVSCKASGYTFTSYWLHWVKQAPGQRLEWIGMIDPSNSD TRFNPNFKDRVTITVDTSANTAYMELSSLRSEDTAVYYCATYGSYVSPLDYWGQGTSV TVSS |
| 1457. | ME62C10-HCDR1 | artificial | aa | SYWLH |
| 1458. | ME62C10-HCDR2 | artificial | aa | MIDPSNSDTRFNPNFKD |
| 1459. | ME62C10-HCDR3 | artificial | aa | YGSYVSPLDY |
| 1460. | ME62C10-H | artificial | nt | CAGGTGCAGCTGGTCCAGTCTGGGGCTGAACTGAAGAAGCCTGGGGCTTCAGTGA AAGTGTCCTGCAAGGCTTCGGGCTATACCTTCACCAGCTACTGGTTGCACTGGGTT AAACAGGCGCCTGGACAAAGGCTTGAGTGGATAGGCATGATTGATCCTTCCAATAG TGACACTAGGTTTAATCCGAACTTCAAGGACAGGGTCACAATCACTGTAGACACATC TGCCAACACAGCCTACATGGAGCTCAGCAGCCTGAGATCTGAGGACACTGCAGTCT |

| | | | | |
|---|---|---|---|---|
| | | | | ATTACTGTGCCACATATGGTAGCTACGTTTCCCCTCTGGACTACTGGGGTCAAGGAACCTCGGTCACCGTCTCCTCA |
| 1461. | ME62C10-L | artificial | aa | DIVMTQTPPSLTVTPGEQVSISCKSSQSLLYTSSQKNYLAWYLQKPGQSPKLLIYWASTRESGVPDRFSGSGSGTDFTLKISSVEADDVGVYYCQQYYAYPWTFGGGTKLEIK |
| 1462. | ME62C10-LCDR1 | artificial | aa | KSSQSLLYTSSQKNYLA |
| 1463. | ME62C10-LCDR2 | artificial | aa | WASTRES |
| 1464. | ME62C10-LCDR3 | artificial | aa | QQYYAYPWT |
| 1465. | ME62C10-L | artificial | nt | GACATCGTGATGACCCAGACTCCACCCTCCCTAACTGTGACACCTGGAGAGCAGGTTTCTATCAGCTGCAAGTCCAGTCAGTCCCTTTTATATACTAGCAGTCAGAAGAACTACTTGGCCTGGTACCTGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCAAAATCTCCAGTGTGGAGGCTGACGACGTGGGAGTTTATTACTGTCAGCAATATTATGCCTATCCGTGGACGTTCGGTGGAGGCACAAAGTTGGAGATCAAA |
| 1466. | ME62C10-HL | artificial | aa | QVQLVQSGAELKKPGASVKVSCKASGYTFTSYWLHWVKQAPGQRLEWIGMIDPSNSDTRFNPNFKDRVTITVDTSANTAYMELSSLRSEDTAVYYCATYGSYVSPLDYWGQGTSVTVSSGGGGSGGGGSGGGGSDIVMTQTPPSLTVTPGEQVSISCKSSQSLLYTSSQKNYLAWYLQKPGQSPKLLIYWASTRESGVPDRFSGSGSGTDFTLKISSVEADDVGVYYCQQYYAYPWTFGGGTKLEIK |
| 1467. | ME62C10-HL | artificial | nt | CAGGTGCAGCTGGTCCAGTCTGGGGCTGAACTGAAGAAGCCTGGGGCTTCAGTGAAAGTGTCCTGCAAGGCTTCGGGCTATACCTTCACCAGCTACTGGTTGCACTGGGTTAAACAGGCGCCTGGACAAAGGCTTGAGTGGATAGGCATGATTGATCCTTCCAATAGTGACACTAGGTTTAATCCGAACTTCAAGGACAGGGTCACAATCACTGTAGACACATCTGCCAACACAGCCTACATGGAGCTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCACCCTCCCTAACTGTGACACCTGGAGAGCAGGTTTCTATCAGCTGCAAGTCCAGTCAGTCCCTTTTATATACTAGCAGTCAGAAGAACTACTTGGCCTGGTACCTGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCAAAATCTCCAGTGTGGAGGCTGACGACGTGGGAGTTTATTACTGTCAGCAATATTATGCCTATCCGTGGACGTTCGGTGGAGGCACAAAGTTGGAGATCAAA |
| 1468. | ME62C10 HL x I2C HL | artificial | aa | QVQLVQSGAELKKPGASVKVSCKASGYTFTSYWLHWVKQAPGQRLEWIGMIDPSNSDTRFNPNFKDRVTITVDTSANTAYMELSSLRSEDTAVYYCATYGSYVSPLDYWGQGTSVTVSSGGGGSGGGGSGGGGSDIVMTQTPPSLTVTPGEQVSISCKSSQSLLYTSSQKNYL |

| | | | | |
|---|---|---|---|---|
| | | | | AWYLQKPGQSPKLLIYWASTRESGVPDRFSGSGSGTDFTLKISSVEADDVGVYYCQQYYAYPWTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1469. | ME62C10 HL x I2C HL | artificial | nt | CAGGTGCAGCTGGTCCAGTCTGGGGCTGAACTGAAGAAGCCTGGGGCTTCAGTGAAAGTGTCCTGCAAGGCTTCGGGCTATACCTTCACCAGCTACTGGTTGCACTGGGTTAAACAGGCGCCTGGACAAAGGCTTGAGTGGATAGGCATGATTGATCCTTCCAATAGTGACACTAGGTTTAATCCGAACTTCAAGGACAGGGTCACAATCACTGTAGACACATCTGCCAACACAGCCTACATGGAGCTCAGCAGCCTGAGATCTGAGGACACTGCAGTCTATTACTGTGCCACATATGGTAGCTACGTTTCCCCTCTGGACTACTGGGGTCAAGGAACCTCGGTCACCGTCTCCTCAGGTGGTGGTGGTTCTGGCGGCGGCGGCTCCGGTGGTGGTGGTTCTGACATCGTGATGACCCAGACTCCACCCTCCCTAACTGTGACACCTGGAGAGCAGGTTTCTATCAGCTGCAAGTCCAGTCAGTCCCTTTTATATACTAGCAGTCAGAAGAACTACTTGGCCTGGTACCTGCAGAAACCAGGTCAGTCTCCTAAACTGCTGATTTACTGGGCATCCACTAGGGAATCTGGGGTCCCTGATCGCTTCTCAGGCAGTGGATCTGGGACAGATTTCACTCTCAAAATCTCCAGTGTGGAGGCTGACGACGTGGGAGTTTATTACTGTCAGCAATATTATGCCTATCCGTGGACGTTCGGTGGAGGCACAAAGTTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1470. | ME62A4-H | artificial | aa | QVQLVQSGAEVMKPGASVKVSCRASGYTFTSYWLHWVRQAPGQGLEWIGMIDPSNSDTRFNPNFKDRVTMTVDTSSSTAYMELSSLRSEDTAVYYCATYGSYVSPLDYWGQGTSVTVSS |

| | | | | |
|---|---|---|---|---|
| 1471. | ME62A4-HCDR1 | artificial | aa | SYWLH |
| 1472. | ME62A4-HCDR2 | artificial | aa | MIDPSNSDTRFNPNFKD |
| 1473. | ME62A4-HCDR3 | artificial | aa | YGSYVSPLDY |
| 1474. | ME62A4-H | artificial | nt | CAGGTGCAGCTGGTCCAGTCTGGGGCTGAAGTGATGAAGCCTGGGGCTTCAGTGA AAGTGTCCTGCAGGGCTTCGGGCTATACCTTCACCAGCTACTGGTTGCACTGGGTT AGACAGGCGCCTGGACAAGGGCTTGAGTGGATAGGCATGATTGATCCTTCCAATAG TGACACTAGGTTTAATCCGAACTTCAAGGACAGGGTCACAATGACTGTAGACACATC TTCCAGCACAGCCTACATGGAGCTCAGCAGCCTGAGATCTGAGGACACTGCAGTCT ATTACTGTGCCACATATGGTAGCTACGTTTCCCCTCTGGACTACTGGGGTCAAGGA ACCTCGGTCACCGTCTCCTCA |
| 1475. | ME62A4-L | artificial | aa | DIVMTQTPFSLPVTAGETVSISCKSSQSLLYTSSQKNYLAWYLQKPGQSPQLLIYWAST RESGVPDRFSGSGSGTDFTLKISRVEAEDVGVYYCQQYYAYPWTFGGGTKLEIK |
| 1476. | ME62A4-LCDR1 | artificial | aa | KSSQSLLYTSSQKNYLA |
| 1477. | ME62A4-LCDR2 | artificial | aa | WASTRES |
| 1478. | ME62A4-LCDR3 | artificial | aa | QQYYAYPWT |
| 1479. | ME62A4-L | artificial | nt | GACATCGTGATGACCCAGACTCCATTCTCCCTACCTGTGACAGCTGGAGAGACGGT TTCTATCAGCTGCAAGTCCAGTCAGTCCCTTTTATATACTAGCAGTCAGAAGAACTA CTTGGCCTGGTACCTGCAGAAACCAGGTCAGTCTCCTCAACTGCTGATTTACTGGG CATCCACTAGGGAATCTGGGGTCCCTGATCGCTTCTCAGGCAGTGGATCTGGGACA GATTTCACTCTCAAAATCTCCAGAGTGGAGGCTGAGGACGTGGGAGTTTATTACTGT CAGCAATATTATGCCTATCCGTGGACGTTCGGTGGAGGCACAAAGTTGGAGATCAA A |
| 1480. | ME62A4-HL | artificial | aa | QVQLVQSGAEVMKPGASVKVSCRASGYTFTSYWLHWVRQAPGQGLEWIGMIDPSNS DTRFNPNFKDRVTMTVDTSSSTAYMELSSLRSEDTAVYYCATYGSYVSPLDYWGQGT SVTVSSGGGGSGGGGSGGGGSDIVMTQTPFSLPVTAGETVSISCKSSQSLLYTSSQKN YLAWYLQKPGQSPQLLIYWASTRESGVPDRFSGSGSGTDFTLKISRVEAEDVGVYYCQ QYYAYPWTFGGGTKLEIK |
| 1481. | ME62A4-HL | artificial | nt | CAGGTGCAGCTGGTCCAGTCTGGGGCTGAAGTGATGAAGCCTGGGGCTTCAGTGA AAGTGTCCTGCAGGGCTTCGGGCTATACCTTCACCAGCTACTGGTTGCACTGGGTT AGACAGGCGCCTGGACAAGGGCTTGAGTGGATAGGCATGATTGATCCTTCCAATAG TGACACTAGGTTTAATCCGAACTTCAAGGACAGGGTCACAATGACTGTAGACACATC TTCCAGCACAGCCTACATGGAGCTCAGCAGCCTGAGATCTGAGGACACTGCAGTCT ATTACTGTGCCACATATGGTAGCTACGTTTCCCCTCTGGACTACTGGGGTCAAGGA ACCTCGGTCACCGTCTCCTCAGGTGGTGGTGGTTCTGGCGGCGGCGGCTCCGGTG GTGGTGGTTCTGACATCGTGATGACCCAGACTCCATTCTCCCTACCTGTGACAGCT GGAGAGACGGTTTCTATCAGCTGCAAGTCCAGTCAGTCCCTTTTATATACTAGCAGT |

| | | | | |
|---|---|---|---|---|
| | | | | CAGAAGAACTACTTGGCCTGGTACCTGCAGAAACCAGGTCAGTCTCCTCAACTGCT<br>GATTTACTGGGCATCCACTAGGGAATCTGGGGTCCCTGATCGCTTCTCAGGCAGTG<br>GATCTGGGACAGATTTCACTCTCAAAATCTCCAGAGTGGAGGCTGAGGACGTGGGA<br>GTTTATTACTGTCAGCAATATTATGCCTATCCGTGGACGTTCGGTGGAGGCACAAAG<br>TTGGAGATCAAA |
| 1482. | ME62A4 HL x I2C HL | artificial | aa | QVQLVQSGAEVMKPGASVKVSCRASGYTFTSYWLHWVRQAPGQGLEWIGMIDPSNS<br>DTRFNPNFKDRVTMTVDTSSSTAYMELSSLRSEDTAVYYCATYGSYVSPLDYWGQGT<br>SVTVSSGGGGSGGGGSGGGGSDIVMTQTPFSLPVTAGETVSISCKSSQSLLYTSSQKN<br>YLAWYLQKPGQSPQLLIYWASTRESGVPDRFSGSGSGTDFTLKISRVEAEDVGVYYCQ<br>QYYAYPWTFGGGTKLEIKSGGGGSEVQLVESGGGLVQPGGSLKLSCAASGFTFNKYA<br>MNWVRQAPGKGLEWVARIRSKYNNYATYYADSVKDRFTISRDDSKNTAYLQMNNLKT<br>EDTAVYYCVRHGNFGNSYISYWAYWGQGTLVTVSSGGGGSGGGGSGGGGSQTVVT<br>QEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 1483. | ME62A4 HL x I2C HL | artificial | nt | CAGGTGCAGCTGGTCCAGTCTGGGGCTGAAGTGATGAAGCCTGGGGCTTCAGTGA<br>AAGTGTCCTGCAGGGCTTCGGGCTATACCTTCACCAGCTACTGGTTGCACTGGGTT<br>AGACAGGCGCCTGGACAAGGGCTTGAGTGGATAGGCATGATTGATCCTTCCAATAG<br>TGACACTAGGTTTAATCCGAACTTCAAGGACAGGGTCACAATGACTGTAGACACATC<br>TTCCAGCACAGCCTACATGGAGCTCAGCAGCCTGAGATCTGAGGACACTGCAGTCT<br>ATTACTGTGCCACATATGGTAGCTACGTTTCCCCTCTGGACTACTGGGGTCAAGGA<br>ACCTCGGTCACCGTCTCCTCAGGTGGTGGTGGTTCTGGCGGCGGCGGCTCCGGTG<br>GTGGTGGTTCTGACATCGTGATGACCCAGACTCCATTCTCCCTACCTGTGACAGCT<br>GGAGAGACGGTTTCTATCAGCTGCAAGTCCAGTCAGTCCCTTTTATATACTAGCAGT<br>CAGAAGAACTACTTGGCCTGGTACCTGCAGAAACCAGGTCAGTCTCCTCAACTGCT<br>GATTTACTGGGCATCCACTAGGGAATCTGGGGTCCCTGATCGCTTCTCAGGCAGTG<br>GATCTGGGACAGATTTCACTCTCAAAATCTCCAGAGTGGAGGCTGAGGACGTGGGA<br>GTTTATTACTGTCAGCAATATTATGCCTATCCGTGGACGTTCGGTGGAGGCACAAAG<br>TTGGAGATCAAATCCGGAGGTGGTGGATCCGAGGTGCAGCTGGTCGAGTCTGGAG<br>GAGGATTGGTGCAGCCTGGAGGGTCATTGAAACTCTCATGTGCAGCCTCTGGATTC<br>ACCTTCAATAAGTACGCCATGAACTGGGTCCGCCAGGCTCCAGGAAAGGGTTTGGA<br>ATGGGTTGCTCGCATAAGAAGTAAATATAATAATTATGCAACATATTATGCCGATTCA<br>GTGAAAGACAGGTTCACCATCTCCAGAGATGATTCAAAAAACACTGCCTATCTACAA<br>ATGAACAACTTGAAAACTGAGGACACTGCCGTGTACTACTGTGTGAGACATGGGAA<br>CTTCGGTAATAGCTACATATCCTACTGGGCTTACTGGGGCCAAGGGACTCTGGTCA<br>CCGTCTCCTCAGGTGGTGGTGGTTCTGGCGGCGGCGGCTCCGGTGGTGGTGGTTC<br>TCAGACTGTTGTGACTCAGGAACCTTCACTCACCGTATCACCTGGTGGAACAGTCA |

| | | | | |
|---|---|---|---|---|
| | | | | CACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1484. | ME75H6-H | murine | aa | EVQLLEQSGAELVRPGTSVKMSCKAAGYTFTKYWIGWVKQRPGHGLEWIGDIHPGGGYANYNEKFKGKATLTADTSSSTAYMQLSRLTSDDSAIYYCARSGYDYGSSYDYHGMDFWGQGTTVTVSS |
| 1485. | ME75H6-HCDR1 | murine | aa | KYWIG |
| 1486. | ME75H6-HCDR2 | murine | aa | DIHPGGGYANYNEKFKG |
| 1487. | ME75H6-HCDR3 | murine | aa | SGYDYGSSYDYHGMDF |
| 1488. | ME75H6-H | murine | nt | GAGGTGCAGCTGCTCGAGCAGTCTGGAGCTGAGCTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTATGCTAACTACAATGAGAAGTTCAAGGGCAAGGCCACACTGACTGCAGACACATCCTCCAGCACAGCCTACATGCAGCTCAGCAGACTGACATCTGATGACTCCGCCATCTATTACTGTGCAAGATCGGGGTATGACTACGGTAGTAGTTACGATTACCATGGTATGGACTTCTGGGGCCAAGGGACCACGGTCACCGTCTCCTCA |
| 1489. | ME75H6-L | murine | aa | ELQMTQSPASLSASVGEPVTFTCRASENIYSYLAWYQQKQGKSPQLLIYGATNLADGMSSRFSGSGSGRQYSLKISSLHPDDVATYYCQNVLSTPYTFGGGTKLEIK |
| 1490. | ME75H6-LCDR1 | murine | aa | RASENIYSYLA |
| 1491. | ME75H6-LCDR2 | murine | aa | GATNLAD |
| 1492. | ME75H6-LCDR3 | murine | aa | QNVLSTPYT |
| 1493. | ME75H6-L | murine | nt | GAGCTCCAGATGACCCAGTCTCCAGCCTCCCTATCTGCATCTGTGGGAGAACCTGTCACCTTCACATGTCGAGCAAGTGAGAATATTTACAGTTATTTAGCATGGTATCAGCAGAAACAGGGAAAATCTCCTCAGCTCCTGATCTATGGTGCAACCAACTTGGCAGATGGCATGTCATCGAGGTTCAGTGGCAGTGGATCTGGTAGACAGTATTCTCTCAAGATCAGTAGCCTGCATCCTGACGATGTTGCAACGTATTACTGTCAAAATGTGTTAAGTACTCCGTACACGTTCGGAGGGGGGACCAAGCTTGAGATCAAA |
| 1494. | ME75H6-HL | murine | aa | EVQLLEQSGAELVRPGTSVKMSCKAAGYTFTKYWIGWVKQRPGHGLEWIGDIHPGGGYANYNEKFKGKATLTADTSSSTAYMQLSRLTSDDSAIYYCARSGYDYGSSYDYHGMDFWGQGTTVTVSSGGGGSGGGGSGGGGSELQMTQSPASLSASVGEPVTFTCRASENIYSYLAWYQQKQGKSPQLLIYGATNLADGMSSRFSGSGSGRQYSLKISSLHPDDVATYYCQNVLSTPYTFGGGTKLEIK |
| 1495. | ME75H6-HL | murine | nt | GAGGTGCAGCTGCTCGAGCAGTCTGGAGCTGAGCTGGTTAGGCCTGGGACTTCAG |

| | | | | |
|---|---|---|---|---|
| | | | | TGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTATGCTAACTACAATGAGAAGTTCAAGGGCAAGGCCACACTGACTGCAGACACATCCTCCAGCACAGCCTACATGCAGCTCAGCAGACTGACATCTGATGACTCCGCCATCTATTACTGTGCAAGATCGGGGTATGACTACGGTAGTAGTTACGATTACCATGGTATGGACTTCTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAACCTGTCACCTTCACATGTCGAGCAAGTGAGAATATTTACAGTTATTTAGCATGGTATCAGCAGAAACAGGGAAAATCTCCTCAGCTCCTGATCTATGGTGCAACCAACTTGGCAGATGGCATGTCATCGAGGTTCAGTGGCAGTGGATCTGGTAGACAGTATTCTCTCAAGATCAGTAGCCTGCATCCTGACGATGTTGCAACGTATTACTGTCAAAATGTGTTAAGTACTCCGTACACGTTCGGAGGGGGGACCAAGCTTGAGATCAAA |
| 1496. | ME75H6 HL x I2C HL | murine | aa | EVQLLEQSGAELVRPGTSVKMSCKAAGYTFTKYWIGWVKQRPGHGLEWIGDIHPGGGYANYNEKFKGKATLTADTSSSTAYMQLSRLTSDDSAIYYCARSGYDYGSSYDYHGMDFWGQGTTVTVSSGGGGSGGGGSGGGGSELQMTQSPASLSASVGEPVTFTCRASENIYSYLAWYQQKQGKSPQLLIYGATNLADGMSSRFSGSGSGRQYSLKISSLHPDDVATYYCQNVLSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1497. | ME75H6 HL x I2C HL | murine | nt | GAGGTGCAGCTGCTCGAGCAGTCTGGAGCTGAGCTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTATGCTAACTACAATGAGAAGTTCAAGGGCAAGGCCACACTGACTGCAGACACATCCTCCAGCACAGCCTACATGCAGCTCAGCAGACTGACATCTGATGACTCCGCCATCTATTACTGTGCAAGATCGGGGTATGACTACGGTAGTAGTTACGATTACCATGGTATGGACTTCTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAACCTGTCACCTTCACATGTCGAGCAAGTGAGAATATTTACAGTTATTTAGCATGGTATCAGCAGAAACAGGGAAAATCTCCTCAGCTCCTGATCTATGGTGCAACCAACTTGGCAGATGGCATGTCATCGAGGTTCAGTGGCAGTGGATCTGGTAGACAGTATTCTCTCAAGATCAGTAGCCTGCATCCTGACGATGTTGCAACGTATTACTGTCAAAATGTGTTAAGTACTCCGTACACGTTCGGAGGGGGGACCAAGCTTGAGATCAAATCCGGAGGTGGTGGATCCGAGGTGCAGCTGGTCGAGTC |

| | | | | |
|---|---|---|---|---|
| | | | | TGGAGGAGGATTGGTGCAGCCTGGAGGGTCATTGAAACTCTCATGTGCAGCCTCTG<br>GATTCACCTTCAATAAGTACGCCATGAACTGGGTCCGCCAGGCTCCAGGAAAGGGT<br>TTGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAA<br>ACTGGGTCCAACAAAAACCAGGTCAGGCACCCCGTGGTCTAATAGGTGGGACTAAG<br>TTCCTCGCCCCCGGTACTCCTGCCAGATTCTCAGGCTCCCTGCTTGGAGGCAAGGC<br>TGCCCTCACCCTCTCAGGGGTACAGCCAGAGGATGAGGCAGAATATTACTGTGTTC<br>TATGGTACAGCAACCGCTGGGTGTTCGGTGGAGGAACCAAACTGACTGTCCTA |

| SEQ ID NO. | DESIGNATION | SOURCE | TYPE | SEQUENCE |
|---|---|---|---|---|
| 1498. | ME99B1-H | murine | aa | EVQLLEQSGAELVRPGTSVKISCKATGYTFSNYWISWVKYRPGHGLEWIGDIYPGGSYTNYNEKFKGKVTLTADTSSRTAYMQLSGLTFEDSAIYYCARSGYDYGSDYDYHGMDYWGQGTTVTVSSG |
| 1499. | ME99B1-HCDR1 | murine | aa | NYWIS |
| 1500. | ME99B1-HCDR2 | murine | aa | DIYPGGSYTNYNEKFKG |
| 1501. | ME99B1-HCDR3 | murine | aa | SGYDYGSDYDYHGMDY |
| 1502. | ME99B1-H | murine | nt | GAGGTGCAGCTGCTCGAGCAGTCTGGAGCTGAGCTGGTAAGGCCTGGGACTTCAGTGAAGATTTCCTGCAAGGCTACTGGGTACACCTTCAGTAATTACTGGATAAGTTGGGTAAAGTATAGGCCTGGACATGGTCTTGAGTGGATTGGAGATATTTACCCTGGAGGCAGTTATACTAATTACAATGAGAAATTCAAGGGCAAGGTCACACTGACTGCAGACACATCCTCCAGGACAGCCTACATGCAGCTCAGCGGCCTGACATTTGAGGACTCTGCCATCTACTACTGTGCAAGATCGGGGTATGACTACGGTAGTGACTACGATTACCATGGTATGGACTACTGGGGCCAAGGGACCACGGTCACCGTCTCCTCA |
| 1503. | ME99B1-L | murine | aa | ELQMTQSPASLSASVGETVTITCRASENIYSNLAWYQQKQGKSPQLLVYAATNLADGVPSRFSGSRSGSDYSLTISSLESEDFVDYYCLQYASYPYTFGGGTKLEIK |
| 1504. | ME99B1-LCDR1 | murine | aa | RASENIYSNLA |
| 1505. | ME99B1-LCDR2 | murine | aa | AATNLAD |
| 1506. | ME99B1-LCDR3 | murine | aa | LQYASYPYT |
| 1507. | ME99B1-L | murine | nt | GAGCTCCAGATGACCCAGTCTCCAGCCTCCCTATCTGCATCTGTGGGAGAAACTGTCACCATCACATGTCGAGCAAGTGAGAATATTTACAGTAATTTAGCATGGTATCAGCAGAAACAGGGAAAATCTCCTCAGCTCCTGGTCTATGCTGCAACAAACTTAGCAGATGGTGTGCCATCAAGGTTCAGTGGCAGTAGGTCTGGGTCAGATTATTCTCTCACCATCAGCAGCCTTGAGTCTGAAGATTTTGTAGACTATTACTGTCTACAATATGCTAGTTATCCGTACACGTTCGGAGGGGGGACCAAGCTTGAGATCAAA |
| 1508. | ME99B1-HL | murine | aa | EVQLLEQSGAELVRPGTSVKISCKATGYTFSNYWISWVKYRPGHGLEWIGDIYPGGSYTNYNEKFKGKVTLTADTSSRTAYMQLSGLTFEDSAIYYCARSGYDYGSDYDYHGMDYWGQGTTVTVSSGGGGSGGGGSGGGGSELQMTQSPASLSASVGETVTITCRASENIYSNLAWYQQKQGKSPQLLVYAATNLADGVPSRFSGSRSGSDYSLTISSLESEDFVDYYCLQYASYPYTFGGGTKLEIK |
| 1509. | ME99B1-HL | murine | nt | GAGGTGCAGCTGCTCGAGCAGTCTGGAGCTGAGCTGGTAAGGCCTGGGACTTCAGTGAAGATTTCCTGCAAGGCTACTGGGTACACCTTCAGTAATTACTGGATAAGTTGGGTAAAGTATAGGCCTGGACATGGTCTTGAGTGGATTGGAGATATTTACCCTGGAGGCAGTTATACTAATTACAATGAGAAATTCAAGGGCAAGGTCACACTGACTGCAGACACATCCTCCAGGACAGCCTACATGCAGCTCAGCGGCCTGACATTTGAGGACTCTGCCATCTACTACTGTGCAAGATCGGGGTATGACTACGGTAGTGACTACGATTACCATGGTAT |

| | | | | |
|---|---|---|---|---|
| | | | | GGACTACTGGGGCCAAGGGACCACGGTCACCGTCTCCTCAGGTGGTGGTGGTTCT GGCGGCGGCGGCTCCGGTGGTGGTGGTTCTGAGCTCCAGATGACCCAGTCTCCAG CCTCCCTATCTGCATCTGTGGGAGAAACTGTCACCATCACATGTCGAGCAAGTGAG AATATTTACAGTAATTTAGCATGGTATCAGCAGAAACAGGGAAAATCTCCTCAGCTC CTGGTCTATGCTGCAACAAACTTAGCAGATGGTGTGCCATCAAGGTTCAGTGGCAG TAGGTCTGGGTCAGATTATTCTCTCACCATCAGCAGCCTTGAGTCTGAAGATTTTGT AGACTATTACTGTCTACAATATGCTAGTTATCCGTACACGTTCGGAGGGGGGACCAA GCTTGAGATCAAA |
| 1510. | ME99B1 HL x I2C HL | murine | aa | EVQLLEQSGAELVRPGTSVKISCKATGYTFSNYWISWVKYRPGHGLEWIGDIYPGGSYT NYNEKFKGKVTLTADTSSRTAYMQLSGLTFEDSAIYYCARSGYDYGSDYDYHGMDYW GQGTTVTVSSGGGGSGGGGSGGGGSELQMTQSPASLSASVGETVTITCRASENIYSN LAWYQQKQGKSPQLLVYAATNLADGVPSRFSGSRSGSDYSLTISSLESEDFVDYYCLQ YASYPYTFGGGTKLEIKSGGGGSEVQLVESGGGLVQPGGSLKLSCAASGFTFNKYAMN WVRQAPGKGLEWVARIRSKYNNYATYYADSVKDRFTISRDDSKNTAYLQMNNLKTEDT AVYYCVRHGNFGNSYISYWAYWGQGTLVTVSSGGGGSGGGGSGGGGSQTVVTQEP SLTVSPGGTVTLTCGSSTGAVTSGNYPNWVQQKPGQAPRGLIGGTKFLAPGTPARFSG SLLGGKAALTLSGVQPEDEAEYYCVLWYSNRWVFGGGTKLTVL |
| 1511. | ME99B1 HL x I2C HL | murine | nt | GAGGTGCAGCTGCTCGAGCAGTCTGGAGCTGAGCTGGTAAGGCCTGGGACTTCAG TGAAGATTTCCTGCAAGGCTACTGGGTACACCTTCAGTAATTACTGGATAAGTTGGG TAAAGTATAGGCCTGGACATGGTCTTGAGTGGATTGGAGATATTTACCCTGGAGGC AGTTATACTAATTACAATGAGAAATTCAAGGGCAAGGTCACACTGACTGCAGACACA TCCTCCAGGACAGCCTACATGCAGCTCAGCGGCCTGACATTTGAGGACTCTGCCAT CTACTACTGTGCAAGATCGGGGTATGACTACGGTAGTGACTACGATTACCATGGTAT GGACTACTGGGGCCAAGGGACCACGGTCACCGTCTCCTCAGGTGGTGGTGGTTCT GGCGGCGGCGGCTCCGGTGGTGGTGGTTCTGAGCTCCAGATGACCCAGTCTCCAG CCTCCCTATCTGCATCTGTGGGAGAAACTGTCACCATCACATGTCGAGCAAGTGAG AATATTTACAGTAATTTAGCATGGTATCAGCAGAAACAGGGAAAATCTCCTCAGCTC CTGGTCTATGCTGCAACAAACTTAGCAGATGGTGTGCCATCAAGGTTCAGTGGCAG TAGGTCTGGGTCAGATTATTCTCTCACCATCAGCAGCCTTGAGTCTGAAGATTTTGT AGACTATTACTGTCTACAATATGCTAGTTATCCGTACACGTTCGGAGGGGGGACCAA GCTTGAGATCAAATCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGG AATGGGTTGCTCGCATAAGAAGTAAATATAATAATTATGCAACATATTATGCCGATTC AGTGAAAGACAGGTTCACCATCTCCAGAGATGATTCAAAAAACACTGCCTATCTACA AATGAACAACTTGAAAACTGAGGACACTGCCGTGTACTACTGTGTGAGACATGGGA |

| | | | | |
|---|---|---|---|---|
| | | | | ACTTCGGTAATAGCTACATATCCTACTGGGCTTACTGGGGCCAAGGGACTCTGGTC ACCGTCTCCTCAGGTGGTGGTGGTTCTGGCGGCGGCGGCTCCGGTGGTGGTGGTT CTCAGACTGTTGTGACTCAGGAACCTTCACTCACCGTATCACCTGGTGGAACAGTC ACACTCACTTGTGGCTCCTCGACTGGGGCTGTTACATCTGGCAACTACCCAAACTG GGTCCAACAAAAACCAGGTCAGGCACCCCGTGGTCTAATAGGTGGGACTAAGTTCC TCGCCCCCGGTACTCCTGCCAGATTCTCAGGCTCCCTGCTTGGAGGCAAGGCTGC CCTCACCCTCTCAGGGGTACAGCCAGAGGATGAGGCAGAATATTACTGTGTTCTAT GGTACAGCAACCGCTGGGTGTTCGGTGGAGGAACCAAACTGACTGTCCTA |
| 1512. | ME05D7-H | murine | aa | EVQLLEQSGAELVRPGTSVKVSCKASGYAFTHYLIEWVKQRPGQGLEWIGMINPENTD TTYNEKFRDKAILTVDKSSNTAYMQLSSLTSDDSAVYFCARQEITSDFDFWGQGTTVTV SS |
| 1513. | ME05D7-HCDR1 | murine | aa | HYLIE |
| 1514. | ME05D7-HCDR2 | murine | aa | MINPENTDTTYNEKFRD |
| 1515. | ME05D7-HCDR3 | murine | aa | QEITSDFDF |
| 1516. | ME05D7-H | murine | nt | GAGGTGCAGCTGCTCGAGCAGTCTGGAGCTGAGCTGGTAAGGCCTGGGACTTCAG TGAAGGTGTCCTGCAAGGCTTCTGGATACGCCTTCACTCATTACTTAATAGAGTGGG TGAAGCAGAGGCCTGGACAGGGCCTTGAGTGGATTGGAATGATTAATCCTGAAAAT ACTGATACTACCTACAATGAGAAGTTCAGGGACAAGGCAATACTGACTGTAGACAAA TCCTCCAACACTGCCTACATGCAGCTCAGCAGCCTGACTTCTGATGACTCTGCGGT CTATTTCTGTGCAAGACAGGAGATTACGTCGGACTTTGACTTCTGGGGCCAAGGGA CCACGGTCACCGTCTCCTCA |
| 1517. | ME05D7-L | murine | aa | ELVLTQSPTTMAASPGEKITITCSASSSISSNYLHWYQQKPGFSPKLLIYRTSNLASGVP ARFSGSGSGTSYSLTIGTMEAEDVATYYCQQGSSIPYTFGGGTKLEIK |
| 1518. | ME05D7-LCDR1 | murine | aa | SASSSISSNYLH |
| 1519. | ME05D7-LCDR2 | murine | aa | RTSNLAS |
| 1520. | ME05D7-LCDR3 | murine | aa | QQGSSIPYT |
| 1521. | ME05D7-L | murine | nt | GAGCTCGTGCTCACCCAGTCTCCAACCACCATGGCTGCATCTCCCGGGGAGAAGA TCACTATCACCTGCAGTGCCAGCTCAAGTATAAGTTCCAATTACTTGCATTGGTATC AGCAGAAGCCAGGATTCTCCCCTAAACTCTTGATTTATAGGACATCCAATCTGGCTT CTGGAGTCCCAGCTCGCTTCAGTGGCAGTGGGTCTGGGACCTCTTACTCTCTCACA ATTGGCACCATGGAGGCTGAAGATGTTGCCACTTACTACTGCCAGCAGGGTAGTAG TATACCGTACACGTTCGGAGGGGGGACCAAGCTTGAGATCAAA |
| 1522. | ME05D7-HL | murine | aa | EVQLLEQSGAELVRPGTSVKVSCKASGYAFTHYLIEWVKQRPGQGLEWIGMINPENTD TTYNEKFRDKAILTVDKSSNTAYMQLSSLTSDDSAVYFCARQEITSDFDFWGQGTTVTV SSGGGGSGGGGSGGGGSELVLTQSPTTMAASPGEKITITCSASSSISSNYLHWYQQKP GFSPKLLIYRTSNLASGVPARFSGSGSGTSYSLTIGTMEAEDVATYYCQQGSSIPYTFG |

| | | | | |
|---|---|---|---|---|
| | | | | GGTKLEIK |
| 1523. | ME05D7-HL | murine | nt | GAGGTGCAGCTGCTCGAGCAGTCTGGAGCTGAGCTGGTAAGGCCTGGGACTTCAGTGAAGGTGTCCTGCAAGGCTTCTGGATACGCCTTCACTCATTACTTAATAGAGTGGGTGAAGCAGAGGCCTGGACAGGGCCTTGAGTGGATTGGAATGATTAATCCTGAAAATACTGATACTACCTACAATGAGAAGTTCAGGGACAAGGCAATACTGACTGTAGACAAATCCTCCAACACTGCCTACATGCAGCTCAGCAGCCTGACTTCTGATGACTCTGCGGTCTATTTCTGTGCAAGACAGGAGATTACGTCGGACTTTGACTTCTGGGGCCAAGGGACCACGGTCACCGTCTCCTCAGGTGGTGGTGGTTCTGGCGGCGGCGGCTCCGGTGGTGGTGGTTCTGAGCTCGTGCTCACCCAGTCTCCAACCACCATGGCTGCATCTCCCGGGGAGAAGATCACTATCACCTGCAGTGCCAGCTCAAGTATAAGTTCCAATTACTTGCATTGGTATCAGCAGAAGCCAGGATTCTCCCCTAAACTCTTGATTTATAGGACATCCAATCTGGCTTCTGGAGTCCCAGCTCGCTTCAGTGGCAGTGGGTCTGGGACCTCTTACTCTCTCACAATTGGCACCATGGAGGCTGAAGATGTTGCCACTTACTACTGCCAGCAGGGTAGTAGTATACCGTACACGTTCGGAGGGGGGACCAAGCTTGAGATCAAA |
| 1524. | ME05D7 HL x I2C HL | murine | aa | EVQLLEQSGAELVRPGTSVKVSCKASGYAFTHYLIEWVKQRPGQGLEWIGMINPENTDTTYNEKFRDKAILTVDKSSNTAYMQLSSLTSDDSAVYFCARQEITSDFDFWGQGTTVTVSSGGGGSGGGGSGGGGSELVLTQSPTTMAASPGEKITITCSASSSISSNYLHWYQQKPGFSPKLLIYRTSNLASGVPARFSGSGSGTSYSLTIGTMEAEDVATYYCQQGSSIPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1525. | ME05D7 HL x I2C HL | murine | nt | GAGGTGCAGCTGCTCGAGCAGTCTGGAGCTGAGCTGGTAAGGCCTGGGACTTCAGTGAAGGTGTCCTGCAAGGCTTCTGGATACGCCTTCACTCATTACTTAATAGAGTGGGTGAAGCAGAGGCCTGGACAGGGCCTTGAGTGGATTGGAATGATTAATCCTGAAAATACTGATACTACCTACAATGAGAAGTTCAGGGACAAGGCAATACTGACTGTAGACAAATCCTCCAACACTGCCTACATGCAGCTCAGCAGCCTGACTTCTGATGACTCTGCGGTCTATTTCTGTGCAAGACAGGAGATTACGTCGGACTTTGACTTCTGGGGCCAAGGGACCACGGTCACCGTCTCCTCAGGTGGTGGTGGTTCTGGCGGCGGCGGCTCCGGTGGTGGTGGTTCTGAGCTCGTGCTCACCCAGTCTCCAACCACCATGGCTGCATCTCCCGGGGAGAAGATCACTATCACCTGCAGTGCCAGCTCAAGTATAAGTTCCAATTACTTGCATTGGTATCAGCAGAAGCCAGGATTCTCCCCTAAACTCTTGATTTATAGGACATCCAATCTGGCTTCTGGAGTCCCAGCTCGCTTCAGTGGCAGTGGGTCTGGGACCTCTTACTCTCTCACAATTGGCACCATGGAGGCTGAAGATGTTGCCACTTACTACTGCCAGCAGGGTAGTAGTATACCGTACACGTTCGGAGGGGGGACCAAGCTTGAGATCAAATCC |

| | | | | |
|---|---|---|---|---|
| | | | | GGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAAGTAC GCCATGAACTGGGTCCGCCAGGCTCCAGGAAAGGGTTTGGAATGGGTTGCTCGCA TAAGAAGTAAATATAATAATTATGCAACATATTATGCCGATTCAGTGAAAGACAGGTT CACCATCTCCAGAGATGATTCAAAAAACACTGCCTATCTACAAATGAACAACTTGAA AACTGAGGACACTGCCGTGTACTACTGTGTGAGACATGGGAACTTCGGTAATAGCT ACATATCCTACTGGGCTTACTGGGGCCAAGGGACTCTGGTCACCGTCTCCTCAGGT GGTGGTGGTTCTGGCGGCGGCGGCTCCGGTGGTGGTGGTTCTCAGACTGTTGTGA CTCAGGAACCTTCACTCACCGTATCACCTGGTGGAACAGTCACACTCACTTGTGGC TCCTCGACTGGGGCTGTTACATCTGGCAACTACCCAAACTGGGTCCAACAAAAACC AGGTCAGGCACCCCGTGGTCTAATAGGTGGGACTAAGTTCCTCGCCCCCGGTACT CCTGCCAGATTCTCAGGCTCCCTGCTTGGAGGCAAGGCTGCCCTCACCCTCTCAG GGGTACAGCCAGAGGATGAGGCAGAATATTACTGTGTTCTATGGTACAGCAACCGC TGGGTGTTCGGTGGAGGAACCAAACTGACTGTCCTA |
| 1526. | ME05B7-H | murine | aa | EVQLLEQSGAELVRPGTSVKISCKTTGYTFGYYWISWLKQRPGHGLEWIGDIYPGGGY TNYHEKFKGKVTLTADTSSRTAYMQLSSLTFEDSAIYYCARSGYDYGSDYDYHGMDYW GQGTTVTVSS |
| 1527. | ME05B7-HCDR1 | murine | aa | YYWIS |
| 1528. | ME05B7-HCDR2 | murine | aa | DIYPGGGYTNYHEKFKG |
| 1529. | ME05B7-HCDR3 | murine | aa | SGYDYGSDYDYHGMDY |
| 1530. | ME05B7-H | murine | nt | GAGGTGCAGCTGCTCGAGCAGTCTGGAGCTGAGCTGGTTAGGCCTGGGACTTCAG TGAAGATTTCCTGCAAGACTACTGGGTACACCTTCGGTTATTACTGGATAAGTTGGT TAAAGCAGAGGCCTGGACATGGCCTTGAGTGGATTGGAGATATTTACCCTGGAGGT GGTTACACTAACTACCATGAGAAATTCAAGGGCAAGGTCACACTGACTGCAGACAC ATCCTCCAGGACAGCCTACATGCAGCTCAGCAGCCTGACATTTGAGGACTCTGCCA TCTATTACTGTGCAAGATCGGGATATGACTACGGTAGTGACTACGATTATCATGGTA TGGATTACTGGGGCCAAGGGACCACGGTCACCGTCTCCTCA |
| 1531. | ME05B7-L | murine | aa | ELVMTQTPSSLSASLGDRVTISCRASQGISNYLNWYQQKPDGSVKLLIYYTSRLHSGVP SRFSGSGSGTDYSLTISNLDQEDIATYFCQQGNTLPWTFGGGTKLEIK |
| 1532. | ME05B7-LCDR1 | murine | aa | RASQGISNYLN |
| 1533. | ME05B7-LCDR2 | murine | aa | YTSRLHS |
| 1534. | ME05B7-LCDR3 | murine | aa | QQGNTLP |
| 1535. | ME05B7-L | murine | nt | GAGCTCGTGATGACCCAGACTCCATCCTCCCTGTCTGCCTCTCTGGGAGACAGAGT CACCATCAGTTGCAGGGCAAGTCAGGGCATTAGCAATTATTTAAACTGGTATCAGCA GAAACCAGATGGATCTGTTAAACTCCTGATCTACTACACATCAAGATTACACTCAGG AGTCCCATCAAGGTTCAGTGGCAGTGGGTCTGGAACAGATTATTCTCTCACCATTAG |

| | | | | |
|---|---|---|---|---|
| | | | | CAACCTGGATCAAGAAGATATTGCCACTTACTTTTGCCAACAGGGTAATACGCTTCCGTGGACGTTCGGAGGGGGGACCAAGCTTGAGATCAAA |
| 1536. | ME05B7-HL | murine | aa | EVQLLEQSGAELVRPGTSVKISCKTTGYTFGYYWISWLKQRPGHGLEWIGDIYPGGGYTNYHEKFKGKVTLTADTSSRTAYMQLSSLTFEDSAIYYCARSGYDYGSDYDYHGMDYWGQGTTVTVSSGGGGSGGGGSGGGGSELVMTQTPSSLSASLGDRVTISCRASQGISNYLNWYQQKPDGSVKLLIYYTSRLHSGVPSRFSGSGSGTDYSLTISNLDQEDIATYFCQQGNTLPWTFGGGTKLEIK |
| 1537. | ME05B7-HL | murine | nt | GAGGTGCAGCTGCTCGAGCAGTCTGGAGCTGAGCTGGTTAGGCCTGGGACTTCAGTGAAGATTTCCTGCAAGACTACTGGGTACACCTTCGGTTATTACTGGATAAGTTGGTTAAAGCAGAGGCCTGGACATGGCCTTGAGTGGATTGGAGATATTTACCCTGGAGGTGGTTACACTAACTACCATGAGAAATTCAAGGGCAAGGTCACACTGACTGCAGACACATCCTCCAGGACAGCCTACATGCAGCTCAGCAGCCTGACATTTGAGGACTCTGCCATCTATTACTGTGCAAGATCGGGATATGACTACGGTAGTGACTACGATTATCATGGTATGGATTACTGGGGCCAAGGGACCACGGTCACCGTCTCCTCAGGTGGTGGTGGTTCTGGCGGCGGCGGCTCCGGTGGTGGTGGTTCTGAGCTCGTGATGACCCAGACTCCATCCTCCCTGTCTGCCTCTCTGGGAGACAGAGTCACCATCAGTTGCAGGGCAAGTCAGGGCATTAGCAATTATTTAAACTGGTATCAGCAGAAACCAGATGGATCTGTTAAACTCCTGATCTACTACACATCAAGATTACACTCAGGAGTCCCATCAAGGTTCAGTGGCAGTGGGTCTGGAACAGATTATTCTCTCACCATTAGCAACCTGGATCAAGAAGATATTGCCACTTACTTTTGCCAACAGGGTAATACGCTTCCGTGGACGTTCGGAGGGGGGACCAAGCTTGAGATCAAA |
| 1538. | ME05B7 HL x I2C HL | murine | aa | EVQLLEQSGAELVRPGTSVKISCKTTGYTFGYYWISWLKQRPGHGLEWIGDIYPGGGYTNYHEKFKGKVTLTADTSSRTAYMQLSSLTFEDSAIYYCARSGYDYGSDYDYHGMDYWGQGTTVTVSSGGGGSGGGGSGGGGSELVMTQTPSSLSASLGDRVTISCRASQGISNYLNWYQQKPDGSVKLLIYYTSRLHSGVPSRFSGSGSGTDYSLTISNLDQEDIATYFCQQGNTLPWTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1539. | ME05B7 HL x I2C HL | murine | nt | GAGGTGCAGCTGCTCGAGCAGTCTGGAGCTGAGCTGGTTAGGCCTGGGACTTCAGTGAAGATTTCCTGCAAGACTACTGGGTACACCTTCGGTTATTACTGGATAAGTTGGTTAAAGCAGAGGCCTGGACATGGCCTTGAGTGGATTGGAGATATTTACCCTGGAGGTGGTTACACTAACTACCATGAGAAATTCAAGGGCAAGGTCACACTGACTGCAGACACATCCTCCAGGACAGCCTACATGCAGCTCAGCAGCCTGACATTTGAGGACTCTGCCATCTATTACTGTGCAAGATCGGGATATGACTACGGTAGTGACTACGATTATCATGGTA |

| | | | | |
|---|---|---|---|---|
| | | | | TGGATTACTGGGGCCAAGGGACCACGGTCACCGTCTCCTCAGGTGGTGGTGGTTCTGGCGGCGGCGGCTCCGGTGGTGGTGGTTCTGAGCTCGTGATGACCCAGACTCCATCCTCCCTGTCTGCCTCTCTGGGAGACAGAGTCACCATCAGTTGCAGGGCAAGTCAGGGCATTAGCAATTATTTAAACTGGTATCAGCAGAAACCAGATGGATCTGTTAAACTCCTGATCTACTACACATCAAGATTACACTCAGGAGTCCCATCAAGGTTCAGTGGCAGTGGGTCTGGAACAGATTATTCTCTCACCATTAGCAACCTGGATCAAGAAGATATTGCCACTTACTTTTGCCAACAGGGTAATACGCTTCCGTGGACGTTCGGAGGGGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1540. | ME05F6-H | murine | aa | EVQLLEQSGAEVVRPGTSVKMSCKAAGYTFTKYWIGWVKQRPGHGLEWIGDIHPGGGFANYNEKFKGKATLTADTSSSTAYMQLSRLTSDDSAIYYCARSGYDYGSSYDYHGMDYWGQGTTVTVSS |
| 1541. | ME05F6-HCDR1 | murine | aa | KYWIG |
| 1542. | ME05F6-HCDR2 | murine | aa | DIHPGGGFANYNEKFKG |
| 1543. | ME05F6-HCDR3 | murine | aa | SGYDYGSSYDYHGMDY |
| 1544. | ME05F6-H | murine | nt | GAGGTGCAGCTGCTCGAGCAGTCTGGAGCTGAGGTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTTTGCTAACTACAATGAGAAGTTCAAGGGCAAGGCCACACTGACTGCAGACACATCCTCCAGCACAGCCTACATGCAGCTCAGCAGACTGACATCTGATGACTCTGCCATCTATTACTGTGCAAGATCGGGTTATGACTACGGTAGTAGTTACGATTACCATGGTATGGACTACTGGGGCCAAGGGACCACGGTCACCGTCTCCTCA |
| 1545. | ME05F6-L | murine | aa | ELVMTQSPSSLSASLGERVSLTCRASQEISGYLSWLQQKPDGTIKRLIYAASTLDSGVPKRFSGSRSGSDYSLTISSLESEDFADYYCLQYASYPPTFGGGTKLEIK |

| | | | | |
|---|---|---|---|---|
| 1546. | ME05F6-LCDR1 | murine | aa | RASQEISGYLS |
| 1547. | ME05F6-LCDR2 | murine | aa | AASTLDS |
| 1548. | ME05F6-LCDR3 | murine | aa | LQYASYPPT |
| 1549. | ME05F6-L | murine | nt | GAGCTCGTGATGACACAGTCTCCATCCTCCTTATCTGCCTCTCTGGGAGAAAGAGTCAGTCTCACTTGTCGGGCAAGTCAGGAAATTAGTGGTTACTTAAGCTGGCTTCAGCAGAAACCAGATGGAACTATTAAACGCCTGATCTACGCCGCATCCACTTTAGATTCTGGTGTCCCAAAAAGGTTCAGTGGCAGTAGGTCTGGGTCAGATTATTCTCTCACCATCAGCAGCCTTGAGTCTGAAGATTTTGCAGACTATTACTGTCTACAATATGCTAGTTATCCTCCCACGTTCGGAGGGGGGACCAAGCTTGAGATCAAA |
| 1550. | ME05F6-HL | murine | aa | EVQLLEQSGAEVVRPGTSVKMSCKAAGYTFTKYWIGWVKQRPGHGLEWIGDIHPGGGFANYNEKFKGKATLTADTSSSTAYMQLSRLTSDDSAIYYCARSGYDYGSSYDYHGMDYWGQGTTVTVSSGGGGSGGGGSGGGGSELVMTQSPSSLSASLGERVSLTCRASQEISGYLSWLQQKPDGTIKRLIYAASTLDSGVPKRFSGSRSGSDYSLTISSLESEDFADYYCLQYASYPPTFGGGTKLEIK |
| 1551. | ME05F6-HL | murine | nt | GAGGTGCAGCTGCTCGAGCAGTCTGGAGCTGAGGTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTTTGCTAACTACAATGAGAAGTTCAAGGGCAAGGCCACACTGACTGCAGACACATCCTCCAGCACAGCCTACATGCAGCTCAGCAGACTGACATCTGATGACTCTGCCATCTATTACTGTGCAAGATCGGGTTATGACTACGGTAGTAGTTACGATTACCATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCCTCCTTATCTGCCTCTCTGGGAGAAAGAGTCAGTCTCACTTGTCGGGCAAGTCAGGAAATTAGTGGTTACTTAAGCTGGCTTCAGCAGAAACCAGATGGAACTATTAAACGCCTGATCTACGCCGCATCCACTTTAGATTCTGGTGTCCCAAAAAGGTTCAGTGGCAGTAGGTCTGGGTCAGATTATTCTCTCACCATCAGCAGCCTTGAGTCTGAAGATTTTGCAGACTATTACTGTCTACAATATGCTAGTTATCCTCCCACGTTCGGAGGGGGGACCAAGCTTGAGATCAAA |
| 1552. | ME05F6 HL x I2C HL | murine | aa | EVQLLEQSGAEVVRPGTSVKMSCKAAGYTFTKYWIGWVKQRPGHGLEWIGDIHPGGGFANYNEKFKGKATLTADTSSSTAYMQLSRLTSDDSAIYYCARSGYDYGSSYDYHGMDYWGQGTTVTVSSGGGGSGGGGSGGGGSELVMTQSPSSLSASLGERVSLTCRASQEISGYLSWLQQKPDGTIKRLIYAASTLDSGVPKRFSGSRSGSDYSLTISSLESEDFADYYCLQYASYPPTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVT |

| | | | | |
|---|---|---|---|---|
| | | | | QEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 1553. | ME05F6 HL x I2C HL | murine | nt | GAGGTGCAGCTGCTCGAGCAGTCTGGAGCTGAGGTGGTTAGGCCTGGGACTTCAG<br>TGAAGATGTCCTGCAAGGCTGCTGGATACACCTTCACTAAGTACTGGATAGGTTGG<br>GTAAAGCAGAGGCCTGGACATGGCCTTGAGTGGATTGGAGATATTCACCCTGGAG<br>GTGGTTTTGCTAACTACAATGAGAAGTTCAAGGGCAAGGCCACACTGACTGCAGAC<br>ACATCCTCCAGCACAGCCTACATGCAGCTCAGCAGACTGACATCTGATGACTCTGC<br>CATCTATTACTGTGCAAGATCGGGTTATGACTACGGTAGTAGTTACGATTACCATGG<br>TATGGACTACTGGGGCCAAGGGACCACGGTCACCGTCTCCTCAGGTGGTGGTGGT<br>TCTGGCGGCGGCGGCTCCGGTGGTGGTGGTTCTGAGCTCGTGATGACACAGTCTC<br>CATCCTCCTTATCTGCCTCTCTGGGAGAAAGAGTCAGTCTCACTTGTCGGGCAAGT<br>CAGGAAATTAGTGGTTACTTAAGCTGGCTTCAGCAGAAACCAGATGGAACTATTAAA<br>CGCCTGATCTACGCCGCATCCACTTTAGATTCTGGTGTCCCAAAAAGGTTCAGTGG<br>CAGTAGGTCTGGGTCAGATTATTCTCTCACCATCAGCAGCCTTGAGTCTGAAGATTT<br>TGCAGACTATTACTGTCTACAATATGCTAGTTATCCTCCCACGTTCGGAGGGGGGA<br>CCAAGCTTGAGATCAAATCCGGAGGTGGTGGATCCGAGGTGCAGCTGGTCGAGTC<br>TGGAGGAGGATTGGTGCAGCCTGGAGGGTCATTGAAACTCTCATGTGCAGCCTCTG<br>GATTCACCTTCAATAAGTACGCCATGAACTGGGTCCGCCAGGCTCCAGGAAAGGGT<br>TTGGAATGGGTTGCTCGCATAAGAAGTAAATATAATAATTATGCAACATATTATGCCG<br>ATTCAGTGAAAGACAGGTTCACCATCTCCAGAGATGATTCAAAAAACACTGCCTATC<br>TACAAATGAACAACTTGAAAACTGAGGACACTGCCGTGTACTACTGTGTGAGACATG<br>GGAACTTCGGTAATAGCTACATATCCTACTGGGCTTACTGGGGCCAAGGGACTCTG<br>GTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAAC<br>AGTCACACTCACTTGTGGCTCCTCGACTGGGGCTGTTACATCTGGCAACTACCCAA<br>ACTGGGTCCAACAAAAACCAGGTCAGGCACCCCGTGGTCTAATAGGTGGGACTAAG<br>TTCCTCGCCCCCGGTACTCCTGCCAGATTCTCAGGCTCCCTGCTTGGAGGCAAGGC<br>TGCCCTCACCCTCTCAGGGGTACAGCCAGAGGATGAGGCAGAATATTACTGTGTTC<br>TATGGTACAGCAACCGCTGGGTGTTCGGTGGAGGAACCAAACTGACTGTCCTA |
| 1554. | ME06C6-H | murine | aa | EVQLLEQSGAELVRPGTSVKIPCKATGYTFSYYWIGWVKQRPGHGLEWIGDIYPGGGY<br>SNYNEKFKGKATLTADTSSSTAYMQLSGLTSEDSAIYYCARSGYDYGSDYDYHGLDYW<br>GQGTTVTVSS |
| 1555. | ME06C6-HCDR1 | murine | aa | YYWIG |
| 1556. | ME06C6-HCDR2 | murine | aa | DIYPGGGYSNYNEKFKG |
| 1557. | ME06C6-HCDR3 | murine | aa | SGYDYGSDYDYHGLDY |

| | | | | |
|---|---|---|---|---|
| 1558. | ME06C6-H | murine | nt | GAGGTGCAGCTGCTCGAGCAGTCTGGAGCTGAACTGGTAAGGCCTGGGACTTCAGTGAAGATTCCCTGCAAGGCTACTGGATACACCTTCAGTTATTACTGGATAGGTTGGGTAAAGCAGAGGCCTGGACATGGCCTTGAGTGGATTGGAGATATTTACCCTGGAGGTGGTTATAGTAACTACAATGAGAAATTCAAGGGAAAGGCCACACTGACTGCAGACACATCCTCCAGCACAGCCTACATGCAGCTCAGCGGCCTGACATCTGAGGACTCTGCCATCTATTACTGTGCAAGATCGGGATATGACTACGGTAGTGACTACGATTACCATGGTCTGGACTACTGGGGCCAAGGGACCACGGTCACCGTCTCCTCA |
| 1559. | ME06C6-L | murine | aa | ELVLTQSPASLAVSLGQRATISCRASESVEYYGTSLMQWYQQKPGQPPKLLIYAASNVESGVPARFSGSGSGTDFSLNIHPVEEDDIAMYFCQQSRKVPYTFGGGTKLEIK |
| 1560. | ME06C6-LCDR1 | murine | aa | RASESVEYYGTSLMQ |
| 1561. | ME06C6-LCDR2 | murine | aa | AASNVES |
| 1562. | ME06C6-LCDR3 | murine | aa | QQSRKVPYT |
| 1563. | ME06C6-L | murine | nt | GAGCTCGTGCTCACCCAGTCTCCAGCTTCTTTGGCTGTGTCTCTAGGGCAGAGAGCCACCATCTCCTGCAGAGCCAGTGAAAGTGTTGAATATTATGGCACAAGTTTAATGCAGTGGTACCAACAGAAACCAGGACAGCCACCCAAACTCCTCATCTATGCTGCATCCAACGTAGAATCTGGGGTCCCTGCCAGGTTTAGTGGCAGTGGGTCTGGGACAGACTTCAGCCTCAACATCCATCCTGTGGAGGAGGATGATATTGCAATGTATTTCTGTCAGCAAAGTAGGAAGGTTCCGTACACGTTCGGAGGGGGGACCAAGCTTGAGATCAAA |
| 1564. | ME06C6-HL | murine | aa | EVQLLEQSGAELVRPGTSVKIPCKATGYTFSYYWIGWVKQRPGHGLEWIGDIYPGGGYSNYNEKFKGKATLTADTSSSTAYMQLSGLTSEDSAIYYCARSGYDYGSDYDYHGLDYWGQGTTVTVSSGGGGSGGGGSGGGGSELVLTQSPASLAVSLGQRATISCRASESVEYYGTSLMQWYQQKPGQPPKLLIYAASNVESGVPARFSGSGSGTDFSLNIHPVEEDDIAMYFCQQSRKVPYTFGGGTKLEIK |
| 1565. | ME06C6-HL | murine | nt | GAGGTGCAGCTGCTCGAGCAGTCTGGAGCTGAACTGGTAAGGCCTGGGACTTCAGTGAAGATTCCCTGCAAGGCTACTGGATACACCTTCAGTTATTACTGGATAGGTTGGGTAAAGCAGAGGCCTGGACATGGCCTTGAGTGGATTGGAGATATTTACCCTGGAGGTGGTTATAGTAACTACAATGAGAAATTCAAGGGAAAGGCCACACTGACTGCAGACACATCCTCCAGCACAGCCTACATGCAGCTCAGCGGCCTGACATCTGAGGACTCTGCCATCTATTACTGTGCAAGATCGGGATATGACTACGGTAGTGACTACGATTACCATGGTCTGGACTACTGGGGCCAAGGGACCACGGTCACCGTCTCCTCAGGTGGTGGTGGTTCTGGCGGCGGCGGCTCCGGTGGTGGTGGTTCTGAGCTCGTGCTCACCCAGTCTCCAGCTTCTTTGGCTGTGTCTCTAGGGCAGAGAGCCACCATCTCCTGCAGAGCCAGTGAAAGTGTTGAATATTATGGCACAAGTTTAATGCAGTGGTACCAACAGAAACCAGGACAGCCACCCAAACTCCTCATCTATGCTGCATCCAACGTAGAATCTGGGGTCCCTGCCAGGTTTAGTGGCAGTGGGTCTGGGACAGACTTCAGCCTCAACATCCATCCTGTGGAGGAGGATGATATTGCAATGTATTTCTGTCAGCAAAGTAGGAAGGTTCCGTACACGTTC |

| | | | | |
|---|---|---|---|---|
| | | | | GGAGGGGGGACCAAGCTTGAGATCAAA |
| 1566. | ME06C6 HL x I2C HL | murine | aa | EVQLLEQSGAELVRPGTSVKIPCKATGYTFSYYWIGWVKQRPGHGLEWIGDIYPGGGY SNYNEKFKGKATLTADTSSSTAYMQLSGLTSEDSAIYYCARSGYDYGSDYDYHGLDYW GQGTTVTVSSGGGGSGGGGSGGGGSELVLTQSPASLAVSLGQRATISCRASESVEYY GTSLMQWYQQKPGQPPKLLIYAASNVESGVPARFSGSGSGTDFSLNIHPVEEDDIAMY FCQQSRKVPYTFGGGTKLEIKSGGGGSEVQLVESGGGLVQPGGSLKLSCAASGFTFN KYAMNWVRQAPGKGLEWVARIRSKYNNYATYYADSVKDRFTISRDDSKNTAYLQMNN LKTEDTAVYYCVRHGNFGNSYISYWAYWGQGTLVTVSSGGGGSGGGGSGGGGSQTV VTQEPSLTVSPGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL |
| 1567. | ME06C6 HL x I2C HL | murine | nt | GAGGTGCAGCTGCTCGAGCAGTCTGGAGCTGAACTGGTAAGGCCTGGGACTTCAG TGAAGATTCCCTGCAAGGCTACTGGATACACCTTCAGTTATTACTGGATAGGTTGGG TAAAGCAGAGGCCTGGACATGGCCTTGAGTGGATTGGAGATATTTACCCTGGAGGT GGTTATAGTAACTACAATGAGAAATTCAAGGGAAAGGCCACACTGACTGCAGACAC ATCCTCCAGCACAGCCTACATGCAGCTCAGCGGCCTGACATCTGAGGACTCTGCCA TCTATTACTGTGCAAGATCGGGATATGACTACGGTAGTGACTACGATTACCATGGTC TGGACTACTGGGGCCAAGGGACCACGGTCACCGTCTCCTCAGGTGGTGGTGGTTC TGGCGGCGGCGGCTCCGGTGGTGGTGGTTCTGAGCTCGTGCTCACCCAGTCTCCA GCTTCTTTGGCTGTGTCTCTAGGGCAGAGAGCCACCATCTCCTGCAGAGCCAGTGA AAGTGTTGAATATTATGGCACAAGTTTAATGCAGTGGTACCAACAGAAACCAGGACA GCCACCCAAACTCCTCATCTATGCTGCATCCAACGTAGAATCTGGGGTCCCTGCCA GGTTTAGTGGCAGTGGGTCTGGGACAGACTTCAGCCTCAACATCCATCCTGTGGAG GAGGATGATATTGCAATGTATTTCTGTCAGCAAAGTAGGAAGGTTCCGTACACGTTC GGAGGGGGGACCAAGCTTGAGATCAAATCCGGAGGTGGTGGATCCGAGGTGCAG CTGGTCGAGTCTGGAGGAGGATTGGTGCAGCCTGGAGGGTCATTGAAACTCTCAT GTGCAGCCTCTGGATTCACCTTCAATAAGTACGCCATGAACTGGGTCCGCCAGGCT CCAGGAAAGGGTTTGGAATGGGTTGCTCGCATAAGAAGTAAATATAATAATTATGCA ACATATTATGCCGATTCAGTGAAAGACAGGTTCACCATCTCCAGAGATGATTCAAAA AACACTGCCTATCTACAAATGAACAACTTGAAAACTGAGGACACTGCCGTGTACTAC TGTGTGAGACATGGGAACTTCGGTAATAGCTACATATCCTACTGGGCTTACTGGGG CCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATC ACCTGGTGGAACAGTCACACTCACTTGTGGCTCCTCGACTGGGGCTGTTACATCTG GCAACTACCCAAACTGGGTCCAACAAAAACCAGGTCAGGCACCCCGTGGTCTAATA GGTGGGACTAAGTTCCTCGCCCCCGGTACTCCTGCCAGATTCTCAGGCTCCCTGCT TGGAGGCAAGGCTGCCCTCACCCTCTCAGGGGTACAGCCAGAGGATGAGGCAGAA |

| | | | | |
|---|---|---|---|---|
| | | | | TATTACTGTGTTCTATGGTACAGCAACCGCTGGGTGTTCGGTGGAGGAACCAAACT<br>GACTGTCCTA |
| 1568. | ME06C7-H | murine | aa | EVQLLEQSGAEVVRPGTSVKMSCKAAGYTFTKYWIGWVKQRPGHGLEWIGDIHPGGG<br>FANYNEKFKGKATLTADTSSSTAYMQLSRLTSDDSAIYYCARSGYDYGSSYDYHGMDY<br>WGQGTTVTVSS |
| 1569. | ME06C7-HCDR1 | murine | aa | KYWIG |
| 1570. | ME06C7-HCDR2 | murine | aa | DIHPGGGFANYNEKFKG |
| 1571. | ME06C7-HCDR3 | murine | aa | SGYDYGSSYDYHGMDY |
| 1572. | ME06C7-H | murine | nt | GAGGTGCAGCTGCTCGAGCAGTCTGGAGCTGAGGTGGTTAGGCCTGGGACTTCAG<br>TGAAGATGTCCTGCAAGGCTGCTGGATACACCTTCACTAAGTACTGGATAGGTTGG<br>GTAAAGCAGAGGCCTGGACATGGCCTTGAGTGGATTGGAGATATTCACCCTGGAG<br>GTGGTTTTGCTAACTACAATGAGAAGTTCAAGGGCAAGGCCACACTGACTGCAGAC<br>ACATCCTCCAGCACAGCCTACATGCAGCTCAGCAGACTGACATCTGATGACTCTGC<br>CATCTATTACTGTGCAAGATCGGGTTATGACTACGGTAGTAGTTACGATTACCATGG<br>TATGGACTACTGGGGCCAAGGGACCACGGTCACCGTCTCCTCA |
| 1573. | ME06C7-L | murine | aa | ELQMTQSPTSLAVSLGQRATISCRASQTVYYDGNNYMNWYQHKPGQPPKLLIYATSNL<br>ESGIPARFSGSGSGTDFTLNIHPVEAEDAATYYCQQSYEDPYTFGGGTKLEIK |
| 1574. | ME06C7-LCDR1 | murine | aa | RASQTVYYDGNNYMN |
| 1575. | ME06C7-LCDR2 | murine | aa | ATSNLES |
| 1576. | ME06C7-LCDR3 | murine | aa | QQSYEDPYT |
| 1577. | ME06C7-L | murine | nt | GAGCTCCAGATGACCCAGTCTCCAACTTCTTTGGCTGTGTCTCTCGGGCAGAGGGC<br>CACCATCTCCTGCAGGGCCAGCCAAACTGTTTACTATGATGGTAATAATTATATGAA<br>CTGGTATCAACACAAACCAGGACAGCCACCCAAACTCCTCATCTATGCTACATCCAA<br>TCTAGAATCTGGGATCCCAGCCAGGTTTAGTGGCAGTGGGTCTGGGACAGACTTCA<br>CCCTCAACATCCATCCTGTGGAGGCGGAGGATGCTGCAACCTATTACTGTCAGCAA<br>AGTTATGAGGATCCGTACACGTTCGGAGGGGGGACCAAGCTTGAGATCAAA |
| 1578. | ME06C7-HL | murine | aa | EVQLLEQSGAEVVRPGTSVKMSCKAAGYTFTKYWIGWVKQRPGHGLEWIGDIHPGGG<br>FANYNEKFKGKATLTADTSSSTAYMQLSRLTSDDSAIYYCARSGYDYGSSYDYHGMDY<br>WGQGTTVTVSSGGGGSGGGGSGGGGSELQMTQSPTSLAVSLGQRATISCRASQTVY<br>YDGNNYMNWYQHKPGQPPKLLIYATSNLESGIPARFSGSGSGTDFTLNIHPVEAEDAAT<br>YYCQQSYEDPYTFGGGTKLEIK |
| 1579. | ME06C7-HL | murine | nt | GAGGTGCAGCTGCTCGAGCAGTCTGGAGCTGAGGTGGTTAGGCCTGGGACTTCAG<br>TGAAGATGTCCTGCAAGGCTGCTGGATACACCTTCACTAAGTACTGGATAGGTTGG<br>GTAAAGCAGAGGCCTGGACATGGCCTTGAGTGGATTGGAGATATTCACCCTGGAG<br>GTGGTTTTGCTAACTACAATGAGAAGTTCAAGGGCAAGGCCACACTGACTGCAGAC |

| | | | | |
|---|---|---|---|---|
| | | | | ACATCCTCCAGCACAGCCTACATGCAGCTCAGCAGACTGACATCTGATGACTCTGC CATCTATTACTGTGCAAGATCGGGTTATGACTACGGTAGTAGTTACGATTACCATGG TATGGACTACTGGGGCCAAGGGACCACGGTCACCGTCTCCTCAGGTGGTGGTGGT TCTGGCGGCGGCGGCTCCGGTGGTGGTGGTTCTGAGCTCCAGATGACCCAGTCTC CAACTTCTTTGGCTGTGTCTCTCGGGCAGAGGGCCACCATCTCCTGCAGGGCCAG CCAAACTGTTTACTATGATGGTAATAATTATATGAACTGGTATCAACACAAACCAGGA CAGCCACCCAAACTCCTCATCTATGCTACATCCAATCTAGAATCTGGGATCCCAGCC AGGTTTAGTGGCAGTGGGTCTGGGACAGACTTCACCCTCAACATCCATCCTGTGGA GGCGGAGGATGCTGCAACCTATTACTGTCAGCAAAGTTATGAGGATCCGTACACGT TCGGAGGGGGGACCAAGCTTGAGATCAAA |
| 1580. | ME06C7 HL x I2C HL | murine | aa | EVQLLEQSGAEVVRPGTSVKMSCKAAGYTFTKYWIGWVKQRPGHGLEWIGDIHPGGG FANYNEKFKGKATLTADTSSSTAYMQLSRLTSDDSAIYYCARSGYDYGSSYDYHGMDY WGQGTTVTVSSGGGGSGGGGSGGGGSELQMTQSPTSLAVSLGQRATISCRASQTVY YDGNNYMNWYQHKPGQPPKLLIYATSNLESGIPARFSGSGSGTDFTLNIHPVEAEDAAT YYCQQSYEDPYTFGGGTKLEIKSGGGGSEVQLVESGGGLVQPGGSLKLSCAASGFTF NKYAMNWVRQAPGKGLEWVARIRSKYNNYATYYADSVKDRFTISRDDSKNTAYLQMN NLKTEDTAVYYCVRHGNFGNSYISYWAYWGQGTLVTVSSGGGGSGGGGSGGGGSQT VVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 1581. | ME06C7 HL x I2C HL | murine | nt | GAGGTGCAGCTGCTCGAGCAGTCTGGAGCTGAGGTGGTTAGGCCTGGGACTTCAG TGAAGATGTCCTGCAAGGCTGCTGGATACACCTTCACTAAGTACTGGATAGGTTGG GTAAAGCAGAGGCCTGGACATGGCCTTGAGTGGATTGGAGATATTCACCCTGGAG GTGGTTTTGCTAACTACAATGAGAAGTTCAAGGGCAAGGCCACACTGACTGCAGAC ACATCCTCCAGCACAGCCTACATGCAGCTCAGCAGACTGACATCTGATGACTCTGC CATCTATTACTGTGCAAGATCGGGTTATGACTACGGTAGTAGTTACGATTACCATGG TATGGACTACTGGGGCCAAGGGACCACGGTCACCGTCTCCTCAGGTGGTGGTGGT TCTGGCGGCGGCGGCTCCGGTGGTGGTGGTTCTGAGCTCCAGATGACCCAGTCTC CAACTTCTTTGGCTGTGTCTCTCGGGCAGAGGGCCACCATCTCCTGCAGGGCCAG CCAAACTGTTTACTATGATGGTAATAATTATATGAACTGGTATCAACACAAACCAGGA CAGCCACCCAAACTCCTCATCTATGCTACATCCAATCTAGAATCTGGGATCCCAGCC AGGTTTAGTGGCAGTGGGTCTGGGACAGACTTCACCCTCAACATCCATCCTGTGGA GGCGGAGGATGCTGCAACCTATTACTGTCAGCAAAGTTATGAGGATCCGTACACGT TCGGAGGGGGGACCAAGCTTGAGATCAAATCCGGAGGTGGTGGATCCGAGGTGCA GCTGGTCGAGTCTGGAGGAGGATTGGTGCAGCCTGGAGGGTCATTGAAACTCTCA TGTGCAGCCTCTGGATTCACCTTCAATAAGTACGCCATGAACTGGGTCCGCCAGGC TCCAGGAAAGGGTTTGGAATGGGTTGCTCGCATAAGAAGTAAATATAATAATTATGC |

| | | | | |
|---|---|---|---|---|
| | | | | AACATATTATGCCGATTCAGTGAAAGACAGGTTCACCATCTCCAGAGATGATTCAAA<br>AAACACTGCCTATCTACAAATGAACAACTTGAAAACTGAGGACACTGCCGTGTACTA<br>CTGTGTGAGACATGGGAACTTCGGTAATAGCTACATATCCTACTGGGCTTACTGGG<br>GCCAAGGGACTCTGGTCACCGTCTCCTCAGGTGGTGGTGGTTCTGGCGGCGGCGG<br>CTCCGGTGGTGGTGGTTCTCAGACTGTTGTGACTCAGGAACCTTCACTCACCGTAT<br>CACCTGGTGGAACAGTCACACTCACTTGTGGCTCCTCGACTGGGGCTGTTACATCT<br>GGCAACTACCCAAACTGGGTCCAACAAAAACCAGGTCAGGCACCCCGTGGTCTAAT<br>AGGTGGGACTAAGTTCCTCGCCCCCGGTACTCCTGCCAGATTCTCAGGCTCCCTGC<br>TTGGAGGCAAGGCTGCCCTCACCCTCTCAGGGGTACAGCCAGAGGATGAGGCAGA<br>ATATTACTGTGTTCTATGGTACAGCAACCGCTGGGTGTTCGGTGGAGGAACCAAAC<br>TGACTGTCCTA |
| 1582. | ME06B7-H | murine | aa | EVQLLEQSGAELVRPGTSVKISCKATGYTFTTYWLGWVKQRPGHGLEWIGEIHPGGGY<br>TNYNEKFKGKATLTADTSSSTAYMQLSRLTSDDSAIYYCARSGYDYGSSYDYYGMDYW<br>GQGTTVTVSS |
| 1583. | ME06B7-HCDR1 | murine | aa | TYWLG |
| 1584. | ME06B7-HCDR2 | murine | aa | EIHPGGGYTNYNEKFKG |
| 1585. | ME06B7-HCDR3 | murine | aa | SGYDYGSSYDYYGMDY |
| 1586. | ME06B7-H | murine | nt | GAGGTGCAGCTGCTCGAGCAGTCTGGAGCTGAACTGGTAAGGCCTGGGACCTCAG<br>TGAAGATTTCCTGCAAGGCTACTGGATACACCTTCACTACTTACTGGCTAGGTTGGG<br>TAAAGCAGAGGCCTGGACATGGCCTTGAGTGGATTGGAGAGATTCACCCTGGAGG<br>TGGTTATACTAACTACAATGAGAAGTTCAAGGGCAAGGCCACACTGACTGCAGACA<br>CATCCTCCAGCACAGCCTACATGCAGCTCAGCAGACTGACATCTGATGACTCCGCC<br>ATCTATTACTGTGCAAGATCGGGGTATGACTACGGTAGTAGTTACGATTACTATGGT<br>ATGGACTACTGGGGCCAAGGGACCACGGTCACCGTCTCCTCA |
| 1587. | ME06B7-L | murine | aa | ELVMTQSPSSLSASLGERVSLTCRASQDIGSSLNWLQQEPDGTIKRLIYATSSLDSGVP<br>KRFSGSRSGSDYSLTISSLESEDFVDYYCLQYASSPYTFGGGTKLEIK |
| 1588. | ME06B7-LCDR1 | murine | aa | RASQDIGSSLN |
| 1589. | ME06B7-LCDR2 | murine | aa | ATSSLDS |
| 1590. | ME06B7-LCDR3 | murine | aa | LQYASSPYT |
| 1591. | ME06B7-L | murine | nt | GAGCTCGTCATGACCCAGTCTCCATCCTCCTTATCTGCCTCTCTGGGAGAAAGAGT<br>CAGTCTCACTTGTCGGGCAAGTCAGGACATTGGTAGTAGCTTAAACTGGCTTCAGC<br>AGGAACCAGATGGAACTATTAAACGCCTGATCTACGCCACATCCAGTTTAGATTCTG<br>GTGTCCCCAAAAGGTTCAGTGGCAGTAGGTCTGGGTCAGATTATTCTCTCACCATC<br>AGCAGCCTTGAGTCTGAAGATTTTGTAGACTATTACTGTCTACAATATGCTAGTTCTC<br>CGTACACGTTCGGAGGGGGGACCAAGCTTGAGATCAAA |
| 1592. | ME06B7-HL | murine | aa | EVQLLEQSGAELVRPGTSVKISCKATGYTFTTYWLGWVKQRPGHGLEWIGEIHPGGGY |

| | | | | |
|---|---|---|---|---|
| | | | | TNYNEKFKGKATLTADTSSSTAYMQLSRLTSDDSAIYYCARSGYDYGSSYDYYGMDYWGQGTTVTVSSGGGGSGGGGSGGGGSELVMTQSPSSLSASLGERVSLTCRASQDIGSSLNWLQQEPDGTIKRLIYATSSLDSGVPKRFSGSRSGSDYSLTISSLESEDFVDYYCLQYASSPYTFGGGTKLEIK |
| 1593. | ME06B7-HL | murine | nt | GAGGTGCAGCTGCTCGAGCAGTCTGGAGCTGAACTGGTAAGGCCTGGGACCTCAGTGAAGATTTCCTGCAAGGCTACTGGATACACCTTCACTACTTACTGGCTAGGTTGGGTAAAGCAGAGGCCTGGACATGGCCTTGAGTGGATTGGAGAGATTCACCCTGGAGGTGGTTATACTAACTACAATGAGAAGTTCAAGGGCAAGGCCACACTGACTGCAGACACATCCTCCAGCACAGCCTACATGCAGCTCAGCAGACTGACATCTGATGACTCCGCCATCTATTACTGTGCAAGATCGGGGTATGACTACGGTAGTAGTTACGATTACTATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTCATGACCCAGTCTCCATCCTCCTTATCTGCCTCTCTGGGAGAAAGAGTCAGTCTCACTTGTCGGGCAAGTCAGGACATTGGTAGTAGCTTAAACTGGCTTCAGCAGGAACCAGATGGAACTATTAAACGCCTGATCTACGCCACATCCAGTTTAGATTCTGGTGTCCCCAAAAGGTTCAGTGGCAGTAGGTCTGGGTCAGATTATTCTCTCACCATCAGCAGCCTTGAGTCTGAAGATTTTGTAGACTATTACTGTCTACAATATGCTAGTTCTCCGTACACGTTCGGAGGGGGGACCAAGCTTGAGATCAAA |
| 1594. | ME06B7 HL x I2C HL | murine | aa | EVQLLEQSGAELVRPGTSVKISCKATGYTFTTYWLGWVKQRPGHGLEWIGEIHPGGGYTNYNEKFKGKATLTADTSSSTAYMQLSRLTSDDSAIYYCARSGYDYGSSYDYYGMDYWGQGTTVTVSSGGGGSGGGGSGGGGSELVMTQSPSSLSASLGERVSLTCRASQDIGSSLNWLQQEPDGTIKRLIYATSSLDSGVPKRFSGSRSGSDYSLTISSLESEDFVDYYCLQYASSPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1595. | ME06B7 HL x I2C HL | murine | nt | GAGGTGCAGCTGCTCGAGCAGTCTGGAGCTGAACTGGTAAGGCCTGGGACCTCAGTGAAGATTTCCTGCAAGGCTACTGGATACACCTTCACTACTTACTGGCTAGGTTGGGTAAAGCAGAGGCCTGGACATGGCCTTGAGTGGATTGGAGAGATTCACCCTGGAGGTGGTTATACTAACTACAATGAGAAGTTCAAGGGCAAGGCCACACTGACTGCAGACACATCCTCCAGCACAGCCTACATGCAGCTCAGCAGACTGACATCTGATGACTCCGCCATCTATTACTGTGCAAGATCGGGGTATGACTACGGTAGTAGTTACGATTACTATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTCATGACCCAGTCTCCATCCTCCTTATCTGCCTCTCTGGGAGAAAGAGTCAGTCTCACTTGTCGGGCAAGTC |

| | | | | |
|---|---|---|---|---|
| | | | | AGGACATTGGTAGTAGCTTAAACTGGCTTCAGCAGGAACCAGATGGAACTATTAAAC GCCTGATCTACGCCACATCCAGTTTAGATTCTGGTGTCCCCAAAAGGTTCAGTGGC AGTAGGTCTGGGTCAGATTATTCTCTCACCATCAGCAGCCTTGAGTCTGAAGATTTT GTAGACTATTACTGTCTACAATATGCTAGTTCTCCGTACACGTTCGGAGGGGGGAC CAAGCTTGAGATCAAATCCGGAGGTGGTGGATCCGAGGTGCAGCTGGTCGAGTCT GGAGGAGGATTGGTGCAGCCTGGAGGGTCATTGAAACTCTCATGTGCAGCCTCTG GATTCACCTTCAATAAGTACGCCATGAACTGGGTCCGCCAGGCTCCAGGAAAGGGT TTGGAATGGGTTGCTCGCATAAGAAGTAAATATAATAATTATGCAACATATTATGCCG ATTCAGTGAAAGACAGGTTCACCATCTCCAGAGATGATTCAAAAAACACTGCCTATC TACAAATGAACAACTTGAAAACTGAGGACACTGCCGTGTACTACTGTGTGAGACATG GGAACTTCGGTAATAGCTACATATCCTACTGGGCTTACTGGGGCCAAGGGACTCTG GTCACCGTCTCCTCAGGTGGTGGTGGTTCTGGCGGCGGCGGCTCCGGTGGTGGT GGTTCTCAGACTGTTGTGACTCAGGAACCTTCACTCACCGTATCACCTGGTGGAAC AGTCACACTCACTTGTGGCTCCTCGACTGGGGCTGTTACATCTGGCAACTACCCAA ACTGGGTCCAACAAAAACCAGGTCAGGCACCCCGTGGTCTAATAGGTGGGACTAAG TTCCTCGCCCCCGGTACTCCTGCCAGATTCTCAGGCTCCCTGCTTGGAGGCAAGGC TGCCCTCACCCTCTCAGGGGTACAGCCAGAGGATGAGGCAGAATATTACTGTGTTC TATGGTACAGCAACCGCTGGGTGTTCGGTGGAGGAACCAAACTGACTGTCCTA |
| 1596. | ME06D1-H | murine | aa | EVQLLEQSGAEVVRPGTSVKMSCKAAGYTFTKYWIGWVKQRPGHGLEWIGDIHPGGG FANYNEKFKGKATLTADTSSSTAYMQLSRLTSDDSAIYYCARSGYDYGSSYDYHGMDY WGQGTTVTVSS |
| 1597. | ME06D1-HCDR1 | murine | aa | KYWIG |
| 1598. | ME06D1-HCDR2 | murine | aa | DIHPGGGFANYNEKFKG |
| 1599. | ME06D1-HCDR3 | murine | aa | SGYDYGSSYDYHGMDY |
| 1600. | ME06D1-H | murine | nt | GAGGTGCAGCTGCTCGAGCAGTCTGGAGCTGAGGTGGTTAGGCCTGGGACTTCAG TGAAGATGTCCTGCAAGGCTGCTGGATACACCTTCACTAAGTACTGGATAGGTTGG GTAAAGCAGAGGCCTGGACATGGCCTTGAGTGGATTGGAGATATTCACCCTGGAG GTGGTTTTGCTAACTACAATGAGAAGTTCAAGGGCAAGGCCACACTGACTGCAGAC ACATCCTCCAGCACAGCCTACATGCAGCTCAGCAGACTGACATCTGATGACTCTGC CATCTATTACTGTGCAAGATCGGGTTATGACTACGGTAGTAGTTACGATTACCATGG TATGGACTACTGGGGCCAAGGGACCACGGTCACCGTCTCCTCA |
| 1601. | ME06D1-L | murine | aa | ELVMTQSPSSLTVTAGEKVTMSCKSSQSLLNSGNQKNYLAWYQQKPGQPPKLLIYGAS TRESGVPDRFTGSGSGTDFTLTISSVQAEDLADYFCQQHYSTPYTFGGGTKLEIK |
| 1602. | ME06D1-LCDR1 | murine | aa | KSSQSLLNSGNQKNYLA |
| 1603. | ME06D1-LCDR2 | murine | aa | GASTRES |

| | | | | |
|---|---|---|---|---|
| 1604. | ME06D1-LCDR3 | murine | aa | QQHYSTPYT |
| 1605. | ME06D1-L | murine | nt | GAGCTCGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGGCCTGGTACCAGCAGAAACCAGGGCAGCCTCCTAAACTGTTGATCTACGGGGCATCCACTAGGGAATCTGGAGTCCCTGATCGCTTCACAGGCAGTGGATCTGGGACAGATTTCACTCTTACCATCAGCAGTGTGCAGGCTGAAGACCTGGCAGATTACTTCTGTCAGCAACATTATAGCACTCCGTACACGTTCGGAGGGGGGACCAAGCTTGAGATCAAA |
| 1606. | ME06D1-HL | murine | aa | EVQLLEQSGAEVVRPGTSVKMSCKAAGYTFTKYWIGWVKQRPGHGLEWIGDIHPGGGFANYNEKFKGKATLTADTSSSTAYMQLSRLTSDDSAIYYCARSGYDYGSSYDYHGMDYWGQGTTVTVSSGGGGSGGGGSGGGGSELVMTQSPSSLTVTAGEKVTMSCKSSQSLLNSGNQKNYLAWYQQKPGQPPKLLIYGASTRESGVPDRFTGSGSGTDFTLTISSVQAEDLADYFCQQHYSTPYTFGGGTKLEIK |
| 1607. | ME06D1-HL | murine | nt | GAGGTGCAGCTGCTCGAGCAGTCTGGAGCTGAGGTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTTTGCTAACTACAATGAGAAGTTCAAGGGCAAGGCCACACTGACTGCAGACACATCCTCCAGCACAGCCTACATGCAGCTCAGCAGACTGACATCTGATGACTCTGCCATCTATTACTGTGCAAGATCGGGTTATGACTACGGTAGTAGTTACGATTACCATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGGCCTGGTACCAGCAGAAACCAGGGCAGCCTCCTAAACTGTTGATCTACGGGGCATCCACTAGGGAATCTGGAGTCCCTGATCGCTTCACAGGCAGTGGATCTGGGACAGATTTCACTCTTACCATCAGCAGTGTGCAGGCTGAAGACCTGGCAGATTACTTCTGTCAGCAACATTATAGCACTCCGTACACGTTCGGAGGGGGGACCAAGCTTGAGATCAAA |
| 1608. | ME06D1 HL x I2C HL | murine | aa | EVQLLEQSGAEVVRPGTSVKMSCKAAGYTFTKYWIGWVKQRPGHGLEWIGDIHPGGGFANYNEKFKGKATLTADTSSSTAYMQLSRLTSDDSAIYYCARSGYDYGSSYDYHGMDYWGQGTTVTVSSGGGGSGGGGSGGGGSELVMTQSPSSLTVTAGEKVTMSCKSSQSLLNSGNQKNYLAWYQQKPGQPPKLLIYGASTRESGVPDRFTGSGSGTDFTLTISSVQAEDLADYFCQQHYSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |

| | | | | |
|---|---|---|---|---|
| 1609. | ME06D1 HL x I2C HL | murine | nt | GAGGTGCAGCTGCTCGAGCAGTCTGGAGCTGAGGTGGTTAGGCCTGGGACTTCAGTGAAGATGTCCTGCAAGGCTGCTGGATACACCTTCACTAAGTACTGGATAGGTTGGGTAAAGCAGAGGCCTGGACATGGCCTTGAGTGGATTGGAGATATTCACCCTGGAGGTGGTTTTGCTAACTACAATGAGAAGTTCAAGGGCAAGGCCACACTGACTGCAGACACATCCTCCAGCACAGCCTACATGCAGCTCAGCAGACTGACATCTGATGACTCTGCCATCTATTACTGTGCAAGATCGGGTTATGACTACGGTAGTAGTTACGATTACCATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGGCCTGGTACCAGCAGAAACCAGGGCAGCCTCCTAAACTGTTGATCTACGGGGCATCCACTAGGGAATCTGGAGTCCCTGATCGCTTCACAGGCAGTGGATCTGGGACAGATTTCACTCTTACCATCAGCAGTGTGCAGGCTGAAGACCTGGCAGATTACTTCTGTCAGCAACATTATAGCACTCCGTACACGTTCGGAGGGGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1610. | ME06D2-H | murine | aa | EVQLLEQSGADLVRPGTSVKLSCKAAGYTFSRYWISWIRQRPGHGLEWIGEIYPGGGYGNYNENFKDKVTLTADTSSSTAYMQLSSLTSEDSAIYYCARSGYDYGSKYDYYGLDYWGQGTTVTVSS |
| 1611. | ME06D2-HCDR1 | murine | aa | RYWIS |
| 1612. | ME06D2-HCDR2 | murine | aa | EIYPGGGYGNYNENFKD |
| 1613. | ME06D2-HCDR3 | murine | aa | SGYDYGSKYDYYGLDY |
| 1614. | ME06D2-H | murine | nt | GAGGTGCAGCTGCTCGAGCAGTCTGGAGCTGACCTGGTAAGGCCTGGGACTTCAGTGAAGCTGTCCTGCAAGGCTGCTGGATACACCTTCAGTAGGTATTGGATAAGTTGG |

| | | | | |
|---|---|---|---|---|
| | | | | ATAAGGCAGAGGCCTGGACATGGCCTTGAGTGGATTGGAGAAATTTACCCTGGAGGTGGTTATGGTAACTACAATGAGAATTTCAAGGACAAGGTCACACTGACTGCAGACACATCCTCCAGCACAGCCTATATGCAGCTCAGCAGCCTGACATCTGAGGACTCTGCCATCTATTATTGTGCAAGATCGGGGTATGACTACGGCAGTAAGTACGATTATTATGGTTTGGACTACTGGGGCCAAGGGACCACGGTCACCGTCTCCTCA |
| 1615. | ME06D2-L | murine | aa | ELVMTQSPSSMYASLGERVTITCKASQDINSYLSWFQQKPGKSPKTLIYRANRLVDGVPSRFSGSGSGQDYSLTISSLEYEDMGIYYCLQYDAFPPTFGAGTKLEIK |
| 1616. | ME06D2-LCDR1 | murine | aa | KASQDINSYLS |
| 1617. | ME06D2-LCDR2 | murine | aa | RANRLVD |
| 1618. | ME06D2-LCDR3 | murine | aa | LQYDAFPPT |
| 1619. | ME06D2-L | murine | nt | GAGCTCGTGATGACACAGTCTCCATCTTCCATGTATGCATCTCTAGGAGAGAGAGTCACTATCACTTGCAAGGCGAGTCAGGACATTAATAGCTATTTAAGCTGGTTCCAGCAGAAACCAGGGAAATCTCCTAAGACCCTGATCTATCGTGCAAACAGATTGGTAGATGGGGTCCCATCAAGGTTCAGTGGCAGTGGATCTGGGCAAGATTATTCTCTCACCATCAGCAGCCTGGAGTATGAAGATATGGGAATTTATTATTGTCTACAGTATGATGCGTTTCCTCCCACGTTCGGTGCTGGGACCAAGCTTGAGATCAAA |
| 1620. | ME06D2-HL | murine | aa | EVQLLEQSGADLVRPGTSVKLSCKAAGYTFSRYWISWIRQRPGHGLEWIGEIYPGGGYGNYNENFKDKVTLTADTSSSTAYMQLSSLTSEDSAIYYCARSGYDYGSKYDYYGLDYWGQGTTVTVSSGGGGSGGGGSGGGGSELVMTQSPSSMYASLGERVTITCKASQDINSYLSWFQQKPGKSPKTLIYRANRLVDGVPSRFSGSGSGQDYSLTISSLEYEDMGIYYCLQYDAFPPTFGAGTKLEIK |
| 1621. | ME06D2-HL | murine | nt | GAGGTGCAGCTGCTCGAGCAGTCTGGAGCTGACCTGGTAAGGCCTGGGACTTCAGTGAAGCTGTCCTGCAAGGCTGCTGGATACACCTTCAGTAGGTATTGGATAAGTTGGATAAGGCAGAGGCCTGGACATGGCCTTGAGTGGATTGGAGAAATTTACCCTGGAGGTGGTTATGGTAACTACAATGAGAATTTCAAGGACAAGGTCACACTGACTGCAGACACATCCTCCAGCACAGCCTATATGCAGCTCAGCAGCCTGACATCTGAGGACTCTGCCATCTATTATTGTGCAAGATCGGGGTATGACTACGGCAGTAAGTACGATTATTATGGTTTGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCTTCCATGTATGCATCTCTAGGAGAGAGAGTCACTATCACTTGCAAGGCGAGTCAGGACATTAATAGCTATTTAAGCTGGTTCCAGCAGAAACCAGGGAAATCTCCTAAGACCCTGATCTATCGTGCAAACAGATTGGTAGATGGGGTCCCATCAAGGTTCAGTGGCAGTGGATCTGGGCAAGATTATTCTCTCACCATCAGCAGCCTGGAGTATGAAGATATGGGAATTTATTATTGTCTACAGTATGATGCGTTTCCTCCCACGTTCGGTGCTGGGACCAAGCTTGAGATCAAA |
| 1622. | ME06D2 HL x I2C | murine | aa | EVQLLEQSGADLVRPGTSVKLSCKAAGYTFSRYWISWIRQRPGHGLEWIGEIYPGGGY |

| | | | | |
|---|---|---|---|---|
| | HL | | | GNYNENFKDKVTLTADTSSSTAYMQLSSLTSEDSAIYYCARSGYDYGSKYDYYGLDYWGQGTTVTVSSGGGGSGGGGSGGGGSELVMTQSPSSMYASLGERVTITCKASQDINSYLSWFQQKPGKSPKTLIYRANRLVDGVPSRFSGSGSGQDYSLTISSLEYEDMGIYYCLQYDAFPPTFGAGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1623. | ME06D2 HL x I2C HL | murine | nt | GAGGTGCAGCTGCTCGAGCAGTCTGGAGCTGACCTGGTAAGGCCTGGGACTTCAGTGAAGCTGTCCTGCAAGGCTGCTGGATACACCTTCAGTAGGTATTGGATAAGTTGGATAAGGCAGAGGCCTGGACATGGCCTTGAGTGGATTGGAGAAATTTACCCTGGAGGTGGTTATGGTAACTACAATGAGAATTTCAAGGACAAGGTCACACTGACTGCAGACACATCCTCCAGCACAGCCTATATGCAGCTCAGCAGCCTGACATCTGAGGACTCTGCCATCTATTATTGTGCAAGATCGGGGTATGACTACGGCAGTAAGTACGATTATTATGGTTTGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCTTCCATGTATGCATCTCTAGGAGAGAGAGTCACTATCACTTGCAAGGCGAGTCAGGACATTAATAGCTATTTAAGCTGGTTCCAGCAGAAACCAGGGAAATCTCCTAAGACCCTGATCTATCGTGCAAACAGATTGGTAGATGGGGTCCCATCAAGGTTCAGTGGCAGTGGATCTGGGCAAGATTATTCTCTCACCATCAGCAGCCTGGAGTATGAAGATATGGGAATTTATTATTGTCTACAGTATGATGCGTTTCCTCCCACGTTCGGTGCTGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1624. | ME06E10-H | murine | aa | EVQLLEQSGADLVRPGTSVKLSCKAAGYTFSRYWISWIRQRPGHGLEWIGEIYPGGGY |

| | | | | |
|---|---|---|---|---|
| | | | | GNYNENFKDKVTLTADTSSSTAYMQLSSLTSEDSAIYYCARSGYDYGSKYDYYGLDYW GQGTTVTVSS |
| 1625. | ME06E10-HCDR1 | murine | aa | RYWIS |
| 1626. | ME06E10-HCDR2 | murine | aa | EIYPGGGYGNYNENFKD |
| 1627. | ME06E10-HCDR3 | murine | aa | SGYDYGSKYDYYGLDY |
| 1628. | ME06E10-H | murine | nt | GAGGTGCAGCTGCTCGAGCAGTCTGGAGCTGACCTGGTAAGGCCTGGGACTTCAG TGAAGCTGTCCTGCAAGGCTGCTGGATACACCTTCAGTAGGTATTGGATAAGTTGG ATAAGGCAGAGGCCTGGACATGGCCTTGAGTGGATTGGAGAAATTTACCCTGGAGG TGGTTATGGTAACTACAATGAGAATTTCAAGGACAAGGTCACACTGACTGCAGACAC ATCCTCCAGCACAGCCTATATGCAGCTCAGCAGCCTGACATCTGAGGACTCTGCCA TCTATTATTGTGCAAGATCGGGGTATGACTACGGCAGTAAGTACGATTATTATGGTT TGGACTACTGGGGCCAAGGGACCACGGTCACCGTCTCCTCA |
| 1629. | ME06E10-L | murine | aa | ELVMTQSPSSLSASLGERVSLTCRASQEISGYLSWLQQKPDGTIKRLIYAASTLDSGVPK RFSGSRSGSDYSLTISSLESEDFADYYCLQYASSPYTFGGGTKLEIK |
| 1630. | ME06E10-LCDR1 | murine | aa | RASQEISGYLS |
| 1631. | ME06E10-LCDR2 | murine | aa | AASTLDS |
| 1632. | ME06E10-LCDR3 | murine | aa | LQYASSPYT |
| 1633. | ME06E10-L | murine | nt | GAGCTCGTCATGACCCAGTCTCCATCCTCCTTATCTGCCTCTCTGGGAGAAAGAGT CAGTCTCACTTGTCGGGCAAGTCAGGAAATTAGTGGTTACTTAAGCTGGCTTCAGC AGAAACCAGATGGAACTATTAAACGCCTGATCTACGCCGCATCCACTTTAGATTCTG GTGTCCCAAAAAGGTTCAGTGGCAGTAGGTCTGGGTCAGATTATTCTCTCACCATCA GCAGCCTTGAGTCTGAAGATTTTGCAGACTATTACTGTCTACAATATGCTAGTTCTC CGTACACGTTCGGAGGGGGGACCAAGCTTGAGATCAAA |
| 1634. | ME06E10-HL | murine | aa | EVQLLEQSGADLVRPGTSVKLSCKAAGYTFSRYWISWIRQRPGHGLEWIGEIYPGGGY GNYNENFKDKVTLTADTSSSTAYMQLSSLTSEDSAIYYCARSGYDYGSKYDYYGLDYW GQGTTVTVSSGGGGSGGGGSGGGGSELVMTQSPSSLSASLGERVSLTCRASQEISGY LSWLQQKPDGTIKRLIYAASTLDSGVPKRFSGSRSGSDYSLTISSLESEDFADYYCLQYA SSPYTFGGGTKLEIK |
| 1635. | ME06E10-HL | murine | nt | GAGGTGCAGCTGCTCGAGCAGTCTGGAGCTGACCTGGTAAGGCCTGGGACTTCAG TGAAGCTGTCCTGCAAGGCTGCTGGATACACCTTCAGTAGGTATTGGATAAGTTGG ATAAGGCAGAGGCCTGGACATGGCCTTGAGTGGATTGGAGAAATTTACCCTGGAGG TGGTTATGGTAACTACAATGAGAATTTCAAGGACAAGGTCACACTGACTGCAGACAC ATCCTCCAGCACAGCCTATATGCAGCTCAGCAGCCTGACATCTGAGGACTCTGCCA TCTATTATTGTGCAAGATCGGGGTATGACTACGGCAGTAAGTACGATTATTATGGTT TGGACTACTGGGGCCAAGGGACCACGGTCACCGTCTCCTCAGGTGGTGGTGGTTC TGGCGGCGGCGGCTCCGGTGGTGGTGGTTCTGAGCTCGTCATGACCCAGTCTCCA |

| | | | | |
|---|---|---|---|---|
| | | | | TCCTCCTTATCTGCCTCTCTGGGAGAAAGAGTCAGTCTCACTTGTCGGGCAAGTCAGGAAATTAGTGGTTACTTAAGCTGGCTTCAGCAGAAACCAGATGGAACTATTAAACGCCTGATCTACGCCGCATCCACTTTAGATTCTGGTGTCCCAAAAAGGTTCAGTGGCAGTAGGTCTGGGTCAGATTATTCTCTCACCATCAGCAGCCTTGAGTCTGAAGATTTTGCAGACTATTACTGTCTACAATATGCTAGTTCTCCGTACACGTTCGGAGGGGGGACCAAGCTTGAGATCAAA |
| 1636. | ME06E10 HL x I2C HL | murine | aa | EVQLLEQSGADLVRPGTSVKLSCKAAGYTFSRYWISWIRQRPGHGLEWIGEIYPGGGYGNYNENFKDKVTLTADTSSSTAYMQLSSLTSEDSAIYYCARSGYDYGSKYDYYGLDYWGQGTTVTVSSGGGGSGGGGSGGGGSELVMTQSPSSLSASLGERVSLTCRASQEISGYLSWLQQKPDGTIKRLIYAASTLDSGVPKRFSGSRSGSDYSLTISSLESEDFADYYCLQYASSPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1637. | ME06E10 HL x I2C HL | murine | nt | GAGGTGCAGCTGCTCGAGCAGTCTGGAGCTGACCTGGTAAGGCCTGGGACTTCAGTGAAGCTGTCCTGCAAGGCTGCTGGATACACCTTCAGTAGGTATTGGATAAGTTGGATAAGGCAGAGGCCTGGACATGGCCTTGAGTGGATTGGAGAAATTTACCCTGGAGGTGGTTATGGTAACTACAATGAGAATTTCAAGGACAAGGTCACACTGACTGCAGACACATCCTCCAGCACAGCCTATATGCAGCTCAGCAGCCTGACATCTGAGGACTCTGCCATCTATTATTGTGCAAGATCGGGGTATGACTACGGCAGTAAGTACGATTATTATGGTTTGGACTACTGGGGCCAAGGGACCACGGTCACCGTCTCCTCAGGTGGTGGTGGTTCTGGCGGCGGCGGCTCCGGTGGTGGTGGTTCTGAGCTCGTCATGACCCAGTCTCCATCCTCCTTATCTGCCTCTCTGGGAGAAAGAGTCAGTCTCACTTGTCGGGCAAGTCAGGAAATTAGTGGTTACTTAAGCTGGCTTCAGCAGAAACCAGATGGAACTATTAAACGCCTGATCTACGCCGCATCCACTTTAGATTCTGGTGTCCCAAAAAGGTTCAGTGGCAGTAGGTCTGGGTCAGATTATTCTCTCACCATCAGCAGCCTTGAGTCTGAAGATTTTGCAGACTATTACTGTCTACAATATGCTAGTTCTCCGTACACGTTCGGAGGGGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGT |

| | | | | |
|---|---|---|---|---|
| | | | | GGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1638. | ME06F2-H | murine | aa | EVQLLEQSGAELMKPGASVKISCKATGYTFSNYWIGWVKQRPGHGLEWIGDIYPGGSYTNYNEKFKGKVTLTADTSSRTAYMQLSSLTFEDSAIYYCARSGYDYASDYDYHGMDYWGQGTTVTVSS |
| 1639. | ME06F2-HCDR1 | murine | aa | NYWIG |
| 1640. | ME06F2-HCDR2 | murine | aa | DIYPGGSYTNYNEKFKG |
| 1641. | ME06F2-HCDR3 | murine | aa | SGYDYASDYDYHGMDY |
| 1642. | ME06F2-H | murine | nt | GAGGTGCAGCTGCTCGAGCAGTCTGGAGCTGAGCTGATGAAGCCTGGGGCCTCAGTGAAGATATCCTGCAAGGCTACTGGATACACCTTCAGTAATTACTGGATAGGTTGGGTAAAGCAGAGGCCTGGACATGGCCTTGAGTGGATTGGAGATATTTACCCTGGAGGTAGTTATACTAACTACAATGAGAAATTCAAGGGCAAGGTCACACTGACTGCAGACACATCCTCCAGGACAGCCTACATGCAGCTCAGCAGCCTGACATTTGAGGACTCTGCCATCTATTACTGTGCAAGATCGGGGTATGACTACGCTAGTGACTACGATTACCATGGTATGGACTACTGGGGCCAAGGGACCACGGTCACCGTCTCCTCA |
| 1643. | ME06F2-L | murine | aa | ELVLTQSPSSMSASLGDRITITCQATQDIVKNLNWYQQKPGKPPSFLIYYATELAEGVPSRFSGSGSGSDYSLTISNLESEDFADYYCLQFYEFPYTFGGGTKLEIK |
| 1644. | ME06F2-LCDR1 | murine | aa | QATQDIVKNLN |
| 1645. | ME06F2-LCDR2 | murine | aa | YATELAE |
| 1646. | ME06F2-LCDR3 | murine | aa | LQFYEFPYT |
| 1647. | ME06F2-L | murine | nt | GAGCTCGTGCTCACCCAGTCTCCATCCTCTATGTCTGCATCTCTGGGAGACAGAATAACCATCACTTGCCAGGCAACTCAAGACATTGTTAAGAATTTAAACTGGTATCAGCAGAAACCAGGGAAACCCCCTTCATTCCTGATCTATTATGCAACTGAACTGGCAGAAGGGGTCCCATCAAGGTTCAGTGGCAGTGGGTCTGGGTCAGACTATTCTCTGACAATCAGCAACCTGGAGTCTGAAGATTTTGCAGACTATTACTGTCTACAGTTTTATGAGTTTCCGTACACGTTCGGAGGGGGGACCAAGCTTGAGATCAAA |
| 1648. | ME06F2-HL | murine | aa | EVQLLEQSGAELMKPGASVKISCKATGYTFSNYWIGWVKQRPGHGLEWIGDIYPGGSYTNYNEKFKGKVTLTADTSSRTAYMQLSSLTFEDSAIYYCARSGYDYASDYDYHGMDYWGQGTTVTVSSGGGGSGGGGSGGGGSELVLTQSPSSMSASLGDRITITCQATQDIVKNLNWYQQKPGKPPSFLIYYATELAEGVPSRFSGSGSGSDYSLTISNLESEDFADYYCLQFYEFPYTFGGGTKLEIK |
| 1649. | ME06F2-HL | murine | nt | GAGGTGCAGCTGCTCGAGCAGTCTGGAGCTGAGCTGATGAAGCCTGGGGCCTCAG |

| | | | | |
|---|---|---|---|---|
| | | | | TGAAGATATCCTGCAAGGCTACTGGATACACCTTCAGTAATTACTGGATAGGTTGGGTAAAGCAGAGGCCTGGACATGGCCTTGAGTGGATTGGAGATATTTACCCTGGAGGTAGTTATACTAACTACAATGAGAAATTCAAGGGCAAGGTCACACTGACTGCAGACACATCCTCCAGGACAGCCTACATGCAGCTCAGCAGCCTGACATTTGAGGACTCTGCCATCTATTACTGTGCAAGATCGGGGTATGACTACGCTAGTGACTACGATTACCATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGCTCACCCAGTCTCCATCCTCTATGTCTGCATCTCTGGGAGACAGAATAACCATCACTTGCCAGGCAACTCAAGACATTGTTAAGAATTTAAACTGGTATCAGCAGAAACCAGGGAAACCCCCTTCATTCCTGATCTATTATGCAACTGAACTGGCAGAAGGGGTCCCATCAAGGTTCAGTGGCAGTGGGTCTGGGTCAGACTATTCTCTGACAATCAGCAACCTGGAGTCTGAAGATTTTGCAGACTATTACTGTCTACAGTTTTATGAGTTTCCGTACACGTTCGGAGGGGGGACCAAGCTTGAGATCAAA |
| 1650. | ME06F2 HL x I2C HL | murine | aa | EVQLLEQSGAELMKPGASVKISCKATGYTFSNYWIGWVKQRPGHGLEWIGDIYPGGSYTNYNEKFKGKVTLTADTSSRTAYMQLSSLTFEDSAIYYCARSGYDYASDYDYHGMDYWGQGTTVTVSSGGGGSGGGGSGGGGSELVLTQSPSSMSASLGDRITITCQATQDIVKNLNWYQQKPGKPPSFLIYYATELAEGVPSRFSGSGSGSDYSLTISNLESEDFADYYCLQFYEFPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1651. | ME06F2 HL x I2C HL | murine | nt | GAGGTGCAGCTGCTCGAGCAGTCTGGAGCTGAGCTGATGAAGCCTGGGGCCTCAGTGAAGATATCCTGCAAGGCTACTGGATACACCTTCAGTAATTACTGGATAGGTTGGGTAAAGCAGAGGCCTGGACATGGCCTTGAGTGGATTGGAGATATTTACCCTGGAGGTAGTTATACTAACTACAATGAGAAATTCAAGGGCAAGGTCACACTGACTGCAGACACATCCTCCAGGACAGCCTACATGCAGCTCAGCAGCCTGACATTTGAGGACTCTGCCATCTATTACTGTGCAAGATCGGGGTATGACTACGCTAGTGACTACGATTACCATGGTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGCTCACCCAGTCTCCATCCTCTATGTCTGCATCTCTGGGAGACAGAATAACCATCACTTGCCAGGCAACTCAAGACATTGTTAAGAATTTAAACTGGTATCAGCAGAAACCAGGGAAACCCCCTTCATTCCTGATCTATTATGCAACTGAACTGGCAGAAGGGGTCCCATCAAGGTTCAGTGGCAGTGGGTCTGGGTCAGACTATTCTCTGACAATCAGCAACCTGGAGTCTGAAGATTTTGCAGACTATTACTGTCTACAGTTTTATGAGTTTCCGTACACGTTCGGAGGGGGGACCAAGCTTGAGATCAAATCCGGAGGTGGTGGATCCGAGGTGCAGCTGGTCGAGTCTGGA |

| | | | | |
|---|---|---|---|---|
| | | | | GGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGG AATGGGTTGCTCGCATAAGAAGTAAATATAATAATTATGCAACATATTATGCCGATTC AGTGAAAGACAGGTTCACCATCTCCAGAGATGATTCAAAAAACACTGCCTATCTACA AATGAACAACTTGAAAACTGAGGACACTGCCGTGTACTACTGTGTGAGACATGGGA ACTTCGGTAATAGCTACATATCCTACTGGGCTTACTGGGGCCAAGGGACTCTGGTC ACCGTCTCCTCAGGTGGTGGTGGTTCTGGCGGCGGCGGCTCCGGTGGTGGTGGTT CTCAGACTGTTGTGACTCAGGAACCTTCACTCACCGTATCACCTGGTGGAACAGTC ACACTCACTTGTGGCTCCTCGACTGGGGCTGTTACATCTGGCAACTACCCAAACTG GGTCCAACAAAAACCAGGTCAGGCACCCCGTGGTCTAATAGGTGGGACTAAGTTCC TCGCCCCCGGTACTCCTGCCAGATTCTCAGGCTCCCTGCTTGGAGGCAAGGCTGC CCTCACCCTCTCAGGGGTACAGCCAGAGGATGAGGCAGAATATTACTGTGTTCTAT GGTACAGCAACCGCTGGGTGTTCGGTGGAGGAACCAAACTGACTGTCCTA |
| 1652. | EN00B12-H | artificial | aa | QVQLVQSGPEVKKPGASVKLSCKASGYTFTDYVIHWVKQAPGQRLEWMGYINPYDDD TTYNQKFKGRVTITVDTSASTAYMELNSLRSEDTAVYYCARRGNSYDGYFDYSMDYW GQGTLVTVSS |
| 1653. | EN00B12-HCDR1 | artificial | aa | DYVIH |
| 1654. | EN00B12-HCDR2 | artificial | aa | YINPYDDDTTYNQKFKG |
| 1655. | EN00B12-HCDR3 | artificial | aa | RGNSYDGYFDYSMDY |
| 1656. | EN00B12-H | artificial | nt | CAGGTGCAGCTGGTCCAGTCAGGACCTGAGGTGAAGAAGCCTGGGGCTTCAGTGA AGTTGTCCTGCAAGGCTTCTGGATACACATTCACTGACTATGTTATACACTGGGTGA AGCAGGCACCTGGACAGCGCCTTGAGTGGATGGGATATATTAATCCTTATGATGAT GATACTACCTACAACCAGAAGTTCAAGGGCCGGGTCACAATTACTGTAGACACATC CGCCAGCACAGCCTACATGGAGCTCAACAGCCTGAGATCTGAGGACACTGCAGTCT ATTACTGTGCAAGAAGGGGGAACTCCTATGATGGTTACTTCGACTATTCTATGGACT ACTGGGGTCAAGGAACCTTGGTCACCGTCTCCTCA |
| 1657. | EN00B12-L | artificial | aa | DIQLTQSPSFLSASVGDRVTITCRASQNVGTAVAWYQQKPGKAPKLLIYSASNRYTGVP SRFSGSGSGTEFTLTISSLQPEDFATYYCQQYTNYPMYTFGQGTKLEIK |
| 1658. | EN00B12-LCDR1 | artificial | aa | RASQNVGTAVA |
| 1659. | EN00B12-LCDR2 | artificial | aa | SASNRYT |
| 1660. | EN00B12-LCDR3 | artificial | aa | QQYTNYPMYT |
| 1661. | EN00B12-L | artificial | nt | GACATCCAGCTGACCCAGTCTCCAAGCTTCCTGTCCGCATCAGTAGGAGACAGAGT CACCATCACCTGCAGGGCCAGTCAGAATGTGGGTACTGCTGTAGCCTGGTATCAAC AGAAACCAGGAAAAGCCCCTAAATTACTGATTTACTCGGCATCGAATCGGTACACTG GAGTCCCTAGTCGCTTCTCAGGCAGTGGATCTGGGACAGAGTTCACTCTCACCATC |

| | | | | |
|---|---|---|---|---|
| | | | | AGCAGTCTGCAGCCTGAAGACTTCGCAACTTATTACTGCCAGCAATATACCAACTATCCCATGTATACGTTTGGACAGGGGACCAAGCTGGAAATAAAA |
| 1662. | EN00B12-HL | artificial | aa | QVQLVQSGPEVKKPGASVKLSCKASGYTFTDYVIHWVKQAPGQRLEWMGYINPYDDDTTYNQKFKGRVTITVDTSASTAYMELNSLRSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSLQPEDFATYYCQQYTNYPMYTFGQGTKLEIK |
| 1663. | EN00B12-HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGGTGAAGCAGGCACCTGGACAGCGCCTTGAGTGGATGGGATATATTAATCCTTATGATGATGATACTACCTACAACCAGAAGTTCAAGGGCCGGGTCACAATTACTGTAGACACATCCGCCAGCACAGCCTACATGGAGCTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGCCTGAAGACTTCGCAACTTATTACTGCCAGCAATATACCAACTATCCCATGTATACGTTTGGACAGGGGACCAAGCTGGAAATAAAA |
| 1664. | EN00B12 HL x I2C HL | artificial | aa | QVQLVQSGPEVKKPGASVKLSCKASGYTFTDYVIHWVKQAPGQRLEWMGYINPYDDDTTYNQKFKGRVTITVDTSASTAYMELNSLRSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSLQPEDFATYYCQQYTNYPMYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1665. | EN00B12 HL x I2C HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGGTGAAGCAGGCACCTGGACAGCGCCTTGAGTGGATGGGATATATTAATCCTTATGATGATGATACTACCTACAACCAGAAGTTCAAGGGCCGGGTCACAATTACTGTAGACACATCCGCCAGCACAGCCTACATGGAGCTCAACAGCCTGAGATCTGAGGACACTGCAGTCTATTACTGTGCAAGAAGGGGGAACTCCTATGATGGTTACTTCGACTATTCTATGGACT |

| | | | | |
|---|---|---|---|---|
| | | | | ACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGCCTGAAGACTTCGCAACTTATTACTGCCAGCAATATACCAACTATCCCATGTATACGTTTGGACAGGGGACCAAGCTGGAAATAAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1666. | EN00C3-H | artificial | aa | QVQLVQSGPEVKKPGASVKMSCKASGYTFTDYVIHWVRQGPGQRLEWMGYINPYDDDTTYNQKFKGRVTMTLDKSASTAYMQLNSLRSEDTAVYYCARRGNSYDGYFDYSMDYWGQGTLVTVSS |
| 1667. | EN00C3-HCDR1 | artificial | aa | DYVIH |
| 1668. | EN00C3-HCDR2 | artificial | aa | YINPYDDDTTYNQKFKG |
| 1669. | EN00C3-HCDR3 | artificial | aa | RGNSYDGYFDYSMDY |
| 1670. | EN00C3-H | artificial | nt | CAGGTGCAGCTGGTCCAGTCAGGACCTGAGGTGAAGAAGCCTGGGGCTTCAGTGAAGATGTCCTGCAAGGCTTCTGGATACACATTCACTGACTATGTTATACACTGGGTGAGGCAGGGACCTGGACAGCGTCTTGAGTGGATGGGATATATTAATCCTTATGATGATGATACTACCTACAACCAGAAGTTCAAGGGCCGGGTCACAATGACTCTAGACAAATCCGCCAGCACAGCCTACATGCAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCA |
| 1671. | EN00C3-L | artificial | aa | DIQLTQSPNFLSASVGDRVTITCRASQNVGTAVAWYQQKPGKAPKLLIYSASNRYTGVPSRFSGSGSGTDFTLTISSLQPEDFATYYCQQYTNYPMYTFGQGTKLEIK |

| | | | | |
|---|---|---|---|---|
| 1672. | EN00C3-LCDR1 | artificial | aa | RASQNVGTAVA |
| 1673. | EN00C3-LCDR2 | artificial | aa | SASNRYT |
| 1674. | EN00C3-LCDR3 | artificial | aa | QQYTNYPMYT |
| 1675. | EN00C3-L | artificial | nt | GACATCCAGCTGACCCAGTCTCCAAACTTCCTGTCCGCATCAGTAGGAGACAGAGT CACCATCACCTGCAGGGCCAGTCAGAATGTGGGTACTGCTGTAGCCTGGTATCAAC AGAAACCAGGAAAAGCCCCTAAATTACTGATTTACTCGGCATCGAATCGGTACACTG GAGTCCCTAGTCGCTTCTCAGGCAGTGGATCTGGGACAGATTTCACTCTCACCATC AGCAGTCTGCAGCCTGAAGACTTCGCAACTTATTACTGCCAGCAATATACCAACTAT CCCATGTATACGTTTGGACAGGGGACCAAGCTGGAAATAAAA |
| 1676. | EN00C3-HL | artificial | aa | QVQLVQSGPEVKKPGASVKMSCKASGYTFTDYVIHWVRQGPGQRLEWMGYINPYDD DTTYNQKFKGRVTMTLDKSASTAYMQLNSLRSEDTAVYYCARRGNSYDGYFDYSMDY WGQGTLVTVSSGGGGSGGGGSGGGGSDIQLTQSPNFLSASVGDRVTITCRASQNVGT AVAWYQQKPGKAPKLLIYSASNRYTGVPSRFSGSGSGTDFTLTISSLQPEDFATYYCQQ YTNYPMYTFGQGTKLEIK |
| 1677. | EN00C3-HL | artificial | nt | CAGGTGCAGCTGGTCCAGTCAGGACCTGAGGTGAAGAAGCCTGGGGCTTCAGTGA AGATGTCCTGCAAGGCTTCTGGATACACATTCACTGACTATGTTATACACTGGGTGA GGCAGGGACCTGGACAGCGTCTTGAGTGGATGGGATATATTAATCCTTATGATGAT GATACTACCTACAACCAGAAGTTCAAGGGCCGGGTCACAATGACTCTAGACAAATC CGCCAGCACAGCCTACATGCAACTCAACAGCCTGAGATCTGAGGACACTGCAGTCT ATTACTGTGCAAGAAGGGGGAACTCCTATGATGGTTACTTCGACTATTCTATGGACT ACTGGGGTCAAGGAACCTTGGTCACCGTCTCCTCAGGTGGTGGTGGTTCTGGCGG CGGCGGCTCCGGTGGTGGTGGTTCTGACATCCAGCTGACCCAGTCTCCAAACTTC CTGTCCGCATCAGTAGGAGACAGAGTCACCATCACCTGCAGGGCCAGTCAGAATGT GGGTACTGCTGTAGCCTGGTATCAACAGAAACCAGGAAAAGCCCCTAAATTACTGA TTTACTCGGCATCGAATCGGTACACTGGAGTCCCTAGTCGCTTCTCAGGCAGTGGA TCTGGGACAGATTTCACTCTCACCATCAGCAGTCTGCAGCCTGAAGACTTCGCAAC TTATTACTGCCAGCAATATACCAACTATCCCATGTATACGTTTGGACAGGGGACCAA GCTGGAAATAAAA |
| 1678. | EN00C3 HL x I2C HL | artificial | aa | QVQLVQSGPEVKKPGASVKMSCKASGYTFTDYVIHWVRQGPGQRLEWMGYINPYDD DTTYNQKFKGRVTMTLDKSASTAYMQLNSLRSEDTAVYYCARRGNSYDGYFDYSMDY WGQGTLVTVSSGGGGSGGGGSGGGGSDIQLTQSPNFLSASVGDRVTITCRASQNVGT AVAWYQQKPGKAPKLLIYSASNRYTGVPSRFSGSGSGTDFTLTISSLQPEDFATYYCQQ YTNYPMYTFGQGTKLEIKSGGGGSEVQLVESGGGLVQPGGSLKLSCAASGFTFNKYA MNWVRQAPGKGLEWVARIRSKYNNYATYYADSVKDRFTISRDDSKNTAYLQMNNLKT EDTAVYYCVRHGNFGNSYISYWAYWGQGTLVTVSSGGGGSGGGGSGGGGSQTVVT QEPSLTVSPGGTVTLTCGSSTGAVTSGNYPNWVQQKPGQAPRGLIGGTKFLAPGTPA |

| | | | | |
|---|---|---|---|---|
| | | | | RFSGSLLGGKAALTLSGVQPEDEAEYYCVLWYSNRWVFGGGTKLTVL |
| 1679. | EN00C3 HL x I2C HL | artificial | nt | CAGGTGCAGCTGGTCCAGTCAGGACCTGAGGTGAAGAAGCCTGGGGCTTCAGTGAAGATGTCCTGCAAGGCTTCTGGATACACATTCACTGACTATGTTATACACTGGGTGAGGCAGGGACCTGGACAGCGTCTTGAGTGGATGGGATATATTAATCCTTATGATGATGATACTACCTACAACCAGAAGTTCAAGGGCCGGGTCACAATGACTCTAGACAAATCCGCCAGCACAGCCTACATGCAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAACTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGATTTCACTCTCACCATCAGCAGTCTGCAGCCTGAAGACTTCGCAACTTATTACTGCCAGCAATATACCAACTATCCCATGTATACGTTTGGACAGGGGACCAAGCTGGAAATAAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1680. | EN01D5-H | artificial | aa | QVQLVQSGPEVMKPGASVKMSCKASGYTFTDYVIHWVRQAPGKRLEWMGYINPYDDDTTYNQKFKGRVTITVDKSASTAYMELNSLTSEDTAVYYCARRGNSYDGYFDYSMDYWGQGTLVTVSS |
| 1681. | EN01D5-HCDR1 | artificial | aa | DYVIH |
| 1682. | EN01D5-HCDR2 | artificial | aa | YINPYDDDTTYNQKFKG |
| 1683. | EN01D5-HCDR3 | artificial | aa | RGNSYDGYFDYSMDY |
| 1684. | EN01D5-H | artificial | nt | CAGGTGCAGCTGGTCCAGTCAGGACCTGAGGTGATGAAGCCTGGGGCTTCAGTGAAGATGTCCTGCAAGGCTTCTGGATACACATTCACTGACTATGTTATACACTGGGTGA |

| | | | | |
|---|---|---|---|---|
| | | | | GGCAGGCACCTGGAAAGCGCCTTGAGTGGATGGGATATATTAATCCTTATGATGATGATACTACCTACAACCAGAAGTTCAAGGGCCGGGTCACAATTACTGTAGACAAATCCGCCAGCACAGCCTACATGGAACTCAACAGCCTGACATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCA |
| 1685. | EN01D5-L | artificial | aa | DIQLTQSPSFMSASVGDRVTITCRASQNVGTAVAWYQQKPGQAPKLLIYSASNRYTGVPSRFSGSGSGTEFTLTISSLQSEDFATYYCQQYTNYPMYTFGQGTKLEIK |
| 1686. | EN01D5-LCDR1 | artificial | aa | RASQNVGTAVA |
| 1687. | EN01D5-LCDR2 | artificial | aa | SASNRYT |
| 1688. | EN01D5-LCDR3 | artificial | aa | QQYTNYPMYT |
| 1689. | EN01D5-L | artificial | nt | GACATCCAGCTGACCCAGTCTCCAAGCTTCATGTCCGCATCAGTAGGAGACAGAGTCACCATCACCTGCAGGGCCAGTCAGAATGTGGGTACTGCTGTAGCCTGGTATCAACAGAAACCAGGACAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGTCTGAAGACTTCGCAACTTATTACTGCCAGCAATATACCAACTATCCCATGTATACGTTTGGACAGGGGACCAAGCTGGAAATAAAA |
| 1690. | EN01D5-HL | artificial | aa | QVQLVQSGPEVMKPGASVKMSCKASGYTFTDYVIHWVRQAPGKRLEWMGYINPYDDDTTYNQKFKGRVTITVDKSASTAYMELNSLTSEDTAVYYCARRGNSYDGYFDYSMDYWGQGTLVTVSSGGGGSGGGGSGGGGSDIQLTQSPSFMSASVGDRVTITCRASQNVGTAVAWYQQKPGQAPKLLIYSASNRYTGVPSRFSGSGSGTEFTLTISSLQSEDFATYYCQQYTNYPMYTFGQGTKLEIK |
| 1691. | EN01D5-HL | artificial | nt | CAGGTGCAGCTGGTCCAGTCAGGACCTGAGGTGATGAAGCCTGGGGCTTCAGTGAAGATGTCCTGCAAGGCTTCTGGATACACATTCACTGACTATGTTATACACTGGGTGAGGCAGGCACCTGGAAAGCGCCTTGAGTGGATGGGATATATTAATCCTTATGATGATGATACTACCTACAACCAGAAGTTCAAGGGCCGGGTCACAATTACTGTAGACAAATCCGCCAGCACAGCCTACATGGAACTCAACAGCCTGACATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCATGTCCGCATCAGTAGGAGACAGAGTCACCATCACCTGCAGGGCCAGTCAGAATGTGGGTACTGCTGTAGCCTGGTATCAACAGAAACCAGGACAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGTCTGAAGACTTCGCAACTTATTACTGCCAGCAATATACCAACTATCCCATGTATACGTTTGGACAGGGGACCAAGCTGGAAATAAAA |
| 1692. | EN01D5 HL x I2C | artificial | aa | QVQLVQSGPEVMKPGASVKMSCKASGYTFTDYVIHWVRQAPGKRLEWMGYINPYDDD |

| | | | | |
|---|---|---|---|---|
| | HL | | | TTYNQKFKGRVTITVDKSASTAYMELNSLTSEDTAVYYCARRGNSYDGYFDYSMDYWGQGTLVTVSSGGGGSGGGGSGGGGSDIQLTQSPSFMSASVGDRVTITCRASQNVGTAVAWYQQKPGQAPKLLIYSASNRYTGVPSRFSGSGSGTEFTLTISSLQSEDFATYYCQQYTNYPMYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1693. | EN01D5 HL x I2C HL | artificial | nt | CAGGTGCAGCTGGTCCAGTCAGGACCTGAGGTGATGAAGCCTGGGGCTTCAGTGAAGATGTCCTGCAAGGCTTCTGGATACACATTCACTGACTATGTTATACACTGGGTGAGGCAGGCACCTGGAAAGCGCCTTGAGTGGATGGGATATATTAATCCTTATGATGATGATACTACCTACAACCAGAAGTTCAAGGGCCGGGTCACAATTACTGTAGACAAATCCGCCAGCACAGCCTACATGGAACTCAACAGCCTGACATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCATGTCCGCATCAGTAGGAGACAGAGTCACCATCACCTGCAGGGCCAGTCAGAATGTGGGTACTGCTGTAGCCTGGTATCAACAGAAACCAGGACAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGTCTGAAGACTTCGCAACTTATTACTGCCAGCAATATACCAACTATCCCATGTATACGTTTGGACAGGGGACCAAGCTGGAAATAAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |

| | | | | |
|---|---|---|---|---|
| 1694. | EN00E4-H | artificial | aa | QVQLVQSGPEVKKPGASVKLSCKASGYTFTDYVIHWMRQAPGQRLEWMGYINPYDDDTTYNQKFKGRVTITLDTSASTAYMELNSLRSEDTAVYYCARRGNSYDGYFDYSMDYWGQGTLVTVSS |
| 1695. | EN00E4-HCDR1 | artificial | aa | DYVIH |
| 1696. | EN00E4-HCDR2 | artificial | aa | YINPYDDDTTYNQKFKG |
| 1697. | EN00E4-HCDR3 | artificial | aa | RGNSYDGYFDYSMDY |
| 1698. | EN00E4-H | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGATGAGGCAGGCACCTGGACAGCGCCTTGAGTGGATGGGATATATTAATCCTTATGATGATGATACTACCTACAACCAGAAGTTCAAGGGCCGGGTCACAATTACTCTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCA |
| 1699. | EN00E4-L | artificial | aa | DIQLTQSPKFMSTSVGDKVTITCRASQNVGTAVAWYQQKPGKAPKLLIYSASNRYTGVPSRFSGSGSGTEFTLTISSLQPEDFANYFCQQYTNYPMYTFGQGTKLEIK |
| 1700. | EN00E4-LCDR1 | artificial | aa | RASQNVGTAVA |
| 1701. | EN00E4-LCDR2 | artificial | aa | SASNRYT |
| 1702. | EN00E4-LCDR3 | artificial | aa | QQYTNYPMYT |
| 1703. | EN00E4-L | artificial | nt | GACATCCAGCTGACCCAGTCTCCAAAATTCATGTCCACATCAGTAGGAGACAAAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGCCTGAAGACTTCGCAAATTATTTCTGCCAGCAATATACCAACTATCCCATGTATACGTTTGGACAGGGGACCAAGCTGGAAATAAAA |
| 1704. | EN00E4-HL | artificial | aa | QVQLVQSGPEVKKPGASVKLSCKASGYTFTDYVIHWMRQAPGQRLEWMGYINPYDDDTTYNQKFKGRVTITLDTSASTAYMELNSLRSEDTAVYYCARRGNSYDGYFDYSMDYWGQGTLVTVSSGGGGSGGGGSGGGGSDIQLTQSPKFMSTSVGDKVTITCRASQNVGTAVAWYQQKPGKAPKLLIYSASNRYTGVPSRFSGSGSGTEFTLTISSLQPEDFANYFCQQYTNYPMYTFGQGTKLEIK |
| 1705. | EN00E4-HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGATGAGGCAGGCACCTGGACAGCGCCTTGAGTGGATGGGATATATTAATCCTTATGATGATGATACTACCTACAACCAGAAGTTCAAGGGCCGGGTCACAATTACTCTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGG |

| | | | | |
|---|---|---|---|---|
| | | | | CGGCGGCTCCGGTGGTGGTGGTTCTGACATCCAGCTGACCCAGTCTCCAAAATTCATGTCCACATCAGTAGGAGACAAAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGCCTGAAGACTTCGCAAATTATTTCTGCCAGCAATATACCAACTATCCCATGTATACGTTTGGACAGGGGACCAAGCTGGAAATAAAA |
| 1706. | EN00E4 HL x I2C HL | artificial | aa | QVQLVQSGPEVKKPGASVKLSCKASGYTFTDYVIHWMRQAPGQRLEWMGYINPYDDDTTYNQKFKGRVTITLDTSASTAYMELNSLRSEDTAVYYCARRGNSYDGYFDYSMDYWGQGTLVTVSSGGGGSGGGGSGGGGSDIQLTQSPKFMSTSVGDKVTITCRASQNVGTAVAWYQQKPGKAPKLLIYSASNRYTGVPSRFSGSGSGTEFTLTISSLQPEDFANYFCQQYTNYPMYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1707. | EN00E4 HL x I2C HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGATGAGGCAGGCACCTGGACAGCGCCTTGAGTGGATGGGATATATTAATCCTTATGATGATGATACTACCTACAACCAGAAGTTCAAGGGCCGGGTCACAATTACTCTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAAATTCATGTCCACATCAGTAGGAGACAAAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGCCTGAAGACTTCGCAAATTATTTCTGCCAGCAATATACCAACTATCCCATGTATACGTTTGGACAGGGGACCAAGCTGGAAATAAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCAC |

| | | | | |
|---|---|---|---|---|
| | | | | CGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1708. | EN00F7-H | artificial | aa | QVQLVQSGPEVKKPGASVKLSCKASGYTFTDYVIHWVKQAPGQRLEWMGYINPYDDDTTYNQKFKGQVTITVDTSASTAYMELNSLRSEDTAVYYCARRGNSYDGYFDYSMDYWGQGTLVTVSS |
| 1709. | EN00F7-HCDR1 | artificial | aa | DYVIH |
| 1710. | EN00F7-HCDR2 | artificial | aa | YINPYDDDTTYNQKFKG |
| 1711. | EN00F7-HCDR3 | artificial | aa | RGNSYDGYFDYSMDY |
| 1712. | EN00F7-H | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGGTGAAGCAGGCACCTGGACAGCGCCTTGAGTGGATGGGATATATTAATCCTTATGATGATGATACTACCTACAACCAGAAGTTCAAGGGCCAGGTCACAATTACTGTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCA |
| 1713. | EN00F7-L | artificial | aa | DIQLTQSPSFLSASVGDRVTITCRASQNVGTAVAWYQQKPGKAPKLLIYSASNRYTGVPSRFSGSGSGTEFTLTISSMQPEDLATYFCQQYTNYPMYTFGQGTKLEIK |
| 1714. | EN00F7-LCDR1 | artificial | aa | RASQNVGTAVA |
| 1715. | EN00F7-LCDR2 | artificial | aa | SASNRYT |
| 1716. | EN00F7-LCDR3 | artificial | aa | QQYTNYPMYT |
| 1717. | EN00F7-L | artificial | nt | GACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTATGCAGCCTGAAGACCTCGCAACTTATTTCTGCCAGCAATATACCAACTATCCCATGTATACGTTTGGACAGGGGACCAAGCTGGAAATAAAA |
| 1718. | EN00F7-HL | artificial | aa | QVQLVQSGPEVKKPGASVKLSCKASGYTFTDYVIHWVKQAPGQRLEWMGYINPYDDDTTYNQKFKGQVTITVDTSASTAYMELNSLRSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSMQPEDLATYFCQQYTNYPMYTFGQGTKLEIK |

| | | | | |
|---|---|---|---|---|
| 1719. | EN00F7-HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGGTGAAGCAGGCACCTGGACAGCGCCTTGAGTGGATGGGATATATTAATCCTTATGATGATGATACTACCTACAACCAGAAGTTCAAGGGCCAGGTCACAATTACTGTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTATGCAGCCTGAAGACCTCGCAACTTATTTCTGCCAGCAATATACCAACTATCCCATGTATACGTTTGGACAGGGGACCAAGCTGGAAATAAAA |
| 1720. | En00F7 HL x I2C HL | artificial | aa | QVQLVQSGPEVKKPGASVKLSCKASGYTFTDYVIHWVKQAPGQRLEWMGYINPYDDDTTYNQKFKGQVTITVDTSASTAYMELNSLRSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSMQPEDLATYFCQQYTNYPMYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1721. | En00F7 HL x I2C HL | artificial | nt | CAGGTGCAGCTGGTCCAGTCAGGACCTGAGGTGAAGAAGCCTGGGGCTTCAGTGAAGTTGTCCTGCAAGGCTTCTGGATACACATTCACTGACTATGTTATACACTGGGTGAAGCAGGCACCTGGACAGCGCCTTGAGTGGATGGGATATATTAATCCTTATGATGATGATACTACCTACAACCAGAAGTTCAAGGGCCAGGTCACAATTACTGTAGACACATCCGCCAGCACAGCCTACATGGAACTCAACAGCCTGAGATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTATGCAGCCTGAAGACCTCGCAACTTATTTCTGCCAGCAATATACCAACTATCCCATGTATACGTTTGGACAGGGGACCAAGC |

| | | | | |
|---|---|---|---|---|
| | | | | TGGAAATAAAATCCGGAGGTGGTGGATCCGAGGTGCAGCTGGTCGAGTCTGGAGG AGGATTGGTGCAGCCTGGAGGGTCATTGAAACTCTCATGTGCAGCCTCTGGATTCA CCTTCAATAAGTACGCCATGAACTGGGTCCGCCAGGCTCCAGGAAAGGGTTTGGAA TGGGTTGCTCGCATAAGAAGTAAATATAATAATTATGCAACATATTATGCCGATTCAG TGAAAGACAGGTTCACCATCTCCAGAGATGATTCAAAAAACACTGCCTATCTACAAA TGAACAACTTGAAAACTGAGGACACTGCCGTGTACTACTGTGTGAGACATGGGAAC TTCGGTAATAGCTACATATCCTACTGGGCTTACTGGGGCCAAGGGACTCTGGTCAC CGTCTCCTCAGGTGGTGGTGGTTCTGGCGGCGGCGGCTCCGGTGGTGGTGGTTCT CAGACTGTTGTGACTCAGGAACCTTCACTCACCGTATCACCTGGTGGAACAGTCAC ACTCACTTGTGGCTCCTCGACTGGGGCTGTTACATCTGGCAACTACCCAAACTGGG TCCAACAAAAACCAGGTCAGGCACCCCGTGGTCTAATAGGTGGGACTAAGTTCCTC GCCCCCGGTACTCCTGCCAGATTCTCAGGCTCCCTGCTTGGAGGCAAGGCTGCCC TCACCCTCTCAGGGGTACAGCCAGAGGATGAGGCAGAATATTACTGTGTTCTATGG TACAGCAACCGCTGGGTGTTCGGTGGAGGAACCAAACTGACTGTCCTA |
| 1722. | EN00H6-H | artificial | aa | QVQLVQSGAEVKKPGASVKLSCKASGYTFTDYVIHWVRQTPGQRLEWMGYINPYDDD TTYNQKFKGRVTITGDTSANTAYMELNSLTSEDTAVYYCARRGNSYDGYFDYSMDYW GQGTLVTVSS |
| 1723. | EN00H6-HCDR1 | artificial | aa | DYVIH |
| 1724. | EN00H6-HCDR2 | artificial | aa | YINPYDDDTTYNQKFKG |
| 1725. | EN00H6-HCDR3 | artificial | aa | RGNSYDGYFDYSMDY |
| 1726. | EN00H6-H | artificial | nt | CAGGTGCAGCTGGTCCAGTCAGGAGCTGAGGTGAAGAAGCCTGGGGCTTCAGTGA AGTTGTCCTGCAAGGCTTCTGGATACACATTCACTGACTATGTTATACACTGGGTGA GGCAGACACCTGGACAGCGTCTTGAGTGGATGGGATATATTAATCCTTATGATGAT GATACTACCTACAACCAGAAGTTCAAGGGCCGGGTCACAATTACTGGAGACACATC CGCCAACACAGCCTACATGGAACTCAACAGCCTGACATCTGAGGACACTGCAGTCT ATTACTGTGCAAGAAGGGGGAACTCCTATGATGGTTACTTCGACTATTCTATGGACT ACTGGGGTCAAGGAACCTTGGTCACCGTCTCCTCA |
| 1727. | EN00H6-L | artificial | aa | DIQLTQSPSFLSASVGDRVTITCRASQNVGTAVAWYQQKPGKAPKLLIYSASNRYTGVP SRFSGSGSGTEFTLTISSLQSEDLATYYCQQYTNYPMYTFGQGTKLEIK |
| 1728. | EN00H6-LCDR1 | artificial | aa | RASQNVGTAVA |
| 1729. | EN00H6-LCDR2 | artificial | aa | SASNRYT |
| 1730. | EN00H6-LCDR3 | artificial | aa | QQYTNYPMYT |
| 1731. | EN00H6-L | artificial | nt | GACATCCAGCTGACCCAGTCTCCAAGCTTCCTGTCCGCATCAGTAGGAGACAGAGT CACCATCACCTGCAGGGCCAGTCAGAATGTGGGTACTGCTGTAGCCTGGTATCAAC AGAAACCAGGAAAAGCCCCTAAATTACTGATTTACTCGGCATCGAATCGGTACACTG GAGTCCCTAGTCGCTTCTCAGGCAGTGGATCTGGGACAGAGTTCACTCTCACCATC |

| | | | | |
|---|---|---|---|---|
| | | | | AGCAGTCTGCAGTCTGAAGACCTCGCAACTTATTACTGCCAGCAATATACCAACTATCCCATGTATACGTTTGGACAGGGGACCAAGCTGGAAATAAAA |
| 1732. | EN00H6-HL | artificial | aa | QVQLVQSGAEVKKPGASVKLSCKASGYTFTDYVIHWVRQTPGQRLEWMGYINPYDDDTTYNQKFKGRVTITGDTSANTAYMELNSLTSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSLQSEDLATYYCQQYTNYPMYTFGQGTKLEIK |
| 1733. | EN00H6-HL | artificial | nt | CAGGTGCAGCTGGTCCAGTCAGGAGCTGAGGTGAAGAAGCCTGGGGCTTCAGTGAAGTTGTCCTGCAAGGCTTCTGGATACACATTCACTGACTATGTTATACACTGGGTGAGGCAGACACCTGGACAGCGTCTTGAGTGGATGGGATATATTAATCCTTATGATGATGATACTACCTACAACCAGAAGTTCAAGGGCCGGGTCACAATTACTGGAGACACATCCGCCAACACAGCCTACATGGAACTCAACAGCCTGACATCTGAGGACACTGCAGTCTATTACTGTGCAAGAAGGGGGAACTCCTATGATGGTTACTTCGACTATTCTATGGACTACTGGGGTCAAGGAACCTTGGTCACCGTCTCCTCAGGTGGTGGTGGTTCTGGCGGCGGCGGCTCCGGTGGTGGTGGTTCTGACATCCAGCTGACCCAGTCTCCAAGCTTCCTGTCCGCATCAGTAGGAGACAGAGTCACCATCACCTGCAGGGCCAGTCAGAATGTGGGTACTGCTGTAGCCTGGTATCAACAGAAACCAGGAAAAGCCCCTAAATTACTGATTTACTCGGCATCGAATCGGTACACTGGAGTCCCTAGTCGCTTCTCAGGCAGTGGATCTGGGACAGAGTTCACTCTCACCATCAGCAGTCTGCAGTCTGAAGACCTCGCAACTTATTACTGCCAGCAATATACCAACTATCCCATGTATACGTTTGGACAGGGGACCAAGCTGGAAATAAAA |
| 1734. | EN00H6 HL x I2C HL | artificial | aa | QVQLVQSGAEVKKPGASVKLSCKASGYTFTDYVIHWVRQTPGQRLEWMGYINPYDDDTTYNQKFKGRVTITGDTSANTAYMELNSLTSEDTAVYYCARRGNSYDGYFDYSMDYWGQGTLVTVSSGGGGSGGGGSGGGGSDIQLTQSPSFLSASVGDRVTITCRASQNVGTAVAWYQQKPGKAPKLLIYSASNRYTGVPSRFSGSGSGTEFTLTISSLQSEDLATYYCQQYTNYPMYTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1735. | EN00H6 HL x I2C HL | artificial | nt | CAGGTGCAGCTGGTCCAGTCAGGAGCTGAGGTGAAGAAGCCTGGGGCTTCAGTGAAGTTGTCCTGCAAGGCTTCTGGATACACATTCACTGACTATGTTATACACTGGGTGAGGCAGACACCTGGACAGCGTCTTGAGTGGATGGGATATATTAATCCTTATGATGATGATACTACCTACAACCAGAAGTTCAAGGGCCGGGTCACAATTACTGGAGACACATCCGCCAACACAGCCTACATGGAACTCAACAGCCTGACATCTGAGGACACTGCAGTCTATTACTGTGCAAGAAGGGGGAACTCCTATGATGGTTACTTCGACTATTCTATGGACT |

| | | | | |
|---|---|---|---|---|
| | | | | ACTGGGGTCAAGGAACCTTGGTCACCGTCTCCTCAGGTGGTGGTGGTTCTGGCGG CGGCGGCTCCGGTGGTGGTGGTTCTGACATCCAGCTGACCCAGTCTCCAAGCTTC CTGTCCGCATCAGTAGGAGACAGAGTCACCATCACCTGCAGGGCCAGTCAGAATGT GGGTACTGCTGTAGCCTGGTATCAACAGAAACCAGGAAAAGCCCCTAAATTACTGA TTTACTCGGCATCGAATCGGTACACTGGAGTCCCTAGTCGCTTCTCAGGCAGTGGA TCTGGGACAGAGTTCACTCTCACCATCAGCAGTCTGCAGTCTGAAGACCTCGCAAC TTATTACTGCCAGCAATATACCAACTATCCCATGTATACGTTTGGACAGGGGACCAA GCTGGAAATAAAATCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATT CACCTTCAATAAGTACGCCATGAACTGGGTCCGCCAGGCTCCAGGAAAGGGTTTGG AATGGGTTGCTCGCATAAGAAGTAAATATAATAATTATGCAACATATTATGCCGATTC AGTGAAAGACAGGTTCACCATCTCCAGAGATGATTCAAAAAACACTGCCTATCTACA AATGAACAACTTGAAAACTGAGGACACTGCCGTGTACTACTGTGTGAGACATGGGA ACTTCGGTAATAGCTACATATCCTACTGGGCTTACTGGGGCCAAGGGACTCTGGTC ACCGTCTCCTCAGGTGGTGGTGGTTCTGGCGGCGGCGGCTCCGGTGGTGGTGGTT CTCAGACTGTTGTGACTCAGGAACCTTCACTCACCGTATCACCTGGTGGAACAGTC ACACTCACTTGTGGCTCCTCGACTGGGGCTGTTACATCTGGCAACTACCCAAACTG GGTCCAACAAAAACCAGGTCAGGCACCCCGTGGTCTAATAGGTGGGACTAAGTTCC TCGCCCCCGGTACTCCTGCCAGATTCTCAGGCTCCCTGCTTGGAGGCAAGGCTGC CCTCACCCTCTCAGGGGTACAGCCAGAGGATGAGGCAGAATATTACTGTGTTCTAT GGTACAGCAACCGCTGGGTGTTCGGTGGAGGAACCAAACTGACTGTCCTA |
| 1736. | HEP14G6-H | artificial | aa | QVQLVQSGAEVKKPGSSVKVSCKASGYTFSNYWLGWVRQAPGQGLEWMGDIFPGSG NIHYNEKFKGRATITADKSTSTAYMELSSLTSEDTAVYYCARLRNWDEPMDYWGQGTT VTVSS |
| 1737. | HEP14G6-HCDR1 | artificial | aa | NYWLG |
| 1738. | HEP14G6-HCDR2 | artificial | aa | DIFPGSGNIHYNEKFKG |
| 1739. | HEP14G6-HCDR3 | artificial | aa | LRNWDEPMDY |
| 1740. | HEP14G6-H | artificial | nt | CAGGTGCAGCTGGTCCAGTCTGGAGCTGAGGTGAAAAAGCCTGGGTCTTCAGTGA AGGTATCCTGCAAGGCTTCTGGATACACCTTCTCTAACTACTGGCTAGGTTGGGTAA GGCAGGCGCCTGGACAGGGACTTGAGTGGATGGGAGATATTTTCCCTGGAAGTGG TAATATCCACTACAATGAGAAGTTCAAGGGCAGAGCCACAATCACTGCAGACAAATC TACGAGCACAGCCTATATGGAACTCAGTAGCCTGACATCTGAGGACACTGCTGTCT ATTACTGTGCAAGACTGAGGAACTGGGACGAGCCTATGGACTACTGGGGCCAAGG GACCACGGTCACCGTCTCCTCC |
| 1741. | HEP14G6-L | artificial | aa | DIVMTQTPLSLTVTPGEPVSISCKSSQSLLNSGNQKNYLTWYLQKPGQPPQLLIYWAST RESGVPDRFSGSGSGTDFTLKISRVQAEDVGVYYCQNDYSYPLTFGQGTKLEIK |

| | | | | |
|---|---|---|---|---|
| 1742. | HEP14G6-LCDR1 | artificial | aa | KSSQSLLNSGNQKNYLT |
| 1743. | HEP14G6-LCDR2 | artificial | aa | WASTRES |
| 1744. | HEP14G6-LCDR3 | artificial | aa | QNDYSYPLT |
| 1745. | HEP14G6-L | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGCAGGCTGAAGACGTGGGAGTTTATTACTGTCAGAATGATTATAGTTATCCGCTCACGTTCGGTCAAGGGACCAAGCTTGAGATCAAA |
| 1746. | HEP14G6-LH | artificial | aa | DIVMTQTPLSLTVTPGEPVSISCKSSQSLLNSGNQKNYLTWYLQKPGQPPQLLIYWASTRESGVPDRFSGSGSGTDFTLKISRVQAEDVGVYYCQNDYSYPLTFGQGTKLEIKGGGGSGGGGSGGGGSQVQLVQSGAEVKKPGSSVKVSCKASGYTFSNYWLGWVRQAPGQGLEWMGDIFPGSGNIHYNEKFKGRATITADKSTSTAYMELSSLTSEDTAVYYCARLRNWDEPMDYWGQGTTVTVSS |
| 1747. | HEP14G6-LH | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGCAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATGGGAGATATTTTCCCTGGAAGTGGTAATATCCACTACAATGAGAAGTTCAAGGGCAGAGCCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCC |
| 1748. | hEp14G6 LH x I2C HL | artificial | aa | DIVMTQTPLSLTVTPGEPVSISCKSSQSLLNSGNQKNYLTWYLQKPGQPPQLLIYWASTRESGVPDRFSGSGSGTDFTLKISRVQAEDVGVYYCQNDYSYPLTFGQGTKLEIKGGGGSGGGGSGGGGSQVQLVQSGAEVKKPGSSVKVSCKASGYTFSNYWLGWVRQAPGQGLEWMGDIFPGSGNIHYNEKFKGRATITADKSTSTAYMELSSLTSEDTAVYYCARLRNWDEPMDYWGQGTTVTVSSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSG |

| | | | | |
|---|---|---|---|---|
| | | | | SLLGGKAALTLSGVQPEDEAEYYCVLWYSNRWVFGGGTKLTVL |
| 1749. | hEp14G6 LH x I2C HL | artificial | nt | GACATCGTGATGACACAGACTCCACTCTCCCTGACTGTGACACCAGGAGAGCCGGTCTCTATCAGCTGCAAGTCCAGTCAGAGTCTGTTAAACAGTGGAAATCAAAAGAACTACTTGACCTGGTACCTGCAGAAACCAGGGCAGCCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGCAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATGGGAGATATTTTCCCTGGAAGTGGTAATATCCACTACAATGAGAAGTTCAAGGGCAGAGCCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1750. | HEP14D2-H | artificial | aa | QVQLVQSGAEVKKPGSSVKVSCKASGYTFSNYWLGWVRQAPGQGLEWMGDIFPGSGNIHYNEKFKGRVTITADKSTSTAYMELSSLTSEDTAVYYCARLRNWDEPMDYWGQGTTVTVSS |
| 1751. | HEP14D2-HCDR1 | artificial | aa | NYWLG |
| 1752. | HEP14D2-HCDR2 | artificial | aa | DIFPGSGNIHYNEKFKG |
| 1753. | HEP14D2-HCDR3 | artificial | aa | LRNWDEPMDY |
| 1754. | HEP14D2-H | artificial | nt | CAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAA |

| | | | | |
|---|---|---|---|---|
| | | | | GGCAGGCGCCTGGACAGGGACTTGAGTGGATGGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCC |
| 1755. | HEP14D2-L | artificial | aa | DIVMTQTPLSLPVTPGEQVSISCKSSQSLLNSGNQKNYLTWYQQKPGQSPQLLIYWASTRESGVPDRFSGSGSGTDFTLKISRVEAEDVGVYYCQNDYSYPLTFGQGTKLEIK |
| 1756. | HEP14D2-LCDR1 | artificial | aa | KSSQSLLNSGNQKNYLT |
| 1757. | HEP14D2-LCDR2 | artificial | aa | WASTRES |
| 1758. | HEP14D2-LCDR3 | artificial | aa | QNDYSYPLT |
| 1759. | HEP14D2-L | artificial | nt | GACATCGTGATGACACAGACTCCACTCTCCCTGCCTGTGACACCAGGAGAGCAGGTCTCTATCAGCTGCAAGTCCAGTCAGAGTCTGTTAAACAGTGGAAATCAAAAGAACTACTTGACCTGGTACCAGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAA |
| 1760. | HEP14D2-LH | artificial | aa | DIVMTQTPLSLPVTPGEQVSISCKSSQSLLNSGNQKNYLTWYQQKPGQSPQLLIYWASTRESGVPDRFSGSGSGTDFTLKISRVEAEDVGVYYCQNDYSYPLTFGQGTKLEIKGGGGSGGGGSGGGGSQVQLVQSGAEVKKPGSSVKVSCKASGYTFSNYWLGWVRQAPGQGLEWMGDIFPGSGNIHYNEKFKGRVTITADKSTSTAYMELSSLTSEDTAVYYCARLRNWDEPMDYWGQGTTVTVSS |
| 1761. | HEP14D2-LH | artificial | nt | GACATCGTGATGACACAGACTCCACTCTCCCTGCCTGTGACACCAGGAGAGCAGGTCTCTATCAGCTGCAAGTCCAGTCAGAGTCTGTTAAACAGTGGAAATCAAAAGAACTACTTGACCTGGTACCAGCAGAAACCAGGGCAGTCTCCTCAACTGTTGATCTACTGGGCATCCACTAGGGAATCTGGGGTCCCTGATCGCTTCTCAGGCAGTGGATCTGGAACAGATTTCACTCTCAAAATCAGCCGTGTGGAGGCTGAAGACGTGGGAGTTTATTACTGTCAGAATGATTATAGTTATCCGCTCACGTTCGGTCAAGGGACCAAGCTTGAGATCAAAGGTGGTGGTGGTTCTGGCGGCGGCGGCTCCGGTGGTGGTGGTTCTCAGGTGCAGCTGGTCCAGTCTGGAGCTGAGGTGAAAAAGCCTGGGTCTTCAGTGAAGGTATCCTGCAAGGCTTCTGGATACACCTTCTCTAACTACTGGCTAGGTTGGGTAAGGCAGGCGCCTGGACAGGGACTTGAGTGGATGGGAGATATTTTCCCTGGAAGTGGTAATATCCACTACAATGAGAAGTTCAAGGGCAGAGTCACAATCACTGCAGACAAATCTACGAGCACAGCCTATATGGAACTCAGTAGCCTGACATCTGAGGACACTGCTGTCTATTACTGTGCAAGACTGAGGAACTGGGACGAGCCTATGGACTACTGGGGCCAAGGGACCACGGTCACCGTCTCCTCC |
| 1762. | hEp14D2 LH x I2C | artificial | aa | DIVMTQTPLSLPVTPGEQVSISCKSSQSLLNSGNQKNYLTWYQQKPGQSPQLLIYWAST |

| | | | | |
|---|---|---|---|---|
| | HL | | | RESGVPDRFSGSGSGTDFTLKISRVEAEDVGVYYCQNDYSYPLTFGQGTKLEIKGGGG<br>SGGGGSGGGGSQVQLVQSGAEVKKPGSSVKVSCKASGYTFSNYWLGWVRQAPGQG<br>LEWMGDIFPGSGNIHYNEKFKGRVTITADKSTSTAYMELSSLTSEDTAVYYCARLRNWD<br>EPMDYWGQGTTVTVSSGGGGSEVQLVESGGGLVQPGGSLKLSCAASGFTFNKYAMN<br>WVRQAPGKGLEWVARIRSKYNNYATYYADSVKDRFTISRDDSKNTAYLQMNNLKTEDT<br>AVYYCVRHGNFGNSYISYWAYWGQGTLVTVSSGGGGSGGGGSGGGGSQTVVTQEP<br>SLTVSPGGTVTLTCGSSTGAVTSGNYPNWVQQKPGQAPRGLIGGTKFLAPGTPARFSG<br>SLLGGKAALTLSGVQPEDEAEYYCVLWYSNRWVFGGGTKLTVL |
| 1763. | hEp14D2 LH x I2C HL | artificial | nt | GACATCGTGATGACACAGACTCCACTCTCCCTGCCTGTGACACCAGGAGAGCAGGT<br>CTCTATCAGCTGCAAGTCCAGTCAGAGTCTGTTAAACAGTGGAAATCAAAAGAACTA<br>CTTGACCTGGTACCAGCAGAAACCAGGGCAGTCTCCTCAACTGTTGATCTACTGGG<br>CATCCACTAGGGAATCTGGGGTCCCTGATCGCTTCTCAGGCAGTGGATCTGGAACA<br>GATTTCACTCTCAAAATCAGCCGTGTGGAGGCTGAAGACGTGGGAGTTTATTACTGT<br>CAGAATGATTATAGTTATCCGCTCACGTTCGGTCAAGGGACCAAGCTTGAGATCAAA<br>GGTGGTGGTGGTTCTGGCGGCGGCGGCTCCGGTGGTGGTGGTTCTCAGGTGCAG<br>CTGGTCCAGTCTGGAGCTGAGGTGAAAAAGCCTGGGTCTTCAGTGAAGGTATCCTG<br>CAAGGCTTCTGGATACACCTTCTCTAACTACTGGCTAGGTTGGGTAAGGCAGGCGC<br>CTGGACAGGGACTTGAGTGGATGGGAGATATTTTCCCTGGAAGTGGTAATATCCAC<br>TACAATGAGAAGTTCAAGGGCAGAGTCACAATCACTGCAGACAAATCTACGAGCAC<br>AGCCTATATGGAACTCAGTAGCCTGACATCTGAGGACACTGCTGTCTATTACTGTGC<br>AAGACTGAGGAACTGGGACGAGCCTATGGACTACTGGGGCCAAGGGACCACGGTC<br>ACCGTCTCCTCCGGAGGTGGTGGATCCGAGGTGCAGCTGGTCGAGTCTGGAGGAG<br>GATTGGTGCAGCCTGGAGGGTCATTGAAACTCTCATGTGCAGCCTCTGGATTCACC<br>TTCAATAAGTACGCCATGAACTGGGTCCGCCAGGCTCCAGGAAAGGGTTTGGAATG<br>GGTTGCTCGCATAAGAAGTAAATATAATAATTATGCAACATATTATGCCGATTCAGTG<br>AAAGACAGGTTCACCATCTCCAGAGATGATTCAAAAAACACTGCCTATCTACAAATG<br>AACAACTTGAAAACTGAGGACACTGCCGTGTACTACTGTGTGAGACATGGGAACTT<br>CGGTAATAGCTACATATCCTACTGGGCTTACTGGGGCCAAGGGACTCTGGTCACCG<br>TCTCCTCAGGTGGTGGTGGTTCTGGCGGCGGCGGCTCCGGTGGTGGTGGTTCTCA<br>GACTGTTGTGACTCAGGAACCTTCACTCACCGTATCACCTGGTGGAACAGTCACAC<br>TCACTTGTGGCTCCTCGACTGGGGCTGTTACATCTGGCAACTACCCAAACTGGGTC<br>CAACAAAAACCAGGTCAGGCACCCCGTGGTCTAATAGGTGGGACTAAGTTCCTCGC<br>CCCCGGTACTCCTGCCAGATTCTCAGGCTCCCTGCTTGGAGGCAAGGCTGCCCTC<br>ACCCTCTCAGGGGTACAGCCAGAGGATGAGGCAGAATATTACTGTGTTCTATGGTA<br>CAGCAACCGCTGGGTGTTCGGTGGAGGAACCAAACTGACTGTCCTA |
| 1764. | HEP18A6-H | artificial | aa | QVQLVQSGAEVKKPGSSVKVSCKASGYTFSNYWLGWVRQTPGQGLEWMGDIFPGSG |

| | | | | |
|---|---|---|---|---|
| | | | | NIHYNEKFKGRVTITADKSTSTAYMELSSLTSEDTAVYYCARLRNWDEPMDYWGQGTT<br>VTVSS |
| 1765. | HEP18A6-HCDR1 | artificial | aa | NYWLG |
| 1766. | HEP18A6-HCDR2 | artificial | aa | DIFPGSGNIHYNEKFKG |
| 1767. | HEP18A6-HCDR3 | artificial | aa | LRNWDEPMDY |
| 1768. | HEP18A6-H | artificial | nt | CAGGTGCAGCTGGTCCAGTCTGGAGCTGAGGTGAAAAAGCCTGGGTCTTCAGTGA<br>AGGTATCCTGCAAGGCTTCTGGATACACCTTCTCTAACTACTGGCTAGGTTGGGTAA<br>GGCAGACGCCTGGACAGGGACTTGAGTGGATGGGAGATATTTTCCCTGGAAGTGG<br>TAATATCCACTACAATGAGAAGTTCAAGGGCAGAGTCACAATCACTGCAGACAAATC<br>TACGAGCACAGCCTATATGGAACTCAGTAGCCTGACATCTGAGGACACTGCTGTCT<br>ATTACTGTGCAAGACTGAGGAACTGGGACGAGCCTATGGACTACTGGGGCCAAGG<br>GACCACGGTCACCGTCTCCTCC |
| 1769. | HEP18A6-L | artificial | aa | DIVMTQTPLSLPVTPGEQVSMSCKSSQSLLNSGNQKNYLTWYLQKPGQSPQLLIYWAS<br>TRESGVPDRFSGSGSGTDFTLNISSVEAEDVGVYYCQNDYSYPLTFGQGTKLEIK |
| 1770. | HEP18A6-LCDR1 | artificial | aa | KSSQSLLNSGNQKNYLT |
| 1771. | HEP18A6-LCDR2 | artificial | aa | WASTRES |
| 1772. | HEP18A6-LCDR3 | artificial | aa | QNDYSYPLT |
| 1773. | HEP18A6-L | artificial | nt | GACATCGTGATGACACAGACTCCACTCTCCCTGCCTGTGACACCAGGAGAGCAGGT<br>CTCTATGAGCTGCAAGTCCAGTCAGAGTCTGTTAAACAGTGGAAATCAAAAGAACTA<br>CTTGACCTGGTACCTGCAGAAACCAGGGCAGTCTCCTCAACTGTTGATCTACTGGG<br>CATCCACTAGGGAATCTGGGGTCCCTGATCGCTTCTCAGGCAGTGGATCTGGAACA<br>GATTTCACTCTCAACATCAGCAGTGTGGAGGCTGAAGACGTGGGAGTTTATTACTGT<br>CAGAATGATTATAGTTATCCGCTCACGTTCGGTCAAGGGACCAAGCTTGAGATCAAA |
| 1774. | HEP18A6-LH | artificial | aa | DIVMTQTPLSLPVTPGEQVSMSCKSSQSLLNSGNQKNYLTWYLQKPGQSPQLLIYWAS<br>TRESGVPDRFSGSGSGTDFTLNISSVEAEDVGVYYCQNDYSYPLTFGQGTKLEIKGGG<br>GSGGGGSGGGGSQVQLVQSGAEVKKPGSSVKVSCKASGYTFSNYWLGWVRQTPGQ<br>GLEWMGDIFPGSGNIHYNEKFKGRVTITADKSTSTAYMELSSLTSEDTAVYYCARLRNW<br>DEPMDYWGQGTTVTVSS |
| 1775. | HEP18A6-LH | artificial | nt | GACATCGTGATGACACAGACTCCACTCTCCCTGCCTGTGACACCAGGAGAGCAGGT<br>CTCTATGAGCTGCAAGTCCAGTCAGAGTCTGTTAAACAGTGGAAATCAAAAGAACTA<br>CTTGACCTGGTACCTGCAGAAACCAGGGCAGTCTCCTCAACTGTTGATCTACTGGG<br>CATCCACTAGGGAATCTGGGGTCCCTGATCGCTTCTCAGGCAGTGGATCTGGAACA<br>GATTTCACTCTCAACATCAGCAGTGTGGAGGCTGAAGACGTGGGAGTTTATTACTGT<br>CAGAATGATTATAGTTATCCGCTCACGTTCGGTCAAGGGACCAAGCTTGAGATCAAA<br>GGTGGTGGTGGTTCTGGCGGCGGCGGCTCCGGTGGTGGTGGTTCTCAGGTGCAG<br>CTGGTCCAGTCTGGAGCTGAGGTGAAAAAGCCTGGGTCTTCAGTGAAGGTATCCTG |

| | | | | |
|---|---|---|---|---|
| | | | | CAAGGCTTCTGGATACACCTTCTCTAACTACTGGCTAGGTTGGGTAAGGCAGACGC CTGGACAGGGACTTGAGTGGATGGGAGATATTTTCCCTGGAAGTGGTAATATCCAC TACAATGAGAAGTTCAAGGGCAGAGTCACAATCACTGCAGACAAATCTACGAGCAC AGCCTATATGGAACTCAGTAGCCTGACATCTGAGGACACTGCTGTCTATTACTGTGC AAGACTGAGGAACTGGGACGAGCCTATGGACTACTGGGGCCAAGGGACCACGGTC ACCGTCTCCTCC |
| 1776. | hEp18A6 LH x I2C HL | artificial | aa | DIVMTQTPLSLPVTPGEQVSMSCKSSQSLLNSGNQKNYLTWYLQKPGQSPQLLIYWAS TRESGVPDRFSGSGSGTDFTLNISSVEAEDVGVYYCQNDYSYPLTFGQGTKLEIKGGG GSGGGGSGGGGSQVQLVQSGAEVKKPGSSVKVSCKASGYTFSNYWLGWVRQTPGQ GLEWMGDIFPGSGNIHYNEKFKGRVTITADKSTSTAYMELSSLTSEDTAVYYCARLRNW DEPMDYWGQGTTVTVSSGGGGSEVQLVESGGGLVQPGGSLKLSCAASGFTFNKYAM NWVRQAPGKGLEWVARIRSKYNNYATYYADSVKDRFTISRDDSKNTAYLQMNNLKTED TAVYYCVRHGNFGNSYISYWAYWGQGTLVTVSSGGGGSGGGGSGGGGSQTVVTQE PSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 1777. | hEp18A6 LH x I2C HL | artificial | nt | GACATCGTGATGACACAGACTCCACTCTCCCTGCCTGTGACACCAGGAGAGCAGGT CTCTATGAGCTGCAAGTCCAGTCAGAGTCTGTTAAACAGTGGAAATCAAAAGAACTA CTTGACCTGGTACCTGCAGAAACCAGGGCAGTCTCCTCAACTGTTGATCTACTGGG CATCCACTAGGGAATCTGGGGTCCCTGATCGCTTCTCAGGCAGTGGATCTGGAACA GATTTCACTCTCAACATCAGCAGTGTGGAGGCTGAAGACGTGGGAGTTTATTACTGT CAGAATGATTATAGTTATCCGCTCACGTTCGGTCAAGGGACCAAGCTTGAGATCAAA GGTGGTGGTGGTTCTGGCGGCGGCGGCTCCGGTGGTGGTGGTTCTCAGGTGCAG CTGGTCCAGTCTGGAGCTGAGGTGAAAAAGCCTGGGTCTTCAGTGAAGGTATCCTG CAAGGCTTCTGGATACACCTTCTCTAACTACTGGCTAGGTTGGGTAAGGCAGACGC CTGGACAGGGACTTGAGTGGATGGGAGATATTTTCCCTGGAAGTGGTAATATCCAC TACAATGAGAAGTTCAAGGGCAGAGTCACAATCACTGCAGACAAATCTACGAGCAC AGCCTATATGGAACTCAGTAGCCTGACATCTGAGGACACTGCTGTCTATTACTGTGC AAGACTGAGGAACTGGGACGAGCCTATGGACTACTGGGGCCAAGGGACCACGGTC ACCGTCTCCTCCGGAGGTGGTGGATCCGAGGTGCAGCTGGTCGAGTCTGGAGGAG GATTGGTGCAGCCTGGAGGGTCATTGAAACTCTCATGTGCAGCCTCTGGATTCACC TTCAATAAGTACGCCATGAACTGGGTCCGCCAGGCTCCAGGAAAGGGTTTGGAATG GGTTGCTCGCATAAGAAGTAAATATAATAATTATGCAACATATTATGCCGATTCAGTG AAAGACAGGTTCACCATCTCCAGAGATGATTCAAAAAACACTGCCTATCTACAAATG AACAACTTGAAAACTGAGGACACTGCCGTGTACTACTGTGTGAGACATGGGAACTT CGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCA |

| | | | | |
|---|---|---|---|---|
| | | | | GACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1778. | FA22A9-H | artificial | aa | QVQLVQSGAEVKKPGASVKVSCKTSGYTFTEYTIHWVRQAPGQRLEWMGGINPNNGIPNYNQKFKGRVTITIDTSSSTAYMELSSLRSEDTAVYYCARRIAYGYDEGHAMDYWGQGTLVTVSS |
| 1779. | FA22A9-HCDR1 | artificial | aa | EYTIH |
| 1780. | FA22A9-HCDR2 | artificial | aa | GINPNNGIPNYNQKFKG |
| 1781. | FA22A9-HCDR3 | artificial | aa | RRIAYGYDEGHAMDY |
| 1782. | FA22A9-H | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGTCACAATAACTATAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCA |
| 1783. | FA22A9-L | artificial | aa | DIVMTQTPLSLAVTVGETASMSCKSSQSLLYSRNQKNYLAWYLQKPGQSPQLLIYWASTRESGVPDRFSGSGSGTDFTLTISRVEAEDVGVYYCQQYFSYPLTFGGGTKVEIK |
| 1784. | FA22A9-LCDR1 | artificial | aa | KSSQSLLYSRNQKNYLA |
| 1785. | FA22A9-LCDR2 | artificial | aa | WASTRES |
| 1786. | FA22A9-LCDR3 | artificial | aa | QQYFSYPLT |
| 1787. | FA22A9-L | artificial | nt | GACATCGTGATGACACAGACTCCACTCTCCCTAGCTGTGACAGTTGGAGAGACGGCTTCTATGAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATCTGGGACGGATTTCACTCTCACGATCAGCAGAGTGGAGGCTGAGGACGTGGGAGTTTATTACTGTCAGCAATATTTTAGCTATCCGCTCACGTTCGGTGGTGGGACCAAGGTGGAGATCAAA |
| 1788. | FA22A9-HL | artificial | aa | QVQLVQSGAEVKKPGASVKVSCKTSGYTFTEYTIHWVRQAPGQRLEWMGGINPNNGIPNYNQKFKGRVTITIDTSSSTAYMELSSLRSEDTAVYYCARRIAYGYDEGHAMDYWGQGTLVTVSSGGGGSGGGGSGGGGSDIVMTQTPLSLAVTVGETASMSCKSSQSLLYSRNQKNYLAWYLQKPGQSPQLLIYWASTRESGVPDRFSGSGSGTDFTLTISRVEAEDVGV |

| | | | | |
|---|---|---|---|---|
| | | | | YYCQQYFSYPLTFGGGTKVEIK |
| 1789. | FA22A9-HL | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGTCACAATAACTATAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTAGCTGTGACAGTTGGAGAGACGGCTTCTATGAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATCTGGGACGGATTTCACTCTCACGATCAGCAGAGTGGAGGCTGAGGACGTGGGAGTTTATTACTGTCAGCAATATTTTAGCTATCCGCTCACGTTCGGTGGTGGGACCAAGGTGGAGATCAAA |
| 1790. | FA22A9 HL x I2C HL | artificial | aa | QVQLVQSGAEVKKPGASVKVSCKTSGYTFTEYTIHWVRQAPGQRLEWMGGINPNNGIPNYNQKFKGRVTITIDTSSSTAYMELSSLRSEDTAVYYCARRIAYGYDEGHAMDYWGQGTLVTVSSGGGGSGGGGSGGGGSDIVMTQTPLSLAVTVGETASMSCKSSQSLLYSRNQKNYLAWYLQKPGQSPQLLIYWASTRESGVPDRFSGSGSGTDFTLTISRVEAEDVGVYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1791. | FA22A9 HL x I2C HL | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGTCACAATAACTATAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTAGCTGTGACAGTTGGAGAGACGGCTTCTATGAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATCTGGGACGGATTTCACTCTCACGATCAGCAGAGTGGAG |

| | | | | |
|---|---|---|---|---|
| | | | | GCTGAGGACGTGGGAGTTTATTACTGTCAGCAATATTTTAGCTATCCGCTCACGTTCGGTGGTGGGACCAAGGTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1792. | FA22C11-H | artificial | aa | QVQLVQSGAEVKKPGASVKVSCKTSGYTFTEYTIHWVRQSPGQRLEWMGGINPNNGIPNYNQKFKGRVTITRDKSSSTAYMELSSLRSEDTAVYYCARRRIAYGYDEGHAMDYWGQGTLVTVSS |
| 1793. | FA22C11-HCDR1 | artificial | aa | EYTIH |
| 1794. | FA22C11-HCDR2 | artificial | aa | GINPNNGIPNYNQKFKG |
| 1795. | FA22C11-HCDR3 | artificial | aa | RRIAYGYDEGHAMDY |
| 1796. | FA22C11-H | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGTCCCCTGGACAGAGACTTGAGTGGATGGGAGGTATTAATCCTAACAATGGTATTCCTAACTACAATCAGAAGTTCAAGGGCAGGGTCACAATAACTAGAGACAAGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCA |
| 1797. | FA22C11-L | artificial | aa | DIVMTQTPLSLPVTVGEPASISCKSSQSLLYSRNQKNYLAWYLQKPGQSPQLLIYWASTRESGVPDRFSGSGSGTDFTLKISRVEAEDVGIYYCQQYFSYPLTFGGGTKVEIK |
| 1798. | FA22C11-LCDR1 | artificial | aa | KSSQSLLYSRNQKNYLA |
| 1799. | FA22C11-LCDR2 | artificial | aa | WASTRES |
| 1800. | FA22C11-LCDR3 | artificial | aa | QQYFSYPLT |
| 1801. | FA22C11-L | artificial | nt | GACATCGTGATGACACAGACTCCACTCTCCCTACCTGTGACAGTTGGAGAGCCGGC |

| | | | | |
|---|---|---|---|---|
| | | | | TTCTATCAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATCTGGGACAGACTTCACCCTCAAGATCTCTCGTGTGGAGGCGGAGGATGTTGGAATCTATTACTGTCAGCAATATTTTAGCTATCCGCTCACGTTCGGTGGTGGGACCAAGGTGGAGATCAAA |
| 1802. | FA22C11-HL | artificial | aa | QVQLVQSGAEVKKPGASVKVSCKTSGYTFTEYTIHWVRQSPGQRLEWMGGINPNNGIPNYNQKFKGRVTITRDKSSSTAYMELSSLRSEDTAVYYCARRRIAYGYDEGHAMDYWGQGTLVTVSSGGGGSGGGGSGGGGSDIVMTQTPLSLPVTVGEPASISCKSSQSLLYSRNQKNYLAWYLQKPGQSPQLLIYWASTRESGVPDRFSGSGSGTDFTLKISRVEAEDVGIYYCQQYFSYPLTFGGGTKVEIK |
| 1803. | FA22C11-HL | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGTCCCCTGGACAGAGACTTGAGTGGATGGGAGGTATTAATCCTAACAATGGTATTCCTAACTACAATCAGAAGTTCAAGGGCAGGGTCACAATAACTAGAGACAAGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTACCTGTGACAGTTGGAGAGCCGGCTTCTATCAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATCTGGGACAGACTTCACCCTCAAGATCTCTCGTGTGGAGGCGGAGGATGTTGGAATCTATTACTGTCAGCAATATTTTAGCTATCCGCTCACGTTCGGTGGTGGGACCAAGGTGGAGATCAAA |
| 1804. | FA22C11 HL x I2C HL | artificial | aa | QVQLVQSGAEVKKPGASVKVSCKTSGYTFTEYTIHWVRQSPGQRLEWMGGINPNNGIPNYNQKFKGRVTITRDKSSSTAYMELSSLRSEDTAVYYCARRRIAYGYDEGHAMDYWGQGTLVTVSSGGGGSGGGGSGGGGSDIVMTQTPLSLPVTVGEPASISCKSSQSLLYSRNQKNYLAWYLQKPGQSPQLLIYWASTRESGVPDRFSGSGSGTDFTLKISRVEAEDVGIYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1805. | FA22C11 HL x I2C HL | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGA |

| | | | | |
|---|---|---|---|---|
| | | | | GACAGTCCCCTGGACAGAGACTTGAGTGGATGGGAGGTATTAATCCTAACAATGGT ATTCCTAACTACAATCAGAAGTTCAAGGGCAGGGTCACAATAACTAGAGACAAGTCC TCCAGCACCGCCTACATGGAGCTCAGCAGCCTGAGATCTGAGGATACTGCGGTCTA TTACTGTGCAAGAAGAAGAATCGCCTATGGTTACGACGAGGGCCATGCTATGGACT ACTGGGGTCAAGGAACCCTGGTCACCGTCTCCTCAGGTGGTGGTGGTTCTGGCGG CGGCGGCTCCGGTGGTGGTGGTTCTGACATCGTGATGACACAGACTCCACTCTCC CTACCTGTGACAGTTGGAGAGCCGGCTTCTATCAGCTGCAAGTCCAGTCAGAGCCT TTTATATAGTCGTAATCAAAAGAACTACTTGGCCTGGTACCTGCAGAAGCCAGGGCA GTCTCCTCAACTGCTGATTTACTGGGCATCCACTAGGGAATCTGGGGTCCCTGATC GCTTCTCAGGCAGTGGATCTGGGACAGACTTCACCCTCAAGATCTCTCGTGTGGAG GCGGAGGATGTTGGAATCTATTACTGTCAGCAATATTTTAGCTATCCGCTCACGTTC GGTGGTGGGACCAAGGTGGAGATCAAATCCGGAGGTGGTGGATCCGAGGTGCAG CTGGTCGAGTCTGGAGGAGGATTGGTGCAGCCTGGAGGGTCATTGAAACTCTCAT GTGCAGCCTCTGGATTCACCTTCAATAAGTACGCCATGAACTGGGTCCGCCAGGCT CCAGGAAAGGGTTTGGAATGGGTTGCTCGCATAAGAAGTAAATATAATAATTATGCA ACATATTATGCCGATTCAGTGAAAGACAGGTTCACCATCTCCAGAGATGATTCAAAA AACACTGCCTATCTACAAATGAACAACTTGAAAACTGAGGACACTGCCGTGTACTAC TGTGTGAGACATGGGAACTTCGGTAATAGCTACATATCCTACTGGGCTTACTGGGG CCAAGGGACTCTGGTCACCGTCTCCTCAGGTGGTGGTGGTTCTGGCGGCGGCGGC TCCGGTGGTGGTGGTTCTCAGACTGTTGTGACTCAGGAACCTTCACTCACCGTATC ACCTGGTGGAACAGTCACACTCACTTGTGGCTCCTCGACTGGGGCTGTTACATCTG GCAACTACCCAAACTGGGTCCAACAAAAACCAGGTCAGGCACCCCGTGGTCTAATA GGTGGGACTAAGTTCCTCGCCCCCGGTACTCCTGCCAGATTCTCAGGCTCCCTGCT TGGAGGCAAGGCTGCCCTCACCCTCTCAGGGGTACAGCCAGAGGATGAGGCAGAA TATTACTGTGTTCTATGGTACAGCAACCGCTGGGTGTTCGGTGGAGGAACCAAACT GACTGTCCTA |
| 1806. | FA22D8-H | artificial | aa | QVQLVQSGAEVEKPGASVKVSCKASGYTFTEYTIHWVRQAPGQRLEWMGGINPNNGI PNYNQKFKGRATITIDTSSSTAYMELSSLTSEDTAVYYCARRRIAYGYDEGHAMDYWG QGTLVTVSS |
| 1807. | FA22D8-HCDR1 | artificial | aa | EYTIH |
| 1808. | FA22D8-HCDR2 | artificial | aa | GINPNNGIPNYNQKFKG |
| 1809. | FA22D8-HCDR3 | artificial | aa | RRIAYGYDEGHAMDY |
| 1810. | FA22D8-H | artificial | nt | CAGGTGCAGCTGGTCCAGTCTGGAGCTGAGGTGGAGAAGCCTGGGGCTTCAGTAA AGGTGTCCTGCAAGGCTTCTGGATACACATTCACTGAATACACCATACACTGGGTG AGACAGGCCCCTGGACAGAGACTTGAGTGGATGGGAGGTATTAATCCTAACAATGG TATTCCTAACTACAATCAGAAGTTCAAGGGCAGGGCCACAATAACTATAGACACGTC |

| | | | | |
|---|---|---|---|---|
| | | | | CTCCAGCACCGCCTACATGGAGCTCAGCAGCCTGACATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCA |
| 1811. | FA22D8-L | artificial | aa | DIVMTQTPLSLPVTVGEQASISCKSSQSLLYSRNQKNYLAWYLQKPGQSPKLLIYWASTRESGVPDRFSGSGSGTDFTLKISSVEAEDVGVYYCQQYFSYPLTFGGGTKVEIK |
| 1812. | FA22D8-LCDR1 | artificial | aa | KSSQSLLYSRNQKNYLA |
| 1813. | FA22D8-LCDR2 | artificial | aa | WASTRES |
| 1814. | FA22D8-LCDR3 | artificial | aa | QQYFSYPLT |
| 1815. | FA22D8-L | artificial | nt | GACATCGTGATGACACAGACTCCACTCTCCCTACCTGTGACAGTTGGAGAGCAGGCTTCTATCAGCTGCAAGTCCAGTCAGAGCCTTTTATATAGTCGTAATCAAAAGAACTACTTGGCCTGGTACCTGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAGATCAGCAGTGTGGAGGCTGAGGATGTTGGAGTCTATTACTGTCAGCAATATTTTAGCTATCCGCTCACGTTCGGTGGTGGGACCAAGGTGGAGATCAAA |
| 1816. | FA22D8-HL | artificial | aa | QVQLVQSGAEVEKPGASVKVSCKASGYTFTEYTIHWVRQAPGQRLEWMGGINPNNGIPNYNQKFKGRATITIDTSSSTAYMELSSLTSEDTAVYYCARRRIAYGYDEGHAMDYWGQGTLVTVSSGGGGSGGGGSGGGGSDIVMTQTPLSLPVTVGEQASISCKSSQSLLYSRNQKNYLAWYLQKPGQSPKLLIYWASTRESGVPDRFSGSGSGTDFTLKISSVEAEDVGVYYCQQYFSYPLTFGGGTKVEIK |
| 1817. | FA22D8-HL | artificial | nt | CAGGTGCAGCTGGTCCAGTCTGGAGCTGAGGTGGAGAAGCCTGGGGCTTCAGTAAAGGTGTCCTGCAAGGCTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTATAGACACGTCCTCCAGCACCGCCTACATGGAGCTCAGCAGCCTGACATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTACCTGTGACAGTTGGAGAGCAGGCTTCTATCAGCTGCAAGTCCAGTCAGAGCCTTTTATATAGTCGTAATCAAAAGAACTACTTGGCCTGGTACCTGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAGATCAGCAGTGTGGAGGCTGAGGATGTTGGAGTCTATTACTGTCAGCAATATTTTAGCTATCCGCTCACGTTCGGTGGTGGGACCAAGGTGGAGATCAAA |
| 1818. | FA22D8 HL x I2C HL | artificial | aa | QVQLVQSGAEVEKPGASVKVSCKASGYTFTEYTIHWVRQAPGQRLEWMGGINPNNGIPNYNQKFKGRATITIDTSSSTAYMELSSLTSEDTAVYYCARRRIAYGYDEGHAMDYWG |

| | | | | |
|---|---|---|---|---|
| | | | | QGTLVTVSSGGGGSGGGGSGGGGSDIVMTQTPLSLPVTVGEQASISCKSSQSLLYSRNQKNYLAWYLQKPGQSPKLLIYWASTRESGVPDRFSGSGSGTDFTLKISSVEAEDVGVYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1819. | FA22D8 HL x I2C HL | artificial | nt | CAGGTGCAGCTGGTCCAGTCTGGAGCTGAGGTGGAGAAGCCTGGGGCTTCAGTAAAGGTGTCCTGCAAGGCTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTATAGACACGTCCTCCAGCACCGCCTACATGGAGCTCAGCAGCCTGACATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTACCTGTGACAGTTGGAGAGCAGGCTTCTATCAGCTGCAAGTCCAGTCAGAGCCTTTTATATAGTCGTAATCAAAAGAACTACTTGGCCTGGTACCTGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAGATCAGCAGTGTGGAGGCTGAGGATGTTGGAGTCTATTACTGTCAGCAATATTTTAGCTATCCGCTCACGTTCGGTGGTGGGACCAAGGTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1820. | FA22E8-H | artificial | aa | QVQLVQSGAELVKPGASVKVSCKASGYTFTEYTIHWVRQAPGQRLEWMGGINPNNGIP |

| | | | | |
|---|---|---|---|---|
| | | | | NYNQKFKGRATITIDTSASTAYMELRSLRSEDTAVYYCARRRIAYGYDEGHAMDYWGQGTLVTVSS |
| 1821. | FA22E8-HCDR1 | artificial | aa | EYTIH |
| 1822. | FA22E8-HCDR2 | artificial | aa | GINPNNGIPNYNQKFKG |
| 1823. | FA22E8-HCDR3 | artificial | aa | RRIAYGYDEGHAMDY |
| 1824. | FA22E8-H | artificial | nt | CAGGTGCAGCTGGTCCAGTCTGGAGCTGAGCTGGTGAAGCCTGGGGCTTCAGTAAAGGTGTCCTGCAAGGCTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTATAGACACGTCCGCCAGCACCGCCTACATGGAGCTCCGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCA |
| 1825. | FA22E8-L | artificial | aa | DIVMTQTPLSLAVTPGEQASISCKSSQSLLYSRNQKNYLAWYLQKPGQSPQLLIYWASTRESGVPDRFSGSGFGTDFTLKISGVEAEDVGVYYCQQYFSYPLTFGGGTKVEIK |
| 1826. | FA22E8-LCDR1 | artificial | aa | KSSQSLLYSRNQKNYLA |
| 1827. | FA22E8-LCDR2 | artificial | aa | WASTRES |
| 1828. | FA22E8-LCDR3 | artificial | aa | QQYFSYPLT |
| 1829. | FA22E8-L | artificial | nt | GACATCGTGATGACACAGACTCCACTCTCCCTAGCTGTGACACCTGGAGAGCAGGCTTCTATCAGCTGCAAGTCCAGTCAGAGCCTTTTATATAGTCGTAATCAAAAGAACTACTTGGCCTGGTACCTGCAGAAGCCAGGGCAGTCTCCTCAACTGCTGATTTACTGGGCATCCACTAGGGAATCTGGGGTCCCTGATCGCTTCTCAGGCAGTGGATTTGGGACGGATTTCACTCTCAAGATCAGCGGAGTGGAGGCTGAGGACGTGGGAGTTTATTACTGTCAGCAATATTTTAGCTATCCGCTCACGTTCGGTGGTGGGACCAAGGTGGAGATCAAA |
| 1830. | FA22E8-HL | artificial | aa | QVQLVQSGAELVKPGASVKVSCKASGYTFTEYTIHWVRQAPGQRLEWMGGINPNNGIPNYNQKFKGRATITIDTSASTAYMELRSLRSEDTAVYYCARRRIAYGYDEGHAMDYWGQGTLVTVSSGGGGSGGGGSGGGGSDIVMTQTPLSLAVTPGEQASISCKSSQSLLYSRNQKNYLAWYLQKPGQSPQLLIYWASTRESGVPDRFSGSGFGTDFTLKISGVEAEDVGVYYCQQYFSYPLTFGGGTKVEIK |
| 1831. | FA22E8-HL | artificial | nt | CAGGTGCAGCTGGTCCAGTCTGGAGCTGAGCTGGTGAAGCCTGGGGCTTCAGTAAAGGTGTCCTGCAAGGCTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTATAGACACGTCCGCCAGCACCGCCTACATGGAGCTCCGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGC |

| | | | | |
|---|---|---|---|---|
| | | | | GGCGGCGGCTCCGGTGGTGGTGGTTCTGACATCGTGATGACACAGACTCCACTCT<br>CCCTAGCTGTGACACCTGGAGAGCAGGCTTCTATCAGCTGCAAGTCCAGTCAGAGC<br>CTTTTATATAGTCGTAATCAAAAGAACTACTTGGCCTGGTACCTGCAGAAGCCAGGG<br>CAGTCTCCTCAACTGCTGATTTACTGGGCATCCACTAGGGAATCTGGGGTCCCTGA<br>TCGCTTCTCAGGCAGTGGATTTGGGACGGATTTCACTCTCAAGATCAGCGGAGTGG<br>AGGCTGAGGACGTGGGAGTTTATTACTGTCAGCAATATTTTAGCTATCCGCTCACGT<br>TCGGTGGTGGGACCAAGGTGGAGATCAAA |
| 1832. | FA22E8 HL x I2C HL | artificial | aa | QVQLVQSGAELVKPGASVKVSCKASGYTFTEYTIHWVRQAPGQRLEWMGGINPNNGIP<br>NYNQKFKGRATITIDTSASTAYMELRSLRSEDTAVYYCARRRIAYGYDEGHAMDYWGQ<br>GTLVTVSSGGGGSGGGGSGGGGSDIVMTQTPLSLAVTPGEQASISCKSSQSLLYSRN<br>QKNYLAWYLQKPGQSPQLLIYWASTRESGVPDRFSGSGFGTDFTLKISGVEAEDVGVY<br>YCQQYFSYPLTFGGGTKVEIKSGGGGSEVQLVESGGGLVQPGGSLKLSCAASGFTFNK<br>YAMNWVRQAPGKGLEWVARIRSKYNNYATYYADSVKDRFTISRDDSKNTAYLQMNNL<br>KTEDTAVYYCVRHGNFGNSYISYWAYWGQGTLVTVSSGGGGSGGGGSGGGGSQTVV<br>TQEPSLTVSPGGTVTLTCGSSTGAVTSGNYPNWVQQKPGQAPRGLIGGTKFLAPGTPA<br>RFSGSLLGGKAALTLSGVQPEDEAEYYCVLWYSNRWVFGGGTKLTVL |
| 1833. | FA22E8 HL x I2C HL | artificial | nt | CAGGTGCAGCTGGTCCAGTCTGGAGCTGAGCTGGTGAAGCCTGGGGCTTCAGTAA<br>AGGTGTCCTGCAAGGCTTCTGGATACACATTCACTGAATACACCATACACTGGGTG<br>AGACAGGCCCCTGGACAGAGACTTGAGTGGATGGGAGGTATTAATCCTAACAATGG<br>TATTCCTAACTACAATCAGAAGTTCAAGGGCAGGGCCACAATAACTATAGACACGTC<br>CGCCAGCACCGCCTACATGGAGCTCCGCAGCCTGAGATCTGAGGATACTGCGGTC<br>TATTACTGTGCAAGAAGAAGAATCGCCTATGGTTACGACGAGGGCCATGCTATGGA<br>CTACTGGGGTCAAGGAACCCTGGTCACCGTCTCCTCAGGTGGTGGTGGTTCTGGC<br>GGCGGCGGCTCCGGTGGTGGTGGTTCTGACATCGTGATGACACAGACTCCACTCT<br>CCCTAGCTGTGACACCTGGAGAGCAGGCTTCTATCAGCTGCAAGTCCAGTCAGAGC<br>CTTTTATATAGTCGTAATCAAAAGAACTACTTGGCCTGGTACCTGCAGAAGCCAGGG<br>CAGTCTCCTCAACTGCTGATTTACTGGGCATCCACTAGGGAATCTGGGGTCCCTGA<br>TCGCTTCTCAGGCAGTGGATTTGGGACGGATTTCACTCTCAAGATCAGCGGAGTGG<br>AGGCTGAGGACGTGGGAGTTTATTACTGTCAGCAATATTTTAGCTATCCGCTCACGT<br>TCGGTGGTGGGACCAAGGTGGAGATCAAATCCGGAGGTGGTGGATCCGAGGTGCA<br>GCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGC<br>TCCAGGAAAGGGTTTGGAATGGGTTGCTCGCATAAGAAGTAAATATAATAATTATGC<br>AACATATTATGCCGATTCAGTGAAAGACAGGTTCACCATCTCCAGAGATGATTCAAA<br>AAACACTGCCTATCTACAAATGAACAACTTGAAAACTGAGGACACTGCCGTGTACTA<br>CTGTGTGAGACATGGGAACTTCGGTAATAGCTACATATCCTACTGGGCTTACTGGG |

| | | | | |
|---|---|---|---|---|
| | | | | GCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1834. | FA20H3-H | artificial | aa | QVQLVQSGAEVKKPGASVKMSCKTSGYTFTEYTIHWVRQAPGQRLEWMGGINPNNGIPNYNQKFKGRATITRDTSSSTAYMELSSLRSEDTAVYYCARRRIAYGYDEGHAMDYWGQGTLVTVSS |
| 1835. | FA20H3-HCDR1 | artificial | aa | EYTIH |
| 1836. | FA20H3-HCDR2 | artificial | aa | GINPNNGIPNYNQKFKG |
| 1837. | FA20H3-HCDR3 | artificial | aa | RRIAYGYDEGHAMDY |
| 1838. | FA20H3-H | artificial | nt | CAGGTGCAGCTGGTCCAGTCTGGAGCTGAGGTGAAGAAGCCTGGGGCTTCAGTAAAGATGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTAGAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCA |
| 1839. | FA20H3-L | artificial | aa | DIVMTQTPFSLPVTPGEPASISCKSSQSLLYSRNQKNYLAWYQQKPGQSPQLLIYWASTRESGVPDRFSGSGSGTDFTLNISRVEAEDVGVYYCQQYFSYPLTFGGGTKVEIK |
| 1840. | FA20H3-LCDR1 | artificial | aa | KSSQSLLYSRNQKNYLA |
| 1841. | FA20H3-LCDR2 | artificial | aa | WASTRES |
| 1842. | FA20H3-LCDR3 | artificial | aa | QQYFSYPLT |
| 1843. | FA20H3-L | artificial | nt | GACATCGTGATGACACAGACTCCATTCTCCCTACCTGTGACACCTGGAGAGCCGGCTTCTATCAGCTGCAAGTCCAGTCAGAGCCTTTTATATAGTCGTAATCAAAAGAACTACTTGGCCTGGTACCAGCAGAAGCCAGGGCAGTCTCCTCAACTGCTGATTTACTGGGCATCCACTAGGGAATCTGGGGTCCCTGATCGCTTCTCAGGCAGTGGATCTGGGACAGACTTCACCCTCAATATCTCTCGTGTGGAGGCGGAGGATGTTGGAGTCTATTACTGTCAGCAATATTTTAGCTATCCGCTCACGTTCGGTGGTGGGACCAAGGTGGAGATCAAA |
| 1844. | FA20H3-HL | artificial | aa | QVQLVQSGAEVKKPGASVKMSCKTSGYTFTEYTIHWVRQAPGQRLEWMGGINPNNGIPNYNQKFKGRATITRDTSSSTAYMELSSLRSEDTAVYYCARRRIAYGYDEGHAMDYWG |

| | | | | |
|---|---|---|---|---|
| | | | | QGTLVTVSSGGGGSGGGGSGGGGSDIVMTQTPFSLPVTPGEPASISCKSSQSLLYSRNQKNYLAWYQQKPGQSPQLLIYWASTRESGVPDRFSGSGSGTDFTLNISRVEAEDVGVYYCQQYFSYPLTFGGGTKVEIK |
| 1845. | FA20H3-HL | artificial | nt | CAGGTGCAGCTGGTCCAGTCTGGAGCTGAGGTGAAGAAGCCTGGGGCTTCAGTAAAGATGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTAGAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCATTCTCCCTACCTGTGACACCTGGAGAGCCGGCTTCTATCAGCTGCAAGTCCAGTCAGAGCCTTTTATATAGTCGTAATCAAAAGAACTACTTGGCCTGGTACCAGCAGAAGCCAGGGCAGTCTCCTCAACTGCTGATTTACTGGGCATCCACTAGGGAATCTGGGGTCCCTGATCGCTTCTCAGGCAGTGGATCTGGGACAGACTTCACCCTCAATATCTCTCGTGTGGAGGCGGAGGATGTTGGAGTCTATTACTGTCAGCAATATTTTAGCTATCCGCTCACGTTCGGTGGTGGGACCAAGGTGGAGATCAAA |
| 1846. | FA20H3 HL x I2C HL | artificial | aa | QVQLVQSGAEVKKPGASVKMSCKTSGYTFTEYTIHWVRQAPGQRLEWMGGINPNNGIPNYNQKFKGRATITRDTSSSTAYMELSSLRSEDTAVYYCARRRIAYGYDEGHAMDYWGQGTLVTVSSGGGGSGGGGSGGGGSDIVMTQTPFSLPVTPGEPASISCKSSQSLLYSRNQKNYLAWYQQKPGQSPQLLIYWASTRESGVPDRFSGSGSGTDFTLNISRVEAEDVGVYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1847. | FA20H3 HL x I2C HL | artificial | nt | CAGGTGCAGCTGGTCCAGTCTGGAGCTGAGGTGAAGAAGCCTGGGGCTTCAGTAAAGATGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACAATAACTAGAGACACGTCCTCCAGCACCGCCTACATGGAGCTCAGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCATTCTCCCTACCTGTGACACCTGGAGAGCCGGCTTCTATCAGCTGCAAGTCCAGTCAGAGCCTTTTATATAGTCGTAATCAAAAGAACTACTTGGCCTGGTACCAGCAGAAGCCAGGGC |

| | | | | |
|---|---|---|---|---|
| | | | | AGTCTCCTCAACTGCTGATTTACTGGGCATCCACTAGGGAATCTGGGGTCCCTGATCGCTTCTCAGGCAGTGGATCTGGGACAGACTTCACCCTCAATATCTCTCGTGTGGAGGCGGAGGATGTTGGAGTCTATTACTGTCAGCAATATTTTAGCTATCCGCTCACGTTCGGTGGTGGGACCAAGGTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1848. | FA19D12-H | artificial | aa | QVQLVQSGAEVKKPGASVKVSCKASGYTFTEYTIHWVRQAHGQSLEWMGGINPNNGIPNYNQKFKGRVTITVDTSASTAYMELRSLRSEDTAVYYCARRRIAYGYDEGHAMDYWGQGTLVTVSS |
| 1849. | FA19D12-HCDR1 | artificial | aa | EYTIH |
| 1850. | FA19D12-HCDR2 | artificial | aa | GINPNNGIPNYNQKFKG |
| 1851. | FA19D12-HCDR3 | artificial | aa | RRIAYGYDEGHAMDY |
| 1852. | FA19D12-H | artificial | nt | CAGGTGCAGCTGGTCCAGTCTGGAGCTGAGGTGAAGAAGCCTGGGGCTTCAGTAAAGGTGTCCTGCAAGGCTTCTGGATACACATTCACTGAATACACCATACACTGGGTGAGACAGGCCCATGGACAGAGCCTTGAGTGGATGGGAGGTATTAATCCTAACAATGGTATTCCTAACTACAATCAGAAGTTCAAGGGCAGGGTCACAATAACTGTAGACACGTCCGCCAGCACCGCCTACATGGAGCTCCGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCT |
| 1853. | FA19D12-L | artificial | aa | DIVMTQTPFSLPVTPGEPASISCKSSQSLLYSRNQKNYLAWYQQKPGQSPKLLIYWASTRESGVPDRFSGSGSGTDFTLKISRVEAEDVGVYYCQQYFSYPLTFGGGTKVEIK |
| 1854. | FA19D12-LCDR1 | artificial | aa | KSSQSLLYSRNQKNYLA |
| 1855. | FA19D12-LCDR2 | artificial | aa | WASTRES |

| | | | | |
|---|---|---|---|---|
| 1856. | FA19D12-LCDR3 | artificial | aa | QQYFSYPLT |
| 1857. | FA19D12-L | artificial | nt | GACATCGTGATGACACAGACTCCATTCTCCCTACCTGTGACACCTGGAGAGCCGGCTTCTATCAGCTGCAAGTCCAGTCAGAGCCTTTTATATAGTCGTAATCAAAAGAACTACTTGGCCTGGTACCAGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAAATCTCCAGAGTGGAGGCTGAGGACGTGGGAGTTTATTACTGTCAGCAATATTTTAGCTATCCGCTCACGTTCGGTGGTGGGACCAAGGTGGAGATCAAA |
| 1858. | FA19D12-HL | artificial | aa | QVQLVQSGAEVKKPGASVKVSCKASGYTFTEYTIHWVRQAHGQSLEWMGGINPNNGIPNYNQKFKGRVTITVDTSASTAYMELRSLRSEDTAVYYCARRRIAYGYDEGHAMDYWGQGTLVTVSSGGGGSGGGGSGGGGSDIVMTQTPFSLPVTPGEPASISCKSSQSLLYSRNQKNYLAWYQQKPGQSPKLLIYWASTRESGVPDRFSGSGSGTDFTLKISRVEAEDVGVYYCQQYFSYPLTFGGGTKVEIK |
| 1859. | FA19D12-HL | artificial | nt | CAGGTGCAGCTGGTCCAGTCTGGAGCTGAGGTGAAGAAGCCTGGGGCTTCAGTAAAGGTGTCCTGCAAGGCTTCTGGATACACATTCACTGAATACACCATACACTGGGTGAGACAGGCCCATGGACAGAGCCTTGAGTGGATGGGAGGTATTAATCCTAACAATGGTATTCCTAACTACAATCAGAAGTTCAAGGGCAGGGTCACAATAACTGTAGACACGTCCGCCAGCACCGCCTACATGGAGCTCCGCAGCCTGAGATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCATTCTCCCTACCTGTGACACCTGGAGAGCCGGCTTCTATCAGCTGCAAGTCCAGTCAGAGCCTTTTATATAGTCGTAATCAAAAGAACTACTTGGCCTGGTACCAGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAAATCTCCAGAGTGGAGGCTGAGGACGTGGGAGTTTATTACTGTCAGCAATATTTTAGCTATCCGCTCACGTTCGGTGGTGGGACCAAGGTGGAGATCAAA |
| 1860. | FA19D12HL x I2C HL | artificial | aa | QVQLVQSGAEVKKPGASVKVSCKASGYTFTEYTIHWVRQAHGQSLEWMGGINPNNGIPNYNQKFKGRVTITVDTSASTAYMELRSLRSEDTAVYYCARRRIAYGYDEGHAMDYWGQGTLVTVSSGGGGSGGGGSGGGGSDIVMTQTPFSLPVTPGEPASISCKSSQSLLYSRNQKNYLAWYQQKPGQSPKLLIYWASTRESGVPDRFSGSGSGTDFTLKISRVEAEDVGVYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |

| | | | | |
|---|---|---|---|---|
| 1861. | FA19D12HL x I2C HL | artificial | nt | CAGGTGCAGCTGGTCCAGTCTGGAGCTGAGGTGAAGAAGCCTGGGGCTTCAGTAA AGGTGTCCTGCAAGGCTTCTGGATACACATTCACTGAATACACCATACACTGGGTG AGACAGGCCCATGGACAGAGCCTTGAGTGGATGGGAGGTATTAATCCTAACAATGG TATTCCTAACTACAATCAGAAGTTCAAGGGCAGGGTCACAATAACTGTAGACACGTC CGCCAGCACCGCCTACATGGAGCTCCGCAGCCTGAGATCTGAGGATACTGCGGTC TATTACTGTGCAAGAAGAAGAATCGCCTATGGTTACGACGAGGGCCATGCTATGGA CTACTGGGGTCAAGGAACCCTGGTCACCGTCTCCTCAGGTGGTGGTGGTTCTGGC GGCGGCGGCTCCGGTGGTGGTGGTTCTGACATCGTGATGACACAGACTCCATTCT CCCTACCTGTGACACCTGGAGAGCCGGCTTCTATCAGCTGCAAGTCCAGTCAGAGC CTTTTATATAGTCGTAATCAAAAGAACTACTTGGCCTGGTACCAGCAGAAGCCAGGG CAGTCTCCTAAACTGCTGATTTACTGGGCATCCACTAGGGAATCTGGGGTCCCTGA TCGCTTCTCAGGCAGTGGATCTGGGACGGATTTCACTCTCAAAATCTCCAGAGTGG AGGCTGAGGACGTGGGAGTTTATTACTGTCAGCAATATTTTAGCTATCCGCTCACGT TCGGTGGTGGGACCAAGGTGGAGATCAAATCCGGAGGTGGTGGATCCGAGGTGCA GCTGGTCGAGTCTGGAGGAGGATTGGTGCAGCCTGGAGGGTCATTGAAACTCTCA TGTGCAGCCTCTGGATTCACCTTCAATAAGTACGCCATGAACTGGGTCCGCCAGGC TCCAGGAAAGGGTTTGGAATGGGTTGCTCGCATAAGAAGTAAATATAATAATTATGC AACATATTATGCCGATTCAGTGAAAGACAGGTTCACCATCTCCAGAGATGATTCAAA AAACACTGCCTATCTACAAATGAACAACTTGAAAACTGAGGACACTGCCGTGTACTA CTGTGTGAGACATGGGAACTTCGGTAATAGCTACATATCCTACTGGGCTTACTGGG GCCAAGGGACTCTGGTCACCGTCTCCTCAGGTGGTGGTGGTTCTGGCGGCGGCGG CTCCGGTGGTGGTGGTTCTCAGACTGTTGTGACTCAGGAACCTTCACTCACCGTAT CACCTGGTGGAACAGTCACACTCACTTGTGGCTCCTCGACTGGGGCTGTTACATCT GGCAACTACCCAAACTGGGTCCAACAAAAACCAGGTCAGGCACCCCGTGGTCTAAT AGGTGGGACTAAGTTCCTCGCCCCCGGTACTCCTGCCAGATTCTCAGGCTCCCTGC TTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAAC TGACTGTCCTA |
| 1862. | FA19D9-H | artificial | aa | QVQLVQSGAEVKKPGASVKVSCKTSGYTFTEYTIHWVRQAPGQRLEWMGGINPNNGI PNYNQKFKGRATLTIDTSASTAYMELSSLTSEDTAVYYCARRRIAYGYDEGHAMDYWG QGTLVTVSS |
| 1863. | FA19D9-HCDR1 | artificial | aa | EYTIH |
| 1864. | FA19D9-HCDR2 | artificial | aa | GINPNNGIPNYNQKFKG |
| 1865. | FA19D9-HCDR3 | artificial | aa | RRIAYGYDEGHAMDY |
| 1866. | FA19D9-H | artificial | nt | CAGGTGCAGCTGGTCCAGTCTGGAGCTGAGGTGAAGAAGCCTGGGGCTTCAGTAA AGGTGTCCTGCAAGACTTCTGGATACACATTCACTGAATACACCATACACTGGGTGA |

| | | | | |
|---|---|---|---|---|
| | | | | GACAGGCCCCTGGACAGAGACTTGAGTGGATGGGAGGTATTAATCCTAACAATGGT ATTCCTAACTACAATCAGAAGTTCAAGGGCAGGGCCACATTAACTATAGACACGTCC GCCAGCACCGCCTACATGGAGCTCAGCAGCCTGACATCTGAGGATACTGCGGTCT ATTACTGTGCAAGAAGAAGAATCGCCTATGGTTACGACGAGGGCCATGCTATGGAC TACTGGGGTCAAGGAACCCTGGTCACCGTCTCCTCA |
| 1867. | FA19D9-L | artificial | aa | DIVMTQTPLSLPVTPGEPASMSCKSSQSLLYSRNQKNYLAWFLQKPGQSPKLLIYWAST RESGVPDRFSGSGSGTDFTLKISRVEAEDVGVYYCQQYFSYPLTFGGGTKVEIK |
| 1868. | FA19D9-LCDR1 | artificial | aa | KSSQSLLYSRNQKNYLA |
| 1869. | FA19D9-LCDR2 | artificial | aa | WASTRES |
| 1870. | FA19D9-LCDR3 | artificial | aa | QQYFSYPLT |
| 1871. | FA19D9-L | artificial | nt | GACATCGTGATGACACAGACTCCACTCTCCCTACCTGTGACACCTGGAGAGCCGGC TTCTATGAGCTGCAAGTCCAGTCAGAGCCTTTTATATAGTCGTAATCAAAAGAACTA CTTGGCCTGGTTCCTGCAGAAGCCAGGGCAGTCTCCTAAACTGCTGATTTACTGGG CATCCACTAGGGAATCTGGGGTCCCTGATCGCTTCTCAGGCAGTGGATCTGGGAC GGATTTCACTCTCAAGATCAGCAGAGTGGAGGCTGAGGACGTGGGAGTTTATTACT GTCAGCAATATTTTAGCTATCCGCTCACGTTCGGTGGTGGGACCAAGGTGGAGATC AAA |
| 1872. | FA19D9-HL | artificial | aa | QVQLVQSGAEVKKPGASVKVSCKTSGYTFTEYTIHWVRQAPGQRLEWMGGINPNNGI PNYNQKFKGRATLTIDTSASTAYMELSSLTSEDTAVYYCARRIAYGYDEGHAMDYWG QGTLVTVSSGGGGSGGGGSGGGGSDIVMTQTPLSLPVTPGEPASMSCKSSQSLLYSR NQKNYLAWFLQKPGQSPKLLIYWASTRESGVPDRFSGSGSGTDFTLKISRVEAEDVGV YYCQQYFSYPLTFGGGTKVEIK |
| 1873. | FA19D9-HL | artificial | nt | CAGGTGCAGCTGGTCCAGTCTGGAGCTGAGGTGAAGAAGCCTGGGGCTTCAGTAA AGGTGTCCTGCAAGACTTCTGGATACACATTCACTGAATACACCATACACTGGGTGA GACAGGCCCCTGGACAGAGACTTGAGTGGATGGGAGGTATTAATCCTAACAATGGT ATTCCTAACTACAATCAGAAGTTCAAGGGCAGGGCCACATTAACTATAGACACGTCC GCCAGCACCGCCTACATGGAGCTCAGCAGCCTGACATCTGAGGATACTGCGGTCT ATTACTGTGCAAGAAGAAGAATCGCCTATGGTTACGACGAGGGCCATGCTATGGAC TACTGGGGTCAAGGAACCCTGGTCACCGTCTCCTCAGGTGGTGGTGGTTCTGGCG GCGGCGGCTCCGGTGGTGGTGGTTCTGACATCGTGATGACACAGACTCCACTCTC CCTACCTGTGACACCTGGAGAGCCGGCTTCTATGAGCTGCAAGTCCAGTCAGAGCC TTTTATATAGTCGTAATCAAAAGAACTACTTGGCCTGGTTCCTGCAGAAGCCAGGGC AGTCTCCTAAACTGCTGATTTACTGGGCATCCACTAGGGAATCTGGGGTCCCTGAT CGCTTCTCAGGCAGTGGATCTGGGACGGATTTCACTCTCAAGATCAGCAGAGTGGA GGCTGAGGACGTGGGAGTTTATTACTGTCAGCAATATTTTAGCTATCCGCTCACGTT CGGTGGTGGGACCAAGGTGGAGATCAAA |

| | | | | |
|---|---|---|---|---|
| 1874. | FA19D9HL x I2C HL | artificial | aa | QVQLVQSGAEVKKPGASVKVSCKTSGYTFTEYTIHWVRQAPGQRLEWMGGINPNNGIPNYNQKFKGRATLTIDTSASTAYMELSSLTSEDTAVYYCARRIAYGYDEGHAMDYWGQGTLVTVSSGGGGSGGGGSGGGGSDIVMTQTPLSLPVTPGEPASMSCKSSQSLLYSRNQKNYLAWFLQKPGQSPKLLIYWASTRESGVPDRFSGSGSGTDFTLKISRVEAEDVGVYYCQQYFSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1875. | FA19D9HL x I2C HL | artificial | nt | CAGGTGCAGCTGGTCCAGTCTGGAGCTGAGGTGAAGAAGCCTGGGGCTTCAGTAAAGGTGTCCTGCAAGACTTCTGGATACACATTCACTGAATACACCATACACTGGGTGAGACAGGCCCCTGGACAGAGACTTGAGTGGATGGGAGGTATTAATCCTAACAATGGTATTCCTAACTACAATCAGAAGTTCAAGGGCAGGGCCACATTAACTATAGACACGTCCGCCAGCACCGCCTACATGGAGCTCAGCAGCCTGACATCTGAGGATACTGCGGTCTATTACTGTGCAAGAAGAAGAATCGCCTATGGTTACGACGAGGGCCATGCTATGGACTACTGGGGTCAAGGAACCCTGGTCACCGTCTCCTCAGGTGGTGGTGGTTCTGGCGGCGGCGGCTCCGGTGGTGGTGGTTCTGACATCGTGATGACACAGACTCCACTCTCCCTACCTGTGACACCTGGAGAGCCGGCTTCTATGAGCTGCAAGTCCAGTCAGAGCCTTTTATATAGTCGTAATCAAAAGAACTACTTGGCCTGGTTCCTGCAGAAGCCAGGGCAGTCTCCTAAACTGCTGATTTACTGGGCATCCACTAGGGAATCTGGGGTCCCTGATCGCTTCTCAGGCAGTGGATCTGGGACGGATTTCACTCTCAAGATCAGCAGAGTGGAGGCTGAGGACGTGGGAGTTTATTACTGTCAGCAATATTTTAGCTATCCGCTCACGTTCGGTGGTGGGACCAAGGTGGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAAC |

| | | | | |
|---|---|---|---|---|
| | | | | TGACTGTCCTA |
| 1876. | FA87G9-H | murine | aa | EVQLLESGGGLVQPGGSLKLSCAASGFDFSRYWMSWVRQAPGKGLEWIGEINPDSSTINYTPSLKDKFIISRDNAKNTLYLQMSKVRSEDTALYYCAGGYYRYPYYYTMDYWGQGTTVTVSS |
| 1877. | FA87G9-HCDR1 | murine | aa | RYWMS |
| 1878. | FA87G9-HCDR2 | murine | aa | EINPDSSTINYTPSLKD |
| 1879. | FA87G9-HCDR3 | murine | aa | GYYRYPYYYTMDY |
| 1880. | FA87G9-H | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAGCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCA |
| 1881. | FA87G9-L | murine | aa | ELVMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1882. | FA87G9-LCDR1 | murine | aa | RASGNIHNYLA |
| 1883. | FA87G9-LCDR2 | murine | aa | NAKTLAD |
| 1884. | FA87G9-LCDR3 | murine | aa | QHFWSTPYT |
| 1885. | FA87G9-L | murine | nt | GAGCTCGTGATGACACAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1886. | FA87G9-HL | murine | aa | EVQLLESGGGLVQPGGSLKLSCAASGFDFSRYWMSWVRQAPGKGLEWIGEINPDSSTINYTPSLKDKFIISRDNAKNTLYLQMSKVRSEDTALYYCAGGYYRYPYYYTMDYWGQGTTVTVSSGGGGSGGGGSGGGGSELVMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1887. | FA87G9-HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAGCAAAGTGAGATCTGAGGACACAGCCCTTTATTACTGTGCAGGAGGATACTATAGGTACCCATATTACTATACTATGGACTACTGGGGC |

| | | | | |
|---|---|---|---|---|
| | | | | CAAGGGACCACGGTCACCGTCTCCTCAGGTGGTGGTGGTTCTGGCGGCGGCGGCT<br>CCGGTGGTGGTGGTTCTGAGCTCGTGATGACACAGTCTCCAGCCTCCCTATCTGCA<br>TCTGTGGGAGAAACTGTCACCATCACATGTCGAGCAAGTGGGAATATTCACAATTAT<br>TTAGCATGGTATCAGCAGAAACAGGGAAAATCTCCTCAGCTCCTGGTCTATAATGCA<br>AAAACCTTAGCAGATGGTGTGCCATCAAGGTTCAGTGGCAGTGGATCAGGAACACA<br>ATATTCTCTCAAGATCAACAGCCTGCAGCCTGAAGATTTTGGGAGTTATTACTGTCA<br>ACATTTTTGGAGTACTCCGTACACGTTCGGAGGGGGGACCAAGCTTGAGATCAAA |
| 1888. | FA87G9 HL x I2C HL | murine | aa | EVQLLESGGGLVQPGGSLKLSCAASGFDFSRYWMSWVRQAPGKGLEWIGEINPDSSTI<br>NYTPSLKDKFIISRDNAKNTLYLQMSKVRSEDTALYYCAGGYYRYPYYYTMDYWGQGT<br>TVTVSSGGGGSGGGGSGGGGSELVMTQSPASLSASVGETVTITCRASGNIHNYLAWY<br>QQKQGKSPQLLVYNAKTLADGVPSRFSGSGSGTQYSLKINSLQPEDFGSYYCQHFWS<br>TPYTFGGGTKLEIKSGGGGSEVQLVESGGGLVQPGGSLKLSCAASGFTFNKYAMNWV<br>RQAPGKGLEWVARIRSKYNNYATYYADSVKDRFTISRDDSKNTAYLQMNNLKTEDTAV<br>YYCVRHGNFGNSYISYWAYWGQGTLVTVSSGGGGSGGGGSGGGGSQTVVTQEPSLT<br>VSPGGTVTLTCGSSTGAVTSGNYPNWVQQKPGQAPRGLIGGTKFLAPGTPARFSGSLL<br>GGKAALTLSGVQPEDEAEYYCVLWYSNRWVFGGGTKLTVL |
| 1889. | FA87G9 HL x I2C HL | murine | nt | GAGGTGCAGCTGCTCGAGTCTGGAGGTGGCCTGGTGCAGCCTGGAGGATCCCTGA<br>AACTCTCCTGTGCAGCCTCAGGATTCGATTTTAGTAGATACTGGATGAGTTGGGTCC<br>GGCAGGCTCCAGGGAAAGGGCTAGAATGGATTGGAGAAATTAATCCAGATAGCAGT<br>ACGATAAACTATACGCCATCTCTAAAGGATAAATTCATCATCTCCAGAGACAACGCC<br>AAAAATACGCTGTACCTGCAAATGAGCAAAGTGAGATCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCAGGTGGTGGTGGTTCTGGCGGCGGCGGCT<br>CCGGTGGTGGTGGTTCTGAGCTCGTGATGACACAGTCTCCAGCCTCCCTATCTGCA<br>TCTGTGGGAGAAACTGTCACCATCACATGTCGAGCAAGTGGGAATATTCACAATTAT<br>TTAGCATGGTATCAGCAGAAACAGGGAAAATCTCCTCAGCTCCTGGTCTATAATGCA<br>AAAACCTTAGCAGATGGTGTGCCATCAAGGTTCAGTGGCAGTGGATCAGGAACACA<br>ATATTCTCTCAAGATCAACAGCCTGCAGCCTGAAGATTTTGGGAGTTATTACTGTCA<br>ACATTTTTGGAGTACTCCGTACACGTTCGGAGGGGGGACCAAGCTTGAGATCAAAT<br>CCGGAGGTGGTGGATCCGAGGTGCAGCTGGTCGAGTCTGGAGGAGGATTGGTGC<br>AGCCTGGAGGGTCATTGAAACTCTCATGTGCAGCCTCTGGATTCACCTTCAATAAGT<br>ACGCCATGAACTGGGTCCGCCAGGCTCCAGGAAAGGGTTTGGAATGGGTTGCTCG<br>CATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGA<br>AAACTGAGGACACTGCCGTGTACTACTGTGTGAGACATGGGAACTTCGGTAATAGC<br>TACATATCCTACTGGGCTTACTGGGGCCAAGGGACTCTGGTCACCGTCTCCTCAGG |

| | | | | |
|---|---|---|---|---|
| | | | | TGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1890. | FA86C12-H | murine | aa | EVQLLESGGGLVQPGGSLKLSCAASGFDFSRSWMSWVRQAPGKGLEWIGEINPDSSTINYTPSLKDKFIISRDNAKNTLYLQMSKVRSEDTALYYCAGGYYRYPYYYTMDYWGQGTTVTVSS |
| 1891. | FA86C12-HCDR1 | murine | aa | RSWMS |
| 1892. | FA86C12-HCDR2 | murine | aa | EINPDSSTINYTPSLKD |
| 1893. | FA86C12-HCDR3 | murine | aa | GYYRYPYYYTMDY |
| 1894. | FA86C12-H | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATCCTGGATGAGTTGGGTCCGGCAGGCTCCAGGGAAAGGGCTTGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAGCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCA |
| 1895. | FA86C12-L | murine | aa | ELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1896. | FA86C12-LCDR1 | murine | aa | RASGNIHNYLA |
| 1897. | FA86C12-LCDR2 | murine | aa | NAKTLAD |
| 1898. | FA86C12-LCDR3 | murine | aa | QHFWSTPYT |
| 1899. | FA86C12-L | murine | nt | GAGCTCCAGATGACCCAGTCTCCAGCCTCCCTATCTGCATCTGTGGGAGAAACTGTCACCATCACATGTCGAGCAAGTGGGAATATTCACAATTATTTGGCATGGTATCAGCAGAAACAGGGAAAATCTCCTCAGCTCCTAGTCTATAATGCAAAAACCTTAGCGGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1900. | FA86C12-HL | murine | aa | EVQLLESGGGLVQPGGSLKLSCAASGFDFSRSWMSWVRQAPGKGLEWIGEINPDSSTINYTPSLKDKFIISRDNAKNTLYLQMSKVRSEDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |

| | | | | |
|---|---|---|---|---|
| 1901. | FA86C12-HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATCCTGGATGAGTTGGGTCCGGCAGGCTCCAGGGAAAGGGCTTGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAGCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTGGCATGGTATCAGCAGAAACAGGGAAAATCTCCTCAGCTCCTAGTCTATAATGCAAAAACCTTAGCGGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1902. | FA86C12 HL x I2C HL | murine | aa | EVQLLESGGGLVQPGGSLKLSCAASGFDFSRSWMSWVRQAPGKGLEWIGEINPDSSTINYTPSLKDKFIISRDNAKNTLYLQMSKVRSEDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1903. | FA86C12 HL x I2C HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATCCTGGATGAGTTGGGTCCGGCAGGCTCCAGGGAAAGGGCTTGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAGCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTGGCATGGTATCAGCAGAAACAGGGAAAATCTCCTCAGCTCCTAGTCTATAATGCAAAAACCTTAGCGGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAAT |

| | | | | |
|---|---|---|---|---|
| | | | | CCGGAGGTGGTGGATCCGAGGTGCAGCTGGTCGAGTCTGGAGGAGGATTGGTGC<br>AGCCTGGAGGGTCATTGAAACTCTCATGTGCAGCCTCTGGATTCACCTTCAATAAGT<br>ACGCCATGAACTGGGTCCGCCAGGCTCCAGGAAAGGGTTTGGAATGGGTTGCTCG<br>CATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGA<br>AAACTGAGGACACTGCCGTGTACTACTGTGTGAGACATGGGAACTTCGGTAATAGC<br>TACATATCCTACTGGGCTTACTGGGGCCAAGGGACTCTGGTCACCGTCTCCTCAGG<br>TGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGG<br>CTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTAC<br>TCCTGCCAGATTCTCAGGCTCCCTGCTTGGAGGCAAGGCTGCCCTCACCCTCTCAG<br>GGGTACAGCCAGAGGATGAGGCAGAATATTACTGTGTTCTATGGTACAGCAACCGC<br>TGGGTGTTCGGTGGAGGAACCAAACTGACTGTCCTA |
| 1904. | FA86D2-H | murine | aa | EVQLLESGGGLVQPGGSLKLSCAASGFDFSTYWMSWVRQAPGKGLEWIGEINPDSSTI<br>NYTPSLKDKFIISRDNAKNTLYLQMNKVRSEDTALYYCAGGYYRYPYYYTMDYWGQGT<br>TVTVSS |
| 1905. | FA86D2-HCDR1 | murine | aa | TYWMS |
| 1906. | FA86D2-HCDR2 | murine | aa | EINPDSSTINYTPSLKD |
| 1907. | FA86D2-HCDR3 | murine | aa | GYYRYPYYYTMDY |
| 1908. | FA86D2-H | murine | nt | GAGGTGCAGCTGCTCGAGTCTGGAGGTGGCCTGGTGCAGCCTGGAGGATCCCTGA<br>AACTCTCCTGTGCAGCCTCAGGATTCGATTTTAGTACATACTGGATGAGTTGGGTCC<br>GGCAGGCTCCAGGGAAAGGGCTAGAATGGATTGGAGAAATTAATCCAGATAGCAGT<br>ACGATAAACTATACGCCATCTCTAAAGGATAAATTCATCATCTCCAGAGACAACGCC<br>AAAAATACGCTGTACCTGCAAATGAACAAAGTGAGATCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCA |
| 1909. | FA86D2-L | murine | aa | ELVMTQSPASLSASVGETVTITCRASGNIHNYLAWYQQKQGKSPQLLVYNAKTLADGV<br>PSRFSGSGSGTQYSLKINSLQPEDFGSYYCQHFWSTPYTFGGGTKLEIK |
| 1910. | FA86D2-LCDR1 | murine | aa | RASGNIHNYLA |
| 1911. | FA86D2-LCDR2 | murine | aa | NAKTLAD |
| 1912. | FA86D2-LCDR3 | murine | aa | QHFWSTPYT |
| 1913. | FA86D2-L | murine | nt | GAGCTCGTCATGACCCAGTCTCCAGCCTCCCTATCTGCATCTGTGGGAGAAACTGT<br>CACCATCACATGTCGAGCAAGTGGGAATATTCACAATTATTTAGCATGGTATCAGCA<br>GAAACAGGGAAAATCTCCTCAGCTCCTGGTCTATAATGCAAAAACCTTAGCAGATGG<br>TGTGCCATCAAGGTTCAGTGGCAGTGGATCAGGAACACAATATTCTCTCAAGATCAA |

| | | | | |
|---|---|---|---|---|
| | | | | CAGCCTGCAGCCTGAAGATTTTGGGAGTTATTACTGTCAACATTTTTGGAGTACTCC GTACACGTTCGGAGGGGGGACCAAGCTTGAGATCAAA |
| 1914. | FA86D2-HL | murine | aa | EVQLLESGGGLVQPGGSLKLSCAASGFDFSTYWMSWVRQAPGKGLEWIGEINPDSSTI NYTPSLKDKFIISRDNAKNTLYLQMNKVRSEDTALYYCAGGYYRYPYYYTMDYWGQGT TVTVSSGGGGSGGGGSGGGGSELVMTQSPASLSASVGETVTITCRASGNIHNYLAWY QQKQGKSPQLLVYNAKTLADGVPSRFSGSGSGTQYSLKINSLQPEDFGSYYCQHFWS TPYTFGGGTKLEIK |
| 1915. | FA86D2-HL | murine | nt | GAGGTGCAGCTGCTCGAGTCTGGAGGTGGCCTGGTGCAGCCTGGAGGATCCCTGA AACTCTCCTGTGCAGCCTCAGGATTCGATTTTAGTACATACTGGATGAGTTGGGTCC GGCAGGCTCCAGGGAAAGGGCTAGAATGGATTGGAGAAATTAATCCAGATAGCAGT ACGATAAACTATACGCCATCTCTAAAGGATAAATTCATCATCTCCAGAGACAACGCC AAAAATACGCTGTACCTGCAAATGAACAAAGTGAGATCTGAGGACACAGCCCTTTAT TACTGTGCAGGAGGATACTATAGGTACCCATATTACTATACTATGGACTACTGGGGC CAAGGGACCACGGTCACCGTCTCCTCAGGTGGTGGTGGTTCTGGCGGCGGCGGCT CCGGTGGTGGTGGTTCTGAGCTCGTCATGACCCAGTCTCCAGCCTCCCTATCTGCA TCTGTGGGAGAAACTGTCACCATCACATGTCGAGCAAGTGGGAATATTCACAATTAT TTAGCATGGTATCAGCAGAAACAGGGAAAATCTCCTCAGCTCCTGGTCTATAATGCA AAAACCTTAGCAGATGGTGTGCCATCAAGGTTCAGTGGCAGTGGATCAGGAACACA ATATTCTCTCAAGATCAACAGCCTGCAGCCTGAAGATTTTGGGAGTTATTACTGTCA ACATTTTTGGAGTACTCCGTACACGTTCGGAGGGGGGACCAAGCTTGAGATCAAA |
| 1916. | FA86D2 HL x I2C HL | murine | aa | EVQLLESGGGLVQPGGSLKLSCAASGFDFSTYWMSWVRQAPGKGLEWIGEINPDSSTI NYTPSLKDKFIISRDNAKNTLYLQMNKVRSEDTALYYCAGGYYRYPYYYTMDYWGQGT TVTVSSGGGGSGGGGSGGGGSELVMTQSPASLSASVGETVTITCRASGNIHNYLAWY QQKQGKSPQLLVYNAKTLADGVPSRFSGSGSGTQYSLKINSLQPEDFGSYYCQHFWS TPYTFGGGTKLEIKSGGGGSEVQLVESGGGLVQPGGSLKLSCAASGFTFNKYAMNWV RQAPGKGLEWVARIRSKYNNYATYYADSVKDRFTISRDDSKNTAYLQMNNLKTEDTAV YYCVRHGNFGNSYISYWAYWGQGTLVTVSSGGGGSGGGGSGGGGSQTVVTQEPSLT VSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 1917. | FA86D2 HL x I2C HL | murine | nt | GAGGTGCAGCTGCTCGAGTCTGGAGGTGGCCTGGTGCAGCCTGGAGGATCCCTGA AACTCTCCTGTGCAGCCTCAGGATTCGATTTTAGTACATACTGGATGAGTTGGGTCC GGCAGGCTCCAGGGAAAGGGCTAGAATGGATTGGAGAAATTAATCCAGATAGCAGT ACGATAAACTATACGCCATCTCTAAAGGATAAATTCATCATCTCCAGAGACAACGCC AAAAATACGCTGTACCTGCAAATGAACAAAGTGAGATCTGAGGACACAGCCCTTTAT TACTGTGCAGGAGGATACTATAGGTACCCATATTACTATACTATGGACTACTGGGGC CAAGGGACCACGGTCACCGTCTCCTCAGGTGGTGGTGGTTCTGGCGGCGGCGGCT |

| | | | | |
|---|---|---|---|---|
| | | | | CCGGTGGTGGTGGTTCTGAGCTCGTCATGACCCAGTCTCCAGCCTCCCTATCTGCA<br>TCTGTGGGAGAAACTGTCACCATCACATGTCGAGCAAGTGGGAATATTCACAATTAT<br>TTAGCATGGTATCAGCAGAAACAGGGAAAATCTCCTCAGCTCCTGGTCTATAATGCA<br>AAAACCTTAGCAGATGGTGTGCCATCAAGGTTCAGTGGCAGTGGATCAGGAACACA<br>ATATTCTCTCAAGATCAACAGCCTGCAGCCTGAAGATTTTGGGAGTTATTACTGTCA<br>ACATTTTTGGAGTACTCCGTACACGTTCGGAGGGGGGACCAAGCTTGAGATCAAAT<br>CCGGAGGTGGTGGATCCGAGGTGCAGCTGGTCGAGTCTGGAGGAGGATTGGTGC<br>AGCCTGGAGGGTCATTGAAACTCTCATGTGCAGCCTCTGGATTCACCTTCAATAAGT<br>ACGCCATGAACTGGGTCCGCCAGGCTCCAGGAAAGGGTTTGGAATGGGTTGCTCG<br>CATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGA<br>AAACTGAGGACACTGCCGTGTACTACTGTGTGAGACATGGGAACTTCGGTAATAGC<br>TACATATCCTACTGGGCTTACTGGGGCCAAGGGACTCTGGTCACCGTCTCCTCAGG<br>TGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGG<br>CTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTAC<br>TCCTGCCAGATTCTCAGGCTCCCTGCTTGGAGGCAAGGCTGCCCTCACCCTCTCAG<br>GGGTACAGCCAGAGGATGAGGCAGAATATTACTGTGTTCTATGGTACAGCAACCGC<br>TGGGTGTTCGGTGGAGGAACCAAACTGACTGTCCTA |
| 1918. | FA86D6-H | murine | aa | EVQLLESGGGLVQPGGSLKLSCAASGFDFSTYWMSWVRQAPGKGLEWIGEINPDSSTI<br>NYTPSLKDKFIISRDNAKNTLYLQMTKVRPEDTALYYCAGGYYRYPYYYTMDYWGQGT<br>TVTVSS |
| 1919. | FA86D6-HCDR1 | murine | aa | TYWMS |
| 1920. | FA86D6-HCDR2 | murine | aa | EINPDSSTINYTPSLKD |
| 1921. | FA86D6-HCDR3 | murine | aa | GYYRYPYYYTMDY |
| 1922. | FA86D6-H | murine | nt | GAGGTGCAGCTGCTCGAGTCTGGAGGTGGCCTGGTGCAGCCTGGAGGATCCCTGA<br>AACTCTCCTGTGCAGCCTCAGGATTCGATTTTAGTACATACTGGATGAGTTGGGTCC<br>GGCAGGCTCCAGGGAAAGGGCTAGAATGGATTGGAGAAATTAATCCAGATAGCAGT<br>ACGATAAACTATACGCCATCTCTAAAGGATAAATTCATCATCTCCAGAGACAACGCC<br>AAAAATACGCTGTACCTGCAAATGACCAAAGTGAGACCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCA |
| 1923. | FA86D6-L | murine | aa | ELVMTQSPASLSASVGETVTITCRASGSIHNYLAWYQQKQGKSPQLLVYNAKTLADGVP<br>SRFSGSGSGTQYSLKINSLQPEDFGSYYCQHFWSTPYTFGGGTKLEIK |
| 1924. | FA86D6-LCDR1 | murine | aa | RASGSIHNYLA |

| | | | | |
|---|---|---|---|---|
| 1925. | FA86D6-LCDR2 | murine | aa | NAKTLAD |
| 1926. | FA86D6-LCDR3 | murine | aa | QHFWSTPYT |
| 1927. | FA86D6-L | murine | nt | GAGCTCGTCATGACCCAGTCTCCAGCCTCCCTATCTGCATCTGTGGGAGAAACTGTCACCATCACATGTCGAGCAAGTGGGAGTATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1928. | FA86D6-HL | murine | aa | EVQLLESGGGLVQPGGSLKLSCAASGFDFSTYWMSWVRQAPGKGLEWIGEINPDSSTINYTPSLKDKFIISRDNAKNTLYLQMTKVRPEDTALYYCAGGYYRYPYYYTMDYWGQGTTVTVSSGGGGSGGGGSGGGGSELVMTQSPASLSASVGETVTITCRASGSIHNYLAWYQQKQGKSPQLLVYNAKTLADGVPSRFSGSGSGTQYSLKINSLQPEDFGSYYCQHFWSTPYTFGGGTKLEIK |
| 1929. | FA86D6-HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGACCAAAGTGAGACCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTCATGACCCAGTCTCCAGCCTCCCTATCTGCATCTGTGGGAGAAACTGTCACCATCACATGTCGAGCAAGTGGGAGTATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1930. | FA86D6 HL x I2C HL | murine | aa | EVQLLESGGGLVQPGGSLKLSCAASGFDFSTYWMSWVRQAPGKGLEWIGEINPDSSTINYTPSLKDKFIISRDNAKNTLYLQMTKVRPEDTALYYCAGGYYRYPYYYTMDYWGQGTTVTVSSGGGGSGGGGSGGGGSELVMTQSPASLSASVGETVTITCRASGSIHNYLAWYQQKQGKSPQLLVYNAKTLADGVPSRFSGSGSGTQYSLKINSLQPEDFGSYYCQHFWSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1931. | FA86D6 HL x I2C | murine | nt | GAGGTGCAGCTGCTCGAGTCTGGAGGTGGCCTGGTGCAGCCTGGAGGATCCCTGA |

| | | | | |
|---|---|---|---|---|
| | HL | | | AACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGACCAAAGTGAGACCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTCATGACCCAGTCTCCAGCCTCCCTATCTGCATCTGTGGGAGAAACTGTCACCATCACATGTCGAGCAAGTGGGAGTATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1932. | FA86E5-H | murine | aa | EVQLLESGGGLVQPGGSLKLSCAASGFDFSRSWMSWVRQAPGKGLEWIGEINPDSSTINYTPSLKDKFIISRDNAKNTLYLQMSKVRSEDTALYYCAGGYYRYPYYYTMDYWGQGTTVTVSS |
| 1933. | FA86E5-HCDR1 | murine | aa | RSWMS |
| 1934. | FA86E5-HCDR2 | murine | aa | EINPDSSTINYTPSLKD |
| 1935. | FA86E5-HCDR3 | murine | aa | GYYRYPYYYTMDY |
| 1936. | FA86E5-H | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTAGATCCTGGATGAGTTGGGTCCGGCAGGCTCCAGGGAAAGGGCTTGAATGGATTGGAGAAATTAATCCAGATAGCAGTACGATAAACTATACGCCATCTCTAAAGGATAAATTCATCATCTCCAGAGACAACGCC |

| | | | | |
|---|---|---|---|---|
| | | | | AAAAATACGCTGTACCTGCAAATGAGCAAAGTGAGATCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCA |
| 1937. | FA86E5-L | murine | aa | ELQMTQSPASLSASVGETVTITCRASENIYSYLAWYQQKQGKSPQLLVYNAKTLAEGVP<br>SRFSGSGSRTQYSLKINSLQPEDFGSYYCQHFWSTPYTFGGGTKLEIK |
| 1938. | FA86E5-LCDR1 | murine | aa | RASENIYSYLA |
| 1939. | FA86E5-LCDR2 | murine | aa | NAKTLAE |
| 1940. | FA86E5-LCDR3 | murine | aa | QHFWSTPYT |
| 1941. | FA86E5-L | murine | nt | GAGCTCCAGATGACCCAGTCTCCAGCCTCCCTATCTGCATCTGTGGGAGAAACTGT<br>CACCATCACATGTCGAGCAAGTGAGAATATTTACAGTTATTTAGCATGGTATCAGCA<br>GAAACAGGGAAAATCTCCTCAGCTCCTGGTCTATAATGCAAAAACCTTAGCAGAAG<br>GTGTGCCATCAAGGTTCAGTGGCAGTGGATCAAGAACACAATATTCTCTCAAGATCA<br>ACAGCCTGCAGCCTGAAGATTTTGGGAGTTATTACTGTCAACATTTTTGGAGTACTC<br>CGTACACGTTCGGAGGGGGGACCAAGCTTGAGATCAAA |
| 1942. | FA86E5-HL | murine | aa | EVQLLESGGGLVQPGGSLKLSCAASGFDFSRSWMSWVRQAPGKGLEWIGEINPDSSTI<br>NYTPSLKDKFIISRDNAKNTLYLQMSKVRSEDTALYYCAGGYYRYPYYYTMDYWGQGT<br>TVTVSSGGGGSGGGGSGGGGSELQMTQSPASLSASVGETVTITCRASENIYSYLAWY<br>QQKQGKSPQLLVYNAKTLAEGVPSRFSGSGSRTQYSLKINSLQPEDFGSYYCQHFWST<br>PYTFGGGTKLEIK |
| 1943. | FA86E5-HL | murine | nt | GAGGTGCAGCTGCTCGAGTCTGGAGGTGGCCTGGTGCAGCCTGGAGGATCCCTGA<br>AACTCTCCTGTGCAGCCTCAGGATTCGATTTTAGTAGATCCTGGATGAGTTGGGTCC<br>GGCAGGCTCCAGGGAAAGGGCTTGAATGGATTGGAGAAATTAATCCAGATAGCAGT<br>ACGATAAACTATACGCCATCTCTAAAGGATAAATTCATCATCTCCAGAGACAACGCC<br>AAAAATACGCTGTACCTGCAAATGAGCAAAGTGAGATCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCAGGTGGTGGTGGTTCTGGCGGCGGCGGCT<br>CCGGTGGTGGTGGTTCTGAGCTCCAGATGACCCAGTCTCCAGCCTCCCTATCTGCA<br>TCTGTGGGAGAAACTGTCACCATCACATGTCGAGCAAGTGAGAATATTTACAGTTAT<br>TTAGCATGGTATCAGCAGAAACAGGGAAAATCTCCTCAGCTCCTGGTCTATAATGCA<br>AAAACCTTAGCAGAAGGTGTGCCATCAAGGTTCAGTGGCAGTGGATCAAGAACACA<br>ATATTCTCTCAAGATCAACAGCCTGCAGCCTGAAGATTTTGGGAGTTATTACTGTCA<br>ACATTTTTGGAGTACTCCGTACACGTTCGGAGGGGGGACCAAGCTTGAGATCAAA |
| 1944. | FA86E5 HL x I2C HL | murine | aa | EVQLLESGGGLVQPGGSLKLSCAASGFDFSRSWMSWVRQAPGKGLEWIGEINPDSSTI<br>NYTPSLKDKFIISRDNAKNTLYLQMSKVRSEDTALYYCAGGYYRYPYYYTMDYWGQGT<br>TVTVSSGGGGSGGGGSGGGGSELQMTQSPASLSASVGETVTITCRASENIYSYLAWY<br>QQKQGKSPQLLVYNAKTLAEGVPSRFSGSGSRTQYSLKINSLQPEDFGSYYCQHFWST |

| | | | | |
|---|---|---|---|---|
| | | | | PYTFGGGTKLEIKSGGGGSEVQLVESGGGLVQPGGSLKLSCAASGFTFNKYAMNWVR<br>QAPGKGLEWVARIRSKYNNYATYYADSVKDRFTISRDDSKNTAYLQMNNLKTEDTAVY<br>YCVRHGNFGNSYISYWAYWGQGTLVTVSSGGGGSGGGGSGGGGSQTVVTQEPSLTV<br>SPGGTVTLTCGSSTGAVTSGNYPNWVQQKPGQAPRGLIGGTKFLAPGTPARFSGSLL<br>GGKAALTLSGVQPEDEAEYYCVLWYSNRWVFGGGTKLTVL |
| 1945. | FA86E5 HL x I2C HL | murine | nt | GAGGTGCAGCTGCTCGAGTCTGGAGGTGGCCTGGTGCAGCCTGGAGGATCCCTGA<br>AACTCTCCTGTGCAGCCTCAGGATTCGATTTTAGTAGATCCTGGATGAGTTGGGTCC<br>GGCAGGCTCCAGGGAAAGGGCTTGAATGGATTGGAGAAATTAATCCAGATAGCAGT<br>ACGATAAACTATACGCCATCTCTAAAGGATAAATTCATCATCTCCAGAGACAACGCC<br>AAAAATACGCTGTACCTGCAAATGAGCAAAGTGAGATCTGAGGACACAGCCCTTTAT<br>TACTGTGCAGGAGGATACTATAGGTACCCATATTACTATACTATGGACTACTGGGGC<br>CAAGGGACCACGGTCACCGTCTCCTCAGGTGGTGGTGGTTCTGGCGGCGGCGGCT<br>CCGGTGGTGGTGGTTCTGAGCTCCAGATGACCCAGTCTCCAGCCTCCCTATCTGCA<br>TCTGTGGGAGAAACTGTCACCATCACATGTCGAGCAAGTGAGAATATTTACAGTTAT<br>TTAGCATGGTATCAGCAGAAACAGGGAAAATCTCCTCAGCTCCTGGTCTATAATGCA<br>AAAACCTTAGCAGAAGGTGTGCCATCAAGGTTCAGTGGCAGTGGATCAAGAACACA<br>ATATTCTCTCAAGATCAACAGCCTGCAGCCTGAAGATTTTGGGAGTTATTACTGTCA<br>ACATTTTTGGAGTACTCCGTACACGTTCGGAGGGGGGACCAAGCTTGAGATCAAAT<br>CCGGAGGTGGTGGATCCGAGGTGCAGCTGGTCGAGTCTGGAGGAGGATTGGTGC<br>AGCCTGGAGGGTCATTGAAACTCTCATGTGCAGCCTCTGGATTCACCTTCAATAAGT<br>ACGCCATGAACTGGGTCCGCCAGGCTCCAGGAAAGGGTTTGGAATGGGTTGCTCG<br>CATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGA<br>AAACTGAGGACACTGCCGTGTACTACTGTGTGAGACATGGGAACTTCGGTAATAGC<br>TACATATCCTACTGGGCTTACTGGGGCCAAGGGACTCTGGTCACCGTCTCCTCAGG<br>TGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGG<br>CTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTAC<br>TCCTGCCAGATTCTCAGGCTCCCTGCTTGGAGGCAAGGCTGCCCTCACCCTCTCAG<br>GGGTACAGCCAGAGGATGAGGCAGAATATTACTGTGTTCTATGGTACAGCAACCGC<br>TGGGTGTTCGGTGGAGGAACCAAACTGACTGTCCTA |
| 1946. | FA86F10-H | murine | aa | EVQLLESGGGLVQPGGSLKLSCAASGFDFSTYWMSWVRQAPGKGLEWIGEINPDSSTI<br>NYTPSLKDKFIISRDNAKNTLYLQMNKVRSDDTALYYCAGGYYRYPYYYTMDYWGQGT<br>TVTVSS |
| 1947. | FA86F10-HCDR1 | murine | aa | TYWMS |

| | | | | |
|---|---|---|---|---|
| 1948. | FA86F10-HCDR2 | murine | aa | EINPDSSTINYTPSLKD |
| 1949. | FA86F10-HCDR3 | murine | aa | GYYRYPYYYTMDY |
| 1950. | FA86F10-H | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAACAAAGTGAGATCTGACGACACAGCCCTTTATTACTGTGCAGGAGGATACTATAGGTACCCATATTACTATACTATGGACTACTGGGGCCAAGGGACCACGGTCACCGTCTCCTCA |
| 1951. | FA86F10-L | murine | aa | ELVMTQSPSSLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1952. | FA86F10-LCDR1 | murine | aa | RASGNIHNYLA |
| 1953. | FA86F10-LCDR2 | murine | aa | NAKTLAD |
| 1954. | FA86F10-LCDR3 | murine | aa | QHFWSTPYT |
| 1955. | FA86F10-L | murine | nt | GAGCTCGTGATGACACAGTCTCCATCCTCCTTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1956. | FA86F10-HL | murine | aa | EVQLLESGGGLVQPGGSLKLSCAASGFDFSTYWMSWVRQAPGKGLEWIGEINPDSSTINYTPSLKDKFIISRDNAKNTLYLQMNKVRSDDTALYYCAGGYYRYPYYYTMDYWGQGTTVTVSSGGGGSGGGGSGGGGSELVMTQSPSSLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1957. | FA86F10-HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAACAAAGTGAGATCTGACGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCGTGATGACACAGTCTCCATCCTCCTTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACA |

| | | | | |
|---|---|---|---|---|
| | | | | ATATTCTCTCAAGATCAACAGCCTGCAGCCTGAAGATTTTGGGAGTTATTACTGTCA ACATTTTTGGAGTACTCCGTACACGTTCGGAGGGGGGACCAAGCTTGAGATCAAA |
| 1958. | FA86F10 HL x I2C HL | murine | aa | EVQLLESGGGLVQPGGSLKLSCAASGFDFSTYWMSWVRQAPGKGLEWIGEINPDSSTI NYTPSLKDKFIISRDNAKNTLYLQMNKVRSDDTALYYCAGGYYRYPYYYTMDYWGQGT TVTVSSGGGGSGGGGSGGGGSELVMTQSPSSLSASVGETVTITCRASGNIHNYLAWY QQKQGKSPQLLVYNAKTLADGVPSRFSGSGSGTQYSLKINSLQPEDFGSYYCQHFWS TPYTFGGGTKLEIKSGGGGSEVQLVESGGGLVQPGGSLKLSCAASGFTFNKYAMNWV RQAPGKGLEWVARIRSKYNNYATYYADSVKDRFTISRDDSKNTAYLQMNNLKTEDTAV YYCVRHGNFGNSYISYWAYWGQGTLVTVSSGGGGSGGGGSGGGGSQTVVTQEPSLT VSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 1959. | FA86F10 HL x I2C HL | murine | nt | GAGGTGCAGCTGCTCGAGTCTGGAGGTGGCCTGGTGCAGCCTGGAGGATCCCTGA AACTCTCCTGTGCAGCCTCAGGATTCGATTTTAGTACATACTGGATGAGTTGGGTCC GGCAGGCTCCAGGGAAAGGGCTAGAATGGATTGGAGAAATTAATCCAGATAGCAGT ACGATAAACTATACGCCATCTCTAAAGGATAAATTCATCATCTCCAGAGACAACGCC AAAAATACGCTGTACCTGCAAATGAACAAAGTGAGATCTGACGACACAGCCCTTTAT TACTGTGCAGGAGGATACTATAGGTACCCATATTACTATACTATGGACTACTGGGGC CAAGGGACCACGGTCACCGTCTCCTCAGGTGGTGGTGGTTCTGGCGGCGGCGGCT CCGGTGGTGGTGGTTCTGAGCTCGTGATGACACAGTCTCCATCCTCCTTATCTGCA TCTGTGGGAGAAACTGTCACCATCACATGTCGAGCAAGTGGGAATATTCACAATTAT TTAGCATGGTATCAGCAGAAACAGGGAAAATCTCCTCAGCTCCTGGTCTATAATGCA AAAACCTTAGCAGATGGTGTGCCATCAAGGTTCAGTGGCAGTGGATCAGGAACACA ATATTCTCTCAAGATCAACAGCCTGCAGCCTGAAGATTTTGGGAGTTATTACTGTCA ACATTTTTGGAGTACTCCGTACACGTTCGGAGGGGGGACCAAGCTTGAGATCAAAT CCGGAGGTGGTGGATCCGAGGTGCAGCTGGTCGAGTCTGGAGGAGGATTGGTGC AGCCTGGAGGGTCATTGAAACTCTCATGTGCAGCCTCTGGATTCACCTTCAATAAGT ACGCCATGAACTGGGTCCGCCAGGCTCCAGGAAAGGGTTTGGAATGGGTTGCTCG CATAAGAAGTAAATATAATAATTATGCAACATATTATGCCGATTCAGTGAAAGACAGG TTCACCATCTCCAGAGATGATTCAAAAAACACTGCCTATCTACAAATGAACAACTTGA AAACTGAGGACACTGCCGTGTACTACTGTGTGAGACATGGGAACTTCGGTAATAGC TACATATCCTACTGGGCTTACTGGGGCCAAGGGACTCTGGTCACCGTCTCCTCAGG TGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGG CTCCTCGACTGGGGCTGTTACATCTGGCAACTACCCAAACTGGGTCCAACAAAAAC CAGGTCAGGCACCCCGTGGTCTAATAGGTGGGACTAAGTTCCTCGCCCCCGGTAC TCCTGCCAGATTCTCAGGCTCCCTGCTTGGAGGCAAGGCTGCCCTCACCCTCTCAG |

| | | | | |
|---|---|---|---|---|
| | | | | GGGTACAGCCAGAGGATGAGGCAGAATATTACTGTGTTCTATGGTACAGCAACCGCTGGGTGTTCGGTGGAGGAACCAAACTGACTGTCCTA |
| 1960. | FA86F12-H | murine | aa | EVQLLESGGGLVQPGGSLNLSCAASGFDFSTYWMSWVRQAPGKGLEWIGEINPDSSTINYTPSLKDKFIISRDNAKNTLYLQMNKVRSDDTALYYCAGGYYRYPYYYTMDYWGQGTTVTVSS |
| 1961. | FA86F12-HCDR1 | murine | aa | TYWMS |
| 1962. | FA86F12-HCDR2 | murine | aa | EINPDSSTINYTPSLKD |
| 1963. | FA86F12-HCDR3 | murine | aa | GYYRYPYYYTMDY |
| 1964. | FA86F12-H | murine | nt | GAGGTGCAGCTGCTCGAGTCTGGAGGTGGCCTGGTGCAGCCTGGAGGATCCCTGAATCTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAACAAAGTGAGATCTGACGACACAGCCCTTTATTACTGTGCAGGAGGATACTATAGGTACCCATATTACTATACTATGGACTACTGGGGCCAAGGGACCACGGTCACCGTCTCCTCA |
| 1965. | FA86F12-L | murine | aa | ELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1966. | FA86F12-LCDR1 | murine | aa | RASGNIHNYLA |
| 1967. | FA86F12-LCDR2 | murine | aa | NAKTLAD |
| 1968. | FA86F12-LCDR3 | murine | aa | QHFWSTPYT |
| 1969. | FA86F12-L | murine | nt | GAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1970. | FA86F12-HL | murine | aa | EVQLLESGGGLVQPGGSLNLSCAASGFDFSTYWMSWVRQAPGKGLEWIGEINPDSSTINYTPSLKDKFIISRDNAKNTLYLQMNKVRSDDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1971. | FA86F12-HL | murine | nt | GAGGTGCAGCTGCTCGAGTCTGGAGGTGGCCTGGTGCAGCCTGGAGGATCCCTGAATCTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAACAAAGTGAGATCTGACGACACAGCCCTTTAT |

| | | | | |
|---|---|---|---|---|
| | | | | TACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1972. | FA86F12 HL x I2C HL | murine | aa | EVQLLESGGGLVQPGGSLNLSCAASGFDFSTYWMSWVRQAPGKGLEWIGEINPDSSTINYTPSLKDKFIISRDNAKNTLYLQMNKVRSDDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1973. | FA86F12 HL x I2C HL | murine | nt | GAGGTGCAGCTGCTCGAGTCTGGAGGTGGCCTGGTGCAGCCTGGAGGATCCCTGAATCTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGATAAACTATACGCCATCTCTAAAGGATAAATTCATCATCTCCAGAGACAACGCCAAAAATACGCTGTACCTGCAAATGAACAAAGTGAGATCTGACGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAATCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGC |

| | | | | |
|---|---|---|---|---|
| | | | | TACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 1974. | FA86F2-H | murine | aa | EVQLLESGGGLVQPGGSLKLSCAASGFDFSTYWMSWVRQAPGKGLEWIGEINPDSSTVNYTPSLKDKFIISRDNAKNTLYLQMTKVRSEDTALYYCAGGYYRYPYYYTMDYWGQGTTVTVSS |
| 1975. | FA86F2-HCDR1 | murine | aa | TYWMS |
| 1976. | FA86F2-HCDR2 | murine | aa | EINPDSSTVNYTPSLKD |
| 1977. | FA86F2-HCDR3 | murine | aa | GYYRYPYYYTMDY |
| 1978. | FA86F2-H | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGGTAAACTATACGCCATCTCTAAAGGATAAATTCATCATCTCCAGAGACAACGCCAAAAATACGCTGTACCTGCAAATGACCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCA |
| 1979. | FA86F2-L | murine | aa | ELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIK |
| 1980. | FA86F2-LCDR1 | murine | aa | RASGNIHNYLA |
| 1981. | FA86F2-LCDR2 | murine | aa | NAKTLAD |
| 1982. | FA86F2-LCDR3 | murine | aa | QHFWSTPYT |
| 1983. | FA86F2-L | murine | nt | GAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1984. | FA86F2-HL | murine | aa | EVQLLESGGGLVQPGGSLKLSCAASGFDFSTYWMSWVRQAPGKGLEWIGEINPDSSTVNYTPSLKDKFIISRDNAKNTLYLQMTKVRSEDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFW |

| | | | | |
|---|---|---|---|---|
| | | | | STPYTFGGGTKLEIK |
| 1985. | FA86F2-HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGGTAAACTATACGCCATCTCTAAAGGATAAATTCATCATCTCCAGAGACAACGCCAAAAATACGCTGTACCTGCAAATGACCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAA |
| 1986. | FA86F2 HL x I2C HL | murine | aa | EVQLLESGGGLVQPGGSLKLSCAASGFDFSTYWMSWVRQAPGKGLEWIGEINPDSSTVNYTPSLKDKFIISRDNAKNTLYLQMTKVRSEDTALYYCAGGYYRYPYYYTMDYWGQGTTVTVSSGGGGSGGGGSGGGGSELQMTQSPASLSASVGETVTITCRASGNIHNYLAWYQQKQGKSPQLLVYNAKTLADGVPSRFSGSGSGTQYSLKINSLQPEDFGSYYCQHFWSTPYTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 1987. | FA86F2 HL x I2C HL | murine | nt | GAGGTGCAGCTGCTCGAGTCTGGAGGTGGCCTGGTGCAGCCTGGAGGATCCCTGAAACTCTCCTGTGCAGCCTCAGGATTCGATTTTAGTACATACTGGATGAGTTGGGTCCGGCAGGCTCCAGGGAAAGGGCTAGAATGGATTGGAGAAATTAATCCAGATAGCAGTACGGTAAACTATACGCCATCTCTAAAGGATAAATTCATCATCTCCAGAGACAACGCCAAAAATACGCTGTACCTGCAAATGACCAAAGTGAGATCTGAGGACACAGCCCTTTATTACTGTGCAGGAGGATACTATAGGTACCCATATTACTATACTATGGACTACTGGGGCCAAGGGACCACGGTCACCGTCTCCTCAGGTGGTGGTGGTTCTGGCGGCGGCGGCTCCGGTGGTGGTGGTTCTGAGCTCCAGATGACCCAGTCTCCAGCCTCCCTATCTGCATCTGTGGGAGAAACTGTCACCATCACATGTCGAGCAAGTGGGAATATTCACAATTATTTAGCATGGTATCAGCAGAAACAGGGAAAATCTCCTCAGCTCCTGGTCTATAATGCAAAAACCTTAGCAGATGGTGTGCCATCAAGGTTCAGTGGCAGTGGATCAGGAACACAATATTCTCTCAAGATCAACAGCCTGCAGCCTGAAGATTTTGGGAGTTATTACTGTCAACATTTTTGGAGTACTCCGTACACGTTCGGAGGGGGGACCAAGCTTGAGATCAAAT |

| | | | | |
|---|---|---|---|---|
| | | | | CCGGAGGTGGTGGATCCGAGGTGCAGCTGGTCGAGTCTGGAGGAGGATTGGTGC<br>AGCCTGGAGGGTCATTGAAACTCTCATGTGCAGCCTCTGGATTCACCTTCAATAAGT<br>ACGCCATGAACTGGGTCCGCCAGGCTCCAGGAAAGGGTTTGGAATGGGTTGCTCG<br>CATAAGAAGTAAATATAATAATTATGCAACATATTATGCCGATTCAGTGAAAGACAGG<br>TTCACCATCTCCAGAGATGATTCAAAAAACACTGCCTATCTACAAATGAACAACTTGA<br>AAACTGAGGACACTGCCGTGTACTACTGTGTGAGACATGGGAACTTCGGTAATAGC<br>TACATATCCTACTGGGCTTACTGGGGCCAAGGGACTCTGGTCACCGTCTCCTCAGG<br>TGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGG<br>CTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTAC<br>TCCTGCCAGATTCTCAGGCTCCCTGCTTGGAGGCAAGGCTGCCCTCACCCTCTCAG<br>GGGTACAGCCAGAGGATGAGGCAGAATATTACTGTGTTCTATGGTACAGCAACCGC<br>TGGGTGTTCGGTGGAGGAACCAAACTGACTGTCCTA |
| 1988. | human IGF-R1 construct | artificial | nt | ATGAAGTCTGGCTCCGGCGGAGGGTCCCCGACCTCGCTGTGGGGCTCCTGTTTCTCTCCGCCGC<br>GCTCTCGCTCTGGCCGACGAGTGGAGAAATCTGCGGGCCAGGCATCGACATCCGCAACGACTATC<br>AGCAGCTGAAGCGCCTGGAGAACTGCACGGTGATCGAGGGCTACCTCCACATCCTGCTCATCTCC<br>AAGGCCGAGGACTACCGCAGCTACCGCTTCCCCAAGCTCACGGTCATTACCGAGTACTTGCTGCT<br>GTTCCGAGTGGCTGGCCTCGAGAGCCTCGGAGACCTCTTCCCCAACCTCACGGTCATCCGCGGCT<br>GGAAACTCTTCTACAACTACGCCCTGGTCATCTTCGAGATGACCAATCTCAAGGATATTGGGCTT<br>TACAACCTGAGGAACATTACTCGGGGGGCCATCAGGATTGAGAAAAATGCTGACCTCTGTTACCT<br>CTCCACTGTGGACTGGTCCCTGATCCTGGATGCGGTGTCCAATAACTACATTGTGGGGAATAAGC<br>CCCCAAAGGAATGTGGGGACCTGTGTCCAGGGACCATGGAGGAGAAGCCGATGTGTGAGAAGACC<br>ACCATCAACAATGAGTACAACTACCGCTGCTGGACCACAAACCGCTGCCAGAAAATGTGCCCAAG<br>CACGTGTGGGAAGCGGGCGTGCACCGAGAACAATGAGTGCTGCCACCCCGAGTGCCTGGGCAGCT<br>GCAGCGCGCCTGACAACGACACGGCCTGTGTAGCTTGCCGCCACTACTACTATGCCGGTGTCTGT<br>GTGCCTGCCTGCCCGCCCAACACCTACAGGTTTGAGGGCTGGCGCTGTGTGGACCGTGACTTCTG<br>CGCCAACATCCTCAGCGCCGAGAGCAGCGACTCCGAGGGGTTTGTGATCCACGACGGCGAGTGCA<br>TGCAGGAGTGCCCCTCGGGCTTCATCCGCAACGGCAGCCAGAGCATGTACTGCATCCCTTGTGAA<br>GGTCCTTGCCCGAAGGTCTGTGAGGAAGAAAAGAAAACAAAGACCATTGATTCTGTTACTTCTGC<br>TCAGATGCTCCAAGGATGCACCATCTTCAAGGGCAATTTGCTCATTAACATCCGACGGGGGAATA<br>ACATTGCTTCAGAGCTGGAGAACTTCATGGGGCTCATCGAGGTGGTGACGGGCTACGTGAAGATC<br>CGCCATTCTCATGCCTTGGTCTCCTTGTCCTTCCTAAAAAACCTTCGCCTCATCCTAGGAGAGGA<br>GCAGCTAGAAGGGAATTACTCCTTCTACGTCCTCGACAACCAGAACTTGCAGCAACTGTGGGACT<br>GGGACCACCGCAACCTGACCATCAAAGCAGGGAAAATGTACTTTGCTTTCAATCCCAAATTATGT<br>GTTTCCGAAATTTACCGCATGGAGGAAGTGACGGGGACTAAAGGGCGCCAAAGCAAAGGGGACAT<br>AAACACCAGGAACAACGGGGAGAGAGCCTCCTGTGAAAGTGACGTCCTGCATTTCACCTCCACCA |

| | | | | |
|---|---|---|---|---|
| CCACGTCGAAGAATCGCATCATCATAACCTGGCACCGGTACCGGCCCCCTGACTACAGGGATCTC<br>ATCAGCTTCACCGTTTACTACAAGGAAGCACCCTTTAAGAATGTCACAGAGTATGATGGGCAGGA<br>TGCCTGCGGCTCCAACAGCTGGAACATGGTGGACGTGGACCTCCCGCCCAACAAGGACGTGGAGC<br>CCGGCATCTTACTACATGGGCTGAAGCCCTGGACTCAGTACGCCGTTTACGTCAAGGCTGTGACC<br>CTCACCATGGTGGAGAACGACCATATCCGTGGGGCCAAGAGTGAGATCTTGTACATTCGCACCAA<br>TGCTTCAGTTCCTTCCATTCCCTTGGACGTTCTTTCAGCATCGAACTCCTCTTCTCAGTTAATCG<br>TGAAGTGGAACCCTCCCTCTCTGCCCAACGGCAACCTGAGTTACTACATTGTGCGCTGGCAGCGG<br>CAGCCTCAGGACGGCTACCTTTACCGGCACAATTACTGCTCCAAAGACAAAATCCCCATCAGGAA<br>GTATGCCGACGGCACCATCGACATTGAGGAGGTCACAGAGAACCCCAAGACTGAGGTGTGTGGTG<br>GGGAGAAAGGGCCTTGCTGCGCCTGCCCCAAAACTGAAGCCGAGAAGCAGGCCGAGAAGGAGGAG<br>GCTGAATACCGCAAAGTCTTTGAGAATTTCCTGCACAACTCCATCTTCGTGCCCAGACCTGAAAG<br>GAAGCGGAGAGATGTCATGCAAGTGGCCAACACCACCATGTCCAGCCGAAGCAGGAACACCACGG<br>CCGCAGACACCTACAACATCACCGACCCGGAAGAGCTGGAGACAGAGTACCCTTTCTTTGAGAGC<br>AGAGTGGATAACAAGGAGAGAACTGTCATTTCTAACCTTCGGCCTTTCACATTGTACCGCATCGA<br>TATCCACAGCTGCAACCACGAGGCTGAGAAGCTGGGCTGCAGCGCCTCCAACTTCGTCTTTGCAA<br>GGACTATGCCCGCAGAAGGAGCAGATGACATTCCTGGGCCAGTGACCTGGGAGCCAAGGCCTGAA<br>AACTCCATCTTTTTAAAGTGGCCGGAACCTGAGAATCCCAATGGATTGATTCTAATGTATGAAAT<br>AAAATACGGATCACAAGTTGAGGATCAGCGAGAATGTGTGTCCAGACAGGAATACAGGAAGTATG<br>GAGGGGCCAAGCTAAACCGGCTAAACCCGGGGAACTACACAGCCCGGATTCAGGCCACATCTCTC<br>TCTGGGAATGGGTCGTGGACAGATCCTGTGTTCTTCTATGTCCAGGCCAAAACAGGATATGAAAA<br>CTTCATCCATCTGATCATCGCTCTGCCCGTCGCTGTCCTGTTGATCGTGGGAGGGTTGGTGATTA<br>TGCTGTACGTCTTCCATAGAAAGAGAAATAACAGCAGGCTGGGGAATGGAGTGCTGTATGCCTCT<br>GTGAACCCGGAGTACTTCAGCGCTGCTGATGTGTACGTTCCTGATGAGTGGGAGGTGGCTCGGGA<br>GAAGATCACCATGAGCCGGGAACTTGGGCAGGGGTCGTTTGGGATGGTCTATGAAGGAGTTGCCA<br>AGGGTGTGGTGAAAGATGAACCTGAAACCAGAGTGGCCATTAAAACAGTGAACGAGGCCGCAAGC<br>ATGCGTGAGAGGATTGAGTTTCTCAACGAAGCTTCTGTGATGAAGGAGTTCAATTGTCACCATGT<br>GGTGCGATTGCTGGGTGTGGTGTCCCAAGGCCAGCCAACACTGGTCATCATGGAACTGATGACAC<br>GGGGCGATCTCAAAAGTTATCTCCGGTCTCTGAGGCCAGAAATGGAGAATAATCCAGTCCTAGCA<br>CCTCCAAGCCTGAGCAAGATGATTCAGATGGCCGGAGAGATTGCAGACGGCATGGCATACCTCAA<br>CGCCAATAAGTTCGTCCACAGAGACCTTGCTGCCCGGAATTGCATGGTAGCCGAAGATTTCACAG<br>TCAAAATCGGAGATTTTGGTATGACGCGAGATATCTATGAGACAGACTATTACCGGAAAGGAGGG<br>AAAGGGCTGCTGCCCGTGCGCTGGATGTCTCCTGAGTCCCTCAAGGATGGAGTCTTCACCACTTA<br>CTCGGACGTCTGGTCCTTCGGGGTCGTCCTCTGGGAGATCGCCACACTGGCCGAGCAGCCCTACC<br>AGGGCTTGTCCAACGAGCAAGTCCTTCGCTTCGTCATGGAGGGCGGCCTTCTGGACAAGCCAGAC<br>AACTGTCCTGACATGCTGTTTGAACTGATGCGCATGTGCTGGCAGTATAACCCCAAGATGAGGCC<br>TTCCTTCCTGGAGATCATCAGCAGCATCAAAGAGGAGATGGAGCCTGGCTTCCGGGAGGTCTCCT<br>TCTACTACAGCGAGGAGAACAAGCTGCCCGAGCCGGAGGAGCTGGACCTGGAGCCAGAGAACATG | | | | |

| | | | | |
|---|---|---|---|---|
| | | | | GAGAGCGTCCCCCTGGACCCCTCGGCCTCCTCGTCCTCCCTGCCACTGCCCGACAGACACTCAGG<br>ACACAAGGCCGAGAACGGCCCCGGCCCTGGGGTGCTGGTCCTCCGCGCCAGCTTCGACGAGAGAC<br>AGCCTTACGCCCACATGAACGGGGGCCGCAAGAACGAGCGGGCCTTGCCGCTGCCCCAGTCTTCG<br>ACCTGCTGA |
| 1989. | human IGF-R1 construct | artificial | aa | MKSGSGGGSPTSLWGLLFLSAALSLWPTSGEICGPGIDIRNDYQQLKRLENCTVIEGYLHILLIS<br>KAEDYRSYRFPKLTVITEYLLLFRVAGLESLGDLFPNLTVIRGWKLFYNYALVIFEMTNLKDIGL<br>YNLRNITRGAIRIEKNADLCYLSTVDWSLILDAVSNNYIVGNKPPKECGDLCPGTMEEKPMCEKT<br>TINNEYNYRCWTTNRCQKMCPSTCGKRACTENNECCHPECLGSCSAPDNDTACVACRHYYYAGVC<br>VPACPPNTYRFEGWRCVDRDFCANILSAESSDSEGFVIHDGECMQECPSGFIRNGSQSMYCIPCE<br>GPCPKVCEEEKKTKTIDSVTSAQMLQGCTIFKGNLLINIRRGNNIASELENFMGLIEVVTGYVKI<br>RHSHALVSLSFLKNLRLILGEEQLEGNYSFYVLDNQNLQQLWDWDHRNLTIKAGKMYFAFNPKLC<br>VSEIYRMEEVTGTKGRQSKGDINTRNNGERASCESDVLHFTSTTTSKNRIIITWHRYRPPDYRDL<br>ISFTVYYKEAPFKNVTEYDGQDACGSNSWNMVDVDLPPNKDVEPGILLHGLKPWTQYAVYVKAVT<br>LTMVENDHIRGAKSEILYIRTNASVPSIPLDVLSASNSSSQLIVKWNPPSLPNGNLSYYIVRWQR<br>QPQDGYLYRHNYCSKDKIPIRKYADGTIDIEEVTENPKTEVCGGEKGPCCACPKTEAEKQAEKEE<br>AEYRKVFENFLHNSIFVPRPERKRRDVMQVANTTMSSRSRNTTAADTYNITDPEELETEYPFFES<br>RVDNKERTVISNLRPFTLYRIDIHSCNHEAEKLGCSASNFVFARTMPAEGADDIPGPVTWEPRPE<br>NSIFLKWPEPENPNGLILMYEIKYGSQVEDQRECVSRQEYRKYGGAKLNRLNPGNYTARIQATSL<br>SGNGSWTDPVFFYVQAKTGYENFIHLIIALPVAVLLIVGGLVIMLYVFHRKRNNSRLGNGVLYAS<br>VNPEYFSAADVYVPDEWEVAREKITMSRELGQGSFGMVYEGVAKGVVKDEPETRVAIKTVNEAAS<br>MRERIEFLNEASVMKEFNCHHVVRLLGVVSQGQPTLVIMELMTRGDLKSYLRSLRPEMENNPVLA<br>PPSLSKMIQMAGEIADGMAYLNANKFVHRDLAARNCMVAEDFTVKIGDFGMTRDIYETDYYRKGG<br>KGLLPVRWMSPESLKDGVFTTYSDVWSFGVVLWEIATLAEQPYQGLSNEQVLRFVMEGGLLDKPD<br>NCPDMLFELMRMCWQYNPKMRPSFLEIISSIKEEMEPGFREVSFYYSEENKLPEPEELDLEPENM<br>ESVPLDPSASSSSLPLPDRHSGHKAENGPGPGVLVLRASFDERQPYAHMNGGRKNERALPLPQSS<br>TC |
| 1990. | soluble fusion protein of h IGF-R1 and m IgG1 Fc | artificial | nt | ATGAAGTCTGGCTCCGGCGGAGGGTCCCCGACCTCGCTGTGGGGGCTCCTGTTTCTCTCCGCCGC<br>GCTCTCGCTCTGGCCGACGAGTGGAGAAATCTGCGGGCCAGGCATCGACATCCGCAACGACTATC<br>AGCAGCTGAAGCGCCTGGAGAACTGCACGGTGATCGAGGGCTACCTCCACATCCTGCTCATCTCC<br>AAGGCCGAGGACTACCGCAGCTACCGCTTCCCCAAGCTCACGGTCATTACCGAGTACTTGCTGCT<br>GTTCCGAGTGGCTGGCCTCGAGAGCCTCGGAGACCTCTTCCCCAACCTCACGGTCATCCGCGGCT<br>GGAAACTCTTCTACAACTACGCCCTGGTCATCTTCGAGATGACCAATCTCAAGGATATTGGGCTT<br>TACAACCTGAGGAACATTACTCGGGGGGCCATCAGGATTGAGAAAAATGCTGACCTCTGTTACCT<br>CTCCACTGTGGACTGGTCCCTGATCCTGGATGCGGTGTCCAATAACTACATTGTGGGGAATAAGC<br>CCCCAAAGGAATGTGGGGACCTGTGTCCAGGGACCATGGAGGAGAAGCCGATGTGTGAGAAGACC<br>ACCATCAACAATGAGTACAACTACCGCTGCTGGACCACAAACCGCTGCCAGAAAATGTGCCCAAG<br>CACGTGTGGGAAGCGGGCGTGCACCGAGAACAATGAGTGCTGCCACCCCGAGTGCCTGGGCAGCT |

GCAGCGCGCCTGACAACGACACGGCCTGTGTAGCTTGCCGCCACTACTACTATGCCGGTGTCTGT
GTGCCTGCCTGCCCGCCCAACACCTACAGGTTTGAGGGCTGGCGCTGTGTGGACCGTGACTTCTG
CGCCAACATCCTCAGCGCCGAGAGCAGCGACTCCGAGGGGTTTGTGATCCACGACGGCGAGTGCA
TGCAGGAGTGCCCCTCGGGCTTCATCCGCAACGGCAGCCAGAGCATGTACTGCATCCCTTGTGAA
GGTCCTTGCCCGAAGGTCTGTGAGGAAGAAAAGAAAACAAAGACCATTGATTCTGTTACTTCTGC
TCAGATGCTCCAAGGATGCACCATCTTCAAGGGCAATTTGCTCATTAACATCCGACGGGGGAATA
ACATTGCTTCAGAGCTGGAGAACTTCATGGGGCTCATCGAGGTGGTGACGGGCTACGTGAAGATC
CGCCATTCTCATGCCTTGGTCTCCTTGTCCTTCCTAAAAAACCTTCGCCTCATCCTAGGAGAGGA
GCAGCTAGAAGGGAATTACTCCTTCTACGTCCTCGACAACCAGAACTTGCAGCAACTGTGGGACT
GGGACCACCGCAACCTGACCATCAAAGCAGGGAAAATGTACTTTGCTTTCAATCCCAAATTATGT
GTTTCCGAAATTTACCGCATGGAGGAAGTGACGGGGACTAAAGGGCGCCAAAGCAAAGGGGACAT
AAACACCAGGAACAACGGGGAGAGAGCCTCCTGTGAAAGTGACGTCCTGCATTTCACCTCCACCA
CCACGTCGAAGAATCGCATCATCATAACCTGGCACCGGTACCGGCCCCCTGACTACAGGGATCTC
ATCAGCTTCACCGTTTACTACAAGGAAGCACCCTTTAAGAATGTCACAGAGTATGATGGGCAGGA
TGCCTGCGGCTCCAACAGCTGGAACATGGTGGACGTGGACCTCCCGCCCAACAAGGACGTGGAGC
CCGGCATCTTACTACATGGGCTGAAGCCCTGGACTCAGTACGCCGTTTACGTCAAGGCTGTGACC
CTCACCATGGTGGAGAACGACCATATCCGTGGGGCCAAGAGTGAGATCTTGTACATTCGCACCAA
TGCTTCAGTTCCTTCCATTCCCTTGGACGTTCTTTCAGCATCGAACTCCTCTTCTCAGTTAATCG
TGAAGTGGAACCCTCCCTCTCTGCCCAACGGCAACCTGAGTTACTACATTGTGCGCTGGCAGCGG
CAGCCTCAGGACGGCTACCTTTACCGGCACAATTACTGCTCCAAAGACAAAATCCCCATCAGGAA
GTATGCCGACGGCACCATCGACATTGAGGAGGTCACAGAGAACCCCAAGACTGAGGTGTGTGGTG
GGGAGAAAGGGCCTTGCTGCGCCTGCCCCAAAACTGAAGCCGAGAAGCAGGCCGAGAAGGAGGAG
GCTGAATACCGCAAAGTCTTTGAGAATTTCCTGCACAACTCCATCTTCGTGCCCAGACCTGAAAG
GAAGCGGAGAGATGTCATGCAAGTGGCCAACACCACCATGTCCAGCCGAAGCAGGAACACCACGG
CCGCAGACACCTACAACATCACCGACCCGGAAGAGCTGGAGACAGAGTACCCTTTCTTTGAGAGC
AGAGTGGATAACAAGGAGAGAACTGTCATTTCTAACCTTCGGCCTTTCACATTGTACCGCATCGA
TATCCACAGCTGCAACCACGAGGCTGAGAAGCTGGGCTGCAGCGCCTCCAACTTCGTCTTTGCAA
GGACTATGCCCGCAGAAGGAGCAGATGACATTCCTGGGCCAGTGACCTGGGAGCCAAGGCCTGAA
AACTCCATCTTTTTAAAGTGGCCGGAACCTGAGAATCCCAATGGATTGATTCTAATGTATGAAAT
AAAATACGGATCACAAGTTGAGGATCAGCGAGAATGTGTGTCCAGACAGGAATACAGGAAGTATG
GAGGGGCCAAGCTAAACCGGCTAAACCCGGGGAACTACACAGCCCGGATTCAGGCCACATCTCTC
TCTGGGAATGGGTCGTGGACAGATCCTGTGTTCTTCTATGTCCAGGCCAAAACAGGATATGAAAA
CTTCATCCATTCCGGAGGTGGTGGATCCGTGCCCAGGGATTGTGGTTGTAAGCCTTGCATATGTA
CAGTCCCAGAAGTATCATCTGTCTTCATCTTCCCCCCAAAGCCCAAGGATGTGCTCACCATTACT
CTGACTCCTAAGGTCACGTGTGTTGTGGTAGACATCAGCAAGGATGATCCCGAGGTCCAGTTCAG
CTGGTTTGTAGATGATGTGGAGGTGCACACAGCTCAGACGAAACCCCGGGAGGAGCAGATCAACA
GCACTTTCCGTTCAGTCAGTGAACTTCCCATCATGCACCAGGACTGGCTCAATGGCAAGGAGTTC

| | | | | |
|---|---|---|---|---|
| | | | | AAATGCAGGGTCAACAGTGCAGCTTTCCCTGCCCCCATCGAGAAAACCATCTCCAAAACCAAAGG<br>CAGACCGAAGGCTCCACAGGTGTACACCATTCCACCTCCCAAGGAGCAGATGGCCAAGGATAAAG<br>TCAGTCTGACCTGCATGATAACAAACTTCTTCCCTGAAGACATTACTGTGGAGTGGCAGTGGAAT<br>GGGCAGCCAGCGGAGAACTACAAGAACACTCAGCCCATCATGGACACAGATGGCTCTTACTTCGT<br>CTACAGCAAGCTCAATGTGCAGAAGAGCAACTGGGAGGCAGGAAATACTTTCACCTGCTCTGTGT<br>TACATGAGGGCCTGCACAACCACCATACTGAGAAGAGCCTCTCCCACTCTCCTGGTAAATCCGGG<br>CATCATCACCATCATCATTGA |
| 1991. | soluble fusion protein of h IGF-R1 and m IgG1 Fc | artificial | aa | MKSGSGGGSPTSLWGLLFLSAALSLWPTSGEICGPGIDIRNDYQQLKRLENCTVIEGYLHILLIS<br>KAEDYRSYRFPKLTVITEYLLLFRVAGLESLGDLFPNLTVIRGWKLFYNYALVIFEMTNLKDIGL<br>YNLRNITRGAIRIEKNADLCYLSTVDWSLILDAVSNNYIVGNKPPKECGDLCPGTMEEKPMCEKT<br>TINNEYNYRCWTTNRCQKMCPSTCGKRACTENNECCHPECLGSCSAPDNDTACVACRHYYYAGVC<br>VPACPPNTYRFEGWRCVDRDFCANILSAESSDSEGFVIHDGECMQECPSGFIRNGSQSMYCIPCE<br>GPCPKVCEEEKKTKTIDSVTSAQMLQGCTIFKGNLLINIRRGNNIASELENFMGLIEVVTGYVKI<br>RHSHALVSLSFLKNLRLILGEEQLEGNYSFYVLDNQNLQQLWDWDHRNLTIKAGKMYFAFNPKLC<br>VSEIYRMEEVTGTKGRQSKGDINTRNNGERASCESDVLHFTSTTTSKNRIIITWHRYRPPDYRDL<br>ISFTVYYKEAPFKNVTEYDGQDACGSNSWNMVDVDLPPNKDVEPGILLHGLKPWTQYAVYVKAVT<br>LTMVENDHIRGAKSEILYIRTNASVPSIPLDVLSASNSSSQLIVKWNPPSLPNGNLSYYIVRWQR<br>QPQDGYLYRHNYCSKDKIPIRKYADGTIDIEEVTENPKTEVCGGEKGPCCACPKTEAEKQAEKEE<br>AEYRKVFENFLHNSIFVPRPERKRRDVMQVANTTMSSRSRNTTAADTYNITDPEELETEYPFFES<br>RVDNKERTVISNLRPFTLYRIDIHSCNHEAEKLGCSASNFVFARTMPAEGADDIPGPVTWEPRPE<br>NSIFLKWPEPENPNGLILMYEIKYGSQVEDQRECVSRQEYRKYGGAKLNRLNPGNYTARIQATSL<br>SGNGSWTDPVFFYVQAKTGYENFIHSGGGGSVPRDCGCKPCICTVPEVSSVFIFPPKPKDVLTIT<br>LTPKVTCVVVDISKDDPEVQFSWFVDDVEVHTAQTKPREEQINSTFRSVSELPIMHQDWLNGKEF<br>KCRVNSAAFPAPIEKTISKTKGRPKAPQVYTIPPPKEQMAKDKVSLTCMITNFFPEDITVEWQWN<br>GQPAENYKNTQPIMDTDGSYFVYSKLNVQKSNWEAGNTFTCSVLHEGLHNHHTEKSLSHSPGKSG<br>HHHHHH |
| 1992. | murine IGF-R1 construct | artificial | nt | ATGGGATGGAGCTGTATCATCCTCTTCTTGGTAGCAACAGCTACAGGTGTACACTCCGATTACAA<br>GGACGACGATGACAAGGAAATCTGTGGGCCCGGCATTGACATCCGCAACGACTATCAGCAGCTGA<br>AGCGCCTGGAAAACTGCACGGTGATCGAGGGCTTCCTCCACATCCTGCTCATCTCCAAGGCCGAG<br>GACTACCGAAGCTACCGCTTCCCCAAGCTCACCGTCATCACTGAGTACTTGCTGCTCTTCCGAGT<br>CGCTGGCCTCGAGAGCCTGGGAGACCTCTTCCCCAACCTCACAGTCATCCGTGGCTGGAAACTCT<br>TCTACAACTACGCACTGGTCATCTTCGAGATGACCAATCTCAAGGATATTGGGCTTTATAATCTG<br>AGGAACATTACTCGGGGGGCCATCAGGATTGAGAAGAACGCCGACCTCTGTTACCTCTCCACCAT<br>AGACTGGTCTCTCATCTTGGATGCGGTGTCCAATAACTACATTGTGGGGAACAAGCCCCCGAAGG<br>AATGTGGGGACCTGTGTCCAGGGACATTGGAGGAGAAGCCCATGTGTGAGAAGACCACCATCAAC<br>AATGAGTACAACTACCGCTGCTGGACCACAAATCGCTGCCAGAAAATGTGCCCAAGTGTGTGCGG<br>GAAGCGAGCCTGCACCGAGAACAACGAGTGCTGCCACCCGGAGTGCCTGGGCAGCTGCCACACAC |

CGGACGACAACACAACCTGCGTGGCCTGCAGACACTACTACTACAAAGGCGTGTGTGTGCCTGCC
TGCCCGCCTGGCACCTACAGGTTCGAGGGCTGGCGCTGTGTGGATCGCGATTTCTGCGCCAACAT
CCCCAACGCTGAGAGCAGTGACTCGGATGGCTTCGTTATCCACGACGATGAGTGCATGCAGGAGT
GTCCCTCAGGCTTCATCCGCAACAGCACCCAGAGCATGTACTGTATCCCCTGCGAAGGCCCCTGC
CCCAAAGTCTGCGGCGATGAAGAGAAGAAAACGAAAACCATCGATTCGGTGACTTCTGCTCAAAT
GCTCCAAGGATGCACCATCCTGAAGGGCAATCTGCTTATTAACATCCGGAGAGGCAATAACATTG
CCTCGGAGTTGGAGAACTTCATGGGGCTCATCGAGGTGGTGACCGGCTACGTGAAGATCCGCCAT
TCTCATGCCTTGGTCTCCTTGTCCTTCCTGAAGAACCTTCGTCTCATCTTAGGAGAGGAGCAGCT
GGAAGGGAACTACTCCTTCTATGTCCTAGACAACCAGAACTTGCAGCAGCTGTGGGACTGGAACC
ACCGGAACCTGACCGTCAGGTCCGGAAAGATGTACTTTGCTTTCAATCCCAAGCTGTGTGTCTCC
GAAATTTACCGCATGGAGGAAGTGACCGGAACCAAGGGACGCCAGAGCAAAGGGGACATAAACAC
CAGGAACAACGGAGAGCGAGCTTCCTGTGAAAGTGATGTTCTCCGTTTCACCTCCACCACGACCT
GGAAGAACCGAATCATCATAACGTGGCACCGGTACCGGCCGCCGGACTACCGGGATCTCATCAGC
TTCACAGTTTACTACAAGGAGGCACCATTTAAAAACGTTACGGAATATGACGGGCAGGATGCCTG
TGGCTCCAACAGCTGGAACATGGTGGATGTAGACCTGCCTCCGAACAAGGAGGGCGAGCCTGGCA
TTTTACTGCATGGGCTGAAGCCCTGGACCCAGTATGCTGTCTATGTCAAGGCTGTGACCCTCACC
ATGGTGGAAAACGACCATATCCGTGGGGCCAAAAGTGAAATCTTGTACATTCGCACCAATGCTTC
AGTCCCTTCCATTCCCCTAGATGTCCTCTCAGCATCAAACTCTTCCTCTCAGCTGATTGTGAAGT
GGAATCCTCCAACTCTGCCCAATGGTAACTTGAGTTACTACATTGTGAGGTGGCAGCGGCAGCCC
CAGGATGGTTACCTGTACCGGCACAACTACTGCTCCAAAGACAAAATACCCATCAGAAAGTACGC
CGATGGTACCATCGACGTGGAGGAGGTGACGGAAAATCCCAAGACAGAAGTGTGTGGTGGTGATA
AAGGGCCATGCTGCGCTTGCCCTAAAACTGAAGCTGAGAAGCAGGCTGAGAAGGAGGAGGCTGAG
TACCGTAAAGTCTTTGAGAATTTCCTTCACAATTCCATCTTTGTGCCCAGGCCCGAAAGGAGGCG
GAGAGACGTCATGCAAGTGGCCAACACGACCATGTCCAGCCGAAGCAGGAACACCACGGTAGCTG
ACACCTACAATATCACAGACCCGGAGGAGTTCGAGACAGAGTACCCTTTCTTTGAGAGCAGAGTG
GATAACAAGGAGAGGACTGTCATCTCCAACCTCCGGCCTTTCACTCTGTACCGCATCGATATCCA
CAGCTGCAACCACGAGGCTGAGAAGCTGGGCTGCAGCGCCTCCAACTTCGTCTTTGCGAGAACCA
TGCCAGCAGAAGGAGCAGATGATATCCCTGGTCCGGTGACCTGGGAGCCAAGACCCGAAAACTCC
ATCTTTTTAAAGTGGCCAGAACCCGAGAACCCCAACGGATTGATCCTAATGTATGAAATTAAATA
CGGGTCGCAAGTCGAGGATCAGCGGGAATGTGTGTCCAGACAGGAGTACAGGAAGTACGGAGGGG
CCAAACTCAACCGTCTAAACCCAGGGAACTATACAGCCCGGATTCAGGCTACCTCCCTCTCTGGG
AATGGGTCATGGACAGATCCTGTGTTCTTCTATGTCCCCGCCAAAACGACGTATGAGAACTTCAT
GCATCTGATCATTGCTCTGCCGGTTGCCATCCTGCTGATCGTTGGGGGGCTGGTTATCATGCTGT
ATGTCTTCCATAGAAAGAGAAATAACAGCAGGTTGGGCAATGGAGTGCTGTATGCTTCTGTGAAC
CCCGAGTATTTCAGCGCAGCTGATGTGTACGTGCCTGATGAATGGGAGGTAGCTCGAGAGAAGAT
CACCATGAACCGGGAGCTCGGACAAGGGTCCTTTGGGATGGTCTATGAAGGAGTGGCCAAGGGTG
TGGTCAAGGATGAACCCGAAACCAGAGTGGCCATCAAGACGGTAAACGAGGCTGCAAGTATGCGT

| | | | | |
|---|---|---|---|---|
| | | | | GAAAGAATCGAGTTTCTCAACGAGGCCTCGGTGATGAAGGAGTTCAATTGTCACCATGTGGTCCG<br>GTTGCTGGGTGTGGTATCCCAAGGCCAGCCCACCCTGGTCATCATGGAACTAATGACACGCGGTG<br>ATCTCAAAAGTTATCTCCGGTCTCTGAGGCCAGAAGTGGAGCAGAATAATCTAGTCCTCATTCCT<br>CCGAGCTTAAGCAAGATGATCCAGATGGCTGGAGAGATTGCAGATGGCATGGCCTACCTCAATGC<br>CAACAAGTTCGTCCACAGAGACCTTGCTGCTAGGAACTGCATGGTAGCCGAAGATTTCACAGTCA<br>AAATTGGAGATTTCGGTATGACACGAGACATCTACGAGACGGACTACTACCGGAAAGGCGGGAAG<br>GGGTTGCTGCCTGTGCGCTGGATGTCTCCCGAGTCCCTCAAGGATGGTGTCTTCACTACTCATTC<br>TGATGTCTGGTCCTTCGGGGTCGTCCTCTGGGAGATCGCCACGCTGGCTGAGCAGCCCTACCAGG<br>GCTTGTCCAACGAGCAAGTTCTTCGTTTCGTCATGGAGGGTGGCCTTCTGGACAAGCCGGACAAC<br>TGCCCTGATATGCTGTTTGAACTTATGCGCATGTGCTGGCAGTATAACCCCAAGATGCGGCCCTC<br>CTTCCTGGAGATCATCGGCAGCATCAAGGATGAGATGGAGCCCAGCTTCCAGGAGGTCTCCTTCT<br>ACTACAGCGAGGAGAACAAGCCTCCCGAGCCAGAGGAGCTGGAGATGGAGCCTGAGAACATGGAG<br>AGCGTCCCACTGGACCCTTCGGCCTCCTCAGCCTCCCTGCCTCTGCCTGAAAGACACTCAGGACA<br>CAAGGCTGAGAATGGCCCGGGCCCTGGCGTGCTCGTTCTCCGCGCCAGTTTTGATGAGAGACAGC<br>CTTACGCTCACATGAACGGGGGACGCGCCAACGAGAGGGCCTTGCCTCTGCCCCAGTCCTCGACC<br>TGCTGA |
| 1993. | murine IGF-R1 construct | artificial | aa | MGWSCIILFLVATATGVHSDYKDDDDKEICGPGIDIRNDYQQLKRLENCTVIEGFLHILLISKAE<br>DYRSYRFPKLTVITEYLLLFRVAGLESLGDLFPNLTVIRGWKLFYNYALVIFEMTNLKDIGLYNL<br>RNITRGAIRIEKNADLCYLSTIDWSLILDAVSNNYIVGNKPPKECGDLCPGTLEEKPMCEKTTIN<br>NEYNYRCWTTNRCQKMCPSVCGKRACTENNECCHPECLGSCHTPDDNTTCVACRHYYYKGVCVPA<br>CPPGTYRFEGWRCVDRDFCANIPNAESSDSDGFVIHDDECMQECPSGFIRNSTQSMYCIPCEGPC<br>PKVCGDEEKKTKTIDSVTSAQMLQGCTILKGNLLINIRRGNNIASELENFMGLIEVVTGYVKIRH<br>SHALVSLSFLKNLRLILGEEQLEGNYSFYVLDNQNLQQLWDWNHRNLTVRSGKMYFAFNPKLCVS<br>EIYRMEEVTGTKGRQSKGDINTRNNGERASCESDVLRFTSTTTWKNRIIITWHRYRPPDYRDLIS<br>FTVYYKEAPFKNVTEYDGQDACGSNSWNMVDVDLPPNKEGEPGILLHGLKPWTQYAVYVKAVTLT<br>MVENDHIRGAKSEILYIRTNASVPSIPLDVLSASNSSSQLIVKWNPPTLPNGNLSYYIVRWQRQP<br>QDGYLYRHNYCSKDKIPIRKYADGTIDVEEVTENPKTEVCGGDKGPCCACPKTEAEKQAEKEEAE<br>YRKVFENFLHNSIFVPRPERRRRDVMQVANTTMSSRSRNTTVADTYNITDPEEFETEYPFFESRV<br>DNKERTVISNLRPFTLYRIDIHSCNHEAEKLGCSASNFVFARTMPAEGADDIPGPVTWEPRPENS<br>IFLKWPEPENPNGLILMYEIKYGSQVEDQRECVSRQEYRKYGGAKLNRLNPGNYTARIQATSLSG<br>NGSWTDPVFFYVPAKTTYENFMHLIIALPVAILLIVGGLVIMLYVFHRKRNNSRLGNGVLYASVN<br>PEYFSAADVYVPDEWEVAREKITMNRELGQGSFGMVYEGVAKGVVKDEPETRVAIKTVNEAASMR<br>ERIEFLNEASVMKEFNCHHVVRLLGVVSQGQPTLVIMELMTRGDLKSYLRSLRPEVEQNNLVLIP<br>PSLSKMIQMAGEIADGMAYLNANKFVHRDLAARNCMVAEDFTVKIGDFGMTRDIYETDYYRKGGK<br>GLLPVRWMSPESLKDGVFTTHSDVWSFGVVLWEIATLAEQPYQGLSNEQVLRFVMEGGLLDKPDN<br>CPDMLFELMRMCWQYNPKMRPSFLEIIGSIKDEMEPSFQEVSFYYSEENKPPEPEELEMEPENME<br>SVPLDPSASSASLPLPERHSGHKAENGPGPGVLVLRASFDERQPYAHMNGGRANERALPLPQSST |

| | | | | C |
|---|---|---|---|---|
| 1994. | mutated human IGF-R1 antigen | artificial | nt | ATGGGATGGAGCTGTATCATCCTCTTCTTGGTAGCAACAGCTACAGGTGTACACTCCGATTACAAGGACGACGATGACAAGGAAATCTGTGGGCCCGGCATTGACATCCGCAACGACTATCAGCAGCTGAAGCGCCTGGAAAACTGCACGGTGATCGAGGGCTTCCTCCACATCCTGCTCATCTCCAAGGCCGAGGACTACCGAAGCTACCGCTTCCCCAAGCTCACCGTCATCACTGAGTACTTGCTGCTCTTCCGAGTCGCTGGCCTCGAGAGCCTGGGAGACCTCTTCCCCAACCTCACAGTCATCCGTGGCTGGAAACTCTTCTACAACTACGCACTGGTCATCTTCGAGATGACCAATCTCAAGGATATTGGGCTTTATAATCTGAGGAACATTACTCGGGGGGCCATCAGGATTGAGAAGAACGCCGACCTCTGTTACCTCTCCACCATAGACTGGTCTCTCATCTTGGATGCGGTGTCCAATAACTACATTGTGGGGAACAAGCCCCCGAAGGAATGTGGGGACCTGTGTCCAGGGACATTGGAGGAGAAGCCCATGTGTGAGAAGACCACCATCAACAATGAGTACAACTACCGCTGCTGGACCACAAATCGCTGCCAGAAAATGTGCCCAAGTGTGTGCGGGAAGCGAGCCTGCACCGAGAACAACGAGTGCTGCCACCCGGAGTGCCTGGGCAGCTGCCACACACCGGACGACAACACAACCTGCGTGGCCTGCAGACACTACTACTACAAAGGCGTGTGTGTGCCTGCCTGCCCGCCTGGCACCTACAGGTTCGAGGGCTGGCGCTGTGTGGATCGCGATTTCTGCGCCAACATCCCCAACGCTGAGAGCAGTGACTCGGATGGCTTCGTTATCCACGACGATGAGTGCATGCAGGAGTGTCCCTCAGGCTTCATCCGCAACAGCACCCAGAGCATGTACTGTATCCCCTGCGAAGGCCCCTGCCCCAAGGTCTGTGAGGAAGAAAAGAAAACAAAGACCATTGATTCTGTTACTTCTGCTCAGATGCTCCAAGGATGCACCATCTTCAAGGGCAATTTGCTCATTAACATCCGACGGGGGAATAACATTGCTTCAGAGCTGGAGAACTTCATGGGGCTCATCGAGGTGGTGACGGGCTACGTGAAGATCCGCCATTCTCATGCCTTGGTCTCCTTGTCCTTCCTAAAAAACCTTCGCCTCATCCTAGGAGAGGAGCAGCTAGAAGGGAATTACTCCTTCTACGTCCTCGACAACCAGAACTTGCAGCAACTGTGGGACTGGGACCACCGCAACCTGACCATCAAAGCAGGGAAAATGTACTTTGCTTTCAATCCCAAATTATGTGTTTCCGAAATTTACCGCATGGAGGAAGTGACGGGGACTAAAGGGCGCCAAAGCAAAGGGGACATAAACACCAGGAACAACGGGGAGAGAGCCTCCTGTGAAAGTGACGTCCTGCATTTCACCTCCACCACCACGTCGAAGAATCGCATCATCATAACCTGGCACCGGTACCGGCCCCCTGACTACAGGGATCTCATCAGCTTCACCGTTTACTACAAGGAAGCACCCTTTAAGAATGTCACAGAGTATGATGGGCAGGATGCCTGCGGCTCCAACAGCTGGAACATGGTGGACGTGGACCTCCCGCCCAACAAGGACGTGGAGCCCGGCATCTTACTACATGGGCTGAAGCCCTGGACTCAGTACGCCGTTTACGTCAAGGCTGTGACCCTCACCATGGTGGAGAACGACCATATCCGTGGGGCCAAGAGTGAGATCTTGTACATTCGCACCAATGCTTCAGTTCCTTCCATTCCCTTGGACGTTCTTTCAGCATCGAACTCCTCTTCTCAGTTAATCGTGAAGTGGAACCCTCCCTCTCTGCCCAACGGCAACCTGAGTTACTACATTGTGCGCTGGCAGCGGCAGCCTCAGGACGGCTACCTTTACCGGCACAATTACTGCTCCAAAGACAAAATCCCCATCAGGAAGTATGCCGACGGCACCATCGACATTGAGGAGGTCACAGAGAACCCCAAGACTGAGGTGTGTGGTGGGGAGAAAGGGCCTTGCTGCGCCTGCCCCAAAACTGAAGCCGAGAAGCAGGCCGAGAAGGAGGAGGCTGAATACCGCAAAGTCTTTGAGAATTTCCTGCACAACTCCATCTTCGTGCCCAGACCTGAAAGGAAGCGGAGAGATGTCATGCAAGTGGCCAACACCACCATGTCCAGCCGAAGCAGGAACACCACGGCCGCAGACACCTACAACATCACCGACCCGGAAGAGCTGGAGACAGAGTACCCTTTCTTTGAGAGCAGAGTGGAT |

| | | | | |
|---|---|---|---|---|
| | | | | AACAAGGAGAGAACTGTCATTTCTAACCTTCGGCCTTTCACATTGTACCGCATCGATATCCACAG<br>CTGCAACCACGAGGCTGAGAAGCTGGGCTGCAGCGCCTCCAACTTCGTCTTTGCAAGGACTATGC<br>CCGCAGAAGGAGCAGATGACATTCCTGGGCCAGTGACCTGGGAGCCAAGGCCTGAAAACTCCATC<br>TTTTTAAAGTGGCCGGAACCTGAGAATCCCAATGGATTGATTCTAATGTATGAAATAAAATACGG<br>ATCACAAGTTGAGGATCAGCGAGAATGTGTGTCCAGACAGGAATACAGGAAGTATGGAGGGGCCA<br>AGCTAAACCGGCTAAACCCGGGGAACTACACAGCCCGGATTCAGGCCACATCTCTCTCTGGGAAT<br>GGGTCGTGGACAGATCCTGTGTTCTTCTATGTCCAGGCCAAAACAGGATATGAAAACTTCATCCA<br>TCTGATCATCGCTCTGCCCGTCGCTGTCCTGTTGATCGTGGGAGGGTTGGTGATTATGCTGTACG<br>TCTTCCATAGAAAGAGAAATAACAGCAGGCTGGGGAATGGAGTGCTGTATGCCTCTGTGAACCCG<br>GAGTACTTCAGCGCTGCTGATGTGTACGTTCCTGATGAGTGGGAGGTGGCTCGGGAGAAGATCAC<br>CATGAGCCGGGAACTTGGGCAGGGGTCGTTTGGGATGGTCTATGAAGGAGTTGCCAAGGGTGTGG<br>TGAAAGATGAACCTGAAACCAGAGTGGCCATTAAAACAGTGAACGAGGCCGCAAGCATGCGTGAG<br>AGGATTGAGTTTCTCAACGAAGCTTCTGTGATGAAGGAGTTCAATTGTCACCATGTGGTGCGATT<br>GCTGGGTGTGGTGTCCCAAGGCCAGCCAACACTGGTCATCATGGAACTGATGACACGGGGCGATC<br>TCAAAAGTTATCTCCGGTCTCTGAGGCCAGAAATGGAGAATAATCCAGTCCTAGCACCTCCAAGC<br>CTGAGCAAGATGATTCAGATGGCCGGAGAGATTGCAGACGGCATGGCATACCTCAACGCCAATAA<br>GTTCGTCCACAGAGACCTTGCTGCCCGGAATTGCATGGTAGCCGAAGATTTCACAGTCAAAATCG<br>GAGATTTTGGTATGACGCGAGATATCTATGAGACAGACTATTACCGGAAAGGAGGGAAAGGGCTG<br>CTGCCCGTGCGCTGGATGTCTCCTGAGTCCCTCAAGGATGGAGTCTTCACCACTTACTCGGACGT<br>CTGGTCCTTCGGGGTCGTCCTCTGGGAGATCGCCACACTGGCCGAGCAGCCCTACCAGGGCTTGT<br>CCAACGAGCAAGTCCTTCGCTTCGTCATGGAGGGCGGCCTTCTGGACAAGCCAGACAACTGTCCT<br>GACATGCTGTTTGAACTGATGCGCATGTGCTGGCAGTATAACCCCAAGATGAGGCCTTCCTTCCT<br>GGAGATCATCAGCAGCATCAAAGAGGAGATGGAGCCTGGCTTCCGGGAGGTCTCCTTCTACTACA<br>GCGAGGAGAACAAGCTGCCCGAGCCGGAGGAGCTGGACCTGGAGCCAGAGAACATGGAGAGCGTC<br>CCCCTGGACCCCTCGGCCTCCTCGTCCTCCCTGCCACTGCCCGACAGACACTCAGGACACAAGGC<br>CGAGAACGGCCCCGGCCCTGGGGTGCTGGTCCTCCGCGCCAGCTTCGACGAGAGACAGCCTTACG<br>CCCACATGAACGGGGGCCGCAAGAACGAGCGGGCCTTGCCGCTGCCCCAGTCTTCGACCTGCTGA |
| 1995. | mutated human IGF-R1 antigen | artificial | aa | MGWSCIILFLVATATGVHSDYKDDDDKEICGPGIDIRNDYQQLKRLENCTVIEGFLHILLISKAE<br>DYRSYRFPKLTVITEYLLLFRVAGLESLGDLFPNLTVIRGWKLFYNYALVIFEMTNLKDIGLYNL<br>RNITRGAIRIEKNADLCYLSTIDWSLILDAVSNNYIVGNKPPKECGDLCPGTLEEKPMCEKTTIN<br>NEYNYRCWTTNRCQKMCPSVCGKRACTENNECCHPECLGSCHTPDDNTTCVACRHYYYKGVCVPA<br>CPPGTYRFEGWRCVDRDFCANIPNAESSDSDGFVIHDDECMQECPSGFIRNSTQSMYCIPCEGPC<br>PKVCEEEKKTKTIDSVTSAQMLQGCTIFKGNLLINIRRGNNIASELENFMGLIEVVTGYVKIRHS<br>HALVSLSFLKNLRLILGEEQLEGNYSFYVLDNQNLQQLWDWDHRNLTIKAGKMYFAFNPKLCVSE<br>IYRMEEVTGTKGRQSKGDINTRNNGERASCESDVLHFTSTTTSKNRIIITWHRYRPPDYRDLISF<br>TVYYKEAPFKNVTEYDGQDACGSNSWNMVDVDLPPNKDVEPGILLHGLKPWTQYAVYVKAVTLTM<br>VENDHIRGAKSEILYIRTNASVPSIPLDVLSASNSSSQLIVKWNPPSLPNGNLSYYIVRWQRQPQ |

| | | | | |
|---|---|---|---|---|
| | | | | DGYLYRHNYCSKDKIPIRKYADGTIDIEEVTENPKTEVCGGEKGPCCACPKTEAEKQAEKEEAEY<br>RKVFENFLHNSIFVPRPERKRRDVMQVANTTMSSRSRNTTAADTYNITDPEELETEYPFFESRVD<br>NKERTVISNLRPFTLYRIDIHSCNHEAEKLGCSASNFVFARTMPAEGADDIPGPVTWEPRPENSI<br>FLKWPEPENPNGLILMYEIKYGSQVEDQRECVSRQEYRKYGGAKLNRLNPGNYTARIQATSLSGN<br>GSWTDPVFFYVQAKTGYENFIHLIIALPVAVLLIVGGLVIMLYVFHRKRNNSRLGNGVLYASVNP<br>EYFSAADVYVPDEWEVAREKITMSRELGQGSFGMVYEGVAKGVVKDEPETRVAIKTVNEAASMRE<br>RIEFLNEASVMKEFNCHHVVRLLGVVSQGQPTLVIMELMTRGDLKSYLRSLRPEMENNPVLAPPS<br>LSKMIQMAGEIADGMAYLNANKFVHRDLAARNCMVAEDFTVKIGDFGMTRDIYETDYYRKGGKGL<br>LPVRWMSPESLKDGVFTTYSDVWSFGVVLWEIATLAEQPYQGLSNEQVLRFVMEGGLLDKPDNCP<br>DMLFELMRMCWQYNPKMRPSFLEIISSIKEEMEPGFREVSFYYSEENKLPEPEELDLEPENMESV<br>PLDPSASSSSLPLPDRHSGHKAENGPGPGVLVLRASFDERQPYAHMNGGRKNERALPLPQSSTC |
| 1996. | mutated murine IGF-R1 antigen | artificial | nt | ATGGGATGGAGCTGTATCATCCTCTTCTTGGTAGCAACAGCTACAGGTGTACACTCCGATTACAA<br>GGACGACGATGACAAGGAAATCTGCGGGCCAGGCATCGACATCCGCAACGACTATCAGCAGCTGA<br>AGCGCCTGGAGAACTGCACGGTGATCGAGGGCTACCTCCACATCCTGCTCATCTCCAAGGCCGAG<br>GACTACCGCAGCTACCGCTTCCCCAAGCTCACGGTCATTACCGAGTACTTGCTGCTGTTCCGAGT<br>GGCTGGCCTCGAGAGCCTCGGAGACCTCTTCCCCAACCTCACGGTCATCCGCGGCTGGAAACTCT<br>TCTACAACTACGCCCTGGTCATCTTCGAGATGACCAATCTCAAGGATATTGGGCTTTACAACCTG<br>AGGAACATTACTCGGGGGGCCATCAGGATTGAGAAAAATGCTGACCTCTGTTACCTCTCCACTGT<br>GGACTGGTCCCTGATCCTGGATGCGGTGTCCAATAACTACATTGTGGGGAATAAGCCCCCAAAGG<br>AATGTGGGGACCTGTGTCCAGGGACCATGGAGGAGAAGCCGATGTGTGAGAAGACCACCATCAAC<br>AATGAGTACAACTACCGCTGCTGGACCACAAACCGCTGCCAGAAAATGTGCCCAAGCACGTGTGG<br>GAAGCGGGCGTGCACCGAGAACAATGAGTGCTGCCACCCCGAGTGCCTGGGCAGCTGCAGCGCGC<br>CTGACAACGACACGGCCTGTGTAGCTTGCCGCCACTACTACTATGCCGGTGTCTGTGTGCCTGCC<br>TGCCCGCCCAACACCTACAGGTTTGAGGGCTGGCGCTGTGTGGACCGTGACTTCTGCGCCAACAT<br>CCTCAGCGCCGAGAGCAGCGACTCCGAGGGGTTTGTGATCCACGACGGCGAGTGCATGCAGGAGT<br>GCCCCTCGGGCTTCATCCGCAACGGCAGCCAGAGCATGTACTGCATCCCTTGTGAAGGTCCTTGC<br>CCGAAAGTCTGCGGCGATGAAGAGAAGAAAACGAAAACCATCGATTCGGTGACTTCTGCTCAAAT<br>GCTCCAAGGATGCACCATCCTGAAGGGCAATCTGCTTATTAACATCCGGAGAGGCAATAACATTG<br>CCTCGGAGTTGGAGAACTTCATGGGGCTCATCGAGGTGGTGACCGGCTACGTGAAGATCCGCCAT<br>TCTCATGCCTTGGTCTCCTTGTCCTTCCTGAAGAACCTTCGTCTCATCTTAGGAGAGGAGCAGCT<br>GGAAGGGAACTACTCCTTCTATGTCCTAGACAACCAGAACTTGCAGCAGCTGTGGGACTGGAACC<br>ACCGGAACCTGACCGTCAGGTCCGGAAAGATGTACTTTGCTTTCAATCCCAAGCTGTGTGTCTCC<br>GAAATTTACCGCATGGAGGAAGTGACCGGAACCAAGGGACGCCAGAGCAAAGGGGACATAAACAC<br>CAGGAACAACGGAGAGCGAGCTTCCTGTGAAAGTGATGTTCTCCGTTTCACCTCCACCACGACCT<br>GGAAGAACCGAATCATCATAACGTGGCACCGGTACCGGCCGCCGGACTACCGGGATCTCATCAGC<br>TTCACAGTTTACTACAAGGAGGCACCATTTAAAAACGTTACGGAATATGACGGGCAGGATGCCTG<br>TGGCTCCAACAGCTGGAACATGGTGGATGTAGACCTGCCTCCGAACAAGGAGGGCGAGCCTGGCA |

| | | | | |
|---|---|---|---|---|
| TTTTACTGCATGGGCTGAAGCCCTGGACCCAGTATGCTGTCTATGTCAAGGCTGTGACCCTCACC<br>ATGGTGGAAAACGACCATATCCGTGGGGCCAAAAGTGAAATCTTGTACATTCGCACCAATGCTTC<br>AGTCCCTTCCATTCCCCTAGATGTCCTCTCAGCATCAAACTCTTCCTCTCAGCTGATTGTGAAGT<br>GGAATCCTCCAACTCTGCCCAATGGTAACTTGAGTTACTACATTGTGAGGTGGCAGCGGCAGCCC<br>CAGGATGGTTACCTGTACCGGCACAACTACTGCTCCAAAGACAAAATACCCATCAGAAAGTACGC<br>CGATGGTACCATCGACGTGGAGGAGGTGACGGAAAATCCCAAGACAGAAGTGTGTGGTGGTGATA<br>AAGGGCCATGCTGCGCTTGCCCTAAAACTGAAGCTGAGAAGCAGGCTGAGAAGGAGGAGGCTGAG<br>TACCGTAAAGTCTTTGAGAATTTCCTTCACAATTCCATCTTTGTGCCCAGGCCCGAAAGGAGGCG<br>GAGAGACGTCATGCAAGTGGCCAACACGACCATGTCCAGCCGAAGCAGGAACACCACGGTAGCTG<br>ACACCTACAATATCACAGACCCGGAGGAGTTCGAGACAGAGTACCCTTTCTTTGAGAGCAGAGTG<br>GATAACAAGGAGAGGACTGTCATCTCCAACCTCCGGCCTTTCACTCTGTACCGCATCGATATCCA<br>CAGCTGCAACCACGAGGCTGAGAAGCTGGGCTGCAGCGCCTCCAACTTCGTCTTTGCGAGAACCA<br>TGCCAGCAGAAGGAGCAGATGATATCCCTGGTCCGGTGACCTGGGAGCCAAGACCCGAAAACTCC<br>ATCTTTTTAAAGTGGCCAGAACCCGAGAACCCCAACGGATTGATCCTAATGTATGAAATTAAATA<br>CGGGTCGCAAGTCGAGGATCAGCGGGAATGTGTGTCCAGACAGGAGTACAGGAAGTACGGAGGGG<br>CCAAACTCAACCGTCTAAACCCAGGGAACTATACAGCCCGGATTCAGGCTACCTCCCTCTCTGGG<br>AATGGGTCATGGACAGATCCTGTGTTCTTCTATGTCCCCGCCAAAACGACGTATGAGAACTTCAT<br>GCATCTGATCATTGCTCTGCCGGTTGCCATCCTGCTGATCGTTGGGGGGCTGGTTATCATGCTGT<br>ATGTCTTCCATAGAAAGAGAAATAACAGCAGGTTGGGCAATGGAGTGCTGTATGCTTCTGTGAAC<br>CCCGAGTATTTCAGCGCAGCTGATGTGTACGTGCCTGATGAATGGGAGGTAGCTCGAGAGAAGAT<br>CACCATGAACCGGGAGCTCGGACAAGGGTCCTTTGGGATGGTCTATGAAGGAGTGGCCAAGGGTG<br>TGGTCAAGGATGAACCCGAAACCAGAGTGGCCATCAAGACGGTAAACGAGGCTGCAAGTATGCGT<br>GAAAGAATCGAGTTTCTCAACGAGGCCTCGGTGATGAAGGAGTTCAATTGTCACCATGTGGTCCG<br>GTTGCTGGGTGTGGTATCCCAAGGCCAGCCCACCCTGGTCATCATGGAACTAATGACACGCGGTG<br>ATCTCAAAAGTTATCTCCGGTCTCTGAGGCCAGAAGTGGAGCAGAATAATCTAGTCCTCATTCCT<br>CCGAGCTTAAGCAAGATGATCCAGATGGCTGGAGAGATTGCAGATGGCATGGCCTACCTCAATGC<br>CAACAAGTTCGTCCACAGAGACCTTGCTGCTAGGAACTGCATGGTAGCCGAAGATTTCACAGTCA<br>AAATTGGAGATTTCGGTATGACACGAGACATCTACGAGACGGACTACTACCGGAAAGGCGGGAAG<br>GGGTTGCTGCCTGTGCGCTGGATGTCTCCCGAGTCCCTCAAGGATGGTGTCTTCACTACTCATTC<br>TGATGTCTGGTCCTTCGGGGTCGTCCTCTGGGAGATCGCCACGCTGGCTGAGCAGCCCTACCAGG<br>GCTTGTCCAACGAGCAAGTTCTTCGTTTCGTCATGGAGGGTGGCCTTCTGGACAAGCCGGACAAC<br>TGCCCTGATATGCTGTTTGAACTTATGCGCATGTGCTGGCAGTATAACCCCAAGATGCGGCCCTC<br>CTTCCTGGAGATCATCGGCAGCATCAAGGATGAGATGGAGCCCAGCTTCCAGGAGGTCTCCTTCT<br>ACTACAGCGAGGAGAACAAGCCTCCCGAGCCAGAGGAGCTGGAGATGGAGCCTGAGAACATGGAG<br>AGCGTCCCACTGGACCCTTCGGCCTCCTCAGCCTCCCTGCCTCTGCCTGAAAGACACTCAGGACA<br>CAAGGCTGAGAATGGCCCGGGCCCTGGCGTGCTCGTTCTCCGCGCCAGTTTTGATGAGAGACAGC<br>CTTACGCTCACATGAACGGGGGACGCGCCAACGAGAGGGCCTTGCCTCTGCCCCAGTCCTCGACC | | | | |

| | | | | |
|---|---|---|---|---|
| | | | | TGCTGA |
| 1997. | mutated murine IGF-R1 antigen | artificial | aa | MGWSCIILFLVATATGVHSDYKDDDDKEICGPGIDIRNDYQQLKRLENCTVIEGYLHILLISKAE<br>DYRSYRFPKLTVITEYLLLFRVAGLESLGDLFPNLTVIRGWKLFYNYALVIFEMTNLKDIGLYNL<br>RNITRGAIRIEKNADLCYLSTVDWSLILDAVSNNYIVGNKPPKECGDLCPGTMEEKPMCEKTTIN<br>NEYNYRCWTTNRCQKMCPSTCGKRACTENNECCHPECLGSCSAPDNDTACVACRHYYYAGVCVPA<br>CPPNTYRFEGWRCVDRDFCANILSAESSDSEGFVIHDGECMQECPSGFIRNGSQSMYCIPCEGPC<br>PKVCGDEEKKTKTIDSVTSAQMLQGCTILKGNLLINIRRGNNIASELENFMGLIEVVTGYVKIRH<br>SHALVSLSFLKNLRLILGEEQLEGNYSFYVLDNQNLQQLWDWNHRNLTVRSGKMYFAFNPKLCVS<br>EIYRMEEVTGTKGRQSKGDINTRNNGERASCESDVLRFTSTTTWKNRIIITWHRYRPPDYRDLIS<br>FTVYYKEAPFKNVTEYDGQDACGSNSWNMVDVDLPPNKEGEPGILLHGLKPWTQYAVYVKAVTLT<br>MVENDHIRGAKSEILYIRTNASVPSIPLDVLSASNSSSQLIVKWNPPTLPNGNLSYYIVRWQRQP<br>QDGYLYRHNYCSKDKIPIRKYADGTIDVEEVTENPKTEVCGGDKGPCCACPKTEAEKQAEKEEAE<br>YRKVFENFLHNSIFVPRPERRRRDVMQVANTTMSSRSRNTTVADTYNITDPEEFETEYPFFESRV<br>DNKERTVISNLRPFTLYRIDIHSCNHEAEKLGCSASNFVFARTMPAEGADDIPGPVTWEPRPENS<br>IFLKWPEPENPNGLILMYEIKYGSQVEDQRECVSRQEYRKYGGAKLNRLNPGNYTARIQATSLSG<br>NGSWTDPVFFYVPAKTTYENFMHLIIALPVAILLIVGGLVIMLYVFHRKRNNSRLGNGVLYASVN<br>PEYFSAADVYVPDEWEVAREKITMNRELGQGSFGMVYEGVAKGVVKDEPETRVAIKTVNEAASMR<br>ERIEFLNEASVMKEFNCHHVVRLLGVVSQGQPTLVIMELMTRGDLKSYLRSLRPEVEQNNLVLIP<br>PSLSKMIQMAGEIADGMAYLNANKFVHRDLAARNCMVAEDFTVKIGDFGMTRDIYETDYYRKGGK<br>GLLPVRWMSPESLKDGVFTTHSDVWSFGVVLWEIATLAEQPYQGLSNEQVLRFVMEGGLLDKPDN<br>CPDMLFELMRMCWQYNPKMRPSFLEIIGSIKDEMEPSFQEVSFYYSEENKPPEPEELEMEPENME<br>SVPLDPSASSASLPLPERHSGHKAENGPGPGVLVLRASFDERQPYAHMNGGRANERALPLPQSST<br>C |
| 1998. | macaque IGF-R1 | artificial | nt | ATGAAGTCTGGCTCTGGAGAAGGGTCCCCGACCTCGCTGTGGGGGCTCCTGTTTCTCTCCGCCGC<br>GCTCTCGCTCTGGCCGACGAGTGGAGAAATCTGTGGGCCGGGCATCGACATCCGCAACGACTATC<br>AGCAGCTGAAGCGCCTGGAGAACTGCACGGTGATCGAGGGCTACCTCCACATCCTGCTCATCTCC<br>AAGGCCGAGGACTACCGCAGCTACCGCTTCCCCAAGCTCACGGTCATCACCGAGTACTTGCTGTT<br>GTTCCGAGTGGCTGGCCTAGAGAGCCTCGGAGACCTGTTCCCCAACCTCACGGTAATCCGCGGCT<br>GGAAACTCTTCTACAACTACGCCCTGGTCATCTTTGAGATGACCAATCTCAAGGATATTGGGCTT<br>TACAACCTGAGGAACATTACTCGGGGGGCCATCAGGATTGAGAAAAATGCTGACCTCTGTTACCT<br>CTCCACTGTGGACTGGTCCCTGATCCTGGATGCAGTGTCCAATAACTACATTGTGGGGAATAAGC<br>CCCCAAAGGAATGCGGGGACCTGTGTCCGGGGACCATGGAGGAGAAGCCGATGTGCGAGAAGACC<br>ACCATCAACAATGAGTACAACTACCGCTGCTGGACCACAAACCGCTGCCAGAAAATGTGCCCGAG<br>TGCCTGTGGGAAGAGGGCATGCACCGAGAACAACGAGTGCTGCCACCCCGAGTGCCTGGGCAGCT<br>GCAGCGCGCCTGACAACGACACGGCCTGTGTAGCTTGCCGCCACTACTACTACGCCGGTGTCTGC<br>GTGCCTGCCTGCCCGCCCAACACCTACAGGTTTGAGGGCTGGCGCTGTGTGGACCGTGACTTCTG<br>CGCCAACATCCTCAGCGCCGAGAGCAGCGACTCCGAGGGTTTCGTGATCCACGACGGCGAGTGCA |

TGCAGGAGTGCCCCTCAGGCTTCATCCGCAACGGCAGCCAGAGCATGTACTGCATCCCTTGTGAA
GGTCCTTGCCCCAAGGTCTGTGAGGAAGAAAAGAAAACAAAGACCATTGATTCTGTTACTTCTGC
TCAGATGCTCCAAGGATGCACCATCTTCAAGGGCAATTTGCTCATTAACATCCGACGGGGGAATA
ACATTGCTTCAGAACTGGAGAACTTCATGGGGCTCATCGAGGTGGTGACGGGCTACGTGAAGATC
CGCCATTCCCATGCCTTGGTCTCCTTGTCCTTCCTAAAAAACCTTCGCCTCATCTTAGGAGAGGA
GCAGCTAGAAGGGAATTACTCCTTCTACGTCCTCGACAACCAGAACTTGCAGCAACTATGGGACT
GGGACCACCGCAACCTGACCATCAAAGCAGGGAAAATGTACTTTGCTTTCAATCCCAAATTGTGT
GTTTCGGAAATTTACCGCATGGAGGAAGTGACGGGGACTAAAGGGCGCCAAAGCAAAGGGGACAT
AAACACCAGGAACAACGGGGAAAGAGCCTCCTGTGAAAGTGACGTCCTGCATTTCACCTCCACCA
CCACGTGGAAGAATCGCATCATCATAACCTGGCACCGGTACCGGCCCCCTGACTACAGGGATCTC
ATCAGCTTCACCGTTTACTACAAGGAAGCACCTTTTAAGAATGTCACGGAGTATGATGGGCAGGA
TGCCTGCGGCTCCAACAGCTGGAACATGGTGGATGTGGACCTCCCGCCCAACAAGGACGTGGAGC
CCGGCATCTTACTGCATGGGCTGAAGCCCTGGACTCAGTACGCCGTTTACGTCAAGGCTGTGACC
CTCACCATGGTGGAGAACGACCATATCCGTGGGGCCAAGAGTGAGATCTTGTACATTCGCACCAA
TGCTTCAGTTCCTTCCATTCCCTTGGACGTTCTTTCAGCATCGAACTCCTCTTCTCAGTTAATCG
TGAAGTGGAACCCTCCCTCTCTGCCCAACGGCAACCTGAGTTACTACATTGTGCGCTGGCAGCGG
CAGCCTCAGGACGGCTACCTTTACCGGCACAATTACTGCTCCAAAGACAAAATCCCCATCAGGAA
GTATGCCGACGGCACCATTGACATTGAGGAGGTCACAGAGAACCCGAAGACTGAGGTGTGTGGTG
GAGAGAAAGGGCCTTGCTGCGCCTGCCCCAAAACTGAAGCTGAGAAGCAGGCCGAGAAGGAGGAG
GCTGAGTACCGCAAAGTCTTTGAGAATTTCCTGCACAACTCCATCTTTGTGCCCAGACCTGAAAG
GAAGCGGAGAGATGTCATGCAAGTGGCCAACACCACCATGTCCAGCCGAAGCAGGAACACCACGG
TGGCAGACACCTACAACATCACAGATCTGGAAGAGCTAGAGACAGAGTACCCTTTCTTTGAGAGC
AGAGTGGATAATAAGGAGAGAACTGTCATTTCTAACCTTCGGCCTTTCACATTGTACCGCATTGA
TATCCACAGCTGCAACCACGAGGCTGAGAAACTGGGCTGCAGCGCCTCCAACTTTGTCTTTGCAA
GGACTATGCCTGCAGAAGGAGCAGATGACATTCCTGGGCCAGTGACCTGGGAGCCAAGGCCTGAA
AACTCCATCTTTTTAAAGTGGCCAGAACCTGAGAATCCCAATGGATTGATTCTAATGTATGAAAT
AAAATACGGATCACAAGTTGAGGATCAGCGAGAATGTGTGTCCAGACAGGAATACAGGAAGTATG
GAGGGGCCAAGCTAAACCGGCTAAACCCAGGGAACTACACAGCCCGGATTCAGGCTACATCTCTC
TCTGGGAATGGGTCGTGGACAGATCCTGTGTTCTTCTATGTCCAGGCCAAAACAGGATATGAAAA
CTTCATCCATCTGATCATCGCTCTGCCCGTCGCTGTCCTGTTGATCGTGGGAGGGTTGGTGATCA
TGCTGTACGTCTTCCATAGAAAGAGAAATAACAGCAGGCTGGGGAATGGAGTGCTGTACGCGTCT
GTGAACCCGGAGTACTTCAGCGCTGCGGATGTGTACGTTCCTGATGAGTGGGAGGTGGCTCGGGA
GAAGATCACCATGAGCCGGGAACTTGGGCAGGGGTCGTTTGGGATGGTCTATGAAGGAGTTGCCA
AGGGTGTGGTGAAAGACGAACCTGAAACCAGAGTGGCCATTAAAACAGTGAACGAGGCCGCGAGC
ATGCGTGAAAGGATCGAGTTTCTCAACGAGGCTTCTGTGATGAAGGAGTTCAATTGTCACCATGT
GGTGCGGTTGCTGGGTGTGGTGTCCCAGGGCCAGCCAACGCTGGTCATCATGGAACTGATGACGC
GGGGCGATCTCAAAAGTTATCTCCGGTCTCTGAGGCCAGAAATGGAGAATAATCCAGTCCTAGCA

| | | | | |
|---|---|---|---|---|
| | | | | CCTCCAAGCCTAAGCAAGATGATTCAGATGGCTGGAGAGATTGCAGACGGCATGGCATACCTCAACGCCAACAAGTTCGTCCACAGAGACCTTGCTGCCCGGAATTGCATGGTAGCCGAGGATTTCACAGTCAAAATTGGAGATTTTGGGATGACGCGAGATATCTATGAGACAGACTATTACCGGAAAGGAGGGAAAGGGCTGTTGCCCGTGCGCTGGATGTCTCCCGAGTCCCTCAAGGATGGAGTCTTCACCACTTACTCGGACGTCTGGTCCTTCGGGGTTGTCCTCTGGGAGATCGCCACACTGGCCGAGCAGCCCTACCAGGGCTTGTCCAACGAGCAAGTCCTTCGCTTCGTCATGGAGGGCGGCCTTCTGGACAAGCCAGACAACTGCCCCGACATGCTGTTTGAATTGATGCGCATGTGCTGGCAGTACAACCCCAAGATGAGGCCTTCCTTCCTGGAGATCATCAGCAGCATCAAAGACGAGATGGAGCCTGGCTTCCGGGAGGTCTCCTTCTACTACAGTGAGGAGAACAAGCTGCCCGAGCCGGAGGAGCTGGACCTGGAGCCAGAGAACATGGAGAGCGTCCCCCTGGACCCCTCGGCCTCCTCGTCCTCCCTGCCACTGCCCGACAGACACTCAGGACACAAGGCCGAGAACGGCCCCGGCCCTGGAGTGCTGGTGCTCCGCGCCAGCTTCGATGAGAGACAGCCTTACGCACACATGAACGGTGGCCGCAAGAACGAGCGGGCCTTGCCGCTGCCCCAGTCTTCGACCTGCTGA |
| 1999. | macaque IGF-R1 | artificial | aa | MKSGSGEGSPTSLWGLLFLSAALSLWPTSGEICGPGIDIRNDYQQLKRLENCTVIEGYLHILLISKAEDYRSYRFPKLTVITEYLLLFRVAGLESLGDLFPNLTVIRGWKLFYNYALVIFEMTNLKDIGLYNLRNITRGAIRIEKNADLCYLSTVDWSLILDAVSNNYIVGNKPPKECGDLCPGTMEEKPMCEKTTINNEYNYRCWTTNRCQKMCPSACGKRACTENNECCHPECLGSCSAPDNDTACVACRHYYYAGVCVPACPPNTYRFEGWRCVDRDFCANILSAESSDSEGFVIHDGECMQECPSGFIRNGSQSMYCIPCEGPCPKVCEEEKKTKTIDSVTSAQMLQGCTIFKGNLLINIRRGNNIASELENFMGLIEVVTGYVKIRHSHALVSLSFLKNLRLILGEEQLEGNYSFYVLDNQNLQQLWDWDHRNLTIKAGKMYFAFNPKLCVSEIYRMEEVTGTKGRQSKGDINTRNNGERASCESDVLHFTSTTTWKNRIIITWHRYRPPDYRDLISFTVYYKEAPFKNVTEYDGQDACGSNSWNMVDVDLPPNKDVEPGILLHGLKPWTQYAVYVKAVTLTMVENDHIRGAKSEILYIRTNASVPSIPLDVLSASNSSSQLIVKWNPPSLPNGNLSYYIVRWQRQPQDGYLYRHNYCSKDKIPIRKYADGTIDIEEVTENPKTEVCGGEKGPCCACPKTEAEKQAEKEEAEYRKVFENFLHNSIFVPRPERKRRDVMQVANTTMSSRSRNTTVADTYNITDLEELETEYPFFESRVDNKERTVISNLRPFTLYRIDIHSCNHEAEKLGCSASNFVFARTMPAEGADDIPGPVTWEPRPENSIFLKWPEPENPNGLILMYEIKYGSQVEDQRECVSRQEYRKYGGAKLNRLNPGNYTARIQATSLSGNGSWTDPVFFYVQAKTGYENFIHLIIALPVAVLLIVGGLVIMLYVFHRKRNNSRLGNGVLYASVNPEYFSAADVYVPDEWEVAREKITMSRELGQGSFGMVYEGVAKGVVKDEPETRVAIKTVNEAASMRERIEFLNEASVMKEFNCHHVVRLLGVVSQGQPTLVIMELMTRGDLKSYLRSLRPEMENNPVLAPPSLSKMIQMAGEIADGMAYLNANKFVHRDLAARNCMVAEDFTVKIGDFGMTRDIYETDYYRKGGKGLLPVRWMSPESLKDGVFTTYSDVWSFGVVLWEIATLAEQPYQGLSNEQVLRFVMEGGLLDKPDNCPDMLFELMRMCWQYNPKMRPSFLEIISSIKDEMEPGFREVSFYYSEENKLPEPEELDLEPENMESVPLDPSASSSSLPLPDRHSGHKAENGPGPGVLVLRASFDERQPYAHMNGGRKNERALPLPQSSTC |
| 2000. | human c-MET extracellular four Ig | human | aa | PAIYKVFPNSAPLEGGTRLTICGWDFGFRRNNKFDLKKTRVLLGNESCTLTLSESTMNTLKCTVGPAMNKHFNMSIIISNGHGTTQYSTFSYVDPVITSISPKYGPMAGGTLLTLTGNYLNSGNSRHISI |

| | | | | |
|---|---|---|---|---|
| | domains | | | GGKTCTLKSVSNSILECYTPAQTISTEFAVKLKIDLANRETSIFSYREDPIVYEIHPTKSFISGGSTITGVGKNLNSVSVPRMVINVHEAGRNFTVACQHRSNSEIICCTTPSLQQLNLQLPLKTKAFFMLDGILSKYFDLIYVHNPVFKPFEKPVMISMGNENVLEIKGNDIDPEAVKGEVLKVGNKSCENIHLHSEAVLCTVPNDLLKLNSELNIEWKQAISSTVLGKVI |
| 2001. | human c-MET extracellular cystein-rich domain | human | aa | CRHFQSCSQCLSAPPFVQCGWCHDKCVRSEECLSGTWTQQIC |
| 2002. | human c-MET extracellular beta-chain of a sema domain | human | aa | STKKEVFNILQAAYVSKPGAQLARQIGASLNDDILFGVFAQSKPDSAEPMDRSAMCAFPIKYVNDFFNKIVNKNNVRCLQHFYGPNHEHCFNRTLLRNSSGCEARRDEYRTEFTTALQRVDLFMGQFSEVLLTSISTFIKGDLTIANLGTSEGRFMQVVVSRSGPSTPHVNFLLDSHPVSPEVIVEHTLNQNGYTLVITGKKITKIPLNGLG |
| 2003. | human c-MET extracellular domain | human | aa | ECKEALAKSEMNVNMKYQLPNFTAETPIQNVILHEHHIFLGATNYIYVLNEEDLQKVAEYKTGPVLEHPDCFPCQDCSSKANLSGGVWKDNINMALVVDTYYDDQLISCGSVNRGTCQRHVFPHNHTADIQSEVHCIFSPQIEEPSQCPDCVVSALGAKVLSSVKDRFINFFVGNTINSSYFPDHPLHSISVRRLKETKDGFMFLTDQSYIDVLPEFRDSYPIKYVHAFESNNFIYFLTVQRETLDAQTFHTRIIRFCSINSGLHSYMEMPLECILTEKRKKRSTKKEVFNILQAAYVSKPGAQLARQIGASLNDDILFGVFAQSKPDSAEPMDRSAMCAFPIKYVNDFFNKIVNKNNVRCLQHFYGPNHEHCFNRTLLRNSSGCEARRDEYRTEFTTALQRVDLFMGQFSEVLLTSISTFIKGDLTIANLGTSEGRFMQVVVSRSGPSTPHVNFLLDSHPVSPEVIVEHTLNQNGYTLVITGKKITKIPLNGLGCRHFQSCSQCLSAPPFVQCGWCHDKCVRSEECLSGTWTQQICLPAIYKVFPNSAPLEGGTRLTICGWDFGFRRNNKFDLKKTRVLLGNESCTLTLSESTMNTLKCTVGPAMNKHFNMSIIISNGHGTTQYSTFSYVDPVITSISPKYGPMAGGTLLTLTGNYLNSGNSRHISIGGKTCTLKSVSNSILECYTPAQTISTEFAVKLKIDLANRETSIFSYREDPIVYEIHPTKSFISGGSTITGVGKNLNSVSVPRMVINVHEAGRNFTVACQHRSNSEIICCTTPSLQQLNLQLPLKTKAFFMLDGILSKYFDLIYVHNPVFKPFEKPVMISMGNENVLEIKGNDIDPEAVKGEVLKVGNKSCENIHLHSEAVLCTVPNDLLKLNSELNIEWKQAISSTVLGKVIVQPDQNF |
| 2004. | human Endosialin extracellular mucin domain | human | aa | ASQDLGDELLDDGEDEEDEDEAWKAFNGGWTEMPGILWMEPTQPPDFALAYRPSFPEDREPQIPYPEPTWPPPLSAPRVPYHSSVLSVTRPVVVSATHPTLPSAHQPPVIPATHPALSRDHQIPVIAANYPDLPSAYQPGILSVSHSAQPPAHQPPMISTKYPELFPAHQSPMFPDTRVAGTQTTTHLPGIPPNHAPLVTTLGAQLPPQAPDALVLRTQATQLPIIPTAQPSLTTTSRSPVSPAHQISVPAATQPAALPTLLPSQSPTNQTSPISPTHPHSKAPQIPREDGPSPKLALWLPSPAPTAAPTALGEAGLAEHSQRDDR |
| 2005. | human Endosialin extracellular three EGF-like domains | human | aa | CSPDNGGCEHECVEEVDGHVSCRCTEGFRLAADGRSCEDPCAQAPCEQQCEPGGPQGYSCHCRLGFRPAEDDPHRCVDTDECQIAGVCQQMCVNYVGGFECYCSEGHELEADGISC |
| 2006. | human Endosialin extracellular Sushi/SCR/CCP | human | aa | PAVYTTPFHLVSTEFEWLPFGSVAAVQCQAGRGASLLCVKQPEGGVGWSRAGPLC |

| | | | | |
|---|---|---|---|---|
| | domain | | | |
| 2007. | human Endosialin extracellular domain | human | aa | WAAEPRAACGPSSCYALFPRRRTFLEAWRACRELGGDLATPRTPEEAQRVDSLVGAGPASRLLWI<br>GLQRQARQCQLQRPLRGFTWTTGDQDTAFTNWAQPASGGPCPAQRCVALEASGEHRWLEGSCTLA<br>VDGYLCQFGFEGACPALQDEAGQAGPAVYTTPFHLVSTEFEWLPFGSVAAVQCQAGRGASLLCVK<br>QPEGGVGWSRAGPLCLGTGCSPDNGGCEHECVEEVDGHVSCRCTEGFRLAADGRSCEDPCAQAPC<br>EQQCEPGGPQGYSCHCRLGFRPAEDDPHRCVDTDECQIAGVCQQMCVNYVGGFECYCSEGHELEA<br>DGISCSPAGAMGAQASQDLGDELLDDGEDEEDEDEAWKAFNGGWTEMPGILWMEPTQPPDFALAY<br>RPSFPEDREPQIPYPEPTWPPPLSAPRVPYHSSVLSVTRPVVVSATHPTLPSAHQPPVIPATHPA<br>LSRDHQIPVIAANYPDLPSAYQPGILSVSHSAQPPAHQPPMISTKYPELFPAHQSPMFPDTRVAG<br>TQTTTHLPGIPPNHAPLVTTLGAQLPPQAPDALVLRTQATQLPIIPTAQPSLTTTSRSPVSPAHQ<br>ISVPAATQPAALPTLLPSQSPTNQTSPISPTHPHSKAPQIPREDGPSPKLALWLPSPAPTAAPTA<br>LGEAGLAEHSQRDDR |
| 2008. | human IGF-R1 extracellular three fibronectin type III domains | human | aa | SDVLHFTSTTTSKNRIIITWHRYRPPDYRDLISFTVYYKEAPFKNVTEYDGQDACGSNSWNMVDV<br>DLPPNKDVEPGILLHGLKPWTQYAVYVKAVTLTMVENDHIRGAKSEILYIRTNASVPSIPLDVLS<br>ASNSSSQLIVKWNPPSLPNGNLSYYIVRWQRQPQDGYLYRHNYCSKDKIPIRKYADGTIDIEEVT<br>ENPKTEVCGGEKGPCCACPKTEAEKQAEKEEAEYRKVFENFLHNSIFVPRPERKRRDVMQVANTT<br>MSSRSRNTTAADTYNITDPEELETEYPFFESRVDNKERTVISNLRPFTLYRIDIHSCNHEAEKLG<br>CSASNFVFARTMPAEGADDIPGPVTWEPRPENSIFLKWPEPENPNGLILMYEIKYGSQVEDQREC<br>VSRQEYRKYGGAKLNRLNPGNYTARIQATSLSGNGSWTDPVFFYVQAKTGYENFIHL |
| 2009. | human IGF-R1 extracellular L2 domain | human | aa | KVCEEEKKTKTIDSVTSAQMLQGCTIFKGNLLINIRRGNNIASELENFMGLIEVVTGYVKIRHSH<br>ALVSLSFLKNLRLILGEEQLEGNYSFYVLDNQNLQQLWDWDHRNLTIKAGKMYFAFNPKLCVSEI<br>YRMEEVTGTKGRQSKGDINTRNNGERASCE |
| 2010. | human IGF-R1 extracellular domain | human | aa | EICGPGIDIRNDYQQLKRLENCTVIEGYLHILLISKAEDYRSYRFPKLTVITEYLLLFRVAGLES<br>LGDLFPNLTVIRGWKLFYNYALVIFEMTNLKDIGLYNLRNITRGAIRIEKNADLCYLSTVDWSLI<br>LDAVSNNYIVGNKPPKECGDLCPGTMEEKPMCEKTTINNEYNYRCWTTNRCQKMCPSTCGKRACT<br>ENNECCHPECLGSCSAPDNDTACVACRHYYYAGVCVPACPPNTYRFEGWRCVDRDFCANILSAES<br>SDSEGFVIHDGECMQECPSGFIRNGSQSMYCIPCEGPCPKVCEEEKKTKTIDSVTSAQMLQGCTI<br>FKGNLLINIRRGNNIASELENFMGLIEVVTGYVKIRHSHALVSLSFLKNLRLILGEEQLEGNYSF<br>YVLDNQNLQQLWDWDHRNLTIKAGKMYFAFNPKLCVSEIYRMEEVTGTKGRQSKGDINTRNNGER<br>ASCESDVLHFTSTTTSKNRIIITWHRYRPPDYRDLISFTVYYKEAPFKNVTEYDGQDACGSNSWN<br>MVDVDLPPNKDVEPGILLHGLKPWTQYAVYVKAVTLTMVENDHIRGAKSEILYIRTNASVPSIPL<br>DVLSASNSSSQLIVKWNPPSLPNGNLSYYIVRWQRQPQDGYLYRHNYCSKDKIPIRKYADGTIDI<br>EEVTENPKTEVCGGEKGPCCACPKTEAEKQAEKEEAEYRKVFENFLHNSIFVPRPERKRRDVMQV<br>ANTTMSSRSRNTTAADTYNITDPEELETEYPFFESRVDNKERTVISNLRPFTLYRIDIHSCNHEA<br>EKLGCSASNFVFARTMPAEGADDIPGPVTWEPRPENSIFLKWPEPENPNGLILMYEIKYGSQVED<br>QRECVSRQEYRKYGGAKLNRLNPGNYTARIQATSLSGNGSWTDPVFFYVQAKTGYENFIHL |
| 2011. | IGF-1R-1 | artificial | nt | ATGAAGTCTGGCTCCGGCGGAGGGTCCCCGACCTCGCTGTGGGGGCTCCTGTTTCTCTCCGCCGC |

GCTCTCGCTCTGGCCGACGAGTGGAGAAATCTGCGGGCCAGGCATCGACATCCGCAACGACTATC
AGCAGCTGAAGCGCCTGGAGAACTGCACGGTGATCGAGGGCTACCTCCACATCCTGCTCATCTCC
AAGGCCGAGGACTACCGCAGCTACCGCTTCCCCAAGCTCACGGTCATTACCGAGTACTTGCTGCT
GTTCCGAGTGGCTGGCCTCGAGAGCCTCGGAGACCTCTTCCCCAACCTCACGGTCATCCGCGGCT
GGAAACTCTTCTACAACTACGCCCTGGTCATCTTCGAGATGACCAATCTCAAGGATATTGGGCTT
TACAACCTGAGGAACATTACTCGGGGGGCCATCAGGATTGAGAAAAATGCTGACCTCTGTTACCT
CTCCACTGTGGACTGGTCCCTGATCCTGGATGCGGTGTCCAATAACTACATTGTGGGGAATAAGC
CCCCAAAGGAATGTGGGGACCTGTGTCCAGGGACCATGGAGGAGAAGCCGATGTGTGAGAAGACC
ACCATCAACAATGAGTACAACTACCGCTGCTGGACCACAAACCGCTGCCAGAAAATGTGCCCAAG
CACGTGTGGGAAGCGGGCGTGCACCGAGAACAATGAGTGCTGCCACCCCGAGTGCCTGGGCAGCT
GCAGCGCGCCTGACAACGACACGGCCTGTGTAGCTTGCCGCCACTACTACTATGCCGGTGTCTGT
GTGCCTGCCTGCCCGCCCAACACCTACAGGTTTGAGGGCTGGCGCTGTGTGGACCGTGACTTCTG
CGCCAACATCCTCAGCGCCGAGAGCAGCGACTCCGAGGGGTTTGTGATCCACGACGGCGAGTGCA
TGCAGGAGTGCCCCTCGGGCTTCATCCGCAACGGCAGCCAGAGCATGTACTGCATCCCTTGTGAA
GGTCCTTGCCCGAAGGTCTGTGAGGAAGAAAAGAAAACAAAGACCATTGATTCTGTTACTTCTGC
TCAGATGCTCCAAGGATGCACCATCTTCAAGGGCAATTTGCTCATTAACATCCGACGGGGGAATA
ACATTGCTTCAGAGCTGGAGAACTTCATGGGGCTCATCGAGGTGGTGACGGGCTACGTGAAGATC
CGCCATTCTCATGCCTTGGTCTCCTTGTCCTTCCTAAAAAACCTTCGCCTCATCCTAGGAGAGGA
GCAGCTAGAAGGGAATTACTCCTTCTACGTCCTCGACAACCAGAACTTGCAGCAACTGTGGGACT
GGGACCACCGCAACCTGACCATCAAAGCAGGGAAAATGTACTTTGCTTTCAATCCCAAATTATGT
GTTTCCGAAATTTACCGCATGGAGGAAGTGACGGGGACTAAAGGGCGCCAAAGCAAAGGGGACAT
AAACACCAGGAACAACGGGGAGAGAGCCTCCTGTGAAAGTGACGTCCTGCATTTCACCTCCACCA
CCACGTCGAAGAATCGCATCATCATAACCTGGCACCGGTACCGGCCCCCTGACTACAGGGATCTC
ATCAGCTTCACCGTTTACTACAAGGAAGCACCCTTTAAGAATGTCACAGAGTATGATGGGCAGGA
TGCCTGCGGCTCCAACAGCTGGAACATGGTGGACGTGGACCTCCCGCCCAACAAGGACGTGGAGC
CCGGCATCTTACTACATGGGCTGAAGCCCTGGACTCAGTACGCCGTTTACGTCAAGGCTGTGACC
CTCACCATGGTGGAGAACGACCATATCCGTGGGGCCAAGAGTGAGATCTTGTACATTCGCACCAA
TGCTTCAGTTCCTTCCATTCCCTTGGACGTTCTTTCAGCATCGAACTCCTCTTCTCAGTTAATCG
TGAAGTGGAACCCTCCCTCTCTGCCCAACGGCAACCTGAGTTACTACATTGTGCGCTGGCAGCGG
CAGCCTCAGGACGGCTACCTTTACCGGCACAATTACTGCTCCAAAGACAAAATCCCCATCAGGAA
GTATGCCGACGGCACCATCGACATTGAGGAGGTCACAGAGAACCCCAAGACTGAGGTGTGTGGTG
GGGAGAAAGGGCCTTGCTGCGCCTGCCCCAAAACTGAAGCCGAGAAGCAGGCCGAGAAGGAGGAG
GCTGAATACCGCAAAGTCTTTGAGAATTTCCTGCACAACTCCATCTTCGTGCCCAGACCTGAAAG
GAAGCGGAGAGATGTCATGCAAGTGGCCAACACCACCATGTCCAGCCGAAGCAGGAACACCACGG
CCGCAGACACCTACAACATCACCGACCCGGAAGAGCTGGAGACAGAGTACCCTTTCTTTGAGAGC
AGAGTGGATAACAAGGAGAGAACTGTCATTTCTAACCTTCGGCCTTTCACATTGTACCGCATCGA
TATCCACAGCTGCAACCACGAGGCTGAGAAGCTGGGCTGCAGCGCCTCCAACTTCGTCTTTGCAA

| | | | | |
|---|---|---|---|---|
| | | | | GGACTATGCCCGCAGAAGGAGCAGATGACATTCCTGGGCCAGTGACCTGGGAGCCAAGGCCTGAA<br>AACTCCATCTTTTTAAAGTGGCCGGAACCTGAGAATCCCAATGGATTGATTCTAATGTATGAAAT<br>AAAATACGGATCACAAGTTGAGGATCAGCGAGAATGTGTGTCCAGACAGGAATACAGGAAGTATG<br>GAGGGGCCAAGCTAAACCGGCTAAACCCGGGGAACTACACAGCCCGGATTCAGGCCACATCTCTC<br>TCTGGGAATGGGTCGTGGACAGATCCTGTGTTCTTCTATGTCCAGGCCAAAACAGGATATGAAAA<br>CTTCATCCATCTGATCATCGCTCTGCCCGTCGCTGTCCTGTTGATCGTGGGAGGGTTGGTGATTA<br>TGCTGTACGTCTTCCATAGAAAGAGAAATAACAGCAGGCTGGGGAATGGAGTGCTGTATGCCTCT<br>GTGAACCCGGAGTACTTCAGCGCTGCTGATGTGTACGTTCCTGATGAGTGGGAGGTGGCTCGGGA<br>GAAGATCACCATGAGCCGGGAACTTGGGCAGGGGTCGTTTGGGATGGTCTATGAAGGAGTTGCCA<br>AGGGTGTGGTGAAAGATGAACCTGAAACCAGAGTGGCCATTAAAACAGTGAACGAGGCCGCAAGC<br>ATGCGTGAGAGGATTGAGTTTCTCAACGAAGCTTCTGTGATGAAGGAGTTCAATTGTCACCATGT<br>GGTGCGATTGCTGGGTGTGGTGTCCCAAGGCCAGCCAACACTGGTCATCATGGAACTGATGACAC<br>GGGGCGATCTCAAAAGTTATCTCCGGTCTCTGAGGCCAGAAATGGAGAATAATCCAGTCCTAGCA<br>CCTCCAAGCCTGAGCAAGATGATTCAGATGGCCGGAGAGATTGCAGACGGCATGGCATACCTCAA<br>CGCCAATAAGTTCGTCCACAGAGACCTTGCTGCCCGGAATTGCATGGTAGCCGAAGATTTCACAG<br>TCAAAATCGGAGATTTTGGTATGACGCGAGATATCTATGAGACAGACTATTACCGGAAAGGAGGG<br>AAAGGGCTGCTGCCCGTGCGCTGGATGTCTCCTGAGTCCCTCAAGGATGGAGTCTTCACCACTTA<br>CTCGGACGTCTGGTCCTTCGGGGTCGTCCTCTGGGAGATCGCCACACTGGCCGAGCAGCCCTACC<br>AGGGCTTGTCCAACGAGCAAGTCCTTCGCTTCGTCATGGAGGGCGGCCTTCTGGACAAGCCAGAC<br>AACTGTCCTGACATGCTGTTTGAACTGATGCGCATGTGCTGGCAGTATAACCCCAAGATGAGGCC<br>TTCCTTCCTGGAGATCATCAGCAGCATCAAAGAGGAGATGGAGCCTGGCTTCCGGGAGGTCTCCT<br>TCTACTACAGCGAGGAGAACAAGCTGCCCGAGCCGGAGGAGCTGGACCTGGAGCCAGAGAACATG<br>GAGAGCGTCCCCCTGGACCCCTCGGCCTCCTCGTCCTCCCTGCCACTGCCCGACAGACACTCAGG<br>ACACAAGGCCGAGAACGGCCCCGGCCCTGGGGTGCTGGTCCTCCGCGCCAGCTTCGACGAGAGAC<br>AGCCTTACGCCCACATGAACGGGGGCCGCAAGAACGAGCGGGCCTTGCCGCTGCCCCAGTCTTCG<br>ACCTGCTGA |
| 2012. | IGF-1R-2 | artificial | aa | MKSGSGGGSPTSLWGLLFLSAALSLWPTSGEICGPGIDIRNDYQQLKRLENCTVIEGYLHILLIS<br>KAEDYRSYRFPKLTVITEYLLLFRVAGLESLGDLFPNLTVIRGWKLFYNYALVIFEMTNLKDIGL<br>YNLRNITRGAIRIEKNADLCYLSTVDWSLILDAVSNNYIVGNKPPKECGDLCPGTMEEKPMCEKT<br>TINNEYNYRCWTTNRCQKMCPSTCGKRACTENNECCHPECLGSCSAPDNDTACVACRHYYYAGVC<br>VPACPPNTYRFEGWRCVDRDFCANILSAESSDSEGFVIHDGECMQECPSGFIRNGSQSMYCIPCE<br>GPCPKVCEEEKKTKTIDSVTSAQMLQGCTIFKGNLLINIRRGNNIASELENFMGLIEVVTGYVKI<br>RHSHALVSLSFLKNLRLILGEEQLEGNYSFYVLDNQNLQQLWDWDHRNLTIKAGKMYFAFNPKLC<br>VSEIYRMEEVTGTKGRQSKGDINTRNNGERASCESDVLHFTSTTSKNRIIITWHRYRPPDYRDL<br>ISFTVYYKEAPFKNVTEYDGQDACGSNSWNMVDVDLPPNKDVEPGILLHGLKPWTQYAVYVKAVT<br>LTMVENDHIRGAKSEILYIRTNASVPSIPLDVLSASNSSSQLIVKWNPPSLPNGNLSYYIVRWQR<br>QPQDGYLYRHNYCSKDKIPIRKYADGTIDIEEVTENPKTEVCGGEKGPCCACPKTEAEKQAEKEE |

| | | | | |
|---|---|---|---|---|
| | | | | AEYRKVFENFLHNSIFVPRPERKRRDVMQVANTTMSSRSRNTTAADTYNITDPEELETEYPFFES<br>RVDNKERTVISNLRPFTLYRIDIHSCNHEAEKLGCSASNFVFARTMPAEGADDIPGPVTWEPRPE<br>NSIFLKWPEPENPNGLILMYEIKYGSQVEDQRECVSRQEYRKYGGAKLNRLNPGNYTARIQATSL<br>SGNGSWTDPVFFYVQAKTGYENFIHLIIALPVAVLLIVGGLVIMLYVFHRKRNNSRLGNGVLYAS<br>VNPEYFSAADVYVPDEWEVAREKITMSRELGQGSFGMVYEGVAKGVVKDEPETRVAIKTVNEAAS<br>MRERIEFLNEASVMKEFNCHHVVRLLGVVSQGQPTLVIMELMTRGDLKSYLRSLRPEMENNPVLA<br>PPSLSKMIQMAGEIADGMAYLNANKFVHRDLAARNCMVAEDFTVKIGDFGMTRDIYETDYYRKGG<br>KGLLPVRWMSPESLKDGVFTTYSDVWSFGVVLWEIATLAEQPYQGLSNEQVLRFVMEGGLLDKPD<br>NCPDMLFELMRMCWQYNPKMRPSFLEIISSIKEEMEPGFREVSFYYSEENKLPEPEELDLEPENM<br>ESVPLDPSASSSSLPLPDRHSGHKAENGPGPGVLVLRASFDERQPYAHMNGGRKNERALPLPQSS<br>TC |
| 2013. | IGF-1R-3 | artificial | nt | ATGAAGTCTGGCTCTGGAGAAGGGTCCCCGACCTCGCTGTGGGGGCTCCTGTTTCTCTCCGCCGC<br>GCTCTCGCTCTGGCCGACGAGTGGAGAAATCTGTGGGCCGGGCATCGACATCCGCAACGACTATC<br>AGCAGCTGAAGCGCCTGGAGAACTGCACGGTGATCGAGGGCTACCTCCACATCCTGCTCATCTCC<br>AAGGCCGAGGACTACCGCAGCTACCGCTTCCCCAAGCTCACGGTCATCACCGAGTACTTGCTGTT<br>GTTCCGAGTGGCTGGCCTAGAGAGCCTCGGAGACCTGTTCCCCAACCTCACGGTAATCCGCGGCT<br>GGAAACTCTTCTACAACTACGCACTAGTCATCTTTGAGATGACCAATCTCAAGGATATTGGGCTT<br>TACAACCTGAGGAACATTACTCGGGGGGCCATCAGGATTGAGAAAAATGCTGACCTCTGTTACCT<br>CTCCACTGTGGACTGGTCCCTGATCCTGGATGCAGTGTCCAATAACTACATTGTGGGGAATAAGC<br>CCCCAAAGGAATGCGGGGACCTGTGTCCGGGGACCATGGAGGAGAAGCCGATGTGCGAGAAGACC<br>ACCATCAACAATGAGTACAACTACCGCTGCTGGACCACAAACCGCTGCCAGAAAATGTGCCCGAG<br>TGCCTGTGGGAAGAGGGCATGCACCGAGAACAACGAGTGCTGCCACCCCGAGTGCCTGGGCAGCT<br>GCAGCGCGCCTGACAACGACACGGCCTGTGTAGCTTGCCGCCACTACTACTACGCCGGTGTCTGC<br>GTGCCTGCCTGCCCGCCCAACACCTACAGGTTTGAGGGCTGGCGCTGTGTGGACCGTGACTTCTG<br>CGCCAACATCCTCAGCGCCGAGAGCAGCGACTCCGAGGGTTTCGTGATCCACGACGGCGAGTGCA<br>TGCAGGAGTGCCCCTCAGGCTTCATCCGCAACGGCAGCCAGAGCATGTACTGCATCCCTTGTGAA<br>GGTCCTTGCCCCAAGGTCTGTGAGGAAGAAAAGAAAACAAAGACCATTGATTCTGTTACTTCTGC<br>TCAGATGCTCCAAGGATGCACCATCTTCAAGGGCAATTTGCTCATTAACATCCGACGGGGGAATA<br>ACATTGCTTCAGAACTGGAGAACTTCATGGGGCTCATCGAGGTGGTGACGGGCTACGTGAAGATC<br>CGCCATTCCCATGCCTTGGTCTCCTTGTCCTTCCTAAAAAACCTTCGCCTCATCTTAGGAGAGGA<br>GCAGCTAGAAGGGAATTACTCCTTCTACGTCCTCGACAACCAGAACTTGCAGCAACTATGGGACT<br>GGGACCACCGCAACCTGACCATCAAAGCAGGGAAAATGTACTTTGCTTTCAATCCCAAATTGTGT<br>GTTTCGGAAATTTACCGCATGGAGGAAGTGACGGGGACTAAAGGGCGCCAAAGCAAAGGGGACAT<br>AAACACCAGGAACAACGGGGAAAGAGCCTCCTGTGAAAGTGACGTCCTGCATTTCACCTCCACCA<br>CCACGTGGAAGAATCGCATCATCATAACCTGGCACCGGTACCGGCCCCCTGACTACAGGGATCTC<br>ATCAGCTTCACCGTTTACTACAAGGAAGCACCTTTTAAGAATGTCACGGAGTATGATGGGCAGGA |

TGCCTGCGGCTCCAACAGCTGGAACATGGTGGATGTGGACCTCCCGCCCAACAAGGACGTGGAGC
CCGGCATCTTACTGCATGGGCTGAAGCCCTGGACTCAGTACGCCGTTTACGTCAAGGCTGTGACC
CTCACCATGGTGGAGAACGACCATATCCGTGGGGCCAAGAGTGAGATCTTGTACATTCGCACCAA
TGCTTCAGTTCCTTCCATTCCCTTGGACGTTCTTTCAGCATCGAACTCCTCTTCTCAGTTAATCG
TGAAGTGGAACCCTCCCTCTCTGCCCAACGGCAACCTGAGTTACTACATTGTGCGCTGGCAGCGG
CAGCCTCAGGACGGCTACCTTTACCGGCACAATTACTGCTCCAAAGACAAAATCCCCATCAGGAA
GTATGCCGACGGCACCATTGACATTGAGGAGGTCACAGAGAACCCGAAGACTGAGGTGTGTGGTG
GAGAGAAAGGGCCTTGCTGCGCCTGCCCCAAAACTGAAGCTGAGAAGCAGGCCGAGAAGGAGGAG
GCTGAGTACCGCAAAGTCTTTGAGAATTTCCTGCACAACTCCATCTTTGTGCCCAGACCTGAAAG
GAAGCGGAGAGATGTCATGCAAGTGGCCAACACCACCATGTCCAGCCGAAGCAGGAACACCACGG
TGGCAGACACCTACAACATCACAGATCTGGAAGAGCTAGAGACAGAGTACCCTTTCTTTGAGAGC
AGAGTGGATAATAAGGAGAGAACTGTCATTTCTAACCTTCGGCCTTTCACATTGTACCGCATTGA
TATCCACAGCTGCAACCACGAGGCTGAGAAACTGGGCTGCAGCGCCTCCAACTTTGTCTTTGCAA
GGACTATGCCTGCAGAAGGAGCAGATGACATTCCTGGGCCAGTGACCTGGGAGCCAAGGCCTGAA
AACTCCATCTTTTTAAAGTGGCCAGAACCTGAGAATCCCAATGGATTGATTCTAATGTATGAAAT
AAAATACGGATCACAAGTTGAGGATCAGCGAGAATGTGTGTCCAGACAGGAATACAGGAAGTATG
GAGGGGCCAAGCTAAACCGGCTAAACCCCGGGAACTACACAGCCCGGATTCAGGCTACATCTCTC
TCTGGGAATGGGTCGTGGACAGATCCTGTGTTCTTCTATGTCCAGGCCAAAACAGGATATGAAAA
CTTCATCCATCTGATCATCGCTCTGCCCGTCGCTGTCCTGTTGATCGTGGGAGGGTTGGTGATCA
TGCTGTACGTCTTCCATAGAAAGAGAAATAACAGCAGGCTGGGGAATGGAGTGCTGTACGCGTCT
GTGAACCCGGAGTACTTCAGCGCTGCGGATGTGTACGTTCCTGATGAGTGGGAGGTGGCTCGGGA
GAAGATCACCATGAGCCGGGAACTTGGGCAGGGGTCGTTTGGGATGGTCTATGAAGGAGTTGCCA
AGGGTGTGGTGAAAGACGAACCTGAAACCAGAGTGGCCATTAAAACAGTGAACGAGGCCGCGAGC
ATGCGTGAAAGGATCGAGTTTCTCAACGAGGCTTCTGTGATGAAGGAGTTCAATTGTCACCATGT
GGTGCGGTTGCTGGGTGTGGTGTCCCAGGGCCAGCCAACGCTGGTCATCATGGAACTGATGACGC
GGGGCGATCTCAAAAGTTATCTCCGGTCTCTGAGGCCAGAAATGGAGAATAATCCAGTCCTAGCA
CCTCCAAGCCTAAGCAAGATGATTCAGATGGCTGGAGAGATTGCAGACGGCATGGCATACCTCAA
CGCCAACAAGTTCGTCCACAGAGACCTTGCTGCCCGGAATTGCATGGTAGCCGAGGATTTCACAG
TCAAAATTGGAGATTTTGGGATGACGCGAGATATCTATGAGACAGACTATTACCGGAAAGGAGGG
AAAGGGCTGTTGCCCGTGCGCTGGATGTCTCCCGAGTCCCTCAAGGATGGAGTCTTCACCACTTA
CTCGGACGTCTGGTCCTTCGGGGTTGTCCTCTGGGAGATCGCCACACTGGCCGAGCAGCCCTACC
AGGGCTTGTCCAACGAGCAAGTCCTTCGCTTCGTCATGGAGGGCGGCCTTCTGGACAAGCCAGAC
AACTGCCCCGACATGCTGTTTGAATTGATGCGCATGTGCTGGCAGTACAACCCCAAGATGAGGCC
TTCCTTCCTGGAGATCATCAGCAGCATCAAAGACGAGATGGAGCCTGGCTTCCGGGAGGTCTCCT
TCTACTACAGTGAGGAGAACAAGCTGCCCGAGCCGGAGGAGCTGGACCTGGAGCCAGAGAACATG
GAGAGCGTCCCCCTGGACCCCTCGGCCTCCTCGTCCTCCCTGCCACTGCCCGACAGACACTCAGG
ACACAAGGCCGAGAACGGCCCCGGCCCTGGAGTGCTGGTGCTCCGCGCCAGCTTCGATGAGAGAC

| | | | | |
|---|---|---|---|---|
| | | | | AGCCTTACGCACACATGAACGGTGGCCGCAAGAACGAGCGGGCCTTGCCGCTGCCCCAGTCTTCGACCTGCTGA |
| 2014. | IGF-1R-4 | artificial | aa | MKSGSGEGSPTSLWGLLFLSAALSLWPTSGEICGPGIDIRNDYQQLKRLENCTVIEGYLHILLISKAEDYRSYRFPKLTVITEYLLLFRVAGLESLGDLFPNLTVIRGWKLFYNYALVIFEMTNLKDIGLYNLRNITRGAIRIEKNADLCYLSTVDWSLILDAVSNNYIVGNKPPKECGDLCPGTMEEKPMCEKTTINNEYNYRCWTTNRCQKMCPSACGKRACTENNECCHPECLGSCSAPDNDTACVACRHYYYAGVCVPACPPNTYRFEGWRCVDRDFCANILSAESSDSEGFVIHDGECMQECPSGFIRNGSQSMYCIPCEGPCPKVCEEEKKTKTIDSVTSAQMLQGCTIFKGNLLINIRRGNNIASELENFMGLIEVVTGYVKIRHSHALVSLSFLKNLRLILGEEQLEGNYSFYVLDNQNLQQLWDWDHRNLTIKAGKMYFAFNPKLCVSEIYRMEEVTGTKGRQSKGDINTRNNGERASCESDVLHFTSTTTWKNRIIITWHRYRPPDYRDLISFTVYYKEAPFKNVTEYDGQDACGSNSWNMVDVDLPPNKDVEPGILLHGLKPWTQYAVYVKAVTLTMVENDHIRGAKSEILYIRTNASVPSIPLDVLSASNSSSQLIVKWNPPSLPNGNLSYYIVRWQRQPQDGYLYRHNYCSKDKIPIRKYADGTIDIEEVTENPKTEVCGGEKGPCCACPKTEAEKQAEKEEAEYRKVFENFLHNSIFVPRPERKRRDVMQVANTTMSSRSRNTTVADTYNITDLEELETEYPFFESRVDNKERTVISNLRPFTLYRIDIHSCNHEAEKLGCSASNFVFARTMPAEGADDIPGPVTWEPRPENSIFLKWPEPENPNGLILMYEIKYGSQVEDQRECVSRQEYRKYGGAKLNRLNPGNYTARIQATSLSGNGSWTDPVFFYVQAKTGYENFIHLIIALPVAVLLIVGGLVIMLYVFHRKRNNSRLGNGVLYASVNPEYFSAADVYVPDEWEVAREKITMSRELGQGSFGMVYEGVAKGVVKDEPETRVAIKTVNEAASMRERIEFLNEASVMKEFNCHHVVRLLGVVSQGQPTLVIMELMTRGDLKSYLRSLRPEMENNPVLAPPSLSKMIQMAGEIADGMAYLNANKFVHRDLAARNCMVAEDFTVKIGDFGMTRDIYETDYYRKGGKGLLPVRWMSPESLKDGVFTTYSDVWSFGVVLWEIATLAEQPYQGLSNEQVLRFVMEGGLLDKPDNCPDMLFELMRMCWQYNPKMRPSFLEIISSIKDEMEPGFREVSFYYSEENKLPEPEELDLEPENMESVPLDPSASSSSLPLPDRHSGHKAENGPGPGVLVLRASFDERQPYAHMNGGRKNERALPLPQSSTC |
| 2015. | IGF1R2-H | artificial | aa | EVQLVQSGAEVKKPGSSVKVSCKASGGTFSSYAISWVRQAPGQGLEWMGGIIPIFGTANYAQKFQGRVTITADKSTSTAYMELSSLRSEDTAVYYCARAPLRFLEWSTQDHYYYYYMDVWGKGTTVTVSS |
| 2016. | IGF1R2-HCDR1 | artificial | aa | SYAIS |
| 2017. | IGF1R2-HCDR2 | artificial | aa | GIIPIFGTANYAQKFQG |
| 2018. | IGF1R2-HCDR3 | artificial | aa | APLRFLEWSTQDHYYYYYMDV |
| 2019. | IGF1R2-H | artificial | nt | GAGGTGCAACTAGTCCAGAGCGGAGCGGAGGTGAAGAAACCTGGATCCAGCGTGAAAGTGAGCTGTAAGGCTAGCGGAGGCACATTCTCCTCGTATGCCATTAGCTGGGTTAGACAAGCACCTGGACAAGGACTGGAGTGGATGGGAGGGATAATCCCAATCTTCGGAACTGCCAACTACGCACAGAAGTTCCAAGGACGGGTCACCATCACTGCGGATAAGTCCACCTCGACTGCCTACATGGAGCTCAGTAGCTTACGGAGCGAAGACACAGCCGTATACTATTGCGCACGAGCACCCCTAAGATTCCTGGAGTGGAGCACGCAGGATCACTATTACTATTACTACATGGACGTGTGGGGAAAGGGAACGACTGTCACCGTTTCAAGC |

| | | | | |
|---|---|---|---|---|
| 2020. | IGF1R2-L | artificial | aa | SSELTQDPAVSVALGQTVRITCQGDSLRSYYATWYQQKPGQAPILVIYGENKRPSGIPDRFSGSSSGNTASLTITGAQAEDEADYYCKSRDGSGQHLVFGGGTKLTVL |
| 2021. | IGF1R2-LCDR1 | artificial | aa | QGDSLRSYYAT |
| 2022. | IGF1R2-LCDR2 | artificial | aa | GENKRPS |
| 2023. | IGF1R2-LCDR3 | artificial | aa | KSRDGSGQHLV |
| 2024. | IGF1R2-L | artificial | nt | AGCTCGGAATTGACCCAGGATCCTGCTGTATCCGTCGCTCTCGGACAAACCGTGCGCATAACATGTCAAGGGGACTCCCTGAGGAGCTACTACGCCACATGGTACCAGCAAAAACCCGGACAAGCTCCAATCCTGGTGATTTATGGCGAAAACAAGCGTCCCTCAGGGATCCCAGACAGGTTTAGCGGAAGTTCGAGCGGCAATACTGCCAGTCTGACGATTACCGGAGCTCAAGCTGAGGATGAAGCCGACTACTATTGCAAATCACGCGACGGAAGCGGCCAGCACCTGGTGTTTGGTGGCGGGACAAAGCTGACCGTTTTG |
| 2025. | IGF1R2-HL | artificial | aa | EVQLVQSGAEVKKPGSSVKVSCKASGGTFSSYAISWVRQAPGQGLEWMGGIIPIFGTANYAQKFQGRVTITADKSTSTAYMELSSLRSEDTAVYYCARAPLRFLEWSTQDHYYYYYMDVWGKGTTVTVSSGGGGSGGGGSGGGGSSSELTQDPAVSVALGQTVRITCQGDSLRSYYATWYQQKPGQAPILVIYGENKRPSGIPDRFSGSSSGNTASLTITGAQAEDEADYYCKSRDGSGQHLVFGGGTKLTVL |
| 2026. | IGF1R2-HL | artificial | nt | GAGGTGCAACTAGTCCAGAGCGGAGCGGAGGTGAAGAAACCTGGATCCAGCGTGAAAGTGAGCTGTAAGGCTAGCGGAGGCACATTCTCCTCGTATGCCATTAGCTGGGTTAGACAAGCACCTGGACAAGGACTGGAGTGGATGGGAGGGATAATCCCAATCTTCGGAACTGCCAACTACGCACAGAAGTTCCAAGGACGGGTCACCATCACTGCGGATAAGTCCACCTCGACTGCCTACATGGAGCTCAGTAGCTTACGGAGCGAAGACACAGCCGTATACTATTGCGCACGAGCACCCCTAAGATTCCTGGAGTGGAGCACGCAGGATCACTATTACTATTACTACATGGACGTGTGGGGAAAGGGAACGACTGTCACCGTTTCAAGCGGTGGAGGTGGAAGTGGTGGAGGAGGGAGCGGTGGGGGAGGATCAAGCTCGGAATTGACCCAGGATCCTGCTGTATCCGTCGCTCTCGGACAAACCGTGCGCATAACATGTCAAGGGGACTCCCTGAGGAGCTACTACGCCACATGGTACCAGCAAAAACCCGGACAAGCTCCAATCCTGGTGATTTATGGCGAAAACAAGCGTCCCTCAGGGATCCCAGACAGGTTTAGCGGAAGTTCGAGCGGCAATACTGCCAGTCTGACGATTACCGGAGCTCAAGCTGAGGATGAAGCCGACTACTATTGCAAATCACGCGACGGAAGCGGCCAGCACCTGGTGTTTGGTGGCGGGACAAAGCTGACCGTTTTG |
| 2027. | IGF1R2HLxI2CHL | artificial | aa | EVQLVQSGAEVKKPGSSVKVSCKASGGTFSSYAISWVRQAPGQGLEWMGGIIPIFGTANYAQKFQGRVTITADKSTSTAYMELSSLRSEDTAVYYCARAPLRFLEWSTQDHYYYYYMDVWGKGTTVTVSSGGGGSGGGGSGGGGSSSELTQDPAVSVALGQTVRITCQGDSLRSYYATWYQQKPGQAPILVIYGENKRPSGIPDRFSGSSSGNTASLTITGAQAEDEADYYCKSRDGSGQHLVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMN |

| | | | | |
|---|---|---|---|---|
| | | | | NLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2028. | IGF1R2HLxI2CHL | artificial | nt | GAGGTGCAACTAGTCCAGAGCGGAGCGGAGGTGAAGAAACCTGGATCCAGCGTGAAAGTGAGCTGTAAGGCTAGCGGAGGCACATTCTCCTCGTATGCCATTAGCTGGGTTAGACAAGCACCTGGACAAGGACTGGAGTGGATGGGAGGGATAATCCCAATCTTCGGAACTGCCAACTACGCACAGAAGTTCCAAGGACGGGTCACCATCACTGCGGATAAGTCCACCTCGACTGCCTACATGGAGCTCAGTAGCTTACGGAGCGAAGACACAGCCGTATACTATTGCGCACGAGCACCCCTAAGATTCCTGGAGTGGAGCACGCAGGATCACTATTACTATTACTACATGGACGTGTGGGGAAAGGGAACGACTGTCACCGTTTCAAGCGGTGGAGGTGGAAGTGGTGGAGGAGGGAGCGGTGGGGGAGGATCAAGCTCGGAATTGACCCAGGATCCTGCTGTATCCGTCGCTCTCGGACAAACCGTGCGCATAACATGTCAAGGGGACTCCCTGAGGAGCTACTACGCCACATGGTACCAGCAAAAACCCGGACAAGCTCCAATCCTGGTGATTTATGGCGAAAACAAGCGTCCCTCAGGGATCCCAGACAGGTTTAGCGGAAGTTCGAGCGGCAATACTGCCAGTCTGACGATTACCGGAGCTCAAGCTGAGGATGAAGCCGACTACTATTGCAAATCACGCGACGGAAGCGGCCAGCACCTGGTGTTTGGTGGCGGGACAAAGCTGACCGTTTTGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2029. | IGF1R7-H | artificial | aa | QVELVESGGGVVQPGRSQRLSCAASGFTFSSYGMHWVRQAPGKGLEWVAIIWFDGSSTYYADSVRGRFTISRDNSKNTLYLQMNSLRAEDTAVYFCARELGRRYFDLWGRGTLVSVSS |
| 2030. | IGF1R7-HCDR1 | artificial | aa | SYGMH |
| 2031. | IGF1R7-HCDR2 | artificial | aa | IIWFDGSSTYYADSVRG |

| | | | | |
|---|---|---|---|---|
| 2032. | IGF1R7-HCDR3 | artificial | aa | ELGRRYFDL |
| 2033. | IGF1R7-H | artificial | nt | CAGGTCGAATTGGTGGAAAGCGGGGGAGGCGTCGTTCAGCCTGGTAGGAGCCAGAGGCTTAGCTGTGCTGCCTCCGGTTTCACCTTCTCTAGCTACGGAATGCACTGGGTGAGACAGGCACCCGGAAAAGGACTGGAATGGGTGGCCATCATCTGGTTCGACGGCTCTTCCACCTACTATGCCGATAGCGTGAGAGGACGCTTCACCATCTCCCGTGACAACTCCAAGAACACCCTGTACCTCCAGATGAATAGCCTGCGAGCTGAGGACACTGCCGTGTACTTTTGTGCACGGGAGCTGGGACGTAGGTACTTTGATCTGTGGGGAAGAGGCACACTGGTTAGCGTTAGCTCC |
| 2034. | IGF1R7-L | artificial | aa | EIVLTQSPATLSLSPGERATLSCRASQSVSSYLAWYQQKPGQAPRLLIYDASKRAYGIPARFSGSGSGTDFTLTISSLEPEDFAVYYCQQRSKWPPWTFGQGTKVESK |
| 2035. | IGF1R7-LCDR1 | artificial | aa | RASQSVSSYLA |
| 2036. | IGF1R7-LCDR2 | artificial | aa | DASKRAY |
| 2037. | IGF1R7-LCDR3 | artificial | aa | QQRSKWPP |
| 2038. | IGF1R7-L | artificial | nt | GAAATCGTGCTCACCCAGAGCCCTGCCACATTGAGCCTTTCACCTGGCGAGCGAGCTACATTGTCATGCCGAGCTAGCCAGAGCGTGAGCTCTTACCTGGCATGGTATCAGCAGAAACCTGGGCAGGCTCCACGACTGCTCATCTATGACGCCTCCAAGCGAGCCTATGGGATTCCTGCAAGGTTTAGCGGAAGCGGAAGCGGAACCGATTTCACCCTGACTATCTCCTCACTCGAGCCTGAGGACTTTGCCGTGTATTACTGCCAGCAGCGGAGCAAGTGGCCTCCATGGACATTCGGCCAGGGTACCAAAGTGGAGAGCAAG |
| 2039. | IGF1R7-HL | artificial | aa | QVELVESGGGVVQPGRSQRLSCAASGFTFSSYGMHWVRQAPGKGLEWVAIIWFDGSSTYYADSVRGRFTISRDNSKNTLYLQMNSLRAEDTAVYFCARELGRRYFDLWGRGTLVSVSSGGGGSGGGGSGGGGSEIVLTQSPATLSLSPGERATLSCRASQSVSSYLAWYQQKPGQAPRLLIYDASKRAYGIPARFSGSGSGTDFTLTISSLEPEDFAVYYCQQRSKWPPWTFGQGTKVESK |
| 2040. | IGF1R7-HL | artificial | nt | CAGGTCGAATTGGTGGAAAGCGGGGGAGGCGTCGTTCAGCCTGGTAGGAGCCAGAGGCTTAGCTGTGCTGCCTCCGGTTTCACCTTCTCTAGCTACGGAATGCACTGGGTGAGACAGGCACCCGGAAAAGGACTGGAATGGGTGGCCATCATCTGGTTCGACGGCTCTTCCACCTACTATGCCGATAGCGTGAGAGGACGCTTCACCATCTCCCGTGACAACTCCAAGAACACCCTGTACCTCCAGATGAATAGCCTGCGAGCTGAGGACACTGCCGTGTACTTTTGTGCACGGGAGCTGGGACGTAGGTACTTTGATCTGTGGGGAAGAGGCACACTGGTTAGCGTTAGCTCCGGCGGAGGGGGAAGCGGAGGCGGAGGTAGCGGGGGAGGTGGAAGCGAAATCGTGCTCACCCAGAGCCCTGCCACATTGAGCCTTTCACCTGGCGAGCGAGCTACATTGTCATGCCGAGCTAGCCAGAGCGTGAGCTCTTACCTGGCATGGTATCAGCAGAAACCTGGGCAGGCTCCACGACTGCTCATCTATGACGCCTCCAAGCGAGCCTATGGGATTCCTGCAAGGTTTAGCGGAAGCGGAAGCGGAACCGATTTCACCCTGACTATCTCCTCACTCGAGCCTGAGGACTTTGCCGTGTATTACTGCCAG |

| | | | | |
|---|---|---|---|---|
| | | | | CAGCGGAGCAAGTGGCCTCCATGGACATTCGGCCAGGGTACCAAAGTGGAGAGCA<br>AG |
| 2041. | IGF1R7HLxI2CHL | artificial | aa | QVELVESGGGVVQPGRSQRLSCAASGFTFSSYGMHWVRQAPGKGLEWVAIIWFDGS<br>STYYADSVRGRFTISRDNSKNTLYLQMNSLRAEDTAVYFCARELGRRYFDLWGRGTLV<br>SVSSGGGGSGGGGSGGGGSEIVLTQSPATLSLSPGERATLSCRASQSVSSYLAWYQQ<br>KPGQAPRLLIYDASKRAYGIPARFSGSGSGTDFTLTISSLEPEDFAVYYCQQRSKWPPW<br>TFGQGTKVESKSGGGGSEVQLVESGGGLVQPGGSLKLSCAASGFTFNKYAMNWVRQ<br>APGKGLEWVARIRSKYNNYATYYADSVKDRFTISRDDSKNTAYLQMNNLKTEDTAVYY<br>CVRHGNFGNSYISYWAYWGQGTLVTVSSGGGGSGGGGSGGGGSQTVVTQEPSLTVS<br>PGGTVTLTCGSSTGAVTSGNYPNWVQQKPGQAPRGLIGGTKFLAPGTPARFSGSLLG<br>GKAALTLSGVQPEDEAEYYCVLWYSNRWVFGGGTKLTVL |
| 2042. | IGF1R7HLxI2CHL | artificial | nt | CAGGTCGAATTGGTGGAAAGCGGGGGAGGCGTCGTTCAGCCTGGTAGGAGCCAGA<br>GGCTTAGCTGTGCTGCCTCCGGTTTCACCTTCTCTAGCTACGGAATGCACTGGGTG<br>AGACAGGCACCCGGAAAAGGACTGGAATGGGTGGCCATCATCTGGTTCGACGGCT<br>CTTCCACCTACTATGCCGATAGCGTGAGAGGACGCTTCACCATCTCCCGTGACAAC<br>TCCAAGAACACCCTGTACCTCCAGATGAATAGCCTGCGAGCTGAGGACACTGCCGT<br>GTACTTTTGTGCACGGGAGCTGGGACGTAGGTACTTTGATCTGTGGGGAAGAGGCA<br>CACTGGTTAGCGTTAGCTCCGGCGGAGGGGGAAGCGGAGGCGGAGGTAGCGGGG<br>GAGGTGGAAGCGAAATCGTGCTCACCCAGAGCCCTGCCACATTGAGCCTTTCACCT<br>GGCGAGCGAGCTACATTGTCATGCCGAGCTAGCCAGAGCGTGAGCTCTTACCTGG<br>CATGGTATCAGCAGAAACCTGGGCAGGCTCCACGACTGCTCATCTATGACGCCTCC<br>AAGCGAGCCTATGGGATTCCTGCAAGGTTTAGCGGAAGCGGAAGCGGAACCGATT<br>TCACCCTGACTATCTCCTCACTCGAGCCTGAGGACTTTGCCGTGTATTACTGCCAG<br>CAGCGGAGCAAGTGGCCTCCATGGACATTCGGCCAGGGTACCAAAGTGGAGAGCA<br>AGTCCGGAGGTGGTGGATCCGAGGTGCAGCTGGTCGAGTCTGGAGGAGGATTGGT<br>GCAGCCTGGAGGGTCATTGAAACTCTCATGTGCAGCCTCTGGATTCACCTTCAATA<br>AGTACGCCATGAACTGGGTCCGCCAGGCTCCAGGAAAGGGTTTGGAATGGGTTGC<br>TCGCATAAGAAGTAAATATAATAATTATGCAACATATTATGCCGATTCAGTGAAAGAC<br>AGGTTCACCATCTCCAGAGATGATTCAAAAAACACTGCCTATCTACAAATGAACAAC<br>TTGAAGACTGAGGACACTGCCGTGTACTACTGTGTGAGACATGGGAACTTCGGTAA<br>TAGCTACATATCCTACTGGGCTTACTGGGGCCAAGGGACTCTGGTCACCGTCTCCT<br>CAGGTGGTGGTGGTTCTGGCGGCGGCGGCTCCGGTGGTGGTGGTTCTCAGACTGT<br>TGTGACTCAGGAACCTTCACTCACCGTATCACCTGGTGGAACAGTCACACTCACTT<br>GTGGCTCCTCGACTGGGGCTGTTACATCTGGCAACTACCCAAACTGGGTCCAACAA<br>AAACCAGGTCAGGCACCCCGTGGTCTAATAGGTGGGACTAAGTTCCTCGCCCCCG<br>GTACTCCTGCCAGATTCTCAGGCTCCCTGCTTGGAGGCAAGGCTGCCCTCACCCTC |

| | | | | |
|---|---|---|---|---|
| | | | | TCAGGGGTACAGCCAGAGGATGAGGCAGAATATTACTGTGTTCTATGGTACAGCAACCGCTGGGTGTTCGGTGGAGGAACCAAACTGACTGTCCTA |
| 2043. | IGF1R9-H | artificial | aa | EVQLLESGGGLVQPGGSLRLSCAASGFTFSIYRMQWVRQAPGKGLEWVSGISPSGGTTWYADSVKGRFTISRDNSKNTLYLQMNSLRAEDTAVYYCARWSGGSGYAFDIWGQGTMVTVSS |
| 2044. | IGF1R9-HCDR1 | artificial | aa | IYRMQ |
| 2045. | IGF1R9-HCDR2 | artificial | aa | GISPSGGTTWYADSVKG |
| 2046. | IGF1R9-HCDR3 | artificial | aa | WSGGSGYAFDI |
| 2047. | IGF1R9-H | artificial | nt | GAAGTTCAGCTGCTCGAGAGCGGAGGTGGACTTGTGCAGCCTGGGGGATCACTGAGATTGTCATGCGCAGCTAGCGGCTTTACCTTCTCCATCTATCGCATGCAGTGGGTTCGACAGGCACCCGGAAAGGGATTGGAATGGGTCTCCGGCATTAGCCCTAGCGGGGGAACAACCTGGTATGCCGATAGCGTGAAGGGCAGGTTCACCATCTCTAGGGACAACAGCAAAAATACTCTGTACCTGCAGATGAACTCCCTGCGTGCCGAGGATACTGCCGTCTACTACTGTGCTCGATGGTCCGGTGGCTCCGGCTATGCCTTCGATATCTGGGGACAGGGTACAATGGTGACTGTGAGTAGC |
| 2048. | IGF1R9-L | artificial | aa | DIQMTQSPLSLSASVGDRVTITCQASRDIRNYLNWYQQKPGKAPKLLIYDASSLQTGVPSRFGGSGSGTDFSFTIGSLQPEDIATYYCQQFDSLPHTFGQGTKLEIK |
| 2049. | IGF1R9-LCDR1 | artificial | aa | QASRDIRNYLN |
| 2050. | IGF1R9-LCDR2 | artificial | aa | DASSLQT |
| 2051. | IGF1R9-LCDR3 | artificial | aa | QQFDSLPHT |
| 2052. | IGF1R9-L | artificial | nt | GACATTCAGATGACCCAGAGCCCATTGTCACTGTCAGCTTCCGTGGGCGATCGGGTGACCATTACATGCCAGGCTTCACGCGACATCAGGAACTACCTGAACTGGTATCAGCAGAAGCCTGGCAAAGCCCCCAAGCTCCTTATCTACGACGCAAGCTCACTGCAGACTGGTGTTCCCTCAAGATTCGGTGGAAGCGGGAGTGGCACCGACTTTAGCTTTACCATCGGCTCTCTGCAGCCTGAGGACATCGCCACATATTACTGCCAGCAGTTTGACTCCCTGCCACACACATTTGGCCAGGGCACCAAACTGGAGATCAAG |
| 2053. | IGF1R9-HL | artificial | aa | EVQLLESGGGLVQPGGSLRLSCAASGFTFSIYRMQWVRQAPGKGLEWVSGISPSGGTTWYADSVKGRFTISRDNSKNTLYLQMNSLRAEDTAVYYCARWSGGSGYAFDIWGQGTMVTVSSGGGGSGGGGSGGGGSDIQMTQSPLSLSASVGDRVTITCQASRDIRNYLNWYQQKPGKAPKLLIYDASSLQTGVPSRFGGSGSGTDFSFTIGSLQPEDIATYYCQQFDSLPHTFGQGTKLEIK |
| 2054. | IGF1R9-HL | artificial | nt | GAAGTTCAGCTGCTCGAGAGCGGAGGTGGACTTGTGCAGCCTGGGGGATCACTGAGATTGTCATGCGCAGCTAGCGGCTTTACCTTCTCCATCTATCGCATGCAGTGGGTTCGACAGGCACCCGGAAAGGGATTGGAATGGGTCTCCGGCATTAGCCCTAGCGGGGGAACAACCTGGTATGCCGATAGCGTGAAGGGCAGGTTCACCATCTCTAGGGACAACAGCAAAAATACTCTGTACCTGCAGATGAACTCCCTGCGTGCCGAGGATACTGCCGT |

| | | | | |
|---|---|---|---|---|
| | | | | CTACTACTGTGCTCGATGGTCCGGTGGCTCCGGCTATGCCTTCGATATCTGGGGACAGGGTACAATGGTGACTGTGAGTAGCGGAGGGGGAGGTAGCGGAGGAGGTGGAAGTGGCGGAGGTGGAAGCGACATTCAGATGACCCAGAGCCCATTGTCACTGTCAGCTTCCGTGGGCGATCGGGTGACCATTACATGCCAGGCTTCACGCGACATCAGGAACTACCTGAACTGGTATCAGCAGAAGCCTGGCAAAGCCCCCAAGCTCCTTATCTACGACGCAAGCTCACTGCAGACTGGTGTTCCCTCAAGATTCGGTGGAAGCGGGAGTGGCACCGACTTTAGCTTTACCATCGGCTCTCTGCAGCCTGAGGACATCGCCACATATTACTGCCAGCAGTTTGACTCCCTGCCACACACATTTGGCCAGGGCACCAAACTGGAGATCAAG |
| 2055. | IGF1R9HLxI2CHL | artificial | aa | EVQLLESGGGLVQPGGSLRLSCAASGFTFSIYRMQWVRQAPGKGLEWVSGISPSGGTTWYADSVKGRFTISRDNSKNTLYLQMNSLRAEDTAVYYCARWSGGSGYAFDIWGQGTMVTVSSGGGGSGGGGSGGGGSDIQMTQSPLSLSASVGDRVTITCQASRDIRNYLNWYQQKPGKAPKLLIYDASSLQTGVPSRFGGSGSGTDFSFTIGSLQPEDIATYYCQQFDSLPHTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2056. | IGF1R9HLxI2CHL | artificial | nt | GAAGTTCAGCTGCTCGAGAGCGGAGGTGGACTTGTGCAGCCTGGGGGATCACTGAGATTGTCATGCGCAGCTAGCGGCTTTACCTTCTCCATCTATCGCATGCAGTGGGTTCGACAGGCACCCGGAAAGGGATTGGAATGGGTCTCCGGCATTAGCCCTAGCGGGGGAACAACCTGGTATGCCGATAGCGTGAAGGGCAGGTTCACCATCTCTAGGGACAACAGCAAAAATACTCTGTACCTGCAGATGAACTCCCTGCGTGCCGAGGATACTGCCGTCTACTACTGTGCTCGATGGTCCGGTGGCTCCGGCTATGCCTTCGATATCTGGGGACAGGGTACAATGGTGACTGTGAGTAGCGGAGGGGGAGGTAGCGGAGGAGGTGGAAGTGGCGGAGGTGGAAGCGACATTCAGATGACCCAGAGCCCATTGTCACTGTCAGCTTCCGTGGGCGATCGGGTGACCATTACATGCCAGGCTTCACGCGACATCAGGAACTACCTGAACTGGTATCAGCAGAAGCCTGGCAAAGCCCCCAAGCTCCTTATCTACGACGCAAGCTCACTGCAGACTGGTGTTCCCTCAAGATTCGGTGGAAGCGGGAGTGGCACCGACTTTAGCTTTACCATCGGCTCTCTGCAGCCTGAGGACATCGCCACATATTACTGCCAGCAGTTTGACTCCCTGCCACACACATTTGGCCAGGGCACCAAACTGGAGATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAAC |

| | | | | |
|---|---|---|---|---|
| | | | | TTGAAGACTGAGGACACTGCCGTGTACTACTGTGTGAGACATGGGAACTTCGGTAA<br>TAGCTACATATCCTACTGGGCTTACTGGGGCCAAGGGACTCTGGTCACCGTCTCCT<br>CAGGTGGTGGTGGTTCTGGCGGCGGCGGCTCCGGTGGTGGTGGTTCTCAGACTGT<br>TGTGACTCAGGAACCTTCACTCACCGTATCACCTGGTGGAACAGTCACACTCACTT<br>GTGGCTCCTCGACTGGGGCTGTTACATCTGGCAACTACCCAAACTGGGTCCAACAA<br>AAACCAGGTCAGGCACCCCGTGGTCTAATAGGTGGGACTAAGTTCCTCGCCCCCG<br>GTACTCCTGCCAGATTCTCAGGCTCCCTGCTTGGAGGCAAGGCTGCCCTCACCCTC<br>TCAGGGGTACAGCCAGAGGATGAGGCAGAATATTACTGTGTTCTATGGTACAGCAA<br>CCGCTGGGTGTTCGGTGGAGGAACCAAACTGACTGTCCTA |
| 2057. | IGF1R10-H | artificial | aa | EVQLLESGGGLVQPGGSLRLSCAASGFTFSNYHMAWVRQAPGKGLEWVSVISPTGGR<br>TTYADSVKGRFTISRDNSKNTLYLQMNSLRAEDTATYYCARAGYSYGYGYFDYWGQG<br>TLVTVSS |
| 2058. | IGF1R10-HCDR1 | artificial | aa | NYHMA |
| 2059. | IGF1R10-HCDR2 | artificial | aa | VISPTGGRTTYADSVKG |
| 2060. | IGF1R10-HCDR3 | artificial | aa | AGYSYGYGYFDY |
| 2061. | IGF1R10-H | artificial | nt | GAAGTGCAGCTCTTGGAGAGCGGAGGGGGACTTGTGCAGCCTGGCGGATCCTTGA<br>GACTTTCTTGCGCTGCCTCCGGTTTCACCTTCTCAAATTACCACATGGCCTGGGTGA<br>GACAGGCTCCTGGAAAAGGGCTCGAATGGGTTTCCGTGATTAGCCCAACCGGAGG<br>GAGGACCACATATGCCGATAGCGTCAAGGGGAGGTTTACAATCAGCCGTGACAACT<br>CCAAGAACACCCTGTACCTCCAGATGAACTCACTGCGAGCCGAGGATACTGCAACC<br>TACTACTGCGCTCGAGCCGGATACTCATACGGCTATGGCTACTTCGACTACTGGGG<br>ACAGGGAACACTGGTGACCGTTAGTAGC |
| 2062. | IGF1R10-L | artificial | aa | DIQMTQFPATLSVSPGERATLSCRASQSVMRNLAWYQQKPGQPPRLLIYGASKRATGI<br>PARFSGSGSGTAFTLTISNLEPEDFAVYYCHQRSTWPLGTFGPGTKLEAK |
| 2063. | IGF1R10-LCDR1 | artificial | aa | RASQSVMRNLA |
| 2064. | IGF1R10-LCDR2 | artificial | aa | GASKRAT |
| 2065. | IGF1R10-LCDR3 | artificial | aa | HQRSTWPLGT |
| 2066. | IGF1R10-L | artificial | nt | GATATTCAGATGACCCAGTTCCCTGCAACCTTGAGCGTGTCACCTGGCGAAAGAGC<br>CACACTGTCTTGCCGAGCAAGCCAGAGCGTGATGCGGAACCTGGCATGGTACCAG<br>CAGAAACCTGGCCAGCCTCCACGACTGCTGATCTATGGAGCTAGCAAAAGAGCCAC<br>CGGAATCCCCGCTAGGTTTAGCGGAAGCGGAAGCGGAACTGCCTTTACCCTGACC<br>ATCTCCAATCTCGAGCCTGAGGACTTTGCCGTCTACTACTGTCACCAGCGTAGCAC<br>TTGGCCTCTGGGGACATTTGGACCTGGCACAAAGCTGGAAGCTAAG |
| 2067. | IGF1R10-HL | artificial | aa | EVQLLESGGGLVQPGGSLRLSCAASGFTFSNYHMAWVRQAPGKGLEWVSVISPTGGR<br>TTYADSVKGRFTISRDNSKNTLYLQMNSLRAEDTATYYCARAGYSYGYGYFDYWGQG<br>TLVTVSSGGGGSGGGGSGGGGSDIQMTQFPATLSVSPGERATLSCRASQSVMRNLA |

| | | | | |
|---|---|---|---|---|
| | | | | WYQQKPGQPPRLLIYGASKRATGIPARFSGSGSGTAFTLTISNLEPEDFAVYYCHQRST<br>WPLGTFGPGTKLEAK |
| 2068. | IGF1R10-HL | artificial | nt | GAAGTGCAGCTCTTGGAGAGCGGAGGGGGACTTGTGCAGCCTGGCGGATCCTTGA<br>GACTTTCTTGCGCTGCCTCCGGTTTCACCTTCTCAAATTACCACATGGCCTGGGTGA<br>GACAGGCTCCTGGAAAAGGGCTCGAATGGGTTTCCGTGATTAGCCCAACCGGAGG<br>GAGGACCACATATGCCGATAGCGTCAAGGGGAGGTTTACAATCAGCCGTGACAACT<br>CCAAGAACACCCTGTACCTCCAGATGAACTCACTGCGAGCCGAGGATACTGCAACC<br>TACTACTGCGCTCGAGCCGGATACTCATACGGCTATGGCTACTTCGACTACTGGGG<br>ACAGGGAACACTGGTGACCGTTAGTAGCGGAGGGGGAGGTTCTGGCGGAGGTGG<br>AAGCGGGGGCGGGGGAAGCGATATTCAGATGACCCAGTTCCCTGCAACCTTGAGC<br>GTGTCACCTGGCGAAAGAGCCACACTGTCTTGCCGAGCAAGCCAGAGCGTGATGC<br>GGAACCTGGCATGGTACCAGCAGAAACCTGGCCAGCCTCCACGACTGCTGATCTAT<br>GGAGCTAGCAAAAGAGCCACCGGAATCCCCGCTAGGTTTAGCGGAAGCGGAAGCG<br>GAACTGCCTTTACCCTGACCATCTCCAATCTCGAGCCTGAGGACTTTGCCGTCTACT<br>ACTGTCACCAGCGTAGCACTTGGCCTCTGGGGACATTTGGACCTGGCACAAAGCTG<br>GAAGCTAAG |
| 2069. | IGF1R10HLxI2CHL | artificial | aa | EVQLLESGGGLVQPGGSLRLSCAASGFTFSNYHMAWVRQAPGKGLEWVSVISPTGGR<br>TTYADSVKGRFTISRDNSKNTLYLQMNSLRAEDTATYYCARAGYSYGYGYFDYWGQG<br>TLVTVSSGGGGSGGGGSGGGGSDIQMTQFPATLSVSPGERATLSCRASQSVMRNLA<br>WYQQKPGQPPRLLIYGASKRATGIPARFSGSGSGTAFTLTISNLEPEDFAVYYCHQRST<br>WPLGTFGPGTKLEAKSGGGGSEVQLVESGGGLVQPGGSLKLSCAASGFTFNKYAMN<br>WVRQAPGKGLEWVARIRSKYNNYATYYADSVKDRFTISRDDSKNTAYLQMNNLKTEDT<br>AVYYCVRHGNFGNSYISYWAYWGQGTLVTVSSGGGGSGGGGSGGGGSQTVVTQEP<br>SLTVSPGGTVTLTCGSSTGAVTSGNYPNWVQQKPGQAPRGLIGGTKFLAPGTPARFSG<br>SLLGGKAALTLSGVQPEDEAEYYCVLWYSNRWVFGGGTKLTVL |
| 2070. | IGF1R10HLxI2CHL | artificial | nt | GAAGTGCAGCTCTTGGAGAGCGGAGGGGGACTTGTGCAGCCTGGCGGATCCTTGA<br>GACTTTCTTGCGCTGCCTCCGGTTTCACCTTCTCAAATTACCACATGGCCTGGGTGA<br>GACAGGCTCCTGGAAAAGGGCTCGAATGGGTTTCCGTGATTAGCCCAACCGGAGG<br>GAGGACCACATATGCCGATAGCGTCAAGGGGAGGTTTACAATCAGCCGTGACAACT<br>CCAAGAACACCCTGTACCTCCAGATGAACTCACTGCGAGCCGAGGATACTGCAACC<br>TACTACTGCGCTCGAGCCGGATACTCATACGGCTATGGCTACTTCGACTACTGGGG<br>ACAGGGAACACTGGTGACCGTTAGTAGCGGAGGGGGAGGTTCTGGCGGAGGTGG<br>AAGCGGGGGCGGGGGAAGCGATATTCAGATGACCCAGTTCCCTGCAACCTTGAGC<br>GTGTCACCTGGCGAAAGAGCCACACTGTCTTGCCGAGCAAGCCAGAGCGTGATGC<br>GGAACCTGGCATGGTACCAGCAGAAACCTGGCCAGCCTCCACGACTGCTGATCTAT<br>GGAGCTAGCAAAAGAGCCACCGGAATCCCCGCTAGGTTTAGCGGAAGCGGAAGCG |

| | | | | |
|---|---|---|---|---|
| | | | | GAACTGCCTTTACCCTGACCATCTCCAATCTCGAGCCTGAGGACTTTGCCGTCTACT<br>ACTGTCACCAGCGTAGCACTTGGCCTCTGGGGACATTTGGACCTGGCACAAAGCTG<br>GAAGCTAAGTCCGGAGGTGGTGGATCCGAGGTGCAGCTGGTCGAGTCTGGAGGAG<br>GATTGGTGCAGCCTGGAGGGTCATTGAAACTCTCATGTGCAGCCTCTGGATTCACC<br>TTCAATAAGTACGCCATGAACTGGGTCCGCCAGGCTCCAGGAAAGGGTTTGGAATG<br>GGTTGCTCGCATAAGAAGTAAATATAATAATTATGCAACATATTATGCCGATTCAGTG<br>AAAGACAGGTTCACCATCTCCAGAGATGATTCAAAAAACACTGCCTATCTACAAATG<br>AACAACTTGAAGACTGAGGACACTGCCGTGTACTACTGTGTGAGACATGGGAACTT<br>CGGTAATAGCTACATATCCTACTGGGCTTACTGGGGCCAAGGGACTCTGGTCACCG<br>TCTCCTCAGGTGGTGGTGGTTCTGGCGGCGGCGGCTCCGGTGGTGGTGGTTCTCA<br>GACTGTTGTGACTCAGGAACCTTCACTCACCGTATCACCTGGTGGAACAGTCACAC<br>TCACTTGTGGCTCCTCGACTGGGGCTGTTACATCTGGCAACTACCCAAACTGGGTC<br>CAACAAAAACCAGGTCAGGCACCCCGTGGTCTAATAGGTGGGACTAAGTTCCTCGC<br>CCCCGGTACTCCTGCCAGATTCTCAGGCTCCCTGCTTGGAGGCAAGGCTGCCCTC<br>ACCCTCTCAGGGGTACAGCCAGAGGATGAGGCAGAATATTACTGTGTTCTATGGTA<br>CAGCAACCGCTGGGTGTTCGGTGGAGGAACCAAACTGACTGTCCTA |
| 2071. | IGF1R11-H | artificial | aa | EVQLLESGGGLVQPGGSLRLSCAASGFTFSKYMMSWVRQAPGKGLEWVSYISPSGGL<br>TWYADSVKGRFTISRDNSKNTLYLQMNSLREADTAVYYCARDGARGYGMDVWGQGT<br>TVTVSS |
| 2072. | IGF1R11-HCDR1 | artificial | aa | KYMMS |
| 2073. | IGF1R11-HCDR2 | artificial | aa | YISPSGGLTWYADSVKG |
| 2074. | IGF1R11-HCDR3 | artificial | aa | DGARGYGMDV |
| 2075. | IGF1R11-H | artificial | nt | GAGGTCCAGCTGTTGGAAAGCGGAGGCGGACTCGTGCAGCCTGGCGGATCTTTGC<br>GACTTTCCTGTGCTGCCTCCGGTTTCACCTTCTCCAAGTACATGATGAGCTGGGTG<br>AGACAGGCACCAGGCAAAGGACTGGAATGGGTGAGCTACATTAGCCCATCTGGCG<br>GACTGACATGGTATGCCGATAGTGTTAAGGGGAGGTTCACCATCAGTAGGGACAAT<br>AGCAAGAACACCCTGTACCTGCAGATGAACTCACTGCGAGAGGCTGATACTGCCGT<br>GTATTACTGCGCAAGGGATGGAGCTAGAGGCTATGGAATGGACGTGTGGGGACAG<br>GGAACAACCGTCACCGTGAGCAGT |
| 2076. | IGF1R11-L | artificial | aa | DIQMTQSPATLSLSPGERATLSCRASQSVSSYLAWYQQKPGQAPRLLIYDASNRATGIP<br>ARFSGSGSGTDFTLTISSLEPEDFAVYYCQQRSNWPPEVTFGPGTKVDIK |
| 2077. | IGF1R11-LCDR1 | artificial | aa | RASQSVSSYLA |
| 2078. | IGF1R11-LCDR2 | artificial | aa | DASNRAT |
| 2079. | IGF1R11-LCDR3 | artificial | aa | QQRSNWPPEVT |
| 2080. | IGF1R11-L | artificial | nt | GACATTCAGATGACACAGAGCCCTGCCACACTGAGCCTGTCACCTGGAGAGCGAG<br>CTACATTGAGCTGTCGCGCTAGCCAGAGCGTGTCTTCCTATCTTGCCTGGTACCAG |

| | | | | |
|---|---|---|---|---|
| | | | | CAGAAACCTGGACAGGCACCAAGACTCCTGATCTACGACGCCTCCAACCGTGCAACTGGCATCCCTGCTCGCTTTTCTGGCTCTGGCTCTGGGACCGACTTTACCTTGACCATCTCCTCACTGGAACCCGAGGATTTTGCCGTGTACTATTGCCAGCAGCGGAGCAATTGGCCTCCCGAAGTCACTTTTGGCCCTGGCACCAAAGTGGATATCAAG |
| 2081. | IGF1R11-HL | artificial | aa | EVQLLESGGGLVQPGGSLRLSCAASGFTFSKYMMSWVRQAPGKGLEWVSYISPSGGLTWYADSVKGRFTISRDNSKNTLYLQMNSLREADTAVYYCARDGARGYGMDVWGQGTTVTVSSGGGGSGGGGSGGGGSDIQMTQSPATLSLSPGERATLSCRASQSVSSYLAWYQQKPGQAPRLLIYDASNRATGIPARFSGSGSGTDFTLTISSLEPEDFAVYYCQQRSNWPPEVTFGPGTKVDIK |
| 2082. | IGF1R11-HL | artificial | nt | GAGGTCCAGCTGTTGGAAAGCGGAGGCGGACTCGTGCAGCCTGGCGGATCTTTGCGACTTTCCTGTGCTGCCTCCGGTTTCACCTTCTCCAAGTACATGATGAGCTGGGTGAGACAGGCACCAGGCAAAGGACTGGAATGGGTGAGCTACATTAGCCCATCTGGCGGACTGACATGGTATGCCGATAGTGTTAAGGGGAGGTTCACCATCAGTAGGGACAATAGCAAGAACACCCTGTACCTGCAGATGAACTCACTGCGAGAGGCTGATACTGCCGTGTATTACTGCGCAAGGGATGGAGCTAGAGGCTATGGAATGGACGTGTGGGGACAGGGAACAACCGTCACCGTGAGCAGTGGGGGAGGCGGAAGCGGAGGGGGAGGTAGTGGAGGGGGAGGCTCAGACATTCAGATGACACAGAGCCCTGCCACACTGAGCCTGTCACCTGGAGAGCGAGCTACATTGAGCTGTCGCGCTAGCCAGAGCGTGTCTTCCTATCTTGCCTGGTACCAGCAGAAACCTGGACAGGCACCAAGACTCCTGATCTACGACGCCTCCAACCGTGCAACTGGCATCCCTGCTCGCTTTTCTGGCTCTGGCTCTGGGACCGACTTTACCTTGACCATCTCCTCACTGGAACCCGAGGATTTTGCCGTGTACTATTGCCAGCAGCGGAGCAATTGGCCTCCCGAAGTCACTTTTGGCCCTGGCACCAAAGTGGATATCAAG |
| 2083. | IGF1R11HLxI2CHL | artificial | aa | EVQLLESGGGLVQPGGSLRLSCAASGFTFSKYMMSWVRQAPGKGLEWVSYISPSGGLTWYADSVKGRFTISRDNSKNTLYLQMNSLREADTAVYYCARDGARGYGMDVWGQGTTVTVSSGGGGSGGGGSGGGGSDIQMTQSPATLSLSPGERATLSCRASQSVSSYLAWYQQKPGQAPRLLIYDASNRATGIPARFSGSGSGTDFTLTISSLEPEDFAVYYCQQRSNWPPEVTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2084. | IGF1R11HLxI2CHL | artificial | nt | GAGGTCCAGCTGTTGGAAAGCGGAGGCGGACTCGTGCAGCCTGGCGGATCTTTGCGACTTTCCTGTGCTGCCTCCGGTTTCACCTTCTCCAAGTACATGATGAGCTGGGTGAGACAGGCACCAGGCAAAGGACTGGAATGGGTGAGCTACATTAGCCCATCTGGCG |

| | | | | |
|---|---|---|---|---|
| | | | | GACTGACATGGTATGCCGATAGTGTTAAGGGGAGGTTCACCATCAGTAGGGACAATAGCAAGAACACCCTGTACCTGCAGATGAACTCACTGCGAGAGGCTGATACTGCCGTGTATTACTGCGCAAGGGATGGAGCTAGAGGCTATGGAATGGACGTGTGGGGACAGGGAACAACCGTCACCGTGAGCAGTGGGGGAGGCGGAAGCGGAGGGGGAGGTAGTGGAGGGGGAGGCTCAGACATTCAGATGACACAGAGCCCTGCCACACTGAGCCTGTCACCTGGAGAGCGAGCTACATTGAGCTGTCGCGCTAGCCAGAGCGTGTCTTCCTATCTTGCCTGGTACCAGCAGAAACCTGGACAGGCACCAAGACTCCTGATCTACGACGCCTCCAACCGTGCAACTGGCATCCCTGCTCGCTTTTCTGGCTCTGGCTCTGGGACCGACTTTACCTTGACCATCTCCTCACTGGAACCCGAGGATTTTGCCGTGTACTATTGCCAGCAGCGGAGCAATTGGCCTCCCGAAGTCACTTTTGGCCCTGGCACCAAAGTGGATATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2085. | IGF1R12-H | artificial | aa | EVQLLESGGGLVQPGGSLRLSCAASGFTFSNYPMYWVRQAPGKGLEWVSRISSSGGRTVYADSVKGRFTISRDNSKNTLYLQMNSLRAEDTAVYYCARDRWSRSAAEYGLGGYWGQGTLVTVSS |
| 2086. | IGF1R12-HCDR1 | artificial | aa | NYPMY |
| 2087. | IGF1R12-HCDR2 | artificial | aa | RISSSGGRTVYADSVKG |
| 2088. | IGF1R12-HCDR3 | artificial | aa | DRWSRSAAEYGLGGY |
| 2089. | IGF1R12-H | artificial | nt | GAAGTCCAGCTCCTTGAAAGCGGTGGCGGATTGGTCCAGCCTGGAGGCTCTTTGAGACTCTCCTGTGCCGCATCAGGCTTCACCTTTTCCAACTATCCTATGTACTGGGTTCGCCAGGCTCCTGGAAAAGGCTTGGAATGGGTGTCACGGATTAGCTCTAGCGGGGGAAGGACTGTCTATGCCGATTCCGTGAAAGGGAGATTCACCATCTCTCGGGACAATAGCAAGAACACCCTGTATCTCCAGATGAACTCTCTTCGTGCCGAAGACACTGCCGTGTATTACTGCGCACGAGACCGATGGAGCCGATCAGCAGCTGAATACGGCTTGGGAG |

| | | | | |
|---|---|---|---|---|
| | | | | GCTACTGGGGTCAGGGAACTCTTGTGACTGTCTCTAGC |
| 2090. | IGF1R12-L | artificial | aa | DIQMTQSPDSLAVSLGERATINCKSSQSVLYSSNNKNYLAWYQQKPGQPPKLLIYLAST RESGVPDRFSGSGSGTDFTLTISSLQAEDVAVYYCQQYYSTWTFGQGTKVEIK |
| 2091. | IGF1R12-LCDR1 | artificial | aa | KSSQSVLYSSNNKNYLA |
| 2092. | IGF1R12-LCDR2 | artificial | aa | LASTRES |
| 2093. | IGF1R12-LCDR3 | artificial | aa | QQYYSTWT |
| 2094. | IGF1R12-L | artificial | nt | GATATCCAGATGACTCAGAGCCCTGATTCACTGGCAGTGTCTCTCGGAGAACGTGC CACAATCAACTGCAAGAGTAGCCAGTCCGTCCTTTACTCCTCCAACAACAAAAACTA CCTCGCATGGTACCAGCAGAAACCTGGGCAGCCACCCAAACTTCTCATCTATCTTG CTTCCACACGTGAGAGTGGCGTGCCTGACCGTTTTAGCGGATCCGGTAGCGGAAC AGATTTCACTCTCACAATCTCAAGCCTGCAGGCTGAAGATGTCGCAGTCTACTATTG CCAGCAGTATTACTCAACCTGGACATTCGGACAGGGGACAAAAGTGGAGATCAAA |
| 2095. | IGF1R12-HL | artificial | aa | EVQLLESGGGLVQPGGSLRLSCAASGFTFSNYPMYWVRQAPGKGLEWVSRISSSGGR TVYADSVKGRFTISRDNSKNTLYLQMNSLRAEDTAVYYCARDRWSRSAAEYGLGGYW GQGTLVTVSSGGGGSGGGGSGGGGSDIQMTQSPDSLAVSLGERATINCKSSQSVLYS SNNKNYLAWYQQKPGQPPKLLIYLASTRESGVPDRFSGSGSGTDFTLTISSLQAEDVAV YYCQQYYSTWTFGQGTKVEIK |
| 2096. | IGF1R12-HL | artificial | nt | GAAGTCCAGCTCCTTGAAAGCGGTGGCGGATTGGTCCAGCCTGGAGGCTCTTTGA GACTCTCCTGTGCCGCATCAGGCTTCACCTTTTCCAACTATCCTATGTACTGGGTTC GCCAGGCTCCTGGAAAAGGCTTGGAATGGGTGTCACGGATTAGCTCTAGCGGGGG AAGGACTGTCTATGCCGATTCCGTGAAAGGGAGATTCACCATCTCTCGGGACAATA GCAAGAACACCCTGTATCTCCAGATGAACTCTCTTCGTGCCGAAGACACTGCCGTG TATTACTGCGCACGAGACCGATGGAGCCGATCAGCAGCTGAATACGGCTTGGGAG GCTACTGGGGTCAGGGAACTCTTGTGACTGTCTCTAGCGGAGGCGGTGGAAGCGG GGGAGGCGGATCTGGTGGAGGCGGAAGCGATATCCAGATGACTCAGAGCCCTGAT TCACTGGCAGTGTCTCTCGGAGAACGTGCCACAATCAACTGCAAGAGTAGCCAGTC CGTCCTTTACTCCTCCAACAACAAAAACTACCTCGCATGGTACCAGCAGAAACCTGG GCAGCCACCCAAACTTCTCATCTATCTTGCTTCCACACGTGAGAGTGGCGTGCCTG ACCGTTTTAGCGGATCCGGTAGCGGAACAGATTTCACTCTCACAATCTCAAGCCTG CAGGCTGAAGATGTCGCAGTCTACTATTGCCAGCAGTATTACTCAACCTGGACATTC GGACAGGGGACAAAAGTGGAGATCAAA |
| 2097. | IGF1R12HLxI2CHL | artificial | aa | EVQLLESGGGLVQPGGSLRLSCAASGFTFSNYPMYWVRQAPGKGLEWVSRISSSGGR TVYADSVKGRFTISRDNSKNTLYLQMNSLRAEDTAVYYCARDRWSRSAAEYGLGGYW GQGTLVTVSSGGGGSGGGGSGGGGSDIQMTQSPDSLAVSLGERATINCKSSQSVLYS SNNKNYLAWYQQKPGQPPKLLIYLASTRESGVPDRFSGSGSGTDFTLTISSLQAEDVAV YYCQQYYSTWTFGQGTKVEIKSGGGGSEVQLVESGGGLVQPGGSLKLSCAASGFTFN |

| | | | | |
|---|---|---|---|---|
| | | | | KYAMNWVRQAPGKGLEWVARIRSKYNNYATYYADSVKDRFTISRDDSKNTAYLQMNN LKTEDTAVYYCVRHGNFGNSYISYWAYWGQGTLVTVSSGGGGSGGGGSGGGGSQTV VTQEPSLTVSPGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL |
| 2098. | IGF1R12HLxI2CHL | artificial | nt | GAAGTCCAGCTCCTTGAAAGCGGTGGCGGATTGGTCCAGCCTGGAGGCTCTTTGA GACTCTCCTGTGCCGCATCAGGCTTCACCTTTTCCAACTATCCTATGTACTGGGTTC GCCAGGCTCCTGGAAAAGGCTTGGAATGGGTGTCACGGATTAGCTCTAGCGGGGG AAGGACTGTCTATGCCGATTCCGTGAAAGGGAGATTCACCATCTCTCGGGACAATA GCAAGAACACCCTGTATCTCCAGATGAACTCTCTTCGTGCCGAAGACACTGCCGTG TATTACTGCGCACGAGACCGATGGAGCCGATCAGCAGCTGAATACGGCTTGGGAG GCTACTGGGGTCAGGGAACTCTTGTGACTGTCTCTAGCGGAGGCGGTGGAAGCGG GGGAGGCGGATCTGGTGGAGGCGGAAGCGATATCCAGATGACTCAGAGCCCTGAT TCACTGGCAGTGTCTCTCGGAGAACGTGCCACAATCAACTGCAAGAGTAGCCAGTC CGTCCTTTACTCCTCCAACAACAAAAACTACCTCGCATGGTACCAGCAGAAACCTGG GCAGCCACCCAAACTTCTCATCTATCTTGCTTCCACACGTGAGAGTGGCGTGCCTG ACCGTTTTAGCGGATCCGGTAGCGGAACAGATTTCACTCTCACAATCTCAAGCCTG CAGGCTGAAGATGTCGCAGTCTACTATTGCCAGCAGTATTACTCAACCTGGACATTC GGACAGGGGACAAAAGTGGAGATCAAATCCGGAGGTGGTGGATCCGAGGTGCAGC TGGTCGAGTCTGGAGGAGGATTGGTGCAGCCTGGAGGGTCATTGAAACTCTCATGT GCAGCCTCTGGATTCACCTTCAATAAGTACGCCATGAACTGGGTCCGCCAGGCTCC AGGAAAGGGTTTGGAATGGGTTGCTCGCATAAGAAGTAAATATAATAATTATGCAAC ATATTATGCCGATTCAGTGAAAGACAGGTTCACCATCTCCAGAGATGATTCAAAAAA CACTGCCTATCTACAAATGAACAACTTGAAGACTGAGGACACTGCCGTGTACTACTG TGTGAGACATGGGAACTTCGGTAATAGCTACATATCCTACTGGGCTTACTGGGGCC AAGGGACTCTGGTCACCGTCTCCTCAGGTGGTGGTGGTTCTGGCGGCGGCGGCTC CGGTGGTGGTGGTTCTCAGACTGTTGTGACTCAGGAACCTTCACTCACCGTATCAC CTGGTGGAACAGTCACACTCACTTGTGGCTCCTCGACTGGGGCTGTTACATCTGGC AACTACCCAAACTGGGTCCAACAAAAACCAGGTCAGGCACCCCGTGGTCTAATAGG TGGGACTAAGTTCCTCGCCCCCGGTACTCCTGCCAGATTCTCAGGCTCCCTGCTTG GAGGCAAGGCTGCCCTCACCCTCTCAGGGGTACAGCCAGAGGATGAGGCAGAATA TTACTGTGTTCTATGGTACAGCAACCGCTGGGTGTTCGGTGGAGGAACCAAACTGA CTGTCCTA |
| 2099. | IGF1R13-H | artificial | aa | EVQLVQSGAEVKKPGESLTISCKGPGYNFFNYWIGWVRQMPGKGLEWMGIIYPTDSDT RYSPSFQGQVTISVDKSISTAYLQWSSLKASDTAMYYCARSIRYCPGGRCYSGYYGMD VWGQGTMVTVSS |
| 2100. | IGF1R13-HCDR1 | artificial | aa | NYWIG |

| | | | | |
|---|---|---|---|---|
| 2101. | IGF1R13-HCDR2 | artificial | aa | IIYPTDSDTRYSPSFQG |
| 2102. | IGF1R13-HCDR3 | artificial | aa | SIRYCPGGRCYSGYYGMDV |
| 2103. | IGF1R13-H | artificial | nt | GAAGTCCAGTTGGTGCAGAGTGGTGCCGAAGTCAAAAAGCCTGGCGAGAGCCTGA<br>CCATCTCATGTAAGGGTCCTGGCTACAACTTCTTCAACTACTGGATCGGATGGGTG<br>CGACAGATGCCTGGAAAAGGGCTCGAATGGATGGGCATTATCTATCCCACCGACTC<br>CGACACACGGTATTCCCCATCATTTCAGGGACAGGTCACCATCTCCGTTGACAAGA<br>GCATCTCCACCGCTTATCTCCAGTGGAGTTCCCTGAAAGCCTCCGACACCGCAATG<br>TATTACTGCGCACGTAGTATCCGTTACTGCCCAGGAGGCAGATGCTATAGCGGATA<br>TTACGGCATGGACGTTTGGGGACAGGGAACAATGGTGACTGTGAGCTCA |
| 2104. | IGF1R13-L | artificial | aa | SSELTQDPAVSVALGQTVRITCQGDSLRSYYASWYQQKPGQAPVLVIYGKNNRPSGIP<br>DRFSGSSSGNTASLTITGAQAEDEADYYCNSRDSSGNHVVFGGGTKLTVL |
| 2105. | IGF1R13-LCDR1 | artificial | aa | QGDSLRSYYAS |
| 2106. | IGF1R13-LCDR2 | artificial | aa | GKNNRPS |
| 2107. | IGF1R13-LCDR3 | artificial | aa | NSRDSSGNHVV |
| 2108. | IGF1R13-L | artificial | nt | TCAAGCGAACTGACTCAGGATCCAGCTGTTTCTGTCGCCTTGGGACAGACTGTGCG<br>CATTACATGTCAGGGTGATAGCTTGCGTAGCTACTACGCTTCATGGTACCAGCAGA<br>AGCCTGGACAGGCACCTGTCCTGGTGATCTACGGCAAGAACAATAGGCCAAGCGG<br>TATTCCCGATCGGTTTTCTGGATCCTCATCCGGCAATACTGCCTCTCTGACAATCAC<br>CGGTGCTCAGGCTGAAGATGAGGCTGACTACTACTGCAACTCCCGAGATTCTAGCG<br>GAAATCACGTGGTCTTCGGAGGTGGGACAAAACTTACCGTGCTC |
| 2109. | IGF1R13-HL | artificial | aa | EVQLVQSGAEVKKPGESLTISCKGPGYNFFNYWIGWVRQMPGKGLEWMGIIYPTDSDT<br>RYSPSFQGQVTISVDKSISTAYLQWSSLKASDTAMYYCARSIRYCPGGRCYSGYYGMD<br>VWGQGTMVTVSSGGGGSGGGGSGGGGSSSELTQDPAVSVALGQTVRITCQGDSLRS<br>YYASWYQQKPGQAPVLVIYGKNNRPSGIPDRFSGSSSGNTASLTITGAQAEDEADYYC<br>NSRDSSGNHVVFGGGTKLTVL |
| 2110. | IGF1R13-HL | artificial | nt | GAAGTCCAGTTGGTGCAGAGTGGTGCCGAAGTCAAAAAGCCTGGCGAGAGCCTGA<br>CCATCTCATGTAAGGGTCCTGGCTACAACTTCTTCAACTACTGGATCGGATGGGTG<br>CGACAGATGCCTGGAAAAGGGCTCGAATGGATGGGCATTATCTATCCCACCGACTC<br>CGACACACGGTATTCCCCATCATTTCAGGGACAGGTCACCATCTCCGTTGACAAGA<br>GCATCTCCACCGCTTATCTCCAGTGGAGTTCCCTGAAAGCCTCCGACACCGCAATG<br>TATTACTGCGCACGTAGTATCCGTTACTGCCCAGGAGGCAGATGCTATAGCGGATA<br>TTACGGCATGGACGTTTGGGGACAGGGAACAATGGTGACTGTGAGCTCAGGAGGG<br>GGAGGCTCTGGTGGGGGAGGTTCTGGAGGTGGAGGCTCTTCAAGCGAACTGACTC<br>AGGATCCAGCTGTTTCTGTCGCCTTGGGACAGACTGTGCGCATTACATGTCAGGGT<br>GATAGCTTGCGTAGCTACTACGCTTCATGGTACCAGCAGAAGCCTGGACAGGCACC<br>TGTCCTGGTGATCTACGGCAAGAACAATAGGCCAAGCGGTATTCCCGATCGGTTTT |

| | | | | |
|---|---|---|---|---|
| | | | | CTGGATCCTCATCCGGCAATACTGCCTCTCTGACAATCACCGGTGCTCAGGCTGAA<br>GATGAGGCTGACTACTACTGCAACTCCCGAGATTCTAGCGGAAATCACGTGGTCTT<br>CGGAGGTGGGACAAAACTTACCGTGCTC |
| 2111. | IGF1R13HLxI2CHL | artificial | aa | EVQLVQSGAEVKKPGESLTISCKGPGYNFFNYWIGWVRQMPGKGLEWMGIIYPTDSDT<br>RYSPSFQGQVTISVDKSISTAYLQWSSLKASDTAMYYCARSIRYCPGGRCYSGYYGMD<br>VWGQGTMVTVSSGGGGSGGGGSGGGGSSSELTQDPAVSVALGQTVRITCQGDSLRS<br>YYASWYQQKPGQAPVLVIYGKNNRPSGIPDRFSGSSSGNTASLTITGAQAEDEADYYC<br>NSRDSSGNHVVFGGGTKLTVLSGGGGSEVQLVESGGGLVQPGGSLKLSCAASGFTFN<br>KYAMNWVRQAPGKGLEWVARIRSKYNNYATYYADSVKDRFTISRDDSKNTAYLQMNN<br>LKTEDTAVYYCVRHGNFGNSYISYWAYWGQGTLVTVSSGGGGSGGGGSGGGGSQTV<br>VTQEPSLTVSPGGTVTLTCGSSTGAVTSGNYPNWVQQKPGQAPRGLIGGTKFLAPGTP<br>ARFSGSLLGGKAALTLSGVQPEDEAEYYCVLWYSNRWVFGGGTKLTVL |
| 2112. | IGF1R13HLxI2CHL | artificial | nt | GAAGTCCAGTTGGTGCAGAGTGGTGCCGAAGTCAAAAAGCCTGGCGAGAGCCTGA<br>CCATCTCATGTAAGGGTCCTGGCTACAACTTCTTCAACTACTGGATCGGATGGGTG<br>CGACAGATGCCTGGAAAAGGGCTCGAATGGATGGGCATTATCTATCCCACCGACTC<br>CGACACACGGTATTCCCCATCATTTCAGGGACAGGTCACCATCTCCGTTGACAAGA<br>GCATCTCCACCGCTTATCTCCAGTGGAGTTCCCTGAAAGCCTCCGACACCGCAATG<br>TATTACTGCGCACGTAGTATCCGTTACTGCCCAGGAGGCAGATGCTATAGCGGATA<br>TTACGGCATGGACGTTTGGGGACAGGGAACAATGGTGACTGTGAGCTCAGGAGGG<br>GGAGGCTCTGGTGGGGGAGGTTCTGGAGGTGGAGGCTCTTCAAGCGAACTGACTC<br>AGGATCCAGCTGTTTCTGTCGCCTTGGGACAGACTGTGCGCATTACATGTCAGGGT<br>GATAGCTTGCGTAGCTACTACGCTTCATGGTACCAGCAGAAGCCTGGACAGGCACC<br>TGTCCTGGTGATCTACGGCAAGAACAATAGGCCAAGCGGTATTCCCGATCGGTTTT<br>CTGGATCCTCATCCGGCAATACTGCCTCTCTGACAATCACCGGTGCTCAGGCTGAA<br>GATGAGGCTGACTACTACTGCAACTCCCGAGATTCTAGCGGAAATCACGTGGTCTT<br>CGGAGGTGGGACAAAACTTACCGTGCTCTCCGGAGGTGGTGGATCCGAGGTGCAG<br>CTGGTCGAGTCTGGAGGAGGATTGGTGCAGCCTGGAGGGTCATTGAAACTCTCAT<br>GTGCAGCCTCTGGATTCACCTTCAATAAGTACGCCATGAACTGGGTCCGCCAGGCT<br>CCAGGAAAGGGTTTGGAATGGGTTGCTCGCATAAGAAGTAAATATAATAATTATGCA<br>ACATATTATGCCGATTCAGTGAAAGACAGGTTCACCATCTCCAGAGATGATTCAAAA<br>AACACTGCCTATCTACAAATGAACAACTTGAAGACTGAGGACACTGCCGTGTACTAC<br>TGTGTGAGACATGGGAACTTCGGTAATAGCTACATATCCTACTGGGCTTACTGGGG<br>CCAAGGGACTCTGGTCACCGTCTCCTCAGGTGGTGGTGGTTCTGGCGGCGGCGGC<br>TCCGGTGGTGGTGGTTCTCAGACTGTTGTGACTCAGGAACCTTCACTCACCGTATC<br>ACCTGGTGGAACAGTCACACTCACTTGTGGCTCCTCGACTGGGGCTGTTACATCTG<br>GCAACTACCCAAACTGGGTCCAACAAAAACCAGGTCAGGCACCCCGTGGTCTAATA |

| | | | | |
|---|---|---|---|---|
| | | | | GGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2113. | IGF1R15-H | artificial | aa | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCASSPYSSRWYSFDPWGQGTMVTVSS |
| 2114. | IGF1R15-HCDR1 | artificial | aa | SYAMS |
| 2115. | IGF1R15-HCDR2 | artificial | aa | AISGSGGSTYYADSVKG |
| 2116. | IGF1R15-HCDR3 | artificial | aa | SPYSSRWYSFDP |
| 2117. | IGF1R15-H | artificial | nt | GAGGTTCAGCTGCTCGAAAGCGGAGGCGGTTTGGTGCAGCCTGGGGGAAGCCTGAGACTTAGCTGTGCTGCAAGCGGATTCACCTTCTCCAGCTATGCCATGAGCTGGGTGAGGCAGGCCCCTGGCAAAGGACTGGAGTGGGTGAGCGCTATCTCAGGAAGCGGCGGTTCTACCTACTACGCCGATAGCGTCAAGGGGAGGTTCACCATCAGCCGTGACAACAGCAAGAACACCCTGTACCTCCAGATGAATAGCCTGCGTGCTGAGGACACTGCCGTTTACTACTGCGCAAGCTCCCCATACTCCTCACGGTGGTATAGCTTCGATCCATGGGGACAGGGGACTATGGTGACCGTGAGTAGT |
| 2118. | IGF1R15-L | artificial | aa | SYELTQPPSVSVSPGQTATITCSGDDLGNKYVSWYQQKPGQSPVLVIYQDTKRPSGIPERFSGSNSGNIATLTISGTQAVDEADYYCQVWDTGTVVFGGGTKLTVL |
| 2119. | IGF1R15-LCDR1 | artificial | aa | SGDDLGNKYVS |
| 2120. | IGF1R15-LCDR2 | artificial | aa | QDTKRPS |
| 2121. | IGF1R15-LCDR3 | artificial | aa | QVWDTGTVV |
| 2122. | IGF1R15-L | artificial | nt | AGCTATGAACTGACACAGCCACCTAGCGTGAGCGTTAGCCCTGGACAGACTGCCACAATCACCTGTAGTGGGGATGACCTTGGCAACAAGTACGTGAGCTGGTATCAGCAGAAGCCTGGCCAGTCTCCCGTGCTGGTGATCTACCAGGACACCAAACGACCTAGCGGAATCCCAGAGCGATTCAGCGGAAGCAACAGCGGGAACATCGCAACTCTCACCATTTCCGGCACTCAGGCCGTGGATGAAGCCGACTACTATTGCCAGGTGTGGGACACTGGAACCGTCGTGTTTGGGGGAGGCACAAAACTGACCGTCCTG |
| 2123. | IGF1R15-HL | artificial | aa | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCASSPYSSRWYSFDPWGQGTMVTVSSGGGGSGGGGSGGGGSSYELTQPPSVSVSPGQTATITCSGDDLGNKYVSWYQQKPGQSPVLVIYQDTKRPSGIPERFSGSNSGNIATLTISGTQAVDEADYYCQVWDTGTVVFGGGTKLTVL |
| 2124. | IGF1R15-HL | artificial | nt | GAGGTTCAGCTGCTCGAAAGCGGAGGCGGTTTGGTGCAGCCTGGGGGAAGCCTGAGACTTAGCTGTGCTGCAAGCGGATTCACCTTCTCCAGCTATGCCATGAGCTGGGTGAGGCAGGCCCCTGGCAAAGGACTGGAGTGGGTGAGCGCTATCTCAGGAAGCGGC |

| | | | | |
|---|---|---|---|---|
| | | | | GGTTCTACCTACTACGCCGATAGCGTCAAGGGGAGGTTCACCATCAGCCGTGACAACAGCAAGAACACCCTGTACCTCCAGATGAATAGCCTGCGTGCTGAGGACACTGCCGTTTACTACTGCGCAAGCTCCCCATACTCCTCACGGTGGTATAGCTTCGATCCATGGGGACAGGGGACTATGGTGACCGTGAGTAGTGGCGGAGGGGGAAGCGGAGGAGGCGGGAGCGGCGGAGGCGGAAGCAGCTATGAACTGACACAGCCACCTAGCGTGAGCGTTAGCCCTGGACAGACTGCCACAATCACCTGTAGTGGGGATGACCTTGGCAACAAGTACGTGAGCTGGTATCAGCAGAAGCCTGGCCAGTCTCCCGTGCTGGTGATCTACCAGGACACCAAACGACCTAGCGGAATCCCAGAGCGATTCAGCGGAAGCAACAGCGGGAACATCGCAACTCTCACCATTTCCGGCACTCAGGCCGTGGATGAAGCCGACTACTATTGCCAGGTGTGGGACACTGGAACCGTCGTGTTTGGGGGAGGCACAAAACTGACCGTCCTG |
| 2125. | IGF1R15HLxI2CHL | artificial | aa | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCASSPYSSRWYSFDPWGQGTMVTVSSGGGGSGGGGSGGGGSSYELTQPPSVSVSPGQTATITCSGDDLGNKYVSWYQQKPGQSPVLVIYQDTKRPSGIPERFSGSNSGNIATLTISGTQAVDEADYYCQVWDTGTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2126. | IGF1R15HLxI2CHL | artificial | nt | GAGGTTCAGCTGCTCGAAAGCGGAGGCGGTTTGGTGCAGCCTGGGGGAAGCCTGAGACTTAGCTGTGCTGCAAGCGGATTCACCTTCTCCAGCTATGCCATGAGCTGGGTGAGGCAGGCCCCTGGCAAAGGACTGGAGTGGGTGAGCGCTATCTCAGGAAGCGGCGGTTCTACCTACTACGCCGATAGCGTCAAGGGGAGGTTCACCATCAGCCGTGACAACAGCAAGAACACCCTGTACCTCCAGATGAATAGCCTGCGTGCTGAGGACACTGCCGTTTACTACTGCGCAAGCTCCCCATACTCCTCACGGTGGTATAGCTTCGATCCATGGGGACAGGGGACTATGGTGACCGTGAGTAGTGGCGGAGGGGGAAGCGGAGGAGGCGGGAGCGGCGGAGGCGGAAGCAGCTATGAACTGACACAGCCACCTAGCGTGAGCGTTAGCCCTGGACAGACTGCCACAATCACCTGTAGTGGGGATGACCTTGGCAACAAGTACGTGAGCTGGTATCAGCAGAAGCCTGGCCAGTCTCCCGTGCTGGTGATCTACCAGGACACCAAACGACCTAGCGGAATCCCAGAGCGATTCAGCGGAAGCAACAGCGGGAACATCGCAACTCTCACCATTTCCGGCACTCAGGCCGTGGATGAAGCCGACTACTATTGCCAGGTGTGGGACACTGGAACCGTCGTGTTTGGGGGAGGCACAAAACTGACCGTCCTGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGG |

| | | | | |
|---|---|---|---|---|
| | | | | GTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2127. | IGF1R16-H | artificial | aa | QVQLQQSGAELVKPGASVKLSCKASGYTFTSYWMHWVKQRPGQGLEWIGEINPSNGRTNYNEKFKRKATLTVDKSSSTAYMQLSSLTSEDSAVYYFARGRPDYYGSSKWYFDVWGAGTTVTVSS |
| 2128. | IGF1R16-HCDR1 | artificial | aa | SYWMH |
| 2129. | IGF1R16-HCDR2 | artificial | aa | EINPSNGRTNYNEKFKR |
| 2130. | IGF1R16-HCDR3 | artificial | aa | GRPDYYGSSKWYFDV |
| 2131. | IGF1R16-H | artificial | nt | CAGGTTCAGCTGCAGCAGAGCGGAGCTGAGCTCGTTAAACCTGGTGCTTCCGTGAAACTGAGCTGCAAAGCCTCCGGCTATACCTTCACAAGCTACTGGATGCACTGGGTCAAACAGCGTCCTGGTCAGGGTCTCGAATGGATTGGGGAGATCAACCCCTCCAACGGCCGCACCAATTACAATGAGAAGTTCAAGCGGAAGGCCACCCTTACCGTGGATAAGAGCTCATCTACCGCCTACATGCAGCTGTCTTCACTGACATCTGAGGACTCTGCCGTGTATTACTTTGCAAGAGGGAGGCCTGACTACTACGGGTCATCCAAGTGGTACTTTGACGTCTGGGGAGCTGGGACTACCGTGACTGTGTCTAGC |
| 2132. | IGF1R16-L | artificial | aa | DVLMTQTPLSLPVSLGDQASISCRSSQSIVHSNVNTYLEWYLQKPGQSPKLLIYKVSNRFSGVPDRFSGSGSGTDFTLRISRVEAEDLGIYYCFQGSHVPPTFGGGTKLEIK |
| 2133. | IGF1R16-LCDR1 | artificial | aa | RSSQSIVHSNVNTYLE |
| 2134. | IGF1R16-LCDR2 | artificial | aa | KVSNRFS |
| 2135. | IGF1R16-LCDR3 | artificial | aa | FQGSHVPPT |
| 2136. | IGF1R16-L | artificial | nt | GATGTCTTGATGACTCAGACTCCACTGTCACTTCCTGTGTCACTGGGAGATCAGGCATCCATTAGCTGCAGGAGTAGCCAGAGCATCGTGCACTCCAACGTCAACACATACCTGGAGTGGTACCTGCAGAAACCTGGCCAGAGCCCAAAGCTGCTCATCTACAAGGTGAGCAATCGGTTTTCCGGCGTTCCCGATAGATTCAGCGGAAGCGGAAGCGGTACAGACTTTACCCTGCGCATTTCTCGAGTGGAAGCCGAAGACCTGGGCATCTACTATTGCTTCCAGGGTTCTCATGTTCCCCCAACCTTCGGTGGCGGAACAAAACTCGAAATCAAG |

| | | | | |
|---|---|---|---|---|
| 2137. | IGF1R16-HL | artificial | aa | QVQLQQSGAELVKPGASVKLSCKASGYTFTSYWMHWVKQRPGQGLEWIGEINPSNGRTNYNEKFKRKATLTVDKSSSTAYMQLSSLTSEDSAVYYFARGRPDYYGSSKWYFDVWGAGTTVTVSSGGGGSGGGGSGGGGSDVLMTQTPLSLPVSLGDQASISCRSSQSIVHSNVNTYLEWYLQKPGQSPKLLIYKVSNRFSGVPDRFSGSGSGTDFTLRISRVEAEDLGIYYCFQGSHVPPTFGGGTKLEIK |
| 2138. | IGF1R16-HL | artificial | nt | CAGGTTCAGCTGCAGCAGAGCGGAGCTGAGCTCGTTAAACCTGGTGCTTCCGTGAAACTGAGCTGCAAAGCCTCCGGCTATACCTTCACAAGCTACTGGATGCACTGGGTCAAACAGCGTCCTGGTCAGGGTCTCGAATGGATTGGGGAGATCAACCCCTCCAACGGCCGCACCAATTACAATGAGAAGTTCAAGCGGAAGGCCACCCTTACCGTGGATAAGAGCTCATCTACCGCCTACATGCAGCTGTCTTCACTGACATCTGAGGACTCTGCCGTGTATTACTTTGCAAGAGGGAGGCCTGACTACTACGGGTCATCCAAGTGGTACTTTGACGTCTGGGGAGCTGGGACTACCGTGACTGTGTCTAGCGGAGGCGGAGGGAGCGGAGGGGGAGGCTCCGGTGGCGGGGGATCTGATGTCTTGATGACTCAGACTCCACTGTCACTTCCTGTGTCACTGGGAGATCAGGCATCCATTAGCTGCAGGAGTAGCCAGAGCATCGTGCACTCCAACGTCAACACATACCTGGAGTGGTACCTGCAGAAACCTGGCCAGAGCCCAAAGCTGCTCATCTACAAGGTGAGCAATCGGTTTTCCGGCGTTCCCGATAGATTCAGCGGAAGCGGAAGCGGTACAGACTTTACCCTGCGCATTTCTCGAGTGGAAGCCGAAGACCTGGGCATCTACTATTGCTTCCAGGGTTCTCATGTTCCCCCAACCTTCGGTGGCGGAACAAAACTCGAAATCAAG |
| 2139. | IGF1R16HLxI2CHL | artificial | aa | QVQLQQSGAELVKPGASVKLSCKASGYTFTSYWMHWVKQRPGQGLEWIGEINPSNGRTNYNEKFKRKATLTVDKSSSTAYMQLSSLTSEDSAVYYFARGRPDYYGSSKWYFDVWGAGTTVTVSSGGGGSGGGGSGGGGSDVLMTQTPLSLPVSLGDQASISCRSSQSIVHSNVNTYLEWYLQKPGQSPKLLIYKVSNRFSGVPDRFSGSGSGTDFTLRISRVEAEDLGIYYCFQGSHVPPTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2140. | IGF1R16HLxI2CHL | artificial | nt | CAGGTTCAGCTGCAGCAGAGCGGAGCTGAGCTCGTTAAACCTGGTGCTTCCGTGAAACTGAGCTGCAAAGCCTCCGGCTATACCTTCACAAGCTACTGGATGCACTGGGTCAAACAGCGTCCTGGTCAGGGTCTCGAATGGATTGGGGAGATCAACCCCTCCAACGGCCGCACCAATTACAATGAGAAGTTCAAGCGGAAGGCCACCCTTACCGTGGATAAGAGCTCATCTACCGCCTACATGCAGCTGTCTTCACTGACATCTGAGGACTCTGCCGTGTATTACTTTGCAAGAGGGAGGCCTGACTACTACGGGTCATCCAAGTGGTACTTTGACGTCTGGGGAGCTGGGACTACCGTGACTGTGTCTAGCGGAGGCGGAGGGAGCGGAGGGGGAGGCTCCGGTGGCGGGGGATCTGATGTCTTGATGACTCAGACTCCACT |

| | | | | |
|---|---|---|---|---|
| | | | | GTCACTTCCTGTGTCACTGGGAGATCAGGCATCCATTAGCTGCAGGAGTAGCCAGAGCATCGTGCACTCCAACGTCAACACATACCTGGAGTGGTACCTGCAGAAACCTGGCCAGAGCCCAAAGCTGCTCATCTACAAGGTGAGCAATCGGTTTTCCGGCGTTCCCGATAGATTCAGCGGAAGCGGAAGCGGTACAGACTTTACCCTGCGCATTTCTCGAGTGGAAGCCGAAGACCTGGGCATCTACTATTGCTTCCAGGGTTCTCATGTTCCCCCAACCTTCGGTGGCGGAACAAAACTCGAAATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2141. | IGF1R17-H | artificial | aa | QAQLVESGGGLVKPGGSLRLSCAASGFTFSDYYMSWIRQAPGKGLEWVSYISSSGSTRDYADSVKGRFTISRDNAKNSLYLQMNSLRAEDTAVYYCVRDGVETTFYYYYYGMDVWGQGTTVTVSS |
| 2142. | IGF1R17-HCDR1 | artificial | aa | DYYMS |
| 2143. | IGF1R17-HCDR2 | artificial | aa | YISSSGSTRDYADSVKG |
| 2144. | IGF1R17-HCDR3 | artificial | aa | DGVETTFYYYYYGMDV |
| 2145. | IGF1R17-H | artificial | nt | CAGGCACAGCTGGTGGAAAGTGGCGGTGGCTTAGTTAAGCCTGGTGGATCCCTGCGACTTTCTTGTGCTGCCTCCGGCTTTACGTTTTCCGACTACTACATGTCCTGGATTAGACAAGCCCCTGGCAAGGGCTTGGAATGGGTGAGCTACATAAGCTCCTCGGGAAGCACTAGGGACTATGCCGATAGCGTTAAGGGGAGATTCACGATAAGCAGGGATAACGCCAAGAACTCCCTGTATCTGCAGATGAATAGTCTCCGTGCTGAGGACACTGCCGTGTACTATTGCGTGAGGGATGGCGTCGAAACAACCTTCTACTACTACTACTATGGCATGGACGTTTGGGGACAGGGAACAACTGTAACCGTGAGCTCA |
| 2146. | IGF1R17-L | artificial | aa | DIQMTQSPSSLSASVGDRVTFTCRASQDIRRDLGWYQQKPGKAPKRLIYAASRLQSGVPSRFSGSGSGTEFTLTISSLQPEDFATYYCLQHNNYPRTFGQGTEVEII |
| 2147. | IGF1R17-LCDR1 | artificial | aa | RASQDIRRDLG |

| | | | | |
|---|---|---|---|---|
| 2148. | IGF1R17-LCDR2 | artificial | aa | AASRLQS |
| 2149. | IGF1R17-LCDR3 | artificial | aa | LQHNNYPRT |
| 2150. | IGF1R17-L | artificial | nt | GATATCCAGATGACACAAAGCCCAAGCTCGCTGAGCGCTAGCGTAGGCGATCGAG TGACATTCACCTGTAGGGCTAGCCAGGATATTAGACGGGACCTAGGGTGGTACCAG CAGAAACCTGGCAAAGCTCCCAAACGCCTTATCTATGCGGCTTCACGGCTGCAATC GGGAGTCCCCTCTCGCTTTTCTGGAAGTGGGAGCGGAACCGAGTTTACATTGACGA TTAGCTCCCTCCAACCTGAGGACTTTGCAACCTATTACTGCCTCCAGCACAATAACT ATCCACGGACCTTCGGACAAGGGACCGAGGTCGAGATCATC |
| 2151. | IGF1R17-HL | artificial | aa | QAQLVESGGGLVKPGGSLRLSCAASGFTFSDYYMSWIRQAPGKGLEWVSYISSSGST RDYADSVKGRFTISRDNAKNSLYLQMNSLRAEDTAVYYCVRDGVETTFYYYYYGMDV WGQGTTVTVSSGGGGSGGGGSGGGGSDIQMTQSPSSLSASVGDRVTFTCRASQDIR RDLGWYQQKPGKAPKRLIYAASRLQSGVPSRFSGSGSGTEFTLTISSLQPEDFATYYCL QHNNYPRTFGQGTEVEII |
| 2152. | IGF1R17-HL | artificial | nt | CAGGCACAGCTGGTGGAAAGTGGCGGTGGCTTAGTTAAGCCTGGTGGATCCCTGC GACTTTCTTGTGCTGCCTCCGGCTTTACGTTTTCCGACTACTACATGTCCTGGATTA GACAAGCCCCTGGCAAGGGCTTGGAATGGGTGAGCTACATAAGCTCCTCGGGAAG CACTAGGGACTATGCCGATAGCGTTAAGGGGAGATTCACGATAAGCAGGGATAACG CCAAGAACTCCCTGTATCTGCAGATGAATAGTCTCCGTGCTGAGGACACTGCCGTG TACTATTGCGTGAGGGATGGCGTCGAAACAACCTTCTACTACTACTACTATGGCATG GACGTTTGGGGACAGGGAACAACTGTAACCGTGAGCTCAGGAGGTGGAGGTAGCG GAGGTGGAGGTAGTGGTGGAGGTGGCTCGGATATCCAGATGACACAAAGCCCAAG CTCGCTGAGCGCTAGCGTAGGCGATCGAGTGACATTCACCTGTAGGGCTAGCCAG GATATTAGACGGGACCTAGGGTGGTACCAGCAGAAACCTGGCAAAGCTCCCAAAC GCCTTATCTATGCGGCTTCACGGCTGCAATCGGGAGTCCCCTCTCGCTTTTCTGGA AGTGGGAGCGGAACCGAGTTTACATTGACGATTAGCTCCCTCCAACCTGAGGACTT TGCAACCTATTACTGCCTCCAGCACAATAACTATCCACGGACCTTCGGACAAGGGA CCGAGGTCGAGATCATC |
| 2153. | IGF1R17HLxI2CHL | artificial | aa | QAQLVESGGGLVKPGGSLRLSCAASGFTFSDYYMSWIRQAPGKGLEWVSYISSSGST RDYADSVKGRFTISRDNAKNSLYLQMNSLRAEDTAVYYCVRDGVETTFYYYYYGMDV WGQGTTVTVSSGGGGSGGGGSGGGGSDIQMTQSPSSLSASVGDRVTFTCRASQDIR RDLGWYQQKPGKAPKRLIYAASRLQSGVPSRFSGSGSGTEFTLTISSLQPEDFATYYCL QHNNYPRTFGQGTEVEIISGGGGSEVQLVESGGGLVQPGGSLKLSCAASGFTFNKYAM NWVRQAPGKGLEWVARIRSKYNNYATYYADSVKDRFTISRDDSKNTAYLQMNNLKTED TAVYYCVRHGNFGNSYISYWAYWGQGTLVTVSSGGGGSGGGGSGGGGSQTVVTQE PSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |

| | | | | |
|---|---|---|---|---|
| 2154. | IGF1R17HLxI2CHL | artificial | nt | CAGGCACAGCTGGTGGAAAGTGGCGGTGGCTTAGTTAAGCCTGGTGGATCCCTGC GACTTTCTTGTGCTGCCTCCGGCTTTACGTTTTCCGACTACTACATGTCCTGGATTA GACAAGCCCCTGGCAAGGGCTTGGAATGGGTGAGCTACATAAGCTCCTCGGGAAG CACTAGGGACTATGCCGATAGCGTTAAGGGGAGATTCACGATAAGCAGGGATAACG CCAAGAACTCCCTGTATCTGCAGATGAATAGTCTCCGTGCTGAGGACACTGCCGTG TACTATTGCGTGAGGGATGGCGTCGAAACAACCTTCTACTACTACTACTATGGCATG GACGTTTGGGGACAGGGAACAACTGTAACCGTGAGCTCAGGAGGTGGAGGTAGCG GAGGTGGAGGTAGTGGTGGAGGTGGCTCGGATATCCAGATGACACAAAGCCCAAG CTCGCTGAGCGCTAGCGTAGGCGATCGAGTGACATTCACCTGTAGGGCTAGCCAG GATATTAGACGGGACCTAGGGTGGTACCAGCAGAAACCTGGCAAAGCTCCCAAAC GCCTTATCTATGCGGCTTCACGGCTGCAATCGGGAGTCCCCTCTCGCTTTTCTGGA AGTGGGAGCGGAACCGAGTTTACATTGACGATTAGCTCCCTCCAACCTGAGGACTT TGCAACCTATTACTGCCTCCAGCACAATAACTATCCACGGACCTTCGGACAAGGGA CCGAGGTCGAGATCATCTCCGGAGGTGGTGGATCCGAGGTGCAGCTGGTCGAGTC TGGAGGAGGATTGGTGCAGCCTGGAGGGTCATTGAAACTCTCATGTGCAGCCTCTG GATTCACCTTCAATAAGTACGCCATGAACTGGGTCCGCCAGGCTCCAGGAAAGGGT TTGGAATGGGTTGCTCGCATAAGAAGTAAATATAATAATTATGCAACATATTATGCCG ATTCAGTGAAAGACAGGTTCACCATCTCCAGAGATGATTCAAAAAACACTGCCTATC TACAAATGAACAACTTGAAGACTGAGGACACTGCCGTGTACTACTGTGTGAGACAT GGGAACTTCGGTAATAGCTACATATCCTACTGGGCTTACTGGGGCCAAGGGACTCT GGTCACCGTCTCCTCAGGTGGTGGTGGTTCTGGCGGCGGCGGCTCCGGTGGTGG TGGTTCTCAGACTGTTGTGACTCAGGAACCTTCACTCACCGTATCACCTGGTGGAA CAGTCACACTCACTTGTGGCTCCTCGACTGGGGCTGTTACATCTGGCAACTACCCA AACTGGGTCCAACAAAAACCAGGTCAGGCACCCCGTGGTCTAATAGGTGGGACTAA GTTCCTCGCCCCCGGTACTCCTGCCAGATTCTCAGGCTCCCTGCTTGGAGGCAAG GCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 2155. | IGF1R19-H | artificial | aa | EVQLLESGGGLVQPGGSLRLSCAASGFTFSSYAMSWVRQAPGKGLEWVSAISGSGGI TYYADSVKGRFTISRDNSKNTLYLQMNSLRAEDTAVYYCAKDLGYGDFYYYYYGMDVW GQGTTVTVSS |
| 2156. | IGF1R19-HCDR1 | artificial | aa | SYAMS |
| 2157. | IGF1R19-HCDR2 | artificial | aa | AISGSGGITYYADSVKG |
| 2158. | IGF1R19-HCDR3 | artificial | aa | DLGYGDFYYYYYGMDV |
| 2159. | IGF1R19-H | artificial | nt | GAGGTTCAGCTGCTTGAATCTGGCGGAGGTTTGGTTCAGCCTGGGGGTTCACTTCG TCTGAGCTGTGCTGCCAGCGGGTTTACATTCTCCTCATATGCCATGTCCTGGGTGC GACAGGCACCAGGCAAAGGCTTGGAATGGGTGTCCGCTATTAGCGGAAGCGGAGG |

| | | | | |
|---|---|---|---|---|
| | | | | CATCACATACTACGCTGATAGCGTCAAGGGGAGATTCACCATCTCACGGGACAATTCCAAGAACACCCTGTATCTCCAGATGAACTCCCTGCGTGCCGAAGATACTGCCGTGTACTATTGCGCCAAGGATCTTGGCTACGGGGACTTCTACTACTACTACTATGGGATGGACGTGTGGGGACAGGGAACAACCGTGACTGTGAGCTCT |
| 2160. | IGF1R19-L | artificial | aa | DIQMTQSPSSLSASVGDRVTITCRASQGIRSDLGWFQQKPGKAPKRLIYAASKLHRGVPSRFSGSGSGTEFTLTISRLQPEDFATYYCLQHNSYPLTFGGGTKVEIK |
| 2161. | IGF1R19-LCDR1 | artificial | aa | RASQGIRSDLG |
| 2162. | IGF1R19-LCDR2 | artificial | aa | AASKLHR |
| 2163. | IGF1R19-LCDR3 | artificial | aa | LQHNSYPLT |
| 2164. | IGF1R19-L | artificial | nt | GACATTCAGATGACTCAGAGCCCAAGCTCTCTGTCTGCTTCTGTGGGAGACCGCGTTACCATCACCTGTAGAGCAAGCCAGGGCATTCGATCCGATCTCGGATGGTTTCAGCAGAAACCTGGCAAGGCACCCAAACGGCTGATCTATGCTGCCAGCAAACTGCATAGGGGAGTGCCCTCACGCTTTAGCGGATCTGGTAGTGGTACCGAGTTCACACTGACAATCTCACGGCTGCAGCCTGAGGATTTCGCCACCTATTACTGCCTCCAGCACAATTCCTACCCACTGACCTTTGGCGGTGGGACTAAGGTCGAGATCAAG |
| 2165. | IGF1R19-HL | artificial | aa | EVQLLESGGGLVQPGGSLRLSCAASGFTFSSYAMSWVRQAPGKGLEWVSAISGSGGITYYADSVKGRFTISRDNSKNTLYLQMNSLRAEDTAVYYCAKDLGYGDFYYYYYGMDVWGQGTTVTVSSGGGGSGGGGSGGGGSDIQMTQSPSSLSASVGDRVTITCRASQGIRSDLGWFQQKPGKAPKRLIYAASKLHRGVPSRFSGSGSGTEFTLTISRLQPEDFATYYCLQHNSYPLTFGGGTKVEIK |
| 2166. | IGF1R19-HL | artificial | nt | GAGGTTCAGCTGCTTGAATCTGGCGGAGGTTTGGTTCAGCCTGGGGGTTCACTTCGTCTGAGCTGTGCTGCCAGCGGGTTTACATTCTCCTCATATGCCATGTCCTGGGTGCGACAGGCACCAGGCAAAGGCTTGGAATGGGTGTCCGCTATTAGCGGAAGCGGAGGCATCACATACTACGCTGATAGCGTCAAGGGGAGATTCACCATCTCACGGGACAATTCCAAGAACACCCTGTATCTCCAGATGAACTCCCTGCGTGCCGAAGATACTGCCGTGTACTATTGCGCCAAGGATCTTGGCTACGGGGACTTCTACTACTACTACTATGGGATGGACGTGTGGGGACAGGGAACAACCGTGACTGTGAGCTCTGGTGGCGGAGGATCTGGCGGAGGTGGAAGCGGAGGGGGAGGGAGCGACATTCAGATGACTCAGAGCCCAAGCTCTCTGTCTGCTTCTGTGGGAGACCGCGTTACCATCACCTGTAGAGCAAGCCAGGGCATTCGATCCGATCTCGGATGGTTTCAGCAGAAACCTGGCAAGGCACCCAAACGGCTGATCTATGCTGCCAGCAAACTGCATAGGGGAGTGCCCTCACGCTTTAGCGGATCTGGTAGTGGTACCGAGTTCACACTGACAATCTCACGGCTGCAGCCTGAGGATTTCGCCACCTATTACTGCCTCCAGCACAATTCCTACCCACTGACCTTTGGCGGTGGGACTAAGGTCGAGATCAAG |
| 2167. | IGF1R19HLxI2CHL | artificial | aa | EVQLLESGGGLVQPGGSLRLSCAASGFTFSSYAMSWVRQAPGKGLEWVSAISGSGGITYYADSVKGRFTISRDNSKNTLYLQMNSLRAEDTAVYYCAKDLGYGDFYYYYYGMDVW |

| | | | | |
|---|---|---|---|---|
| | | | | GQGTTVTVSSGGGGSGGGGSGGGGSDIQMTQSPSSLSASVGDRVTITCRASQGIRSDLGWFQQKPGKAPKRLIYAASKLHRGVPSRFSGSGSGTEFTLTISRLQPEDFATYYCLQHNSYPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2168. | IGF1R19HLxI2CHL | artificial | nt | GAGGTTCAGCTGCTTGAATCTGGCGGAGGTTTGGTTCAGCCTGGGGGTTCACTTCGTCTGAGCTGTGCTGCCAGCGGGTTTACATTCTCCTCATATGCCATGTCCTGGGTGCGACAGGCACCAGGCAAAGGCTTGGAATGGGTGTCCGCTATTAGCGGAAGCGGAGGCATCACATACTACGCTGATAGCGTCAAGGGGAGATTCACCATCTCACGGGACAATTCCAAGAACACCCTGTATCTCCAGATGAACTCCCTGCGTGCCGAAGATACTGCCGTGTACTATTGCGCCAAGGATCTTGGCTACGGGGACTTCTACTACTACTACTATGGGATGGACGTGTGGGGACAGGGAACAACCGTGACTGTGAGCTCTGGTGGCGGAGGATCTGGCGGAGGTGGAAGCGGAGGGGGAGGGAGCGACATTCAGATGACTCAGAGCCCAAGCTCTCTGTCTGCTTCTGTGGGAGACCGCGTTACCATCACCTGTAGAGCAAGCCAGGGCATTCGATCCGATCTCGGATGGTTTCAGCAGAAACCTGGCAAGGCACCCAAACGGCTGATCTATGCTGCCAGCAAACTGCATAGGGGAGTGCCCTCACGCTTTAGCGGATCTGGTAGTGGTACCGAGTTCACACTGACAATCTCACGGCTGCAGCCTGAGGATTTCGCCACCTATTACTGCCTCCAGCACAATTCCTACCCACTGACCTTTGGCGGTGGGACTAAGGTCGAGATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2169. | IGF1R20-H | artificial | aa | QIQLVQSGPELKKPGETVKISCKASGYNLANYGLNWVKQAPGEGLKWMGWINTNTGAPTYAEEFKGRFVFFLETSASTAYLQINNLSDEDTATYFCARSIYYYASRYFNVWGAGTS |

| | | | | |
|---|---|---|---|---|
| | | | | VTVSS |
| 2170. | IGF1R20-HCDR1 | artificial | aa | KASGYNLANYGLN |
| 2171. | IGF1R20-HCDR2 | artificial | aa | WINTNTGAPTYAEEFKG |
| 2172. | IGF1R20-HCDR3 | artificial | aa | SIYYYASRYFNV |
| 2173. | IGF1R20-H | artificial | nt | CAGATCCAGCTGGTGCAGAGCGGACCTGAGCTTAAGAAGCCTGGCGAGACCGTCAAGATCTCATGCAAAGCTAGCGGATACAACCTGGCCAACTATGGCCTCAACTGGGTGAAACAGGCCCCTGGAGAGGGCCTGAAGTGGATGGGATGGATCAACACCAATACTGGCGCCCCAACATACGCAGAGGAATTTAAGGGGAGGTTCGTGTTCTTCCTGGAAACCTCCGCTAGTACTGCCTACCTGCAGATCAACAACCTCTCCGATGAAGATACCGCCACCTACTTTTGTGCCCGCTCTATCTACTACTACGCATCCAGGTACTTCAATGTCTGGGGAGCCGGAACCTCCGTGACTGTTAGCTCT |
| 2174. | IGF1R20-L | artificial | aa | DIQMTQTTSSLSASLGDKVTISCRASQDISNYLNWYQQKPDGTIKLLIYYTSRLHSGIPSRFSGSGSGTDYSLTISNLEQEDIATYFCQQGNTLPWTFGGGTKVEIK |
| 2175. | IGF1R20-LCDR1 | artificial | aa | RASQDISNYLN |
| 2176. | IGF1R20-LCDR2 | artificial | aa | YTSRLHS |
| 2177. | IGF1R20-LCDR3 | artificial | aa | QQGNTLPWT |
| 2178. | IGF1R20-L | artificial | nt | GATATCCAGATGACACAGACTACATCTTCCCTCTCCGCTTCACTTGGCGACAAAGTCACCATTTCATGCCGTGCAAGCCAGGACATCAGCAACTACCTCAATTGGTACCAGCAGAAACCCGACGGGACAATCAAGCTGCTGATCTACTACACAAGCCGACTGCACAGCGGGATTCCATCAAGATTCAGCGGAAGCGGAAGTGGGACCGATTACAGCTTGACAATCAGCAATCTGGAGCAGGAGGACATCGCTACTTACTTTTGCCAGCAGGGGAATACCTTGCCATGGACATTTGGGGGCGGTACCAAAGTGGAAATCAAG |
| 2179. | IGF1R20-HL | artificial | aa | QIQLVQSGPELKKPGETVKISCKASGYNLANYGLNWVKQAPGEGLKWMGWINTNTGAPTYAEEFKGRFVFFLETSASTAYLQINNLSDEDTATYFCARSIYYYASRYFNVWGAGTSVTVSSGGGGSGGGGSGGGGSDIQMTQTTSSLSASLGDKVTISCRASQDISNYLNWYQQKPDGTIKLLIYYTSRLHSGIPSRFSGSGSGTDYSLTISNLEQEDIATYFCQQGNTLPWTFGGGTKVEIK |
| 2180. | IGF1R20-HL | artificial | nt | CAGATCCAGCTGGTGCAGAGCGGACCTGAGCTTAAGAAGCCTGGCGAGACCGTCAAGATCTCATGCAAAGCTAGCGGATACAACCTGGCCAACTATGGCCTCAACTGGGTGAAACAGGCCCCTGGAGAGGGCCTGAAGTGGATGGGATGGATCAACACCAATACTGGCGCCCCAACATACGCAGAGGAATTTAAGGGGAGGTTCGTGTTCTTCCTGGAAACCTCCGCTAGTACTGCCTACCTGCAGATCAACAACCTCTCCGATGAAGATACCGCCACCTACTTTTGTGCCCGCTCTATCTACTACTACGCATCCAGGTACTTCAATGTCTGGGGAGCCGGAACCTCCGTGACTGTTAGCTCTGGAGGAGGCGGTAGCGGTGGCGGTGGAAGTGGCGGAGGTGGCTCTGATATCCAGATGACACAGACTACATCTTCCCTCTCCGCTTCACTTGGCGACAAAGTCACCATTTCATGCCGTGCAAGCCAGGACATCAGCAAC |

| | | | | |
|---|---|---|---|---|
| | | | | TACCTCAATTGGTACCAGCAGAAACCCGACGGGACAATCAAGCTGCTGATCTACTA<br>CACAAGCCGACTGCACAGCGGGATTCCATCAAGATTCAGCGGAAGCGGAAGTGGG<br>ACCGATTACAGCTTGACAATCAGCAATCTGGAGCAGGAGGACATCGCTACTTACTTT<br>TGCCAGCAGGGGAATACCTTGCCATGGACATTTGGGGGCGGTACCAAAGTGGAAA<br>TCAAG |
| 2181. | IGF1R20HLxI2CHL | artificial | aa | QIQLVQSGPELKKPGETVKISCKASGYNLANYGLNWVKQAPGEGLKWMGWINTNTGA<br>PTYAEEFKGRFVFFLETSASTAYLQINNLSDEDTATYFCARSIYYYASRYFNVWGAGTS<br>VTVSSGGGGSGGGGSGGGGSDIQMTQTTSSLSASLGDKVTISCRASQDISNYLNWYQ<br>QKPDGTIKLLIYYTSRLHSGIPSRFSGSGSGTDYSLTISNLEQEDIATYFCQQGNTLPWTF<br>GGGTKVEIKSGGGGSEVQLVESGGGLVQPGGSLKLSCAASGFTFNKYAMNWVRQAP<br>GKGLEWVARIRSKYNNYATYYADSVKDRFTISRDDSKNTAYLQMNNLKTEDTAVYYCV<br>RHGNFGNSYISYWAYWGQGTLVTVSSGGGGSGGGGSGGGGSQTVVTQEPSLTVSPG<br>GTVTLTCGSSTGAVTSGNYPNWVQQKPGQAPRGLIGGTKFLAPGTPARFSGSLLGGK<br>AALTLSGVQPEDEAEYYCVLWYSNRWVFGGGTKLTVL |
| 2182. | IGF1R20HLxI2CHL | artificial | nt | CAGATCCAGCTGGTGCAGAGCGGACCTGAGCTTAAGAAGCCTGGCGAGACCGTCA<br>AGATCTCATGCAAAGCTAGCGGATACAACCTGGCCAACTATGGCCTCAACTGGGTG<br>AAACAGGCCCCTGGAGAGGGCCTGAAGTGGATGGGATGGATCAACACCAATACTG<br>GCGCCCCAACATACGCAGAGGAATTTAAGGGGAGGTTCGTGTTCTTCCTGGAAACC<br>TCCGCTAGTACTGCCTACCTGCAGATCAACAACCTCTCCGATGAAGATACCGCCAC<br>CTACTTTTGTGCCCGCTCTATCTACTACTACGCATCCAGGTACTTCAATGTCTGGGG<br>AGCCGGAACCTCCGTGACTGTTAGCTCTGGAGGAGGCGGTAGCGGTGGCGGTGG<br>AAGTGGCGGAGGTGGCTCTGATATCCAGATGACACAGACTACATCTTCCCTCTCCG<br>CTTCACTTGGCGACAAAGTCACCATTTCATGCCGTGCAAGCCAGGACATCAGCAAC<br>TACCTCAATTGGTACCAGCAGAAACCCGACGGGACAATCAAGCTGCTGATCTACTA<br>CACAAGCCGACTGCACAGCGGGATTCCATCAAGATTCAGCGGAAGCGGAAGTGGG<br>ACCGATTACAGCTTGACAATCAGCAATCTGGAGCAGGAGGACATCGCTACTTACTTT<br>TGCCAGCAGGGGAATACCTTGCCATGGACATTTGGGGGCGGTACCAAAGTGGAAA<br>TCAAGTCCGGAGGTGGTGGATCCGAGGTGCAGCTGGTCGAGTCTGGAGGAGGATT<br>GGTGCAGCCTGGAGGGTCATTGAAACTCTCATGTGCAGCCTCTGGATTCACCTTCA<br>ATAAGTACGCCATGAACTGGGTCCGCCAGGCTCCAGGAAAGGGTTTGGAATGGGTT<br>GCTCGCATAAGAAGTAAATATAATAATTATGCAACATATTATGCCGATTCAGTGAAAG<br>ACAGGTTCACCATCTCCAGAGATGATTCAAAAAACACTGCCTATCTACAAATGAACA<br>ACTTGAAGACTGAGGACACTGCCGTGTACTACTGTGTGAGACATGGGAACTTCGGT<br>AATAGCTACATATCCTACTGGGCTTACTGGGGCCAAGGGACTCTGGTCACCGTCTC<br>CTCAGGTGGTGGTGGTTCTGGCGGCGGCGGCTCCGGTGGTGGTGGTTCTCAGACT<br>GTTGTGACTCAGGAACCTTCACTCACCGTATCACCTGGTGGAACAGTCACACTCAC |

| | | | | |
|---|---|---|---|---|
| | | | | TTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2183. | IGF1R21-H | artificial | aa | QIQLVQSGPELKKPGETVKISCKASGYTLANYGMNWVKQAPGKGFKWMGWINTNTGKPTYSDEFKGRFVFSLETSASTAYLLINNLSNEDTATYFCARSIYYYGSRYFNVWGAGTTVTVSS |
| 2184. | IGF1R21-HCDR1 | artificial | aa | KASGYTLANYGMN |
| 2185. | IGF1R21-HCDR2 | artificial | aa | WINTNTGKPTYSDEFKG |
| 2186. | IGF1R21-HCDR3 | artificial | aa | SIYYYGSRYFNV |
| 2187. | IGF1R21-H | artificial | nt | CAGATCCAGCTTGTCCAGAGTGGTCCCGAGCTGAAGAAACCTGGCGAAACCGTGAAAATCAGCTGCAAGGCAAGCGGCTATACACTGGCCAACTATGGCATGAATTGGGTGAAGCAGGCACCTGGGAAGGGTTTCAAGTGGATGGGCTGGATCAACACCAACACTGGCAAACCAACCTATTCCGACGAGTTCAAGGGGCGTTTCGTGTTTTCACTGGAAACCTCCGCATCAACCGCTTACCTCTTGATCAACAACCTGAGCAACGAGGACACCGCCACATACTTCTGCGCTAGGAGCATCTACTACTATGGGAGCCGCTACTTCAATGTGTGGGGAGCTGGGACAACTGTTACTGTGAGCTCA |
| 2188. | IGF1R21-L | artificial | aa | DIQMTQSTSSLSASLGDRVTISCRASQDINNYLTWYQQKPDGTVKLLIYYTSRLHSGVPSRFSGSGSGTDYSLTITNLEQEDFATYFCQQGNTLPWTFGEGTKVEIK |
| 2189. | IGF1R21-LCDR1 | artificial | aa | RASQDINNYLT |
| 2190. | IGF1R21-LCDR2 | artificial | aa | YTSRLHS |
| 2191. | IGF1R21-LCDR3 | artificial | aa | QQGNTLPWT |
| 2192. | IGF1R21-L | artificial | nt | GATATCCAGATGACACAGAGCACAAGTTCATTGTCCGCCTCTCTTGGCGATCGAGTGACCATTAGCTGCCGAGCAAGCCAGGACATTAACAACTACCTGACCTGGTATCAGCAGAAACCCGACGGGACTGTGAAGCTGCTGATCTATTACACCTCTAGACTGCACAGTGGCGTTCCTAGCCGGTTTAGCGGATCCGGTAGCGGAACAGATTACTCCCTGACCATCACCAATCTCGAGCAGGAAGACTTTGCCACCTACTTTTGCCAGCAGGGCAATACTCTCCCATGGACATTTGGTGAGGGGACCAAAGTCGAGATTAAG |
| 2193. | IGF1R21-HL | artificial | aa | QIQLVQSGPELKKPGETVKISCKASGYTLANYGMNWVKQAPGKGFKWMGWINTNTGKPTYSDEFKGRFVFSLETSASTAYLLINNLSNEDTATYFCARSIYYYGSRYFNVWGAGTTVTVSSGGGGSGGGGSGGGGSDIQMTQSTSSLSASLGDRVTISCRASQDINNYLTWYQQKPDGTVKLLIYYTSRLHSGVPSRFSGSGSGTDYSLTITNLEQEDFATYFCQQGNTLPWTFGEGTKVEIK |
| 2194. | IGF1R21-HL | artificial | nt | CAGATCCAGCTTGTCCAGAGTGGTCCCGAGCTGAAGAAACCTGGCGAAACCGTGAAAATCAGCTGCAAGGCAAGCGGCTATACACTGGCCAACTATGGCATGAATTGGGTG |

| | | | | |
|---|---|---|---|---|
| | | | | AAGCAGGCACCTGGGAAGGGTTTCAAGTGGATGGGCTGGATCAACACCAACACTG<br>GCAAACCAACCTATTCCGACGAGTTCAAGGGGCGTTTCGTGTTTTCACTGGAAACC<br>TCCGCATCAACCGCTTACCTCTTGATCAACAACCTGAGCAACGAGGACACCGCCAC<br>ATACTTCTGCGCTAGGAGCATCTACTACTATGGGAGCCGCTACTTCAATGTGTGGG<br>GAGCTGGGACAACTGTTACTGTGAGCTCAGGAGGGGGAGGTAGCGGAGGAGGCG<br>GTAGCGGAGGCGGTGGATCCGATATCCAGATGACACAGAGCACAAGTTCATTGTCC<br>GCCTCTCTTGGCGATCGAGTGACCATTAGCTGCCGAGCAAGCCAGGACATTAACAA<br>CTACCTGACCTGGTATCAGCAGAAACCCGACGGGACTGTGAAGCTGCTGATCTATT<br>ACACCTCTAGACTGCACAGTGGCGTTCCTAGCCGGTTTAGCGGATCCGGTAGCGG<br>AACAGATTACTCCCTGACCATCACCAATCTCGAGCAGGAAGACTTTGCCACCTACTT<br>TTGCCAGCAGGGCAATACTCTCCCATGGACATTTGGTGAGGGGACCAAAGTCGAGA<br>TTAAG |
| 2195. | IGF1R21HLxI2CHL | artificial | aa | QIQLVQSGPELKKPGETVKISCKASGYTLANYGMNWVKQAPGKGFKWMGWINTNTGK<br>PTYSDEFKGRFVFSLETSASTAYLLINNLSNEDTATYFCARSIYYYGSRYFNVWGAGTTV<br>TVSSGGGGSGGGGSGGGGSDIQMTQSTSSLSASLGDRVTISCRASQDINNYLTWYQQ<br>KPDGTVKLLIYYTSRLHSGVPSRFSGSGSGTDYSLTITNLEQEDFATYFCQQGNTLPWT<br>FGEGTKVEIKSGGGGSEVQLVESGGGLVQPGGSLKLSCAASGFTFNKYAMNWVRQAP<br>GKGLEWVARIRSKYNNYATYYADSVKDRFTISRDDSKNTAYLQMNNLKTEDTAVYYCV<br>RHGNFGNSYISYWAYWGQGTLVTVSSGGGGSGGGGSGGGGSQTVVTQEPSLTVSPG<br>GTVTLTCGSSTGAVTSGNYPNWVQQKPGQAPRGLIGGTKFLAPGTPARFSGSLLGGK<br>AALTLSGVQPEDEAEYYCVLWYSNRWVFGGGTKLTVL |
| 2196. | IGF1R21HLxI2CHL | artificial | nt | CAGATCCAGCTTGTCCAGAGTGGTCCCGAGCTGAAGAAACCTGGCGAAACCGTGA<br>AAATCAGCTGCAAGGCAAGCGGCTATACACTGGCCAACTATGGCATGAATTGGGTG<br>AAGCAGGCACCTGGGAAGGGTTTCAAGTGGATGGGCTGGATCAACACCAACACTG<br>GCAAACCAACCTATTCCGACGAGTTCAAGGGGCGTTTCGTGTTTTCACTGGAAACC<br>TCCGCATCAACCGCTTACCTCTTGATCAACAACCTGAGCAACGAGGACACCGCCAC<br>ATACTTCTGCGCTAGGAGCATCTACTACTATGGGAGCCGCTACTTCAATGTGTGGG<br>GAGCTGGGACAACTGTTACTGTGAGCTCAGGAGGGGGAGGTAGCGGAGGAGGCG<br>GTAGCGGAGGCGGTGGATCCGATATCCAGATGACACAGAGCACAAGTTCATTGTCC<br>GCCTCTCTTGGCGATCGAGTGACCATTAGCTGCCGAGCAAGCCAGGACATTAACAA<br>CTACCTGACCTGGTATCAGCAGAAACCCGACGGGACTGTGAAGCTGCTGATCTATT<br>ACACCTCTAGACTGCACAGTGGCGTTCCTAGCCGGTTTAGCGGATCCGGTAGCGG<br>AACAGATTACTCCCTGACCATCACCAATCTCGAGCAGGAAGACTTTGCCACCTACTT<br>TTGCCAGCAGGGCAATACTCTCCCATGGACATTTGGTGAGGGGACCAAAGTCGAGA<br>TTAAGTCCGGAGGTGGTGGATCCGAGGTGCAGCTGGTCGAGTCTGGAGGAGGATT<br>GGTGCAGCCTGGAGGGTCATTGAAACTCTCATGTGCAGCCTCTGGATTCACCTTCA |

| | | | | |
|---|---|---|---|---|
| | | | | ATAAGTACGCCATGAACTGGGTCCGCCAGGCTCCAGGAAAGGGTTTGGAATGGGTT GCTCGCATAAGAAGTAAATATAATAATTATGCAACATATTATGCCGATTCAGTGAAAG ACAGGTTCACCATCTCCAGAGATGATTCAAAAAACACTGCCTATCTACAAATGAACA ACTTGAAGACTGAGGACACTGCCGTGTACTACTGTGTGAGACATGGGAACTTCGGT AATAGCTACATATCCTACTGGGCTTACTGGGGCCAAGGGACTCTGGTCACCGTCTC CTCAGGTGGTGGTGGTTCTGGCGGCGGCGGCTCCGGTGGTGGTGGTTCTCAGACT GTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAAC AAAAACCAGGTCAGGCACCCCGTGGTCTAATAGGTGGGACTAAGTTCCTCGCCCCC GGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCA ACCGCTGGGTGTTCGGTGGAGGAACCAAACTGACTGTCCTA |
| 2197. | IGF1R23-H | artificial | aa | EVQLVESGGGLVQPGGSLRLSCAASGYTFTRFWIHWVRQAPGKGLEWVGEINPSNGR TNYNENFKNRFTISADTSKNTAYLQMNSLRAEDTAVYYCARGGRLDQWGQGTLVTVSS |
| 2198. | IGF1R23-HCDR1 | artificial | aa | RFWIH |
| 2199. | IGF1R23-HCDR2 | artificial | aa | EINPSNGRTNYNENFKN |
| 2200. | IGF1R23-HCDR3 | artificial | aa | GGRLDQ |
| 2201. | IGF1R23-H | artificial | nt | GAAGTCCAGCTTGTTGAAAGCGGAGGCGGTCTGGTTCAGCCTGGTGGATCTCTGA GACTGAGCTGCGCTGCAAGCGGCTACACATTCACACGCTTTTGGATTCATTGGGTG AGGCAGGCCCCTGGAAAAGGACTCGAATGGGTGGGCGAGATCAATCCTAGCAACG GTCGGACCAACTACAACGAGAACTTCAAGAACCGGTTCACCATCTCTGCCGACACC TCCAAGAATACCGCATACCTGCAGATGAATAGCCTCCGAGCCGAGGACACTGCCGT CTATTACTGTGCTAGGGGAGGACGCCTTGATCAGTGGGGACAGGGGACACTTGTG ACTGTGTCATCC |
| 2202. | IGF1R23-L | artificial | aa | DIQMTQSPSSLSASVGDRVTITCKASQNLRSKVAWYQQKPGKAPKLLIYSASYRYSGVP SRFSGSGSGTDFTLTISSLQPEDFATYYCQQYSNYPYTFGQGTKVEIK |
| 2203. | IGF1R23-LCDR1 | artificial | aa | KASQNLRSKVA |
| 2204. | IGF1R23-LCDR2 | artificial | aa | SASYRYS |
| 2205. | IGF1R23-LCDR3 | artificial | aa | QQYSNYPYT |
| 2206. | IGF1R23-L | artificial | nt | GATATTCAGATGACCCAGAGCCCAAGCTCCTTGAGCGCTAGCGTTGGCGATCGTGT GACTATCACCTGTAAGGCTAGCCAGAACCTGCGAAGCAAGGTCGCATGGTATCAGC AGAAACCCGGCAAAGCCCCAAAGCTGCTGATCTACTCCGCATCCTATCGGTATTCC GGCGTGCCAAGCAGATTCAGTGGGAGTGGGAGCGGAACTGACTTTACCCTCACCA TCTCATCCCTGCAGCCTGAGGACTTTGCCACCTACTATTGCCAGCAGTATTCCAACT ACCCCTACACCTTTGGCCAGGGCACAAAAGTGGAGATCAAG |
| 2207. | IGF1R23-HL | artificial | aa | EVQLVESGGGLVQPGGSLRLSCAASGYTFTRFWIHWVRQAPGKGLEWVGEINPSNGR |

| | | | | |
|---|---|---|---|---|
| | | | | TNYNENFKNRFTISADTSKNTAYLQMNSLRAEDTAVYYCARGGRLDQWGQGTLVTVSS<br>GGGGSGGGGSGGGGSDIQMTQSPSSLSASVGDRVTITCKASQNLRSKVAWYQQKPG<br>KAPKLLIYSASYRYSGVPSRFSGSGSGTDFTLTISSLQPEDFATYYCQQYSNYPYTFGQ<br>GTKVEIK |
| 2208. | IGF1R23-HL | artificial | nt | GAAGTCCAGCTTGTTGAAAGCGGAGGCGGTCTGGTTCAGCCTGGTGGATCTCTGA<br>GACTGAGCTGCGCTGCAAGCGGCTACACATTCACACGCTTTTGGATTCATTGGGTG<br>AGGCAGGCCCCTGGAAAAGGACTCGAATGGGTGGGCGAGATCAATCCTAGCAACG<br>GTCGGACCAACTACAACGAGAACTTCAAGAACCGGTTCACCATCTCTGCCGACACC<br>TCCAAGAATACCGCATACCTGCAGATGAATAGCCTCCGAGCCGAGGACACTGCCGT<br>CTATTACTGTGCTAGGGGAGGACGCCTTGATCAGTGGGGACAGGGGACACTTGTG<br>ACTGTGTCATCCGGTGGGGGAGGTAGTGGGGGAGGTGGAAGCGGCGGAGGAGGC<br>AGCGATATTCAGATGACCCAGAGCCCAAGCTCCTTGAGCGCTAGCGTTGGCGATCG<br>TGTGACTATCACCTGTAAGGCTAGCCAGAACCTGCGAAGCAAGGTCGCATGGTATC<br>AGCAGAAACCCGGCAAAGCCCCAAAGCTGCTGATCTACTCCGCATCCTATCGGTAT<br>TCCGGCGTGCCAAGCAGATTCAGTGGGAGTGGGAGCGGAACTGACTTTACCCTCA<br>CCATCTCATCCCTGCAGCCTGAGGACTTTGCCACCTACTATTGCCAGCAGTATTCCA<br>ACTACCCCTACACCTTTGGCCAGGGCACAAAAGTGGAGATCAAG |
| 2209. | IGF1R23HLxI2CHL | artificial | aa | EVQLVESGGGLVQPGGSLRLSCAASGYTFTRFWIHWVRQAPGKGLEWVGEINPSNGR<br>TNYNENFKNRFTISADTSKNTAYLQMNSLRAEDTAVYYCARGGRLDQWGQGTLVTVSS<br>GGGGSGGGGSGGGGSDIQMTQSPSSLSASVGDRVTITCKASQNLRSKVAWYQQKPG<br>KAPKLLIYSASYRYSGVPSRFSGSGSGTDFTLTISSLQPEDFATYYCQQYSNYPYTFGQ<br>GTKVEIKSGGGGSEVQLVESGGGLVQPGGSLKLSCAASGFTFNKYAMNWVRQAPGKG<br>LEWVARIRSKYNNYATYYADSVKDRFTISRDDSKNTAYLQMNNLKTEDTAVYYCVRHG<br>NFGNSYISYWAYWGQGTLVTVSSGGGGSGGGGSGGGGSQTVVTQEPSLTVSPGGTV<br>TLTCGSSTGAVTSGNYPNWVQQKPGQAPRGLIGGTKFLAPGTPARFSGSLLGGKAALT<br>LSGVQPEDEAEYYCVLWYSNRWVFGGGTKLTVL |
| 2210. | IGF1R23HLxI2CHL | artificial | nt | GAAGTCCAGCTTGTTGAAAGCGGAGGCGGTCTGGTTCAGCCTGGTGGATCTCTGA<br>GACTGAGCTGCGCTGCAAGCGGCTACACATTCACACGCTTTTGGATTCATTGGGTG<br>AGGCAGGCCCCTGGAAAAGGACTCGAATGGGTGGGCGAGATCAATCCTAGCAACG<br>GTCGGACCAACTACAACGAGAACTTCAAGAACCGGTTCACCATCTCTGCCGACACC<br>TCCAAGAATACCGCATACCTGCAGATGAATAGCCTCCGAGCCGAGGACACTGCCGT<br>CTATTACTGTGCTAGGGGAGGACGCCTTGATCAGTGGGGACAGGGGACACTTGTG<br>ACTGTGTCATCCGGTGGGGGAGGTAGTGGGGGAGGTGGAAGCGGCGGAGGAGGC<br>AGCGATATTCAGATGACCCAGAGCCCAAGCTCCTTGAGCGCTAGCGTTGGCGATCG<br>TGTGACTATCACCTGTAAGGCTAGCCAGAACCTGCGAAGCAAGGTCGCATGGTATC<br>AGCAGAAACCCGGCAAAGCCCCAAAGCTGCTGATCTACTCCGCATCCTATCGGTAT |

| | | | | |
|---|---|---|---|---|
| | | | | TCCGGCGTGCCAAGCAGATTCAGTGGGAGTGGGAGCGGAACTGACTTTACCCTCACCATCTCATCCCTGCAGCCTGAGGACTTTGCCACCTACTATTGCCAGCAGTATTCCAACTACCCCTACACCTTTGGCCAGGGCACAAAAGTGGAGATCAAGTCCGGAGGTGGTGGATCCGAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAGACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 2211. | IGF1R24-H | artificial | aa | EVQLVESGGELVQPGGSLRLSCAASGFTFSSYAMSWVRQAPGKGLEWVSRISPSGGSTYYADSVKGRFTISADTSKNTAYLQMNSLRAEDTAVYYCAREEYYYWGAMDVWGQGTLVTVSS |
| 2212. | IGF1R24-HCDR1 | artificial | aa | SYAMS |
| 2213. | IGF1R24-HCDR2 | artificial | aa | RISPSGGSTYYADSVKG |
| 2214. | IGF1R24-HCDR3 | artificial | aa | EEYYYWGAMDV |
| 2215. | IGF1R24-H | artificial | nt | GAAGTTCAGCTGGTGGAATCCGGGGGAGAGCTTGTTCAGCCTGGCGGATCACTTAGGCTGTCCTGTGCTGCAAGCGGCTTCACCTTCTCTAGCTATGCCATGTCATGGGTGCGACAGGCCCCTGGAAAAGGACTGGAGTGGGTCTCACGTATCTCTCCAAGTGGCGGATCCACATACTACGCCGATAGCGTGAAAGGGAGGTTTACCATTTCCGCTGACACCTCCAAGAACACCGCCTACCTGCAGATGAACTCACTGAGAGCCGAAGACACTGCCGTGTACTATTGCGCACGGGAGGAGTACTACTATTGGGGAGCTATGGACGTGTGGGGACAGGGAACATTGGTGACTGTGTCTTCC |
| 2216. | IGF1R24-L | artificial | aa | DIQMTQSPSSLSASVGDRVTITCRASQSISSYLAWYQQKPGKAPKLLIYGASSRASGVPSRFSGSGSGTDFTLTISSLQPEDFATYYCQQYYSSPLTFGQGTKVEIK |
| 2217. | IGF1R24-LCDR1 | artificial | aa | RASQSISSYLA |
| 2218. | IGF1R24-LCDR2 | artificial | aa | GASSRAS |
| 2219. | IGF1R24-LCDR3 | artificial | aa | QQYYSSPLT |
| 2220. | IGF1R24-L | artificial | nt | GATATCCAGATGACACAGAGCCCAAGCTCACTGAGCGCTAGCGTCGGAGATAGAGT |

| | | | | |
|---|---|---|---|---|
| | | | | CACCATCACCTGTAGGGCAAGCCAGAGCATCTCTAGCTACCTGGCATGGTACCAGCAGAAACCTGGCAAGGCTCCCAAGCTCCTGATCTACGGCGCTTCATCTAGAGCTAGCGGCGTTCCCTCACGATTTAGCGGAAGCGGAAGCGGAACTGACTTCACCCTGACCATTTCAAGCCTCCAGCCTGAGGATTTCGCCACCTACTACTGCCAGCAGTACTACTCTAGCCCACTCACATTTGGGCAGGGGACAAAGGTGGAGATCAAG |
| 2221. | IGF1R24-HL | artificial | aa | EVQLVESGGELVQPGGSLRLSCAASGFTFSSYAMSWVRQAPGKGLEWVSRISPSGGSTYYADSVKGRFTISADTSKNTAYLQMNSLRAEDTAVYYCAREEYYYWGAMDVWGQGTLVTVSSGGGGSGGGGSGGGGSDIQMTQSPSSLSASVGDRVTITCRASQSISSYLAWYQQKPGKAPKLLIYGASSRASGVPSRFSGSGSGTDFTLTISSLQPEDFATYYCQQYYSSPLTFGQGTKVEIK |
| 2222. | IGF1R24-HL | artificial | nt | GAAGTTCAGCTGGTGGAATCCGGGGGAGAGCTTGTTCAGCCTGGCGGATCACTTAGGCTGTCCTGTGCTGCAAGCGGCTTCACCTTCTCTAGCTATGCCATGTCATGGGTGCGACAGGCCCCTGGAAAAGGACTGGAGTGGGTCTCACGTATCTCTCCAAGTGGCGGATCCACATACTACGCCGATAGCGTGAAAGGGAGGTTTACCATTTCCGCTGACACCTCCAAGAACACCGCCTACCTGCAGATGAACTCACTGAGAGCCGAAGACACTGCCGTGTACTATTGCGCACGGGAGGAGTACTACTATTGGGGAGCTATGGACGTGTGGGGACAGGGAACATTGGTGACTGTGTCTTCCGGTGGCGGAGGAAGCGGAGGTGGCGGAAGCGGAGGCGGTGGAAGCGATATCCAGATGACACAGAGCCCAAGCTCACTGAGCGCTAGCGTCGGAGATAGAGTCACCATCACCTGTAGGGCAAGCCAGAGCATCTCTAGCTACCTGGCATGGTACCAGCAGAAACCTGGCAAGGCTCCCAAGCTCCTGATCTACGGCGCTTCATCTAGAGCTAGCGGCGTTCCCTCACGATTTAGCGGAAGCGGAAGCGGAACTGACTTCACCCTGACCATTTCAAGCCTCCAGCCTGAGGATTTCGCCACCTACTACTGCCAGCAGTACTACTCTAGCCCACTCACATTTGGGCAGGGGACAAAGGTGGAGATCAAG |
| 2223. | IGF1R24HLxI2CHL | artificial | aa | EVQLVESGGELVQPGGSLRLSCAASGFTFSSYAMSWVRQAPGKGLEWVSRISPSGGSTYYADSVKGRFTISADTSKNTAYLQMNSLRAEDTAVYYCAREEYYYWGAMDVWGQGTLVTVSSGGGGSGGGGSGGGGSDIQMTQSPSSLSASVGDRVTITCRASQSISSYLAWYQQKPGKAPKLLIYGASSRASGVPSRFSGSGSGTDFTLTISSLQPEDFATYYCQQYYSSPLTFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 2224. | IGF1R24HLxI2CHL | artificial | nt | GAAGTTCAGCTGGTGGAATCCGGGGGAGAGCTTGTTCAGCCTGGCGGATCACTTAGGCTGTCCTGTGCTGCAAGCGGCTTCACCTTCTCTAGCTATGCCATGTCATGGGTGCGACAGGCCCCTGGAAAAGGACTGGAGTGGGTCTCACGTATCTCTCCAAGTGGCG |

| | | | | |
|---|---|---|---|---|
| | | | | GATCCACATACTACGCCGATAGCGTGAAAGGGAGGTTTACCATTTCCGCTGACACC<br>TCCAAGAACACCGCCTACCTGCAGATGAACTCACTGAGAGCCGAAGACACTGCCGT<br>GTACTATTGCGCACGGGAGGAGTACTACTATTGGGGAGCTATGGACGTGTGGGGA<br>CAGGGAACATTGGTGACTGTGTCTTCCGGTGGCGGAGGAAGCGGAGGTGGCGGAA<br>GCGGAGGCGGTGGAAGCGATATCCAGATGACACAGAGCCCAAGCTCACTGAGCGC<br>TAGCGTCGGAGATAGAGTCACCATCACCTGTAGGGCAAGCCAGAGCATCTCTAGCT<br>ACCTGGCATGGTACCAGCAGAAACCTGGCAAGGCTCCCAAGCTCCTGATCTACGG<br>CGCTTCATCTAGAGCTAGCGGCGTTCCCTCACGATTTAGCGGAAGCGGAAGCGGA<br>ACTGACTTCACCCTGACCATTTCAAGCCTCCAGCCTGAGGATTTCGCCACCTACTAC<br>TGCCAGCAGTACTACTCTAGCCCACTCACATTTGGGCAGGGGACAAAGGTGGAGAT<br>CAAGTCCGGAGGTGGTGGATCCGAGGTGCAGCTGGTCGAGTCTGGAGGAGGATTG<br>GTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTT<br>GCTCGCATAAGAAGTAAATATAATAATTATGCAACATATTATGCCGATTCAGTGAAAG<br>ACAGGTTCACCATCTCCAGAGATGATTCAAAAAACACTGCCTATCTACAAATGAACA<br>ACTTGAAGACTGAGGACACTGCCGTGTACTACTGTGTGAGACATGGGAACTTCGGT<br>AATAGCTACATATCCTACTGGGCTTACTGGGGCCAAGGGACTCTGGTCACCGTCTC<br>CTCAGGTGGTGGTGGTTCTGGCGGCGGCGGCTCCGGTGGTGGTGGTTCTCAGACT<br>GTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAAC<br>AAAAACCAGGTCAGGCACCCCGTGGTCTAATAGGTGGGACTAAGTTCCTCGCCCCC<br>GGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGTTCTATGGTACAGCA<br>ACCGCTGGGTGTTCGGTGGAGGAACCAAACTGACTGTCCTA |
| 2225. | Macaca fascicularis CD3□ 1-27 | Macaca fasciculari s | aa | QDGNEEMGSITQTPYQVSISGTTILTC |
| 2226. | Macaca fascicularis CD3□ 1-27 | Macaca fasciculari s | aa | QDGNEEMGSITQTPYQVSISGTTVILT |
| 2227. | Macaca mulatta CD3□ 1-27 | Macaca mulatta | aa | QDGNEEMGSITQTPYHVSISGTTVILT |

## Claims

1. A bispecific single chain antibody molecule comprising a first binding domain which specifically binds to an epitope of human and Callithrix jacchus, Saguinus oedipus or Saimiri sciureus CD3ε (epsilon) chain, wherein said epitope is part of an amino acid sequence comprised in the group consisting of SEQ ID NOs. 2, 4, 6, or 8 and comprises at least the amino acid sequence Gln-Asp-Gly-Asn-Glu, and a second binding domain binding to an antigen selected from the group consisting of Prostate Stem Cell Antigen (PSCA), B-Lymphocyte antigen CD19 (CD19), hepatocyte growth factor receptor (C-MET), Endosialin, the EGF-like domain 1 of EpCAM, encoded by exon 2, Fibroblast activation protein alpha (FAP alpha) and Insulin-like growth factor I receptor (IGF-IR or IGF-1R).

2. The bispecific single chain antibody molecule according to claim 1, wherein the first binding domain comprises a VL region comprising CDR-L1, CDR-L2 and CDR-L3 selected from:

   (a) CDR-L1 as depicted in SEQ ID NO. 27, CDR-L2 as depicted in SEQ ID NO. 28 and CDR-L3 as depicted in SEQ ID NO. 29;
   (b) CDR-L1 as depicted in SEQ ID NO. 117, CDR-L2 as depicted in SEQ ID NO. 118 and CDR-L3 as depicted in SEQ ID NO. 119; and
   (c) CDR-L1 as depicted in SEQ ID NO. 153, CDR-L2 as depicted in SEQ ID NO. 154 and CDR-L3 as depicted in SEQ ID NO. 155.

3. The bispecific single chain antibody molecule according to claim 1 or 2, wherein the first binding domain comprises a VH region comprising CDR-H 1, CDR-H2 and CDR-H3 selected from:

   (a) CDR-H1 as depicted in SEQ ID NO. 12, CDR-H2 as depicted in SEQ ID NO. 13 and CDR-H3 as depicted in SEQ ID NO. 14;
   (b) CDR-H1 as depicted in SEQ ID NO. 30, CDR-H2 as depicted in SEQ ID NO. 31 and CDR-H3 as depicted in SEQ ID NO. 32;
   (c) CDR-H1 as depicted in SEQ ID NO. 48, CDR-H2 as depicted in SEQ ID NO. 49 and CDR-H3 as depicted in SEQ ID NO. 50;
   (d) CDR-H1 as depicted in SEQ ID NO. 66, CDR-H2 as depicted in SEQ ID NO. 67 and CDR-H3 as depicted in SEQ ID NO. 68;
   (e) CDR-H1 as depicted in SEQ ID NO. 84, CDR-H2 as depicted in SEQ ID NO. 85 and CDR-H3 as depicted in SEQ ID NO. 86;
   (f) CDR-H1 as depicted in SEQ ID NO. 102, CDR-H2 as depicted in SEQ ID NO. 103 and CDR-H3 as depicted in SEQ ID NO. 104;
   (g) CDR-H1 as depicted in SEQ ID NO. 120, CDR-H2 as depicted in SEQ ID NO. 121 and CDR-H3 as depicted in SEQ ID NO. 122;
   (h) CDR-H1 as depicted in SEQ ID NO. 138, CDR-H2 as depicted in SEQ ID NO. 139 and CDR-H3 as depicted in SEQ ID NO. 140;
   (i) CDR-H1 as depicted in SEQ ID NO. 156, CDR-H2 as depicted in SEQ ID NO. 157 and CDR-H3 as depicted in SEQ ID NO. 158; and
   (j) CDR-H1 as depicted in SEQ ID NO. 174, CDR-H2 as depicted in SEQ ID NO. 175 and CDR-H3 as depicted in SEQ ID NO. 176.

4. The bispecific single chain antibody molecule according to any one of claims 1 to 3, wherein the second binding domain is selected from the group consisting of binding domains binding to human Prostate Stem Cell Antigen (PSCA) and/or a non-human primate PSCA, binding to human B-Lymphocyte antigen CD19 (CD19), and/or a non-human primate CD19, binding to human hepatocyte growth factor receptor (C-MET), and/or a non-human primate C-MET, binding to human Endosialin, and/or a non-human primate Endosialin, binding to human EGF-like domain 1 of EpCAM, encoded by exon 2, and/or a non-human primate EGF-like domain 1 of EpCAM, encoded by exon 2, binding to human Fibroblast activation protein alpha (FAP alpha), and/or a non-human primate FAP alpha, and binding to human Insulin-like growth factor I receptor (IGF-IR or IGF-1 R), and/or a non-human primate IGF-1 R.

5. The bispecific single chain antibody molecule according to claim 4 binding to human Prostate Stem Cell Antigen (PSCA) and/or a non-human primate PSCA, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 382 - 384 and CDR-L1-3 of SEQ ID NO: 377 - 379;
b) CDR-H1-3 of SEQ ID NO: 400 - 402 and CDR-L1-3 of SEQ ID NO: 395 - 397;
c) CDR-H1-3 of SEQ ID NO: 414 - 416 and CDR-L1-3 of SEQ ID NO: 409 - 411;
d) CDR-H1-3 of SEQ ID NO: 432 - 434 and CDR-L1-3 of SEQ ID NO: 427 - 429;
e) CDR-H1-3 of SEQ ID NO: 1215 - 1217 and CDR-L1-3 of SEQ ID NO: 1220 - 1222;
f) CDR-H1-3 of SEQ ID NO: 1187 - 1189 and CDR-L1-3 of SEQ ID NO: 1192 - 1194;
g) CDR-H1-3 of SEQ ID NO: 1173 - 1175 and CDR-L1-3 of SEQ ID NO: 1178 - 1180;
h) CDR-H1-3 of SEQ ID NO: 1229 - 1231 and CDR-L1-3 of SEQ ID NO: 1234 - 1236;
i) CDR-H1-3 of SEQ ID NO: 1201 - 1203 and CDR-L1-3 of SEQ ID NO: 1206 - 1208;
k) CDR-H1-3 of SEQ ID NO: 1257 - 1259 and CDR-L1-3 of SEQ ID NO: 1262 - 1264; and
l) CDR-H1-3 of SEQ ID NO: 1243 - 1245 and CDR-L1-3 of SEQ ID NO: 1248 - 1250.

**6.** The bispecific single chain antibody molecule according to claim 4 binding to human B-Lymphocyte antigen CD19 (CD19), and/or a non-human primate CD19, wherein the bispecific single chain antibody molecule comprises a group of the following sequences as CDR H1, CDR H2, CDR H3, CDR L1, CDR L2 and CDR L3 in the second binding domain selected from:

a) CDR-H1-3 of SEQ ID NO: 478 - 480 and CDR-L1-3 of SEQ ID NO: 473 - 475;
b) CDR-H1-3 of SEQ ID NO: 530 - 532 and CDR-L1-3 of SEQ ID NO: 525 - 527;
c) CDR-H1-3 of SEQ ID NO: 518 - 520 and CDR-L1-3 of SEQ ID NO: 513 - 515;
d) CDR-H1-3 of SEQ ID NO: 506 - 508 and CDR-L1-3 of SEQ ID NO: 501 - 503;
e) CDR-H1-3 of SEQ ID NO: 494 - 496 and CDR-L1-3 of SEQ ID NO: 489 - 491;
f) CDR-H1-3 of SEQ ID NO: 542 - 544 and CDR-L1-3 of SEQ ID NO: 537 - 539;
g) CDR-H1-3 of SEQ ID NO: 554 - 556 and CDR-L1-3 of SEQ ID NO: 549 - 551;
h) CDR-H1-3 of SEQ ID NO: 566 - 568 and CDR-L1-3 of SEQ ID NO: 561 - 563;
i) CDR-H1-3 of SEQ ID NO: 578 - 580 and CDR-L1-3 of SEQ ID NO: 573 - 575;
j) CDR-H1-3 of SEQ ID NO: 590 - 592 and CDR-L1-3 of SEQ ID NO: 585 - 587;
k) CDR-H1-3 of SEQ ID NO: 602 - 604 and CDR-L1-3 of SEQ ID NO: 597 - 599;
l) CDR-H1-3 of SEQ ID NO: 614 - 616 and CDR-L1-3 of SEQ ID NO: 609 - 611;
m) CDR-H1-3 of SEQ ID NO: 626 - 628 and CDR-L1-3 of SEQ ID NO: 621 - 623;
n) CDR-H1-3 of SEQ ID NO: 638 - 640 and CDR-L1-3 of SEQ ID NO: 633 - 635;
o) CDR-H1-3 of SEQ ID NO: 650 - 652 and CDR-L1-3 of SEQ ID NO: 645 - 647;
p) CDR-H1-3 of SEQ ID NO: 662 - 664 and CDR-L1-3 of SEQ ID NO: 657 - 659;
q) CDR-H1-3 of SEQ ID NO: 674 - 676 and CDR-L1-3 of SEQ ID NO: 669 - 671;
r) CDR-H1-3 of SEQ ID NO: 686 - 688 and CDR-L1-3 of SEQ ID NO: 681 - 683;
s) CDR-H1-3 of SEQ ID NO: 698 - 700 and CDR-L1-3 of SEQ ID NO: 693 - 695;
t) CDR-H1-3 of SEQ ID NO: 710 - 712 and CDR-L1-3 of SEQ ID NO: 705 - 707;
u) CDR-H1-3 of SEQ ID NO: 722 - 724 and CDR-L1-3 of SEQ ID NO: 717 - 719;
v) CDR-H1-3 of SEQ ID NO: 734 - 736 and CDR-L1-3 of SEQ ID NO: 729 - 731;
w) CDR-H1-3 of SEQ ID NO: 746 - 748 and CDR-L1-3 of SEQ ID NO: 741 - 743;
x) CDR-H1-3 of SEQ ID NO: 758 - 760 and CDR-L1-3 of SEQ ID NO: 753 - 755;
y) CDR-H1-3 of SEQ ID NO: 1271 - 1273 and CDR-L1-3 of SEQ ID NO: 1276 - 1278;
z) CDR-H1-3 of SEQ ID NO: 1285 - 1287 and CDR-L1-3 of SEQ ID NO: 1290 - 1292;
aa) CDR-H1-3 of SEQ ID NO: 1299 - 1301 and CDR-L1-3 of SEQ ID NO: 1304 - 1306;
ab) CDR-H1-3 of SEQ ID NO: 1313 - 1315 and CDR-L1-3 of SEQ ID NO: 1318 - 1320;
ac) CDR-H1-3 of SEQ ID NO: 1327 - 1329 and CDR-L1-3 of SEQ ID NO: 1332 - 1334;
ad) CDR-H1-3 of SEQ ID NO: 1341 - 1343 and CDR-L1-3 of SEQ ID NO: 1346 - 1348;
ae) CDR-H1-3 of SEQ ID NO: 1355 - 1357 and CDR-L1-3 of SEQ ID NO: 1360 - 1362;
af) CDR-H1-3 of SEQ ID NO: 1369 - 1371 and CDR-L1-3 of SEQ ID NO: 1374 - 1376; and
ag) CDR-H1-3 of SEQ ID NO: 1383 - 1385 and CDR-L1-3 of SEQ ID NO: 1388 - 1390.

**7.** The bispecific single chain antibody molecule according to claim 4 binding to human hepatocyte growth factor receptor (C-MET), and/or a non-human primate C-MET, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 821 - 823 and CDR-L1-3 of SEQ ID NO: 816 - 818;
b) CDR-H1-3 of SEQ ID NO: 836 - 838 and CDR-L1-3 of SEQ ID NO: 833 - 835;

c) CDR-H1-3 of SEQ ID NO: 845 - 847 and CDR-L1-3 of SEQ ID NO: 840 - 842;
d) CDR-H1-3 of SEQ ID NO: 863 - 865 and CDR-L1-3 of SEQ ID NO: 858 - 860;
e) CDR-H1-3 of SEQ ID NO: 881 - 883 and CDR-L1-3 of SEQ ID NO: 876 - 878;
f) CDR-H1-3 of SEQ ID NO: 899 - 901 and CDR-L1-3 of SEQ ID NO: 894 - 896;
g) CDR-H1-3 of SEQ ID NO: 1401 - 1403 and CDR-L1-3 of SEQ ID NO: 1406 - 1408;
h) CDR-H1-3 of SEQ ID NO: 1415 - 1417 and CDR-L1-3 of SEQ ID NO: 1420 - 1422;
i) CDR-H1-3 of SEQ ID NO: 1429 - 1431 and CDR-L1-3 of SEQ ID NO: 1434 - 1436;
j) CDR-H1-3 of SEQ ID NO: 1443 - 1445 and CDR-L1-3 of SEQ ID NO: 1448 - 1450;
k) CDR-H1-3 of SEQ ID NO: 1457 - 1459 and CDR-L1-3 of SEQ ID NO: 1462 - 1464;
l) CDR-H1-3 of SEQ ID NO: 1471 - 1473 and CDR-L1-3 of SEQ ID NO: 1476 - 1478;
m) CDR-H1-3 of SEQ ID NO: 1639 - 1641 and CDR-L1-3 of SEQ ID NO: 1644 - 1646;
n) CDR-H1-3 of SEQ ID NO: 1625 - 1627 and CDR-L1-3 of SEQ ID NO: 1630 - 1632;
o) CDR-H1-3 of SEQ ID NO: 1611 - 1613 and CDR-L1-3 of SEQ ID NO: 1616 - 1618;
p) CDR-H1-3 of SEQ ID NO: 1597 - 1599 and CDR-L1-3 of SEQ ID NO: 1602-1604;
q) CDR-H1-3 of SEQ ID NO: 1569 - 1571 and CDR-L1-3 of SEQ ID NO: 1574-1576;
r) CDR-H1-3 of SEQ ID NO: 1555 - 1557 and CDR-L1-3 of SEQ ID NO: 1560-1562;
s) CDR-H1-3 of SEQ ID NO: 1583 - 1585 and CDR-L1-3 of SEQ ID NO: 1588 - 1590;
t) CDR-H1-3 of SEQ ID NO: 1541 - 1543 and CDR-L1-3 of SEQ ID NO: 1546 - 1548;
u) CDR-H1-3 of SEQ ID NO: 1513 - 1515 and CDR-L1-3 of SEQ ID NO: 1518 - 1520;
v) CDR-H1-3 of SEQ ID NO: 1527 - 1529 and CDR-L1-3 of SEQ ID NO: 1532 - 1534;
w) CDR-H1-3 of SEQ ID NO: 1499 - 1501 and CDR-L1-3 of SEQ ID NO: 1504 - 1506; and
x) CDR-H1-3 of SEQ ID NO: 1485 - 1487 and CDR-L1-3 of SEQ ID NO: 1490 - 1492.

**8.** The bispecific single chain antibody molecule according to claim 4 binding to human Endosialin, and/or a non-human primate Endosialin, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 1653 - 1655 and CDR-L1-3 of SEQ ID NO: 1658 - 1660;
b) CDR-H1-3 of SEQ ID NO: 1667 - 1669 and CDR-L1-3 of SEQ ID NO: 1672 - 1674;
c) CDR-H1-3 of SEQ ID NO: 1681 - 1683 and CDR-L1-3 of SEQ ID NO: 1686 - 1688;
d) CDR-H1-3 of SEQ ID NO: 1695 - 1697 and CDR-L1-3 of SEQ ID NO: 1700-1702;
e) CDR-H1-3 of SEQ ID NO: 1709 - 1711 and CDR-L1-3 of SEQ ID NO: 1714 - 1716; and
f) CDR-H1-3 of SEQ ID NO: 1723 - 1725 and CDR-L1-3 of SEQ ID NO: 1728 - 1730.

**9.** The bispecific single chain antibody molecule according to claim 4 binding to human EGF-like domain 1 of EpCAM, encoded by exon 2, and/or a non-human primate EGF-like domain 1 of EpCAM, encoded by exon 2, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 940 - 942 and CDR-L1-3 of SEQ ID NO: 935 - 937;
b) CDR-H1-3 of SEQ ID NO: 956 - 958 and CDR-L1-3 of SEQ ID NO: 951 - 953;
c) CDR-H1-3 of SEQ ID NO: 968 - 970 and CDR-L1-3 of SEQ ID NO: 963 - 965;
d) CDR-H1-3 of SEQ ID NO: 980 - 982 and CDR-L1-3 of SEQ ID NO: 975-977;
e) CDR-H1-3 of SEQ ID NO: 992 - 994 and CDR-L1-3 of SEQ ID NO: 987 - 989;
f) CDR-H1-3 of SEQ ID NO: 1004 - 1006 and CDR-L1-3 of SEQ ID NO: 999 - 1001;
g) CDR-H1-3 of SEQ ID NO: 1028 - 1030 and CDR-L1-3 of SEQ ID NO: 1023-1025;
h) CDR-H1-3 of SEQ ID NO: 1040 - 1042 and CDR-L1-3 of SEQ ID NO: 1035-1037;
i) CDR-H1-3 of SEQ ID NO: 1052 - 1054 and CDR-L1-3 of SEQ ID NO: 1047 - 1049;
j) CDR-H1-3 of SEQ ID NO: 1074 - 1076 and CDR-L1-3 of SEQ ID NO: 1069 - 1071;
k) CDR-H1-3 of SEQ ID NO: 1086 - 1088 and CDR-L1-3 of SEQ ID NO: 1081 - 1083;
l) CDR-H1-3 of SEQ ID NO: 1098 - 1100 and CDR-L1-3 of SEQ ID NO: 1093 - 1095;
m) CDR-H1-3 of SEQ ID NO: 1110 - 1112 and CDR-L1-3 of SEQ ID NO: 1105-1107;
n) CDR-H1-3 of SEQ ID NO: 1122 - 1124 and CDR-L1-3 of SEQ ID NO: 1117-1119;
o) CDR-H1-3 of SEQ ID NO: 1016 - 1018 and CDR-L1-3 of SEQ ID NO: 1011 - 1013; and
p) CDR-H1-3 of SEQ ID NO: 1765 - 1767 and CDR-L1-3 of SEQ ID NO: 1770-1772.

**10.** The bispecific single chain antibody molecule according to claim 4 binding to human Fibroblast activation protein alpha (FAP alpha), and/or a non-human primate FAP alpha, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 1137 - 1139 and CDR-L1-3 of SEQ ID NO: 1132 - 1134;
b) CDR-H1-3 of SEQ ID NO: 1849 - 1851 and CDR-L1-3 of SEQ ID NO: 1854 - 1856;
c) CDR-H1-3 of SEQ ID NO: 1835 - 1837 and CDR-L1-3 of SEQ ID NO: 1840 - 1842;
d) CDR-H1-3 of SEQ ID NO: 1779 - 1781 and CDR-L1-3 of SEQ ID NO: 1784 - 1786;
e) CDR-H1-3 of SEQ ID NO: 1793 - 1795 and CDR-L1-3 of SEQ ID NO: 1798-1800;
f) CDR-H1-3 of SEQ ID NO: 1863 - 1865 and CDR-L1-3 of SEQ ID NO: 1868 - 1870;
g) CDR-H1-3 of SEQ ID NO: 1807 - 1809 and CDR-L1-3 of SEQ ID NO: 1812-1814;
h) CDR-H1-3 of SEQ ID NO: 1821 - 1823 and CDR-L1-3 of SEQ ID NO: 1826-1828;
i) CDR-H1-3 of SEQ ID NO: 1891 - 1893 and CDR-L1-3 of SEQ ID NO: 1896 - 1898;
j) CDR-H1-3 of SEQ ID NO: 1877 - 1879 and CDR-L1-3 of SEQ ID NO: 1882 - 1884;
k) CDR-H1-3 of SEQ ID NO: 1961 - 1963 and CDR-L1-3 of SEQ ID NO: 1966 - 1968;
l) CDR-H1-3 of SEQ ID NO: 1947 - 1949 and CDR-L1-3 of SEQ ID NO: 1952-1954;
m) CDR-H1-3 of SEQ ID NO: 1975 - 1977 and CDR-L1-3 of SEQ ID NO: 1980-1982;
n) CDR-H1-3 of SEQ ID NO: 1933 - 1935 and CDR-L1-3 of SEQ ID NO: 1938-1940;
o) CDR-H1-3 of SEQ ID NO: 1919 - 1921 and CDR-L1-3 of SEQ ID NO: 1924-1926; and
p) CDR-H1-3 of SEQ ID NO: 1905 - 1907 and CDR-L1-3 of SEQ ID NO: 1910-1912.

**11.** The bispecific single chain antibody molecule according to claim 4 binding to human Insulin-like growth factor I receptor (IGF-IR or IGF-1R), and/or a non-human primate IGF-1R, wherein the bispecific single chain antibody molecule comprises a group of the following sequences as CDR-H1, CDR-H2, CDR-H3, CDR-L1, CDR-L2 and CDR-L3 in the second binding domain selected from:

a) CDR-H1-3 of SEQ ID NO: 2016 - 2018 and CDR-L1-3 of SEQ ID NO: 2021 - 2023;
b) CDR-H1-3 of SEQ ID NO: 2030 - 2032 and CDR-L1-3 of SEQ ID NO: 2035 - 2037;
c) CDR-H1-3 of SEQ ID NO: 2044 - 2046 and CDR-L1-3 of SEQ ID NO: 2049 - 2051;
d) CDR-H1-3 of SEQ ID NO: 2058 - 2060 and CDR-L1-3 of SEQ ID NO: 2063-2065;
e) CDR-H1-3 of SEQ ID NO: 2072 - 2074 and CDR-L1-3 of SEQ ID NO: 2077-2079;
f) CDR-H1-3 of SEQ ID NO: 2086 - 2088 and CDR-L1-3 of SEQ ID NO: 2091 - 2093;
g) CDR-H1-3 of SEQ ID NO: 2100 - 2102 and CDR-L1-3 of SEQ ID NO: 2105-2107;
h) CDR-H1-3 of SEQ ID NO: 2114 - 2116 and CDR-L1-3 of SEQ ID NO: 2119-2121;
i) CDR-H1-3 of SEQ ID NO: 2128 - 2130 and CDR-L1-3 of SEQ ID NO: 2133 - 2135;
j) CDR-H1-3 of SEQ ID NO: 2142 - 2144 and CDR-L1-3 of SEQ ID NO: 2147 - 2149:
k) CDR-H1-3 of SEQ ID NO: 2156 - 2158 and CDR-L1-3 of SEQ ID NO: 2161-2163;
l) CDR-H1-3 of SEQ ID NO: 2170 - 2172 and CDR-L1-3 of SEQ ID NO: 2175 - 2177;
m) CDR-H1-3 of SEQ ID NO: 2184 - 2186 and CDR-L1-3 of SEQ ID NO: 2189 - 2191;
n) CDR-H1-3 of SEQ ID NO: 2198 - 2200 and CDR-L1-3 of SEQ ID NO: 2203-2205; and
o) CDR-H1-3 of SEQ ID NO: 2212 - 2214 and CDR-L1-3 of SEQ ID NO: 2217 - 2219.

**12.** A nucleic acid sequence encoding a bispecific single chain antibody molecule as defined in any of claims 1 to 11.

**13.** A vector, which comprises a nucleic acid sequence as defined in claim 12.

**14.** A host cell transformed or transfected with a vector defined in claim 13.

**15.** A process for the production of a bispecific single chain antibody molecule according to any of claims 1 to 11, said process comprising culturing a host cell defined in claim 14 under conditions allowing the expression of the polypeptide as defined in any of claims 1 to 11 and recovering the produced polypeptide from the culture.

**16.** A pharmaceutical composition comprising a bispecific single chain antibody molecule according to any one of claims 1 to 11, or produced according to the process of claim 15.

**17.** The bispecific single chain antibody molecule of claim 1, wherein said first binding domain binds to the N-terminal CD3 epsilon polypeptide fragment in vitro and to the native CD3 epsilon subunit of the CD3 complex on T cells in vivo with the same binding affinity.

18. The bispecific single chain antibody molecule of claim 1, wherein the homogeneity of the bispecific single chain antibody molecule is at least 90%, 95% or 98%.

**Patentansprüche**

1. Bispezifisches Einzelketten-Antikörpermolekül, umfassend eine erste Bindedomäne, die spezifisch an ein Epitop der menschlichen und Callithrix jacchus, Saguinus oedipus oder Saimiri sciureus CD3ε (epsilon)-Kette bindet, wobei das Epitop Teil einer Aminosäuresequenz ist, die umfasst ist in der Gruppe bestehend aus SEQ ID NOs. 2, 4, 6 oder 8, und mindestens die Aminosäuresequenz Gln-Asp-Gly-Asn-Glu umfasst, und eine zweite Bindedomäne, welches an ein Antigen, ausgewählt aus der Gruppe bestehend aus Prostata-Stammzell-Antigen (PSCA), B-Lymphozyten-Antigen CD19 (CD19), Hepatozyten-Wachstumsfaktor-Rezeptor (C-MET), Endosialin, die EGF-ähnliche Domäne 1 von EpCAM, die von Exon 2 codiert wird, Fibroblasten-Aktivierungsprotein alpha (FAP alpha) und Insulin-ähnlicher Wachstumsfaktor-I-Rezeptor (IGF-IR oder IGF-1R), bindet.

2. Bispezifisches Einzelketten-Antikörpermolekül gemäß Anspruch 1, wobei die erste Bindungsdomäne eine VL-Region umfasst, umfassend CDR-L1, CDR-L2 und CDR-L3 ausgewählt aus:

(a) CDR-L1 wie dargestellt in SEQ ID NO. 27, CDR-L2 wie dargestellt in SEQ ID NO. 28 und CDR-L3 wie dargestellt in SEQ ID NO. 29;
(b) CDR-L1 wie dargestellt in SEQ ID NO. 117, CDR-L2 wie dargestellt in SEQ ID NO. 118 und CDR-L3 wie dargestellt in SEQ ID NO. 119; und
(c) CDR-L1 wie dargestellt in SEQ ID NO. 153, CDR-L2 wie dargestellt in SEQ ID NO. 154 und CDR-L3 wie dargestellt in SEQ ID NO. 155.

3. Bispezifisches Einzelketten-Antikörpermolekül gemäß Anspruch 1 oder 2, wobei die erste Bindungsdomäne eine VH-Region umfasst, umfassend CDR-H1, CDR-H2 und CDR-H3 ausgewählt aus:

(a) CDR-H1 wie dargestellt in SEQ ID NO. 12, CDR-H2 wie dargestellt in SEQ ID NO. 13 und CDR-H3 wie dargestellt in SEQ ID NO. 14;
(b) CDR-H1 wie dargestellt in SEQ ID NO. 30, CDR-H2 wie dargestellt in SEQ ID NO. 31 und CDR-H3 wie dargestellt in SEQ ID NO. 32;
(c) CDR-H1 wie dargestellt in SEQ ID NO. 48, CDR-H2 wie dargestellt in SEQ ID NO. 49 und CDR-H3 wie dargestellt in SEQ ID NO. 50;
(d) CDR-H1 wie dargestellt in SEQ ID NO. 66, CDR-H2 wie dargestellt in SEQ ID NO. 67 und CDR-H3 wie dargestellt in SEQ ID NO. 68;
(e) CDR-H1 wie dargestellt in SEQ ID NO. 84, CDR-H2 wie dargestellt in SEQ ID NO. 85 und CDR-H3 wie dargestellt in SEQ ID NO. 86;
(f) CDR-H1 wie dargestellt in SEQ ID NO. 102, CDR-H2 wie dargestellt in SEQ ID NO. 103 und CDR-H3 wie dargestellt in SEQ ID NO. 104;
(g) CDR-H1 wie dargestellt in SEQ ID NO. 120, CDR-H2 wie dargestellt in SEQ ID NO. 121 und CDR-H3 wie dargestellt in SEQ ID NO. 122;
(h) CDR-H1 wie dargestellt in SEQ ID NO. 138, CDR-H2 wie dargestellt in SEQ ID NO. 139 und CDR-H3 wie dargestellt in SEQ ID NO. 140;
(i) CDR-H1 wie dargestellt in SEQ ID NO. 156, CDR-H2 wie dargestellt in SEQ ID NO. 157 und CDR-H3 wie dargestellt in SEQ ID NO. 158; und
(j) CDR-H1 wie dargestellt in SEQ ID NO. 174, CDR-H2 wie dargestellt in SEQ ID NO. 175 und CDR-H3 wie dargestellt in SEQ ID NO. 176.

4. Bispezifisches Einzelketten-Antikörpermolekül gemäß einem der Ansprüche 1 bis 3, wobei die zweite Bindungsdomäne ausgewählt ist aus der Gruppe bestehend aus Bindedomänen, die an menschliches Prostata-Stammzell-Antigen (PSCA) und/oder ein nicht-menschliches Primaten PSCA binden, die an menschliches B-Lymphozyten-Antigen CD 19 (CD 19) und/oder ein nicht-menschliches Primaten-CD19 binden, die an menschlichen Hepatozyten-Wachstumsfaktor-Rezeptor (C-MET) und/oder einen nicht-menschlichen Primaten-C-MET binden, die an menschliches Endosialin und/oder ein nicht-menschliches Primaten-Endosialin binden, die an menschliche EGF-ähnliche Domäne 1 von EpCAM, die von Exon 2 codiert wird, und/oder eine nicht-menschliche Primaten-EGF-ähnliche Domäne 1 von EpCAM, die von Exon 2 codiert wird, binden, die an menschliches Fibroblasten-Aktivierungsprotein alpha (FAP alpha) und/oder ein nicht-menschliches Primaten-FAP alpha binden, und die an menschlichen Insulin-ähnlicher Wachstumsfaktor-I-Rezeptor (IGF-IR oder IGF-1R) und/oder einen nicht-menschlichen Primaten IGF-1R binden.

**5.** Bispezifisches Einzelketten-Antikörpermolekül gemäß Anspruch 4, das an menschliches Prostata-Stammzell-Antigen (PSCA) und/oder ein nicht-menschliches Primaten-PSCA bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 382 - 384 und CDR-L1-3 von SEQ ID NO: 377 - 379;
b) CDR-H1-3 von SEQ ID NO: 400 - 402 und CDR-L1-3 von SEQ ID NO: 395 - 397;
c) CDR-H1-3 von SEQ ID NO: 414 - 416 und CDR-L1-3 von SEQ ID NO: 409 - 411;
d) CDR-H1-3 von SEQ ID NO: 432 - 434 und CDR-L1-3 von SEQ ID NO: 427 - 429;
e) CDR-H1-3 von SEQ ID NO: 1215 - 1217 und CDR-L1-3 von SEQ ID NO: 1220 - 1222;
f) CDR-H1-3 von SEQ ID NO: 1187 - 1189 und CDR-L1-3 von SEQ ID NO: 1192 - 1194;
g) CDR-H1-3 von SEQ ID NO: 1173 - 1175 und CDR-L1-3 von SEQ ID NO: 1178-1180;
h) CDR-H1-3 von SEQ ID NO: 1229 - 1231 und CDR-L1-3 von SEQ ID NO: 1234 - 1236;
i) CDR-H1-3 von SEQ ID NO: 1201 - 1203 und CDR-L1-3 von SEQ ID NO: 1206 - 1208;
k) CDR-H1-3 von SEQ ID NO: 1257 - 1259 und CDR-L1-3 von SEQ ID NO: 1262 - 1264; und
1) CDR-H1-3 von SEQ ID NO: 1243 - 1245 und CDR-L1-3 von SEQ ID NO: 1248 - 1250.

**6.** Bispezifisches Einzelketten-Antikörpermolekül gemäß Anspruch 4, das an menschliches B-Lymphozyten-Antigen CD 19 (CD 19) und/oder ein nicht-menschliches Primaten-CD19 bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 478 - 480 und CDR-L1-3 von SEQ ID NO: 473 - 475;
b) CDR-H1-3 von SEQ ID NO: 530 - 532 und CDR-L1-3 von SEQ ID NO: 525 - 527;
c) CDR-H1-3 von SEQ ID NO: 518 - 520 und CDR-L1-3 von SEQ ID NO: 513 - 515;
d) CDR-H1-3 von SEQ ID NO: 506 - 508 und CDR-L1-3 von SEQ ID NO: 501 - 503;
e) CDR-H1-3 von SEQ ID NO: 494 - 496 und CDR-L1-3 von SEQ ID NO: 489 - 491;
f) CDR-H1-3 von SEQ ID NO: 542 - 544 und CDR-L1-3 von SEQ ID NO: 537 - 539;
g) CDR-H1-3 von SEQ ID NO: 554 - 556 und CDR-L1-3 von SEQ ID NO: 549 - 551;
h) CDR-H1-3 von SEQ ID NO: 566 - 568 und CDR-L1-3 von SEQ ID NO: 561 - 563;
i) CDR-H1-3 von SEQ ID NO: 578 - 580 und CDR-L1-3 von SEQ ID NO: 573 - 575;
j) CDR-H1-3 von SEQ ID NO: 590 - 592 und CDR-L1-3 von SEQ ID NO: 585 - 587;
k) CDR-H1-3 von SEQ ID NO: 602 - 604 und CDR-L1-3 von SEQ ID NO: 597 - 599;
l) CDR-H1-3 von SEQ ID NO: 614 - 616 und CDR-L1-3 von SEQ ID NO: 609 - 611;
m) CDR-H1-3 von SEQ ID NO: 626 - 628 und CDR-L1-3 von SEQ ID NO: 621 - 623;
n) CDR-H1-3 von SEQ ID NO: 638 - 640 und CDR-L1-3 von SEQ ID NO: 633 - 635;
o) CDR-H1-3 von SEQ ID NO: 650 - 652 und CDR-L1-3 von SEQ ID NO: 645 - 647;
p) CDR-H1-3 von SEQ ID NO: 662 - 664 und CDR-L1-3 von SEQ ID NO: 657 - 659;
q) CDR-H1-3 von SEQ ID NO: 674 - 676 und CDR-L1-3 von SEQ ID NO: 669 - 671;
r) CDR-H1-3 von SEQ ID NO: 686 - 688 und CDR-L1-3 von SEQ ID NO: 681 - 683;
s) CDR-H1-3 von SEQ ID NO: 698 - 700 und CDR-L1-3 von SEQ ID NO: 693 - 695;
t) CDR-H1-3 von SEQ ID NO: 710 - 712 und CDR-L1-3 von SEQ ID NO: 705 - 707;
u) CDR-H1-3 von SEQ ID NO: 722 - 724 und CDR-L1-3 von SEQ ID NO: 717 - 719;
v) CDR-H1-3 von SEQ ID NO: 734 - 736 und CDR-L1-3 von SEQ ID NO: 729 - 731;
w) CDR-H1-3 von SEQ ID NO: 746 - 748 und CDR-L1-3 von SEQ ID NO: 741 - 743;
x) CDR-H1-3 von SEQ ID NO: 758 - 760 und CDR-L1-3 von SEQ ID NO: 753 - 755;
y) CDR-H1-3 von SEQ ID NO: 1271 - 1273 und CDR-L1-3 von SEQ ID NO: 1276 - 1278;
z) CDR-H1-3 von SEQ ID NO: 1285 - 1287 und CDR-L1-3 von SEQ ID NO: 1290 - 1292;
aa) CDR-H1-3 von SEQ ID NO: 1299 - 1301 und CDR-L1-3 von SEQ ID NO: 1304 - 1306;
ab) CDR-H1-3 von SEQ ID NO: 1313 - 1315 und CDR-L1-3 von SEQ ID NO: 1318-1320;
ac) CDR-H1-3 von SEQ ID NO: 1327 - 1329 und CDR-L1-3 von SEQ ID NO: 1332 - 1334;
ad) CDR-H1-3 von SEQ ID NO: 1341 - 1343 und CDR-L1-3 von SEQ ID NO: 1346 - 1348;
ae) CDR-H1-3 von SEQ ID NO: 1355 - 1357 und CDR-L1-3 von SEQ ID NO: 1360 - 1362;
af) CDR-H1-3 von SEQ ID NO: 1369 - 1371 und CDR-L1-3 von SEQ ID NO: 1374-1376; und
ag) CDR-H1-3 von SEQ ID NO: 1383 - 1385 und CDR-L1-3 von SEQ ID NO: 1388 - 1390.

7. Bispezifisches Einzelketten-Antikörpermolekül gemäß Anspruch 4, das an menschlichen Hepatozyten-Wachstumsfaktor-Rezeptor (C-MET) und/oder einen nicht-menschlichen Primaten-C-MET bindet, wobei das bispezifische Einzelketten-Antikörpermolekül eine Gruppe der folgenden Sequenzen als CDR-H1, CDR-H2, CDR-H3, CDR-L1, CDR-L2 und CDRL3 in der zweiten Bindedomäne umfasst, die ausgewählt ist aus:

a) CDR-H1-3 von SEQ ID NO: 821 - 823 und CDR-L1-3 von SEQ ID NO: 816 - 818;
b) CDR-H1-3 von SEQ ID NO: 836 - 838 und CDR-L1-3 von SEQ ID NO: 833 - 835;
c) CDR-H1-3 von SEQ ID NO: 845 - 847 und CDR-L1-3 von SEQ ID NO: 840 - 842;
d) CDR-H1-3 von SEQ ID NO: 863 - 865 und CDR-L1-3 von SEQ ID NO: 858 - 860;
e) CDR-H1-3 von SEQ ID NO: 881 - 883 und CDR-L1-3 von SEQ ID NO: 876 - 878;
f) CDR-H1-3 von SEQ ID NO: 899 - 901 und CDR-L1-3 von SEQ ID NO: 894 - 896;
g) CDR-H1-3 von SEQ ID NO: 1401 - 1403 und CDR-L1-3 von SEQ ID NO: 1406 - 1408;
h) CDR-H1-3 von SEQ ID NO: 1415 - 1417 und CDR-L1-3 von SEQ ID NO: 1420 - 1422;
i) CDR-H1-3 von SEQ ID NO: 1429 - 1431 und CDR-L1-3 von SEQ ID NO: 1434 - 1436;
j) CDR-H1-3 von SEQ ID NO: 1443 - 1445 und CDR-L1-3 von SEQ ID NO: 1448 - 1450;
k) CDR-H1-3 von SEQ ID NO: 1457 - 1459 und CDR-L1-3 von SEQ ID NO: 1462 - 1464;
l) CDR-H1-3 von SEQ ID NO: 1471 - 1473 und CDR-L1-3 von SEQ ID NO: 1476 - 1478;
m) CDR-H1-3 von SEQ ID NO: 1639 - 1641 und CDR-L1-3 von SEQ ID NO: 1644 - 1646;
n) CDR-H1-3 von SEQ ID NO: 1625 - 1627 und CDR-L1-3 von SEQ ID NO: 1630 - 1632;
o) CDR-H1-3 von SEQ ID NO: 1611 - 1613 und CDR-L1-3 von SEQ ID NO: 1616 - 1618;
p) CDR-H1-3 von SEQ ID NO: 1597 - 1599 und CDR-L1-3 von SEQ ID NO: 1602 - 1604;
q) CDR-H1-3 von SEQ ID NO: 1569 - 1571 und CDR-L1-3 von SEQ ID NO: 1574 - 1576;
r) CDR-H1-3 von SEQ ID NO: 1555 - 1557 und CDR-L1-3 von SEQ ID NO: 1560 - 1562;
s) CDR-H1-3 von SEQ ID NO: 1583 - 1585 und CDR-L1-3 von SEQ ID NO: 1588 - 1590;
t) CDR-H1-3 von SEQ ID NO: 1541 - 1543 und CDR-L1-3 von SEQ ID NO: 1546 - 1548;
u) CDR-H1-3 von SEQ ID NO: 1513 - 1515 und CDR-L1-3 von SEQ ID NO: 1518 - 1520;
v) CDR-H1-3 von SEQ ID NO: 1527 - 1529 und CDR-L1-3 von SEQ ID NO: 1532 - 1534;
w) CDR-H1-3 von SEQ ID NO: 1499 - 1501 und CDR-L1-3 von SEQ ID NO: 1504 - 1506; und
x) CDR-H1-3 von SEQ ID NO: 1485 - 1487 und CDR-L1-3 von SEQ ID NO: 1490 - 1492.

8. Bispezifisches Einzelketten-Antikörpermolekül gemäß Anspruch 4, das an menschliches Endosialin und/oder ein nicht-menschliches Primaten-Endosialin bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 1653 - 1655 und CDR-L1-3 von SEQ ID NO: 1658 - 1660;
b) CDR-H1-3 von SEQ ID NO: 1667 - 1669 und CDR-L1-3 von SEQ ID NO: 1672 - 1674;
c) CDR-H1-3 von SEQ ID NO: 1681 - 1683 und CDR-L1-3 von SEQ ID NO: 1686 - 1688;
d) CDR-H1-3 von SEQ ID NO: 1695 - 1697 und CDR-L1-3 von SEQ ID NO: 1700 - 1702;
e) CDR-H1-3 von SEQ ID NO: 1709 - 1711 und CDR-L1-3 von SEQ ID NO: 1714 - 1716; und
f) CDR-H1-3 von SEQ ID NO: 1723 - 1725 und CDR-L1-3 von SEQ ID NO: 1728 - 1730.

9. Bispezifisches Einzelketten-Antikörpermolekül gemäß Anspruch 4, das an menschliche EGF-ähnliche Domäne 1 von EpCAM, die von Exon 2 codiert wird, und/oder eine nicht-menschliche Primaten-EGF-ähnliche Domäne 1 von EpCAM, die von Exon 2 codiert wird, bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 940 - 942 und CDR-L1-3 von SEQ ID NO: 935 - 937;
b) CDR-H1-3 von SEQ ID NO: 956 - 958 und CDR-L1-3 von SEQ ID NO: 951 - 953;
c) CDR-H1-3 von SEQ ID NO: 968 - 970 und CDR-L1-3 von SEQ ID NO: 963 - 965;
d) CDR-H1-3 von SEQ ID NO: 980 - 982 und CDR-L1-3 von SEQ ID NO: 975 - 977;
e) CDR-H1-3 von SEQ ID NO: 992 - 994 und CDR-L1-3 von SEQ ID NO: 987 - 989;
f) CDR-H1-3 von SEQ ID NO: 1004 - 1006 und CDR-L1-3 von SEQ ID NO: 999 - 1001;
g) CDR-H1-3 von SEQ ID NO: 1028 - 1030 und CDR-L1-3 von SEQ ID NO: 1023 - 1025;
h) CDR-H1-3 von SEQ ID NO: 1040 - 1042 und CDR-L1-3 von SEQ ID NO: 1035 - 1037;
i) CDR-H1-3 von SEQ ID NO: 1052 - 1054 und CDR-L1-3 von SEQ ID NO: 1047 - 1049;
j) CDR-H1-3 von SEQ ID NO: 1074 - 1076 und CDR-L1-3 von SEQ ID NO: 1069 - 1071;

k) CDR-H1-3 von SEQ ID NO: 1086 - 1088 und CDR-L1-3 von SEQ ID NO: 1081 - 1083;
l) CDR-H1-3 von SEQ ID NO: 1098 - 1100 und CDR-L1-3 von SEQ ID NO: 1093 - 1095;
m) CDR-H1-3 von SEQ ID NO: 1110 - 1112 und CDR-L1-3 von SEQ ID NO: 1105 - 1107;
n) CDR-H1-3 von SEQ ID NO: 1122 - 1124 und CDR-L1-3 von SEQ ID NO: 1117-1119;
o) CDR-H1-3 von SEQ ID NO: 1016 - 1018 und CDR-L1-3 von SEQ ID NO: 1011 - 1013; und
p) CDR-H1-3 von SEQ ID NO: 1765 - 1767 und CDR-L1-3 von SEQ ID NO: 1770-1772.

**10.** Bispezifisches Einzelketten-Antikörpermolekül gemäß Anspruch 4, das an menschliches Fibroblasten-Aktivierungsprotein alpha (FAP alpha) und/oder ein nicht-menschliches Primaten-FAP alpha bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 1137 - 1139 und CDR-L1-3 von SEQ ID NO: 1132 - 1134;
b) CDR-H1-3 von SEQ ID NO: 1849 - 1851 und CDR-L1-3 von SEQ ID NO: 1854 - 1856;
c) CDR-H1-3 von SEQ ID NO: 1835 - 1837 und CDR-L1-3 von SEQ ID NO: 1840 - 1842;
d) CDR-H1-3 von SEQ ID NO: 1779 - 1781 und CDR-L1-3 von SEQ ID NO: 1784 - 1786;
e) CDR-H1-3 von SEQ ID NO: 1793 - 1795 und CDR-L1-3 von SEQ ID NO: 1798 - 1800;
f) CDR-H1-3 von SEQ ID NO: 1863 - 1865 und CDR-L1-3 von SEQ ID NO: 1868 - 1870;
g) CDR-H1-3 von SEQ ID NO: 1807 - 1809 und CDR-L1-3 von SEQ ID NO: 1812 - 1814;
h) CDR-H1-3 von SEQ ID NO: 1821 - 1823 und CDR-L1-3 von SEQ ID NO: 1826 - 1828;
i) CDR-H1-3 von SEQ ID NO: 1891 - 1893 und CDR-L1-3 von SEQ ID NO: 1896 - 1898;
j) CDR-H1-3 von SEQ ID NO: 1877 - 1879 und CDR-L1-3 von SEQ ID NO: 1882 - 1884;
k) CDR-H1-3 von SEQ ID NO: 1961 - 1963 und CDR-L1-3 von SEQ ID NO: 1966 - 1968;
l) CDR-H1-3 von SEQ ID NO: 1947 - 1949 und CDR-L1-3 von SEQ ID NO: 1952 - 1954;
m) CDR-H1-3 von SEQ ID NO: 1975 - 1977 und CDR-L1-3 von SEQ ID NO: 1980 - 1982;
n) CDR-H1-3 von SEQ ID NO: 1933 - 1935 und CDR-L1-3 von SEQ ID NO: 1938 - 1940;
o) CDR-H1-3 von SEQ ID NO: 1919 - 1921 und CDR-L1-3 von SEQ ID NO: 1924 - 1926; und
p) CDR-H1-3 von SEQ ID NO: 1905 - 1907 und CDR-L1-3 von SEQ ID NO: 1910 - 1912.

**11.** Bispezifisches Einzelketten-Antikörpermolekül gemäß Anspruch 4 das an menschlichen Insulin-ähnlicher Wachstumsfaktor-I-Rezeptor (IGF-IR oder IGF-1R) und/oder einen nicht-menschlichen Primaten-IGF-1R bindet, wobei das bispezifische Einzelketten-Antikörpermolekül eine Gruppe der folgenden Sequenzen als CDR-H1, CDR-H2, CDR-H3, CDR-L1, CDR-L2 und CDR-L3 in der zweiten Bindedomäne umfasst, die ausgewählt ist aus:

a) CDR-H1-3 von SEQ ID NO: 2016 - 2018 und CDR-L1-3 von SEQ ID NO: 2021 - 2023;
b) CDR-H1-3 von SEQ ID NO: 2030 - 2032 und CDR-L1-3 von SEQ ID NO: 2035 - 2037;
c) CDR-H1-3 von SEQ ID NO: 2044 - 2046 und CDR-L1-3 von SEQ ID NO: 2049 - 2051;
d) CDR-H1-3 von SEQ ID NO: 2058 - 2060 und CDR-L1-3 von SEQ ID NO: 2063 - 2065;
e) CDR-H1-3 von SEQ ID NO: 2072 - 2074 und CDR-L1-3 von SEQ ID NO: 2077 - 2079;
f) CDR-H1-3 von SEQ ID NO: 2086 - 2088 und CDR-L1-3 von SEQ ID NO: 2091 - 2093;
g) CDR-H1-3 von SEQ ID NO: 2100 - 2102 und CDR-L1-3 von SEQ ID NO: 2105 - 2107;
h) CDR-H1-3 von SEQ ID NO: 2114 - 2116 und CDR-L1-3 von SEQ ID NO: 2119 - 2121;
i) CDR-H1-3 von SEQ ID NO: 2128 - 2130 und CDR-L1-3 von SEQ ID NO: 2133 - 2135;
j) CDR-H1-3 von SEQ ID NO: 2142 - 2144 und CDR-L1-3 von SEQ ID NO: 2147 - 2149:
k) CDR-H1-3 von SEQ ID NO: 2156 - 2158 und CDR-L1-3 von SEQ ID NO: 2161 - 2163;
l) CDR-H1-3 von SEQ ID NO: 2170 - 2172 und CDR-L1-3 von SEQ ID NO: 2175 - 2177;
m) CDR-H1-3 von SEQ ID NO: 2184 - 2186 und CDR-L1-3 von SEQ ID NO: 2189 - 2191;
n) CDR-H1-3 von SEQ ID NO: 2198 - 2200 und CDR-L1-3 von SEQ ID NO: 2203 - 2205; und
o) CDR-H1-3 von SEQ ID NO: 2212 - 2214 und CDR-L1-3 von SEQ ID NO: 2217 - 2219.

**12.** Nukleinsäuresequenz, die ein bispezifisches Einzelketten-Antikörpermolekül, wie in einem der Ansprüche 1 bis 11 definiert, kodiert.

**13.** Ein Vektor, der eine Nukleinsäuresequenz, wie in Anspruch 12 definiert, umfasst.

**14.** Wirtszelle, transformiert oder transfiziert mit einem Vektor, der in Anspruch 13 definiert ist.

**15.** Verfahren zur Herstellung eines bispezifischen Einzelketten-Antikörpermoleküls gemäß einem der Ansprüche 1 bis 11, umfassend Kultivieren einer in Anspruch 14 definierten Wirtszelle unter Bedingungen, die die Expression von Polypeptiden, wie in einem der Ansprüche 1 bis 11 definiert, erlaubt, und Gewinnen des hergestellten Polypeptids aus der Kultur.

**16.** Pharmazeutische Zusammensetzung, umfassend ein bispezifisches Einzelketten-Antikörpermolekül gemäß einem der Ansprüche 1 bis 11 oder das nach dem Verfahren gemäß Anspruch 15 hergestellt ist.

**17.** Bispezifisches Einzelketten-Antikörpermolekül gemäß Anspruch 1, wobei die erste Bindedomäne an das N-terminale CD3 epsilon-Polypeptidfragment *in vitro* und an die native CD3 epsilon-Untereinheit des CD3-Komplex auf T-Zellen *in vivo* mit der gleichen Bindeaffinität bindet.

**18.** Bispezifisches Einzelketten-Antikörpermolekül gemäß Anspruch 1, wobei die Homogenität des bispezifischen Einzelketten-Antikörpermoleküls mindestens 90%, 95% oder 98% ist.

## Revendications

**1.** Molécule d'anticorps à chaîne unique bispécifique comprenant un premier domaine de liaison qui se lie spécifiquement à un déterminant antigénique de la chaîne CD3ε (epsilon) humaine et de Callithrix jacchus, Saguinus oedipus ou Saimiri sciureus, dans laquelle ledit déterminant antigénique fait partie d'une séquence d'acides aminés comprise dans le groupe constitué de SEQ ID N° 2, 4, 6 ou 8 et comprend au moins la séquence d'acides aminés Gln-Asp-Gly-Asn-Glu, et un second domaine de liaison se liant à un antigène sélectionné parmi le groupe constitué de l'antigène de cellule souche de prostate (PSCA), l'antigène de lymphocyte B CD19 (CD19), le récepteur de facteur de croissance des hépatocytes (C-MET), l'endosialine, le domaine 1 de type EGF de EpCAM, codé par l'exon 2, la protéine d'activation de fibroblastes alpha (FAP alpha) et le récepteur de facteur de croissance insulinomimétique de type I (IGF-IR ou IGF-1R).

**2.** Molécule d'anticorps à chaîne unique bispécifique selon la revendication 1, dans laquelle le premier domaine de liaison comprend une région VL comprenant CDR-L1, CDR-L2 et CDR-L3 sélectionnées parmi :

(a) CDR-L1 telle que représentée dans SEQ ID N° 27, CDR-L2 telle que représentée dans SEQ ID N° 28 et CDR-L3 telle que représentée dans SEQ ID N° 29 ;
(b) CDR-L1 telle que représentée dans SEQ ID N° 117, CDR-L2 telle que représentée dans SEQ ID N° 118 et CDR-L3 telle que représentée dans SEQ ID N° 119 ; et
(c) CDR-L1 telle que représentée dans SEQ ID N° 153, CDR-L2 telle que représentée dans SEQ ID N° 154 et CDR-L3 telle que représentée dans SEQ ID N° 155.

**3.** Molécule d'anticorps à chaîne unique bispécifique selon la revendication 1 ou 2, dans laquelle le premier domaine de liaison comprend une région VH comprenant CDR-H1, CDR-H2 et CDR-H3 sélectionnées parmi :

(a) CDR-H1 telle que représentée dans SEQ ID N° 12, CDR-H2 telle que représentée dans SEQ ID N° 13 et CDR-H3 telle que représentée dans SEQ ID N° 14 ;
(b) CDR-H1 telle que représentée dans SEQ ID N° 30, CDR-H2 telle que représentée dans SEQ ID N° 31 et CDR-H3 telle que représentée dans SEQ ID N° 32 ;
(c) CDR-H1 telle que représentée dans SEQ ID N° 48, CDR-H2 telle que représentée dans SEQ ID N° 49 et CDR-H3 telle que représentée dans SEQ ID N° 50 ;
(d) CDR-H1 telle que représentée dans SEQ ID N° 66, CDR-H2 telle que représentée dans SEQ ID N° 67 et CDR-H3 telle que représentée dans SEQ ID N° 68 ;
(e) CDR-H1 telle que représentée dans SEQ ID N° 84, CDR-H2 telle que représentée dans SEQ ID N° 85 et CDR-H3 telle que représentée dans SEQ ID N° 86 ;
(f) CDR-H1 telle que représentée dans SEQ ID N° 102, CDR-H2 telle que représentée dans SEQ ID N° 103 et CDR-H3 telle que représentée dans SEQ ID N° 104 ;
(g) CDR-H1 telle que représentée dans SEQ ID N° 120, CDR-H2 telle que représentée dans SEQ ID N° 121 et CDR-H3 telle que représentée dans SEQ ID N° 122 ;
(h) CDR-H1 telle que représentée dans SEQ ID N° 138, CDR-H2 telle que représentée dans SEQ ID N° 139 et CDR-H3 telle que représentée dans SEQ ID N° 140 ;
(i) CDR-H1 telle que représentée dans SEQ ID N° 156, CDR-H2 telle que représentée dans SEQ ID N° 157 et CDR-H3 telle que représentée dans SEQ ID N° 158 ; et

(j) CDR-H1 telle que représentée dans SEQ ID N° 174, CDR-H2 telle que représentée dans SEQ ID N° 175 et CDR-H3 telle que représentée dans SEQ ID N° 176.

**4.** Molécule d'anticorps à chaîne unique bispécifique selon l'une quelconque des revendications 1 à 3, dans laquelle le second domaine de liaison est sélectionné parmi le groupe constitué de domaines de liaison se liant à l'antigène de cellule souche de prostate humain (PSCA) et/ou un PSCA de primate non humain, se liant à l'antigène de lymphocyte B CD19 humain (CD19) et/ou un CD19 de primate non humain, se liant au récepteur de facteur de croissance des hépatocytes humain (C-MET) et/ou un C-MET de primate non humain, se liant à l'endosialine humaine et/ou une endosialine de primate non humain, se liant au domaine 1 de type EGF humain de EpCAM, codé par l'exon 2, et/ou un domaine 1 de type EGF de primate non humain de EpCAM, codé par l'exon 2, se liant à la protéine d'activation de fibroblastes alpha humaine (FAP alpha) et/ou une FAP alpha de primate non humain, et se liant au récepteur de facteur de croissance insulinomimétique de type I humain (IGF-IR ou IGF-1R) et/ou un IGF-1R de primate non humain.

**5.** Molécule d'anticorps à chaîne unique bispécifique selon la revendication 4 se liant à l'antigène de cellule souche de prostate humain (PSCA) et/ou un PSCA de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 382 à 384 et CDR-L1 à 3 de SEQ ID N° : 377 à 379 ;
b) CDR-H1 à 3 de SEQ ID N° : 400 à 402 et CDR-L1 à 3 de SEQ ID N° : 395 à 397 ;
c) CDR-H1 à 3 de SEQ ID N° : 414 à 416 et CDR-L1 à 3 de SEQ ID N° : 409 à 411 ;
d) CDR-H1 à 3 de SEQ ID N° : 432 à 434 et CDR-L1 à 3 de SEQ ID N° : 427 à 429 ;
e) CDR-H1 à 3 de SEQ ID N° : 1215 à 1217 et CDR-L1 à 3 de SEQ ID N° : 1220 à 1222 ;
f) CDR-H1 à 3 de SEQ ID N° : 1187 à 1189 et CDR-L1 à 3 de SEQ ID N° : 1192 à 1194 ;
g) CDR-H1 à 3 de SEQ ID N° : 1173 à 1175 et CDR-L1 à 3 de SEQ ID N° : 1178 à 1180 ;
h) CDR-H1 à 3 de SEQ ID N° : 1229 à 1231 et CDR-L1 à 3 de SEQ ID N° : 1234 à 1236 ;
i) CDR-H1 à 3 de SEQ ID N° : 1201 à 1203 et CDR-L1 à 3 de SEQ ID N° : 1206 à 1208 ;
k) CDR-H1 à 3 de SEQ ID N° : 1257 à 1259 et CDR-L1 à 3 de SEQ ID N° : 1262 à 1264 ; et
1) CDR-H1 à 3 de SEQ ID N° : 1243 à 1245 et CDR-L1 à 3 de SEQ ID N° : 1248 à 1250.

**6.** Molécule d'anticorps à chaîne unique bispécifique selon la revendication 4 se liant à l'antigène de lymphocyte B CD19 humain (CD19) et/ou un CD19 de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 478 à 480 et CDR-L1 à 3 de SEQ ID N° : 473 à 475 ;
b) CDR-H1 à 3 de SEQ ID N° : 530 à 532 et CDR-L1 à 3 de SEQ ID N° : 525 à 527 ;
c) CDR-H1 à 3 de SEQ ID N° : 518 à 520 et CDR-L1 à 3 de SEQ ID N° : 513 à 515 ;
d) CDR-H1 à 3 de SEQ ID N° : 506 à 508 et CDR-L1 à 3 de SEQ ID N° : 501 à 503 ;
e) CDR-H1 à 3 de SEQ ID N° : 494 à 496 et CDR-L1 à 3 de SEQ ID N° : 489 à 491 ;
f) CDR-H1 à 3 de SEQ ID N° : 542 à 544 et CDR-L1 à 3 de SEQ ID N° : 537 à 539 ;
g) CDR-H1 à 3 de SEQ ID N° : 554 à 556 et CDR-L1 à 3 de SEQ ID N° : 549 à 551 ;
h) CDR-H1 à 3 de SEQ ID N° : 566 à 568 et CDR-L1 à 3 de SEQ ID N° : 561 à 563 ;
i) CDR-H1 à 3 de SEQ ID N° : 578 à 580 et CDR-L1 à 3 de SEQ ID N° : 573 à 575 ;
j) CDR-H1 à 3 de SEQ ID N° : 590 à 592 et CDR-L1 à 3 de SEQ ID N° : 585 à 587 ;
k) CDR-H1 à 3 de SEQ ID N° : 602 à 604 et CDR-L1 à 3 de SEQ ID N° : 597 à 599 ;
1) CDR-H1 à 3 de SEQ ID N° : 614 à 616 et CDR-L1 à 3 de SEQ ID N° : 609 à 611 ;
m) CDR-H1 à 3 de SEQ ID N° : 626 à 628 et CDR-L1 à 3 de SEQ ID N° : 621 à 623 ;
n) CDR-H1 à 3 de SEQ ID N° : 638 à 640 et CDR-L1 à 3 de SEQ ID N° : 633 à 635 ;
o) CDR-H1 à 3 de SEQ ID N° : 650 à 652 et CDR-L1 à 3 de SEQ ID N° : 645 à 647 ;
p) CDR-H1 à 3 de SEQ ID N° : 662 à 664 et CDR-L1 à 3 de SEQ ID N° : 657 à 659 ;
q) CDR-H1 à 3 de SEQ ID N° : 674 à 676 et CDR-L1 à 3 de SEQ ID N° : 669 à 671 ;
r) CDR-H1 à 3 de SEQ ID N° : 686 à 688 et CDR-L1 à 3 de SEQ ID N° : 681 à 683 ;
s) CDR-H1 à 3 de SEQ ID N° : 698 à 700 et CDR-L1 à 3 de SEQ ID N° : 693 à 695 ;
t) CDR-H1 à 3 de SEQ ID N° : 710 à 712 et CDR-L1 à 3 de SEQ ID N° : 705 à 707 ;
u) CDR-H1 à 3 de SEQ ID N° : 722 à 724 et CDR-L1 à 3 de SEQ ID N° : 717 à 719 ;
v) CDR-H1 à 3 de SEQ ID N° : 734 à 736 et CDR-L1 à 3 de SEQ ID N° : 729 à 731 ;

w) CDR-H1 à 3 de SEQ ID N° : 746 à 748 et CDR-L1 à 3 de SEQ ID N° : 741 à 743 ;
x) CDR-H1 à 3 de SEQ ID N° : 758 à 760 et CDR-L1 à 3 de SEQ ID N° : 753 à 755 ;
y) CDR-H1 à 3 de SEQ ID N° : 1271 à 1273 et CDR-L1 à 3 de SEQ ID N° : 1276 à 1278 ;
z) CDR-H1 à 3 de SEQ ID N° : 1285 à 1287 et CDR-L1 à 3 de SEQ ID N° : 1290 à 1292 ;
aa) CDR-H1 à 3 de SEQ ID N° : 1299 à 1301 et CDR-L1 à 3 de SEQ ID N° : 1304 à 1306 ;
ab) CDR-H1 à 3 de SEQ ID N° : 1313 à 1315 et CDR-L1 à 3 de SEQ ID N° : 1318 à 1320 ;
ac) CDR-H1 à 3 de SEQ ID N° : 1327 à 1329 et CDR-L1 à 3 de SEQ ID N° : 1332 à 1334 ;
ad) CDR-H1 à 3 de SEQ ID N° : 1341 à 1343 et CDR-L1 à 3 de SEQ ID N° : 1346 à 1348 ;
ae) CDR-H1 à 3 de SEQ ID N° : 1355 à 1357 et CDR-L1 à 3 de SEQ ID N° : 1360 à 1362 ;
af) CDR-H1 à 3 de SEQ ID N° : 1369 à 1371 et CDR-L1 à 3 de SEQ ID N° : 1374 à 1376 ; et
ag) CDR-H1 à 3 de SEQ ID N° : 1383 à 1385 et CDR-L1 à 3 de SEQ ID N° : 1388 à 1390.

**7.** Molécule d'anticorps à chaîne unique bispécifique selon la revendication 4 se liant au récepteur de facteur de croissance des hépatocytes humain (C-MET) et/ou un C-MET de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 821 à 823 et CDR-L1 à 3 de SEQ ID N° : 816 à 818 ;
b) CDR-H1 à 3 de SEQ ID N° : 836 à 838 et CDR-L1 à 3 de SEQ ID N° : 833 à 835 ;
c) CDR-H1 à 3 de SEQ ID N° : 845 à 847 et CDR-L1 à 3 de SEQ ID N° : 840 à 842 ;
d) CDR-H1 à 3 de SEQ ID N° : 863 à 865 et CDR-L1 à 3 de SEQ ID N° : 858 à 860 ;
e) CDR-H1 à 3 de SEQ ID N° : 881 à 883 et CDR-L1 à 3 de SEQ ID N° : 876 à 878 ;
f) CDR-H1 à 3 de SEQ ID N° : 899 à 901 et CDR-L1 à 3 de SEQ ID N° : 894 à 896 ;
g) CDR-H1 à 3 de SEQ ID N° : 1401 à 1403 et CDR-L1 à 3 de SEQ ID N° : 1406 à 1408 ;
h) CDR-H1 à 3 de SEQ ID N° : 1415 à 1417 et CDR-L1 à 3 de SEQ ID N° : 1420 à 1422 ;
i) CDR-H1 à 3 de SEQ ID N° : 1429 à 1431 et CDR-L1 à 3 de SEQ ID N° : 1434 à 1436 ;
j) CDR-H1 à 3 de SEQ ID N° : 1443 à 1445 et CDR-L1 à 3 de SEQ ID N° : 1448 à 1450 ;
k) CDR-H1 à 3 de SEQ ID N° : 1457 à 1459 et CDR-L1 à 3 de SEQ ID N° : 1462 à 1464 ;
l) CDR-H1 à 3 de SEQ ID N° : 1471 à 1473 et CDR-L1 à 3 de SEQ ID N° : 1476 à 1478 ;
m) CDR-H1 à 3 de SEQ ID N° : 1639 à 1641 et CDR-L1 à 3 de SEQ ID N° : 1644 à 1646 ;
n) CDR-H1 à 3 de SEQ ID N° : 1625 à 1627 et CDR-L1 à 3 de SEQ ID N° : 1630 à 1632 ;
o) CDR-H1 à 3 de SEQ ID N° : 1611 à 1613 et CDR-L1 à 3 de SEQ ID N° : 1616 à 1618 ;
p) CDR-H1 à 3 de SEQ ID N° : 1597 à 1599 et CDR-L1 à 3 de SEQ ID N° : 1602 à 1604 ;
q) CDR-H1 à 3 de SEQ ID N° : 1569 à 1571 et CDR-L1 à 3 de SEQ ID N° : 1574 à 1576 ;
r) CDR-H1 à 3 de SEQ ID N° : 1555 à 1557 et CDR-L1 à 3 de SEQ ID N° : 1560 à 1562 ;
s) CDR-H1 à 3 de SEQ ID N° : 1583 à 1585 et CDR-L1 à 3 de SEQ ID N° : 1588 à 1590 ;
t) CDR-H1 à 3 de SEQ ID N° : 1541 à 1543 et CDR-L1 à 3 de SEQ ID N° : 1546 à 1548 ;
u) CDR-H1 à 3 de SEQ ID N° : 1513 à 1515 et CDR-L1 à 3 de SEQ ID N° : 1518 à 1520 ;
v) CDR-H1 à 3 de SEQ ID N° : 1527 à 1529 et CDR-L1 à 3 de SEQ ID N° : 1532 à 1534 ;
w) CDR-H1 à 3 de SEQ ID N° : 1499 à 1501 et CDR-L1 à 3 de SEQ ID N° : 1504 à 1506 ; et
x) CDR-H1 à 3 de SEQ ID N° : 1485 à 1487 et CDR-L1 à 3 de SEQ ID N° : 1490 à 1492.

**8.** Molécule d'anticorps à chaîne unique bispécifique selon la revendication 4 se liant à l'endosialine humaine et/ou une endosialine de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 1653 à 1655 et CDR-L1 à 3 de SEQ ID N° : 1658 à 1660 ;
b) CDR-H1 à 3 de SEQ ID N° : 1667 à 1669 et CDR-L1 à 3 de SEQ ID N° : 1672 à 1674 ;
c) CDR-H1 à 3 de SEQ ID N° : 1681 à 1683 et CDR-L1 à 3 de SEQ ID N° : 1686 à 1688 ;
d) CDR-H1 à 3 de SEQ ID N° : 1695 à 1697 et CDR-L1 à 3 de SEQ ID N° : 1700 à 1702 ;
e) CDR-H1 à 3 de SEQ ID N° : 1709 à 1711 et CDR-L1 à 3 de SEQ ID N° : 1714 à 1716 ; et
f) CDR-H1 à 3 de SEQ ID N° : 1723 à 1725 et CDR-L1 à 3 de SEQ ID N° : 1728 à 1730.

**9.** Molécule d'anticorps à chaîne unique bispécifique selon la revendication 4 se liant au domaine 1 de type EGF humain de EpCAM, codé par l'exon 2, et/ou un domaine 1 de type EGF de primate non humain de EpCAM, codé par l'exon 2, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 940 à 942 et CDR-L1 à 3 de SEQ ID N° : 935 à 937 ;
b) CDR-H1 à 3 de SEQ ID N° : 956 à 958 et CDR-L1 à 3 de SEQ ID N° : 951 à 953 ;
c) CDR-H1 à 3 de SEQ ID N° : 968 à 970 et CDR-L1 à 3 de SEQ ID N° : 963 à 965 ;
d) CDR-H1 à 3 de SEQ ID N° : 980 à 982 et CDR-L1 à 3 de SEQ ID N° : 975 à 977 ;
e) CDR-H1 à 3 de SEQ ID N° : 992 à 994 et CDR-L1 à 3 de SEQ ID N° : 987 à 989 ;
f) CDR-H1 à 3 de SEQ ID N° : 1004 à 1006 et CDR-L1 à 3 de SEQ ID N° : 999 à 1001 ;
g) CDR-H1 à 3 de SEQ ID N° : 1028 à 1030 et CDR-L1 à 3 de SEQ ID N° : 1023 à 1025 ;
h) CDR-H1 à 3 de SEQ ID N° : 1040 à 1042 et CDR-L1 à 3 de SEQ ID N° : 1035 à 1037 ;
i) CDR-H1 à 3 de SEQ ID N° : 1052 à 1054 et CDR-L1 à 3 de SEQ ID N° : 1047 à 1049 ;
j) CDR-H1 à 3 de SEQ ID N° : 1074 à 1076 et CDR-L1 à 3 de SEQ ID N° : 1069 à 1071 ;
k) CDR-H1 à 3 de SEQ ID N° : 1086 à 1088 et CDR-L1 à 3 de SEQ ID N° : 1081 à 1083 ;
l) CDR-H1 à 3 de SEQ ID N° : 1098 à 1100 et CDR-L1 à 3 de SEQ ID N° : 1093 à 1095 ;
m) CDR-H1 à 3 de SEQ ID N° : 1110 à 1112 et CDR-L1 à 3 de SEQ ID N° : 1105 à 1107 ;
n) CDR-H1 à 3 de SEQ ID N° : 1122 à 1124 et CDR-L1 à 3 de SEQ ID N° : 1117 à 1119 ;
o) CDR-H1 à 3 de SEQ ID N° : 1016 à 1018 et CDR-L1 à 3 de SEQ ID N° : 1011 à 1013 ; et
p) CDR-H1 à 3 de SEQ ID N° : 1765 à 1767 et CDR-L1 à 3 de SEQ ID N° : 1770 à 1772.

**10.** Molécule d'anticorps à chaîne unique bispécifique selon la revendication 4 se liant à la protéine d'activation de fibroblastes alpha humaine (FAP alpha) et/ou une FAP alpha de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 1137 à 1139 et CDR-L1 à 3 de SEQ ID N° : 1132 à 1134 ;
b) CDR-H1 à 3 de SEQ ID N° : 1849 à 1851 et CDR-L1 à 3 de SEQ ID N° : 1854 à 1856 ;
c) CDR-H1 à 3 de SEQ ID N° : 1835 à 1837 et CDR-L1 à 3 de SEQ ID N° : 1840 à 1842 ;
d) CDR-H1 à 3 de SEQ ID N° : 1779 à 1781 et CDR-L1 à 3 de SEQ ID N° : 1784 à 1786 ;
e) CDR-H1 à 3 de SEQ ID N° : 1793 à 1795 et CDR-L1 à 3 de SEQ ID N° : 1798 à 1800 ;
f) CDR-H1 à 3 de SEQ ID N° : 1863 à 1865 et CDR-L1 à 3 de SEQ ID N° : 1868 à 1870 ;
g) CDR-H1 à 3 de SEQ ID N° : 1807 à 1809 et CDR-L1 à 3 de SEQ ID N° : 1812 à 1814 ;
h) CDR-H1 à 3 de SEQ ID N° : 1821 à 1823 et CDR-L1 à 3 de SEQ ID N° : 1826 à 1828 ;
i) CDR-H1 à 3 de SEQ ID N° : 1891 à 1893 et CDR-L1 à 3 de SEQ ID N° : 1896 à 1898 ;
j) CDR-H1 à 3 de SEQ ID N° : 1877 à 1879 et CDR-L1 à 3 de SEQ ID N° : 1882 à 1884 ;
k) CDR-H1 à 3 de SEQ ID N° : 1961 à 1963 et CDR-L1 à 3 de SEQ ID N° : 1966 à 1968 ;
l) CDR-H1 à 3 de SEQ ID N° : 1947 à 1949 et CDR-L1 à 3 de SEQ ID N° : 1952 à 1954 ;
m) CDR-H1 à 3 de SEQ ID N° : 1975 à 1977 et CDR-L1 à 3 de SEQ ID N° : 1980 à 1982 ;
n) CDR-H1 à 3 de SEQ ID N° : 1933 à 1935 et CDR-L1 à 3 de SEQ ID N° : 1938 à 1940 ;
o) CDR-H1 à 3 de SEQ ID N° : 1919 à 1921 et CDR-L1 à 3 de SEQ ID N° : 1924 à 1926 ; et
p) CDR-H1 à 3 de SEQ ID N° : 1905 à 1907 et CDR-L1 à 3 de SEQ ID N° : 1910 à 1912.

**11.** Molécule d'anticorps à chaîne unique bispécifique selon la revendication 4 se liant au récepteur de facteur de croissance insulinomimétique de type I humain (IGF-IR ou IGF-1R) et/ou un IGF-1R de primate non humain, dans laquelle la molécule d'anticorps à chaîne unique bispécifique comprend un groupe des séquences suivantes comme CDR-H1, CDR-H2, CDR-H3, CDR-L1, CDR-L2 et CDR-L3 dans le second domaine de liaison sélectionnées parmi :

a) CDR-H1 à 3 de SEQ ID N° : 2016 à 2018 et CDR-L1 à 3 de SEQ ID N° : 2021 à 2023 ;
b) CDR-H1 à 3 de SEQ ID N° : 2030 à 2032 et CDR-L1 à 3 de SEQ ID N° : 2035 à 2037 ;
c) CDR-H1 à 3 de SEQ ID N° : 2044 à 2046 et CDR-L1 à 3 de SEQ ID N° : 2049 à 2051 ;
d) CDR-H1 à 3 de SEQ ID N° : 2058 à 2060 et CDR-L1 à 3 de SEQ ID N° : 2063 à 2065 ;
e) CDR-H1 à 3 de SEQ ID N° : 2072 à 2074 et CDR-L1 à 3 de SEQ ID N° : 2077 à 2079 ;
f) CDR-H1 à 3 de SEQ ID N° : 2086 à 2088 et CDR-L1 à 3 de SEQ ID N° : 2091 à 2093 ;
g) CDR-H1 à 3 de SEQ ID N° : 2100 à 2102 et CDR-L1 à 3 de SEQ ID N° : 2105 à 2107 ;
h) CDR-H1 à 3 de SEQ ID N° : 2114 à 2116 et CDR-L1 à 3 de SEQ ID N° : 2119 à 2121 ;
i) CDR-H1 à 3 de SEQ ID N° : 2128 à 2130 et CDR-L1 à 3 de SEQ ID N° : 2133 à 2135 ;
j) CDR-H1 à 3 de SEQ ID N° : 2142 à 2144 et CDR-L1 à 3 de SEQ ID N° : 2147 à 2149 ;
k) CDR-H1 à 3 de SEQ ID N° : 2156 à 2158 et CDR-L1 à 3 de SEQ ID N° : 2161 à 2163 ;
l) CDR-H1 à 3 de SEQ ID N° : 2170 à 2172 et CDR-L1 à 3 de SEQ ID N° : 2175 à 2177 ;

m) CDR-H1 à 3 de SEQ ID N° : 2184 à 2186 et CDR-L1 à 3 de SEQ ID N° : 2189 à 2191 ;
n) CDR-H1 à 3 de SEQ ID N° : 2198 à 2200 et CDR-L1 à 3 de SEQ ID N° : 2203 à 2205 ; et
o) CDR-H1 à 3 de SEQ ID N° : 2212 à 2214 et CDR-L1 à 3 de SEQ ID N° : 2217 à 2219.

**12.** Séquence d'acide nucléique codant pour une molécule d'anticorps à chaîne unique bispécifique telle que définie dans l'une quelconque des revendications 1 à 11.

**13.** Vecteur qui comprend une séquence d'acide nucléique telle que définie dans la revendication 12.

**14.** Cellule hôte transformée ou transfectée avec un vecteur défini dans la revendication 13.

**15.** Procédé de production d'une molécule d'anticorps à chaîne unique bispécifique selon l'une quelconque des revendications 1 à 11, ledit procédé comprenant la mise en culture d'une cellule hôte définie dans la revendication 14 dans des conditions permettant l'expression du polypeptide tel que défini dans l'une quelconque des revendications 1 à 11 et la récupération du polypeptide produit de la culture.

**16.** Composition pharmaceutique comprenant une molécule d'anticorps à chaîne unique bispécifique selon l'une quelconque des revendications 1 à 11 ou produite selon le procédé de la revendication 15.

**17.** Molécule d'anticorps à chaîne unique bispécifique selon la revendication 1, dans laquelle ledit premier domaine de liaison se lie au fragment de polypeptide CD3 epsilon N-terminal in vitro et à la sous-unité CD3 epsilon native du complexe CD3 sur des lymphocytes T in vivo avec la même affinité de liaison.

**18.** Molécule d'anticorps à chaîne unique bispécifique selon la revendication 1, dans laquelle l'homogénéité de la molécule d'anticorps à chaîne unique bispécifique est d'au moins 90 %, 95 % ou 98 %.

Figure 1

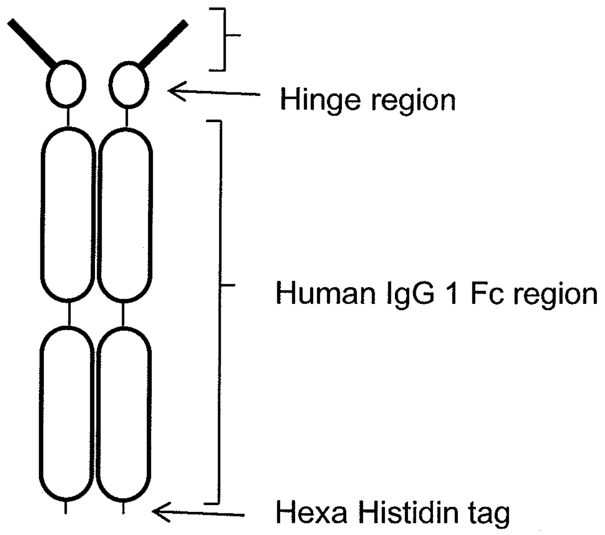


Figure 2

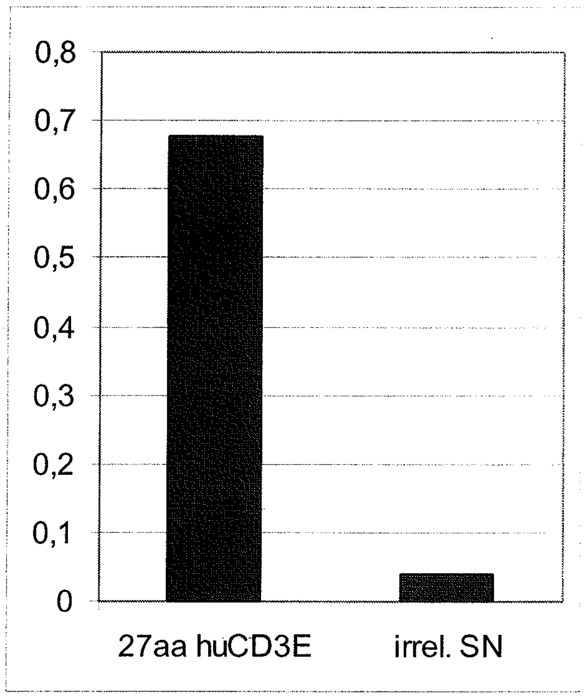

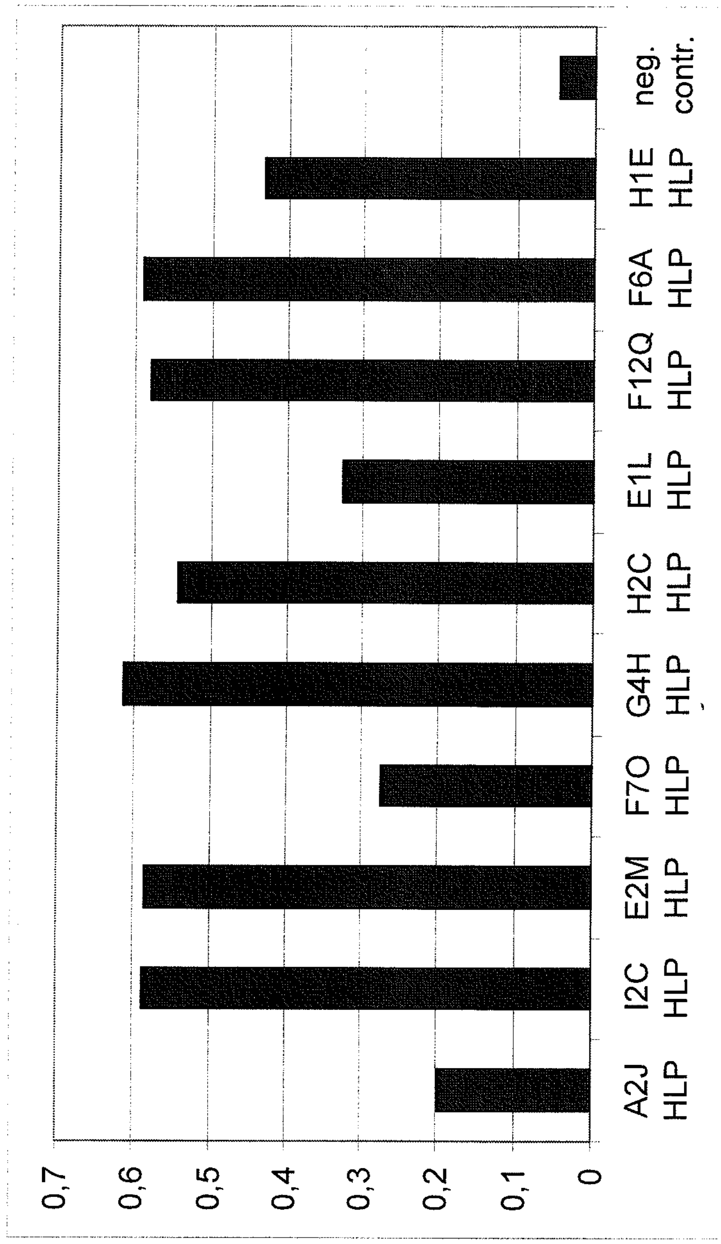
0,7
0,6
0,5
0,4
0,3
0,2
0,1
0
A2J HLP
I2C HLP
E2M HLP
F7O HLP
G4H HLP
H2C HLP
E1L HLP
F12Q HLP
F6A HLP
H1E HLP
neg. contr.

Figure 3

Figure 4

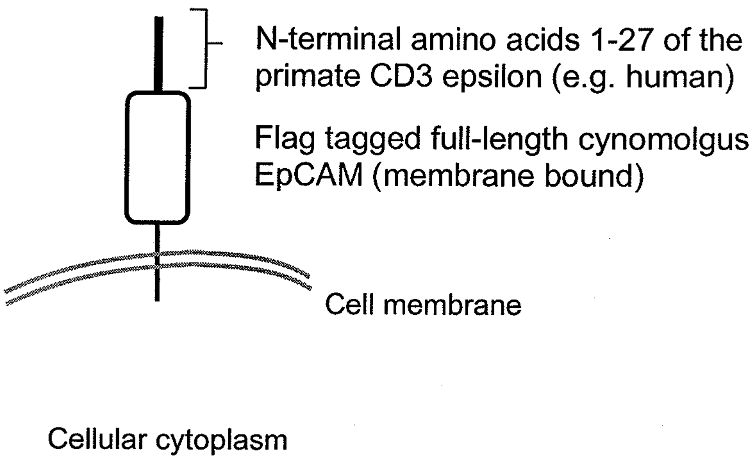
N-terminal amino acids 1-27 of the primate CD3 epsilon (e.g. human)
Flag tagged full-length cynomolgus EpCAM (membrane bound)
Cell membrane
Cellular cytoplasm

Figure 5

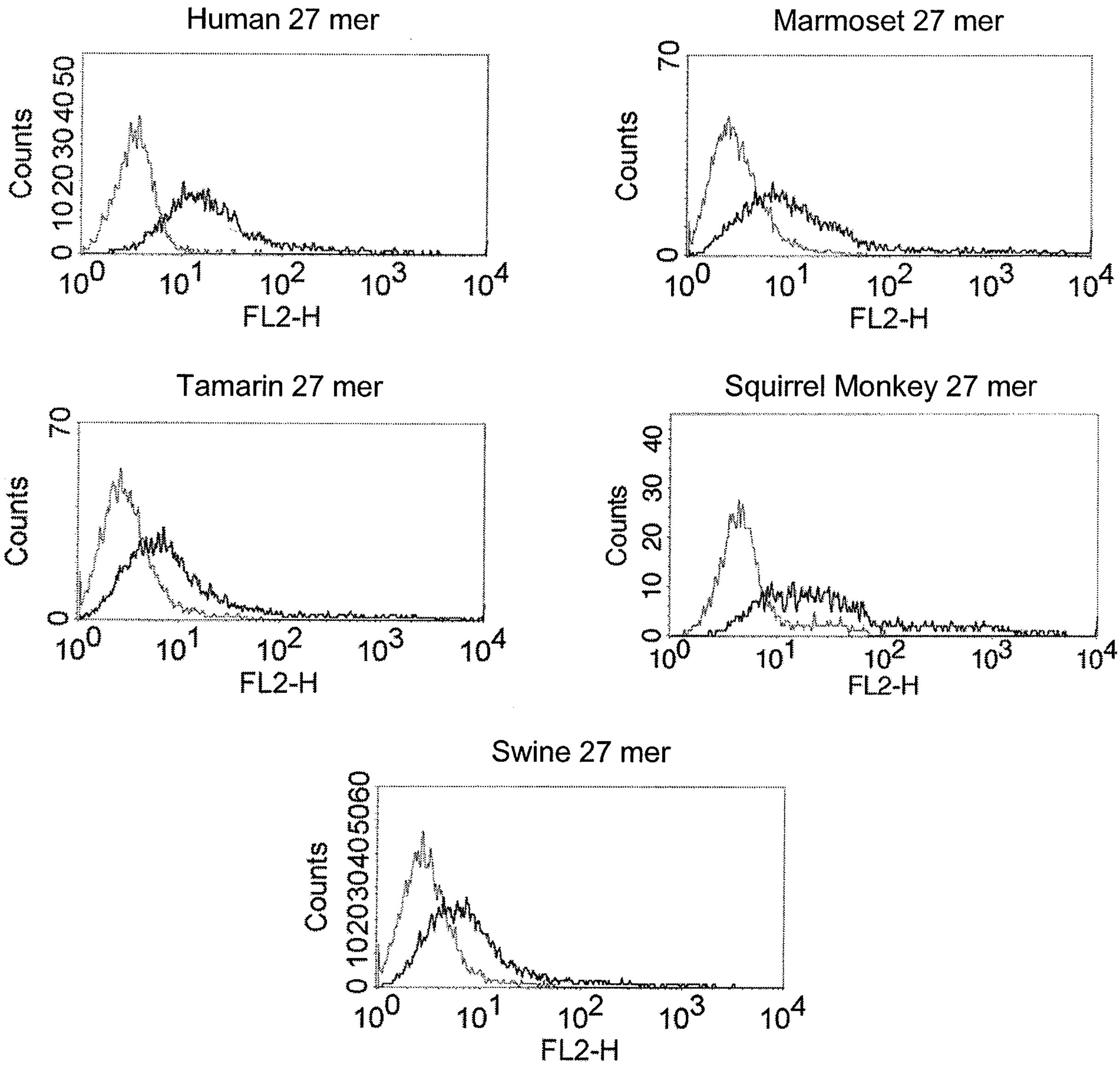
Human 27 mer
Counts
0 10 20 30 40 50
$10^0$ $10^1$ $10^2$ $10^3$ $10^4$
FL2-H
Marmoset 27 mer
Counts
0 70
$10^0$ $10^1$ $10^2$ $10^3$ $10^4$
FL2-H
Tamarin 27 mer
Counts
0 70
$10^0$ $10^1$ $10^2$ $10^3$ $10^4$
FL2-H
Squirrel Monkey 27 mer
Counts
0 10 20 30 40
$10^0$ $10^1$ $10^2$ $10^3$ $10^4$
FL2-H
Swine 27 mer
Counts
0 10 20 30 40 50 60
$10^0$ $10^1$ $10^2$ $10^3$ $10^4$
FL2-H

Figure 6A

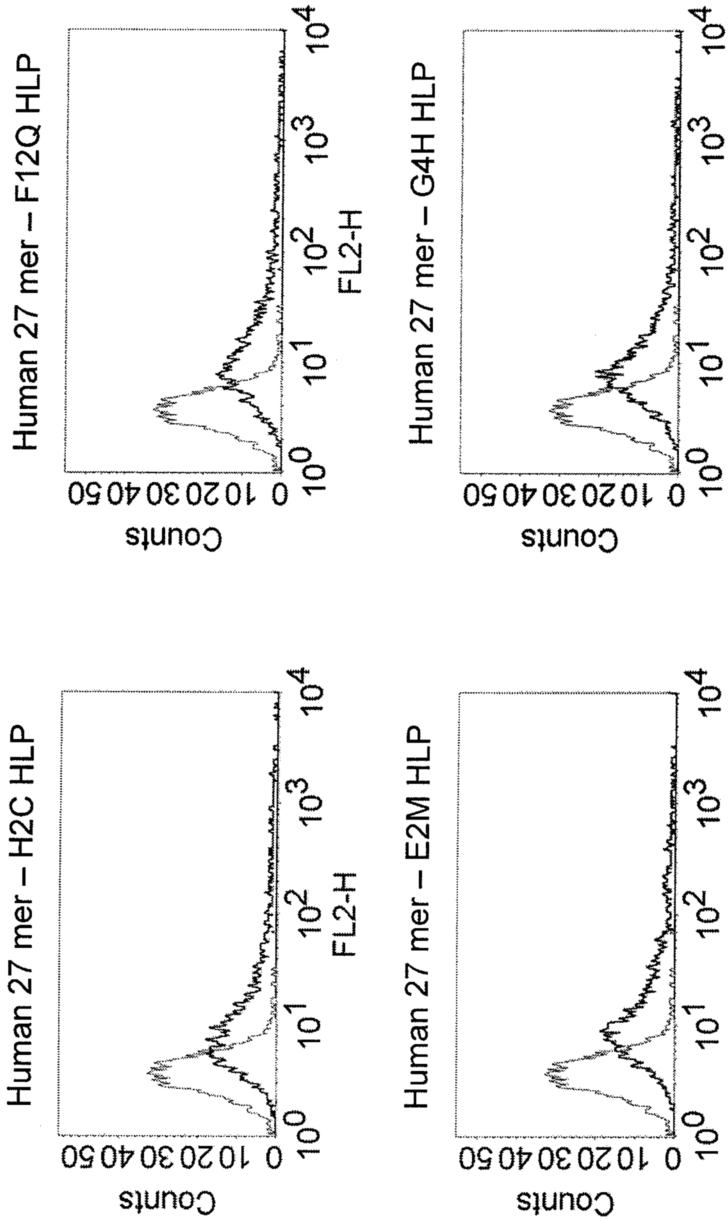
Human 27 mer – H2C HLP
Human 27 mer – F12Q HLP
Human 27 mer – E2M HLP
Human 27 mer – G4H HLP
Counts
0 10 20 30 40 50
FL2-H
$10^0$ $10^1$ $10^2$ $10^3$ $10^4$

Figure 6B

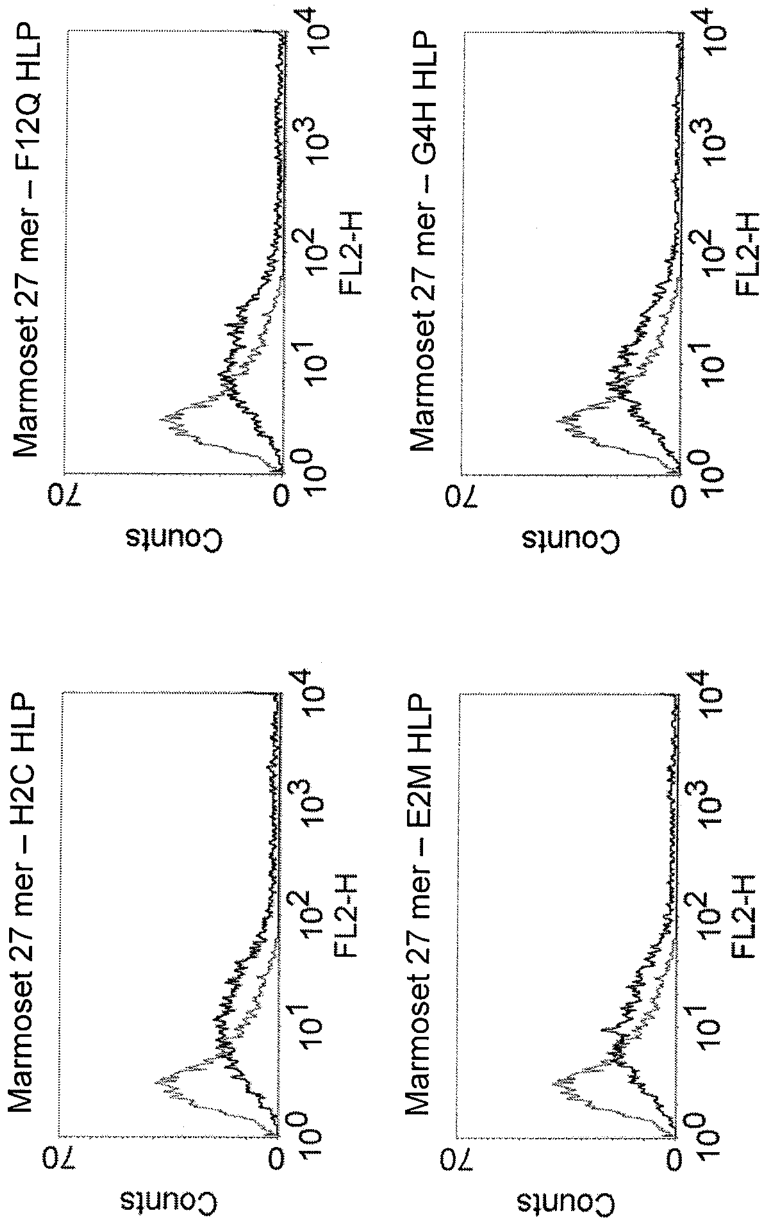
Marmoset 27 mer – H2C HLP
Marmoset 27 mer – F12Q HLP
Marmoset 27 mer – E2M HLP
Marmoset 27 mer – G4H HLP
Counts
70
0
$10^0$
$10^1$
$10^2$
$10^3$
$10^4$
FL2-H

Figure 6C

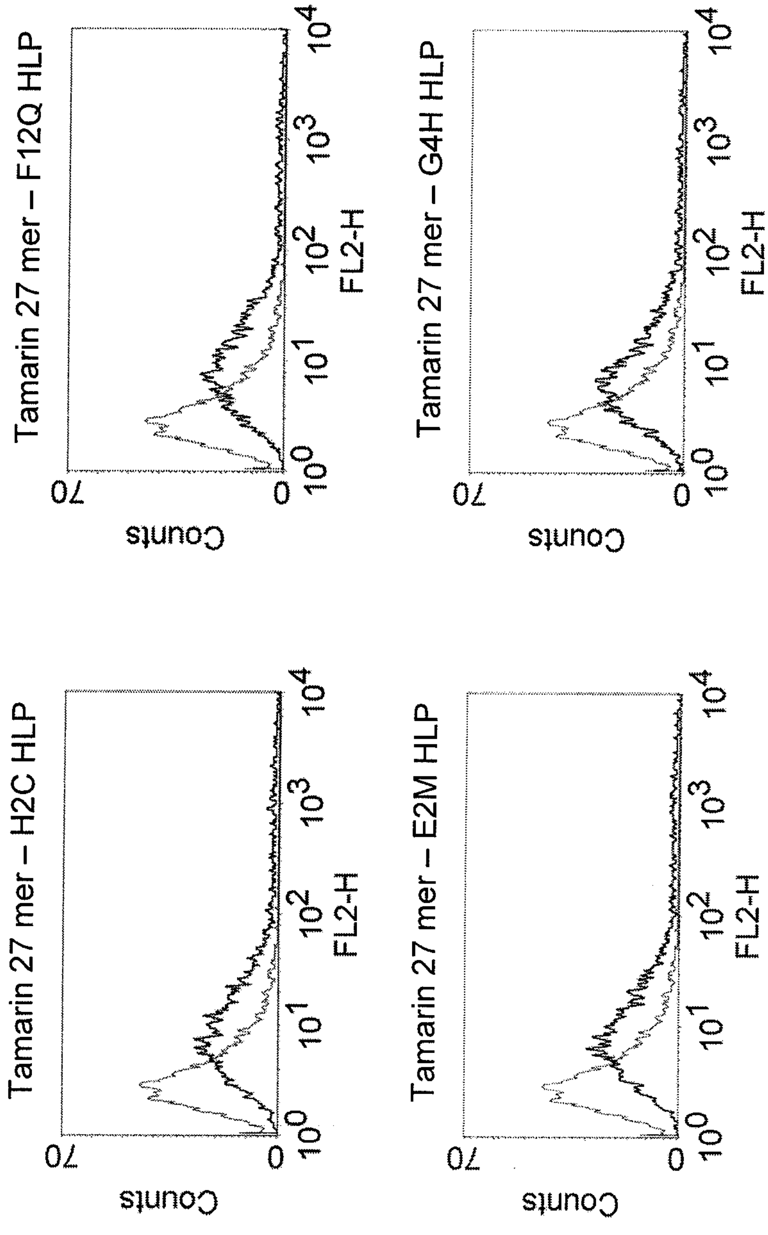
Tamarin 27 mer – H2C HLP
Tamarin 27 mer – F12Q HLP
Tamarin 27 mer – E2M HLP
Tamarin 27 mer – G4H HLP
Counts
70
0
$10^0$
$10^1$
$10^2$
$10^3$
$10^4$
FL2-H

Figure 6D

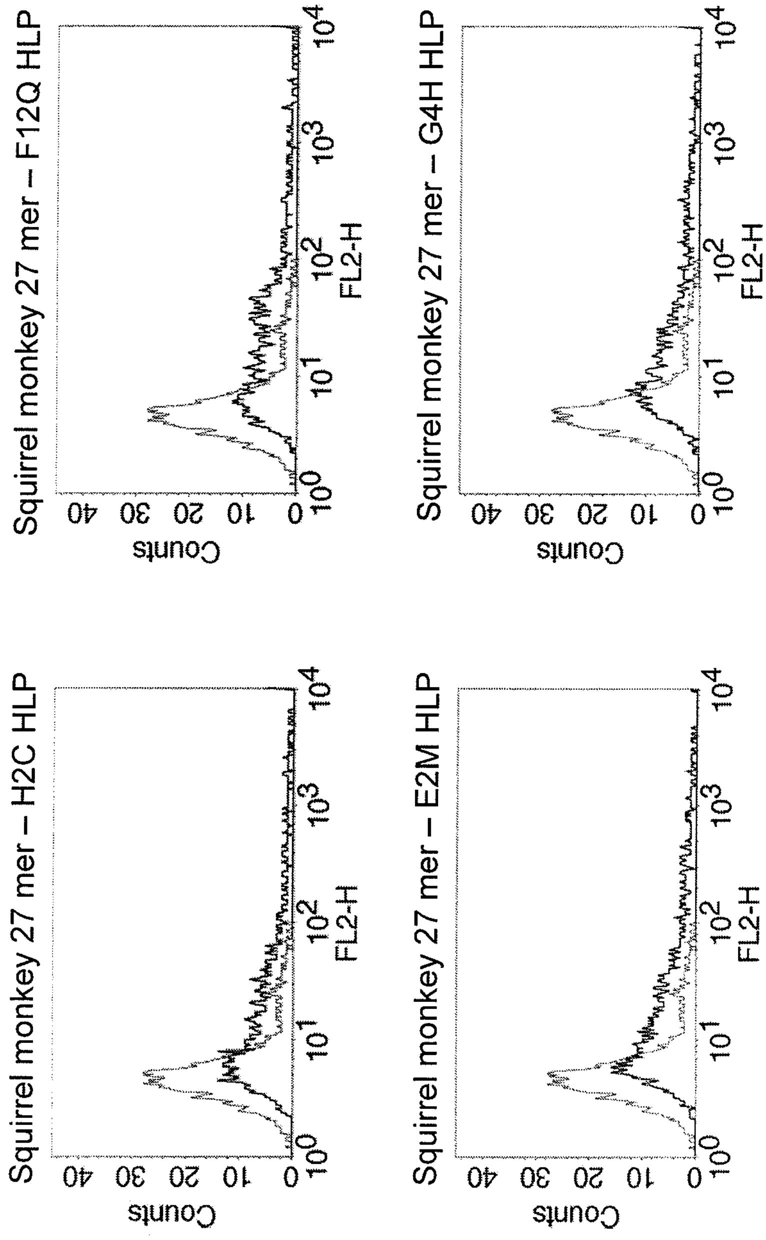
Squirrel monkey 27 mer – H2C HLP
Squirrel monkey 27 mer – F12Q HLP
Squirrel monkey 27 mer – E2M HLP
Squirrel monkey 27 mer – G4H HLP
Counts
0 10 20 30 40
FL2-H
$10^0$ $10^1$ $10^2$ $10^3$ $10^4$

Figure 6E

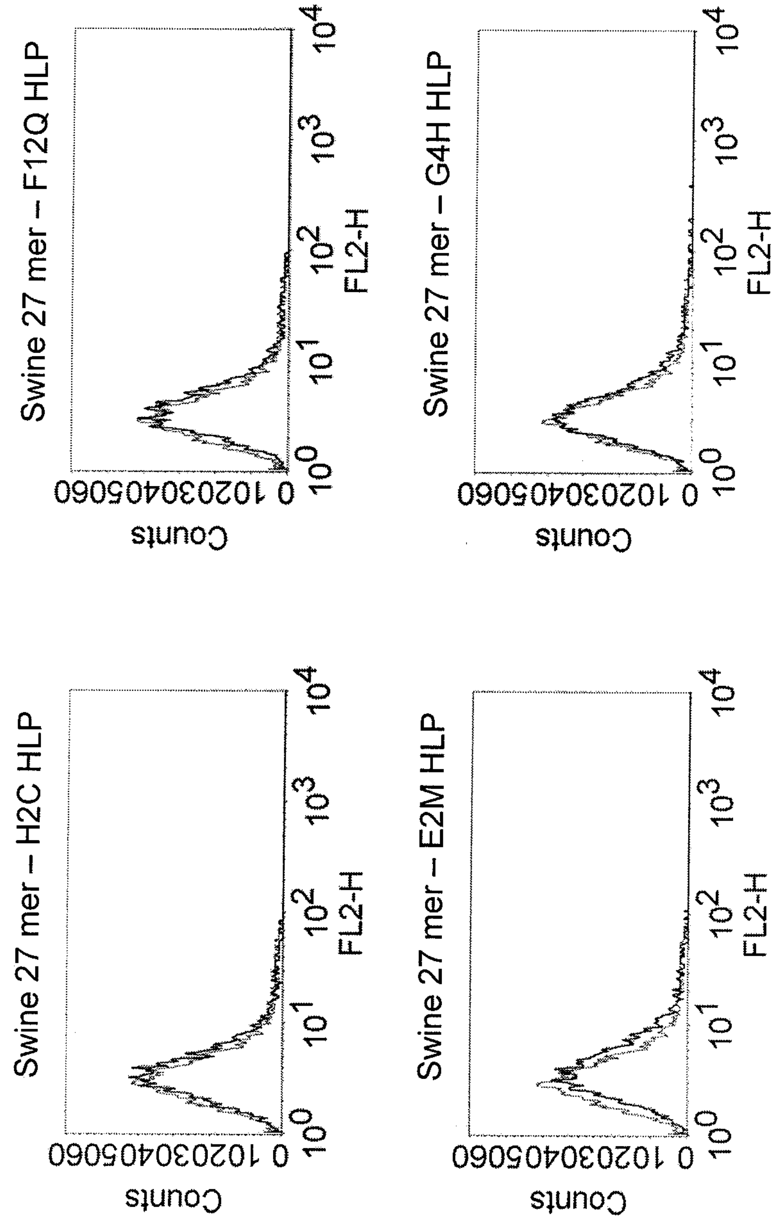
Swine 27 mer – H2C HLP
Swine 27 mer – F12Q HLP
Swine 27 mer – E2M HLP
Swine 27 mer – G4H HLP
Counts
0 10203040505060
FL2-H
$10^0$ $10^1$ $10^2$ $10^3$ $10^4$

Figure 7

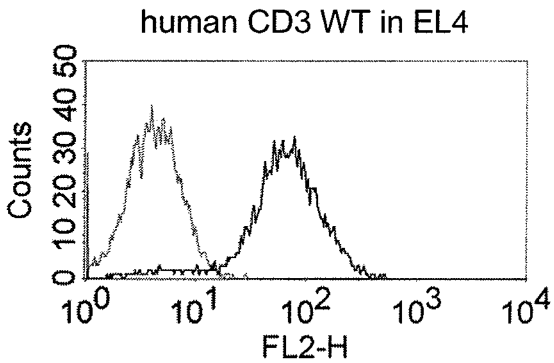
human CD3 WT in EL4
Counts
0
10
20
30
40
50
$10^0$
$10^1$
$10^2$
$10^3$
$10^4$
FL2-H

Figure 8A

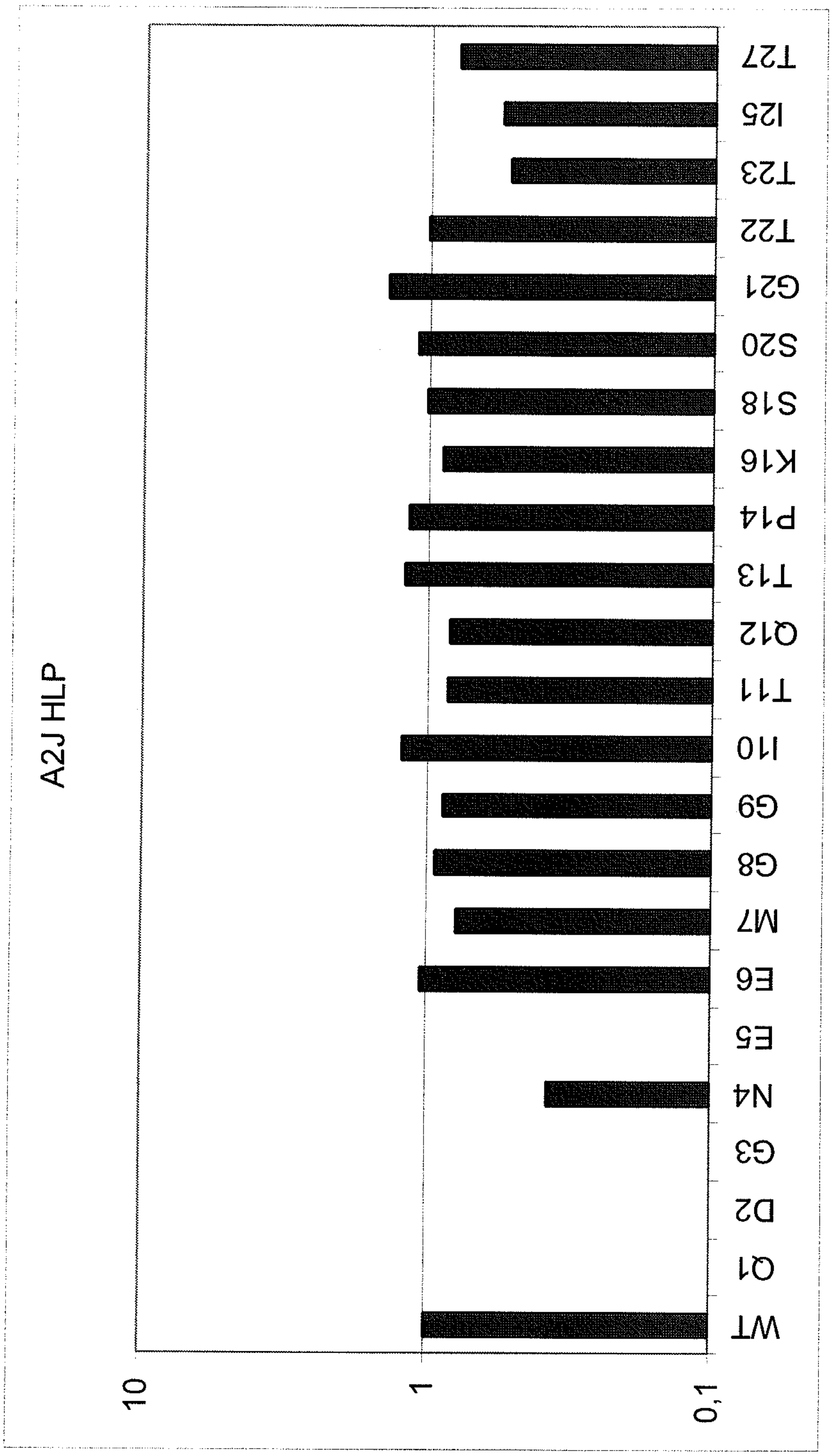
A2J HLP
10
1
0,1
WT
Q1
D2
G3
N4
E5
E6
M7
G8
G9
I10
T11
Q12
T13
P14
K16
S18
S20
G21
T22
T23
I25
T27

Figure 8B

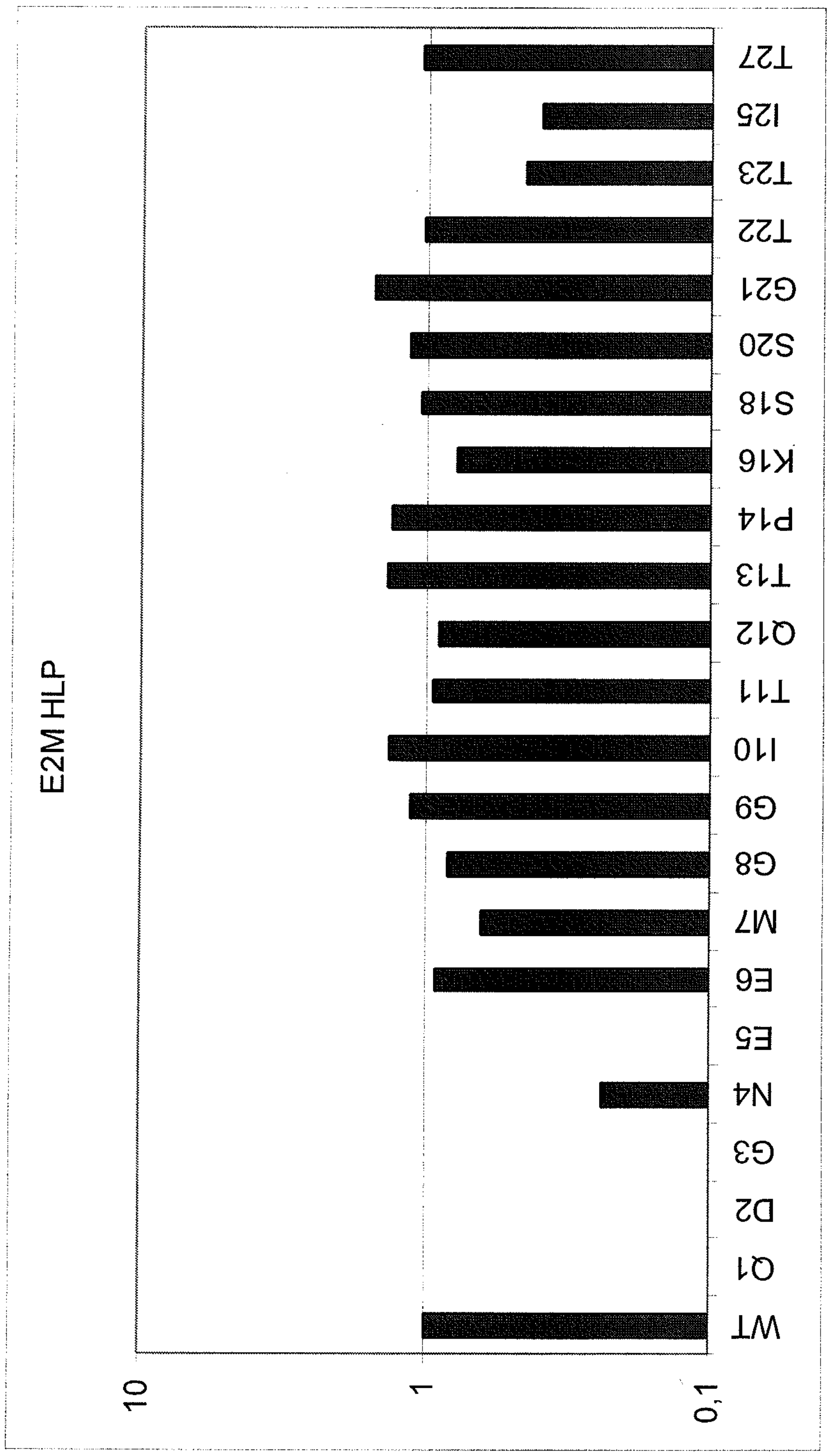
E2M HLP
10
1
0,1
WT
Q1
D2
G3
N4
E5
E6
M7
G8
G9
I10
T11
Q12
T13
P14
K16
S18
S20
G21
T22
T23
I25
T27

Figure 8C

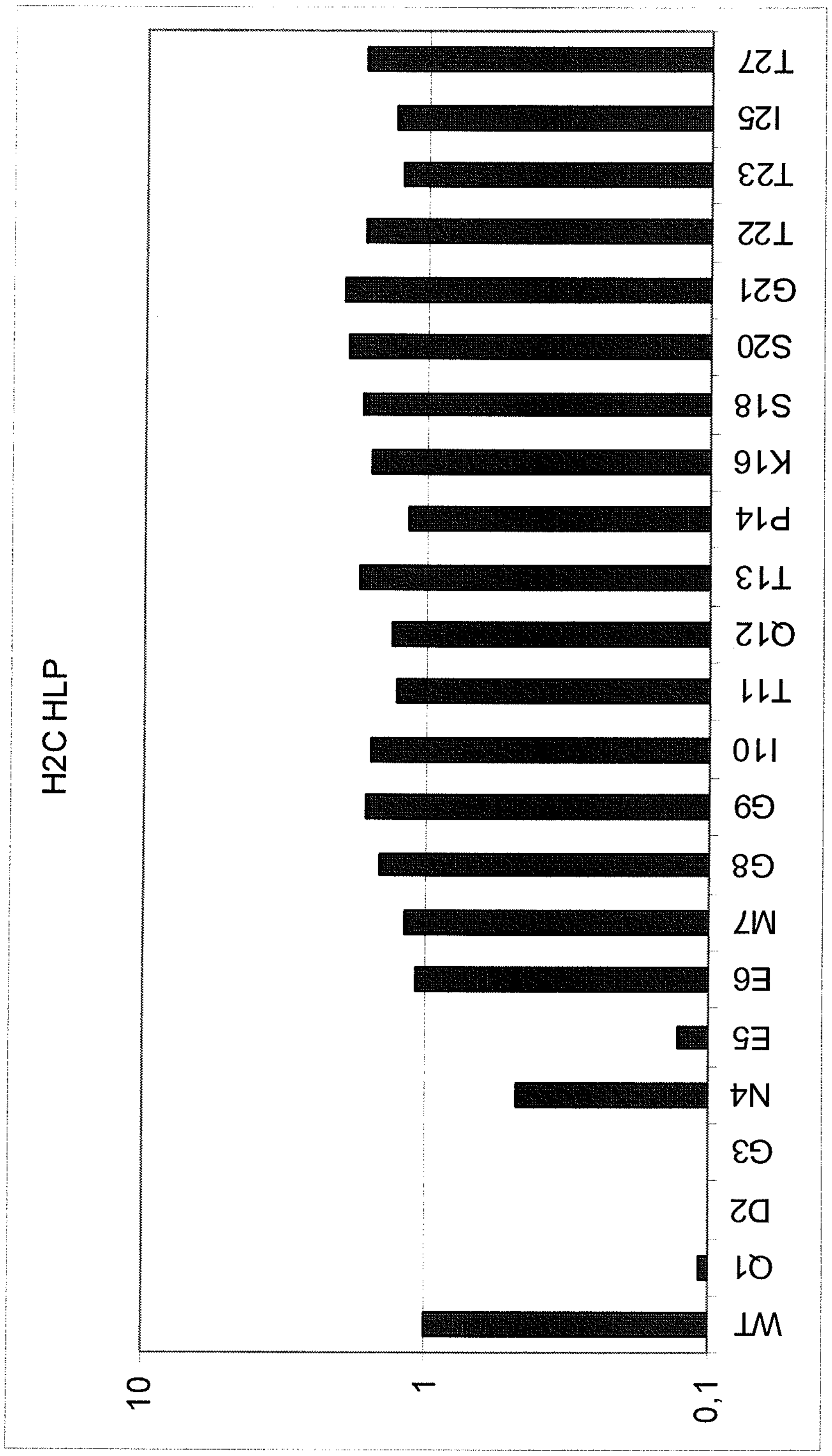
H2C HLP
10
1
0,1
WT
Q1
D2
G3
N4
E5
E6
M7
G8
G9
I10
T11
Q12
T13
P14
K16
S18
S20
G21
T22
T23
I25
T27

Figure 8D

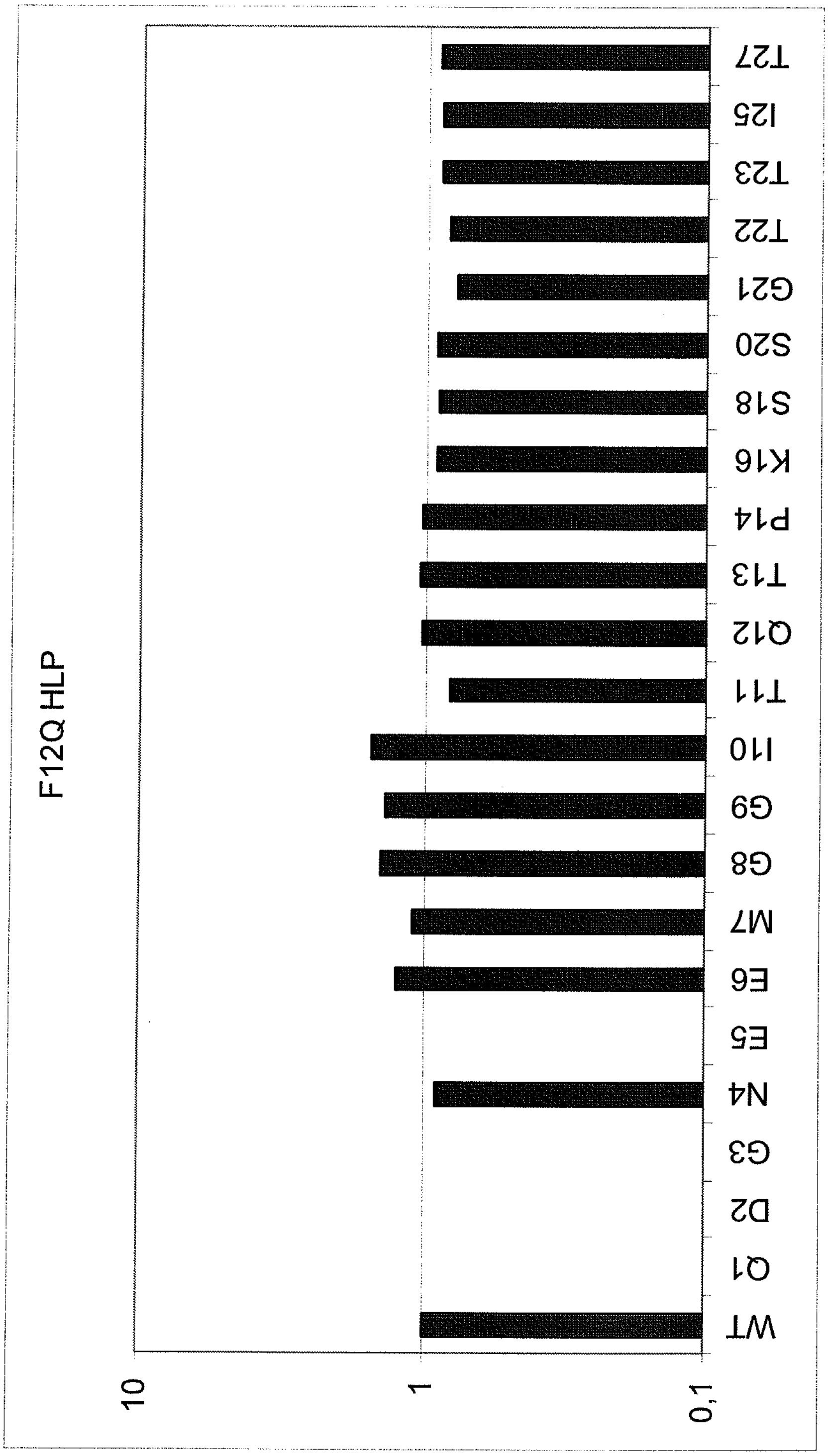
F12Q HLP
10
1
0,1
WT
Q1
D2
G3
N4
E5
E6
M7
G8
G9
I10
T11
Q12
T13
P14
K16
S18
S20
G21
T22
T23
I25
T27

Figure 9

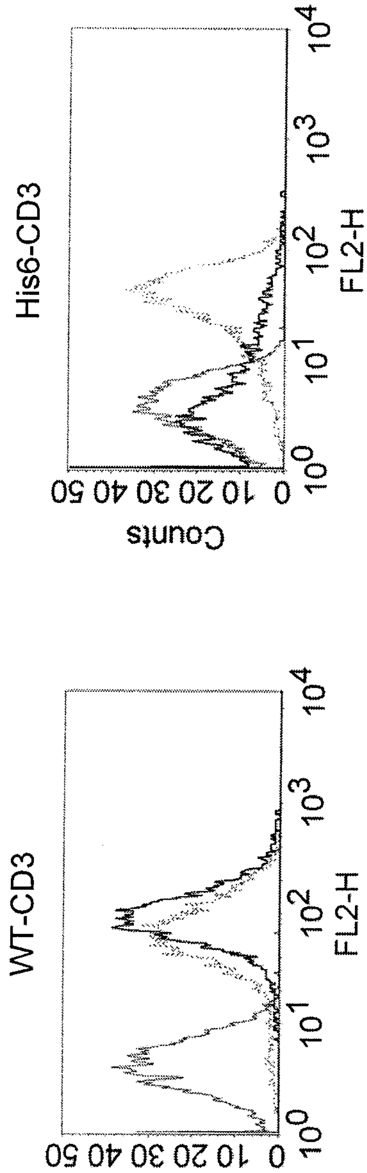

Figure 10

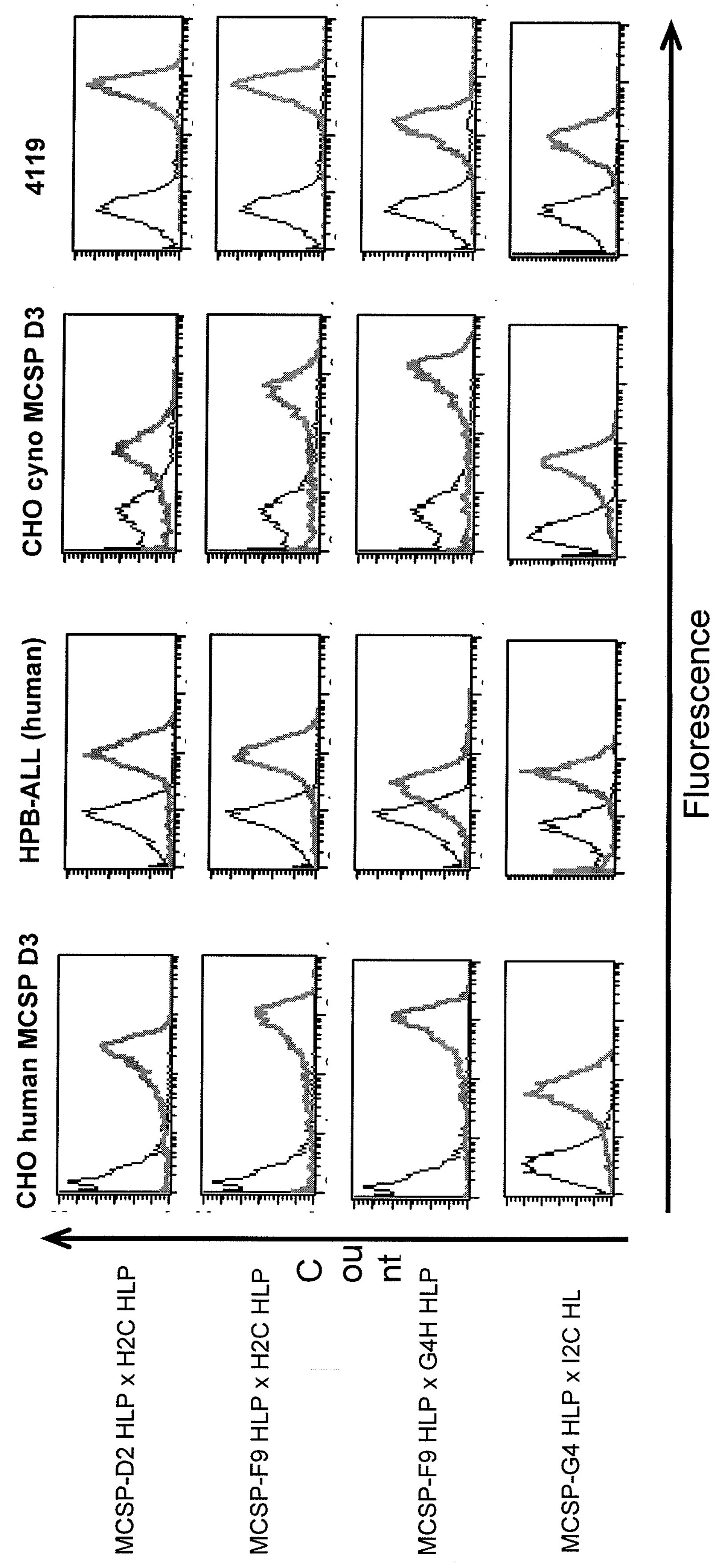
CHO human MCSP D3
HPB-ALL (human)
CHO cyno MCSP D3
4119
MCSP-D2 HLP x H2C HLP
MCSP-F9 HLP x H2C HLP
MCSP-F9 HLP x G4H HLP
MCSP-G4 HLP x I2C HL
Count
Fluorescence

Figure 11

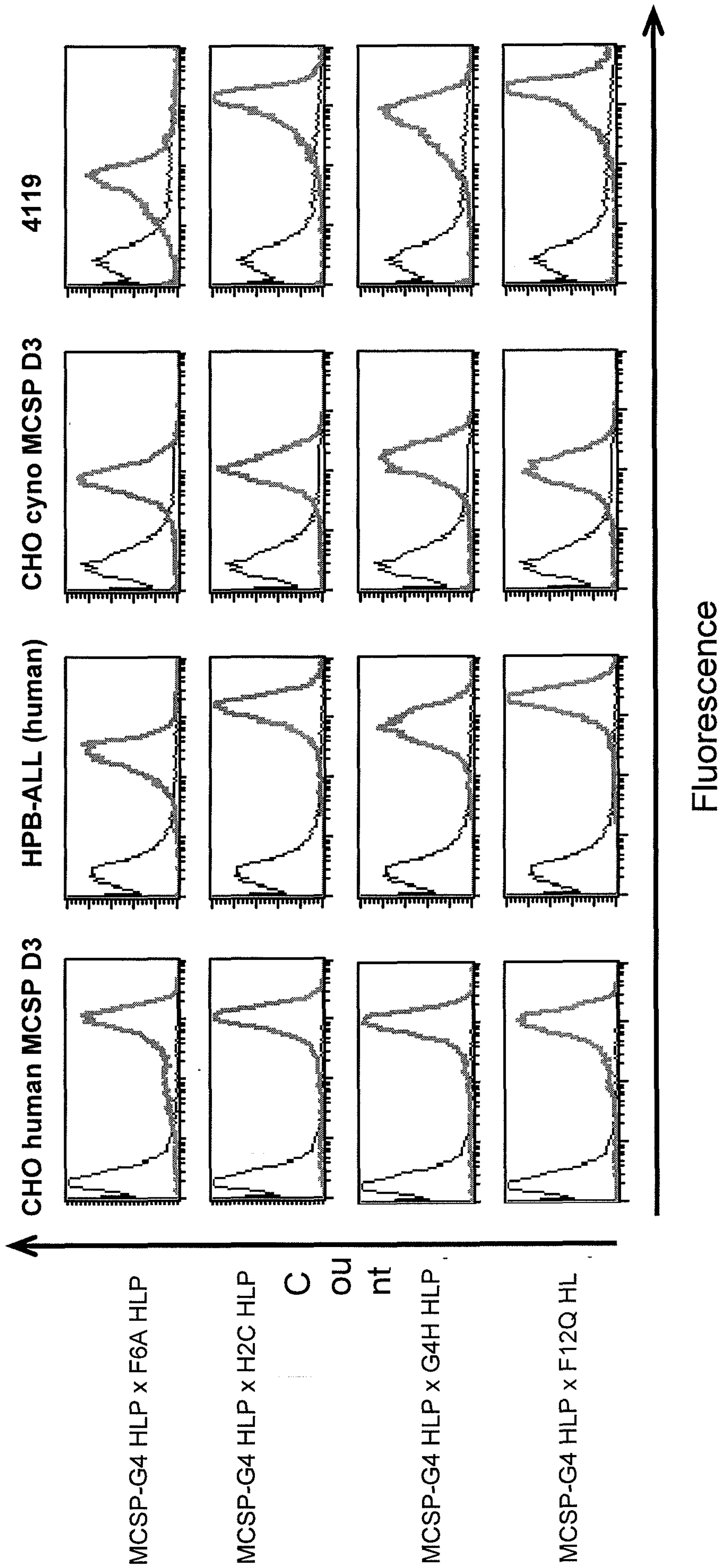
CHO human MCSP D3
HPB-ALL (human)
CHO cyno MCSP D3
4119
Fluorescence
C
ou
nt
MCSP-G4 HLP x F6A HLP
MCSP-G4 HLP x H2C HLP
MCSP-G4 HLP x G4H HLP
MCSP-G4 HLP x F12Q HL

Figure 12

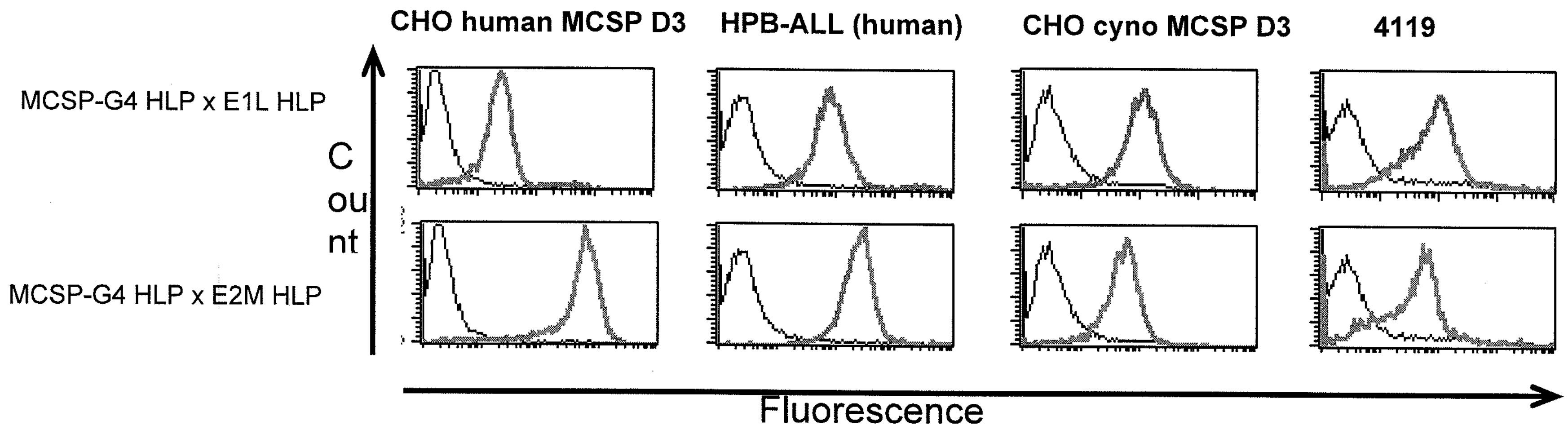
CHO human MCSP D3
HPB-ALL (human)
CHO cyno MCSP D3
4119
MCSP-G4 HLP x E1L HLP
MCSP-G4 HLP x E2M HLP
Count
Fluorescence

**Figure 13**

**A)**

Effector cells: stimulated CD4/CD56 depleted human PBMC
Target cells: CHO transfected with human MCSP D3

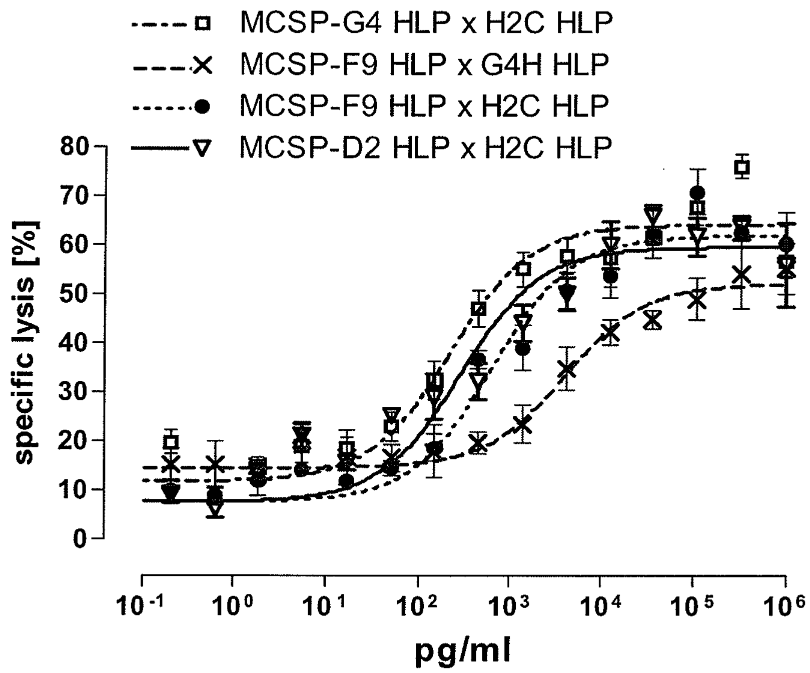


**B)**

Effector cells: macaque T cell line 4119 LnPx
Target cells: CHO transfected with cynomolgus MCSP D3

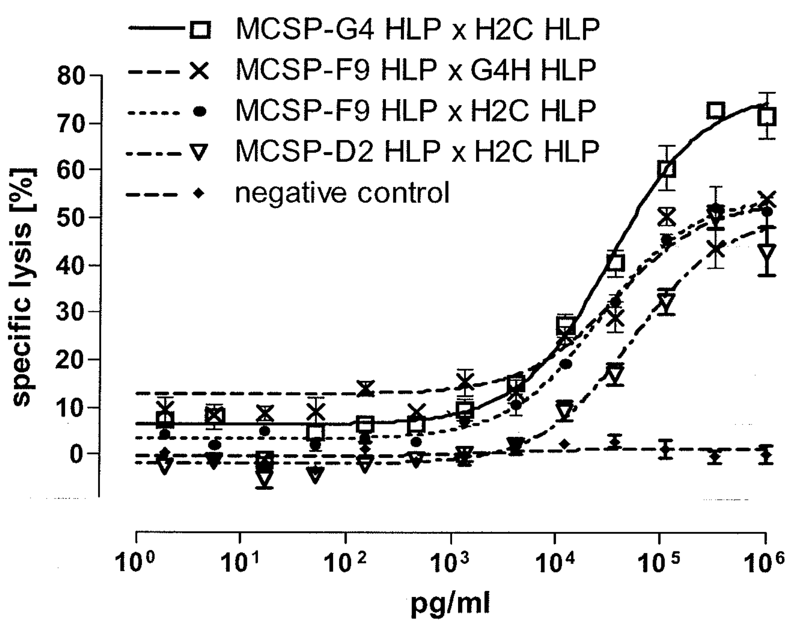

Figure 14

A)

Effector cells: macaque T cell line 4119 LnPx
Target cells: CHO transfected with cynomolgus MCSP D3

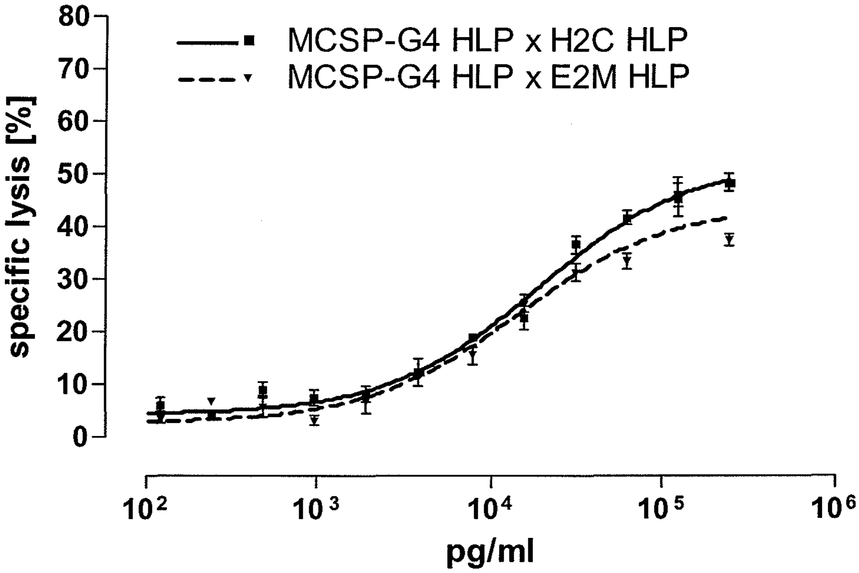


B)

Effector cells: macaque T cell line 4119 LnPx
Target cells: CHO transfected with cynomolgus MCSP D3

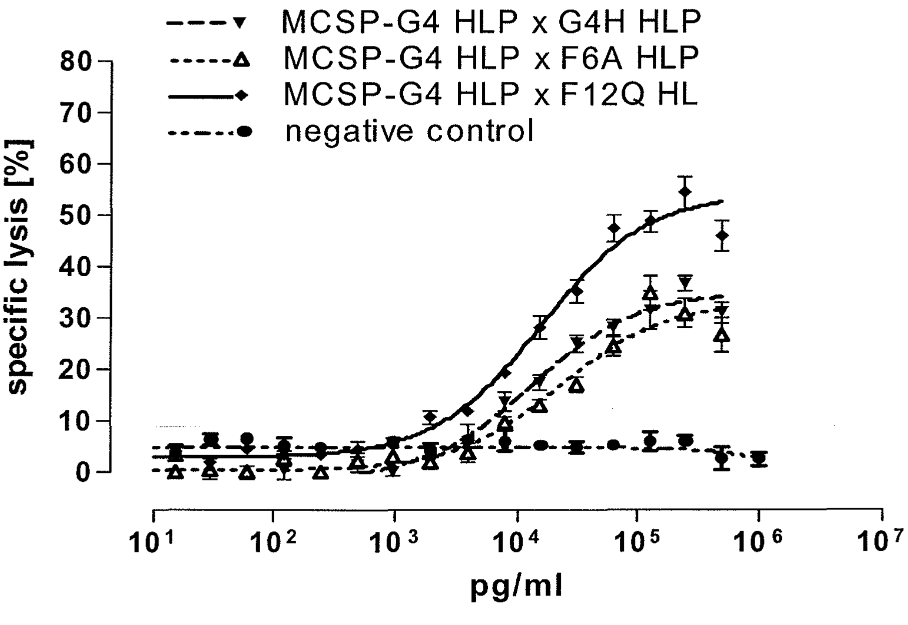

**Figure 15**

**A)**

Effector cells: stimulated CD4/CD56 depleted human PBMC
Target cells: CHO transfected with human MCSP D3

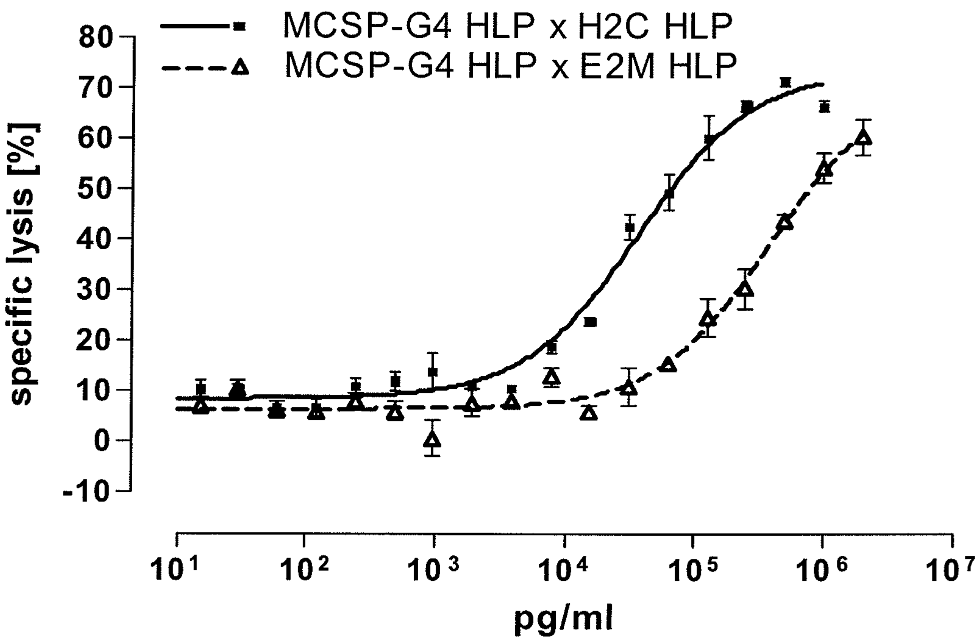


B)

Effector cells: stimulated CD4/CD56 depleted human PBMC
Target cells: CHO transfected with human MCSP D3

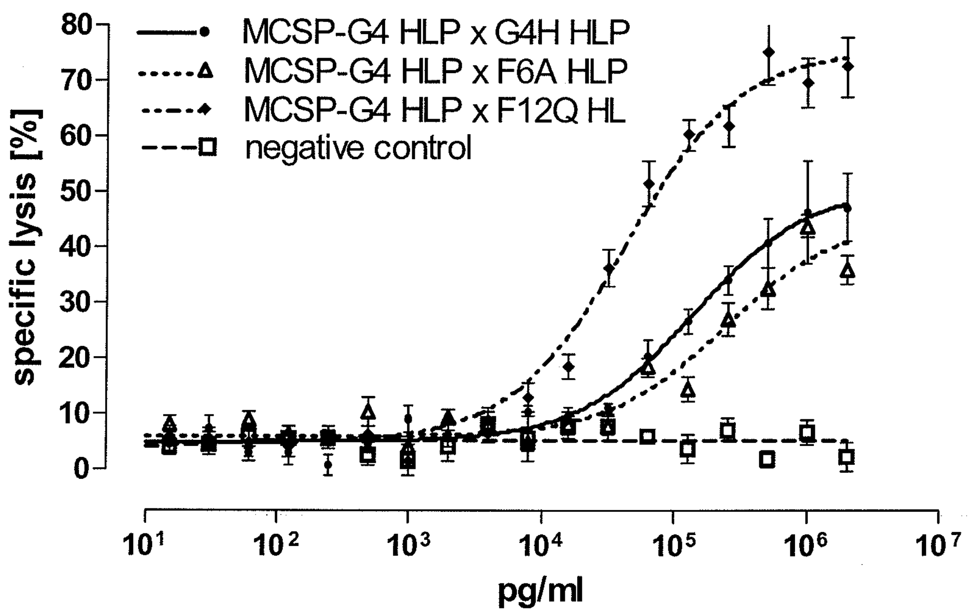

Figure 16

A)

**Effector cells: stimulated CD4/CD56 depleted human PBMC**
**Target cells: CHO transfected with human MCSP D3**

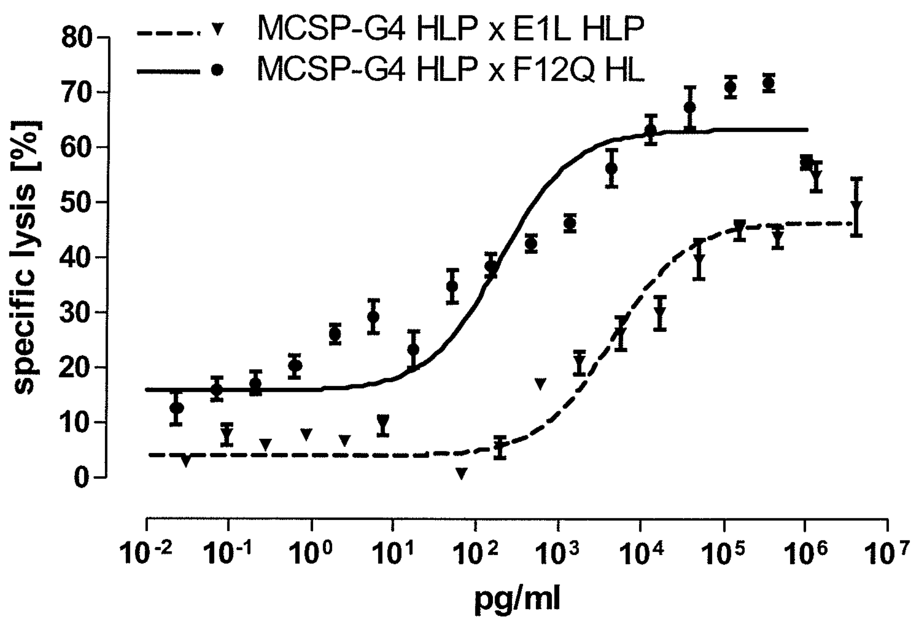


B)

**Effector cells: macaque T cell line 4119 LnPx**
**Target cells: CHO transfected with cynomolgus MCSP D**

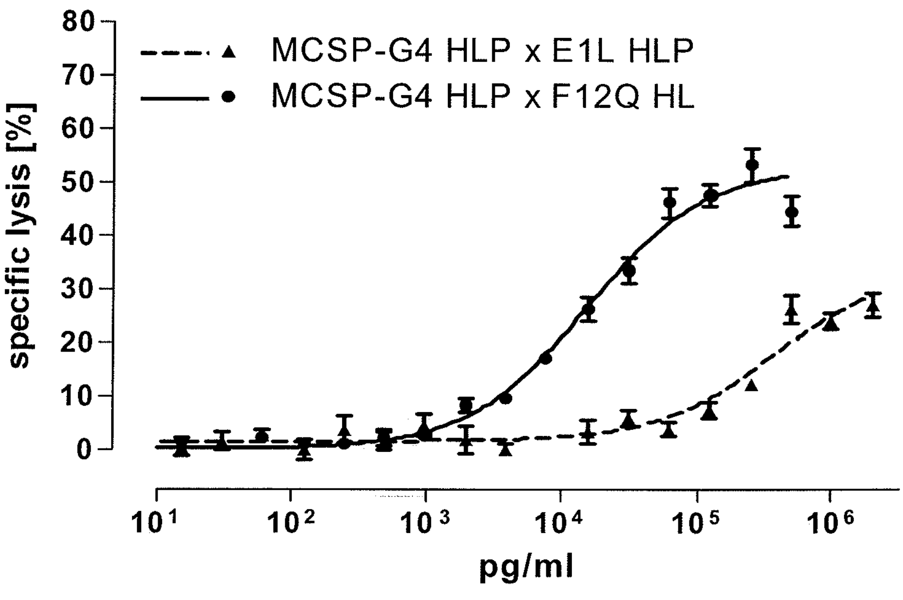

Figure 17

A)

Effector cells: stimulated CD4/CD56 depleted human PBMC
Target cells: CHO transfected with human MCSP D3

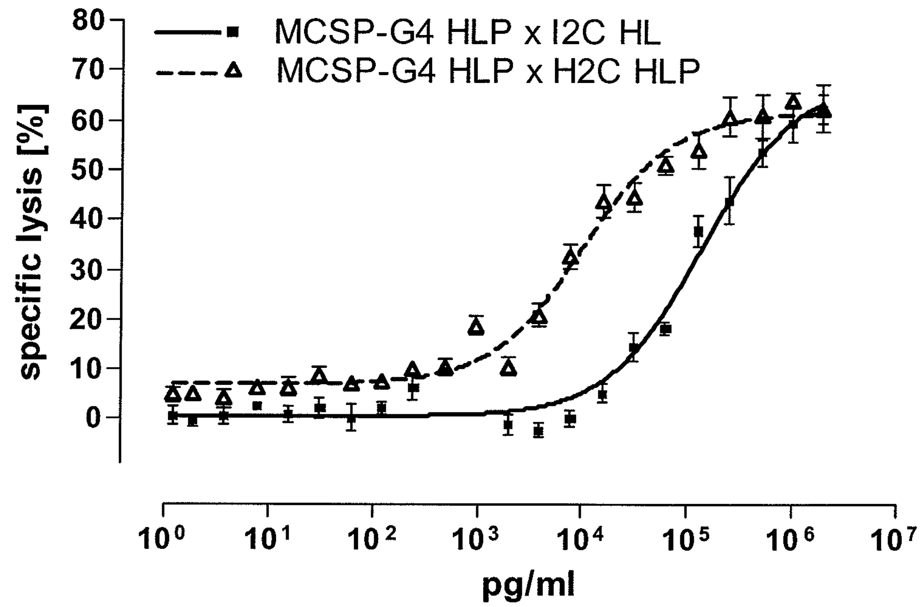


B)

Effector cells: macaque T cell line 4119 LnPx
Target cells: CHO transfected with cynomolgus MCSP D3

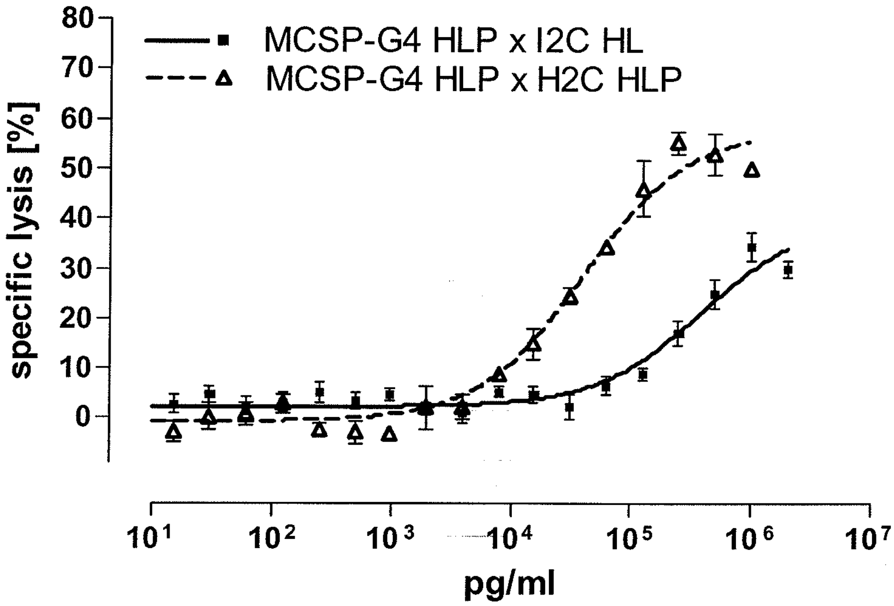

# Figure 18 (1)

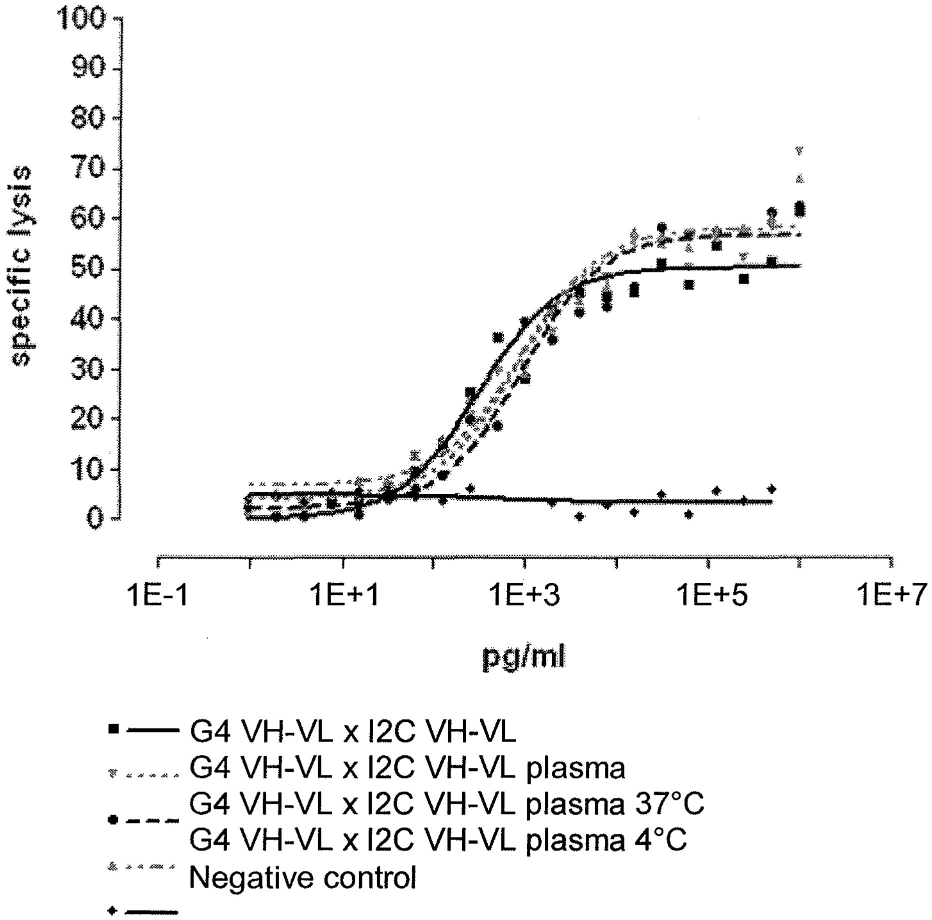
100
90
80
70
60
50
40
30
20
10
0
specific lysis
1E-1
1E+1
1E+3
1E+5
1E+7
pg/ml
G4 VH-VL x I2C VH-VL
G4 VH-VL x I2C VH-VL plasma
G4 VH-VL x I2C VH-VL plasma 37°C
G4 VH-VL x I2C VH-VL plasma 4°C
Negative control

# Figure 18 (2)

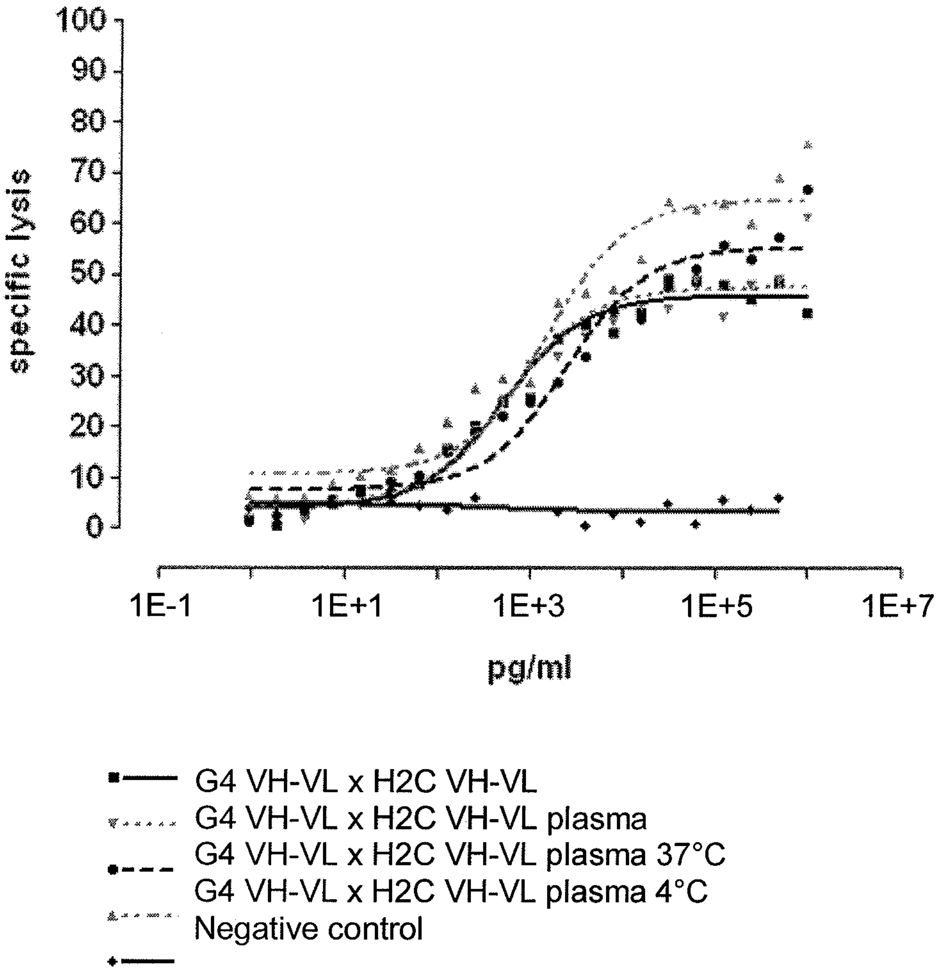
100
90
80
70
60
50
40
30
20
10
0
specific lysis
1E-1
1E+1
1E+3
1E+5
1E+7
pg/ml
G4 VH-VL x H2C VH-VL
G4 VH-VL x H2C VH-VL plasma
G4 VH-VL x H2C VH-VL plasma 37°C
G4 VH-VL x H2C VH-VL plasma 4°C
Negative control

# Figure 18 (3)

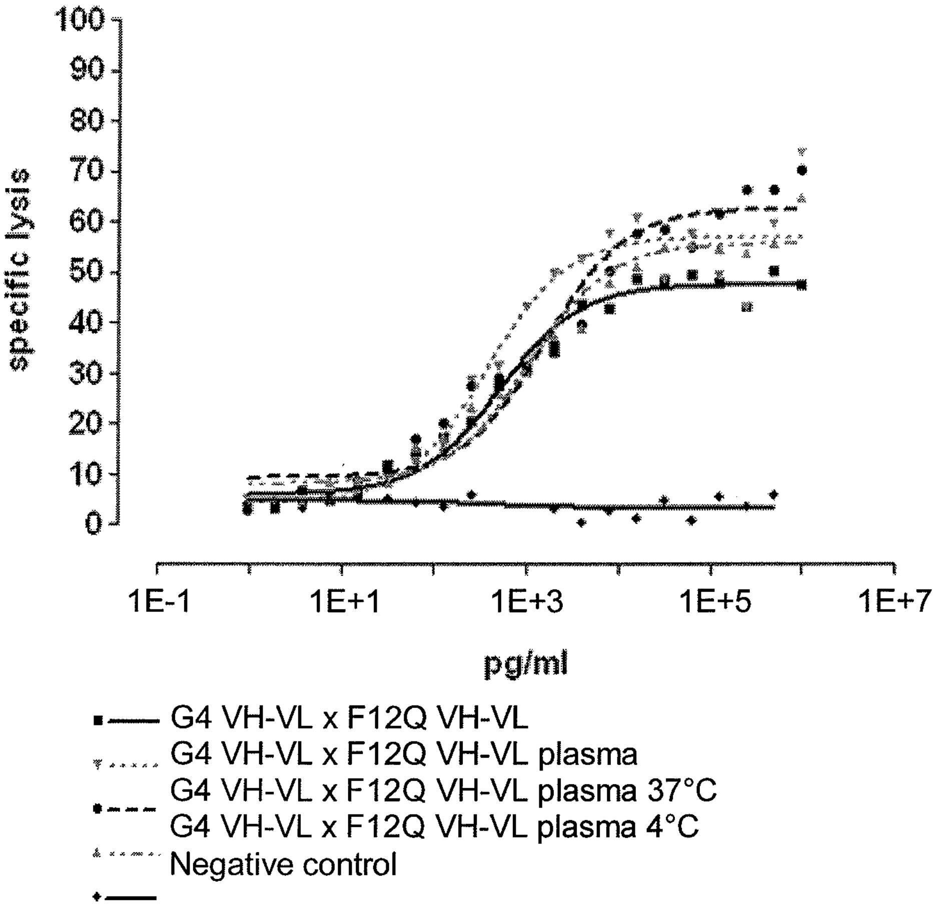
100
90
80
70
60
50
40
30
20
10
0
specific lysis
1E-1
1E+1
1E+3
1E+5
1E+7
pg/ml
G4 VH-VL x F12Q VH-VL
G4 VH-VL x F12Q VH-VL plasma
G4 VH-VL x F12Q VH-VL plasma 37°C
G4 VH-VL x F12Q VH-VL plasma 4°C
Negative control

**Figure 19a**

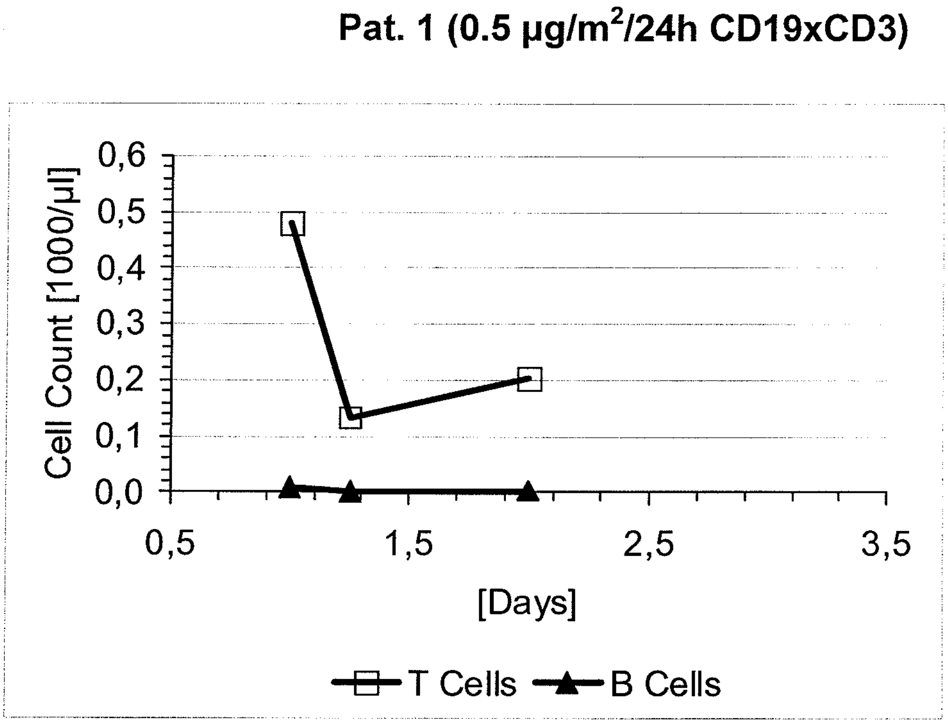


**Figure 19b**

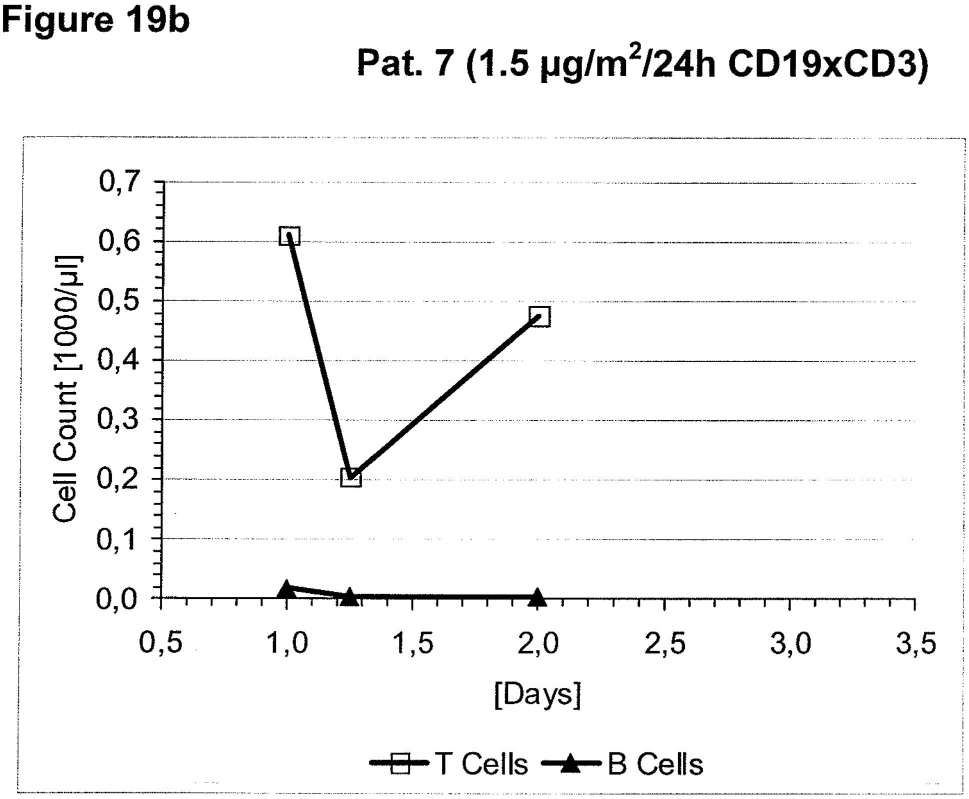

Figure 19c

Pat. 23 (15 µg/m²/24h CD19xCD3)

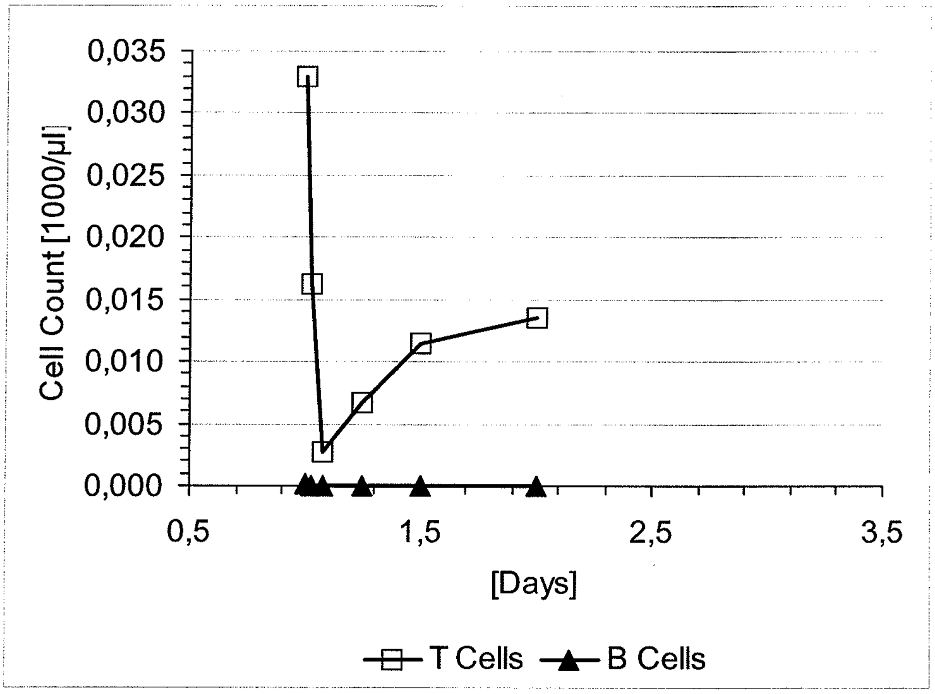


Figure 19d

Pat. 30 (30 µg/m²/24h CD19xCD3)

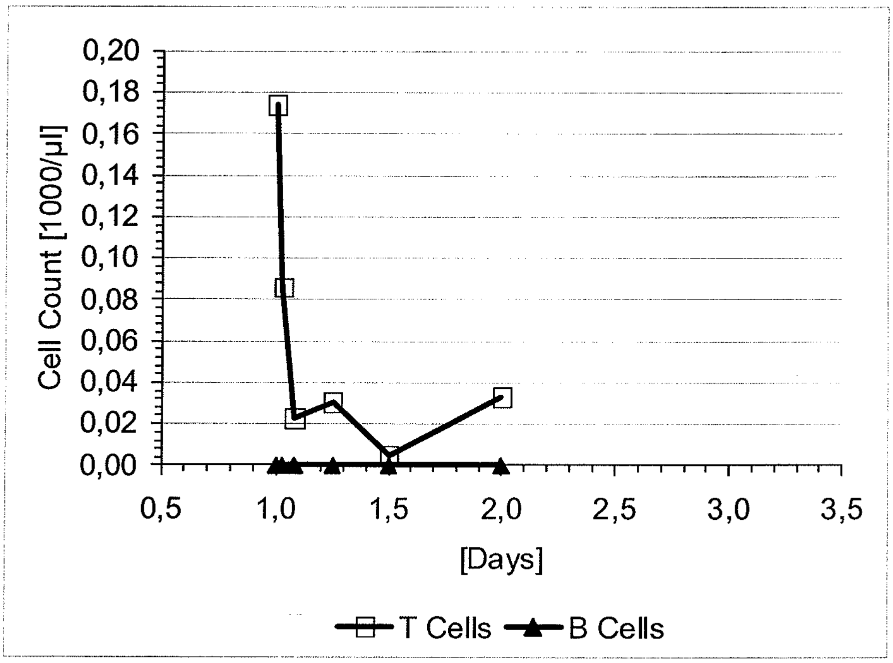

Figure 19e

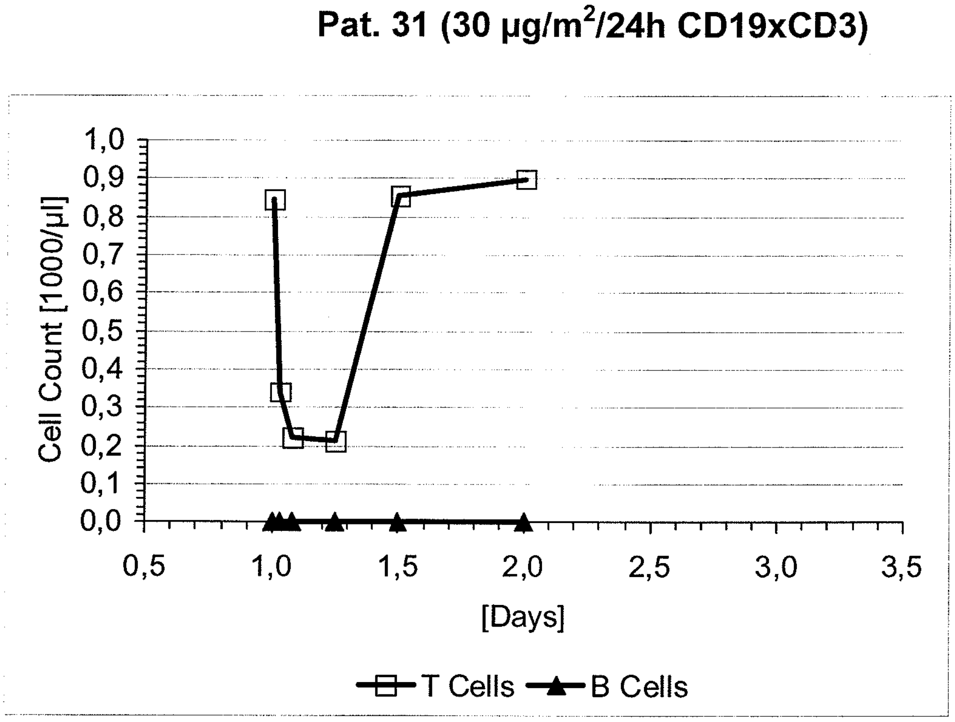


Figure 19f

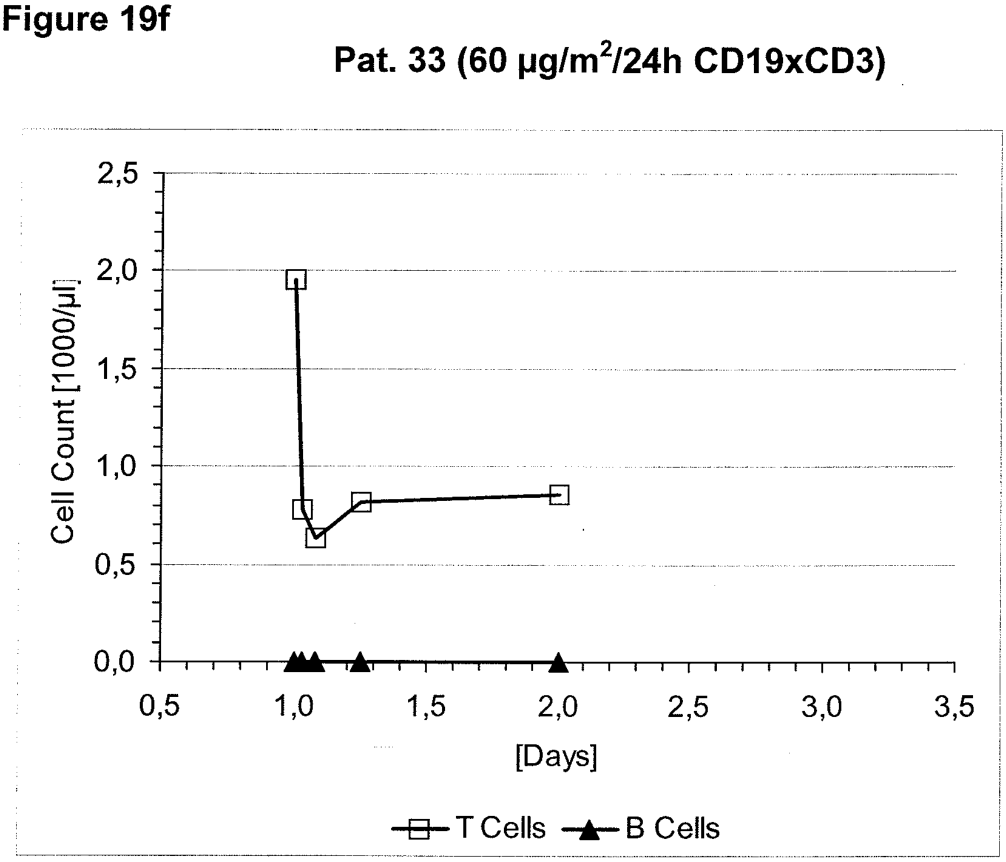

**Figure 20**

A)

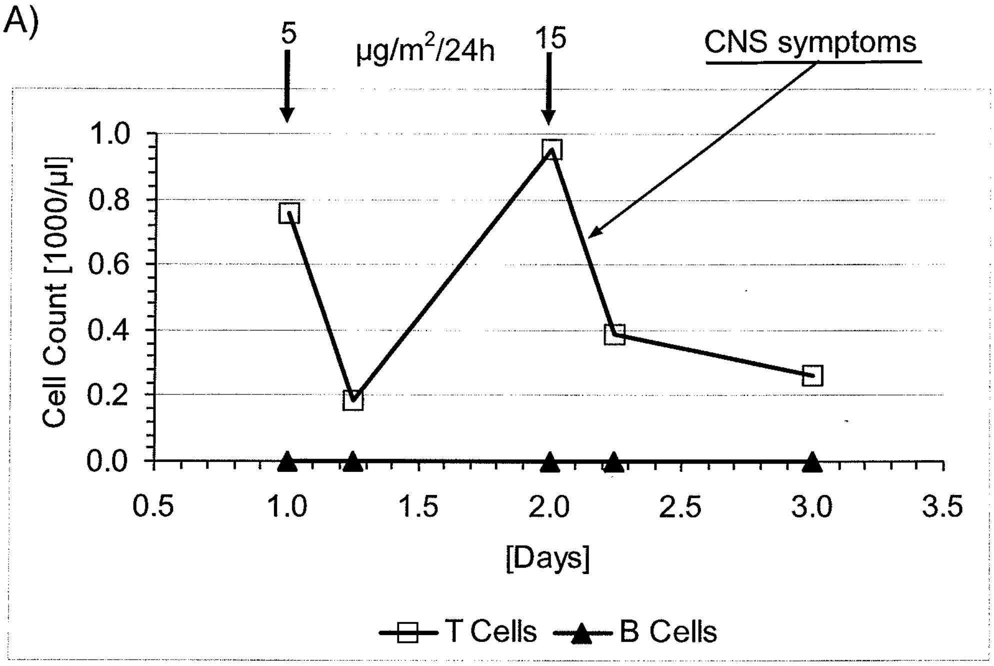


Pat. 19 (continuous infusion: 5 µg/m$^2$/24h for 1 day followed by 15 µg/m$^2$/24h CD19xCD3 maintenance)

B)

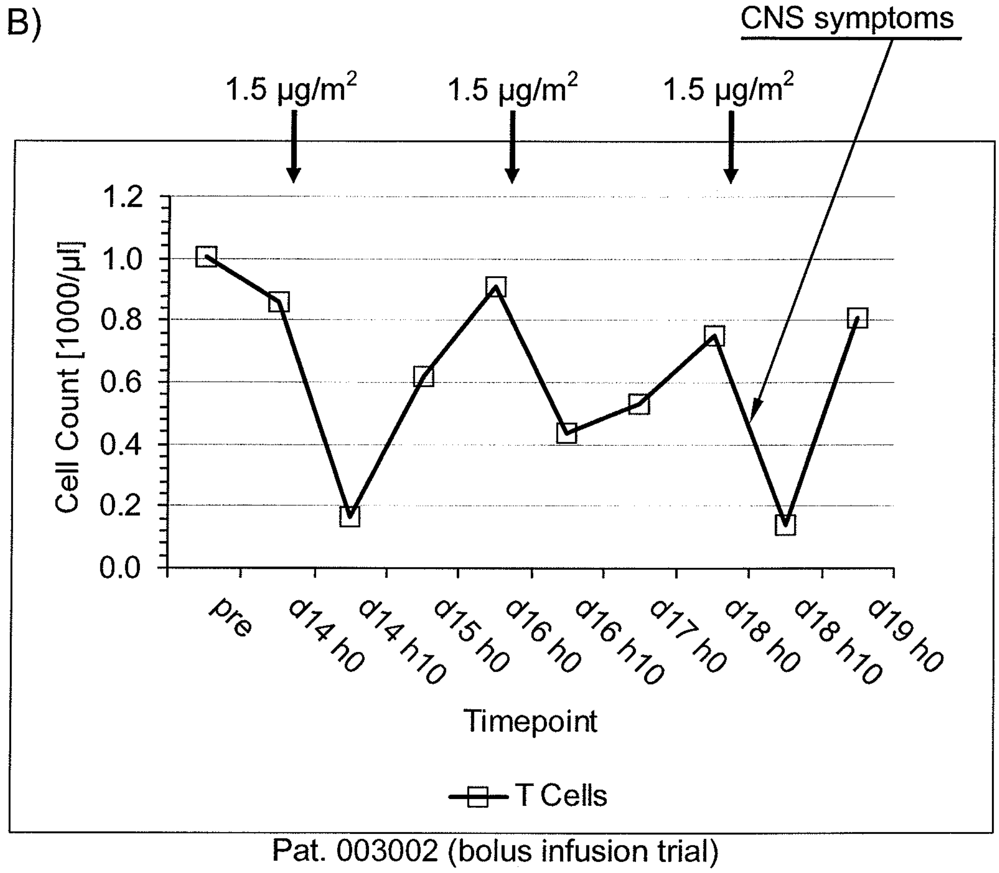


Pat. 003002 (bolus infusion trial)

**Figure 21**

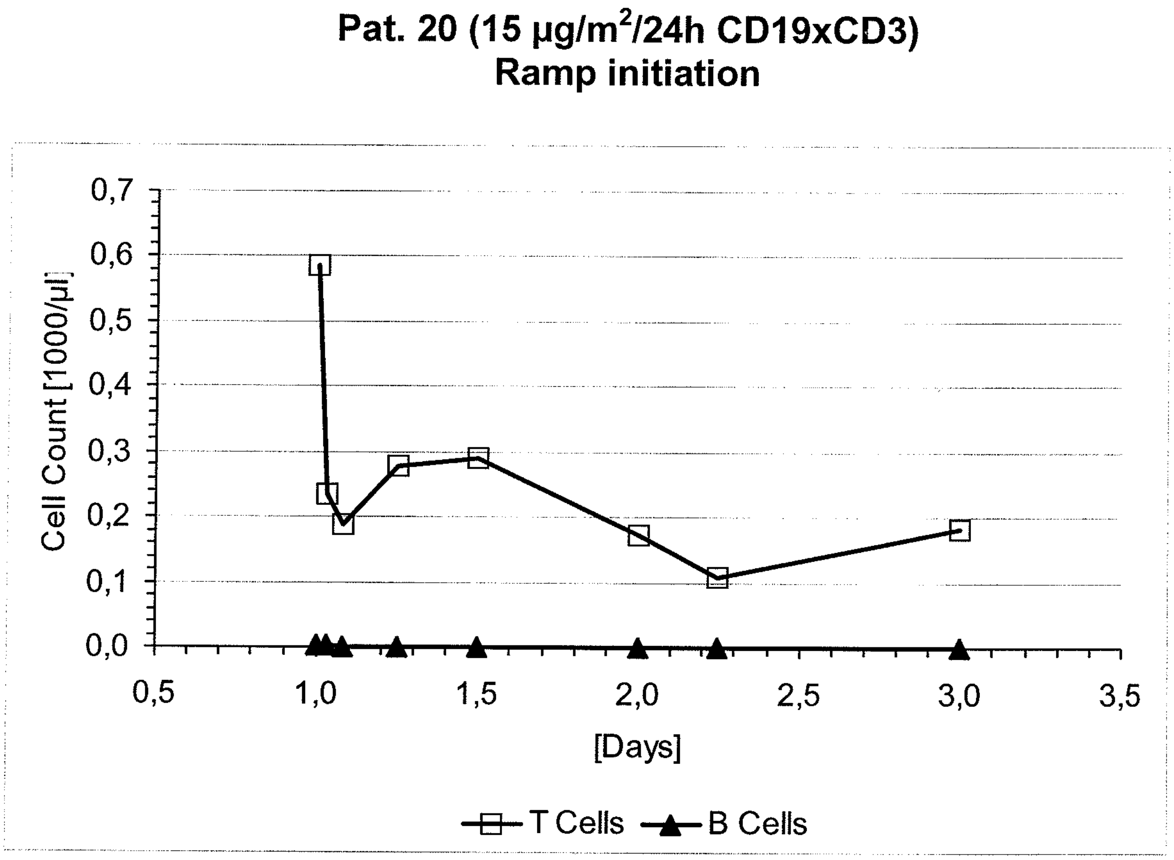
Pat. 20 (15 µg/m²/24h CD19xCD3)
Ramp initiation
0,7
0,6
0,5
0,4
0,3
0,2
0,1
0,0
Cell Count [1000/µl]
0,5
1,0
1,5
2,0
2,5
3,0
3,5
[Days]
T Cells
B Cells

**Figure 22**

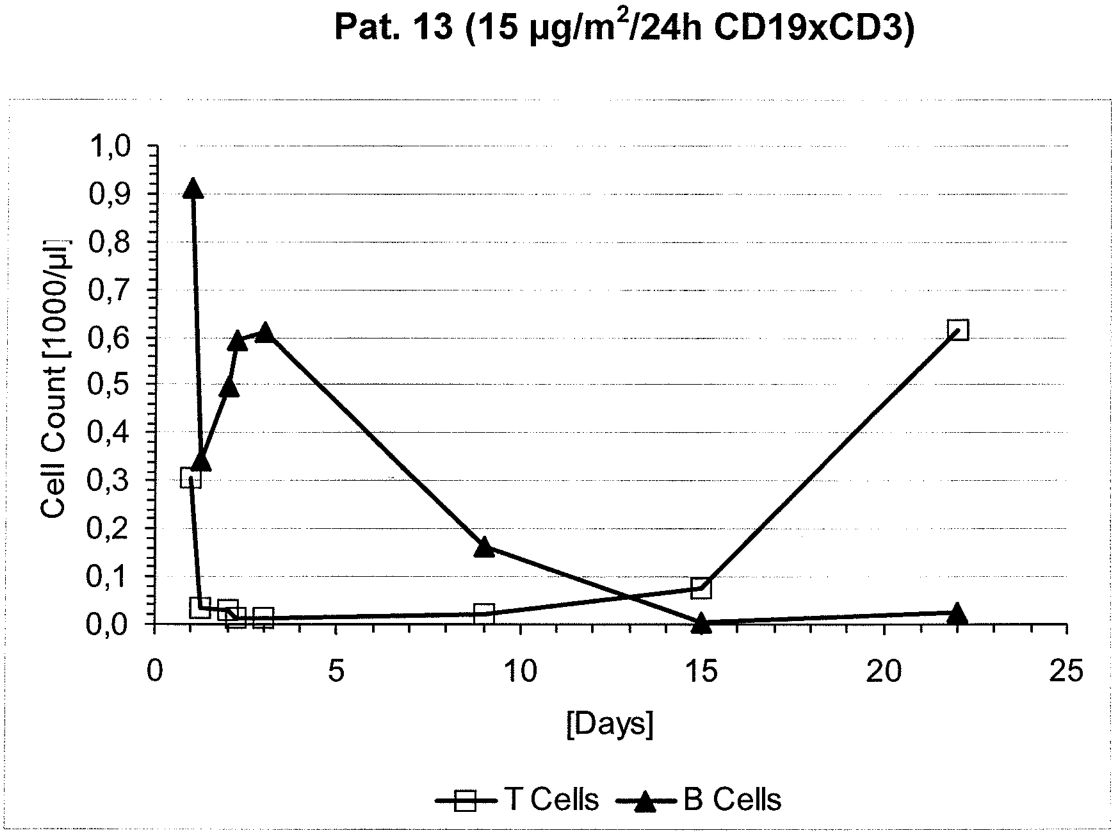
Pat. 13 (15 µg/m2/24h CD19xCD3)
1,0
0,9
0,8
0,7
0,6
0,5
0,4
0,3
0,2
0,1
0,0
Cell Count [1000/µl]
0
5
10
15
20
25
[Days]
T Cells
B Cells

**Figure 23**

**Pat. 24 (15 µg/m²/24h CD19xCD3 without HSA)**
**1st Treatment start**

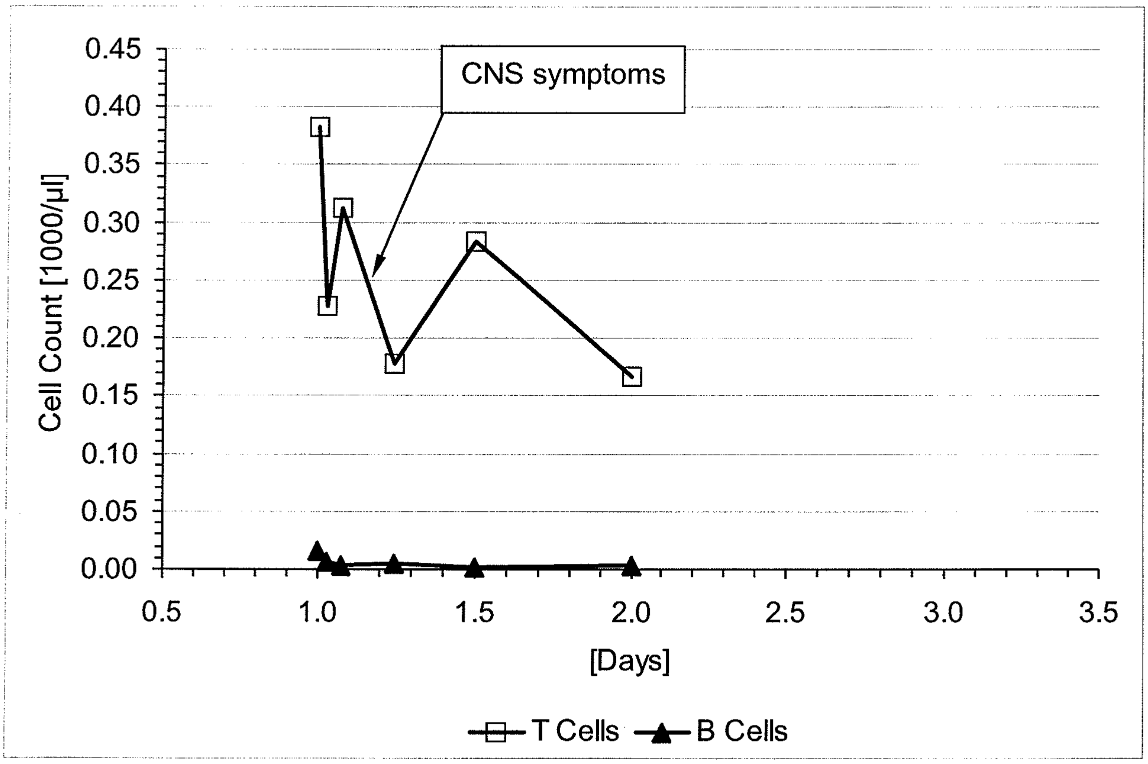


**Pat. 24 (15 µg/m²/24h CD19xCD3 with HSA)**
**2nd Treatment start**

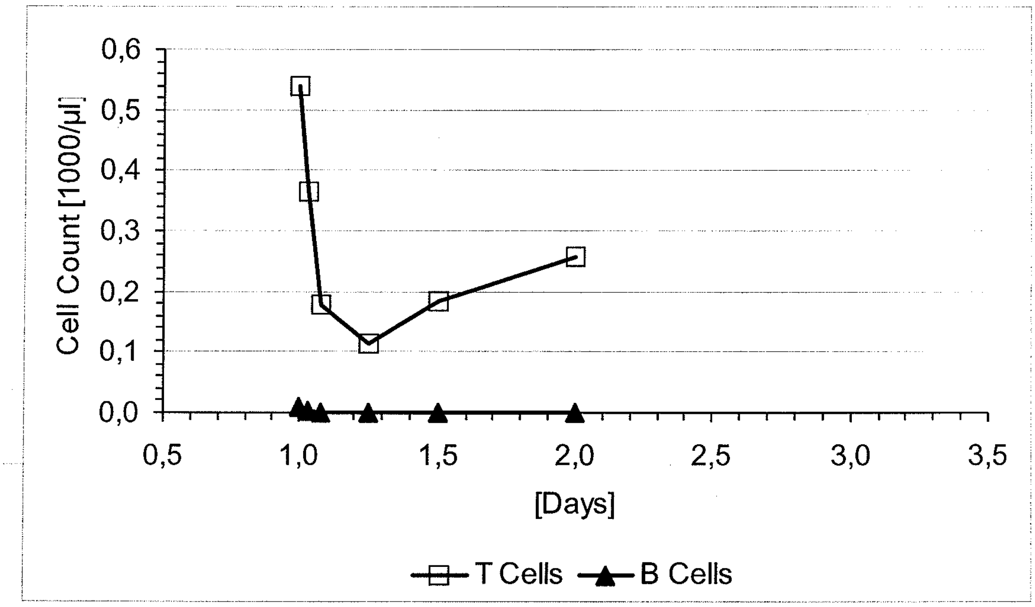

**Model of T cell adhesion to endothelial cells induced by monovalent binding to context dependent CD3 epitopes**

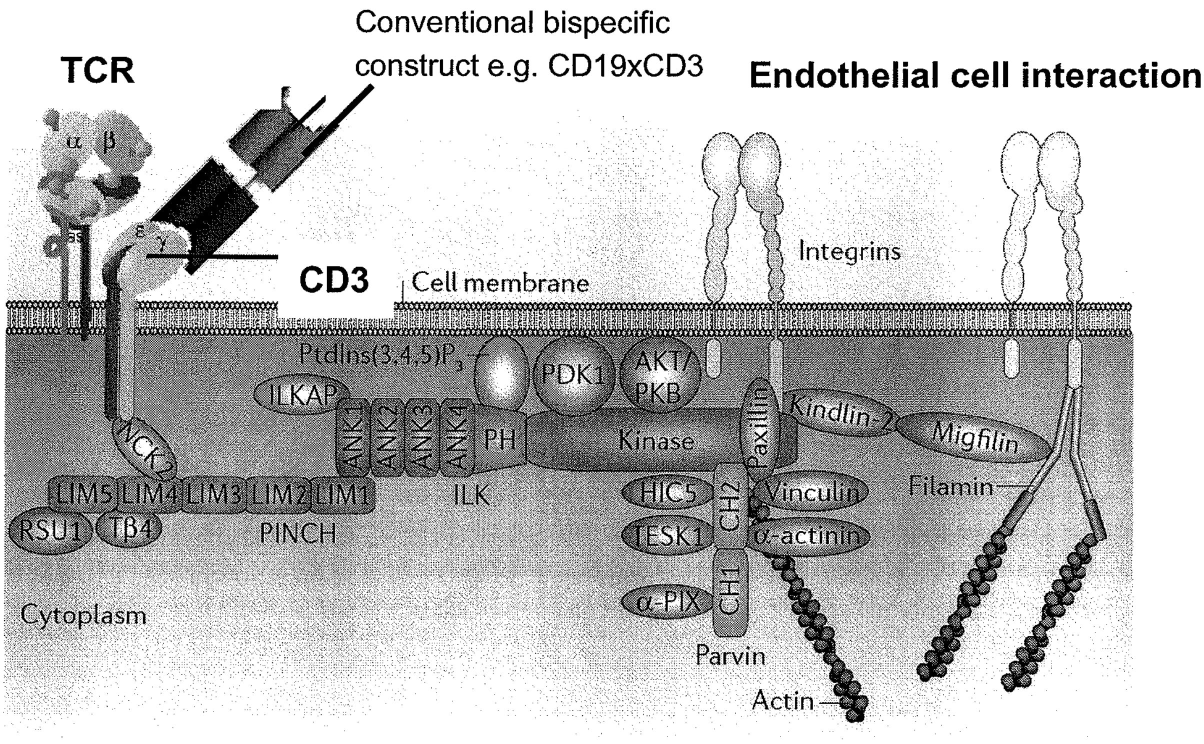


Figure 24

## Figure 25

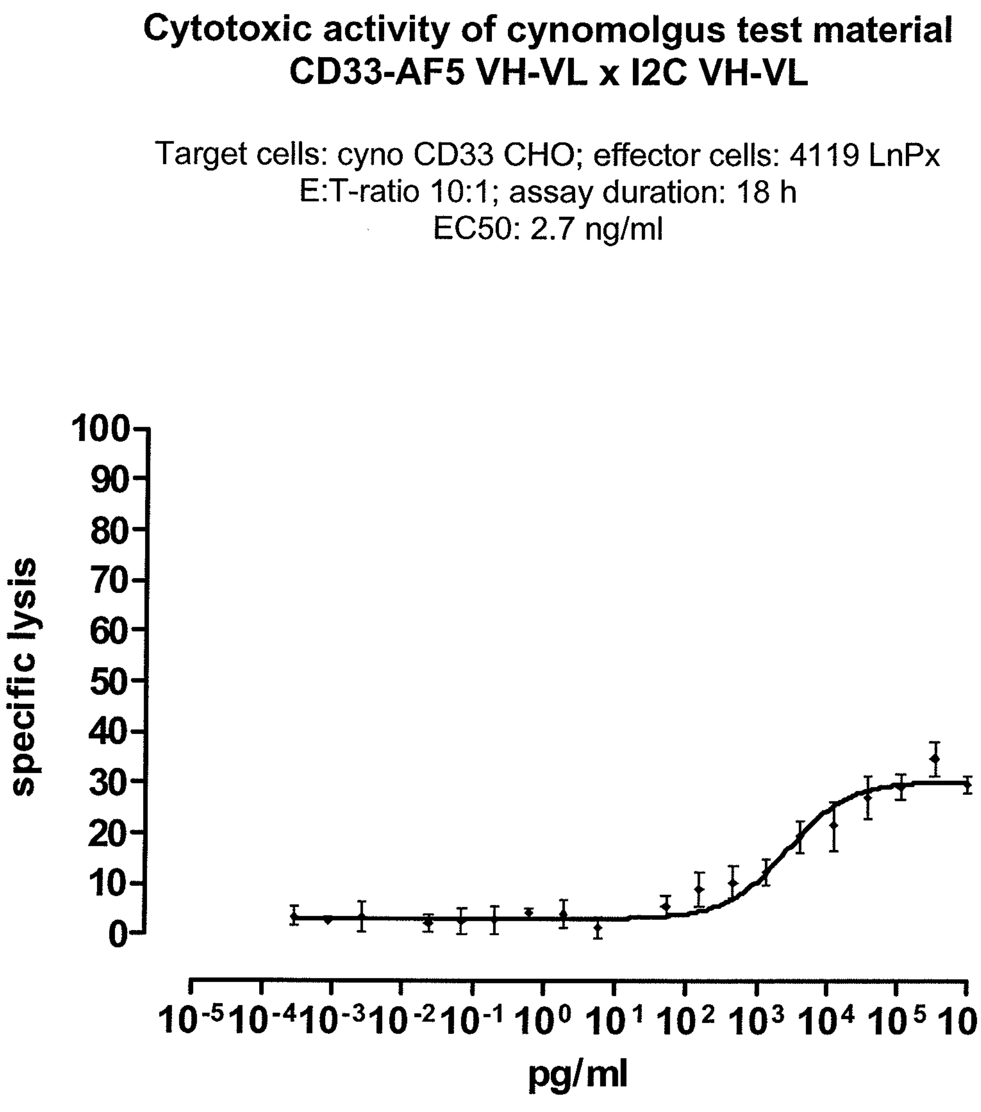
Cytotoxic activity of cynomolgus test material
CD33-AF5 VH-VL x I2C VH-VL
Target cells: cyno CD33 CHO; effector cells: 4119 LnPx
E:T-ratio 10:1; assay duration: 18 h
EC50: 2.7 ng/ml
specific lysis
100
90
80
70
60
50
40
30
20
10
0
10-5 10-4 10-3 10-2 10-1 100 101 102 103 104 105 106
pg/ml

## Figure 26 A

**Dose- and time-dependent depletion of blood CD33-monocytes through CD33-AF5 VH-VL x I2C VH-VL**

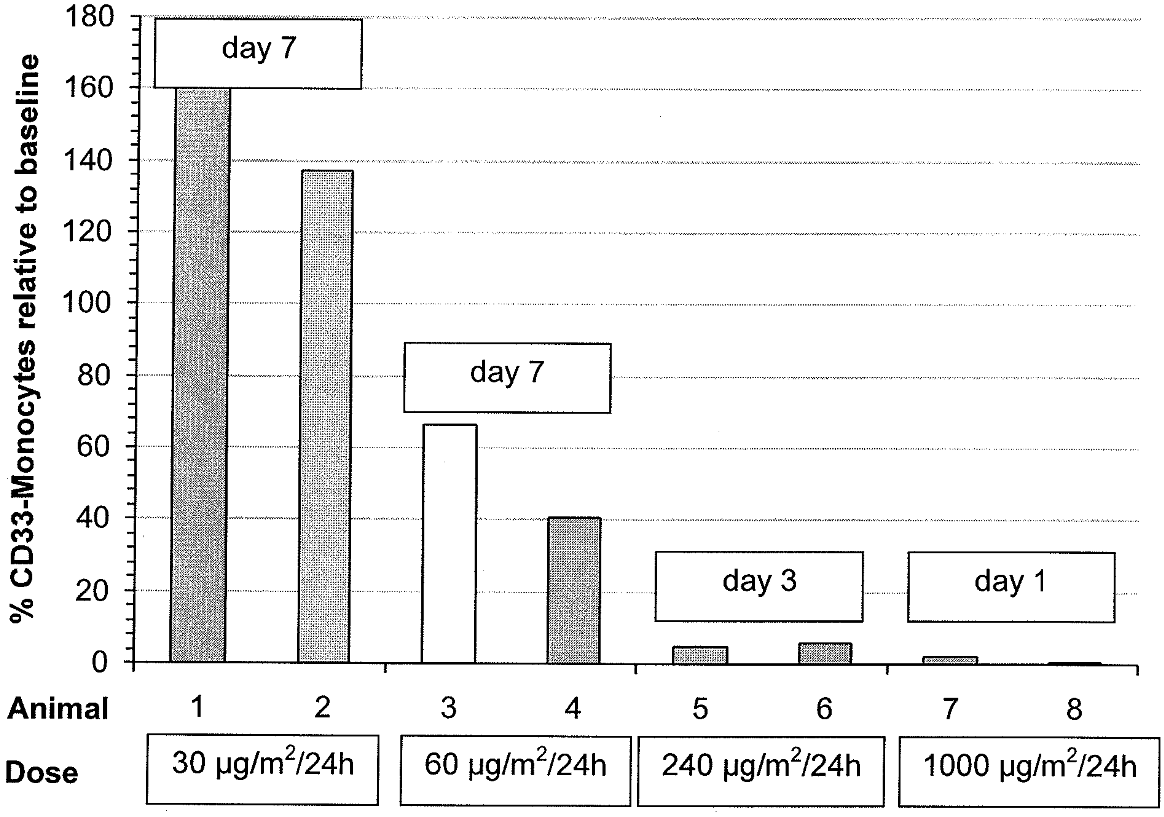

**Figure 26 B (1)**

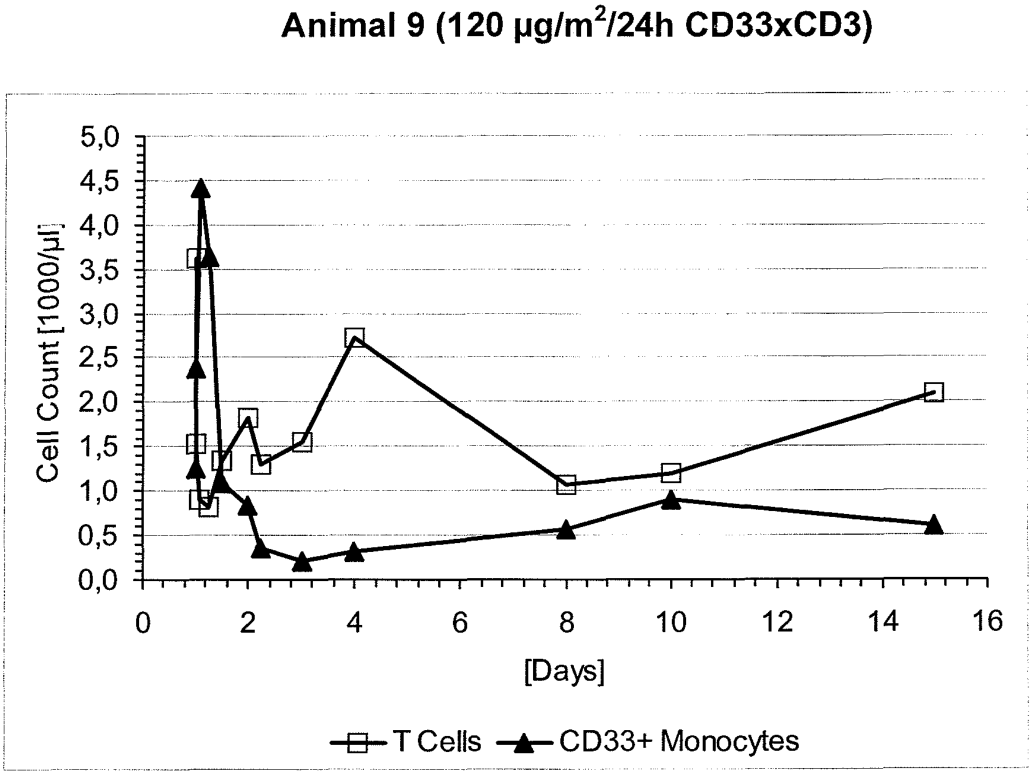


**Figure 26 B (2)**

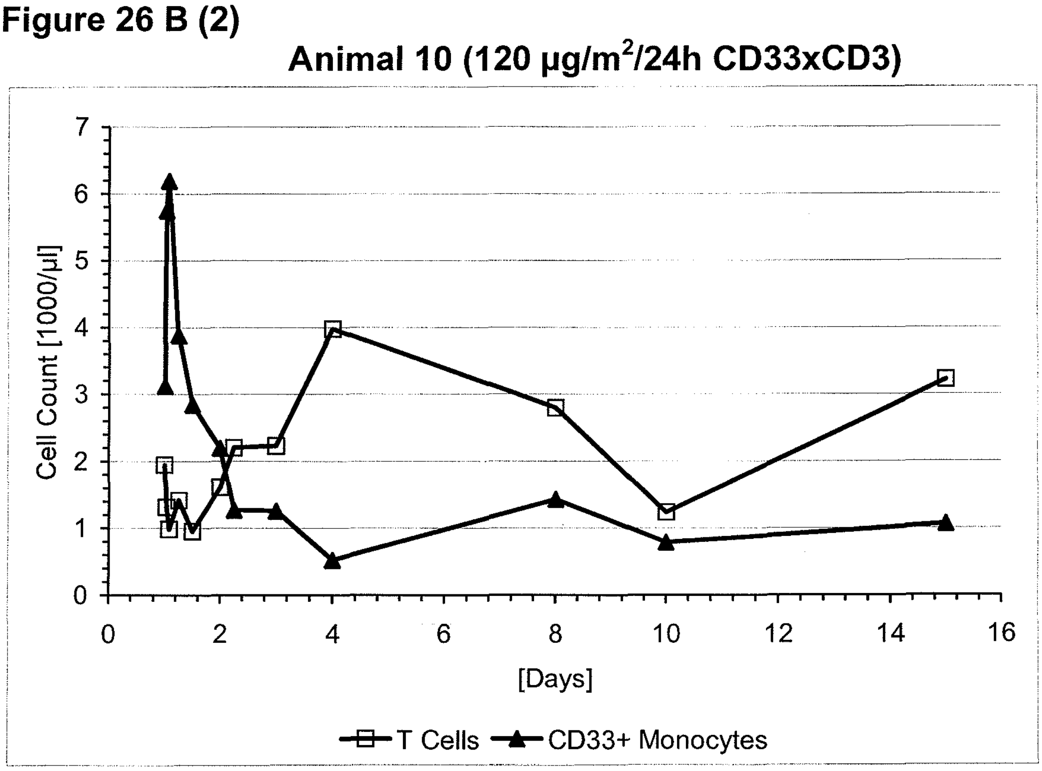

Figure 27

**Cytotoxic activity of cynomolgus test material MCSP-G4 VH-VL x I2C VH-VL**

Target cells: cyno MCSP CHO; effector cells: 4119 LnPx
E:T-ratio 10:1; assay duration: 18 h
EC50: 1.9 ng/ml

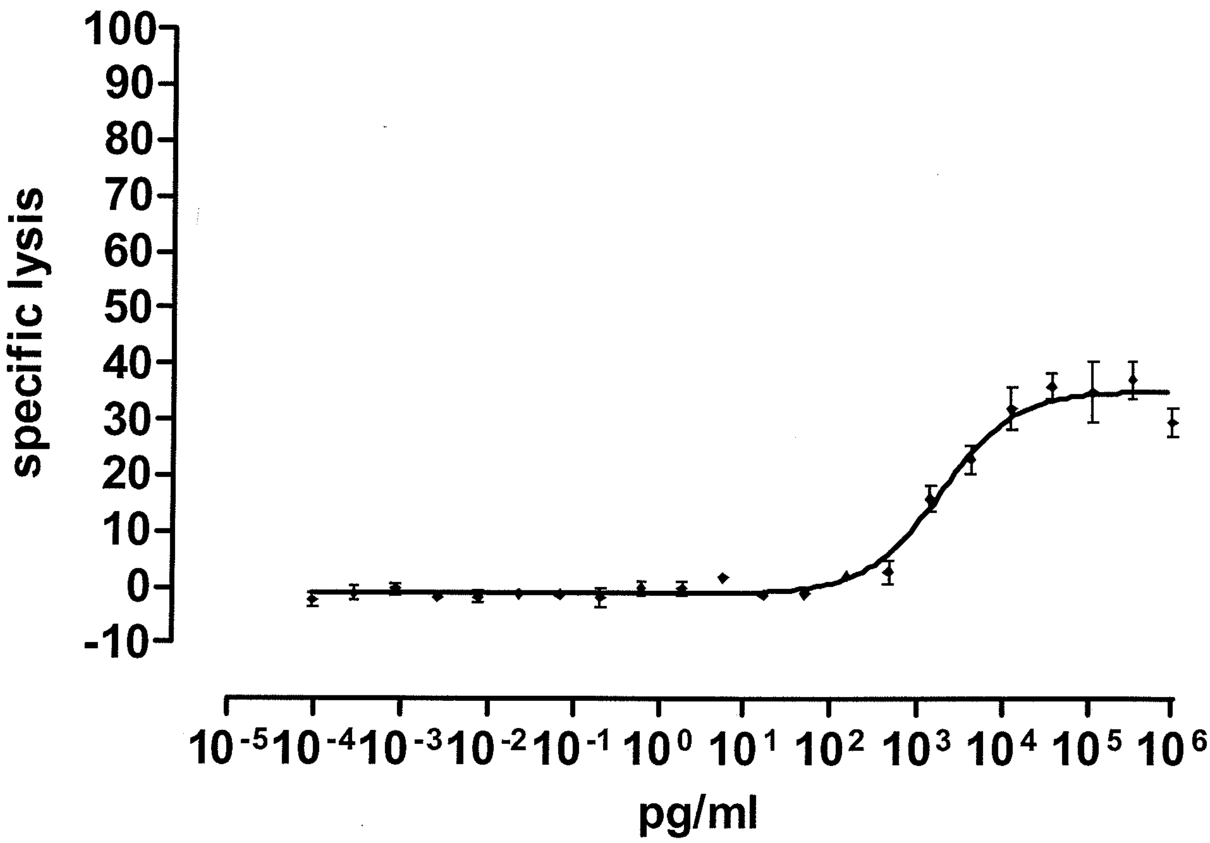

**Figure 28 (1)**

**Animal 1 (60 µg/m²/24h MCSP-G4 VH-VL x I2C VH-VL )**

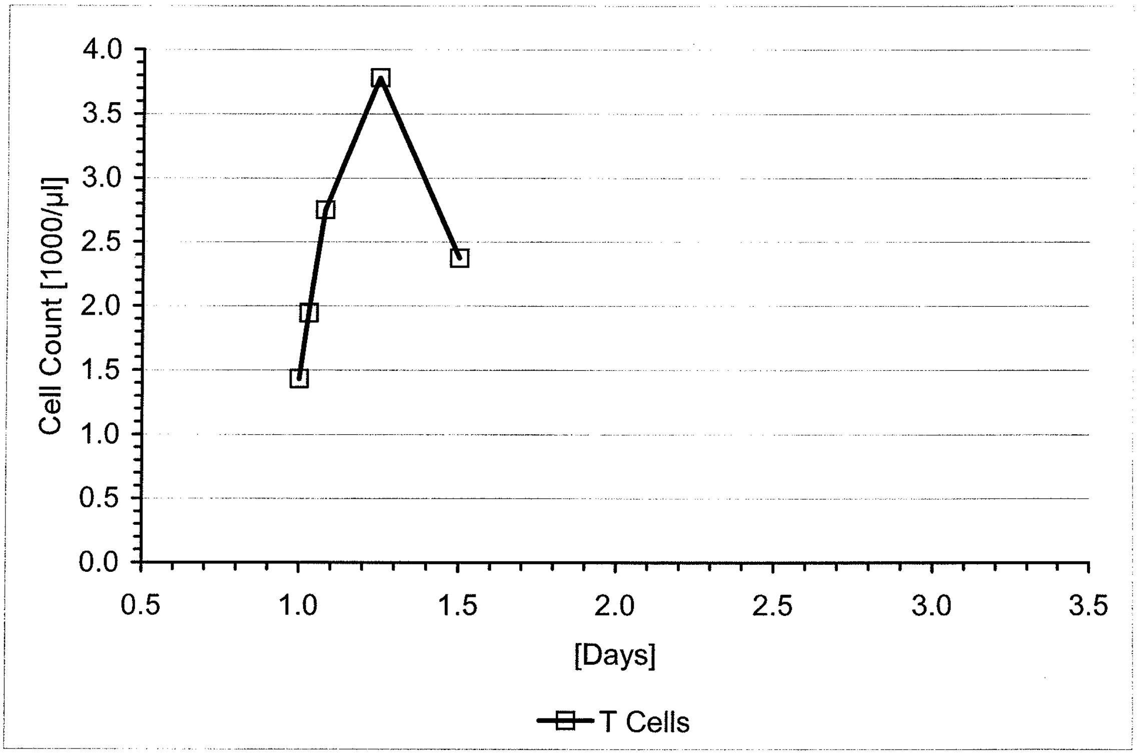


**Animal 2 (240 µg/m²/24h MCSP-G4 VH-VL x I2C VH-VL)**

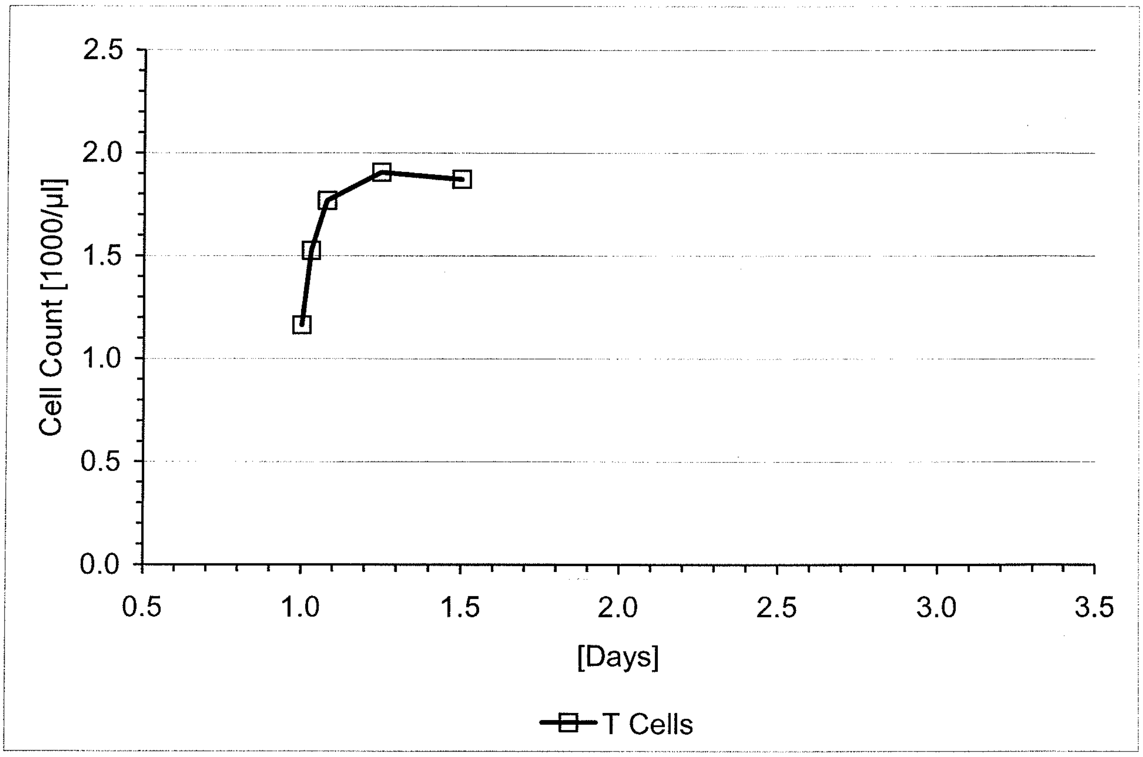

**Figure 28 (2)**

**Animal 3 (240 µg/m²/24h MCSP-G4 VH-VL x I2C VH-VL)**

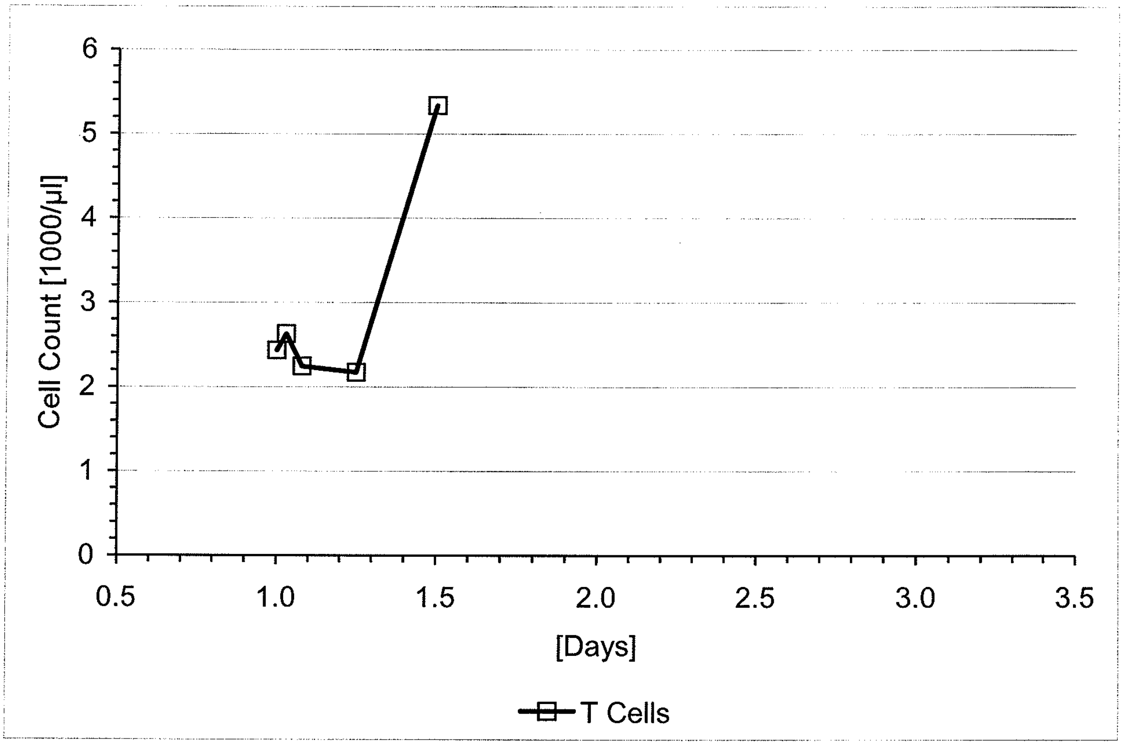


**Animal 4 (1000 µg/m²/24h MCSP-G4 VH-VL x I2C VH-VL)**

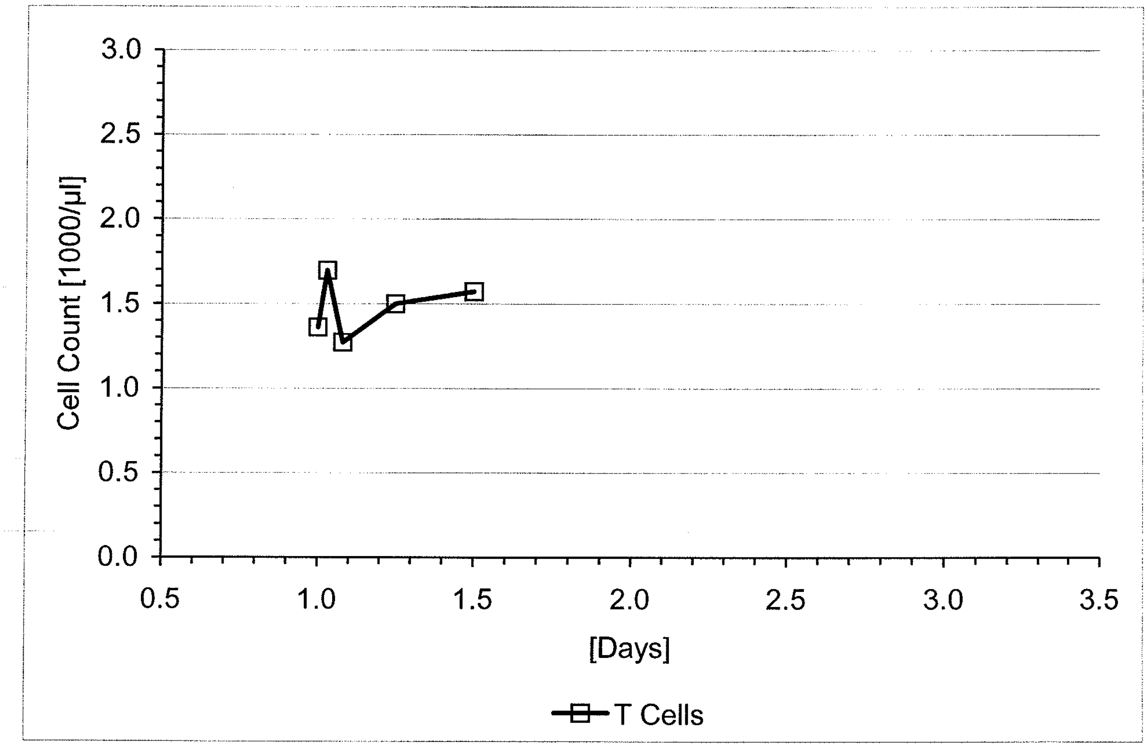

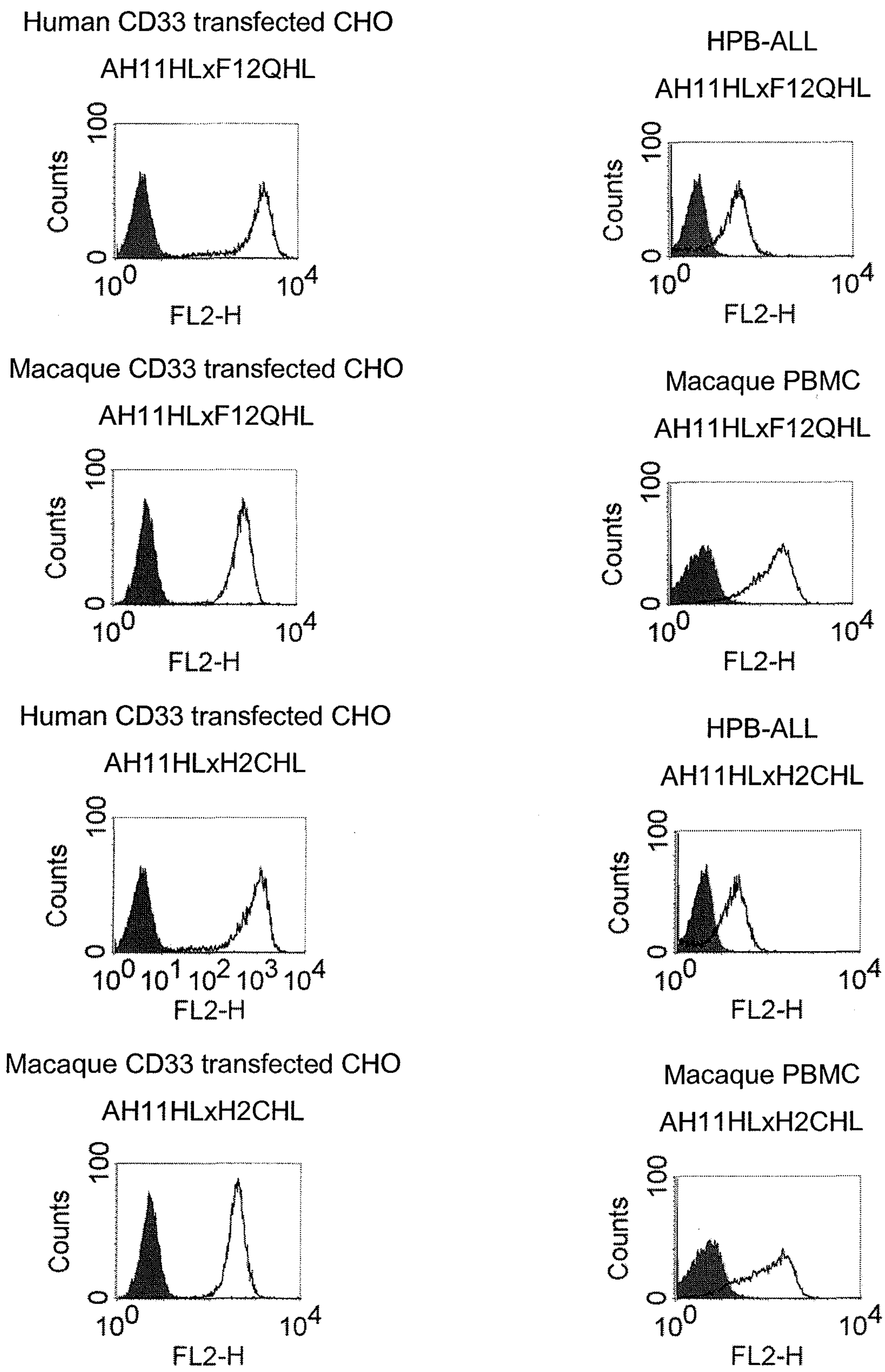
Human CD33 transfected CHO
AH11HLxF12QHL
HPB-ALL
AH11HLxF12QHL
Macaque CD33 transfected CHO
AH11HLxF12QHL
Macaque PBMC
AH11HLxF12QHL
Human CD33 transfected CHO
AH11HLxH2CHL
HPB-ALL
AH11HLxH2CHL
Macaque CD33 transfected CHO
AH11HLxH2CHL
Macaque PBMC
AH11HLxH2CHL
Counts
100
0
$10^0$
$10^1$
$10^2$
$10^3$
$10^4$
FL2-H

**Figure 29(1)**

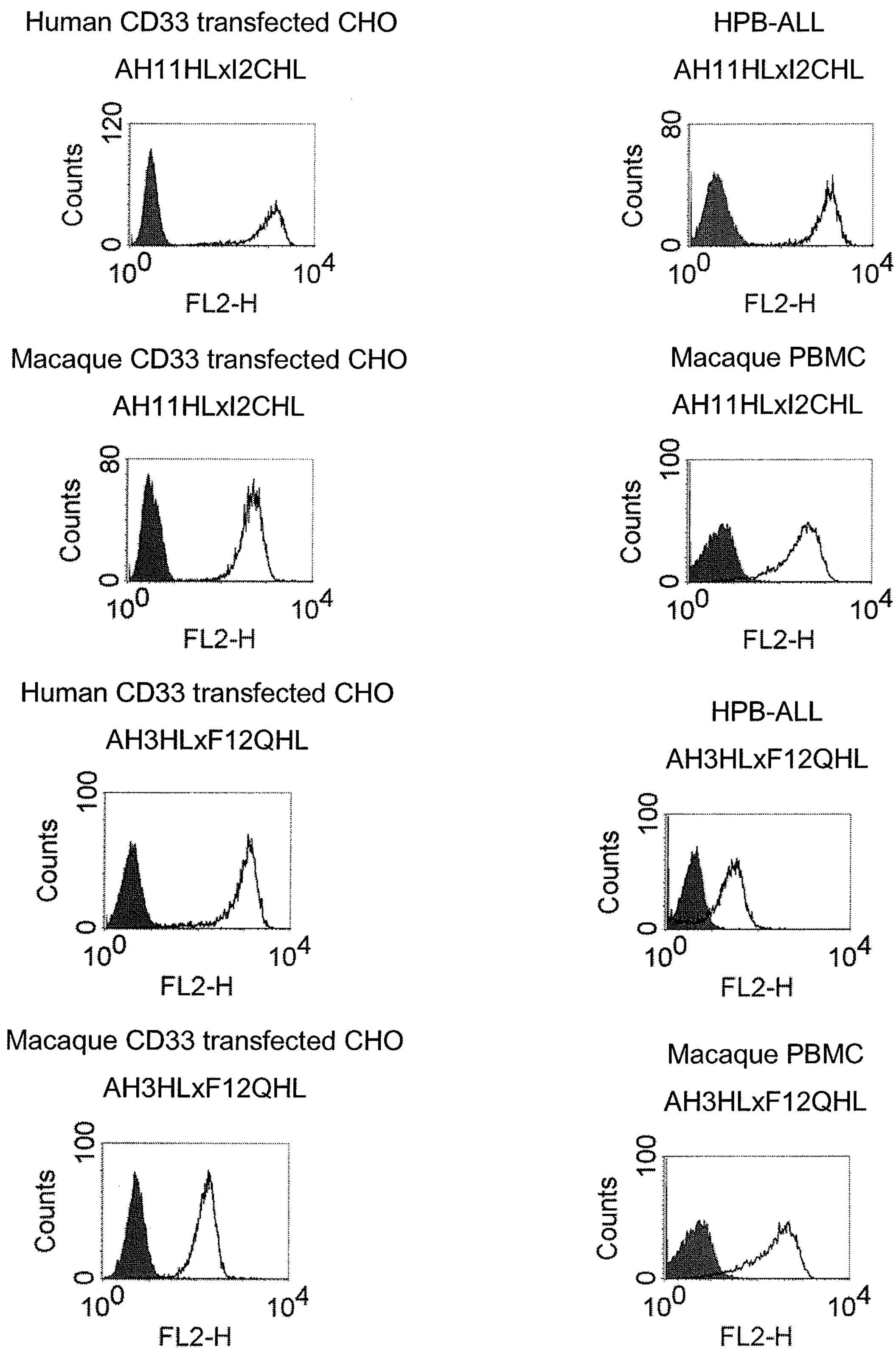
Human CD33 transfected CHO
AH11HLxI2CHL
Counts
0
120
10^0
10^4
FL2-H
HPB-ALL
AH11HLxI2CHL
Counts
0
80
10^0
10^4
FL2-H
Macaque CD33 transfected CHO
AH11HLxI2CHL
Counts
0
80
10^0
10^4
FL2-H
Macaque PBMC
AH11HLxI2CHL
Counts
0
100
10^0
10^4
FL2-H
Human CD33 transfected CHO
AH3HLxF12QHL
Counts
0
100
10^0
10^4
FL2-H
HPB-ALL
AH3HLxF12QHL
Counts
0
100
10^0
10^4
FL2-H
Macaque CD33 transfected CHO
AH3HLxF12QHL
Counts
0
100
10^0
10^4
FL2-H
Macaque PBMC
AH3HLxF12QHL
Counts
0
100
10^0
10^4
FL2-H

**Figure 29(2)**

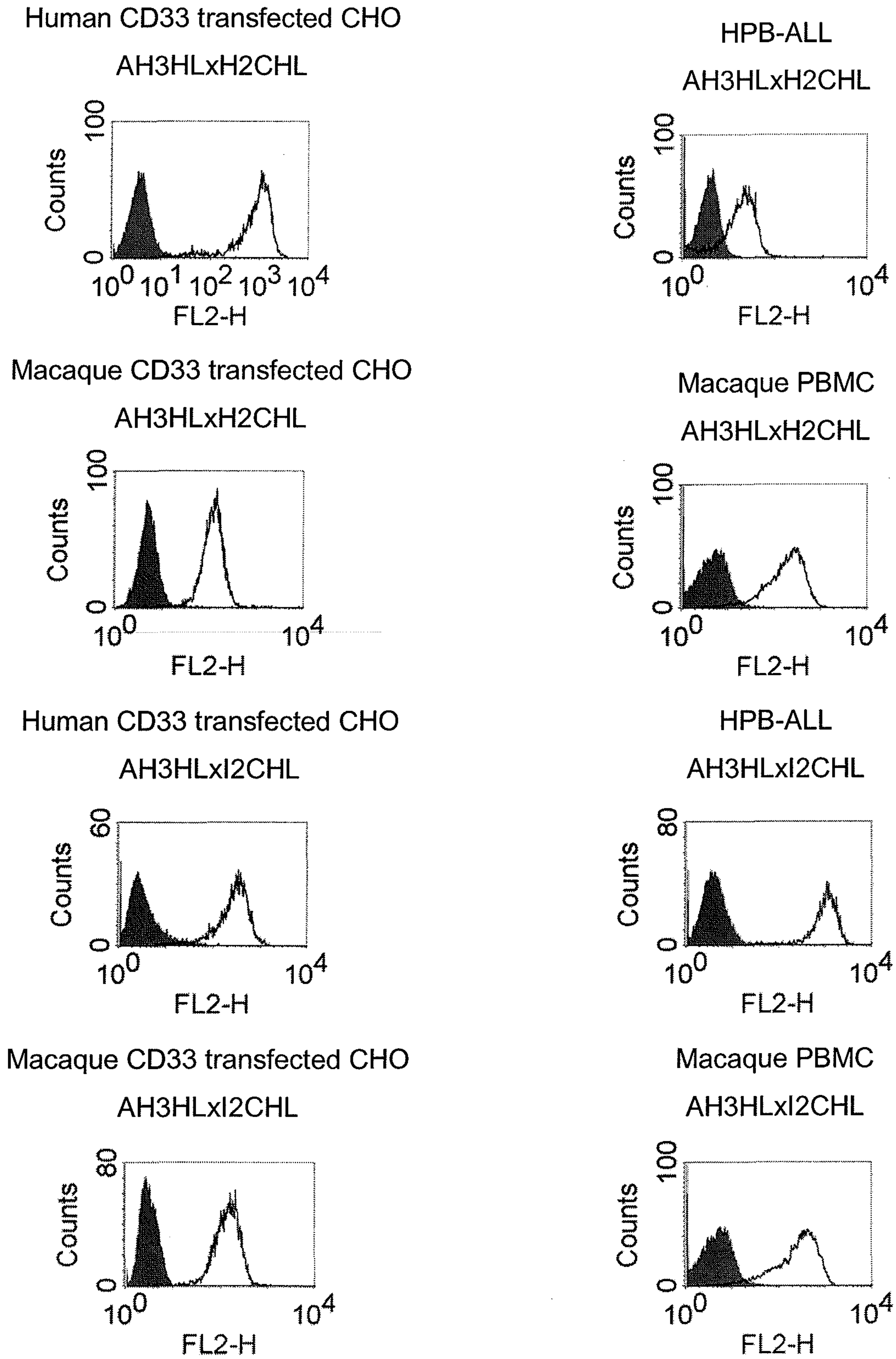
Human CD33 transfected CHO
AH3HLxH2CHL
HPB-ALL
AH3HLxH2CHL
Macaque CD33 transfected CHO
AH3HLxH2CHL
Macaque PBMC
AH3HLxH2CHL
Human CD33 transfected CHO
AH3HLxI2CHL
HPB-ALL
AH3HLxI2CHL
Macaque CD33 transfected CHO
AH3HLxI2CHL
Macaque PBMC
AH3HLxI2CHL
Counts
FL2-H

**Figure 29(3)**

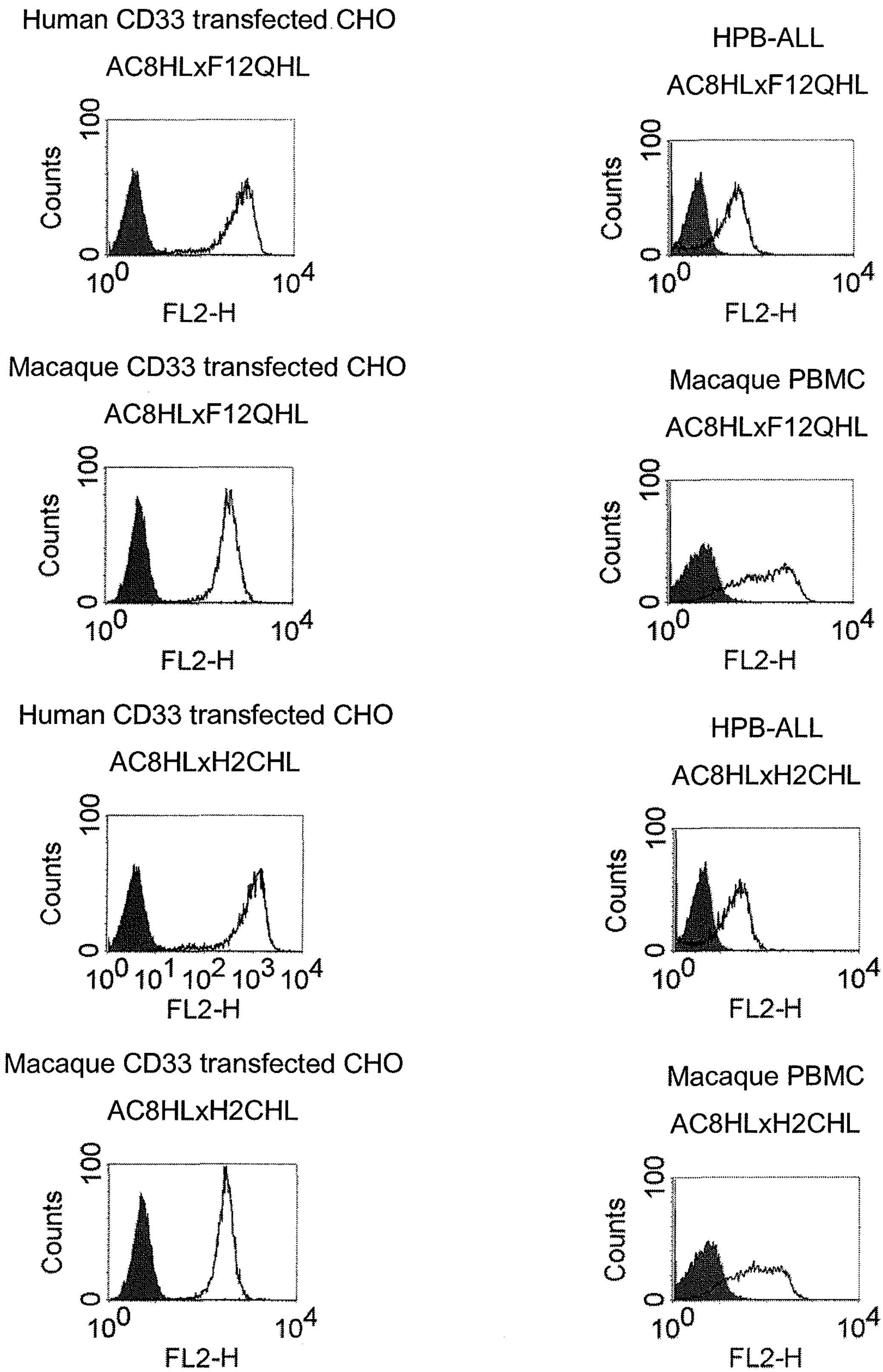
Human CD33 transfected CHO
AC8HLxF12QHL
HPB-ALL
AC8HLxF12QHL
Macaque CD33 transfected CHO
AC8HLxF12QHL
Macaque PBMC
AC8HLxF12QHL
Human CD33 transfected CHO
AC8HLxH2CHL
HPB-ALL
AC8HLxH2CHL
Macaque CD33 transfected CHO
AC8HLxH2CHL
Macaque PBMC
AC8HLxH2CHL
Counts
100
0
$10^0$
$10^1$
$10^2$
$10^3$
$10^4$
FL2-H

**Figure 29(4)**

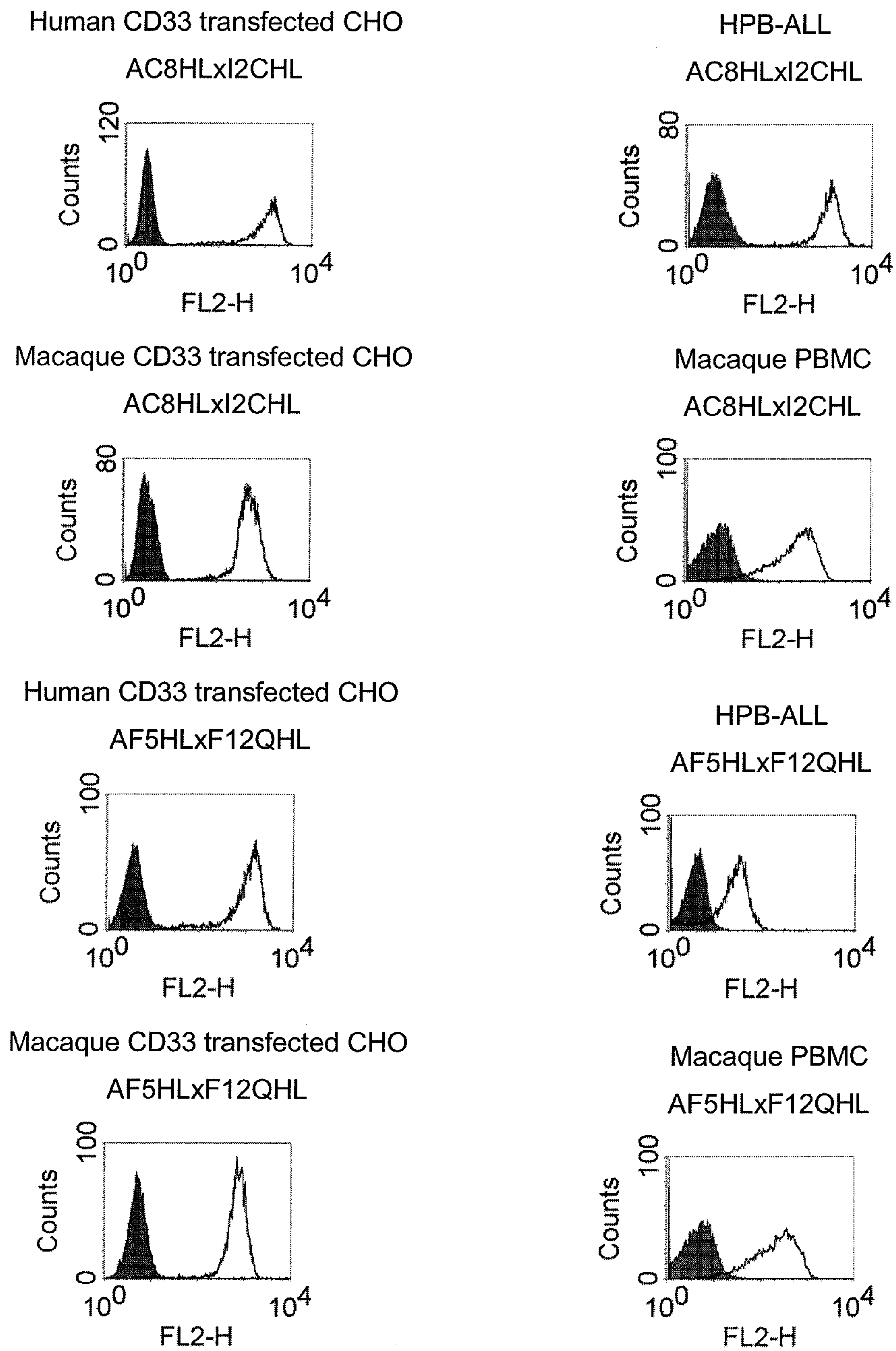
Human CD33 transfected CHO
AC8HLxI2CHL
HPB-ALL
AC8HLxI2CHL
Macaque CD33 transfected CHO
AC8HLxI2CHL
Macaque PBMC
AC8HLxI2CHL
Human CD33 transfected CHO
AF5HLxF12QHL
HPB-ALL
AF5HLxF12QHL
Macaque CD33 transfected CHO
AF5HLxF12QHL
Macaque PBMC
AF5HLxF12QHL
Counts
FL2-H
$10^0$
$10^4$
0
120
80
100

**Figure 29(5)**

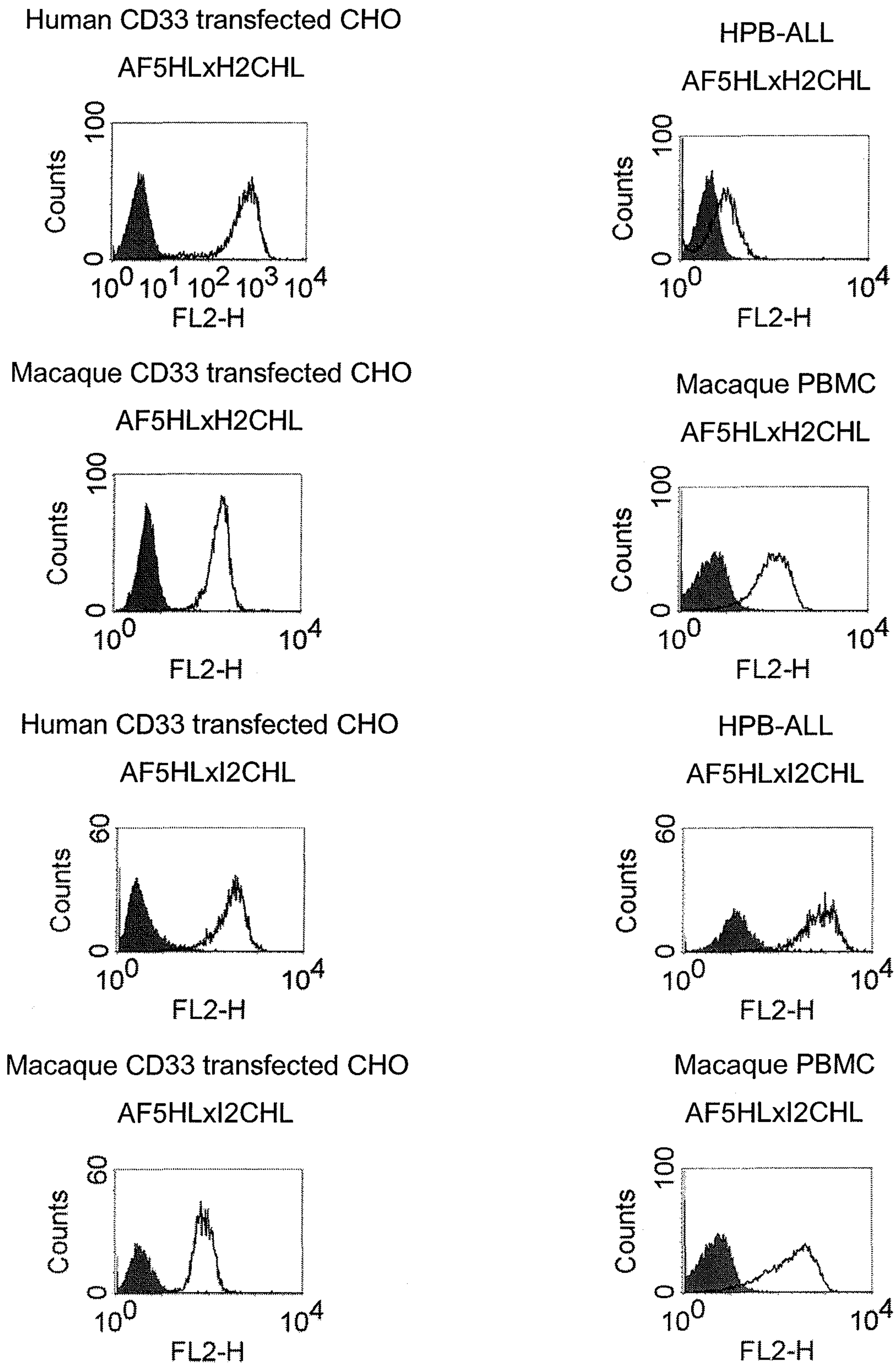
Human CD33 transfected CHO
AF5HLxH2CHL
Counts
0
100
$10^0$ $10^1$ $10^2$ $10^3$ $10^4$
FL2-H
HPB-ALL
AF5HLxH2CHL
Counts
0
100
$10^0$
$10^4$
FL2-H
Macaque CD33 transfected CHO
AF5HLxH2CHL
Counts
0
100
$10^0$
$10^4$
FL2-H
Macaque PBMC
AF5HLxH2CHL
Counts
0
100
$10^0$
$10^4$
FL2-H
Human CD33 transfected CHO
AF5HLxI2CHL
Counts
0
60
$10^0$
$10^4$
FL2-H
HPB-ALL
AF5HLxI2CHL
Counts
0
60
$10^0$
$10^4$
FL2-H
Macaque CD33 transfected CHO
AF5HLxI2CHL
Counts
0
60
$10^0$
$10^4$
FL2-H
Macaque PBMC
AF5HLxI2CHL
Counts
0
100
$10^0$
$10^4$
FL2-H

**Figure 29(6)**

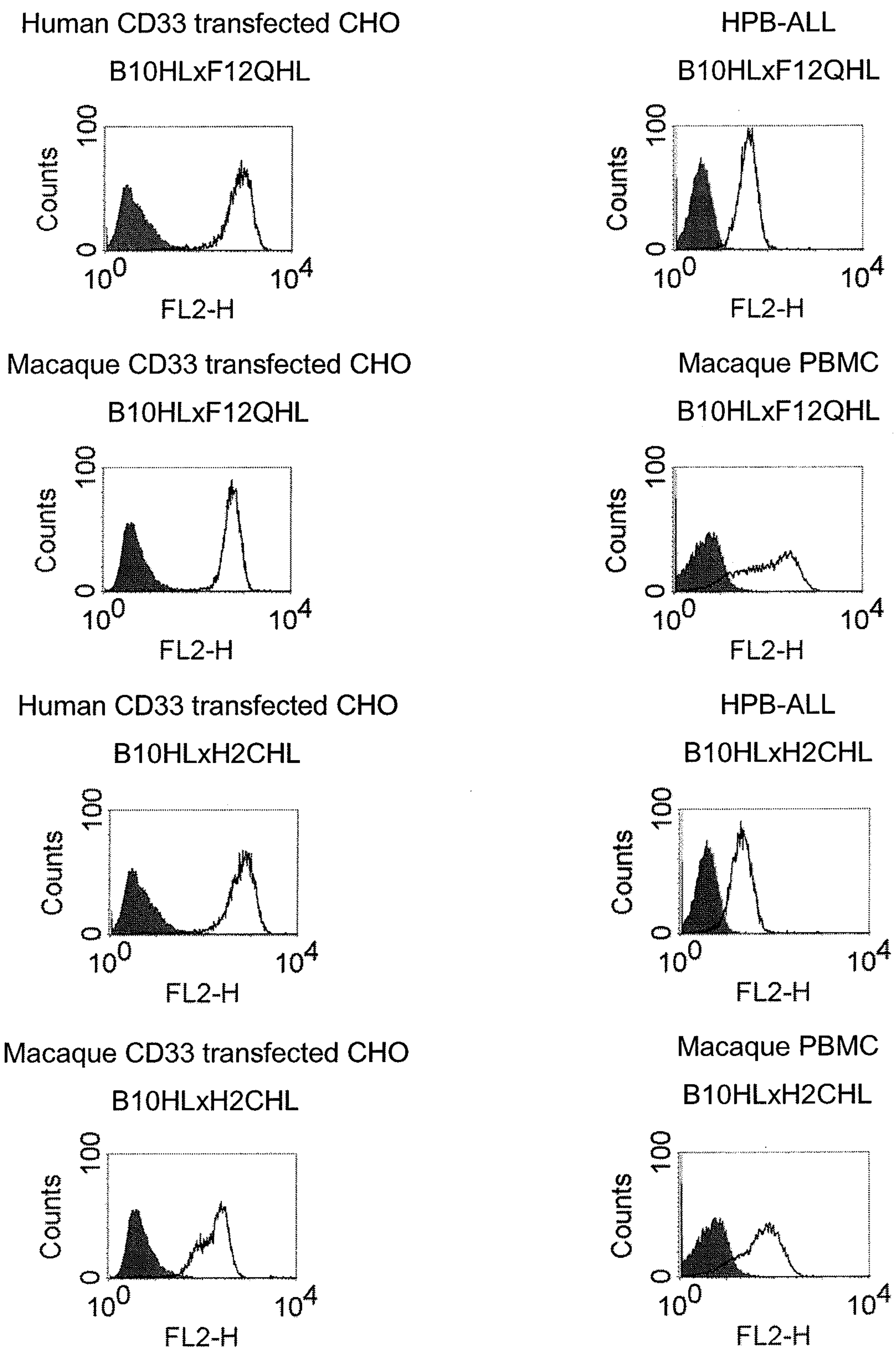
Human CD33 transfected CHO
B10HLxF12QHL
HPB-ALL
B10HLxF12QHL
Macaque CD33 transfected CHO
B10HLxF12QHL
Macaque PBMC
B10HLxF12QHL
Human CD33 transfected CHO
B10HLxH2CHL
HPB-ALL
B10HLxH2CHL
Macaque CD33 transfected CHO
B10HLxH2CHL
Macaque PBMC
B10HLxH2CHL
Counts
0
100
$10^0$
$10^4$
FL2-H

**Figure 29(7)**

Human CD33 transfected CHO

B10HLxI2CHL

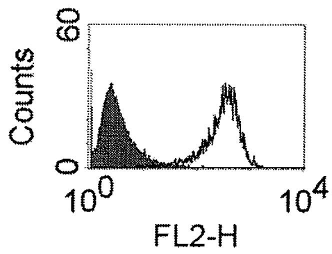


HPB-ALL

B10HLxI2CHL

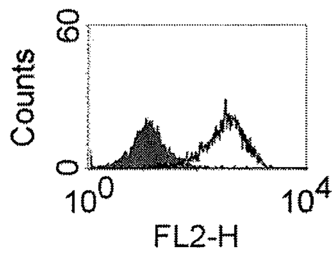


Macaque CD33 transfected CHO

B10HLxI2CHL

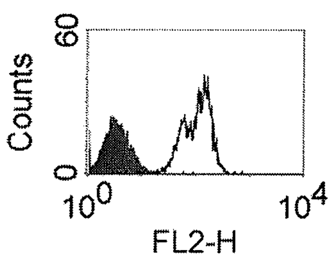


Macaque PBMC

B10HLxI2CHL

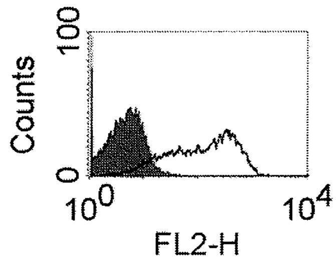


Human CD33 transfected CHO

B3HLxF12QHL

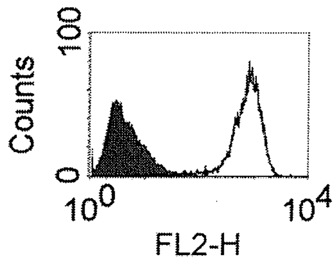


HPB-ALL

B3HLxF12QHL

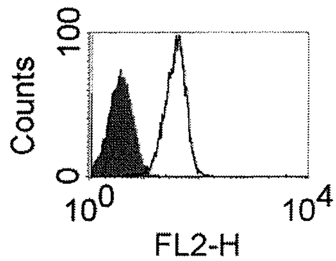


Macaque CD33 transfected CHO

B3HLxF12QHL

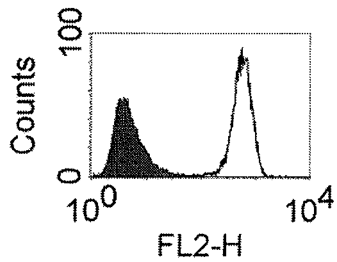


Macaque PBMC

B3HLxF12QHL

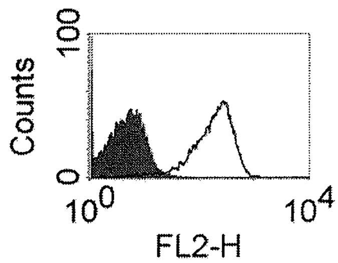


**Figure 29(8)**

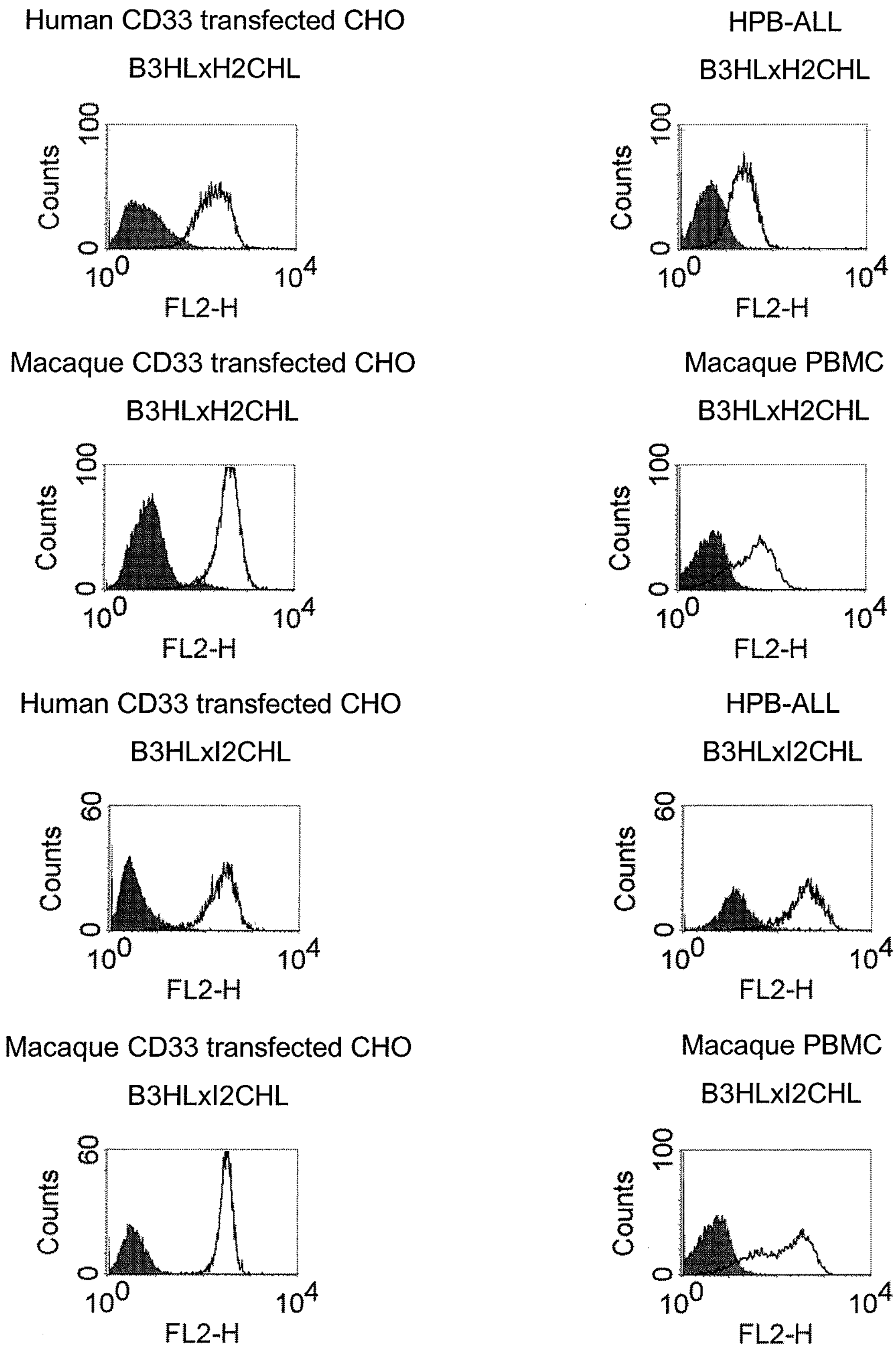
Human CD33 transfected CHO
B3HLxH2CHL
HPB-ALL
B3HLxH2CHL
Macaque CD33 transfected CHO
B3HLxH2CHL
Macaque PBMC
B3HLxH2CHL
Human CD33 transfected CHO
B3HLxI2CHL
HPB-ALL
B3HLxI2CHL
Macaque CD33 transfected CHO
B3HLxI2CHL
Macaque PBMC
B3HLxI2CHL
Counts
FL2-H

Figure 29(9)

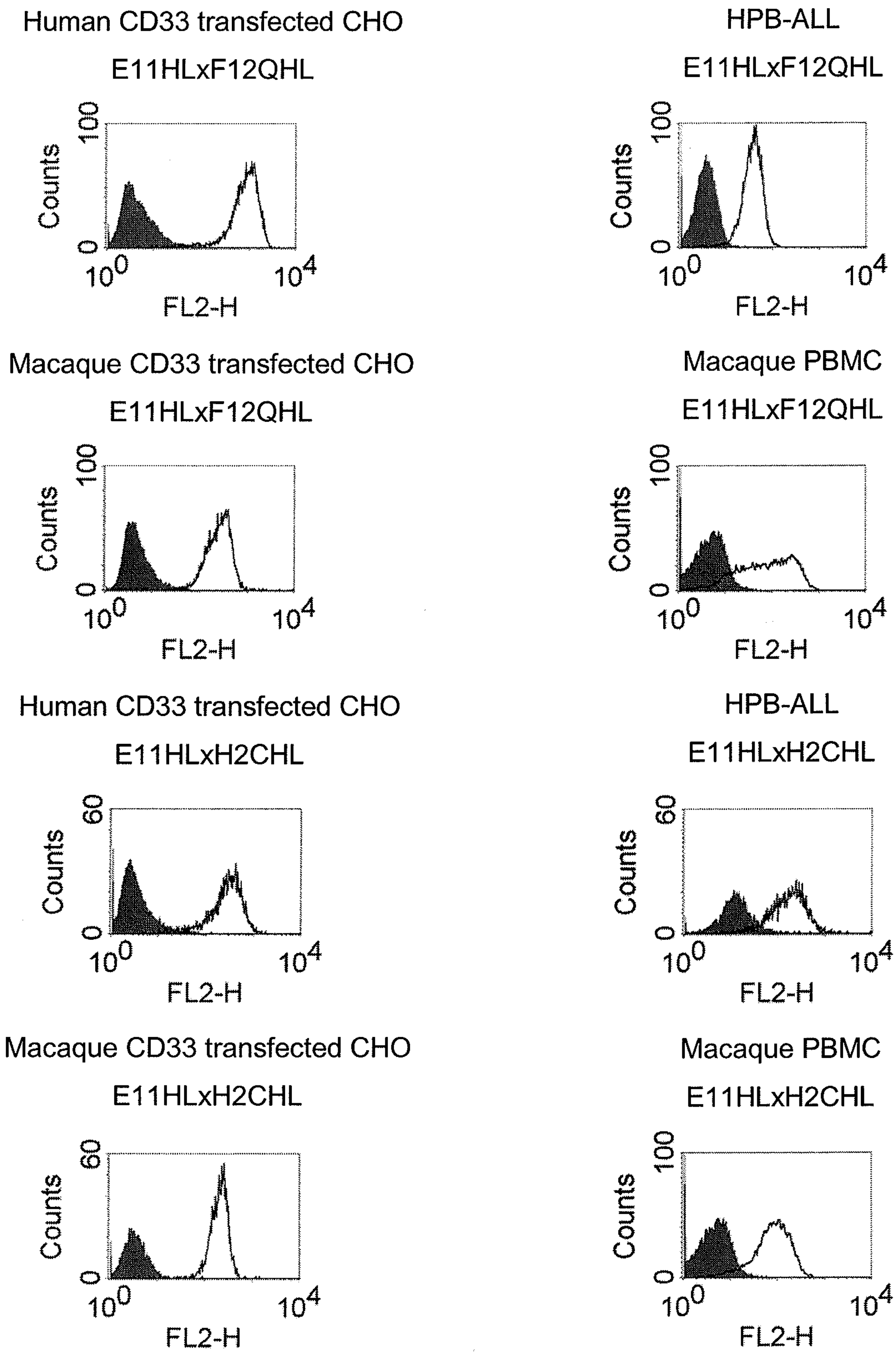
Human CD33 transfected CHO
E11HLxF12QHL
HPB-ALL
E11HLxF12QHL
Macaque CD33 transfected CHO
E11HLxF12QHL
Macaque PBMC
E11HLxF12QHL
Human CD33 transfected CHO
E11HLxH2CHL
HPB-ALL
E11HLxH2CHL
Macaque CD33 transfected CHO
E11HLxH2CHL
Macaque PBMC
E11HLxH2CHL
Counts
FL2-H
0
100
60
$10^0$
$10^4$

**Figure 29(10)**

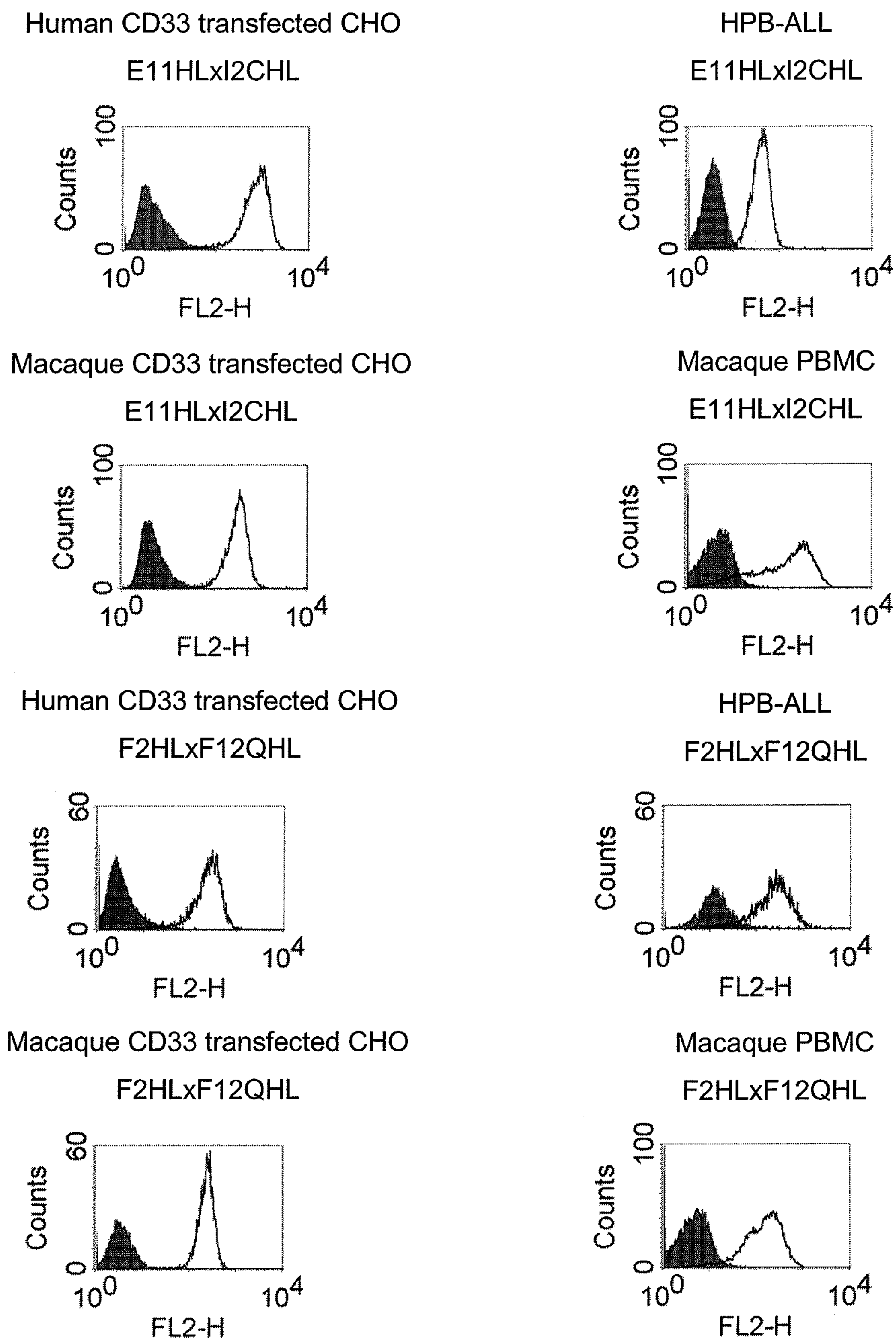
Human CD33 transfected CHO
E11HLxI2CHL
HPB-ALL
E11HLxI2CHL
Macaque CD33 transfected CHO
E11HLxI2CHL
Macaque PBMC
E11HLxI2CHL
Human CD33 transfected CHO
F2HLxF12QHL
HPB-ALL
F2HLxF12QHL
Macaque CD33 transfected CHO
F2HLxF12QHL
Macaque PBMC
F2HLxF12QHL
Counts
FL2-H
0
100
60
$10^0$
$10^4$

**Figure 29(11)**

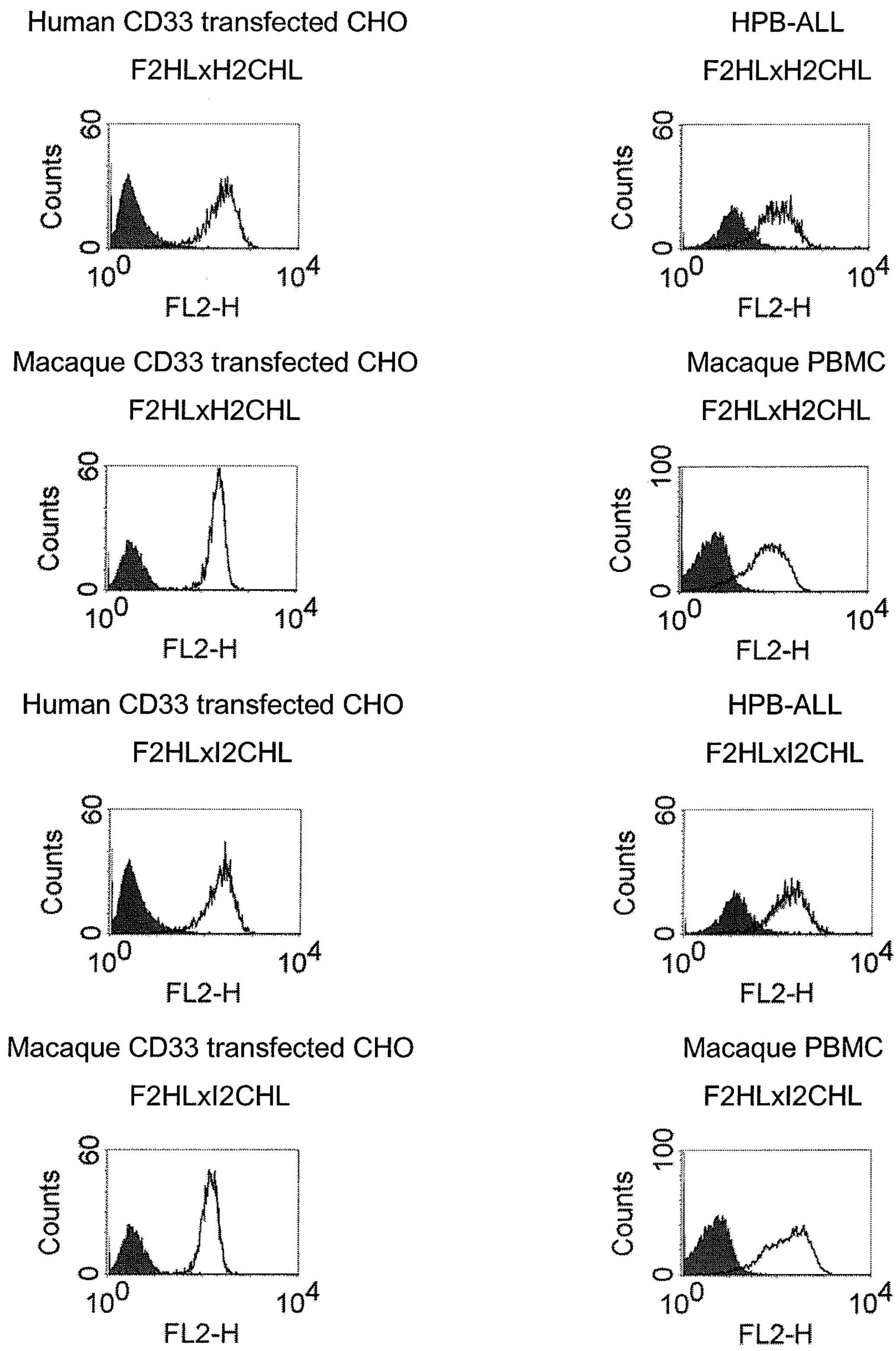

Human CD33 transfected CHO
F2HLxH2CHL
HPB-ALL
F2HLxH2CHL
Macaque CD33 transfected CHO
F2HLxH2CHL
Macaque PBMC
F2HLxH2CHL
Human CD33 transfected CHO
F2HLxI2CHL
HPB-ALL
F2HLxI2CHL
Macaque CD33 transfected CHO
F2HLxI2CHL
Macaque PBMC
F2HLxI2CHL
Counts
FL2-H
0
60
100
$10^0$
$10^4$


**Figure 29(12)**

Effector cells: stimulated CD4/CD56 depleted human PBMC

Target cells: CHO transfected with human CD33

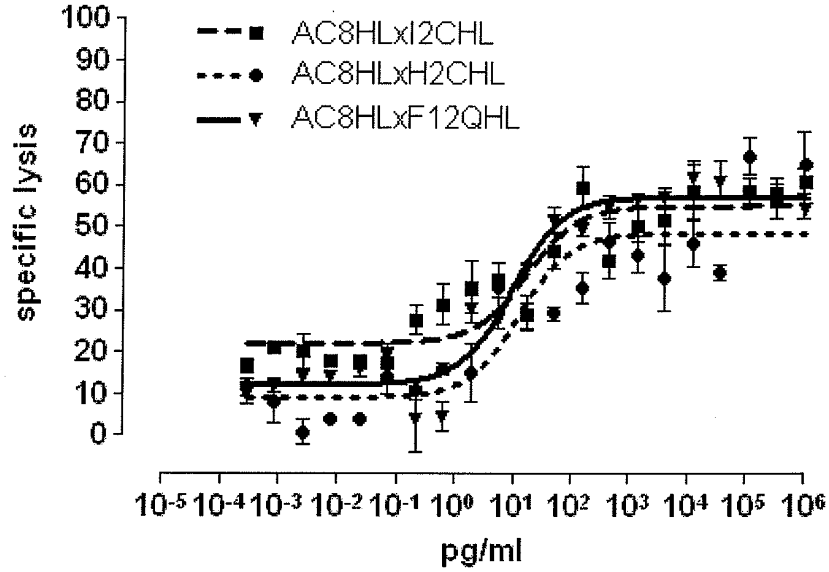


Effector cells: macaque 4119 LnPx

Target cells: CHO transfected with macaque CD33

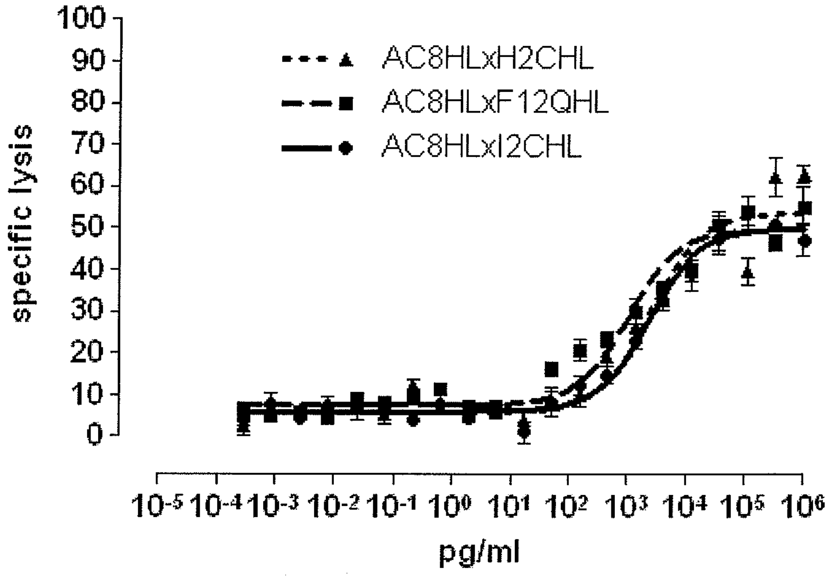


Figure 30(1)

Effector cells: stimulated CD4/CD56 depleted human PBMC

Target cells: CHO transfected with human CD33

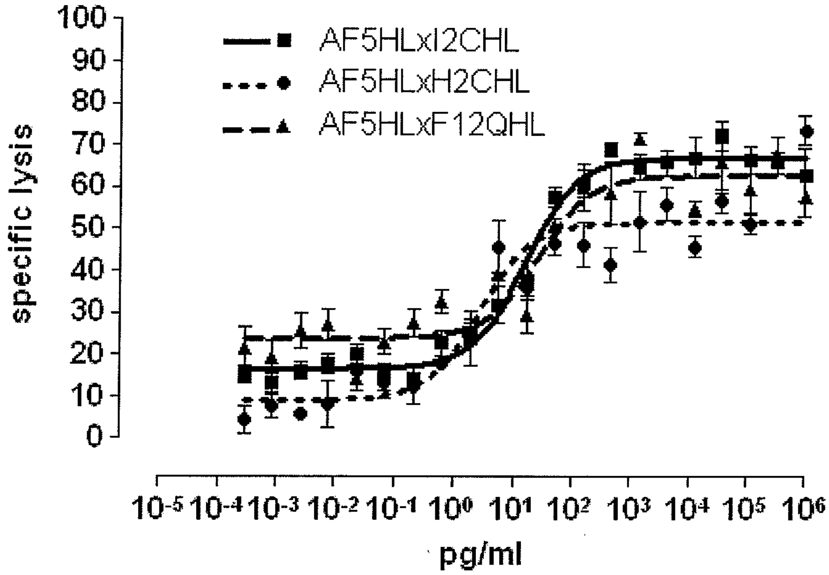


Effector cells: macaque 4119 LnPx

Target cells: CHO transfected with macaque CD33

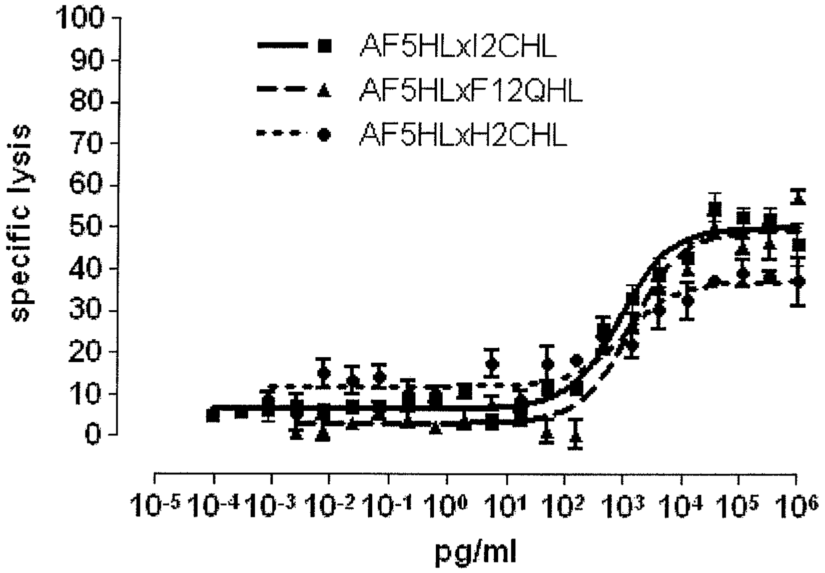


**Figure 30(2)**

Effector cells: stimulated CD4/CD56 depleted human PBMC

Target cells: CHO transfected with human CD33

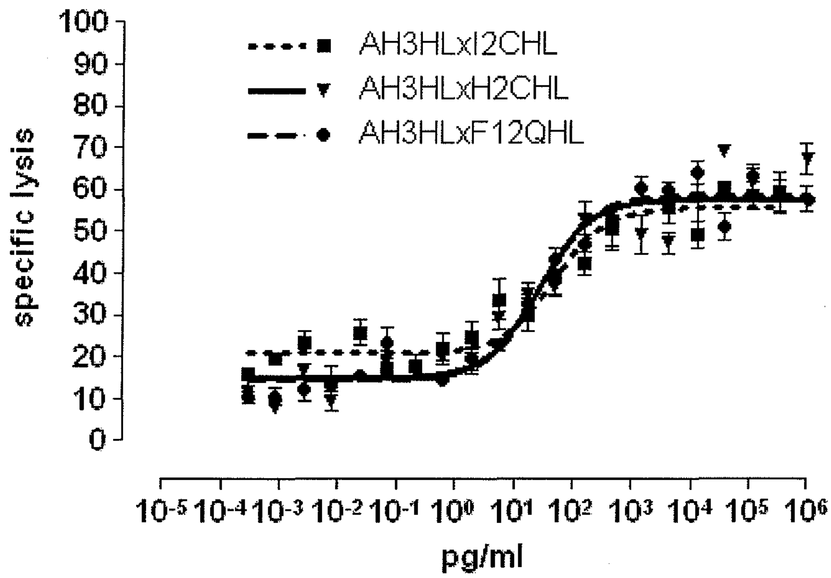


Effector cells: macaque 4119 LnPx

Target cells: CHO transfected with macaque CD33

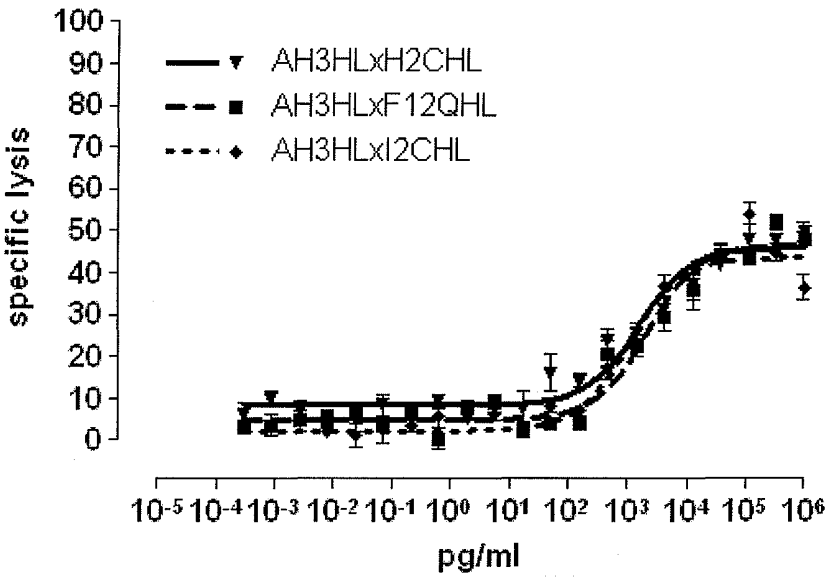


**Figure 30(3)**

Effector cells: stimulated CD4/CD56 depleted human PBMC

Target cells: CHO transfected with human CD33

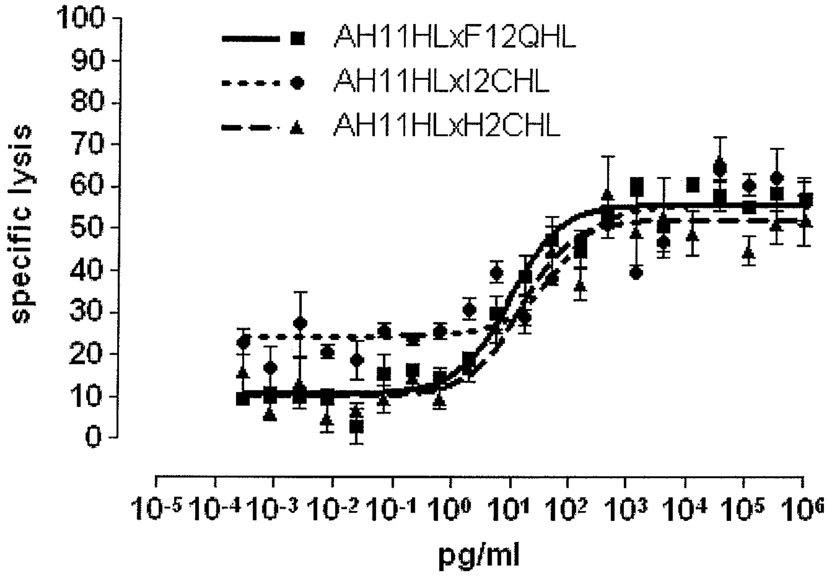


Effector cells: macaque 4119 LnPx

Target cells: CHO transfected with macaque CD33

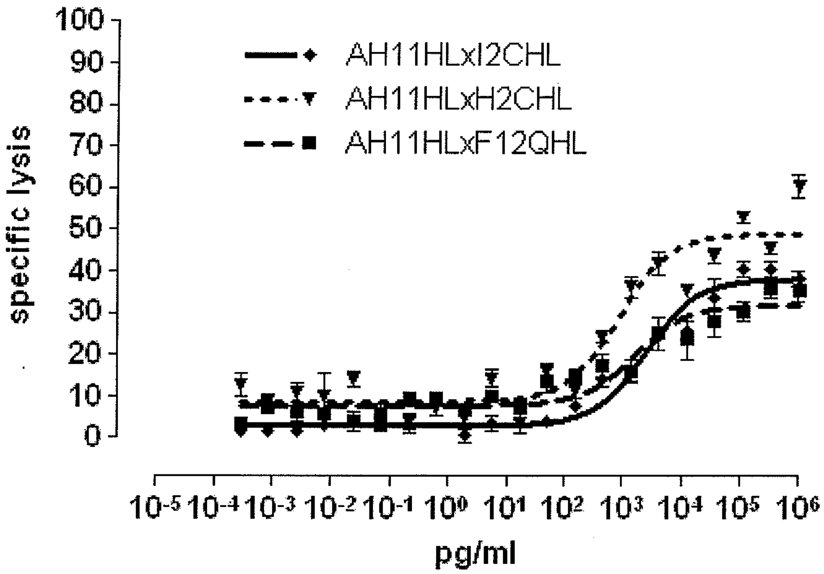


**Figure 30(4)**

Effector cells: stimulated CD4/CD56 depleted human PBMC

Target cells: CHO transfected with human CD33

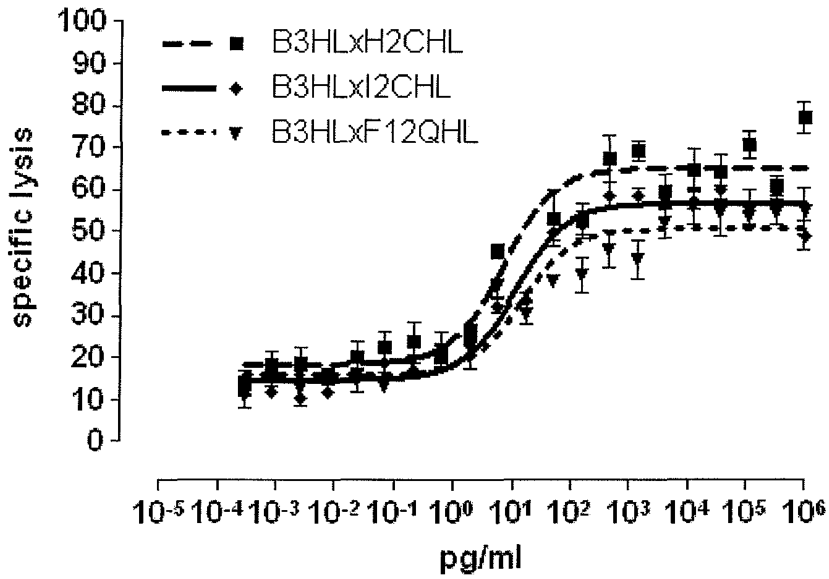


Effector cells: macaque 4119 LnPx

Target cells: CHO transfected with macaque CD33

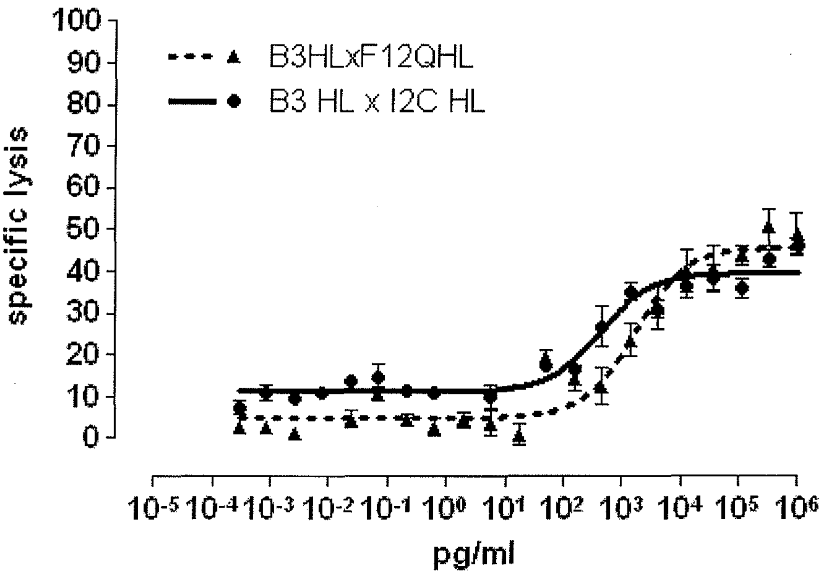


**Figure 30(5)**

Effector cells: stimulated CD4/CD56 depleted human PBMC

Target cells: CHO transfected with human CD33

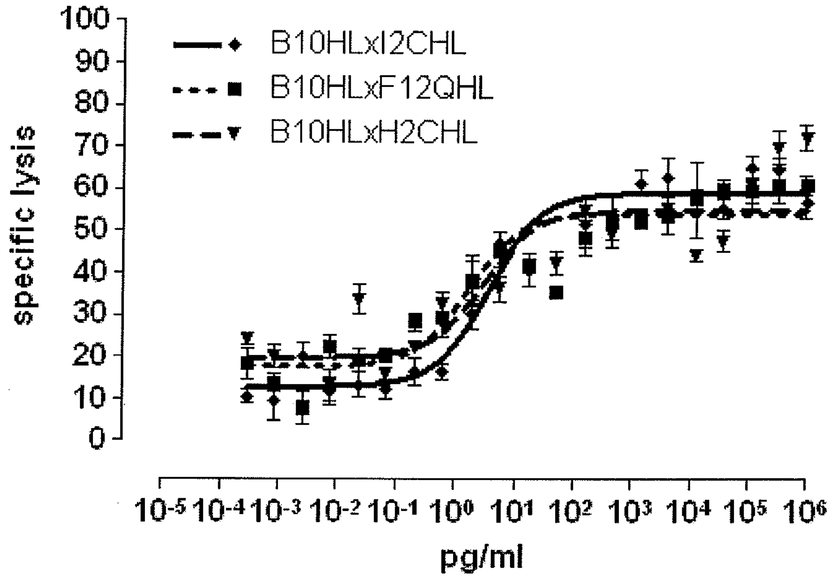


Effector cells: macaque 4119 LnPx

Target cells: CHO transfected with macaque CD33

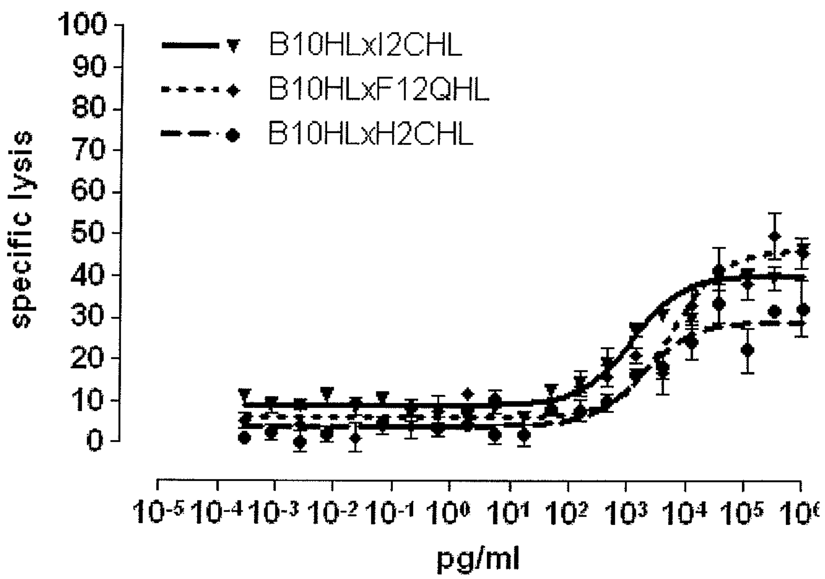


**Figure 30(6)**

Effector cells: stimulated CD4/CD56 depleted human PBMC

Target cells: CHO transfected with human CD33

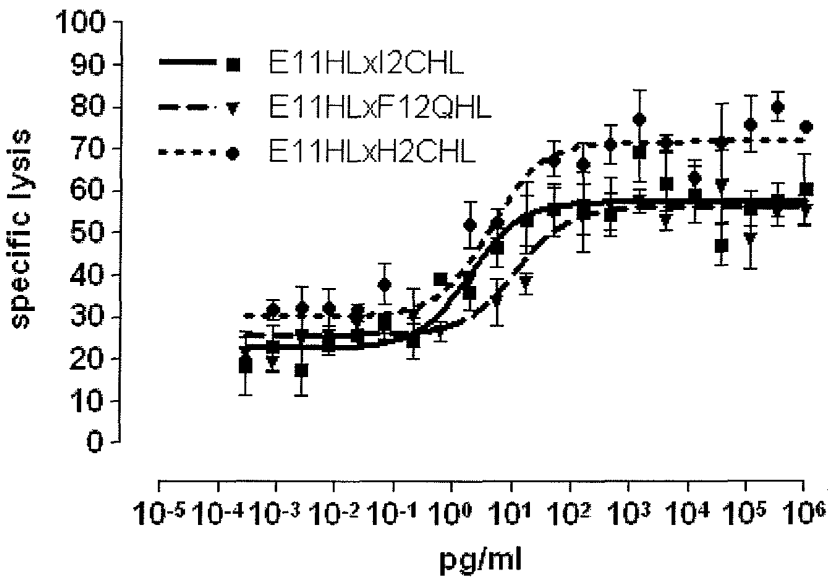


Effector cells: macaque 4119 LnPx

Target cells: CHO transfected with macaque CD33

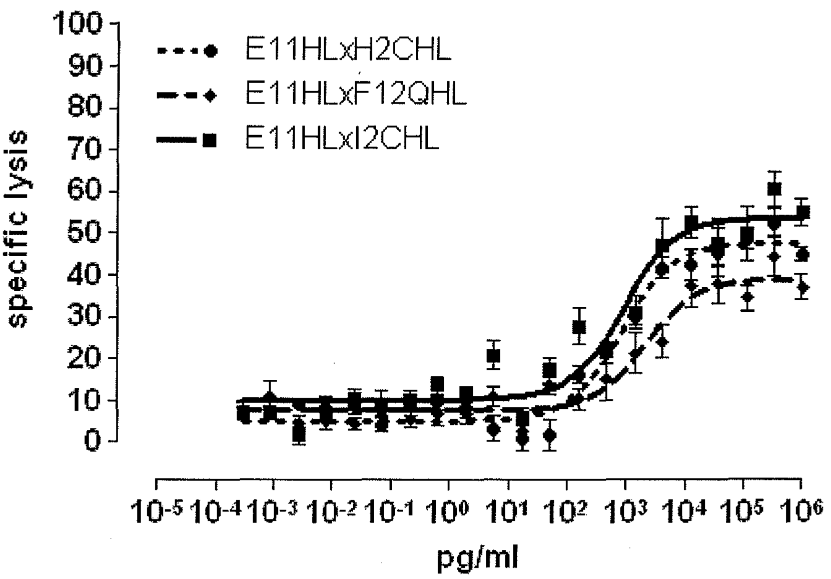


**Figure 30(7)**

Effector cells: stimulated CD4/CD56 depleted human PBMC

Target cells: CHO transfected with human CD33

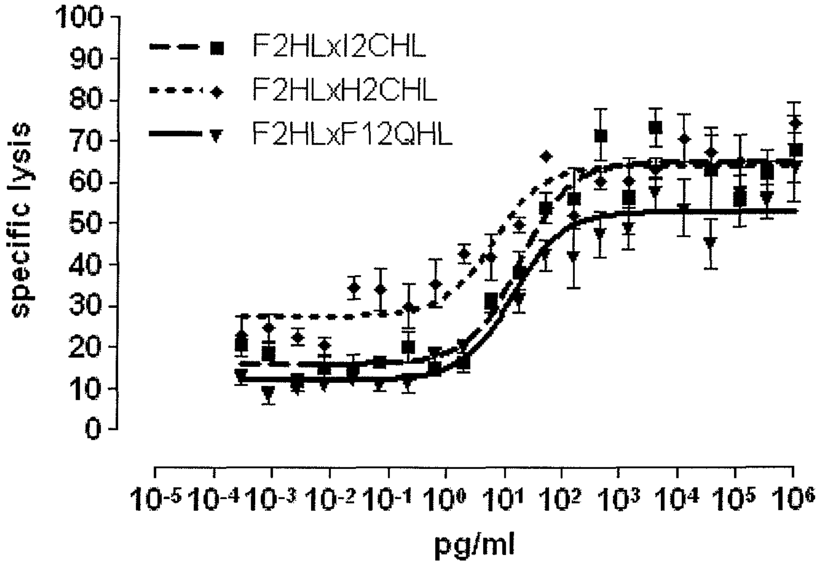


Effector cells: macaque 4119 LnPx

Target cells: CHO transfected with macaque CD33

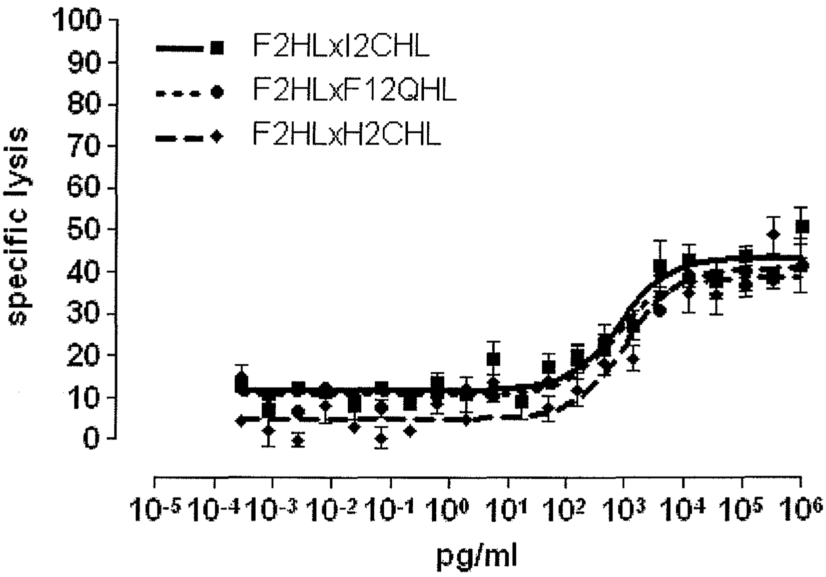


**Figure 30(8)**

# Figure 31

SDS PAGE of the purification of E292F3 HL x I2C HL

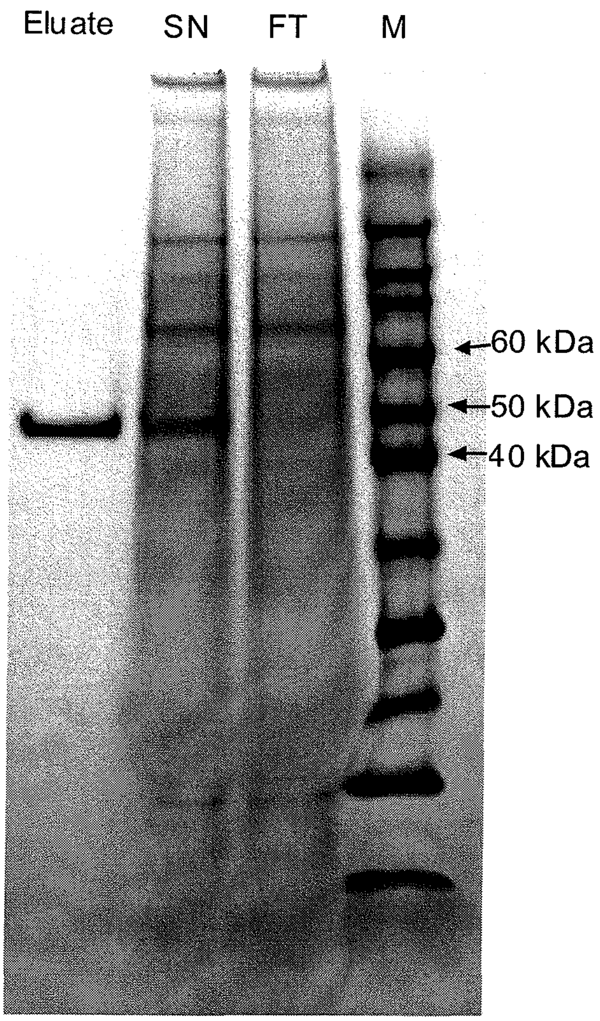


Corresponding Western blot of the purification of E292F3 HL x I2C HL

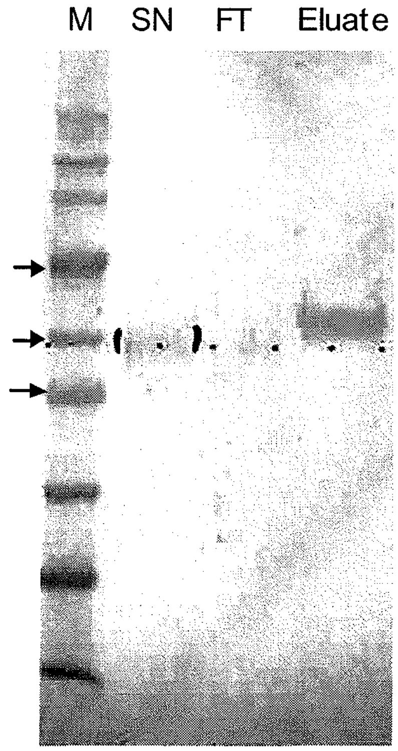

## Figure 32

SDS PAGE of the purification of V207C12 HL x H2C HL

Corresponding Western blot of the purification of V207C12 HL x H2C HL

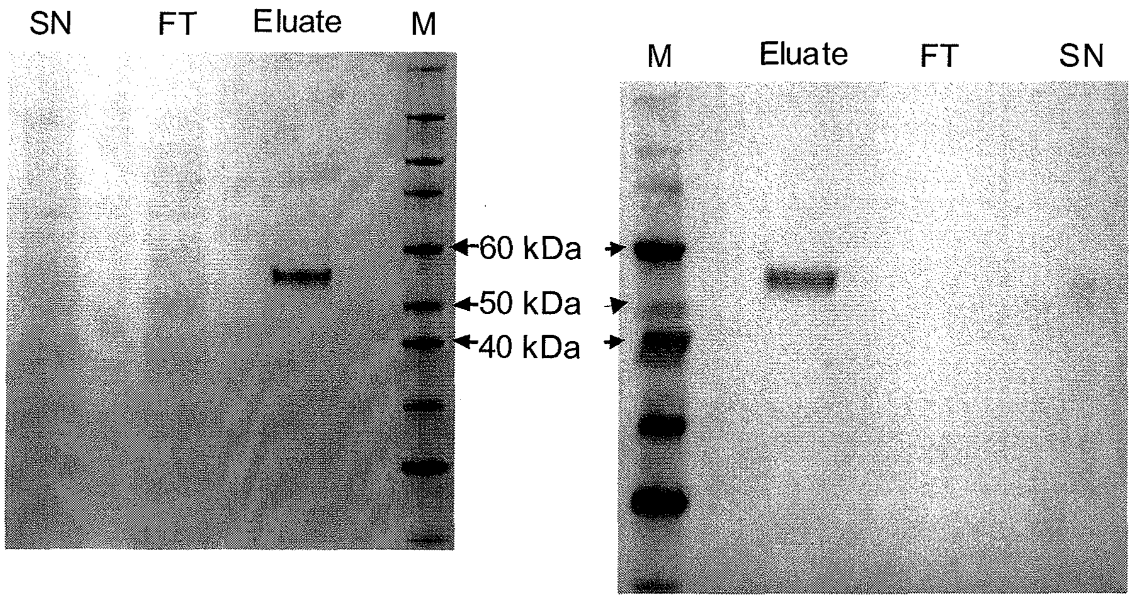


## Figure 33

SDS PAGE of the purification of AF5HLxF12QHL

Corresponding Western blot of the purification of AF5HLxF12QHL

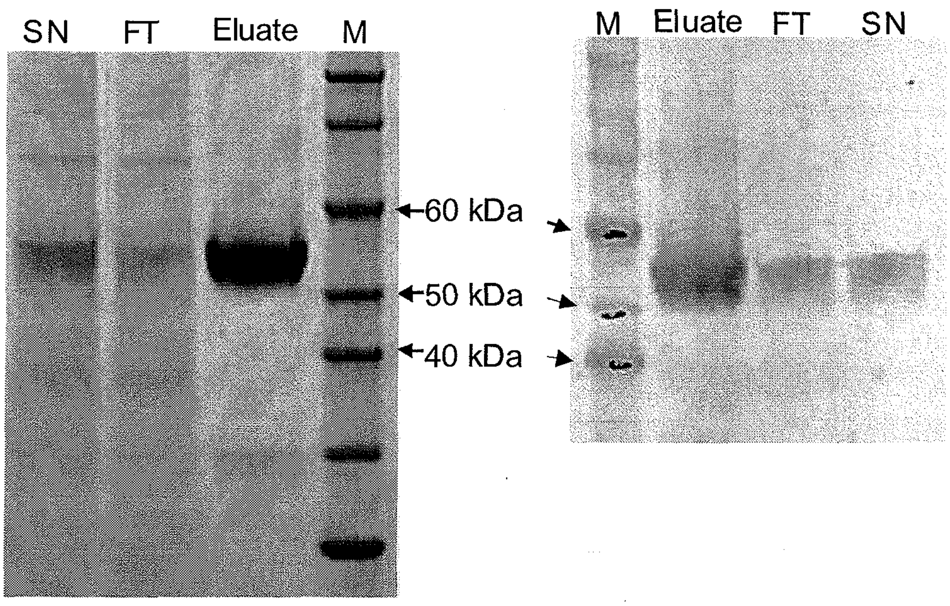

## Figure 34

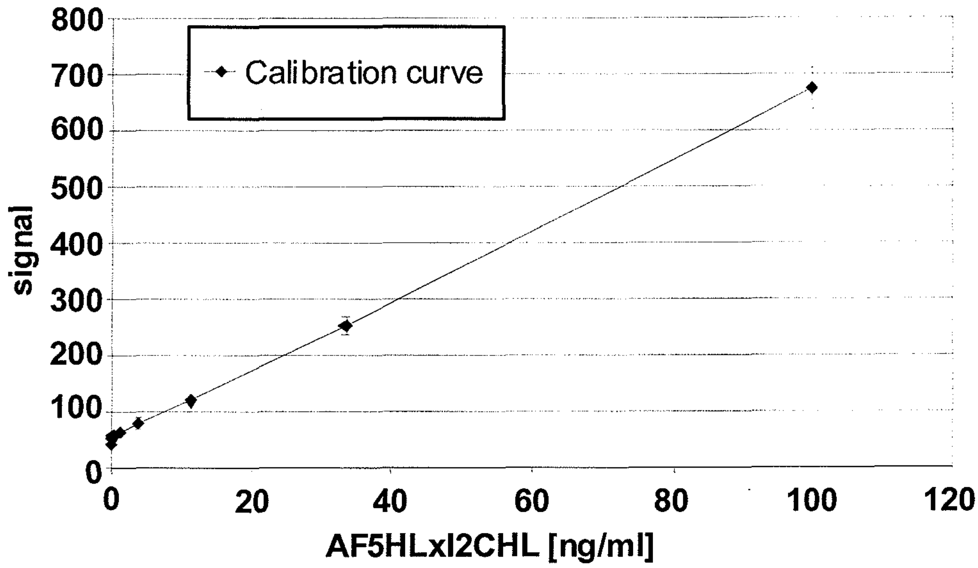
800
700
600
500
400
300
200
100
0
signal
Calibration curve
0
20
40
60
80
100
120
AF5HLxI2CHL [ng/ml]

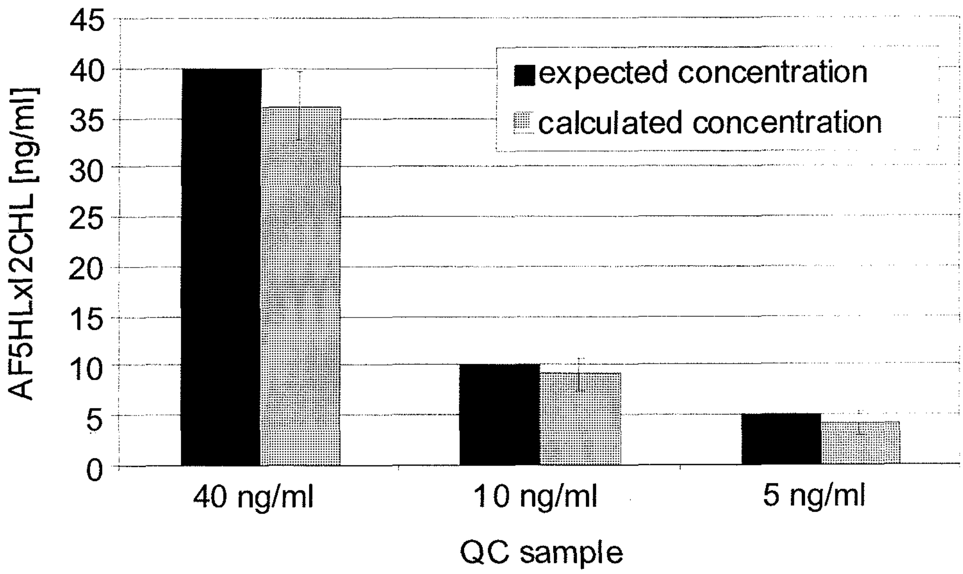
45
40
35
30
25
20
15
10
5
0
AF5HLxI2CHL [ng/ml]
expected concentration
calculated concentration
40 ng/ml
10 ng/ml
5 ng/ml
QC sample

Figure 35

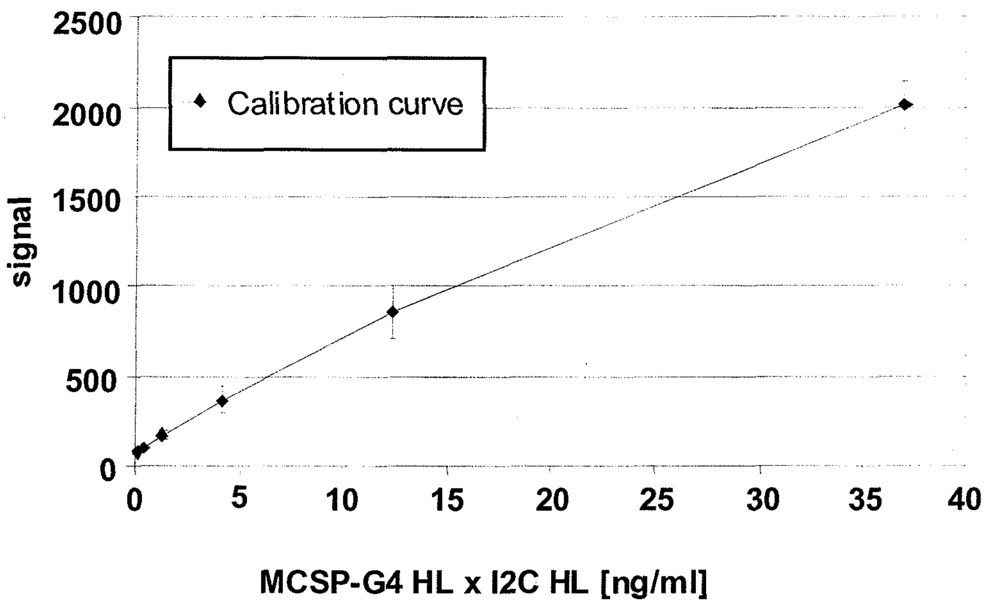

2500
2000
1500
1000
500
0
signal
Calibration curve
0
5
10
15
20
25
30
35
40
MCSP-G4 HL x I2C HL [ng/ml]


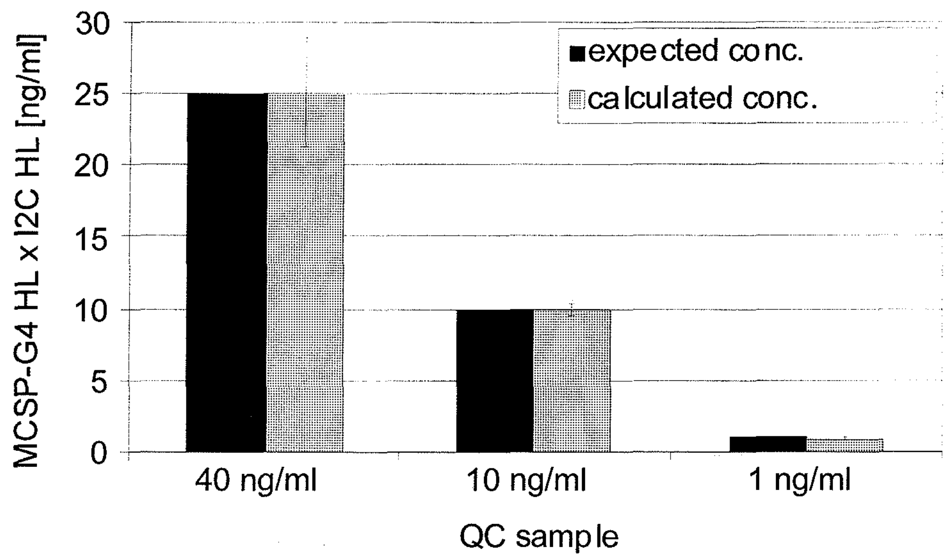

30
25
20
15
10
5
0
MCSP-G4 HL x I2C HL [ng/ml]
expected conc.
calculated conc.
40 ng/ml
10 ng/ml
1 ng/ml
QC sample

**Figure 36**

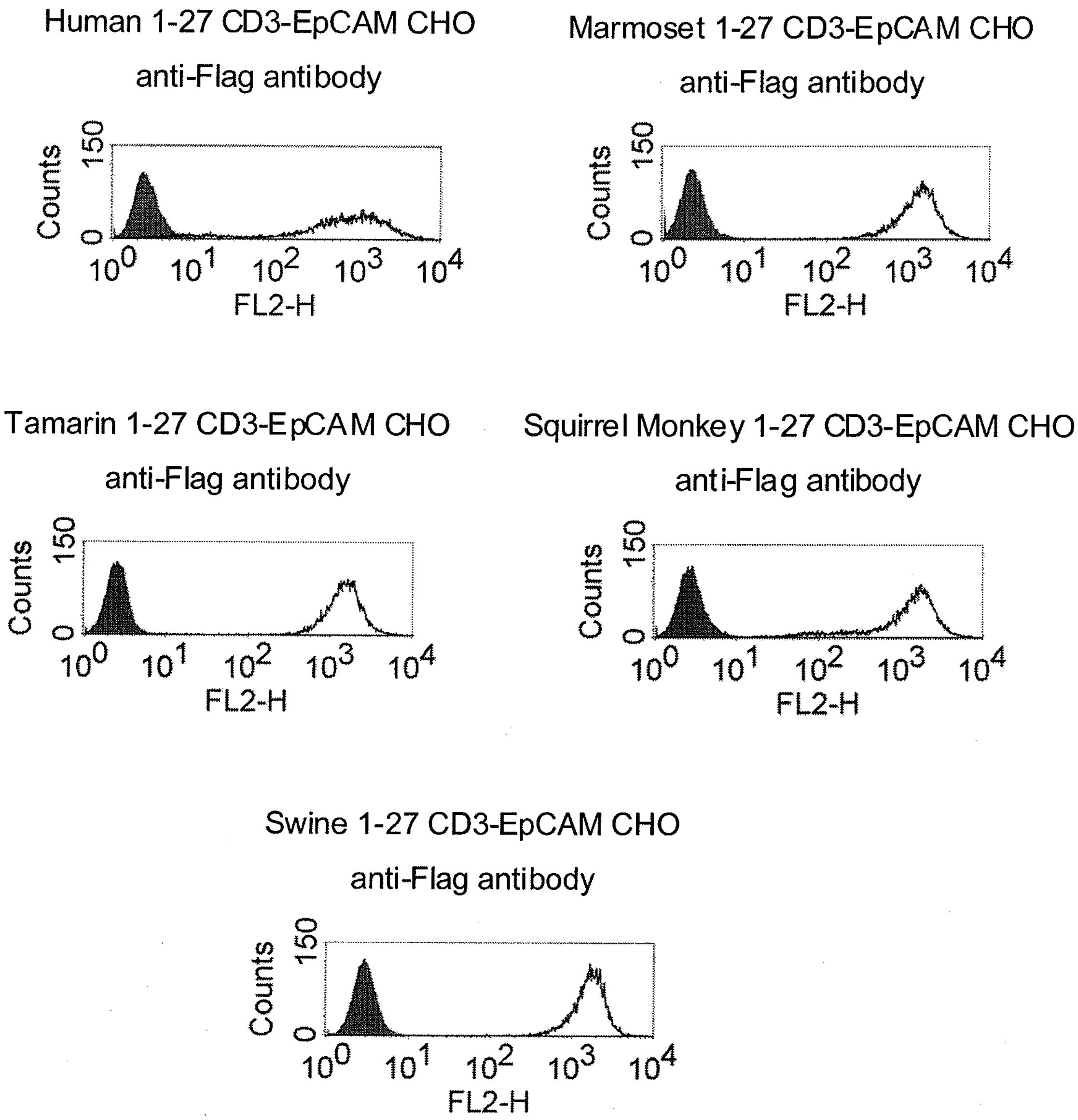
Human 1-27 CD3-EpCAM CHO
anti-Flag antibody
Marmoset 1-27 CD3-EpCAM CHO
anti-Flag antibody
Tamarin 1-27 CD3-EpCAM CHO
anti-Flag antibody
Squirrel Monkey 1-27 CD3-EpCAM CHO
anti-Flag antibody
Swine 1-27 CD3-EpCAM CHO
anti-Flag antibody
Counts
0
150
10^0
10^1
10^2
10^3
10^4
FL2-H

## Figure 37

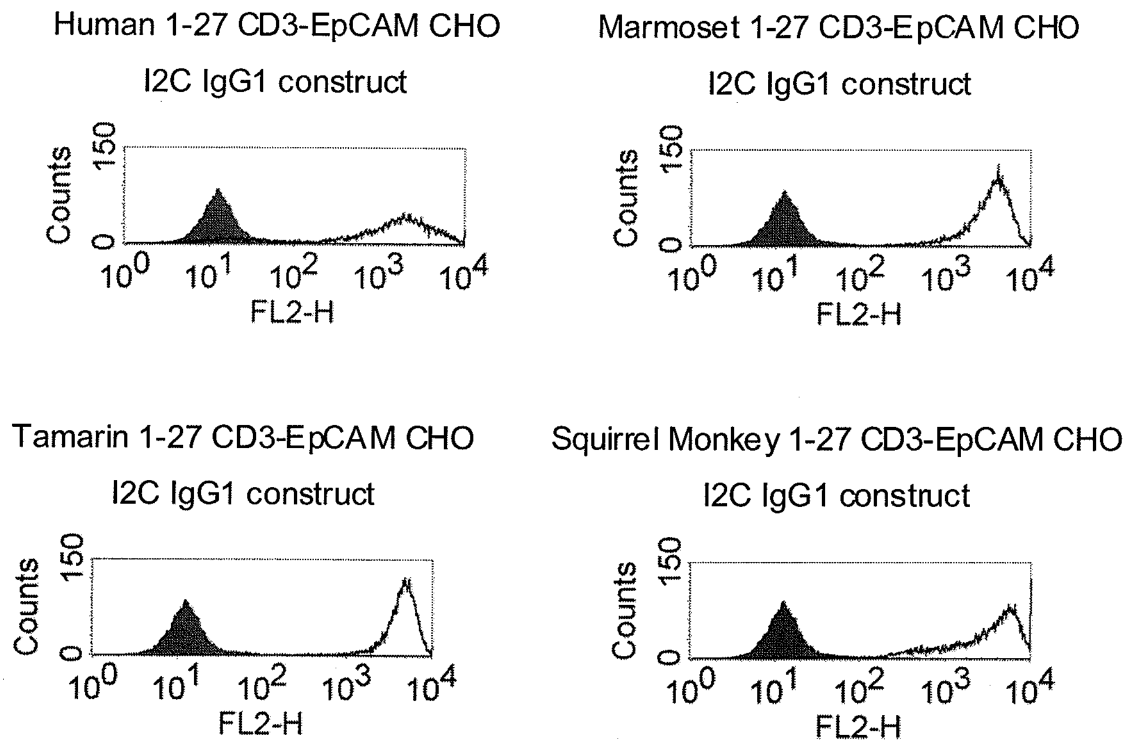
Human 1-27 CD3-EpCAM CHO
I2C IgG1 construct
Marmoset 1-27 CD3-EpCAM CHO
I2C IgG1 construct
Tamarin 1-27 CD3-EpCAM CHO
I2C IgG1 construct
Squirrel Monkey 1-27 CD3-EpCAM CHO
I2C IgG1 construct
Counts
0
150
$10^0$ $10^1$ $10^2$ $10^3$ $10^4$
FL2-H

## Figure 38

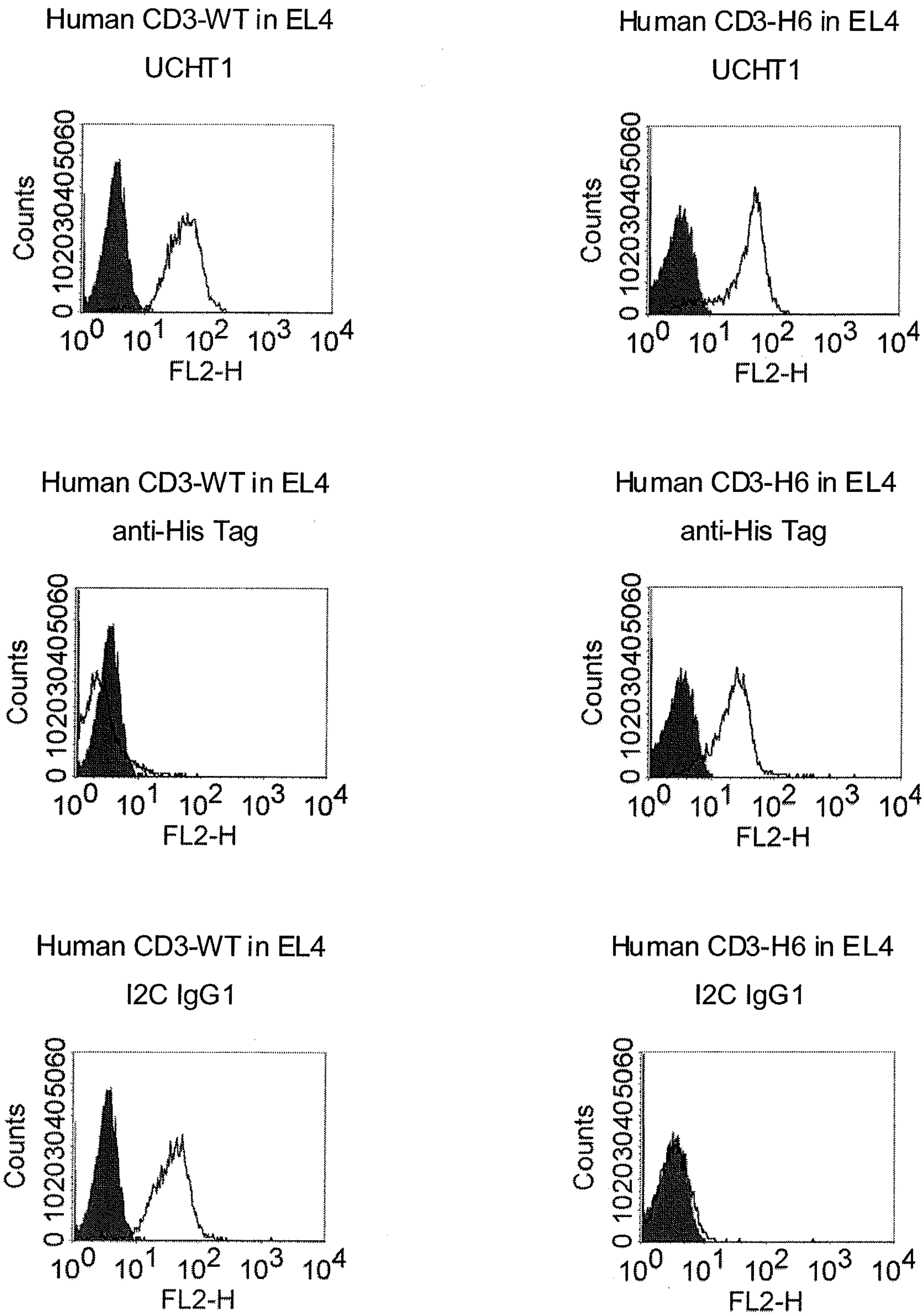
Human CD3-WT in EL4
UCHT1
Human CD3-H6 in EL4
UCHT1
Human CD3-WT in EL4
anti-His Tag
Human CD3-H6 in EL4
anti-His Tag
Human CD3-WT in EL4
I2C IgG1
Human CD3-H6 in EL4
I2C IgG1
Counts
0 10 20 30 40 50 60
$10^0$ $10^1$ $10^2$ $10^3$ $10^4$
FL2-H

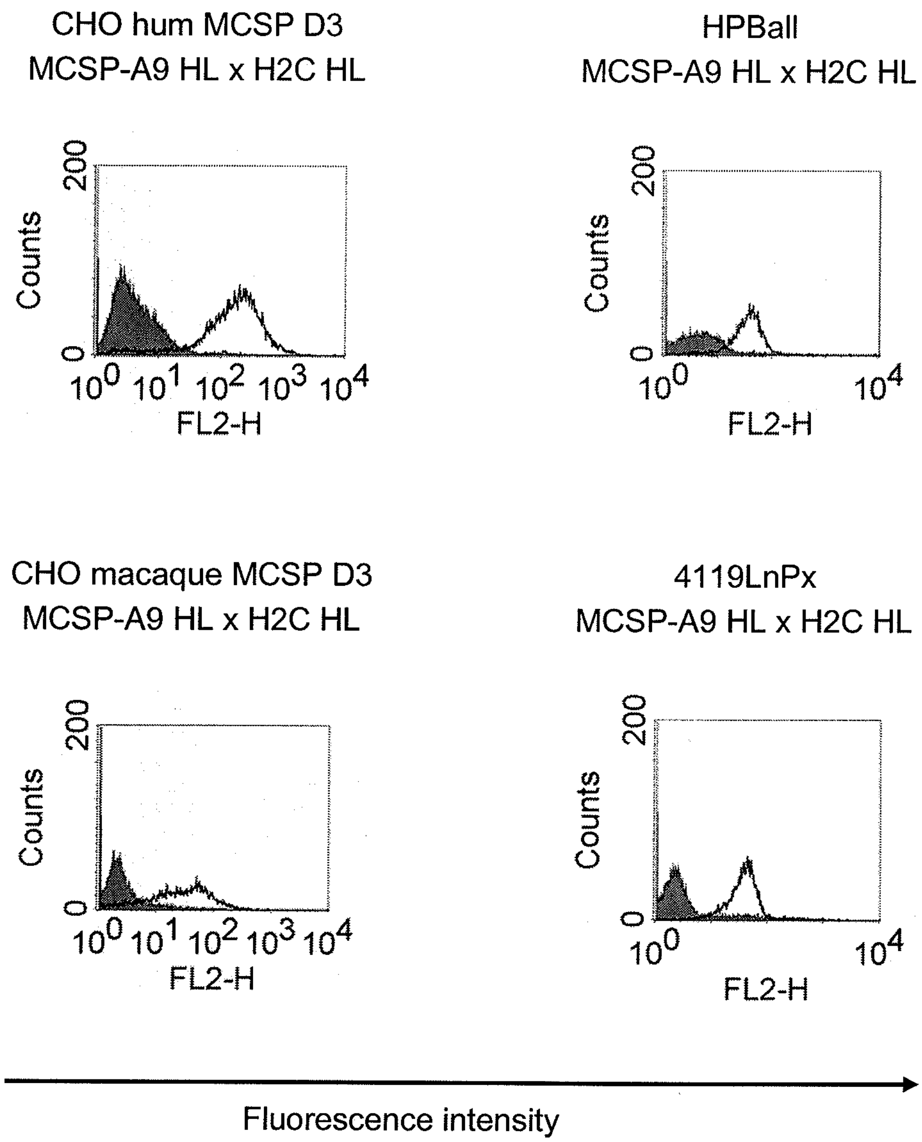
CHO hum MCSP D3
MCSP-A9 HL x H2C HL
HPBall
MCSP-A9 HL x H2C HL
CHO macaque MCSP D3
MCSP-A9 HL x H2C HL
4119LnPx
MCSP-A9 HL x H2C HL
Counts
200
0
FL2-H
$10^0$ $10^1$ $10^2$ $10^3$ $10^4$
Fluorescence intensity

**Figure 39a**

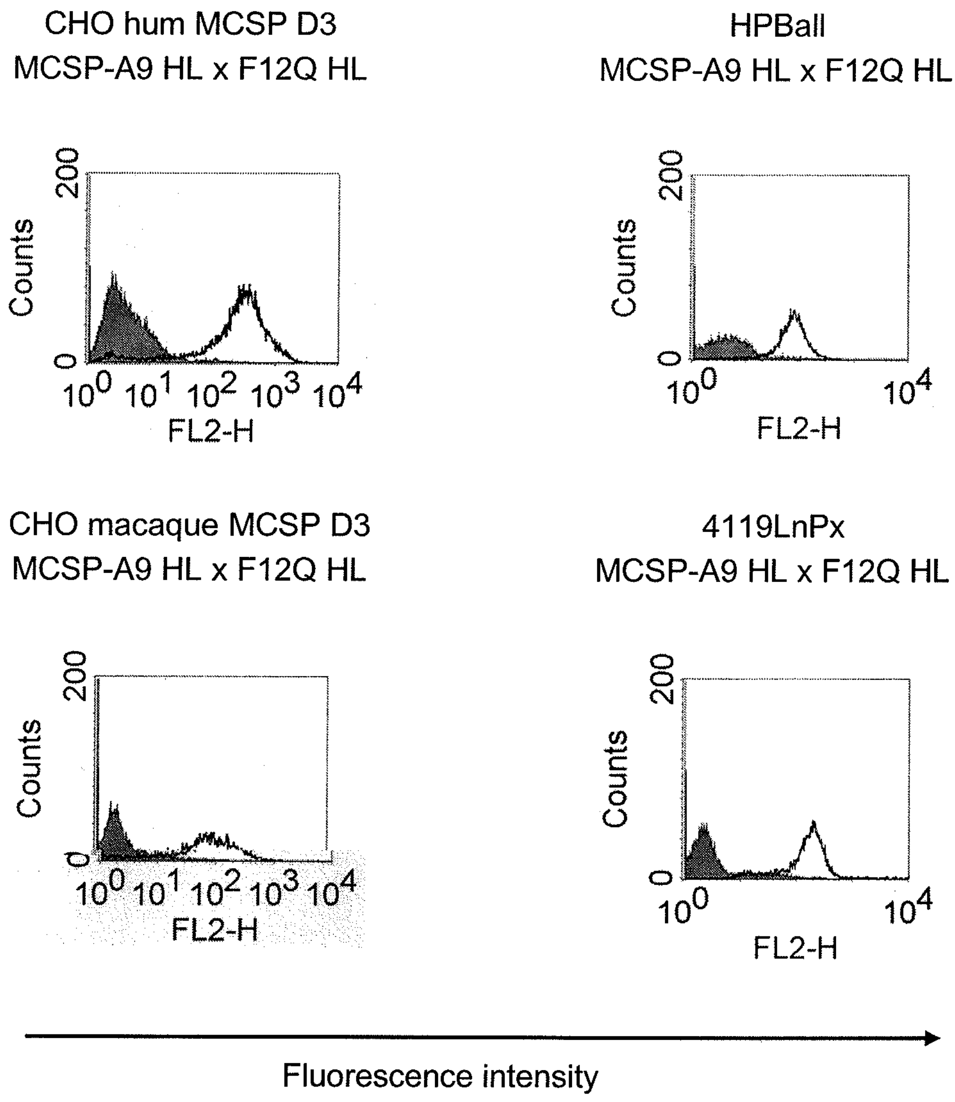
CHO hum MCSP D3
MCSP-A9 HL x F12Q HL
Counts
200
0
10^0 10^1 10^2 10^3 10^4
FL2-H
HPBall
MCSP-A9 HL x F12Q HL
Counts
200
0
10^0
10^4
FL2-H
CHO macaque MCSP D3
MCSP-A9 HL x F12Q HL
Counts
200
0
10^0 10^1 10^2 10^3 10^4
FL2-H
4119LnPx
MCSP-A9 HL x F12Q HL
Counts
200
0
10^0
10^4
FL2-H
Fluorescence intensity

**Figure 39b**

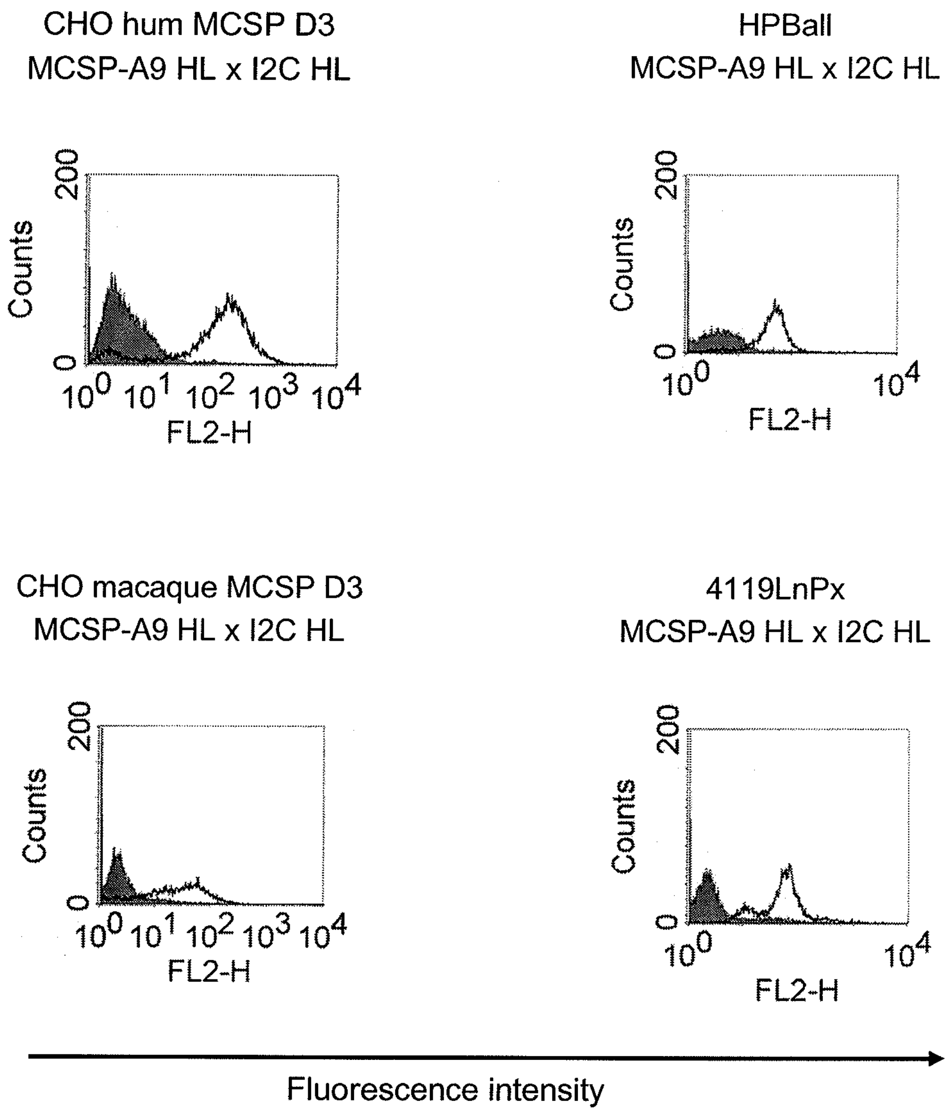
CHO hum MCSP D3
MCSP-A9 HL x I2C HL
Counts
200
0
10^0 10^1 10^2 10^3 10^4
FL2-H
HPBall
MCSP-A9 HL x I2C HL
Counts
200
0
10^0
10^4
FL2-H
CHO macaque MCSP D3
MCSP-A9 HL x I2C HL
Counts
200
0
10^0 10^1 10^2 10^3 10^4
FL2-H
4119LnPx
MCSP-A9 HL x I2C HL
Counts
200
0
10^0
10^4
FL2-H
Fluorescence intensity

**Figure 39c**

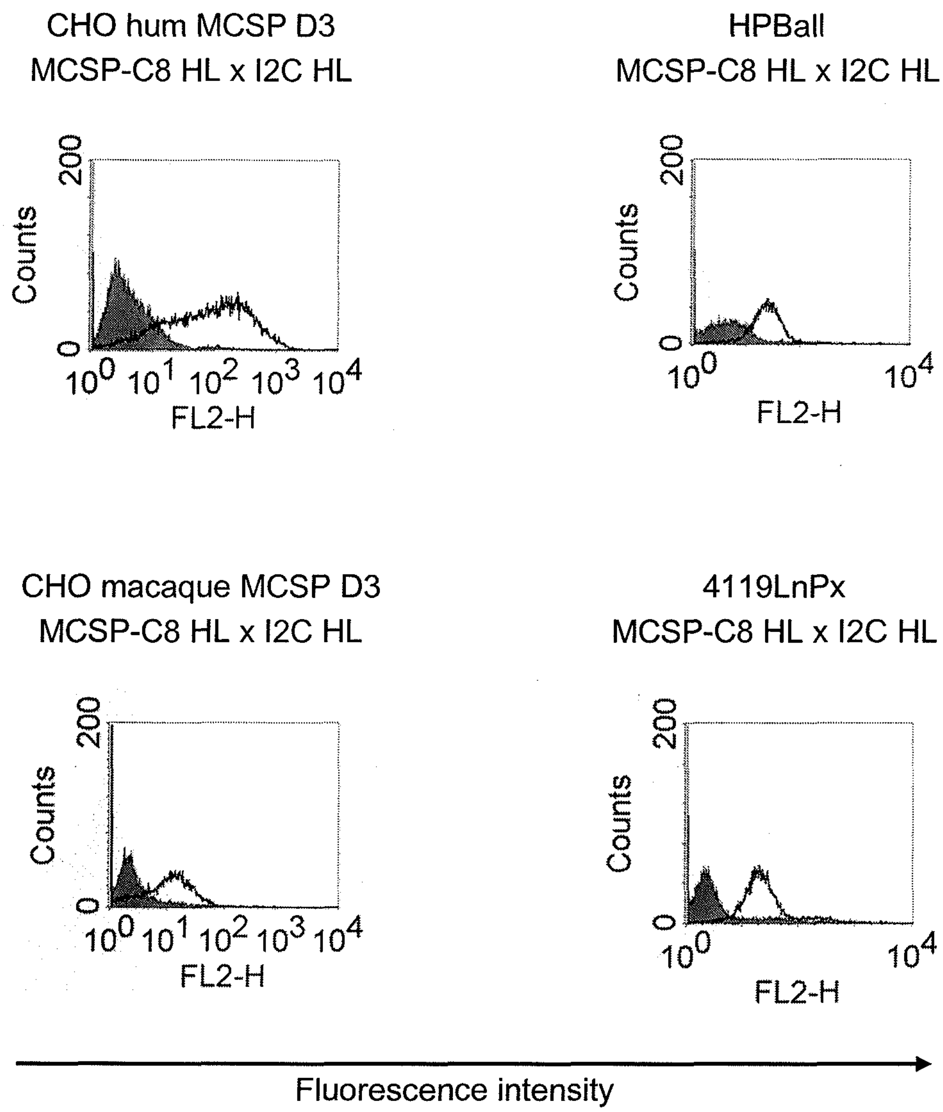
CHO hum MCSP D3
MCSP-C8 HL x I2C HL
HPBall
MCSP-C8 HL x I2C HL
CHO macaque MCSP D3
MCSP-C8 HL x I2C HL
4119LnPx
MCSP-C8 HL x I2C HL
Counts
0
200
$10^0$ $10^1$ $10^2$ $10^3$ $10^4$
FL2-H
Fluorescence intensity

**Figure 39d**

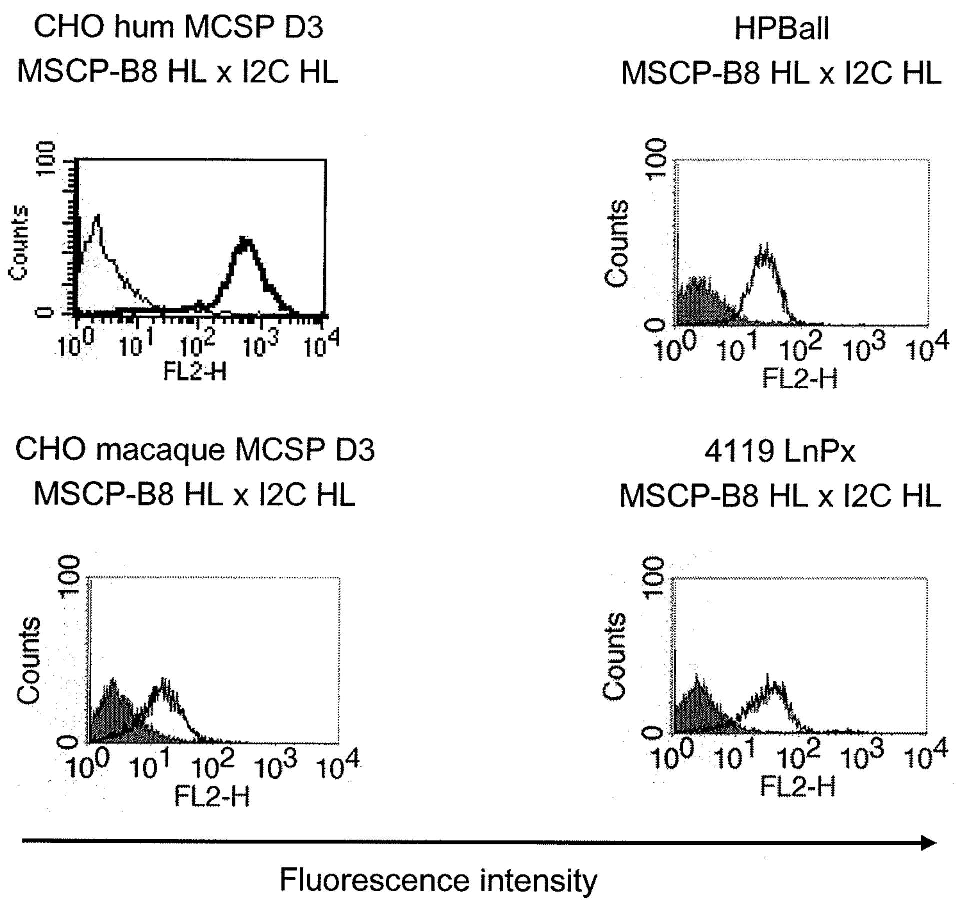
CHO hum MCSP D3
MSCP-B8 HL x I2C HL
Counts
100
0
10^0 10^1 10^2 10^3 10^4
FL2-H
HPBall
MSCP-B8 HL x I2C HL
CHO macaque MCSP D3
MSCP-B8 HL x I2C HL
4119 LnPx
MSCP-B8 HL x I2C HL
Fluorescence intensity

Figure 39e

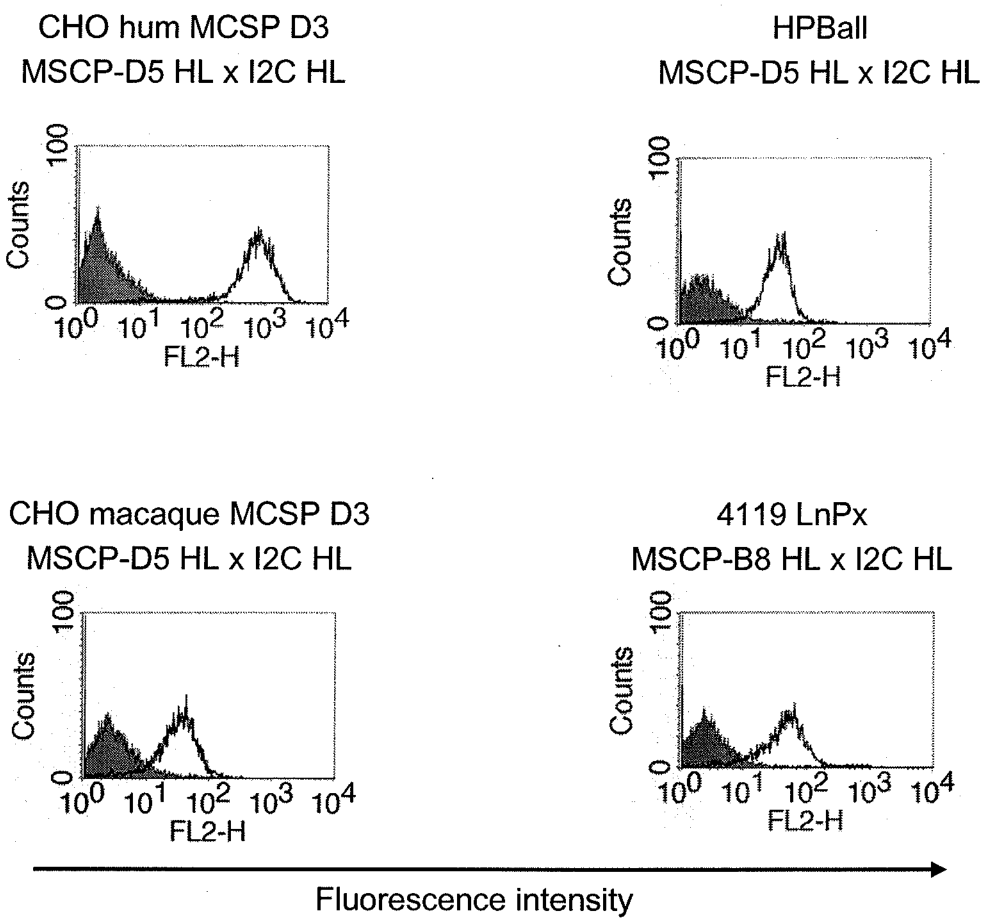
CHO hum MCSP D3
MSCP-D5 HL x I2C HL
Counts
FL2-H
HPBall
MSCP-D5 HL x I2C HL
CHO macaque MCSP D3
MSCP-D5 HL x I2C HL
4119 LnPx
MSCP-B8 HL x I2C HL
Fluorescence intensity

Figure 39f

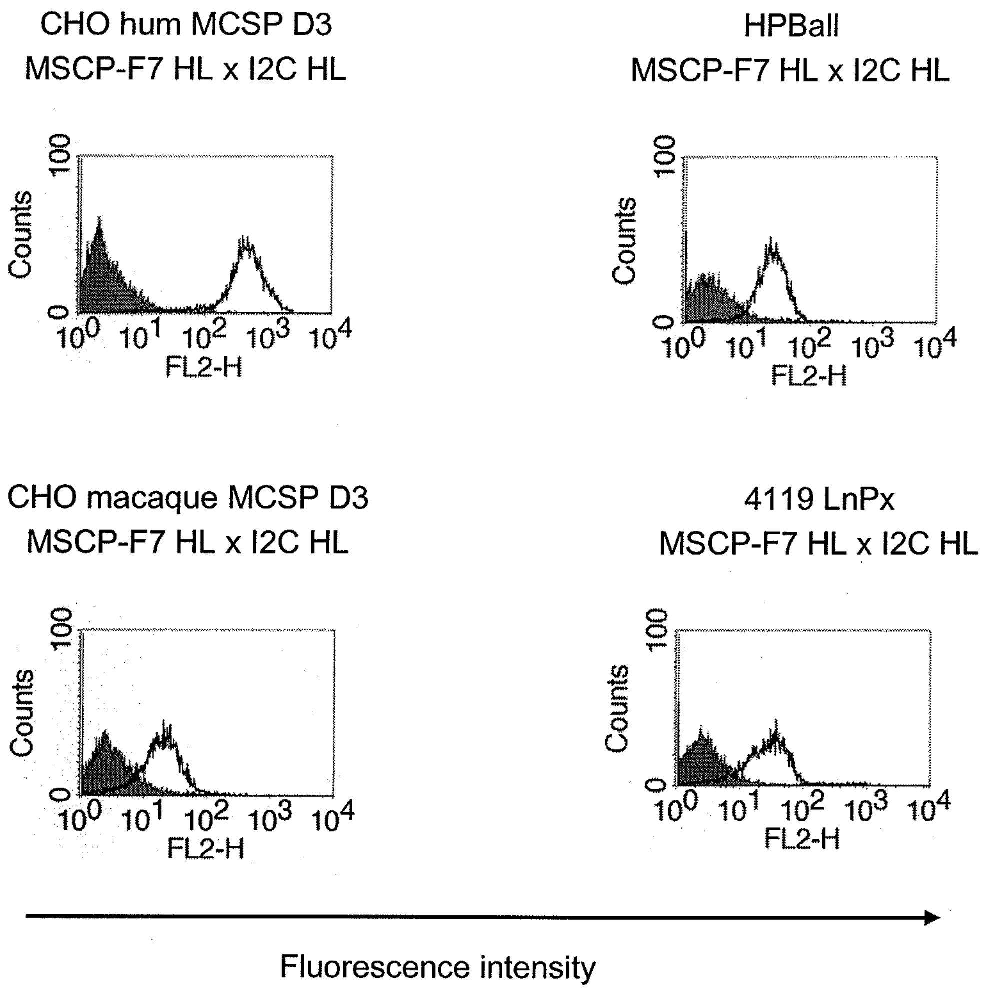
CHO hum MCSP D3
MSCP-F7 HL x I2C HL
HPBall
MSCP-F7 HL x I2C HL
CHO macaque MCSP D3
MSCP-F7 HL x I2C HL
4119 LnPx
MSCP-F7 HL x I2C HL
Counts
0
100
10^0 10^1 10^2 10^3 10^4
FL2-H
Fluorescence intensity

**Figure 39g**

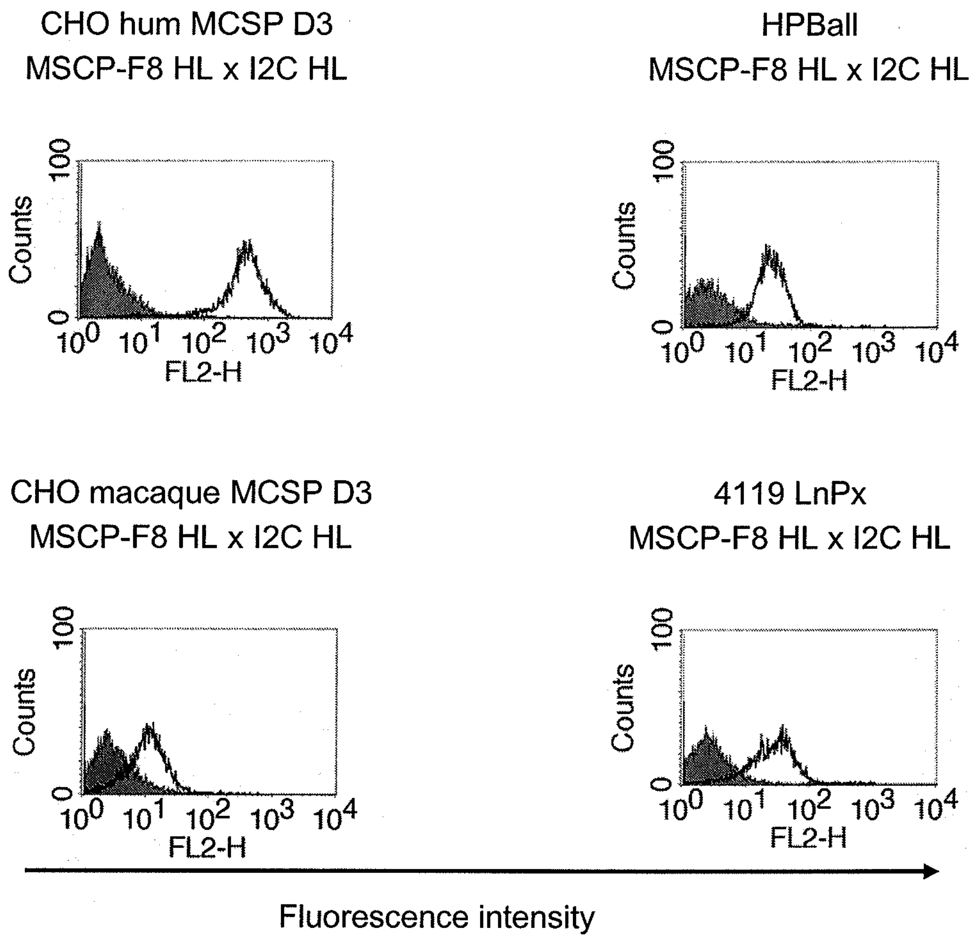
CHO hum MCSP D3
MSCP-F8 HL x I2C HL
HPBall
MSCP-F8 HL x I2C HL
CHO macaque MCSP D3
MSCP-F8 HL x I2C HL
4119 LnPx
MSCP-F8 HL x I2C HL
Counts
100
0
$10^0$ $10^1$ $10^2$ $10^3$ $10^4$
FL2-H
Fluorescence intensity

**Figure 39h**

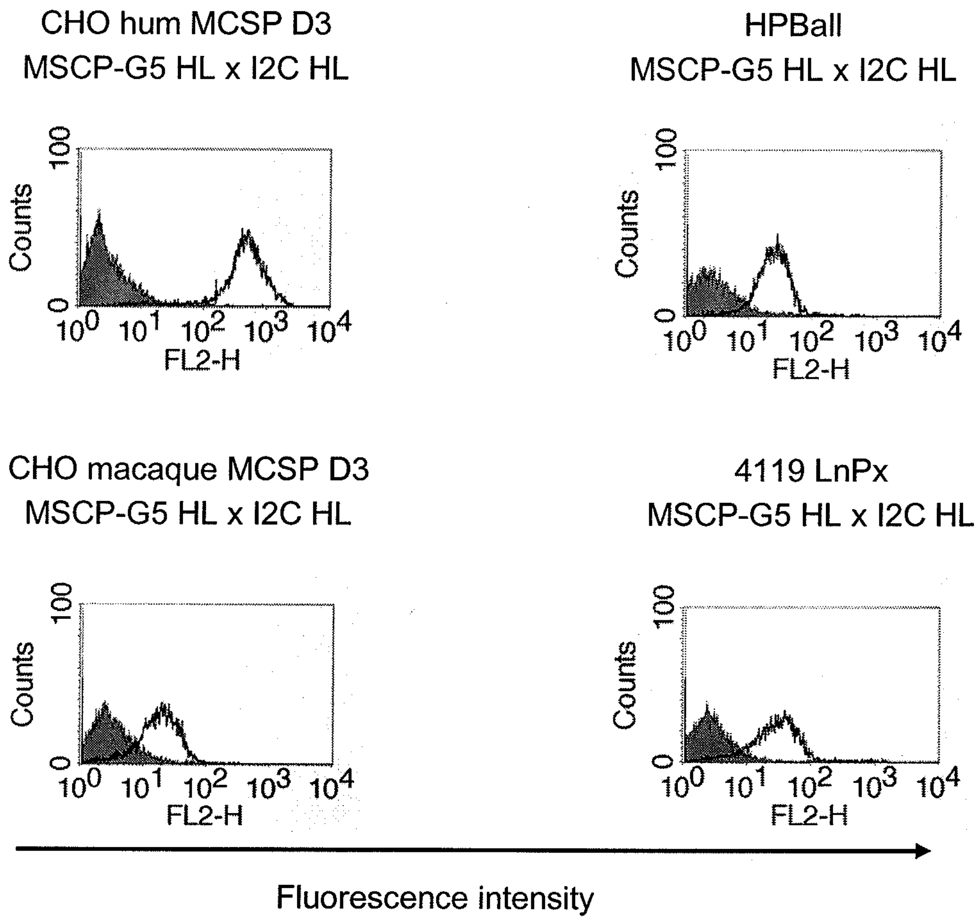
CHO hum MCSP D3
MSCP-G5 HL x I2C HL
HPBall
MSCP-G5 HL x I2C HL
CHO macaque MCSP D3
MSCP-G5 HL x I2C HL
4119 LnPx
MSCP-G5 HL x I2C HL
Counts
100
0
$10^0$ $10^1$ $10^2$ $10^3$ $10^4$
FL2-H
Fluorescence intensity

**Figure 39i**

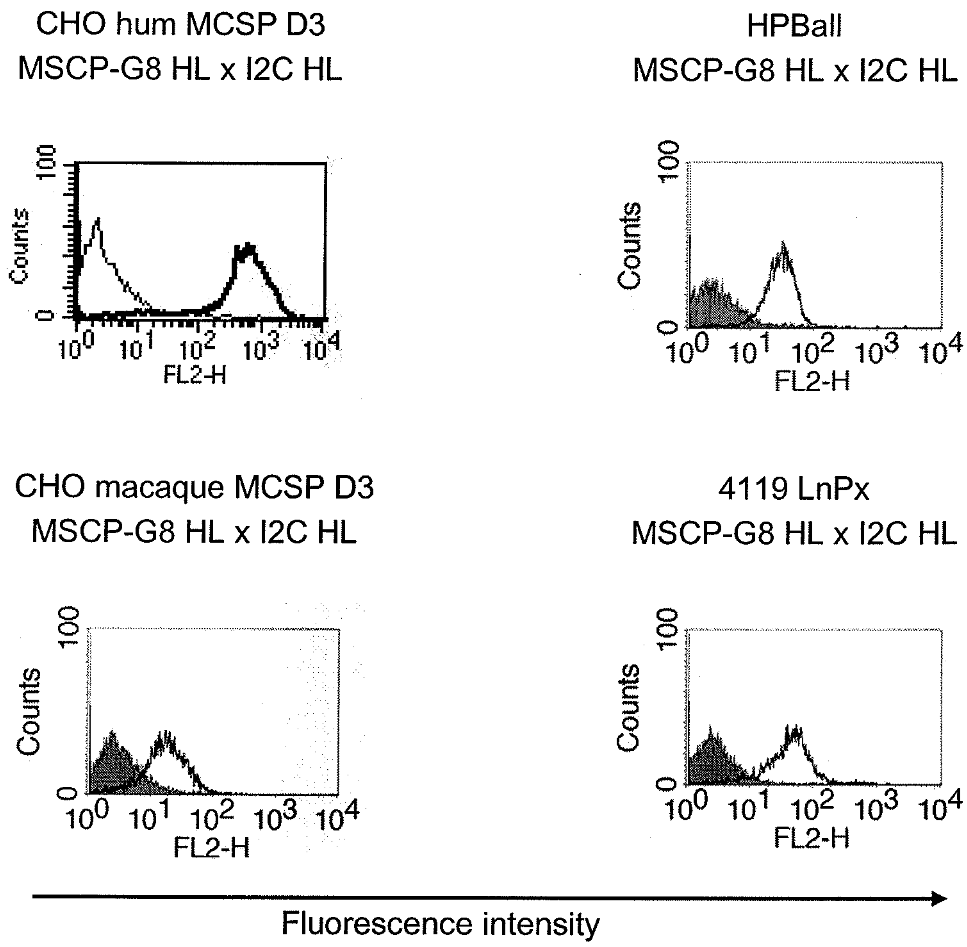
CHO hum MCSP D3
MSCP-G8 HL x I2C HL
HPBall
MSCP-G8 HL x I2C HL
CHO macaque MCSP D3
MSCP-G8 HL x I2C HL
4119 LnPx
MSCP-G8 HL x I2C HL
Counts
100
0
$10^0$ $10^1$ $10^2$ $10^3$ $10^4$
FL2-H
Fluorescence intensity

**Figure 39j**

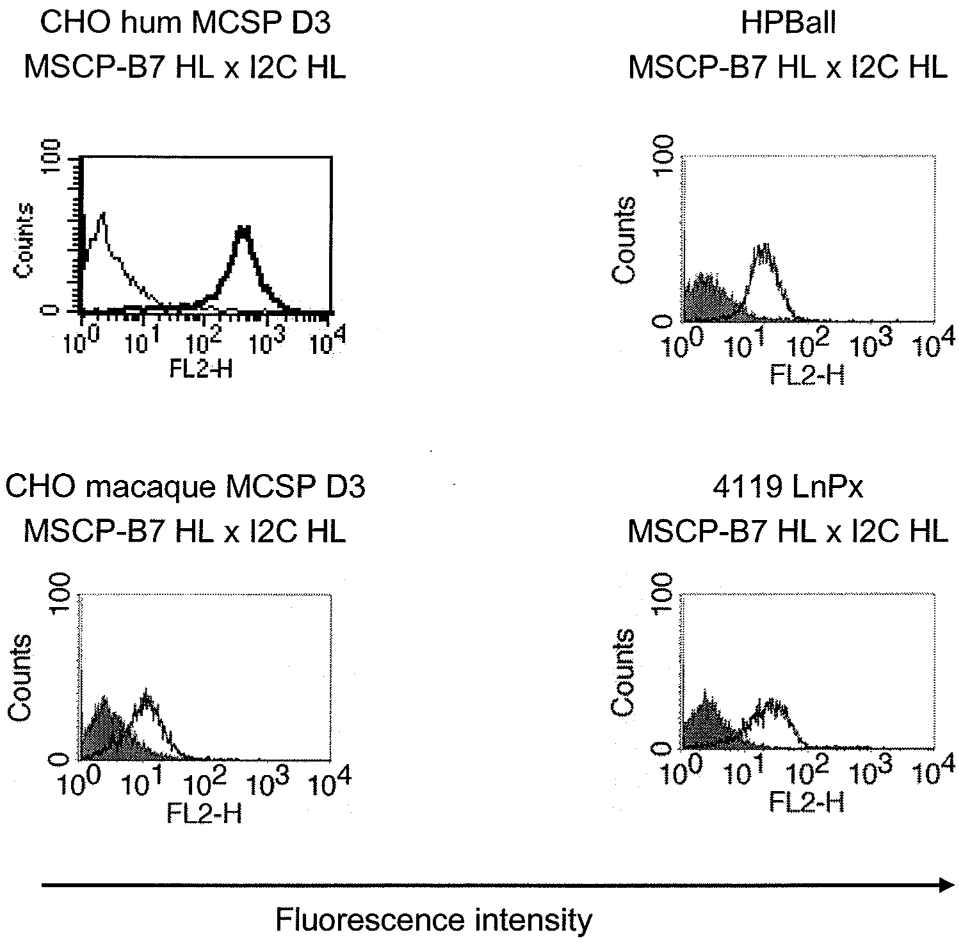
CHO hum MCSP D3
MSCP-B7 HL x I2C HL
HPBall
MSCP-B7 HL x I2C HL
CHO macaque MCSP D3
MSCP-B7 HL x I2C HL
4119 LnPx
MSCP-B7 HL x I2C HL
Counts
100
0
$10^0$ $10^1$ $10^2$ $10^3$ $10^4$
FL2-H
Fluorescence intensity

**Figure 39k**

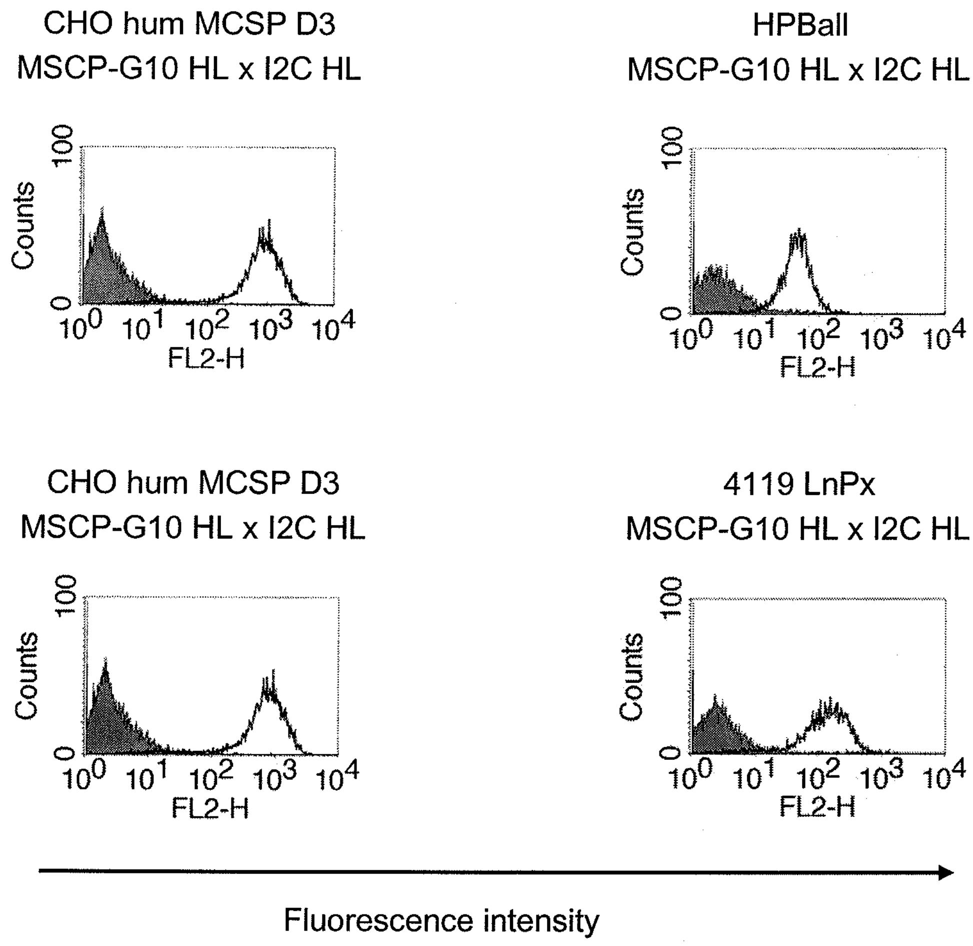
CHO hum MCSP D3
MSCP-G10 HL x I2C HL
HPBall
MSCP-G10 HL x I2C HL
CHO hum MCSP D3
MSCP-G10 HL x I2C HL
4119 LnPx
MSCP-G10 HL x I2C HL
Counts
100
0
10^0 10^1 10^2 10^3 10^4
FL2-H
Fluorescence intensity

**Figure 39I**

**Figure 40a**

Effector cells: stimulated human CD4/CD56 depleted human PBMC
Target cells: CHO transfected with human MCSP D3
E:T ratio: 10:1

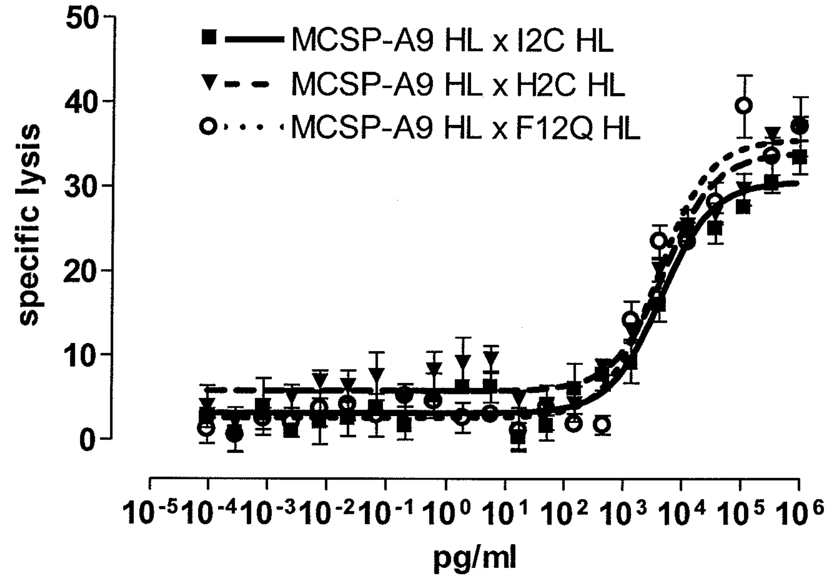


Effector cells: 4119 LnPx
Target cells: CHO transfected with macaque MCSP D3
E:T ratio: 10:1

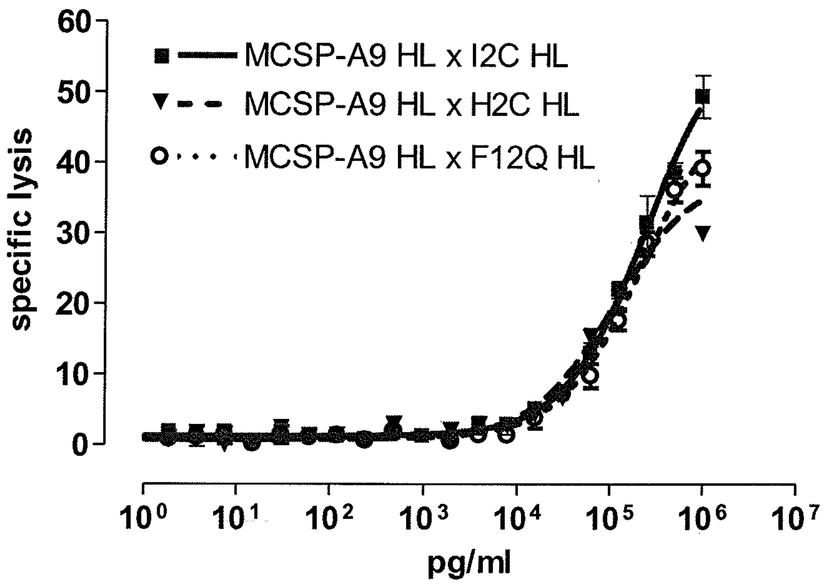

**Figure 40b**

Effector cells: stimulated human CD4/CD56 depleted human PBMC
Target cells: CHO transfected with human MCSP D3
E:T ratio: 10:1

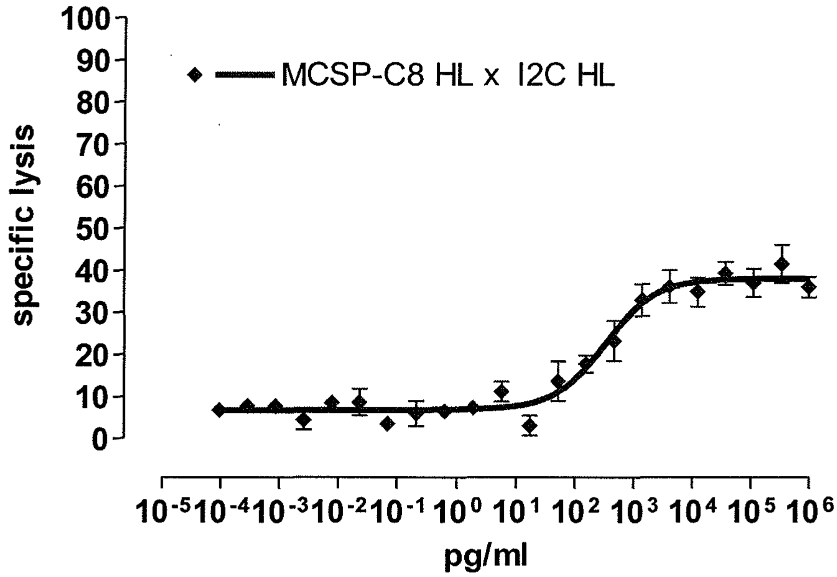


Effector cells: 4119 LnPx
Target cells: CHO transfected with macaque MCSP D3
E:T ratio: 10:1

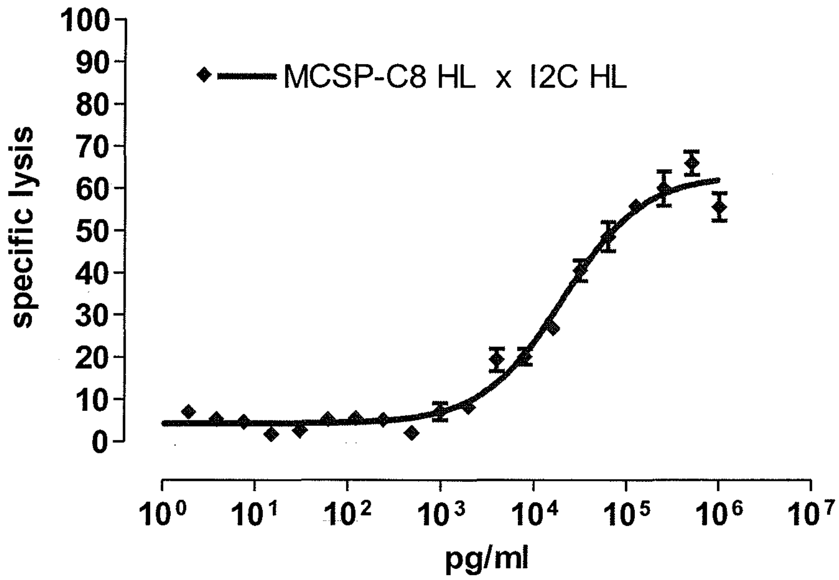

**Figure 40c**

Effector cells: stimulated human CD4/CD56 depleted human PBMC
Target cells: CHO transfected with human MCSP D3
E:T ratio: 10:1

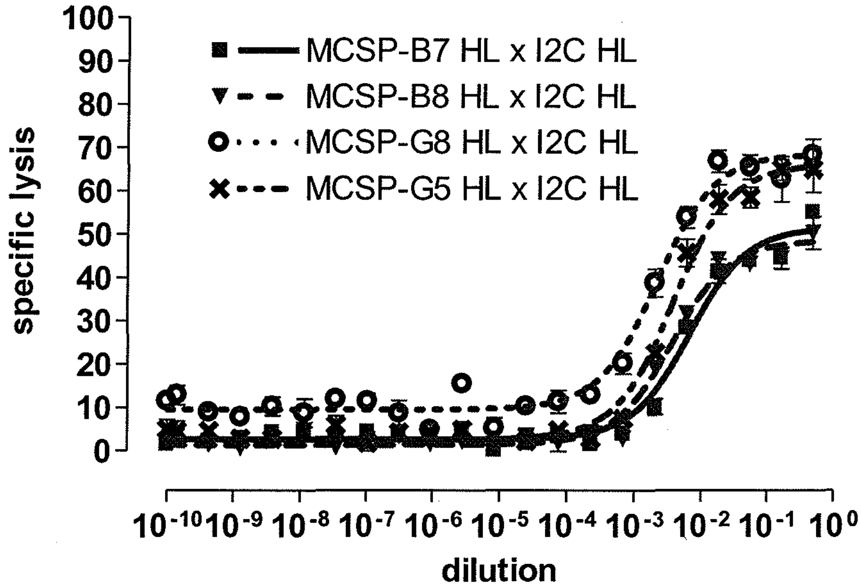


Effector cells: 4119 LnPx
Target cells: CHO transfected with macaque MCSP D3
E:T ratio: 10:1

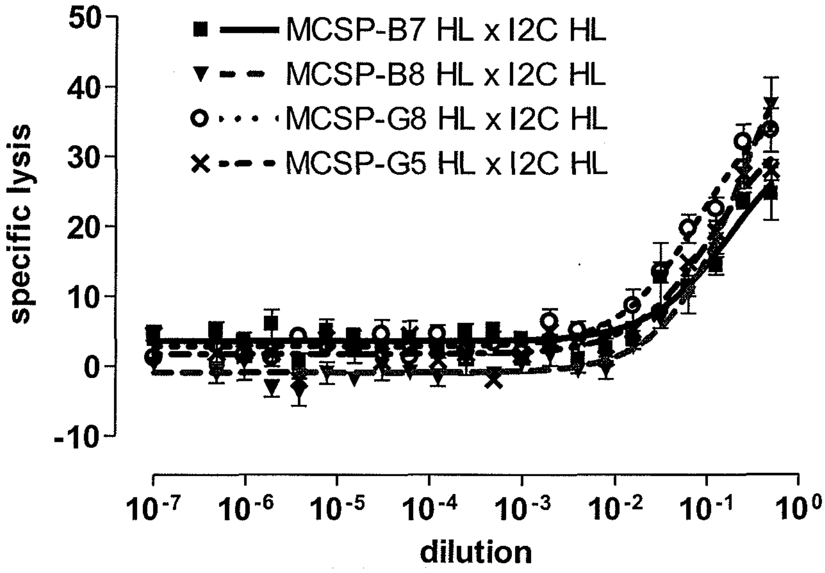

**Figure 40d**

Effector cells: stimulated human CD4/CD56 depleted human PBMC
Target cells: CHO transfected with human MCSP D3
E:T ratio: 10:1

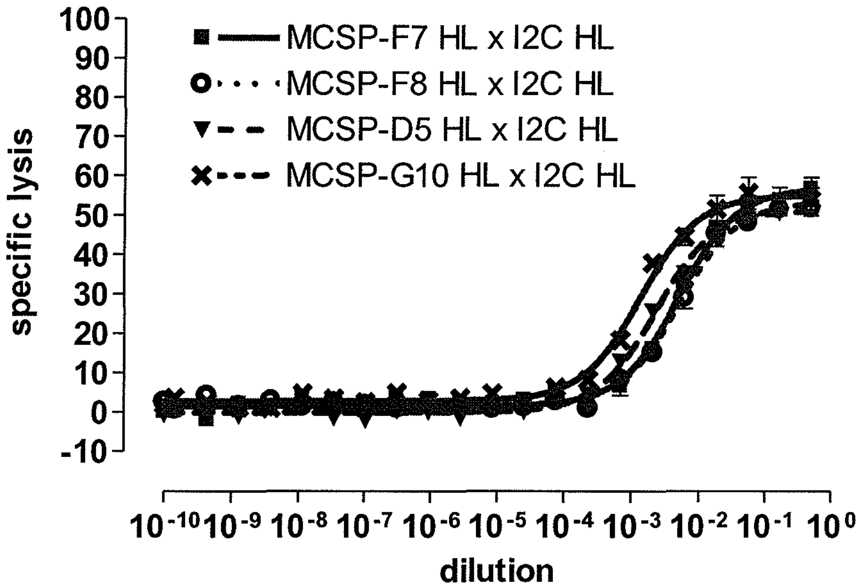


Effector cells: 4119 LnPx
Target cells: CHO transfected with macaque MCSP D3
E:T ratio: 10:1

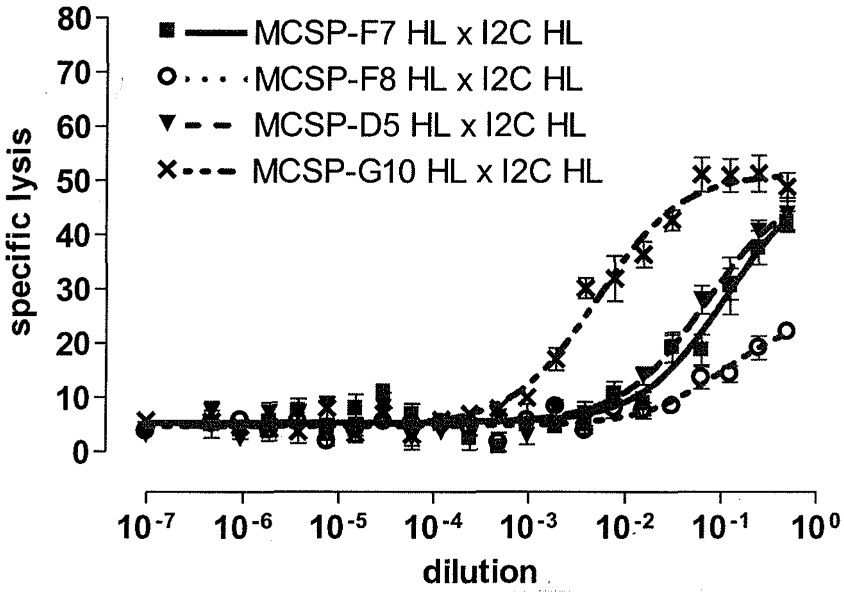

**Figure 41a**

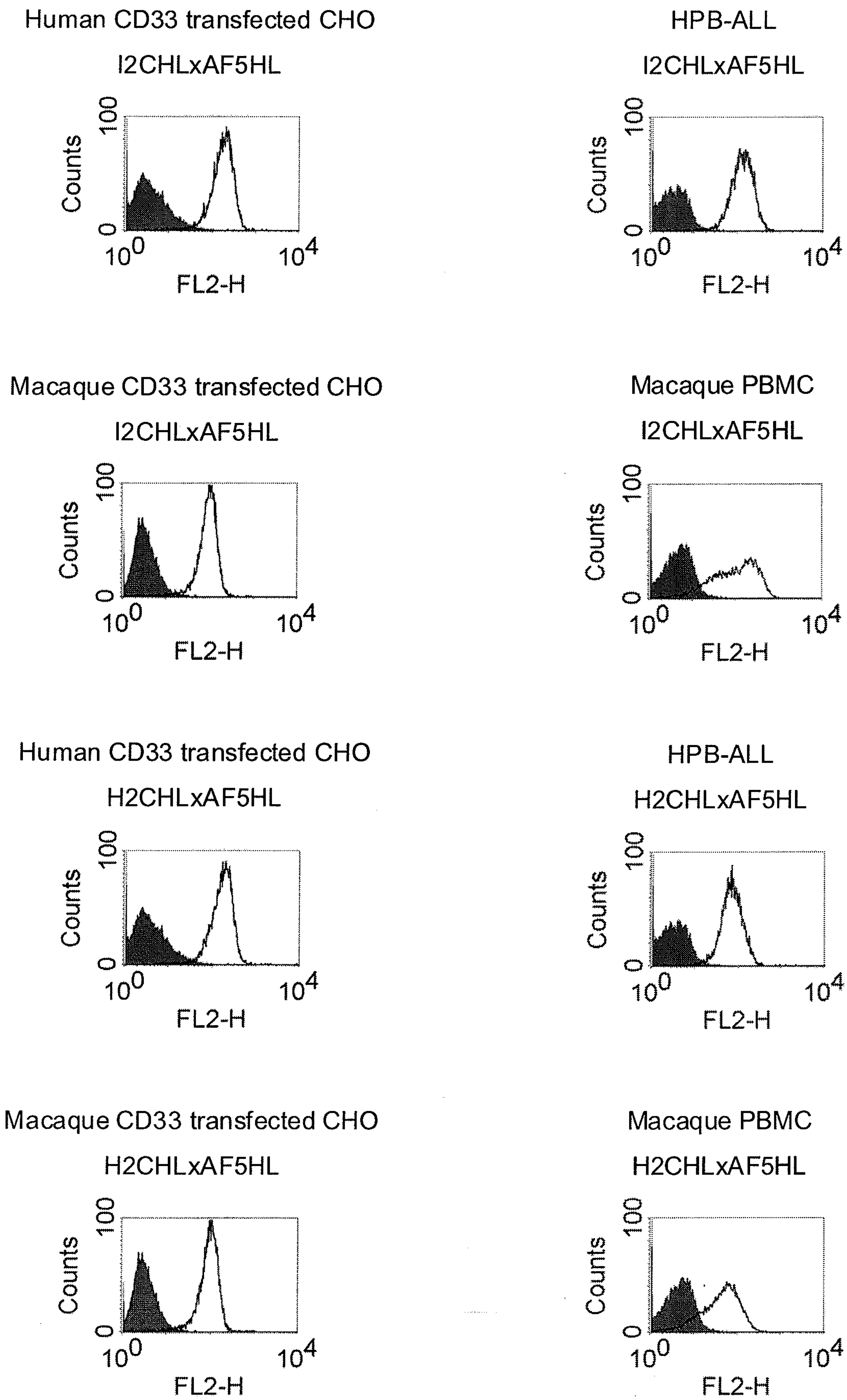
Human CD33 transfected CHO
I2CHLxAF5HL
HPB-ALL
I2CHLxAF5HL
Macaque CD33 transfected CHO
I2CHLxAF5HL
Macaque PBMC
I2CHLxAF5HL
Human CD33 transfected CHO
H2CHLxAF5HL
HPB-ALL
H2CHLxAF5HL
Macaque CD33 transfected CHO
H2CHLxAF5HL
Macaque PBMC
H2CHLxAF5HL
Counts
0
100
$10^0$
$10^4$
FL2-H

## Figure 41b

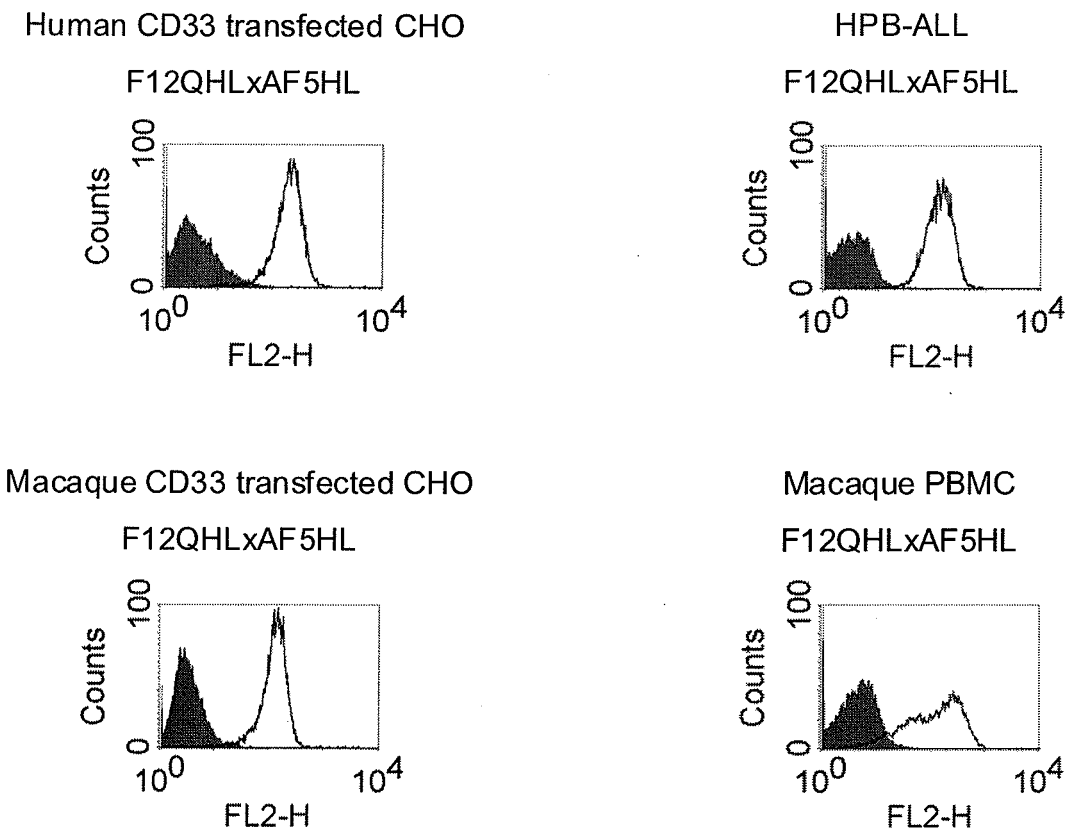
Human CD33 transfected CHO
F12QHLxAF5HL
Counts
100
0
$10^0$
$10^4$
FL2-H
HPB-ALL
F12QHLxAF5HL
Counts
100
0
$10^0$
$10^4$
FL2-H
Macaque CD33 transfected CHO
F12QHLxAF5HL
Counts
100
0
$10^0$
$10^4$
FL2-H
Macaque PBMC
F12QHLxAF5HL
Counts
100
0
$10^0$
$10^4$
FL2-H

# Figure 42

Effector cells: stimulated CD4/CD56 depleted human PBMC

Target cells: CHO transfected with human CD33

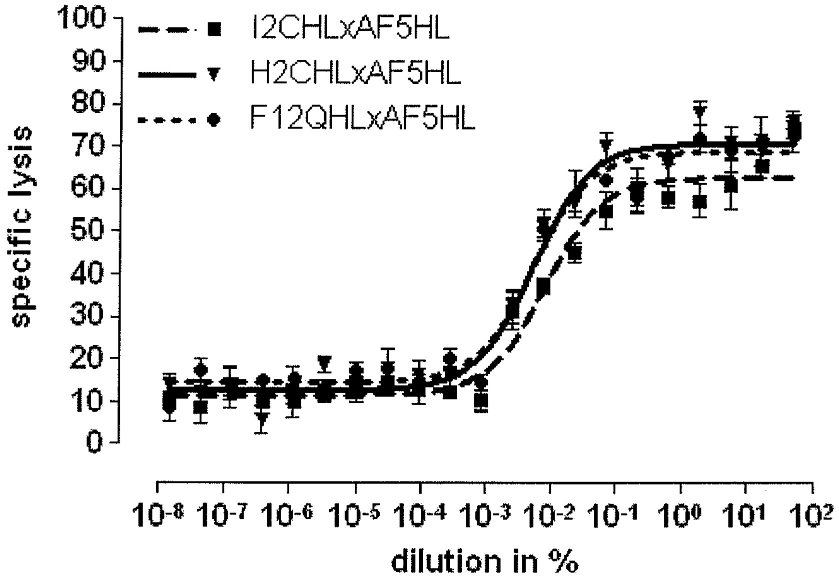


Effector cells: macaque 4119 LnPx

Target cells: CHO transfected with macaque CD33

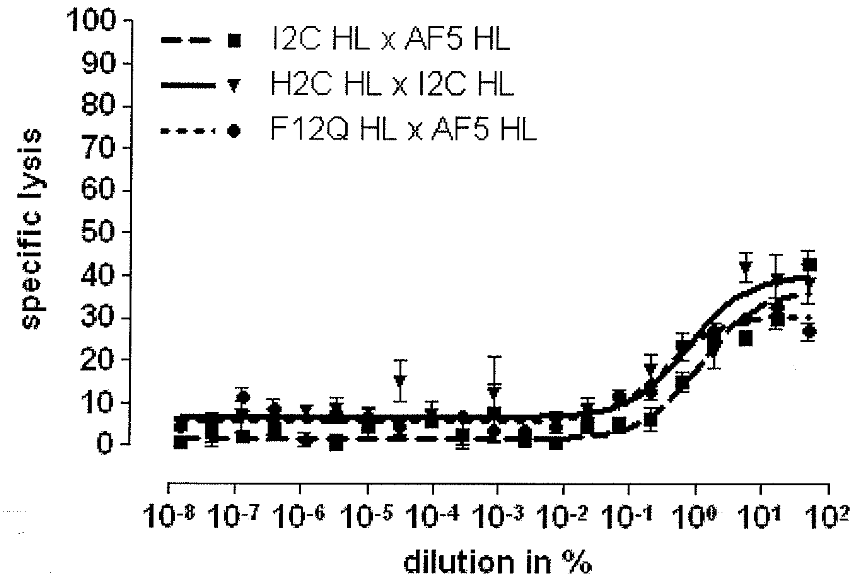

**Figure 43**

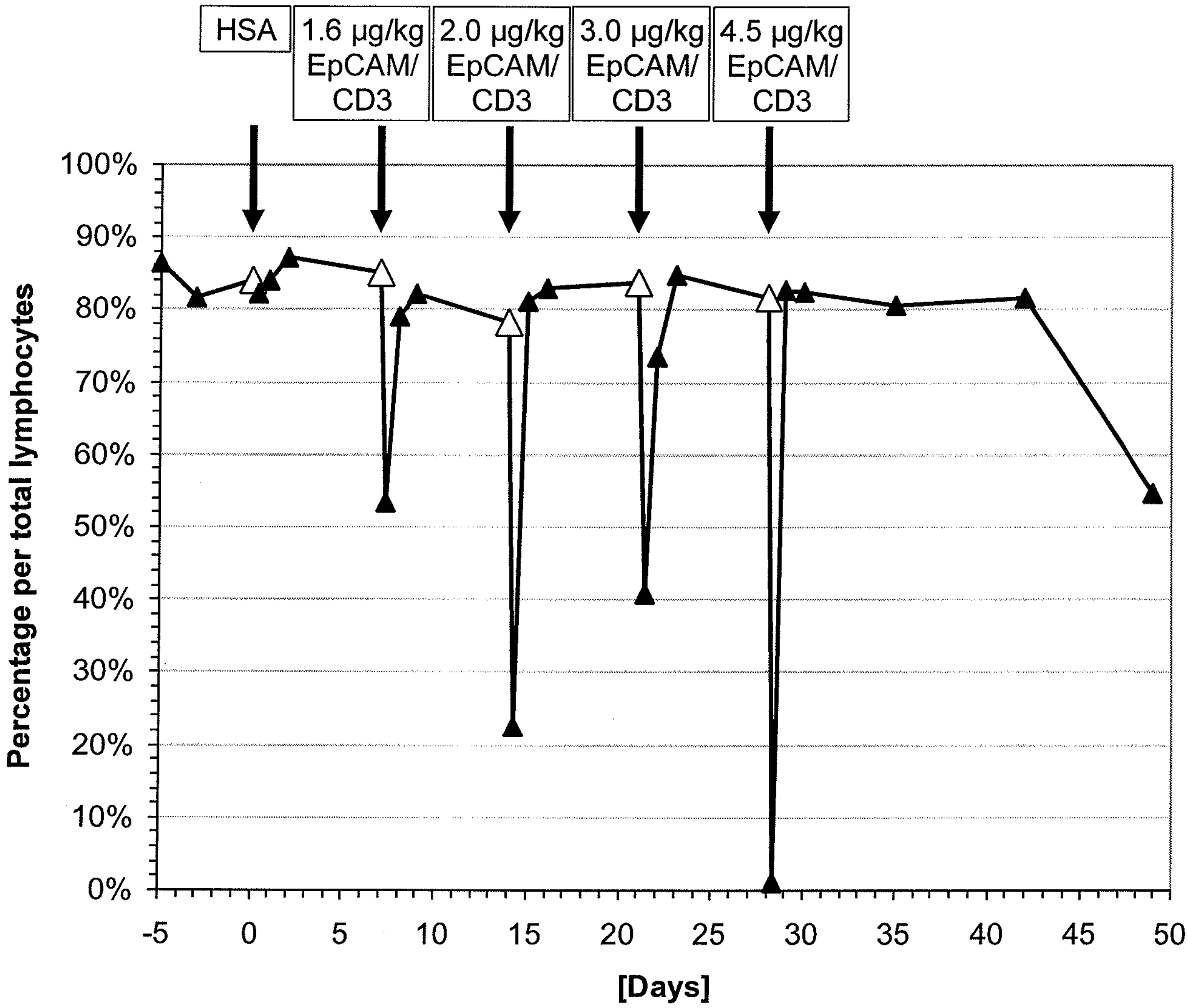
HSA
1.6 µg/kg EpCAM/ CD3
2.0 µg/kg EpCAM/ CD3
3.0 µg/kg EpCAM/ CD3
4.5 µg/kg EpCAM/ CD3
Percentage per total lymphocytes
100%
90%
80%
70%
60%
50%
40%
30%
20%
10%
0%
-5
0
5
10
15
20
25
30
35
40
45
50
[Days]

# Figure 44

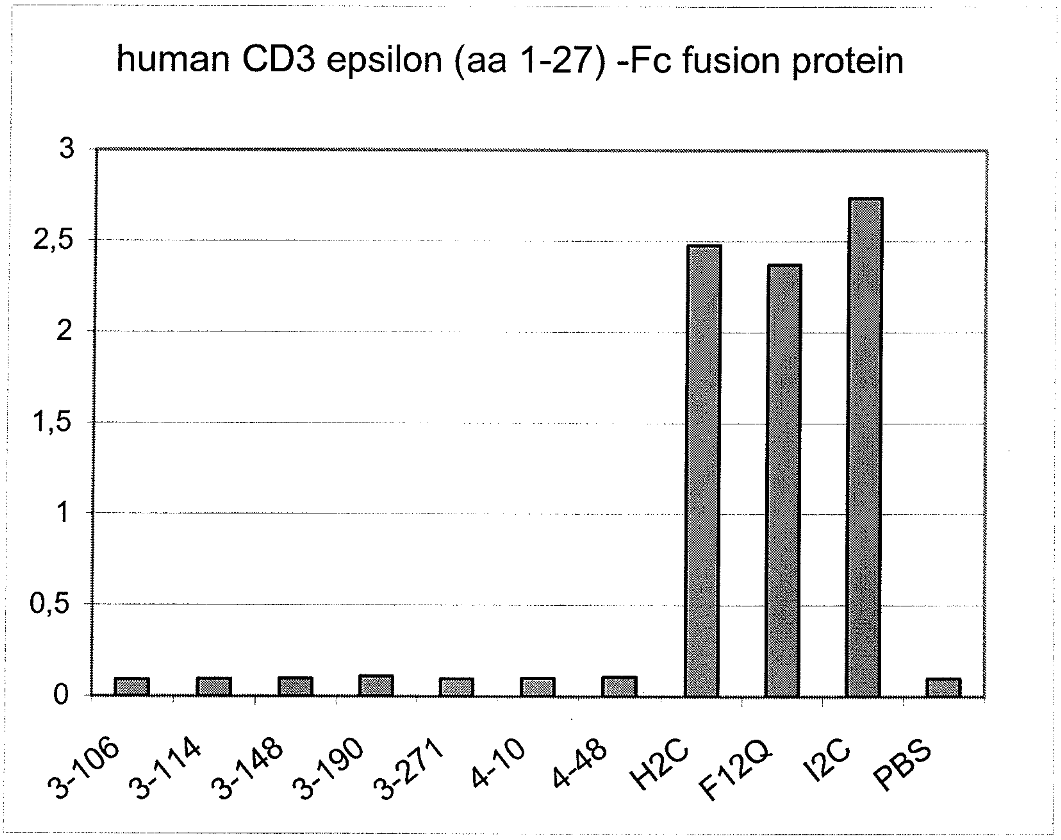
human CD3 epsilon (aa 1-27) -Fc fusion protein
3
2,5
2
1,5
1
0,5
0
3-106
3-114
3-148
3-190
3-271
4-10
4-48
H2C
F12Q
I2C
PBS

Figure 45

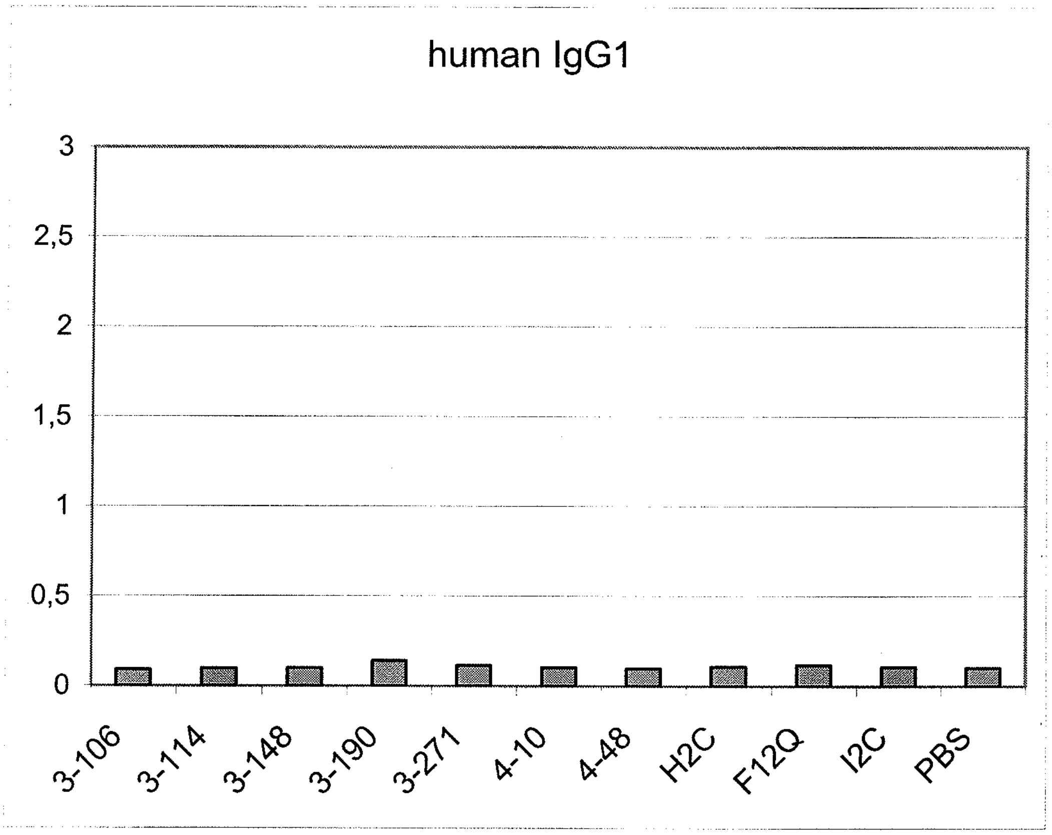
human IgG1
3
2,5
2
1,5
1
0,5
0
3-106
3-114
3-148
3-190
3-271
4-10
4-48
H2C
F12Q
I2C
PBS

## Figure 46A

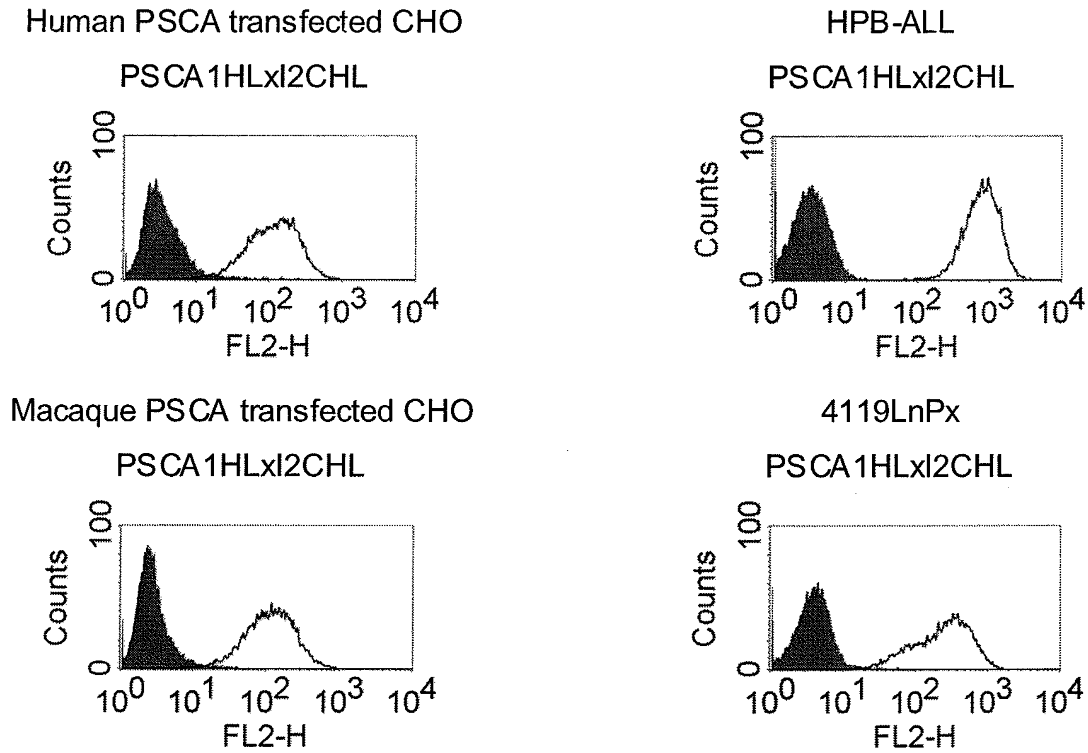


## Figure 46B

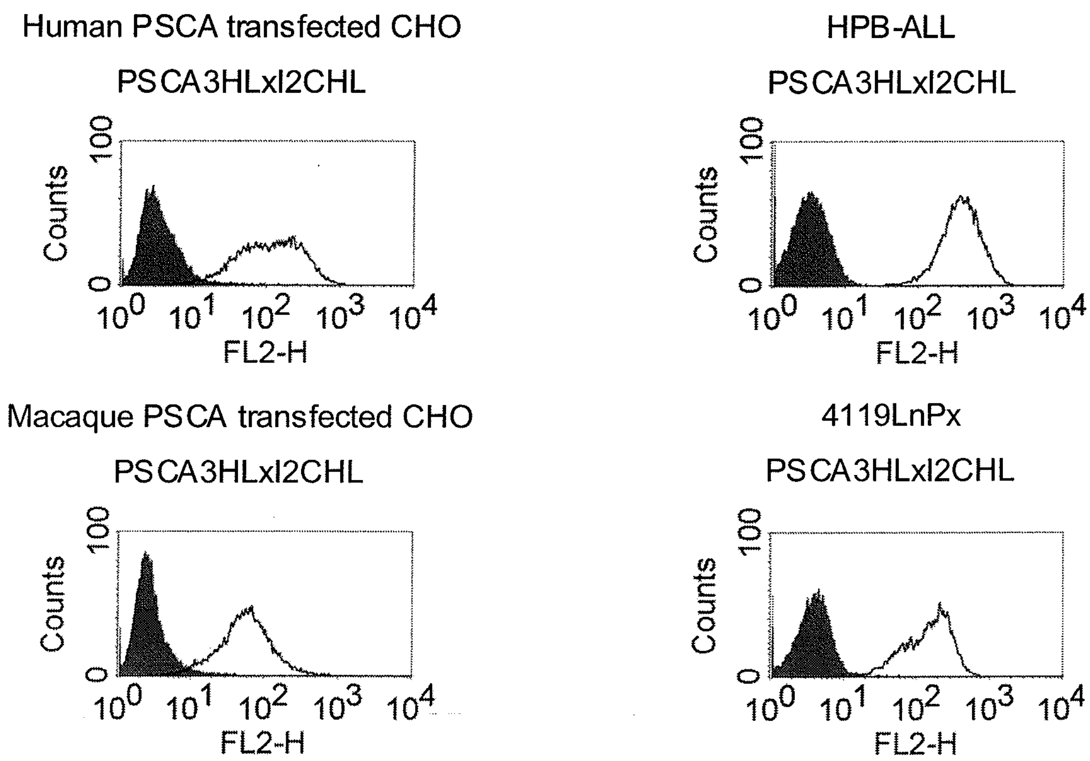

## Figure 46C

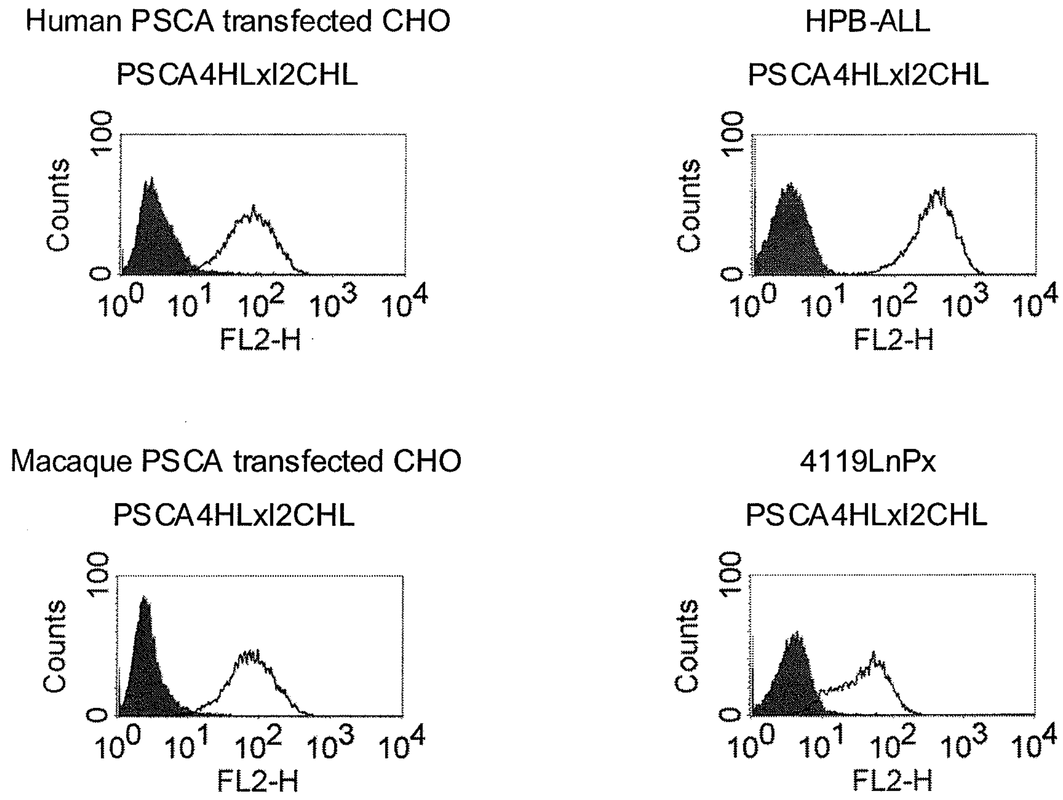


## Figure 46D

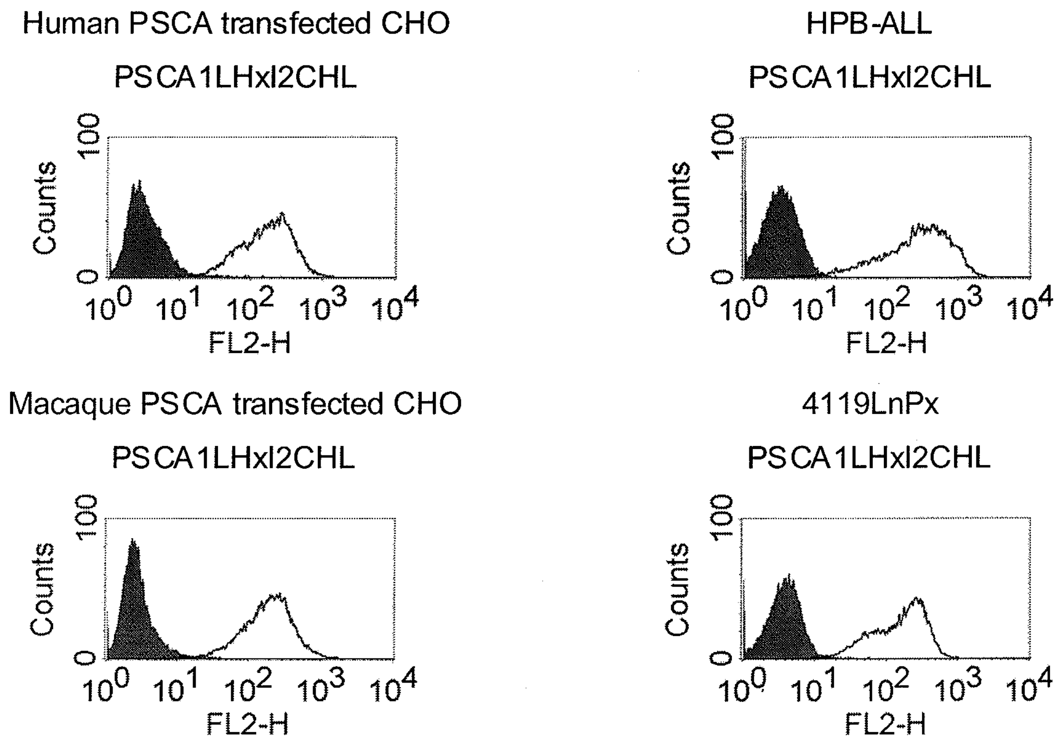

## Figure 46E

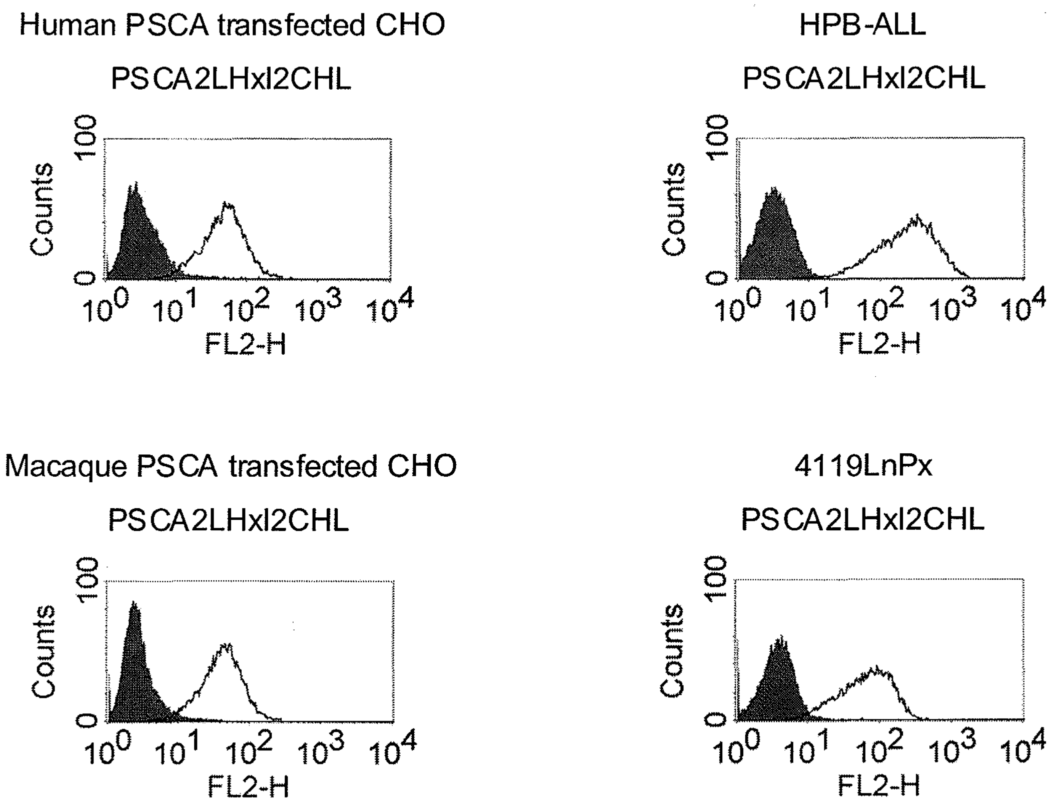


## Figure 46F

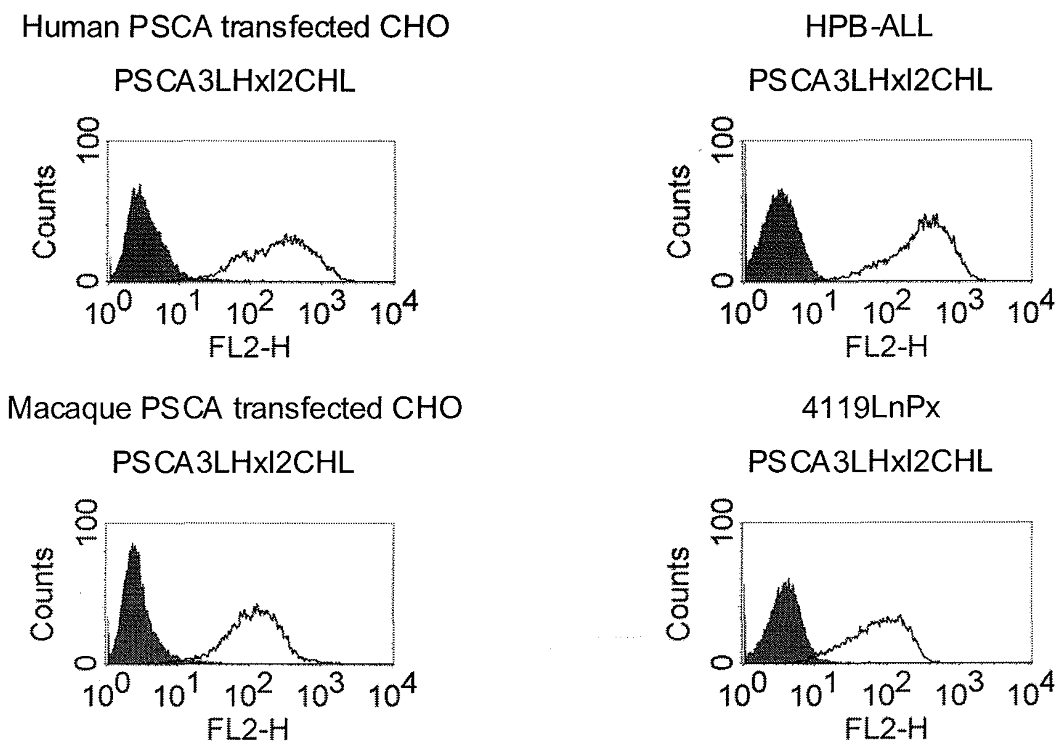

**Figure 46G**

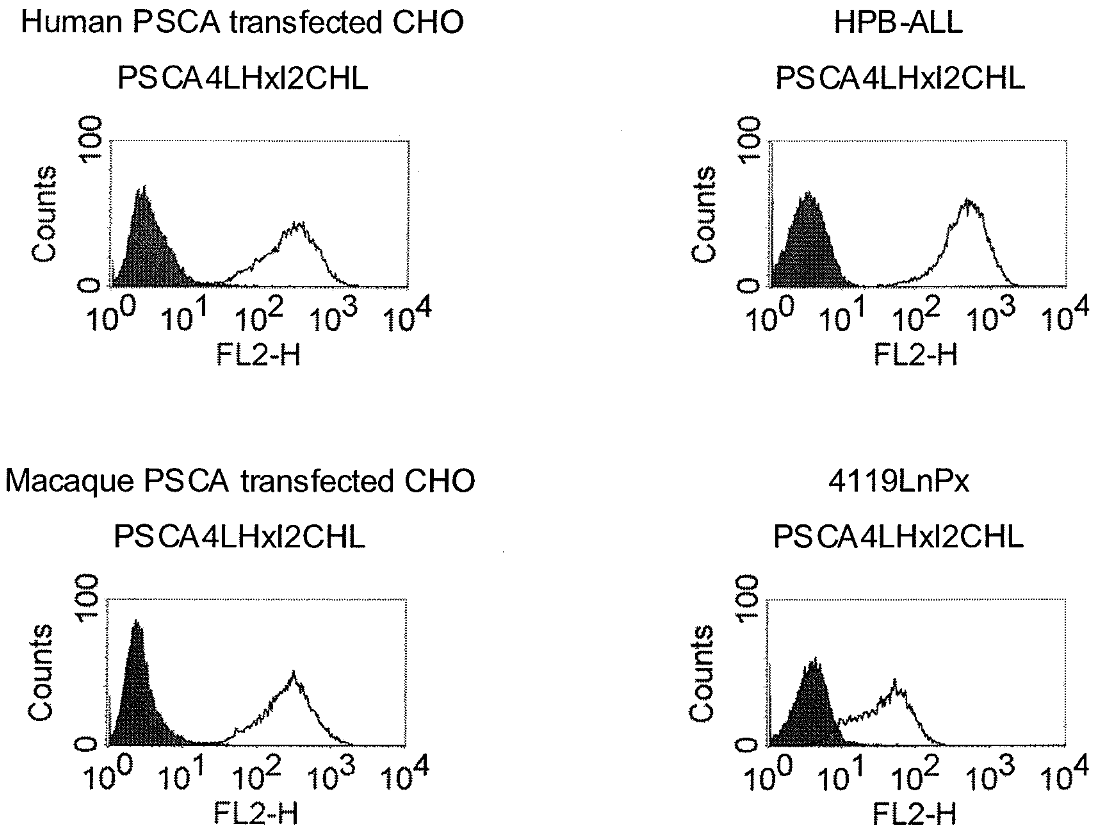
Human PSCA transfected CHO
PSCA4LHxI2CHL
Counts
100
0
10^0 10^1 10^2 10^3 10^4
FL2-H
HPB-ALL
PSCA4LHxI2CHL
Counts
100
0
10^0 10^1 10^2 10^3 10^4
FL2-H
Macaque PSCA transfected CHO
PSCA4LHxI2CHL
Counts
100
0
10^0 10^1 10^2 10^3 10^4
FL2-H
4119LnPx
PSCA4LHxI2CHL
Counts
100
0
10^0 10^1 10^2 10^3 10^4
FL2-H

# Figure 47A

Effector cells: stimulated CD4/CD56 depleted human PBMC

Target cells: CHO transfected with human PSCA

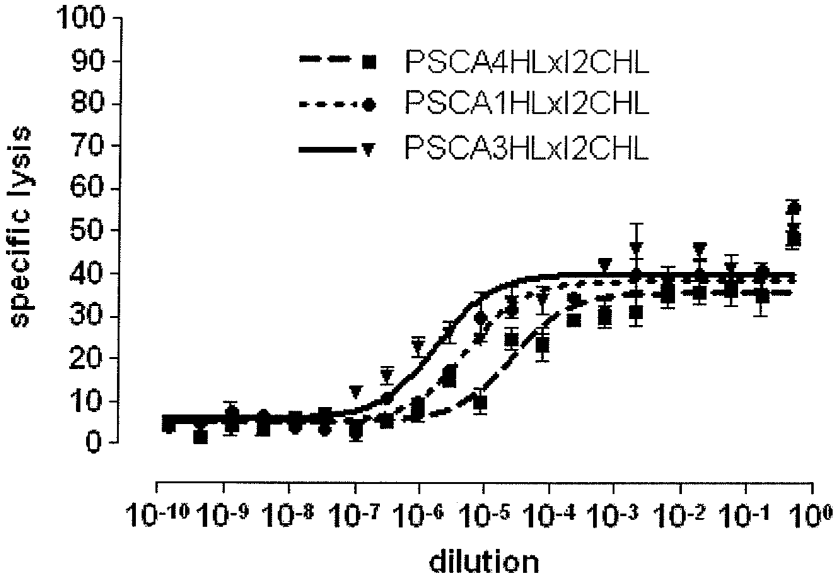


Effector cells: macaque 4119 LnPx

Target cells: CHO transfected with macaque PSCA

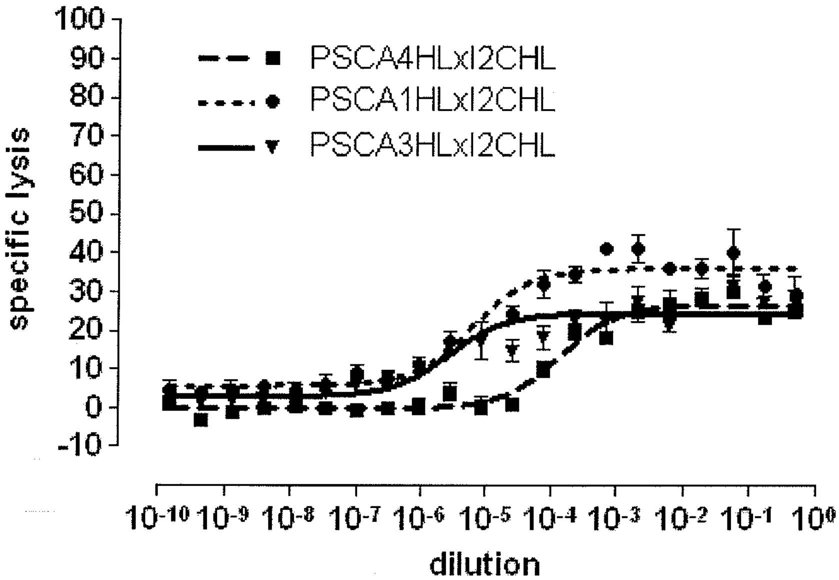

# Figure 47B

Effector cells: stimulated CD4/CD56 depleted human PBMC

Target cells: CHO transfected with human PSCA

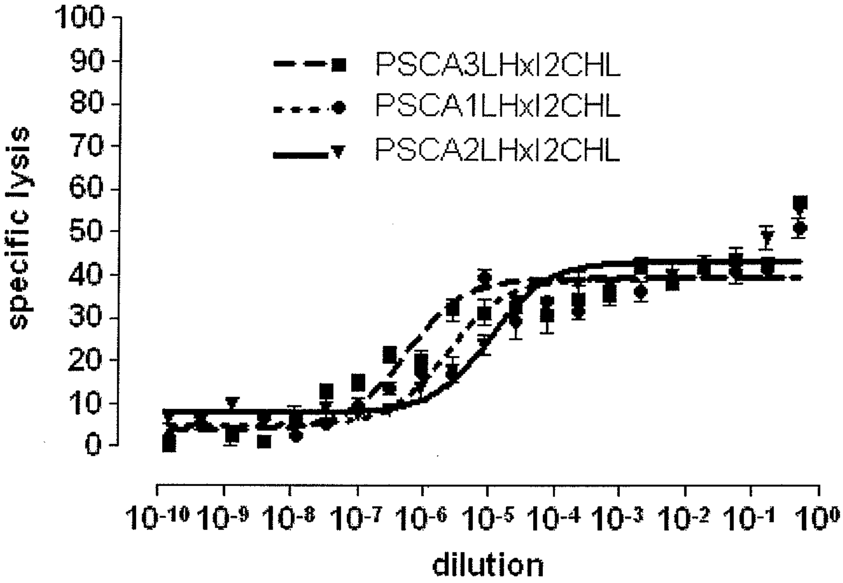


Effector cells: macaque 4119 LnPx

Target cells: CHO transfected with macaque PSCA

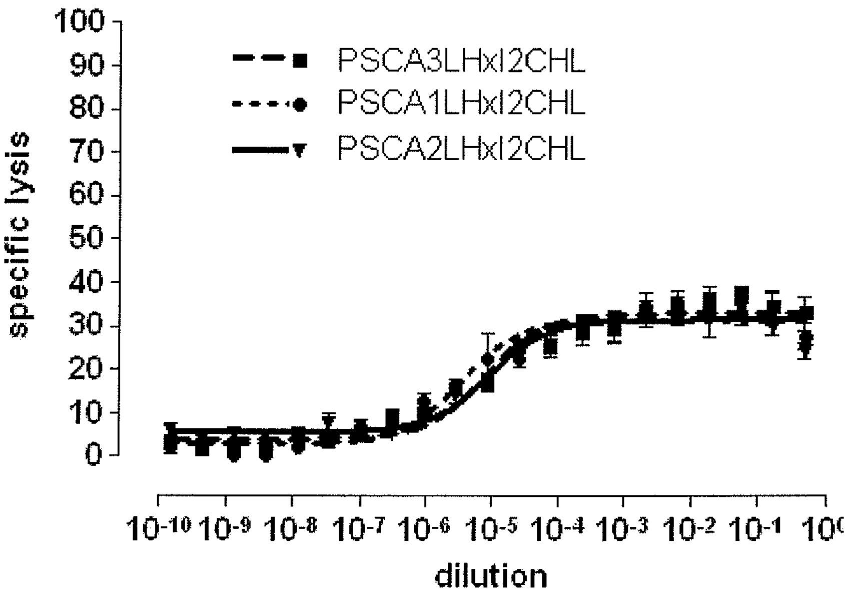

# Figure 47C

Effector cells: stimulated CD4/CD56 depleted human PBMC

Target cells: CHO transfected with human PSCA

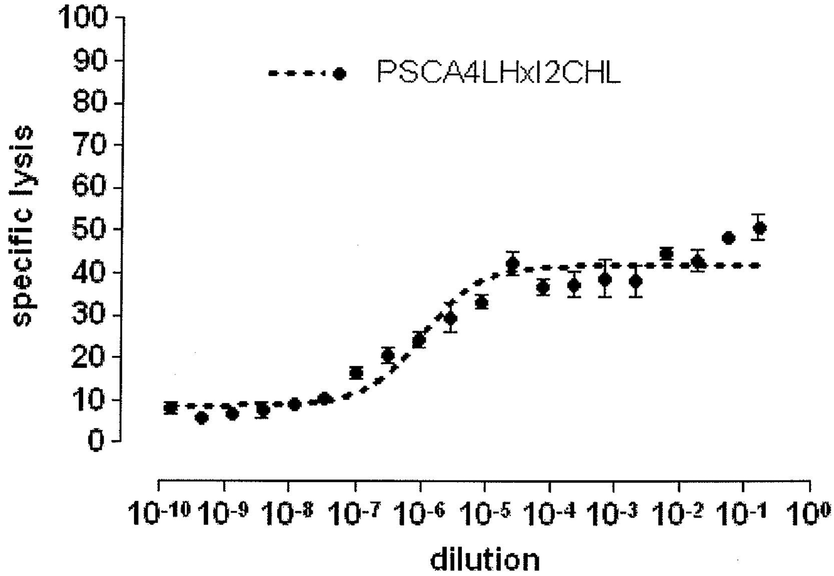


Effector cells: macaque 4119 LnPx

Target cells: CHO transfected with macaque PSCA

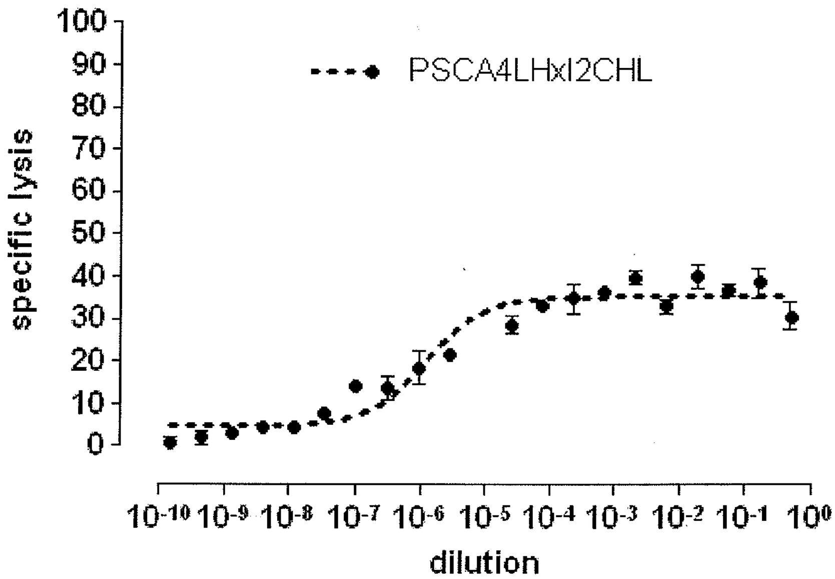

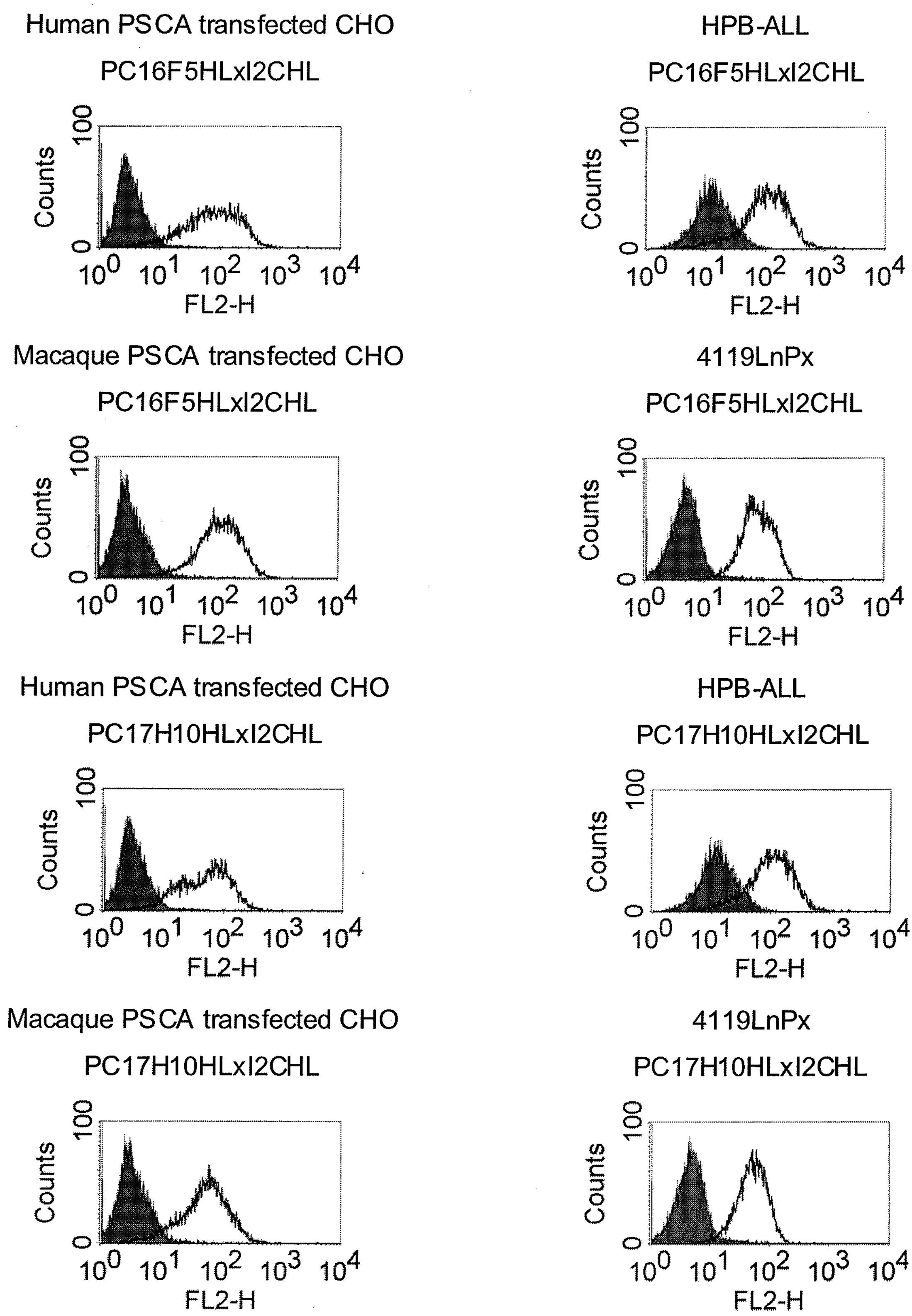
Human PSCA transfected CHO
PC16F5HLxI2CHL
HPB-ALL
PC16F5HLxI2CHL
Macaque PSCA transfected CHO
PC16F5HLxI2CHL
4119LnPx
PC16F5HLxI2CHL
Human PSCA transfected CHO
PC17H10HLxI2CHL
HPB-ALL
PC17H10HLxI2CHL
Macaque PSCA transfected CHO
PC17H10HLxI2CHL
4119LnPx
PC17H10HLxI2CHL
Counts
0
100
$10^0$ $10^1$ $10^2$ $10^3$ $10^4$
FL2-H

**Figure 48a**

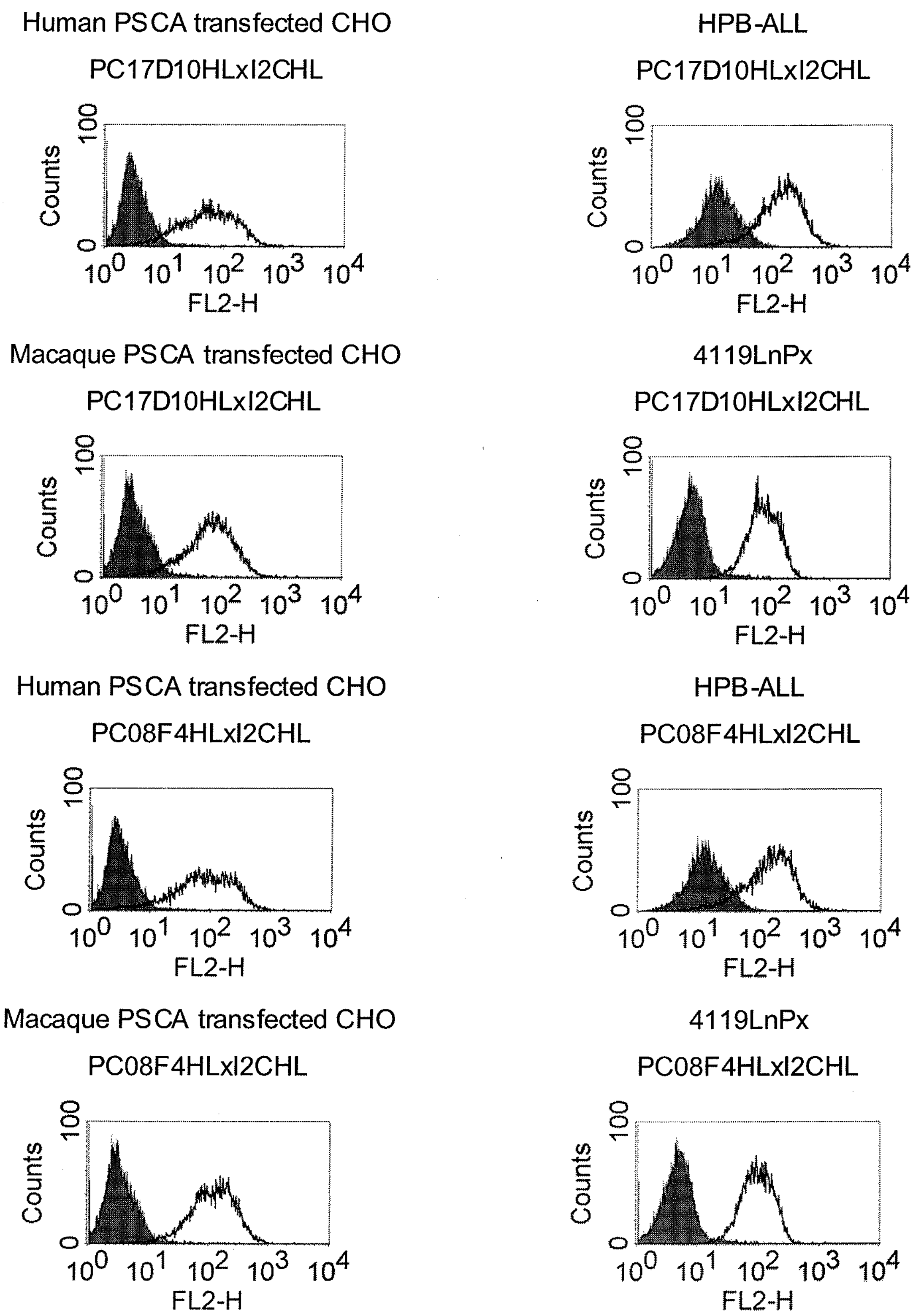
Human PSCA transfected CHO
PC17D10HLxI2CHL
HPB-ALL
PC17D10HLxI2CHL
Macaque PSCA transfected CHO
PC17D10HLxI2CHL
4119LnPx
PC17D10HLxI2CHL
Human PSCA transfected CHO
PC08F4HLxI2CHL
HPB-ALL
PC08F4HLxI2CHL
Macaque PSCA transfected CHO
PC08F4HLxI2CHL
4119LnPx
PC08F4HLxI2CHL
Counts
0
100
FL2-H
$10^0$ $10^1$ $10^2$ $10^3$ $10^4$

**Figure 48b**

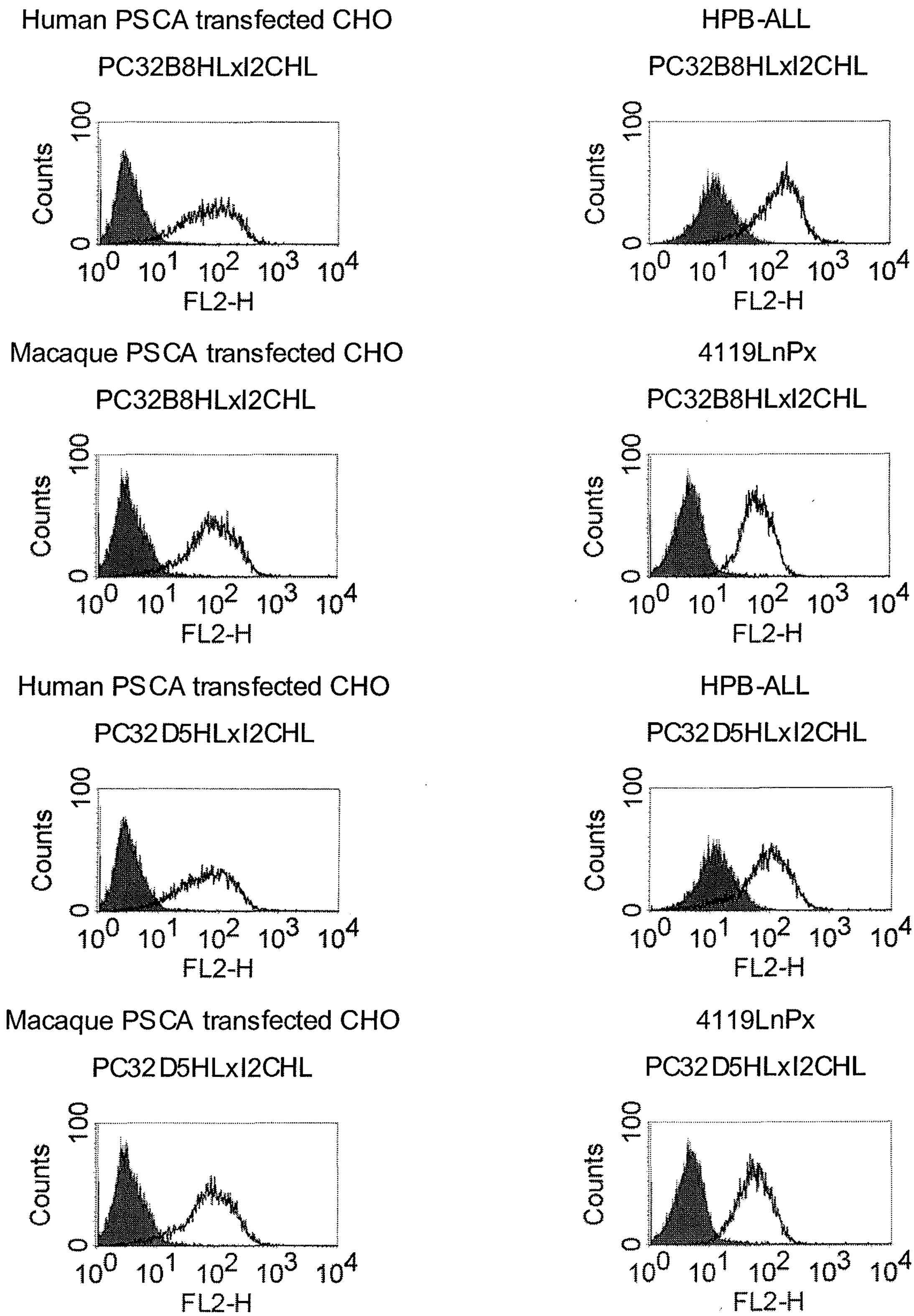
Human PSCA transfected CHO
PC32B8HLxI2CHL
HPB-ALL
PC32B8HLxI2CHL
Macaque PSCA transfected CHO
PC32B8HLxI2CHL
4119LnPx
PC32B8HLxI2CHL
Human PSCA transfected CHO
PC32D5HLxI2CHL
HPB-ALL
PC32D5HLxI2CHL
Macaque PSCA transfected CHO
PC32D5HLxI2CHL
4119LnPx
PC32D5HLxI2CHL
Counts
0
100
$10^0$ $10^1$ $10^2$ $10^3$ $10^4$
FL2-H

**Figure 48c**

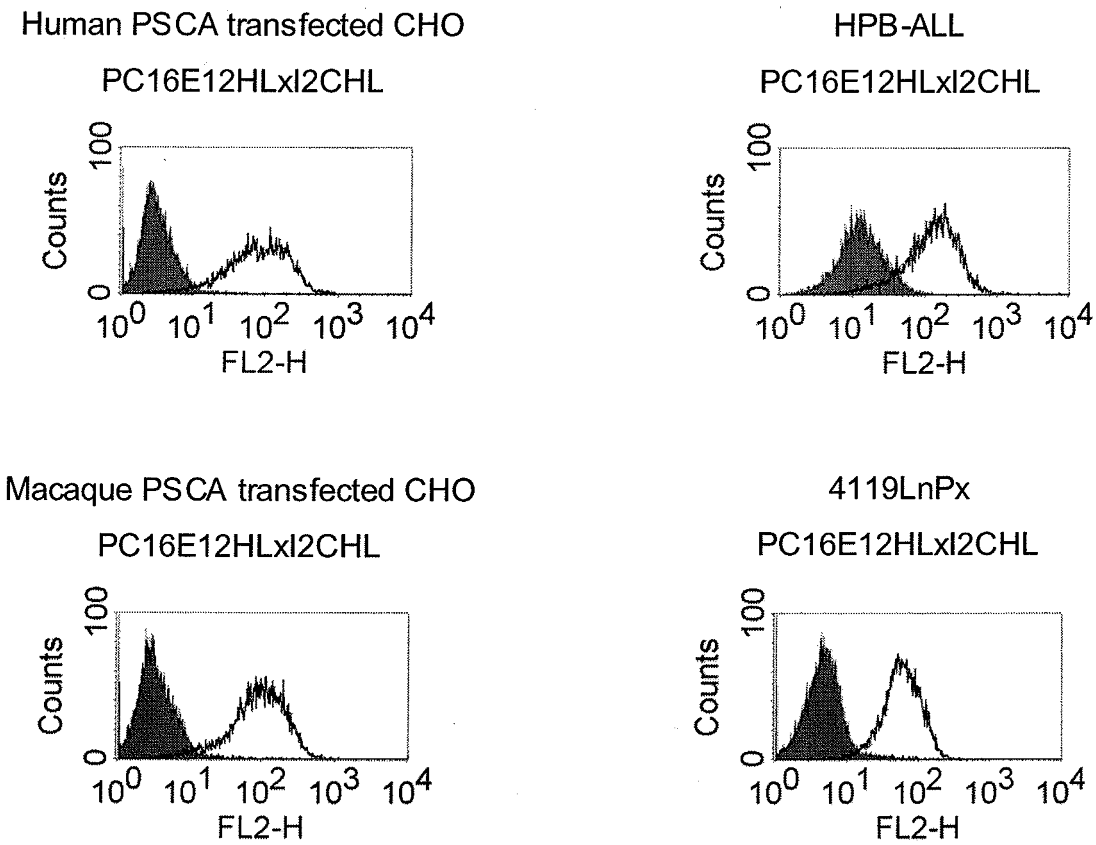
Human PSCA transfected CHO
PC16E12HLxI2CHL
HPB-ALL
PC16E12HLxI2CHL
Macaque PSCA transfected CHO
PC16E12HLxI2CHL
4119LnPx
PC16E12HLxI2CHL
Counts
0
100
$10^0$ $10^1$ $10^2$ $10^3$ $10^4$
FL2-H

**Figure 48d**

Effector T cells: stimulated CD4/CD56 depleted human PBMC

Target cells: CHO transfected with human PSCA

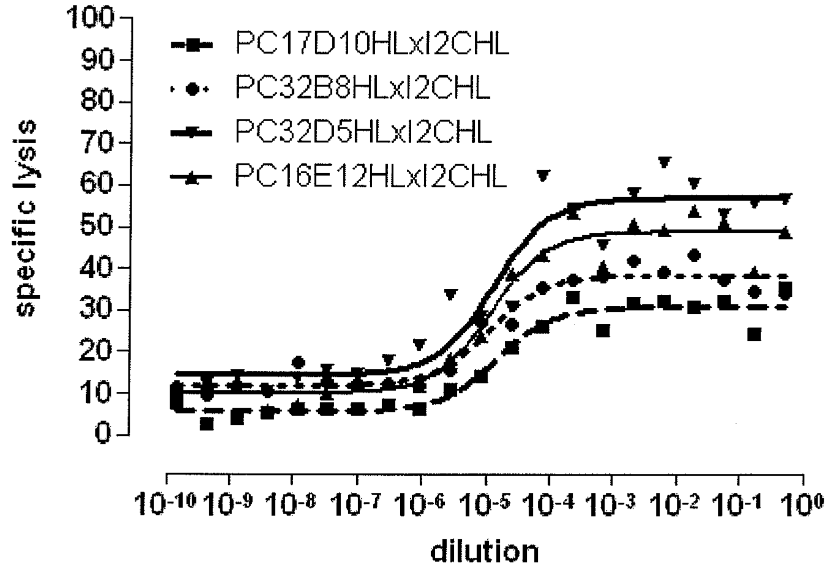


Effector T cells: macaque 4119 LnPx

Target cells: CHO transfected with macaque PSCA

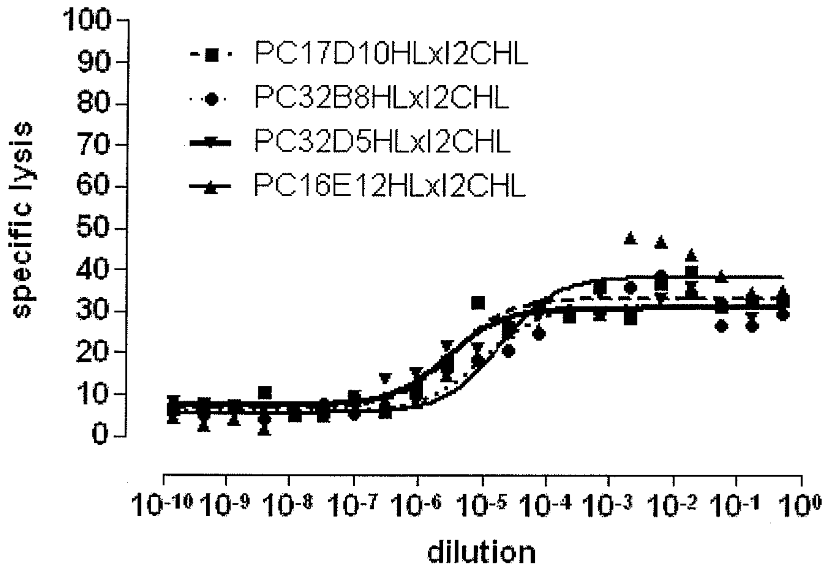


**Figure 49a**

Effector T cells: stimulated CD4/CD56 depleted human PBMC

Target cells: CHO transfected with human PSCA

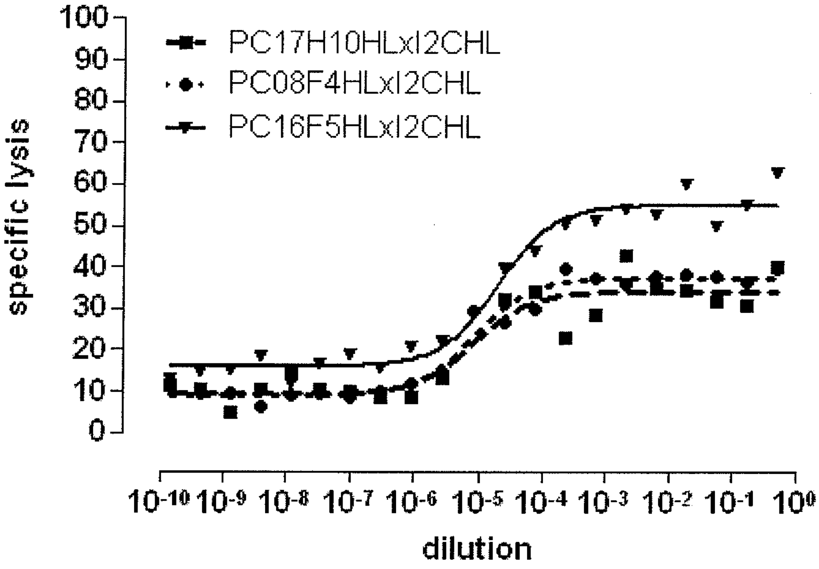


Effector T cells: macaque 4119 LnPx

Target cells: CHO transfected with macaque PSCA

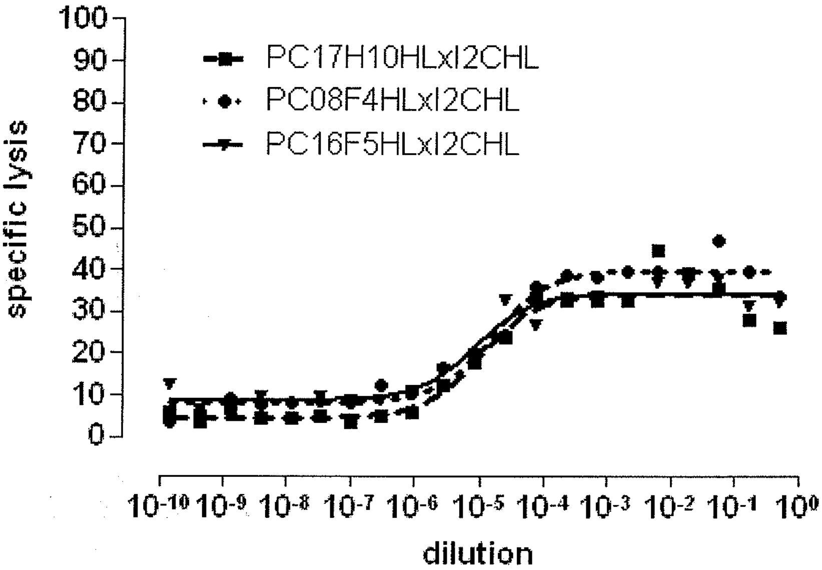


**Figure 49b**

Figure 50a

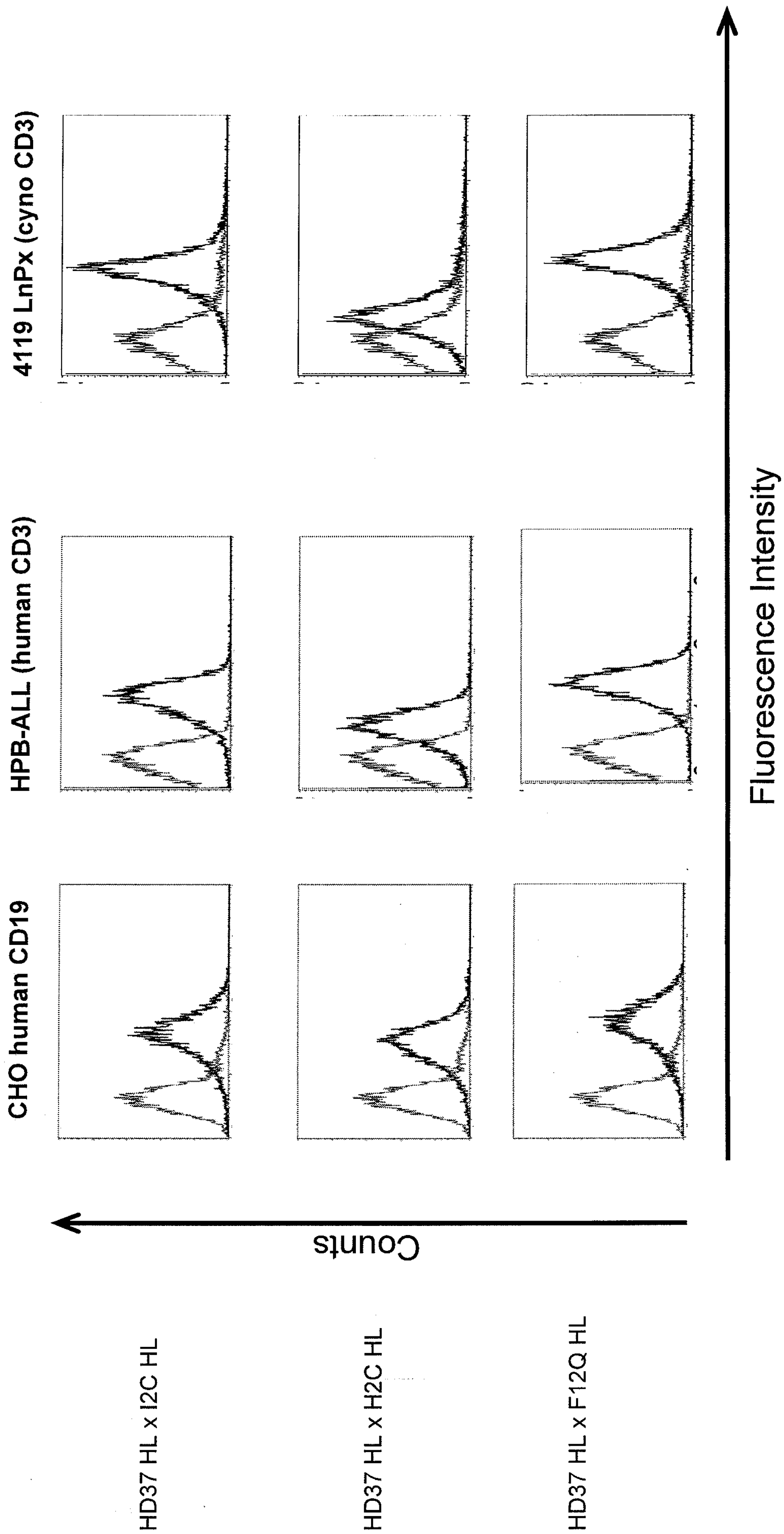

CHO human CD19
HPB-ALL (human CD3)
4119 LnPx (cyno CD3)
HD37 HL x I2C HL
HD37 HL x H2C HL
HD37 HL x F12Q HL
Counts
Fluorescence Intensity

Figure 50b

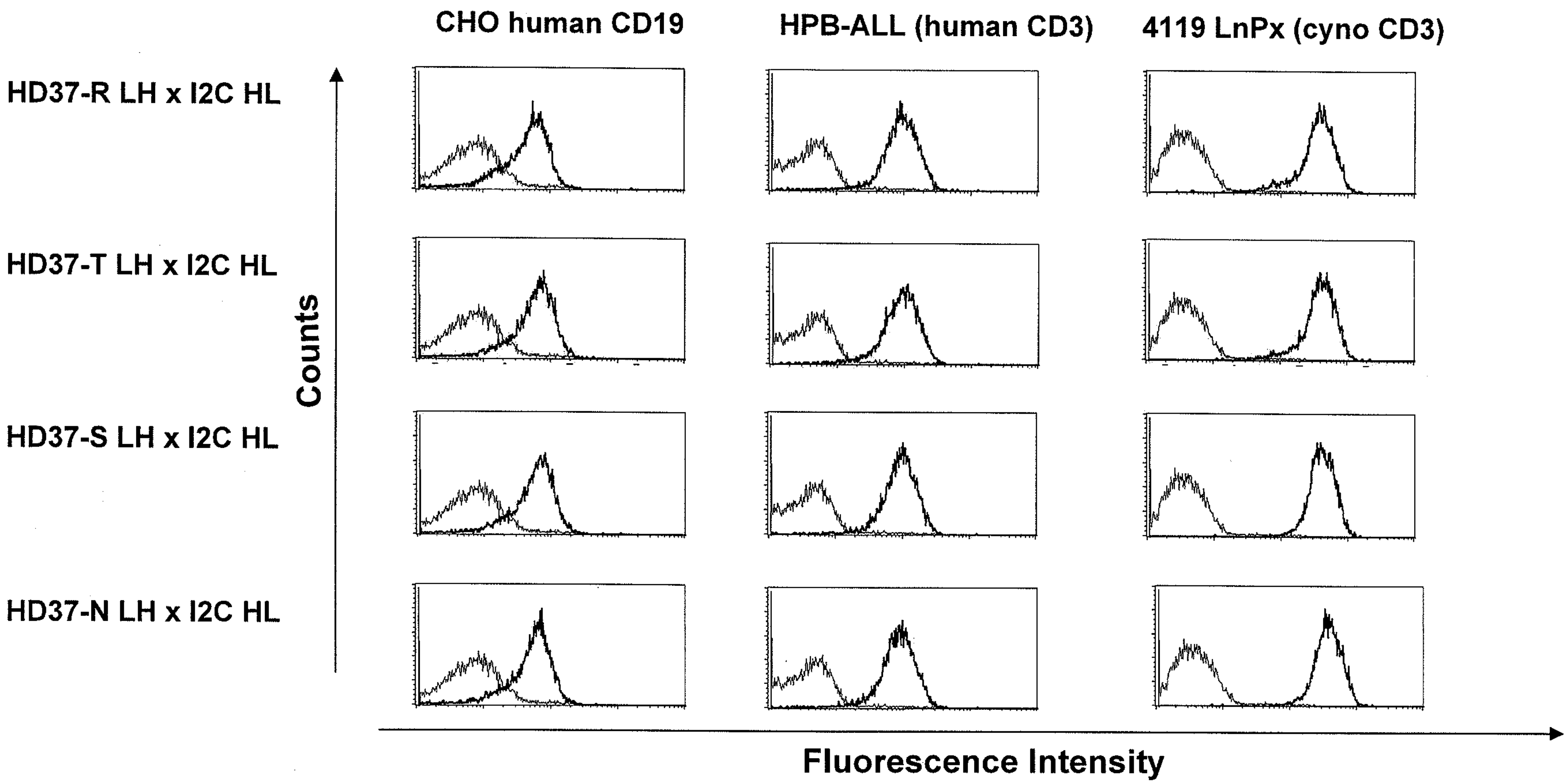
CHO human CD19
HPB-ALL (human CD3)
4119 LnPx (cyno CD3)
HD37-R LH x I2C HL
HD37-T LH x I2C HL
HD37-S LH x I2C HL
HD37-N LH x I2C HL
Counts
Fluorescence Intensity

Figure 50c

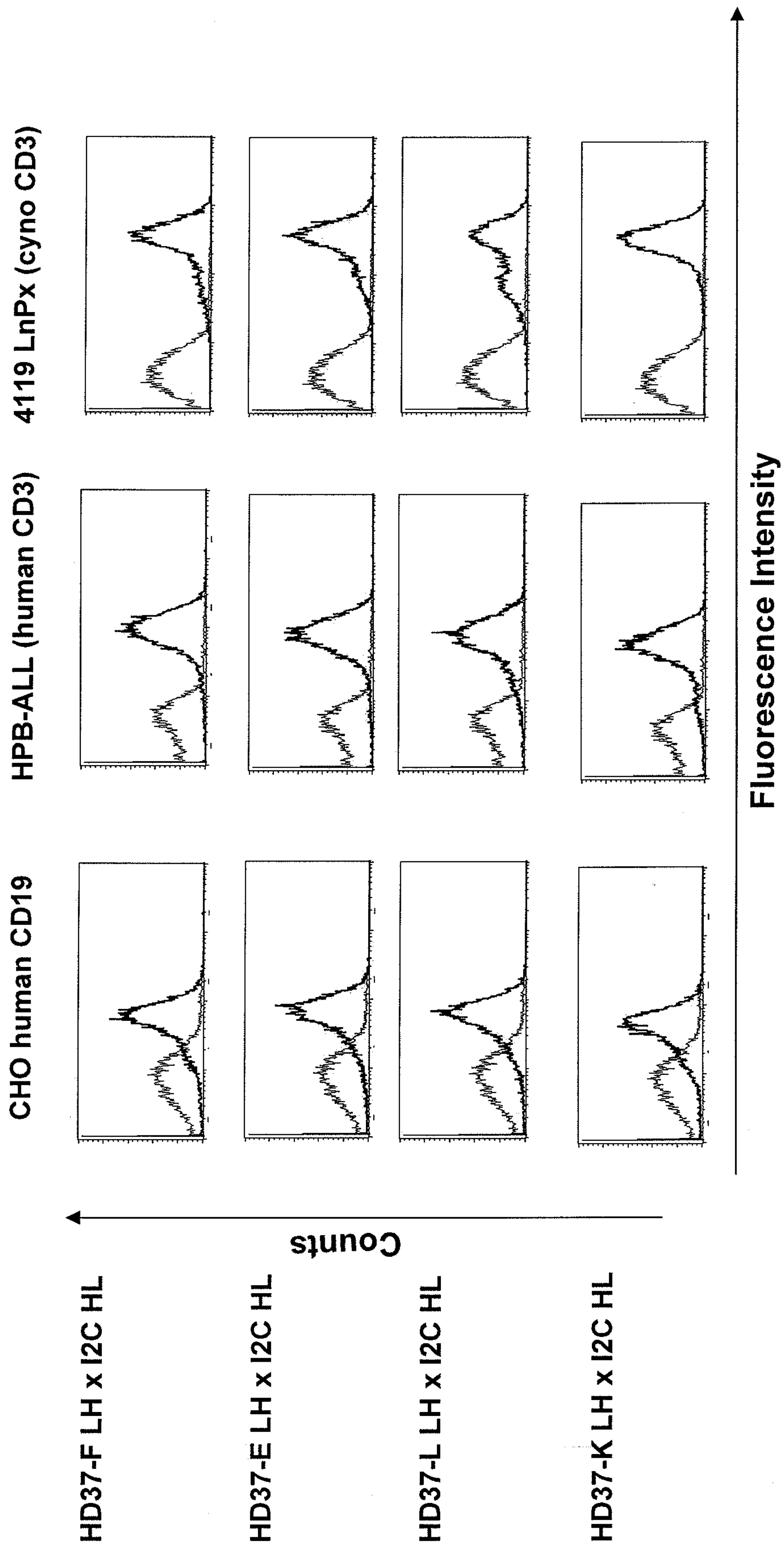
CHO human CD19
HPB-ALL (human CD3)
4119 LnPx (cyno CD3)
HD37-F LH x I2C HL
HD37-E LH x I2C HL
HD37-L LH x I2C HL
HD37-K LH x I2C HL
Counts
Fluorescence Intensity

Figure 50d

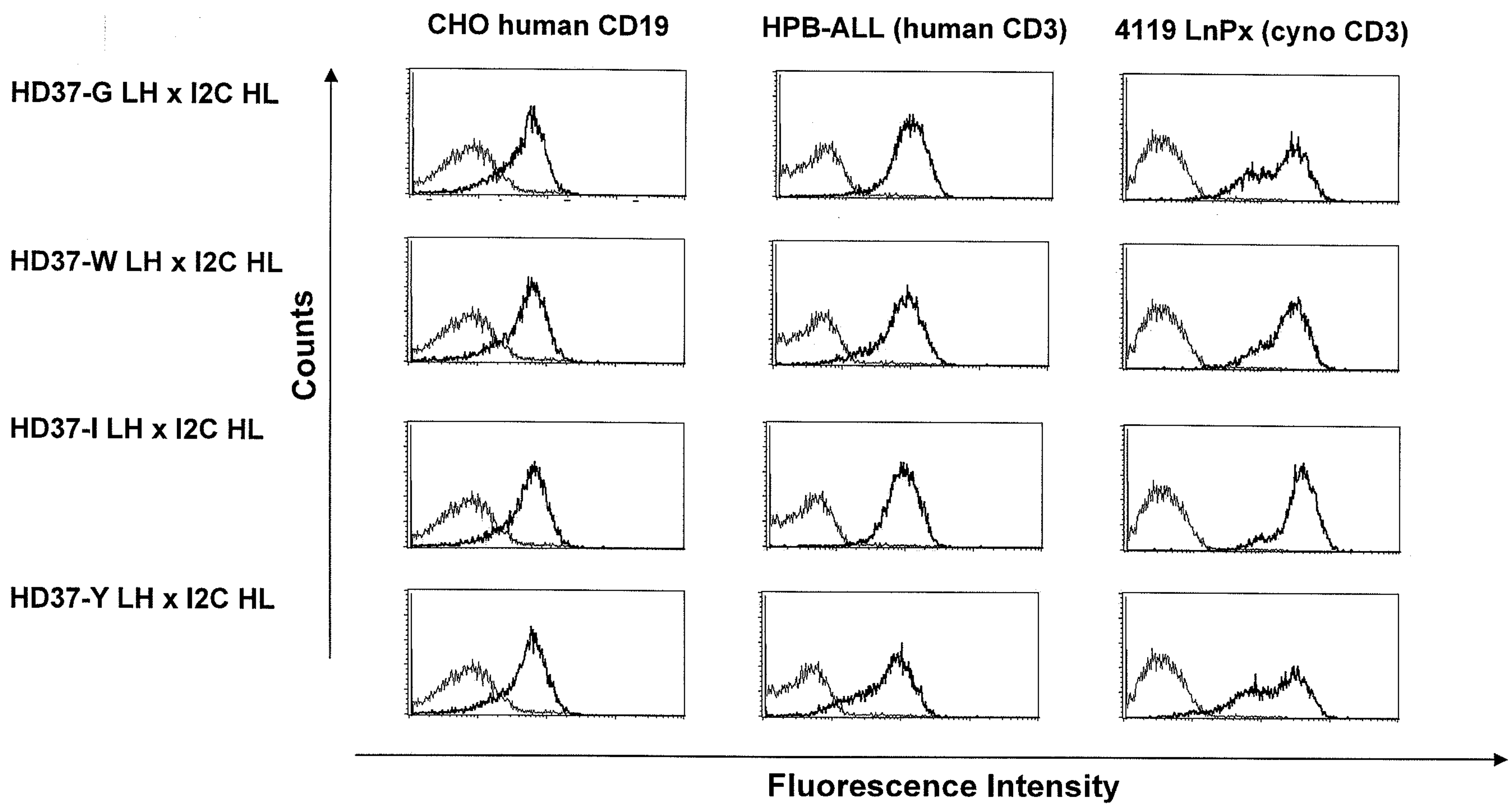
CHO human CD19
HPB-ALL (human CD3)
4119 LnPx (cyno CD3)
HD37-G LH x I2C HL
HD37-W LH x I2C HL
HD37-I LH x I2C HL
HD37-Y LH x I2C HL
Counts
Fluorescence Intensity

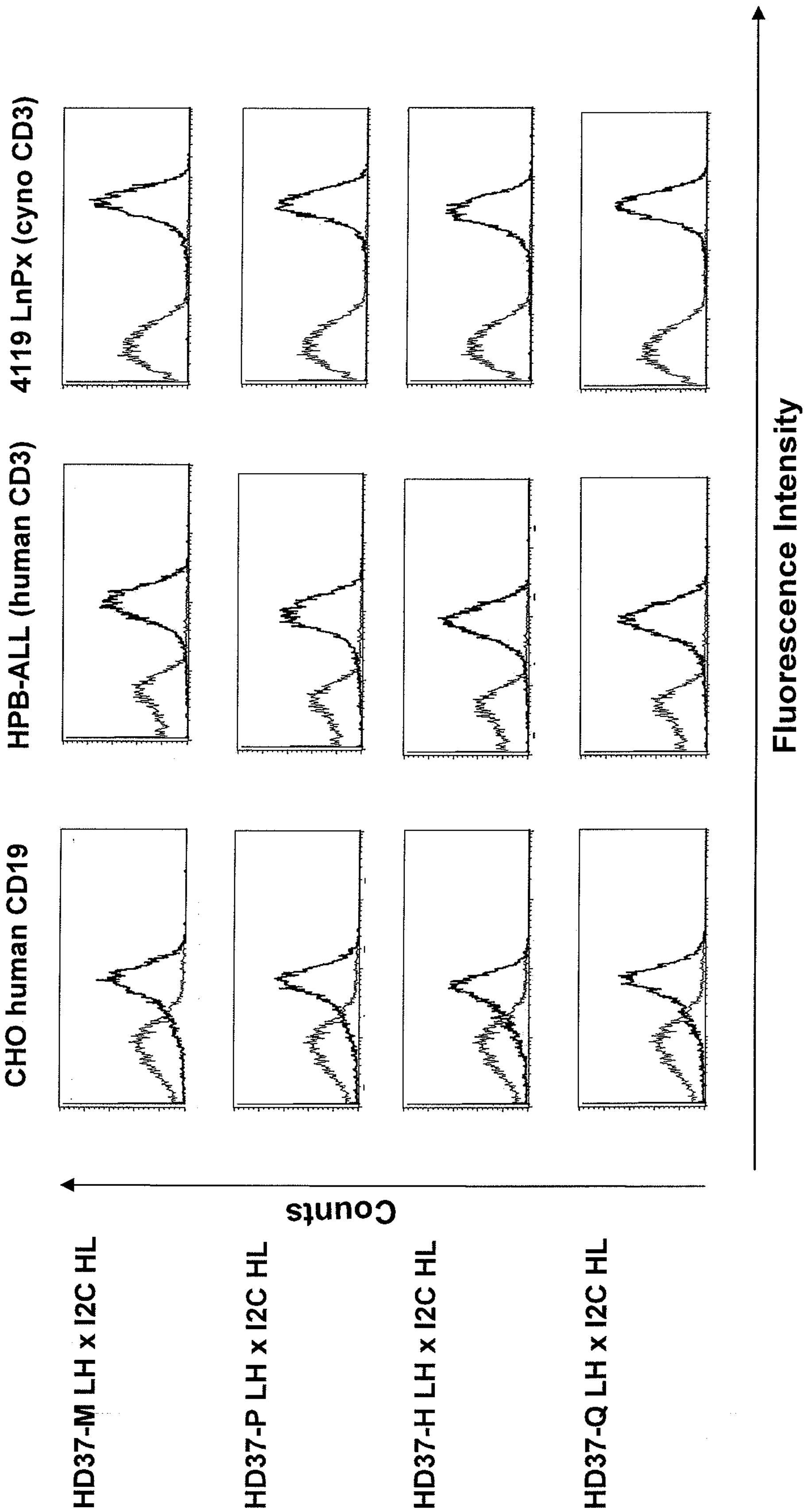

CHO human CD19
HPB-ALL (human CD3)
4119 LnPx (cyno CD3)
HD37-M LH x I2C HL
HD37-P LH x I2C HL
HD37-H LH x I2C HL
HD37-Q LH x I2C HL
Counts
Fluorescence Intensity


Figure 50e

Figure 50f

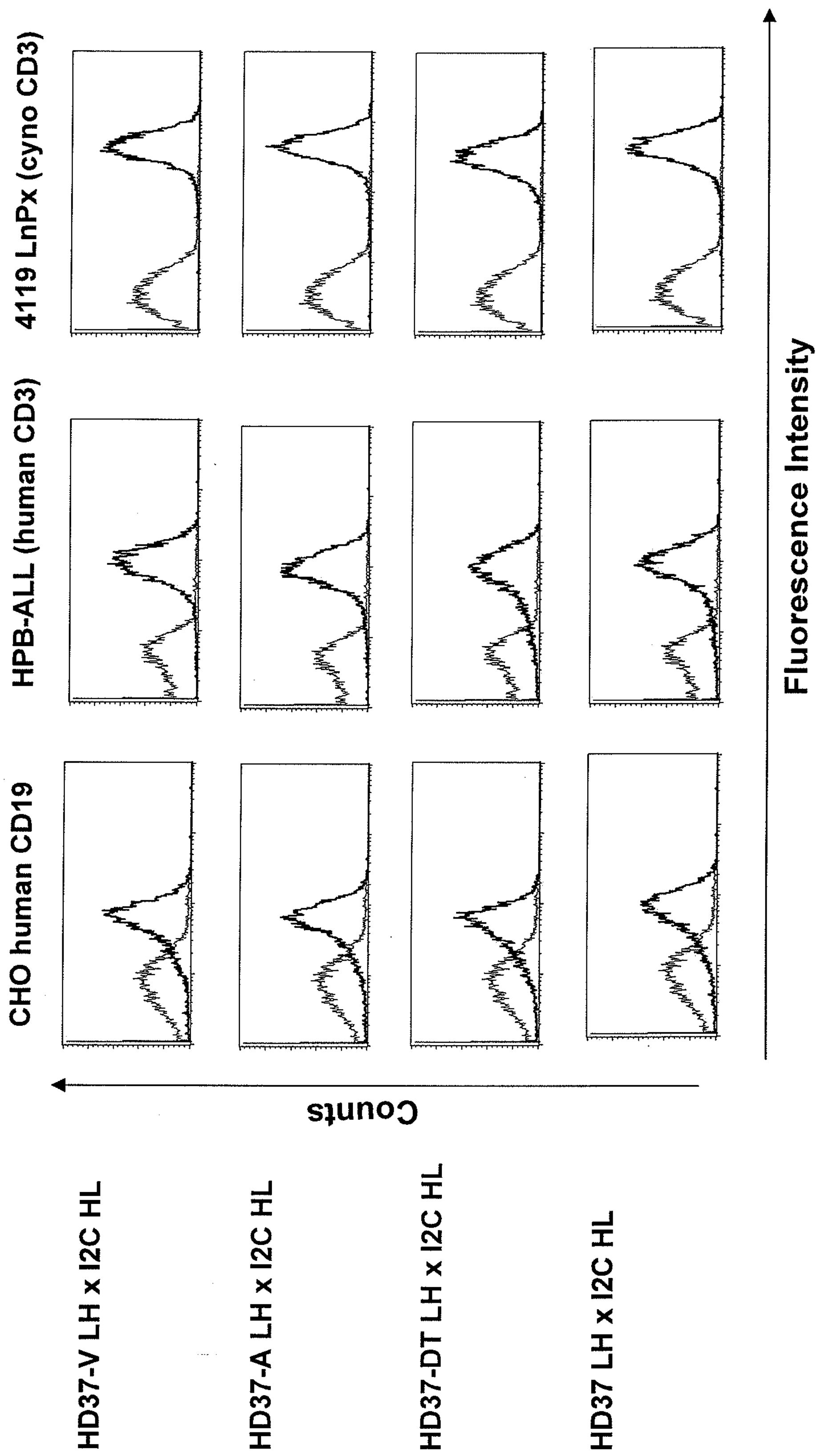
CHO human CD19
HPB-ALL (human CD3)
4119 LnPx (cyno CD3)
Counts
Fluorescence Intensity
HD37-V LH x I2C HL
HD37-A LH x I2C HL
HD37-DT LH x I2C HL
HD37 LH x I2C HL

Figure 50g

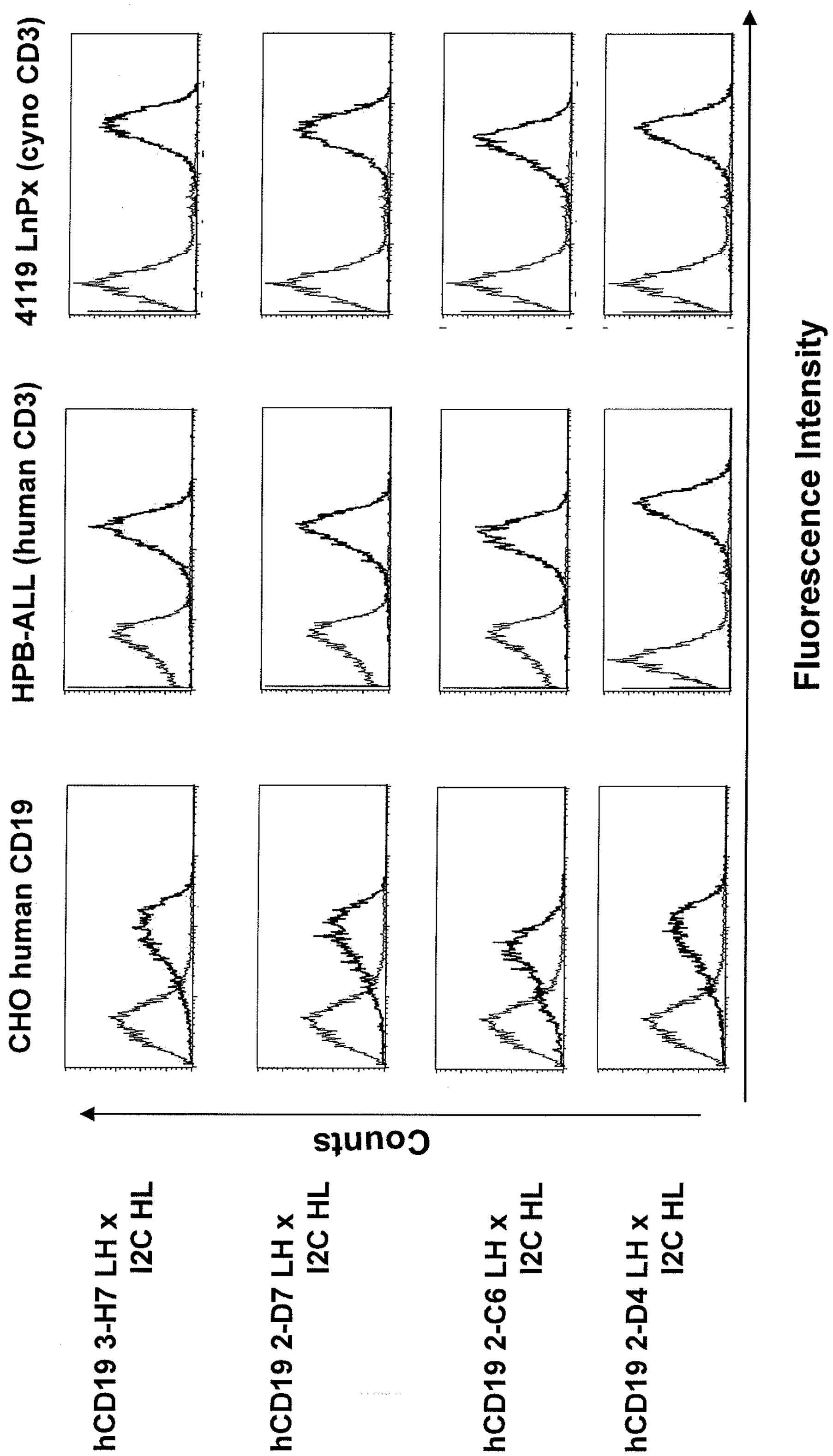
CHO human CD19
HPB-ALL (human CD3)
4119 LnPx (cyno CD3)
hCD19 3-H7 LH x I2C HL
hCD19 2-D7 LH x I2C HL
hCD19 2-C6 LH x I2C HL
hCD19 2-D4 LH x I2C HL
Counts
Fluorescence Intensity

Figure 50h

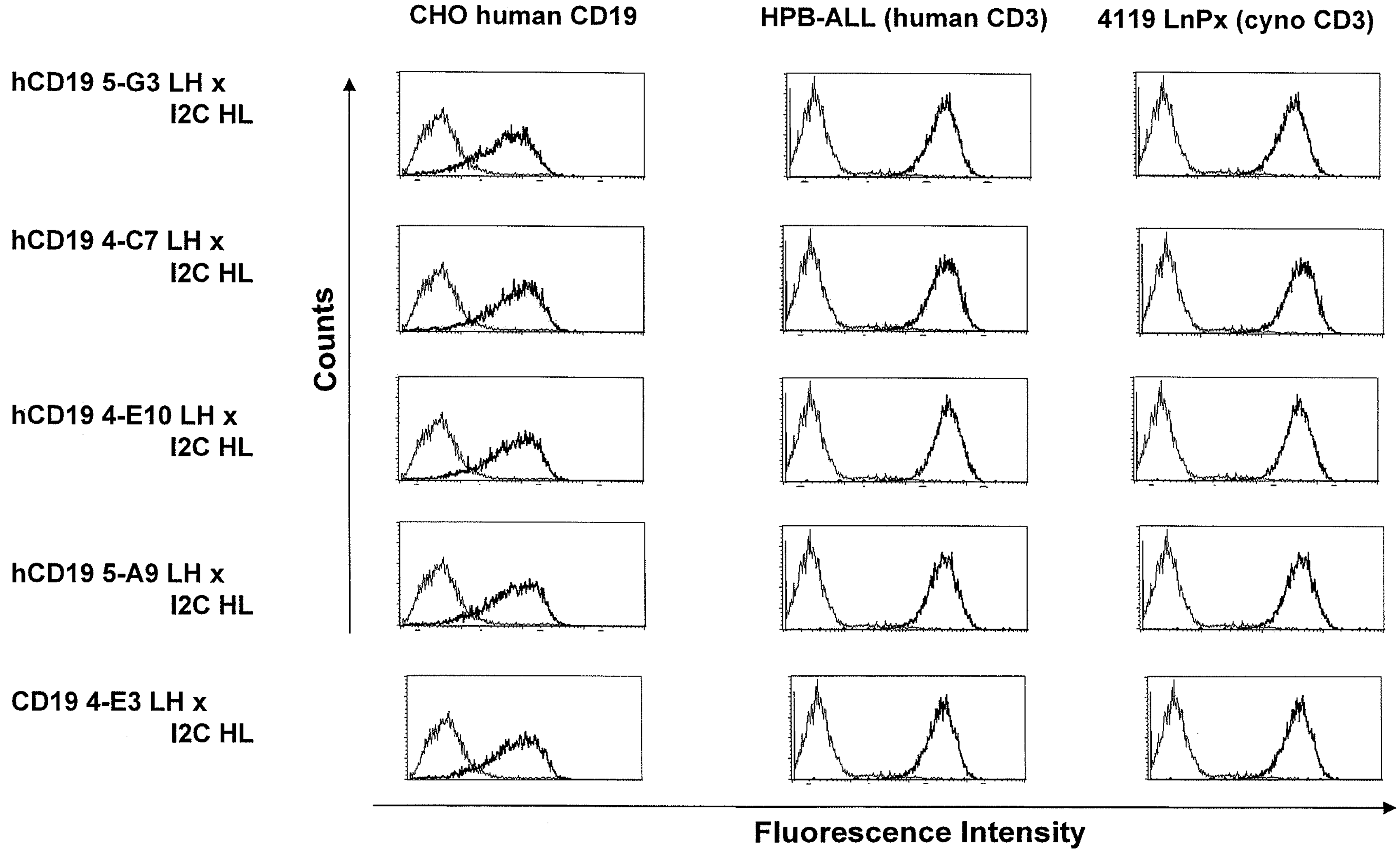
CHO human CD19
HPB-ALL (human CD3)
4119 LnPx (cyno CD3)
hCD19 5-G3 LH x I2C HL
hCD19 4-C7 LH x I2C HL
hCD19 4-E10 LH x I2C HL
hCD19 5-A9 LH x I2C HL
CD19 4-E3 LH x I2C HL
Counts
Fluorescence Intensity

Figure 50i

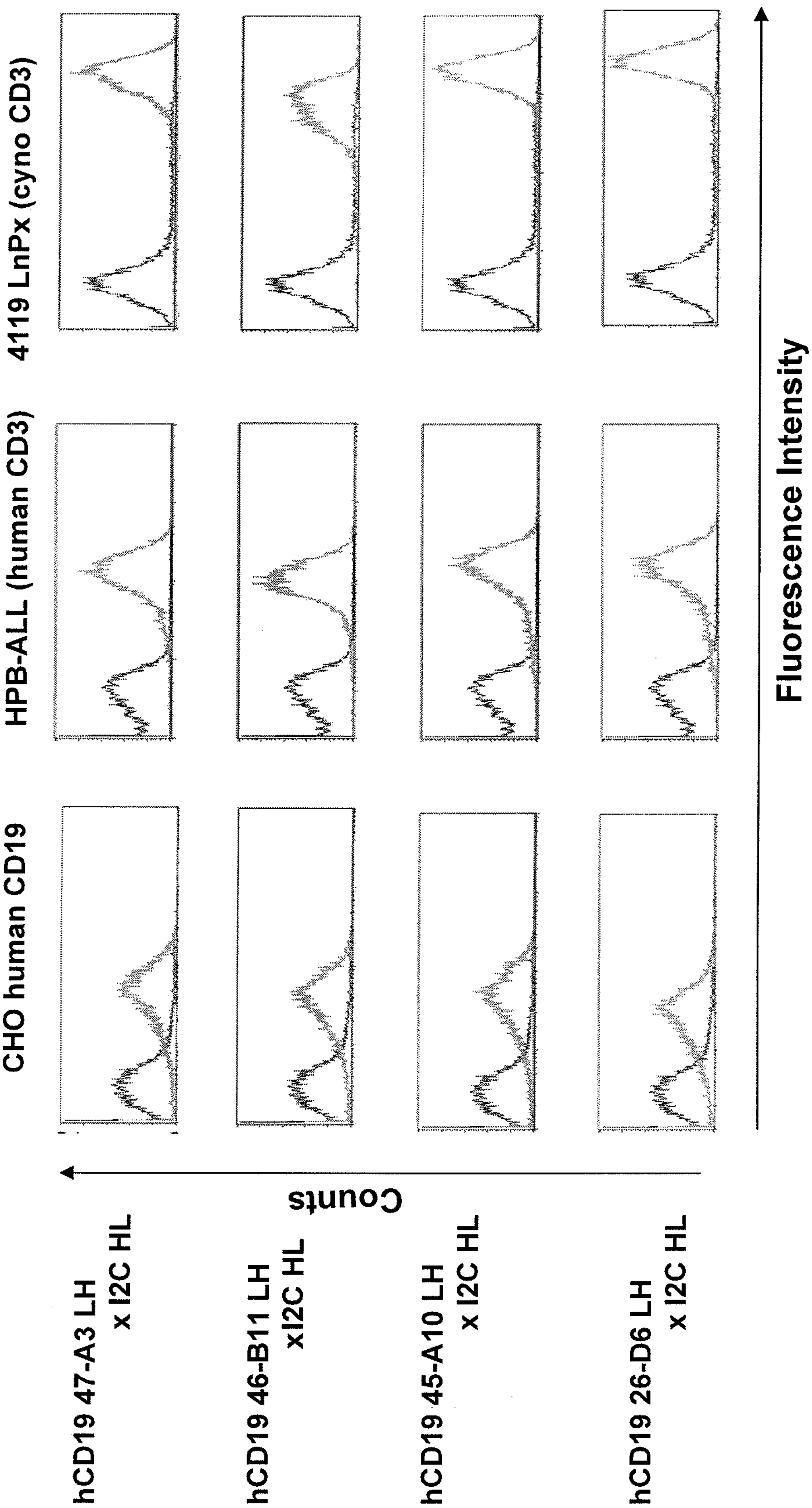
CHO human CD19
HPB-ALL (human CD3)
4119 LnPx (cyno CD3)
hCD19 47-A3 LH x I2C HL
hCD19 46-B11 LH x I2C HL
hCD19 45-A10 LH x I2C HL
hCD19 26-D6 LH x I2C HL
Counts
Fluorescence Intensity

Figure: 51(1)

A) Effector cells: CD56 depleted unstimulated human PBMC
Target cells: CHO transfected with human CD19

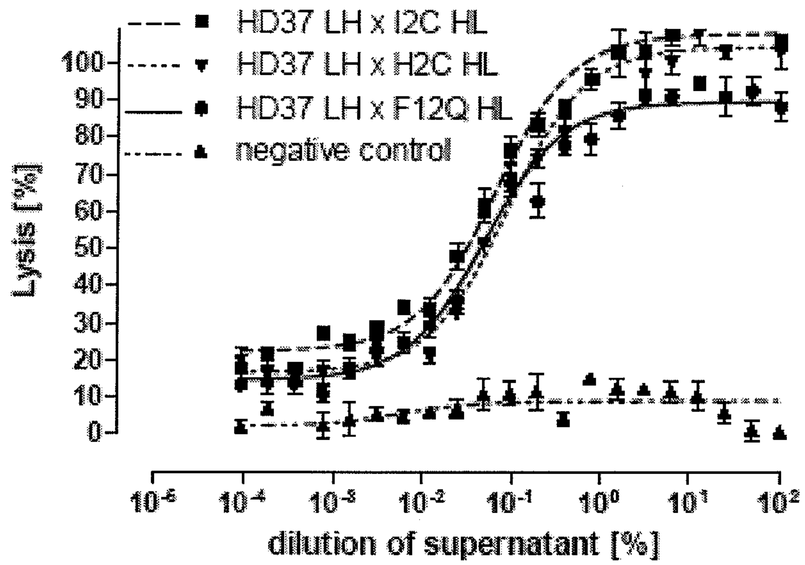


B) Effector cells: 4119LnPx
Target cells: CHO transfected with human CD19

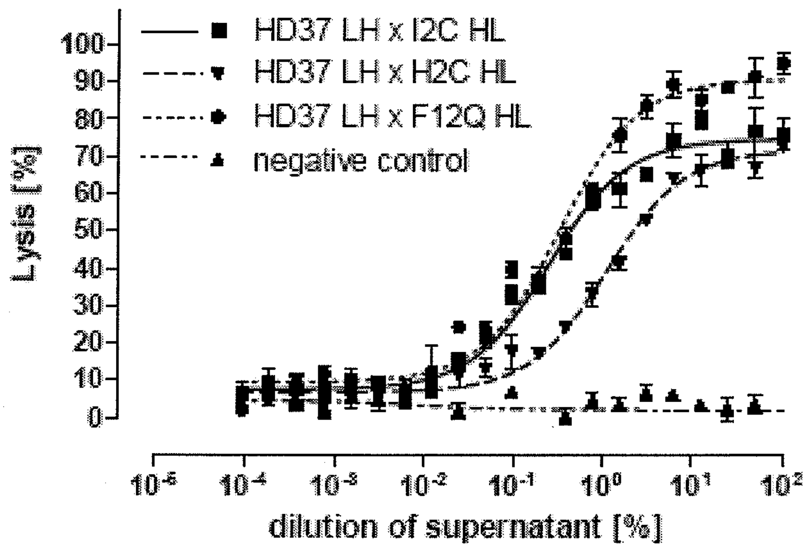

Figure: 51(2)

C) Effector cells: stimulated CD4/CD56 depleted human PBMC
Target cells: CHO transfected with human CD19

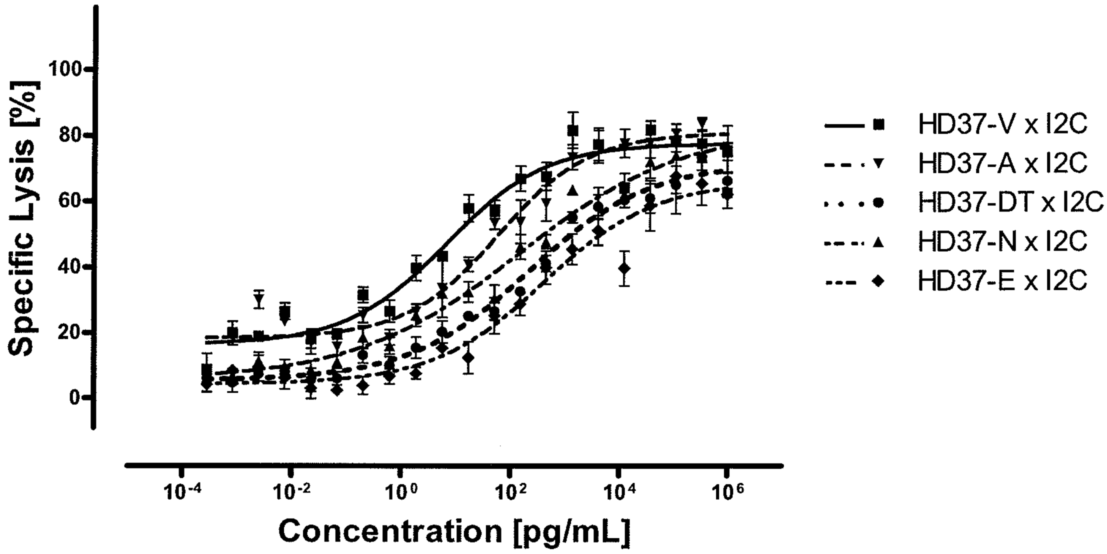


D) Effector cells: stimulated CD4/CD56 depleted human PBMC
Target cells: CHO transfected with human CD19

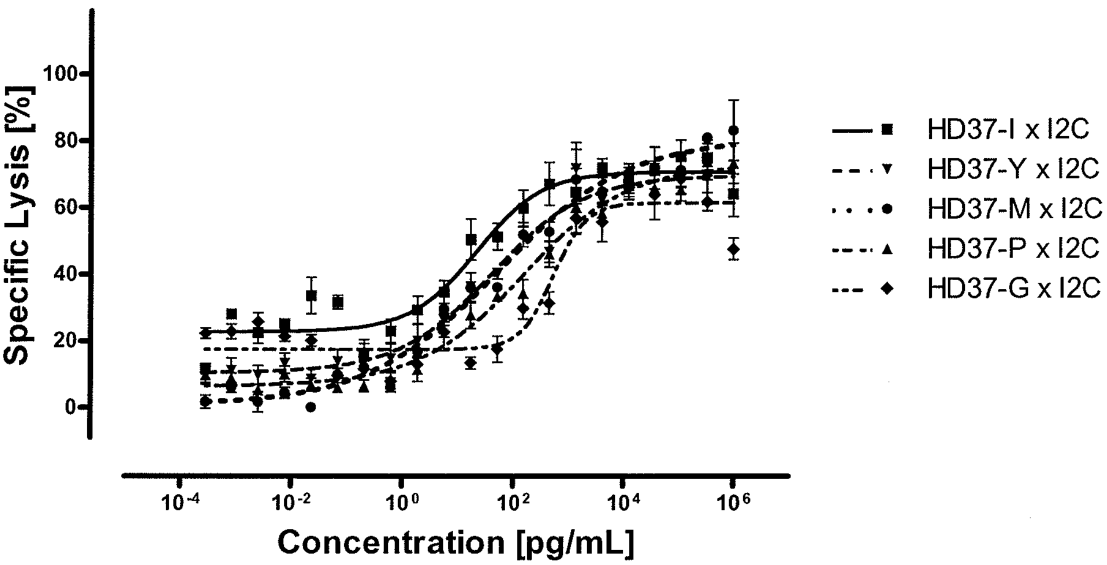

Figure: 51(3)

E) Effector cells: stimulated CD4/CD56 depleted human PBMC
Target cells: CHO transfected with human CD19

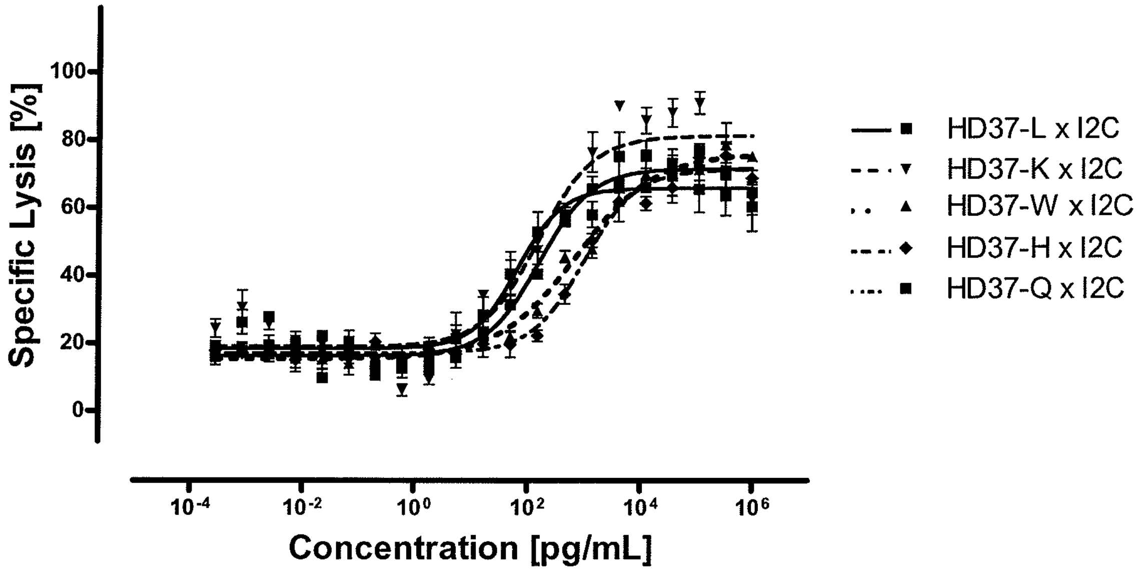


F) Effector cells: stimulated CD4/CD56 depleted human PBMC
Target cells: CHO transfected with human CD19

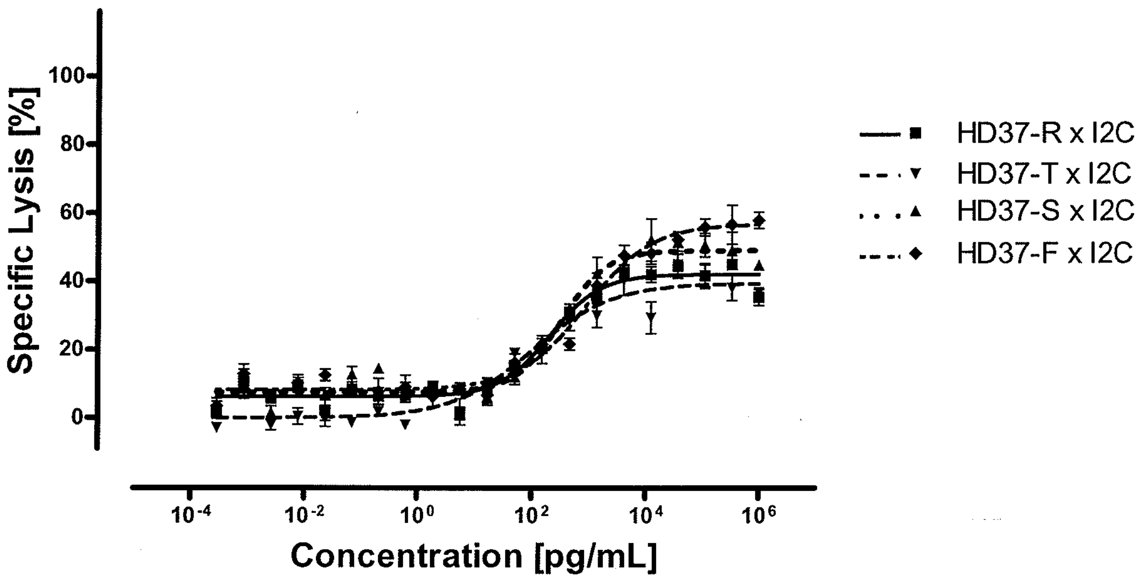

Figure: 51(4)

G) Effector cells: stimulated CD4/CD56 depleted human PBMC
Target cells: CHO transfected with human CD19

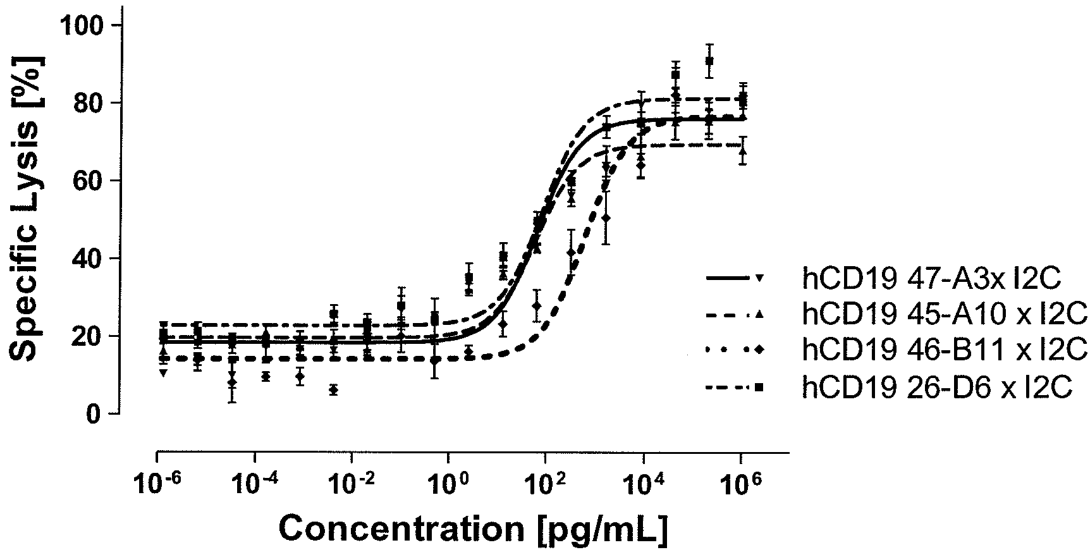


H) Effector cells: stimulated CD4/CD56 depleted human PBMC
Target cells: CHO transfected with human CD19

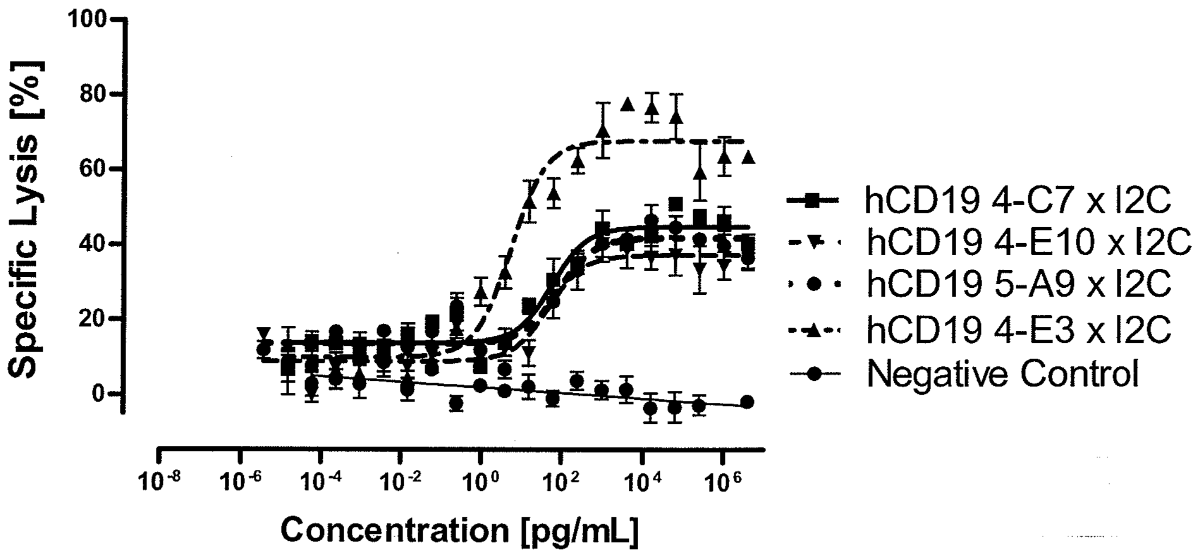

Figure: 51(5)

I) Effector cells: stimulated CD4/CD56 depleted human PBMC
Target cells: CHO transfected with human CD19

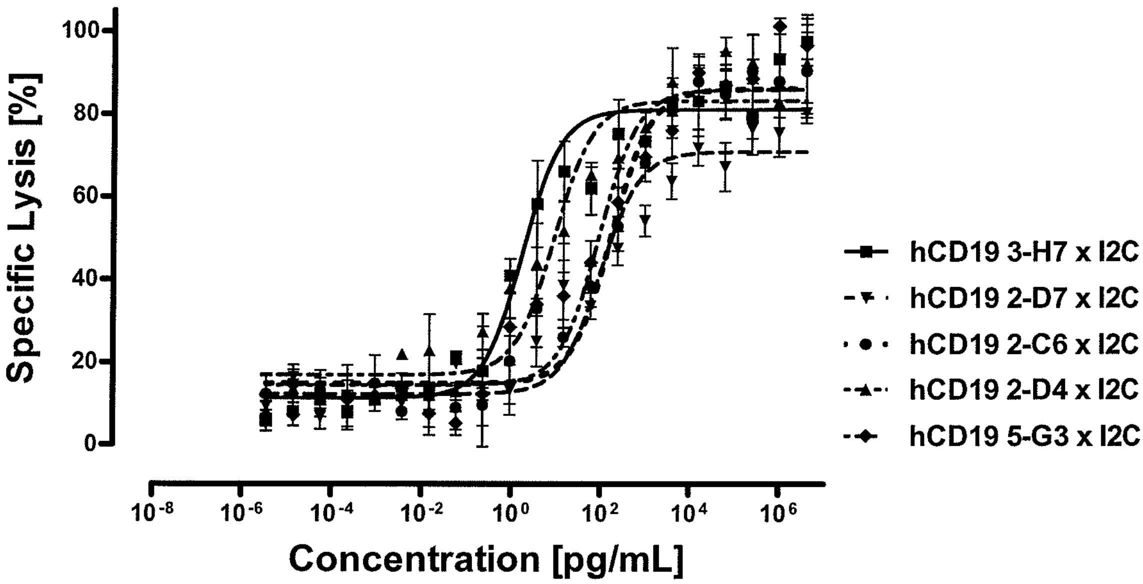

## Figure52a

HPB-ALL

MET1HLxI2CHL

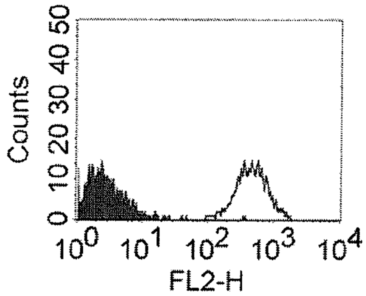


4119LnPx

MET1HLxI2CHL

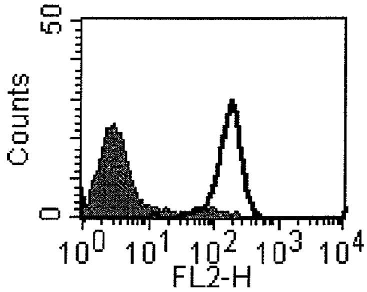


MDA-MB-231

MET1HLxI2CHL

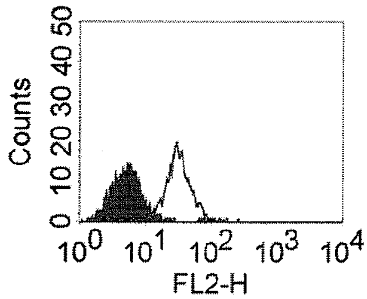


HPB-ALL

MET4HLxI2CHL

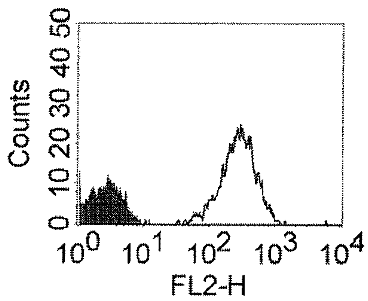


4119LnPx

MET4HLxI2CHL

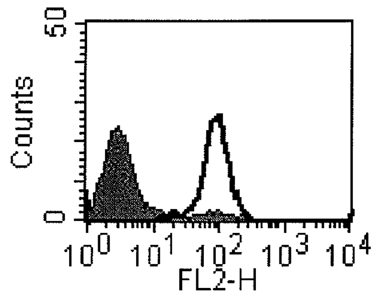


MDA-MB-231

MET4HLxI2CHL

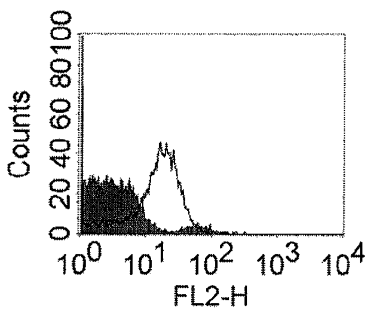

## Figure 52b

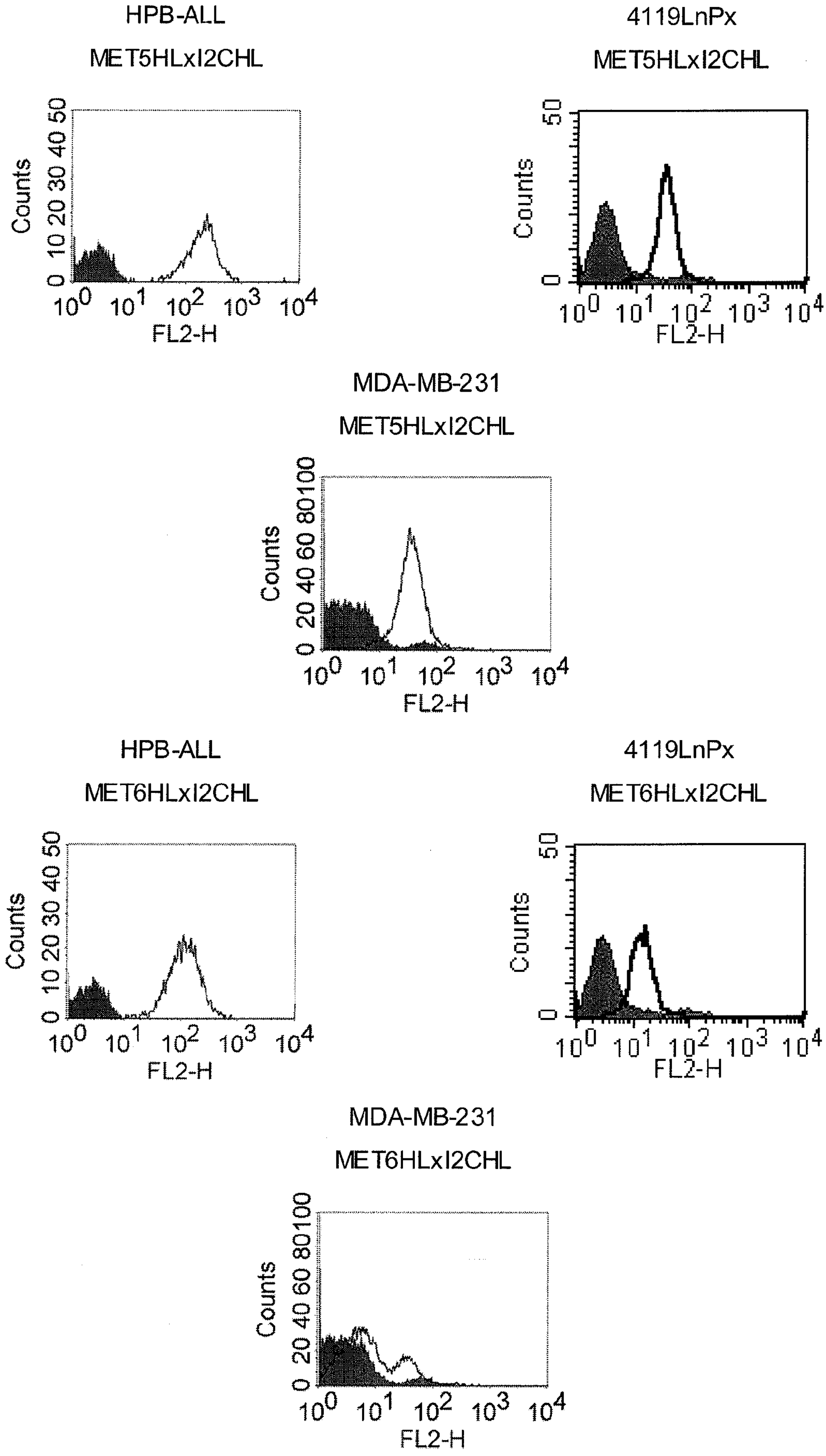
HPB-ALL
MET5HLxI2CHL
4119LnPx
MET5HLxI2CHL
MDA-MB-231
MET5HLxI2CHL
HPB-ALL
MET6HLxI2CHL
4119LnPx
MET6HLxI2CHL
MDA-MB-231
MET6HLxI2CHL
Counts
FL2-H
0 10 20 30 40 50
0 50
0 20 40 60 80 100
$10^0$ $10^1$ $10^2$ $10^3$ $10^4$

**Figure 52c**

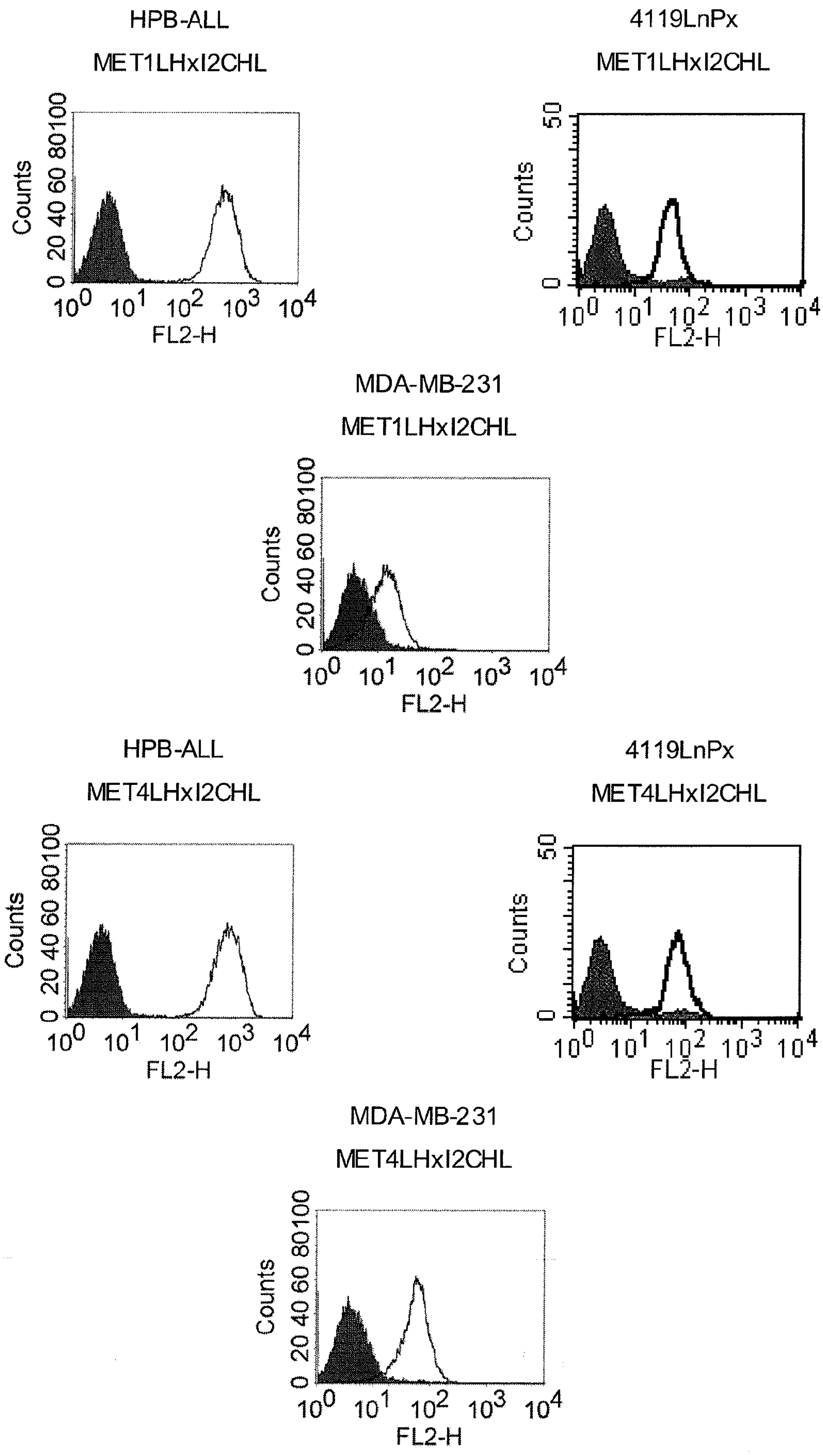
HPB-ALL
MET1LHxI2CHL
4119LnPx
MET1LHxI2CHL
MDA-MB-231
MET1LHxI2CHL
HPB-ALL
MET4LHxI2CHL
4119LnPx
MET4LHxI2CHL
MDA-MB-231
MET4LHxI2CHL
Counts
FL2-H
0 20 40 60 80100
0 50
$10^0$ $10^1$ $10^2$ $10^3$ $10^4$

Figure 52d

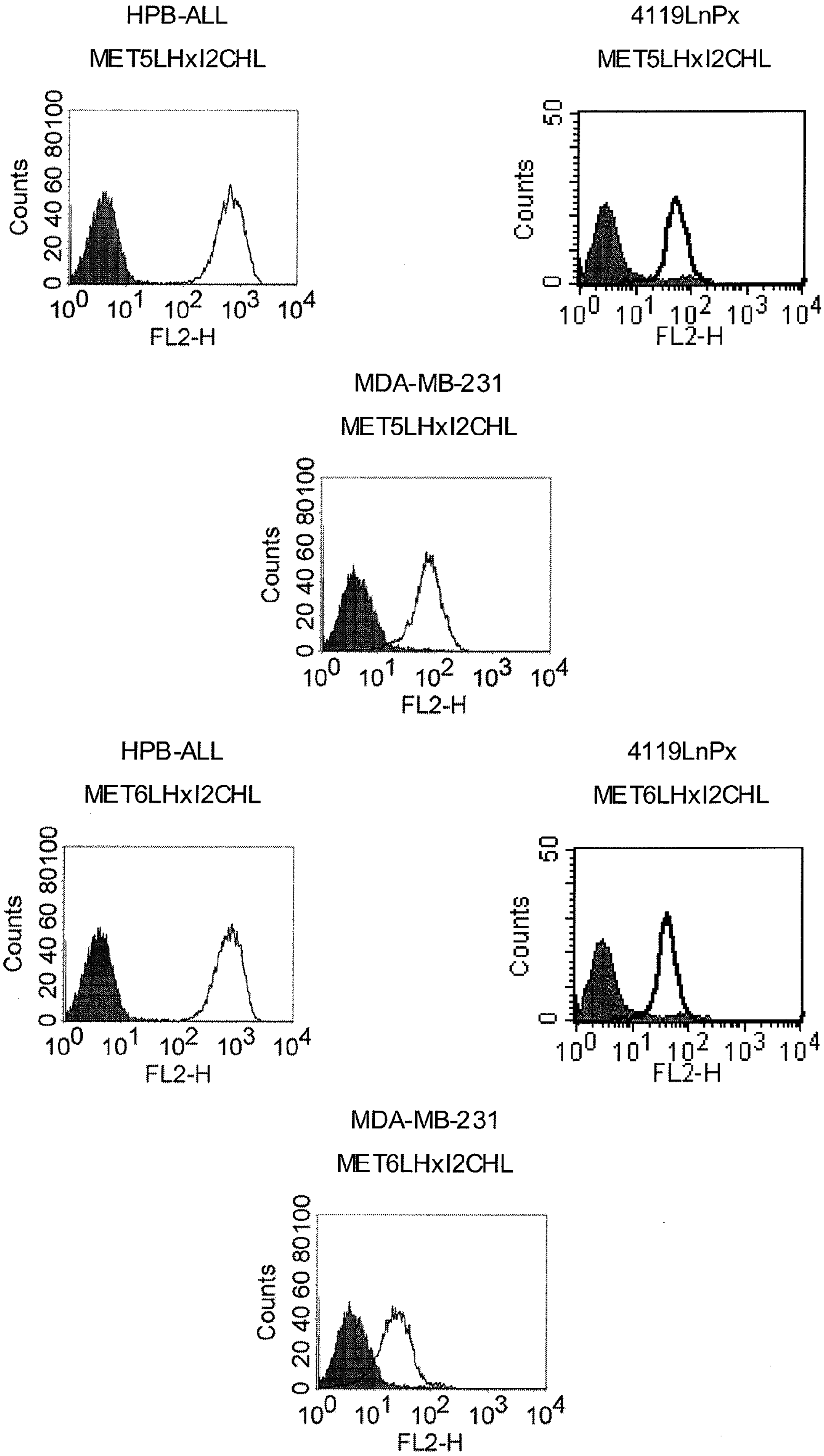
HPB-ALL
MET5LHxI2CHL
4119LnPx
MET5LHxI2CHL
MDA-MB-231
MET5LHxI2CHL
HPB-ALL
MET6LHxI2CHL
4119LnPx
MET6LHxI2CHL
MDA-MB-231
MET6LHxI2CHL
Counts
FL2-H
0 20 40 60 80 100
0 50
$10^0$ $10^1$ $10^2$ $10^3$ $10^4$

**Figure 52e**

HPB-ALL

MET7LHxI2CHL

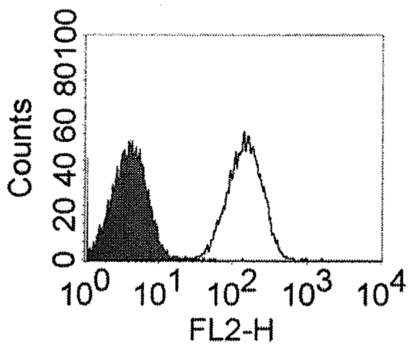


4119LnPx

MET7LHxI2CHL

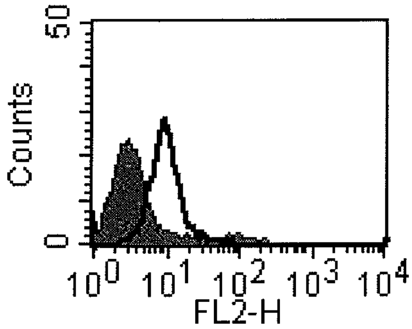


MDA-MB-231

MET7LHxI2CHL

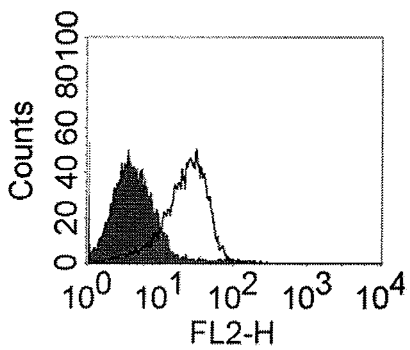

# Figure 53a

Effector cells: stimulated CD4/CD56 depleted human PBMC
Target cells: MDA-MB-231

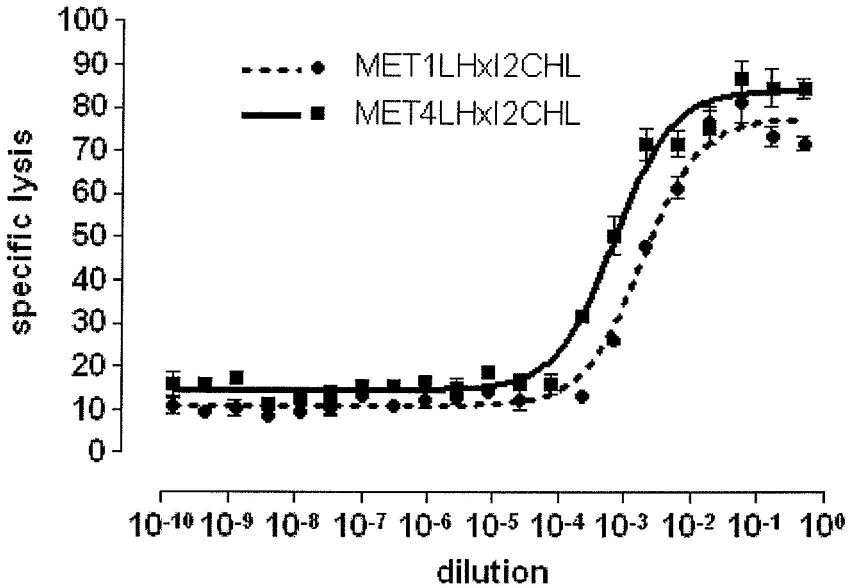


Effector cells: stimulated CD4/CD56 depleted human PBMC
Target cells: MDA-MB-231

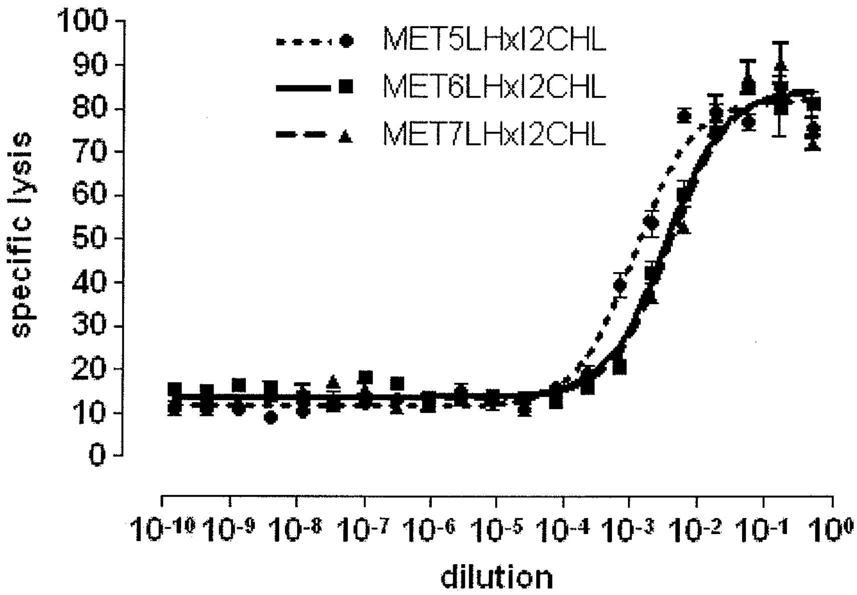

## Figure 53b

Effector cells: stimulated CD4/CD56 depleted human PBMC
Target cells: MDA-MB-231

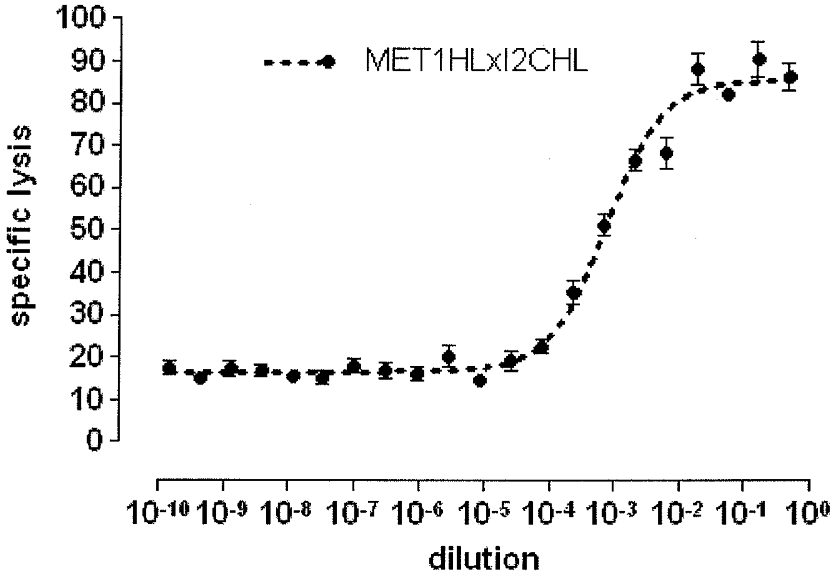


Effector cells: stimulated CD4/CD56 depleted human PBMC
Target cells: MDA-MB-231

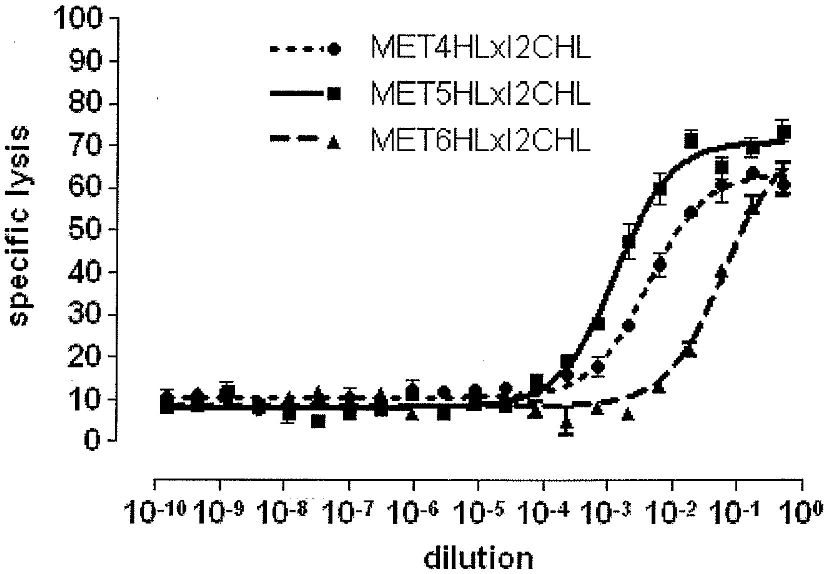

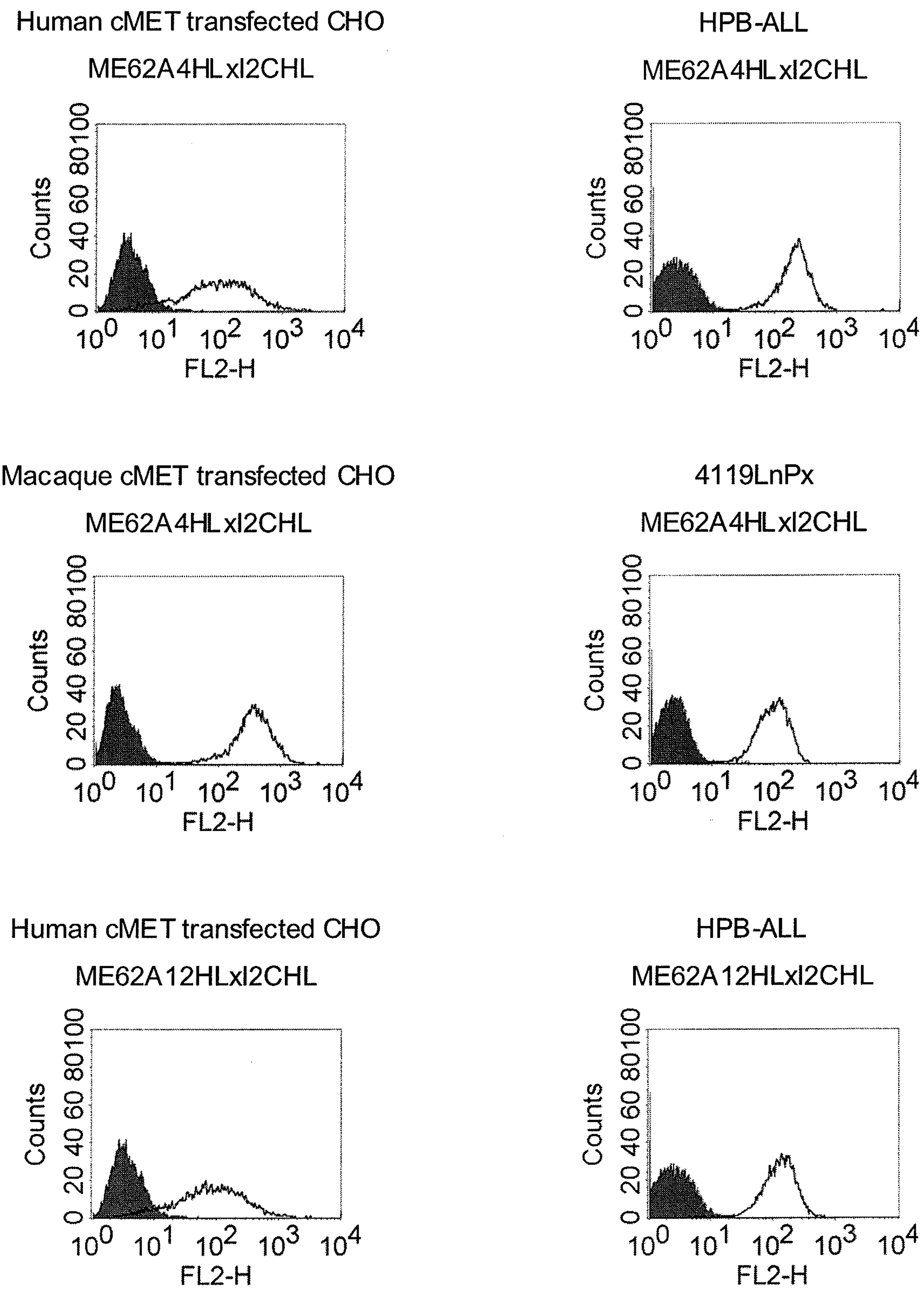
Human cMET transfected CHO
ME62A4HLxI2CHL
HPB-ALL
ME62A4HLxI2CHL
Macaque cMET transfected CHO
ME62A4HLxI2CHL
4119LnPx
ME62A4HLxI2CHL
Human cMET transfected CHO
ME62A12HLxI2CHL
HPB-ALL
ME62A12HLxI2CHL
Counts
0 20 40 60 80 100
FL2-H
$10^0$ $10^1$ $10^2$ $10^3$ $10^4$

Figure 54a

Macaque cMET transfected CHO

ME62A12HLxI2CHL

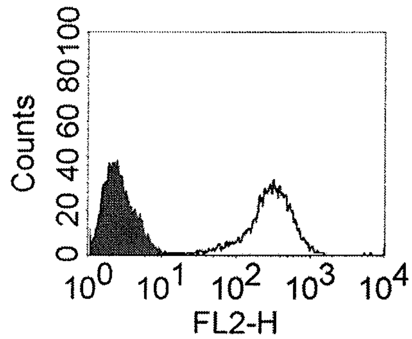


4119LnPx

ME62A12HLxI2CHL

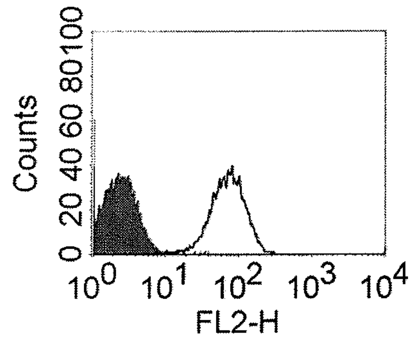


Human cMET transfected CHO

ME62C10HLxI2CHL

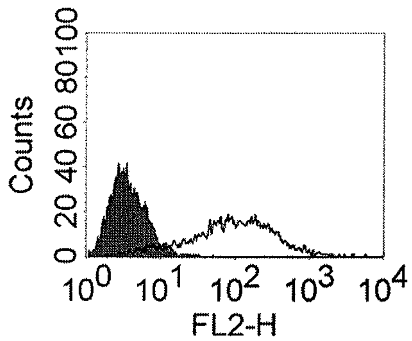


HPB-ALL

ME62C10HLxI2CHL

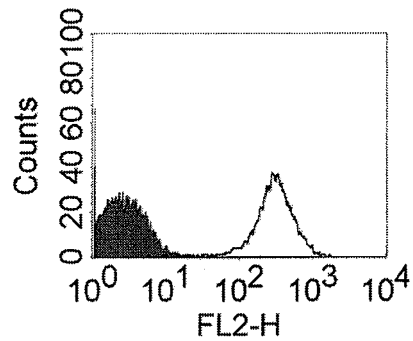


Macaque cMET transfected CHO

ME62C10HLxI2CHL

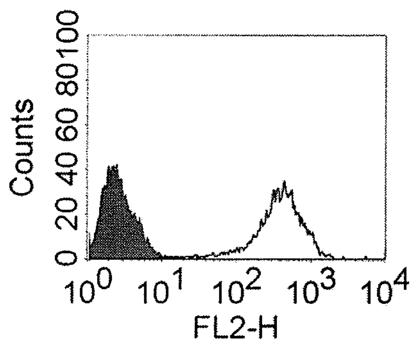


4119LnPx

ME62C10HLxI2CHL

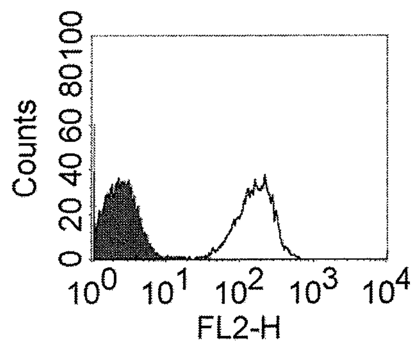


**Figure 54b**

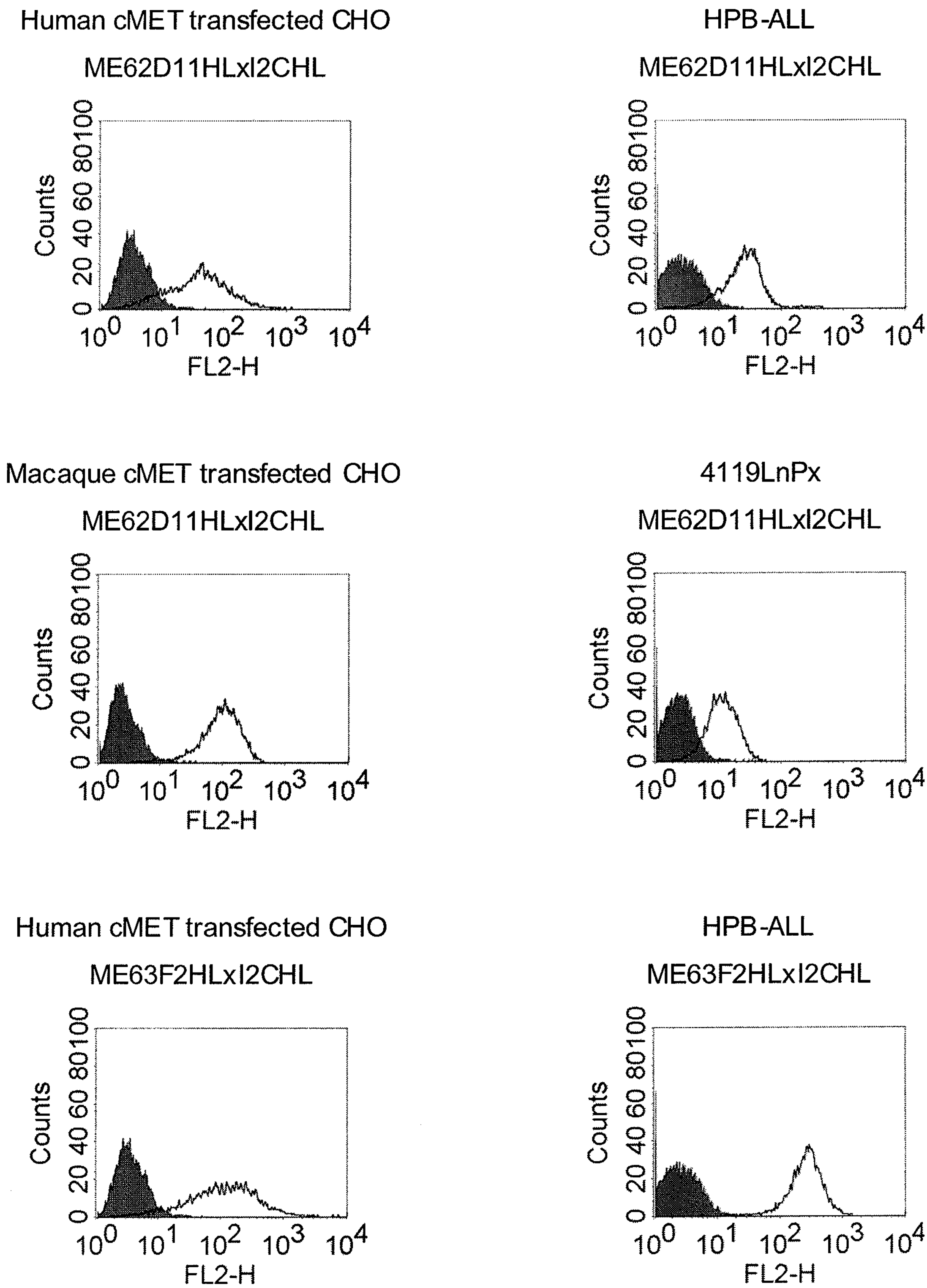
Human cMET transfected CHO
ME62D11HLxI2CHL
HPB-ALL
ME62D11HLxI2CHL
Macaque cMET transfected CHO
ME62D11HLxI2CHL
4119LnPx
ME62D11HLxI2CHL
Human cMET transfected CHO
ME63F2HLxI2CHL
HPB-ALL
ME63F2HLxI2CHL
Counts
0 20 40 60 80 100
FL2-H
$10^0$ $10^1$ $10^2$ $10^3$ $10^4$

**Figure 54c**

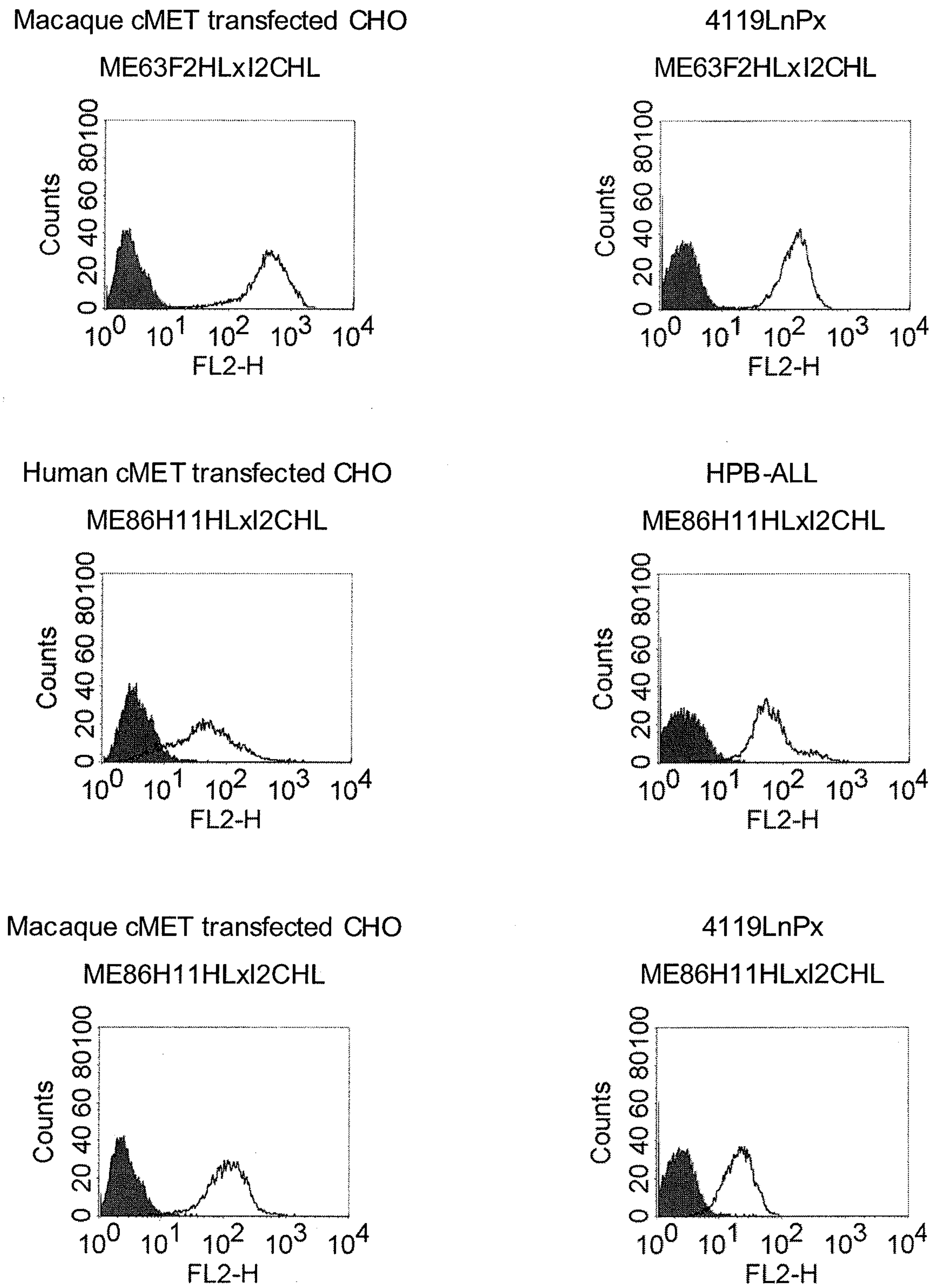
Macaque cMET transfected CHO
ME63F2HLxI2CHL
4119LnPx
ME63F2HLxI2CHL
Human cMET transfected CHO
ME86H11HLxI2CHL
HPB-ALL
ME86H11HLxI2CHL
Macaque cMET transfected CHO
ME86H11HLxI2CHL
4119LnPx
ME86H11HLxI2CHL
Counts
0 20 40 60 80100
$10^0$ $10^1$ $10^2$ $10^3$ $10^4$
FL2-H

**Figure 54d**

Effector T cells: macaque 4119 LnPx

Target cells: CHO transfected with macaque cMET

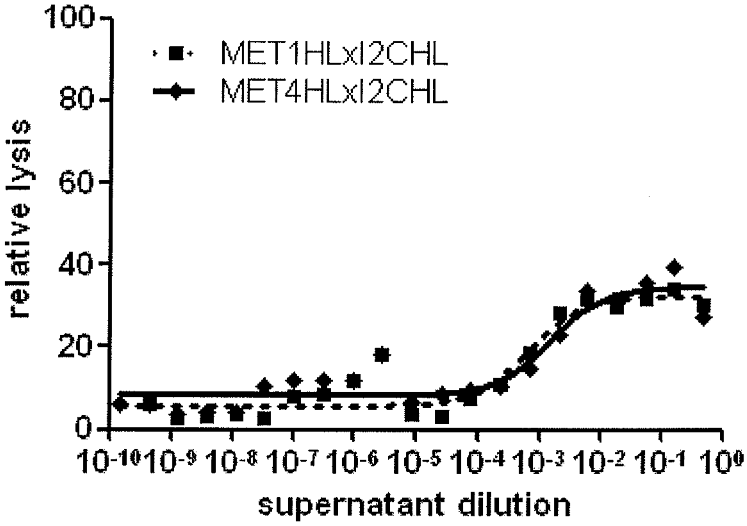


Effector T cells: macaque 4119 LnPx

Target cells: CHO transfected with macaque cMET

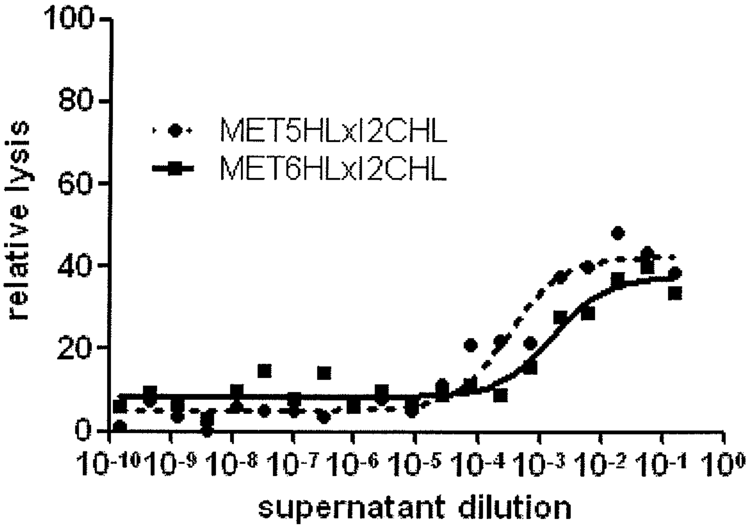


**Figure 55a**

Effector T cells: macaque 4119 LnPx

Target cells: CHO transfected with macaque cMET

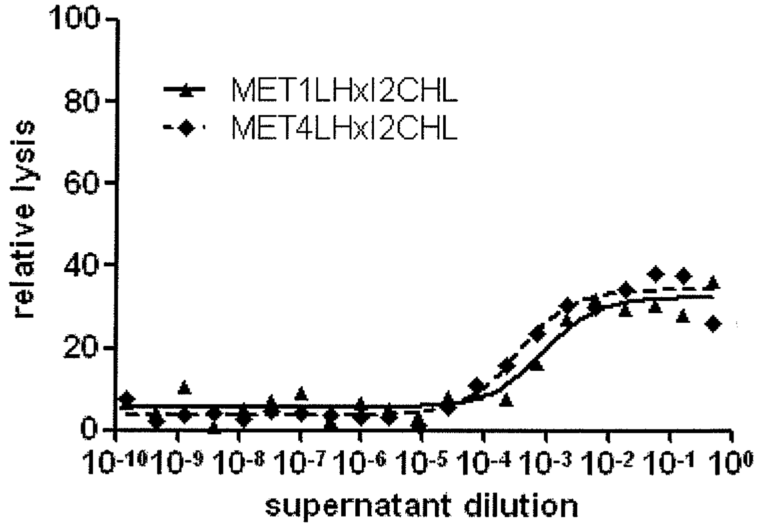


Effector T cells: macaque 4119 LnPx

Target cells: CHO transfected with macaque cMET

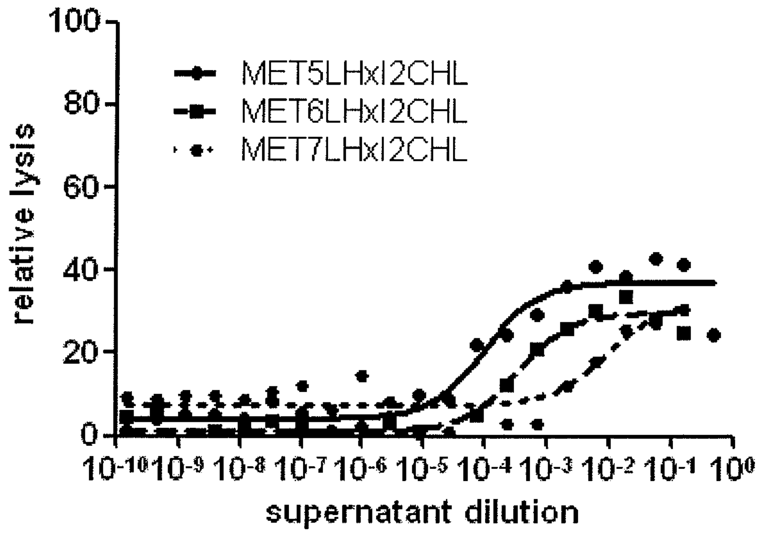


**Figure 55b**

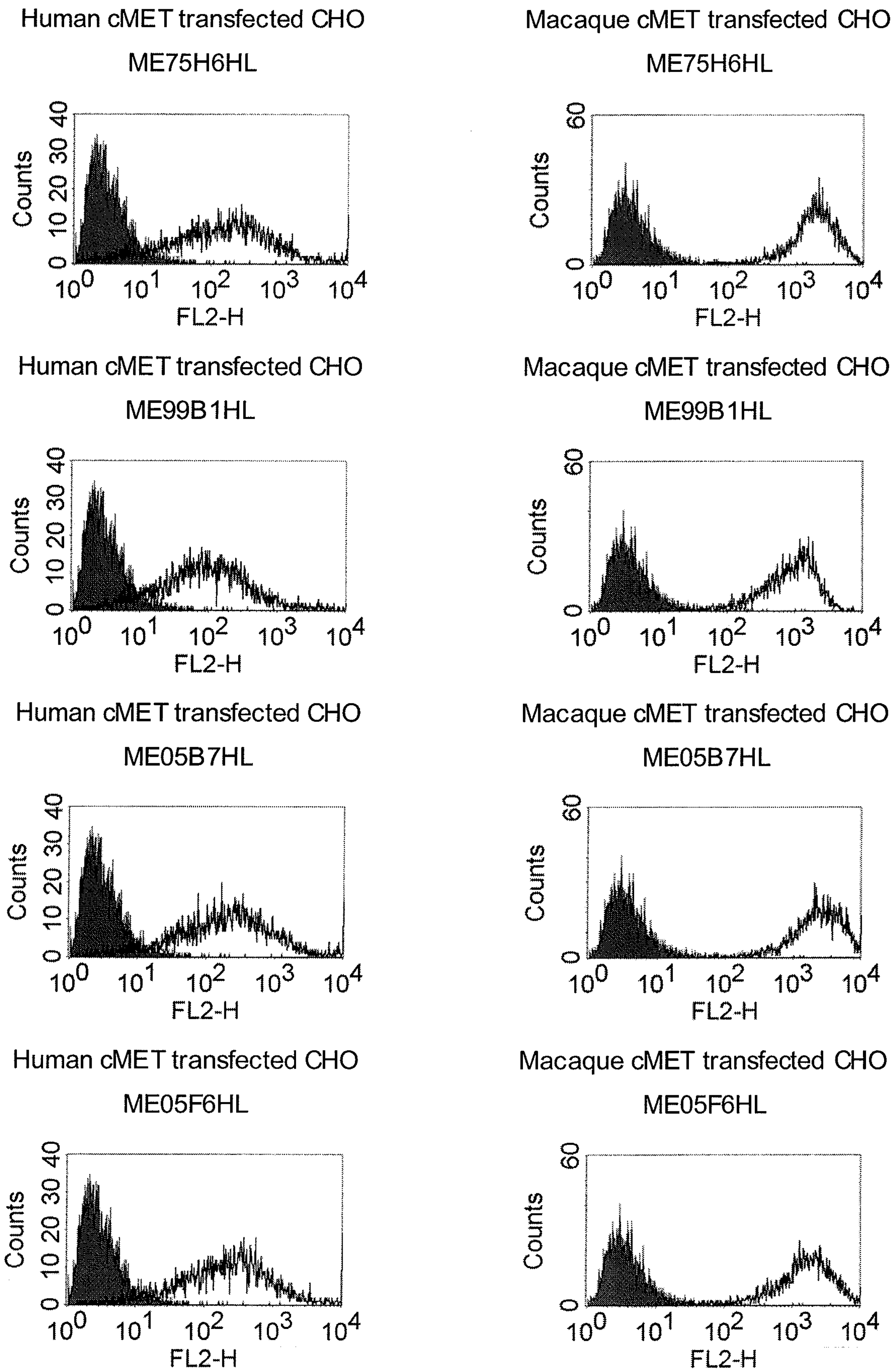
Human cMET transfected CHO
ME75H6HL
Macaque cMET transfected CHO
ME75H6HL
Human cMET transfected CHO
ME99B1HL
Macaque cMET transfected CHO
ME99B1HL
Human cMET transfected CHO
ME05B7HL
Macaque cMET transfected CHO
ME05B7HL
Human cMET transfected CHO
ME05F6HL
Macaque cMET transfected CHO
ME05F6HL
Counts
FL2-H
0
10
20
30
40
60
$10^0$
$10^1$
$10^2$
$10^3$
$10^4$

Figure 56a

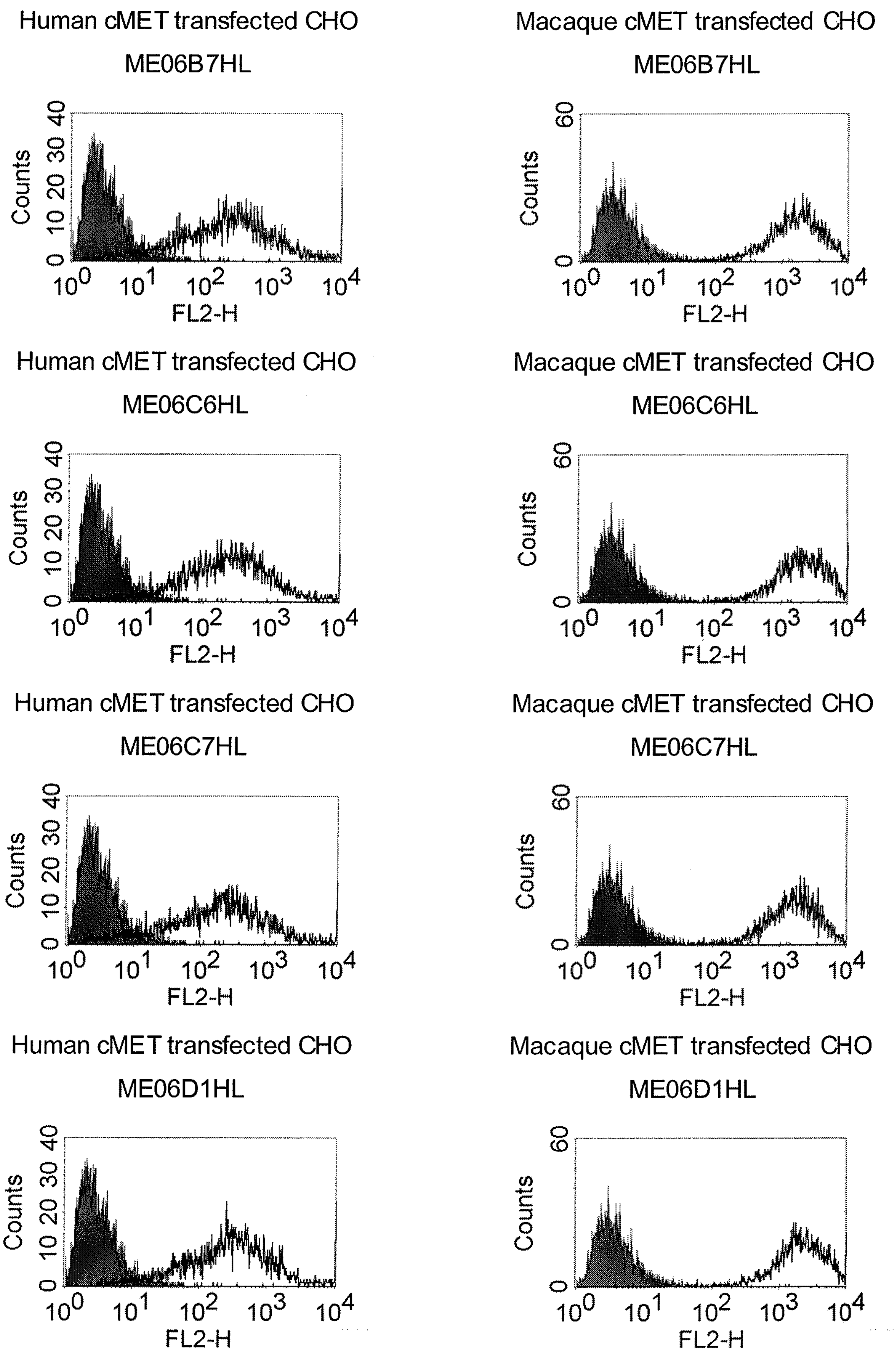
Human cMET transfected CHO
ME06B7HL
Macaque cMET transfected CHO
ME06B7HL
Human cMET transfected CHO
ME06C6HL
Macaque cMET transfected CHO
ME06C6HL
Human cMET transfected CHO
ME06C7HL
Macaque cMET transfected CHO
ME06C7HL
Human cMET transfected CHO
ME06D1HL
Macaque cMET transfected CHO
ME06D1HL
Counts
FL2-H
0 10 20 30 40
0 60
$10^0$ $10^1$ $10^2$ $10^3$ $10^4$

**Figure 56b**

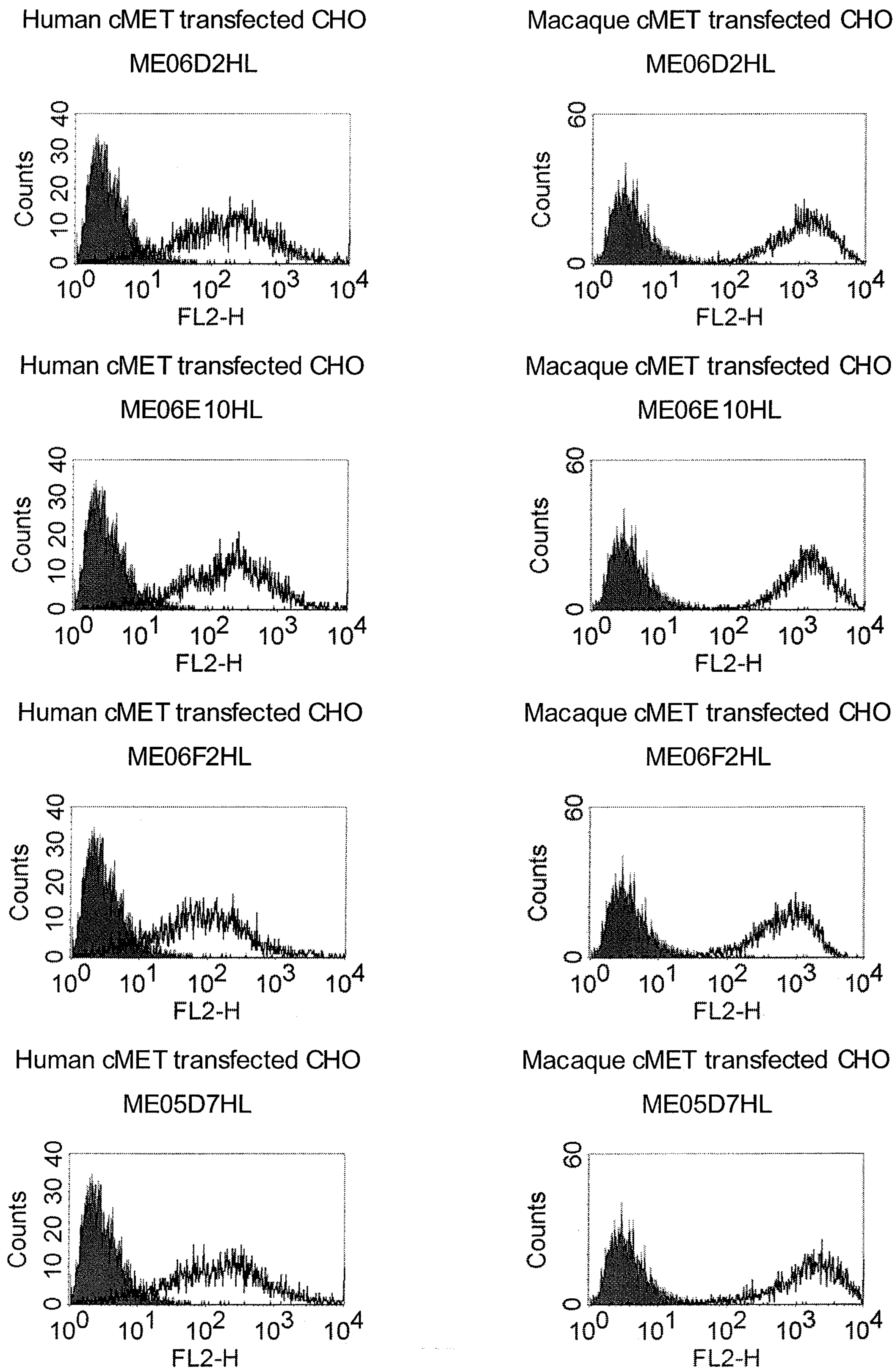
Human cMET transfected CHO
ME06D2HL
Macaque cMET transfected CHO
ME06D2HL
Human cMET transfected CHO
ME06E10HL
Macaque cMET transfected CHO
ME06E10HL
Human cMET transfected CHO
ME06F2HL
Macaque cMET transfected CHO
ME06F2HL
Human cMET transfected CHO
ME05D7HL
Macaque cMET transfected CHO
ME05D7HL
Counts
FL2-H

**Figure 56c**

## Figure 57

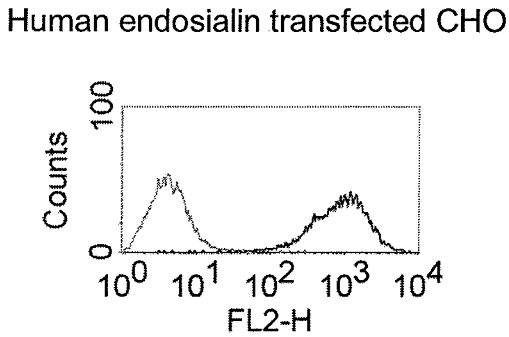
Human endosialin transfected CHO
Counts
100
0
10^0 10^1 10^2 10^3 10^4
FL2-H

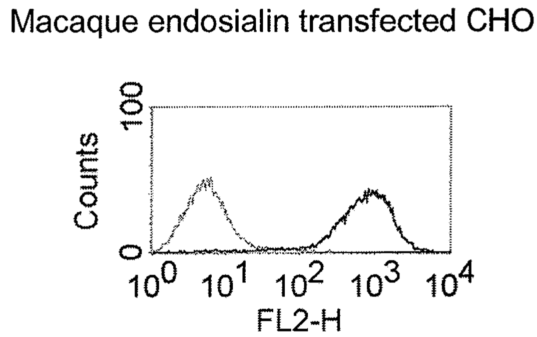
Macaque endosialin transfected CHO
Counts
100
0
10^0 10^1 10^2 10^3 10^4
FL2-H

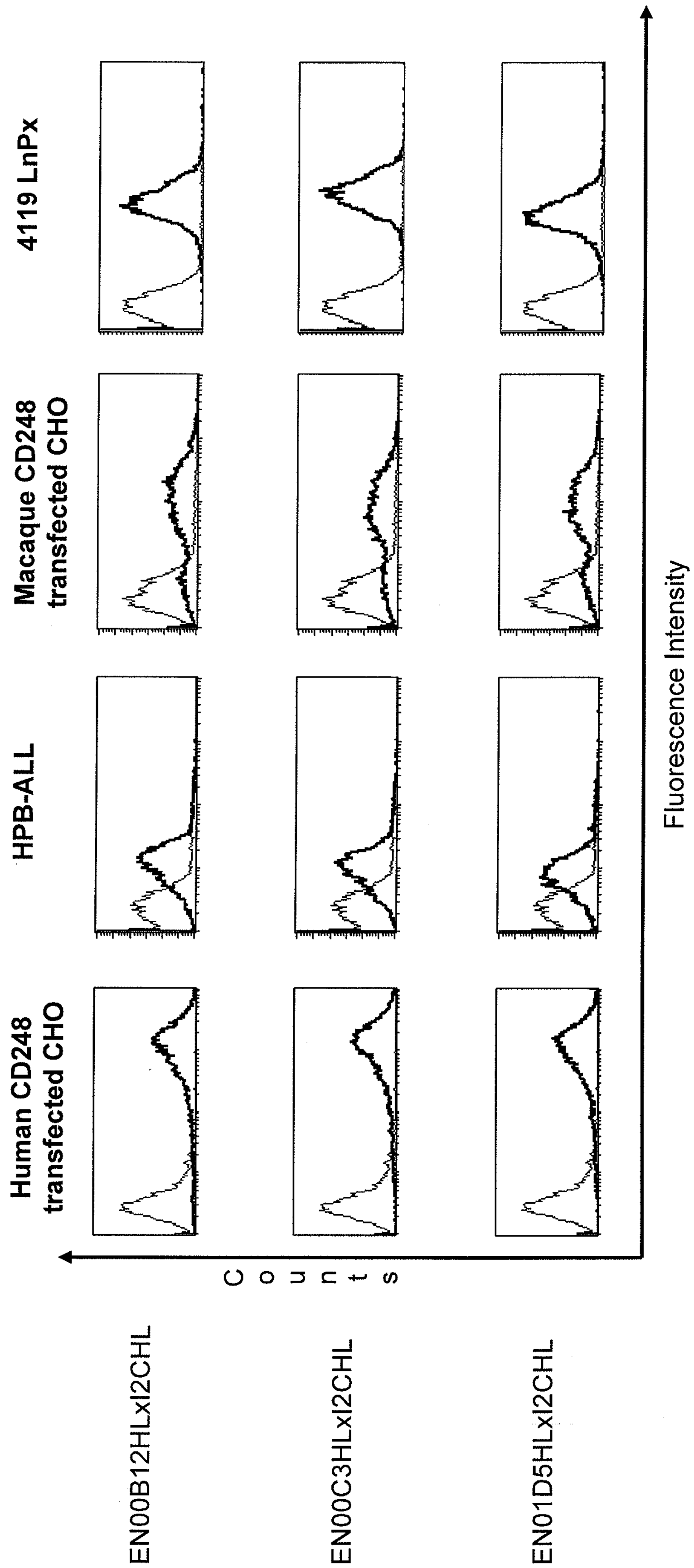

Human CD248 transfected CHO
HPB-ALL
Macaque CD248 transfected CHO
4119 LnPx
EN00B12HLxI2CHL
EN00C3HLxI2CHL
EN01D5HLxI2CHL
Counts
Fluorescence Intensity


Figure 58(1)

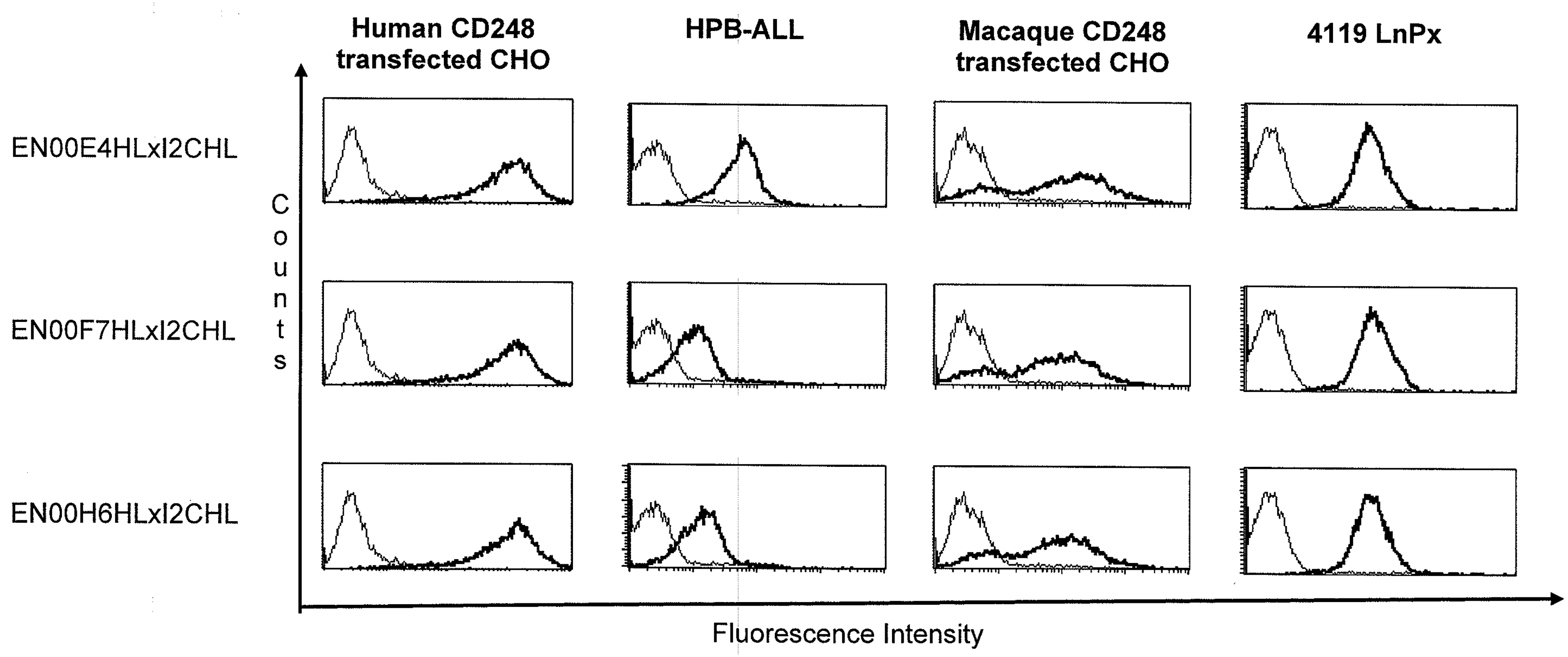
Human CD248
transfected CHO
HPB-ALL
Macaque CD248
transfected CHO
4119 LnPx
EN00E4HLxI2CHL
EN00F7HLxI2CHL
EN00H6HLxI2CHL
Counts
Fluorescence Intensity

Figure 58(2)

Effector T cells: stimulated human CD4/CD56 depleted human PBMC
Target cells: CHO transfected with human CD248
E:T ratio: 10:1

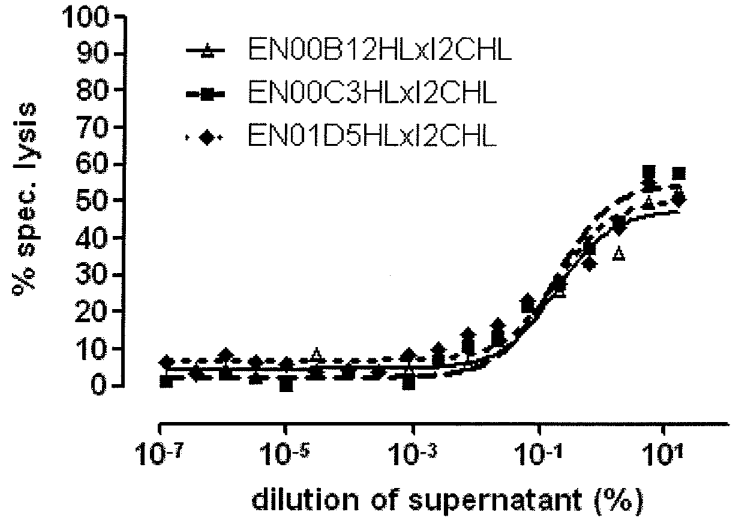


Effector T cells: stimulated human CD4/CD56 depleted human PBMC
Target cells: CHO transfected with human CD248
E:T ratio: 10:1

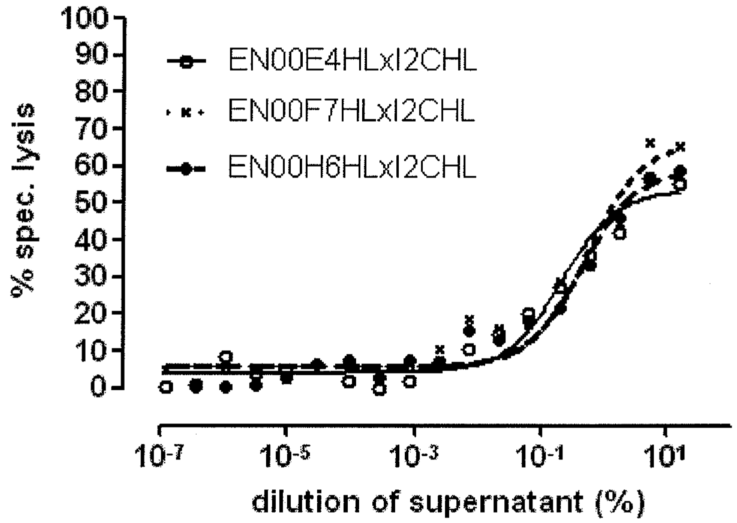


**Figure 59(1)**

Effector T cells: 4119 LnPx
Target cells: CHO transfected with macaque CD248
E:T ratio: 10:1

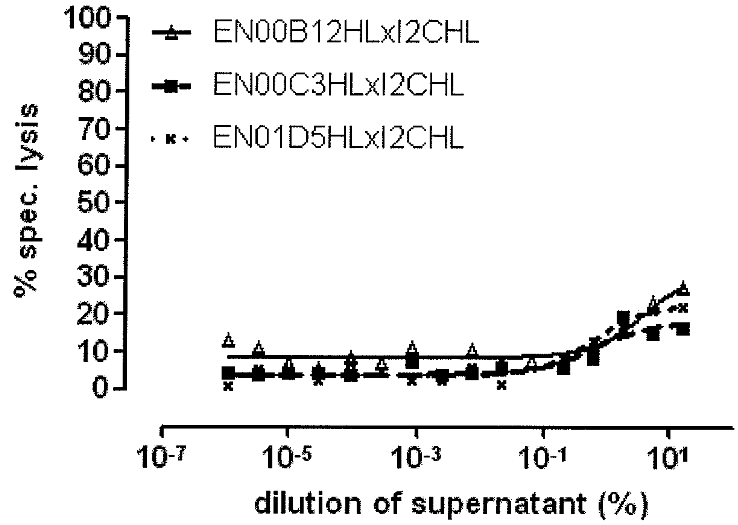


Effector T cells: 4119 LnPx
Target cells: CHO transfected with macaque CD248
E:T ratio: 10:1

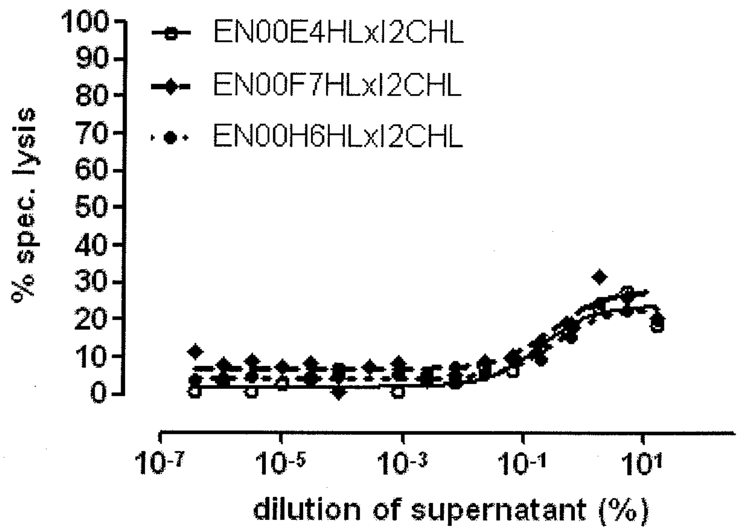


**Figure 59(2)**

Figure 60.1

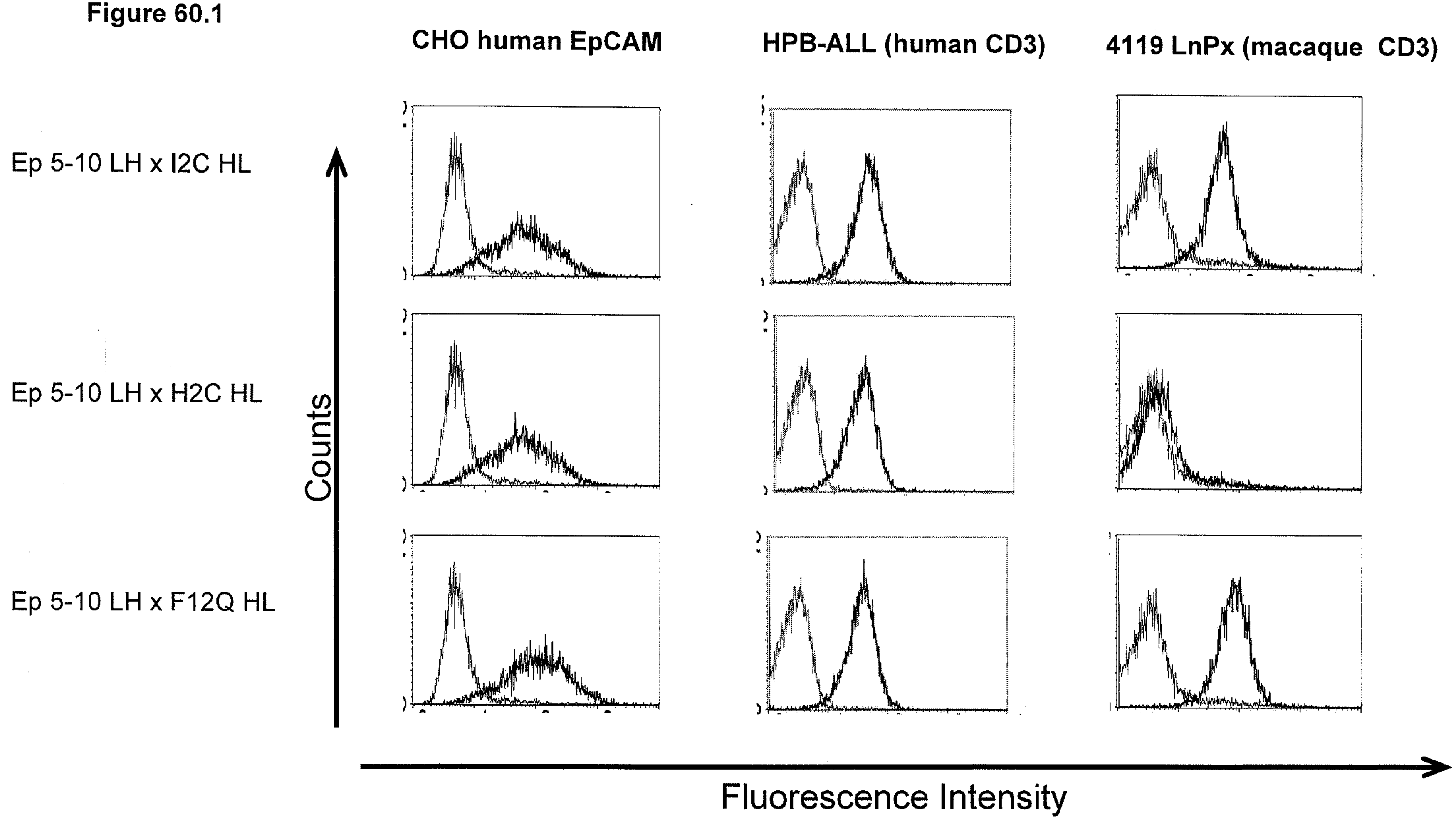
CHO human EpCAM
HPB-ALL (human CD3)
4119 LnPx (macaque CD3)
Ep 5-10 LH x I2C HL
Ep 5-10 LH x H2C HL
Ep 5-10 LH x F12Q HL
Counts
Fluorescence Intensity

Figure 60.2

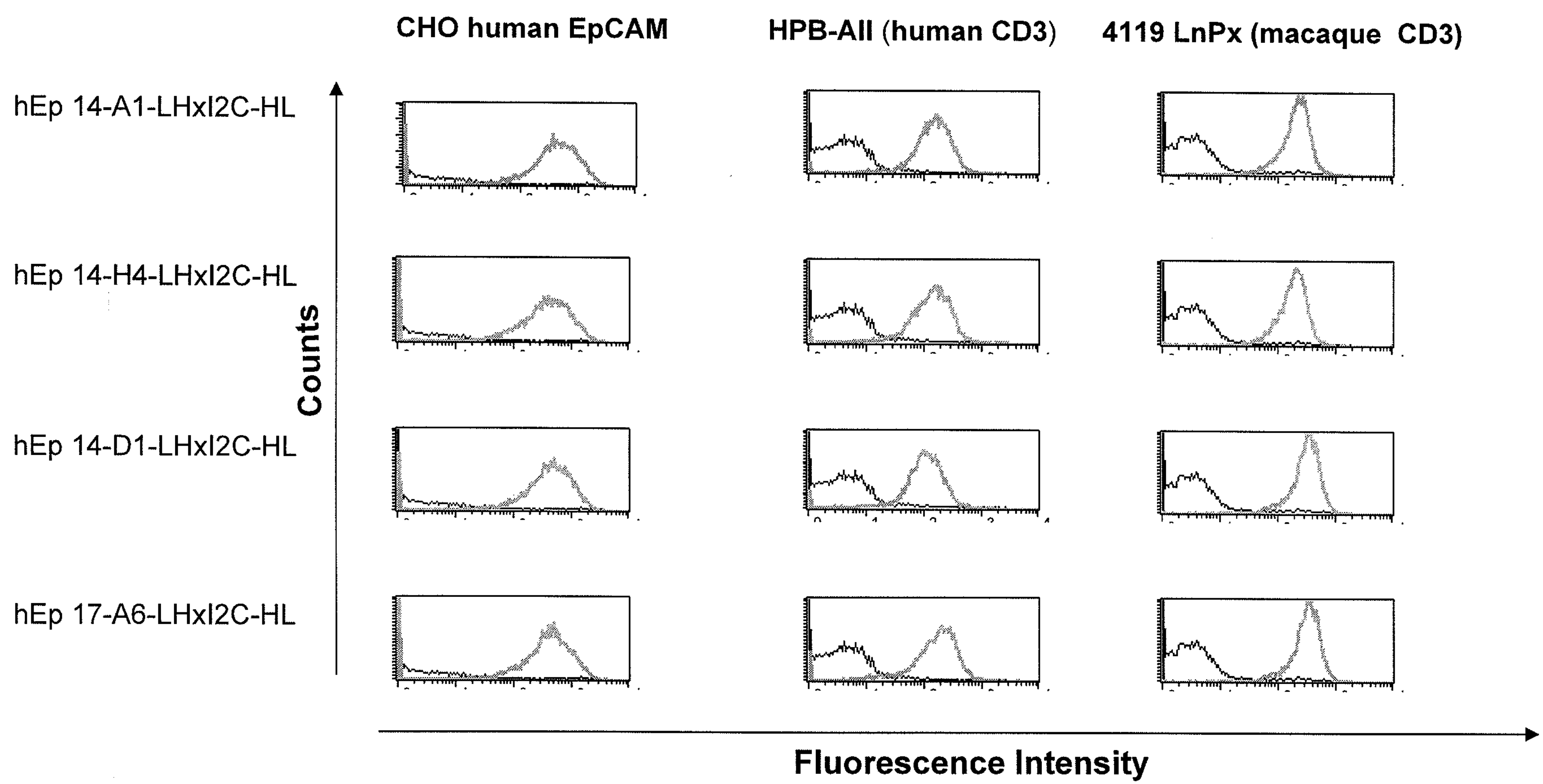
CHO human EpCAM
HPB-All (human CD3)
4119 LnPx (macaque CD3)
hEp 14-A1-LHxI2C-HL
hEp 14-H4-LHxI2C-HL
hEp 14-D1-LHxI2C-HL
hEp 17-A6-LHxI2C-HL
Counts
Fluorescence Intensity

Figure 60.3

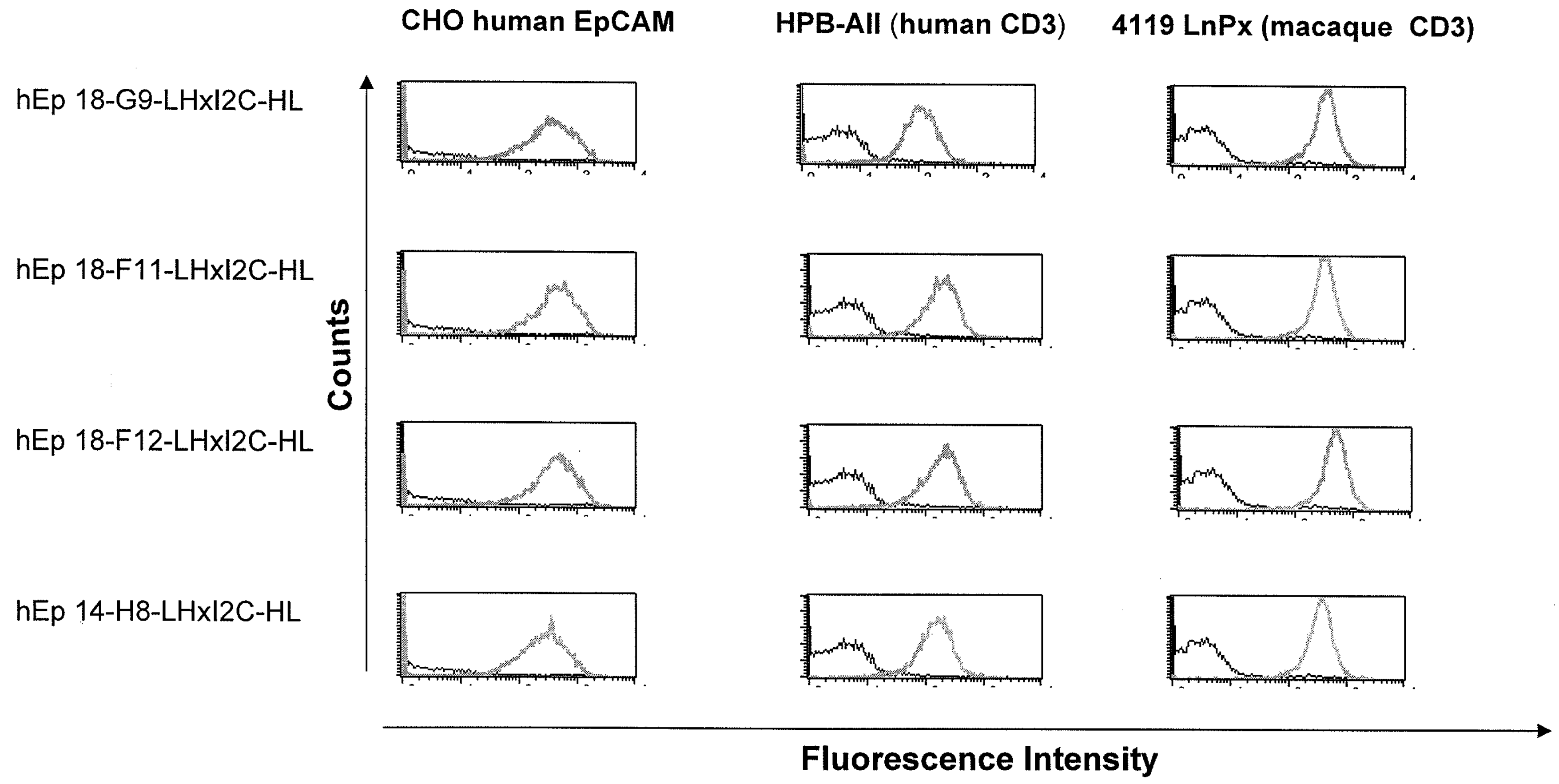
CHO human EpCAM
HPB-All (human CD3)
4119 LnPx (macaque CD3)
hEp 18-G9-LHxI2C-HL
hEp 18-F11-LHxI2C-HL
hEp 18-F12-LHxI2C-HL
hEp 14-H8-LHxI2C-HL
Counts
Fluorescence Intensity

Figure 60.4

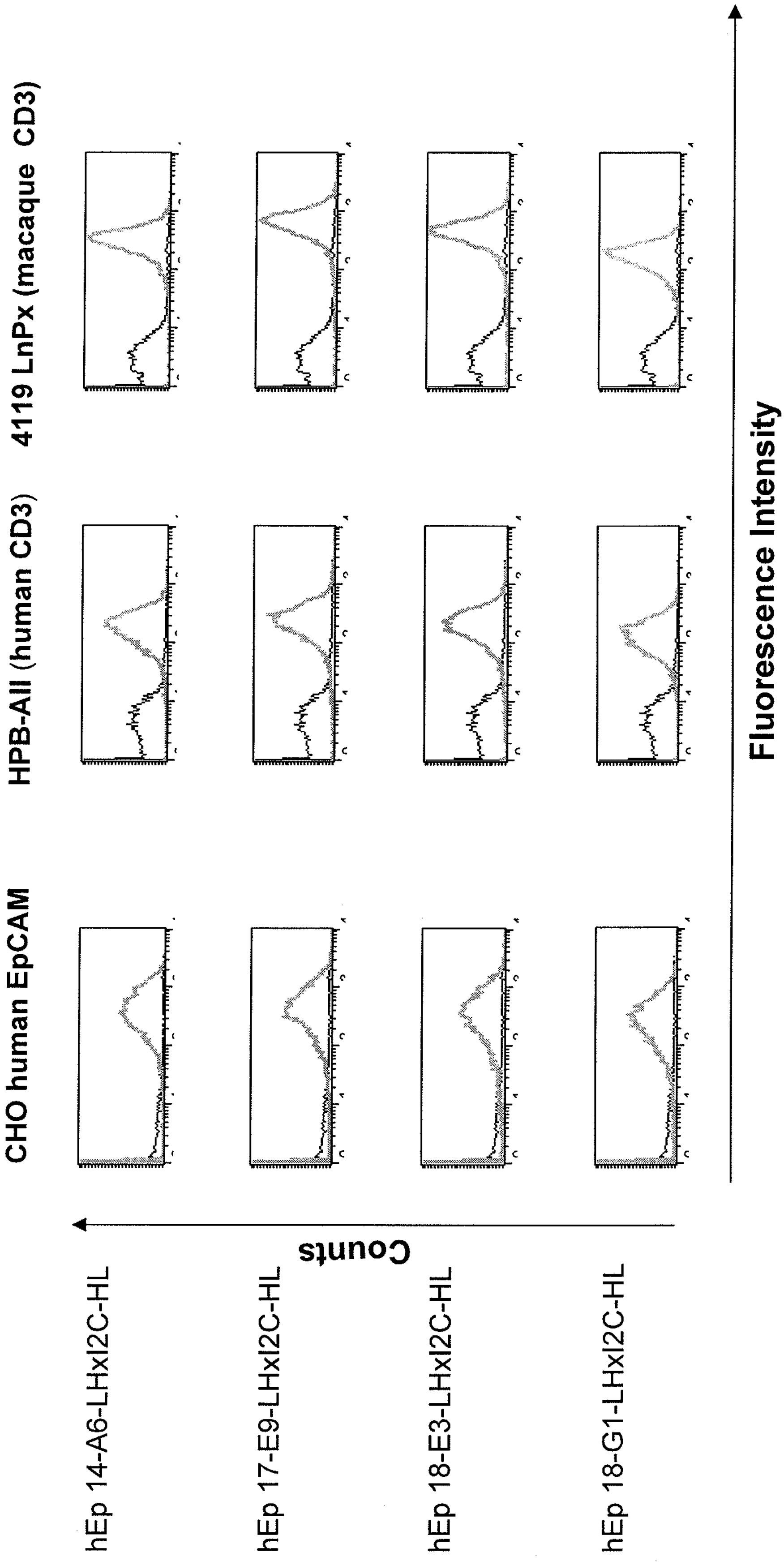
CHO human EpCAM
HPB-All (human CD3)
4119 LnPx (macaque CD3)
hEp 14-A6-LHxI2C-HL
hEp 17-E9-LHxI2C-HL
hEp 18-E3-LHxI2C-HL
hEp 18-G1-LHxI2C-HL
Counts
Fluorescence Intensity

Figure: 60.5

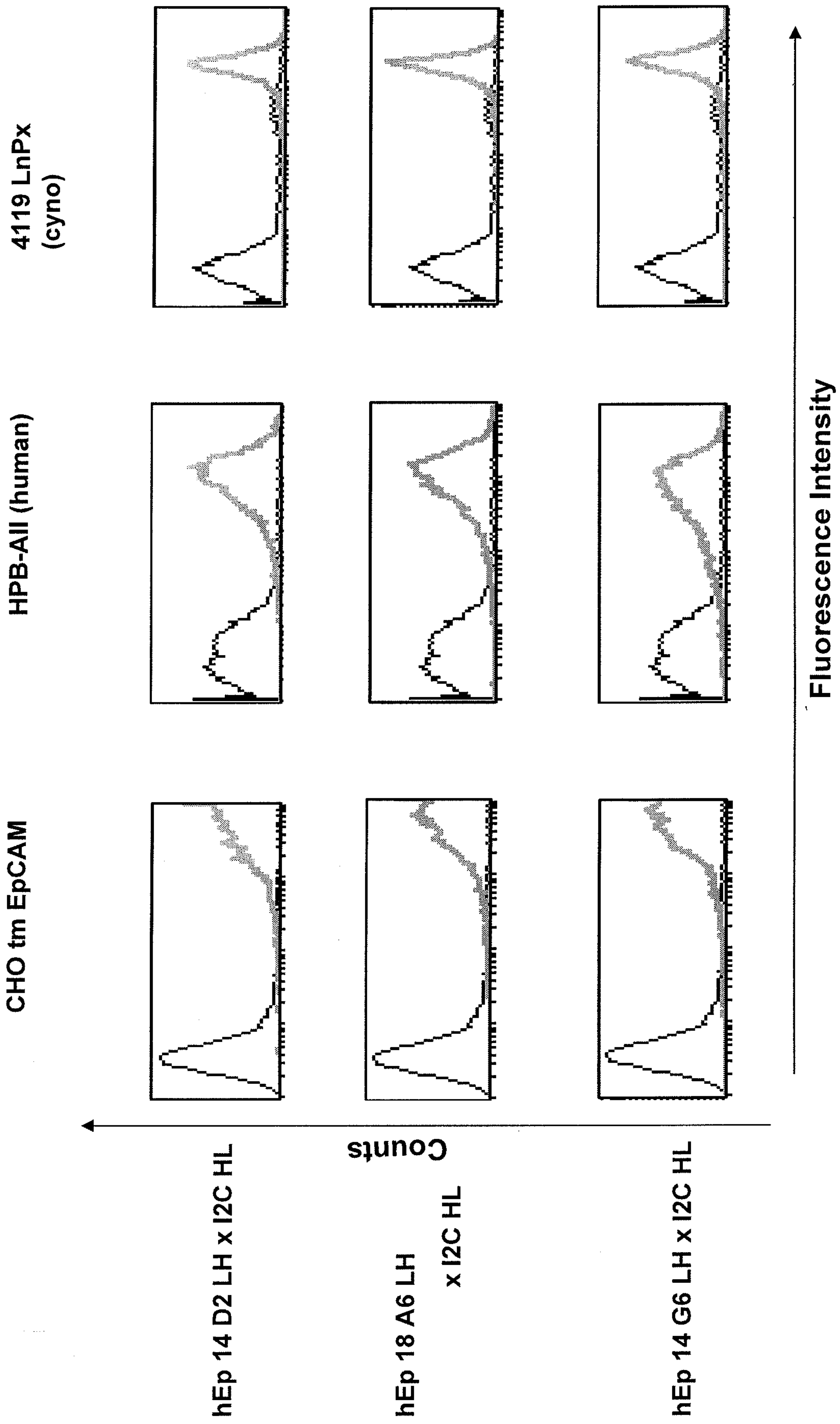
CHO tm EpCAM
HPB-All (human)
4119 LnPx
(cyno)
hEp 14 D2 LH x I2C HL
hEp 18 A6 LH
x I2C HL
hEp 14 G6 LH x I2C HL
Counts
Fluorescence Intensity

## Figure 61

**A) Effector cells: CD56 depleted unstimulated human PBMC**
**Target cells: CHO transfected with human EpCAM**

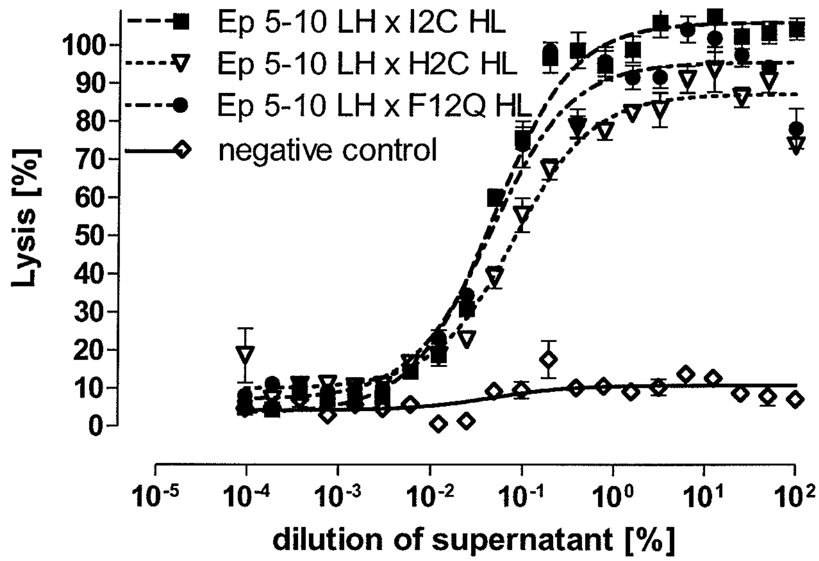


**B) Effector cells: 4119LnPx**
**Target cells: CHO transfected with human EpCAM**

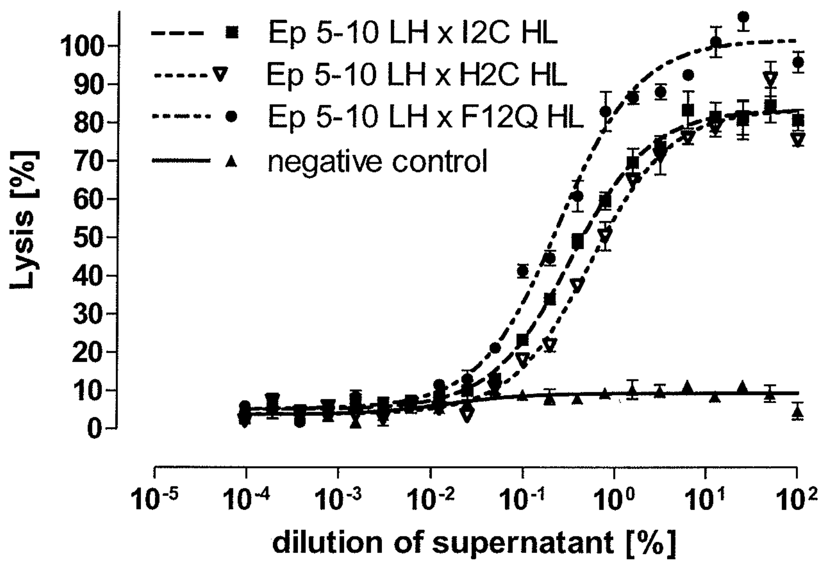

## Figure 62

**A) Effector cells: stimulated CD4/CD56 depleted human PBMC Target cells: CHO transfected with human EpCAM**

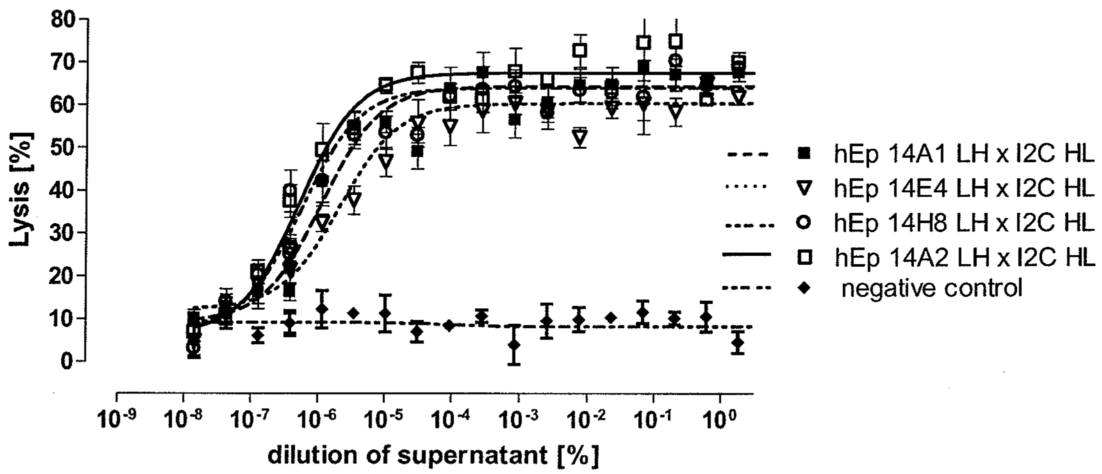


**B) Effector cells: stimulated CD4/CD56 depleted human PBMC Target cells: CHO transfected with human EpCAM**

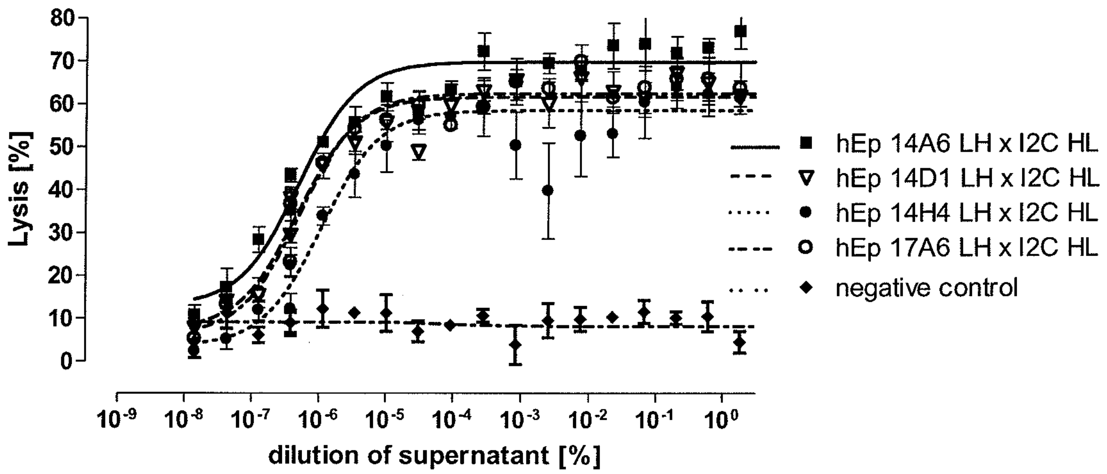

## Figure 63

### A) Effector cells: stimulated CD4/CD56 depleted human PBMC Target cells: CHO transfected with human EpCAM

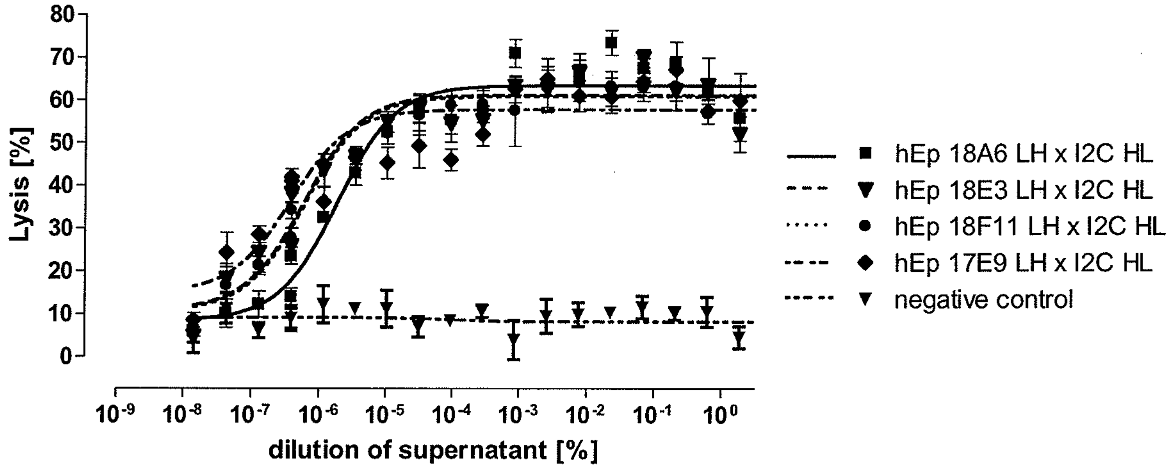


### B) Effector cells: stimulated CD4/CD56 depleted human PBMC Target cells: CHO transfected with human EpCAM

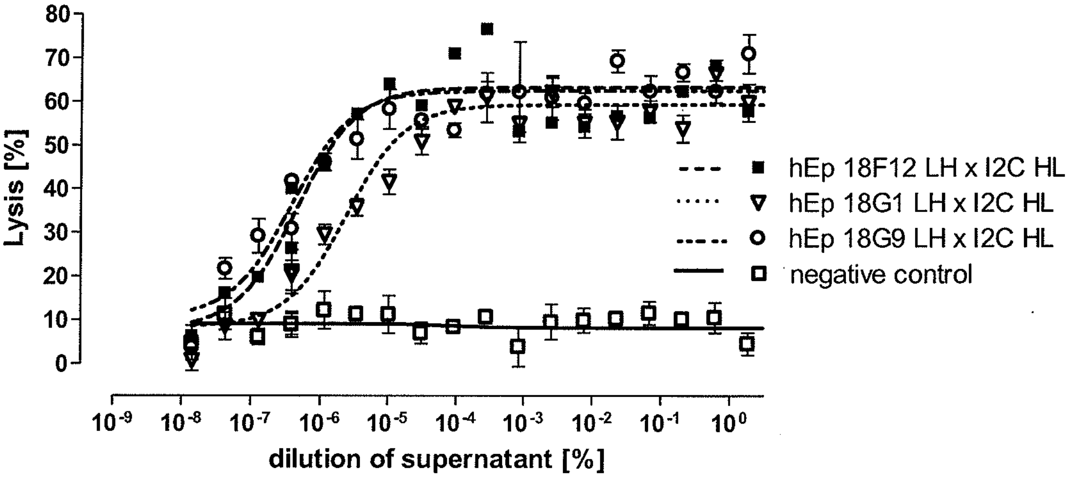

## Figure 64

### A) Effector cells: 4119 LnPx
### Target cells: CHO transfected with human EpCAM

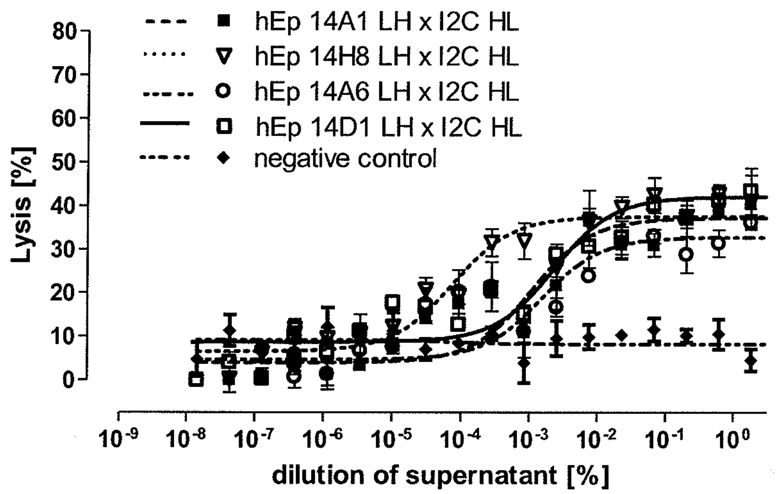


### B) Effector cells: 4119 LnPx
### Target cells: CHO transfected with human EpCAM

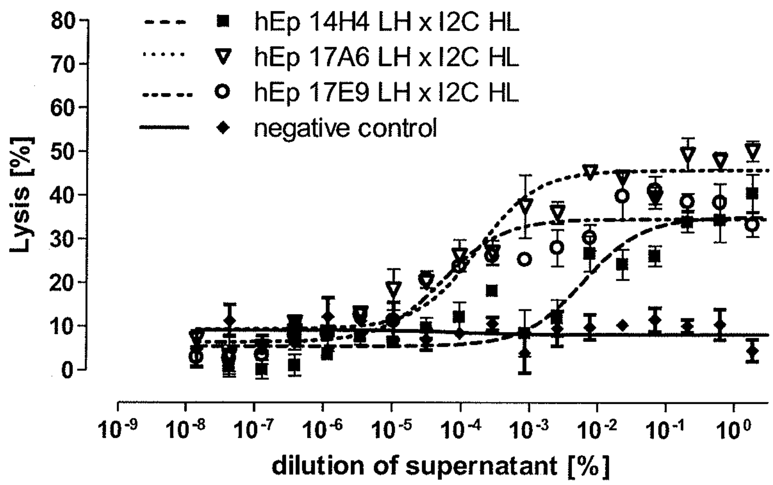

**Figure 65**

**A) Effector cells: 4119 LnPx**
**Target cells: CHO transfected with human EpCAM**

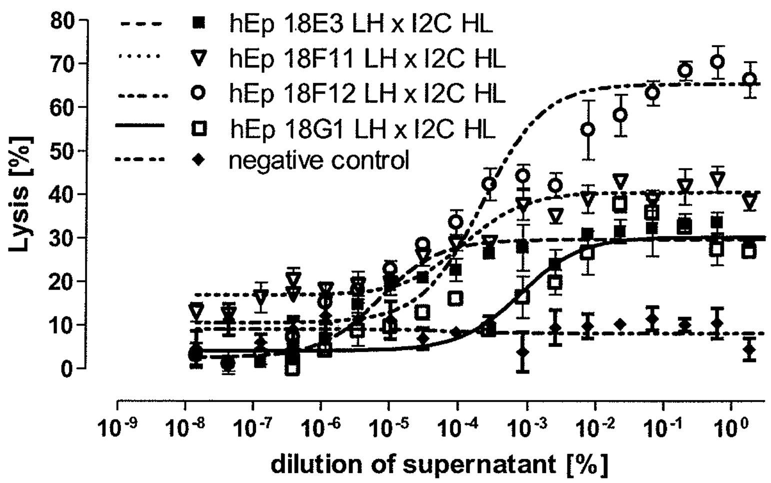


**B) Effector cells: 4119 LnPx**
**Target cells: CHO transfected with human EpCAM**

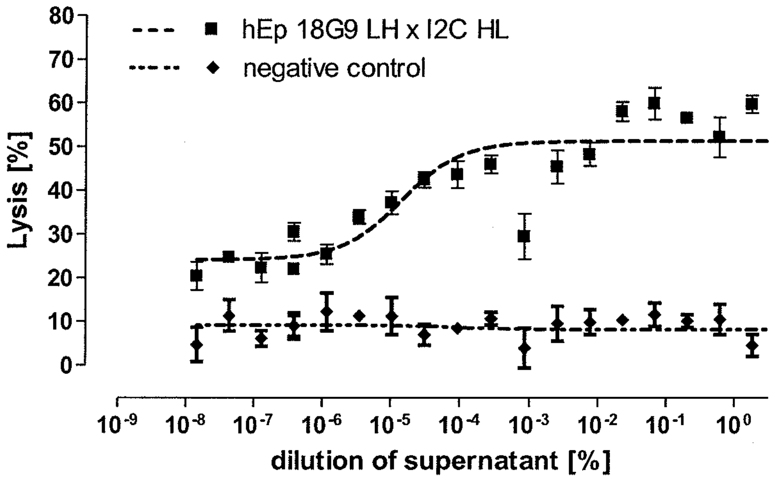

## Figure 66

A) **Effector cells: stimulated CD4/CD56 depleted human PBMC**
**Target cells: CHO transfected with human EpCAM**

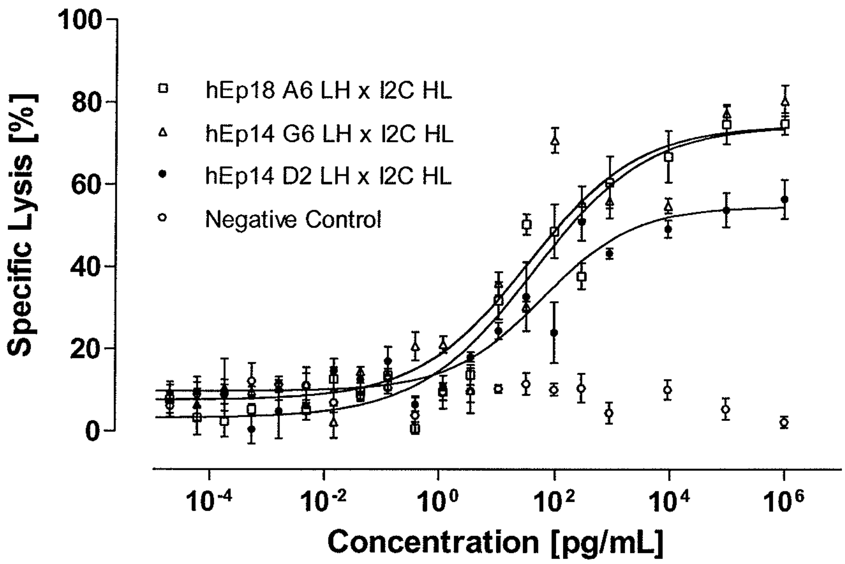


B) **Effector cells: 4119 LnPx**
**Target cells: CHO transfected with human EpCAM**

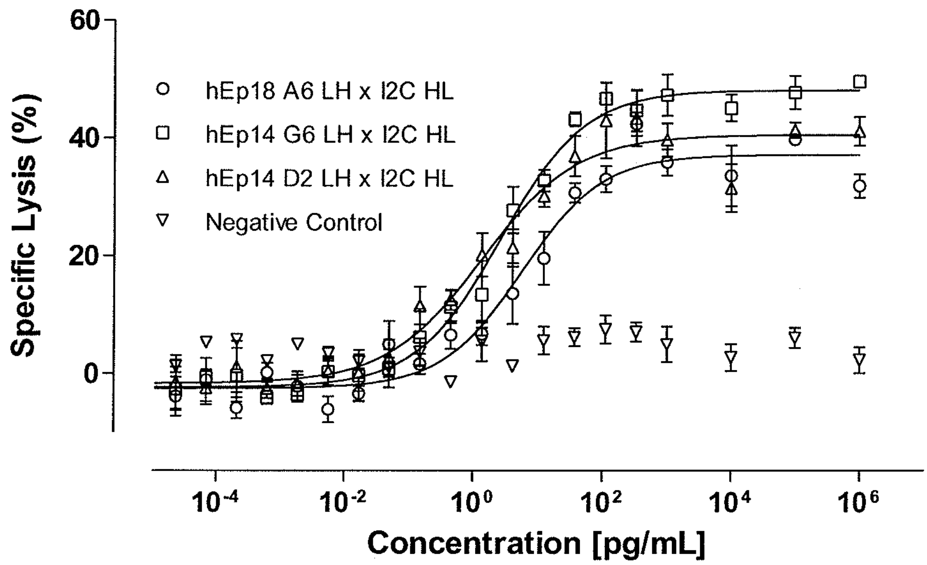

Figure 67

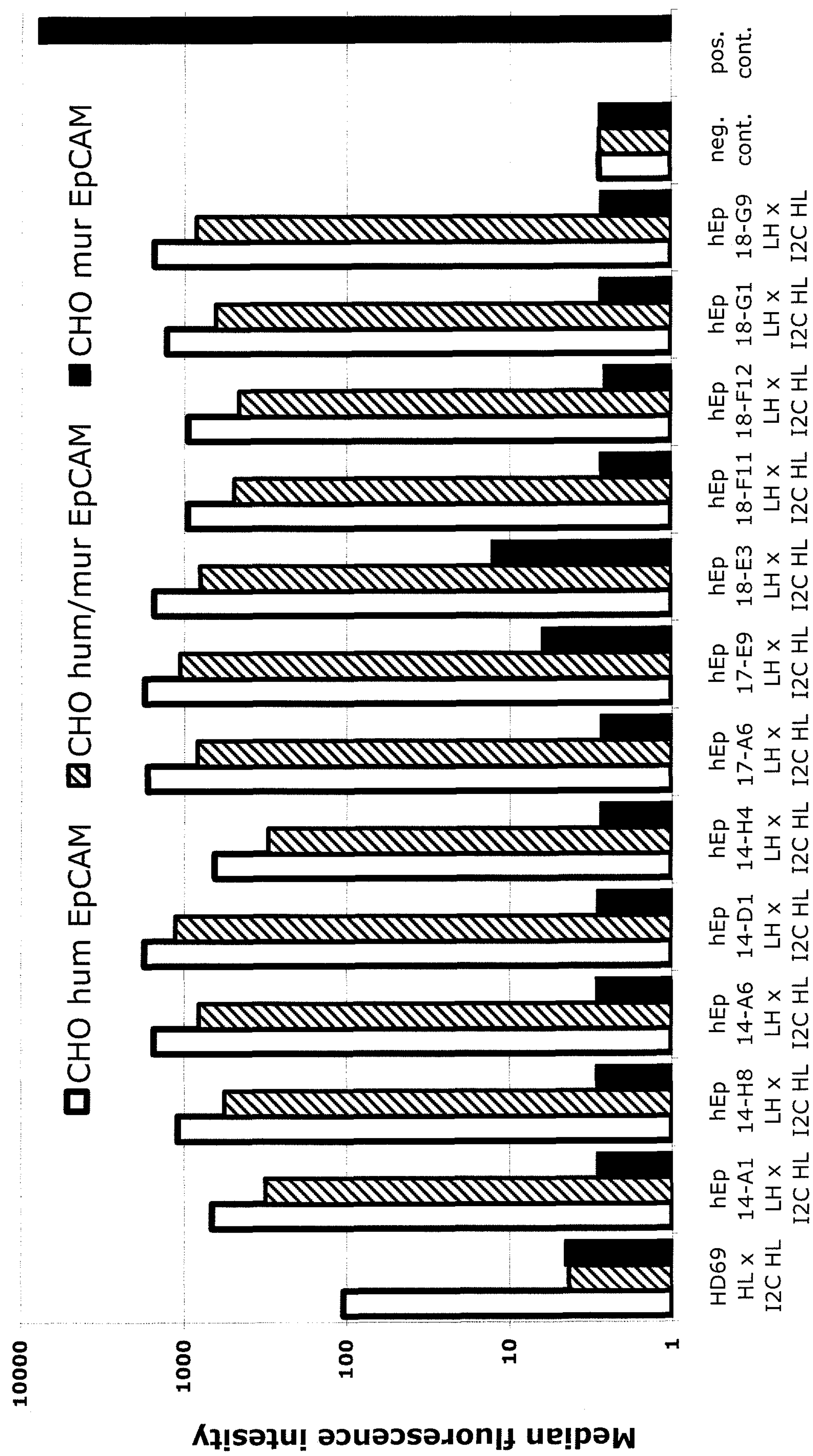
CHO hum EpCAM
CHO hum/mur EpCAM
CHO mur EpCAM
Median fluorescence intesity
10000
1000
100
10
1
HD69 HL x I2C HL
hEp 14-A1 LH x I2C HL
hEp 14-H8 LH x I2C HL
hEp 14-A6 LH x I2C HL
hEp 14-D1 LH x I2C HL
hEp 14-H4 LH x I2C HL
hEp 17-A6 LH x I2C HL
hEp 17-E9 LH x I2C HL
hEp 18-E3 LH x I2C HL
hEp 18-F11 LH x I2C HL
hEp 18-F12 LH x I2C HL
hEp 18-G1 LH x I2C HL
hEp 18-G9 LH x I2C HL
neg. cont.
pos. cont.

# Figure 68

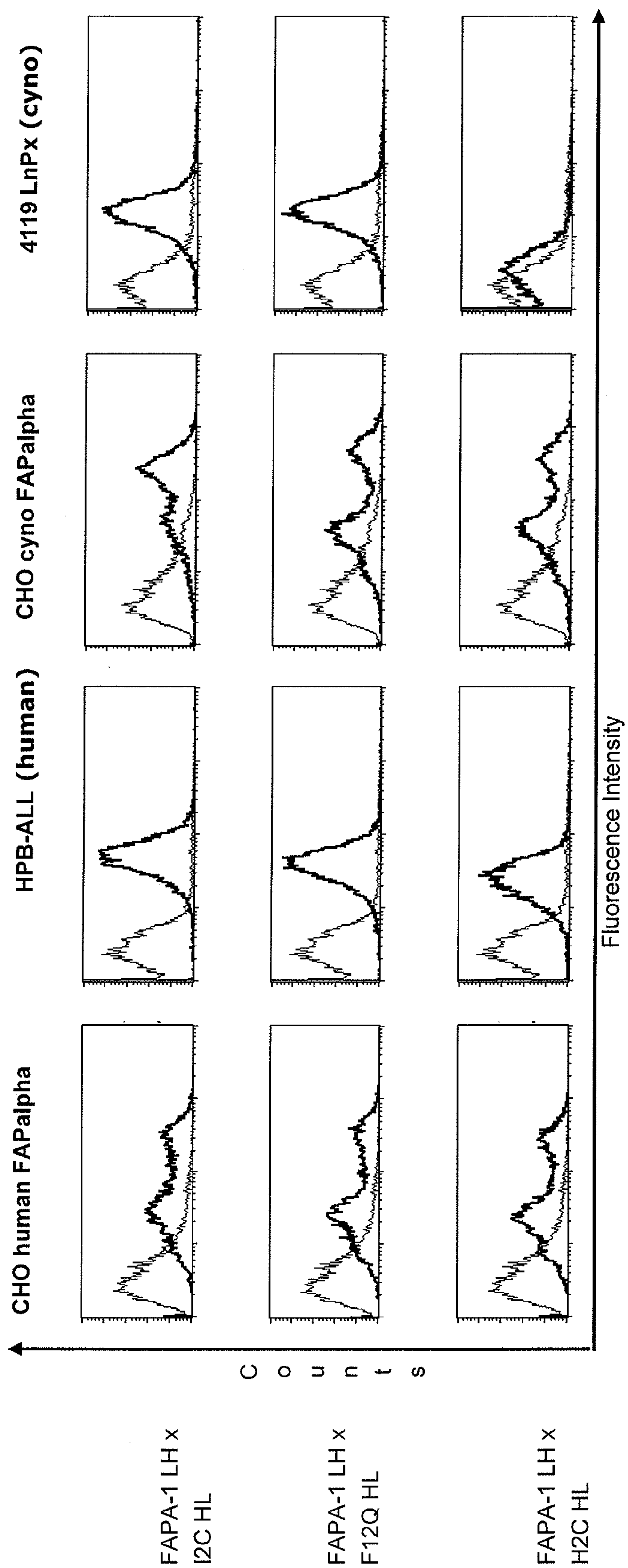
CHO human FAPalpha
HPB-ALL (human)
CHO cyno FAPalpha
4119 LnPx (cyno)
FAPA-1 LH x I2C HL
FAPA-1 LH x F12Q HL
FAPA-1 LH x H2C HL
Counts
Fluorescence Intensity

# Figure 69

Effector cells: stimulated CD4/CD56 depleted human PBMC
Target cells: CHO transfected with human FAPalpha
E:T ratio: 10:1

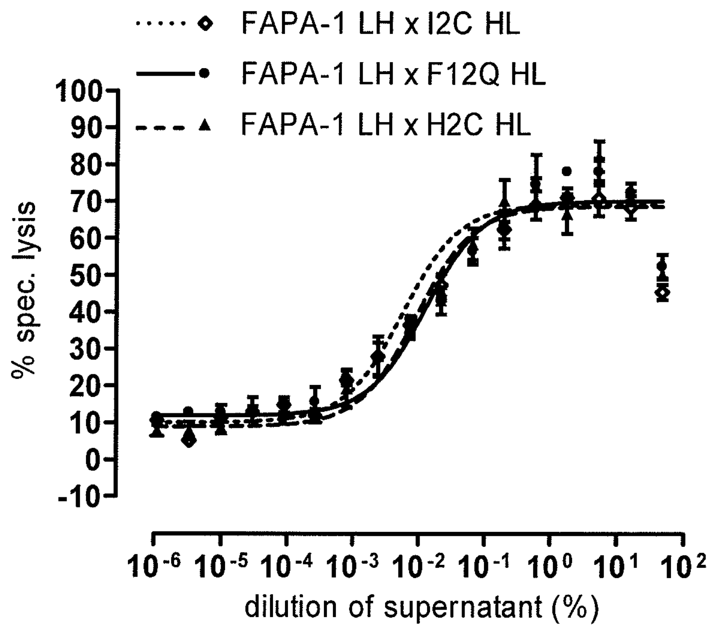


Effector cells: 4119 LnPx
Target cells: CHO transfected with macaque FAPalpha
E:T ratio: 10:1

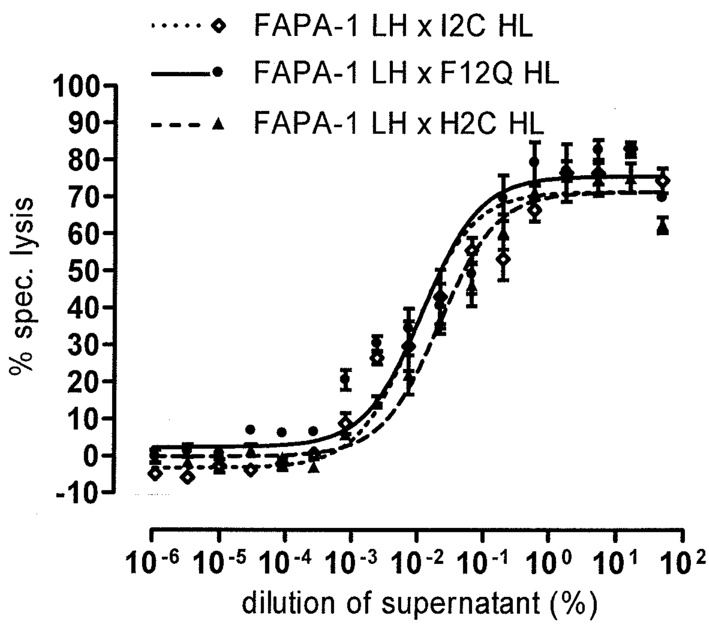

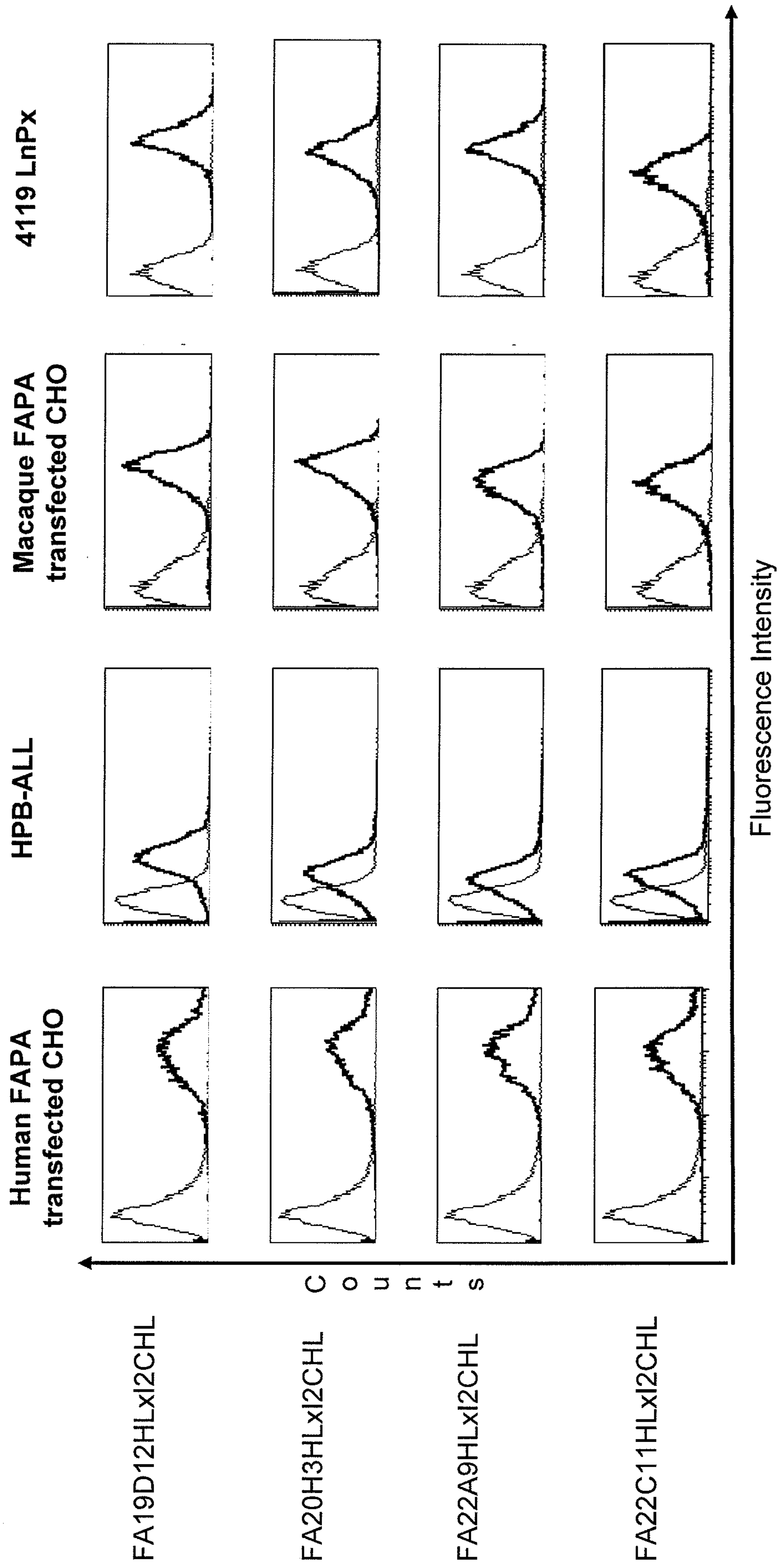
Human FAPA transfected CHO
HPB-ALL
Macaque FAPA transfected CHO
4119 LnPx
FA19D12HLxI2CHL
FA20H3HLxI2CHL
FA22A9HLxI2CHL
FA22C11HLxI2CHL
Counts
Fluorescence Intensity

Figure 70(1)

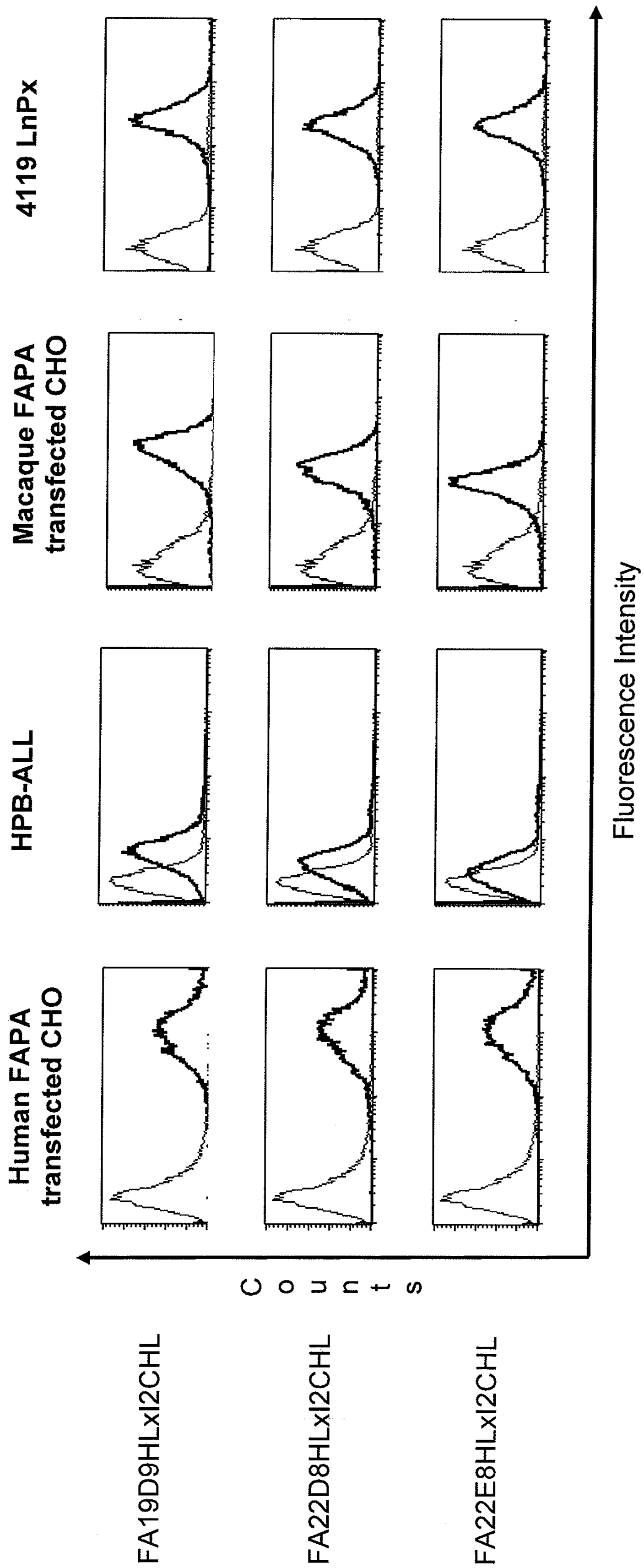

Human FAPA transfected CHO
HPB-ALL
Macaque FAPA transfected CHO
4119 LnPx
FA19D9HLxI2CHL
FA22D8HLxI2CHL
FA22E8HLxI2CHL
Counts
Fluorescence Intensity


Figure 70(2)

Effector cells: stimulated human CD4/CD56 depleted human PBMC
Target cells: CHO transfected with human FAPA
E:T ratio: 10:1

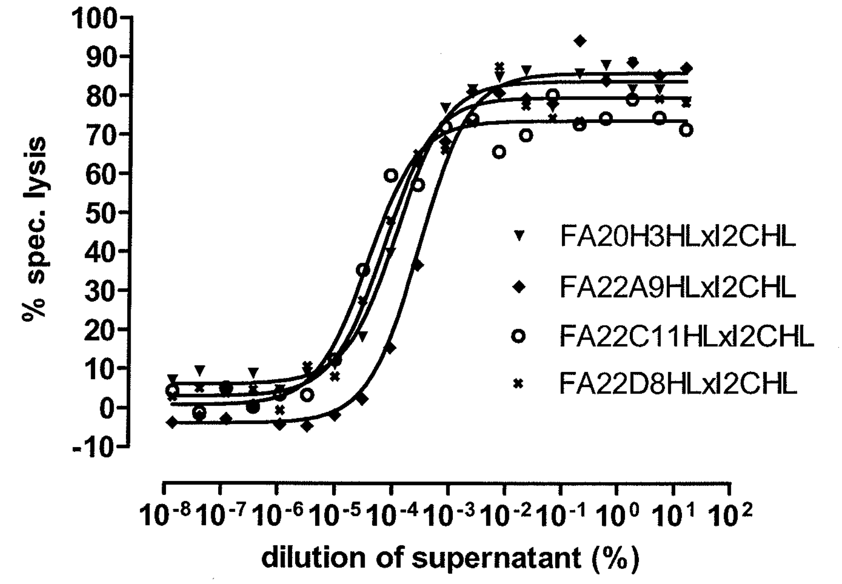


Effector cells: stimulated human CD4/CD56 depleted human PBMC
Target cells: CHO transfected with human FAPA
E:T ratio: 10:1

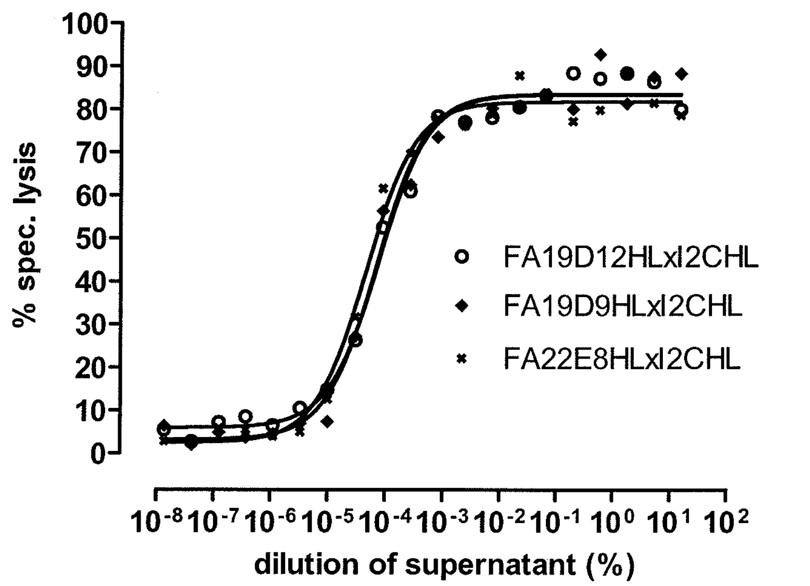


**Figure 71(1)**

Effector cells: 4119 LnPx
Target cells: CHO transfected with macaque FAPA
E:T ratio: 10:1

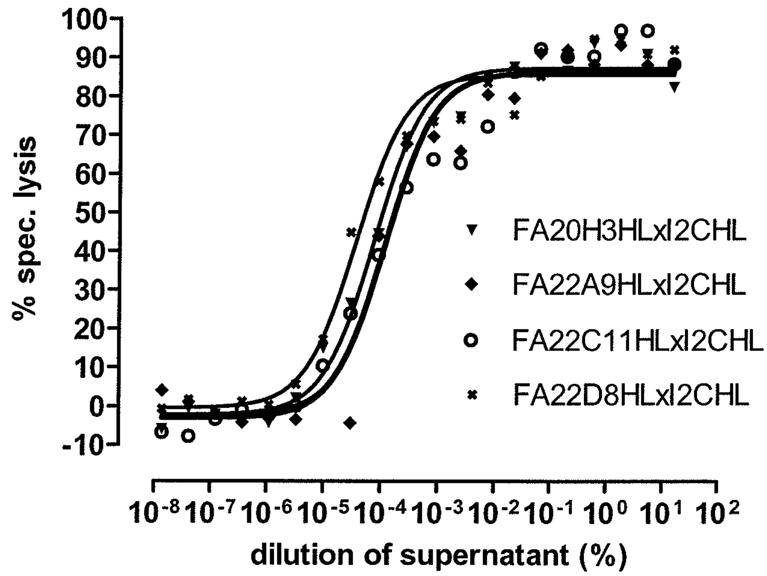


Effector cells: 4119 LnPx
Target cells: CHO transfected with macaque FAPA
E:T ratio: 10:1

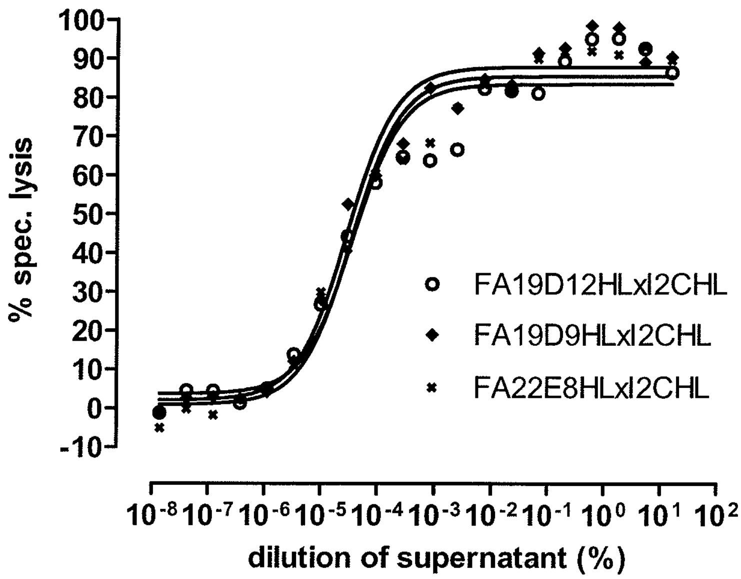


**Figure 71(2)**

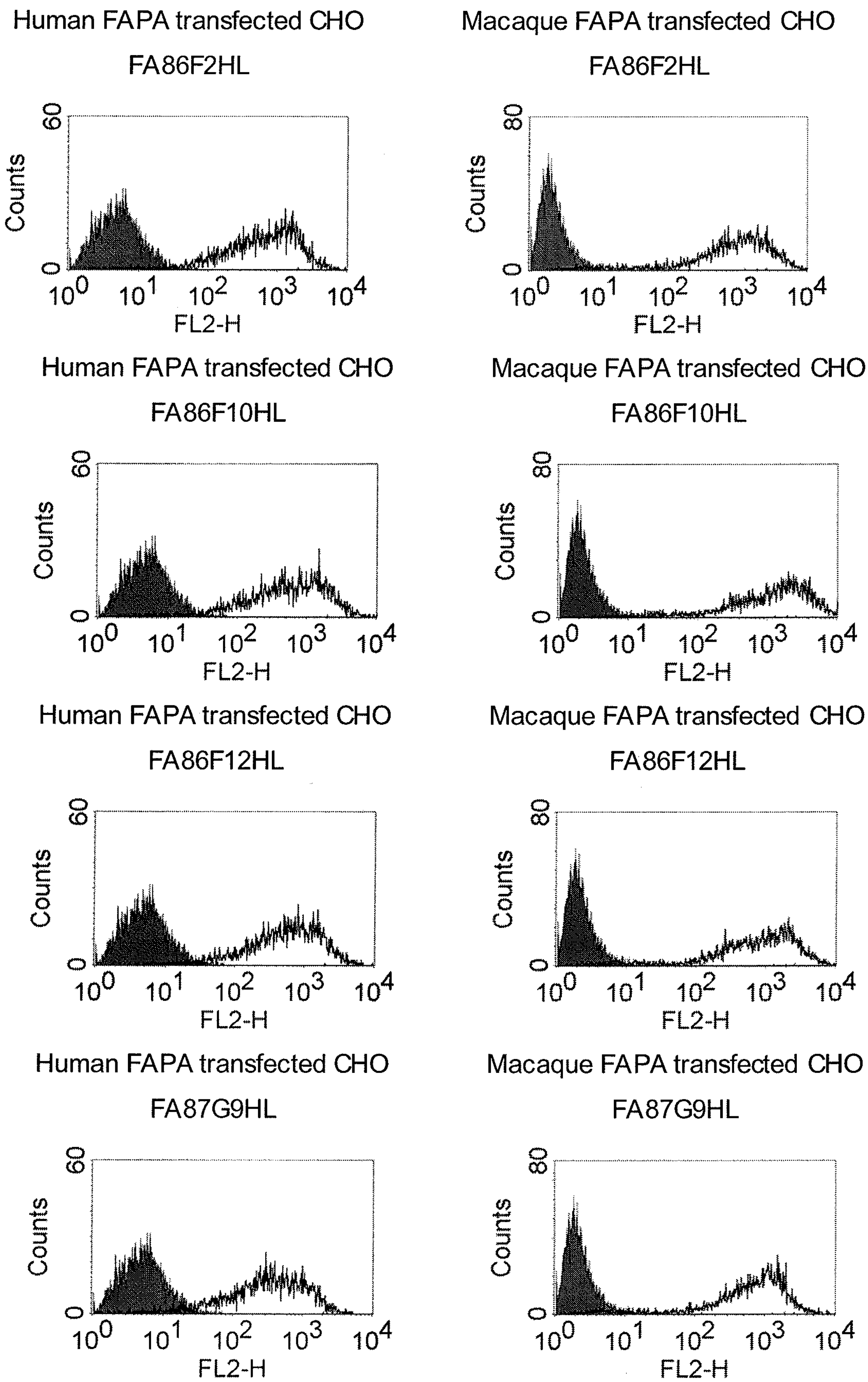

Human FAPA transfected CHO
FA86F2HL
Macaque FAPA transfected CHO
FA86F2HL
Human FAPA transfected CHO
FA86F10HL
Macaque FAPA transfected CHO
FA86F10HL
Human FAPA transfected CHO
FA86F12HL
Macaque FAPA transfected CHO
FA86F12HL
Human FAPA transfected CHO
FA87G9HL
Macaque FAPA transfected CHO
FA87G9HL
Counts
0
60
80
FL2-H
$10^0$
$10^1$
$10^2$
$10^3$
$10^4$


**Figure 72(1)**

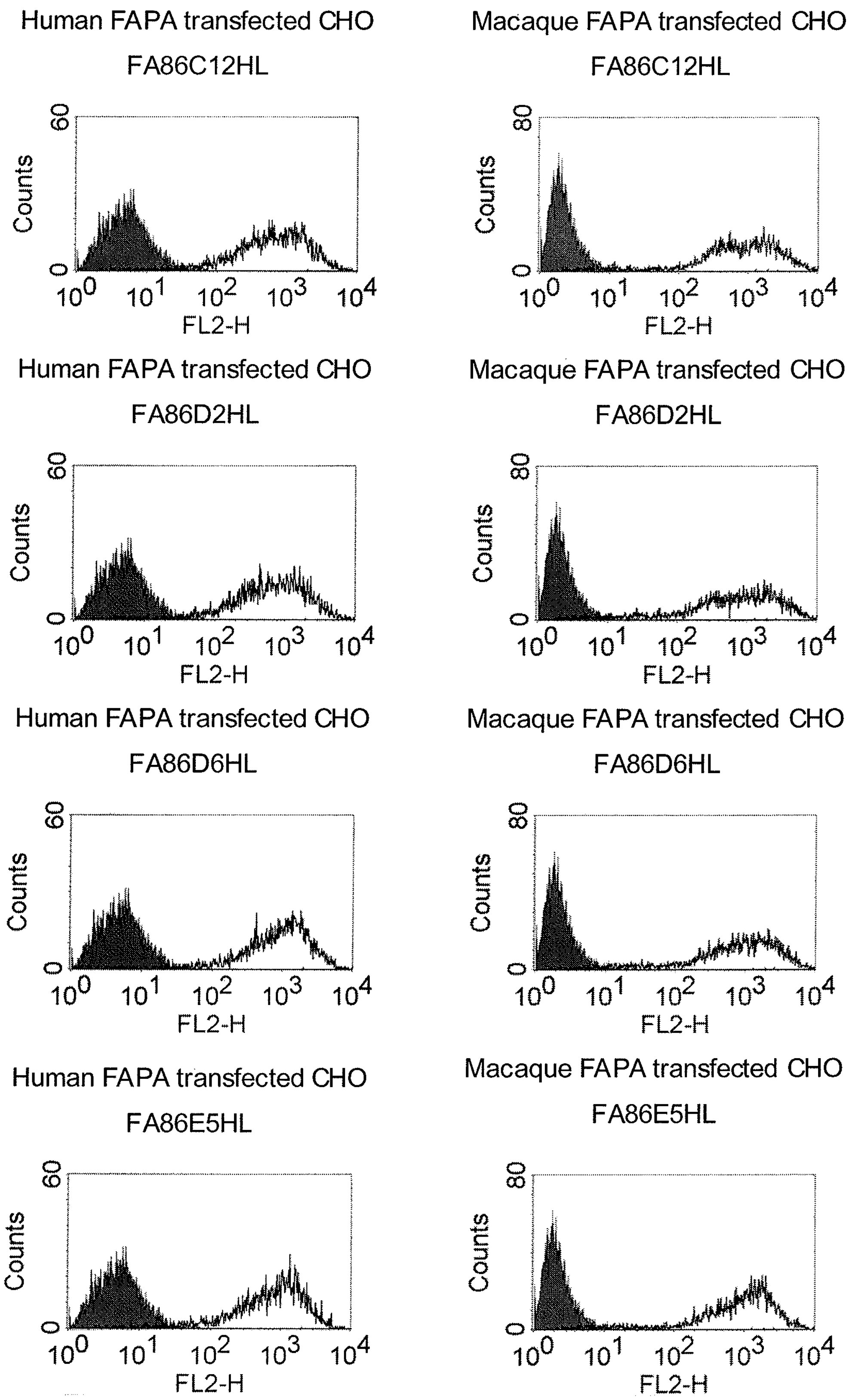
Human FAPA transfected CHO
FA86C12HL
Macaque FAPA transfected CHO
FA86C12HL
Human FAPA transfected CHO
FA86D2HL
Macaque FAPA transfected CHO
FA86D2HL
Human FAPA transfected CHO
FA86D6HL
Macaque FAPA transfected CHO
FA86D6HL
Human FAPA transfected CHO
FA86E5HL
Macaque FAPA transfected CHO
FA86E5HL
Counts
0
60
80
$10^0$ $10^1$ $10^2$ $10^3$ $10^4$
FL2-H

**Figure 72(2)**

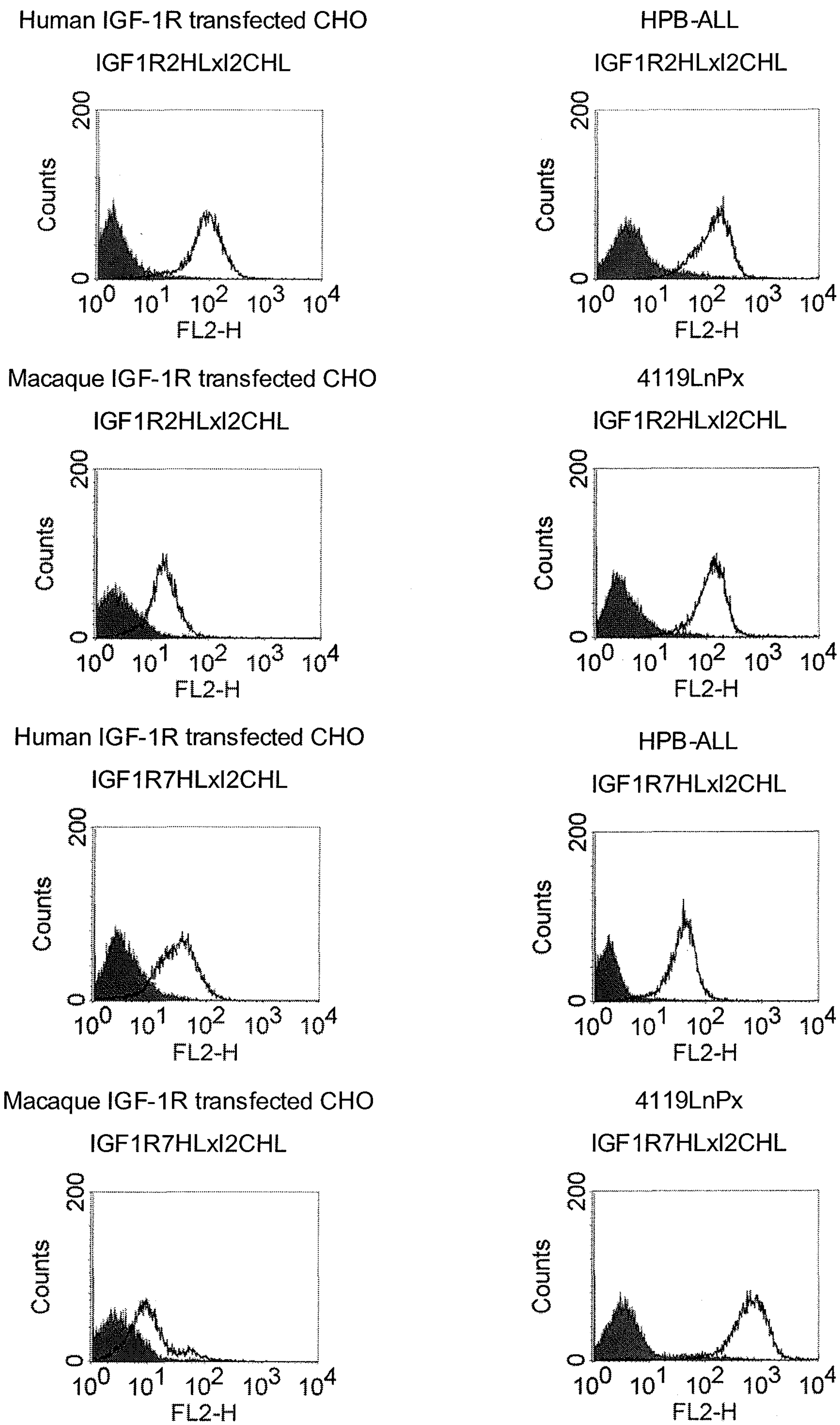
Human IGF-1R transfected CHO
IGF1R2HLxI2CHL
HPB-ALL
IGF1R2HLxI2CHL
Macaque IGF-1R transfected CHO
IGF1R2HLxI2CHL
4119LnPx
IGF1R2HLxI2CHL
Human IGF-1R transfected CHO
IGF1R7HLxI2CHL
HPB-ALL
IGF1R7HLxI2CHL
Macaque IGF-1R transfected CHO
IGF1R7HLxI2CHL
4119LnPx
IGF1R7HLxI2CHL
Counts
FL2-H

**Figure 73a**

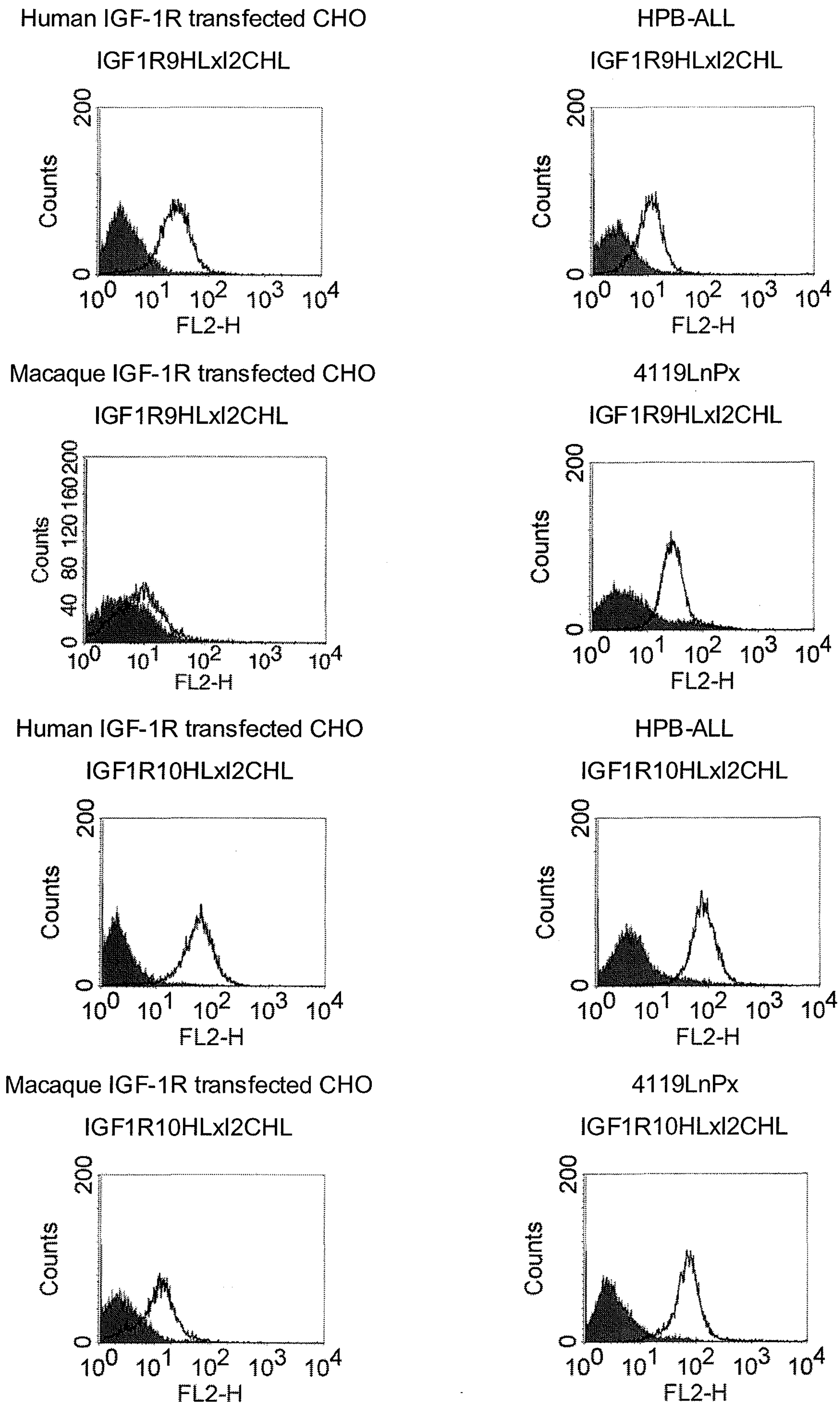
Human IGF-1R transfected CHO
IGF1R9HLxI2CHL
HPB-ALL
IGF1R9HLxI2CHL
Macaque IGF-1R transfected CHO
IGF1R9HLxI2CHL
4119LnPx
IGF1R9HLxI2CHL
Human IGF-1R transfected CHO
IGF1R10HLxI2CHL
HPB-ALL
IGF1R10HLxI2CHL
Macaque IGF-1R transfected CHO
IGF1R10HLxI2CHL
4119LnPx
IGF1R10HLxI2CHL
Counts
FL2-H
0
40
80
120
160
200
$10^0$
$10^1$
$10^2$
$10^3$
$10^4$

**Figure 73b**

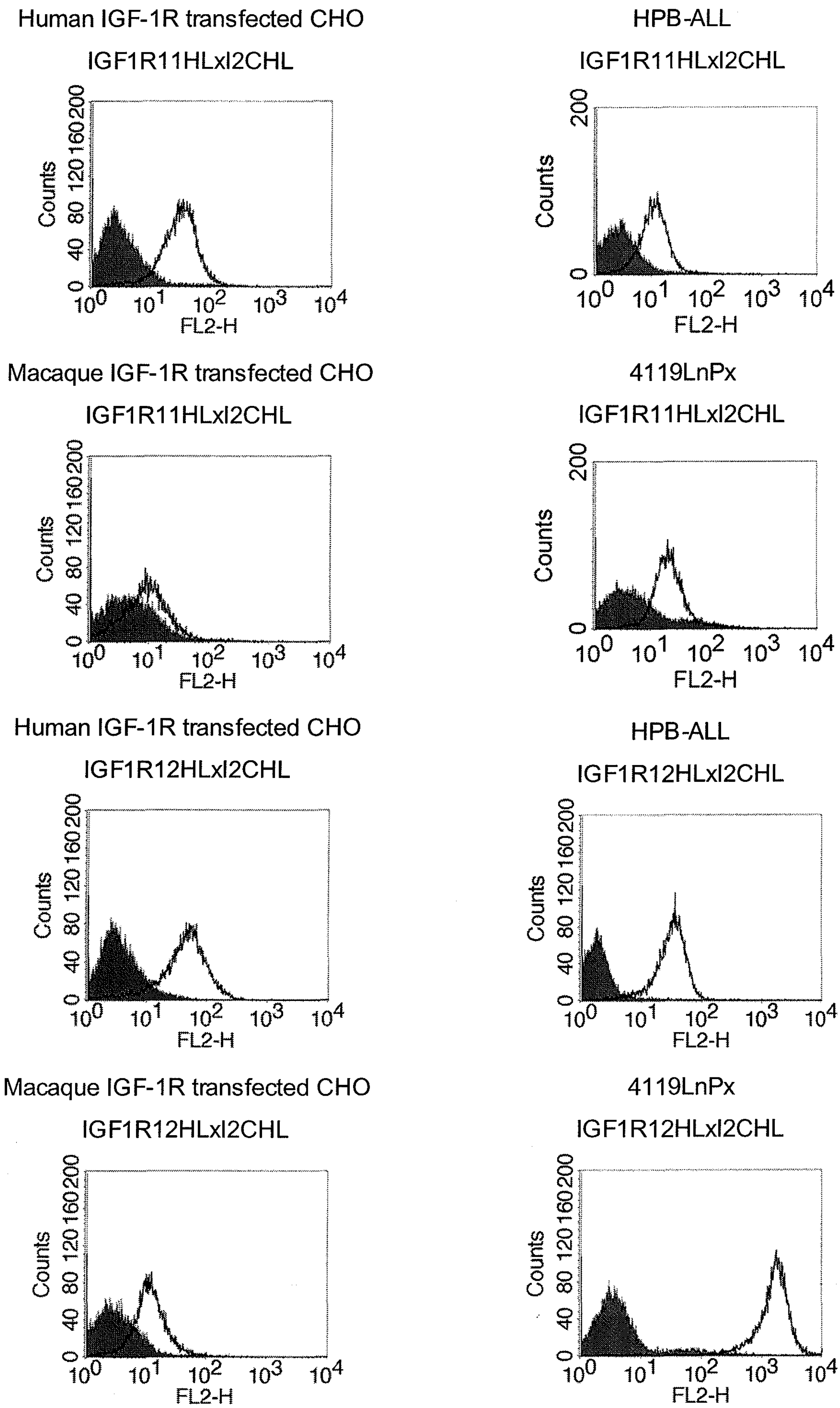
Human IGF-1R transfected CHO
IGF1R11HLxI2CHL
HPB-ALL
IGF1R11HLxI2CHL
Macaque IGF-1R transfected CHO
IGF1R11HLxI2CHL
4119LnPx
IGF1R11HLxI2CHL
Human IGF-1R transfected CHO
IGF1R12HLxI2CHL
HPB-ALL
IGF1R12HLxI2CHL
Macaque IGF-1R transfected CHO
IGF1R12HLxI2CHL
4119LnPx
IGF1R12HLxI2CHL
Counts
0 40 80 120 160 200
FL2-H
$10^0$ $10^1$ $10^2$ $10^3$ $10^4$

**Figure 73c**

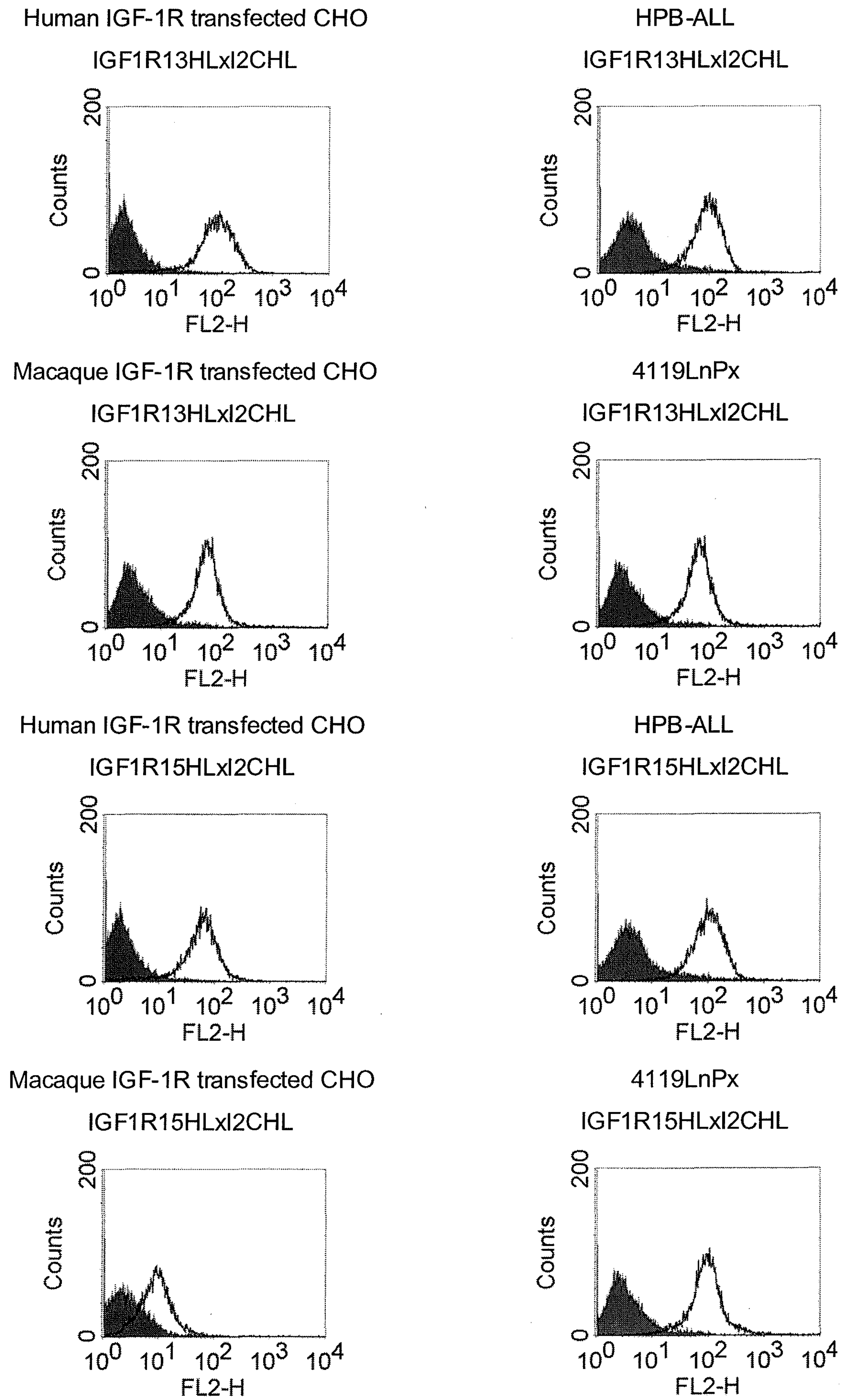
Human IGF-1R transfected CHO
IGF1R13HLxI2CHL
HPB-ALL
IGF1R13HLxI2CHL
Macaque IGF-1R transfected CHO
IGF1R13HLxI2CHL
4119LnPx
IGF1R13HLxI2CHL
Human IGF-1R transfected CHO
IGF1R15HLxI2CHL
HPB-ALL
IGF1R15HLxI2CHL
Macaque IGF-1R transfected CHO
IGF1R15HLxI2CHL
4119LnPx
IGF1R15HLxI2CHL
Counts
0
200
$10^0$ $10^1$ $10^2$ $10^3$ $10^4$
FL2-H

Figure 73d

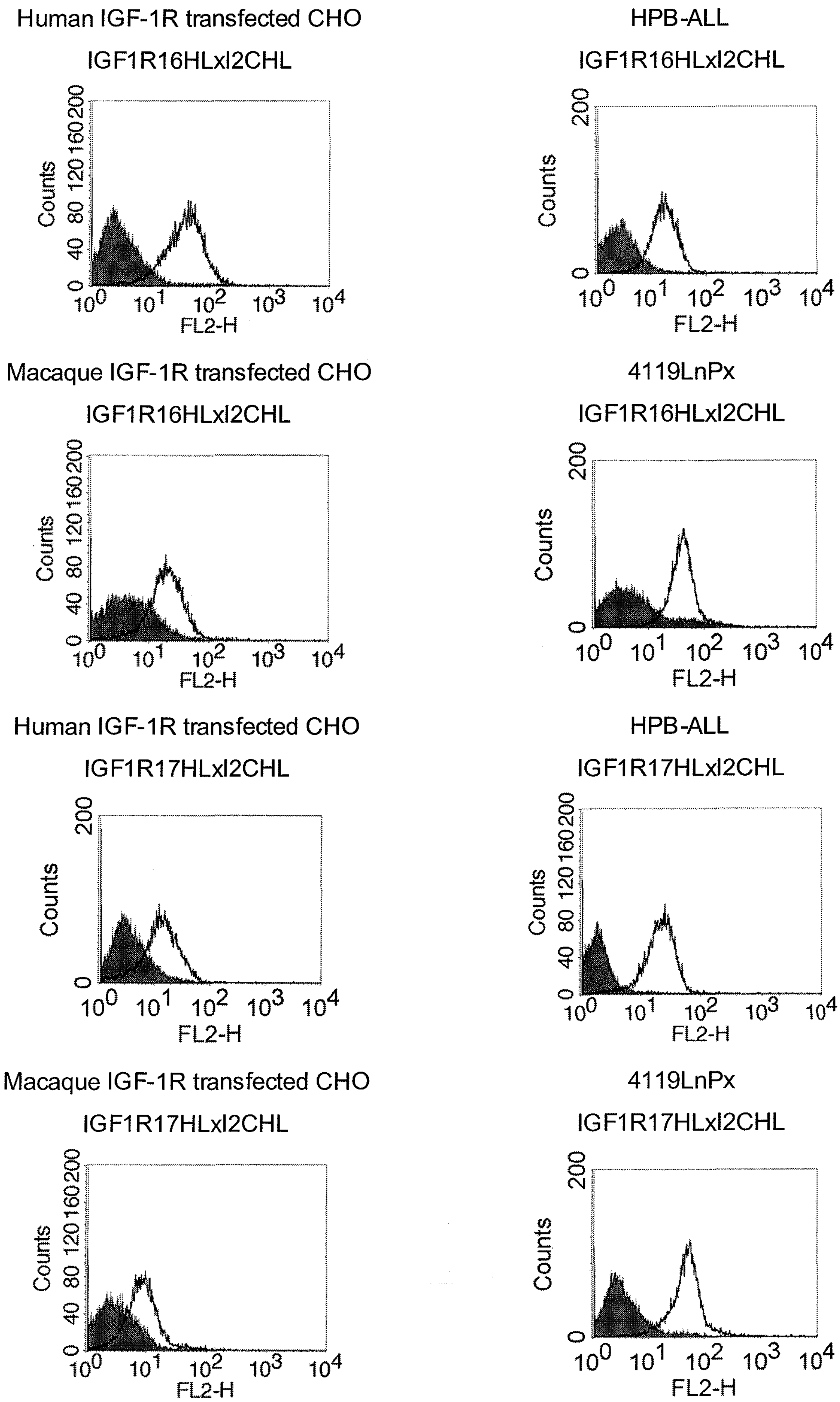
Human IGF-1R transfected CHO
IGF1R16HLxI2CHL
HPB-ALL
IGF1R16HLxI2CHL
Macaque IGF-1R transfected CHO
IGF1R16HLxI2CHL
4119LnPx
IGF1R16HLxI2CHL
Human IGF-1R transfected CHO
IGF1R17HLxI2CHL
HPB-ALL
IGF1R17HLxI2CHL
Macaque IGF-1R transfected CHO
IGF1R17HLxI2CHL
4119LnPx
IGF1R17HLxI2CHL
Counts
FL2-H

**Figure 73e**

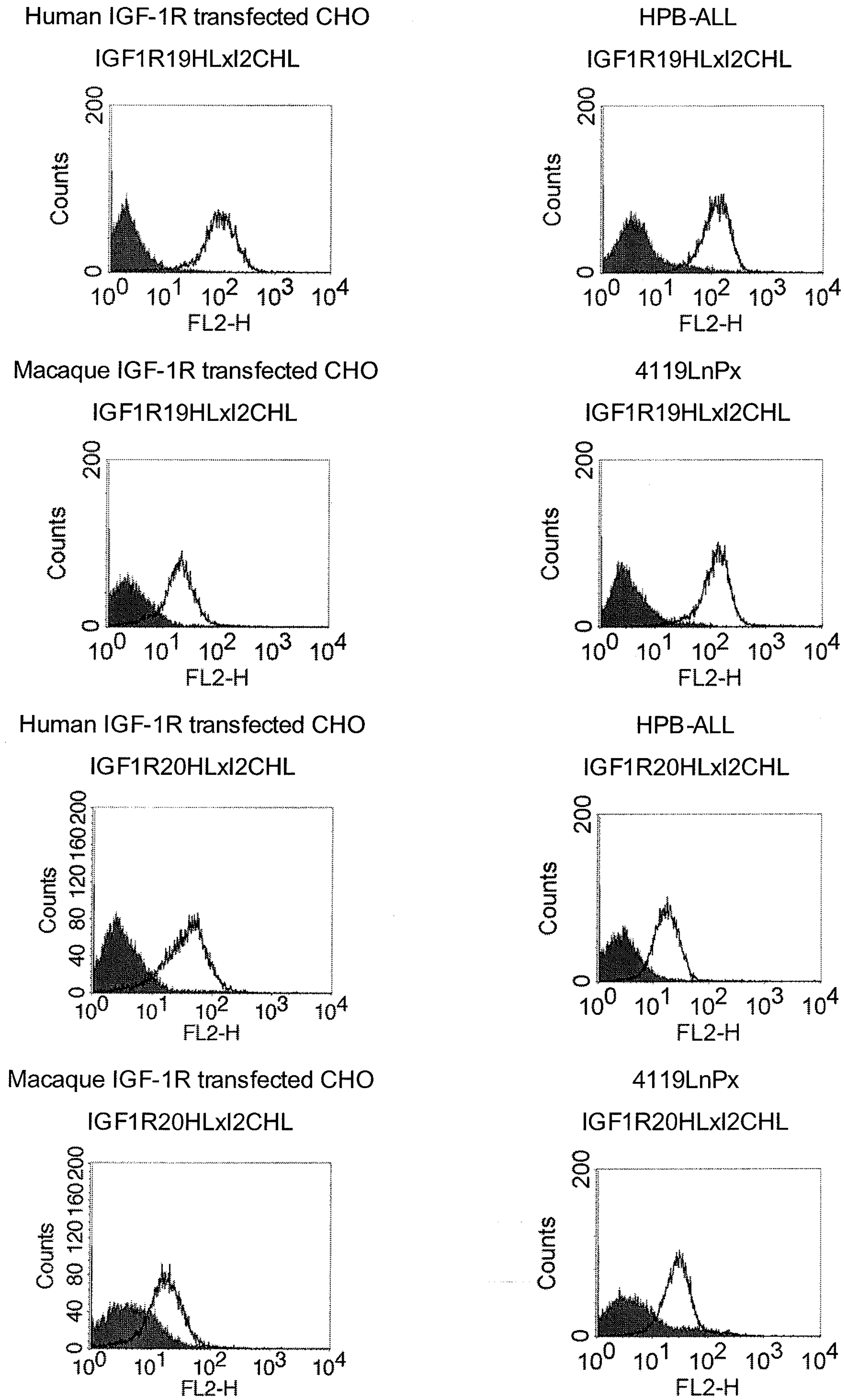
Human IGF-1R transfected CHO
IGF1R19HLxI2CHL
HPB-ALL
IGF1R19HLxI2CHL
Macaque IGF-1R transfected CHO
IGF1R19HLxI2CHL
4119LnPx
IGF1R19HLxI2CHL
Human IGF-1R transfected CHO
IGF1R20HLxI2CHL
HPB-ALL
IGF1R20HLxI2CHL
Macaque IGF-1R transfected CHO
IGF1R20HLxI2CHL
4119LnPx
IGF1R20HLxI2CHL
Counts
FL2-H

**Figure 73f**

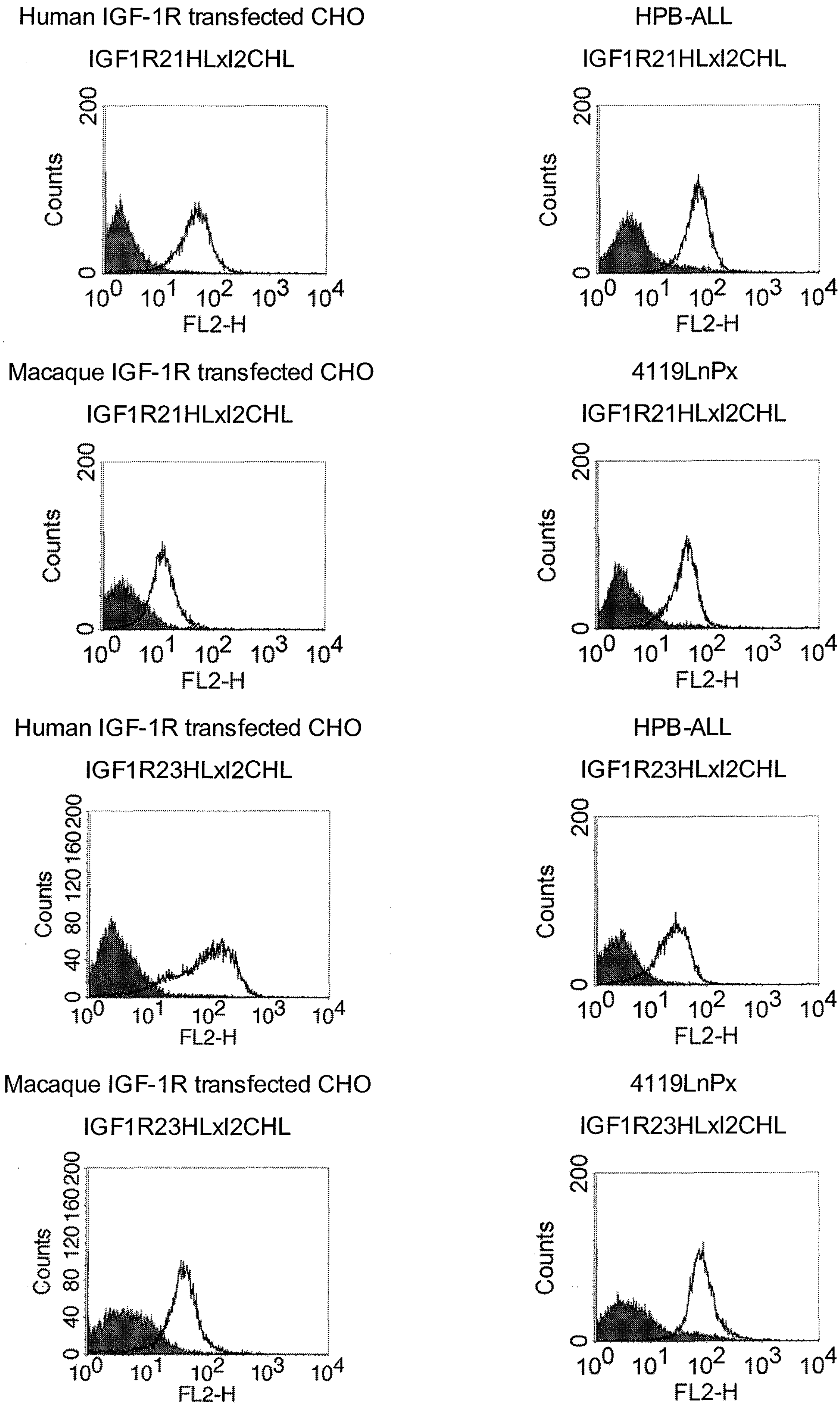
Human IGF-1R transfected CHO
IGF1R21HLxI2CHL
HPB-ALL
IGF1R21HLxI2CHL
Macaque IGF-1R transfected CHO
IGF1R21HLxI2CHL
4119LnPx
IGF1R21HLxI2CHL
Human IGF-1R transfected CHO
IGF1R23HLxI2CHL
HPB-ALL
IGF1R23HLxI2CHL
Macaque IGF-1R transfected CHO
IGF1R23HLxI2CHL
4119LnPx
IGF1R23HLxI2CHL
Counts
FL2-H

**Figure 73g**

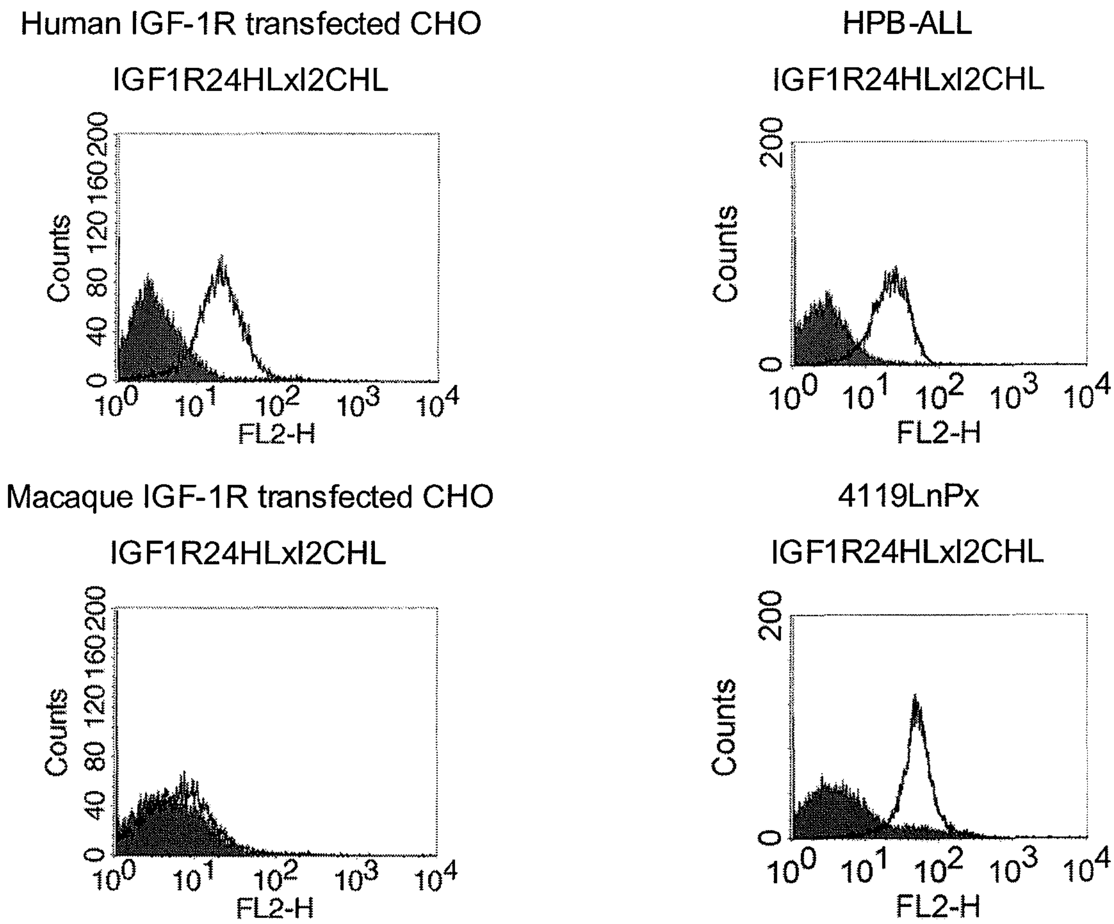
Human IGF-1R transfected CHO
IGF1R24HLxI2CHL
HPB-ALL
IGF1R24HLxI2CHL
Macaque IGF-1R transfected CHO
IGF1R24HLxI2CHL
4119LnPx
IGF1R24HLxI2CHL
Counts
FL2-H
0
40
80
120
160
200
$10^0$
$10^1$
$10^2$
$10^3$
$10^4$

Figure 73h

Effector T cells: stimulated CD4/CD56 depleted human PBMC

Target cells: CHO transfected with human IGF-1R

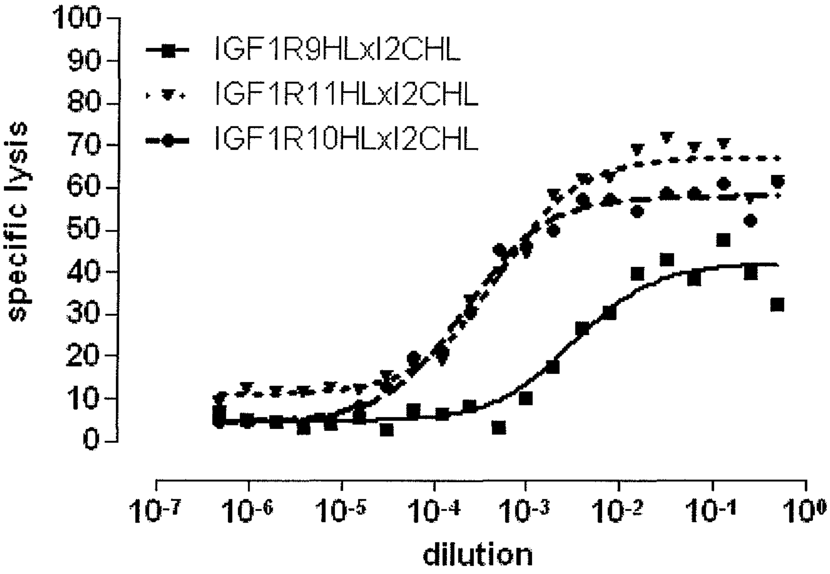


Effector T cells: macaque 4119 LnPx

Target cells: CHO transfected with macaque IGF-1R

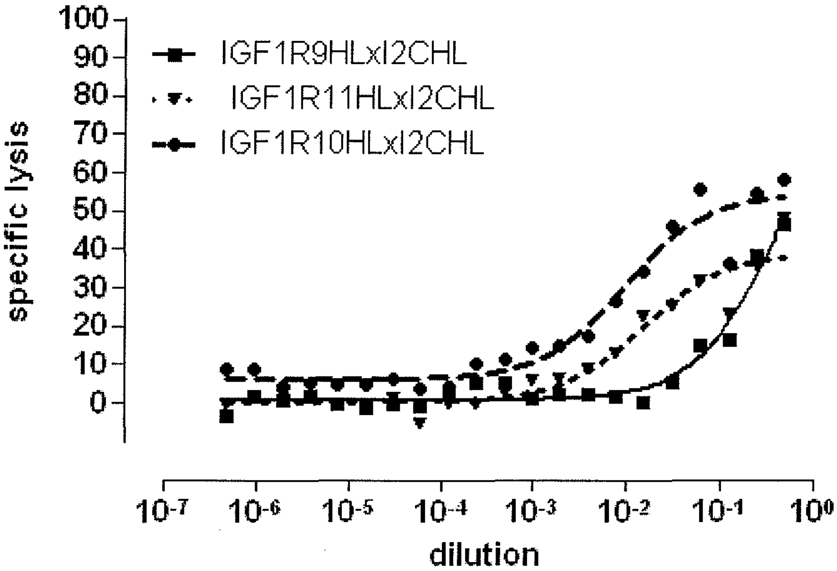


**Figure 74a**

Effector T cells: stimulated CD4/CD56 depleted human PBMC
Target cells: CHO transfected with human IGF-1R

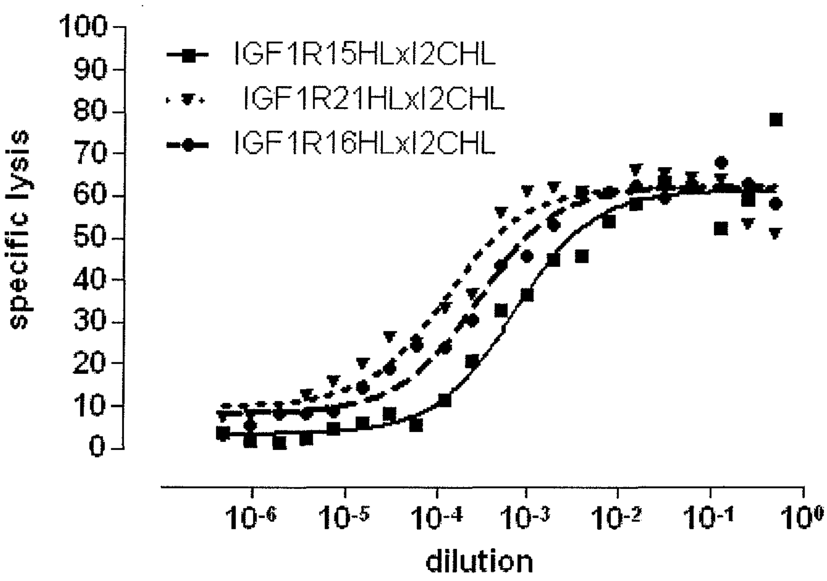


Effector T cells: macaque 4119 LnPx
Target cells: CHO transfected with macaque IGF-1R

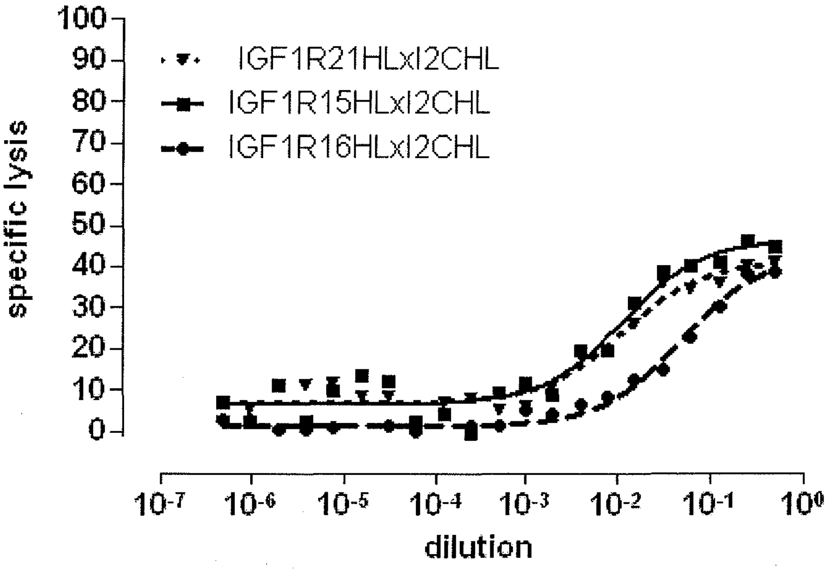


**Figure 74b**

Effector T cells: stimulated CD4/CD56 depleted human PBMC

Target cells: CHO transfected with human IGF-1R

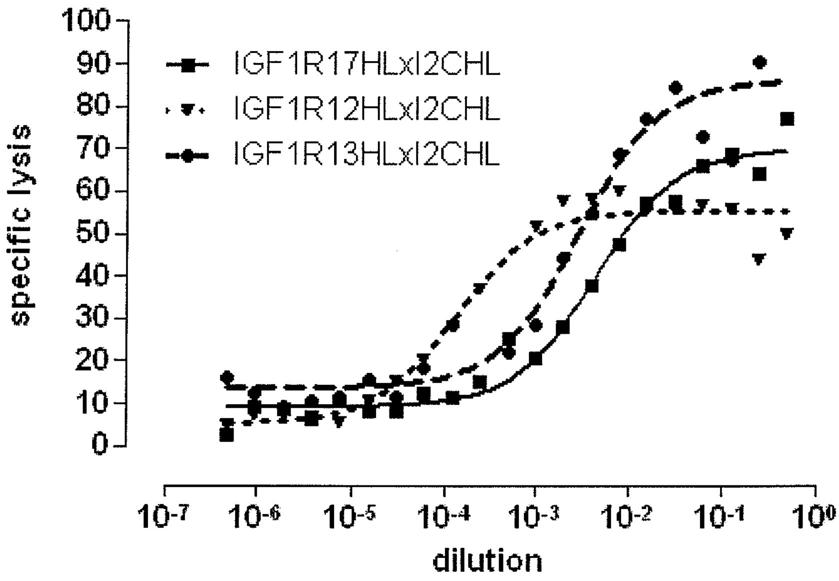


Effector T cells: macaque 4119 LnPx

Target cells: CHO transfected with macaque IGF-1R

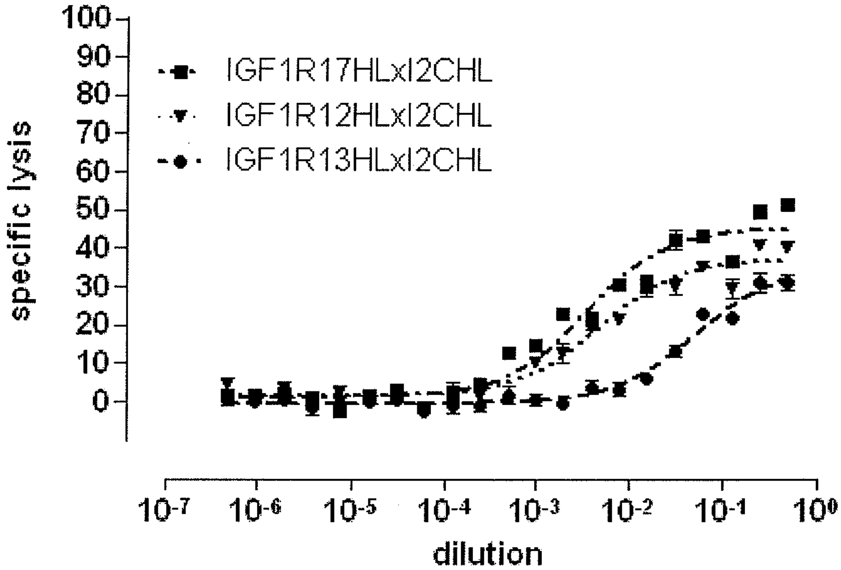


**Figure 74c**

Effector T cells: stimulated CD4/CD56 depleted human PBMC

Target cells: CHO transfected with human IGF-1R

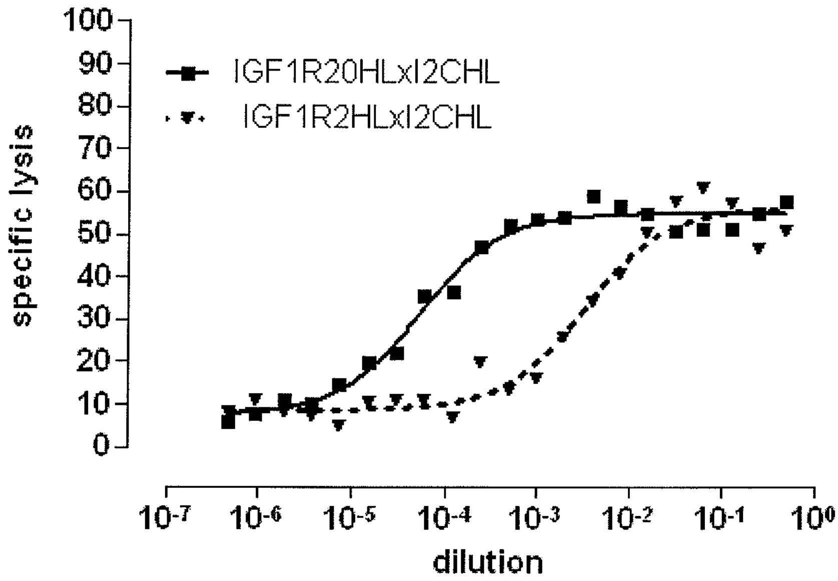


Effector T cells: macaque 4119 LnPx

Target cells: CHO transfected with macaque IGF-1R

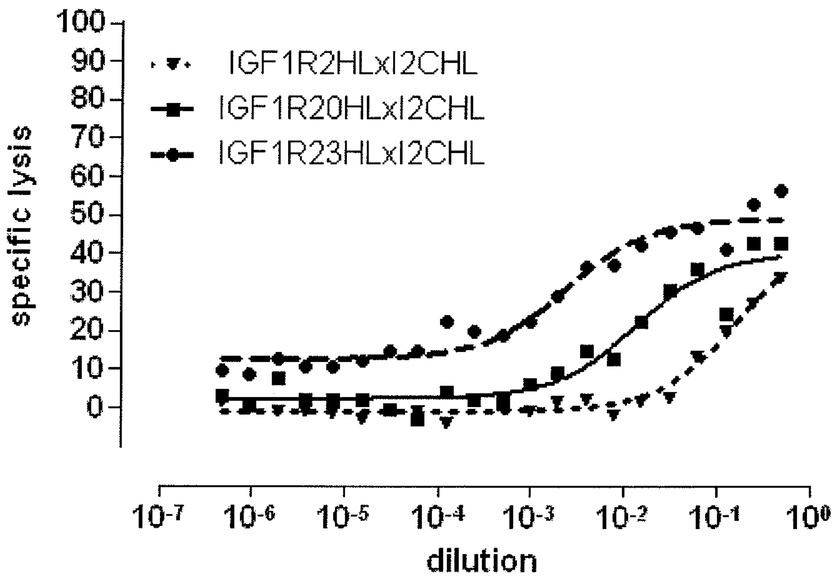


**Figure 74d**

Effector T cells: stimulated CD4/CD56 depleted human PBMC

Target cells: CHO transfected with human IGF-1R

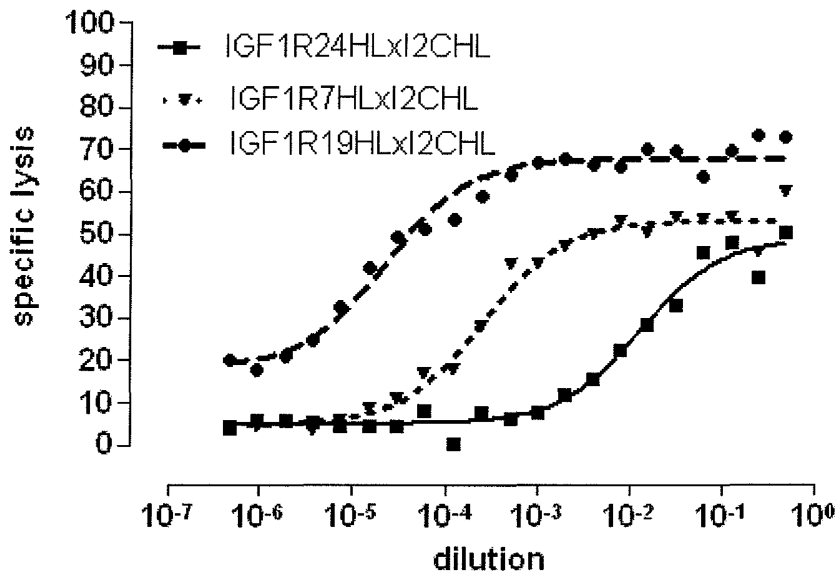


Effector T cells: macaque 4119 LnPx

Target cells: CHO transfected with macaque IGF-1R

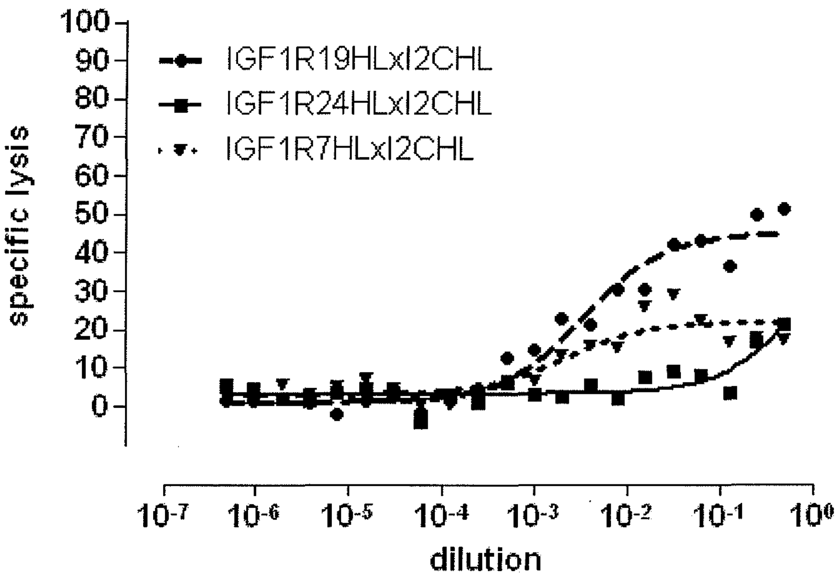


**Figure 74e**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005118635 A, Similarly
- WO 2007033230 A
- WO 2007042261 A
- WO 2004106381 A
- WO 2008119565 A
- WO 2008119566 A
- WO 2008119567 A
- WO 2006008096 A
- WO 9954440 A
- WO 04106380 A
- EP 623679 B1
- US 4946778 A
- WO 2004106380 A
- WO 9109968 A
- US 6407213 B
- WO 9420627 A
- WO 9429469 A
- WO 9700957 A
- US 5580859 A
- US 5589466 A
- WO 010903 A


### Non-patent literature cited in the description


- **DAVIS ; BJORKMAN.** *Nature,* 1988, vol. 334, 395-402
- **CALL.** *Cell,* 2002, vol. 111, 967-979
- **ALARCON.** *Immunol. Rev.,* 2003, vol. 191, 38-46
- *Malissen Immunol. Rev.,* 2003, vol. 191, 7-27
- **DAVIS.** *Cell,* 2002, vol. 110, 285-287
- **DAVIS ; VAN DER MERWE.** *Curr. Biol.,* 2001, vol. 11, R289-R291
- **DAVIS.** *Nat. Immunol.,* 2003, vol. 4, 217-224
- **GIL.** *J. Biol. Chem.,* 2001, vol. 276, 11174-11179
- **GIL.** *Cell,* 2002, vol. 109, 901-912
- **BERKHOUT.** *J. Biol. Chem.,* 1988, vol. 263, 8528-8536
- **WANG.** *J. Exp. Med.,* 1998, vol. 188, 1375-1380
- **KAPPES.** *Curr. Opin. Immunol.,* 1995, vol. 7, 441-447
- **SUN.** *Cell,* 2001, vol. 105, 913-923
- **BORROTO.** *J. Biol. Chem.,* 1998, vol. 273, 12807-12816
- **MANOLIOS.** *Eur. J. Immunol.,* 1994, vol. 24, 84-92
- **MANOLIOS ; LI.** *Immunol. Cell Biol.,* 1995, vol. 73, 532-536
- **KJER-NIELSEN.** *PNAS,* 2004, vol. 101, 7675-7680
- **SGRO.** *Toxicology,* 1995, vol. 105, 23-29
- **CHATENOUD.** *Clin. Transplant,* 1993, vol. 7, 422-430
- **CHATENOUD.** *Nat. Rev. Immunol.,* 2003, vol. 3, 123-132
- **KUMAR.** *Transplant. Proc.,* 1998, vol. 30, 1351-1352
- **UTSET.** *J. Rheumatol.,* 2002, vol. 29, 1907-1913
- **SMITH.** *J. Exp. Med.,* 1997, vol. 185, 1413-1422
- **VAN WAUVE.** *J. Immunol.,* 1980, vol. 124, 2708-18
- **WONG.** *Transplantation,* 1990, vol. 50, 683-9
- **TUNNACLIFFE.** *Int. Immunol.,* 1989, vol. 1, 546-50
- **SALMERON.** *J. Immunol.,* 1991, vol. 147, 3047-52
- **WUSSOW.** *J. Immunol.,* 1981, vol. 127, 1197
- **PALACIOUS.** *J. Immunol.,* 1982, vol. 128, 337
- **KUNICKA.** *Lymphocyte Typing II,* 1986, 223
- **LEEWENBERG.** *J. Immunol.,* 1985, vol. 134, 3770
- **PHILLIPS.** *J. Immunol.,* 1986, vol. 136, 1579
- **PLATSOUCAS.** *Proc. Natl. Acad. Sci. USA,* 1981, vol. 78, 4500
- **ITOH.** *Cell. Immunol.,* 1987, vol. 108, 283-96
- **MENTZER.** *J. Immunol.,* 1985, vol. 135, 34
- **LANDEGREN.** *J. Exp. Med.,* 1982, vol. 155, 1579
- **CHOI.** *Eur. J. Immunol.,* 2001, vol. 31, 94-106
- **XU.** *Cell Immunol.,* 2000, vol. 200, 16-26
- **KIMBALL.** *Transpl. Immunol.,* 1995, vol. 3, 212-221
- **SELA.** *Science,* 1969, vol. 166, 1365
- **LAVER.** *Cell,* 1990, vol. 61, 553-6
- **LAW.** *Int. Immunol.,* 2002, vol. 14, 389-400

- **COULIE.** *Eur. J. Immunol.,* 1991, vol. 21, 1703-9
- **RISUENO.** *Blood,* 2005, vol. 106, 601-8
- **SANDUSKY et al.** *J. Med. Primatol.,* 1986, vol. 15, 441-451
- **UDA et al.** *J. Med. Primatol.,* 2001, vol. 30, 141-147
- **UDA et al.** *J Med Primatol.,* 2003, vol. 32, 105-10
- **UDA et al.** *J Med Primatol.,* 2004, vol. 33, 34-7
- **IM et al.** *Biochem Biophys Res Comm,* 2006, vol. 339 (1), 305-312
- **WUEST et al.** *J. Biotechnology,* 2001, vol. 92 (2), 159-168
- **BARGOU et al.** *Science,* 2008, vol. 321, 974-7
- **HUBERT et al.** *PNAS,* 1999, vol. 96, 14523-14528
- **ISRAELI et al.** *Cancer Res.,* 1993, vol. 53, 227-230
- **REITER et al.** *Proc. Nat. Acad. Sci.,* 1998, vol. 95, 1735-1740
- **CLASSON ; COVERDALE.** *PNAS,* 1994, vol. 91, 5296-5300
- **AMOUI et al.** *Eur. J. Immunol.,* 1997, vol. 27, 1881-86
- **SCHRIJVERS et al.** *Exp. Cell. Res.,* 1991, vol. 196, 264-69
- **GU et al.** *Oncogene,* 2000, vol. 19, 1288-96
- **ROSS et al.** *Cancer Res.,* 2002, vol. 62, 2546-53
- **AMARA et al.** *Cancer Res.,* 2001, vol. 61, 4660-4665
- **ARGANI et al.** *Cancer Res.,* 2001, vol. 61, 4320-24
- **HAAGEN.** *Clin Exp Immunol,* 1992, vol. 90 (14), 368-75
- **UCKUN.** *Proc. Natl. Acad. Sci. USA,* 1988, vol. 85, 8603-7
- **TRUSOLINO, L. ; COMOGLIO, P. M.** *Nat. Rev. Cancer,* 2002, vol. 2, 289-300
- **SCHMIDT et al.** *Nat. Genet.,* 1997, vol. 16, 68-73
- **KIM et al.** *J. Med. Genet.,* 2003, vol. 40, e97
- **DI RENZO et al.** *Clin. Cancer Res.,* 1995, vol. 1, 147-154
- *Giordano,* 1989, vol. 339, 155-156
- **BIRCHMEIER et al.** *Nature,* 2003, vol. 4, 915-25
- **GANDINO et al.** *J. Biol. Chem.,* 1994, vol. 269, 1815-20
- **PESCHARD et al.** *Mol. Cell,* 2001, vol. 8, 995-1004
- **MAINA et al.** *Cell,* 1996, vol. 87, 531-542
- **PONZETTO et al.** *Cell,* 1994, vol. 77, 261-71
- **ST. CROIX et al.** *Science,* 2000, vol. 289, 1197-1202
- **CARSON-WALTER et al.** *Cancer Res.,* 2001, vol. 61, 6649-6655
- **CHRISTIAN et al.** *J. Biol. Chem.,* 2001, vol. 276, 7408-7414
- **OPAVSKY et al.** *J. Biol. Chem.,* 2001, vol. 276, 38795-38807
- **RETTIG et al.** *PNAS,* 1992, vol. 89, 10832-36
- **BRADY et al.** *J. Neuropathol. Exp. Neurol.,* 2004, vol. 63, 1274-83
- **WENT et al.** *Br. J. Cancer,* vol. 94 (20006), 128-135
- **MUNZ et al.** *Oncogene,* 2004, vol. 23, 5748-58
- **OSTA et al.** *Cancer Res.,* 2004, vol. 64, 5818-24
- **SPIZZO et al.** *Gynecol. Oncol.,* 2006, vol. 103, 483-8
- **OBERNEDER et al.** *Eur. J. Cancer,* 2006, vol. 42, 2530-8
- **SCANLAN et al.** *Proc. Nat. Acad. Sci.,* 1994, vol. 91, 5657-5661
- **GOLDSTEIN et al.** *Biochim. Biophys. Acta,* 19 November 1997
- **PINEIRO-SANCHEZ et al.** *J. Biol. Chem.,* 1997, vol. 272, 7595-7601
- **WELT et al.** *J. Clin. Oncol.,* 1994, vol. 12, 1193-203
- **NARRA et al.** *Cancer Biol. Ther.,* 2007, vol. 6, 1691-9
- **HENRY et al.** *Clinical Cancer Research,* 2007, vol. 13, 1736-1741
- **BASERGA et al.** *Biochim. Biophys. Acta,* 1997, vol. 1332, F105-126
- **BASERGA R.** *Exp. Cell. Res.,* 1999, vol. 253, 1-6
- **PERUZZI F. et al.** *J. Cancer Res. Clin. Oncol.,* 1999, vol. 125, 166-173
- **VALENTINIS B. et al.** *J. Biol. Chem.,* 1999, vol. 274, 12423-12430
- **PRISCO M. et al.** *Horm. Metab. Res.,* 1999, vol. 31, 80-89
- **SELL C. et al.** *Proc. Natl. Acad. Sci.,* 1993, vol. 90, 11217-11221
- **SELL C. et al.** *Mol. Cell. Biol.,* 1994, vol. 14, 3604-3612
- **MORRIONE A. J.** *Virol.,* 1995, vol. 69, 5300-5303
- **COPPOLA D. et al.** *Mol. Cell. Biol.,* 1994, vol. 14, 458A-4595
- **DEANGELIS T et al.** *J. Cell. Physiol.,* 1995, vol. 164, 214-221
- **ARTEAGA C. et al.** *Cancer Res.,* 1989, vol. 49, 6237-6241
- **LI et al.** *Biochem. Biophys. Res. Com.,* 1993, vol. 196, 92-98
- **ZIA F et al.** *J. Cell. Biol.,* 1996, vol. 24, 269-275
- **SCOTLANDI K et al.** *Cancer Res.,* 1998, vol. 58, 4127-4131
- **JIANG et al.** *Oncogene,* 1999, vol. 18, 6071-6077

- **AMARA et al.** *Cancer Res,* 2001, vol. 61, 4660-4665
- **ARGANI et al.** *Cancer Res,* 2001, vol. 61, 4320-4324
- **DALERBA et al.** *PNAS,* 2007, vol. 104, 10158-63
- **DALERBA et al.** *Ann. Rev. Med.,* 2007, vol. 58, 267-84
- **SCANLAN et al.** *PNAS,* 1994, vol. 91, 5657-5661
- *Lancet,* 2006, vol. 368, 2206-7
- **WILDMAN et al.** *PNAS,* 2003, vol. 100, 7181
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **HARLOW ; LANE.** Antibodies: A Laboratory Manual. Cold Spring Harbor Laboratory Press, 1988
- Using Antibodies: A Laboratory Manual. Cold Spring Harbor Laboratory Press, 1999
- **SAMBROOK et al.** Molecular Cloning: A Laboratory Manual. Cold Spring Harbor Laboratory Press, 2001
- **MILSTEIN ; KÖHLER.** *Nature,* 1975, vol. 256, 495-7
- **NEEDLEMAN ; WUNSCH.** *J. Mol. Biol.,* 1970, vol. 48, 443-453
- **SMITH ; WATERMAN.** *Adv. Appl. Math,* 1981, vol. 2, 482-489
- **CRICK.** *J Mol Biol,* 1966, vol. 19, 548-55, http://en.wikipedia.org/wiki/Wobble_Hypothesis
- **MADISON.** Genetics Computer Group. *Science Drive,* 1991, vol. 575
- **NEEDLEMAN.** *Wunsch J. Mol. Biol.,* 1970, vol. 48, 443-453
- **AUSUBEL.** Current Protocols in Molecular Biology. Green Publishing Associates and Wiley Interscience, 1989
- **MACK et al.** *PNAS,* 1995, vol. 92, 7021-7025
- **RAUM et al.** *Cancer Immunol Immunother,* 2001, vol. 50 (3), 141-150
- **SMITH.** *J. Virol.,* 1983, vol. 46, 584
- **ENGELHARD.** *Proc. Nat. Acad. Sci. USA,* 1994, vol. 91, 3224-3227
- **REISS.** Plant Physiol. *Life Sci. Adv.,* 1994, vol. 13, 143-149
- **HERRERA-ESTRELLA.** *EMBO J.,* 1983, vol. 2, 987-995
- **MARSH.** *Gene,* 1984, vol. 32, 481-485
- **HARTMAN.** *Proc. Natl. Acad. Sci. USA,* 1988, vol. 85, 8047
- **MCCONLOGUE.** In: Current Communications in Molecular Biology. Cold Spring Harbor Laboratory, 1987
- **TAMURA.** *Biosci. Biotechnol. Biochem.,* 1995, vol. 59, 2336-2338
- **GIACOMIN, PI.** *Sci.,* 1996, vol. 116, 59-72
- **SCIKANTHA.** *J. Bact.,* 1996, vol. 178, 121
- **GERDES.** *FEBS Lett.,* 1996, vol. 389, 44-47
- **JEFFERSON.** *EMBO J.,* 1987, vol. 6, 3901-3907
- **GIORDANO.** *Nature Medicine,* 1996, vol. 2, 534-539
- **SCHAPER.** *Circ. Res.,* 1996, vol. 79, 911-919
- **ANDERSON.** *Science,* 1992, vol. 256, 808-813
- **VERMA.** *Nature,* 1994, vol. 389, 239
- **ISNER.** *Lancet,* 1996, vol. 348, 370-374
- **MUHLHAUSER.** *Circ. Res.,* 1995, vol. 77, 1077-1086
- **ONODERA.** *Blood,* 1998, vol. 91, 30-36
- **VERMA.** *Gene Ther.,* 1998, vol. 5, 692-699
- **NABEL.** *Ann. N.Y. Acad. Sci.,* 1997, vol. 811, 289-292
- **VERZELETTI.** *Hum. Gene Ther.,* 1998, vol. 9, 2243-51
- **WANG.** *Nature Medicine,* 1996, vol. 2, 714-716
- **SCHAPER.** *Current Opinion in Biotechnology,* 1996, vol. 7, 635-640
- *Santos Coura and Nardi Virol J.,* 2007, vol. 4, 99
- **KROOS.** *Biotechnol. Prog.,* 2003, vol. 19, 163-168
- **SCOPES.** Protein Purification. Springer-Verlag, 1982
- **HERMANSON.** Bioconjugate Techniques. Academic Press, Inc, 1996
- Phage Display: A Laboratory Manual. Cold Spring Harbor Laboratory Press, 2001
- **KNAPPIK et al.** *J Mol. Biol.,* 2000, vol. 296, 57 ff
- **HOLT et al.** *Trends Biotechnol.,* 2003, vol. 21, 484 ff
- **MUYLDERMANS.** *J Biotechnol.,* vol. 74, 277
- **DE GENST et al.** *Dev Como Immunol.,* 2006, vol. 30, 187 ff
- **GROVES ; OSBOURN.** *Expert Opin Biol Ther.,* 2005, vol. 5, 125 ff

- **LIPOVSEK ; PLUCKTHUN.** *J Immunol Methods,* 2004, vol. 290, 52 ff
- **SCHLERETH et al.** *Cancer Immunol. Immunother,* 2005, vol. 20, 1-12
- **CHESON BD ; HORNING SJ ; COIFFIER B ; SHIPP MA ; FISHER RI ; CONNORS JM ; LISTER TA ; VOSE J ; GRILLO-LOPEZ A ; HAGENBEEK A.** Report of an international workshop to standardize response criteria for non-Hodgkin's lymphomas. NCI Sponsored International Working Group. *J Clin Oncol.,* April 1999, vol. 17 (4), 1244
- **SCHLERETH et al.** *Cancer Immunol. Immunother.,* 2005, vol. 20, 1-12
- **LEFKOVITS.** Immunology Methods Manual; The Comprehensive Sourcebook of Techniques. Academic Press, 1997
- **GOLEMIS.** Protein-Protein Interactions: A Molecular Cloning Manual. Cold Spring Laboratory Press, 2002
- **GIL et al.** *Cell,* 2002, vol. 109, 901
- **LEGATE et al.** *Nat Rev Mol Cell Biol,* 2006, vol. 7, 20
- **MACK et al.** *Proc. Natl. Acad. Sci. USA,* 1995, vol. 92, 7021-7025
- **RAUM et al.** *Cancer Immunol Immunother,* 2001, vol. 50, 141-150
- **RAUM et al.** *Cancer Immunol Immunother.,* 2001, vol. 50 (3), 141-50
- **SAMBROOK.** Molecular Cloning; A Laboratory Manual. Cold Spring Harbour Laboratory Press, 2001
- **KAUFMANN R.J.** *Methods Enzymol.,* 1990, vol. 185, 537-566
- **FICKENSCHER.** Virology. Hygiene Institute
- **FICKENSCHER H.** *Blood,* 2000, vol. 95, 3256-61
- **COLIGAN ; KRUISBEEK ; MARGULIES ; SHEVACH ; STROBER.** Current Protocols in Immunology. Wiley-Interscience, 2002
- **LOFFLER.** *Blood,* 2000, vol. 95 (6
- **MONACO et al.** *J Leukoc Biol,* 2002, vol. 71, 659-668
- **SCHLERETH.** *Cancer Res,* 2005, vol. 65, 2882
- **FICKENSCHER H.** Herpesvirus saimiri-transformed macaque T cells are tolerated and do not cause lymphoma after autologous reinfusion. *Blood,* 2000, vol. 95, 3256-61
- Molecular Cloning. **SAMBROOK.** A Laboratory Manual. Cold Spring Harbour Laboratory Press, 2001